# EUROPEAN PATENT SPECIFICATION

(11) **EP 4 041 229 B1**
(45) Date of publication and mention of the grant of the patent: **24.06.2026**
(21) Application number: 20874462.3
(22) Date of filing: 06.10.2020
(51) Int. Cl.: A61K 31/4245, A61K 31/433, A61K 45/06, C07D 205/04, A61P 25/28

(54) **ARYLMETHYLENE HETEROCYCLIC COMPOUNDS AS KV1.3 POTASSIUM SHAKER CHANNEL BLOCKERS**
ARYLMETHYLENHETEROZYKLISCHE VERBINDUNGEN ALS KV1.3-KALIUMSCHÜTTELKANALBLOCKER
COMPOSÉS HÉTÉROCYCLIQUES D'ARYLMÉTHYLÈNE UTILISÉS EN TANT QUE BLOQUEURS DES CANAUX POTASSIQUES KV1.3 DE TYPE SHAKER

(30) Priority: 07.10.2019 US 201962911655 P
(43) Date of publication of application: 17.08.2022
(73) Proprietor: D.E. Shaw Research, LLC, New York, New York 10036 (US)
(72) Inventor: GIORDANETTO, Fabrizio, New York, NY 10036 (US); JENSEN, Morten, Østergaard, 2900 Hellerup (DK); JOGINI, Vishwanath, Hyderabad 500034 (IN); SNOW, Roger, John, Danbury, CT 06811 (US)
(74) Representative: J A Kemp LLP
(86) International application number: PCT/US2020/054352
(87) International publication number: WO 2021/071806

(56) References cited:
- WO-A1-2006/042150
- WO-A1-2017/216280
- WO-A1-2020/035424
- WO-A2-2006/029182
- WO-A2-2007/117482
- US-A1- 2018 273 476
- UTO Y ET AL: "Novel benzoylpiperidine-based stearoyl-CoA desaturase-1 inhibitors: Identification of 6-[4-(2-methylbenzoyl)piperidin-1-yl]pyridazine-3-carboxylic acid (2-hydroxy-2-pyridin-3-ylethyl)amide and its plasma triglyceride-lowering effects in Zucker fatty rats", BIOORGANIC & MEDICINAL CHEMISTRY LETTERS, ELSEVIER, AMSTERDAM NL, vol. 20, no. 1, 1 January 2010 (2010-01-01), pages 341 - 345, XP026808839, ISSN: 0960-894X, [retrieved on 20091029]
- MEANWELL N A ET AL: "N-benzylated benzimidazol-2-one derivatives: activators of large-conductance Ca^2^+-dependent K^+ channels", BIOORGANIC & MEDICINAL CHEMISTRY LETTERS, ELSEVIER, AMSTERDAM NL, vol. 6, no. 14, 23 July 1996 (1996-07-23), pages 1641 - 1646, XP004134913, ISSN: 0960-894X, DOI: 10.1016/0960-894X(96)00296-X
- DATABASE PubChem 13 January 2016 (2016-01-13), retrieved from NCBI Database accession no. 282860499
- DATABASE Pubchem Compound 21 October 2014 (2014-10-21), "1-(Piperidin-4-ylmethyl)naphthalen-2-ol", XP055817188, retrieved from NCBI Database accession no. 84801094

## Description

This patent disclosure contains material that is subject to copyright protection. The copyright owner has no objection to the facsimile reproduction of the patent document or the patent disclosure as it appears in the U.S. Patent and Trademark Office patent file or records, but otherwise reserves any and all copyright rights.

### FIELD OF THE INVENTION

The invention relates generally to the field of pharmaceutical science. More particularly, the invention relates to compounds and compositions useful as pharmaceuticals as potassium channel blockers.

### BACKGROUND

Voltage-gated Kv1.3 potassium (K⁺) channels are expressed in lymphocytes (T and B lymphocytes), the central nervous system, and other tissues and regulate a large number of physiological processes such as neurotransmitter release, heart rate, insulin secretion, and neuronal excitability. Kv1.3 channels can regulate membrane potential and thereby indirectly influence calcium signaling in human effector memory T cells. Effector memory T cells are mediators of several conditions, including multiple sclerosis, Type I diabetes mellitus, psoriasis, spondylitis, parodontitis, and rheumatoid arthritis. Upon activation, effector-memory T cells increase expression of the Kv1.3 channel. Amongst human B cells, naive and early memory B cells express small numbers of Kv1.3 channels when they are quiescent. In contrast, class-switched memory B cells express high numbers of Kv1.3 channels. Furthermore, the Kv1.3 channel promotes the calcium homeostasis required for T-cell receptor-mediated cell activation, gene transcription, and proliferation (Panyi, G., et al., 2004, Trends Immunol., 565-569). Blockade of Kv1.3 channels in effector memory T cells suppresses activities like calcium signaling, cytokine production (interferon-gamma, interleukin 2) and cell proliferation.

Autoimmune Disease is a family of disorders resulting from tissue damage caused by attack from the body's own immune system. Such diseases may affect a single organ, as in multiple sclerosis and Type I diabetes mellitus, or may involve multiple organs as in the case of rheumatoid arthritis and systemic lupus erythematosus. Treatment is generally palliative, with anti-inflammatory and immunosuppressive drugs, which can have severe side effects. A need for more effective therapies has led to search for drugs that can selectively inhibit the function of effector memory T cells, known to be involved in the etiology of autoimmune diseases. These inhibitors are thought to be able to ameliorate autoimmune diseases symptoms without compromising the protective immune response. Effector memory T cells (TEMs) express high numbers of the Kv1.3 channel and depend on these channels for their function. *In vivo,* Kv1.3 channel blockers paralyze TEMs at the sites of inflammation and prevent their reactivation in inflamed tissues. Kv1.3 channel blockers do not affect the motility within lymph nodes of naive and central memory T cells. Suppressing the function of these cells by selectively blocking the Kv1.3 channel offers the potential for effective therapy of autoimmune diseases with minimal side effects.

Multiple Sclerosis (MS) is caused by autoimmune damage to the Central Nervous System (CNS). Symptoms include muscle weakness and paralysis, which severely affect quality of life for patients. MS progresses rapidly and unpredictably and eventually leads to death. The Kv1.3 channel is also highly expressed in auto-reactive effector memory T cells from MS patients (Wulff H., et al., 2003, J. Clin. Invest., 1703-1713; Rus H., et al., 2005, PNAS, 11094-11099). Animal models of multiple sclerosis have been successfully treated using blockers of the Kv1.3 channel.

Compounds which are selective Kv1.3 channel blockers are thus potential therapeutic agents as immunosuppressants or immune system modulators. The Kv1.3 channel is also considered as a therapeutic target for the treatment of obesity and for enhancing peripheral insulin sensitivity in patients with type-2 diabetes mellitus. These compounds can also be utilized in the prevention of graft rejection, and the treatment of immunological (*e.g*., autoimmune) and inflammatory disorders.

Tubulointerstitial fibrosis is a progressive connective tissue deposition on the kidney parenchyma, leading to renal function deterioration and is involved in the pathology of chronic kidney disease, chronic renal failure, nephritis, and inflammation in glomeruli and is a common cause of end-stage renal failure. Overexpression of Kv1.3 channels in lymphocytes can promote their proliferation leading to chronic inflammation and overstimulation of cellular immunity, which are involved in the underlying pathology of these renal diseases and are contributing factors in the progression of tubulointerstitial fibrosis. Inhibition of the lymphocyte Kv1.3 channel currents suppress proliferation of kidney lymphocytes and ameliorate the progression of renal fibrosis (Kazama I., et al., 2015, Mediators Inflamm., 1-12).

Kv1.3 channels also play a role in gastroenterological disorders including inflammatory bowel diseases (IBD) such as ulcerative colitis (UC) and Crohn's disease. Ulcerative colitis is a chronic IBD characterized by excessive T-cell infiltration and cytokine production. Ulcerative colitis can impair quality of life and can lead to life-threatening complications. High levels of Kv1.3 channels in CD4 and CD8 positive T-cells in the inflamed mucosa of UC patients have been associated with production of pro-inflammatory compounds in active UC. Kv1.3 channels are thought to serve as a marker of disease activity and pharmacological blockade might constitute a novel immunosuppressive strategy in UC. Present treatment regimens for UC, including corticosteroids, salicylates, and anti-TNF-α reagents, are insufficient for many patients (Hansen L.K., et al., 2014, J. Crohns Colitis, 1378-1391). Crohn's disease is a type of IBD which may affect any part of the gastrointestinal tract. Crohn's disease is thought to be the result of intestinal inflammation due to a T-cell-driven process initiated by normally safe bacteria. Thus, Kv1.3 channel inhibition can be utilized in treating the Crohn's disease.

In addition to T cells, Kv1.3 channels are also expressed in microglia, where the channel is involved in inflammatory cytokine and nitric oxide production and in microglia-mediated neuronal killing. In humans, strong Kv1.3 channel expression has been found in microglia in the frontal cortex of patients with Alzheimer's disease and on CD68⁺ cells in multiple sclerosis brain lesions. It has been suggested that Kv1.3 channel blockers might be able to preferentially target detrimental proinflammatory microglia functions. Kv1.3 channels are expressed on activated microglia in infarcted rodent and human brain. Higher Kv1.3 channel current densities are observed in acutely isolated microglia from the infarcted hemisphere than in microglia isolated from the contralateral hemisphere of a mouse model of stroke (Chen Y.J., et al., 2017, Ann. Clin. Transl. Neurol., 147-161).

Expression of Kv1.3 channels is elevated in microglia of human Alzheimer's disease brains, suggesting that Kv1.3 channel is a pathologically relevant microglial target in Alzheimer's disease (Rangaraju S., et al., 2015, J. Alzheimers Dis., 797-808). Soluble AβO enhances microglial Kv1.3 channel activity. Kv1.3 channels are required for AβO-induced microglial pro-inflammatory activation and neurotoxicity. Kv1.3 channel expression/activity is upregulated in transgenic Alzheimer's disease animals and human Alzheimer's disease brains. Pharmacological targeting of microglial Kv1.3 channels can affect hippocampal synaptic plasticity and reduce amyloid deposition in APP/PS1 mice. Thus, Kv1.3 channel may be a therapeutic target for Alzheimer's disease.

Kv1.3 channel blockers could be also useful for ameliorating pathology in cardiovascular disorders such as ischemic stroke, where activated microglia significantly contributes to the secondary expansion of the infarct.

Kv1.3 channel expression is associated with the control of proliferation in multiple cell types, apoptosis, and cell survival. These processes are crucial for cancer progression. In this context, Kv1.3 channels located in the inner mitochondrial membrane can interact with the apoptosis regulator Bax (Serrano-Albarras, A., et al., 2018, Expert Opin. Ther. Targets, 101-105). Thus, inhibitors of Kv1.3 channels may be used as anticancer agents.

A number of peptide toxins with multiple disulfide bonds from spiders, scorpions, and anemones are known to block Kv1.3 channels. A few selective, potent peptide inhibitors of the Kv1.3 channel have been developed. A synthetic derivative of stichodactyla toxin (shk) with an unnatural amino acid (shk-186) is the most advanced peptide toxin. Shk has demonstrated efficacy in preclinical models and is currently in a phase I clinical trial for treatment of psoriasis. Shk can suppress proliferation of TEM cells resulting in improved condition in animal models of multiple sclerosis. Unfortunately, Shk also binds to the closely-related Kvi channel subtype found in CNS and heart. There is a need for Kv1.3 channel-selective inhibitors to avoid potential cardio- and neuro-toxicity. Additionally, small peptides like shk-186 are rapidly cleared from the body after administration, resulting in short circulating half-lives, frequent administration events. Thus, there is a need for the development of long-acting, selective Kv1.3 channel inhibitors for the treatment of chronic inflammatory diseases.

Thus, there remains a need for development of novel Kv1.3 channel blockers as pharmaceutical agents. WO 2006/042150 A1 describes diaminoalkane aspartic protease inhibitors allegedly useful in the treatment or amelioration of diseases associated with elevated levels of aspartic protease activity. WO 2007/117482 A2 describes renin inhibitors allegedly useful in the treatment or amelioration of diseases associated with aspartic protease activity. WO 2006/029182 A2. UTO Y ET AL: "Novel benzoylpiperidine-based stearoyl-CoA desaturase-1 inhibitors: Identification of 6-[4-(2-methylbenzoyl)piperidin-1-yl]pyridazine-3-carboxylic acid (2-hydroxy-2-pyridin-3-ylethyl)amide and its plasma triglyceride-lowering effects in Zucker fatty rats", BIOORGANIC & MEDICINAL CHEMISTRY LETTERS, vol. 20, no. 1, 1 January 2010, pages 341-345 describes benzoylpiperidine-based stearoyl-CoA desaturase-1 inhibitors and identifies 6-[4-(2-methylbenzoyl)piperidin-1-yl]pyridazine-3-carboxylic acid (2-hydroxy-2-pyridin-3-ylethyl)amide and its alleged plasma triglyceride-lowering effects in Zucker fatty rats. WO 2020/035424 A1 describes heterocyclic compounds as monoacylglycerol lipase inhibitors. MEANWELL N A ET AL: "N-benzylated benzimidazol-2-one derivatives: activators of large-conductance Ca2+ dependent K+ channels", BIOORGANIC & MEDICINAL CHEMISTRY LETTERS, vol. 6, no. 14, 1996, pages 1641-1646 describes a series of N-benzylated benzimidazol-2-one derivatives and their alleged use as potassium channel modulators.

### SUMMARY OF THE INVENTION

In one aspect, compounds useful as potassium channel blockers having a structure of Formula I, 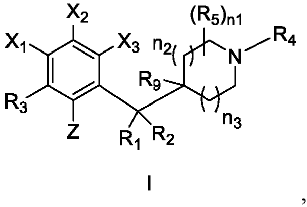 are described, where the various substituents are defined herein. The compounds of Formula I described herein can block Kv1.3 potassium (K⁺) channels and be used in the treatment of a variety of conditions. Methods for synthesizing these compounds are also described herein. Pharmaceutical compositions and methods of using these compositions described herein are useful for treating conditions *in vitro* and *in vivo.* Such compounds, pharmaceutical compositions, and methods of treatment have a number of clinical applications, including as pharmaceutically active agents and methods for treating cancer, an immunological disorder, a Central Nerve System (CNS) disorder, an inflammatory disorder, a gastroenterological disorder, a metabolic disorder, a cardiovascular disorder, a kidney disease or a combination thereof. For clarity, all references herein to methods of treatment utilizing a particular compound or composition are to be interpreted as references to the compound or composition for use in that method of treatment.

In one aspect of the presently claimed invention, a compound of Formula I or a pharmaceutically acceptable salt thereof is described, 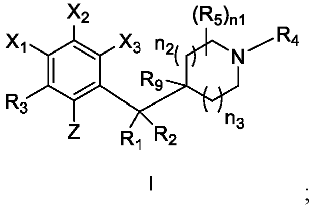 wherein:
the structural moiety 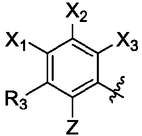 has the structure of 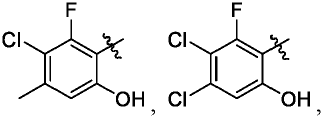 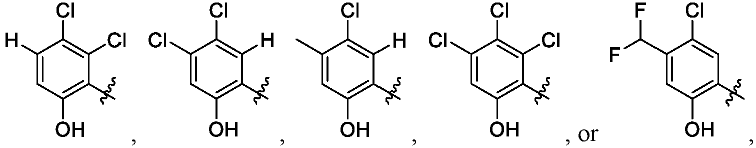 each of which is substituted by R₃;
R₃ is H, halogen, or alkyl;
R₁ and R₂ are each independently H, alkyl, (CR₆R₇)ₙ₄ORₐ, (CR₆R₇)ₙ₄NRₐR_{b}, (CR₆R₇)ₙ₄NRₐ(C=O)R_{b}, (CR₆R₇)ₙ₄NRₐSO₂R_{b}, or (CR₆R₇)ₙ₄CONRₐR_{b}, wherein at least one occurrence of R₁ and R₂ is (CR₆R₇)ₙ₄NRₐR_{b}, (CR₆R₇)ₙ₄ORₐ, (CR₆R₇)ₙ₄NRₐ(C=O)R_{b}, (CR₆R₇)ₙ₄NRₐSO₂R_{b}, or (CR₆R₇)ₙ₄CONRₐR_{b}; or alternatively R₁, R₂ and the carbon atom they are connected to taken together form a 3-5 membered carbocycle;
R₄ is (CR₆R₇)ₙ₄(C=O)R_{c}, (C=O)(CR₆R₇)ₙ₄R_{c}, (CR₆R₇)ₙ₄OR_{c}, (CR₆R₇)ₙ₄COOR_{c}, (CR₆R₇)ₙ₄NR_{c}(C=O)R_{d}, (C=O)(CR₆R₇)ₙ₄OR_{c}, (CR₆R₇)ₙ₄SO₂R_{c}, optionally substituted saturated heterocycle, optionally substituted aryl, optionally substituted heteroaryl, optionally substituted cycloalkyl, or optionally substituted -cycloalkyl-alkyl;
each occurrence of R₅ is independently H, alkyl, cycloalkyl, or oxo;
or two R₅ groups taken together with the carbon atom(s) that they are connected to form a 3-7 membered optionally substituted saturated carbocycle;
or two R₅ groups are connected to different carbon atoms on the ring and taken together form a bond or an alkyl chain containing 1-3 carbons;
each occurrence of R₆ and R₇ are independently H, alkyl, or cycloalkyl;
each occurrence of Rₐ and R_{b} are independently H, alkyl, cycycloalkyl, saturated heterocycle, aryl, or heteroaryl; or alternatively Rₐ and R_{b} together with the nitrogen atom that they are connected to form an optionally substituted heterocycle including the nitrogen atom and 0-3 additional heteroatoms each selected from the group consisting of N, O, and S;
each occurrence of R_{c} and R_{d} are independently H, alkyl, alkyl substituted by 1-4 substituents each of which is independently halogen, OR₈ or N(R₈)₂, alkenyl, optionally substituted cycloalkyl, optionally substituted bicycloalkyl, optionally substituted spiroalkyl, optionally substituted saturated heterocycle, optionally substituted aryl, optionally substituted heteroaryl, optionally substituted -alkyl-aryl, optionally substituted -alkyl-heteroaryl, optionally substituted -alkyl-heterocycle, optionally substituted -alkyl-cycloalkyl, or optionally substituted -cycloalkyl-alkyl; or alternatively R_{c} and R_{d} together with the nitrogen atom that they are connected to form an optionally substituted heterocycle including the nitrogen atom and 0-3 additional heteroatoms each selected from the group consisting of N, O, and S;
each occurrence of R₈ is independently H, alkyl, or an optionally substituted heterocycle; or alternatively the two R₈ groups together with the nitrogen atom that they are connected to form an optionally substituted heterocycle including the nitrogen atom and 0-3 additional heteroatoms each selected from the group consisting of N, O, and S;
R₉ is H, alkyl, halogen, or (CR₆R₇)ₙ₄OR_{b};
the alkyl, cycloalkyl, spiroalkyl, bicycloalkyl, heterocycle, aryl, and heteroaryl in R₁, R₂, R₃, R₄, R₅, R₆, R₇, R₉, Rₐ, R_{b}, R_{c}, and R_{d}, where applicable, are optionally substituted by 1-4 substituents each independently selected from the group consisting of alkyl, cycloalkyl, halogenated cycloalkyl, halogenated alkyl, halogen, CN, -(CH₂)₀₋₂OR₈, N(R₈)₂, (C=O)C₁₋₄alkyl, (C=O)N(R₈)₂, and oxo where valence permits;
each occurrence of n₁ is independently an integer from 0-3 where valence permits;
each occurrence of n₂ and n₃ is independently an integer that is 1, 2 or 0; and
each occurrence of n₄ is independently an integer from 0-3.

In any one of the embodiments described herein, each occurrence of n₂ and n₃ is independently an integer from 0-1.

In any one of the embodiments described herein, the structural motif 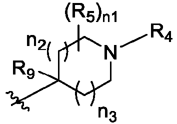 has the structure of

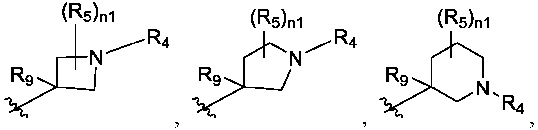

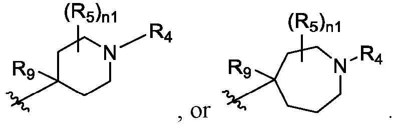

In any one of the embodiments described herein, the structural motif 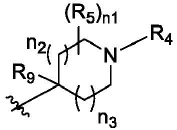 has the structure of

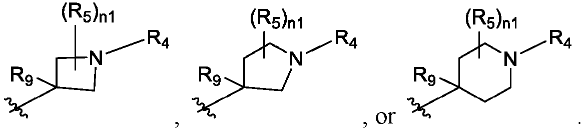

In any one of the embodiments described herein, the structural motif 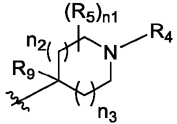 has the structure of

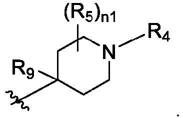

In any one of the embodiments described herein, at least one occurrence of R₁ and R₂ is H or alkyl.

In any one of the embodiments described herein, at least one occurrence of R₁ and R₂ is (CR₆R₇)ₙ₄ORₐ, (CR₆R₇)ₙ₄NRₐR_{b}, (CR₆R₇)ₙ₄NRₐ(C=O)R_{b}, (CR₆R₇)ₙ₄NRₐSO₂R_{b}, or (CR₆R₇)ₙ₄CONRₐR_{b}.

In any one of the embodiments described herein, at least one occurrence of R₁ and R₂ is ORₐ or NRₐR_{b}.

In any one of the embodiments described herein, R₁, R₂, and the carbon atom they are connected to taken together form a 3-5 membered carbocycle.

In any one of the embodiments described herein, R₁ and R₂ are each independently H, Me, OH, CH₂OH, NH₂, NHMe, NMe₂, CH₂NH₂, CONH₂, CONHMe₂, CONMe₂, NH(C=O)Me, NMe(C=O)Me, 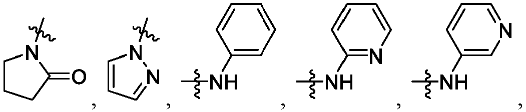 or

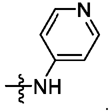

In any one of the embodiments described herein, R₄ is (CR₆R₇)ₙ₄OR_{c}, (CR₆R₇)ₙ₄COR_{c}, (C=O)(CR₆R₇)ₙ₄R_{c}, (CR₆R₇)ₙ₄COOR_{c}, (CR₆R₇)ₙ₄NR_{c}(C=O)R_{d}, (C=O)(CR₆R₇)ₙ₄OR_{c} or (CR₆R₇)ₙ₄SO₂R_{c}.

In any one of the embodiments described herein, R₄ is (CR₆R₇)₂OR_{c}, (C=O)R_{c}, (C=O)(CR₆R₇)₁₋₂R_{c}, COOR_{c}, (CR₆R₇)₁₋₂NR_{c}(C=O)R_{d}, (C=O)(CR₆R₇)₁₋₂OR_{c} or SO₂R_{c}.

In any one of the embodiments described herein, R₄ is (CH₂)₂OH, (CH₂)₂OMe, (C=O)H, (C=O)Me, (C=O)CH₂OH, (C=O)CH₂OMe, (C=O)Et, (C=O)Ph, (C=O)isopropyl, (C=O)CH(OH)CH₂OH, (C=O)CH(OMe)CH₂OH, (C=O)CH(OH)CH₂OMe (C=O)OMe, SO₂Me, SO₂Et, SO₂CH₂OH or SO₂CH₂OMe.

In any one of the embodiments described herein, R₄ is (C=O)R_{c}, (C=O)(CR₆R₇)₁₋₂R_{c}, (C=O)(CR₆R₇)₁₋₂OR_{c}, or SO₂R_{c}; and where R_{c} is selected from the group consisting of H, alkyl, alkyl substituted by 1-4 substituents each independently selected from the group consisting of halogen, OR₈ and N(R₈)₂, alkenyl, optionally substituted cycloalkyl, optionally substituted bicycloalkyl, optionally substituted spiroalkyl, optionally substituted saturated heterocycle, optionally substituted aryl, optionally substituted heteroaryl, optionally substituted -alkyl-aryl, optionally substituted -alkyl-heteroaryl, optionally substituted -alkyl-heterocycle, optionally substituted -alkyl-cycloalkyl, and optionally substituted -cycloalkyl-alkyl.

In any one of the embodiments described herein, R_{c} or R_{d} is H, Me, Et,

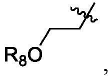

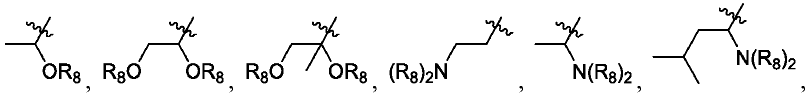

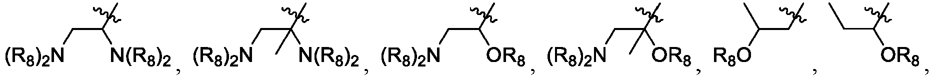

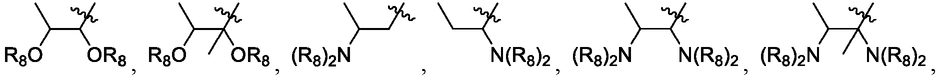

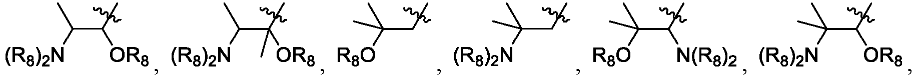

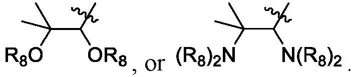

In any one of the embodiments described herein, R_{c} or R_{d} is a heterocycle selected from the group consisting of 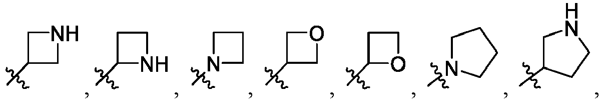 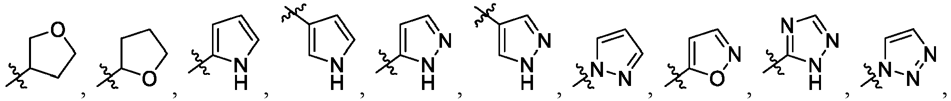 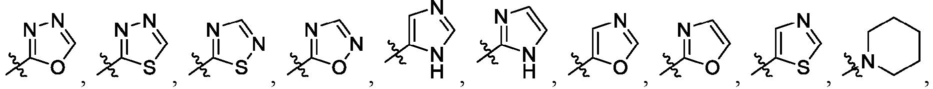 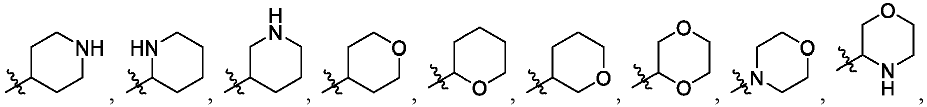 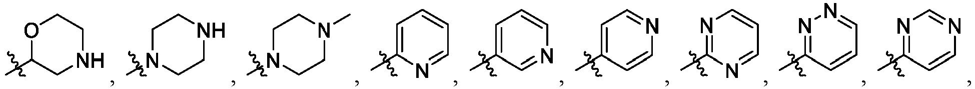 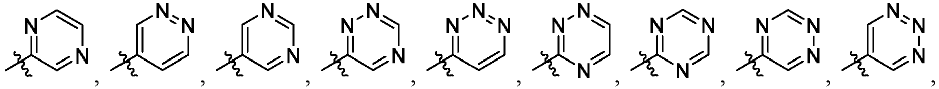 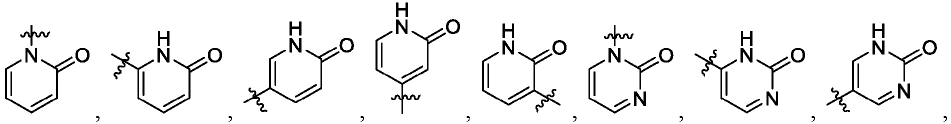 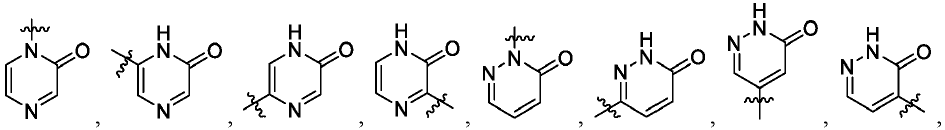 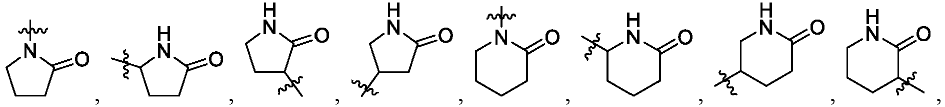 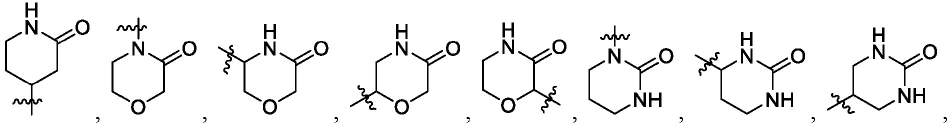 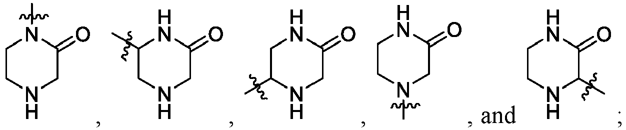 where the heterocycle is optionally substituted by one or more cyano, cycloalkyl, fluorinated alkyl, fluorinated cycloalkyl, halogen, OH, NH₂, oxo, or (C=O)C₁₋₄alkyl where valence permits.

In any one of the embodiments described herein, R_{c} is cycloalkyl, spiroalkyl, or bicycloalkyl each optionally substituted by 1-4 substituents each independently selected from the group consisting of alkyl, halogen, CN, -(CH₂)₀₋₂OR₈, N(R₈)₂, (C=O)N(R₈)₂, and oxo where valence permits.

In any one of the embodiments described herein, R_{c} is 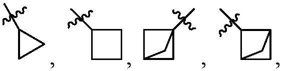 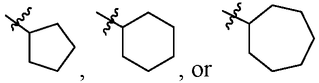 each optionally substituted by 1-4 substituents each independently selected from the group consisting of alkyl, halogen, CN, -(CH₂)₀₋₂OR₈, N(R₈)₂, (C=O)N(R₈)₂, and oxo where valence permits.

In any one of the embodiments described herein, R₄ is optionally substituted saturated heterocycle, optionally substituted aryl, optionally substituted heteroaryl, optionally substituted cycloalkyl or optionally substituted -cycloalkyl-alkyl.

In any one of the embodiments described herein, R₄ is a heterocycle selected from the group consisting of 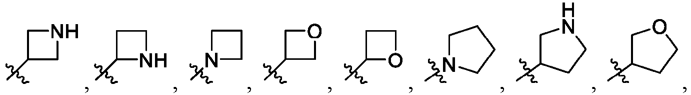 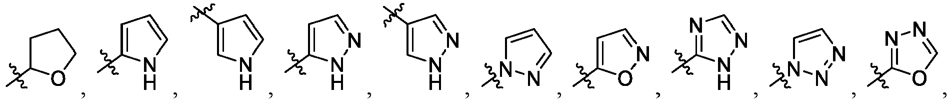 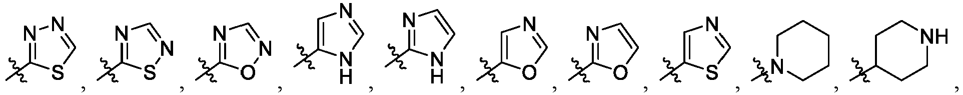 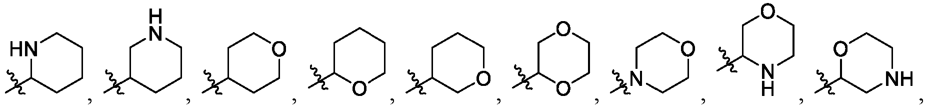 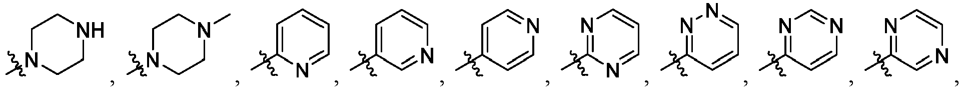 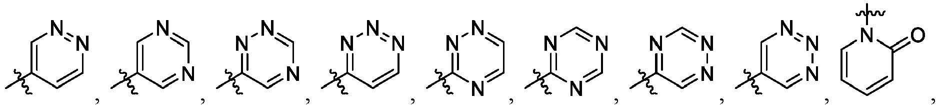 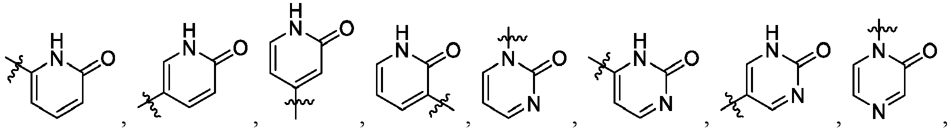 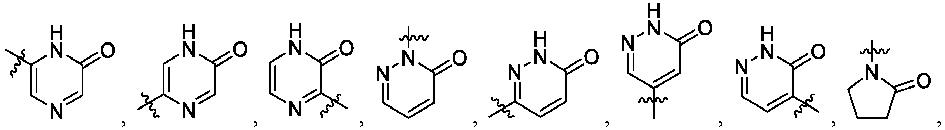 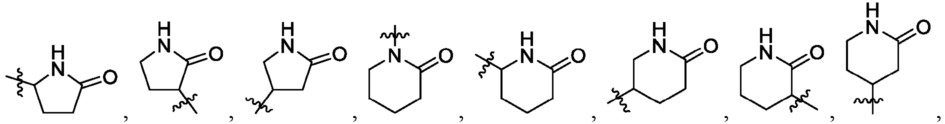 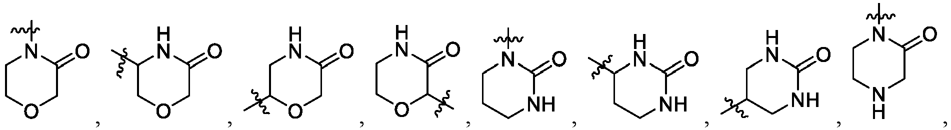 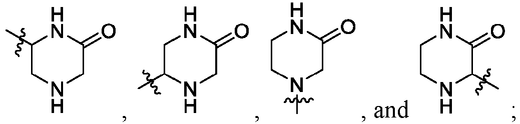 where the heterocycle is optionally substituted by one or more cyano, cycloalkyl, fluorinated alkyl, fluorinated cycloalkyl, halogen, OH, NH₂, oxo, or (C=O)C₁₋₄alkyl where valence permits.

In any one of the embodiments described herein, R₄ is cycloalkyl optionally substituted by 1-4 substituents each independently selected from the group consisting of alkyl, halogen, CN, -(CH₂)₀₋₂OR₈, N(R₈)₂, (C=O)N(R₈)₂, and oxo where valence permits.

In any one of the embodiments described herein, R₄ is 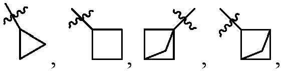 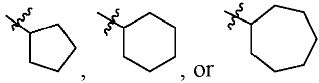 each optionally substituted by 1-4 substituents each independently selected from the group consisting of alkyl, halogen, CN, -(CH₂)₀₋₂OR₈, N(R₈)₂, (C=O)N(R₈)₂, and oxo where valence permits.

In any one of the embodiments described herein, R₄ is 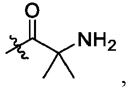 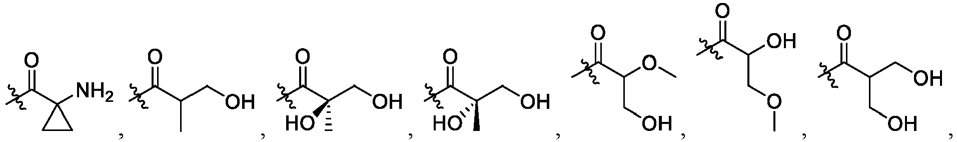 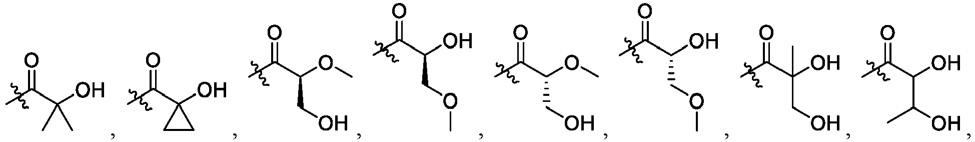 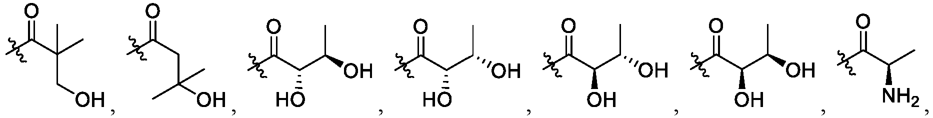 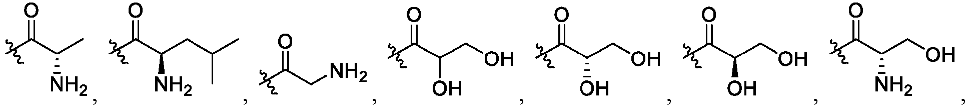 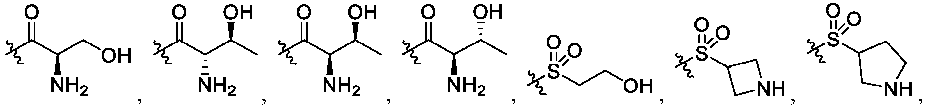 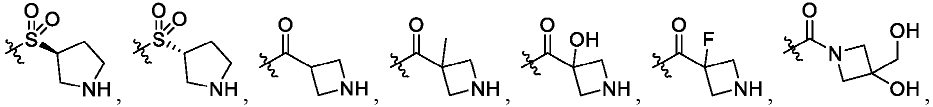 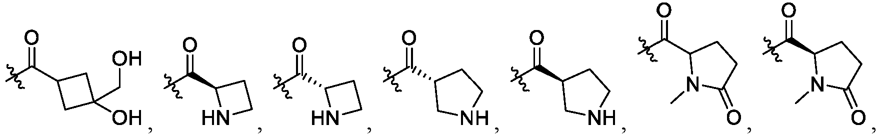 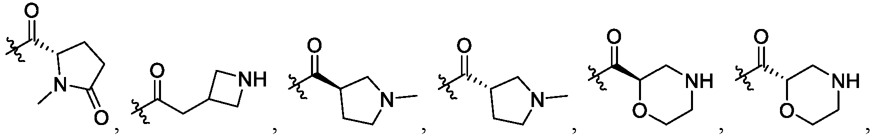 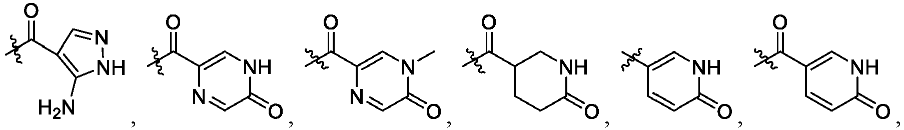 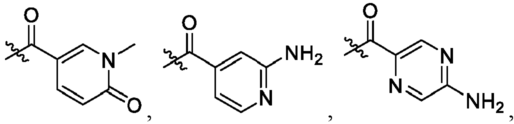 or a tautomer thereof.

In any one of the embodiments described herein, at least one occurrence of R₅ is H, alkyl or cycloalkyl.

In any one of the embodiments described herein, at least one occurrence of R₅ is oxo.

In any one of the embodiments described herein, two R₅ groups are connected to different carbon atoms on the ring and taken together form a bond or an alkyl chain containing 1-3 carbons.

In any one of the embodiments described herein, two R₅ groups taken together with the carbon atom(s) that they are connected to form a 3-7 membered optionally substituted saturated carbocycle.

In any one of the embodiments described herein, at least one of Rₐ and R_{b} is independently H, alkyl, cycloalkyl, saturated heterocycle, aryl, or heteroaryl.

In any one of the embodiments described herein, Rₐ and R_{b} together with the nitrogen atom that they are connected to form an optionally substituted heterocycle including the nitrogen atom and 0-3 additional heteroatoms each selected from the group consisting of N, O, and S.

In any one of the embodiments described herein, each occurrence of R₆ and R₇ are independently H or alkyl.

In any one of the embodiments described herein, R₉ is H, alkyl, or halogen.

In any one of the embodiments described herein, R₉ is (CR₆R₇)ₙ₄OR_{b}.

In any one of the embodiments described herein, R₉ is H, F, or OH.

In any one of the embodiments described herein, R₃ is H or alkyl.

In any one of the embodiments described herein, R₃ is halogen.

In any one of the embodiments described herein, at least one occurrence of R₈ is H, alkyl, or optionally substituted heterocycle.

In any one of the embodiments described herein, R₈ is H, Me, Et, Pr, Bu, or a heterocycle selected from the group consisting of 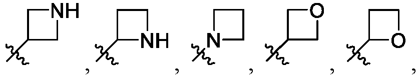 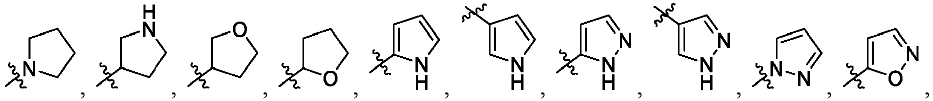 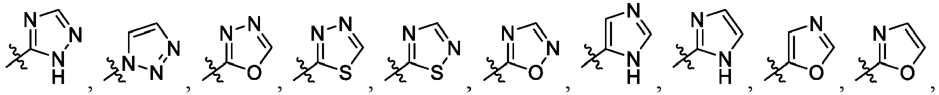 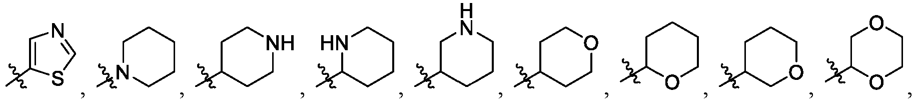 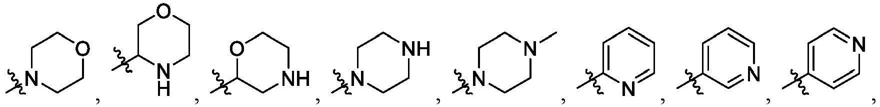 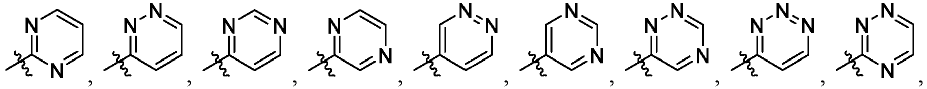 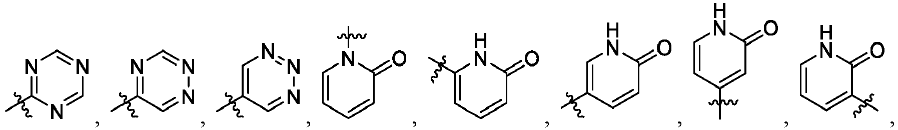 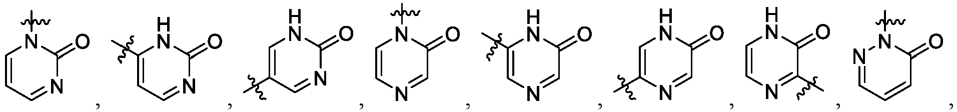 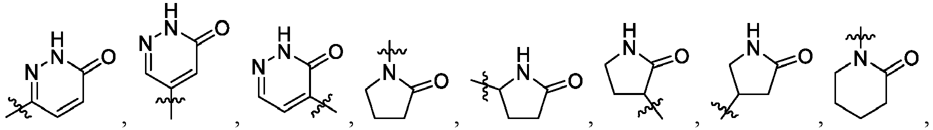 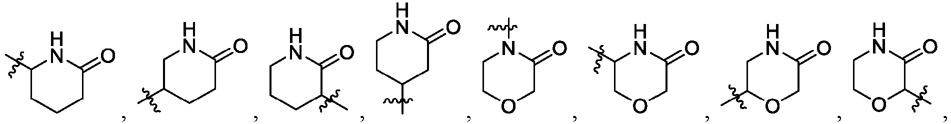 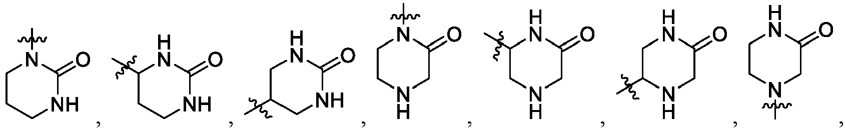 and 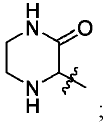 where the heterocycle is optionally substituted by one or more cyano, cycloalkyl, fluorinated alkyl, fluorinated cycloalkyl, halogen, OH, NH₂, oxo, or (C=O)C₁₋₄alkyl where valence permits.

In any one of the embodiments described herein, the two R₈ groups together with the nitrogen atom that they are connected to form an optionally substituted heterocycle including the nitrogen atom and 0-3 additional heteroatoms each selected from the group consisting of N, O, and S.

In any one of the embodiments described herein, n₁ is 0, 1, 2, or 3.

In any one of the embodiments described herein, n₄ is 0, 1, or 2.

In any one of the embodiments described herein, at least one occurrence of R_{c} or R_{d} is independently H, alkyl, alkyl substituted by 1-4 substituents each independently selected from the group consisting of halogen, OR₈ and N(R₈)₂, alkenyl, optionally substituted cycloalkyl, optionally substituted bicycloalkyl, optionally substituted spiroalkyl, optionally substituted saturated heterocycle, optionally substituted aryl, optionally substituted heteroaryl, optionally substituted -alkyl-aryl, optionally substituted -alkyl-heteroaryl, optionally substituted -alkyl-heterocycle, optionally substituted -alkyl-cycloalkyl, or optionally substituted -cycloalkyl-alkyl.

In any one of the embodiments described herein, R_{c} and R_{d} together with the nitrogen atom that they are connected to form an optionally substituted heterocycle including the nitrogen atom and 0-3 additional heteroatoms each selected from the group consisting of N, O, and S.

In any one of the embodiments described herein, at least one occurrence of R_{c} or R_{d} is independently H, Me, Et,

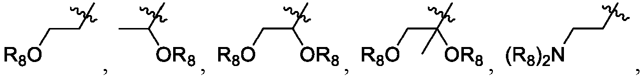

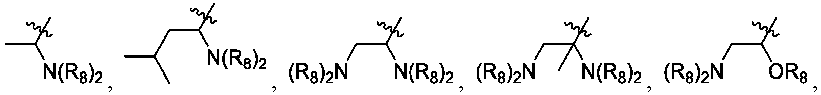

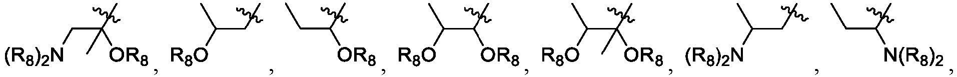

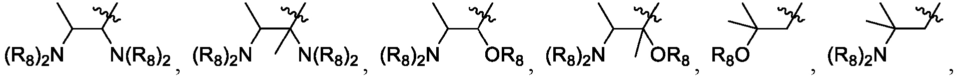

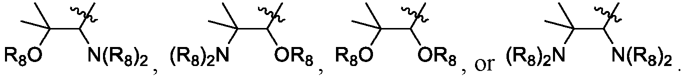

In any one of the embodiments described herein, at least one occurrence of R_{c} or R_{d} is independently a heterocycle selected from the group consisting of 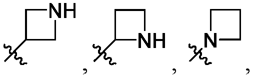 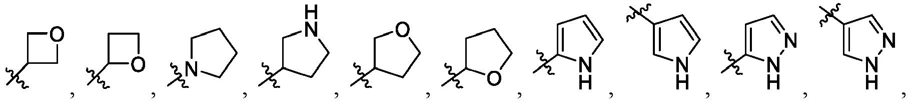 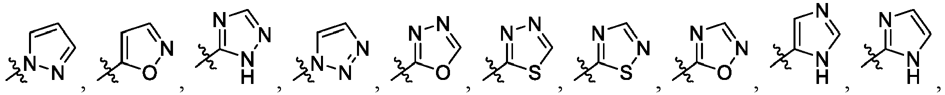 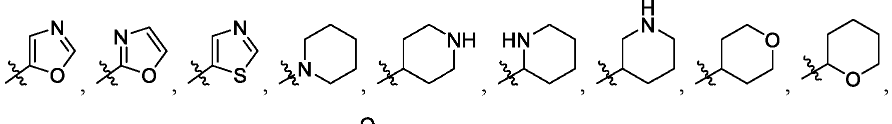 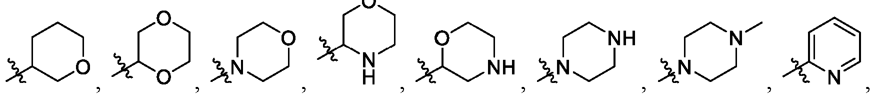 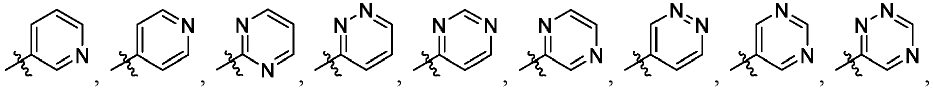 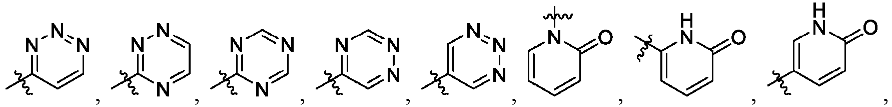 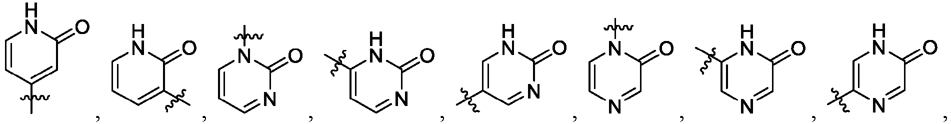 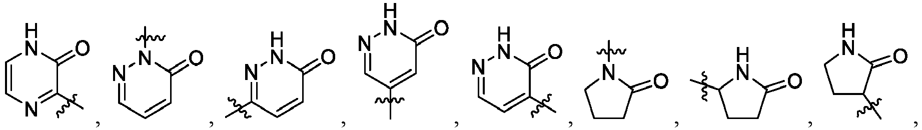 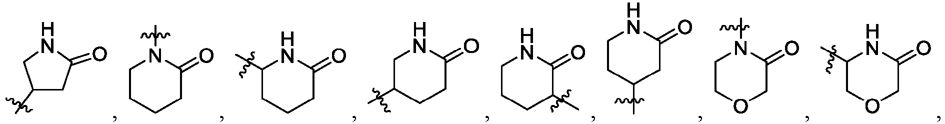 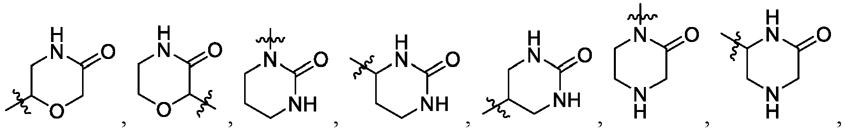 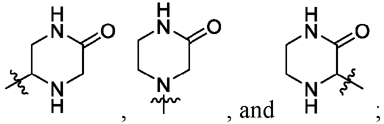 where the heterocycle is optionally substituted by one or more cyano, cycloalkyl, fluorinated alkyl, fluorinated cycloalkyl, halogen, OH, NH₂, oxo, or (C=O)C₁₋₄alkyl where valence permits.

In any one of the embodiments described herein, at least one occurrence of R_{c} or R_{d} is independently cycloalkyl, spiroalkyl, or bicycloalkyl each optionally substituted by 1-4 substituents each independently selected from the group consisting of alkyl, halogen, CN, -(CH₂)₀₋₂OR₈, N(R₈)₂, (C=O)N(R₈)₂, and oxo where valence permits.

In any one of the embodiments described herein, at least one occurrence of R_{c} or R_{d} is independently 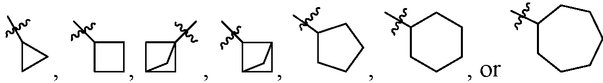 each optionally substituted by 1-4 substituents each independently selected from the group consisting of alkyl, halogen, CN, -(CH₂)₀₋₂OR₈, N(R₈)₂, (C=O)N(R₈)₂, and oxo where valence permits.

In any one of the embodiments described herein, the compound is a specific compound as set out in the appended set of claims or a pharmaceutically acceptable salt thereof.

In another aspect of the presently claimed invention, a pharmaceutical composition is described, including at least one compound according to any one of the embodiments described herein or a pharmaceutically acceptable salt thereof and a pharmaceutically acceptable carrier or diluent.

In yet another aspect of the presently claimed invention, there is described a compound according to any one of the embodiments described herein or a pharmaceutically-acceptable salt thereof for use as a medicament.
In yet another aspect of the presently claimed invention, there is described a compound according to any one of the embodiments described herein or a pharmaceutically-acceptable salt thereof for use in a method of treating a condition in a mammalian species in need thereof, the method including administering to the mammalian species a therapeutically effective amount of the compound or a pharmaceutically acceptable salt thereof, where the condition is selected from the group consisting of cancer, an immunological disorder, a Central Nerve System (CNS) disorder, an inflammatory disorder, a gastroenterological disorder, a metabolic disorder, a cardiovascular disorder, and a kidney disease.

In any one of the embodiments described herein, the condition is an immunological disorder that is transplant rejection or an autoimmune disease.

In any one of the embodiments described herein, the condition is an immunological disorder that is an autoimmune disease that is is rheumatoid arthritis, multiple sclerosis, systemic lupus erythematosus, or Type I diabetes mellitus.

In any one of the embodiments described herein, the condition is a Central Nerve System (CNS) disorder that is Alzheimer's disease.

In any one of the embodiments described herein, the condition is an inflammatory disorder that is an inflammatory skin condition, arthritis, psoriasis, spondylitis, parodontitits, or an inflammatory neuropathy.

In any one of the embodiments described herein, the condition is a gastroenterological disorder that is an inflammatory bowel disease.

In any one of the embodiments described herein, the condition is a metabolic disorder that is obesity or Type II diabetes mellitus.

In any one of the embodiments described herein, the condition is a cardiovascular disorder that is an ischemic stroke.

In any one of the embodiments described herein, the condition is a kidney disease that is chronic kidney disease, nephritis, or chronic renal failure.

In any one of the embodiments described herein, the condition is selected from the group consisting of cancer, transplant rejection, rheumatoid arthritis, multiple sclerosis, systemic lupus erythematosus, Type I diabetes mellitus, Alzheimer's disease, inflammatory skin condition, inflammatory neuropathy, psoriasis, spondylitis, parodontitis, Crohn's disease, ulcerative colitis, obesity, Type II diabetes mellitus, ischemic stroke, chronic kidney disease, nephritis, chronic renal failure, and a combination thereof.

In any one of the embodiments described herein, the mammalian species is human.

Any one of the embodiments disclosed herein may be properly combined with any other embodiment disclosed herein. The combination of any one of the embodiments disclosed herein with any other embodiments disclosed herein is expressly contemplated. Specifically, the selection of one or more embodiments for one substituent group can be properly combined with the selection of one or more particular embodiments for any other substituent group. Such combination can be made in any one or more embodiments of the application described herein or any formula described herein.

### DETAILED DESCRIPTION OF THE INVENTION

### Definitions

The following are definitions of terms used in the present specification. The initial definition provided for a group or term herein applies to that group or term throughout the present specification individually or as part of another group, unless otherwise indicated. Unless otherwise defined, all technical and scientific terms used herein have the same meaning as commonly understood by one of ordinary skill in the art.

The terms "alkyl" and "alk" refer to a straight or branched chain alkane (hydrocarbon) radical containing from 1 to 12 carbon atoms, preferably 1 to 6 carbon atoms. Exemplary "alkyl" groups include methyl, ethyl, propyl, isopropyl, n-butyl, t-butyl, isobutyl pentyl, hexyl, isohexyl, heptyl, 4,4-dimethylpentyl, octyl, 2,2,4-trimethylpentyl, nonyl, decyl, undecyl, dodecyl, and the like. The term "(C₁₋C₄)alkyl" refers to a straight or branched chain alkane (hydrocarbon) radical containing from 1 to 4 carbon atoms, such as methyl, ethyl, propyl, isopropyl, n-butyl, t-butyl, and isobutyl. "Substituted alkyl" refers to an alkyl group substituted with one or more substituents, preferably 1 to 4 substituents, at any available point of attachment. Exemplary substituents include, but are not limited, to one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e.,* =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. In some embodiments, groups such as alkyl, cycloalkyl, alkenyl, alkynyl, cycloalkenyl, heterocycle and aryl can themselves be optionally substituted.

The term "alkenyl" refers to a straight or branched chain hydrocarbon radical containing from 2 to 12 carbon atoms and at least one carbon-carbon double bond. Exemplary such groups include ethenyl or allyl. The term "C₂-C₆ alkenyl" refers to a straight or branched chain hydrocarbon radical containing from 2 to 6 carbon atoms and at least one carbon-carbon double bond, such as ethylenyl, propenyl, 2-propenyl, (*E*)-but-2-enyl, (*Z*)-but-2-enyl, 2-methy(*E*)-but-2-enyl, 2-methy(*Z*)-but-2-enyl, 2,3-dimethy-but-2-enyl, (*Z*)-pent-2-enyl, (*E*)-pent-1-enyl, (*Z*)-hex-1-enyl, (*E*)-pent-2-enyl, (*Z*)-hex-2-enyl, (*E*)-hex-2-enyl, (*Z*)-hex-1-enyl, (*E*)-hex-1-enyl, (*Z*)-hex-3-enyl, (*E*)-hex-3-enyl, and (*E*)-hex-1,3-dienyl. "Substituted alkenyl" refers to an alkenyl group substituted with one or more substituents, preferably 1 to 4 substituents, at any available point of attachment. Exemplary substituents include, but are not limited, to one or more of the following groups: hydrogen, halogen, alkyl, halogenated alkyl (*i.e.,* an alkyl group bearing a single halogen substituent or multiple halogen substituents such as CF₃ or CCl₃), cyano, nitro, oxo (*i.e.*, =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{d}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted.

The term "alkynyl" refers to a straight or branched chain hydrocarbon radical containing from 2 to 12 carbon atoms and at least one carbon to carbon triple bond. Exemplary such groups include ethynyl. The term "C₂-C₆ alkynyl" refers to a straight or branched chain hydrocarbon radical containing from 2 to 6 carbon atoms and at least one carbon-carbon triple bond, such as ethynyl, prop-1-ynyl, prop-2-ynyl, but-1-ynyl, but-2-ynyl, pent-1-ynyl, pent-2-ynyl, hex-1-ynyl, hex-2-ynyl, hex-3-ynyl. "Substituted alkynyl" refers to an alkynyl group substituted with one or more substituents, preferably 1 to 4 substituents, at any available point of attachment. Exemplary substituents include, but are not limited to, one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e.*, =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{d}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted.

The term "cycloalkyl" refers to a fully saturated cyclic hydrocarbon group containing from 1 to 4 rings and 3 to 8 carbons per ring. "C₃-C₇ cycloalkyl" refers to cyclopropyl, cyclobutyl, cyclopentyl, cyclohexyl, or cycloheptyl. "Substituted cycloalkyl" refers to a cycloalkyl group substituted with one or more substituents, preferably 1 to 4 substituents, at any available point of attachment. Exemplary substituents include, but are not limited to, one or more of the following groups: hydrogen, halogen (*e.g.,* a single halogen substituent or multiple halo substituents forming, in the latter case, groups such as CF₃ or an alkyl group bearing CCl₃₎, cyano, nitro, oxo (*i.e*., =O), CF₃, OCF3, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted. Exemplary substituents also include spiro-attached or fused cyclic substituents, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The term "cycloalkenyl" refers to a partially unsaturated cyclic hydrocarbon group containing 1 to 4 rings and 3 to 8 carbons per ring. Exemplary such groups include cyclobutenyl, cyclopentenyl, cyclohexenyl, etc. "Substituted cycloalkenyl" refers to a cycloalkenyl group substituted with one more substituents, preferably 1 to 4 substituents, at any available point of attachment. Exemplary substituents include, but are not limited to, one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e*., =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted. Exemplary substituents also include spiro-attached or fused cyclic substituents, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The term "aryl" refers to cyclic, aromatic hydrocarbon groups that have 1 to 5 aromatic rings, especially monocyclic or bicyclic groups such as phenyl, biphenyl or naphthyl. Where containing two or more aromatic rings (bicyclic, *etc*.), the aromatic rings of the aryl group may be joined at a single point (*e.g.,* biphenyl), or fused (*e.g.,* naphthyl, phenanthrenyl and the like). The term "fused aromatic ring" refers to a molecular structure having two or more aromatic rings wherein two adjacent aromatic rings have two carbon atoms in common. "Substituted aryl" refers to an aryl group substituted by one or more substituents, preferably 1 to 3 substituents, at any available point of attachment. Exemplary substituents include, but are not limited to, one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e*., =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted. Exemplary substituents also include fused cyclic groups, especially fused cycloalkyl, fused cycloalkenyl, fused heterocycle, or fused aryl, where the aforementioned cycloalkyl, cycloalkenyl, heterocycle and aryl substituents can themselves be optionally substituted.

The term "biaryl" refers to two aryl groups linked by a single bond. The term "biheteroaryl" refers to two heteroaryl groups linked by a single bond. Similarly, the term "heteroaryl-aryl" refers to a heteroaryl group and an aryl group linked by a single bond and the term "aryl-heteroaryl" refers to an aryl group and a heteroaryl group linked by a single bond. In certain embodiments, the numbers of the ring atoms in the heteroaryl and/or aryl rings are used to specify the sizes of the aryl or heteroaryl ring in the substituents. For example, 5,6-heteroaryl-aryl refers to a substituent in which a 5-membered heteroaryl is linked to a 6-membered aryl group. Other combinations and ring sizes can be similarly specified.

The term "carbocycle" or "carbon cycle" refers to a fully saturated or partially saturated cyclic hydrocarbon group containing from 1 to 4 rings and 3 to 8 carbons per ring, or cyclic, aromatic hydrocarbon groups that have 1 to 5 aromatic rings, especially monocyclic or bicyclic groups such as phenyl, biphenyl or naphthyl. The term "carbocycle" encompasses cycloalkyl, cycloalkenyl, cycloalkynyl and aryl as defined hereinabove. The term "substituted carbocycle" refers to carbocycle or carbocyclic groups substituted with one or more substituents, preferably 1 to 4 substituents, at any available point of attachment. Exemplary substituents include, but are not limited to, those described above for substituted cycloalkyl, substituted cycloalkenyl, substituted cycloalkynyl and substituted aryl. Exemplary substituents also include spiro-attached or fused cyclic substituents at any available point or points of attachment, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The terms "heterocycle" and "heterocyclic" refer to fully saturated, or partially or fully unsaturated, including aromatic (*i.e.,* "heteroaryl") cyclic groups (for example, 3 to 7 membered monocyclic, 7 to 11 membered bicyclic, or 8 to 16 membered tricyclic ring systems) which have at least one heteroatom in at least one carbon atom-containing ring. Each ring of the heterocyclic group may independently be saturated, or partially or fully unsaturated. Each ring of the heterocyclic group containing a heteroatom may have 1, 2, 3, or 4 heteroatoms selected from the group consisting of nitrogen atoms, oxygen atoms and sulfur atoms, where the nitrogen and sulfur heteroatoms may optionally be oxidized and the nitrogen heteroatoms may optionally be quaternized. (The term "heteroarylium" refers to a heteroaryl group bearing a quaternary nitrogen atom and thus a positive charge.) The heterocyclic group may be attached to the remainder of the molecule at any heteroatom or carbon atom of the ring or ring system. Exemplary monocyclic heterocyclic groups include azetidinyl, pyrrolidinyl, pyrrolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolodinyl, 2-oxoazepinyl, azepinyl, hexahydrodiazepinyl, 4-piperidonyl, pyridyl, pyrazinyl, pyrimidinyl, pyridazinyl, triazinyl, triazolyl, tetrazolyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl, and the like. Exemplary bicyclic heterocyclic groups include indolyl, indolinyl, isoindolyl, benzothiazolyl, benzoxazolyl, benzoxadiazolyl, benzothienyl, benzo[*d*][1,3]dioxolyl, dihydro-2H-benzo[*b*][1,4]oxazine, 2,3-dihydrobenzo[b][1,4]dioxinyl, quinuclidinyl, quinolinyl, tetrahydroisoquinolinyl, isoquinolinyl, benzimidazolyl, benzopyranyl, indolizinyl, benzofuryl, benzofurazanyl, dihydrobenzo[*d*]oxazole, chromonyl, coumarinyl, benzopyranyl, cinnolinyl, quinoxalinyl, indazolyl, pyrrolopyridyl, furopyridinyl (such as furo[2,3-c]pyridinyl, furo[3,2-b]pyridinyl] or furo[2,3-b]pyridinyl), dihydroisoindolyl, dihydroquinazolinyl (such as 3,4-dihydro-4-oxo-quinazolinyl), triazinylazepinyl, tetrahydroquinolinyl and the like. Exemplary tricyclic heterocyclic groups include carbazolyl, benzidolyl, phenanthrolinyl, acridinyl, phenanthridinyl, xanthenyl and the like.

"Substituted heterocycle" and "substituted heterocyclic" (such as "substituted heteroaryl") refer to heterocycle or heterocyclic groups substituted with one or more substituents, preferably 1 to 4 substituents, at any available point of attachment. Exemplary substituents include, but are not limited to, one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e*., =O), CF₃, OCF₃, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. The exemplary substituents can themselves be optionally substituted. Exemplary substituents also include spiro-attached or fused cyclic substituents at any available point or points of attachment, especially spiro-attached cycloalkyl, spiro-attached cycloalkenyl, spiro-attached heterocycle (excluding heteroaryl), fused cycloalkyl, fused cycloalkenyl, fused heterocycle, or fused aryl, where the aforementioned cycloalkyl, cycloalkenyl, heterocycle and aryl substituents can themselves be optionally substituted.

The term "oxo" refers to 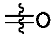 substituent group, which may be attached to a carbon ring atom on a carbocycle or heterocycle. When an oxo substituent group is attached to a carbon ring atom on an aromatic group, *e.g.,* aryl or heteroaryl, the bonds on the aromatic ring may be re-arranged to satisfy the valence requirement. For instance, a pyridine with a 2-oxo substituent group may have the structure of 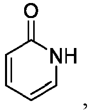 which also includes its tautomeric form of

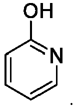

The term "alkylamino" refers to a group having the structure -NHR', wherein R' is hydrogen, alkyl or substituted alkyl, cycloalkyl or substituted cycloalkyl, as defined herein. Examples of alkylamino groups include, but are not limited to, methylamino, ethylamino, n-propylamino, iso-propylamino, cyclopropylamino, n-butylamino, tert-butylamino, neopentylamino, n-pentylamino, hexylamino, cyclohexylamino, and the like.

The term "dialkylamino" refers to a group having the structure -NRR', wherein R and R' are each independently alkyl or substituted alkyl, cycloalkyl or substituted cycloalkyl, cycloalkenyl or substituted cyclolalkenyl, aryl or substituted aryl, heterocycle or substituted heterocycle, as defined herein. R and R' may be the same or different in a dialkyamino moiety. Examples of dialkylamino groups include, but are not limited to, dimethylamino, methyl ethylamino, diethylamino, methylpropylamino, di(n-propyl)amino, di(iso-propyl)amino, di(cyclopropyl)amino, di(n-butyl)amino, di(tert-butyl)amino, di(neopentyl)amino, di(n-pentyl)amino, di(hexyl)amino, di(cyclohexyl)amino, and the like. In certain embodiments, R and R' are linked to form a cyclic structure. The resulting cyclic structure may be aromatic or non-aromatic. Examples of the resulting cyclic structure include, but are not limited to, aziridinyl, pyrrolidinyl, piperidinyl, morpholinyl, pyrrolyl, imidazolyl, 1,3,4-triazinolyl, and tetrazolyl.

The terms "halogen" or "halo" refer to chlorine, bromine, fluorine or iodine.

The term "substituted" refers to the embodiments in which a molecule, molecular moiety or substituent group (*e.g.,* alkyl, cycloalkyl, alkenyl, cycloalkenyl, alkynyl, heterocycle, or aryl group or any other group disclosed herein) is substituted with one or more substituents, where valence permits, preferably 1 to 6 substituents, at any available point of attachment. Exemplary substituents include, but are not limited to, one or more of the following groups: hydrogen, halogen (*e.g.,* a single halogen substituent or multiple halo substituents forming, in the latter case, groups such as CF₃ or an alkyl group bearing CCl₃), cyano, nitro, oxo (*i.e*., =O), CF₃, OCF₃, alkyl, halogen-substituted alkyl, cycloalkyl, alkenyl, cycloalkenyl, alkynyl, heterocycle, aryl, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein each occurrence of Rₐ is independently hydrogen, alkyl, cycloalkyl, alkenyl, cycloalkenyl, alkynyl, heterocycle, or aryl; each occurrence of R_{b}, R_{c} and R_{d} is independently hydrogen, alkyl, cycloalkyl, heterocycle, aryl, or said R_{b} and R_{c} together with the N to which they are bonded optionally form a heterocycle; and each occurrence of Rₑ is independently alkyl, cycloalkyl, alkenyl, cycloalkenyl, alkynyl, heterocycle, or aryl. In the aforementioned exemplary substituents, groups such as alkyl, cycloalkyl, alkenyl, alkynyl, cycloalkenyl, heterocycle and aryl can themselves be optionally substituted. The term "optionally substituted" refers to the embodiments in which a molecule, molecular moiety or substituent group (*e.g.,* alkyl, cycloalkyl, alkenyl, cycloalkenyl, alkynyl, heterocycle, or aryl group or any other group disclosed herein) may or may not be substituted with aforementioned one or more substituents.

Unless otherwise indicated, any heteroatom with unsatisfied valences is assumed to have hydrogen atoms sufficient to satisfy the valences.

The compounds of the present invention may form salts which are also within the scope of this invention. Reference to a compound of the present invention is understood to include reference to salts thereof, unless otherwise indicated. The term "salt(s)", as employed herein, denotes acidic and/or basic salts formed with inorganic and/or organic acids and bases. In addition, when a compound of the present invention contains both a basic moiety, such as but not limited to a pyridine or imidazole, and an acidic moiety such as but not limited to a carboxylic acid, zwitterions ("inner salts") may be formed and are included within the term "salt(s)" as used herein. Pharmaceutically acceptable (*i.e.,* non-toxic, physiologically acceptable) salts are preferred, although other salts are also useful, *e.g.,* in isolation or purification steps which may be employed during preparation. Salts of the compounds of the present invention may be formed, for example, by reacting a compound described herein with an amount of acid or base, such as an equivalent amount, in a medium such as one in which the salt precipitates or in an aqueous medium followed by lyophilization.

The compounds of the present invention which contain a basic moiety, such as but not limited to an amine or a pyridine or imidazole ring, may form salts with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, hydroxyethanesulfonates (*e.g.,* 2-hydroxyethanesulfonates), lactates, maleates, methanesulfonates, naphthalenesulfonates (*e.g.,* 2-naphthalenesulfonates), nicotinates, nitrates, oxalates, pectinates, persulfates, phenylpropionates (*e.g*., 3-phenylpropionates), phosphates, picrates, pivalates, propionates, salicylates, succinates, sulfates (such as those formed with sulfuric acid), sulfonates, tartrates, thiocyanates, toluenesulfonates such as tosylates, undecanoates, and the like.

The compounds of the present invention which contain an acidic moiety, such but not limited to a phenol or carboxylic acid, may form salts with a variety of organic and inorganic bases. Exemplary basic salts include ammonium salts, alkali metal salts such as sodium, lithium and potassium salts, alkaline earth metal salts such as calcium and magnesium salts, salts with organic bases (for example, organic amines) such as benzathines, dicyclohexylamines, hydrabamines (formed with N,N-bis(dehydroabietyl) ethylenediamine), N-methyl-D-glucamines, N-methyl-D-glycamides, t-butyl amines, and salts with amino acids such as arginine, lysine and the like. Basic nitrogen-containing groups may be quaternized with agents such as lower alkyl halides (*e.g*., methyl, ethyl, propyl, and butyl chlorides, bromides and iodides), dialkyl sulfates (*e.g*., dimethyl, diethyl, dibutyl, and diamyl sulfates), long chain halides (*e.g.,* decyl, lauryl, myristyl and stearyl chlorides, bromides and iodides), aralkyl halides (*e.g.,* benzyl and phenethyl bromides), and others.

Compounds of the present invention, and salts or solvates thereof, may exist in their tautomeric form (for example, as an amide or imino ether). All such tautomeric forms are contemplated herein as part of the present invention. As used herein, any depicted structure of the compound includes the tautomeric forms thereof.

All stereoisomers of the present compounds (for example, those which may exist due to asymmetric carbons on various substituents), including enantiomeric forms and diastereomeric forms, are contemplated within the scope of this invention. Individual stereoisomers of the compounds of the invention may, for example, be substantially free of other isomers (*e.g*., as a pure or substantially pure optical isomer having a specified activity), or may be admixed, for example, as racemates or with all other, or other selected, stereoisomers. The chiral centers of the present invention may have the S or R configuration as defined by the International Union of Pure and Applied Chemistry (IUPAC) 1974 Recommendations. The racemic forms can be resolved by physical methods, such as, for example, fractional crystallization, separation or crystallization of diastereomeric derivatives or separation by chiral column chromatography. The individual optical isomers can be obtained from the racemates by any suitable method, including without limitation, conventional methods, such as, for example, salt formation with an optically active acid followed by crystallization.

Compounds of the present invention are, subsequent to their preparation, preferably isolated and purified to obtain a composition containing an amount by weight equal to or greater than 90%, for example, equal to greater than 95%, equal to or greater than 99% of the compounds ("substantially pure" compounds), which is then used or formulated as described herein. Such "substantially pure" compounds of the present invention are also contemplated herein as part of the present invention.

All configurational isomers of the compounds of the present invention are contemplated, either in admixture or in pure or substantially pure form. The definition of compounds of the present invention embraces both *cis* (*Z*) and *trans* (*E*) alkene isomers, as well as *cis* and *trans* isomers of cyclic hydrocarbon or heterocyclic rings.

Throughout the specification, groups and substituents thereof may be chosen to provide stable moieties and compounds.

Definitions of specific functional groups and chemical terms are described in more detail herein. For purposes of this invention, the chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in "Organic Chemistry", Thomas Sorrell, University Science Books, Sausalito (1999).

Certain compounds of the present invention may exist in particular geometric or stereoisomeric forms. The present invention contemplates all such compounds, including *cis-* and *trans*-isomers, *R*- and *S*-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

Isomeric mixtures containing any of a variety of isomer ratios may be utilized in accordance with the present invention. For example, where only two isomers are combined, mixtures containing 50:50, 60:40, 70:30, 80:20, 90:10, 95:5, 96:4, 97:3, 98:2, 99:1, or 100:0 isomer ratios are all contemplated by the present invention. Those of ordinary skill in the art will readily appreciate that analogous ratios are contemplated for more complex isomer mixtures.

The present invention also includes isotopically labeled compounds, which are identical to the compounds disclosed herein, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, sulfur, fluorine and chlorine, such as ²H, ³H ¹³C, ¹¹C, ¹⁴C, ¹⁵N, ¹⁸O, ¹⁷O, ³¹P, ³²P, ³⁵S, ¹⁸F, and ³⁶Cl, respectively. Compounds of the present invention, or an enantiomer, diastereomer, tautomer, or pharmaceutically acceptable salt or solvate thereof, which contain the aforementioned isotopes and/or other isotopes of other atoms are within the scope of this invention. Certain isotopically labeled compounds of the present invention, for example, those into which radioactive isotopes such as ³H and ¹⁴C are incorporated, are useful in drug and/or substrate tissue distribution assays. Tritiated, *i.e.,* ³H, and carbon-14, *i.e.,* ¹⁴C, isotopes are particularly preferred for their ease of preparation and detectability. Further, substitution with heavier isotopes such as deuterium, *i.e.,* ²H, can afford certain therapeutic advantages resulting from greater metabolic stability, for example, increased in vivo half-life or reduced dosage requirements and, hence, may be preferred in some circumstances. Isotopically labeled compounds can generally be prepared by carrying out the procedures disclosed in the Schemes and/or in the Examples below, by substituting a readily available isotopically labeled reagent for a non-isotopically labeled reagent.

If, for instance, a particular enantiomer of a compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

It will be appreciated that the compounds, as described herein, may be substituted with any number of substituents or functional moieties. In general, the term "substituted" whether preceded by the term "optionally" or not, and substituents contained in formulas of this invention, refer to the replacement of hydrogen radicals in a given structure with the radical of a specified substituent. When more than one position in any given structure may be substituted with more than one substituent selected from a specified group, the substituent may be either the same or different at every position. As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. For purposes of this invention, heteroatoms such as nitrogen may have hydrogen substituents and/or any permissible substituents of organic compounds described herein which satisfy the valences of the heteroatoms. Furthermore, this invention is not intended to be limited in any manner by the permissible substituents of organic compounds. Combinations of substituents and variables envisioned by this invention are preferably those that result in the formation of stable compounds useful in the treatment, for example, of proliferative disorders. The term "stable", as used herein, preferably refers to compounds which possess stability sufficient to allow manufacture and which maintain the integrity of the compound for a sufficient period of time to be detected and preferably for a sufficient period of time to be useful for the purposes detailed herein.

As used herein, the terms "cancer" and, equivalently, "tumor" refer to a condition in which abnormally replicating cells of host origin are present in a detectable amount in a subject. The cancer can be a malignant or non-malignant cancer. Cancers or tumors include, but are not limited to, biliary tract cancer; brain cancer; breast cancer; cervical cancer; choriocarcinoma; colon cancer; endometrial cancer; esophageal cancer; gastric (stomach) cancer; intraepithelial neoplasms; leukemias; lymphomas; liver cancer; lung cancer (*e.g*., small cell and non-small cell); melanoma; neuroblastomas; oral cancer; ovarian cancer; pancreatic cancer; prostate cancer; rectal cancer; renal (kidney) cancer; sarcomas; skin cancer; testicular cancer; thyroid cancer; as well as other carcinomas and sarcomas. Cancers can be primary or metastatic. Diseases other than cancers may be associated with mutational alternation of component of Ras signaling pathways and the compound disclosed herein may be used to treat these non-cancer diseases. Such non-cancer diseases may include: neurofibromatosis; Leopard syndrome; Noonan syndrome; Legius syndrome; Costello syndrome; Cardio-facio-cutaneous syndrome; Hereditary gingival fibromatosis type 1; Autoimmune lymphoproliferative syndrome; and capillary malformation-arterovenous malformation.

As used herein, "effective amount" refers to any amount that is necessary or sufficient for achieving or promoting a desired outcome. In some instances, an effective amount is a therapeutically effective amount. A therapeutically effective amount is any amount that is necessary or sufficient for promoting or achieving a desired biological response in a subject. The effective amount for any particular application can vary depending on such factors as the disease or condition being treated, the particular agent being administered, the size of the subject, or the severity of the disease or condition. One of ordinary skill in the art can empirically determine the effective amount of a particular agent without necessitating undue experimentation.

As used herein, the term "subject" refers to a vertebrate animal. In one embodiment, the subject is a mammal or a mammalian species. In one embodiment, the subject is a human. In other embodiments, the subject is a non-human vertebrate animal, including, without limitation, non-human primates, laboratory animals, livestock, racehorses, domesticated animals, and non-domesticated animals.

### Compounds

Novel compounds as Kv1.3 potassium channel blockers are described. Applicants have surprisingly discovered that the compounds disclosed herein exhibit potent Kv1.3 potassium channel-inhibiting properties. Additionally, Applicants have surprisingly discovered that the compounds disclosed herein selectively block the Kv1.3 potassium channel and do not block the hERG channel and thus have desirable cardiovascular safety profiles.

In one aspect, a compound of Formula I or a pharmaceutically acceptable salt thereof is described, 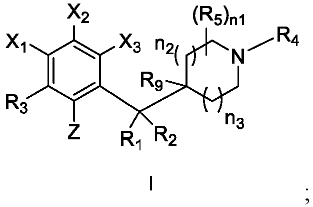 wherein
the structural moiety 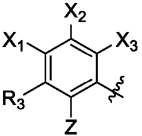 has the structure of 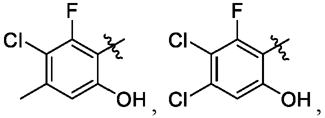 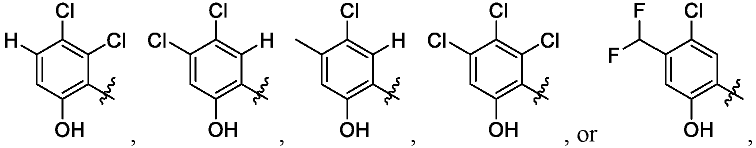 each of which is substituted by R₃;
R₃ is H, halogen, or alkyl;
R₁ and R₂ are each independently H, alkyl, (CR₆R₇)ₙ₄ORₐ, (CR₆R₇)ₙ₄NRₐR_{b}, (CR₆R₇)ₙ₄NRₐ(C=O)R_{b}, (CR₆R₇)ₙ₄NRₐSO₂R_{b}, or (CR₆R₇)ₙ₄CONRₐR_{b}, wherein at least one occurrence of R₁ and R₂ is (CR₆R₇)ₙ₄NRₐR_{b}, (CR₆R₇)ₙ₄ORₐ, (CR₆R₇)ₙ₄NRₐ(C=O)R_{b}, (CR₆R₇)ₙ₄NRₐSO₂R_{b}, or (CR₆R₇)ₙ₄CONRₐR_{b}; or alternatively R₁, R₂ and the carbon atom they are connected to taken together form a 3-5 membered carbocycle;
R₄ is (CR₆R₇)ₙ₄(C=O)R_{c}, (C=O)(CR₆R₇)ₙ₄R_{c}, (CR₆R₇)ₙ₄OR_{c}, (CR₆R₇)ₙ₄COOR_{c}, (CR₆R₇)ₙ₄NR_{c}(C=O)R_{d}, (C=O)(CR₆R₇)ₙ₄OR_{c}, (CR₆R₇)ₙ₄SO₂R_{c}, optionally substituted saturated heterocycle, optionally substituted aryl, optionally substituted heteroaryl, optionally substituted cycloalkyl, or optionally substituted -cycloalkyl-alkyl;
each occurrence of R₅ is independently H, alkyl, cycloalkyl, or oxo;
or two R₅ groups taken together with the carbon atom(s) that they are connected to form a 3-7 membered optionally substituted saturated carbocycle;
or two R₅ groups are connected to different carbon atoms on the ring and taken together form a bond or an alkyl chain containing 1-3 carbons;
each occurrence of R₆ and R₇ are independently H, alkyl, or cycloalkyl;
each occurrence of Rₐ and R_{b} are independently H, alkyl, cycloalkyl, saturated heterocycle, aryl, or heteroaryl; or alternatively Rₐ and R_{b} together with the nitrogen atom that they are connected to form an optionally substituted heterocycle comprising the nitrogen atom and 0-3 additional heteroatoms each selected from the group consisting of N, O, and S;
each occurrence of R_{c} and R_{d} are independently H, alkyl, alkyl substituted by 1-4 substituents each of which is independently halogen, OR₈ or N(R₈)₂, alkenyl, optionally substituted cycloalkyl, optionally substituted bicycloalkyl, optionally substituted spiroalkyl, optionally substituted saturated heterocycle, optionally substituted aryl, optionally substituted heteroaryl, optionally substituted -alkyl-aryl, optionally substituted -alkyl-heteroaryl, optionally substituted -alkyl-heterocycle, optionally substituted -alkyl-cycloalkyl, or optionally substituted -cycloalkyl-alkyl; or alternatively R_{c} and R_{d} together with the nitrogen atom that they are connected to form an optionally substituted heterocycle comprising the nitrogen atom and 0-3 additional heteroatoms each selected from the group consisting of N, O, and S;
each occurrence of R₈ is independently H, alkyl, or an optionally substituted heterocycle; or alternatively the two R₈ groups together with the nitrogen atom that they are connected to form an optionally substituted heterocycle comprising the nitrogen atom and 0-3 additional heteroatoms each selected from the group consisting of N, O, and S;
R₉ is H, alkyl, halogen, or (CR₆R₇)ₙ₄OR_{b};
the alkyl, cycloalkyl, spiroalkyl, bicycloalkyl, heterocycle, aryl, and heteroaryl are optionally substituted by 1-4 substituents each independent selected from the group consisting of alkyl, cycloalkyl, halogenated cycloalkyl, halogenated alkyl, halogen, CN, -(CH₂)₀₋₂OR₈, N(R₈)₂, (C=O)C₁₋₄alkyl, (C=O)N(R₈)₂, and oxo where valence permits;
each occurrence of n₁ is independently an integer from 0-3 where valence permits;
each occurrence of n₂ and n₃ is independently an integer from 0-2; and
each occurrence of n₄ is independently an integer from 0-3.

In some embodiments, each occurrence of n₂ and n₃ is independently an integer from 0-2. In some embodiments, n₂ and n₃ are each 0. In other embodiments, n₂ and n₃ are each 1. In still other embodiments, n₂ and n₃ are each 2. In some embodiments, n₂ and n₃ are 0 and 1, respectively. In some embodiments, n₂ and n₃ are 0 and 2, respectively. In some embodiments, n₂ and n₃ are 1 and 2, respectively.

In some embodiments, the structural motif 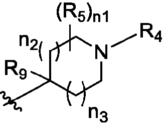 has the structure of 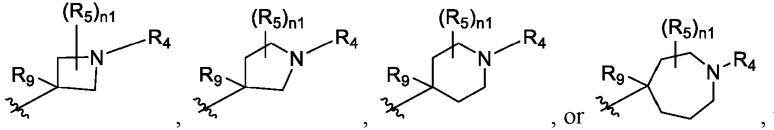 where the various substituents are defined herein. In some embodiments, the structural motif 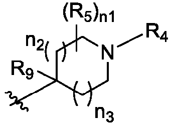 has the structure of 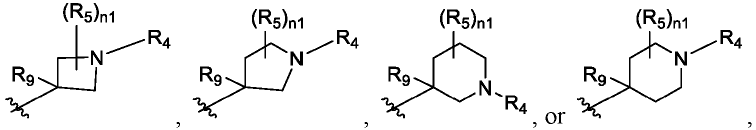 wherein the various substituents are defined herein. In some embodiments, the structural motif 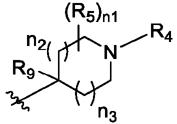 has the structure of 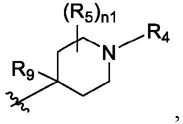 wherein the various substituents are defined herein.

In any one of embodiments described herein, each occurrence of Rₐ and R_{b} may be independently H, alkyl, cycloalkyl, saturated heterocycle, aryl, or heteroaryl. In some embodiments, at least one of Rₐ and R_{b} is independently H, alkyl, or cycloalkyl. In other embodiments, at least one of Rₐ and R_{b} is independently saturated heterocycle, aryl, or heteroaryl. In other embodiments, Rₐ and R_{b} together with the nitrogen atom that they are connected to form an optionally substituted heterocycle comprising the nitrogen atom and 0-3 additional heteroatoms each selected from the group consisting of N, O, and S.

In some embodiments, R₁ and R₂ are each H or alkyl. In some embodiments, R₁ and R₂ are both H. In some embodiments, at least one of R₁ and R₂ is alkyl, such as Me, Et, propyl, isopropyl, *n*-butyl, *iso-*butyl or *sec*-butyl. In some embodiments, R₁ and R₂ are H and alkyl respectively.

In some embodiments, at least one occurrence of R₁ and R₂ is (CR₆R₇)ₙ₄ORₐ or (CR₆R₇)ₙ₄NRₐR_{b}. In some embodiments, at least one occurrence of R₁ and R₂ is (CR₆R₇)₀₋₂NRₐR_{b}. Non-limiting examples of NRₐR_{b} include NH₂, NHMe, NMe₂, NHEt, NMeEt, NEt₂, NHPr, NMePr, NEtPr, NH(*iso*-Pr), and N(*iso*-Pr)₂. In some embodiments, at least one occurrence of R₁ and R₂ is (CR₆R₇)₀₋₂ORₐ. Non-limiting examples of ORₐ include OH, OMe, OEt, OPr, O-*iso*-Pr, OBu, O-*tert*-Bu, and O-*sec*-Bu. In some embodiments, at least one occurrence of R₁ and R₂ is ORₐ or NRₐR_{b}.

In some embodiments, at least one occurrence of R₁ and R₂ is (CR₆R₇)₀₋₂NRₐ(C=O)R_{b}, (CR₆R₇)ₙ₄NRₐSO₂R_{b}, or (CR₆R₇)₀₋₂CONRₐR_{b}. Non-limiting examples of NRₐ(C=O)R_{b} include NH(C=O)Me, NMe(C=O)Me, NH(C=O)Et, NMe(C=O)Et, NEt(C=O)Et, NH(C=O)Pr, NMe(C=O)Pr, NEt(C=O)Pr, NH(C=O)(*iso*-Pr), NMe(C=O)(*iso*-Pr), and NEt(C=O)(*iso*-Pr). Non-limiting examples of CONRₐR_{b} include (C=O)NH₂, (C=O)NHMe, (C=O)NMe₂, (C=O)NHEt, (C=O)NMeEt, (C=O)NEt₂, (C=O)NHPr, (C=O)NMePr, (C=O)NEtPr, (C=O)NH(*iso*-Pr), and (C=O)N(*iso*-Pr)₂.

In other embodiments, R₁, R₂, and the carbon atom they are connected to taken together form a 3-5 membered carbocycle. In some specific embodiments, R₁, R₂ and the carbon atom they are connected to taken together form cyclopropyl, cyclobutyl or cyclopentyl.

In some embodiments, R₁ and R₂ are each independently H, Me, OH, CH₂OH, NH₂, NHMe, NMe₂, CH₂NH₂, CONH₂, CONHMe₂, CONMe₂, NH(C=O)Me, NMe(C=O)Me,

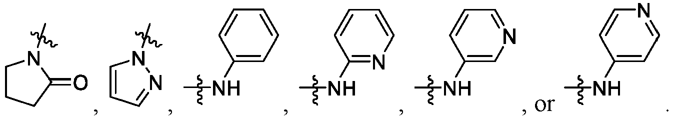

In some specific embodiments, n₄ is 0. In other specific embodiments, n₄ is 1 or 2. In some specific embodiments, R₄ is (CR₆R₇)₂OR_{c}, (C=O)R_{c}, (C=O)(CR₆R₇)₁₋₂R_{c}, COOR_{c}, (CR₆R₇)₁₋₂NR_{c}(C=O)R_{d}, (C=O)(CR₆R₇)₁₋₂OR_{c} or SO₂R_{c}. In some specific embodiments, CR₆R₇ is CH₂, CHMe, CMe₂, CHEt, or CEt₂. In some specific embodiments, R₄ is (CH₂)₂OH, (CH₂)₂OMe, (C=O)H, (C=O)Me, (C=O)CH₂OH, (C=O)CH₂OMe, (C=O)CH(OH)CH₂OH, (C=O)CH(OMe)CH₂OH, (C=O)CH(OH)CH₂OMe, (C=O)Et, (C=O)Ph, (C=O)isopropyl, (C=O)OMe, SO₂Me, SO₂Et, SO₂CH₂OH or SO₂CH₂OMe. In other embodiments, R₄ is (C=O)CH(OH)CH₂OH, (C=O)CH(OMe)CH₂OH, or (C=O)CH(OH)CH₂OMe.

In other embodiments, R₄ is (C=O)R_{c}, (C=O)(CR₆R₇)₁₋₂R_{c}, (C=O)(CR₆R₇)₁₋₂OR_{c}, or SO₂R_{c}; and wherein R_{c} is selected from the group consisting of H, alkyl, alkyl substituted by 1-4 substituents each independently selected from the group consisting of halogen, OR₈ and N(R₈)₂, alkenyl, optionally substituted cycloalkyl, optionally substituted bicycloalkyl, optionally substituted spiroalkyl, optionally substituted saturated heterocycle, optionally substituted aryl, optionally substituted heteroaryl, optionally substituted -alkyl-aryl, optionally substituted -alkyl-heteroaryl, optionally substituted -alkyl-heterocycle, optionally substituted -alkyl-cycloalkyl, and optionally substituted -cycloalkyl-alkyl.

In some embodiments, R_{c} or R_{d} is H, Me, Et, 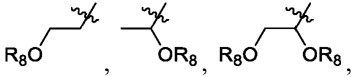 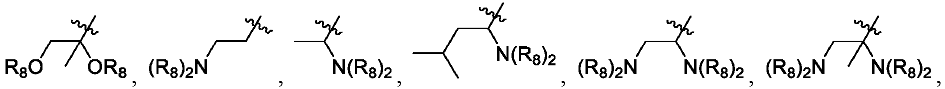 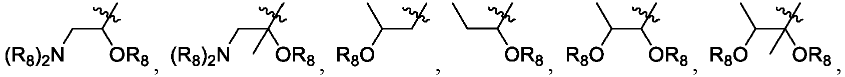 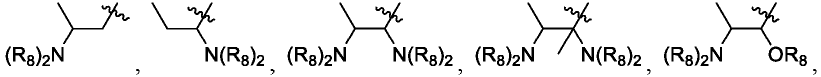 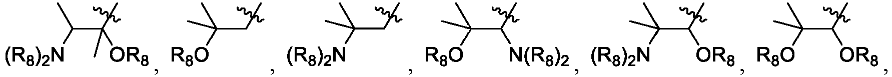 or

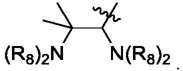

In some embodiments, R_{c} is a heterocycle selected from the group consisting of 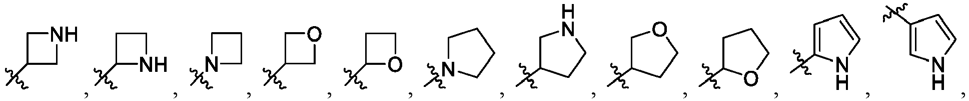 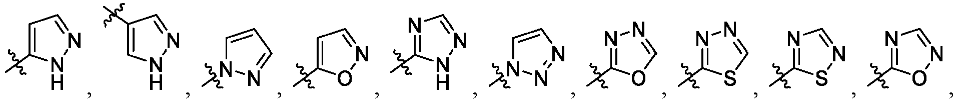 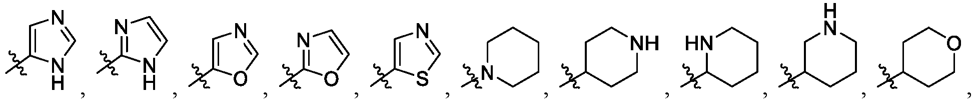 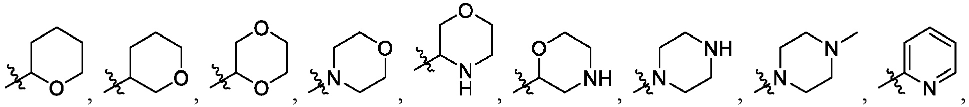 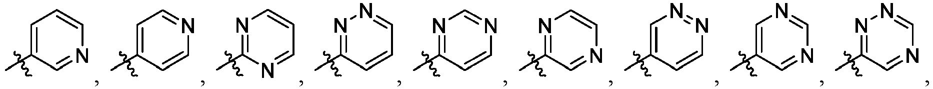 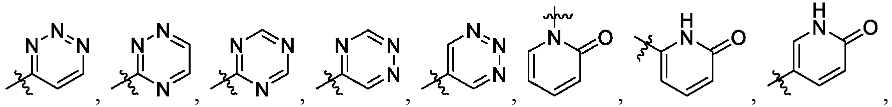 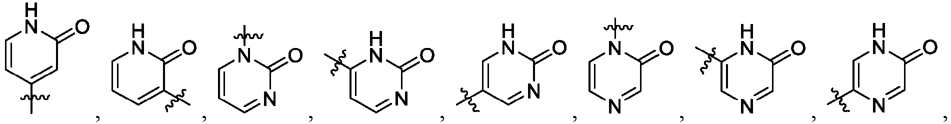 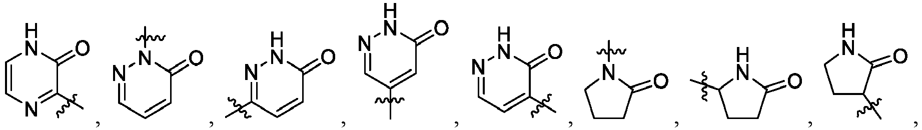 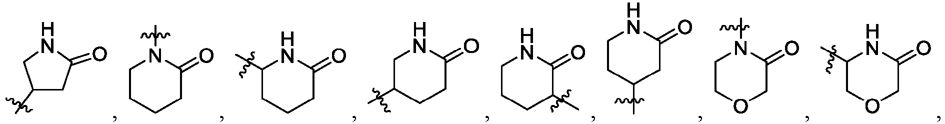 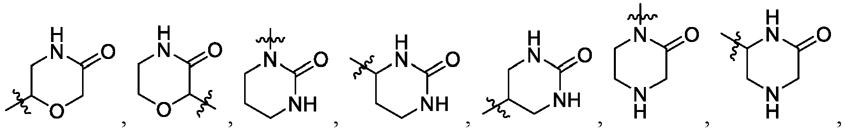 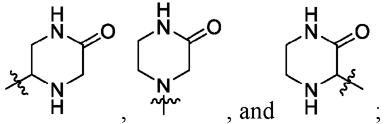 wherein the heterocycle is optionally substituted by one or more cyano, cycloalkyl, fluorinated alkyl, fluorinated cycloalkyl, halogen, OH, NH₂, oxo, or (C=O)C₁₋₄alkyl where valence permits.

In some embodiments, R_{c} is cycloalkyl, spiroalkyl, or bicycloalkyl each optionally substituted by 1-4 substituents each independently selected from the group consisting of alkyl, halogen, CN, -(CH₂)₀₋₂OR₈, N(R₈)₂, (C=O)N(R₈)₂, and oxo where valence permits. In other embodiments, R_{c} is 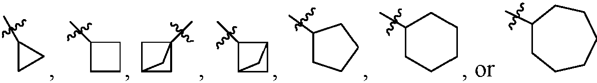 each optionally substituted by 1-4 substituents each independently selected from the group consisting of alkyl, halogen, CN, -(CH₂)₀₋₂OR₈, N(R₈)₂, (C=O)N(R₈)₂, and oxo where valence permits.

In still other embodiments, R₄ is optionally substituted saturated heterocycle, optionally substituted aryl, optionally substituted heteroaryl, optionally substituted cycloalkyl, or optionally substituted -cycloalkyl-alkyl. In some specific embodiments, R₄ is a heterocycle selected from the group consisting of 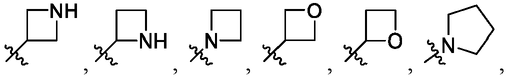 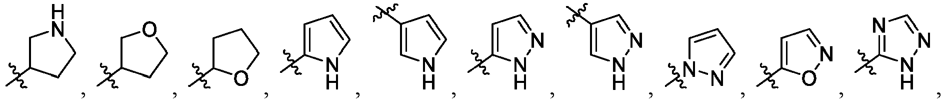 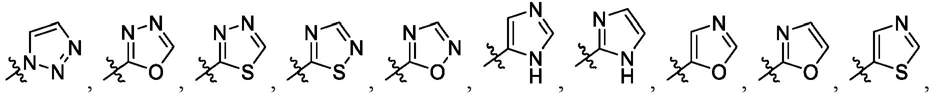 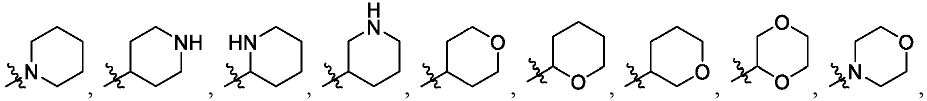 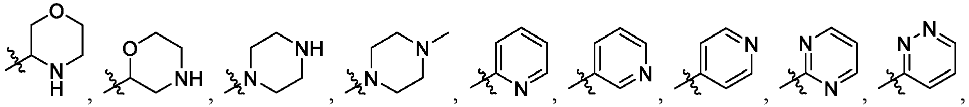 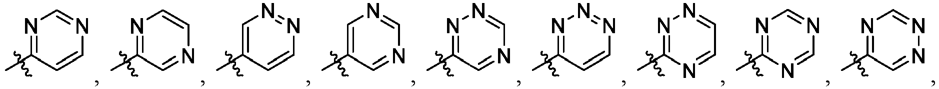 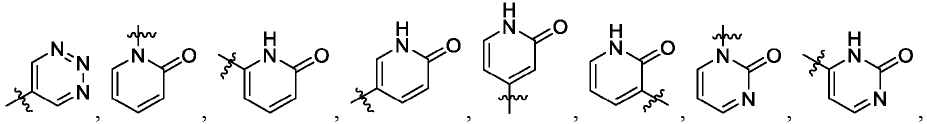 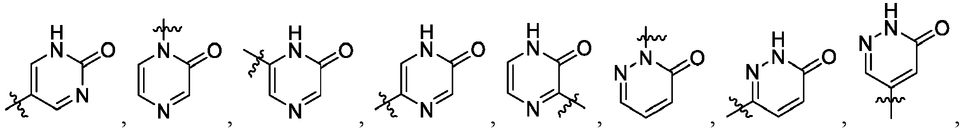 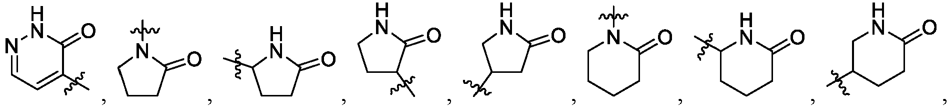 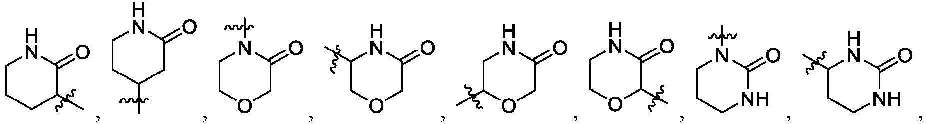 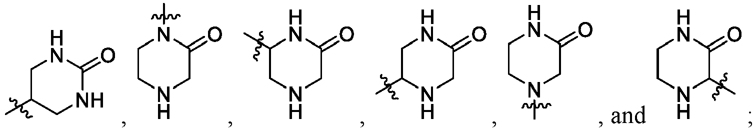 wherein the heterocycle is optionally substituted by one or more cyano, cycloalkyl, fluorinated alkyl, fluorinated cycloalkyl, halogen, OH, NH₂, oxo, or (C=O)C₁₋₄alkyl where valence permits.

In still other embodiments, R₄ is cycloalkyl optionally substituted by 1-4 substituents each independently selected from the group consisting of alkyl, halogen, CN, -(CH₂)₀₋₂OR₈, N(R₈)₂, (C=O)N(R₈)₂, and oxo where valence permits. In other embodiments, R₄ is 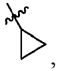 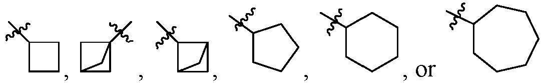 each optionally substituted by 1-4 substituents each independently selected from the group consisting of alkyl, halogen, CN, - (CH₂)₀₋₂OR₈, N(R₈)₂, (C=O)N(R₈)₂, and oxo where valence permits.

In some embodiments, R₈ is H or alkyl. In other embodiments, R₈ is optionally substituted heterocycle. In still other embodiments, the two R₈ groups together with the nitrogen atom that they are connected to form an optionally substituted heterocycle comprising the nitrogen atom and 0-3 additional heteroatoms each selected from the group consisting of N, O, and S.

In some specific embodiments, R₄ is 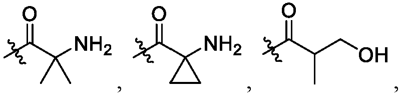 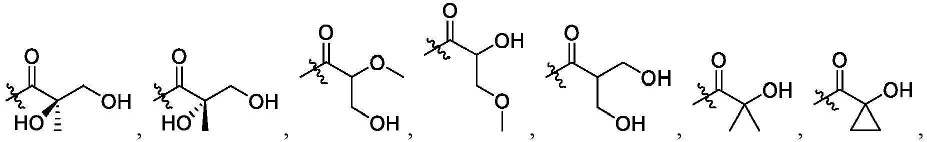 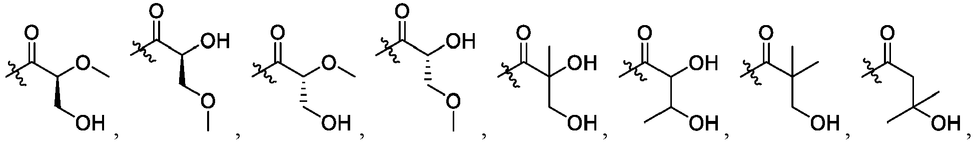 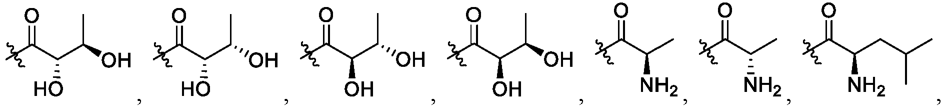 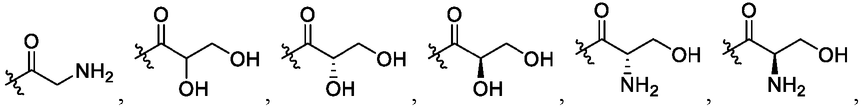 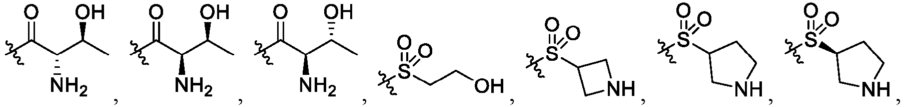 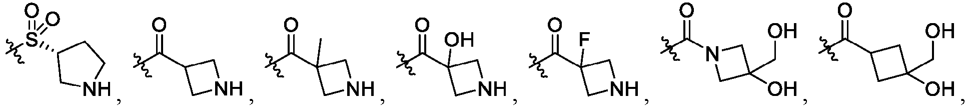 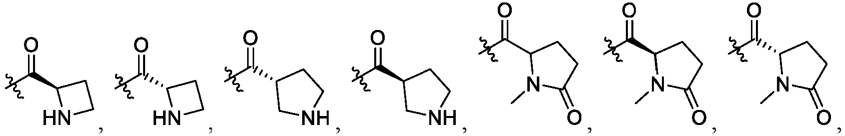 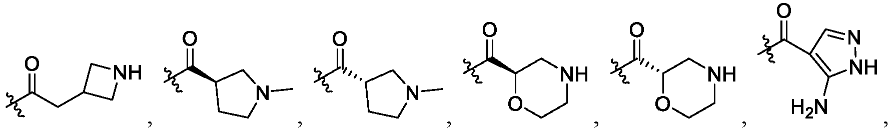 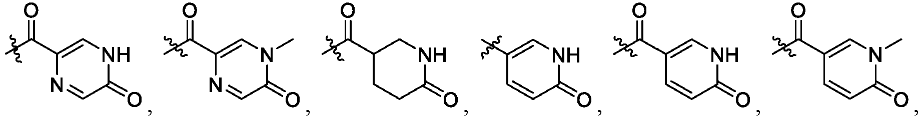 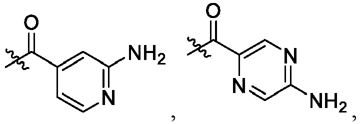 or a tautomer thereof.

In some embodiments, R₅ is H, alkyl, or cycloalkyl. In some specific embodiments, R₅ is H. In other specific embodiments, R₅ is Me, Et, Pr, *iso*-Pr, Bu, *iso*-Bu, *sec*-Bu, or *tert*-Bu. In other specific embodiments, R₅ is cyclopropyl, cyclobutyl, cyclopentyl, or cyclohexyl. In other specific embodiments, R₅ is oxo.

In other embodiments, two R₅ groups taken together with the carbon atom(s) that they are connected to form a 3-7 membered optionally substituted saturated carbocycle. In still other embodiments, two R₅ groups are connected to different carbon atoms on the ring and taken together form a bond or an alkyl chain containing 1-3 carbons.

In some embodiments, each occurrence of R₆ and R₇ are independently H or alkyl. In some specific embodiments, CR₆R₇ is CH₂, CHMe, CMe₂, CHEt, or CEt₂. In some specific embodiments, CR₆R₇ is CH₂.

In some embodiments, R₉ is H, alkyl, or halogen. In other embodiments, R₉ is (CR₆R₇)ₙ₄OR_{b}. In some specific embodiments, R₉ is H, F, or OH.

In any one of the embodiments described herein, R₃ is H, halogen, or alkyl. In some embodiments, R₃ is H. In other embodiments, R₃ is alkyl such as Me, Et, propyl, isopropyl, n-butyl, iso-butyl or sec-butyl. In still other embodiments, R₃ is F, Cl or Br.

In any one of the embodiments described herein, at least one occurrence of R₈ is H, alkyl, or optionally substituted heterocycle. In some embodiments, R₈ is H, Me, Et, Pr, Bu, or a heterocycle selected from the group consisting of 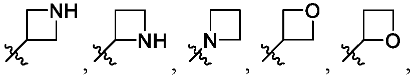 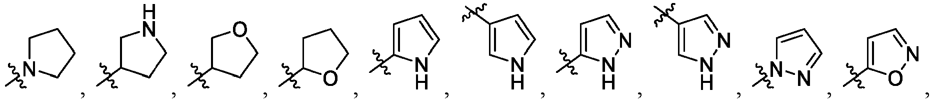 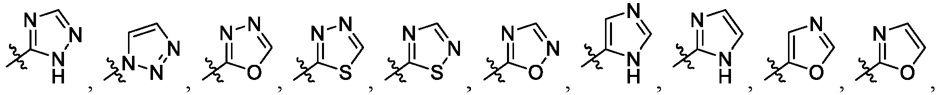 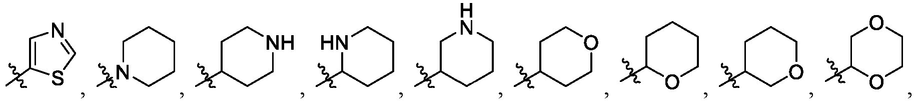 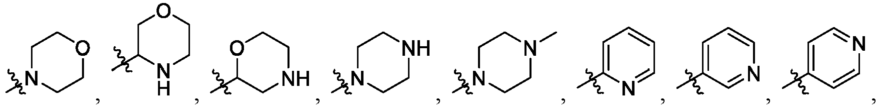 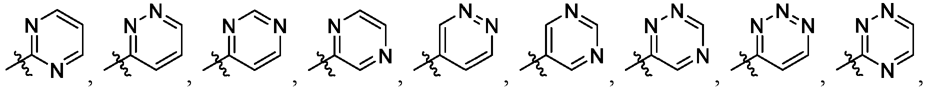 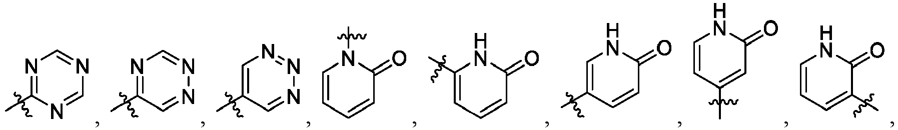 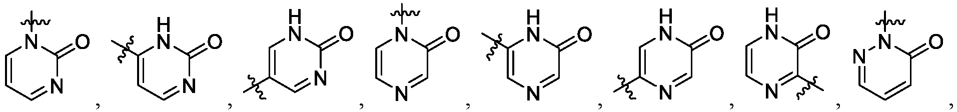 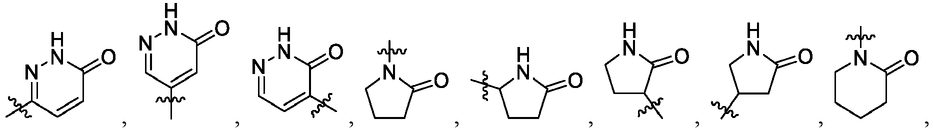 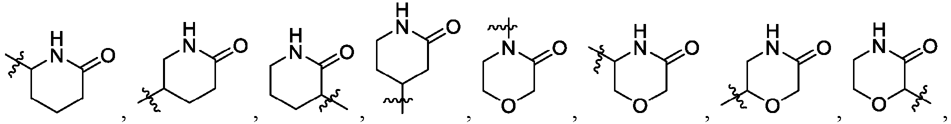 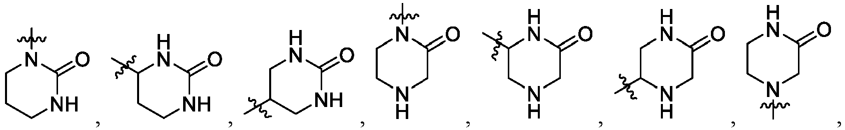 and 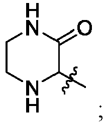 wherein the heterocycle is optionally substituted by one or more cyano, cycloalkyl, fluorinated alkyl, fluorinated cycloalkyl, halogen, OH, NH₂, oxo, or (C=O)C₁₋₄alkyl where valence permits.

In other embodiments, the two R₈ groups together with the nitrogen atom that they are connected to form an optionally substituted heterocycle comprising the nitrogen atom and 0-3 additional heteroatoms each selected from the group consisting of N, O, and S.

In some embodiments, n₁ is 0 or 1. In some embodiments, n₄ is 0, 1, or 2. In some embodiments, n₄ is 0 or 1. In some specific embodiments, n₄ is 0.

In any one of the embodiments described herein, at least one occurrence of R_{c} or R_{d} is independently H, alkyl, alkyl substituted by 1-4 substituents each independently selected from the group consisting of halogen, OR₈ and N(R₈)₂, alkenyl, optionally substituted cycloalkyl, optionally substituted bicycloalkyl, optionally substituted spiroalkyl, optionally substituted saturated heterocycle, optionally substituted aryl, optionally substituted heteroaryl, optionally substituted -alkyl-aryl, optionally substituted -alkyl-heteroaryl, optionally substituted -alkyl-heterocycle, optionally substituted -alkyl-cycloalkyl, or optionally substituted -cycloalkyl-alkyl.

In some embodiments, R_{c} and R_{d} together with the nitrogen atom that they are connected to form an optionally substituted heterocycle comprising the nitrogen atom and 0-3 additional heteroatoms each selected from the group consisting of N, O, and S.

In some specific embodiments, at least one occurrence of R_{c} or R_{d} is independently H, Me, Et,

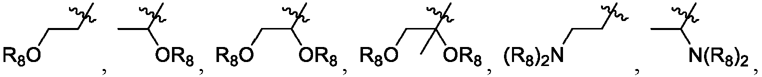

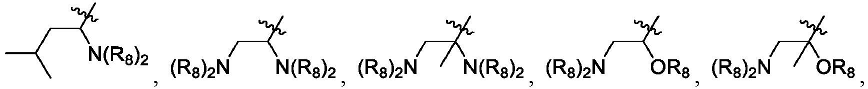

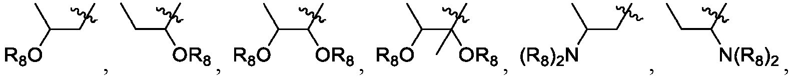

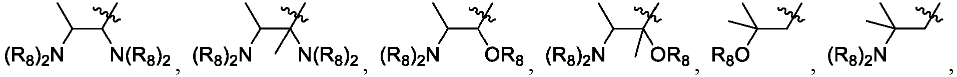

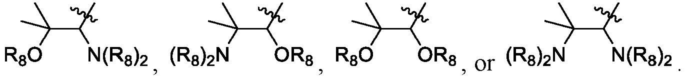

In some specific embodiments, at least one occurrence of R_{c} or R_{d} is independently a heterocycle selected from the group consisting of 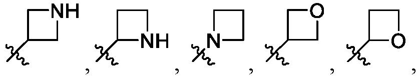 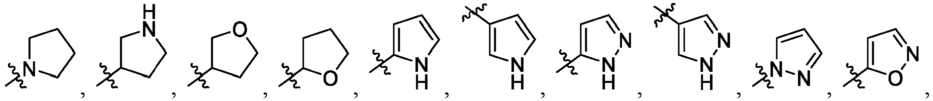 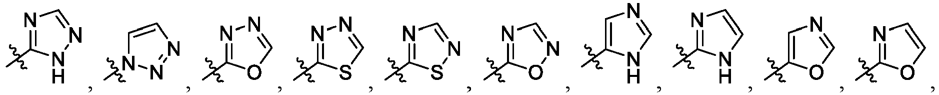 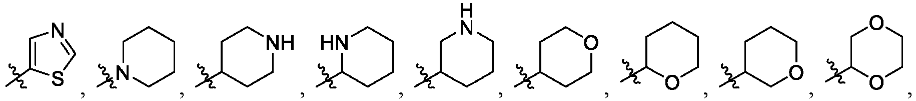 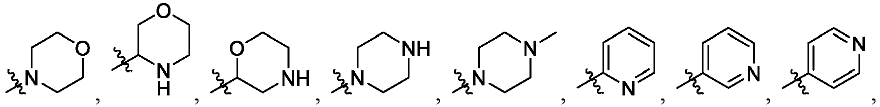 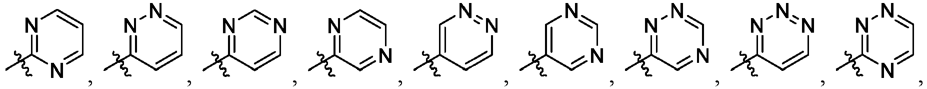 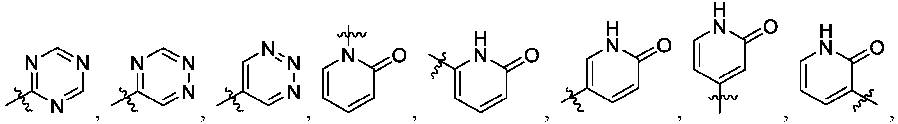 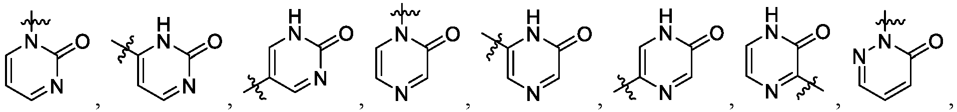 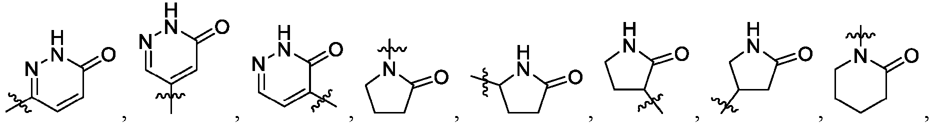 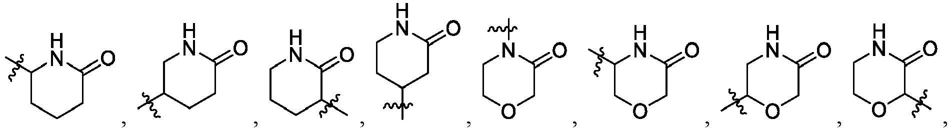 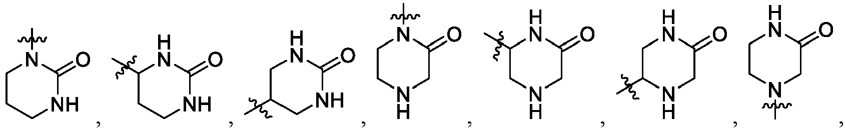 and 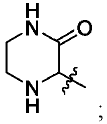 wherein the heterocycle is optionally substituted by one or more cyano, cycloalkyl, fluorinated alkyl, fluorinated cycloalkyl, halogen, OH, NH₂, oxo, or (C=O)C₁₋₄alkyl where valence permits.

In some specific embodiments, at least one occurrence of R_{c} or R_{d} is independently cycloalkyl, spiroalkyl, or bicycloalkyl each optionally substituted by 1-4 substituents each independently selected from the group consisting of alkyl, halogen, CN, -(CH₂)₀₋₂OR₈, N(R₈)₂, (C=O)N(R₈)₂, and oxo where valence permits.

In some specific embodiments, at least one occurrence of R_{c} or R_{d} is independently 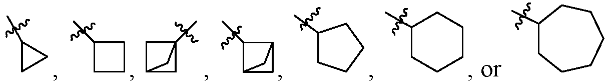 each optionally substituted by 1-4 substituents each independently selected from the group consisting of alkyl, halogen, CN, - (CH₂)₀₋₂OR₈, N(R₈)₂, (C=O)N(R₈)₂, and oxo where valence permits.

In some embodiments, the compound of Formula I is a specific compound as set out in the appended set of claims or a pharamaceutically acceptable salt thereof.

### Abbreviations

- ACN: Acetonitrile
- 9-BBN: 9-Borabicyclo[3.3.1]nonane
- BOP: Benzotriazol-1-yloxytris(diethylamino)phosphonium hexafluorophospahte
- BOPCl: Bis(2-oxo-3-oxazolidinyl)phosphinic chloride
- CDI: Carbonyldiimidazole
- DAST: Diethylaminosulfur trifluoride
- DCE: 1,2-Dichloroethane
- DCM: Dichloromethane
- DEAD: Diethyl azodicarboxylate
- DIBAL-H or DIBAL: Diisobutylaluminum hydride
- DIPEA or DIEA: Diisopropylethylamine
- DMAP: 4-Dimethylaminopyridine
- DMF: Dimethyl formamide
- DMSO: Dimethyl sulfoxide
- EA: Ethyl acetate
- EDCI: 1-Ethyl-3-(3-dimethylaminopropyl)carbodiimide
- HATU: N-[(dimethylamino)(3H-1,2,3-triazolelo(4,4-b)pyridin-3-yloxy)methylene]-N-methylmethaneaminium hexafluorophosphate
- HBTU: 2-(1H-Benzotriazole-1-yl)-1,1,3,3-tetramethyluronium hexafluorophosphate
- HOBT or HOBt: 1-Hydroxybenzotriazole
- LDA: Lithium diisopropylamide
- NBS: *N*-bromosuccinimide
- NCS: *N*-chlorosuccinimide
- NIS: *N*-iodosuccinimide
- NMP: *N*-Methylpyrrolidone
- PCC: Pyridinium chlorochromate
- PDC: Pyridinium dichromate
- PE: Petroleum ether
- PyBOP: 1H-Benzotriazol-1-yloxytripyrrolidinophosphoniumhexafluorophosphate
- SEM: [2-(Trimethylsilyl)ethoxy]methyl acetal
- TEA: Triethylamine
- TEMPO: 2,2,6,6-Tetramethyl-1-piperidinyloxy
- TFA or TFAA: Trifluoroacetic acid
- THF: Tetrahydrofuran
- TMS: Trimethylsilyl
- TsOH: p-Toluenesulfonic acid

### Methods of Preparation

Following are general synthetic schemes for manufacturing compounds of the presently claimed invention and structurally related compounds. These schemes are illustrative and are not meant to limit the possible techniques one skilled in the art may use to manufacture the compounds disclosed herein. Different methods will be evident to those skilled in the art. Additionally, the various steps in the synthesis may be performed in an alternate sequence or order to give the desired compound(s). The following reactions are illustrations but not limitations of the preparation of some of the starting materials and compounds disclosed herein. For avoidance of doubt, to the extent the reactions are directed to compounds other than those defined in the appended set of claims they are provided herein for reference purposes only.

Schemes 1-9 below describe synthetic routes which may be used for the synthesis of compounds, *e*.*g*., compounds having a structure of Formula I or precursors thereof. Various modifications to these methods may be envisioned by those skilled in the art to achieve similar results. In the embodiments below, the synthetic route is described using compounds I-4, I-6, I-4a, I-6a, I-7, I-7b, I-10, I-10a, and I-13 as examples. Other compounds of Formula I can be prepared by using methods similar to those described in Schemes 1-9 or using methods known in the art. The general synthetic route described in Schemes 1-9 and examples described in the Example section below illustrate methods used for the preparation of the compounds described herein.

As shown in Scheme 1, compound I-4 can be prepared from compound I-1a or I-1b.

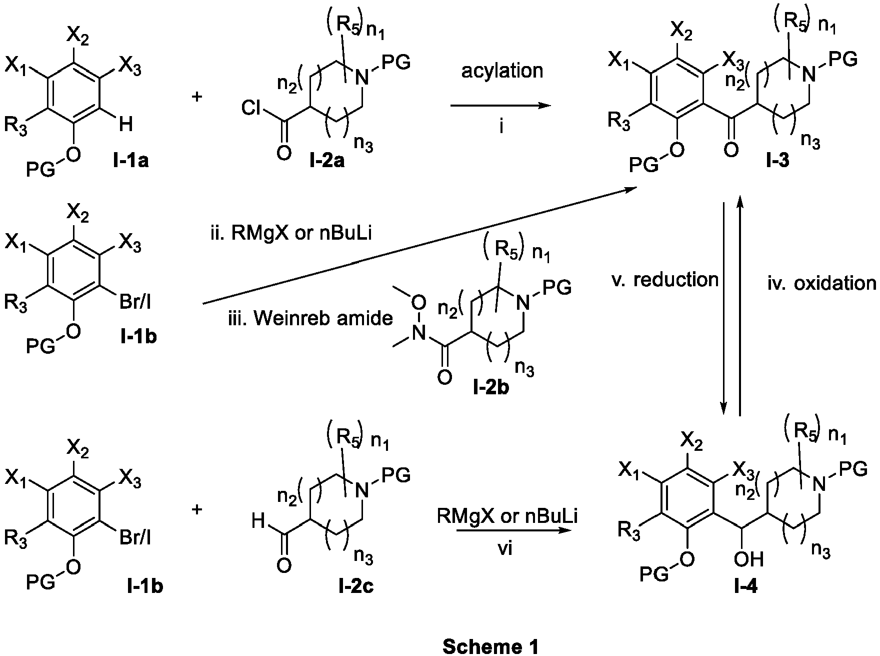

Compound I-1a, I-1b, I-2a, I-2b, and I-2c can be prepared by any method known in the art. As shown in Scheme 1, "Br/I" refers to a Br or I substituent and PG refers to a protecting group. Non-limiting examples of the protecting groups include Me, allyl, Ac, Boc, other alkoxycarbonyl group, dialkylaminocarbonyl, or another protecting group known in the art suitable for use as protecting groups for OH or NH₂. Other substituents are defined herein. As shown in Scheme 1, step i, the intermediate ketone I-3 can be synthesized by Friedel-Crafts reaction of a protected phenol I-1a with a suitably protected acid chloride I-2a in the presence of a Lewis acid. Non-limiting examples of the Lewis acid include aluminum III chloride. Alternatively, aryl bromide or iodide I-1b is converted to the corresponding Grignard reagent, *e.g.,* by treatment with isopropyl magnesium chloride (R is isopropyl), or to the corresponding aryl lithium reagent by treatment with butyl lithium (Step ii). The resulting organometallic reagent reacts with the Weinreb amide I-2b to form ketone I-3. Ketone I-3 can be reduced using a suitable reducing agent to afford alcohol I-4 (Step v). Non-limiting examples of suitable reducing agents include NaBH₄. Still alternatively, aryl bromide or iodide I-1b is converted to the corresponding Grignard reagent, *e.g.,* by treatment with isopropyl magnesium chloride (R is isopropyl), or to the corresponding aryl lithium reagent by treatment with butyl lithium and the resulting organometallic reagent reacts with aldehyde I-2c to give benzylic alcohol I-4 (Step vi). Benzylic alcohol I-4 can be oxidized to ketone I-3 using a oxidation agent, *e*.*g*., Dess-Martin periodinane. The protecting groups in compound I-4 can then be selectively removed, and the resulting compound with free NH and/or phenol OH groups can optionally be converted to a compound of Formula I using methods known in the art.

As shown in Scheme 2, compound I-6 can be prepared from compound I-1b, I-2d or I-3.

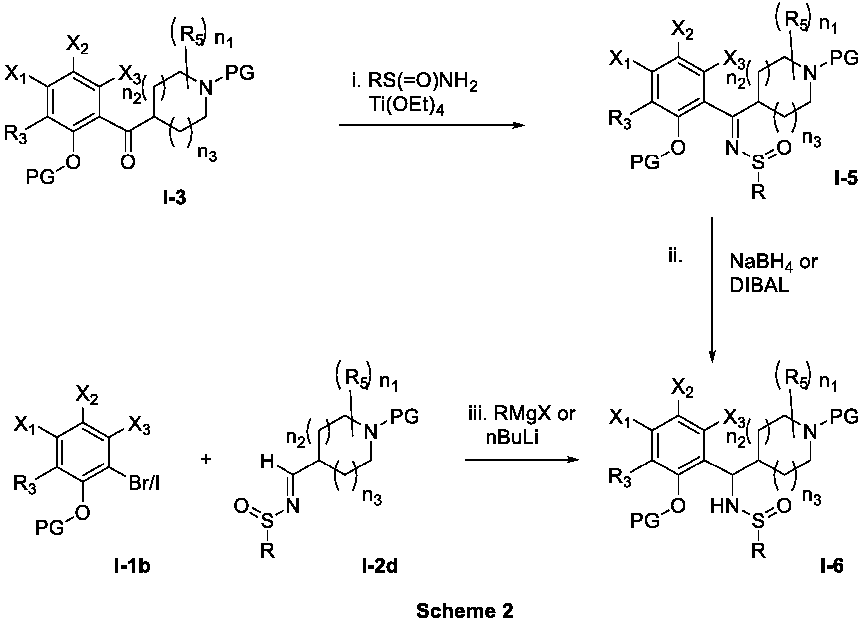

Compounds I-1b and I-2d can be prepared by any method known in the art. Compound I-3 can be prepared by the method shown in Scheme 1. As shown in Scheme 2, "Br/I" refers to a Br or I substituent and PG refers to a protecting group. Non-limiting examples of the protecting group include Me, allyl, Ac, Boc, other alkoxycarbonyl group, dialkylaminocarbonyl, or another protecting group known in the art suitable for use as protecting groups for OH or NH₂. Other substituents are defined herein. As shown in Scheme 2, step i, compounds having Formula I where R₁ includes an amino group (*e*.*g*., compound I-6) can be obtained from ketone I-3 by forming the sulfinyl imine I-5 by heating ketone I-3 with an alkyl sulfinimide for example t-butyl sulfinimide (*i.e.,* R is alkyl such as t-butyl) in the presence of a Lewis acid (*e*.*g*., titanium tetraethoxide). Reduction of the sulfinyl imine I-5 with reducing agent, *e*.*g*., sodium borohydride or diisobutyl aluminum hydride (DIBAL), gives the sulfinimide I-6 (Step ii). The single enantiomer of compound I-6 can be prepared by methods known in the art. Alternatively, as shown in Step iii, compound I-6 can be synthesized by forming the Grignard or organolithium reagent from I-1b and reacting the formed Grignard or organolithium reagent with sulfinyl imine I-2d (prepared from I-2c in Scheme 1 using method known in the art) to give I-6 directly. The protecting groups in compound I-6 can then be selectively removed, and the resulting compound with free NH and/or phenol OH groups can optionally be converted to a compound of Formula I using methods known in the art.

As shown in Scheme 3, compounds I-3a, I-4a, and I-6a can be prepared from compounds I-1d and I-2b, I-1d and I-2c, and I-1d and I-2d, respectively.

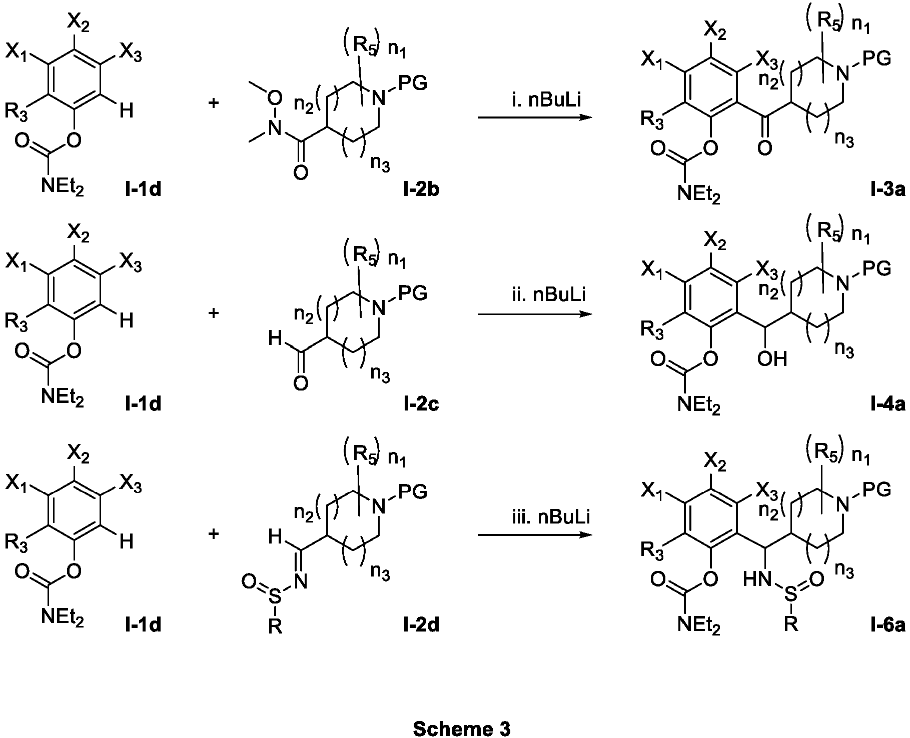

Compounds I-1d, I-2b, I-2c, and I-2d can be prepared by any method known in the art. As shown in Scheme 3, PG refers to a protecting group. Non-limiting examples of the protecting group include Me, allyl, Ac, Boc, other alkoxycarbonyl group, dialkylaminocarbonyl, or another protecting group known in the art suitable for use as protecting groups for OH or NH₂. Other substituents are defined herein. As shown in Scheme 3, Step i, ii, or iii, a suitably protected phenol such as the diethyl carbamate I-1d can be activated by lithiation ortho to the carbamate and reacted with a Weinreb amide I-2b (Step i), aldehyde I-2c (Step ii) or a sulfinyl imine I-2d (Step iii) as shown in Scheme 3. The protecting groups in compounds I-4a and I-6a can then be selectively removed, and the resulting compound with free NH and/or phenol OH groups can optionally be converted to a compound of Formula I using methods known in the art. Ketone I-3a can be reduced using a suitable reducing agent (*e.g*., NaBH₄) to afford the corresponding alcohol (not shown). The resulting alcohol's protecting groups can then be selectively removed and the resulting compound with free NH and/or phenol OH groups can optionally be converted to a compound of Formula I using methods known in the art.

As shown in Scheme 4, compounds I-4, I-3", and I-7 can be prepared from compound I-3.

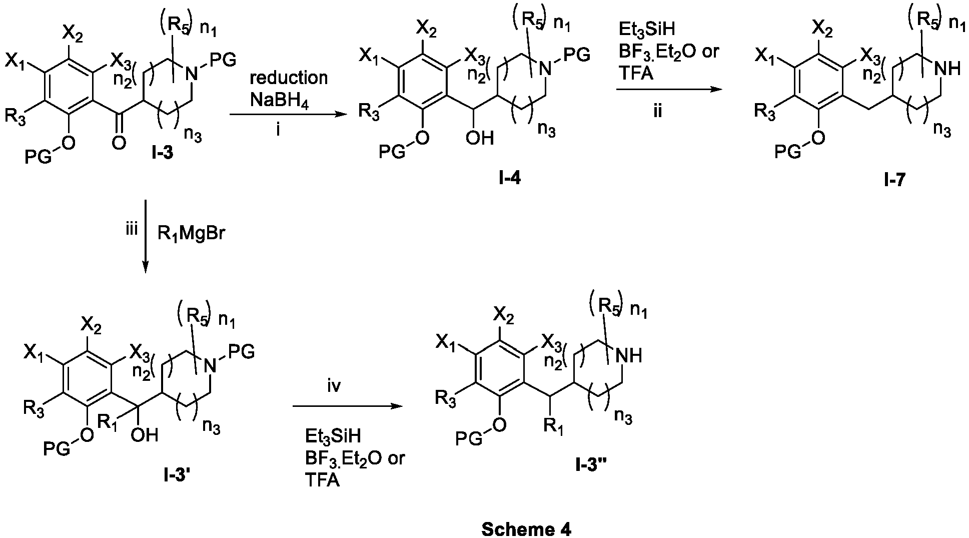

Compound I-3 can be prepared by the method shown in Scheme 1. As shown in Scheme 4, PG refers to a protecting group. Non-limiting examples of the protecting group include Me, allyl, Ac, Boc, other alkoxycarbonyl group, dialkylaminocarbonyl, or another protecting group known in the art suitable for use as protecting groups for OH or NH₂. Other substituents are defined herein. As shown in Scheme 4, for compounds where R₁ = R₂ = H (*e.g.,* compound I-7), ketone I-3 is reduced to alcohol I-4 with a reducing agent (*e*.*g*., sodium borohydride; Step i). Alcohol I-4 is then reduced with a silane such as triethyl silane in the presence of a Lewis acid such as boron trifluoride etherate or an acid such as trifluoroacetic acid to afford compound I-7 (Step ii). Where the nitrogen-protecting group is Boc, the nitrogen-protecting group is removed under these conditions to give the benzyl cyclic amine I-7. For compounds where R₁ is alkyl, ketone I-3 is treated with the appropriate alkyl-Grignard reagent (*e.g.,* R₁MeBr) to afford alcohol I-3' (Step iii). As shown in Scheme 4, Step iv, the alcohol I-3' can be reduced with triethyl silane using procedure similar to those used in Step ii to remove the OH group to afford compound I-3'' (where the nitrogen-protecting group is Boc, the nitrogen-protecting group is removed under these conditions). The protecting groups in compounds I-3'' and I-7 can then be selectively removed, and the resulting compound's free phenol OH group can optionally be converted to a compound of Formula I using methods known in the art.

As shown in Scheme 5, compound I-7b can be prepared from compound I-8 and I-9.

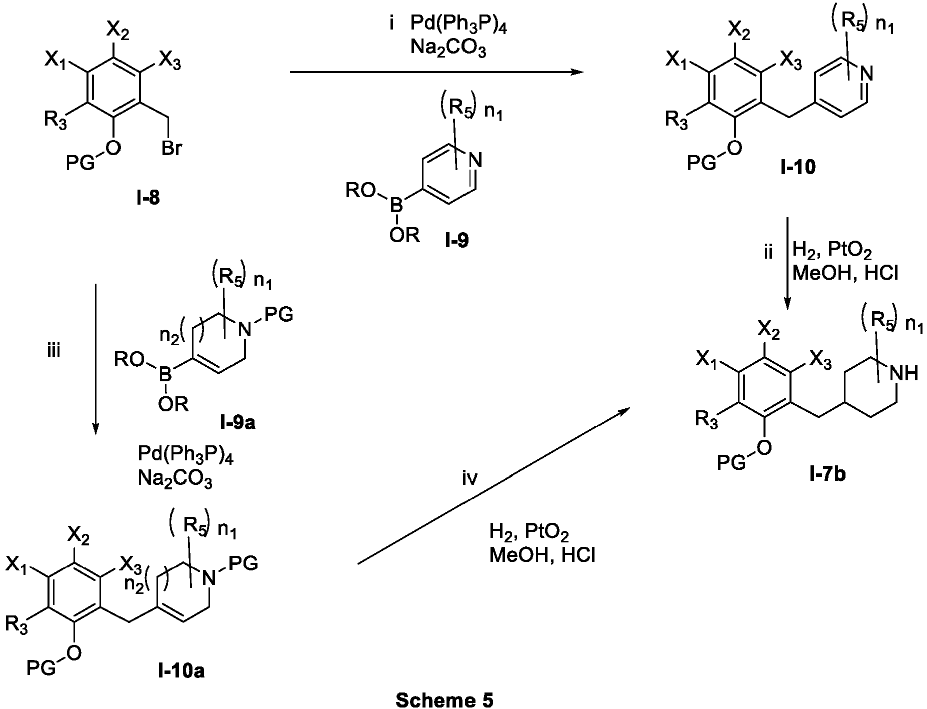

Compounds I-8, 1-9a, and I-9 can be prepared by any method known in the art. As shown in Scheme 5, PG refers to a protecting group. Non-limiting examples of the protecting group include Me, allyl, Ac, Boc, other alkoxycarbonyl group, dialkylaminocarbonyl, or another protecting group known in the art suitable for use as protecting groups for OH or NH₂. Other substituents are defined herein. As shown in Scheme 5, another route to prepare a compound where R₁ = R₂ = H and n₂ = n₃ = 1 is reacting a benzyl bromide I-8 with a pyridine boronic acid or boronate ester I-9 (R is H or alkyl) with a palladium catalyst for example tetrakis triphenylphoshine palladium to give benzyl pyridine I-10 (Step i). Reduction of the pyridine I-10 by a reducing agent (*e.g.,* hydrogen over platinum oxide in the presence of acid (*e.g.,* HCl)) gives benzyl piperidine I-7b. Alternatively, a vinyl boronate I-9a can be used following similar steps (Steps iii and iv) to afford compound I-7b. A base such as Na₂CO₃ can be used in Steps I and iii. The protecting group in compound I-7b can then be selectively removed, and the resulting compound with free NH and phenol OH groups can optionally be converted to a compound of Formula I using methods known in the art.

As shown in Scheme 6, compound I-7 can be prepared from compound I-1b and I-11.

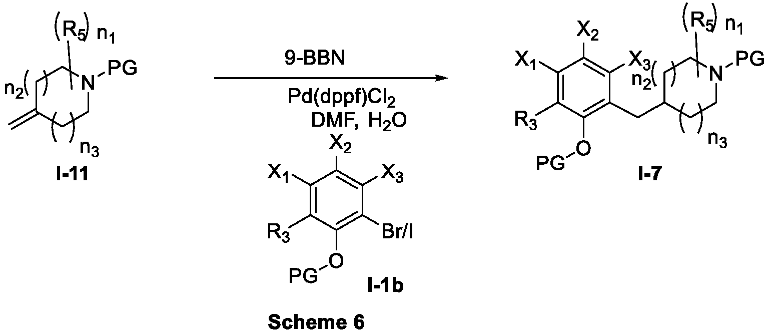

Compounds I-11 and I-1b can be prepared by any method known in the art. As shown in Scheme 6, PG refers to a protecting group. Non-limiting examples of the protecting group include Me, allyl, Ac, Boc, other alkoxycarbonyl group, dialkylaminocarbonyl, or another protecting group known in the art suitable for use as protecting groups for OH or NH₂. Other substituents are defined herein. As shown in Scheme 6, another route to a compound where R₁ = R₂ = H is hydroboration of an exocyclic alkenyl cyclic amine I-8 and coupling of the resulting boronate with a bromo or iodo phenol I-1b using a palladium catalyst (e.g., Pd(dppf)Cl₂). The protecting groups in compound I-7 can then be selectively removed, and the resulting compound with free NH and/or phenol OH groups can optionally be converted to a compound of Formula I using methods known in the art.

As shown in Scheme 7, compound I-7 can be also prepared from compound I-1b and I-12.

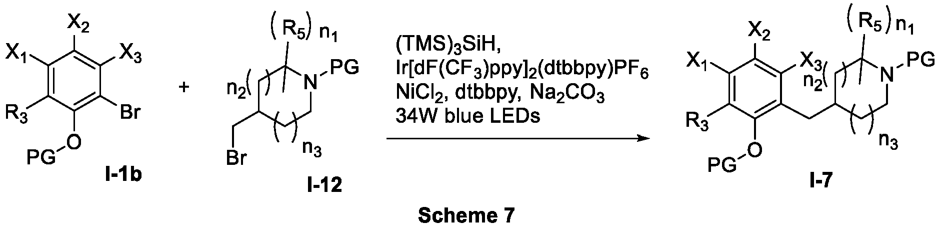

Compounds I-12 and I-1b can be prepared by any method known in the art. As shown in Scheme 7, PG refers to a protecting group. Non-limiting examples of the protecting groups include Me, allyl, Ac, Boc, other alkoxycarbonyl group, dialkylaminocarbonyl, or another protecting group known in the art suitable for use as protecting groups for OH or NH₂. Other substituents are defined herein. As shown in Scheme 7, another route to a compound where R₁ = R₂ = H is photoredox reaction of bromophenol I-1b and a bromomethyl cyclic amine I-12 using tris trimethylsilyl silane, a combination of iridium and nickel catalyst (*e.g*., Ir[dF(CF₃)ppy]₂(dtbbpy)PF₆ and NiCl₂, respectively) under irradiation with blue LED light. The protecting groups in compound I-7 can then be selectively removed, and the resulting compound with free NH and/or phenol OH groups can optionally be converted to a compound of Formula I using methods known in the art.

As shown in Scheme 8, compound I-13 can be prepared from compound I-8 and I-2d.

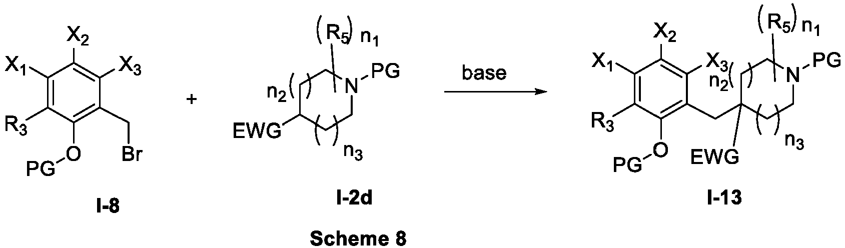

Compounds I-8 and I-2d can be prepared by the method known in the art. As shown in Scheme 8, PG refers to a protecting group. Non-limiting examples of the protecting groups include Me, allyl, Ac, Boc, other alkoxycarbonyl group, dialkylaminocarbonyl, or another protecting group known in the art suitable for use as protecting groups for OH or NH₂. Other substituents are defined herein. As shown in Scheme 8, compounds with a substituent R₉ can be obtained by reacting a cyclic amine I-2d containing an electron withdrawing group (EWG) such as an ester or a nitrile with a benzyl bromide I-8 in the presence of a base such as sodium hexamethyldisilazide to give compound I-13. Any suitable base may be used in this reaction. The ester or nitrile group (or other electro-withdrawing group) in compound I-13 can be converted to a range of R₉ groups as defined herein by methods known in the art. The protecting groups in the resulting compounds with the desirable R₉ groups can then be selectively removed, and the resulting compound with free NH and/or phenol OH groups can optionally be converted to a compound of Formula I using methods known in the art.

Compounds of Formula I having other R₁ and R₂ substituents can be synthesized from ketone I-3 by, for example, reductive amination, Wittig reaction followed by cyclopropanation or hydroboration, or from alcohol I-4 by conversion to the corresponding bromide and displacement the bromide with a nucleophile.

As shown in Scheme 9, compounds where R₁ is NH₂, n₂=1 and n₃=1 (*e.g.,* compounds I-16 and 1-17) can be prepared from compound I-1e and 1-14.

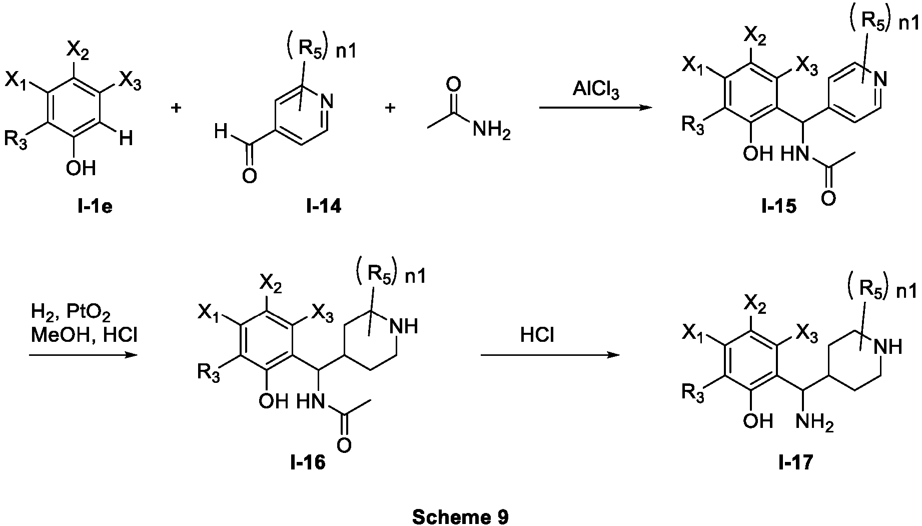

Compounds I-14 and I-1e can be prepared by any method known in the art. As shown in Scheme 9, a three-component reaction of phenol I-1e, pyridine aldehyde I-14 and acetamide is carried out by optionally heating all three components with aluminum trichloride without solvent to provide acetamide I-15. Hydrogenation over platinum oxide reduces the pyridine to piperidine I-16, and acid hydrolysis gives amine I-17.

The reactions described in Schemes 1-9 can be carried out in a suitable solvent. Suitable solvents include, but are not limited to, acetonitrile, methanol, ethanol, dichloromethane, DMF, THF, MTBE, or toluene. The reactions described in Schemes 1-9 may be conducted under inert atmosphere, e.g., under nitrogen or argon, or the reaction may be carried out in a sealed tube. The reaction mixture may be heated in a microwave or heated to an elevated temperature. Suitable elevated temperatures include, but are not limited to, 40, 50, 60, 80, 90, 100, 110, 120 °C or higher or the refluxing/boiling temperature of the solvent used. The reaction mixture may alternatively be cooled in a cold bath at a temperature lower than room temperature, *e.g.,* 0, -10, -20, -30, -40, -50, -78, or -90 °C. The reaction may be worked up by removing the solvent or partitioning of the organic solvent phase with one or more aqueous phases each optionally containing NaCl, NaHCO₃, or NH₄Cl. The solvent in the organic phase can be removed by reduced vacuum evaporation and the resulting residue may be purified using a silica gel column or HPLC.

### Pharmaceutical Compositions

This invention also provides a pharmaceutical composition comprising at least one of the compounds of the presently claimed invention as described herein or a pharmaceutically acceptable salt thereof, and a pharmaceutically acceptable carrier or diluent.

In certain embodiments, the composition is in the form of a hydrate, solvate or pharmaceutically acceptable salt. The composition can be administered to the subject by any suitable route of administration, including, without limitation, oral and parenteral.

The phrase "pharmaceutically acceptable carrier" as used herein means a pharmaceutically acceptable material, composition or vehicle, such as a liquid or solid filler, diluent, excipient, solvent or encapsulating material, involved in carrying or transporting the subject pharmaceutical agent from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as butylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations. The term "carrier" denotes an organic or inorganic ingredient, natural or synthetic, with which the active ingredient is combined to facilitate the application. The components of the pharmaceutical compositions also are capable of being comingled with the compounds of the present invention, and with each other, in a manner such that there is no interaction which would substantially impair the desired pharmaceutical efficiency.

As set out above, certain embodiments of the present pharmaceutical agents may be provided in the form of pharmaceutically acceptable salts. The term "pharmaceutically-acceptable salt", in this respect, refers to the relatively non-toxic, inorganic and organic acid addition salts of compounds of the present invention. These salts can be prepared *in situ* during the final isolation and purification of the compounds of the invention, or by separately reacting a purified compound of the invention in its free base form with a suitable organic or inorganic acid, and isolating the salt thus formed. Representative salts include hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (*See,* for example, Berge et al., (1977) "Pharmaceutical Salts", J. Pharm. Sci. 66:1-19.)

The pharmaceutically acceptable salts of the subject compounds include the conventional nontoxic salts or quaternary ammonium salts of the compounds, *e.g*., from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, butionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In other cases, the compounds of the present invention may contain one or more acidic functional groups and, thus, are capable of forming pharmaceutically acceptable salts with pharmaceutically acceptable bases. The term "pharmaceutically acceptable salts" in these instances refers to the relatively non-toxic, inorganic and organic base addition salts of compounds of the present invention. These salts can likewise be prepared *in situ* during the final isolation and purification of the compounds, or by separately reacting the purified compound in its free acid form with a suitable base, such as the hydroxide, carbonate or bicarbonate of a pharmaceutically acceptable metal cation, with ammonia, or with a pharmaceutically acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (*See,* for example, Berge *et al.,* supra).

Wetting agents, emulsifiers and lubricants, such as sodium lauryl sulfate, magnesium stearate, and polyethylene oxide-polybutylene oxide copolymer as well as coloring agents, release agents, coating agents, sweetening, flavoring and perfuming agents, preservatives and antioxidants can also be present in the compositions.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient, which can be combined with a carrier material to produce a single dosage form will generally be that amount of the compound which produces a therapeutic effect. Generally, out of 100%, this amount will range from about 1% to about 99% of active ingredient, preferably from about 5% to about 70%, most preferably from about 10% to about 30%.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouthwashes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically-acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, sodium carbonate, and sodium starch glycolate; solution retarding agents, such as paraffin; absorption accelerators, such as quaternary ammonium compounds; wetting agents, such as, for example, cetyl alcohol, glycerol monostearate, and polyethylene oxide-polybutylene oxide copolymer; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxybutylmethyl cellulose), lubricant, inert diluent, preservative, disintegrant (for example, sodium starch glycolate or cross-linked sodium carboxymethyl cellulose), surface-active or dispersing agent. Molded tablets, may be, made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxybutylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions, which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions, which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isobutyl alcohol, ethyl carbonate, ethyl acetate, benzyl alcohol, benzyl benzoate, butylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof. Additionally, cyclodextrins, *e.g*., hydroxybutyl-β-cyclodextrin, may be used to solubilize compounds.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar--agar and tragacanth, and mixtures thereof.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as propane and butane.

Transdermal patches have the added advantage of providing controlled delivery of a compound of the present invention to the body. Such dosage forms can be made by dissolving, or dispersing the pharmaceutical agents in the proper medium. Absorption enhancers can also be used to increase the flux of the pharmaceutical agents of the invention across the skin. The rate of such flux can be controlled, by either providing a rate controlling membrane or dispersing the compound in a polymer matrix or gel.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally-administered drug form is accomplished by dissolving or suspending the drug in an oil vehicle. One strategy for depot injections includes the use of polyethylene oxide-polypropylene oxide copolymers wherein the vehicle is fluid at room temperature and solidifies at body temperature.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly (orthoesters) and poly (anhydrides). Depot injectable formulations are also prepared by entrapping the drug in liposomes or microemulsions, which are compatible with body tissue.

When the compounds of the present invention are administered as pharmaceuticals, to humans and animals, they can be given *per se* or as a pharmaceutical composition containing, for example, 0.1% to 99.5% (more preferably, 0.5% to 90%) of active ingredient in combination with a pharmaceutically acceptable carrier.

The compounds and pharmaceutical compositions of the present invention can be employed in combination therapies, that is, the compounds and pharmaceutical compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, the compound of the present invention may be administered concurrently with another therapeutic agent). Non-limiting examples of another therapeutic agent including biological and small molecule anticancer agent, immunomodulator, immunosuppressant, antiinflammatory agent, anti-arthritis agent, corticosteroid, antidiarrheal agent, anticoagulation agent, and antithrombotic agent.

The compounds of the invention may be administered intravenously, intramuscularly, intraperitoneally, subcutaneously, topically, orally, or by other acceptable means. The compounds may be used to treat arthritic conditions in mammals (*e.g*., humans, livestock, and domestic animals), race horses, birds, lizards, and any other organism, which can tolerate the compounds.

A pharmaceutical pack or kit comprising one or more containers filled with one or more of the ingredients of the pharmaceutical compositions of the invention is herein described. Optionally associated with such container(s) can be a notice in the form prescribed by a governmental agency regulating the manufacture, use or sale of pharmaceuticals or biological products, which notice reflects approval by the agency of manufacture, use or sale for human administration.

### Administration to a Subject

In yet another aspect, the present invention provides a compound of the invention, or a pharmaceutically-acceptable salt thereof for use as a medicament. In another aspect, the presently claimed invention provides a compound of the presently claimed invention, or a pharmaceutically-acceptable salt thereof for use in a method for treating a condition in a mammalian species in need thereof, the method comprising administering to the mammalian species a therapeutically effective amount of the compound or a pharmaceutically acceptable salt thereof, wherein the condition is selected from the group consisting of cancer, an immunological disorder, a central nerve system (CNS) disorder, an inflammatory disorder, a gastroenterological disorder, a metabolic disorder, a cardiovascular disorder, and a kidney disease.

In some embodiments, the cancer is selected from the group consisting of biliary tract cancer, brain cancer, breast cancer, cervical cancer, choriocarcinoma, colon cancer, endometrial cancer, esophageal cancer, gastric (stomach) cancer, intraepithelial neoplasms, leukemias, lymphomas, liver cancer, lung cancer, melanoma, neuroblastomas, oral cancer, ovarian cancer, pancreatic cancer, prostate cancer, rectal cancer, renal (kidney) cancer, sarcomas, skin cancer, testicular cancer, and thyroid cancer.

In some embodiments, the inflammatory disorder is an inflammatory skin condition, arthritis, psoriasis, spondylitis, parodontitits, or an inflammatory neuropathy. In some embodiments, the gastroenterological disorder is an inflammatory bowel disease such as Crohn's disease or ulcerative colitis.

In some embodiments, the immunological disorder is transplant rejection or an autoimmune disease (*e.g*., rheumatoid arthritis, multiple sclerosis, systemic lupus erythematosus, or Type I diabetes mellitus). In some embodiments, the Central Nerve System (CNS) disorder is Alzheimer's disease.

In some embodiments, the metabolic disorder is obesity or Type II diabetes mellitus. In some embodiments, the cardiovascular disorder is an ischemic stroke. In some embodiments, the kidney disease is chronic kidney disease, nephritis, or chronic renal failure.

In some embodiments, the mammalian species is human.

In some embodiments, the condition is selected from the group consisting of cancer, transplant rejection, rheumatoid arthritis, multiple sclerosis, systemic lupus erythematosus, Type I diabetes mellitus, Alzheimer's disease, inflammatory skin condition, inflammatory neuropathy, psoriasis, spondylitis, parodontitis, inflammatory bowel disease, obesity, Type II diabetes mellitus, ischemic stroke, chronic kidney disease, nephritis, chronic renal failure, and a combination thereof.

A method of blocking Kv1.3 potassium channel in a mammalian species in need thereof is described herein, including administering to the mammalian species a therapeutically effective amount of at least one compound of the invention, or a pharmaceutically acceptable salt thereof.

In some embodiments, the compounds described herein is selective in blocking the Kv 1.3 potassium channels with minimal or no off-target inhibition activities against other potassium channels, or against calcium or sodium channels. In some embodiments, the compounds described herein do not block the hERG channels and therefore have desirable cardiovascular safety profiles.

Some aspects of the invention involve an effective amount of a composition to a subject to be administered to achieve a specific outcome. The small molecule compositions useful according to the disclosed methods thus can be formulated in any manner suitable for pharmaceutical use.

The formulations of the invention are to be administered in pharmaceutically acceptable solutions, which may routinely contain pharmaceutically acceptable concentrations of salt, buffering agents, preservatives, compatible carriers, adjuvants, and optionally other therapeutic ingredients.

For use in therapy, an effective amount of the compound can be administered to a subject by any mode allowing the compound to be taken up by the appropriate target cells. "Administering" the pharmaceutical composition of the present invention can be accomplished by any means known to the skilled artisan. Specific routes of administration include, but are not limited to, oral, transdermal (*e.g*., via a patch), parenteral injection (subcutaneous, intradermal, intramuscular, intravenous, intraperitoneal, intrathecal, etc.), or mucosal (intranasal, intratracheal, inhalation, intrarectal, intravaginal, etc.). An injection can be in a bolus or a continuous infusion.

For example the pharmaceutical compositions according to the invention are often to be administered by intravenous, intramuscular, or other parenteral means. They can also be to be administered by intranasal application, inhalation, topically, orally, or as implants, and even rectal or vaginal use is possible. Suitable liquid or solid pharmaceutical preparation forms are, for example, aqueous or saline solutions for injection or inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of present methods for drug delivery, *see* Langer R (1990) Science 249:1527-33.

The concentration of compounds included in compositions used in the disclosed methods can range from about 1 nM to about 100 µM. Effective doses are believed to range from about 10 picomole/kg to about 100 micromole/kg.

The pharmaceutical compositions are preferably prepared and to be administered in dose units. Liquid dose units are vials or ampoules for injection or other parenteral administration. Solid dose units are tablets, capsules, powders, and suppositories. For treatment of a patient, depending on activity of the compound, manner of administration, purpose of the administration (*i.e.,* prophylactic or therapeutic), nature and severity of the disorder, age and body weight of the patient, different doses may be necessary. The administration of a given dose can be carried out both by single administration in the form of an individual dose unit or else several smaller dose units. Repeated and multiple administration of doses at specific intervals of days, weeks, or months apart are also contemplated by the invention.

The compositions can be to be administered *per se* (neat) or in the form of a pharmaceutically acceptable salt. When used in medicine the salts should be pharmaceutically acceptable, but non-pharmaceutically acceptable salts can conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

Compositions suitable for parenteral administration conveniently include sterile aqueous preparations, which can be isotonic with the blood of the recipient. Among the acceptable vehicles and solvents are water, Ringer's solution, phosphate buffered saline, and isotonic sodium chloride solution. In addition, sterile, fixed oils are conventionally employed as a solvent or suspending medium. For this purpose, any bland fixed mineral or non-mineral oil may be employed including synthetic mono- or diglycerides. In addition, fatty acids such as oleic acid find use in the preparation of injectables. Carrier formulations suitable for subcutaneous, intramuscular, intraperitoneal, intravenous, etc. administrations can be found in Remington's Pharmaceutical Sciences, Mack Publishing Company, Easton, PA.

The compounds useful in the invention can be to be delivered in mixtures of more than two such compounds. A mixture can further include one or more adjuvants in addition to the combination of compounds.

A variety of administration routes is available. The particular mode selected will depend, of course, upon the particular compound selected, the age and general health status of the subject, the particular condition being treated, and the dosage required for therapeutic efficacy. The methods, generally speaking, can be practiced using any mode of administration that is medically acceptable, meaning any mode that produces effective levels of response without causing clinically unacceptable adverse effects. Preferred modes of administration are discussed above.

The compositions can conveniently be presented in unit dosage form and can be prepared by any of the methods well known in the art of pharmacy. All methods include the step of bringing the compounds into association with a carrier which constitutes one or more accessory ingredients. In general, the compositions are prepared by uniformly and intimately bringing the compounds into association with a liquid carrier, a finely divided solid carrier, or both, and then, if necessary, shaping the product.

Other delivery systems can include time-release, delayed release or sustained release delivery systems. Such systems can avoid repeated administrations of the compounds, increasing convenience to the subject and the physician. Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109. Delivery systems also include non-polymer systems that are: lipids including sterols such as cholesterol, cholesterol esters and fatty acids or neutral fats such as mono-di-and tri-glycerides; hydrogel release systems; silastic systems; peptide-based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Pat. Nos. 4,452,775, 4,675,189, and 5,736,152, and (b) diffusional systems in which an active component permeates at a controlled rate from a polymer such as described in U.S. Pat. Nos. 3,854,480, 5,133,974 and 5,407,686. In addition, pump-based hardware delivery systems can be used, some of which are adapted for implantation.

### Assays for Effectiveness of Kv1.3 potassium channel blockers

In some embodiments, the compounds as described herein are tested for their activities against Kv1.3 potassium channel. In some embodiments, the compounds as described herein are tested for their Kv1.3 potassium channel electrophysiology. In some embodiments, the compounds as described herein are tested for their hERG electrophysiology.

The representative examples which follow are intended to help illustrate the invention, and are not intended to, nor should they be construed to, limit the scope of the invention. The following examples contain important additional information, exemplification and guidance which can be adapted to the practice of this invention in its various embodiments.

### EXAMPLES

Examples 1-76 describe various intermediates used in the syntheses of representative compounds of Formula I disclosed herein.

### Example 1. Intermediate 1 (1-(4-(4,5-dichloro-2-hydroxybenzoyl)piperidin-1-yl)ethanone)

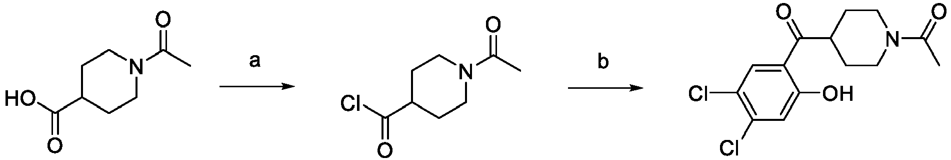

### Step a:

To a stirred solution of 1-acetylpiperidine-4-carboxylic acid (2.00 g, 11.68 mmol) in DCE (20 mL) was added SOCl₂ (10 mL) dropwise at 0 °C under nitrogen atmosphere. The reaction solution was allowed to warm to room temperature. After stirring for additional 1.5 h at room temperature, the resulting solution was concentrated under reduced pressure to afford crude 1-acetylpiperidine-4-carbonyl chloride, which was used in the next step without further purification.

### Step b:

To a solution of 1-acetylpiperidine-4-carbonyl chloride (0.39 g, 2.03 mmol) in DCE (20 mL) was added 1,2-dichloro-4-methoxybenzene (0.30 g, 1.69 mmol) at room temperature under nitrogen atmosphere. After stirring for 5 min, anhydrous AlCl₃ (0.49 g, 3.73 mmol) was added in portions at 0 °C under nitrogen atmosphere. The reaction mixture was allowed to warm to 50 °C and stirred for 2 h under nitrogen atmosphere. After cooling to 0 °C, the resulting mixture was quenched with ice water (30 mL) and extracted with DCM (3 x 30 mL). The combined organic layers were washed with brine (2 x 20 mL), dried over anhydrous Na₂SO₄ and filtered. The filtrate was concentrated under reduced pressure. The residue was purified by silica gel column chromatography, eluted with DCM/MeOH (10/1) to afford the crude product as a dark yellow solid. The crude product was purified by Prep-HPLC with following conditions: Column: Sunfire Prep C18 OBD Column, 10 µm, 19 x 250 mm; Mobile Phase A: water (plus 0.05% TFA), Mobile Phase B: ACN; Flow rate: 20 mL/min; Gradient: 56% B to 64% B in 9 min; Detector: UV 254/210 nm; Retention time: 6.98 min. The fractions containing desired product were collected and concentrated under reduced pressure to afford 1-(4-(4,5-dichloro-2-hydroxybenzoyl)piperidin-1-yl)ethanone as an off-white solid (85.6 mg, 16%): LCMS (ESI) calc'd for C₁₄H₁₅Cl₂NO₃ [M + H]⁺: 316, 318 (3 : 2), found 316, 318 (3 : 2); ¹H NMR (300 MHz, DMSO-*d*₆) δ 11.65 (s, 1H), 7.94 (s, 1H), 7.22 (s, 1H), 4.31 (d, *J =* 13.2 Hz, 1H), 3.85 (d, *J =* 13.2 Hz, 1H), 3.69-3.54 (m, 1H), 3.21-3.03 (m, 1H), 2.75-2.58 (m, 1H), 1.96 (s, 3H), 1.86-1.71 (m, 2H), 1.53-1.18 (m, 2H).

### Example 2. Intermediate 2 ((4,5-dichloro-2-hydroxyphenyl)(piperidin-4-yl)methanone)

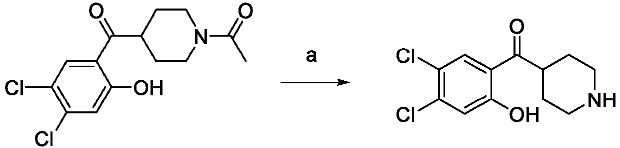

### Step a:

A mixture of 1-[4-[(4,5-dichloro-2-hydroxyphenyl)carbonyl]piperidin-1-yl]ethan-1-one (0.15 g, 0.48 mmol) in aq. HCl (6 *N,* 15 mL) was stirred for 4 h at 105 °C. After cooling to room temperature, the resulting mixture was concentrated under reduced pressure. The residue was purified by Prep-HPLC with the following conditions: Column: XBridge Shield RP18 OBD Column 19 x 250 mm, 10 µm; Mobile Phase A: water with 10 mmol/L NH₄HCO₃, Mobile Phase B: ACN; Flow rate: 25 mL/min; Gradient: 20% B to 60% B in 9 min; Detector: UV 254/210 nm; Retention time: 8.5 min. The fractions containing desired product were collected and concentrated under reduced pressure to afford (4,5-dichloro-2-hydroxyphenyl)(piperidin-4-yl)methanone as a yellow solid (23.5 mg, 18%): LCMS (ESI) calc'd for C₁₂H₁₃Cl₂NO₂ [M + H]⁺: 274, 276 (3 : 2), found 274, 276 (3 : 2); ¹H NMR (300 MHz, DMSO-*d*₆) δ 7.85 (br, 1H), 7.57 (s, 1H), 6.67 (s, 1H), 3.96-3.76 (m, 1H).3.12 (d, *J =* 12.0 Hz, 2H), 2.83-2.62 (m, 2H), 1.81 (d, *J =* 13.1 Hz, 2H), 1.57-1.34 (m, 2H).

### Example 3. Intermediate 3 (tert-butyl 4-(amino(4,5-dichloro-2-methoxyphenyl)methyl)piperidine-1-carboxylate trifluoroacetic acid)

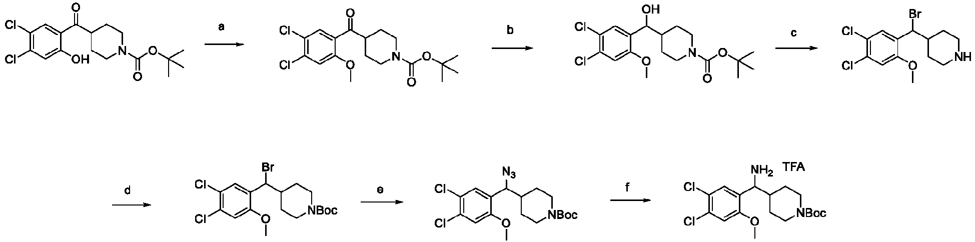

### Step a:

To a stirred solution of tert-butyl 4-(4,5-dichloro-2-hydroxybenzoyl)piperidine-1-carboxylate (1.00 g, 2.68 mmol) in DMF (10 mL) were added K₂CO₃ (0.74 g, 5.36 mmol) and CH₃I (0.75 g, 5.36 mmol) at room temperature under nitrogen atmosphere. The reaction mixture was stirred for 1 h at room temperature under nitrogen atmosphere. The resulting mixture was diluted with water (60 mL) at room temperature and extracted with EA (3 x 50 mL). The combined organic layers were washed with brine (2 x 10 mL), dried over anhydrous Na₂SO₄. After filtration, the filtrate was concentrated under reduced pressure. The residue was purified by silica gel column chromatography, eluted with PE/EA (4/1) to afford *tert*-butyl 4-(4,5-dichloro-2-methoxybenzoyl)piperidine-1-carboxylate as a light yellow solid (0.85 g, 83%): LCMS (ESI) calc'd for C₁₈H₂₃Cl₂NO₄ [M + H]⁺: 388, 390 (3 : 2), found 388, 390 (3 : 2);

### Step b:

To a stirred solution of *tert*-butyl 4-[(4,5-dichloro-2-methoxyphenyl)carbonyl]piperidine-1-carboxylate (1.70 g, 4.38 mmol) in THF (20 mL) was added NaBH₄ (0.25 g, 6.58 mmol) in portions at 0 °C. After stirring for additional 1.5 h, the reaction mixture was quenched with saturated aq. NH₄Cl (50 mL) and extracted with EA (3 x 50 mL). The combined organic layers were washed with brine (2 x 20 mL), dried over anhydrous Na₂SO₄. After filtration, the filtrate was concentrated under reduced pressure. The residue was purified by silica gel column chromatography, eluted with DCM/MeOH (30/1) to afford *tert-*butyl 4-((4,5-dichloro-2-methoxyphenyl)(hydroxy)methyl)cyclohexane-1-carboxylate as an off-white solid (1.40 g, 82%): LCMS (ESI) calc'd for C₁₈H₂₅Cl₂NO₄ [M + H]⁺: 390, 392 (3 : 2), found 390, 392 (3 : 2); ¹H NMR (300 MHz, DMSO-*d*₆) δ 7.41 (s, 1H), 7.19 (s, 1H), 5..22 (d, *J =* 5.0 Hz, 1H), 4.61 (t, *J =* 5.2 Hz, 1H), 4.25 (d, *J =* 5.8 Hz, 1H), 3.99 (d, *J =* 5.8 Hz, 1H), 3.76 (s, 3H), 2.91-2.74 (m, 2H), 1.84-1.71 (m, 2H), 1.35 (s, 9H), 1.32-1.01 (m, 3H).

### Step c:

To a stirred solution of *tert*-butyl 4-[(4,5-dichloro-2-methoxyphenyl)(hydroxy)methyl]piperidine-1-carboxylate (2.50 g, 6.41 mmol) in DCM (18 mL) was added PBr₃ (3.50 g, 12.81 mmol) dropwise at 0 °C. After stirring for additional 1 h, the reaction solution was quenched with saturated aq. NaHCO₃ (30 mL) at 0 °C and extracted with EA (3 x 20 mL). The combined organic layers were washed with brine (2 x 10 mL), dried over anhydrous Na₂SO₄. After filtration, the filtrate was concentrated under reduced pressure to afford crude 4-(bromo(4,5-dichloro-2-methoxyphenyl)methyl)piperidine, which was used in the next step directly without further purification: LCMS (ESI) calc'd for C₁₃H₁₆BrCl₂NO [M + H]⁺: 352, 354, 356 (2 : 3 : 1), found 352, 354, 356 (2 : 3 : 1).

### Step d:

To a stirred solution of 4-[bromo(4,5-dichloro-2-methoxyphenyl)methyl]piperidine (2.00 g, 5.66 mmol) and Et₃N (0.90 g, 8.50 mmol) in DCM (20 mL) was added a solution of Boc₂O (1.90 g, 8.50 mmol) in DCM (5 mL) dropwise at 0 °C. After stirring for additional 1 h at 0 °C, the resulting solution was concentrated under reduced pressure. The residue was purified by silica gel column chromatography, eluted with PE/EA (10/1) to afford *tert*-butyl 4-(bromo(4,5-dichloro-2-methoxyphenyl)methyl)piperidine-1-carboxylate as a yellow oil (0.30 g, 12% overall two steps): LCMS (ESI) calc'd for C₁₈H₂₄BrCl₂NO₃ [M + 1 - 56]⁺: 396, 398, 400 (2 : 3 : 1), found 396, 398, 400 (2 : 3 : 1); ¹H NMR (300 MHz, CDCl₃) δ 7.50 (s, 1H), 6.95 (s, 1H), 5.15 (d, *J =* 9.4 Hz, 1H), 4.18 (d, *J =* 13.5 Hz, 1H), 4.03 (d, *J =* 13.8 Hz, 1H), 3.85 (s, 3H), 2.81-2.54 (m, 2H), 2.27-2.02 (m, 2H), 1.48 (s, 9H), 1.32-0.97 (m, 3H).

### Step e:

To a stirred solution of *tert*-butyl 4-(bromo(4,5-dichloro-2-methoxyphenyl)methyl)piperidine-1-carboxylate (0.26 g, 0.58 mmol) in DMF (5 mL) was added NaN₃ (0.11 g, 1.74 mmol) at room temperature. The reaction mixture was allowed to warm to 100 °C and stirred for 16 h. The reaction mixture was diluted with water (30 mL) and extracted with EA (3 x 20 mL). The combined organic layers were washed with water (2 x 15 mL), dried over anhydrous Na₂SO₄. After filtration, the filtrate was concentrated under reduced pressure to a quarter of the volume. The residue was used in the next step directly without further purification: LCMS (ESI) calc'd for C₁₈H₂₄Cl₂N₄O₃ [M + H]⁺: 415, 417 (3 : 2), found 415, 417 (3 : 2).

### Step f:

To a stirred solution of *tert-*butyl 4-[azido(4,5-dichloro-2-methoxyphenyl)methyl]piperidine-1-carboxylate (0.20 g, 0.48 mmol) and PPh₃ (0.25 g, 0.96 mmol) in THF (2 mL) was added NH₃·H₂O (1 mL, 28% in H₂O) dropwise at room temperature. The reaction solution was stirred for 16 h at room temperature. The resulting solution was concentrated under reduced pressure. The residue was purified by reverse phase chromatography, eluted with 30% ACN in water (plus 0.05% TFA) to afford *tert-*butyl 4-(amino(4,5-dichloro-2-methoxyphenyl)methyl)piperidine-1-carboxylate trifluoroacetic acid as a yellow oil (0.10 g, 45% overall two steps): LCMS (ESI) calc'd for C₁₈H₂₆Cl₂N₂O₃ [M + H]⁺: 389, 391 (3 : 2), found 389, 391 (3 : 2); ¹H NMR (300 MHz, CD₃OD) δ 7.46 (s, 1H), 7.29 (s, 1H), 4.21-4.09 (m, 2H), 4.05-3.93 (m, 1H), 3.86 (s, 3H), 2.82-2.53 (m, 1H), 2.20-2.06 (m, 2H), 1.95-1.83 (m, 1H), 1.40 (s, 9H), 1.32-0.97 (m, 3H).

### Example 4. Intermediate 4 (1-(4-(amino(4,5-dichloro-2-methoxyphenyl)methyl)piperidin-1-yl)ethanone)

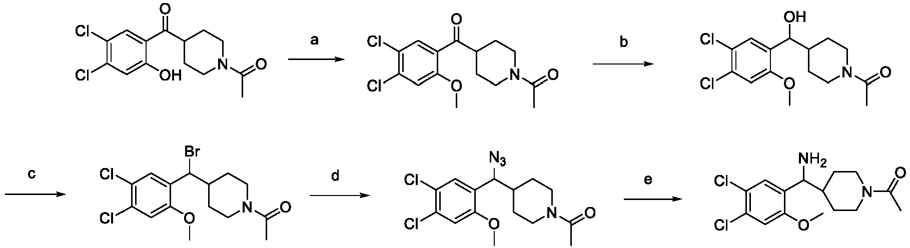

### Step a:

To a stirred solution of 1-[4-(4,5-dichloro-2-hydroxybenzoyl)piperidin-1-yl]ethan-1-one (1.00 g, 3.16 mmol) in DMF (10 mL) were added K₂CO₃ (0.87 g, 6.33 mmol) and CH₃I (0.90 g, 6.33 mmol) at room temperature under nitrogen atmosphere. The reaction mixture was stirred for 1 h at room temperature under nitrogen atmosphere. The resulting mixture was diluted with water (60 mL) at room temperature and extracted with EA (3 x 50 mL). The combined organic layers were washed with brine (2 x 10 mL), dried over anhydrous Na₂SO₄. After filtration, the filtrate was concentrated under reduced pressure. The residue was purified by silica gel column chromatography, eluted with PE/EA (4/1) to afford 1-[4-(4,5-dichloro-2-methoxybenzoyl)piperidin-1-yl]ethan-1-one as a light yellow solid (1.00 g, 95%): LCMS (ESI) calc'd for C₁₅H₁₇Cl₂NO₃ [M + H]⁺: 330, 332 (3 : 2), found 330, 332 (3 : 2).

### Step b:

To a stirred solution of 1-[4-[(4,5-dichloro-2-methoxyphenyl)carbonyl]piperidin-1-yl]ethan-1-one (0.70 g, 2.12 mmol) in MeOH (5 mL) was added NaBH₄ (0.12 g, 3.18 mmol) in portions at 0 °C. The reaction mixture was allowed to warm to room temperature and stirred for 3 h. The reaction mixture was quenched with saturated aq. NH₄Cl (30 mL) at 0 °C and extracted with EA (3 x 50 mL), dried over anhydrous Na₂SO₄. After filtration, the filtrate was concentrated under reduced pressure. The residue was purified by silica gel column chromatography, eluted with PE/EA (3/1) to afford 1-(4-((4,5-dichloro-2-methoxyphenyl)(hydroxy)methyl)piperidin-1-yl)ethanone as a yellow oil (0.46 g, 66%): LCMS (ESI) calc'd for C₁₅H₁₉Cl₂NO₃ [M + H]⁺: 332, 334 (3 : 2), found 332, 334 (3 : 2).

### Step c:

To a stirred solution of 1-[4-[(4,5-dichloro-2-methoxyphenyl)(hydroxy)methyl]piperidin-1-yl]ethan-1-one (0.46 g, 1.38 mmol) in DCM (5 mL) was added PBr₃ (0.75 g, 2.77 mmol) dropwise at 0 °C. After stirring for 1 h at 0 °C, the reaction solution was quenched with water (30 mL) at 0 °C and extracted with EA (4 x 10 mL). The combined organic layers were washed with brine (2 x 10 mL), dried over anhydrous Na₂SO₄. After filtration, the filtrate was concentrated under reduced pressure. The residue was purified by silica gel column chromatography, eluted with PE/EA (2/1) to afford 1-(4-(bromo(4,5-dichloro-2-methoxyphenyl)methyl)piperidin-1-yl)ethanone as a yellow oil (50 mg, 10%): LCMS (ESI) calc'd for C₁₅H₁₈BrCl₂NO₂ [M + H]⁺: 394, 396, 398 (2 : 3 : 1), found 394, 396, 398 (2 : 3 : 1); ¹H NMR (300 MHz, CD₃OD) δ 7.57 (s, 1H), 7.15 (s, 1H), 5.14 (dd, *J =* 9.5, 2.4 Hz, 1H), 3.99-3.90 (m, 1H), 3.85 (s, 3H), 3.84-3.80 (m, 1H), 3.18-2.91 (m, 1H), 2.69-2.45 (m, 1H), 2.38-2.17 (m, 2H), 2.05 (d, *J =* 15.0 Hz, 3H), 1.43-1.15 (m, 3H).

### Step d:

To a solution of 1-(4-(bromo(4,5-dichloro-2-hydroxyphenyl)methyl)piperidin-1-yl)ethanone (50 mg, 0.13 mmol) in DMF (3 mL) was added NaN₃ (26 mg, 0.40 mmol) at room temperature. The reaction mixture was allowed to warm to 100 °C and stirred for 8 h. After cooling to room temperature, the resulting mixture was diluted with water (20 mL) and extracted with EA (3 x 25 mL). The combined organic layers were washed with brine (3 x 10 mL), dried over anhydrous Na₂SO₄. After filtration, the filtrate was concentrated under reduced pressure to a quarter of volume. The residue was used in the next step directly without further purification: LCMS (ESI) calc'd for C₁₅H₁₈Cl₂N₄O₂ [M + H]⁺: 357, 359 (3 : 2), found 357, 359 (3 : 2).

### Step e:

To a solution of 1-[4-[azido(4,5-dichloro-2-methoxyphenyl)methyl]piperidin-1-yl]ethan-1-one (0.10 g, 0.28 mmol) in THF (4 mL) were added PPh₃ (0.15 g, 0.56 mmol) and NH₃·H₂O (1 mL, 28% in H₂O) at room temperature. The reaction mixture was stirred for 16 h at room temperature. The resulting mixture was concentrated under reduced pressure. The residue was purified by reverse phase chromatography, eluted 29% ACN in water (plus 0.05% TFA) to afford 1-(4-(amino(4,5-dichloro-2-methoxyphenyl)methyl)piperidin-1-yl)ethanone as a light yellow solid (30 mg, 71% overall two steps): LCMS (ESI) calc'd for C₁₅H₂₀Cl₂N₂O₂ [M + H]⁺: 331, 333 (3 : 2), found 331, 333 (3 : 2); ¹H NMR (300 MHz, CD₃OD) δ 7.47 (d, *J =* 1.9 Hz, 1H), 7.16 (d, *J =* 1.4 Hz, 1H), 4.60-4.40 (m, 1H), 3.99-3.82 (m, 2H), 3.81 (s, 3H), 3.17-2.89 (m, 1H), 2.66-2.44 (m, 1H), 2.15-1.78 (m, 5H), 1.43-1.03 (m, 3H).

### Example 5. Intermediate 5 (N-((2-(allyloxy)-4,5-dichlorophenyl)(piperidin-4-yl)methyl)-2-methylpropane-2-sulfinamide)

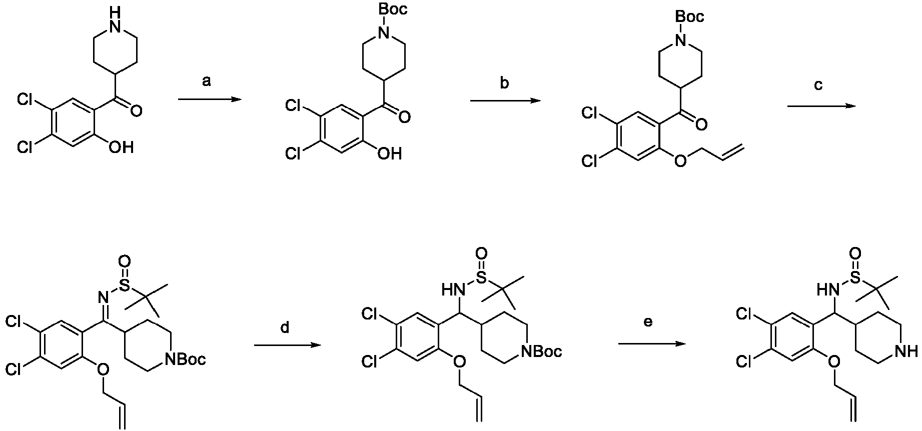

### Step a:

To a stirred solution of (4,5-dichloro-2-hydroxyphenyl)(piperidin-4-yl)methanone (17 g, 62.27 mmol) and Et₃N (31.44 g, 0.31 mol) in DCM (150 mL) was added Boc₂O (14.90 g, 68.21 mmol) in portions at 0 °C. The reaction solution was allowed to warm to room temperature and stirred for 4 h at room temperature. The resulting solution was quenched with water (300 mL) at room temperature and extracted with EA (3 x 300 mL). The combined organic layers were washed with brine (2 x 100 mL), dried over anhydrous Na₂SO₄. After filtration, the filtrate was concentrated under reduced pressure. The crude product was used in the next step directly without further purification: LCMS (ESI) calc'd for C₁₇H₂₁Cl₂NO₄ [M + H]⁺: 374, 376 (3 : 2), found 374, 376 (3 : 2).

### Step b:

To a stirred mixture of *tert*-butyl 4-(4,5-dichloro-2-hydroxybenzoyl)piperidine-1-carboxylate (crude) and K₂CO₃ (33.20 g, 0.24 mmol) in DMF (200 mL) was added allyl bromide (14.48 g, 0.12 mmol) dropwise at room temperature under nitrogen atmosphere. The reaction mixture was allowed to warm to 40 °C and stirred for 12 h. The resulting mixture was diluted with water (500 mL) and extracted with EA (2 x 500 mL). The combined organic layers were washed with brine (2 x 100 mL), dried over anhydrous Na₂SO₄. After filtration, the filtrate was concentrated under reduced pressure. The residue was purified by silica gel column chromatography, eluted with PE/EA (5/1) to afford *tert*-butyl 4-(2-(allyloxy)-4,5-dichlorobenzoyl)piperidine-1-carboxylate as a light yellow solid (15.40 g, 64% overall two steps): LCMS (ESI) calc'd for C₂₀H₂₅Cl₂NO₄ [M + H]⁺: 414, 416 (3 : 2), found 414, 416 (3 : 2); ¹H NMR (400 MHz, CDCl₃) δ 7.69 (s, 1H), 7.07 (s, 1H), 6.16-6.00 (m, 1H), 5.55-5.36 (m, 2H), 4.62 (d, *J =* 5.7 Hz, 2H), 4.18-4.08 (m, 2H), 3.41-3.32 (m, 1H), 2.94-2.74 (m, 2H), 1.93-1.81 (m, 2H), 1.68-1.54 (m, 2H), 1.54 (s, 9H).

### Step c:

To a stirred mixture of *tert-*butyl 4-[4,5-dichloro-2-(prop-2-en-1-yloxy)benzoyl]piperidine-1-carboxylate (8.80 g, 21.24 mmol) and Ti(OEt)₄ (21.51 g, 94.30 mmol) in THF (50 mL) was added 2-methylpropane-2-sulfinamide (3.86 g, 31.85 mmol) in portions at room temperature under nitrogen atmosphere. The reaction mixture was allowed to warm to 70 °C and stirred for 16 h under nitrogen atmosphere. The resulting mixture was quenched with water (200 mL) at room temperature. The solid was formed and filtered. The filtrate was extracted with EA (3 x 200 mL). The combined organic layers were washed with brine (2 x 100 mL), dried over anhydrous Na₂SO₄. After filtration, the filtrate was concentrated under reduced pressure to crude *tert*-butyl 4-((2-(allyloxy)-4,5-dichlorophenyl)((*tert-*butylsulfinyl)imino)methyl)piperidine-1-carboxylate. The crude product was used in the next step directly without further purification: LCMS (ESI) calc'd for C₂₄H₃₄Cl₂N₂O₄S [M + H]⁺: 517, 519 (3 : 2), found 517, 519 (3 : 2); ¹H NMR (400 MHz, CDCl₃) δ 7.16 (s, 1H), 6.95 (s, 1H), 6.05-5.95 (m, 1H), 5.43-5.26 (m, 2H), 4.56 (d, *J =* 5.8 Hz, 2H), 4.25-4.00 (m, 2H), 2.86-2.52 (m, 3H), 1.91-1.54 (m, 4H), 1.45 (s, 9H), 1.21 (s, 9H).

### Step d:

To a solution of *tert*-butyl 4-((2-(allyloxy)-4,5-dichlorophenyl)((*tert-*butylsulfinyl)imino)methyl)piperidine-1-carboxylate (crude) in MeOH (80 mL) was added NaBH₄ (1.21 g, 31.86 mmol) in portions at 0 °C under nitrogen atmosphere. After the addition, the reaction mixture was allowed to warm to room temperature and stirred for 2 h under nitrogen atmosphere. The resulting mixture was quenched with water (150 mL) at 0 °C and extracted with EA (3 x 100 mL). The combined organic layers were washed with water (2 x 50 mL), dried over anhydrous Na₂SO₄. After filtration, the filtrate was concentrated under reduced pressure. The residue was purified by reverse phase chromatography, eluted with 45% ACN in water (plus 0.05% TFA) to afford *tert*-butyl 4-((2-(allyloxy)-4,5-dichlorophenyl)(1,1-dimethylethylsulfinamido)methyl)piperidine-1-carboxylate as a yellow oil (6.58 g, 60% overall two steps): LCMS (ESI) calc'd for C₂₄H₃₆Cl₂N₂O₄S [M + H]⁺: 519, 521 (3 : 2), found 519, 521 (3 : 2); ¹H NMR (300 MHz, CD₃OD) δ 7.51 (s, 1H), 7.15 (s, 1H), 6.19-6.03 (m, 1H), 5.52-5.29 (m, 2H), 4.67-4.58 (m, 2H), 4.30 (d, *J =* 9.1 Hz, 1H), 4.04 (dd, *J =* 35.2, 13.5 Hz, 2H), 2.84-2.50 (m, 2H), 2.14-1.87 (m, 2H), 1.46 (s, 9H), 1.30-1.05 (m, 3H), 1.23 (s, 9H).

### Step e:

To a stirred solution of *tert*-butyl 4-((2-(allyloxy)-4,5-dichlorophenyl)(1,1-dimethylethylsulfinamido)methyl)piperidine-1-carboxylate (6.58 g, 12.68 mmol) in DCM (100 mL) was added TFA (20 mL) dropwise at room temperature. The reaction solution was stirred for 1 h at room temperature. The resulting solution was diluted with water (200 mL). The pH value of the reaction system was adjusted to 9 with saturated aq. NaHCO₃ at 0 °C. The aqueous layer was extracted with EA (3 x 500 mL). The combined organic layers were washed with brine (2 x 50 mL), dried over anhydrous Na₂SO₄. After filtration, the filtrate was concentrated under reduced pressure. The residue was purified by reverse phase chromatography, eluted with 35% ACN in water (plus 0.05% TFA) to afford *N*-((2-(allyloxy)-4,5-dichlorophenyl)(piperidin-4-yl)methyl)-2-methylpropane-2-sulfinamide as a light yellow oil (4.24 g, 80%): LCMS (ESI) calc'd for C₁₉H₂₈Cl₂N₂O₂S [M + H]⁺: 419, 421 (3 : 2), found 419, 421 (3 : 2); ¹H NMR (300 MHz, DMSO-*d*₆ + D₂O) δ 7.50 (s, 1H), 7.19 (s, 1H), 6.06-5.92 (m, 1H), 5.42-5.18 (m, 2H), 4.63-4.48 (d, *J =* 4.8 Hz, 2H), 4.47-4.34 (d, *J =* 8.7 Hz, 1H), 3.38-3.14 (m, 2H), 2.84-2.65 (m, 2H), 2.20-2.05 (m, 1H), 1.96-1.82 (m, 1H), 1.42-1.18 (m, 3H), 0.97 (s, 9H).

### Example 6. Intermediate 6 ((S)-N-((R)-(2-(allyloxy)-4,5-dichlorophenyl)(piperidin-4-yl)methyl)-2-methylpropane-2-sulfinamide), Method A

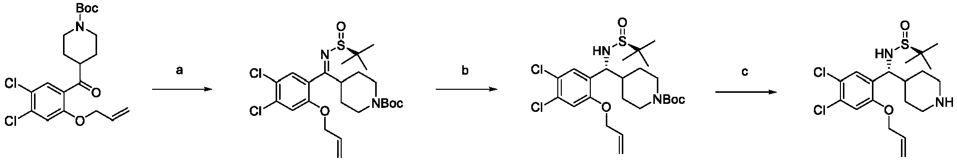

### Step a:

To a stirred solution of *tert*-butyl 4-(2-(allyloxy)-4,5-dichlorobenzoyl)piperidine-1-carboxylate (5.16 g, 12.45 mmol) and Ti(OEt)₄ (8.52 g, 37.36 mmol) in THF (50 mL) was added (*S*)-2-methylpropane-2-sulfinamide (1.67 g, 13.70 mmol) at room temperature under nitrogen atmosphere. The reaction solution was allowed to warm to 70 °C and stirred for 36 h. After cooling to room temperature, the resulting solution was quenched with water (300 mL) at room temperature. The solid was formed and filtered. The filtrate was extracted with EA (2 x 500 mL). The combined organic layers were washed with brine (2 x 100 mL), dried over anhydrous Na₂SO₄. After filtration, the filtrate was concentrated under reduced pressure. The residue was dried in a vacuum oven to afford (*S*)-*tert*-butyl 4-((2-(allyloxy)-4,5-dichlorophenyl)((*tert-*butylsulfinyl)imino)methyl)piperidine-1-carboxylate as a light yellow solid (6.00 g, 93%): LCMS (ESI) calc'd for C₂₄H₃₄Cl₂N₂O₄S [M + H]⁺: 517, 519 (3 : 2), found 517, 519 (3 : 2); ¹H NMR (300 MHz, CDCl₃) δ 7.28 (s, 1H), 6.97 (s, 1H), 6.06-5.94 (m, 1H), 5.46-5.26 (m, 2H), 4.58 (d, *J =* 5.7 Hz, 2H), 4.23-4.01 (m, 2H), 3.81-3.62 (m, 1H), 2.84-2.57 (m, 2H), 1.93-1.81 (m, 2H), 1.68-1.54 (m, 2H), 1.46 (s, 9H), 1.22 (s, 9H).

### Step b:

To a solution of (*S*)-*tert*-butyl 4-((2-(allyloxy)-4,5-dichlorophenyl)((*tert-*butylsulfinyl)imino)methyl)piperidine-1-carboxylate (1.50 g, 2.90 mmol) in toluene (10 mL) was added DIBAL-H (4.35 mL, 4.35 mmol, 1 M in toluene) dropwise over 30 min at -65 °C under nitrogen atmosphere. After the addition, the reaction solution was stirred for 3 h at -65 °C under nitrogen atmosphere. The resulting solution was quenched with water (20 mL) at -65 °C, and then diluted with saturated aq. potassium sodium tartrate (200 mL). The aqueous layers were extracted with EA (3 x 100 mL). The combined organic layers were washed with water (2 x 50 mL), dried over anhydrous Na₂SO₄. After filtration, the filtrate was concentrated under reduced pressure. The residue was purified by reverse phase chromatography, eluted with 45% ACN in water (plus 0.05% TFA) to afford *tert*-butyl 4-((*R*)-(2-(allyloxy)-4,5-dichlorophenyl)((*S*)-1,1-dimethylethylsulfinamido)methyl)piperidine-1-carboxylate as a yellow oil (0.78 g, 52%): LCMS (ESI) calc'd for C₂₄H₃₆Cl₂N₂O₄S [M + H]⁺: 519, 521 (3 : 2), found 519, 521 (3 : 2); ¹H NMR (400 MHz, CDCl₃) δ 7.23 (s, 1H), 6.96 (s, 1H), 6.10-5.92 (m, 1H), 5.46-5.31 (m, 2H), 4.57 (d, *J =* 5.8 Hz, 2H), 4.50-4.37 (m, 1H), 4.28-4.04 (m, 2H), 3.84-3.64 (m, 1H), 2.71-2.49 (m, 2H), 2.01-1.81 (m, 2H), 1.47 (s, 9H), 1.49-1.22 (m, 3H), 1.17 (s, 9H).

### Step c:

To a stirred solution of tert-butyl 4-((*R*)-(2-(allyloxy)-4,5-dichlorophenyl)((*S*)-1,1-dimethylethylsulfinamido)methyl)piperidine-1-carboxylate (16.00 g, 30.80 mmol) in DCM (120 mL) was added TFA (30 mL) dropwise at room temperature. The reaction solution was stirred for 1 h at room temperature. The resulting solution was diluted with water (200 mL). The pH value of the reaction system was adjusted to 8 with saturated aq. NaHCO₃ at 0 °C. The aqueous layer was extracted with EA (3 x 500 mL). The combined organic layers were washed with brine (2 x 50 mL), dried over anhydrous Na₂SO₄. After filtration, the filtrate was concentrated under reduced pressure. The residue was purified by reverse phase chromatography, eluted with 35% ACN in water with 10 mmol/L NH₄HCO₃ to afford (*S*)-*N*-((*R*)-(2-(allyloxy)-4,5-dichlorophenyl)(piperidin-4-yl)methyl)-2-methylpropane-2-sulfinamide as a yellow oil (7.00 g, 46%): LCMS (ESI) calc'd for C₁₉H₂₈Cl₂N₂O₂S [M + H]⁺: 419, 421 (3 : 2), found 419, 421 (3 : 2); ¹H NMR (400 MHz, CDCl₃) δ 7.25 (s, 1H), 6.98 (s, 1H), 6.10-5.97 (m, 1H), 5.46-5.31 (m, 2H), 4.57 (d, *J =* 5.8 Hz, 2H), 4.49-4.37 (m, 1H), 4.88-3.95 (m, 1H), 3.42-3.24 (m, 2H), 2.81-2.67 (m, 2H), 2.21-2.09 (m, 1H), 2.04-1.97 (m, 1H), 1.65-1.42 (m, 3H), 1.17 (s, 9H).

### Intermediate 6 ((S)-N-((R)-(2-(allyloxy)-4,5-dichlorophenyl)(piperidin-4-yl)methyl)-2-methylpropane-2-sulfinamide), Method B

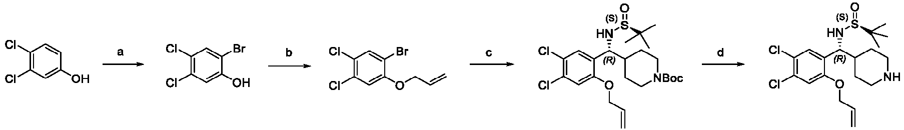

### Step a:

To a stirred solution of 3,4-dichlorophenol (100.00 g, 613.49 mmol) in DCM (1000 mL) was added Br₂ (98.04 g, 613.49 mmol) dropwise at 0 °C under nitrogen atmosphere. The reaction solution was stirred for 16 h at room temperature under nitrogen atmosphere. The reaction was quenched with saturated aq. Na₂S₂O₃ (500 mL) at 0 °C. The resulting mixture was extracted with EA (6 x 400 mL). The combined organic layers were washed with brine (2 x 400 mL), dried over anhydrous Na₂SO₄. After filtration, the filtrate was concentrated under reduced pressure to afford 2-bromo-4,5-dichlorophenol as a yellow oil. The crude product was used in the next step directly without further purification: ¹H NMR (400 MHz, CDCl₃) δ 7.57 (s, 1H), 7.15 (s, 1H).

### Step b:

To a stirred solution of 2-bromo-4,5-dichlorophenol (50.00 g, 206.71 mmol) and K₂CO₃ (57.14 g, 413.41 mmol) in DMF (500 mL) was added 3-bromoprop-1-ene (37.51 g, 310.06 mmol) dropwise at room temperature under nitrogen atmosphere. The reaction mixture was stirred for 16 h at 40 °C under nitrogen atmosphere. The resulting mixture was diluted with water (1.5 L) and extracted with EA (3 x 0.5 L). The combined organic layers were washed with brine (4 x 0.5 L), dried over anhydrous Na₂SO₄. After filtration, the filtrate was concentrated under reduced pressure. The residue was purified by silica gel column chromatography, eluted with PE to afford 1-bromo-4,5-dichloro-2-(prop-2-en-1-yloxy)benzene as a light yellow oil (40.00 g, 61%): ¹H NMR (400 MHz, CDCl₃) δ 7.65 (s, 1H), 6.98 (s, 1H), 6.12-6.06 (m, 1H), 5.60-5.29 (m, 2H), 4.69-4.57 (m, 2H).

### Step c:

To a stirred solution of 1-bromo-4,5-dichloro-2-(prop-2-en-1-yloxy)benzene (30.00 g, 106.39 mmol) in THF (800 mL) was added i-PrMgCl-LiCl (125 mL, 159.59 mmol, 1.3 M in THF) dropwise over 30 min at -15 °C under nitrogen atmosphere. The resulting mixture was stirred for additional 0.5 h at -15 °C under nitrogen atmosphere. To resulting solution was added the *tert-*butyl 4-[[(*S*)-2-methylpropane-2-sulfinyl]imino]methyl]piperidine-1-carboxylate (33.67 g, 106.39 mmol) at -15 °C under nitrogen atmosphere. After addition, the reaction mixture was stirred for 2 h at -15 °C. The reaction was quenched with saturated aq. NH₄Cl (100 mL) at -15 °C and extracted with EA (3 x 500 mL). The combined organic layers were washed with brine (3 x 100 mL), dried over anhydrous Na₂SO₄. After filtration, the filtrate was concentrated under reduced pressure. The residue was purified by silica gel column chromatography, eluted with PE/EA (1/2) to afford *tert*-butyl 4-[(R)-[4,5-dichloro-2-(prop-2-en-1-yloxy)phenyl]([[(*S*)-2-methylpropane-2-sulfinyl]amino])methyl]piperidine-1-carboxylate as an off-white solid (34.00 g, 58%): LCMS (ESI) calc'd for C₂₄H₃₆Cl₂N₂O₄S [M + H]⁺: 519, 521 (3 : 2), found 519, 521 (3 : 2); ¹H NMR (400 MHz, CD₃OD) δ 7.44 (s, 1H), 7.17 (s, 1H), 6.18-6.03 (m, 1H), 5.49-5.31 (m, 2H), 4.62 (d, *J =* 5.2 Hz, 2H), 4.52 (d, *J =* 8.8 Hz, 1H), 4.13 (t, *J* = 10.0 Hz, 1H), 4.01 (d, *J =* 13.4 Hz, 1H), 2.70 (d, *J =* 35.8 Hz, 2H), 2.12 (d, *J =* 13.6 Hz, 1H), 2.01-1.88 (m, 1H), 1.46 (s, 9H), 1.36-1.19 (m, 3H), 1.13 (d, *J* = 1.4 Hz, 9H).

### Step d:

To a stirred mixture of *tert*-butyl 4-[(*R*)-[4,5-dichloro-2-(prop-2-en-1-yloxy)phenyl]([[(*S*)-2-methylpropane-2-sulfinyl]amino])methyl]piperidine-1-carboxylate (5.70 g, 10.97 mmol) in DCM (40 mL) was added TFA (10 mL) dropwise at room temperature. The reaction solution was stirred for 1 h at room temperature. The mixture was neutralized to pH 9 with saturated aq. NaHCO₃ at 0 °C. The resulting mixture was extracted with EA (2 x 100 mL). The combined organic layers were washed with brine (2 x 100 mL), dried over anhydrous Na₂SO₄. After filtration, the filtrate was concentrated under reduced pressure. The crude product was purified by reverse phase chromatography, eluted with the 40% ACN in water with 10 mmol/L NH₄HCO₃ to afford (*S*)-*N*-[(*R*)-[4,5-dichloro-2-(prop-2-en-1-yloxy)phenyl](piperidin-4-yl)methyl]-2-methylpropane-2-sulfinamide as a yellow solid (3.50 g, 68%): LCMS (ESI) calc'd for C₁₉H₂₈Cl₂N₂O₂S [M + H]⁺: 419, 421 (3 : 2), found 419, 421 (3 : 2); ¹H NMR (400 MHz, CDCl₃) δ 7.25 (s, 1H), 6.98 (s, 1H), 6.10-5.97 (m, 1H), 5.46-5.31 (m, 2H), 4.57 (d, *J =* 5.8 Hz, 2H), 4.49-4.37 (m, 1H), 4.88-3.95 (m, 1H), 3.42-3.24 (m, 2H), 2.81-2.67 (m, 2H), 2.21-2.09 (m, 1H), 2.04-1.97 (m, 1H), 1.65-1.42 (m, 3H), 1.17 (s, 9H).

### Example 7. (which is a reference example) Intermediate 7 (N-((2-hydroxynaphthalen-1-yl)(pyridin-4-yl)methyl)acetamide)

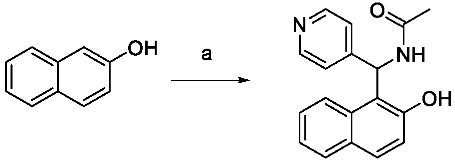

### Step a:

To a stirred mixture of naphthalen-2-ol (3.50 g, 24.28 mmol), acetamide (1.72 g, 29.13 mmol) and pyridine-4-carbaldehyde (2.60 g, 24.28 mmol) was added AlCl₃ (0.49 g, 3.64 mmol) in several portions at 110 °C under nitrogen atmosphere. The reaction mixture was stirred for 8 h at 110 °C under nitrogen atmosphere. After cooling to room temperature, the reaction mixture was quenched with water (60 mL). The aqueous layers were extracted with EA (3 x 50 mL). The combined organic layers were washed with brine (3 x 30 mL), dried over anhydrous Na₂SO₄. After filtration, the filtrate was concentrated under reduced pressure. The residue was purified by silica gel column chromatography, eluted with DCM/MeOH (8/1) to afford *N*-[(2-hydroxynaphthalen-1-yl)(pyridin-4-yl)methyl]acetamide as a yellow green solid (1.00 g, 14%): LCMS (ESI) calc'd for C₁₈H₁₆N₂O₂ [M + H]⁺: 293, found 293; ¹H NMR (400 MHz, CD₃OD) δ 8.39 (d, *J* = 8.4 Hz, 2H), 8.03 (d, *J* = 8.6 Hz, 1H), 7.87 (t, *J* = 9.0 Hz, 2H), 7.54 (t, *J =* 7.7 Hz, 1H), 7.38 (t, *J =* 7.5 Hz, 1H), 7.29-7.24 (m, 3H), 7.21 (d, *J =* 8.6 Hz, 1H), 2.10 (s, 3H).

### Example 8. Intermediate 8 (N-((2-(allyloxy)-4,5-dichlorophenyl)(piperidin-4-yl)methyl)-2,2,2-trifluoroacetamide trifluoroacetic acid)

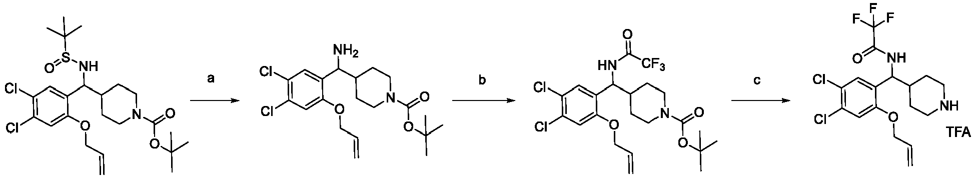

### Step a:

To a stirred solution of *tert*-butyl 4-((2-(allyloxy)-4,5-dichlorophenyl)(1,1-dimethylethylsulfinamido)methyl)piperidine-1-carboxylate (0.50 g, 0.96 mmol) in 1,4-dioxane (5 mL) was added aq. HCl (6 *N,* 0.5 mL) at room temperature. The reaction solution was stirred for 1 h at room temperature. The resulting solution was neutralized to pH 7 with saturated aq. NaHCO₃, and then concentrated under reduced pressure. The residue was purified by reverse phase chromatography, eluted with 60% ACN in water with 20 mmol/L NH₄HCO₃ to afford *tert*-butyl 4-((2-(allyloxy)-4,5-dichlorophenyl)(amino)methyl)piperidine-1-carboxylate as a brown oil (0.40 g, 90%): LCMS (ESI) calc'd for C₂₀H₂₈Cl₂N₂O₃ [M + H]⁺: 415, 417 (3 : 2), found 415, 417 (3 : 2).

### Step b:

To a stirred solution of *tert*-butyl 4-((2-(allyloxy)-4,5-dichlorophenyl)(amino)methyl)piperidine-1-carboxylate (0.11 g, 0.27 mmol) and Et₃N (54 mg, 0.53 mmol) in DCM (3 mL) was added TFAA (61 mg, 0.29 mmol) dropwise at 0 °C. The reaction solution was stirred at 0 °C for 0.5 h. The reaction solution was diluted with DCM (50 mL). The solution was washed with brine (2 x 20 mL), dried over anhydrous Na₂SO₄. After the filtration, the filtrate was concentrated under reduced pressure to afford *tert*-butyl 4-((2-(allyloxy)-4,5-dichlorophenyl)(2,2,2-trifluoroacetamido)methyl)piperidine-1-carboxylate as a yellow solid (0.13 g, 95%): LCMS (ESI) calc'd for C₂₂H₂₇Cl₂F₃N₂O₄ [M + H]⁺: 511, 513 (3 : 2), found 511, 513 (3 : 2); ¹H NMR (400 MHz, CDCl₃) δ 7.25 (s, 1H), 7.02 (s, 1H), 6.11-5.99 (m, 1H), 5.47-5.38 (m, 2H), 4.78 (t, *J =* 12.0 Hz, 1H), 4.86-4.57 (m, 2H), 4.31-3.93 (m, 2H), 2.75-2.48 (m, 2H), 2.01-1.91 (m, 1H), 1.83-1.72 (m, 1H), 1.46 (s, 9H), 1.49-1.22 (m, 3H).

### Step c:

To a solution of tert-butyl 4-((2-(allyloxy)-4,5-dichlorophenyl)(2,2,2-trifluoroacetamido)methyl)piperidine-1-carboxylate (0.13 g, 0.25 mmol) in DCM (3 mL) was added TFA (3 mL) dropwise at room temperature. The reaction solution was stirred at room temperature for 0.5 h. The reaction solution was concentrated under reduced pressure to afford N-((2-(allyloxy)-4,5-dichlorophenyl)(piperidin-4-yl)methyl)-2,2,2-trifluoroacetamide trifluoroacetic acid as a yellow solid (0.10 g, 90%): LCMS (ESI) calc'd for C₁₇H₁₉Cl₂F₃N₂O₂ [M + H]⁺: 411, 413 (3 : 2), found 411, 413 (3 : 2).

### Example 9. Intermediate 9 (1-(allyloxy)-2-bromo-3,4,5-trichlorobenzene)

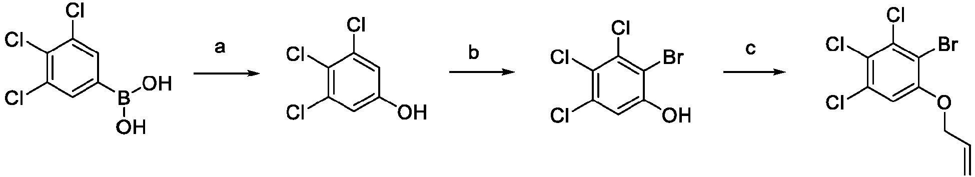

### Step a:

To a stirred solution of (3,4,5-trichlorophenyl)boronic acid (5.00 g, 22.20 mol) in THF (15 mL) were added H₂O₂ (1.51 g, 44.39 mmol, 30%) and NaOH (1.78 g, 44.39 mmol) at room temperature. The resulting mixture was stirred for 2 h at room temperature. The reaction was quenched with saturated aq. Na₂SO₃ (10 mL) at room temperature. The mixture was acidified to pH 3 with aq. HCl (1 N). The resulting mixture was extracted with EA (3 x 80 mL). The combined organic layers were washed with brine (2 x 80 mL) and dried over anhydrous Na₂SO₄. After filtration, the filtrate was concentrated under reduced pressure. The residue was purified by silica gel column chromatography, eluted with PE/EA (5/1) to afford 3,4,5-trichlorophenol as a light yellow solid (4.30 g, 95%): ¹H NMR (300 MHz, CDCl₃) δ 6.92 (s, 2H).

### Step b:

To a stirred solution of 3,4,5-trichlorophenol (4.60 g, 23.30 mol) in AcOH (20 mL) was added Br₂ (3.70 g, 23.15 mol) dropwise at room temperature under argon atmosphere. After stirring for 6 h, the reaction was quenched with saturated aq. Na₂SO₃ (80 mL) and extracted with EA (3 x 80 mL). The combined organic layers were washed with brine (3 x 80 mL) and dried over anhydrous Na₂SO₄. After filtration, the filtrate was concentrated under reduced pressure. The residue was silica gel column chromatography, eluted with PE/EA (20/1) to afford 2-bromo-3,4,5-trichlorophenol as an off-white solid (2.40 g, 37%): ¹H NMR (400 MHz, DMSO-*d*₆) δ 11.43 (s, 1H), 7.15 (s, 1H).

### Step c:

To a stirred solution of 2-bromo-3,4,5-trichlorophenol (2.40 g, 8.69 mmol) and K₂CO₃ (2.40 g, 17.37 mmol) in DMF (15 mL) was added allyl bromide (1.26 g, 10.42 mmol) at room temperature. The reaction was allowed to warm to 50 °C and stirred for 1 h. The reaction mixture was diluted with EA (80 mL) and water (80 mL), and then extracted with EA (3 x 80 mL). The combined organic layers were washed with brine (6 x 80 mL) and dried over anhydrous Na₂SO₄. After filtration, the filtrate was concentrated under reduced pressure. The residue was silica gel column chromatography, eluted with PE/EA (20/1) to afford 2-bromo-3,4,5-trichloro-1-(prop-2-en-1-yloxy)benzene as a light yellow oil (1.80 g, 66%).

### Example 10. Intermediate 10 ((S)-tert-butyl 4-(((tert-butylsulfinyl)imino)methyl)piperidine-1-carboxylate)

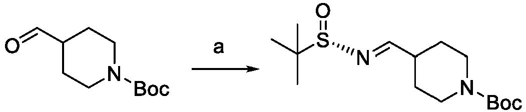

### Step a:

To a stirred solution of *tert*-butyl 4-formylpiperidine-1-carboxylate (50.00 g, 0.23 mol) and (*S*)-2-methylpropane-2-sulfinamide (43.00 g, 0.35 mmol) in THF (300 mL) was added Ti(OEt)₄ (187.00 g, 0.82 mol) dropwise at room temperature under nitrogen atmosphere. The reaction solution was stirred for 4 h at room temperature under nitrogen atmosphere. The resulting solution was quenched with saturated aq. NH₄Cl (200 mL) at room temperature and extracted with EA (3 x 300 mL). The combined organic layers were washed with brine (3 x 200 mL), dried over anhydrous Na₂SO₄. After filtration, the filtrate was concentrated under reduced pressure to afford (*S*)*-tert*-butyl 4-(((*tert*-butylsulfinyl)imino)methyl)piperidine-1-carboxylate as an off-white solid (67 g, 81%): LCMS (ESI) calc'd for C₁₅H₂₈N₂O₃S [M + H]⁺: 317, found 317; ¹H NMR (300 MHz, CDCl₃) δ 8.01 (d, *J =* 3.9 Hz, 1H), 4.09 (d, *J =* 13.4 Hz, 2H), 2.97-2.82 (m, 2H), 2.62 (m, 1H), 1.90 (dd, *J =* 13.4, 3.6 Hz, 2H), 1.71-1.43 (m, 2H), 1.47 (s, 9H), 1.20 (s, 9H).

### Example 11. Intermediate 11 (tert-butyl 4-(((tert-butylsulfinyl)imino)methyl)piperidine-1-carboxylate)

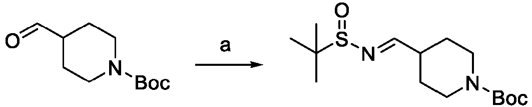

### Step a:

To a stirred solution of *tert*-butyl 4-formylpiperidine-1-carboxylate (20.00 g, 93.90 mmol) and 2-methylpropane-2-sulfinamide (17.00 g, 0.14 mol) in THF (300 mL) was added Ti(OEt)₄ (50.00 g, 0.18 mmol) dropwise at room temperature under nitrogen atmosphere. The reaction solution was stirred for 4 h at room temperature under nitrogen atmosphere. The resulting solution was quenched with saturated aq. NH₄Cl (200 mL) at room temperature and extracted with EA (3 x 500 mL). The combined organic layers were washed with brine (2 x 200 mL), dried over anhydrous Na₂SO₄. After filtration, the filtrate was concentrated under reduced pressure to afford *tert-*butyl 4-(((*tert*-butylsulfinyl)imino)methyl)piperidine-1-carboxylate as a light yellow solid (24.00 g, 80%): LCMS (ESI) calc'd for C₁₅H₂₈N₂O₃S [M + H]⁺: 317, found 317.

### Example 12. Intermediate 12 ((S)-N-((1-((R)-2,2-dimethyl-1,3-dioxolane-4-carbonyl)piperidin-4-yl)methylene)-2-methylpropane-2-sulfinamide)

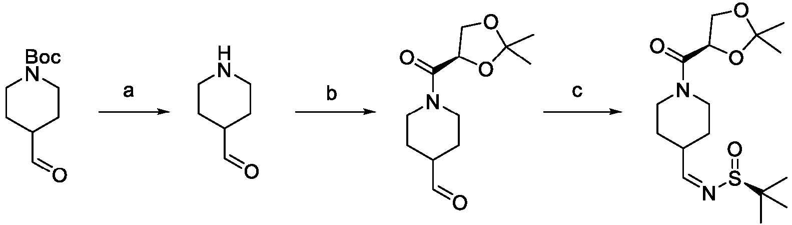

### Step a:

To a stirred solution tert-butyl 4-formylpiperidine-1-carboxylate (9.00 g, 42.20 mmol) in DCM (60 mL) was added TFA (30 mL) at room temperature. The resulting mixture was stirred for 1 h at room temperature. The reaction mixture was dried under reduced pressure. The crude product was used in the next step without further purification: LCMS (ESI) calc'd for C₆H₁₁NO [M + H]⁺: 114, found 114.

### Step b:

To a stirred solution of (4*R*)-2,2-dimethyl-1,3-dioxolane-4-carboxylic acid (8.37 g, 57.27 mmol) and HATU (21.77 g, 57.26 mmol) in DMF (150 mL) were added piperidine-4-carbaldehyde (5.40 g, 47.72 mmol) and Et₃N (72.43 g, 0.72 mol) at room temperature under nitrogen atmosphere. The resulting mixture was stirred for 2 h at room temperature under nitrogen atmosphere. The reaction was quenched with water (100 mL) at room temperature and extracted with EA (3 x 200 mL). The combined organic layers were washed with brine (3 x 100 mL), dried over anhydrous Na₂SO₄. After filtration, the filtrate was concentrated under reduced pressure. The crude product was used in the next step without further purification: LCMS (ESI) calc'd for C₁₂H₁₉NO₄ [M + H]⁺: 242, found 242.

### Step c:

To a stirred solution of 1-[(4*R*)-2,2-dimethyl-1,3-dioxolane-4-carbonyl]piperidine-4-carbaldehyde (4.30 g, 17.82 mmol) and (*S*)-2-methylpropane-2-sulfinamide (3.24 g, 26.73 mmol) in THF (40 mL) was added Ti(OEt)₄ (12.20 g, 53.48 mmol) at room temperature under nitrogen atmosphere. The resulting mixture was stirred for 4 h at room temperature under nitrogen atmosphere. The reaction was quenched with water (150 mL) at room temperature. The resulting mixture was extracted with EA (3 x 50 mL). The combined organic layers were washed with brine (3 x 30 mL), dried over anhydrous Na₂SO₄. After filtration, the filtrate was concentrated under reduced pressure. The residue was purified by silica gel column chromatography, eluted with PE/EA (1 : 3) to afford (*S*)-*N*-((1-((*R*)-2,2-dimethyl-1,3-dioxolane-4-carbonyl)piperidin-4-yl)methylene)-2-methylpropane-2-sulfinamide as a light yellow oil (4.37 g, 71%): LCMS (ESI) calc'd for C₁₆H₂₈N₂O₄S [M + H]⁺: 345, found 345. ¹H NMR (400 MHz, DMSO-*d*₆) δ 7.92 (d, *J =* 3.9 Hz, 1H), 4.91-4.77 (m, 1H), 4.36-4.12 (m, 2H), 4.11-3.94 (m, 1H), 3.16 (dt, *J =* 25.1, 12.4 Hz, 1H), 2.91-2.73 (m, 2H), 1.90 (d, *J =* 14.6 Hz, 2H), 1.56-1.40 (m, 1H), 1.39-1.25 (m, 8H), 1.11 (d, *J =* 1.3 Hz, 9H).

### Example 13. Intermediate 13 (2,2-dimethyl-1,3-dioxolane-4-carboxylic acid)

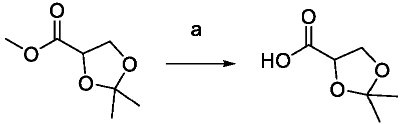

### Step a:

To a stirred mixture of KOH (2.10 g, 37.42 mmol) in H₂O (9 mL) and EtOH (18 mL) was added methyl 2,2-dimethyl-1,3-dioxolane-4-carboxylate (3.00 g, 18.73 mmol) in portions at room temperature. The reaction mixture was stirred for 2 h at room temperature. The mixture was acidified to pH 4 with 10% aq. H₃PO₄. The resulting mixture was extracted with EA (3 x 40 mL). The combined organic layers were washed with brine (2 x 20 mL), dried over anhydrous Na₂SO₄. After filtration, the filtrate was concentrated under reduced pressure to afford 2,2-dimethyl-1,3-dioxolane-4-carboxylic acid as a colorless oil (1.80 g, 65%): ¹H NMR (400 MHz, DMSO-*d*₆) δ 12.81 (s, 1H), 4.58-4.50 (m, 1H), 4.17 (t, *J =* 7.9 Hz, 1H), 4.05-3.89 (m, 1H), 1.37 (s, 3H), 1.30 (s, 3H).

### Example 14. Intermediate 14 ((R)-2,2-dimethyl-1,3-dioxolane-4-carboxylic acid)

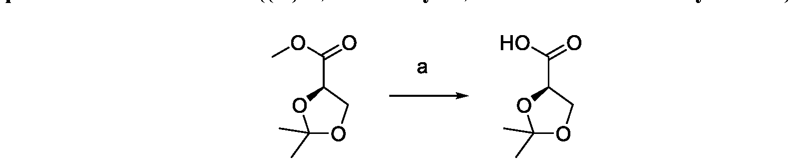

### Step a:

To a stirred solution of KOH (3.50 g, 62.38 mmol) in H₂O (5 mL) and EtOH (10 mL) was added methyl (4*R*)-2,2-dimethyl-1,3-dioxolane-4-carboxylate (5.00 g, 31.22 mmol) in portions at room temperature. The reaction mixture was stirred for additional 2 h at room temperature. The resulting mixture was diluted with H₂O (10 mL) and acidified to pH 3 with 10% aq. H₃PO₄. The resulting mixture was extracted with EA (3 x 60 mL). The combined organic layers were washed with brine (3 x 10 mL), dried over anhydrous Na₂SO₄. After filtration, the filtrate was concentrated under reduced pressure to afford (4*R*)-2,2-dimethyl-1,3-dioxolane-4-carboxylic acid as a light yellow oil (3.00 g, 66%): LCMS (ESI) calc'd for C₆H₁₀O₄ [M - H]⁺: 145, found 145; ¹H NMR (400 MHz, DMSO-*d*₆) δ 12.80 (br, 1H), 4.59-4.50 (m, 1H), 4.27-4.10 (m, 1H), 4.00-3.91 (m, 1H), 1.45 (s, 3H), 1.38 (s, 3H).

### Example 15. Intermediate 15 (1,2-dichloro-3-iodo-4-methoxybenzene)

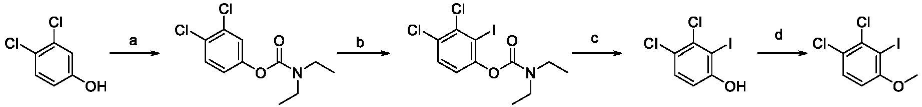

### Step a:

To a stirred solution of 3,4-dichlorophenol (50.00 g, 306.75 mmol), DMAP (74.95 g, 613.50 mmol) and Et₃N (62.08 g, 613.50 mmol) in DCM (500 mL) was added diethylcarbamoyl chloride (62.39 g, 460.12 mmol) dropwise at room temperature under nitrogen atmosphere. The reaction mixture was stirred for 2 h at room temperature under nitrogen atmosphere. The resulting mixture was diluted with water (300 mL) at room temperature and extracted with EA (3 x 500 mL). The combined organic layers were washed with brine (2 x 200 mL), dried over anhydrous Na₂SO₄. After filtration, the filtrate was concentrated under reduced pressure. The residue was purified by silica gel column chromatography, eluted with PE/EA (40/1) to afford 3,4-dichlorophenyl N,N-diethylcarbamate as a yellow oil (72.00 g, 80%): LCMS (ESI) calc'd for C₁₁H₁₃Cl₂NO₂ [M + H]⁺: 262, 264 (3 : 2), found 262, 264 (3 : 2); ¹H NMR (400 MHz, CDCl₃) δ 7.42 (d, *J =* 8.8 Hz, 1H), 7.30 (d, *J =* 2.7 Hz, 1H), 7.03 (dd, *J =* 8.8, 2.7 Hz, 1H), 3.42 (dq, *J =* 14.2, 7.2 Hz, 4H), 1.24 (dt, *J =* 14.8, 7.2 Hz, 6H).

### Step b:

To a solution of diethylpropyl amine (DIPA, 42.46 g, 419.64 mmol) in THF (400 mL) was added n-BuLi (29.32 g, 457.79 mmol, 2.5 M in hexane) dropwise in 0.5 h at -78 °C under nitrogen atmosphere. After stirring for 20 min at -78 °C, to resulting solution was added a solution of 3,4-dichlorophenyl *N,N*-diethylcarbamate (100.00 g, 381.49 mmol) in THF (100 mL) dropwise over 20 min at -78 °C. After addition, the resulting mixture was stirred for additional 0.5 h at -78 °C under nitrogen atmosphere. To the above mixture was added a solution of I₂ (101.67 g, 400.56 mmol) in THF (50 mL) dropwise over 0.5 h at -78 °C. The resulting mixture was stirred for additional 2 h at -78 °C. The resulting mixture was quenched with saturated aq. Na₂SO₃ (300 mL) at -78 °C and extracted with EA (3 x 500 mL). The combined organic layers were washed with brine (2 x 200 mL), dried over anhydrous Na₂SO₄. After filtration, the filtrate was concentrated under reduced pressure. The residue was purified by silica gel column chromatography, eluted with PE/EA (40/1) to afford 3,4-dichloro-2-iodophenyl *N,N-*diethylcarbamate as an off-white solid (117.00 g, 79%): LCMS (ESI) calc'd for C₁₁H₁₂Cl₂INO₂ [M + H]⁺: 388, 390 (3 : 2), found 388, 390 (3 : 2); ¹H NMR (400 MHz, CDCl₃) δ 7.48 (d, *J =* 8.8 Hz, 1H), 7.08 (d, *J =* 8.7 Hz, 1H), 3.55 (q, *J =* 7.1 Hz, 2H), 3.42 (q, *J =* 7.1 Hz, 2H), 1.35 (t, *J* = 7.1 Hz, 3H), 1.25 (t, *J =* 7.1 Hz, 3H).

### Step c:

To a stirred solution of 3,4-dichloro-2-iodophenyl *N,N*-diethylcarbamate (65.80 g, 169.58 mmol) in MeOH (100 mL) was added a solution of NaOH (67.82 g, 1695.75 mmol) in H₂O (200 mL) at 0 °C. The resulting mixture was allowed to warm to 50 °C and stirred for 10 h. The pH value of the solution was adjusted to 6~7 with aq. HCl (1 *N*). The reaction was diluted with water (400 mL) at room temperature and extracted with EA (3 x 400 mL). The combined organic layers were washed with brine (3 x 100 mL), dried over anhydrous Na₂SO₄. After filtration, the filtrate was concentrated under reduced pressure. The residue was purified by silica gel column chromatography, eluted with PE/EA (40/1) to afford 3,4-dichloro-2-iodophenol as a yellow oil (47.00 g, 96%): ¹H NMR (400 MHz, CDCl₃) δ 7.36 (d, *J =* 8.8 Hz, 1H), 6.90 (d, *J =* 8.8 Hz, 1H), 6.09 (s, 1H).

### Step d:

To a stirred solution of 3,4-dichloro-2-iodophenol (100.00 g, 346.15 mmol) in DMF (300 mL) were added CH₃I (73.70 g, 519.23 mmol) and K₂CO₃ (95.68 g, 692.31 mmol) at room temperature under nitrogen atmosphere. The resulting mixture was stirred for 5 h at room temperature under nitrogen atmosphere. The reaction was diluted with water (500 mL) at room temperature and extracted with EA (3 x 600 mL). The combined organic layers were washed with brine (3 x 1000 mL), dried over anhydrous Na₂SO₄. After filtration, the filtrate was concentrated under reduced pressure. The residue was purified by silica gel column chromatography, eluted with PE/EA (20/1) to afford 1,2-dichloro-3-iodo-4-methoxybenzene as an off-white solid (88.00 g, 84%): ¹H NMR (400 MHz, CDCl₃) δ 7.44 (d, *J =* 8.9 Hz, 1H), 6.69 (d, *J* = 8.8 Hz, 1H), 3.91 (s, 3H).

### Example 16. Intermediate 16 (1-(allyloxy)-3,4-dichloro-2-iodobenzene)

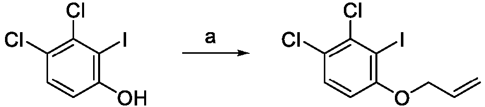

### Step a:

To a stirred solution of 3,4-dichloro-2-iodophenol (25.00 g, 86.54 mmol) and K₂CO₃ (35.88 g, 259.61 mmol) in DMF (100 mL) was added 3-bromoprop-1-ene (15.70 g, 129.81 mmol) dropwise at room temperature. The resulting mixture was allowed to warm to 40 °C and stirred for 4 h under nitrogen atmosphere. After cooling to room temperature, the resulting mixture was diluted with water (300 mL) at room temperature and extracted with EA (3 x 500 mL). The combined organic layers were washed with brine (3 x 500 mL), dried over anhydrous Na₂SO₄. After filtration, the filtrate was concentrated under reduced pressure. The residue was purified by silica gel column chromatography, eluted with PE/EA (5/1) to afford 1,2-dichloro-3-iodo-4-(prop-2-en-1-yloxy)benzene as a yellow solid (16.00 g, 50%): ¹H NMR (400 MHz, CD₃OD) δ 7.49 (d, *J =* 8.9 Hz, 1H), 6.88 (d, *J =* 8.9 Hz, 1H), 6.17-6.00 (m, 1H), 5.54 (dt, *J =* 17.3, 1.7 Hz, 1H), 5.31 (dt, *J =* 10.7, 1.7 Hz, 1H), 4.65 (dd, *J =* 4.0, 2.3 Hz, 2H).

### Example 17. Intermediate 17 (tert-butyl 4-(1-(4,5-dichloro-2-methoxyphenyl)vinyl)piperidine-1-carboxylate)

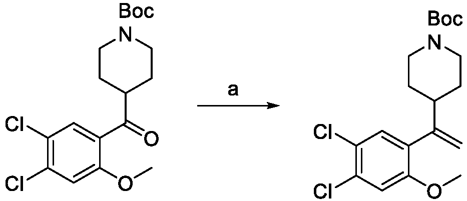

### Step a:

To a stirred solution of methyltriphenylphosphonium bromide (1.10 g, 3.09 mmol) in THF (15 mL) was added NaH (0.12 g, 3.09 mmol, 60% in the mineral oil) in portions at 0 °C under nitrogen atmosphere. The reaction mixture was allowed to warm to room temperature and stirred 15 min. Then the solution of *tert*-butyl 4-[(4,5-dichloro-2-methoxyphenyl)carbonyl]piperidine-1-carboxylate (0.80 g, 2.06 mmol) in THF (3 mL) was added. The reaction mixture was stirred at room temperature for additional 2 h under nitrogen atmosphere. The resulting mixture was quenched with saturated aq. NH₄Cl (40 mL) and extracted with EA (3 x 10 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (5/1) to afford *tert-*butyl 4-[1-(4,5-dichloro-2-methoxyphenyl)ethenyl]piperidine-1-carboxylate as a light yellow oil (0.50 g, 63%): LCMS (ESI) calc'd for C₁₉H₂₅Cl₂NO₃ [M + H]⁺: 386, 388 (3 : 2), found 386, 388 (3 : 2); ¹H NMR (400 MHz, CD₃OD) δ 7.18 (s, 1H), 7.14 (s, 1H), 5.18 (s, 1H), 4.99 (s, 1H), 4.17-4.07 (m, 2H), 3.82 (s, 3H), 2.83-2.70 (s, 2H), 2.64-2.52 (m, 1H), 1.81-1.70 (m, 2H), 1.50-1.37 (s, 9H), 1.35-1.21 (m, 2H).

### Example 18. Intermediate 18 ((S)-N-((R)-(3,4-dichloro-2-fluoro-6-hydroxyphenyl)(piperidin-4-yl)methyl)-2-methylpropane-2-sulfinamide)

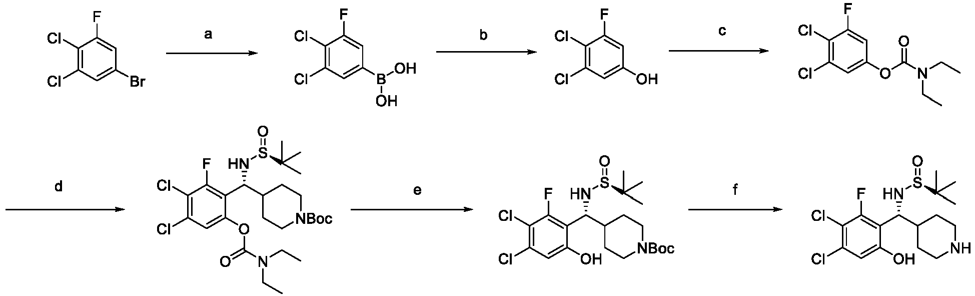

### Step a:

To a solution of 5-bromo-1,2-dichloro-3-fluorobenzene (4.00 g, 16.40 mmol) in THF (50 mL) was added *i*-PrMgCl·LiCl (25 mL, 32.80 mmol, 1.3 M in THF) dropwise for 30 min at -65 °C under nitrogen atmosphere. After stirring for additional 30 min, a solution of B(OMe)₃ (2.56 g, 24.60 mmol) in THF (10 mL) was added dropwise at -65 °C. After the addition, the reaction mixture was stirred for 30 min at -65 °C under nitrogen atmosphere. The resulting mixture was quenched with water (100 mL) at -65 °C and extracted with EA (2 x 300 mL). The combined organic layers were washed with brine (2 x 60 mL), dried over anhydrous Na₂SO₄. After filtration, the filtrate was concentrated under reduced pressure. The residue was purified by reverse phase chromatography, eluted with 50% ACN in water with 10 mmol/L NH₄HCO₃ to afford (3,4-dichloro-5-fluorophenyl)boronic acid as a yellow solid (0.36 g, 94%): LCMS (ESI) calc'd for C₆H₄BCl₂FO₂ [M - H]⁺: 207, 209 (3 : 2), found 207, 209 (3 : 2); ¹H NMR (400 MHz, CD₃OD) δ 7.66 (s, 1H), 7.47 (d, *J =* 9.2 Hz, 1H).

### Step b:

To a stirred mixture of (3,4-dichloro-5-fluorophenyl)boronic acid (3.60 g, 18.66 mmol) and KOH (4.19 g, 74.71 mmol, 4.00 equiv) in MeOH (20 mL) was added H₂O₂ (2.54 g, 74.71 mmol, 30% in water) dropwise at room temperature under air atmosphere. The reaction mixture was stirred for 2 h at room temperature under air atmosphere. The resulting mixture was quenched with saturated aq. NaHSO₃ (100 mL) at room temperature, and then a solution was acidified to pH 4 with citric acid. The resulting mixture was extracted with EA (2 x 400 mL). The combined organic layers were washed with brine (2 x 60 mL), dried over anhydrous Na₂SO₄. After filtration, the filtrate was concentrated under reduced pressure. The residue was purified by silica gel column chromatography, eluted with PE/EA (2/1) to afford 3,4-dichloro-5-fluorophenol as a yellow solid (2.65 g, 70%): ¹H NMR (400 MHz, CDCl₃) 6.83 (s, 1H), 6.64 (dd, *J =* 9.8, 2.8 Hz, 1H), 5.64 (s, 1H).

### Step c:

To a stirred solution of 3,4-dichloro-5-fluorophenol (2.60 g, 14.36 mmol), Et₃N (4.36 g, 43.09 mmol) and DMAP (3.51 g, 28.73 mmol) in DCM (20 mL) was added diethylcarbamoyl-chloride (2.33 g, 17.24 mmol) dropwise at room temperature under nitrogen atmosphere. After stirring for 2 h at room temperature under nitrogen atmosphere, the reaction solution was quenched with water (130 mL) at room temperature and extracted with EA (2 x 200 mL). The combined organic layers were washed with brine (2 x 50 mL), dried over anhydrous Na₂SO₄. After filtration, the filtrate was concentrated under reduced pressure. The residue was purified by silica gel column chromatography, eluted with PE/EA (6/1) to afford 3,4-dichloro-5-fluorophenyl N,N-diethylcarbamate as an off-white solid (3.20 g, 70%): LCMS (ESI) calc'd for C₁₁H₁₂Cl₂FNO₂ [M + H]⁺: 280, 282 (3 : 2), found 280, 282 (3 : 2); ¹H NMR (400 MHz, CDCl₃) δ 7.15 (s, 1H), 7.00 (dd, *J =* 9.3, 2.6 Hz, 1H), 3.49-3.32 (m, 4H), 1.31-1.15 (m, 6H).

### Step d:

To a solution of 3,4-dichloro-5-fluorophenyl *N,N*-diethylcarbamate (0.50 g, 1.79 mmol) in THF (10 mL) was added LDA (1.34 mL, 2.68 mmol, 2 M in THF) over 10 min at -65 °C under nitrogen atmosphere. After addition, the reaction solution was stirred for 30 min at -65 °C, and then a solution of (*S*)-*tert*-butyl 4-(((*tert*-butylsulfinyl)imino)methyl)piperidine-1-carboxylate (0.85 g, 2.68 mmol) in THF (5 mL) was added dropwise over 10 min at -65 °C under nitrogen atmosphere. After stirring for additional 1 h at -65 °C under nitrogen atmosphere, the resulting mixture was quenched with water (30 mL) at -65 °C and extracted with EA (2 x 70 mL). The combined organic layers were washed with brine (2 x 10 mL), dried over anhydrous Na₂SO₄. After filtration, the filtrate was concentrated under reduced pressure to afford crude *tert*-butyl 4-((*R*)-(3,4-dichloro-6-((diethylcarbamoyl)oxy)-2-fluorophenyl)((*S*)-1,1-dimethylethylsulfinamido)methyl)piperidine-1-carboxylate. The crude product was used in next step without further purification: LCMS (ESI) calc'd for C₂₆H₄₀Cl₂FN₃O₅S [M + H]⁺: 596, 598 (3 : 2), found 596, 598 (3 : 2).

### Step e:

To a solution of *tert*-butyl 4-((*R*)-(3,4-dichloro-6-((diethylcarbamoyl)oxy)-2-fluorophenyl)((*S*)-1,1-dimethylethylsulfinamido)methyl)piperidine-1-carboxylate (crude) in MeOH (20 mL) was added NaOH (0.45 g, 11.32 mmol) in portions at room temperature. The resulting mixture was allowed to warm to 50 °C and stirred for 3 h. After cooling to room temperature, the reaction mixture was diluted with water (20 mL) at room temperature and acidified to pH 4 with citric acid. The resulting solution was extracted with EA (2 x 200 mL). The combined organic layers were washed with brine (2 x 10 mL), dried over anhydrous Na₂SO₄. After filtration, the filtrate was concentrated under reduced pressure. The residue was purified by reverse phase chromatography, eluted with 60% ACN in water (plus 0.05% TFA) to afford *tert*-butyl 4-[(*S*)-(3,4-dichloro-2-fluoro-6-hydroxyphenyl)([[(*R*)-2-methylpropane-2-sulfinyl]amino])methyl]piperidine-1-carboxylate as a yellow solid (0.60 g, 68% overall two steps): LCMS (ESI) calc'd for C₂₁H₃₁Cl₂FN₂O₄S [M + H]⁺: 497, 499 (3 : 2), found 497, 499 (3 : 2); ¹H NMR (400 MHz, CDCl₃) δ 6.89 (d, *J =* 1.8 Hz, 1H), 4.59 (d, *J =* 8.2 Hz, 1H), 4.27-3.92 (m, 2H), 2.80-2.51 (m, 2H), 2.09-1.98 (m, 2H), 1.54-1.46 (m, 3H), 1.45 (s, 9H), 1.20 (s, 9H).

### Step f:

To a solution of *tert*-butyl 4-[(*S*)-(3,4-dichloro-2-fluoro-6-hydroxyphenyl)([[(*R*)-2-methylpropane-2-sulfinyl]amino])methyl]piperidine-1-carboxylate (crude) in DCM (10 mL) was added TFA (2 mL) dropwise at room temperature. The reaction solution was stirred for 1 h at room temperature. The resulting solution was adjusted to pH 8 with saturated aq. NaHCO₃ and extracted with EA (2 x 80 mL). The combined organic layers were washed with brine (2 x 50 mL), dried over anhydrous Na₂SO₄. After filtration, the filtrate was concentrated under reduced pressure to afford crude (*R*)-*N*-[(*S*)-(3,4-dichloro-2-fluoro-6-hydroxyphenyl)(piperidin-4-yl)methyl]-2-methylpropane-2-sulfinamide as a light yellow oil (0.40 g, 83%). The crude product was used in the next step directly without further purification: LCMS (ESI) calc'd for C₁₆H₂₃Cl₂FN₂O₂S [M + H]⁺: 397, 399 (3 : 2), found 397, 399 (3 : 2); ¹H NMR (400 MHz, CD₃OD) δ 6.76 (s, 1H), 4.28 (d, *J =* 7.6 Hz, 1H), 3.52-3.40 (m, 1H), 3.30-3.20 (m, 1H), 3.03-2.80 (m, 2H), 2.55-2.40 (m, 1H), 2.38-2.22 (m, 1H), 1.59-1.37 (m, 3H), 1.10 (s, 9H).

### Example 19. Intermediate 19 (lithio (2R)-1-methyl-S-oxopyrrolidine-2-carboxylate)

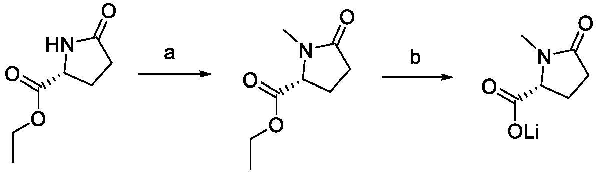

### Step a:

To a stirred solution of ethyl (2R)-5-oxopyrrolidine-2-carboxylate (0.50 g, 3.18 mmol) in THF (4 mL) was added NaH (0.25 g, 6.36 mmol, 60% in mineral oil) at 0 °C under nitrogen atmosphere. The resulting mixture was stirred for 10 min at 0 °C. Then MeI (0.90 g, 6.36 mmol) was added into the mixture. The resulting mixture was stirred for 1 h at 0 °C under nitrogen atmosphere. The reaction was quenched with water (3 mL) at 0 °C. The resulting mixture was diluted with DCM (30 mL) and water (30 mL), and then the aqueous layer was extracted with DCM (3 x 30 mL). The combined organic layers were washed with brine (3 x 20 mL), dried over anhydrous Na₂SO₄. After filtration, the filtrate was concentrated under reduced pressure to afford ethyl (2R)-1-methyl-5-oxopyrrolidine-2-carboxylate as a light yellow oil (0.22 g, 40%): LCMS (ESI) calc'd for C₈H₁₃NO₃ [M + H]⁺: 172 found 172; ¹H NMR (400 MHz, CDCl₃) δ 4.26 (q, *J =* 8.6, 7.9 Hz, 2H), 4.18-4.08 (m, 1H), 2.89 (s, 3H), 2.58-2.29 (m, 3H), 2.16-2.05 (m, 1H), 1.44-1.30 (m, 3H).

### Step b:

To a stirred solution of ethyl (2*R*)-1-methyl-5-oxopyrrolidine-2-carboxylate (0.15 g, 0.95 mmol) in MeOH (2 mL) was added a solution of LiOH·H₂O (48 mg, 1.15 mmol) in H₂O (2 mL) at room temperature. The reaction mixture was stirred at room temperature for 16 h. The resulting solution was concentrated under reduced pressure to afford lithio (2R)-1-methyl-5-oxopyrrolidine-2-carboxylate as an off-white solid (0.15 g, crude), which was used in next step directly without further purification: LCMS (ESI) calc'd for C₆H₉NO₃ [M + H]⁺: 144, found 144.

### Example 20. (which is a reference example) Intermediate 20 (1-bromo-5-chloro-4-cyclobutyl-2-methoxybenzene)

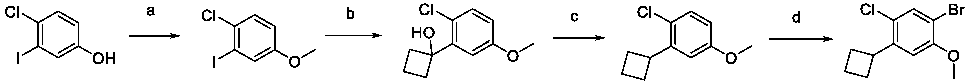

### Step a:

To a stirred mixture of 4-chloro-3-iodophenol (3.00 g, 11.79 mmol) and K₂CO₃ (3.26 g, 23.59 mmol) in DMF (30 mL) was added CH₃I (2.51 g, 17.69 mmol) dropwise at room temperature. The resulting mixture was stirred for 1 h at room temperature. The reaction mixture was diluted with EA (50 mL) and water (100 mL). The aqueous solution was extracted with EA (3 x 50 mL). The combined organic layers were washed with brine (6 x 30 mL) and dried over anhydrous Na₂SO₄. After filtration, the filtrate was concentrated under reduced pressure. The residue was purified by silica gel column chromatography, eluted with PE/EA (20/1) to afford 1-chloro-2-iodo-4-methylbenzene as a colorless oil (2.90 g, 92%): ¹H NMR (400 MHz, CD₃OD) δ 7.47-7.41 (m, 1H), 7.41-7.33 (m, 1H), 6.99-6.90 (m, 1H), 3.78 (s, 3H).

### Step b:

To a stirred solution of 1-chloro-2-iodo-4-methoxybenzene (1.00 g, 3.73 mmol) in THF (5 mL) was added n-BuLi (2.2 mL, 5.59 mmol, 2.5 M solution in hexane) dropwise at -78 °C under nitrogen atmosphere. The solution was stirred for 30 min at -78 °C under nitrogen atmosphere. Then cyclobutanone (0.39 g, 5.59 mmol) was added into the solution. The resulting solution was stirred for 1 h at -78 °C under nitrogen atmosphere. The reaction was quenched with saturated aq. NH₄Cl (3 mL). The resulting mixture was diluted with EA (30 mL) and water (30 mL), and then the aqueous solution was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (3/1) to afford 1-(2-chloro-5-methoxyphenyl)cyclobutan-1-ol as a light yellow oil (0.56 g, 71%): ¹H NMR (400 MHz, CD₃OD) δ 7.28 (dd, *J =* 8.7, 1.5 Hz, 1H), 6.96 (d, *J =* 3.0 Hz, 1H), 6.83 (dd, *J =* 8.7, 2.2 Hz, 1H), 3.81 (s, 3H), 2.75-2.63 (m, 2H), 2.50-2.37 (m, 2H), 2.23-2.12 (m, 1H), 1.78-1.66 (m, 1H).

### Step c:

To a stirred solution of 1-(2-chloro-5-methoxyphenyl)cyclobutan-1-ol (0.56 g, 2.63 mmol) and Et₃SiH (0.61 mg, 5.27 mmol) in DCM (3 mL) was added BF₃·Et₂O (0.75 g, 5.27 mmol) at room temperature under nitrogen atmosphere. The solution was stirred at room temperature for 1 h under nitrogen atmosphere. The reaction was quenched with water (5 mL) and concentrated under reduced pressure. The residue was purified by silica gel column chromatography, eluted with PE/EA (20/1) to afford 1-chloro-2-cyclobutyl-4-methoxybenzene as a colorless oil (0.49 g, 95%): ¹H NMR (400 MHz, CD₃OD) δ 7.21 (d, *J* = 8.7 Hz, 1H), 6.90 (d, *J =* 3.0 Hz, 1H), 6.74 (dd, *J =* 8.7, 3.0 Hz, 1H), 3.81 (s, 3H), 3.79-3.72 (m, 1H), 2.47-2.33 (m, 2H), 2.19-1.97 (m, 3H), 1.92-1.81 (m, 1H).

### Step d:

To a stirred solution of 1-chloro-2-cyclobutyl-4-methoxybenzene (0.48 g, 2.44 mmol) in HOAc (5 mL) was added Br₂ (0.43 g, 2.69 mmol) dropwise at room temperature. The solution was stirred at room temperature for 1 h. The reaction was quenched with saturated aq. Na₂SO₃ (2 mL), and diluted with EA (30 mL) and water (30 mL). The aqueous solution was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (20/1) to afford 1-bromo-5-chloro-4-cyclobutyl-2-methoxybenzene as a colorless liquid (0.60 g, 72%): ¹H NMR (400 MHz, CD₃OD) δ 7.48 (s, 1H), 6.98 (s, 1H), 3.92 (s, 3H), 3.83-3.71 (m, 1H), 2.51-2.34 (m, 2H), 2.24-2.02 (m, 3H), 1.94-1.80 (m, 1H).

### Example 21. (which is a reference example) Intermediate 21 (1-bromo-5-chloro-4-fluoro-2-(prop-2-en-1-yloxy)benzene)

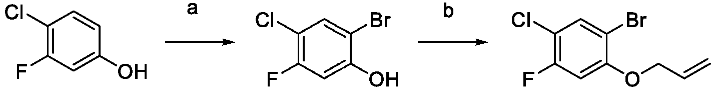

### Step a:

To a stirred solution of 4-chloro-3-fluorophenol (2.00 g, 13.65 mmol) in DCM (15 mL) was added Br₂ (2.62 g, 16.40 mmol) at room temperature. The resulting solution was stirred for 1 h at room temperature. The reaction was quenched with saturated aq. Na₂SO₃ (30 mL) at room temperature. The aqueous layers were extracted with EA (3 x 50 mL). The combined organic layers were washed with brine (3 x 20 mL) and dried over anhydrous Na₂SO₄. After filtration, the filtrate was concentrated under reduced pressure to afford 2-bromo-4-chloro-5-fluorophenol as a light yellow oil (3.40 g, crude), which was used in the next step directly without further purification: ¹H NMR (400 MHz, CD₃OD) δ 7.58 (d, *J* = 7.9 Hz, 1H), 6.79 (d, *J* = 10.5 Hz, 1H).

### Step b:

To a stirred mixture of 2-bromo-4-chloro-5-fluorophenol (3.40 g, 15.08 mmol) and K₂CO₃ (4.17 g, 30.17 mmol) in DMF (30 mL) was added 3-bromoprop-1-ene (2.74 g, 22.65 mmol) dropwise at room temperature. The resulting mixture was stirred for 1 h at room temperature. The reaction was diluted with EA (50 mL) and water (50 mL). The aqueous solution was extracted with EA (3 x 30 mL). The combined organic layers were washed with brine (6 x 20 mL), dried over anhydrous Na₂SO₄. After filtration, the filtrate was concentrated under reduced pressure. The residue was purified by silica gel column chromatography, eluted with PE/EA (20/1) to afford 1-bromo-5-chloro-4-fluoro-2-(prop-2-en-1-yloxy)benzene as a colorless oil (2.40 g, 60%): ¹H NMR (400 MHz, CD₃OD) δ 7.68 (d, *J =* 7.9 Hz, 1H), 7.05 (d, *J* = 11.0 Hz, 1H), 6.14-6.02 (m, 1H), 5.50 (d, *J =* 17.2 Hz, 1H), 5.33 (d, *J =* 10.6 Hz, 1H), 4.65 (d*, J* = 4.3 Hz, 2H).

### Example 22. Intermediate 22 (tert-butyl 8-[methoxy(methyl)carbamoyl]-3-azabicyclo[3.2.1]octane-3-carboxylate)

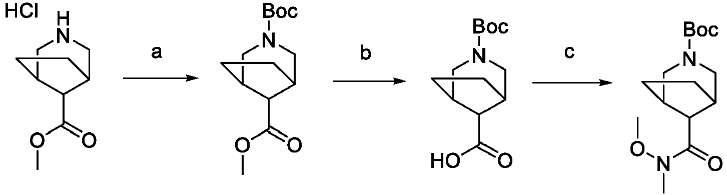

### Step a:

To a stirred solution of methyl 3-azabicyclo[*3.2.1*]octane-8-carboxylate hydrochloride (0.50 g, 2.43 mmol) and Et₃N (0.98 g, 9.72 mmol) in DCM (6 mL) was added Boc₂O (0.80 g, 3.65 mmol) at room temperature. The reaction was stirred at room temperature for 1 h. The reaction was diluted with EA (20 mL) and water (30 mL). The aqueous solution was extracted with EA (3 x 20 mL). The combined organic layers were washed with brine (2 x 20 mL) and dried over anhydrous Na₂SO₄. After filtration, the filtrate was evaporated under reduced pressure to afford 3-tert-butyl 8-methyl 3-azabicyclo[*3.2.1*]octane-3,8-dicarboxylate as a yellow oil (0.65 g, crude), which was used in the next step directly without further purification: LCMS (ESI) calc'd for C₁₄H₂₃NO₄ [M + H - 15]⁺: 255, found 255; ¹H NMR (400 MHz, CDCl₃) δ 3.90 (d, *J =* 60.4 Hz, 1H), 3.79-3.60 (m, 4H), 3.17 (d, *J =* 41.4 Hz, 1H), 2.97-2.77 (m, 1H), 2.69-2.41 (m, 3H), 1.85-1.61 (m, 4H), 1.47 (d, *J =* 3.7 Hz, 9H).

### Step b:

To a stirred solution of 3-*tert*-butyl 8-methyl 3-azabicyclo[*3.2.1*]octane-3,8-dicarboxylate (0.65 g, 2.41 mmol) in MeOH (5 mL) was added a solution of NaOH (0.19 g, 4.83 mmol) in water (2 mL) at room temperature. The reaction was stirred at 40 °C for 1 h. After cooling to room temperature, the resulting solution was diluted with EA (20 mL) and water (30 mL). The aqueous solution was acidified with citric acid to pH 3, and then extracted with EA (3 x 30 mL). The combined organic layers were washed with brine (2 x 20 mL) and dried over anhydrous Na₂SO₄. After filtration, the filtrate was evaporated under reduced pressure to afford 3-(tert-butoxycarbonyl)-3-azabicyclo[*3.2.1*]octane-8-carboxylic acid as a yellow oil (0.70 g, crude), which was used in the next step directly without further purification: LCMS (ESI) calc'd for C₁₃H₂₁NO₄ [M + H -56]⁺: 200, found 200; ¹H NMR (400 MHz, CD₃OD) δ 3.88 (d, *J* = 12.5 Hz, 1H), 3.68 (d, *J =* 13.1 Hz, 1H), 3.32-3.13 (m, 1H), 3.05-2.75 (m, 3H), 2.73-2.36 (m, 2H), 1.89-1.74 (m, 2H), 1.64-1.55 (m, 1H), 1.47 (s, 9H).

### Step c:

To a stirred solution of 3-(*tert-*butoxycarbonyl)-3-azabicyclo[3.2.1]octane-8-carboxylic acid (0.70 g, 2.74 mmol), HOBt (0.56 g, 4.11 mmol) and EDCI (0.79 g, 4.11 mmol) in DMF (6 mL) were added *N*,*O*-dimethylhydroxylamine hydrochloride (0.40 g, 4.11 mmol) and Et₃N (0.55 g, 5.48 mmol) at room temperature. The reaction was stirred at room temperature for 16 h. The reaction was diluted with EA (30 mL) and water (30 mL). The aqueous solution was extracted with EA (3 x 30 mL). The combined organic layers were washed with brine (6 x 10 mL) and dried over anhydrous Na₂SO₄. After filtration, the filtrate was evaporated under reduced pressure. The residue was purified by silica gel column chromatography, eluted with PE/EA (2/1) to afford *tert-*butyl 8-[methoxy(methyl)carbamoyl]-3-azabicyclo[*3.2.1*]octane-3-carboxylate as a light yellow oil (0.43 g, 53%): LCMS (ESI) calc'd for C₁₅H₂₆N₂O₄ [M + H - 56]⁺: 243, found 243; ¹H NMR (400 MHz, CDCl₃) δ 3.91 (d, *J =* 47.8 Hz, 1H), 3.73 (d, *J =* 10.9 Hz, 3H), 3.69-3.39 (m, 2H), 3.21 (d, *J* = 12.8 Hz, 3H), 3.03-2.84 (m, 1H), 2.74 (d, *J* = 17.2 Hz, 1H), 2.55-2.33 (m, 3H), 2.03-1.88 (m, 1H), 1.84-1.57 (m, 2H), 1.47 (d, *J =* 7.2 Hz, 9H).

### Example 23. (which is a reference example) Intermediate 23 ((S)-N-[(R)-(4-bromo-5-chloro-2-methoxyphenyl)([1-[(4R)-2,2-dimethyl-1,3-dioxolane-4-carbonyl]piperidin-4-yl])methyl]-2-methylpropane-2-sulfinamide)

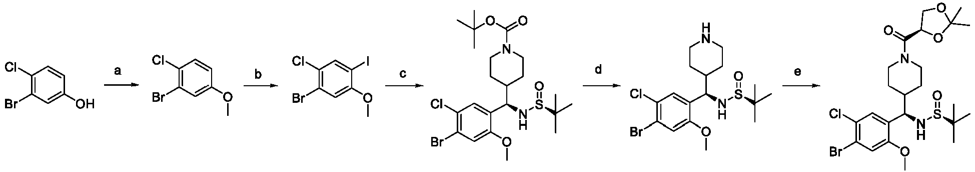

### Step a:

To a stirred mixture of 3-bromo-4-chlorophenol (5.00 g, 24.10 mmol) and K₂CO₃ (9.99 g, 72.31 mmol) in THF (50 mL) was added MeI (10.26 g, 72.31 mmol) dropwise at 40 °C. The reaction mixture was stirred for 16 h at 40 °C. The resulting mixture was diluted with water (50 mL) and extracted with EA (3 x 100 mL). The combined organic layers were washed with brine (2 x 100 mL), dried over anhydrous Na₂SO₄. After filtration, the filtrate was concentrated under reduced pressure. The residue was purified by silica gel column chromatography, eluted with PE to afford 2-bromo-1-chloro-4-methoxybenzene as a colorless oil (4.50 g, 84%): ¹H NMR (400 MHz, CD₃OD) δ 7.41 (d, *J =* 8.9 Hz, 1H), 7.26 (d, *J =* 2.9 Hz, 1H), 6.93 (dd, *J =* 8.9, 2.9 Hz, 1H), 3.81 (s, 3H).

### Step b:

To a stirred solution of 2-bromo-1-chloro-4-methoxybenzene (2.00 g, 9.03 mmol) in DCM (20 mL) was added AgOTf (2.55 g, 9.93 mmol) at room temperature. After addition, I₂ (2.52 g, 9.93 mmol) was added to the reaction and the mixture was stirred for 3 h at room temperature. The reaction was quenched with saturated aq. Na₂SO₃ (50 mL) at room temperature and extracted with EA (3 x 60 mL). The combined organic layers were washed with brine (3 x 50 mL), dried over anhydrous Na₂SO₄. After filtration, the filtrate was concentrated under reduced pressure. The residue was purified by silica gel column chromatography, eluted with PE to afford 1-bromo-2-chloro-4-iodo-5-methoxybenzene as an off-white solid (2.50 g, 80%): ¹H NMR (400 MHz, CDCl₃) δ 7.84 (s, 1H), 7.04 (s, 1H), 3.89 (s, 3H).

### Step c:

To a solution of 1-bromo-2-chloro-4-iodo-5-methoxybenzene (10.00 g, 28.79 mmol) in THF (20 mL) was dropwise added *n*-BuLi (11.5 mL, 28.75 mmol, 2.5 M in hexane) at -90 °C over 10 min under nitrogen atmosphere. The solution was stirred for 30 min at same temperature. The solution of *tert*-butyl 4-[[(*S*)-2-methylpropane-2-sulfinyl]imino]methyl]piperidine-1-carboxylate (9.10 g, 28.79 mmol) in THF (10 mL) was quickly added to above solution at -90 °C (the reaction temperature was increased from -90 to - 70 °C). After the addition, the reaction was stirred for additional 1.5 h at -75 °C. The reaction was quenched by saturated aq. NH₄Cl (20 mL), and then diluted with water (100 mL). The aqueous phase was extracted with EA (3 x 100 mL). The combined organic layers were washed with brine (2 x 50 mL), dried over anhydrous Na₂SO₄ and filtered. The filtrate was concentrated under reduced pressure. The residue was purified by reverse phase chromatography, eluted with 60% ACN in water with 10 mmol/L NH₄HCO₄ to afford *tert-*butyl 4-[(*R*)-(4-bromo-5-chloro-2-methoxyphenyl)([[(*S*)-2-methylpropane-2-sulfinyl]amino])methyl]piperidine-1-carboxylate as an off-white solid (12.40 g, 80%): LCMS (ESI) calc'd for C₂₂H₃₄BrClN₂O₄S [M + H]⁺: 537, 539 (2 : 3 : 1), found 537, 539 (2 : 3 : 1); ¹H NMR (400 MHz, CD₃OD) δ 7.43 (s, 1H), 7.30 (s, 1H), 4.44 (d, *J =* 8.9 Hz, 1H), 4.16 (d, *J =* 13.4 Hz, 1H), 4.01 (d, *J =* 13.1 Hz, 1H), 3.85 (s, 3H), 2.84-2.57 (m, 2H), 2.17-2.05 (m, 1H), 2.02-1.84 (m, 1H), 1.545 (s, 9H), 1.30-1.08 (m, 3H), 1.13 (s, 9H).

### Step d:

To a stirred solution of *tert*-butyl 4-[(*R*)-(4-bromo-5-chloro-2-methoxyphenyl)([[(*S*)-2-methylpropane-2-sulfinyl]amino])methyl]piperidine-1-carboxylate (6.00 g, 11.19 mmol) in DCM (60 mL) was added TFA (10 mL) dropwise at room temperature. The resulting mixture was stirred for 30 min at room temperature. The reaction was neutralized with saturated aq. NaHCO₃ (50 mL) at room temperature. The aqueous layers were extracted with DCM (3 x 200 mL). The combined organic layers were concentrated under reduced pressure to afford (*S*)*-N-*[(*R*)-(4-bromo-5-chloro-2-methoxyphenyl)(piperidin-4-yl)methyl]-2-methylpropane-2-sulfinamide as a yellow oil (4.60 g, crude). The crude product was used in the next step directly without further purification: LCMS (ESI) calc'd for C₁₇H₂₆BrClN₂O₂S [M + H]⁺: 437, 439 (2 : 3: 1), found 437, 439 (2 : 3: 1); ¹H NMR (400 MHz, CD₃OD) δ 7.45 (s, 1H), 7.35 (s, 1H), 4.50 (d, *J* = 9.1 Hz, 1H), 3.90 (s, 3H), 3.47 (d, *J =* 12.8 Hz, 1H), 3.30-3.25 (m, 1H), 3.01-2.80 (m, 2H), 2.42-2.33 (m, 1H), 2.19-2.06 (m, 1H), 1.57-1.28 (m, 3H), 1.14 (s, 9H).

### Step e:

To a stirred solution of (4R)-2,2-dimethyl-1,3-dioxolane-4-carboxylic acid (1.70 g, 11.63 mmol) and HATU (4.43 g, 11.65 mmol) in DMF (10 mL) were added (*S*)*-N-*[(*R*)-(4-bromo-5-chloro-2-methoxyphenyl)(piperidin-4-yl)methyl]-2-methylpropane-2-sulfinamide (3.40 g, 7.76 mmol) and Et₃N (2.36 g, 23.29 mmol) at room temperature. The resulting solution was stirred for 1 h at room temperature. The resulting solution was quenched with water (60 mL) and extracted with EA (3 x 100 mL). The combined organic layers were washed with brine (2 x 30 mL), dried over anhydrous Na₂SO₄. After filtration, the filtrate was concentrated under reduced pressure. The residue was purified by reverse phase chromatography, eluted with 70% ACN in water with 10 mmol/L NH₄HCO₃ to afford (*S*)-*N*-[(*R*)-(4-bromo-5-chloro-2-methoxyphenyl)([1-[(4*R*)-2,2-dimethyl-1,3-dioxolane-4-carbonyl]piperidin-4-yl])methyl]-2-methylpropane-2-sulfinamide as an off-white solid (3.80 g, 86%): LCMS (ESI) calc'd for C₂₃H₃₄BrClN₂O₅S [M + H]⁺: 565, 567 (2 : 3: 1), found 565, 567 (2 : 3: 1); ¹H NMR (400 MHz, CD₃OD) δ 7.43 (d, *J =* 2.7 Hz, 1H), 7.31 (d, *J =* 1.8 Hz, 1H), 5.48-5.32 (dd, *J =* 33.5, 7.5 Hz, 1H), 4.59-4.37 (m, 2H), 4.20-4.10 (m, 1H), 4.32-4.18 (m, 2H), 3.88 (d, *J =* 2.2 Hz, 3H), 3.20-2.90 (m, 1H), 2.73-2.56 (m, 1H), 2.25-2.06 (m, 2H), 1.48-1.33 (m, 8H), 1.17-1.09 (m, 10H).

### Example 24. (which is a reference example) Intermediate 24 ((S)-N-[(R)-[4-bromo-5-chloro-2-(prop-2-en-1-yloxy)phenyl]([1-[(4R)-2,2-dimethyl-1,3-dioxolane-4-carbonyl]piperidin-4-yl])methyl]-2-methylpropane-2-sulfinamide)

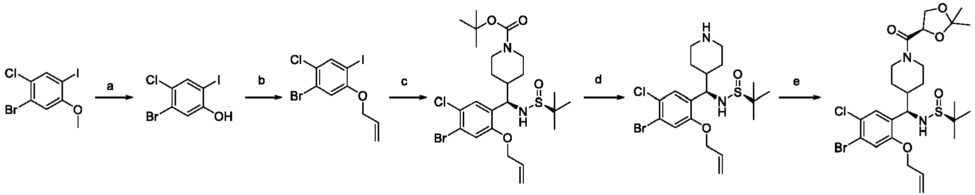

### Step a:

To a stirred solution of 1-bromo-2-chloro-4-iodo-5-methoxybenzene (47.00 g, 135.30 mmol) in DCM (470 mL) was added BBr₃ (101.00 g, 405.90 mmol) dropwise at 0 °C under nitrogen atmosphere. The reaction solution was stirred for 20 h at room temperature under nitrogen atmosphere. The reaction was quenched with water (500 mL) at 0 °C and extracted with EA (3 x 500 mL). The combined organic layers were washed with brine (3 x 500 mL), dried over anhydrous Na₂SO₄. After filtration, the filtrate was concentrated under reduced pressure to afford 5-bromo-4-chloro-2-iodophenol as a light yellow solid (45.50 g, 95%), which was used in next step without further purification.

### Step b:

To a stirred solution of 5-bromo-4-chloro-2-iodophenol (45.20 g, 141.59 mmol) in DMF (100 mL) were added K₂CO₃ (39.14 g, 283.18 mmol) and allyl bromide (29.12 g, 240.70 mmol) dropwise at room temperature under nitrogen atmosphere. The resulting mixture was stirred for 1 h at room temperature under nitrogen atmosphere. The resulting mixture was diluted with water (200 mL). The resulting mixture was extracted with EA (3 x 200 mL). The combined organic layers were washed with brine (3 x 100 mL), dried over anhydrous Na₂SO₄. After filtration, the filtrate was concentrated under reduced pressure. The residue was purified by silica gel column chromatography, eluted with PE to afford 1-bromo-2-chloro-4-iodo-5-(prop-2-en-1-yloxy)benzene as a colorless oil (28.50 g, 54%): ¹H NMR (400 MHz, CDCl₃) δ 7.85 (d, *J =* 1.0 Hz, 1H), 7.02 (d, *J =* 0.9 Hz, 1H), 6.12-5.98 (m, 1H), 5.53 (dq, *J =* 17.2, 1.6 Hz, 1H), 5.37 (dq, *J =* 10.7, 1.4 Hz, 1H), 4.59 (dq, *J =* 4.6, 1.5 Hz, 2H).

### Step c:

To a stirred solution of 1-bromo-2-chloro-4-iodo-5-(prop-2-en-1-yloxy)benzene (10.00 g, 26.78 mmol) in THF (250 mL) was added n-BuLi (10.71 mL, 26.78 mmol, 2.5 M in hexane) dropwise to -100 °C under nitrogen atmosphere. After stirring for 30 min at -100 °C under nitrogen atmosphere, a solution of *tert*-butyl 4-[[(*S*)-2-methylpropane-2-sulfinyl]imino]methyl]piperidine-1-carboxylate (8.47 g, 26.7 mmol) in THF (40 mL) was added dropwise to the above mixture at -100 °C. The resulting mixture was stirred for additional 1 h at -100 °C. The reaction was quenched with saturated aq. NH₄Cl (200 mL) at -100 °C. The aqueous layer was extracted with EA (3 x 200 mL). The combined organic layers were concentrated under reduced pressure. The residue was purified by reverse phase chromatography, eluted with 70% ACN in water (plus 0.05% TFA) to afford *tert*-butyl 4-[(*R*)-[4-bromo-5-chloro-2-(prop-2-en-1-yloxy)phenyl]([[(S)-2-methylpropane-2-sulfinyl]amino])methyl]piperidine-1-carboxylate as a light yellow oil (10.00 g, 60%): LCMS (ESI) calc'd for C₂₄H₃₆BrClN₂O₄S [M + H]⁺: 563, 565 (2 : 3 : 1), found 563, 565 (2 : 3 : 1); ¹H NMR (400 MHz, CDCl₃) δ 7.22 (s, 1H), 7.12 (s, 1H), 6.11-5.96 (m, 1H), 5.48-5.30 (m, 2H), 4.61-4.55 (m, 2H), 4.46 (s, 1H), 4.24-4.02 (m, 2H), 2.74-2.47 (m, 2H), 2.03-1.77 (m, 2H), 1.46 (s, 9H), 1.40-1.11 (m, 3H), 1.18 (s, 9H).

### Step d:

To a stirred solution of *tert*-butyl 4-[(*R*)-[4-bromo-5-chloro-2-(prop-2-en-1-yloxy)phenyl]([[(*S*)-2-methylpropane-2-sulfinyl]amino])methyl]piperidine-1-carboxylate (5.60 g, 9.96 mol) in DCM (50 mL) was added TFA (10 mL) dropwise at room temperature. The reaction solution was stirred for 0.5 h at room temperature. The reaction was neutralized with saturated aq. NaHCO₃ at room temperature. The aqueous layer was extracted with EA (3 x 100 mL). The combined organic layers were concentrated under reduced pressure to afford (S)-N-[(R)-[4-bromo-5-chloro-2-(prop-2-en-1-yloxy)phenyl](piperidin-4-yl)methyl]-2-methylpropane-2-sulfinamide as a light yellow semi-solid (5.30 g, crude). The crude product was used in the next step directly without further purification: LCMS (ESI) calc'd for C₁₉H₂₈BrClN₂O₂S [M + H]⁺: 463, 465 (2 : 3 : 1), found 463, 465 (2 : 3: 1); ¹H NMR (400 MHz, CDCl₃) δ 7.25 (s, 1H), 7.13 (s, 1H), 6.08-5.98 (m, 1H), 5.46-5.34 (m, 2H), 4.62-4.51 (m, 2H), 4.45-4.36 (s, 1H), 4.25-4.18 (m, 1H), 3.49-3.34 (dd, *J =* 32.3, 12.9 Hz, 2H), 2.90-2.73 (m, 2H), 2.26-1.97 (m, 2H), 1.80-1.52 (m, 3H), 1.18 - 1.09 (s, 9H).

### Step e:

To a stirred solution of (4*R*)-2,2-dimethyl-1,3-dioxolane-4-carboxylic acid (2.50 g, 17.13 mmol) and HATU (6.52 g, 17.13 mmol) in DMF (30 mL) were added (*S*)*-N-*[(*R*)-[4-bromo-5-chloro-2-(prop-2-en-1-yloxy)phenyl](piperidin-4-yl)methyl]-2-methylpropane-2-sulfinamide (5.30 g, 11.42 mmol) and Et₃N (3.47 g, 34.27 mmol) at room temperature. The reaction solution was stirred for 1 h at room temperature. The resulting solution was quenched with water (60 mL) and extracted with EA (3 x 100 mL). The combined organic layers were washed with brine (2 x 30 mL), dried over anhydrous Na₂SO₄. After filtration, the filtrate was concentrated under reduced pressure. The residue was purified by reverse phase chromatography, eluted with 70% ACN in water with 10 mmol/L NH₄HCO₃ to afford (*S*)*-N-*[(*R*)-[4-bromo-5-chloro-2-(prop-2-en-1-yloxy)phenyl]([1-[(4*R*)-2,2-dimethyl-1,3-dioxolane-4-carbonyl]piperidin-4-yl])methyl]-2-methylpropane-2-sulfinamide as an off-white solid (3.80 g, 54%): LCMS (ESI) calc'd for C₂₅H₃₆BrClN₂O₅S [M + H]⁺: 591, 593 (2 : 3 : 1), found 591, 593 (2 : 3 : 1); ¹H NMR (400 MHz, CDCl₃) δ 7.24 (d, *J* = 22.6 Hz, 1H), 7.12 (s, 1H), 6.07-5.97 (m, 1H), 5.46-5.30 (m, 2H), 4.68-4.58 (m, 2H), 4.60-4.50 (m, 3H), 4.49-4.33 (m, 1H), 4.21-4.01 (m, 2H), 3.92-3.88 (m, 1H) 3.09-2.82 (m, 1H), 2.56 (t, *J =* 12.7 Hz, 1H), 2.19-1.96 (m, 2H), 1.57-1.25 (m, 9H), 1.17 (d, *J* = 11.7 Hz, 9H).

### Example 25. Intermediate 25 ((S)-N-((R)-(2-(allyloxy)-5-chloro-4-methylphenyl)(piperidin-4-yl)methyl)-2-methylpropane-2-sulfinamide)

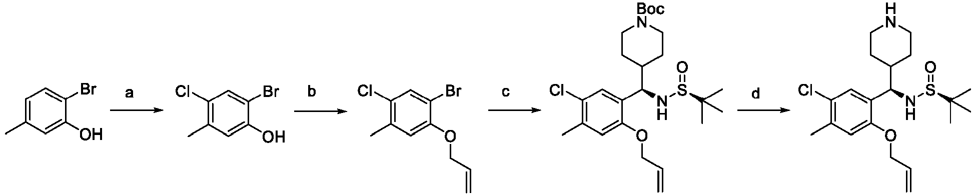

### Step a:

To a stirred solution of 2-bromo-5-methylphenol (42.00 g, 224.56 mmol) in 1,1,1,3,3,3-hexafluoropropan-2-ol (500 mL) was added NCS (31.00 g, 235.78 mmol) in portions at room temperature under air atmosphere. The reaction solution was allowed to warm to 50 °C and stirred for 16 h under air atmosphere. After cooling to room temperature, the resulting solution was concentrated under reduced pressure. The residue was purified by silica gel column chromatography, eluted with PE/EA (50/1) to afford 2-bromo-4-chloro-5-methylphenol as a light yellow solid (47.00 g, 90%): ¹H NMR (400 MHz, CDCl₃) δ 7.45 (s, 1H), 6.93 (d, *J =* 0.8 Hz, 1H), 2.32 (d, *J =* 0.7 Hz, 3H).

### Step b:

To a stirred mixture of 2-bromo-4-chloro-5-methylphenol (31.00 g, 0.14 mol) and K₂CO₃ (39.00 g, 0.28 mol) in DMF (300 mL) was added allyl bromide (29.00 g, 0.24 mol) dropwise at room temperature under air atmosphere. The reaction mixture was stirred for 16 h at 40 °C under air atmosphere. After cooling to room temperature, the resulting mixture was diluted with water (300 mL) and extracted with EA (3 x 150 mL). The combined organic layers were washed with brine (6 x 100 mL), dried over anhydrous Na₂SO₄. After filtration, the filtrate was concentrated under reduced pressure. The residue was purified by silica gel column chromatography, eluted with PE/EA (100/1) to afford 1-bromo-5-chloro-4-methyl-2-(prop-2-en-1-yloxy)benzene as a colorless oil (24.50 g, 66%): ¹H NMR (400 MHz, CDCl₃) δ 7.53 (s, 1H), 6.77 (s, 1H), 6.12-6.02 (m, 1H), 5.50 (d, *J =* 17.3 Hz, 1H), 5.34 (d, *J =* 10.4 Hz, 1H), 4.63-4.56 (m, 2H), 2.34 (s, 3H).

### Step c:

To a stirred solution of 1-bromo-5-chloro-4-methyl-2-(prop-2-en-1-yloxy)benzene (29.00 g, 0.12 mol) in THF (900 mL) was added *n*-BuLi (48 mL, 0.12 mol, 2.5 M in hexane) dropwise at -90 °C under nitrogen atmosphere. After stirring for 40 min at -90 °C under nitrogen atmosphere, a solution of *tert*-butyl 4-[[(*S*)-2-methylpropane-2-sulfinyl]imino]methyl]piperidine-1-carboxylate (35.57 g, 0.12 mol) in THF (80 mL) was added dropwise to a stirred solution over 20 min at -90 °C under nitrogen atmosphere. The resulting mixture was stirred for additional 1 h at -90 °C under nitrogen atmosphere. The reaction was quenched with saturated aq. NH₄Cl (100 mL) at -90 °C. The reaction solution was concentrated under reduced pressure to remove THF. The aqueous layer was extracted with EA (3 x 600 mL). The combined organic layers were washed with brine (3 x 200 mL), dried over anhydrous Na₂SO₄. After filtration, the filtrate was concentrated under reduced pressure. The residue was purified by silica gel column chromatography, eluted with PE/EA (1/1) to afford *tert*-butyl 4-[(*R*)-[5-chloro-4-methyl-2-(prop-2-en-1-yloxy)phenyl]([[(*S*)-2-methylpropane-2-sulfinyl]amino])methyl]piperidine-1-carboxylate as a light yellow oil (25.40 g, 44%): LCMS (ESI) calc'd for C₂₅H₃₉ClN₂O₄S [M + H]⁺: 499, 501 (3 : 1), found 499, 501 (3 : 1); ¹H NMR (400 MHz, CDCl₃) δ 7.09 (s, 1H), 6.74 (s, 1H), 6.10-5.93 (m, 1H), 5.47-5.26 (m, 2H), 4.61-4.50 (m, 2H), 4.41-4.28 (m, 1H), 4.21-4.04 (m, 2H), 3.95-3.73 (m, 1H), 2.82-2.47 (m, 2H), 2.41 (s, 3H), 2.10-1.96 (m, 1H), 1.93-1.75 (m, 1H), 1.47 (s, 9H), 1.37-1.21 (m, 3H), 1.12 (s, 9H).

### Step d:

To a stirred solution of *tert*-butyl 4-[(*R*)-[5-chloro-4-methyl-2-(prop-2-en-1-yloxy)phenyl]([[(*S*)-2-methylpropane-2-sulfinyl]amino])methyl]piperidine-1-carboxylate (48.00 g, 95.77 mmol) in DCM (380 mL) was added TFA (96 mL) dropwise at room temperature. The resulting mixture was stirred for additional 1 h at room temperature. The mixture was basified to pH 8 with saturated aq. NaHCO₃. The resulting mixture was extracted with EA (3 x 1 L). The combined organic layers were washed with brine (3 x 300 mL), dried over anhydrous Na₂SO₄. After filtration, the filtrate was concentrated under reduced pressure to afford (*S*)*-N-*[(*R*)-[5-chloro-4-methyl-2-(prop-2-en-1-yloxy)phenyl](piperidin-4-yl)methyl]-2-methylpropane-2-sulfinamide as a light yellow oil (33.10 g, 88%): LCMS (ESI) calc'd for C₂₀H₃₁ClN₂O₂S [M + 1]⁺: 399, 401 (3 : 1), found 399, 401 (3 : 1); ¹H NMR (400 MHz, CDCl₃) δ 7.10 (s, 1H), 6.75 (s, 1H), 6.08-5.93 (m, 1H), 5.44-5.21 (m, 2H), 4.60-4.47 (m, 2H), 4.34-4.22 (m, 2H), 3.43 (d, *J =* 13.0 Hz, 1H), 3.34 (d, *J =* 13.0 Hz, 1H), 2.88-2.72 (m, 2H), 2.34 (s, 3H), 2.12 (d, *J =* 10.5 Hz, 2H), 1.69-1.51 (m, 3H), 1.10 (s, 9H).

### Example 26. Intermediate 26 (N-[(1-acetylpiperidin-4-yl)(4,5-dichloro-2-methoxyphenyl)methylidene]-2-methylpropane-2-sulfinamide)

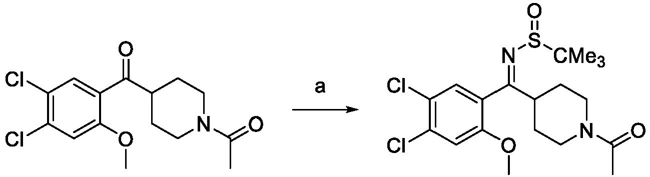

### Step a:

To a stirred solution of 1-[4-[(4,5-dichloro-2-methoxyphenyl)carbonyl]piperidin-1-yl]ethan-1-one (0.30 g, 0.91 mmol) and Ti(OEt)₄ (0.41 g, 1.82 mmol) in THF (10 mL) was added 2-methylpropane-2-sulfinamide (0.22 g, 1.82 mmol) at room temperature under nitrogen atmosphere. The resulting mixture was stirred for 16 h at 70 °C under nitrogen atmosphere. After cooling to room temperature, the reaction was quenched with saturated aq. NH₄Cl (50 mL) at room temperature and extracted with EA (3 x 50 mL). The combined organic layers were washed with brine (2 x 50 mL), dried over anhydrous Na₂SO₄. After filtration, the filtrate was concentrated under reduced pressure. The residue was purified by silica gel column chromatography, eluted with PE/EA (2/1) to afford *N*-[(1-acetylpiperidin-4-yl)(4,5-dichloro-2-methoxyphenyl)methylidene]-2-methylpropane-2-sulfinamide as a light yellow oil (0.27 g, 68%): LCMS (ESI) calc'd for C₁₉H₂₆Cl₂N₂O₃S [M + H]⁺: 433, 435 (3 : 2), found 433, 435 (3 : 2).

### Example 27. Intermediate 27 ((2R,3S)-3-(benzoyloxy)oxolane-2-carboxylic acid)

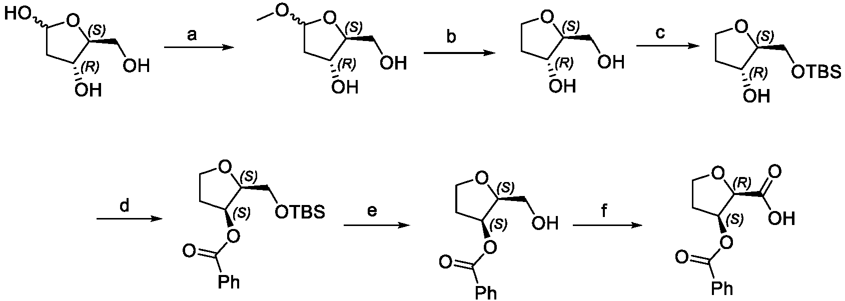

### Step a:

To a solution of (*4R,5S*)-5-(hydroxymethyl)oxolane-2,4-diol (15.00 g, 111.83 mmol) in MeOH (130 mL) was added H₂SO₄ (1.49 mL, 27.95 mmol, 97%) in MeOH (20 mL) at 0 °C. The reaction was stirred for 16 h at 0 °C to room temperature. The mixture was neutralized to pH 7 with saturated aq. NaHCO₃. The resulting mixture was filtered and the filter cake was washed with MeOH (3 x 20 mL). The filtrate was concentrated under reduced pressure. The residue was purified by silica gel column chromatography, eluted with PE/EA (1/4) to afford (2*S*,3*R*)-2-(hydroxymethyl)-5-methoxyoxolan-3-ol as a light yellow oil (15.00 g, 91%): ¹H NMR (400 MHz, DMSO-*d*₆) δ 4.64 (t, *J =* 3.3 Hz, 1H), 4.52 (d, *J =* 5.5 Hz, 1H), 4.48-4.44 (m, 1H), 3.80-3.73 (m, 1H), 3.57-3.52 (m, 2H), 3.52-3.46 (m, 1H), 3.24-3.22 (m, 3H), 1.87-1.80 (m, 1H), 1.53 (dt, *J =* 12.8, 4.0 Hz, 1H).

### Step b:

A solution of (2*S*,3*R*)-2-(hydroxymethyl)-5-methoxyoxolan-3-ol (5.00 g, 33.75 mmol) and bis(trimethylsilyl)trifluoroacetamide (17.37 g, 67.48 mmol) in ACN (5 mL) was stirred for 5 h at 80 °C. After cooling to room temperature, Et₃SiH (19.62 g, 168.74 mmol) and TMSOTf (37.50 g, 168.72 mmol) were added to the above mixture in portions over 15 min at room temperature. The resulting mixture was stirred for additional 16 h at room temperature. The reaction was quenched with water (30 mL) at 0 °C and neutralized to pH 7 with saturated aq. NaHCO₃. The resulting mixture was concentrated under reduced pressure. The residue was purified by silica gel column chromatography, eluted with EA to afford (2S, 3R)-2-(hydroxymethyl)oxolan-3-ol as a light yellow semi-solid (1.40 g, 35%): ¹H NMR (400 MHz, DMSO-*d*₆) δ 4.93-4.77 (m, 1H), 4.71-4.54 (m, 1H), 3.82-3.67 (m, 2H), 3.57 (td, *J =* 5.3, 2.9 Hz, 1H), 3.40-3.33 (m, 1H), 1.98-1.85 (m, 1H), 1.74-1.63 (m, 1H).

### Step c:

To a stirred solution of (2S,3R)-2-(hydroxymethyl)oxolan-3-ol (1.30 g, 11.01 mmol) and imidazole (0.76 g, 11.12 mmol) in DMF (8 mL) was added TBDMSCl (1.66 g, 11.01 mmol) dropwise at room temperature. The resulting solution was stirred for 0.5 h at room temperature. The reaction was diluted with EA (50 mL) and water (50 mL). The aqueous solution was extracted with EA (2 x 50 mL). The combined organic layers were washed with brine (3 x 30 mL) and dried over anhydrous Na₂SO₄. After filtration, the filtrate was purified by silica gel column chromatography, eluted with PE/EA (2/1) to afford (*2S,3R*)-2-[[(*tert*butyldimethylsilyl)oxy]methyl]oxolan-3-ol as a colorless oil (1.54 g, 60%): ¹H NMR (400 MHz, DMSO-*d*₆) δ 4.88 (d, *J =* 4.2 Hz, 1H), 4.08-3.99 (m, 1H), 3.82-3.68 (m, 2H), 3.62-3.55 (m, 1H), 3.55-3.44 (m, 2H), 2.01-1.85 (m, 1H), 1.78-1.63 (m, 1H), 0.87 (s, 9H), 0.04 (d, *J =* 2.5 Hz, 6H).

### Step d:

To a stirred solution of (2S,3R)-2-[[(tert-butyldimethylsilyl)oxy]methyl]oxolan-3-ol (0.82 g, 3.53 mmol) and benzoic acid (0.56 g, 4.59 mmol) in THF (8 mL) were added Ph₃P (1.85 g, 7.05 mmol) and DEAD (1.23 g, 7.06 mmol) at room temperature. The resulting solution was stirred for 1 h at room temperature. The reaction was diluted with EA (30 mL) and water (30 mL). The aqueous solution was extracted with EA (2 x 30 mL). The combined organic layers were washed with brine (3 x 30 mL) and dried over anhydrous Na₂SO₄. After filtration, the filtrate was purified by silica gel column chromatography, eluted with PE/EA (6/1) to afford (2*S*,3*S*)-2-[[(*tert*-butyldimethylsilyl)oxy]methyl]oxolan-3-yl benzoate as a colorless oil (1.00 g, 84%): ¹H NMR (400 MHz, CDCl₃) δ 8.06 (dt, *J =* 8.2, 1.1 Hz, 2H), 7.66-7.55 (m, 1H), 7.47 (t, *J* = 7.7 Hz, 2H), 5.72-5.59 (m, 1H), 4.20-4.01 (m, 1H), 4.01-3.81 (m, 1H), 3.96 (td, *J =* 8.7, 4.8 Hz, 1H), 3.91 (dd, *J =* 6.3, 2.0 Hz, 2H), 2.48-2.32 (m, 1H), 2.20-2.08 (m, 1H), 0.85 (s, 9H), 0.05--0.05 (m, 6H).

### Step e:

To a stirred solution of (2*S*,3*S*)-2-[[(*tert*-butyldimethylsilyl)oxy]methyl]oxolan-3-yl benzoate (0.50 g, 1.49 mmol) in THF (3 mL) and HOAc (0.1 mL) was added TBAF (2.97 mL, 2.970 mmol, 1 M in THF) at room temperature. The reaction was stirred room temperature for 3 h. The reaction was diluted with EA (30 mL) and water (30 mL). The aqueous solution was extracted with EA (2 x 30 mL). The combined organic layers were washed with brine (3 x 30 mL) and dried over anhydrous Na₂SO₄. After filtration, the filtrate was purified by silica gel column chromatography, eluted with PE/EA (1/4) to afford (2S,3S)-2-(hydroxymethyl)oxolan-3-yl benzoate as a colorless oil (0.30 g, 91%): ¹H NMR (400 MHz, DMSO-*d*₆) δ 7.96-7.91 (m, 2H), 7.68-7.62 (m, 1H), 7.53 (t, *J =* 7.6 Hz, 2H), 5.51-5.46 (m, 1H), 3.97-3.86 (m, 2H), 3.81-3.72 (m, 1H), 3.65-3.57 (m, 2H), 2.40-2.29 (m, 1H), 2.02-1.93 (m, 1H).

### Step f:

To a stirred solution of (2S,3S)-2-(hydroxymethyl)oxolan-3-yl benzoate (0.34 g, 1.53 mmol) and (acetyloxy)(phenyl)-lambda3-iodanyl acetate (0.74 g, 2.30 mmol) in ACN (3 mL) and water (3 mL) was added TEMPO (48 mg, 0.31 mmol) at room temperature. The reaction was stirred at room temperature for 16 h. The resulting mixture was quenched with saturated aq. Na₂SO₃ (10 mL) and the mixture was concentrated under reduced pressure. The residue was purified by silica gel column chromatography, eluted with PE/EA (1/1) to afford (*2R,3S*)*-*3-(benzoyloxy)oxolane-2-carboxylic acid as a colorless oil (0.20 g, 55%): ¹H NMR (400 MHz, DMSO-*d*₆) δ 12.77 (s, 1H), 8.07-7.81 (m, 2H), 7.68 (t, *J =* 7.3 Hz, 1H), 7.56 (t, *J =* 7.3 Hz, 2H), 5.93-5.64 (m, 1H), 4.69-4.51 (m, 1H), 4.17-3.84 (m, 2H), 2.38-2.23 (m, 1H), 2.20-2.02 (m, 1H).

### Example 28. Intermediate 28 (lithio 4-methyl-5-oxomorpholine-2-carboxylate)

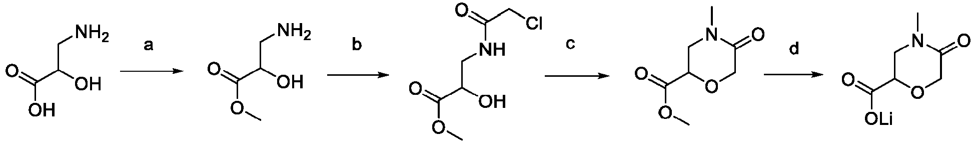

### Step a:

To a stirred mixture of isoserine (50.00 g, 475.77 mmol) in MeOH (300 mL) was added SOCl₂ (41. mL, 570.97 mmol) dropwise at 0 °C. The resulting mixture was stirred for 1 h at room temperature. The resulting mixture was concentrated under reduced pressure to afford methyl 3-amino-2-hydroxypropanoate as an off-white solid (50.00 g, 71%): LCMS (ESI) calc'd for C₄H₉NO₃ [M + H]⁺: 120, found 120; ¹H NMR (400 MHz, CD₃OD) δ 4.50 (dd, *J =* 8.2, 4.0 Hz, 1H), 3.82 (s, 3H), 3.36-3.30 (m, 1H), (dd, *J =* 13.0, 8.3 Hz, 1H).

### Step b:

To a stirred solution of methyl 3-amino-2-hydroxypropanoate (22.50 g, 188.89 mmol) and Et₃N (57.34 g, 566.66 mmol) in DCM (300 mL) was added chloroacetyl chloride (21.33 g, 188.89 mmol) dropwise at room temperature. The resulting mixture was stirred for 1 h at room temperature. The reaction was concentrated under reduced pressure. The residue was purified by silica gel column chromatography, eluted with PE/EA (1/2) to afford methyl 3-(2-chloroacetamido)-2-hydroxypropanoate as a yellow solid (13.00 g, 35%): LCMS (ESI) calc'd for C₆H₁₀ClNO₄ [M + H]⁺: 196, 198 (3 : 1), found 196, 198 (3 : 1); ¹H NMR (400 MHz, CDCl₃) δ 6.97 (s, 1H), 4.35 (t, *J =* 5.0 Hz, 1H), 4.08 (s, 2H), 3.84 (s, 3H), 3.79-3.62 (m, 2H), 3.26 (s, 1H).

### Step c:

To a stirred solution of methyl 3-(2-chloroacetamido)-2-hydroxypropanoate (5.00 g, 25.56 mmol) in THF (300 mL) was added t-BuOK (5.74 g, 51.15 mmol) at room temperature under nitrogen atmosphere. The reaction was stirred at room temperature for 1 h. After starting material was consumed completely, MeI (4.35 g, 30.65 mmol) was added into the reaction. The mixture was stirred at room temperature for additional 2 h. The solution was concentrated under reduced pressure. The residue was purified by silica gel column chromatography, eluted with EA to afford methyl 4-methyl-5-oxomorpholine-2-carboxylate as a light yellow semi-solid (0.90 g, 20%): LCMS (ESI) calc'd for C₇H₁₁NO₄ [M + H]⁺: 174, found 174; ¹H NMR (400 MHz, CD₃OD) δ 4.61 (dd, *J =* 7.5, 4.8 Hz, 1H), 4.30 (d, *J =* 16.5 Hz, 1H), 4.19 (d, *J =* 16.6 Hz, 1H), 3.78 (s, 3H), 3.63 (dd, *J* = 6.2, 4.1 Hz, 2H), 2.98 (s, 3H).

### Step d:

To a stirred solution of methyl 4-methyl-5-oxomorpholine-2-carboxylate (0.15 g, 0.87 mmol) in MeOH (3 mL) was added a solution of LiOH·H₂O (73 mg, 1.73 mmol) in water (1 mL) at room temperature. The reaction solution was stirred at 40 °C for 1 h. The reaction was concentrated under reduced pressure to afford lithio 4-methyl-5-oxomorpholine-2-carboxylate as an off-white solid (0.14 g, crude), which was used in the next step directly without further purification: LCMS (ESI) calc'd for C₆H₉NO₄ [M + H]⁺: 160, found 160.

### Example 29. Intermediate 29 (tert-butyl 3-[methoxy(methyl)carbamoyl]-8-azabicyclo[3.2.1]octane-8-carboxylate)

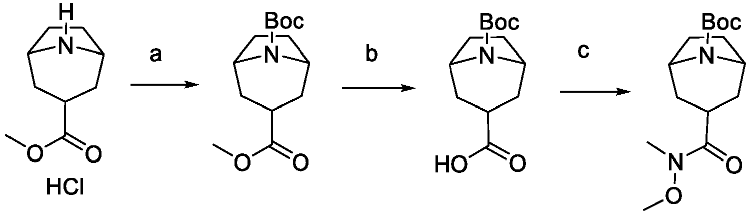

### Step a:

To a stirred solution of methyl 8-azabicyclo[3.2.1]octane-3-carboxylate (1.00 g, 5.91 mmol) and Et₃N (1.20 g, 11.86 mol) in DCM (10 mL) was added Boc₂O (1.60 g, 7.33 mmol) at room temperature. The reaction was stirred at room temperature for 1 h. The reaction was diluted with EA (50 mL) and water (50 mL). The isolated aqueous solution was extracted with EA (3 x 20 mL). The combined organic layers were washed with brine (3 x 30 mL) and dried over anhydrous Na₂SO₄. After filtration, the filtrate was concentrated under reduced pressure to afford 8-*tert*-butyl 3-methyl 8-azabicyclo[*3.2.1*]octane-3,8-dicarboxylate as a light yellow oil (1.30 g, 99%): LCMS (ESI) calc'd for C₁₄H₂₃NO₄ [M + H -15]⁺: 255, found 255.

### Step b:

To a stirred solution of 8-*tert*-butyl 3-methyl 8-azabicyclo[*3.2.1*]octane-3,8-dicarboxylate (1.30 g, 4.83 mol) in MeOH (5 mL) was added a solution of NaOH (0.39 g, 9.65 mol) in H₂O (0.5 mL) at room temperature. The reaction was stirred at room temperature for 16 h. The reaction was diluted with EA (50 mL) and water (50 mL). The aqueous solution was extracted with EA (3 x 20 mL). The combined aqueous layers were acidified with citric acid to pH 3 and extracted with EA (3 x 50 mL). The combined organic layers were washed with brine (3 x 30 mL) and dried over anhydrous Na₂SO₄, After filtration, the filtrate was concentrated under reduced pressure to afford (8-[(*tert-*butoxy)carbonyl]-8-azabicyclo[*3.2.1*]octane-3-carboxylic acid) as a light yellow oil (1.20 g, 97%): LCMS (ESI) calc'd for C₁₃H₂₁NO₄ [M + Na]⁺: 278, found 278; ¹H NMR (300 MHz, CDCl₃) δ 4.40-4.21 (m, 2H), 2.94-2.79 (m, 1H), 2.06-1.98 (m, 2H), 1.98-1.84 (m, 2H), 1.84-1.73 (m, 2H), 1.73-1.59 (m, 2H), 1.49 (s, 9H).

### Step c:

To a stirred solution of 8-[(*tert*-butoxy)carbonyl]-8-azabicyclo[*3.2.1*]octane-3-carboxylic acid (1.20 g, 4.70 mmol) and Et₃N (0.95 g, 9.40 mmol) in DCM (10 mL) were added CDI (0.91g, 5.64 mmol) and *N,O*-methoxy(methyl)amine hydrochloride (0.69 g, 7.05 mmol) at room temperature. The reaction was stirred at room temperature for 3 h. The reaction was concentrated under reduced pressure. The residue was purified by silica gel column chromatography, eluted with PE/EA (1/1) to afford *tert*-butyl 3-[methoxy(methyl)carbamoyl]-8-azabicyclo[*3.2.1*]octane-8-carboxylate as a light yellow oil (1.10 g, 69% overall three steps): LCMS (ESI) calc'd for C₁₅H₂₆N₂O₄ [M + H - 56]⁺: 243, found 243; ¹H NMR (400 MHz, CDCl₃) δ 4.40-4.23 (m, 1H), 3.82-3.76 (m, 1H), 3.76-3.71 (m, 3H), 3.35-3.21 (m, 1H), 3.19 (d, *J =* 2.6 Hz, 3H), 2.18-1.93 (m, 4H), 1.81-1.56 (m, 4H), 1.56-1.44 (m, 9H).

### Example 30. Intermediate 30 (tert-butyl 3-[methoxy(methyl)carbamoyl]azetidine-1-carboxylate)

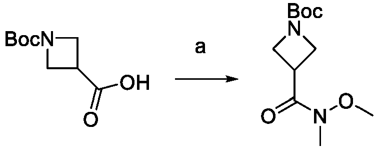

### Step a:

To a stirred solution of 1-[(*tert*-butoxy)carbonyl]azetidine-3-carboxylic acid (2.00 g, 9.94 mmol) and CDI (1.80 g, 10.9 mmol) in DCM (10 mL) were added Et₃N (1.20 g, 11.93 mmol) and *N*,*O*-methoxy(methyl)amine hydrochloride (0.90 g, 14.91 mmol) at room temperature. The reaction solution was stirred at room temperature for 1 h. The resulting solution was diluted with water (30 mL) at room temperature and extracted with EA (3 x 30 mL). The combined organic layers were washed with brine (2 x 20 mL), dried over anhydrous Na₂SO₄. After filtration, the filtrate was concentrated under reduced pressure. The residue was purified by silica gel column chromatography, eluted with PE/EA (1/1) to afford *tert*-butyl 3-[methoxy(methyl)carbamoyl]azetidine-1-carboxylate as a light yellow oil (2.10 g, 78%): LCMS (ESI) calc'd for C₁₁H₂₀N₂O₄ [M + H - 56]⁺: 189, found 189; ¹H NMR (300 MHz, CD₃OD) δ 4.14-3.99 (m, 4H), 3.89-3.76 (m, 1H), 3.72 (s, 3H), 3.22 (s, 3H), 1.46 (s, 9H).

### Example 31. Intermediate 31 (tert-butyl 4-fluoro-4-[methoxy(methyl)carbamoyl]piperidine-1-carboxylate)

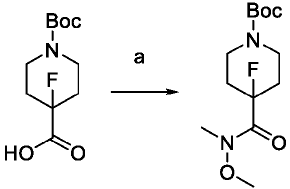

### Step a:

To a stirred solution of 1-[(*tert*-butoxy)carbonyl]-4-fluoropiperidine-4-carboxylic acid (2.00 g, 8.09 mmol) and CDI (2.60 g, 16.18 mmol) in DCM (10 mL) were added Et₃N (2.50 g, 24.27 mmol) and *N*,*O*-methoxy(methyl)amine hydrochloride (1.00 g, 16.18 mmol) at room temperature. The reaction solution was stirred at room temperature for 16 h at room temperature. The resulting solution was diluted with water (30 mL) at room temperature and extracted with EA (3 x 30 mL). The combined organic layers were washed with brine (2 x 20 mL), dried over anhydrous Na₂SO₄. After filtration, the filtrate was concentrated under reduced pressure. The residue was purified by silica gel column chromatography, eluted with PE/EA (3/1) to afford *tert-*butyl 4-fluoro-4-[methoxy(methyl)carbamoyl]piperidine-1-carboxylate as a colorless oil (1.50 g, 57%): LCMS (ESI) calc'd for C₁₃H₂₃FN₂O₄ [M + H - 56]⁺: 235, found 235; ¹H NMR (400 MHz, CDCl₃) δ 4.01 (d, *J =* 13.5 Hz, 2H), 3.75 (s, 3H), 3.26 (s, 3H), 3.21-3.01 (m, 1H), 2.06 (dd, *J =* 19.6, 10.9 Hz, 5H), 1.49 (s, 9H).

### Example 32. Intermediate 32 (litho 5-((tert-butoxycarbonyl)amino)-1,3,4-oxadiazole-2-carboxylate)

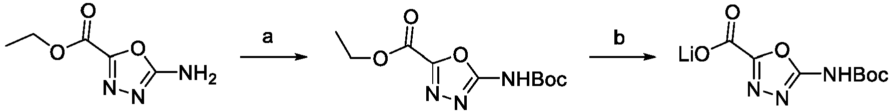

### Step a:

To a stirred solution of ethyl 5-amino-1,3,4-oxadiazole-2-carboxylate (0.10 g, 0.64 mmol) and Et₃N (0.19 g, 1.91 mmol) in DMF (1 mL) was added Boc₂O (0.17 g, 0.76 mmol) at room temperature. The resulting solution was stirred for 12 h at room temperature. The reaction was concentrated under reduced pressure. The residue was purified by reverse phase chromatography, eluted with 55% ACN in water with 10 mmoL/L NH₄HCO₃ to afford ethyl 5-[[(*tert*-butoxy)carbonyl]amino]-1,3,4-oxadiazole-2-carboxylate as a light yellow oil (0.10 g, 55%): LCMS (ESI) calc'd for C₁₀H₁₅N₃O₅ [M + H - 56]⁺: 202, found 202; ¹H NMR (400 MHz, DMSO-*d*₆) δ 4.38 (q, *J =* 7.1 Hz, 2H), 1.46 (d, *J =* 3.1 Hz, 9H), 1.32 (t, *J =* 7.1 Hz, 3H).

### Step b:

To a stirred solution of ethyl 5-[[(tert-butoxy)carbonyl]amino]-1,3,4-oxadiazole-2-carboxylate (0.16 g, 0.62 mmol) in MeOH (2 mL) was added a solution of LiOH·H₂O (78 mg, 1.87 mmol) in water in MeOH (3 mL) a solution of LiOH·H₂O (48 mg, 1.15 mmol) in H₂O (2 mL) was added at room temperature. The resulting mixture was stirred for 2 h at room temperature. The resulting mixture was concentrated under reduced pressure to afford litho 5-((*tert*-butoxycarbonyl)amino)-1,3,4-oxadiazole-2-carboxylate as an off-white solid (0.16 g, crude), which was used in the next step directly without further purification: LCMS (ESI) calc'd for C₈H₁₁N₃O₅ [M + H]⁺: 230, found 230; ¹H NMR (400 MHz, DMSO-*d*₆) δ 8.53 (s, 1H), 2.50 (s, 9H).

### Example 33. Intermediate 33 (3-iodo-1-(triphenylmethyl)-1H-pyrazole)

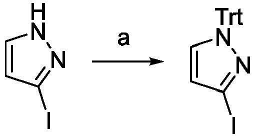

### Step a:

To a stirred solution of 3-iodo-1*H*-pyrazole (0.50 g, 2.59 mmol) in DCM (5 mL) were added TrtCl (0.86 g, 3.09 mmol) and Et₃N (0.52 g, 5.16 mmol) at room temperature. The resulting mixture was stirred for 1 h at room temperature. The resulting mixture was concentrated under reduced pressure. The residue was purified by silica gel column chromatography, eluted with PE/EA (12/1) to afford 3-iodo-1-(triphenylmethyl)-1*H*-pyrazole as an off-white solid (0.60 g, 48%): ¹H NMR (400 MHz, CDCl₃) δ 7.19-7.10 (m, 15H), 6.42 (d, *J =* 2.5 Hz, 2H).

### Example 34. Intermediate 34 (4-iodo-1-(triphenylmethyl)-1H-pyrazole)

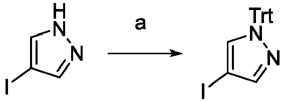

### Step a:

To a stirred solution of 4-iodo-1*H*-pyrazole (0.50 g, 2.59 mmol) in DCM (5 mL) were added TrtCl (0.86 g, 3.09 mmol) and Et₃N (0.52 g, 5.16 mmol) at room temperature. The resulting mixture was stirred for 1 h at room temperature. The resulting mixture was concentrated under reduced pressure. The residue was purified by silica gel column chromatography, eluted with PE/EA (10/1) to afford 4-iodo-1-(triphenylmethyl)-1*H*-pyrazole as an off-white solid (0.80 g, 64%): ¹H NMR (400 MHz, DMSO-*d*₆) δ 7.75 (s, 1H), 7.45 (s, 1H), 7.40-7.35 (m, 9H), 7.07-7.00 (m, 6H).

### Example 35. Intermediate 35 (lithio (S)-3-methoxy-2-(trityloxy)propanoate)

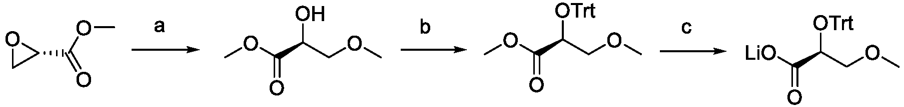

### Step a:

To a stirred solution of methyl (2*S*)-oxirane-2-carboxylate (2.00 g, 19.59 mmol) in MeOH (15 mL) was added Mg(OSO₂CF₃)₂ (3.15 g, 9.80 mmol) at room temperature. The resulting mixture was stirred for 16 h at 40 °C. The resulting mixture was concentrated under reduced pressure. The residue was purified by silica gel column chromatography, eluted with DCM/MeOH (10/1) to afford methyl (2*S*)-2-hydroxy-3-methoxypropanoate as a light yellow oil (1.50 g, 46%): LCMS (ESI) calc'd for C₅H₁₀O₄ [M + H]⁺: 135, found 135; ¹H NMR (400 MHz, CDCl₃) δ 4.34 (t, *J =* 3.4 Hz, 1H), 3.83 (s, 3H), 3.75-3.65 (m, 2H), 3.41 (s, 3H), 2.65 (brs, 1H).

### Step b:

To a stirred solution of methyl (2*S*)-2-hydroxy-3-methoxypropanoate (0.70 g, 5.22 mmol) and DMAP (64 mg, 0.52 mmol) in pyridine (8 mL) was added TrtCl (1.60 g, 5.74 mmol) at room temperature. The resulting solution was allowed to warm to 80 °C and stirred for 24 h. The resulting mixture was diluted with water (30 mL). The resulting mixture was extracted with EA (3 x 30 mL). The combined organic layers were washed with brine (3 x 30 mL), dried over anhydrous Na₂SO₄. After filtration, the filtrate was concentrated under reduced pressure. The residue was purified by silica gel column chromatography, eluted with PE/EA (10/1) to afford methyl (2S)-3-methoxy-2-(triphenylmethoxy)propanoate as an off-white solid (0.80 g, 33%): ¹H NMR (400 MHz, DMSO-*d*₆) δ 7.45-7.19 (m, 15H), 4.16 (t, *J =* 5.2 Hz, 1H), 3.53 (dd, *J =* 10.6, 5.5 Hz, 1H), 3.32-3.27 (m, 1H), 3.23 (s, 3H), 3.21 (s, 3H).

### Step c:

To a stirred solution of methyl (2S)-3-methoxy-2-(triphenylmethoxy)propanoate (0.80 g, 2.13 mmol) in MeOH (8 mL) was added a solution of LiOH·H₂O (0.45 g, 10.63 mmol) in water (3 mL) at room temperature. The resulting mixture was stirred for 2 h at room temperature. The resulting mixture was concentrated under reduced pressure to afford lithio (S)-3-methoxy-2-(trityloxy)propanoate (0.80 g, crude), which was used in the next step directly without further purification: LCMS (ESI) calc'd for C₂₃H₂₂O₄ [M + Na]⁺: 385, found 385.

### Example 36. Intermediate 36 (lithium 1-oxo-1,2-dihydroisoquinoline-4-carboxylate)

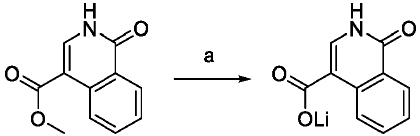

### Step a:

To a stirred solution of methyl 1-oxo-2*H*-isoquinoline-4-carboxylate (0.20 g, 1.00 mmol) in MeOH (2 mL) was added was added a solution of LiOH·H₂O (84 mg, 2.00 mmol) in water (3 mL) at room temperature. The resulting mixture was stirred for 16 h at room temperature. The resulting mixture was concentrated under reduced pressure to afford lithium 1-oxo-1,2-dihydroisoquinoline-4-carboxylate (0.20 g, crude), which was used in the next step directly without further purification: LCMS (ESI) calc'd for C₁₀H₇NO₃ [M - H]⁺: 188, found 188.

### Example 37. Intermediate 37 (lithium 5-cyano-6-oxo-1,6-dihydropyridine-3-carboxylate)

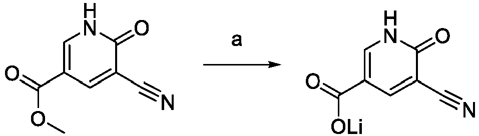

### Step a:

To a stirred solution of methyl 5-ethynyl-6-oxo-1*H*-pyridine-3-carboxylate (0.18 g, 1.00 mmol) in MeOH (2 mL) was added was added a solution of LiOH·H₂O (84 mg, 2.00 mmol) in water (3 mL) at room temperature. The resulting mixture was stirred for 0.5 h at 40 °C. After cooling to room temperature, the resulting mixture was concentrated under reduced pressure to afford lithium 5-cyano-6-oxo-1,6-dihydropyridine-3-carboxylate (0.20 g, crude), which was used in the next step directly without further purification: LCMS (ESI) calc'd for C₇H₄N₂O₃ [M - H]⁺: 163, found 163.

### Example 38. Intermediate 38 (N-[(R)-[4,5-dichloro-2-(prop-2-en-1-yloxy)phenyl](piperidin-4-yl)methyl]-2,2,2-trifluoroacetamide)

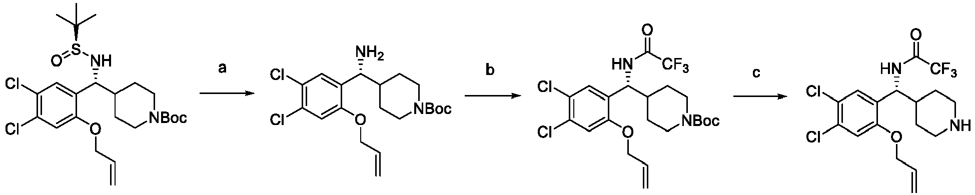

### Step a:

To a stirred solution of *tert*-butyl 4-[(*R*)-[4,5-dichloro-2-(prop-2-en-1-yloxy)phenyl]([[(*S*)-2-methylpropane-2-sulfinyl]amino])methyl]piperidine-1-carboxylate(0.50 g, 0.96 mmol) in 1,4-dioxane (8 mL) was added aq. HCl (4 *N,* 2 mL) at room temperature. The reaction solution was stirred at room temperature for 20 min. The reaction solution was adjusted pH to 8 with saturated aq. Na₂CO₃ at room temperature. The resulting mixture was extracted with EA (3 x 50 mL). The combined organic layers were washed with brine (3 x 20 mL), dried over anhydrous Na₂SO₄. After filtration, the filtrate was concentrated under reduced pressure to afford *tert*-butyl 4-[(*R*)-amino[4,5-dichloro-2-(prop-2-en-1-yloxy)phenylmethyl]piperidine-1-carboxylate as a yellow oil (0.40 g, crude), which was used in the next step directly without further purification: LCMS (ESI) calc'd for C₂₀H₂₈Cl₂N₂O₃ [M + H]⁺: 415, 417 (3 : 2), found 415, 417 (3 : 2).

### Step b:

To a stirred solution of *tert*-butyl 4-[(*R*)-amino[4,5-dichloro-2-(prop-2-en-1-yloxy)phenyl]methyl]piperidine-1-carboxylate (0.40 g, 0.96 mmol) and Et₃N (0.29 g, 2.89 mmol) in DCM (5 mL) was added TFAA (0.24 g, 1.16 mmol) at 0 °C. The reaction solution was stirred at 0 °C for 0.5 h. The reaction mixture was diluted with DCM (50 mL) and water (30 mL). The aqueous solution was extracted with DCM (2 x 20 mL). The combined organic layers were washed with brine (2 x 20 mL), dried over Na₂SO₄. After filtration, the filtrate was concentrated under reduced pressure to afford *tert*-butyl 4-[(*R*)-[4,5-dichloro-2-(prop-2-en-1-yloxy)phenyl](2,2,2-trifluoroacetamido)methyl]piperidine-1-carboxylate as a yellow oil (0.40 g, crude), which was used in the next step directly without further purification: LCMS (ESI) calc'd for C₂₂H₂₇Cl₂F₃N₂O₄ [M + Na]⁺: 533, 535 (3 : 2), found 533, 535 (3 : 2); ¹H NMR (400 MHz, CDCl₃) δ 7.25 (s, 1H), 7.02 (s, 1H), 6.12-5.98 (m, 1H), 5.48-5.39 (m, 2H), 4.77 (t, *J =* 9.7 Hz, 1H), 4.69-4.56 (m, 2H), 4.10 (d, *J =* 16.0 Hz, 2H), 2.75-2.48 (m, 2H), 2.08-1.97 (m, 1H), 1.78 (d, *J* = 13.3 Hz, 1H), 1.45 (s, 9H), 1.31-1.20 (m, 3H).

### Step c:

To a stirred solution of *tert*-butyl 4-[(*R*)-[4,5-dichloro-2-(prop-2-en-1-yloxy)phenyl](2,2,2-trifluoroacetamido)methyl]piperidine-1-carboxylate (0.40 g, 0.78 mmol) in DCM (2 mL) was added TFA (2 mL) at room temperature. The reaction solution was stirred for 1 h at room temperature. The resulting mixture was concentrated under reduced pressure. The residue was purified by reverse phase chromatography, eluted with 50% ACN in water with 10 mmol/L NH₄HCO₃ to afford *N*-[(*R*)-[4,5-dichloro-2-(prop-2-en-1-yloxy)phenyl](piperidin-4-yl)methyl]-2,2,2-trifluoroacetamide as a light yellow solid (0.30 g, 76% overall three steps): LCMS (ESI) calc'd for C₁₇H₁₉Cl₂F₃N₂O₂ [M + H]⁺: 411, 413 (3 : 2), found 411, 413 (3 : 2).

### Example 39. Intermediate 39 (lithio 2-(oxetan-3-yloxy)acetate)

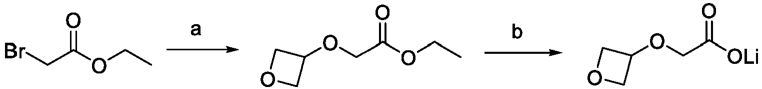

### Step a:

To a stirred mixture of NaH (12.65 g, 316.16 mmol, 60%) in THF (300 mL) was added oxetan-3-ol (19.52 g, 263.47 mmol) dropwise at 0 °C under nitrogen atmosphere. The reaction solution was stirred at room temperature for 0.5 h under nitrogen atmosphere. To the above mixture was added ethyl 2-bromoacetate (44.00 g, 263.47 mmol) dropwise at 0 °C under nitrogen atmosphere. The reaction mixture was stirred at room temperature for additional 2 h at room temperature. The resulting mixture was quenched with water (200 mL) and extracted with EA (3 x 200 mL). The combined organic layers were washed with brine (30 x 100 mL) and dried over anhydrous Na₂SO₄. After filtration, the filtrate was concentrated under reduced pressure. The residue was purified by silica gel column chromatography, eluted with PE/EA (5/1) to afford ethyl 2-(oxetan-3-yloxy)acetate as a colorless oil (25.00 g, 59%): ¹H NMR (400 MHz, CDCl₃) δ 4.79-4.72 (m, 2H), 4.72-4.67 (m, 2H), 4.67-4.59 (m, 1H), 4.21 (q, *J* = 7.1 Hz, 2H), 4.05 (s, 2H), 1.28 (t, *J* = 7.1 Hz, 3H).

### Step b:

To a stirred solution of ethyl 2-(oxetan-3-yloxy)acetate (1.00 g, 6.24 mmol) in THF (2 mL) and MeOH (2 mL) was added LiOH·H₂O (0.29 g, 6.89 mmol) at 0 °C. The reaction solution was stirred at room temperature for 1 h. The resulting solution was concentrated under reduced pressure to afford lithio 2-(oxetan-3-yloxy)acetate as an off-white solid (0.70 g, crude), which was used in the next step directly without further purification: ¹H NMR (400 MHz, D₂O) δ 4.77-4.74 (m, 1H), 4.71-4.68 (m, 2H), 4.59-4.53 (m, 2H), 3.79 (s, 2H).

### Example 40. Intermediate 40 (lithio (R)-3-methoxy-2-(trityloxy)propanoate)

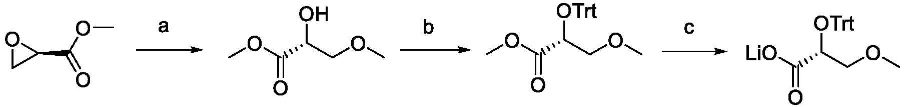

### Step a:

To a stirred solution of methyl (2R)-oxirane-2-carboxylate (1.00 g, 9.80 mmol) in MeOH (10 mL) was added Mg(OSO₂CF₃)₂ (1.58 g, 4.90 mmol) at room temperature. The resulting mixture was stirred for 16 h at 40 °C. The resulting mixture was concentrated under reduced pressure. The residue was purified by silica gel column chromatography, eluted with DCM/MeOH (10/1) to afford methyl (2*R*)-2-hydroxy-3-methoxypropanoate as a light yellow oil (0.90 g, 55%): LCMS (ESI) calc'd for C₅H₁₀O₄ [M + H]⁺: 135, found 135; ¹H NMR (400 MHz, CDCl₃) δ 4.33 (t, *J =* 3.7 Hz, 1H), 3.83 (d, *J =* 1.5 Hz, 3H), 3.76-3.65 (m, 2H), 3.41 (d, *J =* 1.5 Hz, 3H), 2.91-2.39 (brs, 1H).

### Step b:

To a stirred solution of methyl (2*R*)-2-hydroxy-3-methoxypropanoate (0.50 g, 3.73 mmol) and DMAP (46 mg, 0.37 mmol) in pyridine (4 mL) was added TrtCl (1.14 g, 4.10 mmol) at room temperature. The resulting solution was stirred for 24 h at 80 °C. The resulting mixture was diluted with water (20 mL) and extracted with EA (3 x 20 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (10/1) to afford methyl (2*R*)-3-methoxy-2-(triphenylmethoxy)propanoate as an off-white solid (0.60 g, 34%): ¹H NMR (400 MHz, DMSO-*d*₆) δ 7.49-7.09 (m, 15H), 4.16 (t, *J =* 5.2 Hz, 1H), 3.53 (dd, *J =* 10.6, 5.5 Hz, 1H), 3.32-3.27 (m, 1H), 3.23 (s, 3H), 3.21 (s, 3H).

### Step c:

To a stirred solution of methyl (2R)-3-methoxy-2-(triphenylmethoxy)propanoate (0.60 g, 1.59 mmol) in MeOH (6 mL) was added a solution of LiOH-H₂O (0.33 g, 7.97 mmol) in water (2 mL) at room temperature. The resulting mixture was stirred for 2 h at room temperature. The resulting mixture was concentrated under reduced pressure to afford lithio (R)-3-methoxy-2-(trityloxy)propanoate (0.80 g, crude), which was used in the next step directly without further purification: LCMS (ESI) calc'd for C₂₃H₂₂O₄ [M + Na]⁻: 385, found 385.

### Example 41. Intermediate 41 (tert-butyl (1R,5S,6R)-6-[methoxy(methyl)carbamoyl]-3-azabicyclo[3.1.0]hexane-3-carboxylate)

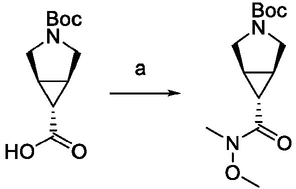

### Step a:

To a stirred solution of (1*R*,5*S*,6*R*)-3-[(*tert*-butoxy)carbonyl]-3-azabicyclo[*3.1.0*]hexane-6-carboxylic acid (0.50 g, 2.20 mmol), HOBt (0.45 g, 3.30 mmol) and EDCI (0.63 g, 3.30 mmol) in DMF (6 mL) were added *N,O*-dimethylhydroxylamine hydrochloride (0.40 g, 4.11 mmol) and Et₃N (0.45 g, 4.40 mmol) at room temperature. The reaction solution was stirred at room temperature for 2 h. The reaction was diluted with EA (30 mL) and water (30 mL). The aqueous solution was extracted with EA (3 x 30 mL). The combined organic layers were washed with brine (6 x 20 mL) and dried over anhydrous Na₂SO₄. After filtration, the filtrate was evaporated under reduced pressure. The residue was purified by reverse phase chromatography, eluted with 70% ACN in water (plus 0.05% TFA) to afford *tert-*butyl (1*R*,5*S*,6*R*)-6-[methoxy(methyl)carbamoyl]-3-azabicyclo[*3.1.0*]hexane-3-carboxylate a light yellow oil (0.30 g, 45%): LCMS (ESI) calc'd for C₁₃H₂₂N₂O₄ [M + H - 56]⁺: 215, found 215; ¹H NMR (400 MHz, CDCl₃) δ 3.76 (s, 3H), 3.72-3.64 (m, 1H), 3.59 (d, *J =* 11.2 Hz, 1H), 3.52-3.41 (m, 3H), 3.22 (s, 3H), 2.15-2.04 (m, 1H), 2.02-1.93 (m, 1H), 1.47 (s, 9H).

### Example 42. Intermediate 42 (tert-butyl 4-[methoxy(methyl)carbamoyl]-2-methylpiperidine-1-carboxylate)

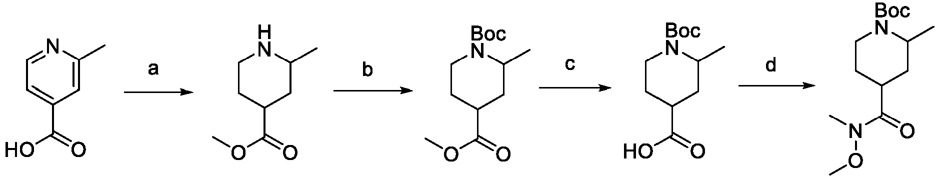

### Step a:

To a stirred solution of 2-methylpyridine-4-carboxylic acid (2.00 g, 14.58 mmol) in MeOH (10 mL) was added PtO₂ (0.40 g, 1.75 mmol) and HCl (6 *N,* 1 mL) at room temperature. The reaction was degassed for three times under hydrogen and stirred at 30 °C for 16 h under H₂ (50 atm). The reaction was filtered and the filtrate was concentrated under reduced pressure to afford methyl 2-methylpiperidine-4-carboxylate as a light yellow oil (2.00 g, 96%), which was used in the next step directly without further purification: LCMS (ESI) calc'd for C₈H₁₅NO₂ [M + H]⁺: 158, found 158; ¹H NMR (400 MHz, CD₃OD) δ 3.51-3.43 (m, 1H), 3.37 (s, 3H), 3.31-3.24 (m, 1H), 3.12-3.02 (m, 1H), 2.77 (tt, *J =* 12.4, 3.8 Hz, 1H), 2.30-2.16 (m, 2H), 1.77 (qd, *J* = 13.8, 4.3 Hz, 1H), 1.63-1.49 (m, 1H), 1.37 (d, *J =* 6.5 Hz, 3H).

### Step b:

To a stirred mixture of methyl 2-methylpiperidine-4-carboxylate (2.00 g, 12.72 mmol) and Et₃N (2.57 g, 25.40 mmol) in DCM (10 mL) was added Boc₂O (4.16 g, 19.06 mmol) at room temperature. The reaction was stirred at room temperature for 1 h. The reaction was diluted with EA (50 mL) and water (50 mL). The aqueous solution was extracted with EA (3 x 30 mL). The combined organic layers were washed with brine (2 x 30 mL) and dried over anhydrous Na₂SO₄. After filtration, the filtrate was concentrated under reduced pressure to afford 1-*tert*-butyl 4-methyl 2-methylpiperidine-1,4-dicarboxylate as a light yellow oil (3.3 g, crude), which was used in the next step directly without further purification. LCMS (ESI) calc'd for C₁₃H₂₃NO₄ [M + H - 56]⁺: 202 found 202; ¹H NMR (400 MHz, CDCl₃) δ 4.28-4.09 (m, 1H), 3.91-3.76 (m, 1H), 3.72 (s, 3H), 3.11 (td, *J =* 13.1, 4.1 Hz, 1H), 2.76-2.51 (m, 1H), 2.10-1.86 (m, 3H), 1.85-1.69 (m, 1H), 1.47 (s, 9H), 1.09 (d, *J =* 6.5 Hz, 3H).

### Step c:

To a stirred solution of 1-*tert*-butyl 4-methyl 2-methylpiperidine-1,4-dicarboxylate (3.30 g, 12.82 mmol) in MeOH (10 mL) was added a solution of NaOH (1.03 g, 25.75 mmol) in water (2 mL) at room temperature. The reaction solution was stirred at 40 °C for 1 h. The reaction was diluted with water (50 mL). The aqueous solution was acidified with citric acid to pH 3, and then extracted with EA (3 x 50 mL). The combined organic layers were washed with brine (2 x 30 mL) and dried over anhydrous Na₂SO₄. After filtration, the filtrate was concentrated under reduced pressure. The residue was purified by reverse phase chromatography, eluted with 23% ACN in water (plus 0.05% TFA) to afford 1-[(*tert-*butoxy)carbonyl]-2-methylpiperidine-4-carboxylic acid as a light yellow semi-solid (2.20 g, 71%): LCMS (ESI) calc'd for C₁₂H₂₁NO₄ [M + H -56]⁺: 188, found 188.

### Step d:

To a stirred solution of 1-[(*tert*-butoxy)carbonyl]-2-methylpiperidine-4-carboxylic acid (1.80 g, 7.40 mmol), HOBt (1.50 g, 11.10 mmol) and EDCI (1.80 g, 11.10 mmol) in DCM (10 mL) were added *N,O-*dimethylhydroxylamine hydrochloride (1.08 g, 11.10 mmol) and Et₃N (1.50 g, 14.80 mmol) at room temperature. The reaction solution was stirred at room temperature for 1 h. The reaction was diluted with water (50 mL). The aqueous solution was extracted with EA (3 x 50 mL). The combined organic layers were washed with brine (2 x 30 mL) and dried over anhydrous Na₂SO₄. After filtration, the filtrate was evaporated under reduced pressure. The residue was purified by silica gel column chromatography, eluted with PE/EA (4/1) to afford *tert-*butyl 4-[methoxy(methyl)carbamoyl]-2-methylpiperidine-1-carboxylate as a light yellow oil (1.50 g, 71%): LCMS (ESI) calc'd for C₁₄H₂₆N₂O₄ [M + H]⁺: 287 found 287; ¹H NMR (400 MHz, CDCl₃) δ 4.04-3.92 (m, 1H), 3.89-3.81 (m, 1H), 3.71 (s, 3H), 3.21 (s, 3H), 3.20-3.10 (m, 1H), 2.88 (d, *J =* 10.4 Hz, 1H), 2.01-1.90 (m, 1H), 1.90-1.76 (m, 2H), 1.73-1.60 (m, 1H), 1.49 (s, 9H), 1.19 (d, *J* = 6.4 Hz, 3H).

### Example 43. Intermediate 43a (tert-butyl 4-[4,5-dichloro-2-(prop-2-en-1-yloxy)benzoyl]-2-methylpiperidine-1-carboxylate containing cis isomer, racemic); Intermediate 43b (tert-butyl 4-[4,5-dichloro-2-(prop-2-en-1-yloxy)benzoyl]-2-methylpiperidine-1-carboxylate containing trans isomer, racemic)

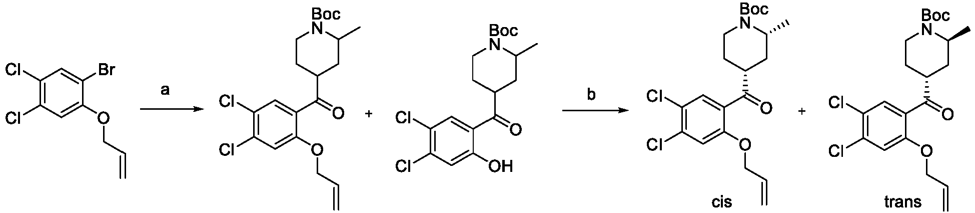

### Step a:

To a stirred solution of 1-bromo-4,5-dichloro-2-(prop-2-en-1-yloxy)benzene (2.76 g, 9.78 mmol) in THF (10 mL) was added *i*-PrMgCl (4.86 mL, 9.72 mmol, 2 M solution in THF) dropwise at 0 °C under nitrogen atmosphere. The reaction was stirred at 0 °C for 30 min. Then *tert*-butyl 4-[methoxy(methyl)carbamoyl]-2-methylpiperidine-1-carboxylate (1.40 g, 4.89 mmol) was added into the reaction. The reaction mixture was stirred at 0 °C for 1 h under nitrogen atmosphere. The resulting mixture was quenched with saturated aq. NH₄Cl (5 mL) and diluted with water (30 mL). The aqueous solution was extracted with EA (3 x 30 mL). The combined organic layers were washed with brine (2 x 30 mL) and dried over anhydrous Na₂SO₄. After filtration, the filtrate was concentrated under reduced pressure. The residue was purified by silica gel column chromatography, eluted with PE/EA (4/1) to afford *tert*-butyl 4-[4,5-dichloro-2-(prop-2-en-1-yloxy)benzoyl]-2-methylpiperidine-1-carboxylate as a light yellow semi-solid (0.50 g, 24%): LCMS (ESI) calc'd for C₂₁H₂₇Cl₂NO₄ [M + H -56]⁺: 372, 374 (3 : 2), found 372, 374 (3 : 2); ¹H NMR (400 MHz, CDCl₃) δ 7.70 (d, *J =* 10.3 Hz, 1H), 7.07 (s, 1H), 6.17-5.98 (m, 1H), 5.56-5.28 (m, 2H), 4.69-4.59 (m, 2H), 4.23-4.02 (m, 1H), 3.93-3.78 (m, 1H), 3.56-3.39 (m, 1H), 3.17-3.02 (m, 1H), 2.06-1.81 (m, 1H), 1.81-1.57 (m, 2H), 1.57-1.43 (m, 10H), 1.09 (d, *J =* 6.6 Hz, 3H). And *tert-*butyl 4-(4,5-dichloro-2-hydroxybenzoyl)-2-methylpiperidine-1-carboxylate as a light yellow oil (1.00 g, 53%): LCMS (ESI) calc'd for C₁₈H₂₃Cl₂NO₄ [M + H - 56]⁺: 332, 334 (3 : 2), found 332, 334 (3 : 2); ¹H NMR (400 MHz, CDCl₃) δ 12.30 (s, 1H), 7.79 (s, 1H), 7.17 (s, 1H), 4.21-4.02 (m, 1H), 3.96 (dd, *J =* 14.0, 6.9 Hz, 1H), 3.50-3.37 (m, 1H), 3.25-3.06 (m, 1H), 2.08-1.96 (m, 2H), 1.96-1.81 (m, 1H), 1.81-1.67 (m, 1H), 1.53 (s, 9H), 1.20 (d, *J =* 6.4 Hz, 3H).

To a stirred mixture of *tert*-butyl 4-(4,5-dichloro-2-hydroxybenzoyl)-2-methylpiperidine-1-carboxylate (1.00 g, 2.56 mmol) and K₂CO₃ (0.71 g, 5.15 mmol) in DMF (5 mL) was added 3-bromoprop-1-ene (0.47 g, 3.86 mmol) at room temperature. The resulting mixture was stirred for 1 h at room temperature. The resulting mixture was diluted with EA (30 mL) and water (30 mL) and the aqueous solution was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (10/1) to afford *tert*-butyl 4-[4,5-dichloro-2-(prop-2-en-1-yloxy)benzoyl]-2-methylpiperidine-1-carboxylate as a light yellow oil (1.00 g, 90%). The total amount of *tert*-butyl 4-[4,5-dichloro-2-(prop-2-en-1-yloxy)benzoyl]-2-methylpiperidine-1-carboxylate was 1.50 g (72%).

### Step b:

*tert-*Butyl 4-[4,5-dichloro-2-(prop-2-en-1-yloxy)benzoyl]-2-methylpiperidine-1-carboxylate (1.50 g, 3.51 mmol) was separated under SFC with following condtions: Column: CHIRALPAK IF, 2 x 25 cm, 5 µm; Mobile Phase A: CO₂: 75%, Mobile Phase B: MeOH: 25%; Flow rate: 40 mL/min; Detector: UV: 220/254 nm; Retention time: RT₁: 3.29 min; RT₂: 3.85 min.

The first peak at 3.29 min was obtained from which *tert*-butyl 4-[4,5-dichloro-2-(prop-2-en-1-yloxy)benzoyl]-2-methylpiperidine-1-carboxylate containing two cis enantiomers was isolated as a light yellow semi-solid (0.50 g, 45%): LCMS (ESI) calc'd for C₂₁H₂₇Cl₂NO₄ [M + H -56]⁺: 372, 374 (3 : 2), found 372, 374 (3 : 2); and the second peak at 3.85 min was obtained from which *tert-*butyl 4-[4,5-dichloro-2-(prop-2-en-1-yloxy)benzoyl]-2-methylpiperidine-1-carboxylate containing two trans enantiomers was isoalted as a light yellow semi-solid (0.40 g, 36%): LCMS (ESI) calc'd for C₂₁H₂₇Cl₂NO₄ [M + H -56]⁺: 372, 374 (3 : 2), found 372, 374 (3 : 2).

### Example 44. Intermediate 44 ((2,3-dichloro-6-methoxyphenyl)(piperidin-4-yl)methanol trifluoroacetic acid)

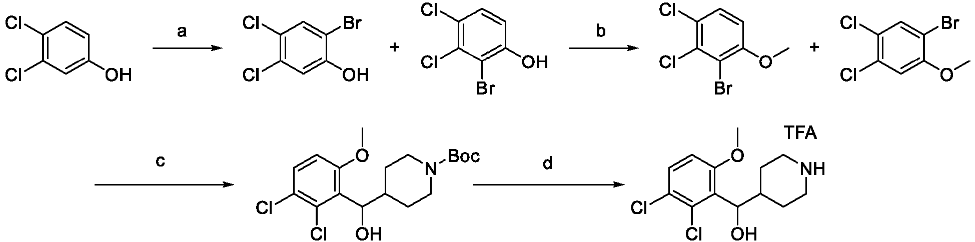

### Step a:

To a stirred solution of 3,4-dichlorophenol (100.00 g, 613.49 mmol) in DCM (1000 mL) was added Br₂ (98.04 g, 613.49 mmol) dropwise at 0 °C under nitrogen atmosphere. The reaction solution was stirred for 16 h at room temperature under nitrogen atmosphere. The reaction was quenched with saturated aq. Na₂S₂O₃ (500 mL) at 0 °C. The resulting mixture was extracted with EA (6 x 400 mL). The combined organic layers were washed with brine (2 x 400 mL) and dried over anhydrous Na₂SO₄. After filtration, the filtrate was concentrated under reduced pressure to afford a mixture of 2-bromo-4,5-dichlorophenol and 2-bromo-3,4-dichlorophenol (100 g, crude) as a yellow oil. The crude product was used in the next step directly without further purification.

### Step b:

To a crude mixture of 2-bromo-4,5-dichlorophenol and 2-bromo-3,4-dichlorophenol (32 g, 125.04 mmol, 1 equiv) and K₂CO₃ (54.9 g, 396.87 mmol, 3 equiv) in MeCN (210 mL) was added MeI (16.5 mL, 116.05 mmol, 2 equiv) dropwise at 0 °C. The reaction mixture was stirred at 50 °C for 4 h. The reaction mixture was filtered and concentrated. The residue was purified by silica gel column chromatography, eluted with PE to afford 2-bromo-3,4-dichloro-1-methoxybenzene (8.7 g, 25.7%) as a white solid: ¹H NMR (300 MHz, CDCl₃) δ 7.40 (dd, *J =* 9.0, 1.1 Hz, 1H), 6.79 (d, *J =* 8.9 Hz, 1H), 3.92 (s, 3H), and 1-bromo-4,5-dichloro-2-methoxybenzene (24.3 g, 71.77%) as a white solid: ¹H NMR (300 MHz, CDCl₃) δ 7.64 (s, 1H), 6.99 (s, 1H), 3.91 (s, 3H).

### Step c:

To a solution of 2-bromo-3,4-dichloro-1-methoxybenzene (0.80 g, 3.13 mmol) in THF (8 mL) was added *i*-PrMgCl (2.0 mL, 19.76 mmol, 2 M in THF) dropwise at 0 °C under nitrogen atmosphere. The reaction solution was stirred at 0 °C for 0.5 h. Then a solution of *tert-*butyl 4-formylpiperidine-1-carboxylate (0.67 g, 3.13 mmol) in THF (2 mL) was added dropwise at 0 °C under nitrogen atmosphere. After stirring at 0 °C for additional 0.5 h, the reaction mixture was allowed to warm to room temperature and stirred for additional 0.5 h under nitrogen atmosphere. The reaction was quenched with saturated aq. NH₄Cl (40 mL) and extracted with EA (2 x 40 mL). The organic layers were combined, dried over anhydrous Na₂SO₄. After filtration, the filtrate was concentrated under reduced pressure. The residue was purified with silica gel column chromatography, eluted with PE/EA (3/2) to afford *tert*-butyl 4-[(2,3-dichloro-6-methoxyphenyl)(hydroxy) methyl] piperidine-1-carboxylate as an off-white foam (1.00 g, 82%): LCMS (ESI) calc'd for C₁₈H₂₅Cl₂NO₄ [M + Na]⁺: 412, 414 (3 : 2), found 412, 414 (3 : 2); ¹H NMR (400 MHz, CDCl₃) δ 7.38 (d, *J =* 8.9 Hz, 1H), 6.83 (d, *J =* 8.9 Hz, 1H), 4.98 (d, *J* = 8.9 Hz, 1H), 4.21 (d, *J* = 13.5 Hz, 1H), 4.07 (d, *J* = 13.5 Hz, 1H), 3.92 (s, 3H), 2.70 (td, *J =* 12.9, 2.8 Hz, 1H), 2.59 (td, *J =* 12.8, 3.0 Hz, 1H), 2.17-1.97 (m, 2H), 1.48 (s, 9H), 1.42-1.24 (m, 3H).

### Step d:

To a solution of tert-butyl 4-[(2,3-dichloro-6-methoxyphenyl)(hydroxy) methyl] piperidine-1-carboxylate (0.50 g, 1.28 mmol) in DCM (5 mL) was added TFA (2 mL) at room temperature. The reaction solution was stirred at room temperature for 1 h and concentrated to afford (2,3-dichloro-6-methoxyphenyl)(piperidin-4-yl) methanol trifluoroacetic acid as a colorless oil (0.60 g, crude): LCMS (ESI) calc'd for C₁₃H₁₇Cl₂NO₂ [M + H]⁺: 290, 292 (3 : 2), found 290, 292 (3 : 2).

### Example 45. (which is a reference example) Intermediate 45 ((S)-N-((R)-(2-(allyloxy)-4-chloro-5-methylphenyl)(piperidin-4-yl)methyl)-2-methylpropane-2-sulfinamide)

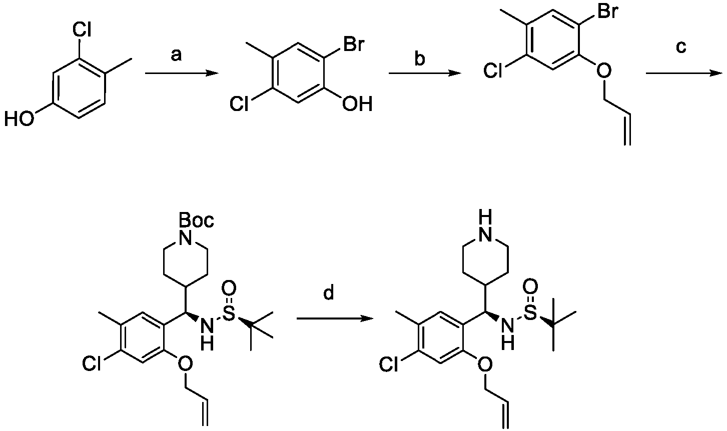

### Step a:

To a stirred solution of 3-chloro-4-methylphenol (0.50 g, 3.51 mmol) in HOAc (5 mL) was added Br₂ (0.56 g, 3.51 mmol) dropwise at room temperature under nitrogen atmosphere. The reaction solution was stirred at room temperature for 3 h. The reaction solution was quenched with saturated aq. Na₂SO₃ (50 mL) and extracted with EA (2 x 30 mL). The combined organic layers were washed with saturated aq. NaHCO₃ (2 x 30 mL), dried over anhydrous Na₂SO₄. After filtration, the filtrate was concentrated under reduced pressure. The residue was purified with silica gel column chromatography, eluted with PE/EA (5/1) to afford 2-bromo-5-chloro-4-methylphenol as an off-white solid (0.70 g, 90%): ¹H NMR (400 MHz, CDCl₃) δ 7.33 (s, 1H), 7.06 (s, 1H), 5.46 (s, 1H), 2.30 (s, 3H).

### Step b:

To a stirred mixture of 2-bromo-5-chloro-4-methylphenol (0.70 g, 3.16 mmol) and K₂CO₃ (0.87 g, 6.32 mmol) in DMF (5 mL) was added 3-bromoprop-1-ene (0.50 g, 4.11 mmol) at room temperature. The reaction mixture was allowed to warm to 40 °C and stirred for 3 h. The reaction mixture was diluted with water (50 mL) and extracted with EA (2 x 15 mL). The combined organic layers were washed with brine (3 x 10 mL), dried over anhydrous Na₂SO₄. After filtration, the filtrate was concentrated under reduced pressure. The residue was purified by silica gel column chromatography, eluted with PE to afford 1-bromo-4-chloro-5-methyl-2-(prop-2-en-1-yloxy)benzene as an off-white solid (0.45 g, 54%): ¹H NMR (400 MHz, CDCl₃) δ 7.42 (s, 1H), 6.90 (s, 1H), 6.12-6.02 (m, 1H), 5.58-5.40 (m, 1H), 5.40-5.30 (m, 1H), 4.59 (dt, *J =* 5.0, 1.6 Hz, 2H), 2.30 (s, 3H).

### Step c:

To a stirred solution of 1-bromo-4-chloro-5-methyl-2-(prop-2-en-1-yloxy)benzene (0.43 g, 1.64 mmol) in THF (4 mL) was added n-BuLi (0.76 mL, 1.90 mmol, 2.5 M in hexane) dropwise at -65 °C under nitrogen atmosphere. After stirring for 30 min, a solution of *tert-*butyl 4-[[(*S*)-2-methylpropane-2-sulfinyl]imino]methyl]piperidine-1-carboxylate (0.40 g, 1.26 mmol) in THF (2 mL) was added dropwise at -65 °C under nitrogen atmosphere. The resulting mixture was allowed to warm to room temperature and stirred for additional 1 h. The reaction was quenched with saturated aq. NH₄Cl (30 mL). The resulting mixture was extracted with EA (3 x 30 mL). The combined organic layers were washed with brine (2 x 30 mL), dried over anhydrous Na₂SO₄. After filtration, the filtrate was concentrated under reduced pressure. The residue was purified by silica gel column chromatography, eluted with PE/EA (1/5) to afford *tert*-butyl 4-((*R*)-(2-(allyloxy)-4-chloro-5-methylphenyl)(((*S*)-*tert-*butylsulfinyl)amino)methyl)piperidine-1-carboxylate as a light yellow oil (0.18 g, 30%): LCMS (ESI) calc'd for C₂₅H₃₉ClN₂O₄S [M + H]⁺: 499, 501 (3 : 1), found 499, 501 (3 : 1); ¹H NMR (400 MHz, CDCl₃) δ 6.96 (s, 1H), 6.87 (s, 1H), 6.09-5.99 (m, 1H), 5.50-5.27 (m, 2H), 4.61-4.45 (m, 2H), 4.42-3.82 (m, 4H), 2.66-2.57 (m, 2H), 2.29 (s, 3H), 1.92-1.91 (m, 1H), 1.66 (s, 3H), 1.45 (s, 9H), 1.13 (s, 9H).

### Step d:

To a stirred solution of *tert*-butyl 4-((R)-(2-(allyloxy)-4-chloro-5-methylphenyl)(((*S*)-*tert*-butylsulfinyl)amino)methyl)piperidine-1-carboxylate (0.18 g, 0.38 mmol) in DCM (4 mL) was added TFA (1 mL) at room temperature. The reaction solution was stirred for 1 h at room temperature. The resulting solution was neutralized with saturated aq. NaHCO₃ (20 mL) and extracted with DCM (3 x 20 mL). The combined organic layers were washed with brine (2 x 10 mL), dried over anhydrous Na₂SO₄. After filtration, the filtrate was concentrated under reduced pressure to afford (*S*)-*N*-((*R*)-(2-(allyloxy)-4-chloro-5-methylphenyl)(piperidin-4-yl)methyl)-2-methylpropane-2-sulfinamide as a light yellow oil (0.10 g, 70%): LCMS (ESI) calc'd for C₂₀H₃₁ClN₂O₂S [M + H]⁺: 399, 401 (3 : 1), found 399, 401 (3 : 1).

### Example 46. (which is a reference example) Intermediate 46 (1-bromo-4,5-dichloro-3-fluoro-2-(prop-2-en-1-yloxy)benzene)

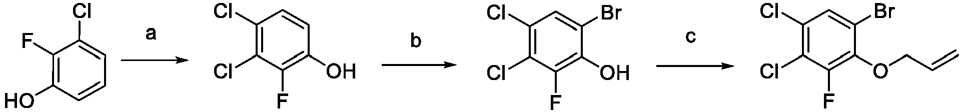

### Step a:

To a solution of 3-chloro-2-fluorophenol (1.50 g, 10.23 mmol) in MeCN (60 mL) and TFA (1.00 mL) was added NCS (1.37 g, 10.24 mmol) at room temperature under nitrogen atmosphere. The reaction mixture was stirred at room temperature for 16 h. The reaction mixture was concentrated under reduced pressure. The residue was purified by silica gel column chromatography, eluted with PE/EA (5/1) to afford 3,4-dichloro-2-fluorophenol as an off-white solid (1.60 g, 86%): ¹H NMR (400 MHz, DMSO-*d*₆) δ 10.64 (s, 1H), 7.27 (dd, *J =* 9.0, 2.1 Hz, 1H), 6.97 (t, *J* = 8.9 Hz, 1H).

### Step b:

To a stirred solution of 3,4-dichloro-2-fluorophenol (1.60 g, 8.84 mmol) in AcOH (20 mL) was added Br₂ (1.55 g, 9.72 mmol) dropwise at room temperature. The reaction solution was stirred at room temperature for 3 h. The reaction mixture was poured into water (100 mL). Then the reaction mixture was extracted with EA (2 x 50 mL). The organic phase were washed with saturated aq. Na₂SO₃ (2 x 50 mL), saturated aq. NaHCO₃ (2 x 50 mL) and brine (2 x 50 mL). Then solution was dried over anhydrous Na₂SO₄. After filtration, the filtrate was concentrated under reduced pressure. The residue was purified with silica gel column chromatography, eluted with PE/EA (5/1) to afford 6-bromo-3,4-dichloro-2-fluorophenol as off-white solid (1.80 g, 78%): ¹H NMR (400 MHz, CDCl₃) δ 7.45 (d, *J* = 2.3 Hz, 1H).

### Step c:

To a stirred mixture of 6-bromo-3,4-dichloro-2-fluorophenol (1.80 g, 6.97 mmol) and K₂CO₃ (2.00 g, 14.47 mmol) in DMF (20 mL) was added 3-bromoprop-1-ene (1.26 g, 10.39 mmol) at room temperature. Then the reaction was stirred at 40 °C for 3 h. The reaction mixture was diluted with water (80 mL) and extracted with EA (2 x 50 mL). The combined organic layers were washed with brine (3 x 30 mL) and dried over anhydrous Na₂SO₄. After filtration, the filtrate was evaporated under reduced pressure. The residue was purified by silica gel column chromatography, eluted with PE to afford 1-bromo-4,5-dichloro-3-fluoro-2-(prop-2-en-1-yloxy)benzene as a colorless liquid (1.70 g, 82%): ¹H NMR (400 MHz, CDCl₃) δ 7.50 (d, *J* = 2.3 Hz, 1H), 6.18-6.03 (m, 1H), 5.47-5.37 (m, 1H), 5.32 (dd, *J =* 10.2, 1.5 Hz, 1H), 4.70-4.60 (m, 2H); ¹⁹F NMR (376 MHz, CDCl₃) δ -121.32.

### Example 47. Intermediate 47 (lithio (R)-2-methoxy-3-(trityloxy)propanoate)

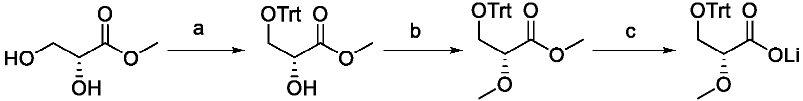

### Step a:

To a stirred solution of methyl (2*R*)-2,3-dihydroxypropanoate (0.50 g, 4.16 mmol) and (chlorodiphenylmethyl)benzene (3.50 g, 12.5 mmol) in DCM (4 mL) were added DMAP (30 mg, 0.25 mmol) and Et₃N (1.26 g, 12.5 mmol) in portions at room temperature. The resulting mixture was stirred for 72 h at room temperature under nitrogen atmosphere. The resulting mixture was diluted with water (20 mL) and extracted with EA (3 x 25 mL). The combined organic layers were washed with brine (3 x 5 mL), dried over anhydrous Na₂SO₄. After filtration, the filtrate was concentrated under reduced pressure. The residue was purified by silica gel column chromatography, eluted with PE/EA (8/1) to afford methyl (2R)-2-methoxy-3-(triphenylmethoxy)propanoate as an off-white solid (0.10 g, 6%). ¹H NMR (400 MHz, CDCl₃) δ 7.37-7.29 (m, 15H), 4.31 (t, *J* = 3.5 Hz, 1H), 3.91 (dd, *J* = 14.0, 3.6 Hz, 2H), 3.87 (s, 3H).

### Step b:

To a stirred solution of methyl (2R)-2-hydroxy-3-(triphenylmethoxy)propanoate (0.50 g, 1.38 mmol) and CH₃I (1 mL) in Et₂O (5 mL) was added Ag₂O (0.96 g, 4.14 mmol) in portions at room temperature. The resulting mixture was stirred for 16 h at room temperature under nitrogen atmosphere. The resulting mixture was diluted with water (20 mL) and extracted with DCM (3 x 20 mL). The combined organic layers were washed with brine (3 x 5 mL), dried over anhydrous Na₂SO₄. After filtration, the filtrate was concentrated under reduced pressure. The residue was purified by silica gel column chromatography, eluted with PE/EA (6/1) to afford methyl (2*R*)-2-methoxy-3-(triphenylmethoxy)propanoate as an off-white solid (0.40 g, 77%): LCMS (ESI) calc'd for C₂₄H₂₄O₄ [M + Na]⁺: 399, found 399; ¹H NMR (400 MHz, CDCl₃) δ 7.39-7.21 (m, 15H), 4.00-3.90 (m, 2H), 3.82 (s, 3H), 3.87-3.76 (m, 1H), 3.53 (s, 3H).

### Step c:

To a stirred mixture of methyl (2*R*)-2-methoxy-3-(triphenylmethoxy)propanoate (50 mg, 0.13 mmol) in MeOH (2 mL) was added a solution of LiOH·H₂O (9 mg, 0.4 mmol) in H₂O (1 mL) at room temperature. The resulting mixture was stirred for 16 h at room temperature and concentrated under reduced pressure to afford lithio (2*R*)-2-methoxy-3-(triphenylmethoxy)propanoate (50 mg, crude). The crude product was used in the next step directly without further purification: LCMS (ESI) calc'd for C₂₃H₂₂O₄ [M + Na]⁺: 385, found 385.

### Example 48. Intermediate 48 (lithio (2S)-2-methoxy-3-(triphenylmethoxy)propanoate)

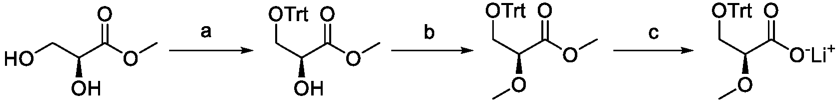

### Step a:

To a stirred solution of methyl (2*S*)-2,3-dihydroxypropanoate (1.00 g, 8.32 mmol) and (chlorodiphenylmethyl)benzene (2.50 g, 9.16 mmol) in DCM (10 mL) were added Et₃N (1.26 g, 12.5 mmol) and DMAP (61 mg, 0.50 mmol) at room temperature. The resulting mixture was stirred for 16 h at room temperature. The resulting mixture was diluted with water (15 mL) and extracted with DCM (3 x 20 mL). The combined organic layers were washed with brine (3 x 10 mL), dried over anhydrous Na₂SO₄. After filtration, the filtrate was concentrated under reduced pressure. The residue was purified by silica gel column chromatography, eluted with PE/EA (5/1) to afford methyl (2S)-2-hydroxy-3-(triphenylmethoxy)propanoate as an off-white solid (1.00 g, 33%): ¹H NMR (400 MHz, CDCl₃) δ 7.49-7.39 (m, 5H), 7.33 (dt, *J =* 6.9, 1.5 Hz, 5H), 7.33-7.22 (m, 5H), 4.32-4.27 (m, 1H), 3.80 (s, 3H), 3.54-3.45 (m, 1H), 3.38 (dd, *J* = 9.4, 3.4 Hz, 1H).

### Step b:

To a stirred solution of methyl (2S)-2-hydroxy-3-(triphenylmethoxy)propanoate (0.50 g, 1.38 mmol) and CH₃I (1 mL) in Et₂O (5 mL) was added Ag₂O (0.96 g, 4.14 mmol) in portions at room temperature under air atmosphere. The resulting mixture was stirred for 16 h at room temperature under nitrogen atmosphere. The resulting mixture was diluted with water (30 mL). The resulting mixture was extracted with DCM (3 x 20 mL). The combined organic layers were washed with brine (3 x 25 mL), dried over anhydrous Na₂SO₄. After filtration, the filtrate was concentrated under reduced pressure. The residue was purified by silica gel column chromatography, eluted with PE/EA (6/1) to afford methyl (2S)-2-methoxy-3-(triphenylmethoxy)propanoate as an off-white solid (0.40 g, 77%): LCMS (ESI) calc'd for C₂₄H₂₄O₄ [M + Na]⁺: 399, found 399; ¹H NMR (400 MHz, CDCl₃) δ 7.49-7.42 (m, 6H), 7.36-7.25 (m, 6H), 7.29-7.21 (m, 3H), 3.97 (dd, *J* = 5.4, 4.0 Hz, 1H), 3.78 (s, 3H), 3.47 (s, 3H), 3.45-3.36 (m, 2H).

### Step c:

To a stirred solution of methyl (2*S*)-2-methoxy-3-(triphenylmethoxy)propanoate (0.20 g, 0.53 mmol) in MeOH (3 mL) was added LiOH·H₂O (38 mg, 1.59 mmol) in H₂O (1 mL) at room temperature under air atmosphere. The resulting mixture was stirred for 16 h at room temperature. The resulting mixture was concentrated under reduced pressure to afford lithio (2S)-2-methoxy-3-(triphenylmethoxy)propanoate as an off-white solid (0.10 g, crude). The crude product was used in the next step directly without further purification: LCMS (ESI) calc'd for C₂₃H₂₂O₄ [M + Na]⁺: 385, found 385.

### Example 49. Intermediate 49 (tert-butyl 4-(1-(4,5-dichloro-2-methoxyphenyl)-2-oxoethyl)piperidine-1-carboxylate)

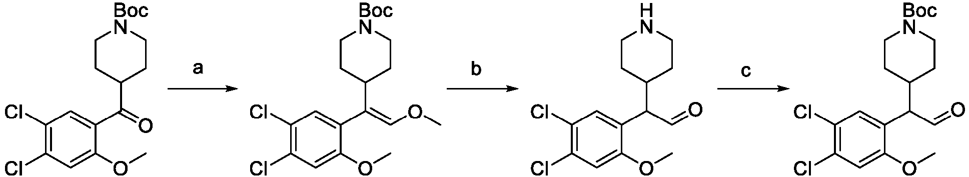

### Step a:

To a stirred mixture of (methoxymethyl)triphenylphosphanium chloride (12.36 g, 36.06 mmol) in THF (60 mL) was added *n*-BuLi (10.30 mL, 25.75 mmol, 2.5 M in hexane) dropwise at -78 °C under nitrogen atmosphere. The resulting mixture was stirred for 30 min at - 78 °C under nitrogen atmosphere. To the above mixture was added a solution of *tert*-butyl 4-(4,5-dichloro-2-methoxybenzoyl)piperidine-1-carboxylate (2.00 g, 5.15 mmol) in THF (5 mL) dropwise over 5 min at -78 °C. The resulting mixture was stirred for additional 2 h at -78 °C. The reaction was quenched with saturated aq. NH₄Cl (80 mL) at 0 °C. The resulting mixture was extracted with EA (3 x 50 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (1/1) to afford *tert*-butyl 4-[1-(4,5-dichloro-2-methoxyphenyl)-2-methoxyethenyl]piperidine-1-carboxylate as a light yellow oil (1.60 g, 59%): LCMS (ESI) calc'd for C₂₀H₂₇Cl₂NO₄ [M + H]⁺: 416, 418 (3 : 2), found 416, 418 (3 : 2); ¹H NMR (400 MHz, CDCl₃) δ 7.09 (d, *J =* 1.3 Hz, 1H), 6.91 (s, 1H), 5.87 (d, *J =* 1.3 Hz, 1H), 3.79 (s, 3H), 3.66 (s, 3H), 2.74-2.58 (m, 4H), 1.71-1.53 (m, 5H), 1.46 (d, *J =* 1.3 Hz, 9H).

### Step b:

To a stirred solution of *tert*-butyl 4-[(*E*)-1-(4,5-dichloro-2-methoxyphenyl)-2-methoxyethenyl]piperidine-1-carboxylate (1.80 g, 4.32 mmol) in 1,4-dioxane (10 mL) was added aq. HCl (6 *N,* 10 mL) dropwise at 0 °C. Then the resulting mixture was allowed to warm to room temperature and stirred for 3 h. The resulting mixture was concentrated under reduced pressure. The crude resulting mixture was used in the next step directly without further purification: LCMS (ESI) calc'd for C₁₄H₁₇Cl₂NO₂ [M + H]⁺: 302, 304 (3 : 2), found 302, 304 (3 : 2)

### Step c:

To a stirred mixture of 2-(4,5-dichloro-2-methoxyphenyl)-2-(piperidin-4-yl)acetaldehyde (1.50 g, 4.96 mmol) in THF (15 mL) and saturated aq. NaHCO₃ (20 mL) was added Boc₂O (1.62 g, 7.445 mmol) dropwise at 0 °C. The reaction mixture was stirred for 1 h at room temperature. The resulting mixture was extracted with EA (3 x 30 mL). The combined organic layers were washed with brine (2 x 10 mL), dried over anhydrous Na₂SO₄. After filtration, the filtrate was concentrated under reduced pressure to afford *tert*-butyl 4-[1-(4,5-dichloro-2-methoxyphenyl)-2-oxoethyl]piperidine-1-carboxylate as a light yellow oil (1.30 g, 65%): LCMS (ESI) calc'd for C₁₉H₂₅Cl₂NO₄ [M + H]⁺: 402, 404 (3 : 2), found 402, 404 (3 : 2); ¹H NMR (400 MHz, CDCl₃) δ 9.68 (d, *J =* 1.6 Hz, 1H), 7.13 (s, 1H), 7.00 (s, 1H), 4.04 (d, *J =* 13.6 Hz, 1H), 3.83 (s, 3H), 3.67 (dd, *J =* 8.9, 1.7 Hz, 1H), 2.82-2.71 (m, 1H), 2.66 (t, *J =* 12.6 Hz, 1H), 2.36-2.21 (m, 1H), 1.89 (dt, *J =* 13.3, 2.9 Hz, 1H), 1.67 (s, 1H), 1.45 (s, 9H), 1.41 (s, 1H), 1.26-1.16 (m, 1H), 1.10-0.95 (m, 1H).

### Example 50. Intermediate 50 (tert-butyl 4-[(4,5-dichloro-2-[[2-(trimethylsilyl)ethoxy]methoxy]phenyl)[(2-methylpropane-2-sulfinyl)amino]methyl]-2,2-dimethylpiperidine-1-carboxylate)

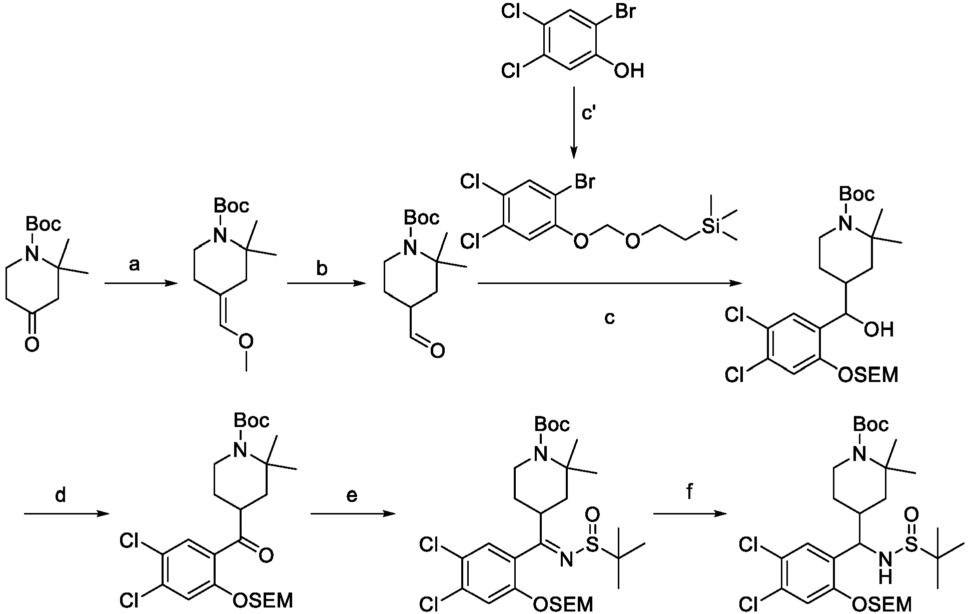

### Step a:

To a solution of (methoxymethyl) triphenylphosphanium chloride (1508 mg, 4.40 mmol) in THF (7 mL) was added *n*-BuLi (2.5 mL, 38.46 mmol, 2.5 M in hexane) dropwise at - 10 °C under nitrogen atmosphere. The solution was stirred at 0 °C for 1 h. Then a solution of *tert-*butyl 2, 2-dimethyl-4-oxopiperidine-1-carboxylate (1 g, 4.40 mmol) in THF (5 mL) was added dropwise at -20 °C under nitrogen atmosphere. The mixture was stirred at room temperature for 15 h. The reaction mixture was quenched with saturated aq. NH₄Cl (30 mL) and extracted with EA (2 x 20 mL). The organic phase was dried over anhydrous Na₂SO₄, filtered and concentrated. The residue was purified by silica gel column chromatography, eluted with PE/EA (1/1) to afford *tert*-butyl 4 (methoxymethylidene)-2,2-dimethylpiperidine-1-carboxylate as a colorless oil (0.80 g, 71%): LCMS (ESI) calc'd for C₁₄H₂₅NO₃ [M + H]⁺: 256, found 256; ¹H NMR (300 MHz, Methanol) δ 5.97 (d, *J =* 24.0 Hz, 1H), 3.62-3.45 (m, 5H), 2.37-2.27 (m, 2H), 2.26-2.20 (m, 1H), 2.18 (s, 1H), 1.46 (d, *J* = 1.7 Hz, 9H), 1.38 (d, *J* = 2.7 Hz, 6H).

### Step b:

To a solution of *tert*-butyl 4-(methoxymethylidene)-2,2-dimethylpiperidine-1-carboxylate (0.80 g, 3.13 mmol) in THF (10 mL) was added aq. HCl (4 *N,* 5 mL) at room temperature. The reaction mixture was stirred at 50 °C for 4 h. Then the reaction mixture was basified to pH 9 with saturated aq. NaHCO₃, then Boc₂O (821 mg, 3.76 mmol) was added. The mixture was stirred at room temperature for 1 h. The resulting mixture was extracted with EA (2 x 15 mL). The organic phase was dried over anhydrous Na₂SO₄, filtered and concentrated. The residue was purified by silica gel column chromatography, eluted with PE/EA (2/1) to afford *tert*-butyl 4-formyl-2,2-dimethylpiperidine-1-carboxylate as colorless oil (360 mg, 48%): LCMS (ESI) calc'd for C₁₃H₂₃NO₃ [M + H]⁺: 242, found 242.

### Step c':

To a stirred solution of 2-bromo-4,5-dichlorophenol (31.00 g, 128.15 mmol) and [2-(chloromethoxy)ethyl]trimethylsilane (32.00 g, 192.23 mmol) in DCM (100 mL) was added DIEA (49.70 g, 384.46 mmol) at room temperature. The resulting mixture was stirred at room temperature for 5 h. The reaction was quenched with water (200 mL). The resulting mixture was extracted with DCM (3 x 400 mL). The combined organic layers were washed with brine (3 x 200 mL) and dried over Na₂SO₄. After filtration, the filtrate was concentrated under reduced pressure. The residue was purified by silica gel column chromatography, eluted with PE/EA (50/1) to afford [2-(2-bromo-4,5-dichlorophenoxymethoxy)ethyl]trimethylsilane (44.00 g, 83%) as a light yellow oil: 1H NMR (300 MHz, DMSO-d6) δ 7.86 (s, 1H), 7.46 (s, 1H), 5.39 (s, 2H), 3.74 (t, J = 6.0 Hz, 2H), 0.79 (t, J = 6.0 Hz, 2H), -0.05 (s, 9H).

### Step c:

To a solution of [2-(2-bromo-4,5-dichlorophenoxymethoxy) ethyl] trimethylsilane (product of Step c') (0.69 g, 1.86 mol) in THF (7 mL) was added dropwise *i*-PrMgCl (0.2 mL, 1.61 mmol, 2 M in THF) at -10 °C under nitrogen atmosphere. Then reaction mixture was stirred at -10 °C for 0.5 h under nitrogen atmosphere. A solution of *tert*-butyl 4-formyl-2, 2-dimethylpiperidine-1-carboxylate (300 mg, 1.24 mmol) in THF (3 mL) was added dropwise to resulting solution at -10 °C under nitrogen atmosphere. The reaction mixture was allowed to warm to room temperature and stirred for 2 h under nitrogen atmosphere. The resulting mixture was quenched with saturated aq. NH₄Cl (40 mL). The resulting mixture was extracted with EA (3 x 10 mL). The organic phases were combined, dried over anhydrous Na₂SO₄. After filtration, the filtrate was concentrated under reduced pressure. The residue was purified by silica gel column chromatography, eluted with PE/EA (9/1) to afford *tert*-butyl 4-[(4,5-dichloro-2-[[2-(trimethylsilyl)ethoxy]methoxy]phenyl)(hydroxy)methyl]-2,2-dimethylpiperidine-1-carboxylate as a colorless oil (0.20 g, 30%): LCMS (ESI) calc'd for C₂₅H₄₁Cl₂NO₅Si [M + H]⁺: 534, 536 (3 : 2), found 534, 536 (3 : 2); ¹H NMR (300 MHz, CDCl₃) δ 7.67 (s, 1H), 7.41 (s, 1H), 5.30 (s, 2H), 3.83-3.72 (m, 2H), 3.71-3.50 (m, 2H), 3.46-3.28 (m, 1H), 1.97-1.89 (m, 1H), 1.84-1.73 (m, 2H), 1.71-1.65 (m, 1H), 1.52 (d, *J* = 4.0 Hz, 6H), 1.49 (s, 9H), 1.40 (s, 2H), 0.04 (s, 9H).

### Step d:

To a solution of *tert-*butyl 4-[(4,5-dichloro-2-[[2-(trimethylsilyl)ethoxy]methoxy]phenyl)(hydroxy)methyl]-2,2-dimethylpiperidine-1-carboxylate (0.18 g, 0.34 mmol) in DCM (8 mL) was added Dess-Martin reagent (0.14 g, 0.34 mmol) in three portions at room temperature. Then the reaction mixture was stirred at room temperature for 2 h. The reaction was quenched with saturated aq. Na₂SO₃, and then extracted with DCM (2 x 10 mL). The organic phase was dried over anhydrous Na₂SO₄. After filtration, the filtrate was concentrated under reduced pressure to afford *tert*-butyl 4-[(4,5-dichloro-2-[[2-(trimethylsilyl)ethoxy]methoxy]phenyl)carbonyl]-2,2-dimethylpiperidine-1-carboxylate as a colorless oil (0.16 g, crude): LCMS (ESI) calc'd for C₂₅H₃₉Cl₂NO₅Si [M + H]⁺: 532, 534 (3 : 2), found 532, 534 (3 : 2).

### Step e:

To a solution of *tert*-butyl 4-(4,5-dichloro-2-[[2-(trimethylsilyl)ethoxy]methoxy]benzoyl)-2,2-dimethylpiperidine-1-carboxylate (0.16 g, crude) in THF (10 mL) was added 2-methylpropane-2-sulfinamide (44 mg, 360 mmol) and Ti(OEt)₄ (0.90 g, 3.95 mmol) in one portion at room temperature. The reaction mixture was stirred at 70 °C for 15 h under nitrogen atmosphere. The reaction mixture was quenched with water (50 mL). The solid was formed and filtered. The filtrate was extracted with EA (2 x 40 mL). The organic layers were combined, dried over anhydrous Na₂SO₄. After filtration, the filtrate was concentrated under reduced pressure to afford *tert*-butyl 4-(4,5-dichloro-2-[[2-(trimethylsilyl)ethoxy]methoxy]phenyl)[(2-methylpropane-2-sulfinyl)imino]methyl]-2,2-dimethylpiperidine-1-carboxylate as a colorless oil (0.20 g, crude): LCMS (ESI) calc'd for C₂₉H₄₈Cl₂N₂O₅SSi [M + Na]⁺: 657, 659 (3 : 2), found 657, 659 (3 : 2).

### Step f:

To a solution of *tert*-butyl 4-[(1*E*)-(4,5-dichloro-2-[[2-(trimethylsilyl)ethoxy]methoxy]phenyl)[(2-methylpropane-2-sulfinyl)imino]methyl]-2,2-dimethylpiperidine-1-carboxylate (0.20 g, 0.31 mmol) in MeOH (5 mL) was added NaBH₄ (58 mg, 1.54 mmol) in 5 portions at room temperature under nitrogen atmosphere. The reaction mixture was stirred at room temperature for 2 h under nitrogen atmosphere. The reaction mixture was quenched with saturated aq. NH₄Cl (10 mL). The resulting mixture was concentrated under reduced pressure. The residue was purified with Prep-TLC, eluted with PE/EA (1/1) to afford *tert*-butyl 4-[(4,5-dichloro-2-[[2-(trimethylsilyl)ethoxy]methoxy] phenyl)[(2-methylpropane-2-sulfinyl)amino]methyl]-2,2-dimethylpiperidine-1-carboxylate as a colorless oil (75 mg, 37%): LCMS (ESI) calc'd for C₂₉H₅₀Cl₂N₂O₅SSi [M + Na]⁺: 659, 661 (3 : 2), found 659, 661 (3 : 2).

### Example 51. Intermediate 51 ((S)-N-((R)-(6-(allyloxy)-2,3-dichlorophenyl)(piperidin-4-yl)methyl)-2-methylpropane-2-sulfinamide)

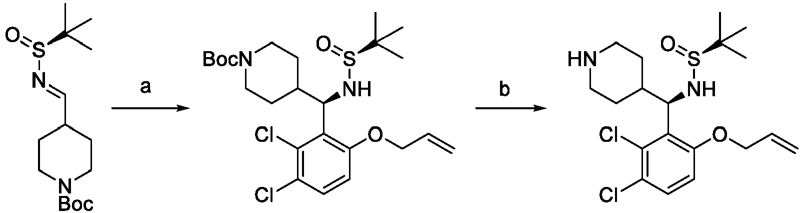

### Step a:

To a stirred solution of 1,2-dichloro-3-iodo-4-(prop-2-en-1-yloxy)benzene (1.25 g, 3.80 mmol) in THF (10 mL) was added *n*-BuLi (1.2 mL, 3.80 mmol, 2.5 M in hexane) at -65 °C under nitrogen atmosphere. After stirring for 30 min at -65 °C under nitrogen atmosphere, a solution of *tert*-butyl 4-[[(*S*)-2-methylpropane-2-sulfinyl]imino]methyl]piperidine-1-carboxylate (0.80 g, 2.53 mmol) in THF (5 mL) was added dropwise at -65 °C under nitrogen atmosphere. After addition, the reaction mixture was stirred for additional 1 h at -65 °C under nitrogen atmosphere. The reaction was quenched with water (40 mL) at -65 °C and extracted with EA (3 x 30 mL). The combined organic layers were washed with brine (3 x 10 mL), dried over anhydrous Na₂SO₄. After filtration, the filtrate was concentrated under reduced pressure. The residue was purified by silica gel column chromatography, eluted with PE/EA (7/1) to afford *tert*-butyl 4-((*R*)-(6-(allyloxy)-2,3-dichlorophenyl)(((*S*)-*tert-*butylsulfinyl)amino)methyl)piperidine-1-carboxylate as a light yellow oil (0.80 g, 57%): LCMS (ESI) calc'd for C₂₄H₃₆Cl₂N₂O₄S [M + H]⁺: 519, 521 (3 : 2), found 519, 521 (3 : 2); ¹H NMR (400 MHz, CDCL₃) δ 7.34 (d, *J =* 8.8 Hz, 1H), 6.79 (dd, *J =* 9.0, 4.9 Hz, 1H), 6.11-5.94 (m, 1H), 5.49-5.28 (m, 2H), 4.79 (t, *J =* 9.7 Hz, 1H), 4.68-4.50 (m, 3H), 4.36-3.91 (m, 3H), 2.77-2.47 (m, 2H), 2.40-2.29 (m, 1H), 2.18-2.02 (m, 1H), 1.46 (s, 9H), 1.34-1.22 (m, 1H), 1.04 (s, 9H).

### Step b:

To a stirred solution of *tert*-butyl 4-[(*R*)-[2,3-dichloro-6-(prop-2-en-1-yloxy)phenyl]([[(*S*)-2-methylpropane-2-sulfinyl]amino])methyl]piperidine-1-carboxylate (0.80 g, 1.54 mmol) in DCM (6 mL) was added TFA (1.5 mL) at room temperature. The reaction solution was stirred for 1 h at room temperature. The resulting solution was diluted with water (6 mL) and was neutralized to pH 8 with saturated aq. NaHCO₃. The resulting mixture was extracted with EA (3 x 30 mL). The combined organic layers were washed with brine (3 x 30 mL), dried over anhydrous Na₂SO₄. After filtration, the filtrate was concentrated under reduced pressure to afford (*S*)-*N*-[(*R*)-[2,3-dichloro-6-(prop-2-en-1-yloxy)phenyl](piperidin-4-yl)methyl]-2-methylpropane-2-sulfinamide as a light yellow oil (0.60 g, 74%): LCMS (ESI) calc'd for C₁₉H₂₈Cl₂N₂O₂S [M + H]⁺: 419, 421 (3 : 2), found 419, 421 (3 : 2); ¹H NMR (400 MHz, CDCl₃) δ 7.36-7.31 (m, 1H), 6.81-6.76 (m, 1H), 6.11-5.95 (m, 1H), 5.49-5.34 (m, 2H), 4.81 (t, *J =* 10.0 Hz, 1H), 4.67-4.50 (m, 3H), 3.26 (d, *J =* 12.5 Hz, 1H), 3.08 (d, *J =* 12.4 Hz, 1H), 2.68-2.36 (m, 5H), 2.13 (t, *J =* 10.7 Hz, 1H), 1.04 (s, 9H).

### Example 52. Intermediate 52 (lithio 4-methyl-5-oxo-4,5-dihydropyrazine-2-carboxylate)

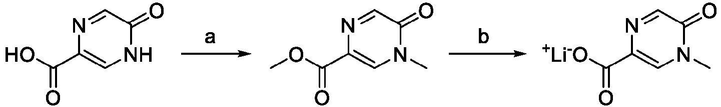

### Step a:

To a stirred mixture of 5-oxo-4*H*-pyrazine-2-carboxylic acid (3.00 g, 21.41 mmol) and K₂CO₃ (14.8 g, 1.07 mol) in DMF (30 mL) was added CH₃I (15.2 g, 1.08 mol) at room temperature. The resulting mixture was stirred for 16 h at 40 °C under nitrogen atmosphere. The resulting mixture was diluted with water (50 mL) and extracted with EA (5 x 60 mL). The combined organic layers were washed with brine (5 x 80 mL), dried over anhydrous Na₂SO₄. After filtration, the filtrate was concentrated under reduced pressure to afford methyl 4-methyl-5-oxopyrazine-2-carboxylate as a dark brown solid (2.50 g, 69%): LCMS (ESI) calc'd for C₇H₈N₂O₃ [M + H]⁺: 169, found 169; ¹H NMR (400 MHz, CD₃OD) δ 8.48 (s, 1H), 8.02 (s, 1H), 3.92 (s, 3H), 3.61 (s, 3H).

### Step b:

To a stirred mixture of methyl 4-methyl-5-oxopyrazine-2-carboxylate (0.60 g, 3.57 mmol) in MeOH (7 mL) was added a solution of LiOH (0.26 g, 10.71 mmol) in H₂O (1 mL) at room temperature. After stirring for 20 h at room temperature, the resulting mixture was concentrated under reduced pressure. The crude product was used in the next step directly without further purification. LCMS (ESI) calc'd for C₆H₆N₂O₃ [M + H]⁺: 155, found 155.

### Example 53. Intermediate 53 (1-bromo-4-methyl-2-(prop-2-en-1-yloxy)benzene)

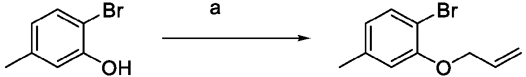

### Step a:

To a stirred solution of 2-bromo-5-methylphenol (2.00 g, 10.69 mmol) in DMF (20 mL) were added K₂CO₃ (2.96 g, 21.41 mmol) and 3-bromoprop-1-ene (1.94 g, 16.04 mmol) at room temperature. The resulting mixture was stirred for 1 h at room temperature. The reaction mixture was diluted with EA (50 mL) and water (50 mL). The aqueous solution was extracted with EA (3 x 30 mL). The combined organic layers were washed with brine (3 x 30 mL) and dried over anhydrous Na₂SO₄. After filtration, the filtrate was concentrated under reduced pressure. The residue was purified by silica gel column chromatography, eluted with PE/EA (10/1) to afford 1-bromo-4-methyl-2-(prop-2-en-1-yloxy)benzene as a light yellow oil (1.29 g, 53%): ¹H NMR (400 MHz, CD₃OD) δ 7.38 (d, *J =* 8.0 Hz, 1H), 6.86 (d, *J =* 1.9 Hz, 1H), 6.69 (dd, *J =* 7.9, 2.0 Hz, 1H), 6.16-6.02 (m, 1H), 5.40-5.30 (m, 1H), 5.28-5.20 (m, 1H), 4.60 (d, *J =* 5.0 Hz, 2H), 2.32 (s, 3H).

### Example 54. Intermediate 54 (tert-butyl 4-[methoxy(methyl)carbamoyl]-3-methylpiperidine-1-carboxylate)

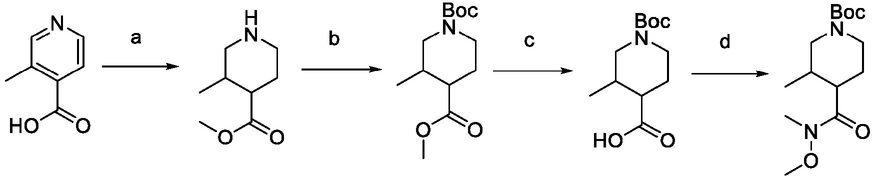

### Step a:

To a stirred mixture of 3-methylpyridine-4-carboxylic acid (2.00 g, 14.58 mmol) and HCl (6 *N,* 3 mL) in MeOH (15 mL) was added PtO₂ (0.33 g, 1.46 mmol) at room temperature. The resulting mixture was stirred for 12 h at 50 °C under H₂ (50 atm) atmosphere. The reaction mixture was filtered and the filtrate was concentrated under reduced pressure to afford methyl 3-methylpiperidine-4-carboxylate HCl as a yellow oil (2.30 g, 90%): LCMS (ESI) calc'd for C₈H₁₅NO₂ [M + H]⁺: 158, found 158; ¹H NMR (400 MHz, DMSO-*d*₆) δ 3.86-3.69 (m, 2H), 3.64 (s, 3H), 3.15-2.90 (m, 2H), 2.83 (q, *J =* 5.7 Hz, 1H), 2.35-2.23 (m, 1H), 1.91-1.81 (m, 2H), 0.93 (d, *J* = 7.2 Hz, 3H).

### Step b:

To a stirred solution of methyl 3-methylpiperidine-4-carboxylate (2.30 g, 14.63 mmol) and Et₃N (2.96 g, 29.26 mmol) in THF (15 mL) and MeOH (30 mL) was added Boc₂O (4.79 g, 21.95 mmol) at room temperature. The reaction solution was stirred for 2 h at room temperature. The resulting mixture was diluted with EA (50 mL) and water (30 mL). The aqueous solution was extracted with EA (3 x 20 mL). The combined organic layers were washed with brine (2 x 10 mL), dried over anhydrous Na₂SO₄. After filtration, the filtrate was concentrated under reduced pressure. The residue was purified by reverse phase chromatography, eluted with 20% ACN in water (plus 0.05% TFA) to afford 1-*tert*-butyl 4-methyl 3-methylpiperidine-1,4-dicarboxylate as a yellow oil (2.90 g, 77% overall two steps): LCMS (ESI) calc'd for C₁₃H₂₃NO₄ [M + H - 15]⁺: 243, found 243; ¹H NMR (400 MHz, CDCl₃) δ 4.02 (s, 1H), 3.80 (dd, *J =* 13.3, 1.5 Hz, 1H), 3.70 (s, 3H), 3.31-2.81 (m, 2H), 2.62 (dt, *J =* 10.5, 4.3 Hz, 1H), 2.27-2.15 (m, 1H), 1.92-1.77 (m, 1H), 1.75-1.58 (m, 1H), 1.47 (s, 9H), 0.90 (dd, *J =* 6.8, 2.5 Hz, 3H).

### Step c:

To a stirred solution of 1-*tert*-butyl 4-methyl 3-methylpiperidine-1,4-dicarboxylate (2.70 g, 10.49 mmol) in MeOH (20 mL) was added a solution of NaOH (0.83 g, 20.98 mmol) in H₂O (4 mL) at room temperature. The reaction mixture was stirred for 12 h at room temperature and was acidified to pH 3 with citric acid. The mixture was extracted with EA (3 x 50 mL). The combined organic layers were washed with brine (2 x 10 mL), dried over anhydrous Na₂SO₄. After filtration, the filtrate was concentrated under reduced pressure to afford 1-[(*tert-*butoxy)carbonyl]-3-methylpiperidine-4-carboxylic acid as a yellow oil (1.30 g, 63%): LCMS (ESI) calc'd for C₁₂H₂₁NO₄ [M +Na]⁺: 266, found 266; ¹H NMR (400 MHz, DMSO-*d*₆) δ 12.24 (s, 1H), 3.88 (s, 1H), 3.70 (dd, *J =* 13.1, 3.6 Hz, 1H), 3.09-2.65 (m, 2H), 2.56 (dt, *J =* 10.0, 4.5 Hz, 1H), 2.19-2.08 (m, 1H), 1.65-1.49 (m, 2H), 1.39 (s, 9H), 0.80 (d, *J =* 6.9 Hz, 3H).

### Step d:

To a stirred mixture of 1-[(*tert*-butoxy)carbonyl]-3-methylpiperidine-4-carboxylic acid (1.10 g, 4.52 mmol) and HOBt (0.91 g, 6.78 mmol) in DMF (10 mL) were added EDCI (1.30 g, 6.78 mmol), Et₃N (0.55 g, 5.43 mmol) and *N,O*-methoxy(methyl)amine (0.30 g, 4.97 mmol) at room temperature. The reaction solution was stirred for 12 h at room temperature. The resulting solution was quenched with water (50 mL) at room temperature and extracted with EA (2 x 50 mL). The combined organic layers were washed with brine (2 x 50 mL), dried over anhydrous Na₂SO₄. After filtration, the filtrate was concentrated under reduced pressure. The residue was purified by silica gel column chromatography, eluted with PE/EA (4/1) to afford *tert-*butyl 4-[methoxy(methyl)carbamoyl]-3-methylpiperidine-1-carboxylate as a colorless oil (1.00 g, 77%): LCMS (ESI) calc'd for C₁₄H₂₆N₂O₄ [M + H - 56]⁺: 231 found 231; ¹H NMR (400 MHz, CDCl₃) δ 4.17-3.89 (m, 2H), 3.86-3.76 (m, 1H), 3.73 (s, 3H), 3.29-3.13 (m, 4H), 3.13-2.94 (m, 2H), 2.24-2.09 (m, 1H), 2.06-1.87 (m, 1H), 1.48 (s, 9H), 0.93 (d, *J =* 7.0 Hz, 3H).

### Example 55. Intermediate 55 (tert-butyl (3S,4S)-rel-4-(2-(prop-2-en-1-yloxy)-4,5-dichlorobenzoyl)-3-methylpiperidine-1-carboxylate; tert-butyl (3R,4S)-rel-4-(2-(prop-2-en-1-yloxy)-4,5-dichlorobenzoyl)-3-methylpiperidine-1-carboxylate)

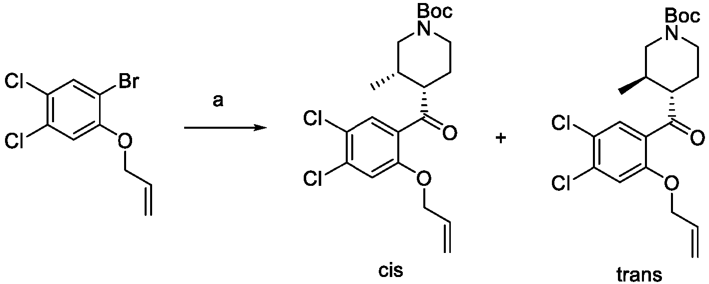

### Step a:

To a stirred solution of 1-bromo-4,5-dichloro-2-(prop-2-en-1-yloxy)benzene (2.12 g, 7.68 mmol) in THF (10 mL) was added *i*-PrMgCl (3.84 mL, 7.68 mmol, 2 M solution in THF) dropwise at 0 °C under nitrogen atmosphere. The reaction was stirred at 0 °C for 30 min. Then a solution of *tert*-butyl 4-[methoxy(methyl)carbamoyl]-3-methylpiperidine-1-carboxylate (1.10 g, 3.84 mmol) in THF (8 mL) was added dropwise into the solution. After addition, the resulting solution was stirred at 0 °C for additional 1 h. The reaction was quenched with saturated aq. NH₄Cl (5 mL). The reaction mixture was diluted with EA (30 mL) and water (30 mL), and then the aqueous solution was extracted with EA (3 x 30 mL). The combined organic layers were washed with brine (3 x 20 mL), dried over anhydrous Na₂SO₄. After filtration, the filtrate was concentrated under reduced pressure to afford a mixture of *tert*-butyl 4-(2-(allyloxy)-4,5-dichlorobenzoyl)-3-methylpiperidine-1-carboxylate and *tert*-butyl 4-(4,5-dichloro-2-hydroxybenzoyl)-3-methylpiperidine-1-carboxylate. The residue was dissolved in DMF (10 mL) and K₂CO₃ (0.88 g, 6.37 mmol) and allyl bromide (0.77 g, 6.37 mmol) was added at room temperature. The resulting mixture was stirred for 1 h at room temperature. The resulting mixture was diluted with EA (30 mL) and water (30 mL). The aqueous solution was extracted with EA (3 x 30 mL). The combined organic layers were washed with brine (6 x 20 mL), dried over anhydrous Na₂SO₄. After filtration, the filtrate was concentrated under reduced pressure. The residue was purified by silica gel column chromatography, eluted with PE/EA (10/1) to afford *tert*-butyl 4-[4,5-dichloro-2-(prop-2-en-1-yloxy)benzoyl]-3-methylpiperidine-1-carboxylate containing two *cis* enantiomers as a light yellow oil (0.90 g, 55%): LCMS (ESI) calc'd for C₂₁H₂₇Cl₂NO₄ [M + H - 56]⁺ 372, 374 (3 : 2), found 372, 374 (3 : 2); ¹H NMR (400 MHz, CDCl₃) δ 7.67 (s, 1H), 7.06 (s, 1H), 6.15-5.97 (m, 1H), 5.52-5.38 (m, 2H), 4.69-4.54 (m, 2H), 4.25-4.03 (m, 1H), 4.01-3.86 (m, 1H), 3.68-3.52 (m, 1H), 3.12-2.94 (m, 1H), 2.91-2.76 (m, 1H), 2.24-2.10 (m, 1H), 2.01-1.84 (m, 1H), 1.76-1.55 (m, 1H), 1.55-1.39 (m, 9H), 0.85 (d, *J =* 6.5 Hz, 3H).

*tert-*Butyl 4-[4,5-dichloro-2-(prop-2-en-1-yloxy)benzoyl]-3-methylpiperidine-1-carboxylate) containing two trans enantiomers was isolated as a light yellow oil (0.12 g, 7%): LCMS (ESI) calc'd for C₂₁H₂₇Cl₂NO₄ [M + Na]⁺: 450, 452 (3 : 2), found 450, 452 (3 : 2); ¹H NMR (400 MHz, CDCl₃) δ 7.68 (s, 1H), 7.07 (s, 1H), 6.11-5.93 (m, 1H), 5.44 (q, *J =* 11.2 Hz, 2H), 4.62 (d, *J =* 5.5 Hz, 2H), 4.19-4.10 (m, 1H), 3.92 (d, *J =* 13.0 Hz, 1H), 3.57 (d, *J =* 11.2 Hz, 1H), 3.01 (d, *J =* 13.1 Hz, 1H), 2.87-2.78 (m, 1H), 2.23-2.08 (m, 1H), 1.97-1.82 (m, 1H), 1.76-1.58 (m, 1H), 1.56-1.32 (m, 9H), 0.82-0.75 (m, 3H).

### Example 56. Intermediate 56 ((2S)-1-methyl-5-oxopyrrolidine-2-carboxylic acid)

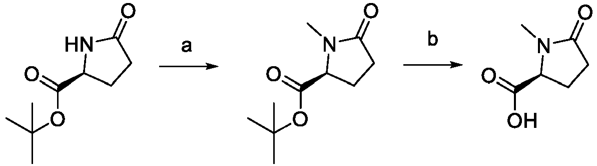

### Step a:

A stirred solution of *tert*-butyl (2*S*)-5-oxopyrrolidine-2-carboxylate (0.50 g, 2.70 mmol) in THF (5 mL) was added NaH (0.22 g, 5.40 mmol, 60% in mineral oil) at 0 °C under nitrogen atmosphere. The resulting mixture was stirred for 30 min at 0°C under nitrogen atmosphere. To the above mixture was added MeI (1.15 g, 8.10 mmol) dropwise at 0°C. The reaction mixture was stirred for 16 h at room temperature. The resulting mixture was quenched with water (40 mL) and extracted with EA (3 x 50 mL). The combined organic layers were washed with brine (3 x 20 mL) and dried over anhydrous Na₂SO₄. After filtration, the filtrate was concentrated under reduced pressure. The residue was purified by reverse phase chromatography, eluted with 50% ACN in water (plus 0.05% TFA) to afford *tert*-butyl (2*S*)-1-methyl-5-oxopyrrolidine-2-carboxylate as a light yellow oil (0.34 g, 63%): LCMS (ESI) calc'd for C₁₀H₁₇NO₃ [2M + H]⁺: 399, found 399; ¹H NMR (400 MHz, CDCl₃) δ 4.09-3.92 (m, 1H), 2.87 (s, 3H), 2.57-2.43 (m, 1H), 2.43-2.24 (m, 2H), 2.13-1.98 (m, 1H), 1.50 (s, 9H).

### Step b:

To a stirred solution of *tert*-butyl (2*S*)-1-methyl-5-oxopyrrolidine-2-carboxylate (50 mg, 0.25 mmol) in DCM (1 mL) was added TFA (1 mL) at room temperature. The reaction solution was stirred for 2 h at room temperature. The resulting solution was concentrated under reduced pressure to afford (2*S*)-1-methyl-5-oxopyrrolidine-2-carboxylic acid as a yellow oil (64 mg, crude), which was used in the next step directly without further purification: LCMS (ESI) calc'd for C₆H₉NO₃ [M + H]⁺: 144, found 144; ¹H NMR (400 MHz, CDCl₃) δ 4.35-4.12 (m, 1H), 2.96 (s, 3H), 2.75-2.36 (m, 3H), 2.31-2.10 (m, 1H).

### Example 57. (which is a reference example) Intermediate 57 (bromo-5-chloro-4-ethyl-2-(prop-2-en-1-yloxy)benzene)

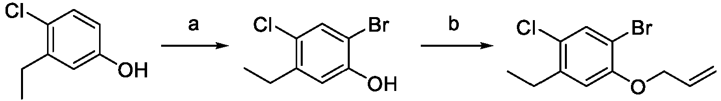

### Step a:

To a stirred solution of 4-chloro-3-ethylphenol (3.00 g, 19.15 mmol) in DCM (20 mL) was added Br₂ (3.67 g, 22.96 mmol) dropwise at room temperature. The resulting solution was stirred for 1 h at room temperature. The reaction was quenched with saturated aq. Na₂SO₃ (20 mL) at room temperature and extracted with EA (3 x 30 mL). The combined organic layers were washed with brine (3 x 20 mL) and dried over anhydrous Na₂SO₄. After filtration, the filtrate was concentrated under reduced pressure to afford 2-bromo-4-chloro-5-ethylphenol as a colorless oil (4.50 g, crude), which was used in the next step directly without further purification: ¹H NMR (400 MHz, CD₃OD) δ 7.42 (s, 1H), 6.83 (s, 1H), 2.65 (q, *J =* 7.5 Hz, 2H), 1.20 (t, *J =* 7.5 Hz, 3H).

### Step b:

To a stirred solution of 2-bromo-4-chloro-5-ethylphenol (4.50 g, 19.10 mmol) and K₂CO₃ (5.28 g, 38.20 mmol) in DMF (40 mL) was added 3-bromoprop-1-ene (3.47 g, 28.68 mmol) at room temperature. The resulting mixture was stirred for 1 h at room temperature. The reaction mixture was diluted with EA (50 mL) and water (50 mL). The aqueous solution was extracted with EA (3 x 30 mL). The combined organic layers were washed with brine (6 x 30 mL) and dried over anhydrous Na₂SO₄. After filtration, the filtrate was concentrated under reduced pressure. The residue was purified by silica gel column chromatography, eluted with PE/EA (10/1) to afford 1-bromo-5-chloro-4-ethyl-2-(prop-2-en-1-yloxy)benzene as a colorless oil (2.40 g, 45% overall two steps); ¹H NMR (400 MHz, CD₃OD) δ 7.52 (s, 1H), 6.96 (s, 1H), 6.16-5.91 (m, 1H), 5.53-5.38 (m, 1H), 5.35-5.21 (m, 1H), 4.73-4.48 (m, 2H), 2.72 (q, *J =* 7.5 Hz, 2H), 1.23 (t, *J =* 7.5 Hz, 3H).

### Example 58. Intermediate 58 (tert-butyl 5-[methoxy(methyl)carbamoyl]-2-azabicyclo[2.2.1]heptane-2-carboxylate)

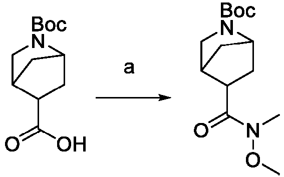

### Step a:

To a stirred solution of 2-[(*tert*-butoxy)carbonyl]-2-azabicyclo[*2.2.1*]heptane-5-carboxylic acid (0.50 g, 2.07 mmol) in DCM (5 mL) were added EDCI (0.60 g, 3.11 mmol) and HOBt (0.42 g, 3.11 mmol) at room temperature. After stirring for 10 min at room temperature, to the above mixture were added *N,O*-methoxy(methyl)amine hydrochloride (0.30 g, 3.11 mmol) and Et₃N (0.63 g, 6.22 mmol) at room temperature. After stirring for 5 h at room temperature, the resulting solution was diluted with water (30 mL) and extracted with DCM (3 x 30 mL). The combined organic layers were washed with brine (3 x 20 mL) and dried over anhydrous Na₂SO₄. After filtration, the filtrate was concentrated under reduced pressure. The residue was purified by reverse phase chromatography, eluted with 40% ACN in water (plus 0.05% TFA) to afford *tert*-butyl 5-[methoxy(methyl)carbamoyl]-2-azabicyclo[*2.2.1*]heptane-2-carboxylate as a yellow oil (0.31g, 52%): LCMS (ESI) calc'd for C₁₄H₂₄N₂O₄ [M + H - 56]⁺: 229, found 229; ¹H NMR (400 MHz, CDCl₃) δ 4.25 (s, 1H), 3.73 (s, 3H), 3.29 (dd, *J* = 9.9, 3.4 Hz, 1H), 3.22 (s, 3H), 3.17-2.98 (m, 1H), 2.98-2.81 (m, 1H), 2.75-2.67 (m, 1H), 2.03-1.84 (m, 2H), 1.81 (d, *J =* 10.1 Hz, 1H), 1.62 (d, *J =* 10.1 Hz, 1H), 1.49 (s, 9H).

### Example 59. Intermediate 59 (lithio 2-methyl-6-oxo-1H-pyridine-3-carboxylate)

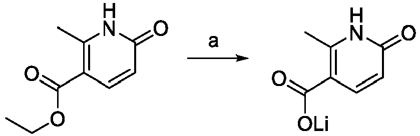

### Step a:

To a stirred solution of ethyl 2-methyl-6-oxo-1*H*-pyridine-3-carboxylate (0.10 g, 0.55 mmol) in MeOH (1 mL) was added a solution of LiOH·H₂O (46 mg, 1.10 mmol) in water (0.5 mL) at room temperature. The resulting mixture was stirred for 1 h at 40 °C. After cooling to room temperature, the resulting mixture was concentrated under reduced pressure to afford lithio 2-methyl-6-oxo-1*H*-pyridine-3-carboxylate as an off-white solid (0.15 g, crude), which was used in the next step directly without further purification: LCMS (ESI) calc'd for C₇H₇NO₃ [M + H]⁺: 154, found 154.

### Example 60. Intermediate 60 (lithio 1-[2-(oxan-2-yloxy)ethyl]-6-oxopyridine-3-carboxylate)

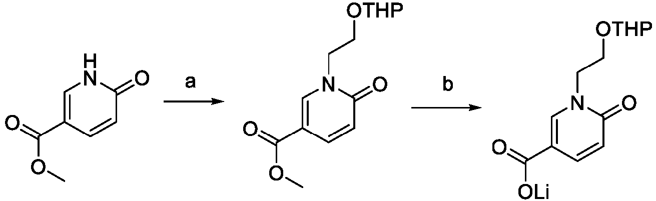

### Step a:

To a stirred solution of methyl 6-oxo-1*H*-pyridine-3-carboxylate (0.50 g, 3.26 mmol) in DMF (5 mL) was added NaH (0.26 g, 6.53 mmol, 60% in mineral oil) at 0 °C under nitrogen atmosphere. The reaction mixture was stirred at 0 °C for 10 min. Then 2-(2-chloroethoxy)oxane (1.61 g, 9.79 mmol) and NaI (97 mg, 0.65 mmol) were added into the mixture. After addition, the reaction mixture was allowed to warm to 70 °C and stirred for 5 h. After cooling to room temperature, the resulting mixture was quenched with water (30 mL) at 0 °C and extracted with EA (3 x 20 mL). The combined organic layers were washed with brine (6 x 20 mL) and dried over anhydrous Na₂SO₄. After filtration, the solution was concentrated under reduced pressure. The residue was purified by silica gel column chromatography, eluted with PE/EA (2/1) to afford methyl 1-[2-(oxan-2-yloxy)ethyl]-6-oxopyridine-3-carboxylate as a yellow oil (0.50 g, 54%): LCMS (ESI) calc'd for C₁₄H₁₉NO₅ [M + H]⁺: 282 found 282; ¹H NMR (400 MHz, CDCl₃) δ 8.83 (s, 1H), 8.18 (dt, *J =* 8.8, 2.1 Hz, 1H), 6.84 (d, *J =* 8.7 Hz, 1H), 4.81-4.49 (m, 3H), 4.17-3.93 (m, 1H), 3.93-3.77 (m, 4H), 3.68-3.45 (m, 2H), 1.95-1.46 (m, 6H).

### Step b:

To a stirred solution of methyl 1-[2-(oxan-2-yloxy)ethyl]-6-oxopyridine-3-carboxylate (0.20 g, 0.711 mmol) in MeOH (5 mL) was added a solution of LiOH·H₂O (59 mg, 1.42 mmol) in water (0.5 mL) at room temperature. The resulting mixture was stirred for 1 h at 40 °C. After cooling to room temperature, the resulting mixture was concentrated under reduced pressure to afford lithio 1-[2-(oxan-2-yloxy)ethyl]-6-oxopyridine-3-carboxylate (0.16 g, crude), which was used in the next step directly without further purification: LCMS (ESI) calc'd for C₁₃H₁₇NO₅ [M + Na]⁺: 290, found 290.

### Example 61. Intermediate 61 (lithio 3-[(tert-butoxycarbonyl)amino]-2-[[(tert-butoxycarbonyl)amino]methyl]propanoate)

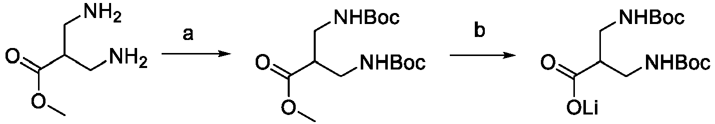

### Step a:

To a stirred solution of methyl 3-amino-2-(aminomethyl)propanoate dihydrochloride (0.50 g, 2.44 mmol) and Et₃N (0.74 g, 7.31 mmol) in DCM (5 mL) was added Boc₂O (1.17 g, 5.36 mmol) at room temperature. The reaction solution was stirred for 1 h at room temperature. The resulting solution was diluted with water (20 mL) and extracted with EA (3 x 30 mL). The combined organic layers were washed with brine (3 x 20 mL), dried over anhydrous Na₂SO₄. After filtration, the filtrate was concentrated under reduced pressure to afford methyl 3-[(*tert-*butoxycarbonyl)amino]-2-[[(*tert*-butoxycarbonyl)amino]methyl]propanoate as a yellow oil (1.00 g, crude), which was used in the next step directly without further purification: LCMS (ESI) calc'd for C₁₅H₂₈N₂O₆ [M + H]⁺: 333, found 333; ¹H NMR (400 MHz, CDCl₃) δ 3.73 (s, 3H), 3.62-3.51 (m, 2H), 3.23 (dt, *J =* 13.6, 5.7 Hz, 2H), 2.80-2.64 (m, 1H), 1.55 (s, 9H), 1.46 (s, 9H).

### Step b:

To a stirred solution of methyl 3-[(*tert*-butoxycarbonyl)amino]-2-[[(*tert-*butoxycarbonyl)amino]methyl]propanoate (1.00 g, 3.00 mmol) in MeOH (10 mL) was added a solution of LiOH·H₂O (0.25 g, 6.01 mmol) in water (1 mL) at room temperature. The resulting mixture was stirred for 1 h at 40 °C. After cooling to room temperature, the resulting mixture was concentrated under reduced pressure to afford lithio 3-[(*tert*-butoxycarbonyl)amino]-2-[[(*tert*-butoxycarbonyl)amino]methyl]propanoate as an off-white solid (1.00 g, crude), which was used in the next step directly without further purification: LCMS (ESI) calc'd for C₁₄H₂₆N₂O₆ [M + H]⁺: 319, found 319.

### Example 62. Intermediate 62 (1-acetylpyrrolidine-3-carboxylic acid)

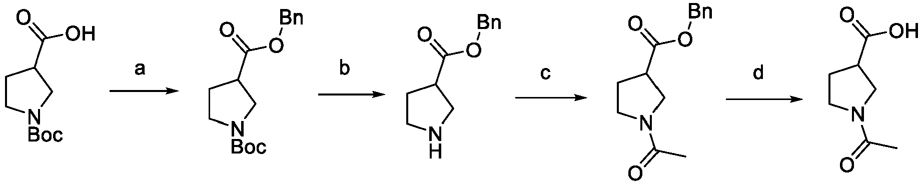

### Step a:

To a mixture of 1-[(*tert*-butoxy)carbonyl]pyrrolidine-3-carboxylic acid (4.20 g, 19.51 mmol) and K₂CO₃ (5.40 g, 39.07 mmol) in DMF (10 mL) was added (bromomethyl)benzene (5.00 g, 29.23 mmol) at room temperature. The resulting mixture was allowed to warm to 50 °C and stirred for 1 h. After cooling to room temperature, the reaction mixture was diluted with water (50 mL) and extracted with EA (3 x 40 mL). The combined organic layers were washed with brine (5 x 10 mL), dried over anhydrous Na₂SO₄. After filtration, filtrate was concentrated under reduced pressure. The residue was purified by silica gel column chromatography, eluted with PE/EA (2/1) to afford 3-benzyl 1-*tert*-butyl pyrrolidine-1,3-dicarboxylate as a light yellow oil (2.77 g, 47%): LCMS (ESI) calc'd for C₁₇H₂₃NO₄ [M + Na]⁺: 328, found 328; ¹H NMR (300 MHz, CD₃OD) δ 7.42-7.28 (m, 5H), 5.15 (s, 2H), 3.58-3.50 (m, 2H), 3.45-3.34 (m, 2H), 3.25-3.08 (m, 1H), 2.24-2.04 (m, 2H), 1.45 (s, 9H).

### Step b:

To a stirred solution of 3-benzyl 1-*tert*-butyl pyrrolidine-1,3-dicarboxylate (2.77 g, 8.84 mmol) in DCM (5 mL) was added TFA (1 mL) at room temperature. The resulting solution was stirred at room temperature for 1 h. The resulting solution was concentrated under reduced pressure to afford benzyl pyrrolidine-3-carboxylate as a light yellow oil (1.81 g, crude), which was used in next step directly without further purification: LCMS (ESI) calc'd for C₁₂H₁₅NO₂ [M + H]⁺: 206, found 206.

### Step c:

To a stirred solution of benzyl pyrrolidine-3-carboxylate (1.81 g, 8.82 mmol) and Et₃N (2.70 g, 26.46 mmol) in DMF (20 mL) was added acetic anhydride (1.40 g, 13.23 mmol) dropwise at room temperature. The resulting mixture was stirred for 1 h at room temperature. The reaction was diluted with water (40 mL) at room temperature and extracted with EA (3 x 30 mL). The combined organic layers were washed with brine (3 x 20 mL), dried over anhydrous Na₂SO₄. After filtration, filtrate was concentrated under reduced pressure. The residue was purified by silica gel column chromatography, eluted with DCM/MeOH (10/1) to afford benzyl 1-acetylpyrrolidine-3-carboxylate as a light yellow oil (1.60 g, 71% overall two steps): LCMS (ESI) calc'd for C₁₄H₁₇NO₃ [M + H]⁺: 248, found 248; ¹H NMR (400 MHz, CD₃OD) δ 7.46-7.28 (m, 5H), 5.18 (d, *J* = 4.0 Hz, 2H), 3.75 (d, *J* = 6.9 Hz, 1H), 3.67 (d, *J* = 6.9 Hz, 1H), 3.63-3.54 (m, 1H), 3.54-3.40 (m, 1H), 3.32-3.18 (m, 1H), 2.34-2.10 (m, 2H), 2.05 (d, *J =* 3.5 Hz, 3H).

### Step d:

A mixture of benzyl 1-acetylpyrrolidine-3-carboxylate (1.60 g, 6.47 mmol) and Pd/C (0.16 g, 1.50 mmol) in MeOH (15 mL) was stirred for 1 h at room temperature under hydrogen (1.5 atm). The resulting mixture was filtered, and the filter cake were washed with MeOH (3 x 10 mL). The filtrate was concentrated under reduced pressure to afford 1-acetylpyrrolidine-3-carboxylic acid as a light yellow oil (0.79 g, 62%): LCMS (ESI) calc'd for C₇H₁₁NO₃ [2M + Na]⁺: 337, found 337; ¹H NMR (300 MHz, CD₃OD) δ 3.73 (d, *J =* 7.0 Hz, 1H), 3.64 (d, *J =* 6.9 Hz, 1H), 3.61-3.35 (m, 2H), 3.27-3.06 (m, 1H), 2.31-2.10 (m, 2H), 2.06 (d, *J =* 5.2 Hz, 3H).

### Example 63. (which is a reference example) Intermediate 63 ((S)-N-[(S)-[1-[(4R)-2,2-dimethyl-1,3-dioxolane-4-carbonyl]piperidin-4-yl][5-fluoro-4-methyl-2-(prop-2-en-1-yloxy)phenyl]methyl]-2-methylpropane-2-sulfinamide)

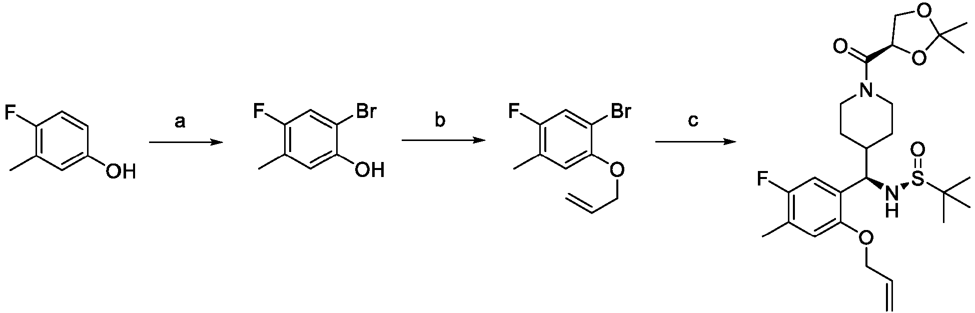

### Step a:

To a stirred solution of 4-fluoro-3-methylphenol (2.00 g, 15.86 mmol) and in DCM (20 mL) was added Br₂ (3.04 g, 19.02 mmol) at room temperature under air atmosphere. The resulting mixture was stirred for 1 h at room temperature under air atmosphere. The reaction was quenched with saturated aq. Na₂SO₃ (1 mL) at room temperature. The resulting mixture was extracted with EA (3 x 50 mL). The combined organic layers were combined and concentrated under reduced pressure to afford 2-bromo-4-fluoro-5-methylphenol, which was used in the next step directly without further purification: ¹H NMR (400 MHz, CD₃OD) δ 7.15 (d, *J* = 8.9 Hz, 1H), 6.76 (d, *J =* 7.0 Hz, 1H), 2.17 (d, *J =* 2.1 Hz, 3H).

### Step b:

To a stirred solution of 2-bromo-4-fluoro-5-methylphenol (3.50 g, 17.07 mmol) and K₂CO₃ (4.72 g, 34.14 mmol) in DMF (30 mL) was added 3-bromoprop-1-ene (3.10 g, 25.61 mmol) dropwise at room temperature under air atmosphere. The resulting mixture was stirred for 1 h at room temperature under air atmosphere. The resulting mixture was concentrated under reduced pressure. The residue was purified by silica gel column chromatography, eluted with PE/EA (10/1) to afford 1-bromo-5-fluoro-4-methyl-2-(prop-2-en-1-yloxy)benzene as a colorless oil (2.30 g, 55%): ¹H NMR (400 MHz, CD₃OD) δ 7.26 (d, *J =* 8.8 Hz, 1H), 6.95-6.85 (m, 1H), 6.15-6.01 (m, 1H), 5.48 (dq, *J =* 17.3, 1.9 Hz, 1H), 5.28 (dq, *J =* 10.6, 1.6 Hz, 1H), 4.62-4.54 (m, 2H), 2.24 (d, *J =* 2.1 Hz, 3H).

### Step c:

To a stirred solution of 1-bromo-5-fluoro-4-methyl-2-(prop-2-en-1-yloxy)benzene (0.11 g, 0.435 mmol) in THF (3 mL) was added n-BuLi (0.17 mL, 0.425 mmol) dropwise at -78 °C under nitrogen atmosphere. The reaction was stirred at -78 °C for 30 min. Then (*S*)-*N*-[[1-[(4R)-2,2-dimethyl-1,3-dioxolane-4-carbonyl]piperidin-4-yl]methylidene]-2-methylpropane-2-sulfinamide (0.10 g, 0.29 mmol) was added into the mixture. The resulting solution was stirred at -78 °C for 2 h. The reaction was quenched with NH₄Cl (1 mL). The resulting mixture was diluted with EA (30 mL) and water (30 mL) and the aqueous solution was extracted with EA (3 x 30 mL). The combined organic layers were washed with brine (3 x 20 mL), dried over anhydrous Na₂SO₄. After filtration, the filtrate was concentrated under reduced pressure. The residue was purified by Prep-TLC, eluted with PE/EA (1/4) to afford (*S*)-*N-*[(*S*)-[1-[(4*R*)-2,2-dimethyl-1,3-dioxolane-4-carbonyl]piperidin-4-yl][5-fluoro-4-methyl-2-(prop-2-en-1-yloxy)phenyl]methyl]-2-methylpropane-2-sulfinamide as a colorless oil (40 mg, 27%): LCMS (ESI) calc'd for C₂₆H₃₉FN₂O₅S [M + H]⁺: 511, found 511.

### Example 64. Intermediate 64 (N-[(4,5-dichloro-2-hydroxyphenyl)(pyridin-4-yl)methyl]acetamide, N-[(2,3-dichloro-6-hydroxyphenyl)(pyridin-4-yl)methyl]acetamide)

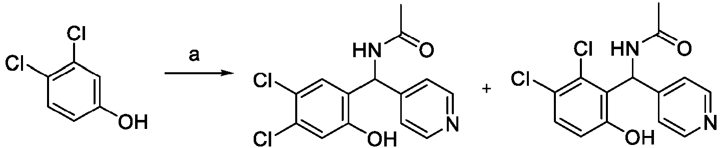

### Step a:

A mixture of 3,4-dichlorophenol (2.00 g, 12.27 mmol), pyridine-4-carbaldehyde (1.31 g, 12.27 mmol) and acetamide (0.87 g, 14.72 mmol), FeCl₃ (0.30 g, 1.84 mmol) was stirred at 110 °C for 1 h. After cooling to room temperature, the resulting mixture was diluted with EA (200 mL). The mixture was stirred for 20 min and filtered. The filtrate was concentrated under reduced pressure. The residue was purified by silica gel column chromatography, eluted with DCM/MeOH (10/1) to afford crude product. The crude product was purified with Prep-TLC, eluted with DCM/MeOH (10/1) to afford mixture of *N*-[(4,5-dichloro-2-hydroxyphenyl)(pyridin-4-yl)methyl]acetamide and *N*-[(2,3-dichloro-6-hydroxyphenyl)(pyridin-4-yl)methyl]acetamide as a light brown solid (0.12 g, 3%): LCMS (ESI) calc'd for C₁₄H₁₂Cl₂N₂O₂ [M + H]⁺: 311, 313 (3 : 2), found 311, 313 (3 : 2).

### Example 65. Intermediate 65 (tert-butyl 6-[methoxy(methyl)carbamoyl]-3-azabicyclo[3.1.1]heptane-3-carboxylate)

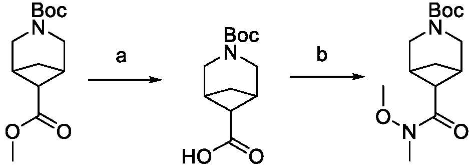

### Step a:

To a stirred solution of 3-*tert*-butyl 6-methyl 3-azabicyclo[*3.1.1*]heptane-3,6-dicarboxylate (0.40 g, 1.57 mmol) in MeOH (4 mL) were added a solution of KOH (0.18 g, 3.13 mmol) in H₂O (1 mL) at room temperature. The resulting mixture was stirred for 1 h at room temperature. The reaction was acidified to pH 3 with citric acid (30 mL). The resulting mixture was extracted with EA (3 x 50 mL). The combined organic layers were washed with brine (3 x 20 mL), dried over anhydrous Na₂SO₄. After filtration, the filtrate was concentrated under reduced pressure to afford 3-[(*tert-*butoxy)carbonyl]-3-azabicyclo[*3.1.1*]heptane-6-carboxylic acid as a light yellow oil (0.40 g, crude), which was used in the next step directly without further purification: LCMS (ESI) calc'd for C₁₂H₁₉NO₄ [M + Na]⁺: 264, found 264; ¹H NMR (400 MHz, CD₃OD) δ 3.92-3.79 (m, 1H), 3.64-3.56 (m, 1H), 3.43-3.32 (m, 1H), 3.11-3.02 (m, 1H), 2.72-2.57 (m, 2H), 2.56-2.43 (m, 1H), 2.13-2.03 (m, 1H), 1.47 (d, *J =* 11.9 Hz, 9H), 1.38-1.29 (m, 1H).

### Step b:

To a stirred solution of 3-[(*tert*-butoxy)carbonyl]-3-azabicyclo[*3.1.1*]heptane-6-carboxylic acid (0.40 g, 1.66 mmol), HOBt (0.34 g, 2.49 mmol) and EDCI (0.48 g, 2.49 mmol) in DCM (5 mL) were added *N,O*-methoxy(methyl)amine hydrochloride (0.24 g, 2.49 mmol) and Et₃N (0.50 g, 4.97 mmol) at room temperature. After stirring for 1 h at room temperature, the reaction was quenched with water (50 mL) at room temperature and extracted with EA (3 x 40 mL). The combined organic layers were washed with brine (2 x 20 mL), dried over anhydrous Na₂SO₄. After filtration, the filtrate was concentrated under reduced pressure. The residue was purified by reverse phase chromatography, eluted with 70% ACN in water (plus 0.05% TFA) to afford *tert*-butyl 6-[methoxy(methyl)carbamoyl]-3-azabicyclo[*3.1.1*]heptane-3-carboxylate as a light yellow oil (0.36 g, 81% overall two steps): LCMS (ESI) calc'd for C₁₄H₂₄N₂O₄ [M + H - 56]⁺: 229, found 229; ¹H NMR (400 MHz, CDCl₃) δ 4.04 (d, *J* = 11.8 Hz, 1H), 3.88-3.76 (m, 1H), 3.70 (s, 3H), 3.52-3.36 (m, 2H), 3.19 (s, 3H), 3.13-3.05 (m, 1H), 2.86-2.79 (m, 1H), 2.78-2.66 (m, 1H), 2.09 (q, *J* = 6.9 Hz, 1H), 1.48 (s, 9H), 1.34 (d, *J* = 9.4 Hz, 1H).

### Example 66. Intermediate 66 (tert-butyl (3R)-3-[methoxy(methyl)carbamoyl]pyrrolidine-1-carboxylate)

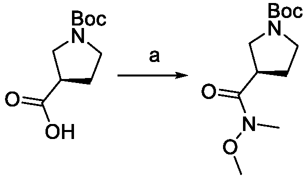

### Step a:

To a stirred solution of (3R)-1-(*tert*-butoxycarbonyl)pyrrolidine-3-carboxylic acid (2.50 g, 11.61 mmol), HOBt (2.35 g, 17.39 mmol) and EDCI (3.34 g, 17.42 mmol) in DCM (20 mL) were added *N*,*O*-methoxy(methyl)amine hydrochloride (1.70 g, 17.42 mmol) and Et₃N (2.35 g, 23.23 mmol) at room temperature. The reaction solution was stirred at room temperature for 1 h. The resulting mixture was diluted with water (50 mL) and extracted with EA (3 x 50 mL). The combined organic layers were washed with brine (3 x 20 mL), dried over anhydrous Na₂SO₄. After filtration, the filtrate was concentrated under reduced pressure. The residue was purified by reverse phase chromatography, eluted with 40% ACN in water (plus 0.05% TFA) to afford *tert-*butyl (3*R*)-3-[methoxy(methyl)carbamoyl]pyrrolidine-1-carboxylate as a colorless oil (2.50 g, 67%): LCMS (ESI) calc'd for C₁₂H₂₂N₂O₄ [M + H - 56]⁺: 203, found 203; ¹H NMR (400 MHz, CDCl₃) δ 3.73 (s, 3H), 3.71-3.50 (m, 2H), 3.50-3.30 (m, 3H), 3.22 (s, 3H), 2.23-2.00 (m, 2H), 1.48 (s, 9H).

### Example 67. Intermediate 67 (tert-butyl (3S)-3-[methoxy(methyl)carbamoyl]pyrrolidine-1-carboxylate)

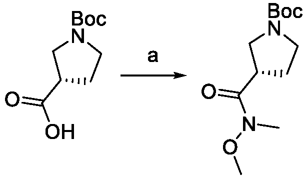

### Step a:

To a stirred solution of (3*S*)-1-(*tert*-butoxycarbonyl)pyrrolidine-3-carboxylic acid (2.50 g, 11.61 mmol), HOBt (2.35 g, 17.39 mmol) and EDCI (3.34 g, 17.42 mmol) in DCM (20 mL) were added *N*,*O*-methoxy(methyl)amine hydrochloride (1.70 g, 17.42 mmol) and Et₃N (2.35 g, 23.23 mmol) at room temperature. The reaction solution was stirred at room temperature for 1 h. The resulting mixture was diluted with water (50 mL) and extracted with EA (3 x 50 mL). The combined organic layers were washed with brine (3 x 20 mL), dried over anhydrous Na₂SO₄. After filtration, the filtrate was concentrated under reduced pressure. The residue was purified by reverse phase chromatography, eluted with 50% ACN in water (plus 0.05% TFA) to afford *tert*-butyl (3*S*)-3-[methoxy(methyl)carbamoyl]pyrrolidine-1-carboxylate as a colorless oil (2.50 g, 67%): LCMS (ESI) calc'd for C₁₂H₂₂N₂O₄ [M + H - 56]⁺: 203, found 203; ¹H NMR (400 MHz, CDCl₃) δ 3.74 (s, 3H), 3.66 (t, *J =* 9.1 Hz, 1H), 3.61-3.53 (m, 1H), 3.51-3.30 (m, 3H), 3.22 (s, 3H), 2.20-2.03 (m, 2H), 1.48 (s, 9H).

### Example 68. Intermediate 68 (lithio 5-chloro-6-oxo-1,6-dihydropyridine-3-carboxylate)

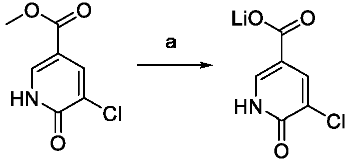

### Step a:

To a stirred solution of methyl 5-chloro-6-oxo-1*H*-pyridine-3-carboxylate (0.20 g, 1.07 mmol) in MeOH (2 mL) and H₂O (1 mL) was added LiOH·H₂O (90 mg, 2.13 mmol) at room temperature. The resulting mixture was allowed to warm to 40 °C and stirred for 1 h. After cooling to room temperature, the resulting mixture was concentrated under reduced pressure to afford lithio 5-chloro-6-oxo-1,6-dihydropyridine-3-carboxylate as a light yellow solid (0.20 g, crude), which was used in the next step directly without further purification: LCMS (ESI) calc'd for C₆H₄ClNO₃ [M + H]⁺: 174, 176 (3 : 1), found 174, 176 (3 : 1).

### Example 69. Intermediate 69 (lithio 6-(hydroxymethyl)pyridine-3-carboxylate)

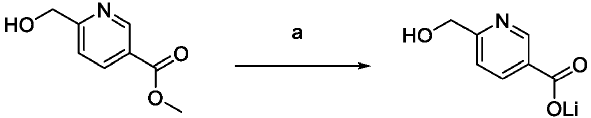

### Step a:

To a stirred mixture of methyl 6-(hydroxymethyl)pyridine-3-carboxylate (0.20 g, 1.20 mmol) in MeOH (2 mL) and H₂O (1 mL) was added LiOH·H₂O (0.10 g, 2.39 mmol) at room temperature. The resulting mixture was stirred for 1 h at 40 °C. After cooling to room temperature, the resulting mixture was concentrated under reduced pressure to afford lithio 6-(hydroxymethyl)pyridine-3-carboxylate as a light yellow solid (0.20 g, crude), which was used in the next step directly without further purification: LCMS (ESI) calc'd for C₇H₇NO₃ [M + H]⁺: 154, found 154.

### Example 70. Intermediate 70 (3-[(4-methoxyphenyl)methyl]-2-oxo-1,3-oxazinane-5-carboxylic acid)

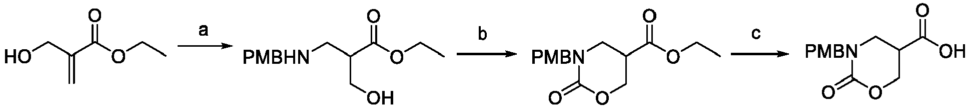

### Step a:

To a stirred mixture of ethyl 2-(hydroxymethyl)prop-2-enoate (1.00 g, 7.68 mmol) in MeOH (20 mL) was added (4-methoxyphenyl)methanamine (1.16 g, 8.46 mmol) dropwise at room temperature. The resulting solution was stirred for 16 h at room temperature. The resulting mixture was concentrated under reduced pressure. The residue was purified by silica gel column chromatography, eluted with DCM/MeOH (10/1) to afford ethyl 3-hydroxy-2-([[(4-methoxyphenyl)methyl]amino]methyl)propanoate as a light yellow oil (1.60 g, 78%): LCMS (ESI) calc'd for C₁₄H₂₁NO₄ [M + H]⁺: 268, found 268; ¹H NMR (400 MHz, CDCl₃) δ 7.24 (d, *J* = 8.1 Hz, 2H), 6.93-6.85 (m, 2H), 4.24-4.15 (m, 1H), 4.06-3.94 (m, 2H), 3.82 (s, 3H), 3.75 (s, 2H), 3.38-3.08 (m, 2H), 2.98-2.89 (m, 1H), 2.77-2.66 (m, 1H), 1.29 (td, *J =* 7.2, 1.4 Hz, 3H).

### Step b:

To a stirred solution of ethyl 3-hydroxy-2-([[(4-methoxyphenyl)methyl]amino]methyl)propanoate (0.70 g, 2.62 mmol) in DCM (80 mL) was added CDI (0.42 g, 2.62 mmol) in portions at 0 °C. The resulting mixture was allowed to warm to room temperature and stirred for 3 h. The resulting solution was concentrated under reduced pressure. The residue was purified by silica gel column chromatography, eluted with PE/EA (1/2) to afford ethyl 3-[(4-methoxyphenyl)methyl]-2-oxo-1,3-oxazinane-5-carboxylate as a light yellow oil (0.60 g, 55%): LCMS (ESI) calc'd for C₁₅H₁₉NO₅ [M + H]⁺: 294, found 294; ¹H NMR (400 MHz, CDCl₃) δ 7.27 (d, *J =* 9.4 Hz, 2H), 6.92-6.87 (m, 2H), 4.64-4.36 (m, 3H), 4.23-4.11 (m, 1H), 3.83 (s, 3H), 3.73 (s, 2H), 3.54-3.45 (m, 1H), 3.43-3.35 (m, 1H), 3.10-2.99 (m, 1H), 1.26 (t, *J =* 7.2 Hz, 3H).

### Step c:

To a stirred mixture of ethyl 3-[(4-methoxyphenyl)methyl]-2-oxo-1,3-oxazinane-5-carboxylate (0.40 g, 1.36 mmol) in 1,4-dioxane (3 mL) was added aq. HCl (4 *N,* 3.00 mL) at room temperature. The resulting mixture was stirred for 3 h at 50 °C. The resulting mixture was concentrated under reduced pressure to afford 3-[(4-methoxyphenyl)methyl]-2-oxo-1,3-oxazinane-5-carboxylic acid as an off-white solid (0.40 g, crude), which was used in the next step directly without further purification: LCMS (ESI) calc'd for C₁₃H₁₅NO₅ [M + H]⁺: 266, found 266; ¹H NMR (400 MHz, CD₃OD) δ 7.31-7.22 (m, 2H), 6.94-6.89 (m, 2H), 4.58-4.40 (m, 4H), 3.80 (s, 3H), 3.57-3.41 (m, 2H), 3.16-3.08 (m, 1H).

### Example 71. Intermediate 71 (3-(hydroxymethyl)azetidin-3-ol trifluoroacetic acid)

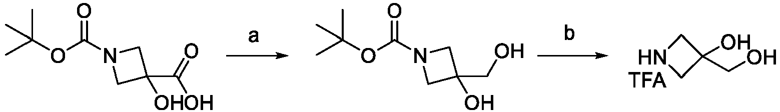

### Step a:

To a stirred solution of 1-[(*tert*-butoxy)carbonyl]-3-methylazetidine-3-carboxylic acid (0.45 g, 2.09 mmol) in THF (4 mL) was added BH₃·THF (6 mL, 6.27 mmol, 1 M in THF) dropwise at -10 °C under nitrogen atmosphere. The reaction solution was allowed to warm to 0 °C and stirred for 12 h under nitrogen atmosphere. The reaction was quenched with H₂O (20 mL) at 0 °C. The resulting mixture was concentrated under reduced pressure to afford *tert*-butyl 3-(hydroxymethyl)-3-methylazetidine-1-carboxylate as an off-white semi-solid (0.45 g, crude): ¹H NMR (400 MHz, CDCl₃) δ 4.35-4.22 (m, 2H), 4.17-3.97 (m, 4H), 1.47 (s, 9H).

### Step b:

To a stirred solution of *tert-*butyl 3-hydroxy-3-(hydroxymethyl)azetidine-1-carboxylate (0.45 g, 2.24 mmol) in DCM (4 mL) was added TFA (1 mL) at 0° C. The reaction solution was stirred for 1 h at room temperature. The resulting solution was concentrated under reduced pressure to afford 3-(hydroxymethyl)azetidin-3-ol trifluoroacetic acid as a colorless oil (0.35 g, crude): LCMS (ESI) calc'd for C₄H₉NO₂ [M + H]⁺: 104, found, 104.

### Example 72. (which is a reference example) Intermediate 72 (1-bromo-5-chloro-4-cyclopropyl-2-(prop-2-en-1-yloxy)benzene)

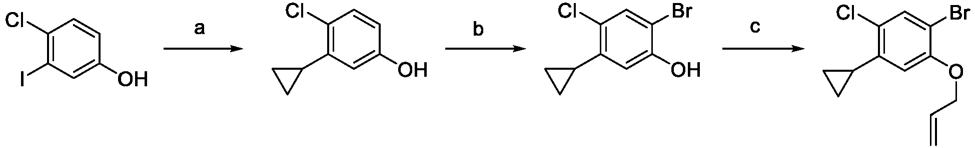

### Step a:

To a stirred solution of 4-chloro-3-iodophenol(1.00 g, 3.90 mmol) and cyclopropylboronic acid (0.67 g, 7.86 mmol) in 1,4-dioxane (15 mL) were added a solution of Na₂CO₃ (1.20 g, 11.71 mmol) in water (5 mL) and Pd(dppf)Cl₂ CH₂Cl₂ (0.30 g, 0.39 mmol) at room temperature under nitrogen atmosphere. The reaction mixture was degassed with nitrogen and stirred for 24 h at 90 °C under nitrogen atmosphere. After cooling to room temperature, the reaction mixture was diluted with water (30 mL), the resulting mixture was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (4/1) to afford 4-chloro-3-cyclopropylphenol as colorless oil (0.48 g, 72%): ¹H NMR (400 MHz, CD₃OD) δ 7.13 (d, *J =* 8.5 Hz, 1H), 6.56 (dd, *J =* 8.7, 2.9 Hz, 1H), 6.39 (d, *J =* 2.9 Hz, 1H), 2.19-2.09 (m, 1H), 1.02-0.95 (m, 2H), 0.67-0.59 (m, 2H).

### Step b:

To a stirred solution of 4-chloro-3-cyclopropylphenol (1.00 g, 5.93 mmol) in DCM (10 mL) was added Br₂ (1.14 g, 7.13 mmol) dropwise at room temperature. The reaction mixture was stirred for 1 h at room temperature. The resulting solution was quenched with saturated aq. Na₂SO₃ (10 mL) at room temperature. The resulting mixture was diluted with water (20 mL) and extracted with EA (3 x 20 mL). The combined organic layers were washed with brine (2 x 15 mL), dried over anhydrous Na₂SO₄. After filtration, the filtrate was concentrated under reduced pressure to afford crude 2-bromo-4-chloro-5-cyclopropylphenol as a colorless oil (0.94 g): ¹H NMR (400 MHz, CDCl₃) δ 8.04 (s, 1H), 6.61 (s, 1H), 2.21-2.03 (m, 1H), 1.10-0.98 (m, 2H), 0.70-0.61(m, 2H).

### Step c:

To a solution of 2-bromo-4-chloro-5-cyclopropylphenol (0.94 g, 3.79 mmol) in DMF (10 mL) were added K₂CO₃ (1.00 g, 7.59 mmol) and 3-bromoprop-1-ene (0.69 g, 5.69 mmol) dropwise at room temperature under air atmosphere. The reaction mixture was stirred for 1 h at room temperature. The resulting mixture was diluted with water (80 mL) and extracted with EA (3 x 30 mL). The combined organic layers were washed with brine (2 x 50 mL), dried over anhydrous Na₂SO₄. After filtration, the filtrate was concentrated under reduced pressure. The residue was purified by silica gel column chromatography, eluted with PE/EA (10/1) to afford 1-bromo-5-chloro-4-cyclopropyl-2-(prop-2-en-1-yloxy)benzene as a yellow oil (0.27 g, 25 %): ¹H NMR (400 MHz, CD₃OD) δ 7.53 (s, 1H), 6.60 (s, 1H), 6.12-6.01 (m, 1H), 5.55-5.42 (m, 1H), 5.33-5.26 (m, 1H), 4.61 (dt, *J =* 5.0, 1.7 Hz, 2H), 2.20-2.12 (m, 1H), 1.09-0.96 (m, 2H), 0.77-0.68 (m, 2H).

### Example 73. Intermediate 73 ((4-chloro-3-fluoro-5-methylphenyl N,N-diethylcarbamate)

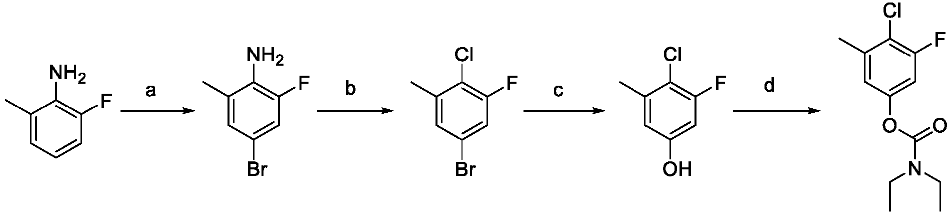

### Step a:

To a solution of 2-fluoro-6-methylaniline (50.00 g, 399.53 mmol) in DMF (300 mL), NBS (71.11 g, 399.53 mmol) was added in 5 portions at 0 °C. Then the reaction was allowed to warm to room temperature and stirred for 1 h. The reaction mixture was poured into water (3 L) and extracted with EA (3 x 500 mL). The combined organic layers were washed with brine (5 x 200 mL), dried over anhydrous Na₂SO₄. After filtration, the filtrate was concentrated under reduced pressure to afford 4-bromo-2-fluoro-6-methylaniline as a light yellow oil (90.00 g, crude): ¹H NMR (400 MHz, CDCl₃) δ 7.05 (dd, *J =* 10.1, 2.2 Hz, 1H), 7.00 (dt, *J =* 2.1, 1.1 Hz, 1H), 3.63 (s, 2H), 2.18 (s, 3H).

### Step b:

To a suspension of 4-bromo-2-fluoro-6-methylaniline (80.00 g, 392.08 mmol) in aq. HCl (12 *N,* 600 mL) was added a solution of NaNO₂ (31.92 g, 462.65 mmol) in H₂O (200 mL) dropwise at 0 °C. The reaction mixture was stirred at 0 °C for 1 h. To resulting mixture was added copper chloride (116.83 g, 1180.15 mmol) in 20 portions at 0 °C. After addition, the resulting mixture was stirred for 0.5 h at room temperature, and then for 12 h at 50 °C. The mixture was neutralized to pH 8 with saturated aq. Na₂CO₃. The resulting mixture was diluted with water (1 L) and extracted with EA (3 x 500 mL). The combined organic layers were washed with brine (2 x 500 mL), dried over anhydrous Na₂SO₄. After filtration, the filtrate was concentrated under reduced pressure. The residue was purified with silica gel column chromatography, eluted with PE/EA (20/1) to afford 5-bromo-2-chloro-1-fluoro-3-methylbenzene as a light yellow oil (41.00 g, 47%): ¹H NMR (400 MHz, CDCl₃) δ 7.24-7.21 (m, 1H), 7.19 (dd, *J =* 8.0, 2.3 Hz, 1H), 2.40 (s, 3H); ¹⁹F NMR (376 MHz, CDCl₃) δ -111.76.

### Step c:

To a solution of 5-bromo-2-chloro-1-fluoro-3-methylbenzene (41.00 g, 183.47 mmol) in DMSO (320 mL) and H₂O (80 mL) were added *N,N*-bis(4-hydroxy-2,6-dimethylphenyl)ethanediamide (3.01 g, 9.17 mmol), LiOH·H₂O (16.17 g, 385.29 mmol) and *N*¹,*N*²-bis(4-hydroxy-2,6-dimethylphenyl)oxalamide (2.40 g, 9.17 mmol) under nitrogen atmosphere. The suspension was degassed under nitrogen atmosphere. Then the reaction was stirred at 80 °C for 16 h. After cooling to room temperature, resulting mixture was acidified with aq. HCl (2 *N,* 200 mL), diluted with water (2 L), and then extracted with EA (3 x 500 mL). The combined organic layers were washed with brine (5 x 300 mL), and dried over anhydrous Na₂SO₄. After filtration, the filtrate was concentrated under reduced pressure. The residue was purified with silica gel column chromatography, eluted with PE/EA (2/1) to afford 4-chloro-3-fluoro-5-methylphenol as a colorless oil (30.00 g, 92%): LCMS (ESI) calc'd for C₇H₆ClFO [M - H]⁻: 159, 161 (3 : 1), found 159, 161 (3 : 1); ¹H NMR (400 MHz, CDCl₃) δ 6.60-6.50 (m, 2H), 5.00 (s, 1H), 2.36 (s, 3H); ¹⁹F NMR (376 MHz, CDCl₃) δ -112.60.

### Step d:

To a solution of 4-chloro-3-fluoro-5-methylphenol (37.00 g, 230.43 mmol), Et₃N (95.90 g, 949.57 mmol) and DMAP (56.30 g, 460.86 mmol) in DCM (500 mL) was added diethylcarbamoyl-chloride (37.49 g, 276.51 mmol) dropwise over 15 min at 0 °C under nitrogen atmosphere. The reaction solution was stirred at room temperature for 12 h under nitrogen atmosphere. The resulting solution was diluted with water (500 mL) and extracted with DCM (2 x 250 mL). The combined organic layers were washed with brine (2 x 200 mL), and dried over anhydrous Na₂SO₄. After filtration, the filtrate was concentrated under reduced pressure. The residue was purified by silica gel column chromatography, eluted with PE/EA (5/1) to afford 4-chloro-3-fluoro-5-methylphenyl *N,N*-diethylcarbamate as a colorless oil (40.00 g, 82%): LCMS (ESI) calc'd for C₁₂H₁₅ClFNO₂ [M + H]⁺: 260, 262 (3 : 1), found 260, 262 (3 : 1);¹H NMR (400 MHz, CDCl₃) δ 6.89-6.85 (m, 2H), 73.46-3.37 (m, 4H), 2.40 (s, 3H), 1.28-1.20 (m, 6H).

### Example 74. Intermediate 74 (5-chloro-1-methyl-6-oxopyridine-3-carboxylic acid)

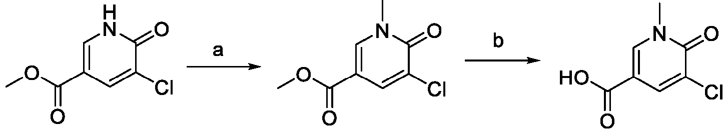

### Step a:

To a stirred mixture of methyl 5-chloro-6-oxo-1*H*-pyridine-3-carboxylate (1.00 g, 5.33 mmol) and K₂CO₃ (2.21 g, 15.99 mmol) in DMF (6 mL) was added MeI (3.78 g, 26.65 mmol) dropwise at room temperature. The reaction mixture was stirred for 5 h at 40 °C. The reaction mixture was diluted with water (50 mL). The resulting mixture was extracted with EA (3 x 10 mL). The combined organic layers were washed with brine (2 x 20 mL) dried over anhydrous Na₂SO₄. After filtration, the filtrate was concentrated under reduced pressure to afford methyl 5-chloro-1-methyl-6-oxopyridine-3-carboxylate as a yellow solid (0.98 g, 90%): LCMS (ESI) calc'd for C₈H₈ClNO₃ [M + H]⁺: 202, 204 (3 : 1), found 202, 204 (3 : 1); ¹H NMR (400 MHz, CD₃OD) δ 8.46 (d, *J =* 2.3 Hz, 1H), 8.13 (d, *J =* 2.3 Hz, 1H), 3.88 (s, 3H), 3.68 (s, 3H).

### Step b:

To a stirred mixture of methyl 5-chloro-1-methyl-6-oxopyridine-3-carboxylate (0.50 g, 2.48 mmol) in MeOH (3 mL, 74.09 mmol) was added aq. NaOH (1 *N,* 3 mL) at 0 °C under air atmosphere. The resulting mixture was stirred for 3 h at room temperature. The mixture was acidified to pH 6 with aq. HCl (1 *N,* 0.5 mL). The precipitated solids were collected by filtration and washed with MeOH (2 x 2 mL) to afford 5-chloro-1-methyl-6-oxopyridine-3-carboxylic acid as an off-white solid (0.45 g, 87%): LCMS (ESI) calc'd for C₇H₆ClNO₃ [M + H]⁺: 188, 190 (3 : 1), found 188, 190 (3 : 1); ¹H NMR (400 MHz, DMSO-*d*₆) δ 13.06 (s, 1H), 8.51 (d, *J* = 2.3 Hz, 1H), 7.99 (d, *J =* 2.3 Hz, 1H), 3.58 (s, 3H).

### Example 75. (which is a reference example) Intermediate 75 ((S)-N-((R)-(2-(allyloxy)-5-chloro-4-(trifluoromethyl)phenyl)(piperidin-4-yl)methyl)-2-methylpropane-2-sulfinamide)

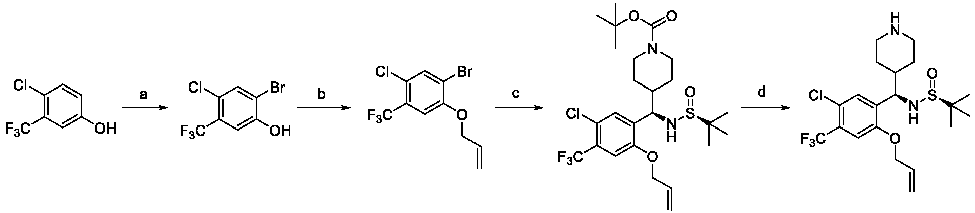

### Step a:

To a stirred solution of 4-chloro-3-(trifluoromethyl)phenol (3.00 g, 15.26 mmol) in DCM (30 mL) was added Br₂ (2.44 g, 15.26 mmol) dropwise at room temperature. The resulting mixture was stirred for 4 h at room temperature under nitrogen atmosphere. The reaction was quenched with saturated aq. Na₂S₂O₃ (100 mL) at 0 °C. The resulting mixture was extracted with EA (4 x 200 mL). The combined organic layers were washed with brine (2 x 200mL), dried over anhydrous Na₂SO₄. After filtration, the filtrate was concentrated under reduced pressure to afford 2-bromo-4-chloro-5-(trifluoromethyl)phenol as a yellow oil. The crude product was used in the next step directly without further purification: LCMS (ESI) calc'd for C₇H₃BrClF₃O [M - H]⁺: 273, 275 (2 : 3),found 273, 275 (2 : 3).

### Step b:

To a stirred mixture of 2-bromo-4-chloro-5-(trifluoromethyl)phenol (crude) and K₂CO₃ (4.01 g, 29.04 mmol) in DMF (20 mL) was added allyl bromide (2.64 g, 21.78 mmol) dropwise at room temperature. The resulting mixture was stirred for 16 h at 40 °C. After cooling to room temperature, the resulting mixture was diluted with water (30 mL). The resulting mixture was extracted with EA (3 x 60 mL). The combined organic layers were washed with brine (6 x 60 mL), dried over anhydrous Na₂SO₄. After filtration, the filtrate was concentrated under reduced pressure. The residue was purified by silica gel column chromatography, eluted with PE/EA (4/1) to afford to afford 1-bromo-5-chloro-2- (prop-2-en-1-yloxy)-4-(trifluoromethyl) benzene as a light yellow oil (1.00 g, 22%): ¹H NMR (400 MHz, CDCl₃) δ 7.72 (s, 1H), 7.17 (s, 1H), 6.15-6.00 (m, 1H), 5.59-5.34 (m, 2H), 4.67 (dt, *J =* 5.1, 1.7 Hz, 2H).

### Step c:

To a stirred solution of 1-bromo-5-chloro-2-(prop-2-en-1-yloxy)-4-(trifluoromethyl)benzene (0.94 g, 2.97 mmol) in THF (100 mL) was added *n*-BuLi (1.19 mL, 2.97 mmol, 2.5M in hexane) dropwise at -90 °C under nitrogen atmosphere. The resulting mixture was stirred for 40 min at -90 °C under nitrogen atmosphere. To a stirred solution was added *tert*-butyl 4-[[(*S*)-2-methylpropane-2-sulfinyl]imino]methyl]piperidine-1-carboxylate (0.94 g, 2.97 mmol) dropwise at -90 °C under nitrogen atmosphere. The resulting mixture was stirred for 1 h at -90 °C under nitrogen atmosphere. The reaction was quenched with water (150 mL) at -90 °C. The resulting mixture was extracted with EA (3 x 100 mL). The combined organic layers were washed with brine (3 x 100 mL), dried over anhydrous Na₂SO₄. After filtration, the filtrate was concentrated under reduced pressure. The residue was purified by reverse phase chromatography, eluted with 75% ACN in water with 20 mmol/L NH₄HCO₃ afford *tert*-butyl 4-[(*R*)-[5-chloro-2-(prop-2-en-1-yloxy)-4-(trifluoromethyl)phenyl[([[[*S*)-2-methylpropane-2-sulfinyl]amino])methyl]piperidine-1-carboxylate as a light yellow oil (1.00 g, 47%). LCMS (ESI) calc'd for C₂₅H₃₆ClF₃N₂O₄S [M + H]⁺: 553, 555 (3 : 1), found 553, 555 (3 : 1).

### Step d:

To a stirred solution of *tert*-butyl 4-[(*R*)-[5-chloro-2-(prop-2-en-1-yloxy)-4-(trifluoromethyl)phenyl][(2-methylpropane-2-sulfinyl)amino]methyl]piperidine-1-carboxylate (1.00 g, 1.81 mmol) in DCM (10 mL) was added TFA (2.5 mL) dropwise at room temperature under air atmosphere. The final reaction mixture was stirred for 1 h at room temperature. The mixture was acidified to pH 8 with saturated aq. NaHCO₃. The resulting mixture was extracted with EA (3 x 20 mL). The combined organic layers were washed with brine (2 x 10 mL), dried over anhydrous Na₂SO₄. After filtration, the filtrate was concentrated under reduced pressure to afford (*S*)-*N*-((*R*)-(2-(allyloxy)-5-chloro-4-(trifluoromethyl)phenyl)(piperidin-4-yl)methyl)-2-methylpropane-2-sulfinamide as a light yellow oil (0.94 g, 87%): LCMS (ESI) calc'd for C₂₀H₂₈ClF₃N₂O₂S [M + H]⁺: 453, 455 (3 : 1), found 453, 455 (3 : 1).

### Example 76. Intermediate 76 (2,3-dihydroxybutanoic acid)

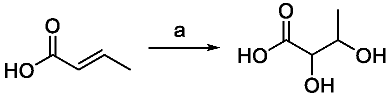

### Step a:

To a stirred solution of butenoic acid (5.00 g, 58.08 mmol) and 3-chlorobenzene-1-carboperoxoic acid (9.00 g, 52.27 mmol) in chloroform (60.00 mL) was added H₂O (120 mL) in portions at room temperature. The resulting mixture was stirred for 16 h at room temperature. The two layers were separated and the chloroform layer extracted with H₂O (3 x 60 mL). The aqueous layer was concentrated under reduced pressure. The residue was dissolved into water (50 mL) and stirred for 16 h at 50 °C. The resulting solution was concentrated under reduced pressure to afford 2,3-dihydroxybutanoic acid (1.00 g, 15%) as an off-white solid: ¹H NMR (400 MHz, DMSO-*d*₆) δ 12.82 (s, 1H), 3.19 (d, *J =* 1.9 Hz, 1H), 3.16-3.08 (m, 1H), 1.30 (d, *J =* 5.1 Hz, 3H).

Examples 77-254 describe the syntheses of representative disclosed herein, including representative compounds of the presently claimed invention.

### Example 77. (which is a reference example) Compound 1 (2-(amino(piperidin-4-yl)methyl)-4,5-dichlorophenol trifluoroacetic acid)

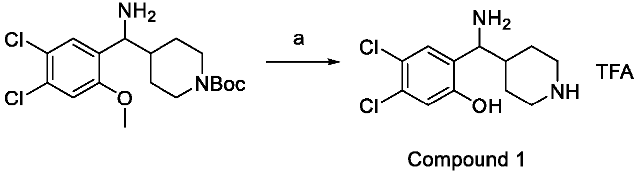

### Step a:

To a stirred solution of *tert*-butyl 4-[amino(4,5-dichloro-2-methoxyphenyl)methyl]piperidine-1-carboxylate (70 mg, 0.18 mmol) in DCM (3 mL) was added BBr₃ (0.64 g, 2.58 mmol) dropwise at 0 °C under argon atmosphere. The reaction mixture was allowed to warm to room temperature and stirred for 2 h under argon atmosphere. The resulting mixture was quenched with water (2 mL) at 0 °C and concentrated under reduced pressure. The residue was purified by Prep-HPLC with the following conditions: Column: XBridge Shield RP18 OBD Column 19 x 250 mm, 10 µm; Mobile Phase A: water (plus 0.05% TFA), Mobile Phase B: ACN; Flow rate: 25 mL/min; Gradient: 1% B to 30% B in 15 min; Detector: UV 254/210 nm; Retention time: 12.10 min. The fractions containing desired product were collected and concentrated under reduced pressure to afford Compound 1 (2-(amino(piperidin-4-yl)methyl)-4,5-dichlorophenol trifluoroacetic acid) as a purple solid (22.5 mg, 34%): LCMS (ESI) calc'd for C₁₂H₁₆Cl₂N₂O [M + H]+: 275, 277 (3 : 2), found 275, 277 (3 : 2); ¹H NMR (300 MHz, CD3OD) δ 7.43 (s, 1H), 7.06 (s, 1H), 4.22 (d, *J =* 9.6 Hz, 1H), 3.48 (d, *J* = 13.0 Hz, 1H), 3.33 (d, *J =* 13.0 Hz, 1H), 3.06-2.83 (m, 2H), 2.49-2.30 (m, 1H), 2.20-2.07 (m, 1H), 1.67-1.36 (m, 3H); ¹⁹F NMR (282 MHz, CD3OD) δ -76.96.

### Example 78. (which is a reference example) Compound 2 (4,5-dichloro-2-[hydroxy(piperidin-4-yl)methyl]phenol)

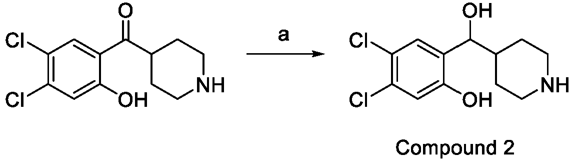

### Step a:

To a stirred solution of 4,5-dichloro-2-[(piperidin-4-yl)carbonyl]phenol (Intermediate 2, Example 2) (0.20 g, 0.73 mmol) in THF (10 mL) was added NaBH₄ (30 mg, 0.80 mmol) in portions at -40°C under nitrogen atmosphere. The reaction mixture was stirred for 2 h at -40 °C under nitrogen atmosphere. The reaction mixture was quenched by the addition of saturated aq. NH₄Cl (4 mL) and concentrated under reduced pressure. The residue was purified by Prep-HPLC with the following conditions: Column: XBridge C18 OBD Prep Column 100 Å, 10 µm, 19 mm x 250 mm; Mobile Phase A: water with 10 mmol/L NH₄HCO₃, Mobile Phase B: ACN; Flow rate: 20 mL/min; Gradient: 15% B to 50% B in 9 min; Detector: UV 254/210 nm; Retention time: 7.30 min. The fractions containing desired product was collected and concentrated under reduced pressure to afford Compound 2 (4,5-dichloro-2-[hydroxy(piperidin-4-yl)methyl]phenol) as an off-white solid (22.9 mg, 12%): LCMS (ESI) calc'd for C₁₂H₁₅Cl₂NO₂ [M + H]+: 276, 278 (3 : 2), found 276, 278 (3 : 2); ¹H NMR (300 MHz, DMSO-*d*₆) δ 7.28 (s, 1H), 6.85 (s, 1H), 6.35 (br, 1H), 4.53 (d, *J =* 5.4 Hz, 1H), 2.96-2.77 (m, 2H), 2.40-2.16 (m, 2H), 1.63-1.40 (m, 2H), 1.37-0.98 (m, 3H).

### Example 79. (which is a reference example) Compound 3 (4,5-dichloro-2-[1-hydroxy-1-(piperidin-4-yl)ethyl]phenol)

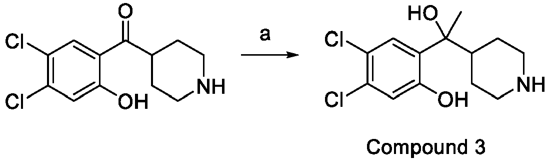

### Step a:

To a stirred solution of 4,5-dichloro-2-[(piperidin-4-yl)carbonyl]phenol (Intermediate 2, Example 2) (70 mg, 0.26 mmol) in THF (5 mL) was added MeMgBr (1.3 mL, 1.30 mmol, 1 M in THF) dropwise at -20 °C under nitrogen atmosphere. After stirring for additional 2 h at -20 °C, the resulting solution was quenched with saturated aq. NH₄Cl (20 mL) and concentrated under reduced pressure. The residue was purified by Prep-HPLC with the following conditions: Column: X Bridge C18, OBD, 100 Å, 10 µm, 19 mm x 250 mm; Mobile Phase A: water with 10 mmol/L NH₄HCO₃, Mobile Phase B: ACN; Flow rate: 20 mL/min; Gradient: 15% B to 50% B in 9 min; Detector: UV 254/210 nm; Retention time: 8.69 min. The fractions containing desired product were collected and concentrated under reduced pressure to afford Compound 3 (4,5-dichloro-2-[1-hydroxy-1-(piperidin-4-yl)ethyl]phenol) as an off-white solid (20 mg, 26%): LCMS (ESI) calc'd for C₁₃H₁₇Cl₂NO₂ [M + H]⁺: 290, 292 (3 : 2), found 290, 292 (3 : 2); ¹H NMR (400 MHz, CD₃OD) δ 7.16 (s, 1H), 6.74 (s, 1H), 3.30-3.18 (m, 2H), 2.82-2.70 (m, 2H), 2.06 (t, *J* = 12.2 Hz, 1H), 1.82-1,68 (m, 2H), 1.54-1.47 (m, 5H).

### Example 80. Compound 4 (1-(4-((4,5-dichloro-2-hydroxyphenyl)(hydroxy)methyl)piperidin-1-yl)ethanone)

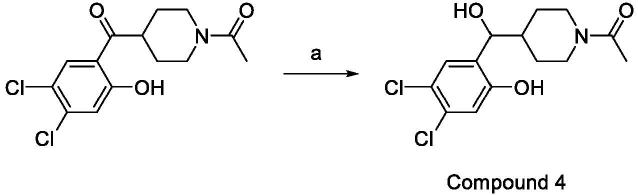

### Step a:

To a stirred solution of 1-(4-(4,5-dichloro-2-hydroxybenzoyl)piperidin-1-yl)ethanone (Intermediate 1, Example 1) (0.20 g, 0.63 mmol) in THF (10 mL) was added NaBH₄ (26 mg, 0.69 mmol) at -40 °C under nitrogen atmosphere. After stirring at -40 °C under nitrogen atmosphere for additional 2 h, the reaction mixture was quenched by saturated aq. NH₄Cl (40 mL) and extracted with EA (3 x 30 mL). The combined organic layers were washed with brine (2 x 20 mL) and dried over anhydrous Na₂SO₄. After filtration, the filtrate was concentrated under reduced pressure. The residue was purified by Prep-HPLC with the following conditions: Column: XBridge C18 OBD Prep Column 100 Å, 10 µm, 19 mm x 250 mm; Mobile Phase A: water with 10 mmol/L NH₄HCO₃, Mobile Phase B: ACN; Flow rate: 20 mL/min; Gradient: 10% B to 60% B in 8 min; Detector: UV 254/210 nm; Retention time: 6.33 min. The fractions containing desired product was collected and concentrated under reduced pressure to afford Compound 4 (1-(4-((4,5-dichloro-2-hydroxyphenyl) (hydroxy)methyl)piperidin-1-yl)ethanone) as an off-white solid (39.4 mg, 26%): LCMS (ESI) calc'd for C₁₄H₁₇Cl₂NO₃ [M + H]⁺: 318, 320 (3 : 2), found 318, 320 (3 : 2); ¹H NMR (300 MHz, DMSO-d₆) δ 7.45 (br, 1H), 7.34 (s, 1H), 6.90 (s, 1H), 4.60 (d, *J =* 5.4 Hz, 1H), 4.32 (d, *J =* 12.8 Hz, 1H), 3.74 (d, *J =* 13.4 Hz, 1H), 2.94-2.73 (m, 1H), 2.42-2.20 (m, 1H), 1.91 (s, 3H), 1.79-1.61 (m, 1H), 1.51 (d, *J =* 13.5 Hz, 1H), 1.40-0.98 (m, 3H).

### Example 81. (which is a reference example) Compound 5 (4,5-dichloro-2-[2-hydroxy-1-(piperidin-4-yl)ethyl]phenol)

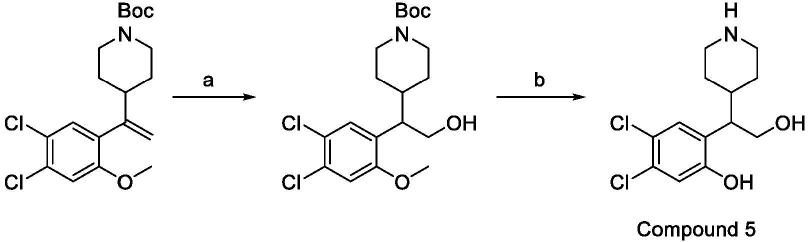

### Step a:

To a solution of *tert*-butyl 4-[1-(4,5-dichloro-2-methoxyphenyl)ethenyl]piperidine-1-carboxylate (Intermediate 17, Example 17) (2.00 g, 5.18 mmol) in THF (16 mL) was added BH₃ (0.9 mL, 1 M in THF) dropwise at room temperature. After stirring for 5 h at room temperature, NaOH (0.52 g, 12.94 mmol) and H₂O₂ (1.5 mL, 30% in water) were added at room temperature. The reaction solution was stirred for 16 h at room temperature. The resulting solution was quenched with saturated aq. Na₂SO₃ (100 mL) at room temperature and extracted with EA (3 x 300 mL). The combined organic layers were washed with brine (2 x 10 mL), dried over anhydrous Na₂SO₄. After filtration, the filtrate was concentrated under reduced pressure. The residue was purified by silica gel column chromatography, eluted with CH₂Cl₂/MeOH (25/1) to afford *tert*-butyl 4-[1-(4,5-dichloro-2-methoxyphenyl)-2-hydroxyethyl]piperidine-1-carboxylate as an off-white solid (1.80 g, 86%): LCMS (ESI) calc'd for C₁₉H₂₇Cl₂NO₄ [M + H]⁺: 404, 406 (3 : 2), found 404, 406 (3 : 2); ¹H NMR (300 MHz, CD₃OD) δ 7.34 (s, 1H), 7.10 (s, 1H), 4.08 (d, *J* = 12.9 Hz, 1H), 3.95 (d, *J =* 13.5 Hz, 1H), 3.85-3.70 (m, 4H), 3.30-3.20 (m, 1H), 3.12-2.96 (m, 1H), 2.89-2.55 (m, 2H), 2.01-1.76 (m, 2H), 1.42 (s, 9H), 1.41-1.20 (m, 2H), 1.07 -0.92 (m, 1H).

### Step b:

To a solution of *tert*-butyl 4-[1-(4,5-dichloro-2-methoxyphenyl)-2-hydroxyethyl]piperidine-1-carboxylate (0.20 g, 0.51 mmol) in DCM (2 mL) was added BBr₃ (0.64 g, 2.56 mmol) at room temperature. The reaction mixture was stirred for 4 h at room temperature. The resulting mixture was quenched with water (10 mL) and concentrated under reduced pressure. The residue was purified by Prep-HPLC with the following conditions: Column: XBridge C18 OBD Prep Column, 100 Å, 10 µm, 19 mm x 250 mm; Mobile Phase A: water with 20 mmol/L NH₄HCO₃, Mobile Phase B: ACN; Flow rate: 20 mL/min; Gradient: 30% B to 70% B in 9 min; Detector: UV 254/210 nm; Retention time: 8.15 min. The fractions containing desired product were collected and concentrated under reduced pressure to afford Compound 5 (4,5-dichloro-2-[2-hydroxy-1-(piperidin-4-yl)ethyl]phenol) as a brown solid (50 mg, 29%): LCMS (ESI) calc'd for C₁₃H₁₇Cl₂NO₂ [M + H]⁺: 290, 292 (3 : 2), found 290, 292 (3 : 2); ¹H NMR (300 MHz, CD₃OD) δ 7.20 (s, 1H), 6.80 (s, 1H), 3.90-3.70 (m, 2H), 3.30-3.00 (m, 2H), 2.90-2.58 (m, 3H), 2.10-2.00 (m, 2H), 1.55-1.12 (m, 3H).

### Example 82. (which is a reference example) Compound 6 ((S)-4,5-dichloro-2-(hydroxy(piperidin-4-yl)methyl)phenol) and Compound 7 ((R)-4,5-dichloro-2-(hydroxy(piperidin-4-yl)methyl)phenol)

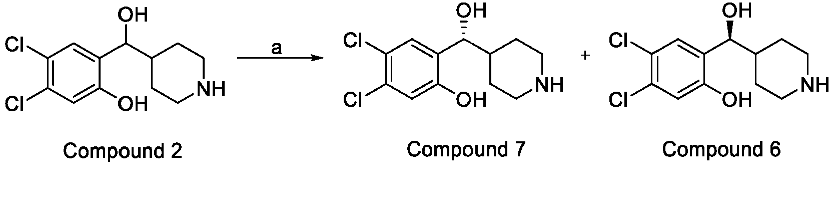

### Step a:

4,5-Dichloro-2-[hydroxy(piperidin-4-yl)methyl]phenol (Compound 2, Example 78) (70 mg) was separated by Prep-SFC with the following conditions: Column: Phenomenex Lux 5 µm, Cellulose-4, AXIA Packed, 12 x 25 cm, 5 µm; Mobile Phase A: CO₂ (70%), Mobile Phase B: MeOH (30%); Flow rate: 40 mL/min; Detector: UV 220 nm; Retention time: RT₁: 5.25 min; RT₂: 5.93 min.

The faster-eluting enantiomer, Compound 6 ((*S*)-4,5-dichloro-2-(hydroxy(piperidin-4-yl)methyl)phenol), was isolated as an off-white solid (15.3 mg, 22%) at 5.25 min: LCMS (ESI) calc'd for C₁₂H₁₅Cl₂NO₂ [M + H]⁺: 276, 278 (3 : 2), found 276, 278 (3 : 2); ¹H NMR (300 MHz, CD₃OD) δ 7.24 (s, 1H), 6.79 (s, 1H), 4.66 (d, *J =* 6.0 Hz, 1H), 3.24-3.08 (m, 2H), 2.67 (td, *J =* 12.5, 2.9 Hz, 2H), 1.92-1.73 (m, 2H), 1.56-1.34 (m, 3H).

The slower-eluting enantiomer, Compound 7 ((R)-4,5-dichloro-2-(hydroxy(piperidin-4-yl)methyl)phenol), was isolated as an off-white solid (8.1 mg, 12%) at 5.93 min: LCMS (ESI) calc'd for C₁₂H₁₅Cl₂NO₂ [M + H]⁺: 276, 278 (3 : 2), found 276, 278 (3 : 2); ¹H NMR (300 MHz, CD₃OD) δ 7.24 (s, 1H), 6.79 (s, 1H), 4.66 (d, *J =* 6.0 Hz, 1H), 3.24-3.08 (m, 2H), 2.67 (td, *J =* 12.5, 2.9 Hz, 2H), 1.92-1.73 (m, 2H), 1.56-1.34 (m, 3H).

### Example 83. (which is a reference example) Compound 8 (2-[2-amino-1-(piperidin-4-yl)ethyl]-4,5-dichlorophenol)

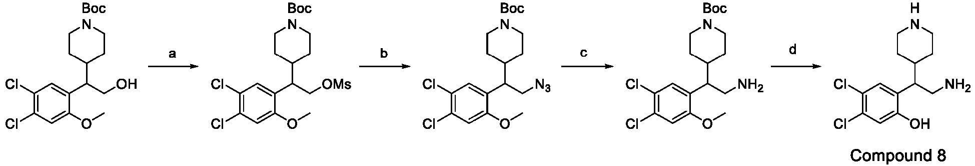

### Step a:

To a solution of tert-butyl 4-[1-(4,5-dichloro-2-methoxyphenyl)-2-hydroxyethyl]piperidine-1-carboxylate (Step a, Example 81) (0.10 g, 0.25 mmol) and Et₃N (50 mg, 0.50 mmol) in DCM (2 mL) was added methanesulfonyl chloride (34 mg, 0.30 mmol) at room temperature. The reaction solution was stirred for 1 h at room temperature. The resulting mixture was quenched with water (30 mL) and extracted with EA (2 x 30 mL). The combined organic layers were washed with brine (2 x 10 mL), dried over anhydrous Na₂SO₄. After filtration, the filtrate was concentrated under reduced pressure to afford crude propan-2-yl 4-[1-(4,5-dichioro-2-methoxyphenyl)-2-(methanesulfonyloxy)ethyl]piperidine-1-carboxylate. The crude product was used in the next step directly without further purification: LCMS (ESI) calc'd for C₂₀H₂₉Cl₂NO₆S [M + H]⁺: 482, 484 (3 : 2), found 482, 484 (3 : 2).

### Step b:

A solution of tert-butyl 4-[1-(4,5-dichloro-2-methoxyphenyl)-2-(methanesulfonyloxy)ethyl]piperidine-1-carboxylate (crude) in DMF (5 mL) was added sodium azide (32 mg, 0.50 mmol). The reaction mixture was allowed to warm to 80 °C and stirred for 16 h. After cooling to room temperature, the reaction mixture was diluted with water (30 mL) and exacted with EA (3 x 30 mL). The combined organic layers were washed with brine (2 x 20 mL), dried over anhydrous Na₂SO₄. After filtration, the filtrate was concentrated to a quarter of volume. The crude *tert*-butyl 4-[2-azido-1-(4,5-dichloro-2-methoxyphenyl)ethyl]piperidine-1-carboxylate was used in the next step directly without further purification: LCMS (ESI) calc'd for C₁₉H₂₆Cl₂N₄O₃ [M + H]⁺: 429, 431 (3 : 2), found 429, 431 (3 : 2).

### Step c:

To a solution of *tert*-butyl 4-[2-azido-1-(4,5-dichloro-2-methoxyphenyl)ethyl]piperidine-1-carboxylate (crude) in THF (3 mL) were added PPh₃ (0.23 g, 0.87 mmol) and NH₃·H₂O (1 mL, 28% in water) at room temperature. The reaction solution was stirred for 2 h at room temperature. The resulting solution was diluted with water (30 mL) and extracted with EA (3 x 100 mL). The combined organic layers were washed with brine (2 x 10 mL), dried over anhydrous Na₂SO₄. After filtration, the filtrate was concentrated under reduced pressure. The residue was purified by Prep-TLC, eluted with DCM/MeOH (15/1) to afford *tert-*butyl 4-[2-amino-1-(4,5-dichloro-2-methoxyphenyl)ethyl]piperidine-1-carboxylate as a yellow oil (80 mg, 34.06% overall three steps): LCMS (ESI) calc'd for C₁₉H₂8Cl₂N₂O₃ [M + H]⁺: 403, 405 (3 : 2), found 403, 405 (3 : 2); ¹H NMR (300 MHz, DMSO- *d*₆) δ 7.37 (s, 1H), 7.22 (s, 1H), 4.13-3.99 (m, 1H), 3.96-3.81 (m, 2H), 3.76 (s, 3H), 3.17-3.12 (s, 2H), 2.96-2.78 (d, *J* = 3.8 Hz, 2H), 2.73-2.56 (m, 2H), 1.81-1.70 (d, *J =* 11.5 Hz, 2H), 1.37 (s, 9H), 1.26-1.20 (m, 1H), 1.08-0.79 (m, 2H).

### Step d:

To a solution of tert-butyl 4-[2-amino-1-(4,5-dichloro-2-methoxyphenyl)ethyl]piperidine-1-carboxylate (80 mg, 0.20 mmol) in DCM (2 mL) was added BBr₃ (0.40 g, 1.59 mmol) at room temperature. After stirring for 2 h at room temperature, the reaction mixture was quenched with water (20 mL) at room temperature and concentrated under reduced pressure. The residue was purified by Prep-HPLC with the following conditions: Column: XBridge C18 OBD Prep Column 100 Å, 10 µm, 19 mm x 250 m; Mobile Phase A: water with 20 mmol/L NH₄HCO₃, Mobile Phase B: ACN; Flow rate: 20 mL/min; Gradient: 30% B to 75% B in 9 min; Detector: UV 254/210 nm; Retention time: 8.40 min. The fractions containing desired product were collected and concentrated under reduced pressure to afford Compound 8 (2-[2-amino-1-(piperidin-4-yl)ethyl]-4,5-dichlorophenol) as an off-white solid (25 mg, 43%): LCMS (ESI) calc'd for C₁₃H₁₈Cl₂N₂O [M + H]⁺: 289, 291 (3 : 2), found 289, 291 (3: 2); ¹H NMR (300 MHz, DMSO-d₆) δ 7.33 (s, 1H), 7.03 (s, 1H), 3.61-3.35 (m, 3H), 3.20-2.81 (m, 4H), 2.23-2.00 (m, 2H), 1.62-1.20 (m, 3H).

### Example 84. Compound 9 (1-(4-(amino(4,5-dichloro-2-hydroxyphenyl)methyl)piperidin-1-yl)ethanone)

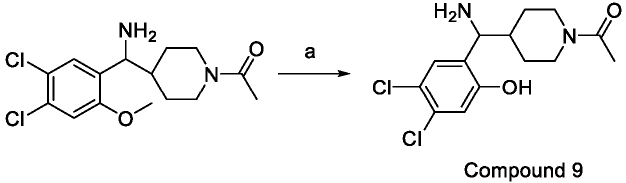

### Step a:

To a stirred solution of 1-[4-[amino(4,5-dichloro-2-methoxyphenyl)methyl]piperidin-1-yl]ethan-1-one (30 mg, 0.09 mmol) in DCM (3 mL) was added BBr₃ (0.14 g, 0.54 mmol) dropwise at 0 °C. After stirring for additional 1 h, the reaction solution was quenched with saturated aq. NaHCO₃ (20 mL) at 0 °C and extracted with EA (3 x 40 mL). The combined organic layers were washed with brine (3 x 10 mL), dried over anhydrous Na₂SO₄. After filtration, the filtrate was concentrated under reduced pressure. The residue was purified by Prep-HPLC with the following conditions: Column: XBridge C18 OBD Prep Column 100 Å, 10 µm, 19 mm x 250 mm; Mobile Phase A: water with 20 mmol/L NH4HCO3, Mobile Phase B: ACN; Flow rate: 20 mL/min; Gradient: 28% B to 43% B in 9 min; Detector: UV 254/210 nm; Retention time: 7.88 min. The fractions containing desired product were collected and concentrated under reduce pressure to afford Compound 9 (1-(4-(amino(4,5-dichloro-2-hydroxyphenyl)methyl)piperidin-1-yl)ethanone) as an off-white solid (4.5 mg, 18%): LCMS (ESI) calc'd for C₁₄H₁₈Cl₂N₂O₂ [M + H]+: 317, 319 (3 : 2), found 317, 319 (3 : 2); ¹H NMR (300 MHz, CD₃OD) δ 7.22 (d, *J =* 2.7 Hz, 1H), 6.86 (d, *J =* 1.8 Hz, 1H), 4.60-4.40 (m, 1H), 4.07-3.82 (m, 2H), 3.15-2.92 (m, 1H), 2.61-2.39 (m, 1H), 2.09 (d, *J =* 7.0 Hz, 3H), 2.09-1.88 (m, 2H), 1.49-1.38 (m, 1H), 1.25-1.04 (m, 2H).

### Example 85. (which is a reference example) Compound 10 (N[(S)-(4,5-dichloro-2-hydroxyphenyl)(piperidin-4-yl)methyl]acetamide and Compound 17 (N-[(R)-(4,5-dichloro-2-hydroxyphenyl)(piperidin-4-yl)methyl]acetamide)

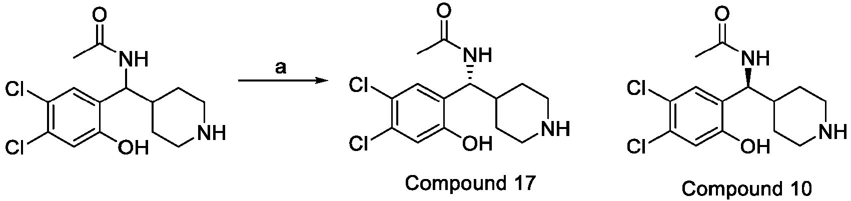

### Step a:

*N*-[(4,5-dichloro-2-hydroxyphenyl)(piperidin-4-yl)methyl]acetamide (47 mg, 0.15 mmol) was separated by Prep-SFC with following conditions: Column: Phenomenex Lux, Cellulose-4Å, AXIA Packed, 2 x 25 cm, 5 µm; Mobile Phase A: CO₂: 70%, Mobile Phase B: MeOH: 30%; Flow rate: 40 mL/min; Detector: UV: 220 nm; Retention time: RT₁: 5.25 min; RT₂: 5.93 min.

The faster-eluting enantiomer was obtained as Compound 17 (N-[(R)-(4,5-dichloro-2-hydroxyphenyl)(piperidin-4-yl)methyl]acetamide) as an off-white solid (4.7 mg, 10%) at 5.25 min: LCMS (ESI) calc'd for C₁₄H₁₈Cl₂N₂O₂ [M + H]⁺: 317, 319 (3 : 2), found 317, 319 (3 : 2); ¹H NMR (400 MHz, CD₃OD) δ 7.26 (d, *J =* 2.1 Hz, 1H), 6.89 (d, *J =* 1.5 Hz, 1H), 4.96-4.93 (m, 1H), 3.19 (dd, *J =* 36.1, 12.3 Hz, 2H), 2.69 (dt, *J =* 23.9, 12.4 Hz, 2H), 2.06 (d, *J =* 10.2 Hz, 1H), 1.99 (s, 3H), 1.94 (d, *J =* 14.0 Hz, 1H), 1.46 (d, *J =* 13.5 Hz, 1H), 1.42-1.24 (m, 2H).

The slower-eluting enantiomer was obtained as Compound 10 (*N*-[(*S*)-(4,5-dichloro-2-hydroxyphenyl)(piperidin-4-yl)methyl]acetamide) as an off-white solid (4.8 mg, 10%) at 5.93 min: LCMS (ESI) calc'd for C₁₄H₁₈Cl₂N₂O₂ [M + H]⁺: 317, 319 (3 : 2), found 317, 319 (3 : 2); ¹H NMR (400 MHz, CD₃OD) δ 7.23 (s, 1H), 6.87 (s, 1H), 4.98-4.92 (m, 1H), 3.19 (d, *J* = 12.7 Hz, 1H), 3.10 (d, *J =* 12.7 Hz, 1H), 2.71-2.55 (m, 2H), 2.09-2.00 (m, 1H), 1.99 (s, 3H), 1.90 (d, *J* = 13.8 Hz, 1H), 1.43 (d, *J =* 13.5 Hz, 1H), 1.38-1.23 (m, 2H).

### Example 86. (which is a reference example) Compound 11 (N-[(4,5-dichloro-2-hydroxyphenyl)(piperidin-4-yl)methyl]acetamide)

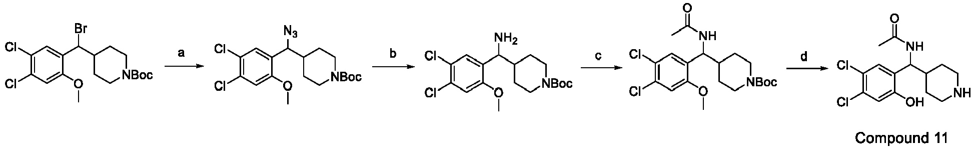

### Step a:

To a solution of tert-butyl 4-[bromo(4,5-dichloro-2-methoxyphenyl)methyl]piperidine-1-carboxylate (1.00 g, 2.21 mmol) in DMF (8 mL) was added NaN₃ (0.43 g, 6.62 mmol) at room temperature. The resulting mixture was stirred at 100 °C for 16 h. The mixture was cooled down to room temperature. The reaction was diluted with water (30 mL) at room temperature. The resulting mixture was extracted with EA (4 x 30 mL). The combined organic layers were washed with brine (6 x 20 mL), dried over anhydrous Na₂SO₄. After filtration, the filtrate was concentrated under reduced pressure to a quarter of volume to afford crude tert-butyl 4-(azido(4,5-dichloro-2-methoxyphenyl)methyl)piperidine-1-carboxylateas, which was used in the next step directly without further purification: LCMS (ESI) calc'd for C₁₈H₂₄Cl₂N₄O₃ [M + H]⁺: 415, 417 (3 : 2), found 415, 417 (3 : 2).

### Step b:

To a stirred solution of tert-butyl 4-[azido(4,5-dichloro-2-methoxyphenyl)methyl]piperidine-1-carboxylate (crude) in THF (8 mL) and NH₃·H₂O (3 mL, 38% in water) was added PPh₃ (1.14 g, 4.33 mmol) at room temperature. The resulting mixture was stirred at room temperature for 16 h, and then the solution of LiOH (0.10 g, 4.33 mmol) in water (4 mL) was added with stirring at 50 °C for 3 h. The reaction was diluted with water (30 mL). The resulting mixture was extracted with EA (3 x 20 mL). The combined organic layers were washed with brine (3 x 20 mL), dried over anhydrous Na₂SO₄. After filtration, the filtrate was concentrated under reduced pressure. The residue was purified by purified by reverse phase chromatography, eluted with 28% ACN in water (plus 0.05% TFA) to afford tert-butyl 4-[amino(4,5-dichloro-2-methoxyphenyl)methyl]piperidine-1-carboxylate as a yellow oil (0.60 g, 70% overall two steps): LCMS (ESI) calc'd for C₁₈H₂₆Cl₂N₂O₃ [M + H]+: 389, 391 (3 : 2), found 389, 391 (3 : 2).

### Step c:

To a stirred solution of tert-butyl 4-[amino(4,5-dichloro-2-methoxyphenyl)methyl]piperidine-1-carboxylate (0.30 g, 0.77 mmol) in DCM (3 mL) was added acetyl acetate (87 mg, 0.85 mmol) at room temperature. The resulting mixture was stirred at room temperature for 1 h. The reaction was diluted with Water (20 mL). The resulting mixture was extracted with DCM (3 x 20 mL). The combined organic layers were washed with brine (2 x 20 mL), dried over anhydrous Na₂SO₄. After filtration, the filtrate was concentrated under reduced pressure to afford tert-butyl 4-[(4,5-dichloro-2-methoxyphenyl)(acetamido)methyl]piperidine-1-carboxylate as a yellow oil (0.16 g, crude), which was used in the next step directly without further purification: LCMS (ESI) calc'd for C₂₀H₂₈Cl₂N₂O₄ [M + H]⁺: 431, 433 (3 : 2), found 431, 433 (3 : 2); ¹H NMR (400 MHz, CD₃OD) δ 7.38 (s, 1H), 7.16 (s, 1H), 5.04 (d, *J =* 8.9 Hz, 1H), 4.06 (dd, *J =* 36.3, 13.8 Hz, 2H), 3.89 (s, 3H), 2.80-2.58 (m, 2H), 1.99 (s, 3H), 1.85 (dd, *J =* 51.0, 11.9 Hz, 2H), 1.46 (s, 9H), 1.36-1.09 (m, 3H).

### Step d:

To a stirred solution of tert-butyl 4-[(4,5-dichloro-2-methoxyphenyl)(acetamido)methyl]piperidine-1-carboxylate (0.16 g, 0.37 mmol) in DCM (3 mL) was added BBr₃ (0.74 g, 2.97 mmol) at room temperature. The resulting mixture was stirred at room temperature for 1 h. The reaction was quenched with water (1 mL) at 0 °C. The resulting mixture was concentrated under reduced pressure. The residue was purified by Prep-HPLC with the following conditions: Column: XBridge C18 OBD Prep Column 100 Å, 10 µm, 19 mm x 250 mm; Mobile Phase A: Water with 20 mmoL/L NH₄HCO₃, Mobile Phase B: ACN; Flow rate: 20 mL/min; Gradient: 40% B to 90% B in 9 min; Detector: UV 254/210 nm; Retention time: 8.71 min. The fractions containing the desired product were collected and concentrated under reduced pressure to afford Compound 11 (N-[(4,5-dichloro-2-hydroxyphenyl)(piperidin-4-yl)methyl]acetamide) as an off-white solid (47.7 mg, 20% overall two steps): LCMS (ESI) calc'd for C₁₄H₁₈Cl₂N₂O₂ [M + H]⁺: 317, 319 (3 : 2), found 317, 319 (3 : 2); ¹H NMR (400 MHz, CD₃OD) δ 7.38 (s, 1H), 6.97 (s, 1H), 5.06 (d, *J =* 9.4 Hz, 1H), 3.45 (d, *J =* 12.9 Hz, 1H), 3.39-3.35 (m, 1H), 3.02-2.85 (m, 2H), 2.22-2.06 (m, 2H), 2.00 (s, 3H), 1.66-1.29 (m, 3H).

### Example 87. (which is a reference example) Compound 12 (N-((4,5-dichloro-2-hydroxyphenyl)(piperidin-4-yl)methyl)methanesulfonamide)

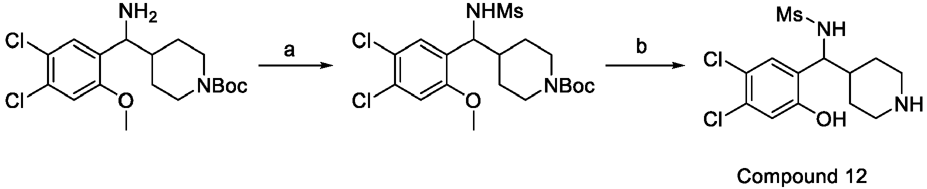

### Step a:

To a stirred solution of tert-butyl 4-[amino(4,5-dichloro-2-methoxyphenyl)methyl]piperidine-1-carboxylate (0.30 g, 0.77 mmol) and Et₃N (0.16 g, 1.54 mmol) in DCM (3 mL) was added methanesulfonyl chloride (0.11 g, 0.92 mmol) dropwise at room temperature. After stirring for additional 1.5 h at room temperature, the resulting mixture was quenched with water (20 mL) at room temperature and extracted with EA (3 x 20 mL). The combined organic layers were washed with brine (2 x 20 mL), dried over anhydrous Na₂SO₄. After filtration, the filtrate was concentrated under reduced pressure. The residue was used in the next step directly without further purification: LCMS (ESI) calc'd for C₁₉H₂₈Cl₂N₂O₅S [M + H]⁺: 467, 469 (3 : 2), found 467, 469 (3 : 2).

### Step b:

To a stirred solution of tert-butyl 4-[(4,5-dichloro-2-methoxyphenyl)(methanesulfonamido) methyl] piperidine-1-carboxylate (crude) in DCM (5 mL) was added BBr₃ (0.51 g, 2.05 mmol) dropwise at 0 °C under nitrogen atmosphere. The reaction mixture was allowed to warm to room temperature and stirred under nitrogen atmosphere for 2 h. The resulting mixture was quenched with saturated aq. NaHCO₃ (5 mL) at 0 °C. The resulting mixture was concentrated under reduced pressure. The residue was purified by Prep-HPLC with the following conditions: Column: XBridge C18 OBD Prep Column 100 Å, 10 µm, 19 mm x 250 mm; Mobile Phase A: water with 20 mmol/L NH₄HCO₃, Mobile Phase B: ACN; Flow rate: 20 mL/min; Gradient: 30% B to 85% B in 9 min; Detector: UV 254/210 nm; Retention time: 7.84 min. The fractions containing desired product were collected and concentrated under reduced pressure to afford Compound 12 (*N*-((4,5-dichloro-2-hydroxyphenyl)(piperidin-4-yl)methyl)methanesulfonamide) as an off-white solid (32.9 mg, 12% overall two steps): LCMS (ESI) calc'd for C₁₃H₁₈Cl₂N₂O₃S [M + H]⁺: 353, 357 (3 : 2), found 353, 357 (3 : 2); ¹H NMR (300 MHz, CD₃OD) δ 7.18 (s, 1H), 6.79 (s, 1H), 4.25 (d, *J =* 8.9 Hz, 1H), 3.24-3.06 (m, 2H), 2.78-2.60 (m, 2H), 2.61 (s, 3H), 2.18-2.07 (m, 1H), 1.99-1.86 (m, 1H), 1.47-1.25 (m, 3H).

### Example 88. (which is a reference example) Compound 13 (4,5-dichloro-2-[piperidin-4-yl(1H-pyrazol-1-yl)methyl]phenol)

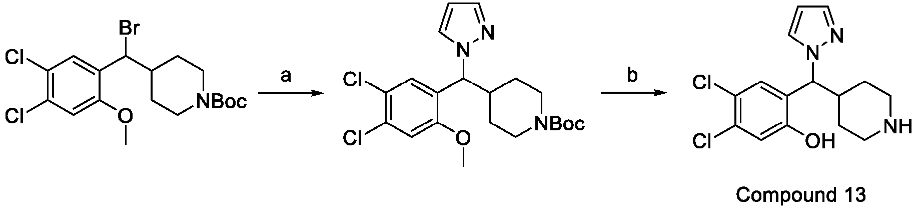

### Step a:

To a stirred mixture of tert-butyl 4-[bromo(4,5-dichloro-2-methoxyphenyl)methyl]piperidine-1-carboxylate (0.20 g, 0.44 mmol) and K₂CO₃(0.12 g, 0.88 mmol) in DMF (3 mL) was added 1H-pyrazole (60 mg, 0.88 mmol) at room temperature. The resulting mixture was stirred at room temperature for 1 h. The reaction was diluted with of water (20 mL). The resulting mixture was extracted with EA (3 x 20 mL). The combined organic layers were washed with brine (2 x 20 mL), dried over anhydrous Na₂SO₄. After filtration, the filtrate was concentrated under reduced pressure. The residue was purified by silica gel column chromatography, eluted with PE/EA (1/1) to afford tert-butyl 4-[(4,5-dichloro-2-methoxyphenyl)(1H-pyrazol-1-yl)methyl]piperidine-1-carboxylate as a light yellow oil (40 mg, 21%): LCMS (ESI) calc'd for C₂₁H₂₇Cl₂N₃O₃ [M + H]⁺: 440, 442 (3 : 2), found 440, 442 (3 : 2).

### Step b:

To a stirred solution of tert-butyl 4-[(4,5-dichloro-2-methoxyphenyl)(1H-pyrazol-1-yl)methyl]piperidine-1-carboxylate (40 mg, 0.09 mmol) in DCM (1 mL) was added BBr₃ (0.18 g, 0.73 mmol) at room temperature. The resulting mixture was stirred at room temperature for 1 h. The reaction was quenched with water (1 mL) at 0 °C. The resulting mixture was concentrated under reduced pressure. The residue was purified by Prep-HPLC with the following conditions: Column: XBridge C18 OBD Prep Column 100 Å, 10 µm, 19 mm x 250 mm; Mobile Phase A: Water with 20 mmoL/L NH₄HCO₃, Mobile Phase B: ACN; Flow rate: 20 mL/min; Gradient: 20% B to 80% B in 9 min; Detector: UV 254/220 nm; Retention time: 8.15 min. The fractions containing the desired product were collected and concentrated under reduced pressure to afford Compound 13 (4,5-dichloro-2-[piperidin-4-yl(1H-pyrazol-1-yl)methyl]phenol) as an off-white solid (5.5 mg, 23%): LCMS (ESI) calc'd for C₁₅H₁₇Cl₂N₃O [M + H]⁺: 326, 328 (3 : 2), found 326, 328 (3 : 2); ¹H NMR (300 MHz, CD₃OD) δ 7.75 (dd, *J =* 2.4, 0.7 Hz, 1H), 7.63-7.51 (m, 2H), 6.90 (s, 1H), 6.27 (t, *J =* 2.2 Hz, 1H), 5.45 (d, *J =* 11.0 Hz, 1H), 3.16-3.04 (m, 2H), 2.74-2.57 (m, 2H), 1.54-1.42 (m, 1H), 1.40-1.17 (m, 4H).

### Example 89. (which is a reference example) Compound 14 (N-[(4,5-dichloro-2-hydroxyphenyl)(1-methylpiperidin-4-yl)methyl]-2,2,2-trifluoroacetamide)

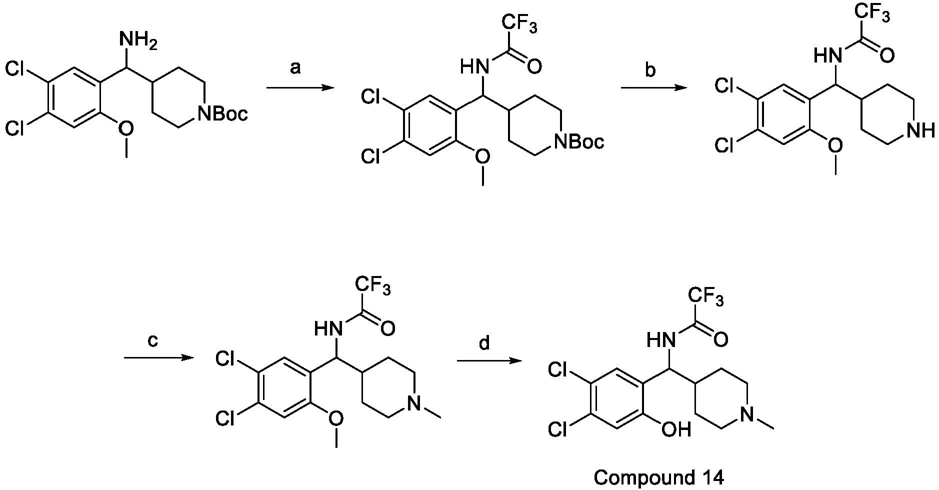

### Step a:

To a stirred solution of tert-butyl 4-[amino(4,5-dichloro-2-methoxyphenyl)methyl]piperidine-1-carboxylate (0.30 g, 0.77 mmol) and Et₃N (0.16 g, 1.54 mmol) in DCM (5 mL) was added TFAA (0.19 g, 0.92 mmol) at 0 °C. After stirring for additional 4 h at room temperature, the reaction solution was quenched with water (50 mL) and extracted with EA (3 x 30 mL). The combined organic layers were washed with brine (2 x 20 mL), dried over anhydrous Na₂SO₄. After filtration, the filtrate was concentrated under reduced pressure. The crude product was used in the next step directly without further purification: LCMS (ESI) calc'd for C₂₀H₂₅Cl₂F₃N₂O₄ [M + H]⁺: 485, 487 (3 : 2), found 485, 487 (3 : 2).

### Step b:

To a stirred solution of tert-butyl 4-[(4,5-dichloro-2-methoxyphenyl)(trifluoroacetamido)methyl]piperidine-1-carboxylate (crude) in DCM (2 mL) was added TFA (2 mL) at 0 °C. The reaction solution was allowed to warm to room temperature and stirred for 3 h. The resulting solution was concentrated under reduced pressure. The crude product (0.35 g as a TFA salt) was used in the next step directly without further purification: LCMS (ESI) calc'd for C₁₅H₁₇Cl₂F₃N₂O₂ [M + H]⁺: 385, 387 (3 : 2), found 385, 387 (3 : 2); ¹H NMR (300 MHz, CD₃OD) δ 7.46 (s, 1H), 7.18 (s, 1H), 5.04 (d, *J* = 10.2 Hz, 1H), 3.88 (s, 3H), 3.11 (d, *J =* 12.7 Hz, 1H), 2.98 (d, *J =* 12.7 Hz, 1H), 2.66-2.42 (m, 2H), 2.06-1.86 (m, 2H), 1.38-1.11 (m, 3H).

### Step c:

To a stirred solution of N-[(4,5-dichloro-2-methoxyphenyl)(piperidin-4-yl)methyl]-2,2,2-trifluoroacetamide (0.35 g, 0.91 mmol) and formaldehyde (33 mg, 1.09 mmol) in MeOH (10 mL) was added NaBH(OAc)₃ (0.58 g, 2.73 mmol) in portions at 0 °C under argon. The reaction mixture was allowed to warm to room temperature and stirred for additional 4 h. The resulting mixture was quenched with saturated aq. NH₄Cl (40 mL) and extracted with EA (3 x 40 mL). The combined organic layers were washed with brine (2 x 30 mL), dried over anhydrous Na₂SO₄ and filtered. The filtrate was concentrated under reduced pressure. The residue was purified by silica gel column chromatography, eluted with DCM/MeOH (6/1) to afford N-((4,5-dichloro-2-methoxyphenyl)(1-methylpiperidin-4-yl)methyl)-2,2,2-trifluoroacetamide as a yellow oil (0.22 g, 70% overall three steps): LCMS (ESI) calc'd for C₁₆H₁₉Cl₂F₃N₂O₂ [M + H]⁺: 399, 401 (3 : 2), found 399, 401 (3 : 2).

### Step d:

To a stirred solution of *N*-[(4,5-dichloro-2-methoxyphenyl)(1-methylpiperidin-4-yl)methyl]-2,2,2-trifluoroacetamide (0.22 g, 0.55 mmol) in DCM (5 mL) was added BBr₃ (0.83 g, 3.31 mmol) dropwise at 0 °C. The reaction mixture was allowed to warm to room temperature and stirred for 4 h. The reaction mixture was quenched with saturated aq. NH₄Cl (5 mL) at 0 °C. The resulting mixture was concentrated under reduced pressure. The residue was purified by reverse phase chromatography, eluted with 22% ACN in water (plus 0.05% TFA) to afford Compound 14 (*N*-[(4,5-dichloro-2-hydroxyphenyl)(1-methylpiperidin-4-yl)methyl]-2,2,2-trifluoroacetamide) as an off-white solid (0.14 g, 65%): LCMS (ESI) calc'd for C₁₅H₁₇Cl₂F₃N₂O₂ [M + H]⁺: 385, 387 (3 : 2), found 385, 387 (3 : 2); ¹H NMR (300 MHz, CD₃OD) δ 7.34 (s, 1H), 6.92 (s, 1H), 4.98 (d, *J =* 9.9 Hz, 1H), 2.98 (d, *J =* 11.8 Hz, 1H), 2.89 (d, *J* = 11.7 Hz, 1H), 2.31 (s, 3H), 2.18-1.84 (m, 4H), 1.51-1.23 (m, 3H); ¹⁹F NMR (282 MHz, CD₃OD) δ -77.04.

### Example 90. (which is a reference example) Compound 15 ((S)-2-(amino(piperidin-4-yl)methyl)-4,5-dichlorophenol trifluoroacetic acid) and Compound 16 ((R)-2-(amino(piperidin-4-yl)methyl)-4,5-dichlorophenol trifluoroacetic acid)

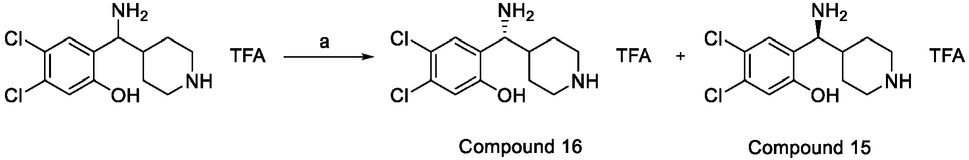

### Step a:

2-[amino(piperidin-4-yl)methyl]-4,5-dichlorophenol trifluoroacetic acid (0.30 g) was purified by Prep-chiral-HPLC with the following conditions: Column: CHIRALPAK IG, 20 x 250 mm, 5 µm; Mobile Phase A: Hexane (0.2% IPA), Mobile Phase B: EtOH; Flow rate: 20 mL/min; Gradient: 20% B in 13 min; Detector: UV 220/254 nm; Retention time: RT₁: 8.568 min, RT₂: 10.754 min.

The faster-eluting enantiomer was obtained as Compound 16 ((R)-2-(amino(piperidin-4-yl)methyl)-4,5-dichlorophenol trifluoroacetic acid) as an off-white solid (51 mg, 17%) at 8.568 min: LCMS (ESI) calc'd for C₁₂H₁₆Cl₂N₂O [M + H]⁺: 275, 275 (3 : 2), found 275, 275 (3 : 2); ¹H NMR (300 MHz, CD₃OD) δ 7.25 (s, 1H), 6.88 (s, 1H), 3.93 (d, *J =* 7.7 Hz, 1H), 3.34 (d, *J =* 12.8 Hz, 1H), 3.23 (d, *J =* 12.8 Hz, 1H), 2.91-2.69 (m, 2H), 2.20-1.89 (m, 2H), 1.63-1.45 (m, 1H), 1.51-1.25 (m, 2H); ¹⁹F NMR (282 MHz, CD₃OD) δ -76.92.

The slower-eluting enantiomer was obtained as Compound 15 ((S)-2-(amino(piperidin-4-yl)methyl)-4,5-dichlorophenol trifluoroacetic acid) as a light yellow solid (37.2 mg, 12%) at 10.754 min: LCMS (ESI) calc'd for C₁₂H₁₆Cl₂N₂O [M + H]⁺: 275, 275 (3 : 2), found 275, 275 (3 : 2); ¹H NMR (300 MHz, CD₃OD) δ 7.19 (s, 1H), 6.84 (s, 1H), 3.88 (d, *J =* 7.7 Hz, 1H), 3.21 (d, *J =* 12.6 Hz, 1H), 3.10 (d, *J =* 12.6 Hz, 1H), 2.80-2.56 (m, 2H), 2.04-1.86 (m, 2H), 1.57-1.41 (m, 1H), 1.40-1.19 (m, 2H); ¹⁹F NMR (282 MHz, CD₃OD) δ -76.92.

### Example 91. (which is a reference example) Compound 18 (2-(4,5-dichloro-2-hydroxyphenyl)-2-(piperidin-4-yl)acetamide)

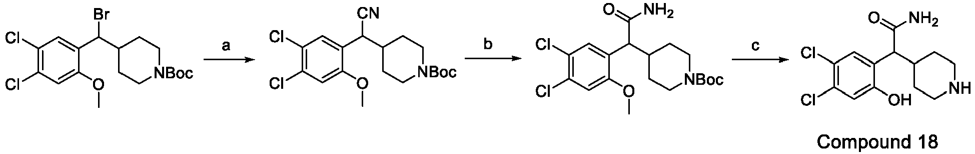

### Step a:

To a stirred solution of tert-butyl 4-[bromo(4,5-dichloro-2-methoxyphenyl)methyl]piperidine-1-carboxylate (0.20 g, 0.44 mmol) in DMSO (3 mL) was added KCN (0.14 g, 2.21 mmol) at room temperature. The mixture was stirred for 16 h at 40 °C. The reaction was quenched with saturated aq. FeSO₄ (5 mL) at room temperature. The resulting mixture was extracted with EA (4 x 20 mL). The combined organic layers were washed with brine (4 x 20 mL), dried over anhydrous Na₂SO₄. After filtration, the filtrate was concentrated under reduced pressure. The residue was purified by silica gel column chromatography, eluted with PE/EA (9/1) to afford tert-butyl 4-[cyano(4,5-dichloro-2-methoxyphenyl)methyl]piperidine-1-carboxylate as a yellow oil (60 mg, 34%): LCMS (ESI) calc'd for C₁₉H₂₄Cl₂N₂O₃ [M + H - 15]⁺: 399, 401 (3 : 2), found 399, 401 (3 : 2); ¹H NMR (300 MHz, CD₃OD) δ 7.44 (s, 1H), 7.22 (s, 1H), 4.20-3.99 (m, 3H), 3.86 (s, 3H), 2.80-2.56 (m, 2H), 1.77 (d, *J* = 13.3 Hz, 1H), 1.53-1.44 (m, 1H), 1.42 (s, 9H), 1.32-1.16 (m, 3H).

### Step b:

To a stirred mixture of *tert*-butyl 4-[cyano(4,5-dichloro-2-methoxyphenyl)methyl]piperidine-1-carboxylate (60 mg, 0.15 mmol) and KOH (84 mg, 1.50 mmol) in MeOH (1 mL) and water (1 mL) was added H₂O₂ (51.1 mg, 1.50 mmol) dropwise at room temperature. The mixture was stirred for 16 h at room temperature. The resulting mixture was concentrated under reduced pressure. The residue was purified by silica gel column chromatography, eluted with PE/EA (2/1) to afford tert-butyl 4-[carbamoyl(4,5-dichloro-2-methoxyphenyl)methyl]piperidine-1-carboxylate as an off-white solid (40 mg, 64%): LCMS (ESI) calc'd for C₁₉H₂₆Cl₂N₂O₄ [M + H]⁺: 417, 419 (3 : 2), found 417, 419 (3 : 2); ¹H NMR (300 MHz, CD₃OD) δ 7.62 (s, 1H), 7.11 (s, 1H), 4.08-3.99 (m, 2H), 3.92 (d, *J* = 13.4 Hz, 1H), 3.83 (s, 3H), 2.87-2.54 (m, 2H), 2.13-2.00 (m, 1H), 1.81 (d, *J =* 13.1 Hz, 1H), 1.41 (s, 9H), 1.3-1.15 (m, 3H).

### Step c:

To a stirred solution of *tert*-butyl 4-[carbamoyl(4,5-dichloro-2-methoxyphenyl)methyl]piperidine-1-carboxylate (40 mg, 0.10 mmol) in DCM (1 mL) was added BBr₃ (0.14 g, 0.58 mmol) at room temperature. The resulting mixture was stirred at room temperature for 3 h. The reaction was quenched with water (1 mL) at 0 °C. The resulting mixture was concentrated under reduced pressure. The residue was purified by Prep-HPLC with the following conditions: Column: XBridge C18 OBD Prep Column 100 Å, 10 µm, 19 mm x 250 mm; Mobile Phase A: Water with 20 mmoL/L NH₄HCO₃, Mobile Phase B: ACN; Flow rate: 20 mL/min; Gradient: 15% B to 35% B in 9 min; Detector: UV 254/210 nm; Retention time: 7.68 min. The fractions containing the desired product were collected and concentrated under reduced pressure to afford Compound 18 (2-(4,5-dichloro-2-hydroxyphenyl)-2-(piperidin-4-yl)acetamide) as an off-white solid (14.7 mg, 48%): LCMS (ESI) calc'd for C₁₃H₁₆Cl₂N₂O₂ [M + H]⁺: 303, 305 (3 : 2), found 303, 305 (3 : 2); ¹H NMR (300 MHz, CD₃OD) δ 7.32 (s, 1H), 6.86 (s, 1H), 3.59 (d, *J* = 10.8 Hz, 1H), 3.24-2.99 (m, 2H), 2.82-2.55 (m, 2H), 2.18 (t, *J* = 11.3 Hz, 1H), 1.91 (t, *J =* 13.4 Hz, 1H), 1.46-1.04 (m, 3H).

### Example 92. Compound 19 ((S)-1-(4-(amino(4,5-dichloro-2-hydroxyphenyl)methyl)piperidin-1-yl)ethanone) and Compound 20 ((R)-1-(4-(amino(4,5-dichloro-2-hydroxyphenyl)methyl)piperidin-1-yl)ethanone)

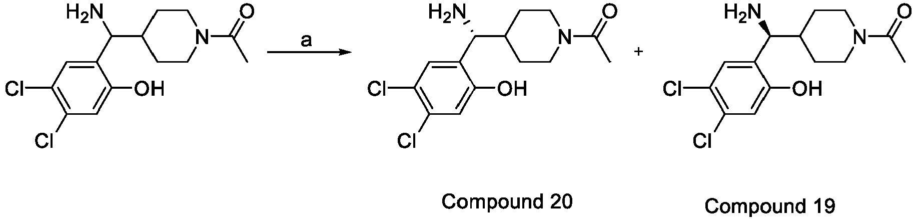

### Step a:

1-(4-(amino(4,5-dichloro-2-hydroxyphenyl)methyl)piperidin-1-yl)ethanone (Compound 9, Example 84) (0.23 g) was purified by Prep-chiral-HPLC with the following conditions: Column: CHIRALPAK IG UL001, 20 x 250 mm, 5 µm; Mobile Phase A: Hex, Mobile Phase B: EtOH; Flow rate: 20 mL/min; Gradient: 20% B in 19 min; Detector: UV 220/254 nm; Retention time: RT₁: 12.36 min, RT₂: 15.05 min.

The faster-eluting enantiomer was obtained as Compound 20 ((R)-1-(4-(amino(4,5-dichloro-2-hydroxyphenyl)methyl)piperidin-1-yl)ethanone) as an off-white solid (60.2 mg, 27%) at 12.36 min: LCMS (ESI) calc'd for C₁₄H₁₈Cl₂N₂O₂ [M + H]⁺: 317, 319 (3 : 2), found 317, 319 (3 : 2); ¹H NMR (300 MHz, CD₃OD) δ 7.22 *(d, J* = 2.7 Hz, 1H), 6.86 (d, *J =* 1.8 Hz, 1H), 4.55 (dd, *J =* 28.9, 13.2 Hz, 1H), 4.04-03.79 (m, 2H), 3.18-2.93 (m, 1H), 2.66-2.44 (m, 1H), 2.08 (*J* = 7.0 Hz, 3H) 5H), 2.04-1.93 (m, 2H), 1.51-1.34 (m, 1H), 1.30-1.10 (m, 2H).

The slower-eluting enantiomer was obtained as Compound 19 ((*S*)-1-(4-(amino(4,5-dichloro-2-hydroxyphenyl)methyl)piperidin-1-yl)ethanone) as an off-white solid (70.8 mg, 30%) at 15.05 min: LCMS (ESI) calc'd for C₁₄H₁₈Cl₂N₂O₂ [M + H]⁺: 317, 319 (3 : 2), found 317, 319 (3 : 2); ¹H NMR (300 MHz, CD₃OD) δ 7.22 *(d, J* = 2.7 Hz, 1H), 6.86 (d, *J =* 1.8 Hz, 1H), 4.55 (dd, *J =* 28.6, 13.0 Hz, 1H), 4.07-3.82 (m, 2H), 3.15-2.92 (m, 1H), 2.63-2.44 (m, 1H), 2.06 (d, *J* = 7.0 Hz, 3H), 2.06-1.92 (m, 2H), 1.51-1.34 (m, 1H), 1.30-1.10 (m, 2H).

### Example 93. (which is a reference example) Compound 21 (2-(amino(1-methylpiperidin-4-yl)methyl)-4,5-dichlorophenol)

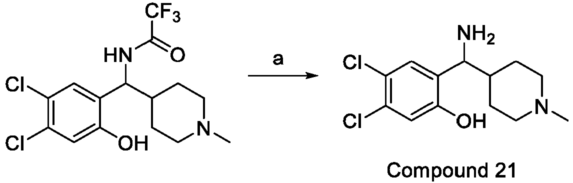

### Step a:

To a stirred solution of N-[(4,5-dichloro-2-hydroxyphenyl)(1-methylpiperidin-4-yl)methyl]-2,2,2-trifluoroacetamide (Compound 14, Example 89) (0.12 g, 0.31 mmol) in MeOH (5 mL) was added a solution of NaOH (62 mg, 1.56 mmol) in water (1 mL) dropwise at room temperature. The reaction solution was stirred for 16 h at room temperature. The resulting mixture was concentrated under reduced pressure. The residue was purified by Prep-HPLC with the following conditions: Column: XBridge C18 OBD Prep Column 100 Å, 10 µm, 19 mm x 250 mm; Mobile Phase A: water with 20 mmol/L NH₄HCO₃, Mobile Phase B: ACN; Flow rate: 20 mL/min; Gradient: 17% B to 52% B in 9 min; Detector: UV 210 nm; Retention time: 8.58 min. The fractions containing desired product were collected and concentrated under reduced pressure to afford Compound 21 (2-(amino(1-methylpiperidin-4-yl)methyl)-4,5-dichlorophenol) as an off-white solid (19 mg, 21%): LCMS (ESI) calc'd for C₁₃H₁₈Cl₂N₂O [M + H]⁺: 289, 291 (3 : 2), found 289, 291 (3 : 2); ¹H NMR (300 MHz, CD₃OD) δ 7.18 (s, 1H), 6.83 (s, 1H), 3.87 (d, *J* = 7.9 Hz, 1H), 3.01-2.77 (m, 2H), 2.26 (s, 3H), 2.09-1.86 (m, 3H), 1.84-1.67 (m, 1H), 1.47-1.25 (m, 3H).

### Example 94. (which is a reference example) Compound 22 (N-((4,5-dichloro-2-hydroxyphenyl)(piperidin-4-yl)methyl)-2,2,2-trifluoroacetamide)

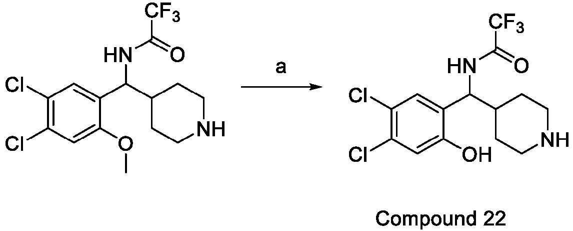

### Step a:

To a stirred solution of *N*-[(4,5-dichloro-2-methoxyphenyl)(piperidin-4-yl)methyl]-2,2,2-trifluoroacetamide (Example 89, step b) (80 mg, 0.21 mmol) in DCM (2 mL) was added tribromoborane (0.42 g, 1.66 mmol) dropwise at 0 °C. After stirring for additional 3 h at room temperature, the reaction mixture was quenched with saturated aq. NaHCO₃ (3 mL) at 0 °C. The resulting mixture was concentrated under reduced pressure. The residue was purified by Prep-HPLC with the following conditions: Column: XBridge C18 OBD Prep Column 100 Å, 10 µm, 19 mm x 250 mm; Mobile Phase A: water with 20 mmol/L NH₄HCO₃, Mobile Phase B: ACN; Flow rate: 20 mL/min; Gradient: 18% B to 55% B in 9 min; Detector: UV 210 nm; Retention time: 8.08 min. The fractions containing desired product were collected and concentrated under reduced pressure to afford Compound 22 (*N*-((4,5-dichloro-2-hydroxyphenyl)(piperidin-4-yl)methyl)-2,2,2-trifluoroacetamide) as an off-white solid (20 mg, 26%): LCMS (ESI) calc'd for C₁₄H₁₅Cl₂F₃N₂O₂ [M + H]⁺: 371, 373 (3 : 2), found 371, 373 (3 : 2); ¹H NMR (300 MHz, CD₃OD) δ 7.24 (d, *J =* 3.0 Hz, 1H), 6.86 (s, 1H), 4.86 (d, *J =* 9.2 Hz, 1H), 3.29-3.13 (m, 2H), 2.92-2.58 (m, 2H), 2.35-2.02 (m, 1H), 1.96-1.80 (m, 1H), 1.56-1.25 (m, 3H); ¹⁹F NMR (282 MHz, CD₃OD) δ -77.08.

### Example 95. Compound 23 (1-(4-((4,5-dichloro-2-hydroxyphenyl)(pyrrolidin-1-yl)methyl)piperidin-1-yl)ethanone trifluoroacetic acid)

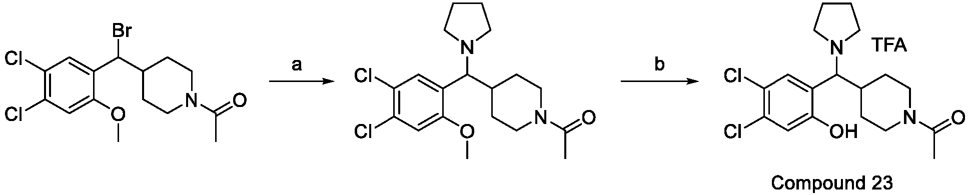

### Step a:

To a stirred solution of 1-[4-[bromo(4,5-dichloro-2-methoxyphenyl)methyl]piperidin-1-yl]ethan-1-one (0.40 g, 1.01 mmol) and pyrrolidine (0.72 g, 10.10 mmol) in DMSO (5 mL) was added Na₂CO₃ (0.32 g, 3.04 mmol) at room temperature. The resulting mixture was allowed to warm to 50 °C and stirred for 3 h. After cooling to room temperature, the reaction mixture was diluted with water (50 mL). The resulting mixture was extracted with EA (3 x 30 mL). The combined organic layers were washed with water (3 x 20 mL), dried over anhydrous Na₂SO₄. After filtration, the filtrate was concentrated under reduced pressure. The residue was purified by Prep-TLC, eluted with CH₂Cl₂/MeOH (20/1) to afford 1-[4-[(4,5-dichloro-2-methoxyphenyl)(pyrrolidin-1-yl)methyl]piperidin-1-yl]ethan-1-one as an off-white solid (0.29 g, 74%): LCMS (ESI) calc'd for C₁₉H₂₆Cl₂N₂O₂ [M + H]+: 385, 387 (3 : 2), found 385, 387 (3 : 2).

### Step b:

A solution of 1-[4-[(4,5-dichloro-2-methoxyphenyl)(pyrrolidin-1-yl)methyl]piperidin-1-yl]ethan-1-one (0.27 g, 0.70 mmol) in hydrogen iodide (4 mL) was stirred for 20 h at 100 °C. The resulting mixture was concentrated under reduced pressure. The residue was purified by Prep-HPLC with the following conditions: Column: XBridge C18 OBD Prep Column 100 Å, 10 µm, 19 mm x 250 mm; Mobile Phase A: water (plus 0.05% TFA), Mobile Phase B: ACN; Flow rate: 20 mL/min; Gradient: 20% B to 50% B in 8 min; Detector: UV 254/210 nm; Retention time: 6.12 min. The fractions containing desired product were collected and concentrated under reduced pressure to afford Compound 23 (1-[4-[(4,5-dichloro-2-hydroxyphenyl)(pyrrolidin-1-yl)methyl]piperidin-1-yl]ethanone trifluoroacetic acid) as a light yellow solid (19 mg, 7%): LCMS (ESI) calc'd for C₁₈H₂₄Cl₂N₂O₂ [M + H]⁺: 371, 373 (3 : 2), found 371, 373 (3 : 2); ¹H NMR (400 MHz, CD₃OD) δ 7.31 (s, 1H), 7.12 (s, 1H), 4.65-4.49 (m, 1H), 4.34-4.29 (m, 1H), 4.26-3.71 (m, 2H), 3.52-3.34 (m, 2H), 3.25-2.95 (m, 2H), 2.73-2.51 (m, 2H), 2.29-1.86 (m, 9H), 1.29-0.92 (m, 2H); ¹⁹F NMR (376 MHz, CD₃OD) δ -76.99.

### Example 96. (which is a reference example) Compound 24 ((R)-4,5-dichloro-2-(hydroxy(1-methylpiperidin-4-yl)methyl)phenol) and Compound 319 ((5)-4,5-dichloro-2-(hydroxy(1-methylpiperidin-4-yl)methyl)phenol)

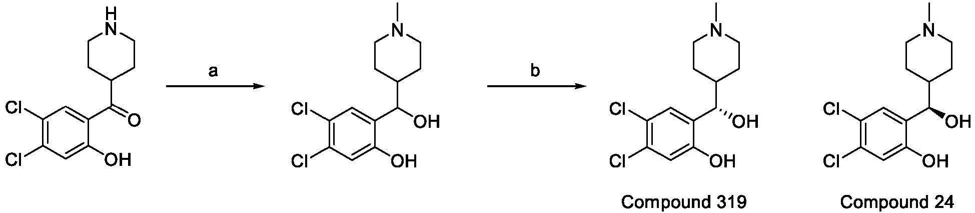

### Step a:

To a stirred mixture of 4,5-dichloro-2-[(piperidin-4-yl)carbonyl]phenol (Intermediate 2, Example 2) (0.31 g, 1.12 mol), AcOH (74 mg, 1.24 mol) and HCHO (41 mg, 1.35 mmol) in MeOH (5 mL) was added NaBH(OAc)₃ (0.71 g, 3.37 mol) in portions at room temperature. The resulting mixture was stirred for 1 h at room temperature. The reaction was quenched with water (30 mL) at room temperature. The resulting mixture was extracted with EA (3 x 100 mL). The combined organic layers was washed with brine (2 x 20 mL) and dried over anhydrous Na₂SO₄. After filtration, the filtrate was concentrated under reduced pressure. The residue was purified by Prep-HPLC with the following conditions: Column: XBridge C18 OBD Prep Column, 100 Å, 10 µm, 19 mm x 250 mm; Mobile Phase A: Water with 20 mmoL/L NH₄HCO₃, Mobile Phase B: ACN; Flow rate: 20 mL/min; Gradient: 16% B to 54% B in 9 min; Detector: UV 254/210 nm; Retention Time: 7.95 min. The fractions containing the desired product were collected and concentrated under reduced pressure to afford 4,5-dichloro-2-[hydroxy(1-methylpiperidin-4-yl)methyl]phenol as an off-white solid (70 mg, 21%): LCMS (ESI) calc'd for C₁₃H₁₇Cl₂NO₂ [M + H]⁺ 290, 292 (3 : 2) found 290, 292 (3 : 2); ¹H NMR (300 MHz, CD₃OD) δ 7.34 (s, 1H), 6.87 (s, 1H), 4.72 (d, *J =* 6.5 Hz, 1H), 3.00-2.83 (m, 2H), 2.27 (s, 3H), 2.11-1.93 (m, 2H), 1.93-1.81 (m, 1H), 1.71-1.55 (m, 1H), 1.55-1.37 (m, 3H).

### Step b:

4,5-Ddichloro-2-(hydroxy(1-methylpiperidin-4-yl)methyl)phenol (25 mg) was purified by Prep-chiral-HPLC with the following conditions: Column: Column: CHIRALPAK AD-H, 2 x 25 cm; Mobile Phase A: Hexane (0.1%DEA); Mobile Phase B: IPA; Flow rate: 20 mL/min; Gradient: 5% B to 5% B in 20 min; Detector: UV 220/254 nm; Retention time: RT₁: 13.55 min, RT₂: 16.98 min; Injection Volume: 0.3 mL.

The faster-eluting enantiomer was obtained as Compound 24 ((*R*)-4,5-dichloro-2-(hydroxy(1-methylpiperidin-4-yl)methyl)phenol) as an off-white solid (7.7 mg, 31%) at 16.98 min: LCMS (ESI) calc'd for C₁₃H₁₇Cl₂NO₂ [M + H]⁺: 290, 292 (3 : 2), found 290, 292 (3 : 2); ¹H NMR (300 MHz, CD₃OD) δ 7.34 (s, 1H), 6.87 (s, 1H), 4.72 (d, *J =* 6.5 Hz, 1H), 2.93 (t, *J* = 12.0 Hz, 2H), 2.28 (s, 3H), 2.09-1.96 (m, 2H), 1.94-1.78 (m, 1H), 1.75-1.33 (m, 4H).

The slower-eluting enantiomer was obtained as Compound 319 ((S)-4,5-dichloro-2-(hydroxy(1-methylpiperidin-4-yl)methyl)phenol) as an off-white solid (8.3 mg, 33%) at 16.98 min: LCMS (ESI) calc'd for C₁₃H₁₇Cl₂NO₂ [M + H]⁺: 290, 292 (3 : 2), found 290, 292 (3 : 2); ¹H NMR (300 MHz, CD₃OD) δ 7.34 (s, 1H), 6.87 (s, 1H), 4.72 (d, *J =* 6.5 Hz, 1H), 2.93 (t, *J* = 12.0 Hz, 2H), 2.28 (s, 3H), 2.09-1.96 (m, 2H), 1.94-1.78 (m, 1H), 1.75-1.33 (m, 4H).

### Example 97. (which is a reference example) Compound 25 4,5-dichloro-2-(1-hydroxy-1-(piperidin-4-yl)ethyl)phenol isomer 1 and Compound 26 4,5-dichloro-2-(1-hydroxy-1-(piperidin-4-yl)ethyl)phenol isomer 2

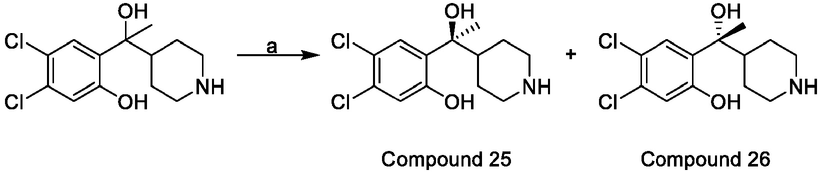

### Step a:

4,5-Dichloro-2-(1-hydroxy-1-(piperidin-4-yl)ethyl)phenol (20 mg, 0.07 mmol) was separated by Prep-SFC with the following conditions: Column: Phenomenex Lux Cellulose-4, AXIA Packed, 2.12 x 25 cm, 5 µm; Mobile Phase A: CO₂ (70%), Mobile Phase B: MeOH (2 mmol NH₃ in MeOH) 30%; Flow rate: 40 mL/min; Detector: UV 220 nm; Retention time: RT₁: 4.9 min; RT₂: 5.8 min.

The faster-eluting enantiomer was obtained as Compound 25 (4,5-dichloro-2-(1-hydroxy-1-(piperidin-4-yl)ethyl)phenol isomer 1) as an off-white solid (3.7 mg, 18%) at 4.9 min: LCMS (ESI) calc'd for C₁₃H₁₇Cl₂NO₂ [M + 1]⁺: 290, 292 (3 : 2), found 290, 292 (3 : 2); ¹H NMR (400 MHz, CD₃OD) δ 7.16 (s, 1H), 6.74 (s, 1H), 3.30-3.18 (m, 2H), 2.82-2.70 (m, 2H), 2.06 (t, *J =* 12.2 Hz, 1H), 1.82-1,68 (m, 2H), 1.54-1.47 (m, 5H).

The slower-eluting enantiomer was obtained as Compound 26 (4,5-dichloro-2-(1-hydroxy-1-(piperidin-4-yl)ethyl)phenol isomer 2) as an off-white solid (3.9 mg, 19%) at 5.8 min: LCMS (ESI) calc'd for C₁₃H₁₇Cl₂NO₂ [M + H]⁺: 290, 292 (3 : 2), found 290, 292 (3 : 2); ¹H NMR (400 MHz, CD₃OD) δ 7.16 (s, 1H), 6.74 (s, 1H), 3.30-3.18 (m, 2H), 2.82-2.70 (m, 2H), 2.06 (t, *J =* 12.2 Hz, 1H), 1.82-1,68 (m, 2H), 1.54-1.47 (m, 5H).

### Example 98. Compound 27 (1-[4-[(4,5-dichloro-2-hydroxyphenyl)[(pyridin-2-yl)amino]methyl]piperidin-1-yl]ethan-1-one trifluoroacetic acid)

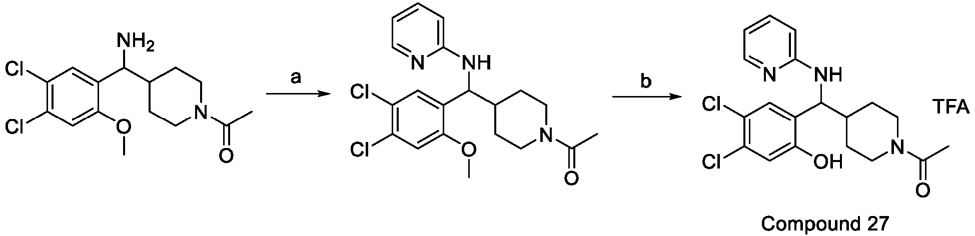

### Step a:

To a stirred mixture of 1-[4-[amino(4,5-dichloro-2-methoxyphenyl)methyl]piperidin-1-yl]ethan-1-one (0.20 g, 0.60 mmol) and 2-bromopyridine (0.14 g, 0.91 mmol) in 1,4-dioxane (3 mL) were added XantPhos (35 mg, 0.06 mmol), *t*-BuONa (0.17 g, 1.81 mmol) and Pd₂(dba)₃·CHCl₃ (63 mg, 0.06 mmol) at room temperature under argon atmosphere. The mixture was stirred for 2 h at 80 °C under argon atmosphere. The mixture was allowed to cool down to room temperature. The resulting mixture was concentrated under reduced pressure. The residue was purified by silica gel column chromatography, eluted with PE/EA (7/1) to afford *N*-[(4,5-dichloro-2-methoxyphenyl)[1-(prop-1-en-2-yl)piperidin-4-yl]methyl]pyridin-2-amine as a yellow oil (56 mg, 18%): LCMS (ESI) calc'd for C₂₀H₂₃Cl₂N₃O₂ [M + H]⁺: 408, 410 (3 : 2), found 408, 410 (3 : 2).

### Step b:

To a stirred solution of *N*-[(4,5-dichloro-2-methoxyphenyl)(piperidin-4-yl)methyl]pyridin-2-amine (56.00 mg, 0.15 mmol in DCM (1 mL) was added BBr₃ (0.23 g, 0.92 mmol) at room temperature. The reaction was stirred at room temperature for 3 h. The reaction was quenched with water (1 mL) at 0 °C. The resulting mixture was concentrated under reduced pressure. The residue was purified by Prep-HPLC with the following conditions: Column: XBridge C18 OBD Prep Column 100 Å, 10 µm, 19 mm x 250 mm; Mobile Phase A: Water (plus 0.05% TFA), Mobile Phase B: ACN; Flow rate: 20 mL/min; Gradient: 20% B to 38% B in 8 min; Detector: UV 254/210 nm; Retention time: 7.38 min. The fractions containing the desired product were collected and concentrated under reduced pressure to afford Compound 27 (1-[4-[(4,5-dichloro-2-hydroxyphenyl)[(pyridin-2-yl)amino]methyl]piperidin-1-yl]ethan-1-one trifluoroacetic acid) as an off-white solid (10 mg, 16%): LCMS (ESI) calc'd for C₁₉H₂₁Cl₂N₃O₂ [M + H]⁺: 394, 396 (3 : 2), found 394, 396 (3 : 2); ¹H NMR (400 MHz, CD₃OD) δ 7.99-7.92 (m, 1H), 7.90 (d, *J =* 6.4 Hz, 1H), 7.46 (d, *J =* 6.9 Hz, 1H), 7.10 (d, *J =* 9.2 Hz, 1H), 7.03 (s, 1H), 6.93 (t, *J =* 6.8 Hz, 1H), 4.78 (t, *J =* 9.1 Hz, 1H), 4.57 (dd, *J =* 31.7, 13.3 Hz, 1H), 3.98 (dd, *J =* 30.2, 13.7 Hz, 1H), 3.12 (dt, *J =* 25.8, 13.4 Hz, 1H), 2.63 (dt, *J =* 24.5, 12.4 Hz, 1H), 2.42-2.30 (m, 1H), 2.11 (d, *J =* 6.3 Hz, 3H), 2.08-1.97 (m, 1H), 1.55-1.18 (m, 3H); ¹⁹F NMR (376 MHz, CD₃OD) δ -77.02.

### Example 99. Compound 28 (1-[4-[2-amino-1-(4,5-dichloro-2-hydroxyphenyl)ethyl]piperidin-1-yl]ethan-1-one)

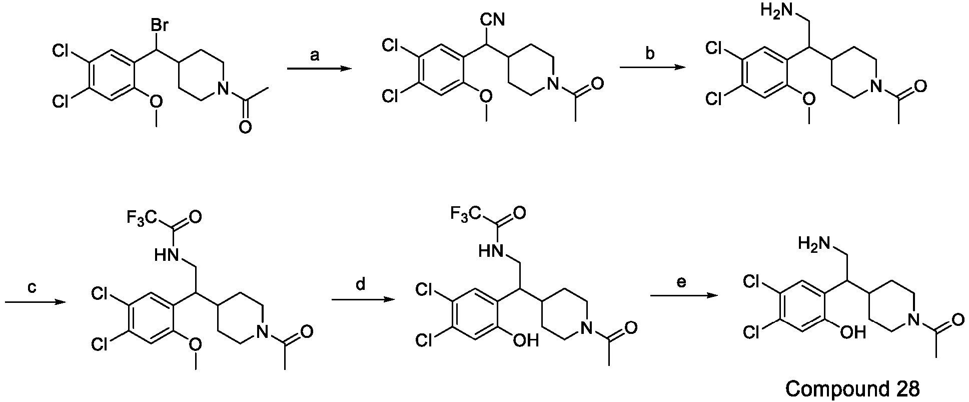

### Step a:

To a solution of 1-[4-[bromo(4,5-dichloro-2-methoxyphenyl)methyl]piperidin-1-yl]ethan-1-one (0.50 g, 1.27 mmol) in DMSO (5 mL) was added KCN (0.25 g, 3.81 mmol) at room temperature. The reaction mixture was allowed to warm to 50 °C and stirred for 19 h. After cooling to room temperature, the resulting mixture was quenched with saturated aq. FeSO₄ (50 mL) and was extracted with EA (3 x 30 mL). The combined organic layers were washed with water (3 x 20 mL), dried over anhydrous Na₂SO₄. After filtration, the filtrate was concentrated under reduced pressure. The residue was purified by Prep-TLC, eluted with PE/EA (5/1) to afford 2-(1-acetylpiperidin-4-yl)-2-(4,5-dichloro-2-methoxyphenyl)acetonitrile as an off-white solid (0.37 g, 86%): LCMS (ESI) calc'd for C₁₆H₁₈Cl₂N₂O₂ [M + H]⁺: 341, 343 (3 : 2), found 341, 343 (3 : 2); ¹H-NMR (400 MHz, DMSO-d₆) δ 7.54 (d, *J =* 2.1 Hz, 1H), 7.42 (s, 1H), 4.38 (dd, *J =* 35.2, 13.3 Hz, 1H), 4.22 (d, *J =* 8.2 Hz, 1H), 3.92 (s, 3H), 3.87-3.69 (m, 1H), 3.09-2.86 (m, 1H), 2.49-2.38 (m, 1H), 2.25-2.08 (m, 1H), 1.97 (s, 3H), 1.91-1.74 (m, 1H), 1.43-1.31 (m, 1H), 1.35-1.26 (m, 1H), 1.08-0.94 (m, 1H).

### Step b:

To a stirred solution of 2-(1-acetylpiperidin-4-yl)-2-(4,5-dichloro-2-methoxyphenyl)acetonitrile (0.37 g, 1.09 mmol) and NH₃·H₂O (0.5 mL, 12.84 mmol, 28% in water) in MeOH (5 mL) was added Raney nickel (37 mg, 0.43 mmol) at room temperature under nitrogen atmosphere. After the addition, the reaction mixture was degassed under hydrogen for three times and stirred for 20 h at room temperature under hydrogen at 1.5 atm. The resulting mixture was filtrated and filter cake washed with MeOH (3 x 10 mL). The filtrate was concentrated under reduced pressure. The residue was purified by Prep-TLC, eluted with CH₂Cl₂/MeOH (15/1) to afford 1-[4-[2-amino-1-(4,5-dichloro-2-methoxyphenyl)ethyl]piperidin-1-yl]ethan-1-one as a green oil (0.33 g, 87%): LCMS (ESI) calc'd for C₁₆H₂₂Cl₂N₂O₂ [M + H]⁺: 345, 347 (3 : 2), found 345, 347 (3 : 2); ¹H-NMR (300 MHz, CD₃OD) δ 7.30 (d, *J =* 2.1 Hz, 1H), 7.16 (s, 1H), 4.47 (dd, *J =* 35.2 13.3 Hz, 1H), 3.98-3.75 (m,1H), 3.85 (s, 3H), 3.13-2.88 (m, 3H), 2.68-2.41 (m, 1H), 2.06 (d, *J =* 10.1 Hz, 3H), 1.99-1.83 (m, 2H), 1.43-0.85 (m, 4H).

### Step c:

To a stirred solution of 1-[4-[2-amino-1-(4,5-dichloro-2-methoxyphenyl)ethyl]piperidin-1-yl]ethan-1-one (0.16 g, 0.46 mmol) and Et₃N (70 mg, 0.70 mmol) in CH₂Cl₂ (2 mL) was added TFAA (0.11 g, 0.51 mmol) at room temperature. The reaction solution was stirred for 1 h at room temperature. The resulting solution was concentrated under reduced pressure to afford *N*-[2-(1-acetylpiperidin-4-yl)-2-(4,5-dichloro-2-methoxyphenyl)ethyl]-2,2,2-trifluoroacetamide as a light yellow oil (0.17 g, crude), which was used in next step without further purification: LCMS (ESI) calc'd for C₁₈H₂₁Cl₂F₃N₂O₃ [M + H]⁺: 441, 443 (3 : 2), found 441, 443 (3 : 2).

### Step d:

To a solution of *N*-[2-(1-acetylpiperidin-4-yl)-2-(4,5-dichloro-2-methoxyphenyl)ethyl]-2,2,2-trifluoroacetamide (0.17 g, crude) in DCM (5 mL) was added BBr₃ (1.00 g, 3.97 mmol) at room temperature. After stirring for 1 h at room temperature, the resulting solution was quenched with saturated aq. Na₂CO₃ (20 mL) at room temperature and extracted with EA (3 x 20 mL). The combined organic layers were washed with water (3 x 10 mL), dried over anhydrous Na₂SO₄. After filtration, the filtrate was concentrated under reduced pressure. The residue was purified by Prep-TLC, eluted with PE/EA (1/3) to afford N-[2-(1-acetylpiperidin-4-yl)-2-(4,5-dichloro-2-hydroxyphenyl)ethyl]-2,2,2-trifluoroacetamide as an off-white solid (0.14 g, 83%): LCMS (ESI) calc'd for C₁₇H₁₉Cl₂F₃N₂O₃ [M + H]⁺: 427, 429, found 427, 429; ¹H NMR (400 MHz, CD₃OD) δ 7.21 (d, *J =* 2.5 Hz, 1H), 6.92 (s, 1H), 4.49 (dd, *J =* 52.9, 13.4 Hz, 1H), 3.91 (dd, *J =* 51.4, 13.9 Hz, 1H), 3.77-3.67 (m, 1H), 3.64-3.50 (m, 1H), 3.23-2.96 (m, 2H), 2.71-2.52 (m, 1H), 2.17-2.05 (m, 2H), 2.10 (d, *J =* 15.8 Hz, 3H), 1.56-1.42 (m, 1H), 1.38-1.20 (m, 1H), 1.14-0.92 (m, 1H); ¹⁹F-NMR (376 MHz, CD₃OD) δ -77.41.

### Step e:

To a stirred solution of *N*-[2-(1-acetylpiperidin-4-yl)-2-(4,5-dichloro-2-hydroxyphenyl)ethyl]-2,2,2-trifluoroacetamide (0.14 g, 0.33 mmol) in MeOH/water (5 mL/1 mL) was added KOH (37 mg, 0.66 mmol) at room temperature. The reaction mixture was stirred for 2 h at room temperature. The resulting mixture was concentrated under reduced pressure. The residue was purified by Prep-HPLC with the following conditions: Column: XBridge C18 OBD, 100 Å, 10 µm, 19 mm x 250 mm; Mobile Phase A: water with 20 mmol/L NH₄HCO₃, Mobile Phase B: ACN; Flow rate: 20 mL/min; Gradient: 25% B to 55% B in 8 min; Detector: UV 254/210 nm; Retention time: 6.22 min. The fractions containing desired product were collected and concentrated under reduced pressure to afford Compound 28 (1-[4-[2-amino-1-(4,5-dichloro-2-hydroxyphenyl)ethyl]piperidin-1-yl]ethan-1-one) as an off-white solid (43 mg, 40%): LCMS (ESI) calc'd for C₁₅H₂₀Cₗ₂N₂O₂ [M + H]⁺: 331, 333 (3 : 2), found 331, 333 (3 : 2); ¹H NMR (400 MHz, CD₃OD) ¹H NMR (400 MHz, CD₃OD) δ 7.10 (s, 1H), 6.85 (d, *J =* 7.5 Hz, 1H), 4.52 (dd, *J =* 51.7, 12.8 Hz, 1H), 3.93 (dd, *J =* 48.6, 13.8 Hz, 1H), 3.28-2.99 (m, 3H), 2.71-2.49 (m, 2H), 2.27-2.17 (m, 1H), 2.08 (d, *J =* 13.9 Hz, 3H), 2.05-1.97 (m, 1H), 1.45-0.90 (m, 3H).

### Example 100. (which is a reference example) Compound 29 (1-((4,5-dichloro-2-hydroxyphenyl)(piperidin-4-yl)methyl)pyrrolidin-2-one trifluoroacetic acid)

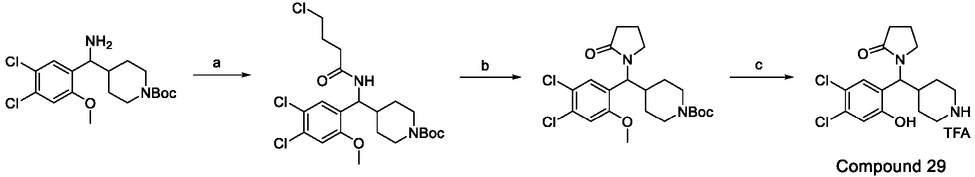

### Step a:

To a stirred solution of tert-butyl 4-[amino(4,5-dichloro-2-methoxyphenyl)methyl]piperidine-1-carboxylate (0.25 g, 0.64 mmol) and Et₃N (97 mg, 0.96 mmol) in CH₂Cl₂ (5 mL) was added 4-chlorobutanoyl chloride (0.10 g, 0.71 mmol) dropwise at room temperature. The reaction solution was stirred for 16 h at room temperature. The resulting solution was quenched with water (30 mL) at room temperature and extracted with EA (3 x 25 mL). The combined organic layers were washed with brine (3 x 20 mL), dried over anhydrous Na₂SO₄. After filtration, the filtrate was concentrated under reduced pressure. The crude product (0.35 g) was used in the next step directly without further purification: LCMS (ESI) calc'd for C₂₂H₃₁Cl₃N₂O₄ [M + H]⁺: 493, 495 (3 : 2), found 493, 495 (3 : 2).

### Step b:

To a stirred solution of tert-butyl 4-((4-chlorobutanamido)(4,5-dichloro-2-methoxyphenyl)methyl)piperidine-1-carboxylate (0.35 g, crude) in DMF (5 mL) was added NaH (0.14 g, 3.54 mmol, 60% in mineral oil) in portions at 0 °C under argon atmosphere. The reaction mixture was stirred for 16 at 50 °C under argon atmosphere. The resulting mixture was quenched with water (40 mL) at 0 °C and extracted with EA (3 x 30 mL). The combined organic layers were washed with brine (2 x 20 mL), dried over anhydrous Na₂SO₄. After filtration, the filtrate was concentrated under reduced pressure. The residue was purified by Prep-TLC, eluted with PE/EA (3/1) to afford tert-butyl 4-((4,5-dichloro-2-methoxyphenyl)(2-oxopyrrolidin-1-yl)methyl)piperidine-1-carboxylateas as a light yellow solid (0.25 g, 83%): LCMS (ESI) calc'd for C₂₂H₃₀Cₗ₂N₂O₄ [M + H]⁺: 457, 459 (3 : 2), found 457, 459 (3 : 2); ¹H NMR (300 MHz, CD₃OD) δ 7.57 (s, 1H), 7.21 (s, 1H), 5.16 (d, *J* = 11.5 Hz, 1H), 4.16-4.05 (m, 1H), 4.05-3.96 (m, 1H), 3.84 (S, 3H), 3.55-3.44 (m, 1H), 3.16-3.08 (m, 1H), 2.97-2.71 (m, 2H), 2.58-2.48 (m, 1H), 2.42-2.28 (m, 2H), 2.07-1.84 (m, 3H), 1.70-160 (m, 1H), 1.50 (s, 9H), 1.621.29 (m, 2H).

### Step c:

To a stirred solution of tert-butyl 4-[(4,5-dichloro-2-methoxyphenyl)(2-oxopyrrolidin-1-yl)methyl]piperidine-1-carboxylate (0.25 g, 0.55 mmol) in DCM (5 mL) was added BBr₃ (1.10 g, 4.37 mmol) dropwise at 0 °C. The reaction mixture was allowed to warm to room temperature and stirred for 3 h. The reaction mixture was quenched with saturated aq. NaHCO₃ (30 mL) at 0 °C. The resulting mixture was concentrated under reduced pressure. The residue was purified by Prep-HPLC with the following conditions: Column: XBridge C18 OBD Prep Column 100 Å, 10 µm, 19 mm x 250 mm; Mobile Phase A: water (plus 0.05% TFA), Mobile Phase B: ACN; Flow rate: 20 mL/min; Gradient: 25% B to 53% B in 6.5 min; Detector: UV 254/210 nm; Retention time: 4.57 min. The fractions containing desired product were collected and concentrated under reduced pressure to afford Compound 29 (1-((4,5-dichloro-2-hydroxyphenyl)(piperidin-4-yl)methyl)pyrrolidin-2-one trifluoroacetic acid) as a purple solid (75.2 mg, 40%): LCMS (ESI) calc'd for C₁₆H₂₀Cl₂N₂O₂ [M + H]⁺: 343, 345 (3 : 2), found 343, 345 (3 : 2); ¹H NMR (400 MHz, CD₃OD) δ 7.51 (s, 1H), 7.01 (s, 1H), 5.17 (d, *J =* 11.5 Hz, 1H), 3.59-3.43 (m, 2H), 3.40-3.30 (m, 1H), 3.25-3.13 (m, 1H), 3.13-2.93 (m, 2H), 2.78-2.60 (m, 1H), 2.47-2.33 (m, 2H), 2.10-1.90 (m, 3H), 1.70 (d, *J =* 14.5 Hz, 1H), 1.62-1.47 (m, 1H), 1.44-1.29 (m, 1H); ¹⁹F NMR (376 MHz, CD₃OD) δ -77.08.

### Example 101. (which is a reference example) Compound 30 (1-(hydroxy(piperidin-4-yl)methyl)naphthalen-2-ol)

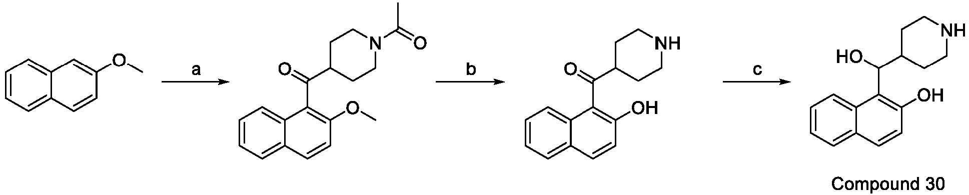

### Step a:

To a stirred solution of 2-methoxynaphthalene (0.38 g, 2.45 mmol) and 1-acetylpiperidine-4-carbonyl chloride (0.55 g, 2.94 mmol) in DCE (5 mL) was added AlCl₃ (0.96 g, 7.21 mmol) at room temperature. The resulting mixture was stirred for 6 h at 50 °C under nitrogen atmosphere. The reaction was quenched with water (20 mL) at 0 °C. The resulting mixture was extracted with EA (3 x 50 mL). The combined organic layers were washed with brine (3 x 30 mL) and dried over anhydrous Na₂SO₄. After filtration, the filtrate was concentrated under reduced pressure. The residue was purified by Prep-TLC (PE/EA 1/1) to afford 1-[4-[(2-methoxynaphthalen-1-yl)carbonyl]piperidin-1-yl]ethan-1-one as a light yellow oil (0.40 g, 53%): LCMS (ESI) calc'd for C₁₀H₂₁NO₃ [M + H]⁺ 312 found 312; ¹H NMR (400 MHz, CD₃OD) δ 8.58 (d, *J =* 1.8 Hz, 1H), 8.05-7.93 (m, 2H), 7.93-7.82 (m, 1H), 7.36-7.29 (m, 1H), 7.25 (dd, *J =* 9.0, 2.5 Hz, 1H), 4.61-4.52 (m, 1H), 4.07-3.99 (m, 1H), 3.97 (s, 3H), 3.93-3.82 (m, 1H), 3.43-3.35 (m, 1H), 2.95 (td, *J =* 12.7, 2.9 Hz, 1H), 2.15 (s, 3H), 2.03-1.93 (m, 2H), 1.82-1.55 (m, 2H).

### Step b:

A solution of 1-(4-(2-methoxy-1-naphthoyl)piperidin-1-yl)ethanone (0.15 g, 0.07 mmol) in aq. HCl (6 *N,* 5 mL) was stirred at 100 °C for 4 h. After cooling to room temperature the reaction solution was concentrated under reduced pressure. The resulting mixture was adjusted pH value to 8 with saturated aq. NaHCO₃, and then extracted with EA (3 x 30 mL). The combined organic layers were washed with brine (3 x 10 mL), dried over anhydrous Na₂SO₄. After the filtration, the filtrate was concentrated under reduced pressure. The residue was purified by Prep-HPLC with the following conditions: Column: XBridge C18 OBD, 100 Å, 10 µm, 19 mm x 250 mm; Mobile Phase A: water with 20 mmol/L NH₄HCO₃; Mobile Phase B: ACN; Flow rate: 25 mL/min; Gradient: 11% B to 50% B in 6.5 min; Detector: UV 254/210 nm; Retention time: 5.52 min. The fractions containing desired product were collected and concentrated under reduced pressure to afford (2-hydroxynaphthalen-1-yl)(piperidin-4-yl)methanone as an off-white solid (35 mg, 30%): LCMS (ESI) calc'd for C₁₆H₁₇NO₂ [M + H]⁺: 256, found 256; ¹H NMR (400 MHz, CD₃OD) δ 7.83-7.73 (m, 2H), 7.64 (d, *J =* 8.6 Hz, 1H), 7.41 (t*, J =* 7.7 Hz, 1H), 7.28 (t*, J* = 7.5 Hz, 1H), 7.12 (d, *J =* 8.9 Hz, 1H), 3.22-3.10 (m, 3H), 2.75 (t, *J =* 12.3 Hz, 2H), 1.97 (d, *J =* 13.7 Hz, 2H), 1.76 (t, *J =* 12.9 Hz, 2H).

### Step c:

To a stirred solution of (2-hydroxynaphthalen-1-yl)(piperidin-4-yl)methanone (30 mg, 0.12 mmol) in MeOH was added NaBH₄ (9 mg, 0.24 mmol) at room temperature under nitrogen atmosphere. The reaction mixture was stirred for 2 h at room temperature under nitrogen atmosphere. The resulting mixture was quenched with water (10 mL) at room temperature and concentrated under reduced pressure. The residue was purified by Prep-HPLC with the following conditions: Column: XBridge C18 OBD, 100 Å, 10 µm, 19 mm x 250 mm; Mobile Phase A: water with 20 mmol/L NH₄HCO₃; Mobile Phase B: ACN; Flow rate: 25 mL/min; Gradient: 10% B to 40% B in 9 min; Detector: UV 254/210 nm; Retention time: 6.10 min. The fractions containing desired product were collected and concentrated under reduced pressure to afford Compound 30 (1-(hydroxy(piperidin-4-yl)methyl)naphthalen-2-ol) as an off-white solid (7.5 mg, 25%): LCMS (ESI) calc'd for C₁₆H₁₉NO₂ [M + H]⁺: 258, found 258; ¹H NMR (400 MHz, CD₃OD) δ 7.94 (s, 1H), 7.74 (d, *J =* 8.2 Hz, 1H), 7.66 (d, *J =* 8.8 Hz, 1H), 7.42 (t, *J* = 7.5 Hz, 1H), 7.26 (t, *J =* 7.5 Hz, 1H), 7.05 (d, *J =* 8.7 Hz, 1H), 5.46 (d, *J =* 6.5 Hz, 1H), 3.18 (d, *J =* 12.9 Hz, 1H), 3.07 (d, *J =* 12.5 Hz, 1H), 2.65-2.52 (m, 2H), 2.21-1.98 (m, 2H), 1.69-1.51 (m, 2H), 1.49-1.37 (m, 1H).

### Example 102. (which is a reference example) Compound 31 (4,5-dichloro-2-[(methylamino)(piperidin-4-yl)methyl]phenol trifluoroacetic acid)

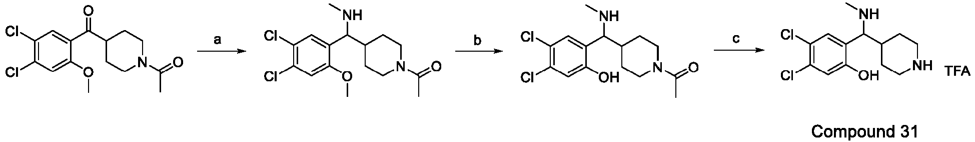

### Step a:

To a stirred mixture of 1-[4-[(4,5-dichloro-2-methoxyphenyl)carbonyl]piperidin-1-yl]ethan-1-one (Example 4, step a) (0.25 g, 0.76 mmol) in MeOH (5 mL) was added methanamine hydrochloride (0.26 g, 3.79 mmol) and NaBH₃CN (0.14 g, 2.27 mmol) at room temperature under nitrogen atmosphere. After stirring for 8 h at 60 °C under nitrogen atmosphere, the reaction mixture was allowed to cool down to room temperature. The resulting mixture was quenched with water (5 mL) at room temperature and concentrated under reduced pressure. The residue was purified by reverse phase chromatography, eluted with 60% ACN in water with 20 mmol/L NH₄HCO₃ to afford 1-[4-[(4,5-dichloro-2-methoxyphenyl)(methylamino)methyl]piperidin-1-yl]ethan-1-one as a light yellow oil (0.20 g, 76%): LCMS (ESI) calc'd for C₁₆H₂₂Cl₂N₂O₂ [M + H]⁺: 345, 347 (3 : 2), found 345, 347 (3 : 2).

### Step b:

To a stirred solution of 1-[4-[(4,5-dichloro-2-methoxyphenyl)(methylamino)methyl]piperidin-1-yl]ethan-1-one (0.20 g, 0.58 mmol) in DCM (2 mL) was added BBr₃ (0.72 g, 2.90 mmol) at 0 °C under nitrogen atmosphere. The reaction mixture was allowed to warm to room temperature and stirred for 1 h under nitrogen atmosphere. The resulting mixture was quenched with water (2 mL) at room temperature and concentrated under reduced pressure to afford crude 1-(4-((4,5-dichloro-2-hydroxyphenyl)(methylamino)methyl)piperidin-1-yl)ethanone. The crude product was used in the next step directly without further purification: LCMS (ESI) calc'd for C₁₅H₂₀Cₗ₂N₂O₂ [M + H]⁺: 331, 333 (3 : 2), found 331, 333 (3 : 2).

### Step c:

A solution of 1-[4-[(4,5-dichloro-2-hydroxyphenyl)(methylamino)methyl]piperidin-1-yl]ethan-1-one (0.18 g, 0.54 mmol) in aq. HCl (6 N, 10 mL) was stirred for 5 h at 120 °C. The resulting mixture was concentrated under reduced pressure. The residue was purified by Prep-HPLC with the following conditions: Column: SunFire Prep C18 OBD Column 19 x 150 mm, 5 µm, 10 nm; Mobile Phase A: water (plus 0.05% TFA), Mobile Phase B: ACN; Flow rate: 20 mL/min; Gradient: 11% B to 25% B in 9 min; Detector: UV 254/210 nm; Retention time: 7.87 min. The fractions containing desired product were collected and concentrated under reduced pressure to afford Compound 31 (4,5-dichloro-2-[(methylamino)(piperidin-4-yl)methyl]phenol trifluoroacetic acid) as an off-white solid (25 mg, 15%): LCMS (ESI) calc'd for C₁₃H₁₈Cl₂N₂O [M + H]⁺: 289, 291 (3 : 2), found 289, 291 (3 : 2); ¹H NMR (300 MHz, CD₃OD) δ 7.48 (s, 1H), 7.13 (s, 1H), 4.27 (d, *J =* 8.6 Hz, 1H), 3.52 (d, *J =* 13.1 Hz, 1H), 3.39 (d, *J =* 13.1 Hz, 1H), 3.13-2.86 (m, 2H), 2. 59 (s, 3H), 2.59-2.44 (m, 1H), 2.24 (d, *J =* 13.7 Hz, 1H), 1.76-1.33 (m, 3H); ¹⁹F NMR (282, CD₃OD) δ -76.96.

### Example 103. (which is a reference example) Compound 32 (2-(amino(pyrrolidin-3-yl)methyl)-4,5-dichlorophenol trifluoroacetic acid diastereoisomer 1) and Compound 33 (2-(amino(pyrrolidin-3-yl)methyl)-4,5-dichlorophenol trifluoroacetic acid diastereoisomer 2)

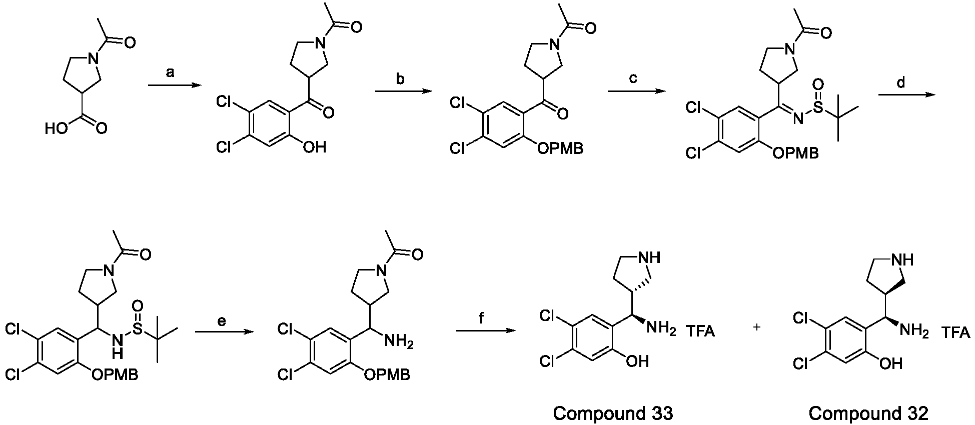

### Step a:

To a stirred mixture of 1-acetylpyrrolidine-3-carboxylic acid (Intermediate 62, Example 62) (0.49 g, 3.09 mmol) in DCM (5 mL) was added sulfuroyl dichloride (1.10 g, 9.28 mmol) dropwise at room temperature under nitrogen atmosphere. The reaction was concentrated under reduced pressure. The residue was dissolved in DCE (6 mL), and then a solution was added dropwise to a mixture of 1,2-dichloro-4-methoxybenzene (0.34 g, 1.90 mmol) and AlCl₃ (1.23 g, 9.26 mmol) in DCE (10 mL) at -5 °C under nitrogen atmosphere. The reaction mixture was stirred at 0 °C for 16 h. The reaction was quenched with water (25 mL) at -5 °C. The resulting mixture was extracted with EA (3 x 30 mL). The combined organic layers were washed with aq. HCl (1 *N,* 3 x 20 mL), dried over anhydrous Na₂SO₄. After filtration, the filtrate was concentrated under reduced pressure. The residue was purified by silica gel column chromatography, eluted with DCM/MeOH (10/1) to afford 1-[3-[(4,5-dichloro-2-hydroxyphenyl)carbonyl]pyrrolidin-1-yl]ethan-1-one as a light yellow oil (0.49 g, 45%): LCMS (ESI) calc'd for C₁₃H₁₃Cl₂NO₃ [M + H]⁺: 302, 304 (3 : 2), found 302, 304 (3 : 2); ¹H NMR (400 MHz, CD₃OD) δ 8.12 (d, *J =* 7.4 Hz, 1H), 7.22 (d, *J =* 2.6 Hz, 1H), 4.42-4.17 (m, 1H), 3.93-3.70 (m, 2H), 3.70-3.48 (m, 2H), 2.49-2.21 (m, 2H), 2.10 (d, *J =* 8.9 Hz, 3H).

### Step b:

A mixture of 1-[3-[(4,5-dichloro-2-hydroxyphenyl)carbonyl]pyrrolidin-1-yl]ethan-1-one (0.48 g, 1.59 mmol), K₂CO₃ (0.44 g, 3.18 mmol) and 1-(chloromethyl)-4-methoxybenzene (0.27 g, 1.75 mmol) in DMF (5 mL) was stirred at 80 °C under nitrogen atmosphere for 1 h. After cooling to room temperature, the resulting mixture was diluted with water (20 mL) at room temperature. The resulting mixture was extracted with EA (3 x 20 mL). The combined organic layers were washed with brine (5 x 20 mL), dried over anhydrous Na₂SO₄. After filtration, the filtrate was concentrated under reduced pressure. The residue was purified by silica gel column chromatography, eluted with DCM/MeOH (12/1) to afford 1-[3-([4,5-dichloro-2-[(4-methoxyphenyl)methoxy]phenyl]carbonyl)pyrrolidin-1-yl]ethan-1-one as a light yellow oil (0.49 g, 73%): LCMS (ESI) calc'd for C₂₁H₂₁Cl₂NO₄ [M + H]⁺: 422, 424 (3 : 2), found 422, 424 (3 : 2); ¹H NMR (300 MHz, CD₃OD) δ 7.78 (s, 1H), 7.51 (d, *J =* 7.0 Hz, 1H), 7.48-7.39 (m, 2H), 6.97 (dd, *J =* 8.6, 2.0 Hz, 2H), 5.15 (s, 2H), 3.95 (dt, *J =* 23.3, 6.8 Hz, 1H), 3.82 (s, 3H), 3.63-3.51 (m, 1H), 3.51-3.36 (m, 3H), 3.26-3.17 (m, 1H), 2.14-1.93 (m, 4H).

### Step c:

To a stirred solution of 1-[3-([4,5-dichloro-2-[(4-methoxyphenyl)methoxy]phenyl]carbonyl)pyrrolidin-1-yl]ethan-1-one (0.42 g, 0.99 mmol) and 2-methylpropane-2-sulfinamide (0.60 g, 4.97 mmol) in THF (5 mL) was added Ti(OEt)₄ (1.81 g, 7.96 mmol) at room temperature under nitrogen atmosphere. The resulting mixture was stirred for 16 h at 70 °C under nitrogen atmosphere. The reaction was quenched with saturated aq. NaHCO₃ (20 mL) at room temperature and extracted with EA (3 x 30 mL). The combined organic layers were washed with brine (3 x 20 mL), dried over anhydrous Na₂SO₄. After filtration, the filtrate was concentrated under reduced pressure to afford N-[(1-acetylpyrrolidin-3-yl)([4,5-dichloro-2-[(4-methoxyphenyl)methoxy]phenyl])methylidene]-2-methylpropane-2-sulfinamide as a light yellow oil (0.81 g, crude), which was used in next step directly without further purification: LCMS (ESI) calc'd for C₂₅H₃₀Cl₂N₂O₄S [M + H]⁺: 525, 527 (3 : 2), found 525, 527 (3 : 2).

### Step d:

To a stirred mixture of N-(1-acetylpyrrolidin-3-yl)([4,5-dichloro-2-[(4-methoxyphenyl)methoxy]phenyl])methylidene]-2-methylpropane-2-sulfinamide (0.81 g, 1.54 mmol) in MeOH (4 mL) was added NaBH₄ (0.12 g, 3.08 mmol) at room temperature. The reaction was stirred at room temperature for 1 h. The resulting solution was quenched with water (15 mL). The resulting mixture was extracted with EA (3 x 20 mL). The combined organic layers were washed with brine (3 x 20 mL), dried over anhydrous Na₂SO₄. After filtration, the filtrate was concentrated under reduced pressure to afford N-[(1-acetylpyrrolidin-3-yl)([4,5-dichloro-2-[(4-methoxyphenyl)methoxy]phenyl])methyl]-2-methylpropane-2-sulfinamide as a light yellow oil (0.61 g, crude), which was used in next step directly without further purification: LCMS (ESI) calc'd for C₂₅H₃₂Cl₂N₂O₄S [M + H]⁺: 527, 529 (3 : 2), found 527, 529 (3 : 2).

### Step e:

To a stirred mixture of N-[(1-acetylpyrrolidin-3-yl)([4,5-dichloro-2-[(4-methoxyphenyl)methoxy]phenyl])methyl]-2-methylpropane-2-sulfinamide (0.61 g, 1.16 mmol) in 1,4-dioxane (2 mL) was added aq. HCl (4 N, 2 mL) at room temperature. The reaction was stirred at room temperature for 1 h. The resulting solution was concentrated under reduced pressure. The residue was purified by Prep-TLC, eluted with DCM/MeOH (20/1) to afford 1-[3-[amino([4,5-dichloro-2-[(4-methoxyphenyl)methoxy]phenyl])methyl]pyrrolidin-1-yl]ethan-1-one as a light yellow oil (0.35 g, 58% overall three steps): LCMS (ESI) calc'd for C₂₁H₂₄Cl₂N₂O₃ [M + H]⁺: 423, 425 (3 : 2), found 423, 425 (3 : 2).

### Step f:

A solution of 1-[3-[amino([4,5-dichloro-2-[(4-methoxyphenyl)methoxy]phenyl])methyl]pyrrolidin-1-yl]ethan-1-one (90 mg, 0.21 mmol) and HCl (6 N, 2 mL) was stirred at 80 °C for 3 h. The resulting solution was concentrated under reduced pressure. The residue was purified by Prep-HPLC with the following conditions: Column: XSelect CSH Prep C18 OBD Column, 19 x 250 mm, 5 µm; Mobile Phase A: Water (plus 0.05% TFA), Mobile Phase B: ACN; Flow rate: 25 mL/min; Gradient: 20% B to 35% B in 5.5 min; Detector: UV 220/254 nm; Retention time: RT₁: 4.70 min, RT₂: 5.00 min. The fractions containing the desired product at 4.70 min were collected and concentrated under reduced pressure to afford Compound 33 (2-(amino(pyrrolidin-3-yl)methyl)-4,5-dichlorophenol trifluoroacetic acid diastereoisomer 2) as a light yellow solid (9 mg, 11%): LCMS (ESI) calc'd for C₁₁H₁₄Cl₂N₂O [M + H]⁺: 261, 263 (3 : 2), found 261, 263 (3 : 2); ¹H NMR (300 MHz, CD₃OD) δ 7.53 (s, 1H), 7.12 (s, 1H), 4.44 (d, *J =* 10.2 Hz, 1H), 3.64-3.47 (m, 1H), 3.44-3.34 (m, 1H), 3.29-3.06 (m, 2H), 2.98-2.84 (m, 1H), 2.48-2.34 (m, 1H), 2.06-1.88 (m, 1H); ¹⁹F NMR (282 MHz, CD₃OD) δ -77.00. And the fractions containing the desired product at 5.00 min were collected and concentrated under reduced pressure to afford Compound 32 (2-(amino(pyrrolidin-3-yl)methyl)-4,5-dichlorophenol trifluoroacetic acid diastereoisomer 1) as a light yellow solid (12 mg, 15%): LCMS (ESI) calc'd for C₁₁H₁₄Cl₂N₂O [M + H]⁺: 261, 263 (3 : 2), found 261, 263 (3 : 2); ¹H NMR (300 MHz, CD₃OD) δ 7.52 (s, 1H), 7.12 (s, 1H), 4.49 (d, *J =* 9.3 Hz, 1H), 3.76-3.57 (m, 1H), 3.48-3.37 (m, 1H), 3.31-3.16 (m, 3H), 1.97-1.81 (m, 1H), 1.80-1.62 (m, 1H); ¹⁹F NMR (282 MHz, CD₃OD) δ -77.01.

### Example 104. Compound 35 (2-(amino(1-tosylpiperidin-4-yl)methyl)-4,5-dichlorophenol)

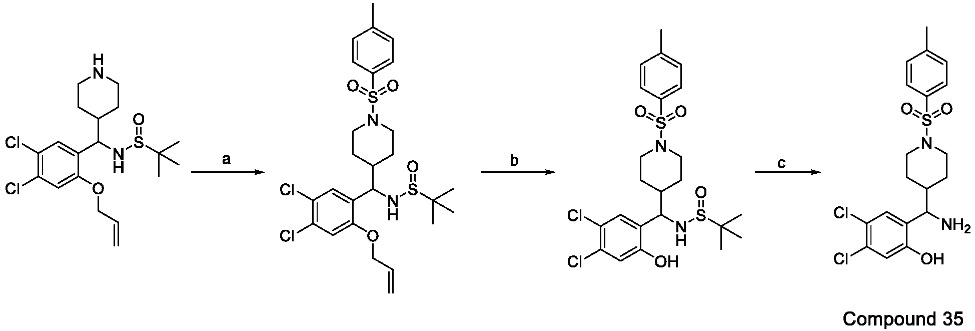

### Step a:

To a stirred solution of *N*-[[4,5-dichloro-2-(prop-2-en-1-yloxy)phenyl](piperidin-4-yl)methyl]-2-methylpropane-2-sulfinamide (0.12 g, 0.29 mmol) and Et₃N (58 mg, 0.58 mmol) in DCM (2 mL) was added 4-methylbenzene-1-sulfonyl chloride (60 mg, 0.31 mmol) in portions at room temperature under nitrogen atmosphere. After stirring for additional 1 h at room temperature under nitrogen atmosphere, the reaction solution was quenched with water (30 mL) at room temperature and extracted with EA (2 x 30 mL). The combined organic layers were washed with brine (2 x 10 mL), dried over anhydrous Na₂SO₄. After filtration, the filtrate was concentrated under reduced pressure. The residue was purified by Prep-TLC, eluted with PE/EA (2/1) to afford *N*-[[4,5-dichloro-2-(prop-2-en-1-yloxy)phenyl]([1-[(4-methylbenzene)sulfonyl]piperidin-4-yl])methyl]-2-methylpropane-2-sulfinamide as a yellow oil (50 mg, 26%): LCMS (ESI) calc'd for C₂₆H₃₄Cl₂N₂O₄S₂ [M + H]⁺: 573, 575 (3 : 2), found 573, 575 (3 : 2); ¹H NMR (300 MHz, CD₃OD) δ 7.66 (d, *J* = 8.0 Hz, 2H), 7.44 (d, *J =* 8.0 Hz, 2H), 7,41 (s, 1H), 7.14 (s, 1H), 6.19-6.02 (m, 1H), 5.48-5.29 (m, 2H), 4.60 (s, 2H), 4.50 (d, *J =* 8.8 Hz, 1H), 4.14 (d, *J =* 7.7 Hz, 1H), 3.87-3.77 (m, 1H), 3.74-3.65 (m, 1H), 2.45 (s, 3H), 2.37-2.12 (m, 2H), 1.77-1.67 (m, 1H), 1.43-1.28 (m, 3H), 1.112 (s, 9H).

### Step b:

To a stirred mixture of *N*-[[4,5-dichloro-2-(prop-2-en-1-yloxy)phenyl]([1-[(4-methylbenzene)sulfonyl]piperidin-4-yl])methyl]-2-methylpropane-2-sulfinamide (50 mg, 0.09 mmol) and Pd(PPh₃)₄ (11 mg, 0.01 mmol) in THF (2 mL) was added NaBH₄ (5 mg, 0.14 mmol) at room temperature under nitrogen atmosphere. The reaction mixture was stirred for 1 h at room temperature under nitrogen atmosphere. The resulting mixture was quenched with water (10 mL) at room temperature and extracted with EA (3 x 20 mL). The combined organic layers were washed with water (2 x 20 mL), dried over anhydrous Na₂SO₄ and filtered. The filtrate was concentrated under reduced pressure to afford crude *N*-((4,5-dichloro-2-hydroxyphenyl)(1-tosylpiperidin-4-yl)methyl)-2-methylpropane-2-sulfinamide. The crude product was used in the next step directly without further purification: LCMS (ESI) calc'd for C₂₃H₃₀Cl₂N₂O₄S₂ [M + H]⁺: 533, 535 (3 : 2), found 533, 535 (3 : 2).

### Step c:

To a solution of *N*-((4,5-dichloro-2-hydroxyphenyl)(1-tosylpiperidin-4-yl)methyl)-2-methylpropane-2-sulfinamide (crude) in 1,4-dioxane (3 mL) was added aq. HCl (6 *N,* 3 mL) at room temperature. The reaction solution was stirred for 2 h at room temperature. The resulting solution was concentrated under reduced pressure. The residue was purified by Prep-HPLC with the following conditions: Column: XBridge C18 OBD Prep Column, 100 Å, 10 µm, 19 mm x 250 mm; Mobile Phase A: water with 20 mmol/L NH₄HCO₃, Mobile Phase B: ACN; Flow rate: 25 mL/min; Gradient: 50% B to 88% B in 6.5 min; Detector: UV 254/210 nm; Retention time: 6.20 min. The fractions containing desired product were collected concentrated under reduced pressure to afford Compound 35 (2-(amino(1-tosylpiperidin-4-yl)methyl)-4,5-dichlorophenol) as a yellow solid (12 mg, 35% overall two steps): LCMS (ESI) calc'd for C₁₉H₂₂Cl₂N₂O₃S [M + H]⁺: 429, 431 (3 : 2), found 429, 431 (3 : 2); ¹H NMR (300 MHz, CD₃OD) δ 7.64 (d, *J* = 8.0 Hz, 2H), 7.41 (d, *J* = 8.0 Hz, 2H), 7.18 (s, 1H), 6.85 (s, 1H), 3.95-3.69 (m, 3H), 2.45 (s, 3H), 2.33-2.11 (m, 2H), 2.03-1.92 (d, *J =* 13.0 Hz, 1H), 1.81-1.54 (m, 1H), 1.56-1.16 (m, 3H).

### Example 105. (which is a reference example) Compound 36 (N-[(2-hydroxynaphthalen-1-yl)(piperidin-4-yl)methyl]acetamide trifluoroacetic acid)

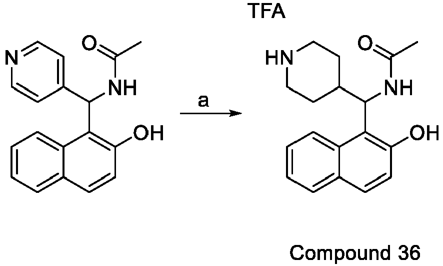

### Step a:

To a solution of N-[(2-hydroxynaphthalen-1-yl)(pyridin-4-yl)methyl]acetamide (Intermediate 7, Example 7) (0.10 g, 0.34 mmol) in MeOH (3 mL) was added PtO₂ (7 mg, 0.03 mmol) at room temperature. The reaction mixture was degassed with hydrogen for three times and stirred under hydrogen atmosphere (50 atm) at room temperature for 18 h. A mixture was filtered through a Celite, and then the filtrate was concentrated under reduced pressure. The residue was purified by Prep-HPLC with the following conditions: Column: XBridge C18 OBD, 100 Å, 10 µm, 19 mm x 250 mm; Mobile Phase A: water (plus 0.05% TFA), Mobile Phase B: ACN; Flow rate: 25 mL/min; Gradient: 15% B to 35% B in 8 min; Detector: UV 254/210 nm; Retention time: 6.67 min. The collected fractions containing desired product was concentrated under reduced pressure to afford Compound 36 (N-[(2-hydroxynaphthalen-1-yl)(piperidin-4-yl)methyl]acetamide trifluoroacetic acid) as a light yellow solid (90 mg, 90%): LCMS (ESI) calc'd for C₁₈H₂₂N₂O₂ [M + H]⁺: 299, found 299; ¹H NMR (400 MHz, CD₃OD) δ 8.20 (d, *J =* 8.6 Hz, 1H), 7.83 (d, *J =* 8.6 Hz, 1H), 7.76 (d, *J =* 8.8 Hz, 1H), 7.54 (d, *J =* 8.6 Hz, 1H), 7.36 *(d, J* = 7.8 Hz, 1H), 7.19 (d, *J =* 8.8 Hz, 1H), 5.85 (d, *J =* 10.2 Hz, 1H), 3.55 (d, *J =* 12.4 Hz, 1H), 3.27 (d, *J =* 12.4 Hz, 1H), 3.07 (t, *J =* 12.8 Hz, 1H), 2.90-2.75 (m, 1H), 2.73-2.60 (m, 1H), 2.35-2.22 (d, *J =* 14.4 Hz, 1H), 2.09 (s, 3H), 1.70-1.59 (m, 1H), 1.49-1.32 (m, 2H); ¹⁹F-NMR (376 MHz, CD₃OD) δ -77.11.

### Example 106. (which is a reference example) Compound 34 (1-(amino(piperidin-4-yl)methyl)naphthalen-2-ol trifluoroacetic acid)

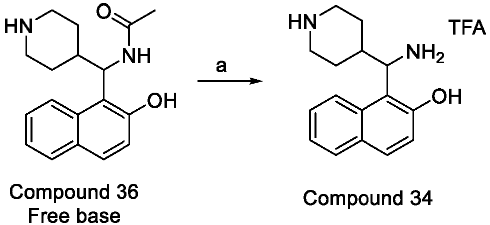

### Step a:

A solution of N-((2-hydroxynaphthalen-1-yl)(piperidin-4-yl)methyl)acetamide (Compound 36's free base, Example 105) (0.20 g, 0.67 mmol) in aq. HCl (6 *N,* 5 mL) was stirred for 8 h at 100 °C. After cooling to room temperature, the resulting solution was concentrated under reduced pressure. The residue was purified by Prep-HPLC with the following conditions: Column: XBridge C18 OBD, 100 Å, 10 µm, 19 mm x 250 mm; Mobile Phase A: water (plus 0.05% TFA), Mobile Phase B: ACN; Flow rate: 25 mL/min; Gradient: 5% B to 25% B in 6.5 min; Detector: UV 254/210 nm; Retention time: 5.35 min. The fractions containing desired product were collected and concentrated to afford Compound 34 (1-(amino(piperidin-4-yl)methyl)naphthalen-2-ol trifluoroacetic acid) as a gray solid (2 mg, 1%): LCMS (ESI) calc'd for C₁₆H₂₀N₂O [M + H]⁺: 257, found 257; ¹H NMR (400 MHz, CD₃OD) δ 8.07 (d, *J =* 8.7 Hz, 1H), 7.93 (t, *J =* 7.8 Hz, 2H), 7.63 (t, *J =* 7.7 Hz, 1H), 7.44 (t, *J =* 7.5 Hz, 1H), 7.28 (d, *J =* 9.0 Hz, 1H), 5.05 (d, *J =* 9.7 Hz, 1H), 3.65 (d, *J =* 12.6 Hz, 1H), 3.31 (d, *J =* 12.6 Hz, 1H), 3.14 (t, *J =* 12.5 Hz, 1H), 2.94-2.75 (m, 2H), 2.41 (d, *J =* 14.2 Hz, 1H), 1.87-1.68 (m, 1H), 1.56-1.39 (m, 2H); ¹⁹F-NMR (376 MHz, CD₃OD) δ -76.97.

### Example 107. (which is a reference example) Compound 37 (2-[amino(piperidin-4-yl)methyl]-3,4,5-trichlorophenol trifluoroacetic acid)

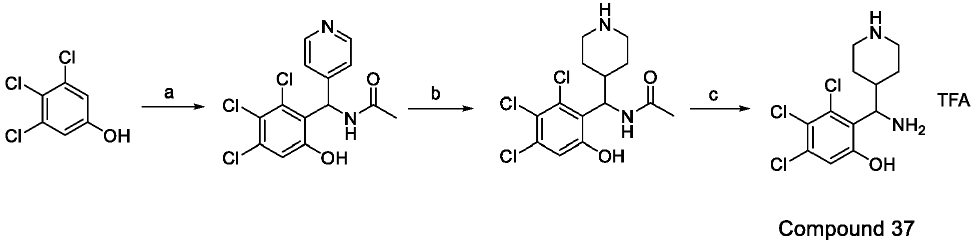

### Step a:

To a stirred mixture of 3,4,5-trichlorophenol (Example 9, step a) (0.59 g, 2.99 mmol), acetamide (0.21 g, 3.59 mmol) and pyridine-4-carbaldehyde (0.32 g, 2.99 mmol) was added AlCl₃ (0.06 g, 0.45 mmol) at room temperature under nitrogen atmosphere. The reaction was stirred at 110 °C for 16 h. The reaction was quenched with water (25 mL) at room temperature and extracted with EA (3 x 30 mL). The combined organic layers were washed with brine (3 x 20 mL), dried over anhydrous Na₂SO₄. After filtration, the filtrate was concentrated under reduced pressure. The residue was purified by reverse phase chromatography, eluted with 50% ACN in water (plus 0.05% TFA) to afford N-[pyridin-4-yl(2,3,4-trichloro-6-hydroxyphenyl)methyl]acetamide as a light yellow oil (0.14 g, 12%): LCMS (ESI) calc'd for C₁₄H₁₁Cl₃N₂O₂ [M + H]⁺: 345, 347, 349 (3 : 3 : 1), found 345, 347, 349 (3 : 3 : 1); ¹H NMR (300 MHz, CD₃OD) δ 8.79-8.70 (m, 2H), 7.94-7.85 (m, 2H), 7.13 (s, 1H), 7.05 (s, 1H), 2.17 (s, 3H).

### Step b:

To a solution of N-[pyridin-4-yl(2,3,4-trichloro-6-hydroxyphenyl)methyl]acetamide (0.10 g, 0.29 mmol) in 20 mL MeOH/AcOH (v/v = 1/1) was added PtO₂ (20 mg, 0.09 mmol) at room temperature. The mixture was stirred at 30 °C under hydrogen atmosphere (50 atm) for 6 h. The reaction was filtered, and the filtrate was concentrated under reduced pressure. The residue was dissolved in EA (20 mL) and water (20 mL). The aqueous layer was extracted with EA (3 x 20 mL). The combined organic layers were washed with brine (3 x 20 mL) and dried over anhydrous Na₂SO₄. After filtration, the filtrate was concentrated under reduced pressure to afford *N*-[piperidin-4-yl(2,3,4-trichloro-6-hydroxyphenyl)methyl]acetamide as a colorless oil (0.10 g, crude), which was used in the next step directly without further purification: LCMS (ESI) calc'd for C₁₄H₁₇Cl₃N₂O₂ [M + H]⁺: 351, 353, 355 (3 : 3 : 1), found 351, 353, 355 (3 : 3 : 1); ¹H NMR (300 MHz, CD₃OD) δ 7.04-6.87 (m, 1H), 5.51 (d, *J =* 10.5 Hz, 1H), 3.52-3.38 (m, 2H), 3.06-2.78 (m, 2H), 2.54-2.03 (m, 2H), 1.98 (s, 3H), 1.61-1.42 (m, 3H).

### Step c:

A solution of *N*-[piperidin-4-yl(2,3,4-trichloro-6-hydroxyphenyl)methyl]acetamide (0.10 g, 0.29 mmol) in conc. HCl (3 mL) was stirred at 100 °C for 3 h. The resulting solution was concentrated under reduced pressure. The residue was purified by Prep-HPLC with the following conditions: Column: XBridge C18 OBD Prep Column 100, 10 µm, 19 mm x 250 mm; Mobile Phase A: water (plus 0.05% TFA), Mobile Phase B: ACN; Flow rate: 20 mL/min; Gradient: 5% B to 40% B in 8 min; Detector: UV 254/210 nm; Retention time: 7.18 min. The fractions containing the desired product were collected and concentrated under reduced pressure to afford Compound 37 (2-[amino(piperidin-4-yl)methyl]-3,4,5-trichlorophenol trifluoroacetic acid) as a light yellow solid (70 mg, 57% overall two steps): LCMS (ESI) calc'd for C₁₂H₁₅Cl₃N₂O [M + H]⁺: 309, 311, 313 (3 : 3 : 1), found 309, 311, 313 (3 : 3 : 1); ¹H NMR (400 MHz, CD₃OD) δ 7.15 (s, 1H), 4.69 (d, *J =* 10.1 Hz, 1H), 3.55 (d, *J =* 12.9 Hz, 1H), 3.42-3.35 (m, 1H), 3.05 (td, *J =* 13.1, 3.1 Hz, 1H), 3.00-2.88 (m, 1H), 2.76-2.63 (m, 1H), 2.26 (d, *J =* 14.0 Hz, 1H), 1.77-1.62 (m, 1H), 1.62-1.50 (m, 2H); ¹⁹F NMR (376 MHz, CD₃OD) δ -77.06.

### Example 108. (which is a reference example) Compound 38 (5-(amino(4,5-dichloro-2-hydroxyphenyl)methyl)piperidin-2-one trifluoroacetic acid diastereoisomer 1) and Compound 39 (5-(amino(4,5-dichloro-2-hydroxyphenyl)methyl)piperidin-2-one diastereoisomer 2)

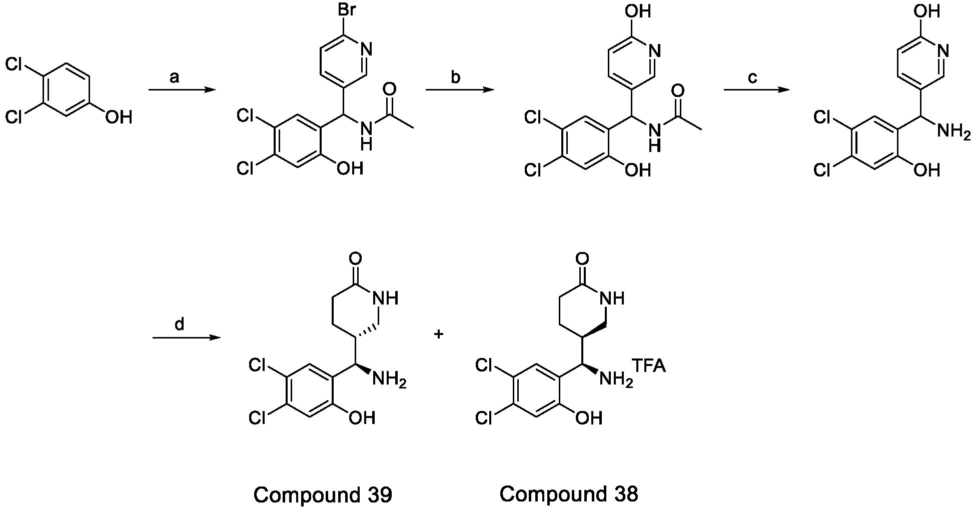

### Step a:

A mixture of 3,4-dichlorophenol (2.00 g, 12.27 mmol), 6-bromopyridine-3-carbaldehyde (2.30 g, 12.27 mmol), acetamide (0.87 g, 14.72 mmol) and AlCl₃ (0.245 g, 1.84 mmol) was stirred for 1.5 h at 110 °C under nitrogen atmosphere. After cooling to room temperature, the mixture was diluted with water (80 mL) and extracted with EA (5 x 50 mL). The combined organic layers were washed with brine (2 x 20 mL), dried over anhydrous Na₂SO₄. After filtration, the filtrate was concentrated under reduced pressure. The residue was purified by silica gel column chromatography, eluted with PE/EA (2/3) to afford N-[(6-bromopyridin-3-yl)(4,5-dichloro-2-hydroxyphenyl)methyl]acetamide as a light yellow solid (1.21 g, 20%): LCMS (ESI) calc'd for C₁₄H₁₁BrCl₂N₂O₂ [M + H]⁺: 389, 391, 393 (2 : 3 : 1), found 389, 391, 393 (2 : 3 : 1); ¹H NMR (400 MHz, DMSO-*d₆*) δ 10.59 (s, 1H), 8.47 (d, *J =* 8.4 Hz, 1H), 8.24 (dd, *J =* 7.3, 2.6 Hz, 1H), 7.69-7.57 (m, 1H), 7.56-7.42 (m, 2H), 6.90-6.80 (m, 2H), 1.99 (s, 3H).

### Step b:

A mixture of N-[(6-bromopyridin-3-yl)(4,5-dichloro-2-hydroxyphenyl)methyl]acetamide (0.51 g, 1.32 mmol), (*E*)*-N-*(phenylmethylidene)hydroxylamine (0.21 g, 1.71 mmol), Pd₂(dba)₃ (0.12 g, 0.13 mmol), RockPhos (0.12 g, 0.26 mmol) and Cs₂CO₃ (0.94 g, 2.9 mmol) in DMF (5 mL)) was stirred for 2 h at 80 °C under nitrogen atmosphere. The reaction mixture was allowed to cool to ambient temperature, quenched with water (30 mL) and extracted with DCM/MeOH (10/1, 3 x 50 mL). The combined organic layers were washed with brine (2 x 20 mL), dried over anhydrous Na₂SO₄ and filtered. The filtrate was concentrated under reduced pressure, the residue was purified by silica gel column chromatography, eluted with DCM/MeOH (10: 1) to afford N-[(4,5-dichloro-2-hydroxyphenyl)(6-hydroxypyridin-3-yl)methyl]acetamide as a light yellow oil (0.26 g, 60%): LCMS (ESI) calc'd for C₁₄H₁₂Cl₂N₂O₃ [M + H]⁺: 327, 329 (3 : 2), found 327, 329 (3 : 2).

### Step c:

A solution of *N*-[(4,5-dichloro-2-hydroxyphenyl)(6-hydroxypyridin-3-yl)methyl]acetamide (65 mg) in aq. HCl (6 *N,* 2 mL) was stirred for 3 h at 80 °C under nitrogen atmosphere. The reaction solution was concentrated under reduced pressure, the reside was purified by Prep-HPLC with the following conditions: Column: XBridge C18 OBD Prep Column 100 Å, 10 µm, 19 mm x 250 mm; Mobile Phase A: water with 20 mmoL/L NH₄HCO₃, Mobile Phase B: ACN; Flow rate: 25 mL/min; Gradient: 15% B to 68% B in 6.5 min; Detector: UV 254/220 nm; Retention time: 5.07 min. The combined fractions containing product were concentrated under reduced pressure to afford 5-[amino(4,5-dichloro-2-hydroxyphenyl)methyl]-1,2-dihydropyridin-2-one as an off-white solid (20 mg, 35%): LCMS (ESI) calc'd for C₁₂H₁₀Cl₂N₂O₂ [M + H]⁺: 285, 287 (3 : 2), found 285, 287 (3 : 2); ¹H NMR (400 MHz, CD₃OD) δ 7.64 (dd, *J* = 9.5, 2.7 Hz, 1H), 7.41 (d, *J* = 2.6 Hz, 1H), 7.33 (s, 1H), 6.92 (s, 1H), 6.54 (d, *J* = 9.6 Hz, 1H), 5.15 (s, 1H).

### Step d:

A mixture of 5-[amino(4,5-dichloro-2-hydroxyphenyl)methyl]-1,2-dihydropyridin-2-one (70 mg, 0.25 mmol) and PtO₂ (7 mg, 0.03 mmol) in aq. HCl (1 *N,* 0.1 mL) and MeOH (1 mL) was stirred for 6.5 h at room temperature under hydrogen atmosphere (50 atm). After filtration, a filtrate was concentrated under reduced pressure, The residue was purified by Prep-HPLC with the flowing conditions: Column: XBridge C18 OBD Prep Column, 100Å, 10 µm, 19 mm x 250 mm; Mobile Phase A: water (plus 0.05% TFA), Mobile Phase B: ACN; Flow rate: 25 mL/min; Gradient: 8% B to 40% B in 6.5 min; Detector: UV 254/210 nm; Retention time: RT₁: 5.33 min, RT₂: 5.72 min.

The faster-eluting enantiomer was obtained as Compound 38 (5-(amino(4,5-dichloro-2-hydroxyphenyl)methyl)piperidin-2-one trifluoroacetic acid diastereoisomer 1) at 5.33 min as an off-white solid (4.5 mg, 6%): LCMS (ESI) calc'd for C₁₂H₁₄Cl₂N₂O₂ [M + H]⁺: 289, 291 (3 : 2), found 289, 291 (3 : 2); ¹H NMR (400 MHz, CD₃OD) δ 7.46 (s, 1H), 7.10 (s, 1H), 4.28 (d, *J* = 10.2 Hz, 1H), 3.03-2.85 (m, 2H), 2.74-2.58 (m, 1H), 2.55-2.35 (m, 2H), 2.19 (d, *J =* 13.4 Hz, 1H), 1.85-1.65 (m, 1H); ¹⁹F NMR (376 MHz, CD₃OD) δ -76.98.

The slower-eluting enantiomer at 5.72 min containing impurity was re-purified by Prep-HPLC with the flowing conditions: Column: XBridge C18 OBD Prep Column, 100Å, 10 µm, 19 mm x 250 mm; Mobile Phase A: water with 10 mmol/L NH₄HCO₃, Mobile Phase B: ACN; Flow rate: 25 mL/min; Gradient: 8% B to 60% B in 6.5 min; Detector: UV 254/210 nm; Retention time: 5.92 min. The combined fractions containing product were concentrated under reduced pressure to afford Compound 39 (5-(amino(4,5-dichloro-2-hydroxyphenyl)methyl)piperidin-2-one diastereoisomer 2) as an off-white solid (2.8 mg, 3%): LCMS (ESI) calc'd for C₁₂H₁₄Cl₂N₂O₂ [M + H]⁺: 289, 291 (3 : 2), found 289, 291 (3 : 2); ¹H NMR (400 MHz, CD₃OD) δ 7.32 (s, 1H), 6.91 (s, 1H), 4.02 (d, *J* = 8.3 Hz, 1H), 3.57-3.49 (m, 1H), 3.17 (dd, *J* = 12.4, 10.2 Hz, 1H), 2.41-2.18 (m, 3H), 1.67-1.63 (m, 1H), 1.57-1.43 (m, 1H).

### Example 109. (which is a reference example) Compound 40 (2-[1-amino-1-(piperidin-4-yl)ethyl]-4,5-dichlorophenol)

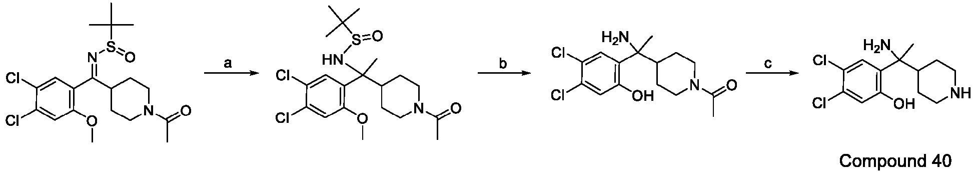

### Step a:

To a stirred solution of CH₃Li (1.4 mL, 2.31 mmol, 1.6 M in ether) in toluene (2 mL) was added the solution of *N*-[(1*Z*)-(1-acetylpiperidin-4-yl)(4,5-dichloro-2-methoxyphenyl)methylidene]-2-methylpropane-2-sulfinamide (0.50 g, 1.15 mmol) and AlMe₃ (0.17 g, 2.31 mmol) in THF (10 mL) dropwise at -78 °C under argon. The reaction was stirred at -78 °C for 8 h. The reaction mixture was quenched with water (20 mL) at -78 °C. The aqueous layer was extracted with EA (3 x 20 mL). The combined organic layers were washed with brine (3 x 30 mL) and dried over anhydrous Na₂SO₄. After filtration, the filtrate was concentrated under reduced pressure. The residue was purified by silica gel column chromatography, eluted with DCM/MeOH (20/1) to afford N-[1-(1-acetylpiperidin-4-yl)-1-(4,5-dichloro-2-methoxyphenyl)ethyl]-2-methylpropane-2-sulfinamide as a light yellow solid (0.10 g, 19%): LCMS (ESI) calc'd for C₂₀H₃₀Cl₂N₂O₃S [M + H]⁺: 449, 451 (3 : 2), found 449, 451 (3 : 2).

### Step b:

To a stirred solution of *N*-[1-(1-acetylpiperidin-4-yl)-1-(4,5-dichloro-2-methoxyphenyl)ethyl]-2-methylpropane-2-sulfinamide (0.10 g, 0.22 mmol) in DCM (2 mL) was added BBr₃ (0.33 g, 1.34 mmol) at room temperature. The reaction was stirred at room temperature for 2 h. The reaction solution was quenched with water (1 mL) and concentrated under reduced pressure to afford 1-[4-[1-amino-1-(4,5-dichloro-2-hydroxyphenyl)ethyl]piperidin-1-yl]ethan-1-one as a light yellow oil (0.10 g, crude), which was used in the next step directly without further purification: LCMS (ESI) calc'd for C₁₅H₂₀Cₗ₂N₂O₂ [M + H]⁺: 331, 333 (3 : 2), found 331, 333 (3 : 2).

### Step c:

A solution of 1-[4-[1-amino-1-(4,5-dichloro-2-hydroxyphenyl)ethyl]piperidin-1-yl]ethan-1-one (0.10 g, 0.30 mmol) in aq. HCl (6 *N*, 4 mL) was stirred at 100 °C for 3 h. The reaction solution was concentrated under reduced pressure. The residue was purified by Prep-HPLC with the following conditions: Column: XBridge C18 OBD Prep Column 100 Å, 10 µm, 19 mm x 250 mm; Mobile Phase A: water with 10 mmoL/L NH₄HCO₃, Mobile Phase B: ACN; Flow rate: 25 mL/min; Gradient: 15% B to 50% B in 8 min; Detector: UV 254/210 nm; Retention time: 6.65 min. The fractions containing the desired product were collected and concentrated under reduced pressure to afford Compound 40 (2-[1-amino-1-(piperidin-4-yl)ethyl]-4,5-dichlorophenol) as a light yellow solid (35 mg, 55% overall two steps): LCMS (ESI) calc'd for C₁₃H₁₈Cl₂N₂O [M + H]⁺ 289, 291 (3 : 2), found 289, 291 (3 : 2); ¹H NMR (300 MHz, CD₃OD) δ 7.15 (s, 1H), 6.78 (s, 1H), 3.29-3.14 (m, 2H), 2.84-2.61 (m, 2H), 2.21-2.03 (m, 1H), 1.98-1.82 (m, 1H), 1.74-1.64 (m, 1H), 1.56 (s, 3H), 1.46-1.28 (m, 2H).

### Example 110. (which is a reference example) Compound 41 (2-(amino(1-benzylpiperidin-4-yl)methyl)-4,5-dichlorophenol trifluoroacetic acid)

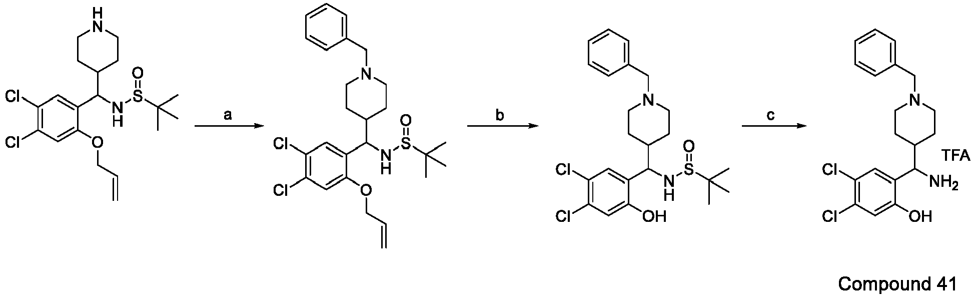

### Step a:

To a stirred mixture of *N*-[[4,5-dichloro-2-(prop-2-en-1-yloxy)phenyl](piperidin-4-yl)methyl]-2-methylpropane-2-sulfinamide (0.10 g, 0.24 mmol) and DIPEA (46 mg, 0.36 mmol) in ACN (5 mL) was added (bromomethyl)benzene (49 mg, 0.29 mmol) in portions at room temperature. The reaction solution was stirred for 1 h at room temperature. The resulting solution was diluted with water (30 mL) at room temperature and extracted with EA (2 x 30 mL). The combined organic layers were washed with brine (2 x 10 mL), dried over anhydrous Na₂SO₄. After filtration, the filtrate was concentrated under reduced pressure. The residue was purified by Prep-TLC, eluted with DCM/MeOH (25/1) to afford *N*-[(1-benzylpiperidin-4-yl)[4,5-dichloro-2-(prop-2-en-1-yloxy)phenyl]methyl]-2-methylpropane-2-sulfinamide as a yellow oil (31 mg, 26%): LCMS (ESI) calc'd for C₂₆H₃₄Cl₂N₂O₂S [M + H]⁺: 509, 511 (3 : 2), found 509, 511 (3 : 2); ¹H NMR (300 MHz, DMSO-d₆) δ 7.56 (s, 1H), 7.36-7.18 (m, 6H), 6.10-5.94 (m, 1H), 5.53-5.23 (m, 3H), 4.63 (d, *J =* 4.8 Hz, 2H), 4.53-4.42 (m, 1H), 3.50 (s, 2H), 2.93-2.67 (m, 2H), 1.98-1.57 (m, 4H), 1.31-1.14 (m, 3H), 1.12 (s, 9H).

### Step b:

To a stirred mixture of *N*-[(1-benzylpiperidin-4-yl)[4,5-dichloro-2-(prop-2-en-1-yloxy)phenyl]methyl]-2-methylpropane-2-sulfinamide (31 mg, 0.06 mmol) and Pd(PPh₃)₄ (7 mg, 0.006 mmol) in THF (2 mL) was added NaBH₄ (4 mg, 0.09 mmol) at room temperature under nitrogen atmosphere. The reaction mixture was stirred for 1 h at room temperature under nitrogen atmosphere. The reaction mixture was quenched with water (10 mL) at room temperature. The aqueous layer was extracted with EA (3 x 20 mL). The combined organic layers were washed with water (2 x 20 mL), dried over anhydrous Na₂SO₄ and filtered. The filtrate was concentrated under reduced pressure to afford crude *N*-((1-benzylpiperidin-4-yl)(4,5-dichloro-2-hydroxyphenyl)methyl)-2-methylpropane-2-sulfinamide. The crude product was used in the next step directly without further purification: LCMS (ESI) calc'd for C₂₃H₃₀Cl₂N₂O₂S [M + H]⁺: 469, 471 (3 : 2), found 469, 471 (3 : 2).

### Step c:

To a solution of *N*-((1-benzylpiperidin-4-yl)(4,5-dichloro-2-hydroxyphenyl)methyl)-2-methylpropane-2-sulfinamide (crude) in 1,4-dioxane (3 mL) was added aq. HCl (6 *N,* 3 mL) at room temperature. The reaction solution was stirred for 2 h at room temperature. The resulting solution was concentrated under reduced pressure. The residue was purified by Prep-HPLC with the following conditions: Column: XSelect CSH Prep C18 OBD Column, 19 x 250 mm, 5 µm; Mobile Phase A: water (plus 0.05% TFA), Mobile Phase B: ACN; Flow rate: 25 mL/min; Gradient: 10% B to 60% B in 6.5 min; Detector: UV 254 nm; Retention time: 5.9 min. The fractions containing desired product were collected concentrated under reduced pressure to afford Compound 41 (2-(amino(1-benzylpiperidin-4-yl)methyl)-4,5-dichlorophenol trifluoroacetic acid) as an off-white solid (12.4 mg, 43% overall two steps): LCMS (ESI) calc'd for C₁₉H₂₂Cl₂N₂O [M + H]⁺: 365, 367 (3 : 2), found 365, 367 (3 : 2); ¹H NMR (300 MHz, CD₃OD) δ 7.62-7.40 (m, 6H), 7.11 (s, 1H), 4.37-4.28 (m, 3H), 3.67-3.58 (m, 1H), 3.54-3.44 (m, 1H), 3.17-2.91 (m, 2H), 2.55-2.38 (m, 1H), 2.32-2.15 (m, 1H), 1.83-1.44 (m, 3H); ¹⁹F NMR (282, CD₃OD) δ -76.98.

### Example 111. (which is a reference example) Compound 42 (2-[amino(8-azabicyclo[3.2.1]octan-3-yl)methyl]-4,5-dichlorophenol trifluoroacetic acid)

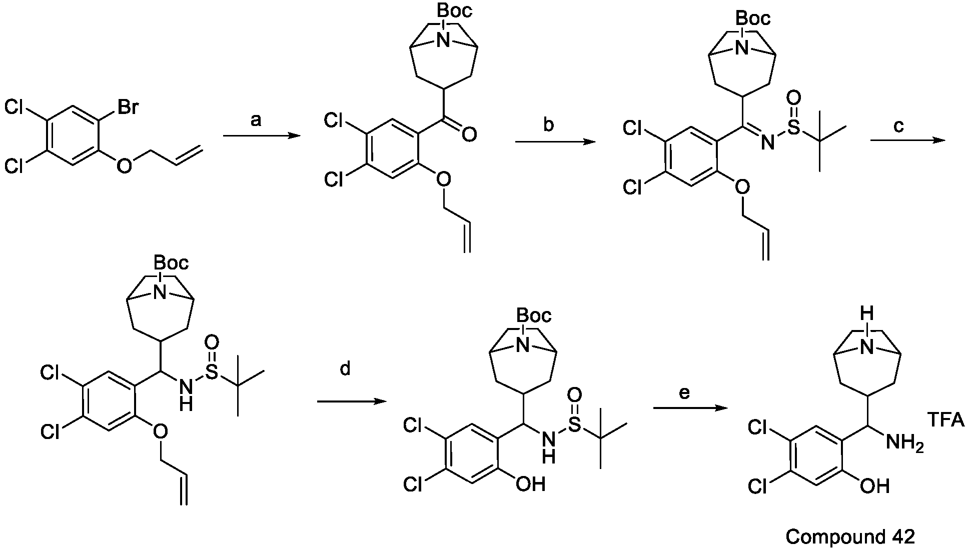

### Step a:

To a stirred solution of 1-bromo-4,5-dichloro-2-(prop-2-en-1-yloxy)benzene (Example 6, step b) (0.71 g, 2.51 mmol) in THF (8 mL) was added *i*-PrMgCl (1.68 mL, 3.36 mmol, 2 M solution in THF) dropwise at 0 °C under nitrogen atmosphere. The reaction was stirred at 0 °C for 30 min. Then *tert*-butyl 3-[methoxy(methyl)carbamoyl]-8-azabicyclo[3.2.1]octane-8-carboxylate (0.50 g, 1.68 mmol) was added into the reaction. The reaction was stirred at 0 °C for 1 h. The reaction was quenched with saturated aq. NH₄Cl (5 mL) and diluted with EA (50 mL) and water (30 mL). The aqueous layer was extracted with EA (3 x 30 mL). The combined organic layers were washed with brine (2 x 30 mL) and dried over anhydrous Na₂SO₄. After filtration, the filtrate was concentrated under reduced pressure. The residue was purified by silica gel column chromatography, eluted with PE/EA (5/1) to afford *tert*-butyl 3-[[2-(but-3-en-1-yl)-4,5-dichlorophenyl]carbonyl]-8-azabicyclo[3.2.1]octane-8-carboxylate as a light yellow oil (0.32 g, 40%): LCMS (ESI) calc'd for C₂₂H₂₇Cl₂NO₄ [M + Na]⁺: 462, 464 (3 : 2), found 462, 464 (3 : 2);

### Step b:

To a stirred solution of tert-butyl 3-[[4,5-dichloro-2-(prop-2-en-1-yloxy)phenyl]carbonyl]-8-azabicyclo[3.2.1]octane-8-carboxylate (0.25 g, 0.57 mmol) and *tert-*butanesulfinamide (83 g, 0.68 mmol) in THF (5 mL) was added Ti(OEt)₄ (0.26 g, 1.14 mmol) at room temperature under nitrogen atmosphere. The reaction was stirred at 70 °C for 16 h. After cooling to room temperature, the reaction solution was quenched with saturated aq. Na₂CO₃ (30 mL). The resulting mixture was filtered through celite and the filtrate was diluted with EA (30 mL) and water (30 mL), and then the aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (5/1) to afford tert-butyl 3-[[4,5-dichloro-2-(prop-2-en-1-yloxy)phenyl][(2-methylpropane-2-sulfinyl)imino]methyl]-8-azabicyclo[3.2.1]octane-8-carboxylate as a yellow solid (0.26 g, 76%): LCMS (ESI) calc'd for C₂₆H₃₆C₁₂N₂O₄S [M + Na]⁺: 565, 567 (3 : 2), found 565, 567 (3 : 2); ¹H NMR (400 MHz, CD₃OD) δ 7.41 (s, 1H), 7.24 (s, 1H), 6.12-5.99 (m, 1H), 5.43 (d, *J =* 17.2 Hz, 1H), 5.31 (dd, *J =* 10.6, 1.6 Hz, 1H), 4.64 (d, *J =* 5.1 Hz, 2H), 4.28-4.16 (m, 2H), 3.31-3.22 (m, 1H), 2.05-1.91 (m, 3H), 1.87-1.66 (m, 5H), 1.48 (s, 9H), 1.22 (s, 9H).

### Step c:

To a stirred solution of tert-butyl 3-[[4,5-dichloro-2-(prop-2-en-1-yloxy)phenyl][(2-methylpropane-2-sulfinyl)imino]methyl]-8-azabicyclo[3.2.1]octane-8-carboxylate (0.20 g, 0.37 mmol) in MeOH (5 mL) was added NaBH₄ (28 mg, 0.74 mmol) at room temperature. The reaction was quenched with saturated aq. NH₄Cl (2 mL) and diluted with EA (30 mL) and water (30 mL). The aqueous layer was extracted with EA (3 x 30 mL). The combined organic layers were washed with brine (2 x 30 mL) and dried over anhydrous Na₂SO₄. After filtration, the filtrate was concentrated under reduced pressure to afford tert-butyl 3-[[4,5-dichloro-2-(prop-2-en-1-yloxy)phenyl][(2-methylpropane-2-sulfinyl)amino]methyl]-8-azabicyclo[3.2.1]octane-8-carboxylate as a light yellow solid (0.20 g, crude), which was used in the next step directly without further purification: LCMS (ESI) calc'd for C₂₆H₃₈Cl₂N₂O₄S [M + H]⁺: 545, 547 (3 : 2), found 545, 547 (3 : 2).

### Step d:

To a stirred solution of tert-butyl 3-[[4,5-dichloro-2-(prop-2-en-1-yloxy)phenyl][(2-methylpropane-2-sulfinyl)amino]methyl]-8-azabicyclo[3.2.1]octane-8-carboxylate (0.20 g, 0.37 mmol) and Pd(PPh₃)₄ (4 mg, 0.004 mmol) in THF (5 mL) was added NaBH₄ (28 mg, 0.73 mmol) at room temperature. The reaction was stirred at room temperature for 30 min. The reaction was quenched with saturated aq. NH₄Cl (1 mL) and concentrated under reduced pressure. After filtration, the filtrate was concentrated under reduced pressure. The residue was purified by Prep-TLC, eluted with PE/EA (1/1) to afford tert-butyl 3-[(4,5-dichloro-2-hydroxyphenyl)[(2-methylpropane-2-sulfinyl)amino]methyl]-8-azabicyclo[3.2.1]octane-8-carboxylate (0.13 g, 54% overall two steps): LCMS (ESI) calc'd for C₂₃H₃₄Cl₂N₂O₄S [M + Na]⁺: 527, 529 (3 : 2), found 527, 529 (3 : 2); ¹H NMR (300 MHz, CD₃OD) δ 7.40 (s, 1H), 6.91 (s, 1H), 4.30-4.00 (m, 3H), 2.42 (s, 1H), 1.97-1.80 (m, 4H), 1.80-1.54 (m, 2H), 1.52-1.43 (m, 10H), 1.27-1.19 (m, 10H).

### Step e:

To a stirred solution of *tert*-butyl 3-[(4,5-dichloro-2-hydroxyphenyl)[(2-methylpropane-2-sulfinyl)amino]methyl]-8-azabicyclo[3.2.1]octane-8-carboxylate (0.13 g, 0.26 mmol) in 1,4-dioxane (2 mL) was added aq. HCl (4 *N,* 1 mL) at room temperature. The resulting mixture was stirred for 1 h at room temperature. The resulting mixture was concentrated under reduced pressure. Then the above mixture was dissolved in DCM (2 mL) was added TFA (0.5 mL) at room temperature. The resulting solution was stirred for 1 h at room temperature. The resulting solution was concentrated under reduced pressure. The residue was purified by Prep-HPLC with the following conditions: Column: XBridge C18 OBD Prep Column 100 Å, 10 µm, 19 mm x 250 mm; Mobile Phase A: water (plus 0.05% TFA), Mobile Phase B: ACN; Flow rate: 25 mL/min; Gradient: 5% B to 60% B in 6.50 min; Detector: UV 254/210 nm; Retention time: 5.40 min. The fractions containing the desired product were collected and concentrated under reduced pressure to afford Compound 42 (2-[amino(8-azabicyclo[3.2.1]octan-3-yl)methyl]-4,5-dichlorophenol trifluoroacetic acid) as a light pink solid (37.7 mg, 47%): LCMS (ESI) calc'd for C₁₄H₁₈Cl₂N₂O [M + H]⁺: 301, 303 (3 : 2), found 301, 303 (3 : 2); ¹H NMR (300 MHz, CD₃OD) δ 7.45 (s, 1H), 7.10 (s, 1H), 4.28-4.09 (m, 2H), 4.06-3.88 (m, 1H), 2.77-2.55 (m, 1H), 2.23-1.99 (m, 4H), 1.99-1.70 (m, 2H), 1.70-1.27 (m, 2H); ¹⁹F NMR (282 MHz, CD₃OD) δ -76.95.

### Example 112. Compound 43 (1-[4-[amino(4,5-dichloro-2-hydroxyphenyl)methyl]piperidin-1-yl]-2-methylpropan-1-one trifluoroacetic acid)

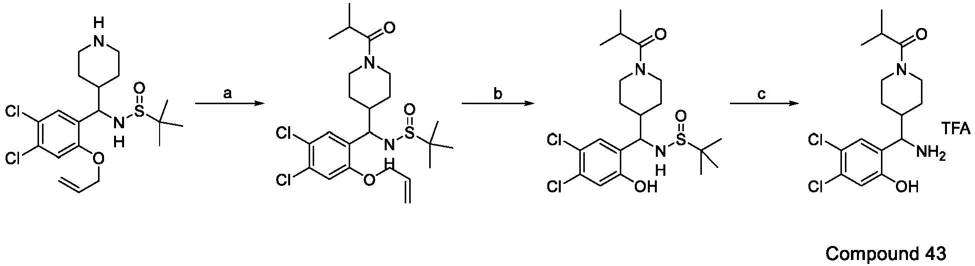

### Step a:

To a stirred solution of HATU (0.27 g, 0.72 mmol) and 2-methylpropanoic acid (63 mg, 0.72 mmol) in DMF (5 mL) were added *N*-[[4,5-dichloro-2-(prop-2-en-1-yloxy)phenyl](piperidin-4-yl)methyl]-2-methylpropane-2-sulfinamide (0.10 g, 0.24 mmol) and Et₃N (72 mg, 0.72 mmol) at room temperature under nitrogen atmosphere. After stirring for 2 h at room temperature under nitrogen atmosphere, the resulting solution was quenched with water (30 mL) at room temperature and extracted with EA (3 x 40 mL). The combined organic layers were washed with brine (2 x 20 mL), dried over anhydrous Na₂SO₄. After filtration, the filtrate was concentrated under reduced pressure. The residue was purified by reverse phase chromatography, eluted with 70% ACN in water with 10 mmol/L NH₄HCO₃ to afford N-[[4,5-dichloro-2-(prop-2-en-1-yloxy)phenyl][1-(2-methylpropanoyl) piperidin-4-yl]methyl]-2-methylpropane-2-sulfinamide as a light yellow oil (70 mg, 60%): LCMS (ESI) calc'd for C₂₃H₃₄Cl₂N₂O₃S [M + H]⁺: 489, 491 (3 : 2), found 489, 491 (3 : 2).

### Step b:

To a stirred solution of *N*-[(4,5-dichloro-2-hydroxyphenyl)[1-(2-methylpropanoyl)piperidin-4-yl]methyl]-2-methylpropane-2-sulfinamide (70 mg, 0.16 mmol) and Pd(PPh₃)₄ (2 mg, 0.002 mmol) in THF (2 mL) were added NaBH₄ (12 mg, 0.31 mmol) at room temperature under nitrogen atmosphere. The reaction mixture was stirred for 1 h at room temperature under nitrogen atmosphere. The resulting mixture was quenched with water (20 mL) and extracted with EA (3 x 20 mL). The combined organic layers were washed with brine (2 x 10 mL), dried over anhydrous Na₂SO₄. After filtration, the filtrate was concentrated under reduced pressure to afford *N*-((4,5-dichloro-2-hydroxyphenyl)(1-isobutyrylpiperidin-4-yl)methyl)-2-methylpropane-2-sulfinamide. The crude product was used in the next step directly without further purification: LCMS (ESI) calc'd for C₂₀H₃₀Cl₂N₂O₃S [M + H]⁺: 449, 451 (3 : 2), found 449, 451 (3 : 2).

### Step c:

To a stirred solution of *N*-((4,5-dichloro-2-hydroxyphenyl)(1-isobutyrylpiperidin-4-yl)methyl)-2-methylpropane-2-sulfinamide (crude) in 1,4-dioxane (3 mL) was added aq. HCl (6 *N,* 1 mL) at room temperature. The reaction solution was stirred for 2 h at room temperature. The resulting solution was concentrated under reduced pressure. The residue was purified by Prep-HPLC with the following conditions: Column: XBridge C18 OBD Prep Column 100 Å, 10 µm, 19 mm x 250 mm; Mobile Phase A: water (plus 0.05% TFA), Mobile Phase B: ACN; Flow rate: 20 mL/min; Gradient: 23% B to 45% B in 9 min; Detector: UV 254/210 nm; Retention time: 6.58 min. The fractions containing desired product were collected and concentrated under reduced pressure to afford Compound 43 (1-[4-[amino(4,5-dichloro-2-hydroxyphenyl)methyl]piperidin-1-yl]-2-methylpropan-1-one trifluoroacetic acid) as a pink solid (12.0 mg, 22% overall two steps): LCMS (ESI) calc'd for C₁₆H₂₂Cl₂N₂O₂ [M + H]⁺: 345, 347 (3 : 2), found 345, 347 (3 : 2); ¹H NMR (300 MHz, CD₃OD) δ 7.43 (d, *J =* 3.3 Hz, 1H), 7.08 (s, 1H), 4.73-4.50 (m, 1H), 4.21-3.98 (m, 2H), 3.14-2.89 (m, 2H), 2.69-2.29 (m, 2H), 2.09-1.93 (m, 1H), 1.43-1.15 (m, 3H), 1.15-0.95 (q, *J =* 6.6 Hz, 6H); ¹⁹F-NMR (282 MHz, CD₃OD) δ -76.98.

### Example 113. Compound 44 (2-(amino(1-(tetrahydro-2H-pyran-4-yl)piperidin-4-yl)methyl)-4,5-dichlorophenol)

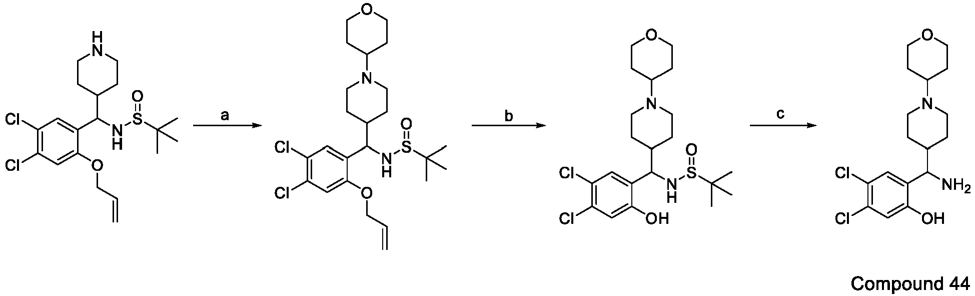

### Step a:

To a stirred mixture of *N*-[[4,5-dichloro-2-(prop-2-en-1-yloxy)phenyl](piperidin-4-yl)methyl]-2-methylpropane-2-sulfinamide (0.20 g, 0.48 mmol), HOAc (29 mg, 0.48 mmol) and dihydro-2*H*-pyran-4(3H)-one (96 mg, 0.95 mmol) in DCM (5 mL) was added NaBH(OAc)₃ (0.30 g, 1.43 mmol) in portions at room temperature. The reaction mixture was stirred for 1 h at room temperature. The resulting mixture was quenched with water (30 mL) at room temperature and extracted with EA (2 x 30 mL). The combined organic layers were washed with brine (2 x 10 mL), dried over anhydrous Na₂SO₄. After filtration, the filtrate was concentrated under reduced pressure. The crude product was purified by reverse phase chromatography, eluted with 50% ACN in water with 10 mmol/L NH₄HCO₃ to afford *N*-[[4,5-dichloro-2-(prop-2-en-1-yloxy)phenyl][1-(oxan-4-yl)piperidin-4-yl]methyl]-2-methylpropane-2-sulfinamide as a yellow oil (0.20 g, 75%): LCMS (ESI) calc'd for C₂₄H₃₆Cl₂N₂O₃S [M + H]⁺: 503, 505 (3 : 2), found 503, 505 (3 : 2).

### Step b:

To a stirred solution *N*-[[4,5-dichloro-2-(prop-2-en-1-yloxy)phenyl][1-(oxan-4-yl)piperidin-4-yl]methyl]-2-methylpropane-2-sulfinamide (0.20 mg, 0.46 mmol) and Pd(PPh₃)₄ (0.11 g, 0.09 mmol) in THF (3 mL) was added NaBH₄ (35 mg, 0.92 mmol) in portions at room temperature under nitrogen atmosphere. The reaction mixture was stirred for 1 h at room temperature under nitrogen atmosphere. The resulting mixture was quenched with water (20 mL) at room temperature and extracted with EA (3 x 20 mL). The combined organic layers were washed with brine (2 x 10 mL), dried over anhydrous Na₂SO₄. After filtration, the filtrate was concentrated under reduced pressure to afford *N*-((4,5-dichloro-2-hydroxyphenyl)(1-(tetrahydro-2*H*-pyran-4-yl)piperidin-4-yl)methyl)-2-methylpropane-2-sulfinamide. The crude product was used in the next step directly without further purification: LCMS (ESI) calc'd for C₂₁H₃₂Cl₂N₂O₃S [M + H]⁺: 463, 465 (3 : 2), found 463, 465 (3 : 2).

### Step c:

To a solution of *N*-((4,5-dichloro-2-hydroxyphenyl)(1-(tetrahydro-2*H*-pyran-4-yl)piperidin-4-yl)methyl)-2-methylpropane-2-sulfinamide (crude) in 1,4-dioxane (3 mL) was added aq. HCl (6 *N,* 3 mL) at room temperature. The reaction solution was stirred for 2 h at room temperature. The resulting solution was concentrated under reduced pressure. The residue was purified by Prep-HPLC with the following conditions: Column: XBridge C18 OBD Prep Column, 100 Å, 10 µm, 19 mm x 250 mm; Mobile Phase A: water with 20 mmol/L NH₄HCO₃, Mobile Phase B: ACN; Flow rate: 25 mL/min; Gradient: 30% B to 40% B in 9 min; Detector: UV 254/210 nm; Retention time: 7.28 min. The fractions containing desired product was collected concentrated under reduced pressure to afford Compound 44 (2-(amino(1-(tetrahydro-2H-pyran-4-yl)piperidin-4-yl)methyl)-4,5-dichlorophenol) as an off-white solid (28 mg, 17% overall two steps): LCMS (ESI) calc'd for C₁₇H₂₄Cl₂N₂O₂ [M + H]⁺: 359, 361 (3 : 2), found 359, 361 (3 : 2); ¹H NMR (400 MHz, CD₃OD) δ 7.19 (s, 1H), 6.85 (s, 1H), 3.99 (d, *J =* 11.9 Hz, 2H), 3.89 (d, *J =* 8.0 Hz, 1H), 3.46-3.35 (m, 2H), 3.12 (d, *J =* 11.4 Hz, 1H), 3.02 (d, *J =* 11.4 Hz, 1H), 2.61-2.43 (m, 1H), 2.27-2.06 (m, 2H), 2.00 (d, *J =* 13.4 Hz, 1H), 1.86-1.73 (m, 3H), 1.62-1.23 (m, 5H).

### Example 114. Compound 45 (2-(amino(1-(pyrrolidin-1-ylsulfonyl)piperidin-4-yl)methyl)-4,5-dichlorophenol trifluoroacetic acid)

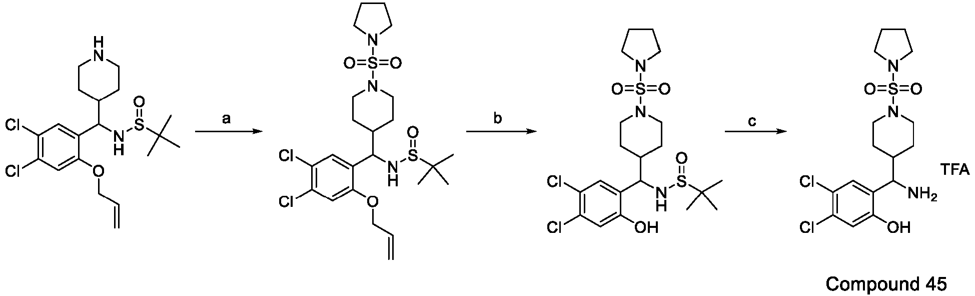

### Step a:

To a stirred solution of *N*-[[4,5-dichloro-2-(prop-2-en-1-yloxy)phenyl](piperidin-4-yl)methyl]-2-methylpropane-2-sulfinamide (0.28 g, 0.67 mmol) and Et₃N (0.20 g, 2.00 mmol) in DCM (5 mL) was added pyrrolidine-1-sulfonyl chloride (0.34 g, 2.00 mmol) in portions at room temperature under nitrogen atmosphere. The reaction solution was stirred for 1 h at room temperature under nitrogen atmosphere. The resulting solution was quenched with water (30 mL) at room temperature and extracted with DCM (3 x 20 mL), dried over anhydrous Na₂SO₄. After filtration, the filtrate was concentrated under reduced pressure. The residue was purified by reverse phase chromatography, eluted with 40% to 80% ACN in water with 20 mmol/L NH₄HCO₃ to afford *N-*[[4,5-dichloro-2-(prop-2-en-1-yloxy)phenyl][1-(pyrrolidine-1-sulfonyl)piperidin-4-yl]methyl]-2-methylpropane-2-sulfinamide as a yellow solid (0.30 g, 73%): LCMS (ESI) calc'd for C₂₃H₃₅Cl₂N₃O₄S₂ [M + H]⁺: 552, 554 (3 : 2), found 552, 554 (3 : 2).

### Step b:

To a stirred solution *N*-[[4,5-dichloro-2-(prop-2-en-1-yloxy)phenyl][1-(pyrrolidine-1-sulfonyl)piperidin-4-yl]methyl]-2-methylpropane-2-sulfinamide (0.30 g, 0.54 mmol) and Pd(PPh₃)₄ (13 mg, 0.01 mmol) in THF (3 mL) was added NaBH₄ (41 mg, 1.09 mmol) in portions at room temperature under nitrogen atmosphere. The reaction mixture was stirred for 1 h at room temperature under nitrogen atmosphere. The resulting mixture was quenched with water (20 mL) at room temperature and extracted with EA (3 x 20 mL). The combined organic layers were washed with brine (2 x 10 mL), dried over anhydrous Na₂SO₄. After filtration, the filtrate was concentrated under reduced pressure to afford crude *N*-((4,5-dichloro-2-hydroxyphenyl)(1-(pyrrolidin-1-ylsulfonyl)piperidin-4-yl)methyl)-2-methylpropane-2-sulfinamide. The crude product was used in the next step directly without further purification: LCMS (ESI) calc'd for C₂₀H₃₁Cl₂N₃O₄S₂ [M + H]⁺: 512, 514 (3 : 2), found 512, 514 (3 : 2).

### Step c:

To a stirred solution of *N*-((4,5-dichloro-2-hydroxyphenyl)(1-(pyrrolidin-1-ylsulfonyl)piperidin-4-yl)methyl)-2-methylpropane-2-sulfinamide (crude) in 1,4-dioxane (2 mL) was added aq. HCl (6 *N,* 1.5 mL) at room temperature. The reaction solution was stirred for 2 h at room temperature. The resulting solution was concentrated under reduced pressure. The residue was purified by Prep-HPLC with the following conditions: Column: XBridge C18 OBD Prep Column, 100 Å, 10 µm, 19 mm x 250 mm; Mobile Phase A: water (plus 0.05% TFA), Mobile Phase B: ACN; Flow rate: 25 mL/min; Gradient: 21% B to 58% B in 6.5 min; Detector: UV 254/210 nm; Retention time: 5.80 min. The fractions containing desired product was collected and concentrated under reduced pressure to afford Compound 45 (2-(amino(1-(pyrrolidin-1-ylsulfonyl)piperidin-4-yl)methyl)-4,5-dichlorophenol trifluoroacetic acid) as an off-white solid (30 mg, 18% overall two steps): LCMS (ESI) calc'd for C₁₆H₂₃Cl₂N₃O₃S [M + H]⁺: 408, 410 (3 : 2), found 408, 410 (3 : 2); ¹H NMR (400 MHz, CD₃OD) δ 7.44 (s, 1H), 7.08 (s, 1H), 4.17 (d, *J =* 9.9 Hz, 1H), 3.81 (d, *J =* 12.7 Hz, 1H), 3.67 (d, *J =* 12.7 Hz, 1H), 3.35-3.24 (m, 4H), 2.85 (td, *J =* 12.5, 2.6 Hz, 1H), 2.73 (td, *J =* 12.3, 3.1 Hz, 1H), 2.27-2.12 (m, 1H), 2.09-1.87 (m, 5H), 1.54-1.24 (m, 3H); ¹⁹F-NMR (376 MHz, CD₃OD) δ -76.95.

### Example 115. (which is a reference example) Compound 46 (4-(amino(4,5-dichloro-2-hydroxyphenyl)methyl)-N,N-dimethylpiperidine-1-carboxamide)

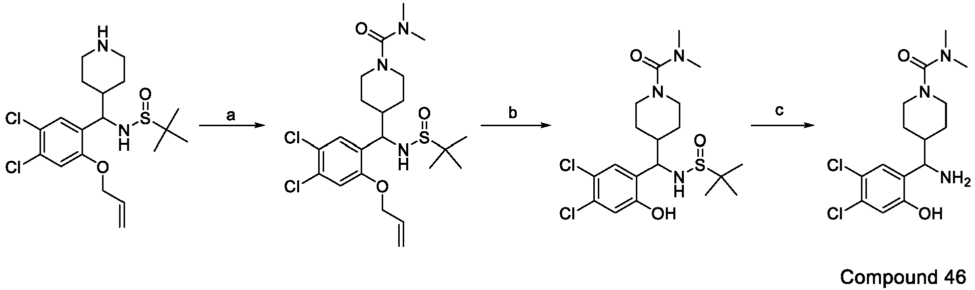

### Step a:

To a stirred solution of *N*-[[4,5-dichloro-2-(prop-2-en-1-yloxy)phenyl](piperidin-4-yl)methyl]-2-methylpropane-2-sulfinamide (0.17 g, 0.41 mmol) and Et₃N (0.12 g, 1.22 mmol) in DCM (3 mL, 47.19 mmol) was added *N,N*-dimethylcarbamoyl chloride (0.13 g, 1.22 mmol) in portions at room temperature under nitrogen atmosphere. The reaction solution was stirred for 1 h at room temperature under nitrogen atmosphere. The reaction solution was quenched with water (30 mL) at room temperature and extracted with EA (2 x 30 mL). The combined organic layers were washed with brine (2 x 10 mL), dried over anhydrous Na₂SO₄. After filtration, the filtrate was concentrated under reduced pressure. The residue was purified by Prep-TLC, eluted with PE/EA (1/1) to afford 4-[[4,5-dichloro-2-(prop-2-en-1-yloxy)phenyl][(2-methylpropane-2-sulfinyl)amino]methyl]-*N,N-*dimethylpiperidine-1-carboxamide as a yellow solid (80 mg, 36%): LCMS (ESI) calc'd for C₂₂H₃₃Cl₂N₃O₃S [M + H]⁺: 490, 492 (3 : 2), found 490, 492 (3 : 2).

### Step b:

To a stirred solution 4-[[4,5-dichloro-2-(prop-2-en-1-yloxy)phenyl][(2-methylpropane-2-sulfinyl)amino]methyl]-*N,N*-dimethylpiperidine-1-carboxamide (80 mg, 0.16 mmol) and Pd(PPh₃)₄ (38 mg, 0.03 mmol) in THF (2 mL) was added NaBH₄ (25 mg, 0.65 mmol) in portions at room temperature under nitrogen atmosphere. The reaction mixture was stirred for 1 h at room temperature under nitrogen atmosphere. The reaction mixture was stirred for 1 h at room temperature. The resulting mixture was quenched with water (20 mL) at room temperature and extracted with EA (3 x 20 mL). The combined organic layers were washed with brine (2 x 10 mL), dried over anhydrous Na₂SO₄. After filtration, the filtrate was concentrated under reduced pressure to afford 4-((4,5-dichloro-2-hydroxyphenyl)(1,1-dimethylethylsulfinamido)methyl)-*N,N*-dimethylpiperidine-1-carboxamide. The crude product was used in the next step directly without further purification: LCMS (ESI) calc'd for C₁₉H₂₉Cl₂N₃O₃S [M + H]⁺: 450, 452 (3 : 2), found 450, 452 (3 : 2).

### Step c:

To a stirred solution of 4-((4,5-dichloro-2-hydroxyphenyl)(1,1-dimethylethylsulfinamido)methyl)-*N*,*N*-dimethylpiperidine-1-carboxamide (crude) in 1,4-dioxane (2 mL) was added aq. HCl (6 *N,* 1.5 mL) at room temperature. The reaction solution was stirred for 2 h at room temperature. The resulting solution was concentrated under reduced pressure. The residue was purified by Prep-HPLC with the following conditions: Column: XBridge C18 OBD Prep Column, 100 Å, 10 µm, 19 mm x 250 mm; Mobile Phase A: water with 20 mmol/L NH₄HCO₃, Mobile Phase B: ACN; Flow rate: 25 mL/min; Gradient: 23% B to 70% B in 6.5 min; Detector: UV 254/210 nm; Retention time: 6.12 min. The fractions containing desired product was collected and concentrated under reduced pressure to afford Compound 46 (4-(amino(4,5-dichloro-2-hydroxyphenyl)methyl)-N,N-dimethylpiperidine-1-carboxamide) as an off-white solid (18 mg, 37% overall two steps): LCMS (ESI) calc'd for C₁₅H₂₁Cl₂N₃O₂ [M + H]⁺: 346, 348 (3 : 2), found 346, 348 (3 : 2); ¹H NMR (400 MHz, CD₃OD) δ 7.21 (s, 1H), 6.85 (s, 1H), 3.89 (d, *J* = 7.5 Hz, 1H), 3.81-3.71 (m, 1H), 3.68-3.59 (m, 1H), 2.84 (s, 6H), 2.83-2.63 (m, 2H), 2.06-1.85 (m, 2H), 1.49-1.18 (m, 3H).

### Example 116. Compound 47 (1-[4-[amino(4,5-dichloro-2-hydroxyphenyl)methyl]piperidin-1-yl]-2-hydroxyethan-1-one)

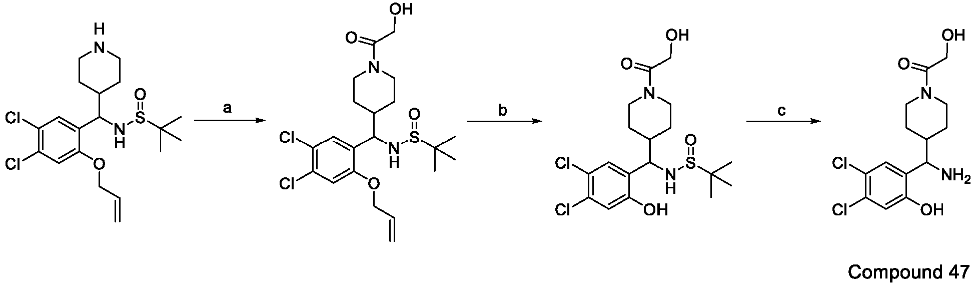

### Step a:

To a stirred solution of HATU (0.27 g, 0.72 mmol) and 2-hydroxyacetic acid (54 mg, 0.72 mmol) in DMF (4 mL) were added a solution of *N*-[[4,5-dichloro-2-(prop-2-en-1-yloxy)phenyl](piperidin-4-yl)methyl]- 2-methylpropane-2-sulfinamide (0.10 g, 0.24 mmol) in DMF (1 mL) and Et₃N (72 mg, 0.72 mmol) dropwise at room temperature under nitrogen atmosphere. The reaction solution was stirred for 2 h at room temperature under nitrogen atmosphere. The reaction solution was quenched with water (30 mL) at room temperature. The aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by reverse phase flash, eluted 50% ACN in water with 10 mmol/L NH₄HCO₃ to afford N-[[4,5-dichloro-2-(prop-2-en-1-yloxy)phenyl][1-(2-hydroxyacetyl)piperidin-4-yl]methyl]-2-methylpropane-2-sulfinamide as a light yellow oil (60 mg, 53%): LCMS (ESI) calc'd for C₂₁H₃₀Cl₂N₂O₄S [M + H]⁺: 477, 479 (3 : 2), found 477, 479 (3 : 2).

### Step b:

To a stirred mixture of *N*-[[4,5-dichloro-2-(prop-2-en-1-yloxy)phenyl][1-(2-hydroxyacetyl)piperidin-4-yl] methyl]-2-methylpropane-2-sulfinamide (60 mg, 0.13 mmol) and Pd(PPh₃)₄ (3 mg, 0.003 mmol) in THF (3 mL) was added NaBH₄ (5 mg, 0.13 mmol) at room temperature under nitrogen atmosphere. The mixture was stirred for 1 h at room temperature under nitrogen atmosphere. The reaction mixture was quenched with water (10 mL). The aqueous layer was extracted with EA (3 x 20 mL). The combined organic layers were washed with water (2 x 20 mL), dried over anhydrous Na₂SO₄ and filtered. The filtrate was concentrated under reduced pressure to afford crude *N*-((4,5-dichloro-2-hydroxyphenyl)(1-(2-hydroxyacetyl)piperidin-4-yl)methyl)-2-methylpropane-2-sulfinamide. The crude product was used in the next step directly without further purification: LCMS (ESI) calc'd for C₁₈H₂₆Cl₂N₂O₄S [M + H]⁺: 437, 439 (3 : 2), found 437, 439 (3 : 2).

### Step c:

To a solution of N-[(4,5-dichloro-2-hydroxyphenyl)[1-(2-hydroxyacetyl)piperidin-4-yl]methyl]-2-methylpropane-2-sulfinamide (0.10 g, 0.23 mmol) in 1,4-dioxane (3 mL) was added aq. HCl (6 *N,* 3 mL) at room temperature. The reaction solution was stirred for 2 h at room temperature. The resulting solution was concentrated under reduced pressure. The residue was purified by Prep-HPLC with the following conditions: Column: XBridge C18 OBD, Prep Column 100 Å, 10 µm, 19 mm x 250 mm; Mobile Phase A: water with 20 mmol/L NH₄HCO₃, Mobile Phase B: ACN; Flow rate: 25 mL/min; Gradient: 23% B to 50% B in 6.5 min; Detector: UV 254/210 nm; Retention time: 5.67 min. The fractions containing desired product were collected and concentrated under reduced pressure to afford Compound 47 (1-[4-[amino(4,5-dichloro-2-hydroxyphenyl)methyl]piperidin-1-yl]-2-hydroxyethan-1-one) as a yellow solid (20 mg, 13%): LCMS (ESI) calc'd for C₁₄H₁₈Cl₂N₂O₃ [M + H]⁺: 333, 335 (3 : 2), found 333, 335 (3 : 2); ¹H NMR (400 MHz, CD₃OD) δ 7.24 (s, 1H), 6.89 (s, 1H), 4.57 (dd, *J* = 38.8 Hz, 12.8 Hz, 1H), 4.27-4.19 (m, 2H), 3.92 (t, *J* = 8.0 Hz, 1H), 3.72 (dd, *J* = 38.8 Hz, 12.8 Hz, 1H), 3.05-2.91 (m, 1H), 2.71-2.57 (m, 1H), 2.15-1.90 (m, 2H), 1.50-1.15 (m, 3H).

### Example 117. Compound 48 (1-(4-(amino(4,5-dichloro-2-hydroxyphenyl)methyl)piperidine-1-carbonyl)cyclopropanecarbonitrile trifluoroacetic acid)

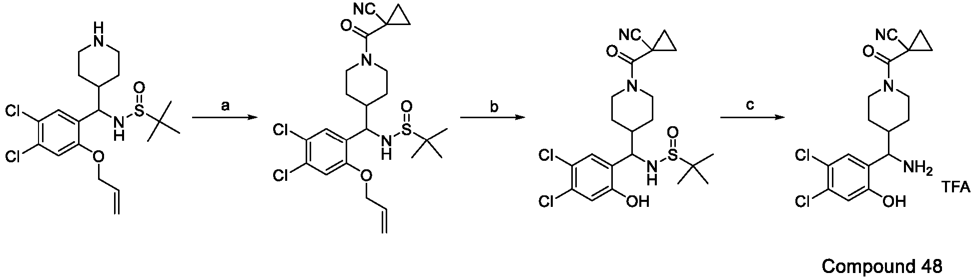

### Step a:

To a stirred solution of HATU (0.42 g, 1.09 mmol) and 1-cyanocyclopropane-1-carboxylic acid (48 mg, 0.43 mmol) in DMF (3 mL) were added Et₃N (43 mg, 0.43 mmol) and a solution of *N*-[[4,5-dichloro-2-(prop-2-en-1-yloxy)phenyl](piperidin-4-yl)methyl]-2-methylpropane-2-sulfinamide (90 mg, 0.21 mmol) in DMF (2 mL) at room temperature under nitrogen atmosphere. The reaction solution was stirred for 1 h at room temperature under nitrogen atmosphere. After stirring for additional 2 h at room temperature, the reaction solution was quenched with water (20 mL) at room temperature and extracted with EA (3 x 30 mL). The combined organic layers were washed with brine (3 x 30 mL), dried over anhydrous Na₂SO₄. After filtration, the filtrate was concentrated under reduced pressure. The residue was purified by reverse phase chromatography, eluted with 30% to 70% ACN in water with 10 mmol/L NH₄HCO₃ to afford N-([1-[(1-cyanocyclopropyl)carbonyl]piperidin-4-yl][4,5-dichloro-2-(prop-2-en-1-yloxy)phenyl]methyl)-2-methylpropane-2-sulfinamide as a yellow oil (0.10 g, 82%): LCMS (ESI) calc'd for C₂₄H₃₁Cl₂N₃O₃S [M + H]⁺: 512, 514 (3 : 2), found 512, 514 (3 : 2).

### Step b:

To a stirred solution *N*-([1-[(1-cyanocyclopropyl)carbonyl]piperidin-4-yl][4,5-dichloro-2-(prop-2-en-1-yloxy)phenyl]methyl)-2-methylpropane-2-sulfinamide (0.10 g, 0.20 mmol) and Pd(PPh₃)₄ (5 mg, 0.02 mmol) in THF (3 mL) was added NaBH₄ (15 mg, 0.39 mmol) in portions at room temperature under nitrogen atmosphere. The reaction mixture was stirred for 1 h at room temperature under nitrogen atmosphere. The resulting mixture was quenched with water (20 mL) and extracted with EA (3 x 20 mL). The combined organic layers were washed with brine (2 x 10 mL), dried over anhydrous Na₂SO₄. After filtration, the filtrate was concentrated under reduced pressure to afford *N*-((1-(1-cyanocyclopropanecarbonyl)piperidin-4-yl)(4,5-dichloro-2-hydroxyphenyl)methyl)-2-methylpropane-2-sulfinamide. The crude product was used in the next step directly without further purification: LCMS (ESI) calc'd for C₂₁H₂₇Cl₂N₃O₃S [M + H]⁺: 472, 474 (3 : 2), found 472, 474 (3 : 2).

### Step c:

To a stirred solution of N-((1-(1-cyanocyclopropanecarbonyl)piperidin-4-yl)(4,5-dichloro-2-hydroxyphenyl)methyl)-2-methylpropane-2-sulfinamide (crude) in 1,4-dioxane (2 mL) was added aq. HCl (6 *N,* 1.5 mL) at room temperature. The reaction solution was stirred for 2 h at room temperature. The resulting solution was concentrated under reduced pressure. The residue was purified by Prep-HPLC with the following conditions: Column: XBridge C18 OBD Prep Column, 100 Å, 10 µm, 19 mm x 250 mm; Mobile Phase A: water (plus 0.05% TFA), Mobile Phase B: ACN; Flow rate: 25 mL/min; Gradient: 18% B to 68% B in 7 min; Detector: UV 254/210 nm; Retention time: 5.20 min. The fractions containing desired product were collected and concentrated under reduced pressure to afford Compound 48 (1-(4-(amino(4,5-dichloro-2-hydroxyphenyl)methyl)piperidine-1-carbonyl)cyclopropanecarbonitrile trifluoroacetic acid) as an off-white solid (28.4 mg, 53% overall two steps): LCMS (ESI) calc'd for C₁₇H₁₉Cl₂N₃O₂ [M + H]⁺: 368, 370 (3 : 2), found 368, 370 (3 : 2); ¹H NMR (400 MHz, CD₃OD) δ 7.45 (s, 1H), 7.09 (s, 1H), 4.54 (d, *J* = 14.0 Hz, 1H), 4.40 (d, *J* = 13.8 Hz, 1H), 4.19 *(d, J* = 9.9 Hz, 1H), 3.30-3.02 (m, 1H), 2.95-2.58 (m, 1H), 2.51-2.38 (m, 1H), 2.14-2.01 (m, 1H), 1.58 (s, 4H), 1.50-1.28 (m, 3H); ¹⁹F-NMR (376 MHz, CD₃OD) δ -77.03.

### Example 118. Compound 49 (1-(4-(amino(4,5-dichloro-2-hydroxyphenyl)methyl)piperidin-1-yl)-3-hydroxy-3-methylbutan-1-one trifluoroacetic acid)

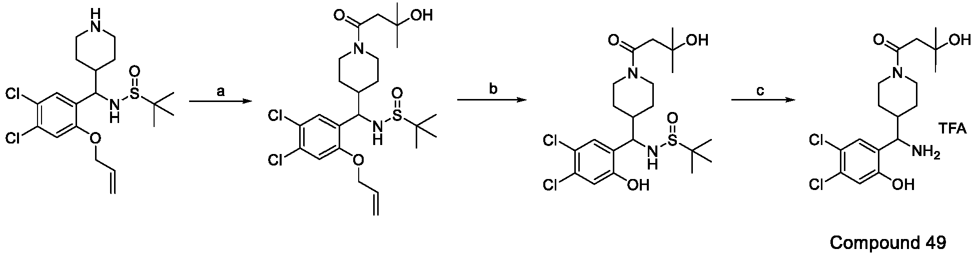

### Step a:

To a stirred solution of HATU (0.49 g, 1.29 mmol) and 3-hydroxy-3-methylbutanoic acid (0.10 g, 0.86 mmol) in DMF (3 mL) were added Et₃N (0.86 g, 0.86 mmol) and a solution of *N*-[[4,5-dichloro-2-(prop-2-en-1-yloxy)phenyl](piperidin-4-yl)methyl]-2-methylpropane-2-sulfinamide (0.18 g, 0.43 mmol) in DMF (2 mL) at room temperature under nitrogen atmosphere. After stirring for additional 2 h at room temperature, the reaction solution was quenched with water (20 mL) at room temperature and extracted with EA (3 x 30 mL). The combined organic layers were washed with brine (3 x 30 mL), dried over anhydrous Na₂SO₄. After filtration, the filtrate was concentrated under reduced pressure. The residue was purified by reverse phase chromatography, eluted with 50% to 80% ACN in water (plus 0.05% TFA) to afford *N*-((2-(allyloxy)-4,5-dichlorophenyl)(1-(3-hydroxy-3-methylbutanoyl)piperidin-4-yl)methyl)-2-methylpropane-2-sulfinamide as a red solid (0.14 g, 54%): LCMS (ESI) calc'd for C₂₄H₃₆Cl₂N₂O₄S [M + H]⁺: 519, 521 (3 : 2), found 519, 521 (3 : 2).

### Step b:

To a stirred mixture of *N*-((2-(allyloxy)-4,5-dichlorophenyl)(1-(3-hydroxy-3-methylbutanoyl)piperidin-4-yl)methyl)-2-methylpropane-2-sulfinamide (0.14, 0.27 mmol) and Pd(PPh₃)₄ (34 mg, 0.03 mmol) in THF (5 mL) was added NaBH₄ (16 mg, 0.41 mmol) at room temperature under nitrogen atmosphere. The mixture was stirred for 1 h at room temperature under nitrogen atmosphere. The reaction mixture was quenched with water (30 mL) and extracted with EA (3 x 30 mL). The combined organic layers were washed with water (2 x 20 mL), dried over anhydrous Na₂SO₄ and filtered. The filtrate was concentrated under reduced pressure to afford crude *N*-((4,5-dichloro-2-hydroxyphenyl)(1-(3-hydroxy-3-methylbutanoyl)piperidin-4-yl)methyl)-2-methylpropane-2-sulfinamide. The crude product was used in the next step directly without further purification: LCMS (ESI) calc'd for C₂₁H₃₂Cl₂N₂O₄S [M + H]⁺: 479, 481 (3 : 2), found 479, 481 (3 : 2).

### Step c:

To a solution of *N*-((4,5-dichloro-2-hydroxyphenyl)(1-(3-hydroxy-3-methylbutanoyl)piperidin-4-yl)methyl)-2-methylpropane-2-sulfinamide (crude) in 1,4-dioxane (3 mL) was added aq. HCl (6 *N,* 3 mL) at room temperature. The reaction solution was stirred for 2 h at room temperature. The resulting solution was concentrated under reduced pressure. The residue was purified by Prep-HPLC with the following conditions: Column: XBridge C18 OBD Prep Column, 100 Å, 10 µm, 19 mm x 250 mm; Mobile Phase A: water (plus 0.05% TFA), Mobile Phase B: ACN; Flow rate: 25 mL/min; Gradient: 5% B to 50% B in 7 min; Detector: UV 254/210 nm; Retention time: 6.28 min. The fractions containing desired product were collected and concentrated under reduced pressure to afford Compound 49 (1-(4-(amino(4,5-dichloro-2-hydroxyphenyl)methyl)piperidin-1-yl)-3-hydroxyl-3-methylbutan-1-one trifluoroacetic acid) as an off-white solid (37 mg, 49% overall two steps): LCMS (ESI) calc'd for C₁₇H₂₄Cl₂N₂O₃ [M + H]⁺: 375, 377 (3 : 2), found 375, 377 (3 : 2); ¹H NMR (400 MHz, CD₃OD) δ 7.44 (d, *J =* 1.5 Hz, 1H), 7.09 (s, 1H), 4.66 (dd, *J* = 54.9, 13.4 Hz, 1H), 4.25-4.03 (m, 2H), 3.20-2.96 (m, 1H), 2.75-2.53 (m, 3H), 2.47-2.33 (m, 1H), 2.01 (d, *J* = 12.8 Hz, 1H), 1.47-1.12 (m, 9H); ¹⁹F-NMR (376 MHz, CD₃OD) δ -77.00.

### Example 119. Compound 50 ((2S)-2-amino-1-(4-(amino(4,5-dichloro-2-hydroxyphenyl)methyl)piperidin-1-yl)propan-1-one trifluoroacetic acid)

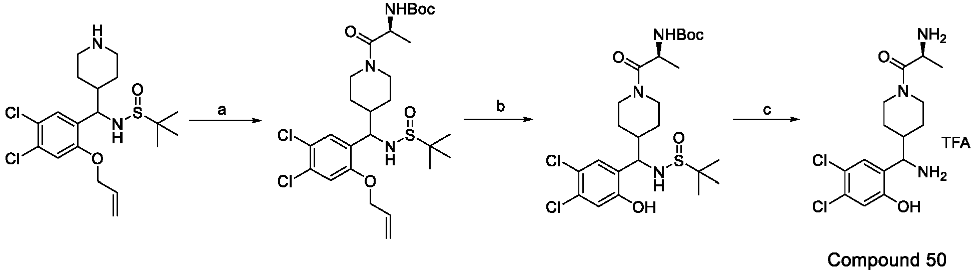

### Step a:

To a stirred solution of (*S*)-2-((*tert*-butoxycarbonyl)amino)propanoic acid (0.20 g, 1.06 mmol) and *N*-[[4,5-dichloro-2-(prop-2-en-1-yloxy)phenyl](piperidin-4-yl)methyl]-2-methylpropane-2-sulfinamide (0.44 g, 1.06 mmol) in DMF (5 mL) were added Et₃N (0.32 g, 3.18 mmol) and EDCI (0.61 g, 3.18 mmol) at room temperature under nitrogen atmosphere. The reaction solution was stirred for 2 h at room temperature under nitrogen atmosphere. The reaction solution was quenched with water (30 mL) at room temperature and extracted with EA (3 x 50 mL). The combined organic layers were washed with brine (3 x 30 mL), dried over anhydrous Na₂SO₄. After filtration, the filtrate was concentrated under reduced pressure. The residue was purified by silica gel column chromatography, eluted with PE/EA (9/1) to afford *tert*-butyl ((2*S*)-1-(4-((2-(allyloxy)-4,5-dichlorophenyl)(1,1-dimethylethylsulfinamido)methyl)piperidin-1-yl)-1-oxopropan-2-yl)carbamate as a yellow solid (0.15 g, 42%): LCMS (ESI) calc'd for C₂₇H₄₁Cl₂N₃O₅S [M + H]⁺: 590, 592 (3 : 2), found 590, 592 (3 : 2).

### Step b:

To a stirred mixture of *tert*-butyl ((2*S*)-1-(4-((2-(allyloxy)-4,5-dichlorophenyl)(1,1-dimethylethylsulfinamido)methyl)piperidin-1-yl)-1-oxopropan-2-yl)carbamate (100 mg, 0.17 mmol) and Pd(PPh₃)₄ (4 mg, 0.002 mmol) in THF (3 mL) was added NaBH₄ (16 mg, 0.34 mmol) at room temperature under nitrogen atmosphere. The mixture was stirred for 1 h at room temperature under nitrogen atmosphere. The reaction mixture was quenched with water (10 mL). The aqueous layer was extracted with EA (3 x 20 mL). The combined organic layers were washed with water (2 x 20 mL), dried over anhydrous Na₂SO₄ and filtered. The filtrate was concentrated under reduced pressure to afford crude *tert*-butyl ((2*S*)-1-(4-((4,5-dichloro-2-hydroxyphenyl)(1,1-dimethylethylsulfinamido)methyl)piperidin-1-yl)-1-oxopropan-2-yl)carbamate. The crude product was used in the next step directly without further purification: LCMS (ESI) calc'd for C₂₄H₃₇Cl₂N₃O₅S [M + H]⁺: 550, 552 (3 : 2), found 550, 552 (3 : 2).

### Step c:

To a solution of *tert*-butyl ((2*S*)-1-(4-((4,5-dichloro-2-hydroxyphenyl)(1,1-dimethylethylsulfinamido)methyl)piperidin-1-yl)-1-oxopropan-2-yl)carbamate (crude) in 1,4-dioxane (3 mL) was added aq. HCl (6 *N,* 3 mL) at room temperature. The reaction solution was stirred for 1 h at room temperature. The reaction solution was concentrated under reduced pressure. The residue was dissolved in DCM (6 mL), and then TFA (3 mL) was added at room temperature. The reaction solution was stirred for additional 1 h at room temperature. The resulting solution was concentrated under reduced pressure. The residue was purified by Prep-HPLC with the following conditions: Column: XBridge C18 OBD Prep Column, 100 Å, 10 µm, 19 mm s 250 mm; Mobile Phase A: water (plus 0.05% TFA), Mobile Phase B: ACN; Flow rate: 25 mL/min; Gradient: 5% B to 42% B in 6.5 min; Detector: UV 254/210 nm; Retention time: 5.80 min. The fractions containing desired product were collected and concentrated under reduced pressure to afford Compound 50 ((2S)-2-amino-1-(4-(amino(4,5-dichloro-2-hydroxyphenyl)methyl)piperidin-1-yl)propan-1-one trifluoroacetic acid) as a yellow solid (47.2 mg, 53% overall two steps): LCMS (ESI) calc'd for C₁₅H₂₁Cl₂N₃O₂ [M + H]⁺: 346, 348 (3 : 2), found 346, 348 (3 : 2); ¹H NMR (300 MHz, CD₃OD) δ 7.44 (d, *J* = 7.2 Hz, 1H), 7.09 (s, 1H), 4.56 (dd, *J* = 38.8 Hz, 12.8 Hz, 1H), 4.45-4.36 (m, 1H), 4.26-4.13 (m, 1H), 3.92 (dd, *J* = 38.8 Hz, 12.8 Hz, 1H), 3.25-3.01 (m, 1H), 2.85-2.57 (m, 1H), 2.52-2.32 (m, 1H), 2.15-2.01 (m, 1H), 1.46 (d, *J* = 12.8 Hz, 3H), 1.44-1.05 (m, 3H); ¹⁹F-NMR (282 MHz, CD₃OD) δ -76.93.

### Example 120. (which is a reference example) Compound 51 (2-[amino[1-(cyclopropylmethyl)piperidin-4-yl]methyl]-4,5-dichlorophenol trifluoroacetic acid)

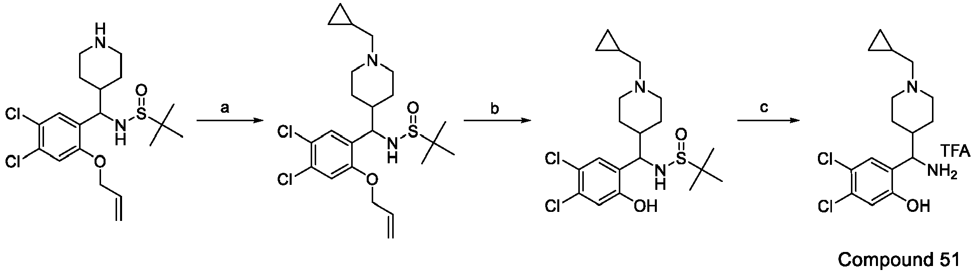

### Step a:

To a stirred solution of *N*-[[4,5-dichloro-2-(prop-2-en-1-yloxy)phenyl](piperidin-4-yl)methyl]-2-methylpropane-2-sulfinamide (0. 20 g, 0.48 mmol) and cyclopropanecarbaldehyde (50 mg, 0.71 mmol) in MeOH (4 mL) were added AcOH (29 mg, 0.48 mmol) and NaBH(OAc)₃ (0.30 g, 1.43 mmo) at room temperature under nitrogen atmosphere. The resulting mixture was stirred for 1 h at room temperature under nitrogen atmosphere. The resulting mixture was quenched with water (20 mL) at room temperature and extracted with EA (3 x 40 mL). The combined organic layers were washed with brine (2 x 20 mL), dried over anhydrous Na₂SO₄. After filtration, the filtrate was concentrated under reduced pressure. The residue was purified by reverse phase chromatography, eluted with 70% MeOH in water with 10 mmol/L NH₄HCO₃ to afford *N*-[[1-(cyclopropylmethyl)piperidin-4-yl][4,5-dichloro-2-(prop-2-en-1-yloxy)phenyl]methyl]-2-methylpropane-2-sulfinamide as a yellow oil (0.17 g, 75%): LCMS (ESI) calc'd for C₂₃H₃₄Cl₂N₂O₂S [M + H]⁺: 473, 475 (3 : 2), found 473, 475 (3 : 2).

### Step b:

To a stirred mixture of *N*-[[1-(cyclopropylmethyl)piperidin-4-yl][4,5-dichloro-2-(prop-2-en-1-yloxy)phenyl]methyl]-2-methylpropane-2-sulfinamide (0.17g, 0.36 mmol) and Pd(PPh₃)₄ (8 mg, 0.01 mmol) in THF (4 mL) was added NaBH₄ (20 mg, 0.54 mmol) at room temperature under nitrogen atmosphere. The reaction mixture was stirred for 1 h at room temperature under nitrogen atmosphere. The resulting mixture was quenched with water (30 mL) at room temperature. The aqueous layer was extracted with EA (3 x 20 mL). The combined organic layers were washed with water (2 x 20 mL), dried over anhydrous Na₂SO₄ and filtered. The filtrate was concentrated under reduced pressure to afford crude *N*-((1-(cyclopropylmethyl)piperidin-4-yl)(4,5-dichloro-2-hydroxyphenyl) methyl) -2-methylpropane-2-sulfinamide. The crude product was used in the next step directly without further purification: LCMS (ESI) calc'd for C₂₀H₃₀Cl₂N₂O₂S [M + H]⁺: 433, 435 (3 : 2), found 433, 435 (3 : 2).

### Step c:

To a stirred solution of *N*-[[1-(cyclopropylmethyl)piperidin-4-yl](4,5-dichloro-2-hydroxyphenyl)methyl]-2-methylpropane-2-sulfinamide (0.12 g, 0.28 mmol) in 1,4-dioxane (3 mL) was added aq. HCl (6 *N,* 1 mL) at room temperature. The reaction solution was stirred for 2 h at room temperature. The resulting solution was concentrated under reduced pressure. The residue was purified by Prep-HPLC with the following conditions: Column: XBridge C18 OBD Prep Column 100 Å, 10 µm, 19 mm x 250 mm; Mobile Phase A: water (plus 0.05% TFA), Mobile Phase B: ACN; Flow rate: 25 mL/min; Gradient: 33% B to 50% B in 6.5 min; Detector: UV 254/210 nm; Retention time: 5.86 min. The fractions containing desired product were collected and concentrated under reduced pressure to afford Compound 51 (2-[amino[1-(cyclopropylmethyl)piperidin-4-yl]methyl]-4,5-dichlorophenol trifluoroacetic acid) as an off-white solid (18 mg, 19% overall two steps): LCMS (ESI) calc'd for C₁₆H₂₂Cl₂N₂O [M + H]⁺: 329, 331 (3 : 2), found 329, 331 (3 : 2); ¹H NMR (300 MHz, CD₃OD) δ 7.48 (s, 1H), 7.11 (s, 1H), 4.26 (d, *J* = 9.5 Hz, 1H), 3.79 (d, *J* = 12.6 Hz, 1H), 3.66 (d, *J* = 12.6 Hz, 1H), 3.10-2.87 (m, 4H), 2.53-2.36 (m, 1H), 2.25 (d, *J* = 14.2 Hz, 1H), 1.80-1.49 (m, 3H), 1.20-1.03 (m, 1H), 0.86-0.71 (m, 2H), 0.50-0.30 (m, 2H).

### Example 121. (which is a reference example) Compound 52 (2-[amino(azetidin-3-yl)methyl]-4,5-dichlorophenol trifluoroacetic acid)

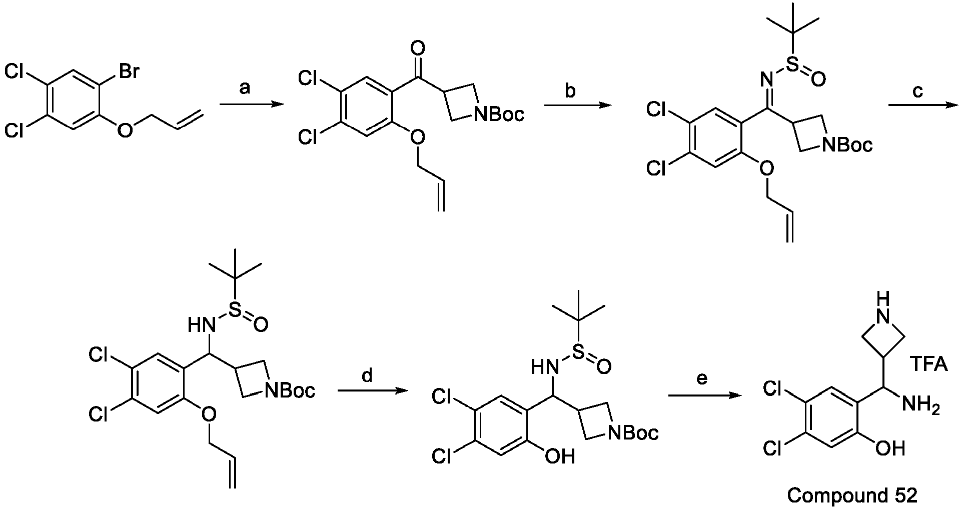

### Step a:

To a stirred solution of 1-bromo-4,5-dichloro-2-(prop-2-en-1-yloxy)benzene (Example 6, step b) (2.40 g, 8.60 mmol) in THF (20 mL) was added i-PrMgCl (5.73 mL, 11.46 mmol, 2 M in THF) dropwise at 0 °C under nitrogen atmosphere. The reaction was stirred at 0 °C for 30 min. Then tert-butyl 3-[methoxy(methyl)carbamoyl]azetidine-1-carboxylate (1.40 g, 5.73 mmol) was added into the reaction. The reaction was stirred at 0 °C for additional 1.5 h. The reaction was quenched with saturated aq. NH₄Cl (5 mL) and diluted with EA (50 mL) and water (30 mL). The aqueous layer was extracted with EA (3 x 30 mL). The combined organic layers were washed with brine (2 x 30 mL) and dried over anhydrous Na₂SO₄. After filtration, the filtrate was concentrated under reduced pressure. The residue was purified by silica gel column chromatography, eluted with PE/EA (4/1) to afford tert-butyl 3-[[4,5-dichloro-2-(prop-2-en-1-yloxy)phenyl]carbonyl]azetidine-1-carboxylate as a light yellow oil (1.30 g, 53%): LCMS (ESI) calc'd for C₁₈H₂₁Cl₂NO₄ [M + Na]⁺: 408, 410 (3 : 2), found 408, 410 (3 : 2); ¹H NMR (400 MHz, CD₃OD) δ 7.90 (s, 1H), 7.37 (s, 1H), 6.28-6.06 (m, 1H), 5.56-5.36 (m, 2H), 4.74 (d, *J* = 5.8, Hz, 2H), 4.19-4.00 (m, 5H), 1.46 (s, 9H).

### Step b:

To a stirred solution of *tert*-butyl 3-[[4,5-dichloro-2-(prop-2-en-1-yloxy)phenyl]carbonyl]azetidine-1-carboxylate (0.50 g, 1.29 mmol) and tert-butanesulfinamide (0.47 g, 3.88 mmol) in THF (8 mL) was added Ti(OEt)₄ (2.36 g, 10.36 mmol) at room temperature under nitrogen atmosphere. The reaction was stirred at 70 °C for 16 h. The reaction was quenched with saturate aq Na₂CO₃ (30 mL). The resulting mixture was filtered through celite and the filtrate was diluted with EA (30 mL) and water (30 mL) and the aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (5/1) to afford tert-butyl 3-[[4,5-dichloro-2-(prop-2-en-1-yloxy)phenyl][(2-methylpropane-2-sulfinyl)imino]methyl]azetidine-1-carboxylate as a yellow oil (0.49 g, 70%): LCMS (ESI) calc'd for C₂₂H₃₀Cl₂N₂O₄S [M + H]⁺: 489, 491 (3 : 2), found 489, 491 (3 : 2).

### Step c:

To a stirred solution of *tert*-butyl 3-[[4,5-dichloro-2-(prop-2-en-1-yloxy)phenyl][(2-methylpropane-2-sulfinyl)imino]methyl]azetidine-1-carboxylate (0.49 g, 1.00 mmol) in MeOH (5 mL) were added NaBH₄ (76 mg, 2.00 mmol) at room temperature. The reaction was quenched with saturated aq. NH₄Cl (2 mL) and diluted with EA (30 mL) and water (30 mL). The aqueous layer was extracted with EA (3 x 30 mL). The combined organic layers were washed with brine (2 x 30 mL) and dried over anhydrous Na₂SO₄. After filtration, the filtrate was concentrated under reduced pressure to afford tert-butyl 3-[[4,5-dichloro-2-(prop-2-en-1-yloxy)phenyl][(2-methylpropane-2-sulfinyl)amino]methyl]azetidine-1-carboxylate as a light yellow solid (0.50 g, crude), which was used in the next step directly without further purification: LCMS (ESI) calc'd for C₂₂H₃₂Cl₂N₂O₄S [M + Na]⁺: 513, 515 (3 : 2), found 513, 515 (3 : 2).

### Step d:

To a stirred solution of *tert*-butyl 3-[[4,5-dichloro-2-(prop-2-en-1-yloxy)phenyl][(2-methylpropane-2-sulfinyl)amino]methyl]azetidine-1-carboxylate (0.50 g, 1.02 mmol) and Pd(PPh₃)₄ (59 mg, 0.05 mmol) in THF (5 mL) was added NaBH₄ (77 mg, 2.03 mmol) at room temperature. The reaction was stirred at room temperature for 1 h. The reaction was quenched with saturated aq. NH₄Cl (30 mL). The resulting mixture was extracted with EA (3 x 30 mL). The combined organic layers were washed with brine (2 x 20 mL), dried over anhydrous Na₂SO₄. After filtration, the filtrate was concentrated under reduced pressure. The residue was purified by Prep-TLC, eluted with PE/EA (5/1) to afford tert-butyl 3-[(4,5-dichloro-2-hydroxyphenyl)[(2-methylpropane-2-sulfinyl)amino]methyl]azetidine-1-carboxylate (0.30 g, 65% overall two steps): LCMS (ESI) calc'd for C₁₉H₂₈Cl₂N₂O₄S [M + Na]⁺ 473, 475 (3 : 2), found 473, 475 (3 : 2).

### Step e:

To a stirred solution of *tert*-butyl 3-[(4,5-dichloro-2-hydroxyphenyl)[(2-methylpropane-2-sulfinyl)amino]methyl]azetidine-1-carboxylate (0.20 g, 0.44 mmol) in 1,4-dioxane (4 mL) was added aq. HCl (4 *N,* 2 mL) at room temperature. The resulting mixture was stirred for 1 h at room temperature. The resulting mixture was concentrated under reduced pressure. Then the above mixture was dissolved in DCM (2 mL) was added TFA (0.5 mL) at room temperature. The resulting solution was stirred for 1 h at room temperature. The resulting solution was concentrated under reduced pressure. The residue was purified by Prep-HPLC with the following conditions: Column: XBridge C18 OBD Prep Column 100 Å, 10 µm, 19 mm x 250 mm; Mobile Phase A: water (plus 0.05% TFA), Mobile Phase B: ACN; Flow rate: 25 mL/min; Gradient: 6% B to 18% B in 9 min; Detector: UV 254/210 nm; Retention time: 6.80 min. The fractions containing the desired product were collected and concentrated under reduced pressure to afford Compound 52 (2-[amino(azetidin-3-yl)methyl]-4,5-dichlorophenol trifluoroacetic acid) as a purple oil (44.7 mg, 28%): LCMS (ESI) calc'd for C₁₀H₁₂Cl₂N₂O [M + H]⁺: 247, 249 (3 : 2), found 247, 249 (3 : 2); ¹H NMR (300 MHz, CD₃OD) δ 7.52 (d, *J* = 1.6 Hz, 1H), 7.13 (s, 1H), 4.83 (d, *J* = 9.7 Hz, 1H), 4.31-4.13 (m, 2H), 4.03-3.91 (m, 1H), 3.90-3.69 (m, 2H); ¹⁹F NMR (282 MHz, CD₃OD) δ -77.11.

### Example 122. (which is a reference example) Compound 53 (4-[amino(4,5-dichloro-2-hydroxyphenyl)methyl]piperidine-1-carboxamide)

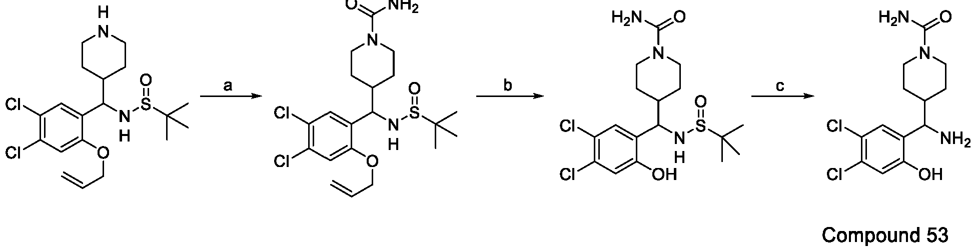

### Step a:

To a stirred solution of *N*-[[4,5-dichloro-2-(prop-2-en-1-yloxy)phenyl](piperidin-4-yl)methyl]-2-methylpropane-2-sulfinamide (0.20 g, 0.48 mmol) and Et₃N (72 mg, 0.72 mmol) in DCM (3 mL) was added isocyanatotrimethylsilane (66 mg, 0.57 mmol) dropwise at room temperature under nitrogen atmosphere. After stirring for 2 h at room temperature under nitrogen atmosphere, the resulting solution was quenched with water (30 mL) at room temperature and extracted with EA (3 x 50 mL). The combined organic layers were washed with brine (2 x 20 mL), dried over anhydrous Na₂SO₄. After filtration, the filtrate was concentrated under reduced pressure. The residue was purified by reverse phase chromatography, eluted with 45% ACN in water (plus 0.05% TFA) to afford 4-[[4,5-dichloro-2-(prop-2-en-1-yloxy)phenyl][(2-methylpropane-2-sulfinyl)amino]methyl]piperidine-1-carboxamide as a colorless oil (0.15 g, 68%): LCMS (ESI) calc'd for C₂₀H₂₉Cl₂N₃O₃S [M + H]⁺: 462, 464 (3 : 2), found 462, 464 (3 : 2).

### Step b:

To a stirred solution of 4-[[4,5-dichloro-2-(prop-2-en-1-yloxy)phenyl][(2-methylpropane-2-sulfinyl)amino]methyl]piperidine-1-carboxamide (0.15 g, 0.33 mmol) and Pd(PPh₃)₄ (8 mg, 0.01 mmol) in THF (5 mL) was added NaBH₄ (18 mg, 0.50 mmol) at room temperature under nitrogen atmosphere. The reaction mixture was stirred for 2 h at room temperature under nitrogen atmosphere. The resulting mixture was quenched with water (2 mL) at room temperature and concentrated under reduced pressure to afford 4-((4,5-dichloro-2-hydroxyphenyl)(1,1-dimethylethylsulfinamido)methyl)piperidine-1-carboxamide. The residue was used in the next step directly without further purification: LCMS (ESI) calc'd for C₁₇H₂₅Cl₂N₃O₃S [M + H]⁺: 422, 424 (3 : 2), found 422, 424 (3 : 2).

### Step c:

To a stirred mixture of 4-((4,5-dichloro-2-hydroxyphenyl)(1,1-dimethylethylsulfinamido)methyl)piperidine-1-carboxamide (crude) in 1,4-dioxane (3 mL) was added aq. HCl (6 *N,* 1 mL) at room temperature. After stirring for 2 h at room temperature, the resulting solution was neutralized to pH 7 with saturated aq. NaHCO₃ and concentrated under reduced pressure. The residue was purified by Prep-HPLC with the following conditions: Column: XBridge C18 OBD Prep Column 100 Å, 10 µm, 19 mm x 250 mm; Mobile Phase A: water with 20 mmol/L NH₄HCO₃, Mobile Phase B: ACN; Flow rate: 25 mL/min; Gradient: 5% B to 90% B in 8 min; Detector: UV 254/210 nm; Retention time: 6.75 min. The fractions containing desired product were collected and concentrated under reduced pressure to afford Compound 53 (4-[amino(4,5-dichloro-2-hydroxyphenyl)methyl]piperidine-1-carboxamide) as an off-white solid (69.6 mg, 71% overall two steps): LCMS (ESI) calc'd for C₁₃H₁₇Cl₂N₃O₂ [M + H]⁺: 318, 320 (3 : 2), found 318, 320 (3 : 2); ¹H NMR (300 MHz, CD₃OD) δ 7.20 (s, 1H), 6.86 (s, 1H), 4.11 (d, *J* = 13.5 Hz, 1H), 3.96 (d, *J* = 13.7 Hz, 1H), 3.88 (d, *J* = 7.6 Hz, 1H), 2.85-2.62 (m, 2H), 2.04-1.86 (m, 2H), 1.50-1.09 (m, 3H).

### Example 123. Compound 54 ((2R)-2-amino-1-(4-(amino(4,5-dichloro-2-hydroxyphenyl)methyl)piperidin-1-yl)propan-1-one trifluoroacetic acid)

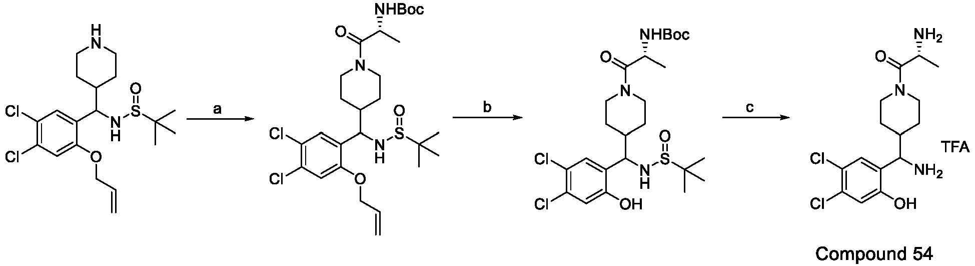

### Step a:

To a stirred solution of (*R*)-2-((tert-butoxycarbonyl)amino)propanoic acid (0.20 g, 1.06 mmol) and *N*-[[4,5-dichloro-2-(prop-2-en-1-yloxy)phenyl](piperidin-4-yl)methyl]-2-methylpropane-2-sulfinamide (0.44 g, 1.06 mmol) in DMF (5 mL) were added Et₃N (0.32 g, 3.18 mmol) and EDCI (0.61 g, 3.18 mmol) at room temperature under nitrogen atmosphere. The reaction solution was stirred for 2 h at room temperature under nitrogen atmosphere. The reaction solution was quenched with water (30 mL) at room temperature and extracted with EA (3 x 50 mL). The combined organic layers were washed with brine (3 x 30 mL), dried over anhydrous Na₂SO₄. After filtration, the filtrate was concentrated under reduced pressure. The residue was purified by silica gel column chromatography, eluted with PE/EA (9/1) to afford *tert*-butyl ((2*R*)-1-(4-((2-(allyloxy)-4,5-dichlorophenyl)(1,1-dimethylethylsulfinamido)methyl)piperidin-1-yl)-1-oxopropan-2-yl)carbamate as a yellow solid (0.10 g, 36%): LCMS (ESI) calc'd for C₂₇H₄₁Cl₂N₃O₅S [M + H]⁺: 590, 592 (3 : 2), found 590, 592 (3 : 2).

### Step b:

To a stirred mixture of *tert*-butyl ((2*R*)-1-(4-((2-(allyloxy)-4,5-dichlorophenyl)(1,1-dimethylethylsulfinamido)methyl)piperidin-1-yl)-1-oxopropan-2-yl)carbamate (0.10 g, 0.17 mmol) and Pd(PPh₃)₄ (4 mg, 0.002 mmol) in THF (3 mL) was added NaBH₄ (16 mg, 0.34 mmol) at room temperature under nitrogen atmosphere. The reaction mixture was stirred for 1 h at room temperature under nitrogen atmosphere. The resulting mixture was quenched with water (10 mL). The aqueous layer was extracted with EA (3 x 20 mL). The combined organic layers was washed with water (2 x 20 mL), dried over anhydrous Na₂SO₄ and filtered. The filtrate was concentrated under reduced pressure to afford crude (*tert-*butyl ((2*R*)-1-(4-((4,5-dichloro-2-hydroxyphenyl)(1,1-dimethylethylsulfinamido)methyl)piperidin-1-yl)-1-oxopropan-2-yl)carbamate. The crude product was used in the next step directly without further purification: LCMS (ESI) calc'd for C₂₄H₃₇Cl₂N₃O₅S [M + H]⁺: 550, 552 (3 : 2), found 550, 552 (3 : 2).

### Step c:

To a solution of *tert*-butyl ((2*R*)-1-(4-((4,5-dichloro-2-hydroxyphenyl)(1,1-dimethylethylsulfinamido)methyl)piperidin-1-yl)-1-oxopropan-2-yl)carbamate (crude) in 1,4-dioxane (3 mL) was added aq. HCl (6 *N,* 3 mL) at room temperature. The reaction solution was stirred for 1 h at room temperature. The reaction solution was concentrated under reduced pressure. The residue was dissolved in DCM (6 mL), and then TFA (3 mL) was added at room temperature. The reaction solution was stirred for additional 1 h at room temperature. The resulting solution was concentrated under reduced pressure. The residue was purified by Prep-HPLC with the following conditions: Column: XBridge C18 OBD Prep Column, 100 Å, 10 µm, 19 mm x 250 mm; Mobile Phase A: water (plus 0.05%TFA ), Mobile Phase B: ACN; Flow rate: 25 mL/min; Gradient: 5% B to 42% B in 6.5 min; Detector: UV 254/210 nm; Retention time: 5.80 min. The fractions containing desired product were collected concentrated under reduced pressure to afford Compound 54 ((2*R*)-2-amino-1-(4-(amino(4,5-dichloro-2-hydroxyphenyl)methyl)piperidin-1-yl)propan-1-one trifluoroacetic acid) as a yellow solid (34.5 mg, 48% overall two steps): LCMS (ESI) calc'd for C₁₅H₂₁Cl₂N₃O₂ [M + H]⁺: 346, 348 (3 : 2), found 346, 348 (3 : 2); ¹H NMR (300 MHz, CD₃OD) δ 7.44 (s, 1H), 7.09 (s, 1H), 4.56 (dd, *J* = 38.8 Hz, 12.8 Hz, 1H), 4.45-4.36 (m, 1H), 4.26-4.13 (m, 1H), 3.91 (dd, *J* = 38.8 Hz, 12.8 Hz, 1H), 3.25-3.01 (m, 1H), 2.83-2.57 (m, 1H), 2.50-2.29 (m, 1H), 2.17-1.99 (m, 1H), 1.41 (d, *J* = 12.8 Hz, 3H), 1.52-1.10 (m, 3H); ¹⁹F-NMR (282 MHz, CD₃OD) δ -76.92.

### Example 124. (which is a reference example) Compound 55 (2-[4-[amino(4,5-dichloro-2-hydroxyphenyl)methyl]piperidin-1-yl]-N,N-dimethylacetamide)

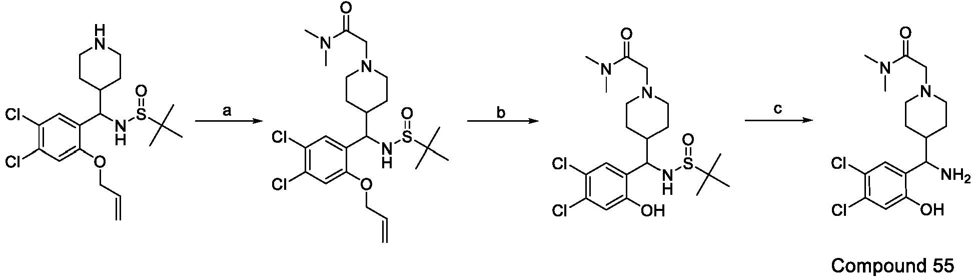

### Step a:

To a stirred solution of *N*-[[4,5-dichloro-2-(prop-2-en-1-yloxy)phenyl](piperidin-4-yl)methyl]-2-methylpropane-2-sulfinamide (0.10 g, 0.24 mmol) and DIEA (62 mg, 0.48 mmol) in DCM (3 mL) was added 2-bromo-*N,N*-dimethylacetamide (48 mg, 0.29 mmol) at room temperature under nitrogen atmosphere. The reaction solution was stirred for 2 h at room temperature under nitrogen atmosphere. The resulting solution was concentrated under reduced pressure. The residue was purified by reverse phase chromatography with 80% ACN in water with 10 mmol/L NH₄HCO₃ to afford 2-(4-[[4,5-dichloro-2-(prop-2-en-1-yloxy)phenyl][(2-methylpropane-2-sulfinyl)amino]methyl]piperidin-1-yl)-*N*,*N-*dimethylacetamide as a yellow oil (50 mg, 42%): LCMS (ESI) calc'd for C₂₃H₃₅Cl₂N₃O₃S [M + H]⁺: 504, 506 (3 : 2), found 504, 506 (3 : 2).

### Step b:

To a stirred solution of 2-(4-[[4,5-dichloro-2-(prop-2-en-1-yloxy)phenyl][(2-methylpropane-2-sulfinyl)amino]methyl]piperidin-1-yl)-*N,N*-dimethylacetamide (50 mg, 0.10 mmol) and Pd(PPh₃)₄ (3 mg, 0.002 mmol) in THF (4 mL) was added NaBH₄ (5 mg, 0.12 mmol) at room temperature under nitrogen atmosphere. The reaction mixture was stirred for 1 h at room temperature under nitrogen atmosphere. The resulting mixture was quenched with water (30 mL) at room temperature and extracted with EA (3 x 20 mL). The combined organic layers was washed with water (2 x 20 mL), dried over anhydrous Na₂SO₄ and filtered. The filtrate was concentrated under reduced pressure to afford crude 2-(4-((4,5-dichloro-2-hydroxyphenyl)(1,1-dimethylethylsulfinamido)methyl)piperidin-1-yl)-*N,N*-dimethylacetamide. The crude product was used in the next step directly without further purification: LCMS (ESI) calc'd for C₂₀H₃₁Cl₂N₃O₃S [M + H]⁺: 464, 466 (3 : 2), found 464, 466 (3 : 2).

### Step c:

To a stirred mixture of 2-(4-((4,5-dichloro-2-hydroxyphenyl)(1,1-dimethylethylsulfinamido)methyl)piperidin-1-yl)-*N,N*-dimethylacetamide (50 mg, 0.11 mmol) in 1,4-dioxane (3 mL) was added aq. HCl (6 *N,* 1 mL) at room temperature. The reaction solution was stirred for 2 h at room temperature. The resulting solution was concentrated under reduced pressure. The residue was purified by Prep-HPLC with the following conditions: Column: XBridge C18 OBD Prep Column 100 Å, 10 µm, 19 mm x 250 mm; Mobile Phase A: water with 20 mmol/L NH₄HCO₃, Mobile Phase B: ACN; Flow rate: 25 mL/min; Gradient: 5% B to 60% B in 6.5 min; Detector: UV 254/210 nm; Retention time: 5.38 min. The fractions containing desired product were collected and concentrated under reduced pressure to afford Compound 55 (2-[4-[amino(4,5-dichloro-2-hydroxyphenyl)methyl]piperidin-1-yl]-*N,N-*dimethylacetamide) as a colorless oil (18 mg, 46% overall two steps): LCMS (ESI) calc'd for C₁₆H₂₃Cl₂N₃O₂ [M + H]⁺: 360, 362 (3 : 2), found 360, 362 (3 : 2); ¹H NMR (300 MHz, CD₃OD) δ 7.18 (s, 1H), 6.84 (s, 1H), 3.88 (d, *J* = 7.8 Hz, 1H), 3.20 (s, 2H), 3.09 (s, 2H), 3.03 (d, *J* = 11.8 Hz, 1H), 2.94 (s, 3H), 2.94-2.85 (m, 2H), 2.16-1.96 (m, 2H), 1.94-1.71 (m, 2H), 1.48-1.32 (m, 3H).

### Example 125. Compound 56 (2-[amino[1-(2-hydroxy-2-methylpropyl)piperidin-4-yl]methyl]-4,5-dichlorophenol trifluoroacetic acid)

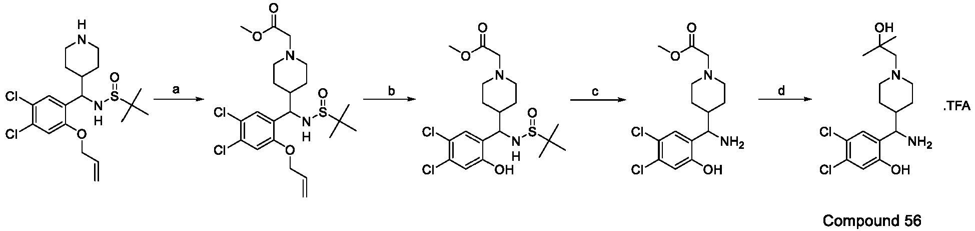

### Step a:

To a stirred mixture of *N*-[[4,5-dichloro-2-(prop-2-en-1-yloxy)phenyl](piperidin-4-yl)methyl]-2-methylpropane-2-sulfinamide (0.20 g, 0.48 mmol) and methyl 2-bromoacetate (0.11 g, 0.72 mmol) in ACN (5 mL) was added DIEA (0.12 g, 0.95 mmol) at room temperature under nitrogen atmosphere. After stirring for 16 h at room temperature under nitrogen atmosphere, the resulting solution was concentrated under reduced pressure. The residue was purified by reverse phase chromatography, eluted with 80% ACN in water with 10 mmol/L NH₄HCO₃ to afford methyl 2-(4-[[4,5-dichloro-2-(prop-2-en-1-yloxy)phenyl][(2-methylpropane-2-sulfinyl)amino]methyl]piperidin-1-yl)acetate as a yellow oil (0.18 g, 77%): LCMS (ESI) calc'd for C₂₂H₃₂Cl₂N₂O₄S [M + H]⁺: 491, 493 (3 : 2), found 491, 493 (3 : 2).

### Step b:

To a stirred mixture of methyl 2-(4-[[4,5-dichloro-2-(prop-2-en-1-yloxy)phenyl][(2-methylpropane-2-sulfinyl)amino]methyl]piperidin-1-yl)acetate (0.18 g, 0.37 mmol) and Pd(PPh₃)₄ (9 mg, 0.01 mmol) in THF (3 mL) were added NaBH₄ (17 mg, 0.44 mmol) at room temperature under nitrogen atmosphere. The reaction mixture was stirred for 1 h at room temperature. The resulting mixture was quenched with water (30 mL) at room temperature and extracted with EA (3 x 20 mL). The combined organic layers was washed with brine (2 x 10 mL), dried over anhydrous Na₂SO₄. After filtration, the filtrate was concentrated under reduced pressure to afford methyl 2-(4-((4,5-dichloro-2-hydroxyphenyl)(1,1-dimethylethylsulfinamido)methyl)piperidin-1-yl)acetate. The crude product was used in the next step directly without further purification: LCMS (ESI) calc'd for C₁₉H₂₈Cl₂N₂O₄S [M + H]⁺: 451, 452 (3 : 2), found 451, 452 (3 : 2).

### Step c:

To a stirred solution of methyl 2-(4-((4,5-dichloro-2-hydroxyphenyl)(1,1-dimethylethylsulfinamido)methyl)piperidin-1-yl)acetate (crude) in 1,4-dioxane (5 mL) was added aq. HCl (4 *N,* 1 mL) at room temperature. The reaction solution was stirred for 2 h at room temperature. The resulting solution was concentrated under reduced pressure. The residue was purified by reverse phase chromatography, eluted with 50% ACN in water with 10 mmol/L NH₄HCO₃ to afford methyl 2-[4-[amino(4,5-dichloro-2-hydroxyphenyl)methyl]piperidin-1-yl]acetate as a light yellow oil (0.10 g, 80% overall two steps): LCMS (ESI) calc'd for C₁₅H₂₀Cl₂N₂O₃ [M + H]⁺: 347, 349 (3 : 2), found 347, 349 (3 : 2).

### Step d:

To a stirred solution of methyl 2-[4-[amino(4,5-dichloro-2-hydroxyphenyl)methyl]piperidin-1-yl]acetate (80 mg, 0.23 mmol) in THF (3 mL) was added MeMgBr (0.27 g, 2.30 mmol) at room temperature under nitrogen atmosphere. The resulting mixture was stirred for 2 h at room temperature under nitrogen atmosphere. The resulting mixture was quenched with water (30 mL) at room temperature and concentrated under reduced pressure. The residue was purified by Prep-HPLC with the following conditions: Column: Sunfire Prep C₁₈ OBD Column, 10 µm, 19 x 250 mm; Mobile Phase A: water (plus 0.05%TFA), Mobile Phase B: ACN; Flow rate: 25 mL/min; Gradient: 5% B to 45% B in 6 min; Detector: UV 220/254 nm; Retention time: 5 min. The fractions containing desired product were collected concentrated under reduced pressure to afford Compound 56 (2-[amino[1-(2-hydroxy-2-methylpropyl)piperidin-4-yl]methyl]-4,5-dichlorophenol trifluoroacetic acid) as an off-white solid (15 mg, 19%): LCMS (ESI) calc'd for C₁₆H₂₄Cl₂N₂O₂ [M + H]⁺: 347, 349 (3 : 2), found 347, 349 (3 : 2); ¹H NMR (300 MHz, CD₃OD) δ 7.47 (s, 1H), 7.09 (s, 1H), 4.24 (d, *J =* 9.4 Hz, 1H), 3.89 (d, *J* = 12.9 Hz, 1H), 3.73 (d, *J* = 12.9 Hz, 1H), 3.29-2.96 (m, 4H), 2.55-2.30 (m, 1H), 2.26-2.09 (m, 1H), 1.95-1.48 (m, 3H), 1.35 (s, 6H).

### Example 126. (which is a reference example) Compound 57 (4-(amino(4,5-dichloro-2-hydroxyphenyl)methyl)-N-cyclopropylpiperidine-1-carboxamide)

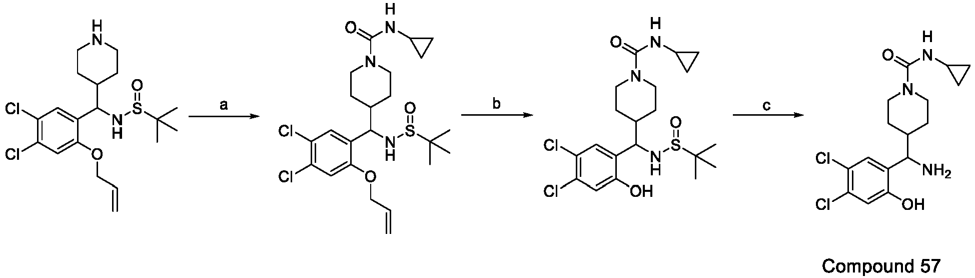

### Step a:

To a stirred solution of CDI (0.21 g, 1.29 mmol) and Et₃N (0.26 g, 2.58 mmol) in THF (5 mL) was added *N*-[[4,5-dichloro-2-(prop-2-en-1-yloxy)phenyl](piperidin-4-yl)methyl]-2-methylpropane-2-sulfinamide (0.18 g, 0.43 mmol) in portions at 0 °C. After stirring for 1 h at 0 °C, cyclopropanamine (73.5 mg, 1.29 mmol) was added to the resulting solution at 0 °C. The reaction solution was allowed to warm to room temperature and stirred for additional 1 h. The resulting solution was quenched with water (30 mL) at room temperature and extracted with EA (3 x 30 mL). The combined organic layers was washed with brine (2 x 10 mL), dried over anhydrous Na₂SO₄. After filtration, the filtrate was concentrated under reduced pressure. The residue was purified by reverse phase chromatography, eluted with 70% ACN in water with 10 mmol/L NH₄HCO₃ to afford N-cyclopropyl-4-[[4,5-dichloro-2-(prop-2-en-1-yloxy)phenyl] [(2-methylpropane-2-sulfinyl)amino]methyl]piperidine-1-carboxamide as a yellow oil (0.17 g, 70%): LCMS (ESI) calc'd for C₂₃H₃₃Cl₂N₃O₃S [M + H]⁺: 502, 504 (3 : 2), found 502, 504 (3 : 2).

### Step b:

To a stirred solution of N-cyclopropyl-4-[[4,5-dichloro-2-(prop-2-en-1-yloxy)phenyl][(2-methylpropane-2-sulfinyl)amino]methyl]piperidine-1-carboxamide (0.17 g, 0.34 mmol) and Pd(PPh₃)₄ (38 mg, 0.03 mmol) in THF (5 mL) was added NaBH₄ (19 mg, 0.65 mmol) in portions at room temperature under nitrogen atmosphere. The reaction mixture was stirred for 1 h at room temperature. The resulting mixture was quenched with water (30 mL) at room temperature and extracted with EA (3 x 20 mL). The combined organic layers w washed with brine (2 x 10 mL), dried over anhydrous Na₂SO₄. After filtration, the filtrate was concentrated under reduced pressure to afford *N*-cyclopropyl-4-((4,5-dichloro-2-hydroxyphenyl)(1,1-dimethylethylsulfinamido)methyl)piperidine-1-carboxamide. The crude product was used in the next step directly without further purification: LCMS (ESI) calc'd for C₂₀H₂₉Cl₂N₃O₃S [M + H]⁺: 462, 464 (3 : 2), found 462, 464 (3 : 2).

### Step c:

To a stirred solution of *N*-cyclopropyl-4-((4,5-dichloro-2-hydroxyphenyl)(1,1-dimethylethylsulfinamido)methyl)piperidine-1-carboxamide (crude) in 1,4-dioxane (4 mL) was added aq. HCl (6 *N,* 2 mL) at room temperature. The reaction solution was stirred for 2 h at room temperature. The resulting solution was concentrated under reduce pressure. The residue was purified by Prep-HPLC with the following conditions: Column: XBridge C18 OBD Prep Column, 100 Å, 10 µm, 19 mm x 250 mm; Mobile Phase A: water with 20 mmol/L NH₄HCO₃, Mobile Phase B: ACN; Flow rate: 25 mL/min; Gradient: 34% B to 46% B in 6.5 min; Detector: UV 254/210 nm; Retention time: 6.0 min. The fractions containing desired product were collected concentrated under reduced pressure to afford Compound 57 (4-(amino(4,5-dichloro-2-hydroxyphenyl)methyl)-*N*-cyclopropylpiperidine-1-carboxamide) as an off-white solid (25 mg, 31% overall two steps): LCMS (ESI) calc'd for C₁₆H₂₁Cl₂N₃O₂ [M + H]⁺: 358, 360 (3 : 2), found 358, 360 (3 : 2); ¹H NMR (400 MHz, CD₃OD) δ 7.21 (s, 1H), 6.86 (s, 1H), 4.09 (d, *J* = 13.5 Hz, 1H), 3.96 (d, *J* = 13.3 Hz, 1H), 3.88 (d, *J* = 7.7 Hz, 1H), 2.78-2.59 (m, 2H), 2.57-2.46 (m, 1H), 2.00-1.86 (m, 2H), 1.40 (d, *J* = 13.3 Hz, 1H), 1.29-1.08 (m, 2H), 0.70-0.62 (m, 2H), 0.52-0.40 (m, 2H).

### Example 127. (which is a reference example) Compound 58 (2-[amino([1-[(5-methyl-1,2-oxazol-3-yl)methyl]piperidin-4-yl])methyl]-4,5-dichlorophenol trifluoroacetic acid)

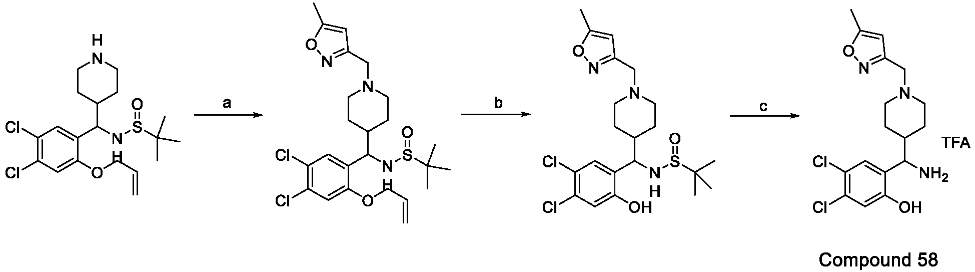

### Step a:

To a stirred solution of N-[[4,5-dichloro-2-(prop-2-en-1-yloxy)phenyl](piperidin-4-yl)methyl]-2-methylpropane-2-sulfinamide (0.20 g, 0.48 mmol) and DIEA (0.12 g, 0.95 mmol) in ACN (8 mL) was added 3-(bromomethyl)-5-methyl-1,2-oxazole (0.13 g, 0.72 mmol) at room temperature under nitrogen atmosphere. The reaction solution was stirred for 4 h at room temperature under nitrogen atmosphere. The resulting solution was concentrated under reduced pressure. The residue was purified by reverse phase chromatography, eluted with 65% ACN in water (plus 0.05% TFA) to afford *N*-[[4,5-dichloro-2-(prop-2-en-1-yloxy)phenyl]([1-[(5-methyl-1,2-oxazol-3-yl)methyl]piperidin-4-yl])methyl]-2-methylpropane-2-sulfinamide as a light yellow oil (90 mg, 37%): LCMS (ESI) calc'd for C₂₄H₃₃Cl₂N₃O₃S [M + H]⁺: 514, 516 (3 : 2), found 514, 516 (3 : 2).

### Step b:

To a stirred solution of *N*-[[4,5-dichloro-2-(prop-2-en-1-yloxy)phenyl]([1-[(5-methyl-1,2-oxazol-3-yl)methyl]piperidin-4-yl])methyl]-2-methylpropane-2-sulfinamide (90 mg, 0.17 mmol) and Pd(PPh₃)₄ (4 mg, 0.003 mmol) in THF (3 mL) was added NaBH₄ (7.9 mg, 0.21 mmol, 1.2 equiv) at room temperature under nitrogen atmosphere. The reaction mixture was stirred for 1 h at room temperature under nitrogen atmosphere. The resulting mixture was quenched with water (30 mL) at room temperature and extracted with EA (3 x 20 mL). The combined organic layers were washed with water (2 x 20 mL), dried over anhydrous Na₂SO₄ and filtered. The filtrate was concentrated under reduced pressure to afford crude *N*-[(4,5-dichloro-2-hydroxyphenyl)([1-[(5-methyl-1,2-oxazol-3-yl)methyl]piperidin-4-yl])methyl]-2-methylpropane-2-sulfinamide. The crude product was used in the next step directly without further purification: LCMS (ESI) calc'd for C₂₁H₂₉Cl₂N₃O₃S [M + H]⁺: 474, 476 (3 : 2), found 474, 476 (3 : 2).

### Step c:

To a stirred solution of *N*-[(4,5-dichloro-2-hydroxyphenyl)([1-[(5-methyl-1,2-oxazol-3-yl)methyl]piperidin-4-yl])methyl]-2-methylpropane-2-sulfinamide (crude) in 1,4-dioxane (3 mL) was added aq. HCl (4 *N,* 1 mL) at room temperature. The reaction solution was stirred for 2 h at room temperature. The resulting solution was concentrated under reduced pressure. The residue was purified by Prep-HPLC with the following conditions: Column: XBridge C18 OBD Prep Column 100 Å, 10 µm, 19 mm x 250 mm; Mobile Phase A: water with 10 mmol/L NH₄HCO₃, Mobile Phase B: ACN; Flow rate: 25 mL/min; Gradient: 10% B to 50% B in 6.5 min; Detector: UV 254/210 nm; Retention time: 5.88 min. The fractions containing desired product were collected and concentrated under reduced pressure to afford Compound 58 (2-[amino([1-[(5-methyl-1,2-oxazol-3-yl)methyl]piperidin-4-yl])methyl]-4,5-dichlorophenol trifluoroacetic acid) as a brown solid (9.8 mg, 20% overall two steps): LCMS (ESI) calc'd for C₁₇H₂₁Cl₂N₃O₂ [M + H]⁺: 370, 372 (3 : 2), found 370, 372 (3 : 2); ¹H NMR (300 MHz, CD₃OD) δ 7.46 (s, 1H), 7.10 (s, 1H), 6.32 (s, 1H), 4.36 (s, 2H), 4.26 (d, *J* = 9.6 Hz, 1H), 3.70 (d, *J* = 12.5 Hz, 1H), 3.55 (d, *J* = 12.5 Hz, 1H), 3.23-2.98 (m, 2H), 2.54-2.38 (m, 1H), 2.47 (s, 3H), 2.33-2.19 (m, 1H), 1.86-1.51 (m, 3H); ¹⁹F NMR (282 MHz, CD₃OD) δ -76.98.

### Example 128. (which is a reference example) Compound 59 (2-[amino(4-fluoropiperidin-4-yl)methyl]-4,5-dichlorophenol trifluoroacetic acid)

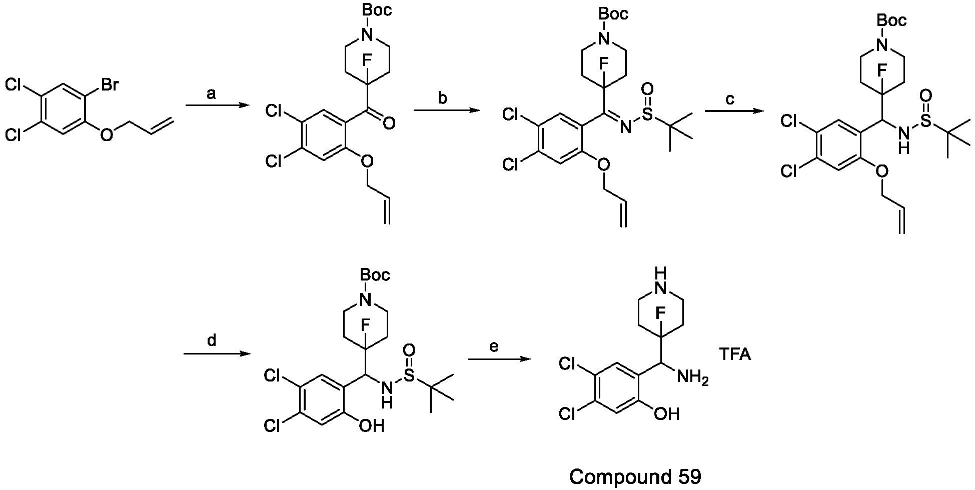

### Step a:

To a stirred solution of 1-bromo-4,5-dichloro-2-(prop-2-en-1-yloxy)benzene (Example 6, step b) (1.50 g, 5.17 mmol) in THF (15 mL) was added i-PrMgCl (3.44 mL, 6.88 mmol, 2 M in THF) dropwise at 0 °C under nitrogen atmosphere. The reaction was stirred at 0 °C for 30 min. Then tert-butyl 4-fluoro-4-[methoxy(methyl)carbamoyl]piperidine-1-carboxylate (1.00 g, 3.44 mmol) was added into the reaction. The reaction was stirred at 0 °C for 1.5 h. The reaction was quenched with saturated aq. NH₄Cl (5 mL) and diluted with EA (50 mL) and water (30 mL). The aqueous layer was extracted with EA (3 x 30 mL). The combined organic layers were washed with brine (2 x 30 mL) and dried over anhydrous Na₂SO₄. After filtration, the filtrate was concentrated under reduced pressure. The residue was purified by silica gel column chromatography, eluted with PE/EA (3/1) to afford *tert*-butyl 4-[[4,5-dichloro-2-(prop-2-en-1-yloxy)phenyl]carbonyl]-4-fluoropiperidine-1-carboxylate as a light yellow oil (0.50 g, 27%): LCMS (ESI) calc'd for C₂₀H₂₄Cl₂FNO₄ [M + H - 56]+: 376, 378 (3 : 2), found 376, 378 (3 : 2); ¹H NMR (400 MHz, CDCl3) δ 7.32 (s, 1H), 7.06 (s, 1H), 6.07-5.94 (m, 1H), 5.46-5.32 (m, 2H), 4.59 (dt, *J* = 5.6, 1.4 Hz, 2H), 4.10 (d, *J* = 13.8 Hz, 2H), 3.12-3.01 (m, 2H), 2.15-1.97 (m, 4H), 1.50 (s, 9H).

### Step b:

To a stirred solution of *tert*-butyl 4-[4,5-dichloro-2-(prop-2-en-1-yloxy)benzoyl]-4-fluoropiperidine-1-carboxylate (0.50 g, 1.16 mmol) and tert-butanesulfinamide (0.21 g, 1.73 mmol) in THF (8 mL) was added Ti(OEt)₄ (0.79 g, 3.47 mmol) at room temperature under nitrogen atmosphere. The reaction was stirred at 70 °C for 16 h. The reaction was quenched with saturate aq. Na₂CO₃ (30 mL). The resulting mixture was filtered through celite and the filtrate was diluted with EA (30 mL) and water (30 mL) and the aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by Prep-TLC, eluted with PE/EA (5/1) to afford tert-butyl 4-[[4,5-dichloro-2-(prop-2-en-1-yloxy)phenyl][(2-methylpropane-2-sulfinyl)imino]methyl]-4-fluoropiperidine-1-carboxylate as a yellow oil (50 mg, 7%): LCMS (ESI) calc'd for C₂₄H₃₃Cl₂FN₂O₄S [M + Na]⁺: 557, 559 (3 : 2), found 557, 559 (3 : 2);

### Step c:

To a stirred solution of *tert*-butyl 4-[[4,5-dichloro-2-(prop-2-en-1-yloxy)phenyl][(2-methylpropane-2-sulfinyl)imino]methyl]-4-fluoropiperidine-1-carboxylate (50 mg, 0.09 mmol) in THF (1 mL) were added NaBH₄ (7 mg, 0.19 mmol) at room temperature. The reaction was quenched with saturated aq. NH₄Cl (1 mL) and diluted with EA (30 mL) and water (30 mL). The aqueous layer was extracted with EA (3 x 30 mL). The combined organic layers were washed with brine (2 x 30 mL) and dried over anhydrous Na₂SO₄. After filtration, the filtrate was concentrated under reduced pressure to afford *tert*-butyl 4-[[4,5-dichloro-2-(prop-2-en-1-yloxy)phenyl][(2-methylpropane-2-sulfinyl)amino]methyl]-4-fluoropiperidine-1-carboxylate as a light yellow solid (50 mg, crude), which was used in the next step directly without further purification: LCMS (ESI) calc'd for C₂₂H₃₂Cl₂N₂O₄S [M + Na]⁺: 559, 561 (3 : 2), found 559, 561 (3 : 2).

### Step d:

To a stirred solution of *tert*-butyl 4-[[4,5-dichloro-2-(prop-2-en-1-yloxy)phenyl][(2-methylpropane-2-sulfinyl)amino]methyl]-4-fluoropiperidine-1-carboxylate (50 mg, 0.09 mmol) and Pd(PPh₃)₄ (5 mg, 0.05 mmol) in THF (1 mL) was added NaBH₄ (7 mg, 0.19 mmol) at room temperature. The reaction was stirred at room temperature for 1 h. The reaction was quenched with saturated aq. NH₄Cl (20 mL). The resulting mixture was extracted with EA (3 x 20 mL). The combined organic layers were washed with brine (2 x 20 mL), dried over anhydrous Na₂SO₄. After filtration, the filtrate was concentrated under reduced pressure. The residue was purified by Prep-TLC, eluted with PE/EA (1/1) to afford tert-butyl 4-[(4,5-dichloro-2-hydroxyphenyl)[(2-methylpropane-2-sulfinyl)amino]methyl]-4-fluoropiperidine-1-carboxylate a brown yellow oil (40 mg, 86% overall two steps): LCMS (ESI) calc'd for C₂₁H₃₁Cl₂FN₂O₄S [M + Na]⁺ 519, 521 (3 : 2), found 519, 521 (3 : 2); ¹H NMR (300 MHz, CD₃OD) δ 7.51 (s, 1H), 6.97 (s, 1H), 4.85-4.76 (m, 1H), 4.08-3.79 (m, 2H), 3.19-2.95 (m, 2H), 2.35-2.16 (m, 2H), 1.81-1.53 (m, 2H), 1.46 (s, 9H), 1.15 (s, 9H).

### Step e:

To a stirred solution of *tert*-butyl 4-[(4,5-dichloro-2-hydroxyphenyl)[(2-methylpropane-2-sulfinyl)amino]methyl]-4-fluoropiperidine-1-carboxylate (40 mg, 0.10 mmol) in 1,4-dioxane (1 mL) was added aq. HCl (4 N, 1 mL) at room temperature. The resulting mixture was stirred for 1 h at room temperature. The resulting mixture was concentrated under reduced pressure. Then the above mixture was dissolved in DCM (2 mL) was added TFA (0.5 mL) at room temperature. The resulting solution was stirred for 1 h at room temperature. The resulting solution was concentrated under reduced pressure. The residue was purified by Prep-HPLC with the following conditions: Column: XBridge C18 OBD Prep Column 100 Å, 10 µm, 19 mm x 250 mm; Mobile Phase A: water (plus 0.05% TFA), Mobile Phase B: ACN; Flow rate: 25 mL/min; Gradient: 15% B to 70% B in 7 min; Detector: UV 254/210 nm; Retention time: 5.90 min. The fractions containing the desired product were collected and concentrated under reduced pressure to afford Compound 59 (2-[amino(4-fluoropiperidin-4-yl)methyl]-4,5-dichlorophenol trifluoroacetic acid) as an off-white solid (8.4 mg, 34%): LCMS (ESI) calc'd for C₁₂H₁₅Cl₂FN₂O [M + H]⁺: 293, 295 (3 : 2), found 293, 295 (3 : 2); ¹H NMR (300 MHz, CD₃OD) δ 7.39 (s, 1H), 6.91 (s, 1H), 4.34 (d, *J* = 17.4 Hz, 1H), 3.05-2.74 (m, 4H), 2.17-2.02 (m, 1H), 1.83-1.47 (m, 3H); ¹⁹F NMR (282 MHz, CD₃OD) δ -76.94, -172.46.

### Example 129. (which is a reference example) Compound 60 ((S)-2-[amino(piperidin-4-yl)methyl]-3,4,5-trichlorophenol trifluoroacetic acid)) and Compound 61 ((R)-2-[amino(piperidin-4-yl)methyl]-3,4,5-trichlorophenol trifluoroacetic acid)

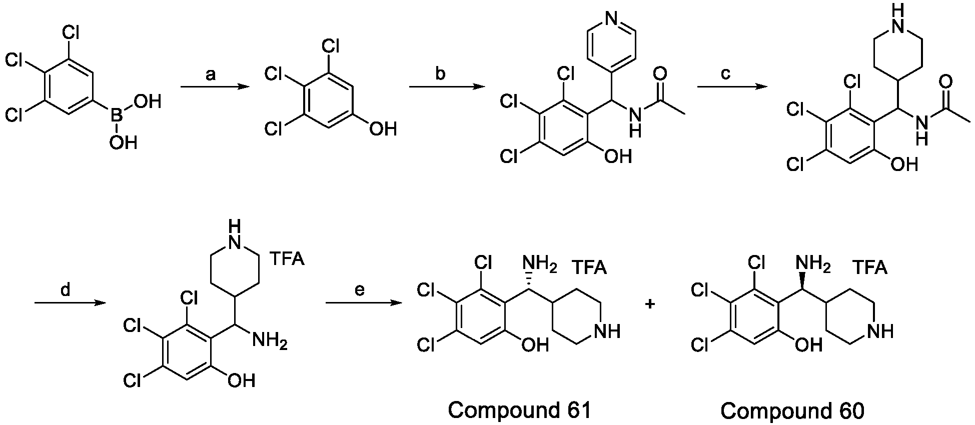

### Step a:

To a stirred solution of (3,4,5-trichlorophenyl)boronic acid (6.00 g, 26.64 mol) and H₂O₂ (1.81 g, 53.27 mmol, 30%) in THF (10 mL) were added NaOH (2.13 g, 53.27 mmol) at room temperature under nitrogen atmosphere. The resulting mixture was stirred for 12 h at room temperature under nitrogen atmosphere. The reaction was quenched with saturated aq. Na₂SO₃ (100 mL) and acidified with aq. HCl (1 *N*) to pH 3-4. The resulting mixture was extracted with EA (3 x 100 mL). The combined organic layers were washed with brine (3 x 100 mL), dried over anhydrous Na₂SO₄. After filtration, the filtrate was concentrated under reduced pressure. The residue was purified by silica gel column chromatography, eluted with PE/EA (9/1) to afford 3,4,5-trichlorophenol as a light yellow solid (3.10 g, 88%). LCMS (ESI) calc'd for C₆H₂Cl₃O [M - H]⁻: 195, 197, 199 (3 : 3 : 1), found 195, 197, 199 (3 : 3 : 1); ¹H NMR (300 MHz, DMSO-*d*₆) δ 10.55 (s, 1H), 6.99 (s, 1H).

### Step b:

To a stirred mixture of 3,4,5-trichlorophenol (0.59 g, 2.99 mmol), acetamide (0.21 g, 3.59 mmol) and pyridine-4-carbaldehyde (0.32 g, 2.99 mmol) was added AlCl₃ (59.8 mg, 0.45 mmol) dropwise at 110 °C under nitrogen atmosphere. The reaction mixture was stirred for 10 h at 110 °C. After cooling to room temperature, the reaction mixture was quenched with water (200 mL) at room temperature. The resulting mixture was extracted with EA (3 x 80 mL). The combined organic layers were washed with brine (3 x 10 mL), dried over anhydrous Na₂SO₄. After filtration, the filtrate was concentrated under reduced pressure. The residue was purified by reverse phase chromatography, eluted with 50% ACN in water (plus 0.05% TFA) to afford N-[pyridin-4-yl(2,3,4-trichloro-6-hydroxyphenyl)methyl]acetamide as a light yellow oil (0.14 g, 12%). LCMS (ESI) calc'd for C₁₄H₁₁Cl₃N₂O₂ [M + H]⁺: 345, 347 (1 : 1), found 345, 347 (1 : 1); ¹H NMR (300 MHz, CD₃OD) δ 8.81-8.67 (m, 2H), 7.97-7.86 (m, 2H), 7.13 (s, 1H), 7.05 (s, 1H), 2.17 (s, 3H).

### Step c:

To a solution of*N*-[pyridin-4-yl(2,3,4-trichloro-6-hydroxyphenyl)methyl]acetamide (0.10 g, 0.29 mmol) in co-solvent MeOH/AcOH (20 mL, v/v=1:1) was added PtO₂ (20 mg) in a pressure tank. The mixture was hydrogenated at 30 °C under hydrogen atmosphere (50 atm) for 6 h. The resulting mixture filtered through a celite and the filtrate was concentrated under reduced pressure to afford *N*-[piperidin-4-yl(2,3,4-trichloro-6-hydroxyphenyl)methyl]acetamide as a colorless oil (0.10 g, crude). LCMS (ESI) calc'd for C₁₄H₁₇Cl₃N₂O₂ [M + H]+: 351, 353, 355 (3 : 3 : 1), found 351, 353, 355 (3 : 3 : 1); ¹H NMR (300 MHz, CD₃OD) δ 6.97 (s, 1H), 5.51 (d, *J* = 10.5 Hz, 1H), 3.45 (d, *J* = 12.7 Hz, 1H), 3.40-3.34 (m, 1H), 3.07-2.82 (m, 2H), 2.30-2.13 (m, 1H), 1.98 (s, 3H), 1.58-1.46 (m, 1H), 1.35-1.25 (m, 3H).

### Step d:

To a stirred solution of *N-*[pyridin-4-yl(2,3,4-trichloro-6-hydroxyphenyl)methyl]acetamide (0.14 g, 0.4 mmol) in conc. HCl (3 mL) at 100 °C for 3 h. After cooling to room temperature, the resulting mixture was concentrated under reduced pressure. The residue was purified by Prep-HPLC with the following conditions: Column: XBridge C18 OBD Prep Column 100 Å, 10 µm, 19 mm x 250 mm; Mobile Phase A: water (plus 0.05% TFA), Mobile Phase B: ACN; Flow rate: 20 mL/min; Gradient: 5% B to 40% B in 8 min; Detector: UV 254/210 nm; Retention time: 7.18 min. The fractions containing the desired product were collected and concentrated under reduced pressure to afford 2-[amino(piperidin-4-yl)methyl]-3,4,5-trichlorophenol trifluoroacetic acid as a light yellow solid (70 mg, 41%). LCMS (ESI) calc'd for C₁₂H₁₅Cl₃N₂O [M + H]⁺: 309, 311, 313 (3 : 3 : 1), found 309, 311, 313 (3 : 3 : 1). ¹H NMR (400 MHz, CD₃OD) δ 7.15 (s, 1H), 4.69 (d, *J* = 10.1 Hz, 1H), 3.55 (d, *J* = 12.9 Hz, 1H), 3.38 (d, *J* = 12.9 Hz, 1H), 3.05 (td, *J* = 13.1, 3.1 Hz, 1H), 2.94 (td, *J* = 12.4, 5.3 Hz, 1H), 2.73-2.66 (m, 1H), 2.26 (d, *J* = 13.9 Hz, 1H), 1.80-1.49 (m, 3H); ¹⁹F NMR (376 MHz, CD₃OD) δ -77.06.

### Step e:

2-[Amino(piperidin-4-yl)methyl]-3,4,5-trichlorophenol trifluoroacetic acid (68 mg, 0.16 mmol) was separated by Prep-SFC with the following conditions: Column: CHIRALPAK IG UL001, 20 x 250 mm, 5 µm; Mobile Phase A: CO₂, Mobile Phase B: MeOH (0.5% IPA); Flow rate: 40 mL/min; Gradient: 35% B; Detector: UV 220 nm; Retention time: RT₁: 2.56 min; RT₂: 5.4 min; Temperature: 25 °C.

The faster-eluting enantiomer was obtained as Compound 61 ((R)-2-[amino(piperidin-4-yl)methyl]-3,4,5-trichlorophenol trifluoroacetic acid) at 2.56 min as a light yellow solid (5.1 mg, 8%): LCMS (ESI) calc'd for C₁₂H₁₅Cₗ₃N₂O [M + H]⁺: 309, 311, 313 (3 : 3 : 1), found 309, 311, 313 (3 : 3 : 1); ¹H NMR (400 MHz, DMSO-*d*₆) δ 7.17 (s, 1H), 4.40 (d, *J* = 10.0 Hz, 1H), 3.38 (d, *J* = 12.9 Hz, 1H), 3.21 (d, *J* = 12.8 Hz, 1H), 2.95-2.70 (m, 2H), 2.48-2.39 (m, 1H), 2.10 (d, *J* = 13.9 Hz, 1H), 1.57-1.45 (m, 1H), 1.39-1.25 (m, 2H); ¹⁹F NMR (376 MHz, CD₃OD) δ -73.50.

The slower-eluting enantiomer was obtained as Compound 60 ((S)-2-[amino(piperidin-4-yl)methyl]-3,4,5-trichlorophenol trifluoroacetic acid) at 5.4 min as a light yellow solid (6.5 mg, 9%): LCMS (ESI) calc'd for C₁₂H₁₅Cl₃N₂O [M + H]⁺: 309, 311, 313 (3 : 3 : 1), found 309, 311, 313 (3 : 3 : 1); ¹H NMR (400 MHz, DMSO-*d*₆) δ 7.19 (s, 1H), 4.40 (d, *J* = 10.1 Hz, 1H), 3.39 (d, *J* = 12.7 Hz, 1H), 3.21 (d, *J* = 12.9 Hz, 1H), 2.93-2.73 (m, 2H), 2.48-2.39 (m, 1H), 2.16-2.02 (m, 1H), 1.57-1.42 (m, 1H), 1.42-1.28 (m, 2H); ¹⁹F NMR (376 MHz, CD₃OD) δ -73.51.

### Example 130. (which is a reference example) Compound 62 (2-(amino(1-(2,2-difluoroethyl)piperidin-4-yl)methyl)-4,5-dichlorophenol trifluoroacetic acid)

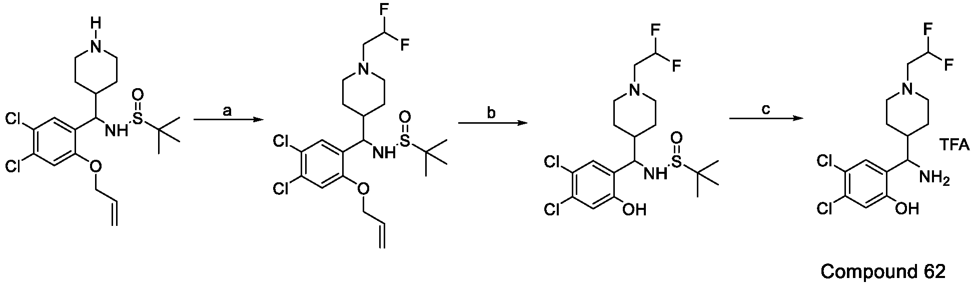

### Step a:

To a stirred solution of *N*-[[4,5-dichloro-2-(prop-2-en-1-yloxy)phenyl](piperidin-4-yl)methyl]-2-methylpropane-2-sulfinamide (0.20 g, 0.48 mmol) and 1,1-difluoro-2-iodoethane (0.27 g, 1.43 mmol) in MeCN (3 mL) was added K₂CO₃ (0.13 g, 0.95 mmol) at room temperature under nitrogen atmosphere. The reaction mixture was allowed to warm to 50 °C and stirred for 2 h under nitrogen atmosphere. After cooling to room temperature, the reaction mixture was diluted with water (30 mL) and extracted with EA (3 x 50 mL). The combined organic layers were washed with brine (3 x 20 mL), dried over anhydrous Na₂SO₄. After the filtration, the filtrate was concentrated under reduced pressure. The residue was purified by reverse phase chromatography, eluted with 70% ACN in water with 10 mmol/L NH₄HCO₃ to afford N-((2-(allyloxy)-4,5-dichlorophenyl)(1-(2,2-difluoroethyl)piperidin-4-yl)methyl)-2-methylpropane-2-sulfinamide as a light yellow solid (0.12 g, 52%): LCMS (ESI) calc'd for C₂₁H₃₀Cl₂F₂N₂O₂S [M + H]⁺: 483, 485 (3 : 2), found 483, 485 (3 : 2).

### Step b:

To a stirred solution of *N*-((2-(allyloxy)-4,5-dichlorophenyl)(1-(2,2-difluoroethyl)piperidin-4-yl)methyl)-2-methylpropane-2-sulfinamide (0.12 g, 0.25 mmol) and Pd(PPh₃)₄ (6 mg, 0.01 mmol) in THF (10 mL) were added NaBH₄ (14 mg, 0.38 mmol) in portions at room temperature under nitrogen atmosphere. The reaction mixture was stirred for 2 h at room temperature under nitrogen atmosphere. The reaction mixture was quenched with water (30 mL) at room temperature. The resulting mixture was extracted with EA (3 x 50 mL). The combined organic layers were washed with brine (3 x 10 mL), dried over anhydrous Na₂SO₄. After the filtration, the filtrate was concentrated under reduced pressure. The residue was purified by Prep-TLC, eluted with PE/EA (4/1) to afford *N*-((4,5-dichloro-2-hydroxyphenyl)(1-(2,2-difluoroethyl)piperidin-4-yl)methyl)-2-methylpropane-2-sulfinamide as a yellow solid (80 mg, 73%): LCMS (ESI) calc'd for C₁₈H₂₆Cl₂F₂N₂O₂S [M + H]⁺: 443, 445 (3 : 2), found 443, 445 (3 : 2);

### Step c:

To a stirred solution of *N*-((4,5-dichloro-2-hydroxyphenyl)(1-(2,2-difluoroethyl)piperidin-4-yl)methyl)-2-methylpropane-2-sulfinamide (50 mg, 0.11 mmol) in DCM (2 mL) were added TFA (1 mL) at room temperature. The reaction solution was stirred for 3 h at room temperature. The reaction solution was concentrated under reduced pressure. The residue was purified by Prep-HPLC with the following conditions: Column: XBridge Shield RP18 OBD Column, 5 µm, 19 x 150 mm; Mobile Phase A: water (plus 0.5% TFA), Mobile Phase B: ACN; Flow rate: 25 mL/min; Gradient: 10% B to 35% B in 7 min; Detector: UV 254/220 nm; Retention time: 6.5 min. The fractions containing desired product were collected and concentrated under reduced pressure to afford Compound 62 (2-(amino(1-(2,2-difluoroethyl)piperidin-4-yl)methyl)-4,5-dichlorophenol trifluoroacetic acid) as a light yellow solid (10 mg, 23%): LCMS (ESI) calc'd for C₁₄H₁₈Cl₂F₂N₂O [M + H]⁺: 339, 341 (3 : 2), found 339, 341 (3 : 2); ¹H NMR (300 MHz, CD₃OD) δ 7.46 (s, 1H), 7.10 (s, 1H), 6.30 (t, *J* = 54.0 Hz; 1 H), 4.25 (d, *J* = 9.6 Hz; 1 H), 3.70-3.33 (m, 4H), 3.12-2.80 (m, 2H), 2.51-2.30 (m, 1H), 2.22-2.10 (m, 1H), 1.80-1.62 (m, 1H), 1.59-1.46 (m, 2H); ¹⁹F NMR (282 MHz, CD₃OD) δ -77.04, - 121.72.

### Example 131. Compound 63 ((4-(amino(4,5-dichloro-2-hydroxyphenyl)methyl)piperidin-1-yl)((S)-pyrrolidin-3-yl)methanone trifluoroacetic acid)

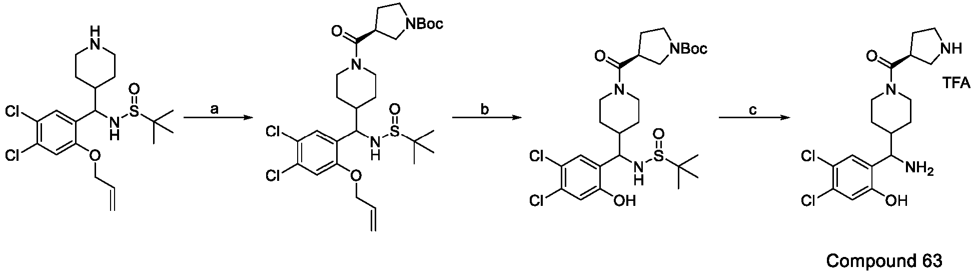

### Step a:

To a stirred solution of *N*-((2-(allyloxy)-4,5-dichlorophenyl)(piperidin-4-yl)methyl)-2-methylpropane-2-sulfinamide (0.20 g, 0.48 mmol) and (*S*)-1-(*tert*-butoxycarbonyl)pyrrolidine-3-carboxylic acid (0.15 g, 0.72 mmol) in DMF (3 mL) were added Et₃N (0.15 g, 1.44 mmol) and EDCI (0.18 g, 0.96 mmol) at room temperature under nitrogen atmosphere. The reaction solution was stirred for 1 h at room temperature under nitrogen atmosphere. The resulting solution was quenched with water (40 mL) at room temperature and extracted with EA (3 x 50 mL). The combined organic layers were washed with brine (2 x 10 mL), dried over anhydrous Na₂SO₄. After filtration, the filtrate was concentrated under reduced pressure. The residue was purified by Prep-TLC, eluted with PE/EA (1/1) to afford (3*S*)-*tert*-butyl 3-(4-((2-(allyloxy)-4,5-dichlorophenyl)(1,1-dimethylethylsulfinamido)methyl)piperidine-1-carbonyl)pyrrolidine-1-carboxylate as a light yellow solid (0.18 g, 60%): LCMS (ESI) calc'd for C₂₉H₄₃Cl₂N₃O₅S [M + H]⁺: 616, 618 (3 : 2), found 616, 618 (3 : 2).

### Step b:

To a stirred solution of (3*S*)-*tert*-butyl 3-(4-((2-(allyloxy)-4,5-dichlorophenyl)(1,1-dimethylethylsulfinamido)methyl)piperidine-1-carbonyl)pyrrolidine-1-carboxylate (0.18 g, 0.29 mmol) and Pd(PPh₃)₄ (32 mg, 0.029 mmol) in THF (5 mL) was added NaBH₄ (16 mg, 0.44 mmol) at room temperature under nitrogen atmosphere. The reaction mixture was stirred for 1 h at room temperature under nitrogen atmosphere. The resulting mixture was quenched with water (40 mL) at room temperature and extracted with EA (3 x 50 mL). The combined organic layers were washed with brine (3 x 30 mL), dried over anhydrous Na₂SO₄. After filtration, the filtrate was concentrated under reduced pressure to afford crude (3*S*)-*tert*-butyl 3-(4-((4,5-dichloro-2-hydroxyphenyl)(1,1-dimethylethylsulfinamido)methyl)piperidine-1-carbonyl)pyrrolidine-1-carboxylate. The crude product was used in the next step directly without further purification: LCMS (ESI) calc'd for C₂₆H₃₉Cl₂N₃O₅S [M + H]⁺: 576, 578 (3 : 2), found 576, 578 (3 : 2).

### Step c:

To a stirred solution of (3*S*)-*tert*-butyl 3-(4-((4,5-dichloro-2-hydroxyphenyl)(1,1-dimethylethylsulfinamido)methyl)piperidine-1-carbonyl)pyrrolidine-1-carboxylate (crude) in 1,4-dioxane (5 mL) was added aq. HCl (6 *N,* 3 mL) at room temperature. The reaction solution was stirred for 1 h at room temperature. The reaction solution was concentrated under reduced pressure. The residue was dissolved in DCM (5 mL), and then TFA (3 mL) was added at room temperature. The reaction solution was stirred for 1 h at room temperature. The resulting solution was concentrated under reduced pressure. The residue was purified by Prep-HPLC with the following conditions: Column: Column: XBridge C18 OBD Prep Column, 100 Å, 10 µm, 19 mm x 250 mm; Mobile Phase A: water (plus 0.05% TFA), Mobile Phase B: ACN; Flow rate: 25 mL/min; Gradient: 5% B to 45% B in 7 min; Detector: UV 254/210 nm; Retention time: 5.52. The fractions containing desired product were collected and concentrated under reduce pressure to afford Compound 63 ((4-(amino(4,5-dichloro-2-hydroxyphenyl)methyl)piperidin-1-yl)((S)-pyrrolidin-3-yl)methanone trifluoroacetic acid) as an off-white solid (56.2 mg, 40% overall two steps): LCMS (ESI) calc'd for C₁₇H₂₃Cl₂N₃O₂ [M + H]⁺: 372, 374 (3 : 2), found 372, 374 (3 : 2); ¹H NMR (300 MHz, CD₃OD) δ 7.44 (d, *J* = 3.6 Hz, 1H), 7.09 (d, *J* = 2.2 Hz, 1H), 4.58 (dd, *J* = 41.8, 13.3 Hz, 1H), 4.26-3.96 (m, 2H), 3.74-3.54 (m, 2H), 3.41-3.30 (m, 3H), 3.26-3.00 (m, 1H), 2.80-2.54 (m, 1H), 2.49-2.31 (m, 2H), 2.21-2.02 (m, 2H), 1.59-1.08 (m, 3H); ¹⁹F NMR (282 MHz, CD₃OD) δ -76.92.

### Example 132. Compound 64 (2-(amino(1-(2-methoxyethyl)piperidin-4-yl)methyl)-4,5-dichlorophenol)

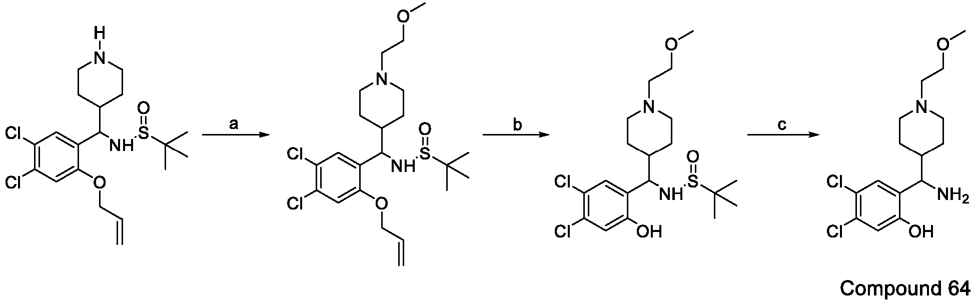

### Step a:

To a stirred solution of *N*-[[4,5-dichloro-2-(prop-2-en-1-yloxy)phenyl](piperidin-4-yl)methyl]-2-methylpropane-2-sulfinamide (0.18 g, 0.43 mmol) and DIEA (0.11 g, 0.86 mmol) in ACN (3 mL) was added 1-bromo-2-methoxyethane (0.18 g, 1.29 mmol) in portions at room temperature. The reaction solution was stirred for 1 h at room temperature. The resulting solution was diluted with water (30 mL) and extracted with EA (3 x 30 mL). The combined organic layers were washed with brine (2 x 20 mL), dried over anhydrous Na₂SO₄. After filtration, the filtrate was concentrated under reduced pressure. The residue was purified by reverse phase chromatography, eluted with 50% ACN in water with 10 mmol/L NH₄HCO₃ to afford N-[[4,5-dichloro-2-(prop-2-en-1-yloxy)phenyl][1-(2-methoxyethyl)piperidin-4-yl]methyl]-2-methylpropane-2-sulfinamide as a yellow solid (0.17 g, 75%): LCMS (ESI) calc'd for C₂₂H₃₄Cl₂N₂O₃S [M + H]⁺: 477, 479 (3 : 2), found 477, 479 (3 : 2).

### Step b:

To a stirred mixture of *N*-[[4,5-dichloro-2-(prop-2-en-1-yloxy)phenyl][1-(2-methoxyethyl)piperidin-4-yl]methyl]-2-methylpropane-2-sulfinamide (0.17 g, 0.36 mmol) and Pd(PPh₃)₄ (45 mg, 0.04 mmol) in THF (2 mL) was added NaBH₄ (27 mg, 0.71 mmol) in portions at room temperature under nitrogen atmosphere. The reaction mixture was stirred for 1 h at room temperature under nitrogen atmosphere. The reaction mixture was quenched with water (30 mL) at room temperature and extracted with EA (3 x 50 mL). The combined organic layers were washed with brine (3 x 10 mL), dried over anhydrous Na₂SO₄. After the filtration, the filtrate was concentrated under reduced pressure to afford crude *N*-((4,5-dichloro-2-hydroxyphenyl)(1-(2-methoxyethyl)piperidin-4-yl)methyl)-2-methylpropane-2-sulfinamide. The crude product was used in the next step directly without further purification: LCMS (ESI) calc'd for C₁₉H₃₀Cl₂N₂O₃S [M + H]⁺: 437, 439 (3 : 2), found 437, 439 (3 : 2);

### Step c:

To a stirred solution of *N*-((4,5-dichloro-2-hydroxyphenyl)(1-(2-methoxyethyl)piperidin-4-yl)methyl)-2-methylpropane-2-sulfinamide (crude) in 1,4-dioxane (2 mL) were added aq. HCl (6 *N,* 1 mL) at room temperature. The reaction solution was stirred for 2 h at room temperature, and then concentrated under reduced pressure. The residue was purified by Prep-HPLC with the following conditions: Column: XBridge C₁₈ OBD Prep Column, 100 Å 10 µm, 19 mm x 250 mm; Mobile Phase A: water with 20 mmol/L NH₄HCO₃, Mobile Phase B: ACN; Flow rate: 25 mL/min; Gradient: 34% B to 50% B in 6.5 min; Detector: UV 254/210 nm; Retention time: 5.80 min. The fractions containing desired product were collected and concentrated to afford Compound 64 (2-(amino(1-(2-methoxyethyl)piperidin-4-yl)methyl)-4,5-dichlorophenol) as an off-white solid (25 mg, 31% overall two steps): LCMS (ESI) calc'd for C₁₅H₂₂Cl₂N₂O₂ [M + H]⁺: 333, 335 (3 : 2), found 333, 335 (3 : 2); ¹H NMR (300 MHz, CD₃OD) δ 7.16 (s, 1H), 6.83 (s, 1H), 3.86 (d, *J* = 7.9 Hz, 1H), 3.51 (t, *J* = 5.7 Hz, 2H), 3.30 (s, 3H), 3.06 (d, *J* = 11.9 Hz, 1H), 2.95 (d, *J* = 11.6 Hz, 1H), 2.55 (t, *J* = 5.7 Hz, 2H), 2.16-1.87 (m, 3H), 1.82-1.69 (m, 1H), 1.46-1.21 (m, 3H).

### Example 133. Compound 65 (3-(4-(amino(4,5-dichloro-2-hydroxyphenyl)methyl)piperidin-1-yl)pyrrolidin-2-one trifluoroacetic acid)

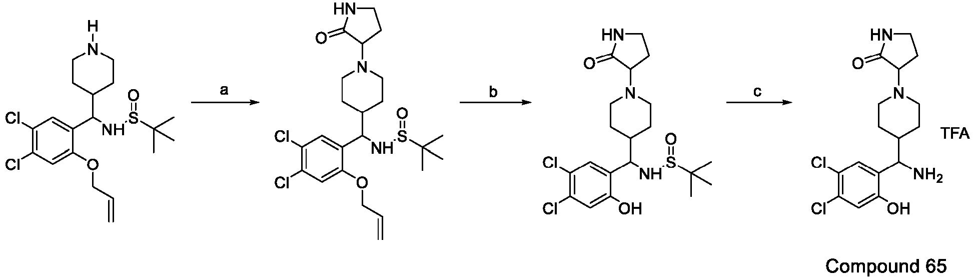

### Step a:

To a stirred solution of *N*-[[4,5-dichloro-2-(prop-2-en-1-yloxy)phenyl](piperidin-4-yl)methyl]-2-methylpropane-2-sulfinamide (0.18 g, 0.43 mmol) and DIEA (0.11 g, 0.86 mmol) in ACN (3 mL) was added 3-bromopyrrolidin-2-one (0.21 g, 1.29 mmol) in portions at room temperature. The reaction solution was stirred for 10 h at room temperature. The resulting solution was diluted with water (30 mL) and extracted with EA (2 x 30 mL). The combined organic layers were washed with brine (2 x 20 mL), dried over anhydrous Na₂SO₄. After filtration, the filtrate was concentrated under reduced pressure. The residue was purified by reverse phase chromatography, eluted with 40% to 70% ACN in water with 10 mmol/L NH₄HCO₃ to afford *N*-((2-(allyloxy)-4,5-dichlorophenyl)(1-(2-oxopyrrolidin-3-yl)piperidin-4-yl)methyl)-2-methylpropane-2-sulfinamide as a yellow solid (0.15 g, 63%): LCMS (ESI) calc'd for C₂₃H₃₃Cl₂N₃O₃S [M + H]⁺: 502, 504 (3 : 2), found 502, 504 (3 : 2).

### Step b:

To a stirred mixture of *N*-((2-(allyloxy)-4,5-dichlorophenyl)(1-(2-oxopyrrolidin-3-yl)piperidin-4-yl)methyl)-2-methylpropane-2-sulfinamide (0.15 g, 0.30 mmol) and Pd(PPh₃)₄ (34 mg, 0.03 mmol) in THF (5 mL) was added NaBH₄ (17 mg, 0.45 mmol) in portions at room temperature under nitrogen atmosphere. The reaction mixture was stirred for 1 h at room temperature under nitrogen atmosphere. The reaction mixture was quenched with water (30 mL) at room temperature. The resulting mixture was extracted with EA (3 x 50 mL). The combined organic layers were washed with brine (3 x 10 mL), dried over anhydrous Na₂SO₄. After the filtration, the filtrate was concentrated under reduced pressure to afford crude *N*-((4,5-dichloro-2-hydroxyphenyl)(1-(2-oxopyrrolidin-3-yl)piperidin-4-yl)methyl)-2-methylpropane-2-sulfinamide. The crude product was used in the next step directly without further purification: LCMS (ESI) calc'd for C₂₀H₂₉Cl₂N₃O₃S [M + H]⁺: 462, 464 (3 : 2), found 462, 464 (3 : 2)

### Step c:

To a stirred solution of *N*-((4,5-dichloro-2-hydroxyphenyl)(1-(2-oxopyrrolidin-3-yl)piperidin-4-yl)methyl)-2-methylpropane-2-sulfinamide (crude) in 1,4-dioxane (4 mL) were added aq. HCl (6 *N,* 2 mL) at room temperature. The reaction solution was stirred for 2 h at room temperature, and then concentrated under reduced pressure. The residue was purified by Prep-HPLC with the following conditions: Column: XBridge C18 OBD Prep Column, 100 Å, 10 µm, 19 mm x 250 mm; Mobile Phase A: water (plus 0.05% TFA), Mobile Phase B: ACN; Flow rate: 25 mL/min; Gradient: 5% B to 30% B in 8 min; Detector: UV 254/210 nm; Retention time: 6.37 min. The fractions containing desired product were collected and concentrated to afford Compound 65 (3-(4-(amino(4,5-dichloro-2-hydroxyphenyl)methyl)piperidin-1-yl)pyrrolidin-2-one trifluoroacetic acid) as an off-white solid (60 mg, 56% overall two steps): LCMS (ESI) calc'd for C₁₆H₂₁Cl₂N₃O₂ [M + H]⁺: 358, 360 (3 : 2), found 358, 360 (3 : 2); ¹H NMR (400 MHz, CD₃OD) δ 7.48 (s, 1H), 7.11 (s, 1H), 4.33-4.18 (m, 2H), 4.15-3.74 (m, 1H), 3.67-3.38 (m, 4H), 3.23-3.00 (m, 1H), 2.58-2.24 (m, 4H), 1.88-1.50 (m, 3H).

### Example 134. Compound 66 ((4-(amino(4,5-dichloro-2-hydroxyphenyl)methyl)piperidin-1-yl)((R)-pyrrolidin-3-yl)methanone trifluoroacetic acid)

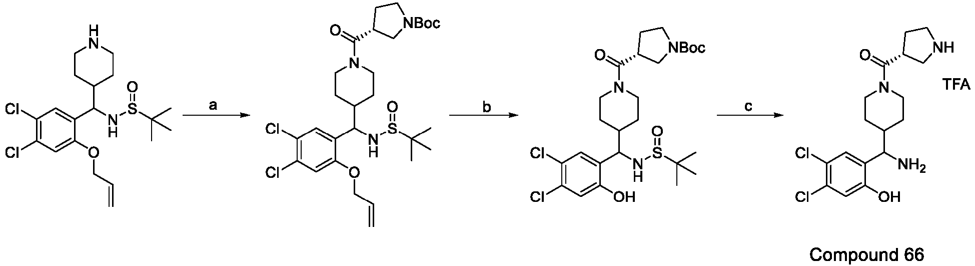

### Step a:

To a stirred solution of *N*-((2-(prop-2-en-1-yloxy)-4,5-dichlorophenyl)(piperidin-4-yl)methyl)-2-methylpropane-2-sulfinamide (Intermediate 5, Example 5) (0.20 g, 0.48 mmol) and (*R*)-1-(*tert*-butoxycarbonyl)pyrrolidine-3-carboxylic acid (0.15 g, 0.72 mmol) in DMF (3 mL) were added Et₃N (0.15 g, 1.44 mmol) and EDCI (0.18 g, 0.96 mmol) at room temperature under nitrogen atmosphere. The reaction solution was stirred for 1 h at room temperature under nitrogen atmosphere. The resulting solution was quenched with water (40 mL) at room temperature and extracted with EA (3 x 50 mL). The combined organic layers were washed with brine (2 x 10 mL), dried over anhydrous Na₂SO₄. After filtration, the filtrate was concentrated under reduced pressure. The residue was purified by Prep-TLC, eluted with PE/EA (1/1) to afford (3*R*)-*tert*-butyl 3-(4-((2-(prop-2-en-1-yloxy)-4,5-dichlorophenyl)(1,1-dimethylethylsulfinamido)methyl)piperidine-1-carbonyl)pyrrolidine-1-carboxylate as a light yellow solid (0.19 g, 62%): LCMS (ESI) calc'd for C₂₉H₄₃Cl₂N₃O₅S [M + H]⁺: 616, 618 (3 : 2), found 616, 618 (3 : 2).

### Step b:

To a stirred solution of (3*R*)-*tert*-butyl 3-(4-((2-(prop-2-en-1-yloxy)-4,5-dichlorophenyl)(1,1-dimethylethylsulfinamido)methyl)piperidine-1-carbonyl)pyrrolidine-1-carboxylate (0.19 g, 0.31 mmol) and Pd(PPh₃)₄ (35 mg, 0.031 mmol) in THF (5 mL) was added NaBH₄ (18 mg, 0.47 mmol) at room temperature under nitrogen atmosphere. The reaction mixture was stirred for 1 h at room temperature under nitrogen atmosphere. The resulting mixture was quenched with water (40 mL) at room temperature and extracted with EA (3 x 50 mL). The combined organic layers were washed with brine (3 x 30 mL), dried over anhydrous Na₂SO₄. After filtration, the filtrate was concentrated under reduced pressure to afford crude (3*R*)-*tert*-butyl 3-(4-((4,5-dichloro-2-hydroxyphenyl)(1,1-dimethylethylsulfinamido)methyl)piperidine-1-carbonyl)pyrrolidine-1-carboxylate. The crude product was used in the next step directly without further purification: LCMS (ESI) calc'd for C₂₆H₃₉Cl₂N₃O₅S [M + H]⁺: 576, 578 (3 : 2), found 576, 578 (3 : 2).

### Step c:

To a stirred solution of (3*R*)-*tert*-butyl 3-(4-((4,5-dichloro-2-hydroxyphenyl)(1,1-dimethylethylsulfinamido)methyl)piperidine-1-carbonyl)pyrrolidine-1-carboxylate (crude) in 1,4-dioxane (5 mL) was added aq. HCl (6 *N,* 3 mL) at room temperature. The reaction solution was stirred for 1 h at room temperature. The reaction solution was concentrated under reduced pressure. The residue was dissolved in DCM (5 mL), and then TFA (3 mL) was added at room temperature. The reaction solution was stirred for 1 h at room temperature. The resulting solution was concentrated under reduced pressure. The residue was purified by Prep-HPLC with the following conditions: Column: Column: XBridge C18 OBD Prep Column, 100 Å, 10 µm, 19 mm x 250 mm; Mobile Phase A: water (plus 0.05% TFA), Mobile Phase B: ACN; Flow rate: 25 mL/min; Gradient: 5% B to 45% B in 7 min; Detector: UV 254/210 nm; Retention time: 5.52. The fractions containing desired product were collected and concentrated under reduced pressure to afford Compound 66 ((4-(amino(4,5-dichloro-2-hydroxyphenyl)methyl)piperidin-1-yl)((R)-pyrrolidin-3-yl)methanone trifluoroacetic acid) as an off-white solid (60.1 mg, 42% overall two steps): LCMS (ESI) calc'd for C₁₇H₂₃Cl₂N₃O₂ [M + H]⁺: 372, 374 (3 : 2), found 372, 374 (3 : 2); ¹H NMR (300 MHz, CD₃OD) δ 7.44 (d, *J* = 3.6 Hz, 1H), 7.09 (d, *J* = 2.2 Hz, 1H), 4.58 (dd, *J* = 41.8, 13.3 Hz, 1H), 4.26-3.96 (m, 2H), 3.74-3.54 (m, 2H), 3.41-3.30 (m, 3H), 3.26-3.00 (m, 1H), 2.80-2.54 (m, 1H), 2.49-2.31 (m, 2H), 2.21-2.02 (m, 2H), 1.59-1.08 (m, 3H); ¹⁹F NMR (282 MHz, CD₃OD) δ -76.92.

### Example 135. Compound 67 (2-[amino[1-(oxetane-3-carbonyl)piperidin-4-yl]methyl]-4,5-dichlorophenol)

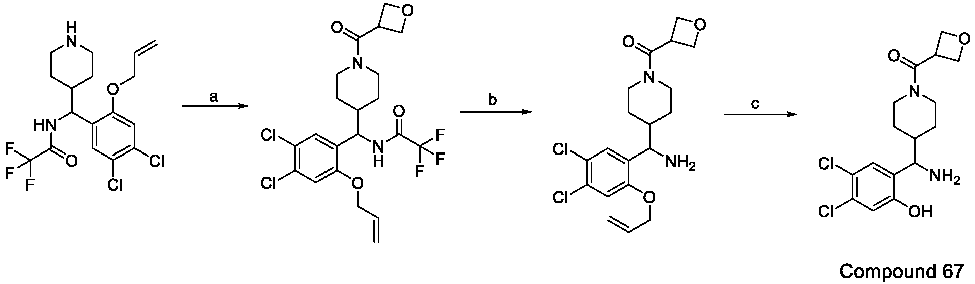

### Step a:

To a stirred solution of HATU (0.42 g, 1.09 mmol) and oxetane-3-carboxylic acid (75 mg, 0.73 mmol) in DMF (3 mL) were added Et₃N (0.12 g, 1.09 mmol) and a solution of *N-*[[4,5-dichloro-2-(prop-2-en-1-yloxy)phenyl](piperidin-4-yl)methyl]-2,2,2-trifluoroacetamide (0.15 g, 0.36 mmol) in DMF (2 mL) at room temperature under nitrogen atmosphere. After stirring for additional 2 h at room temperature, the reaction solution was quenched with water (20 mL) at room temperature and extracted with EA (3 x 30 mL). The combined organic layers were washed with brine (3 x 30 mL), dried over anhydrous Na₂SO₄. After filtration, the filtrate was concentrated under reduced pressure. The residue was purified by silica gel column chromatography, eluted with PE/EA (5/1) to afford N-[[4,5-dichloro-2-(prop-2-en-1-yloxy)phenyl][1-(oxetane-3-carbonyl)piperidin-4-yl]methyl]-2,2,2-trifluoroacetamide as a yellow solid (0.13 g, 72%): LCMS (ESI) calc'd for C₂₁H₂₃Cl₂F₃N₂O₄ [M + H]⁺: 495, 497 (3 : 2), found 495, 497 (3 : 2).

### Step b:

To a stirred solution of *N*-[[4,5-dichloro-2-(prop-2-en-1-yloxy)phenyl][1-(oxetane-3-carbonyl)piperidin-4-yl]methyl]-2,2,2-trifluoroacetamide (0.20 g, 0.40 mmol) in MeOH/water (10 mL/5 mL) was added KOH (0.11 g, 2.00 mmol) at room temperature. The reaction mixture was stirred for 6 h at room temperature and diluted with water (30 mL) at room temperature. The resulting mixture was extracted with EA (3 x 50 mL). The combined organic layers were washed with brine (3 x 30 mL), dried over anhydrous Na₂SO₄. After the filtration, the filtrate was concentrated under reduced pressure to afford 1-[4,5-dichloro-2-(prop-2-en-1-yloxy)phenyl]-1-[1-(oxetane-3-carbonyl)piperidin-4-yl]methanamine (0.10 g, crude). The crude product was used in next step without further purification as a yellow solid: LCMS (ESI) calc'd for C₁₉H₂₄Cl₂N₂O₃ [M + H]⁺: 399, 401 (3 : 2), found 399, 401 (3 : 2).

### Step c:

To a stirred solution of 1-[4,5-dichloro-2-(prop-2-en-1-yloxy)phenyl]-1-[1-(oxetane-3-carbonyl)piperidin-4-yl]methanamine (0.10 g, 0.25 mmol) and Pd(PPh₃)₄ (6 mg, 0.01 mmol) in THF was added NaBH₄ (14 mg, 0.38 mmol) in portions at room temperature under nitrogen atmosphere. After stirring for 1 h at room temperature under nitrogen atmosphere, the reaction mixture was quenched with water (20 mL) at room temperature. The resulting mixture was extracted with EA (2 x 20 mL). The aqueous layer was collected and concentrated under reduced pressure. The residue was purified by Prep-HPLC with the following conditions: Column: XBridge Shield RP18 OBD Column, 5 µm, 19 x 150 mm; Mobile Phase A: water with 10 mmol/L NH₄HCO₃, Mobile Phase B: ACN; Flow rate: 25 mL/min; Gradient: 10% B to 35% B in 7 min; Detector: UV 254/220 nm; Retention time: 6.5 min. The fractions containing desired product were collected and concentrated under reduced pressure to afford Compound 67 (2-[amino[1-(oxetane-3-carbonyl)piperidin-4-yl]methyl]-4,5-dichlorophenol) as an off-white solid (18 mg, 20%): LCMS (ESI) calc'd for C₁₆H₂₀Cl₂N₂O₃ [M + H]+: 359, 361 (3 : 2), found 359, 361 (3 : 2); ¹H NMR (300 MHz, CD₃OD) δ 7.43 (s, 1H), 7.08 (s, 1H), 4.90-4.75 (m, 4 H), 4.60 (dd, *J* = 36.8, 1.3 Hz; 1 H), 4.20-4.08 (m, 2H), 3.50 (dd, *J* = 36.8, 1.3 Hz; 1H), 3.12-2.88 (m, 1H), 2.78-2.58 (m, 1H), 2.45-2.26 (m, 1H), 2.10-1.90 (m, 1H), 1.49-1.01 (m, 3H).

### Example 136. Compound 68 (2-[amino(1-benzoylpiperidin-4-yl)methyl]-4,5-dichlorophenol trifluoroacetic acid)

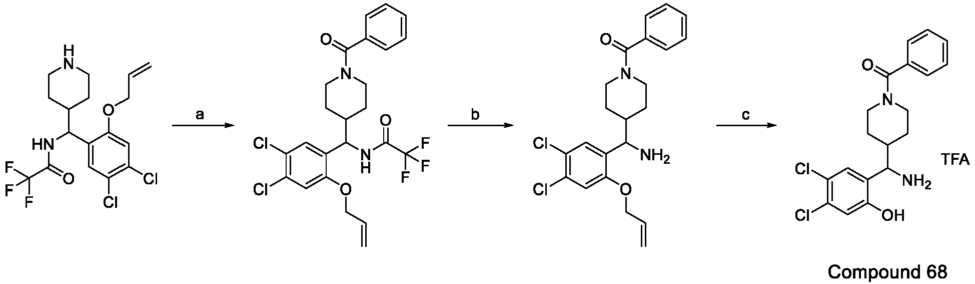

### Step a:

To a stirred solution of HATU (0.42 g, 1.09 mmol) and benzoic acid (89 mg, 0.72 mmol) in DMF (5 mL) were dropwise added a solution of *N*-[[4,5-dichloro-2-(prop-2-en-1-yloxy)phenyl](piperidin-4-yl)methyl]-2,2,2-trifluoroacetamide (0.15 g, 0.36 mmol) in DMF (2 mL) and Et₃N (0.11 g, 1.09 mmol) at room temperature under nitrogen atmosphere. The reaction solution was stirred for 2 h at room temperature under nitrogen atmosphere. The resulting solution was quenched with water (30 mL) at room temperature and extracted with EA (3 x 20 mL). The combined organic layers were washed with brine (3 x 30 mL), dried over anhydrous Na₂SO₄. After the filtration, the filtrate was concentrated under reduced pressure. The residue was purified by silica gel column chromatography, eluted with PE/EA (5/1) to afford *N*-((2-(allyloxy)-4,5-dichlorophenyl)(1-benzoylpiperidin-4-yl)methyl)-2,2,2-trifluoroacetamide as a yellow solid (0.12 g, 78%): LCMS (ESI) calc'd for C₂₄H₂₃Cl₂F₃N₂O₃ [M + H]⁺: 515, 517 (3 : 2), found 515, 517 (3 : 2).

### Step b:

To a stirred solution of *N*-((2-(allyloxy)-4,5-dichlorophenyl)(1-benzoylpiperidin-4-yl)methyl)-2,2,2-trifluoroacetamide (0.12 g, 0.23 mmol) in MeOH/water (10 mL/5 mL) was added KOH (64 mg, 1.15 mmol) at room temperature. The reaction mixture was stirred for 6 h at room temperature. The resulting mixture was diluted with water (30 mL) at room temperature and extracted with EA (3 x 50 mL). The combined organic layers were washed with brine (3 x 30 mL), dried over anhydrous Na₂SO₄. After the filtration, the filtrate was concentrated under reduced pressure. The crude product as a yellow solid was used in the next step without further purification: LCMS (ESI) calc'd for C₂₂H₂₄Cl₂N₂O₂ [M + H]⁺: 419, 421 (3 : 2), found 419, 421 (3 : 2).

### Step c:

To a stirred solution of (4-((2-(allyloxy)-4,5-dichlorophenyl)(amino)methyl)piperidin-1-yl)(phenyl)methanone (0.15 g, crude) and Pd(PPh₃)₄ (7 mg, 0.01 mmol) in THF was added NaBH4 (18 mg, 0.47 mmol) at room temperature under nitrogen atmosphere. The reaction mixture was stirred for 1 h at room temperature under nitrogen atmosphere. The resulting mixture was quenched with water (20 mL) at room temperature and extracted with EA (3 x 30 mL). The aqueous layer was collected and concentrated under reduced pressure. The residue was purified by Prep-HPLC with the following conditions: Column: XBridge Shield RP18 OBD Column, 5 µm, 19 x 150 mm; Mobile Phase A: water (plus 0.5% TFA), Mobile Phase B: ACN; Flow rate: 25 mL/min; Gradient: 10% B to 35% B in 7 min; Detector: UV 254/220 nm; Retention time: 6.5 min. The fractions containing desired product were collected and concentrated under reduced pressure to afford Compound 68 (2-[amino(1-benzoylpiperidin-4-yl)methyl]-4,5-dichlorophenol trifluoroacetic acid) as an off-white solid (63 mg, 52%): LCMS (ESI) calc'd for C₁₉H₂₀Cl₂N₂O₂ [M + H]⁺: 379, 381 (3 : 2), found 379, 381 (3 : 2); ¹H NMR (300 MHz, CD₃OD) δ 7.48-7.42 (m, 6H), 7.08 (s, 1H), 4.80-4.65 (m, 1 H), 4.20 (d, *J* = 9.6 Hz; 1 H), 3.98-3.65 (m, 1 H), 3.20-2.65 (m, 2H), 2.50-2.31 (m, 1H), 2.12-1.88 (m, 1H), 1.59-1.11 (m, 3H); ¹⁹F NMR (282 MHz, CD₃OD) δ -77.00.

### Example 137. Compound 69 ((R)-2-amino-1-(4-((R)-amino(4,5-dichloro-2-hydroxyphenyl)methyl)piperidin-1-yl)propan-1-one) and Compound 87 ((R)-2-amino-1-(4-((S)-amino(4,5-dichloro-2-hydroxyphenyl)methyl)piperidin-1-yl)propan-1-one)

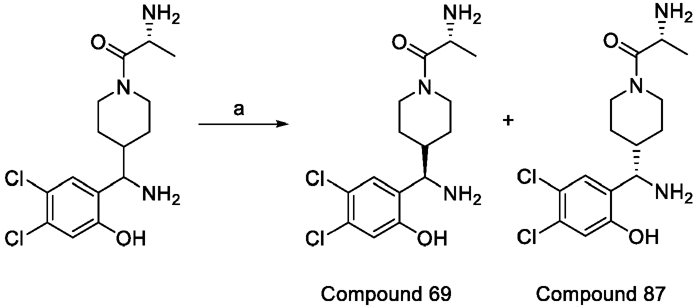

### Step a:

(2*R*)-2-amino-1-(4-(amino(4,5-dichloro-2-hydroxyphenyl)methyl)piperidin-1-yl)propan-1-one (50 mg) was separated by Chiral-Prep-HPLC with the following conditions: Column: Column: CHIRALPAK IG UL001, 20 x 250 mm, 5 µm; Mobile Phase A: Hexane (plus 0.2% IPA), Mobile Phase B: EtOH; Flow rate: 18 mL/min; Gradient: 40% B to 40% B in 16 min; Detector: UV 254/220 nm; Retention time: RT₁: 11.55 min; RT₂: 15.73 min; Injection Volume: 0.3 mL.

The faster-eluting enantiomer was obtained as Compound 87 ((*R*)-2-amino-1-(4-((*S)-*amino(4,5-dichloro-2-hydroxyphenyl)methyl)piperidin-1-yl)propan-1-one) as an off-white solid (6.3 mg, 13%) at 11.55 min: LCMS (ESI) calc'd for C₁₅H₂₁Cl₂N₃O₂ [M + H]⁺: 346, 348 (3 : 2), found 346, 348 (3 : 2); ¹H NMR (400 MHz, CD₃OD) δ 7.22 (d, *J* = 11.9 Hz, 1H), 6.86 (d, *J* = 5.1 Hz, 1H), 4.55 (dd, *J* = 36.6, 13.5 Hz, 1H), 4.08-3.73 (m, 3H), 3.18-2.94 (m, 1H), 2.71-2.55 (m, 1H), 2.14-1.92 (m, 2H), 1.59-1.42 (m, 1H), 1.38-1.11 (m, 5H).

The slower-eluting enantiomer was obtained as Compound 69 ((R)-2-amino-1-(4-((R)-amino(4,5-dichloro-2-hydroxyphenyl)methyl)piperidin-1-yl)propan-1-one) as an off-white solid (2.1 mg, 4%) at 15.73 min: LCMS (ESI) calc'd for C₁₅H₂₁Cl₂N₃O₂ [M + H]⁺: 346, 348 (3 : 2), found 346, 348 (3 : 2); ¹H NMR (400 MHz, CD₃OD) δ 7.22 (d, *J* = 3.7 Hz, 1H), 6.87 (s, 1H), 4.56 (dd, *J* = 13.3, 38.9 Hz, 1H), 4.06-3.85 (m, 3H), 3.18-2.97 (m, 1H), 2.70-2.52 (m, 1H), 2.14-1.92 (m, 2H), 1.59-1.42 (m, 1H), 1.38-1.11 (m, 5H).

### Example 138. Compound 70 (2-[amino[1-(pyridin-2-yl)piperidin-4-yl]methyl]-4,5-dichlorophenol trifluoroacetic acid)

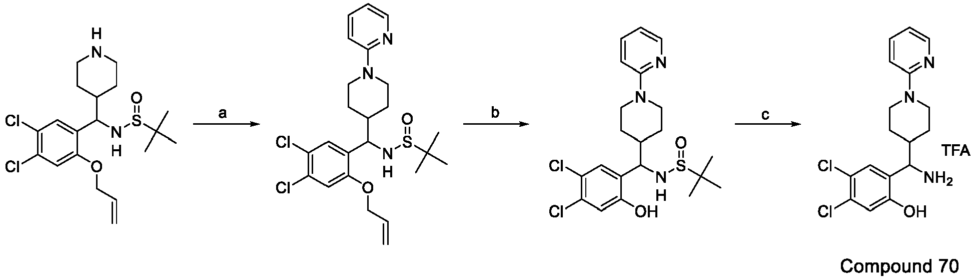

### Step a:

To a stirred solution of *N*-[[4,5-dichloro-2-(prop-2-en-1-yloxy)phenyl](piperidin-4-yl)methyl]-2-methylpropane-2-sulfinamide (0.30 g, 0.72 mmol) and 2-fluoropyridine (0.14 g, 1.43 mmol) in DMF (8 mL) was added *t*-BuOK (0.24 g, 2.15 mmol) at room temperature under nitrogen atmosphere. The reaction mixture was stirred for 2 h at room temperature under nitrogen atmosphere. The resulting mixture was diluted with water (30 mL) and extracted with EA (3 x 30 mL). The combined organic layers were washed with water (2 x 10 mL), dried over anhydrous Na₂SO₄. After filtration, the filtrate was concentrated under reduced pressure. The residue was purified by reverse phase chromatography, eluted with 65% ACN in water with 20 mmol/L NH₄HCO₃ to afford *N*-[[4,5-dichloro-2-(prop-2-en-1-yloxy)phenyl][1-(pyridin-2-yl)piperidin-4-yl]methyl]-2-methylpropane-2-sulfinamide as a light yellow oil (80 mg, 22%): LCMS (ESI) calc'd for C₂₄H₃₁Cl₂N₃O₂S [M + H]⁺: 496, 498 (3 : 2), found 496, 498 (3 : 2).

### Step b:

To a stirred solution of *N*-[[4,5-dichloro-2-(prop-2-en-1-yloxy)phenyl][1-(pyridin-2-yl)piperidin-4-yl]methyl]-2-methylpropane-2-sulfinamide (80 mg, 0.16 mmol) and Pd(PPh₃)₄ (37 mg, 0.03 mmol) in THF (3 mL) was added NaBH₄ (12 mg, 0.32 mmol) at room temperature under nitrogen atmosphere. The reaction mixture was stirred for 1 h at room temperature under nitrogen atmosphere. The resulting mixture was quenched with water (40 mL) at room temperature and extracted with EA (3 x 50 mL). The combined organic layers were washed with brine (3 x 30 mL), dried over anhydrous Na₂SO₄. After filtration, the filtrate was concentrated under reduced pressure to afford crude *N*-((4,5-dichloro-2-hydroxyphenyl)(1-(pyridin-2-yl)piperidin-4-yl)methyl)-2-methylpropane-2-sulfinamide. The crude product was used in the next step directly without further purification: LCMS (ESI) calc'd for C₂₁H₂₇Cl₂N₃O₂S [M + H]⁺: 456, 458 (3 : 2), found 456, 458 (3 : 2).

### Step c:

To a stirred solution of *N*-((4,5-dichloro-2-hydroxyphenyl)(1-(pyridin-2-yl)piperidin-4-yl)methyl)-2-methylpropane-2-sulfinamide (crude) in 1,4-dioxane (3 mL) was added aq. HCl *(6 N,* 1.5 mL) at room temperature. The reaction solution was stirred for 2 h at room temperature. The resulting solution was neutralized to pH 7 with saturated aq. NaHCO₃ and concentrated under reduced pressure. The residue was purified by Prep-HPLC with the following conditions: Column: XBridge C18 OBD Prep Column 100 Å, 10 µm, 19 mm x 250 mm; Mobile Phase A: water (plus 0.05% TFA), Mobile Phase B: ACN; Flow rate: 20 mL/min; Gradient: 16% B to 30% B in 8 min; Detector: UV 254/210 nm; Retention time: 6.23 min. The fractions containing desired product were collected and concentrated under reduced pressure to afford Compound 70 (2-[amino[1-(pyridin-2-yl)piperidin-4-yl]methyl]-4,5-dichlorophenol trifluoroacetic acid) as an off-white solid (5 mg, 11% overall two steps): LCMS (ESI) calc'd for C₁₇H₁₉Cl₂N₃O [M + H]⁺: 352, 354 (3 : 2), found 352, 354 (3 : 2); ¹H NMR (300 MHz, DMSO-*d*₆ + D₂O) δ 8.04 (dd, *J* = 4.9, 2.0 Hz, 1H), 7.50-7.39 (m, 1H), 7.27 (s, 1H), 6.84 (s, 1H), 6.76 (d, *J* = 8.7 Hz, 1H), 6.53 (dd, *J* = 7.1, 4.9 Hz, 1H), 4.29 (t, *J* = 16.4 Hz, 2H), 3.87 (d, *J* = 6.4 Hz, 1H), 2.75-2.55 (m, 2H), 1.90-1.65 (m, 2H), 1.45-1.06 (m, 3H).

### Example 139. Compound 71 (2-[amino(1-methanesulfonylpiperidin-4-yl)methyl]-4,5-dichlorophenol trifluoroacetic acid)

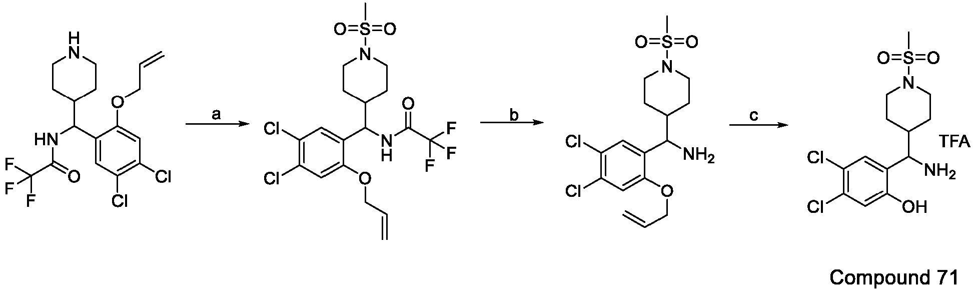

### Step a:

To a stirred solution of *N*-[[4,5-dichloro-2-(prop-2-en-1-yloxy)phenyl](piperidin-4-yl)methyl]-2,2,2-trifluoroacetamide (0.15 g, 0.36 mmol) and Et₃N (0.11 g) in DCM (5 mL) was added methanesulfonyl chloride (84 mg, 0.73 mmol) dropwise at room temperature under nitrogen atmosphere. After stirring for additional 2 h at room temperature under nitrogen atmosphere, the reaction solution was quenched with water (20 mL) at room temperature. The resulting mixture was extracted with EA (3 x 30 mL). The combined organic layers were washed with brine (3 x 30 mL), dried over anhydrous Na₂SO₄. After the filtration, the filtrate was concentrated under reduced pressure. The residue was purified by silica gel column chromatography, eluted with PE/EtOAc (5/1) to afford *N*-[[4,5-dichloro-2-(prop-2-en-1-yloxy)phenyl](1-methanesulfonylpiperidin-4-yl)methyl]-2,2,2-trifluoroacetamide as a yellow solid (0.11 g, 61%): LCMS (ESI) calc'd for C₁₈H₂₁Cl₂F₃N₂O₄S [M + H]⁺: 489, 491 (3 : 2), found 489, 491 (3 : 2).

### Step b:

To a stirred solution of *N*-[[4,5-dichloro-2-(prop-2-en-1-yloxy)phenyl](1-methanesulfonylpiperidin-4-yl)methyl]-2,2,2-trifluoroacetamide (80 mg, 0.16 mmol) in MeOH/water (5 mL/2 mL) was added KOH (0.11 g, 1.96 mmol) at room temperature under nitrogen atmosphere. The reaction mixture was stirred for 3 h at room temperature under nitrogen atmosphere. The resulting mixture was diluted with water (10 mL) at room temperature and extracted with EA (3 x 20 mL). The combined organic layers were washed with brine (3 x 3 mL), dried over anhydrous Na₂SO₄. After filtration, the filtrate was concentrated under reduced pressure. The residue was purified by reverse phase chromatography, eluted with 45% ACN in water with 20 mmol/L NH₄HCO₃ to afford 1-[4,5-dichloro-2-(prop-2-en-1-yloxy)phenyl]-1-(1-methanesulfonylpiperidin-4-yl)methanamine as a light yellow solid (44 mg, 68%): LCMS (ESI) calc'd for C₁₆H₂₂Cl₂N₂O₃S [M + H]⁺: 393, 395 (3 : 2), found 393, 395 (3 : 2).

### Step c:

To a stirred solution of 1-[4,5-dichloro-2-(prop-2-en-1-yloxy)phenyl]-1-(1-methanesulfonylpiperidin-4-yl)methanamine (0.10 g, 0.25 mmol) and Pd(PPh₃)₄ (6 mg, 0.01 mmol) in THF was added NaBH₄ (14 mg, 0.38 mmol) at room temperature under nitrogen atmosphere. The reaction mixture was stirred for 1 h at room temperature under nitrogen atmosphere. The resulting mixture was quenched with water (10 mL) at room temperature and concentrated under reduced pressure. The reside was purified by Prep-HPLC with the following conditions: Column: XBridge Shield RP18 OBD Column, 5 µm, 19 x 150 mm; Mobile Phase A: water (plus 0.05% TFA), Mobile Phase B: ACN; Flow rate: 25 mL/min; Gradient: 10% B to 35% B in 7 min; Detector: UV 254/220 nm; Retention time: 6.5 min. The fractions containing desired product were collected and concentrated under reduced pressure to afford Compound 71 (2-[amino(1-methanesulfonylpiperidin-4-yl)methyl]-4,5-dichlorophenol trifluoroacetic acid) as an off-white solid (15 mg, 16%): LCMS (ESI) calc'd for C₁₃H₁₈Cl₂N₂O₃S [M + H]⁺: 353, 355 (3 : 2), found 353, 355 (3 : 2); ¹H NMR (300 MHz, CD₃OD) δ 7.44 (s, 1H), 7.09 (s, 1H), 4.21 (d, *J* = 9.6 Hz; 1 H), 3.85 (d, *J=* 11.7 Hz; 1 H), 3.70 (d, *J* = 11.7 Hz; 1H), 2.83 (s, 3H), 2.80-2.58 (m, 2H), 2.32-2.18 (m, 1H), 2.10-2.02 (m, 1H), 1.59-1.20 (m, 3H); ¹⁹F NMR (282 MHz, CD₃OD) δ - 76.96.

### Example 140. (which is a reference example) Compound 72 (2-(amino(2,2-dimethylpiperidin-4-yl)methyl)-4,5-dichlorophenol trifluoroacetic acid diastereoisomer 1) and Compound 73 (2-(amino(2,2-dimethylpiperidin-4-yl)methyl)-4,5-dichlorophenol trifluoroacetic acid diastereoisomer 2)

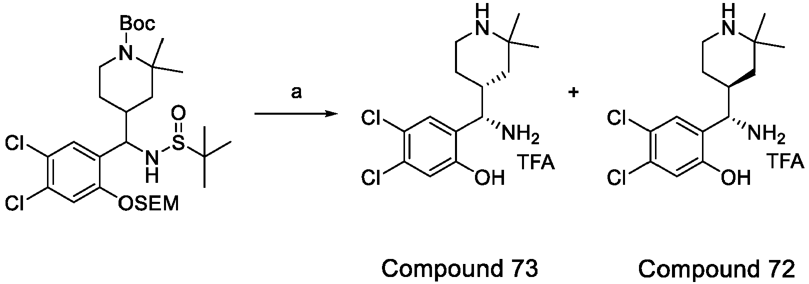

### Step a:

To a solution of *tert*-butyl 4-[(4,5-dichloro-2-[[2-(trimethylsilyl)ethoxy]methoxy]phenyl)[(2-methylpropane-2-sulfinyl)amino]methyl]-2,2-dimethylpiperidine-1-carboxylate (Intermediate 50, Example 50) (65 mg, 0.10 mmol) in 1,4-dioxane (2 mL) was added aq. HCl (12 *N,* 2 mL) at room temperature. The reaction solution was stirred at room temperature for 0.5 h. Then the reaction mixture was concentrated under reduced pressure. The residue was purified by Prep-HPLC with the following conditions: Column: XBridge C18 OBD Prep Column 19 mm x 250 mm, 10 µm; Mobile Phase A: water (plus 0.05% TFA), Mobile Phase B: ACN; Flow rate: 25 mL/min; Gradient: 10% B to 40% B in 8 min; Detector: UV 254/210 nm; Retention time RT₁: 6.55 min; RT₂: 7.05 min.

The faster-eluting enantiomer was obtained as Compound 73 (2-(amino(2,2-dimethylpiperidin-4-yl)methyl)-4,5-dichlorophenol trifluoroacetic acid diastereoisomer 2) as an off-white solid (14 mg, 34%): LCMS (ESI) calc'd for C₁₄H₂₀Cl₂N₂O [M + H]⁺: 303, 304 (3 : 2), found 303, 304 (3 : 2); ¹H NMR (400 MHz, CDCl₃) δ 7.48 (s, 1H), 7.11 (s, 1H), 4.21 (d, *J* = 9.4 Hz, 1H), 3.27-3.11 (m, 2H), 2.67-2.53 (m, 1H), 2.10-2.01 (m, 1H), 1.65-1.21 (m, 9H); ¹⁹F NMR (376 MHz, CD₃OD) δ -77.01.

The slower-eluting enantiomer was obtained as Compound 72 (2-(amino(2,2-dimethylpiperidin-4-yl)methyl)-4,5-dichlorophenol trifluoroacetic acid diastereoisomer 1) as an off-white solid (8 mg, 20%): LCMS (ESI) calc'd for C₁₄H₂₀Cl₂N₂O [M + H]⁺: 303, 304 (3 : 2), found 303, 304 (3 : 2); ¹H NMR (300 MHz, CDCl₃) δ 7.45 (s, 1H), 7.11 (s, 1H), 4.20 (d, *J* = 9.3 Hz, 1H), 3.43-3.35 (m, 1H), 3.30-3.19 (m, 1H), 2.73-2.58 (m, 1H), 2.21 (d, *J* = 14.0 Hz, 1H), 1.61-1.27 (m, 9H).; ¹⁹F NMR (376 MHz, CD₃OD) δ -77.01.

### Example 141. Compound 74 (2-[amino[1-(1,3-oxazol-2-yl)piperidin-4-yl]methyl]-4,5-dichlorophenol)

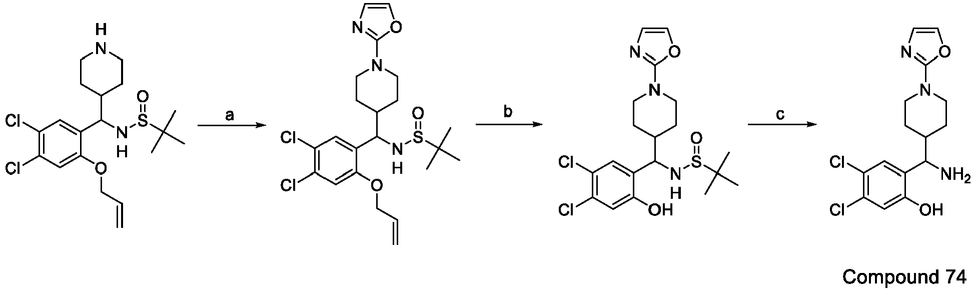

### Step a:

To a stirred solution of N-[[4,5-dichloro-2-(prop-2-en-1-yloxy)phenyl](piperidin-4-yl)methyl]-2-methylpropane-2-sulfinamide (0.20 g, 0.48 mmol) in NMP (5 mL) were added *t-*BuOK (0.16 g, 1.43 mmol) and 2-bromo-1,3-oxazole (0.14 g, 0.95 mmol) at room temperature. The reaction mixture was allowed to warm to 150 °C and stirred for 2 h. After cooling to room temperature, the resulting mixture was concentrated under reduced pressure. The residue was purified by reverse phase chromatography, eluted with 60% ACN in water with 10 mmol/L NH₄HCO₃ to afford *N*-[[4,5-dichloro-2-(prop-2-en-1-yloxy)phenyl][1-(1,3-oxazol-2-yl)piperidin-4-yl]methyl]-2-methylpropane-2-sulfinamide as a light yellow oil (80 mg, 34%): LCMS (ESI) calc'd for C₂₂H₂₉Cl₂N₃O₃S [M + H]⁺: 486, 488 (3 : 2), found 486, 488 (3 : 2).

### Step b:

To a stirred solution of *N*-[[4,5-dichloro-2-(prop-2-en-1-yloxy)phenyl][1-(1,3-oxazol-2-yl)piperidin-4-yl]methyl]-2-methylpropane-2-sulfinamide (80 mg, 0.16 mmol) and Pd(PPh₃)₄ (4 mg, 0.03 mmol) in THF (5 mL) was added NaBH₄ (12 mg, 0.32 mmol) at room temperature under nitrogen atmosphere. The reaction mixture was stirred for 1 h at room temperature under nitrogen atmosphere. The resulting mixture was quenched with water (40 mL) at room temperature and extracted with EA (3 x 50 mL). The combined organic layers were washed with brine (3 x 30 mL), dried over anhydrous Na₂SO₄. After filtration, the filtrate was concentrated under reduced pressure to afford crude N-[(4,5-dichloro-2-hydroxyphenyl)[1-(1,3-oxazol-2-yl)piperidin-4-yl] methyl]-2-methylpropane-2-sulfinamide. The crude product was used in the next step directly without further purification: LCMS (ESI) calc'd for C₁₉H₂₅Cl₂N₃O₃S [M + H]⁺: 446, 448 (3 : 2), found 446, 448 (3 : 2).

### Step c:

To a stirred solution of *N*-[(4,5-dichloro-2-hydroxyphenyl)[1-(1,3-oxazol-2-yl)piperidin-4-yl]methyl]-2-methylpropane-2-sulfinamide (50 mg, 0.11 mmol) in 1,4-dioxane (4 mL) was added aq. HCl (6 *N,* 2 mL) at room temperature. The reaction solution was stirred for 2 h at room temperature. The resulting solution was neutralized to pH 7 with saturated aq. NaHCO₃ and concentrated under reduced pressure. The residue was purified by Prep-HPLC with the following conditions: Column: XBridge C18 OBD Prep Column 100 Å, 10 µm, 19 mm x 250 mm; Mobile Phase A: water with 20 mmol/L NH₄HCO₃, Mobile Phase B: ACN; Flow rate: 25 mL/min; Gradient: 48% B to 52% B in 6.5 min; Detector: UV 254/210 nm; Retention time: 5.63 min. The fractions containing desired product were collected and concentrated under reduced pressure to afford Compound 74 (2-[amino[1-(1,3-oxazol-2-yl)piperidin-4-yl]methyl]-4,5-dichlorophenol) as an off-white solid (10 mg, 9% overall two steps): LCMS (ESI) calc'd for C₁₅H₁₇Cl₂N₃O₂ [M + H]⁺: 342, 344 (3 : 2), found 342, 344 (3 : 2); ¹H NMR (300 MHz, DMOS*d*₆ + D₂OD) δ 7.49 (d, *J* = 1.0 Hz, 1H), 7.28 (s, 1H), 6.84 (s, 1H), 6.78 (d, *J* = 1.1 Hz, 1H), 3.97-3.82 (m, 3H), 2.87-2.68 (m, 2H), 1.87-1.61 (m, 2H), 1.42-1.13 (m, 3H).

### Example 142. Compound 75 ((S)-2-amino-1-(4-((S)-amino(4,5-dichloro-2-hydroxyphenyl)methyl)piperidin-1-yl)propan-1-one) and Compound 76 ((S)-2-amino-1-(4-((R)-amino(4,5-dichloro-2-hydroxyphenyl)methyl)piperidin-1-yl)propan-1-one)

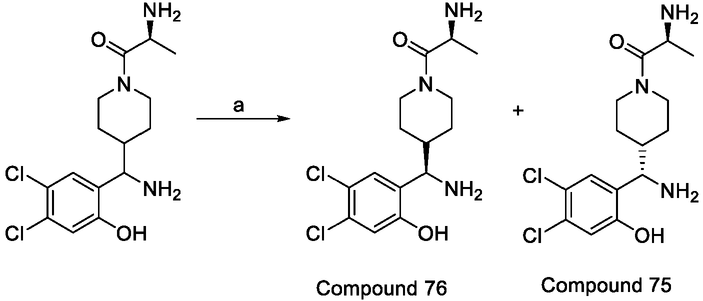

### Step a:

(2*S*)-2-Amino-1-(4-(amino(4,5-dichloro-2-hydroxyphenyl)methyl)piperidin-1-yl)propan-1-one (50 mg) was separated by Chiral-Prep-HPLC with the following conditions: Column: CHIRALPAK IC, 2 x 25 cm, 5 µm; Mobile Phase A: Hexane (plus 0.2%IPA), Mobile Phase B: EtOH; Flow rate: 20 mL/min; Gradient: 30% B to 30% B in 18 min; Detector: UV 220/254 nm; Retention time: RT₁: 12.47 min; RT₂: 15.87 min; Injection Volume: 0.3 mL.

The faster-eluting enantiomer was obtained as Compound 75 ((*S*)-2-amino-1-(4-((*S*)-amino(4,5-dichloro-2-hydroxyphenyl)methyl)piperidin-1-yl)propan-1-one) as an off-white solid (8.8 mg, 18%) at 12.47 min: LCMS (ESI) calc'd for C₁₅H₂₁Cl₂N₃O₂ [M + H]⁺: 346, 348 (3 : 2), found 346, 348 (3 : 2); ¹H NMR (300 MHz, CD₃OD) δ 7.25 (d, *J* = 7.9 Hz, 1H), 6.90 (d, *J* = 3.2 Hz, 1H), 4.56 (dd, *J* = 37.8, 13.4 Hz, 1H), 4.15-3.79 (m, 3H), 3.16-2.94 (m, 1H), 2.74-2.52 (m, 1H), 2.16-1.95 (m, 2H), 1.59-1.43 (m, 1H), 1.42-1.09 (m, 5H).

The slower-eluting enantiomer was obtained as Compound 76 ((S)-2-amino-1-(4-((R)-amino(4,5-dichloro-2-hydroxyphenyl)methyl)piperidin-1-yl)propan-1-one) as an off-white solid (2.7 mg, 5%) at 15.87 min: LCMS (ESI) calc'd for C₁₅H₂₁Cl₂N₃O₂ [M + H]⁺: 346, 348 (3 : 2), found 346, 348 (3 : 2); ¹H NMR (400 MHz, CD₃OD) δ 7.25 (d, *J* = 7.9 Hz, 1H), 6.90 (d, *J* = 3.2 Hz, 1H), 4.56 (dd, *J* = 37.8, 13.4 Hz, 1H), 4.15-3.79 (m, 3H), 3.16-2.94 (m, 1H), 2.74-2.52 (m, 1H), 2.16-1.95 (m, 2H), 1.59-1.43 (m, 1H), 1.42-1.09 (m, 5H).

### Example 143. Compound 77 (1-[4-[amino(4,5-dichloro-2-hydroxyphenyl)methyl]piperidin-1-yl]-2,2,2-trifluoroethan-1-one trifluoroacetic acid)

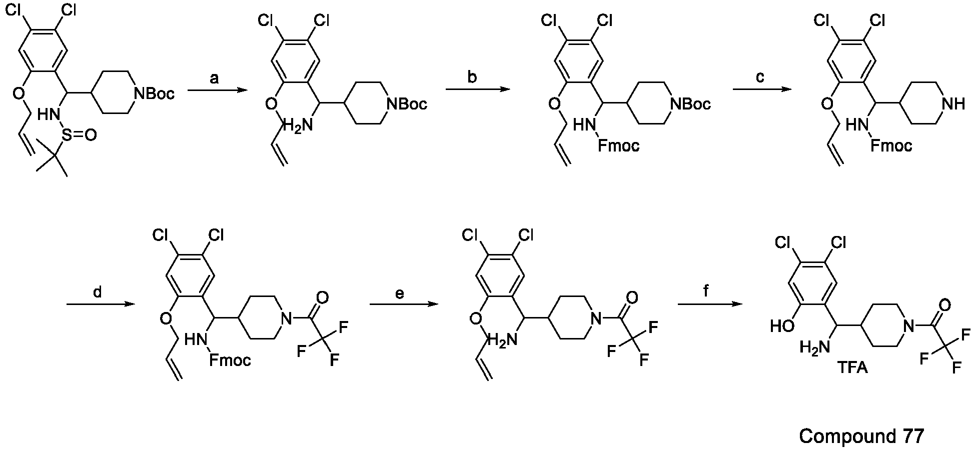

### Step a:

To a stirred solution of N-[[4,5-dichloro-2-(prop-2-en-1-yloxy)phenyl](piperidin-4-yl)methyl]-2-methylpropane-2-sulfinamide (0.30 g, 0.72 mmol) in 1,4-dioxane (5 mL) was added aq. HCl (4 *N,* 1 mL) at room temperature. The reaction solution was stirred for 2 h at room temperature. The resulting mixture was adjusted pH value to 7 with saturated aq. NaHCO₃ and concentrated under reduced pressure. The residue was purified by reverse phase chromatography, eluted with 85% ACN in water with 20 mmol/L NH₄HCO₃ to afford tert-butyl 4-((2-(allyloxy)-4,5-dichlorophenyl)(amino)methyl)piperidine-1-carboxylate as a brown oil (0.35 g, 89%): LCMS (ESI) calc'd for C₂₀H₂₈Cl₂N₂O₃ [M + H]⁺: 415, 417 (3 : 2), found 415, 417 (3 : 2).

### Step b:

To a stirred solution of *tert*-butyl 4-((2-(allyloxy)-4,5-dichlorophenyl)(amino)methyl)piperidine-1-carboxylate (0.35 g, 0.96 mmol) and Et₃N (0.19 g, 1.93 mmol) in DCM (5 mL) was added (9*H*-fluoren-9-yl)methyl carbonochloridate (0.37 g, 1.44 mmol) at room temperature under nitrogen atmosphere. The reaction solution was stirred for 2 h at room temperature under nitrogen atmosphere. The resulting solution was concentrated under reduced pressure. The residue was purified by silica gel column chromatography, eluted with PE/EA (5/1) to afford *tert*-butyl 4-[[4,5-dichloro-2-(prop-2-en-1-yloxy)phenyl]([[(9*H*-fluoren-9-yl)methoxy]carbonyl]amino)methyl]piperidine-1-carboxylate as an off-white foam (0.35 g, 65%): LCMS (ESI) calc'd for C₃₅H₃₈Cl₂N₂O₅ [M + H]⁺: 637, 639 (3 : 2), found 637, 639 (3 : 2).

### Step c:

To a stirred solution of *tert*-butyl 4-[[4,5-dichloro-2-(prop-2-en-1-yloxy)phenyl]([[(9*H*-fluoren-9-yl)methoxy]carbonyl]amino)methyl]piperidine-1-carboxylate (0.35 g, 0.55 mmol) in DCM (2 mL) was added TFA (2 mL) at room temperature. The reaction solution was stirred for 1 h at room temperature. The resulting solution was concentrated under reduced pressure. The residue was purified by Prep-TLC, eluted with DCM/MeOH (15/1) to afford (9*H*-fluoren-9-yl)methyl *N*-[[4,5-dichloro-2-(prop-2-en-1-yloxy)phenyl](piperidin-4-yl)methyl]carbamate as an off-white solid (0.16 g, 54%): LCMS (ESI) calc'd for C₃₀H₃₀Cl₂N₂O₃ [M + H]⁺: 537, 539 (3 : 2), found 537, 539 (3 : 2).

### Step d:

To a stirred solution of (9*H*-fluoren-9-yl)methyl *N*-[[4,5-dichloro-2-(prop-2-en-1-yloxy)phenyl](piperidin-4-yl)methyl]carbamate (160 mg, 0.30 mmol) and Et₃N (60 mg, 0.60 mmol) in DCM (2 mL) was added 2,2,2-trifluoroacetic anhydride (0.13 g, 0.60 mmol) at room temperature under nitrogen atmosphere. The reaction solution was stirred for 1 h at room temperature under nitrogen atmosphere. The resulting solution was concentrated under reduced pressure to afford (9*H*-fluoren-9-yl)methyl ((2-(allyloxy)-4,5-dichlorophenyl)(1-(2,2,2-trifluoroacetyl)piperidin-4-yl)methyl)carbamate. The crude product was used in the next step directly without further purification: LCMS (ESI) calc'd for C₃₂H₂₉Cl₂F₃N₂O₄ [M + H]⁺: 633, 635 (3 : 2), found 633, 635 (3 : 2).

### Step e:

To a stirred solution of (9*H*-fluoren-9-yl)methyl ((2-(allyloxy)-4,5-dichlorophenyl)(1-(2,2,2-trifluoroacetyl)piperidin-4-yl)methyl)carbamate (crude) in DCM (2 mL) was added diethylamine (55 mg, 0.76 mmol) at room temperature. The reaction solution was stirred for 16 h at room temperature. The resulting solution was concentrated under reduced pressure. The residue was purified by reverse phase chromatography, eluted with 55% ACN in water to afford 1-(4-[amino[4,5-dichloro-2-(prop-2-en-1-yloxy)phenyl]methyl]piperidin-1-yl)-2,2,2-trifluoroethan-1-one as a brown oil (70 mg, 58% overall two steps): LCMS (ESI) calc'd for C₁₇H₁₉Cl₂F₃N₂O₂ [M + H]⁺: 411, 413 (3 : 2), found 411, 413 (3 : 2).

### Step f:

To a stirred solution of 1-(4-[amino[4,5-dichloro-2-(prop-2-en-1-yloxy)phenyl]methyl]piperidin-1-yl)-2,2,2-trifluoroethan-1-one (70 mg, 0.17 mmol) and Pd(PPh₃)₄ (4 mg, 0.003 mmol) in THF (2 mL) was added NaBH₄ (8 mg, 0.20 mmol) at room temperature under nitrogen atmosphere. The resulting mixture was stirred for 1 h at room temperature under nitrogen atmosphere. The resulting mixture was quenched with water (5 mL) at room temperature and concentrated under reduced pressure. The residue was purified by Prep-HPLC with the following conditions: Column: XBridge C18 OBD Prep Column 100 Å, 10 µm, 19 mm x 250 mm; Mobile Phase A: water (plus 0.05% TFA), Mobile Phase B: ACN; Flow rate: 25 mL/min; Gradient: 36% B to 46% B in 7 min; Detector: UV 254/210 nm; Retention time: 4.90 min. The fractions containing desired product were collected and concentrated under reduced pressure to afford Compound 77 (1-[4-[amino(4,5-dichloro-2-hydroxyphenyl)methyl]piperidin-1-yl]-2,2,2-trifluoroethan-1-one trifluoroacetic acid) as an off-white solid (10 mg, 16%): LCMS (ESI) calc'd for C₁₄H₁₅Cl₂F₃N₂O₂ [M + H]⁺: 371, 372 (3 : 2), found 371, 372 (3 : 2); ¹H NMR (300 MHz, CD₃OD) δ 7.45 (s, 1H), 7.00 (s, 1H), 5.08 (d, *J* = 10.3 Hz, 1H), 3.54-3.43 (m, 2H), 3.13-2.86 (m, 2H), 2.31-2.05 (m, 2H), 1.63-1.45 (m, 3H).

### Example 144. Compound 79 ((4-((S)-amino(4,5-dichloro-2-hydroxyphenyl)methyl)piperidin-1-yl)((S)-pyrrolidin-3-yl)methanone) and Compound 85 ((4-((R)-amino(4,5-dichloro-2-hydroxyphenyl)methyl)piperidin-1-yl)((S)-pyrrolidin-3-yl)methanone)

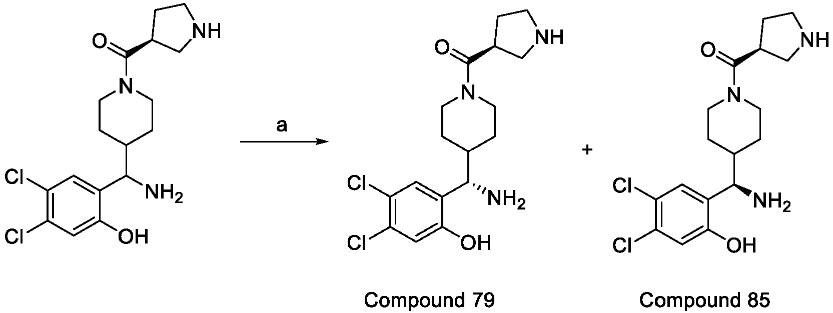

### Step a:

(4-(Amino(4,5-dichloro-2-hydroxyphenyl)methyl)piperidin-1-yl)((*S*)-pyrrolidin-3-yl)methanone (Compound 63's free base, Example 131) (50 mg) was separated by Chiral-Prep-HPLC with the following conditions: Column: CHIRALPAK IG UL001, 20 x 250 mm, 5 µm; Mobile Phase A: Hexane (0.3% IPA), Mobile Phase B: EtOH; Flow rate: 20 mL/min; Gradient: 40% B to 40% B in 22 min; Detector: UV 254/220 nm; Retention time: RT₁: 14.492 min; RT₂: 19.856 min; Injection Volume: 0.6 ml.

The faster-eluting enantiomer was obtained as Compound 79 ((4-((S)-amino(4,5-dichloro-2-hydroxyphenyl)methyl)piperidin-1-yl)((S)-pyrrolidin-3-yl)methanone) as an off-white solid (26.1 mg, 52%) at 14.492 min: LCMS (ESI) calc'd for C₁₇H₂₃Cl₂N₃O₂ [M + H]⁺: 372, 374 (3 : 2), found 372, 374 (3 : 2); ¹H NMR (300 MHz, CD₃OD) δ 7.21 (d, *J* = 3.8 Hz, 1H), 6.86 (d, *J* = 2.4 Hz, 1H), 4.56 (dd, *J* = 28.0, 13.2 Hz, 1H), 4.10 (dd, *J* = 28.9, 13.7 Hz, 1H), 3.96-3.83 (m, 1H), 3.30-3.25 (m, 1H), 3.19-2.83 (m, 5H), 2.70-2.49 (m, 1H), 2.15-1.82 (m, 4H), 1.61-1.06 (m, 3H).

The slower-eluting enantiomer was obtained as Compound 85 ((4-((*R*)-amino(4,5-dichloro-2-hydroxyphenyl)methyl)piperidin-1-yl)((*S*)-pyrrolidin-3-yl)methanone) as an off-white solid (8.2 mg, 16%) at 19.856 min: LCMS (ESI) calc'd for C₁₇H₂₃Cl₂N₃O₂ [M + H]⁺: 372, 374 (3 : 2), found 372, 374 (3 : 2); ¹H NMR (300 MHz, CD₃OD) δ 7.21 (d, *J =* 3.8 Hz, 1H), 6.86 (d, *J* = 2.4 Hz, 1H), 4.56 (dd, *J* = 28.0, 13.2 Hz, 1H), 4.10 (dd, *J* = 28.9, 13.7 Hz, 1H), 3.96-3.83 (m, 1H), 3.30-3.25 (m, 1H), 3.19-2.83 (m, 5H), 2.70-2.49 (m, 1H), 2.15-1.82 (m, 4H), 1.61-1.06 (m, 3H).

### Example 145. Compound 83 (2-[(R)-amino[1-(azetidine-3-carbonyl)piperidin-4-yl]methyl]-4,5-dichlorophenol trifluoroacetic acid)

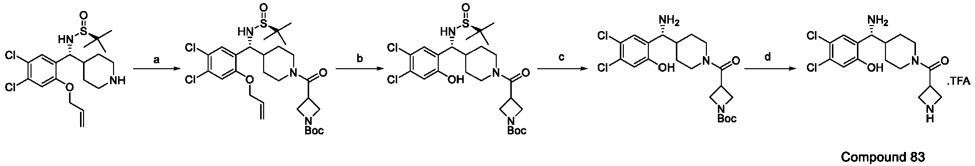

### Step a:

To a stirred solution of (*S*)-N-((*R*)-(2-(prop-2-en-1-yloxy)-4,5-dichlorophenyl)(piperidin-4-yl)methyl)-2-methylpropane-2-sulfinamide (Intermediate 6, Example 6) (0.50 g, 1.19 mmol) and 1-[(*tert*-butoxy)carbonyl]azetidine-3-carboxylic acid (0.72 g, 3.58 mmol) in DMF (5 mL) were added Et₃N (0.37 g, 3.66 mmol) and EDCI (0.68 g, 3.54 mmol) in portions at room temperature under nitrogen atmosphere. The resulting mixture was stirred for 1 h at room temperature under nitrogen atmosphere. The reaction was quenched with water (50 mL) at room temperature and extracted with EA (3 x 50 mL). The combined organic layers were washed with brine (3 x 30 mL), dried over anhydrous Na₂SO₄. After filtration, the filtrate was concentrated under reduced pressure. The residue was purified by Prep-TLC, eluted with PE/EA (5/1) to afford *tert*-butyl 3-(4-((*R*)-(2-(prop-2-en-1-yloxy)-4,5-dichlorophenyl)((*S*)-1,1-dimethylethylsulfinamido)methyl)piperidine-1-carbonyl)azetidine-1-carboxylate as a yellow solid (0.39 g, 54%): LCMS (ESI) calc'd for C₂₈H₄₁Cl₂N₃O₅S [M + H]⁺: 602, 604 (3 : 2), found 602, 604 (3 : 2); ¹H NMR (400 MHz, CD₃OD) δ 7.45 (d, *J =* 1.9 Hz, 1H), 7.18 (d, *J* = 1.6 Hz, 1H), 6.17-6.03 (m, 1H), 5.50-5.40 (m, 1H), 5.39-5.30 (m, 1H), 4.68-4.55 (m, 2H), 4.46 (d, *J* = 13.2 Hz, 1H), 4.13-4.03 (m, 5H), 3.80-3.54 (m, 2H), 3.10-2.90 (m, 1H), 2.72-2.53 (m, 1H), 2.24-2.13 (m, 1H), 2.08-2.01 (m, 1H), 1.41 (s, 9H), 1.40-1.17 (m, 3H), 1.14 (d, *J* = 2.0 Hz, 9H).

### Step b:

To a stirred solution of tert-butyl 3-(4-((R)-(2-(prop-2-en-1-yloxy)-4,5-dichlorophenyl)((*S*)-1,1-dimethylethylsulfinamido)methyl)piperidine-1-carbonyl)azetidine-1-carboxylate (0.39 g, 0.65 mmol) and Pd(PPh₃)₄ (15 mg, 0.01 mmol) in THF (8 mL) was added NaBH₄ (37 mg, 0.97 mmol) at room temperature under nitrogen atmosphere. The resulting mixture was stirred for 40 min at room temperature under nitrogen. The reaction was quenched with water (20 mL) and extracted with EA (3 x 30 mL). The combined organic layers were washed with brine (2 x 20 mL), dried over anhydrous Na₂SO₄ and filtered. The filtrate was concentrated under reduced pressure to afford *tert*-butyl 3-(4-((*R*)-(4,5-dichloro-2-hydroxyphenyl)((*S*)-1,1-dimethylethylsulfinamido)methyl)piperidine-1-carbonyl)azetidine-1-carboxylate as a dark yellow solid (0.34 g, crude), which was used in next step directly without further purification: LCMS (ESI) calc'd for C₂₅H₃₇Cl₂N₃O₅S [M + H]⁺: 562, 564 (3 : 2), found 562, 564 (3 : 2).

### Step c:

To a stirred solution of *tert*-butyl 3-(4-((*R*)-(4,5-dichloro-2-hydroxyphenyl)((S)-1,1-dimethylethylsulfinamido)methyl)piperidine-1-carbonyl)azetidine-1-carboxylate (0.34 g, 0.65 mmol) in 1,4-dioxane (3 mL) was added aq. HCl (4 *N,* 3 mL) at room temperature. The reaction solution was stirred for 1 h at room temperature. The resulting solution was concentrated under reduced pressure to afford crude *tert*-butyl 3-[4-[(*R*)-amino(4,5-dichloro-2-hydroxyphenyl)methyl]piperidine-1-carbonyl]azetidine-1-carboxylate as a dark yellow solid, which was directly used in next step without further purification: LCMS (ESI) calc'd for C₂₁H₂₉Cl₂N₃O₄ [M + H]⁺: 458, 460 (3 : 2), found 458, 460 (3 : 2).

### Step d:

To a stirred solution of *tert*-butyl 3-[4-[(*R*)-amino(4,5-dichloro-2-hydroxyphenyl)methyl]piperidine-1-carbonyl]azetidine-1-carboxylate (crude) in DCM (3 mL) was added TFA (1 mL) at room temperature. The reaction solution was stirred at room temperature for 30 min. The resulting solution was concentrated under reduced pressure. The residue was purified by Prep-HPLC with the following conditions: Column: X Bridge C18 OBD Prep Column 100 Å, 10 µm, 19 mm x 250 mm; Mobile Phase A: water (plus 0.05% TFA), Mobile Phase B: ACN; Flow rate: 25 mL/min; Gradient: 2% B to 30% B in 6 min; Detector: UV 254/210 nm; Retention time: 4.94 min. The fractions containing desired product were collected and concentrated under reduced pressure to afford Compound 83 (2-[(*R*)-amino[1-(azetidine-3-carbonyl)piperidin-4-yl]methyl]-4,5-dichlorophenol trifluoroacetic acid) as an off-white solid (0.11 g, 36% overall three steps): LCMS (ESI) calc'd for C₁₆H₂₁Cl₂N₃O₂ [M + H]⁺: 358, 360 (3 : 2), found 358, 360 (3 : 2); ¹H NMR (400 MHz, CD₃OD) δ 7.22 (d, *J* = 6.0 Hz, 1H), 6.86 (d, *J* = 4.0 Hz, 1H), 4.54 (dd, *J* = 38.1, 13.4 Hz, 1H), 4.39-3.95 (m, 6H), 3.62 (dd, *J* = 37.6, 13.8 Hz, 1H), 3.10-2.86 (m, 1H), 2.42-2.28 (m, 1H), 2.16-1.91 (m, 1H), 1.56-1.41 (m, 2H), 1.31-1.06 (m, 1H); ¹⁹F NMR (376 MHz, CD₃OD) δ -77.02.

The compounds in the Table 1a below were prepared in an analogous fashion to that described for Compound 83, starting from (*S*)-*N*-((*R*)-(2-(allyloxy)-4,5-dichlorophenyl)(piperidin-4-yl)methyl)-2-methylpropane-2-sulfinamide and the corresponding acids, which were prepared as described herein, or which were available from commercial sources.

**Table 1a**

| **Compound Number** | **Structure** | **Chemical Name** | **MS & NMR** |
|---|---|---|---|
| **88** | 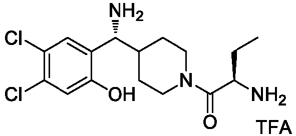 | (*R*)-2-amino-1-(4-((*R*)-amino(4,5-dichloro-2-hydroxyphenyl)methyl )piperidin-1-yl)butan-1-one trifluoroacetic acid | [M + H]⁺: 360, 362 (3 : 2), found 360, 362 (3 : 2); ¹H NMR (400 MHz, CD₃OD) δ 7.44 (d, *J* = 4.1 Hz, 1H), 7.09 (s, 1H), 4.58 (dd, *J* = 58.3, 13.6 Hz, 1H), 4.41-4.32 (m, 1H), 4.25-4.15 (m, 1H), 3.95 (dd, *J* = 52.0, 14.0 Hz, 1H), 3.28-3.05 (m, 1H), 2.85-2.62 (m, 1H), 2.49-2.34 (m, 1H), 2.20-1.74 (m, 3H), 1.51-1.11 (m, 3H), 1.04 (dt, *J* = 22.6, 7.5 Hz, 3H); ¹⁹F NMR (376 MHz, CD₃OD) δ -76.91. |
| **89** | 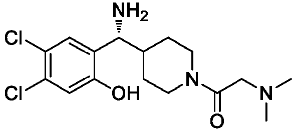 | (*R*)-1-(4-(amino(4,5-dichloro-2-hydroxyphenyl)methyl )piperidin-1-yl)-2-(dimethylamino)ethan one | [M + H]⁺: 360, 362 (3 : 2); ¹H NMR (400 MHz, CD₃OD) δ 7.21 (d, *J* = 3.6 Hz, 1H), 6.86 (s, 1H), 4.53 (dd, *J* = 38.2, 13.3 Hz 1H), 4.07 (dd, *J* = 40.2, 13.7 Hz, 1H), 3.88 (t, *J* = 7.9 Hz, 1H), 3.28-3.08 (m, 2H), 3.08-2.90 (m, 1H), 2.70-2.51 (m, 1H), 2.26 (s, 6H), 2.08-1.93 (m, 2H), 1.53-1.40 (d, *J* = 13.5 Hz, 1H), 1.39-1.10 (m, 2H). |
| **90** | 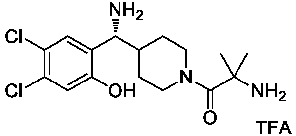 | (*R*)-2-amino-1-(4-(amino(4,5-dichloro-2-hydroxyphenyl)methyl )piperidin-1-yl)-2-methylpropan-1-one trifluoroacetic acid | [M + H]⁺: 360, 362 (3 : 2), found 360, 362 (3 : 2); ¹H NMR (400 MHz, CD₃OD) δ 7.21 (s, 1H), 6.86 (s, 1H), 4.60 (d*, J =* 13.3 Hz, 1H), 4.50 *(d, J* = 13.5 Hz, 1H), 3.89 (d, *J* = 7.6 Hz, 1H), 2.91-2.70 (m, 2H), 2.08-1.95 (m, 2H), 1.53-1.46 (m, 1H), 1.43 (s, 6H), 1.30-1.16 (m, 2H), ¹⁹F NMR (376 MHz, CD₃OD) δ -77.21. |
| **91** | 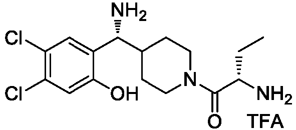 | (*S*)-2-amino-1-(4-((*R*)-amino(4,5-dichloro-2-hydroxyphenyl)methyl )piperidin-1-yl)butan-1-one trifluoroacetic acid | [M + H]⁺: 360, 362 (3 : 2); ¹H NMR (400 MHz, CD₃OD) δ 7.44 (d, *J* = 11.6 Hz, 1H), 7.09 (d, *J* = 3.8 Hz, 1H), 4.58 (dd, *J* = 46.3, 13.7 Hz, 1H), 4.44-4.29 (m, 1H), 4.26-4.15 (m, 1H), 3.95 (dd, *J* = 57.5, 13.8 Hz, 1H), 3.27-3.03 (m, 1H), 2.84-2.59 (m, 1H), 2.49-2.33 (m, 1H), 2. 07 (d, *J* = 12.9 Hz, 1H), 1.98-1.74 (m, 2H), 1.53-1.12 (m, 3H), 1.10-0.96 (q, *J* = 7.2 Hz, 3H); ¹⁹F NMR (376 MHz, CD₃OD) δ -76.88. |
| **93** | 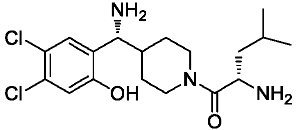 | (*S*)-2-amino-1-(4-((*R*)-amino(4,5-dichloro-2-hydroxyphenyl)methyl )piperidin-1-yl)-4-methylpentan-1-one | [M + H]⁺: 388, 390 (3 : 2); ¹H NMR (400 MHz, CD₃OD) δ 7.22 (d*, J* = 4.2 Hz, 1H), 6.87 (s, 1H), 4.57 (dd, *J* = 36.2, 13.3 Hz, 1H), 4.09-3.78 (m, 3H), 3.18-2.97 (m, 1H), 2.73-2.48 (m, 1H), 2.12-1.94 (m, 2H), 1.85-1.65 (m, 1H), 1.62-1.45 (m, 1H), 1.44-1.31 (m, 2H), 1.27-1.09 (m, 2H), 1.01-0.88 (m, 6H). |
| **94** | 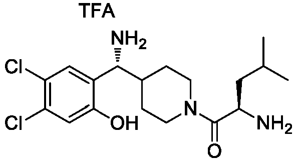 | (*R*)-2-amino-1-(4-((*R*)-amino(4,5-dichloro-2-hydroxyphenyl)methyl )piperidin-1-yl)-4-methylpentan-1-one trifluoroacetic acid | [M + H]⁺: 388, 390 (3 : 2); ¹H NMR (400 MHz, CD₃OD) δ 7.21 (d, *J* = 5.8 Hz, 1H), 6.86 (s, 1H), 4.56 (dd, *J =* 33.9, 13.4 Hz, 1H), 4.08-3.81 (m, 3H), 3.16-3.00 (m, 1H), 2.68-2.53 (m, 1H), 2.10-1.93 (m, 2H), 1.77-1.67 (m, 1H), 1.52-1.47 (m, 1H), 1.57-1.12 (m, 4H), 1.02-0.85 (m, 6H); ¹⁹F NMR (376 MHz, CD₃OD) δ -77.04. |
| **95** | 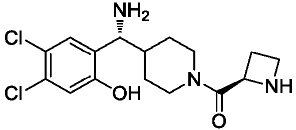 | (4-((*R*)-amino(4,5-dichloro-2-hydroxyphenyl)methyl )piperidin-1-yl)((*R*)-azetidin-2-yl)methanone | [M + H]⁺: 358, 360 (3 : 2); ¹H NMR (400 MHz, CD₃OD) δ 7.23 (d, *J* = 9.5 Hz, 1H), 6.88 (d, *J* = 5.8 Hz, 1H), 4.66-4.45 (m, 2H), 3.96-3.86 (m, 1H), 3.65-3.47 (m, 3H), 3.05-2.57 (m, 3H), 2.50-2.29 (m, 1H), 2.10-1.94 (m, 2H), 1.56-1.43 (m, 1H), 1.34-1.14 (m, 2H). |
| **96** | 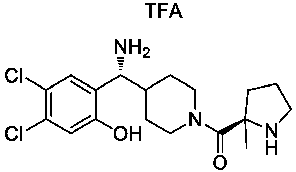 | (4-((*R*)-amino(4,5-dichloro-2-hydroxyphenyl)methyl )piperidin-1-yl)((*R*)-2-methylpyrrolidin-2-yl)methanone trifluoroacetic acid | [M + H]⁺: 386, 388 (3: 2),¹H NMR (400 MHz, CD₃OD) δ 7.42 (s, 1H), 7.07 (s, 1H), 4.76-4.36 (m, 1H), 4.20 (d, *J =* 8.0 Hz, 1H), 4.16-3.82 (m, 1H), 3..30-2.99 (m, 3H), 2.99-2.57 (m, 1H), 2.51-2.00 (m, 6H), 1.70 (s, 3H), 1.51-1.30 (m, 2H), 1.31-1.14 (m, 1H), ¹⁹F NMR (376 MHz, CD₃OD) δ -76.93. |
| **97** | 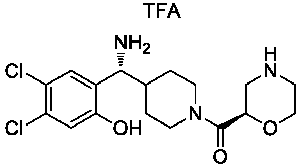 | (4-((*R*)-amino(4,5-dichloro-2-hydroxyphenyl)methyl )piperidin-1-yl)((*R*)-morpholin-2-yl)methanone trifluoroacetic acid | [M + H]⁺: 388, 390 (3 : 2); ¹H NMR (400 MHz, CD₃OD) δ 7.21 (d, *J* = 3.5 Hz, 1H), 6.87 (s, 1H), 4.51 (dd, *J =* 41.4, 13.3 Hz, 1H), 4.34-4.22 (m, 1H), 4.08 (dd, *J* = 40.6, 13.7 Hz, 1H), 3.95-3.78 (m, 2H), 3.71-3.57 (m, 1H), 3.16-2.76 (m, 5H), 2.70-2.53 (m, 1H), 2.11-1.92 (m, 2H), 1.54 (d, *J* = 13.3 Hz, 1H), 1.36-1.09 (m, 2H). |
| **98** | 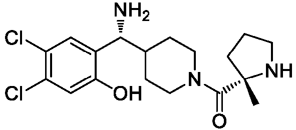 | (4-((*R*)-amino(4,5-dichloro-2-hydroxyphenyl)methyl )piperidin-1-yl)((*S*)-2-methylpyrrolidin-2-yl)methanone | [M + H]⁺: 386, 388 (3: 2);¹H NMR (400 MHz, CD₃OD) δ 7.42 (s, 1H), 7.07 (s, 1H), 4.76-4.06 (m, 2H), 3.89 (d, *J* = 8.0 Hz, 1H), 2.99-2.68 (m, 4H), 2.20-1.60 (m, 6H), 1.50-1.40 (m, 1H), 1.38 (s, 3H), 1.20-1.10 (m, 2H). |
| **99** | 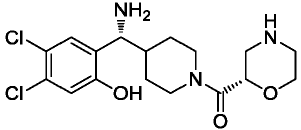 | (4-((*R*)-amino(4,5-dichloro-2-hydroxyphenyl)methyl )piperidin-1-yl)((*S*)-morpholin-2-yl)methanone | [M + H]⁺: 388, 390 (3 : 2); ¹H NMR (400 MHz, CD₃OD) δ 7.21 (s, 1H), 6.86 (s, 1H), 4.52 (dd, *J* = 33.7, 13.3 Hz, 1H), 4.34-4.21 *(d, J* = 8.5 Hz, 1H), 4.10 (dd, *J* = 35.1, 13.6 Hz, 1H), 3.95-3.78 (m, 2H), 3.73-3.60 (m, 1H), 3.09-2.97 (m, 1H), 2.96-2.73 (m, 4H), 2.70-2.47 (m, 1H), 1.99 (d, *J =* 13.6 Hz, 2H), 1.58-1.08 (m, 3H). |
| **103** | 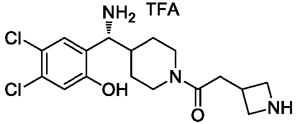 | (*R*)-1-(4-(amino(4,5-dichloro-2-hydroxyphenyl)methyl )piperidin-1-yl)-2-(azetidin-3-yl)ethanone trifluoroacetic acid | [M + H]⁺: 372, 374 (3 : 2); ¹H NMR (400 MHz, CD₃OD) δ 7.44 (d, *J* = 3.2 Hz, 1H), 7.08 (d, *J* = 2.5 Hz, 1H), 4.67-4.46 (m, 1H), 4.23-4.09 (m, 3H), 4.05-3.82 (m, 3H), 3.30-2.95 (m, 2H), 2.90-2.75 (m, 2H), 2.70-2.50 (m, 1H), 2.42-2.25 (m, 1H), 2.11-1.91 (m, 1H), 1.50-1.00 (m, 3H); ¹⁹F NMR (376 MHz, CD₃OD) δ -76.93. |
| **109** | 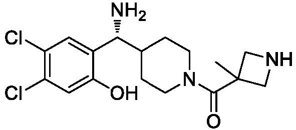 | (*R*)-(4-(amino(4,5-dichloro-2-hydroxyphenyl)methyl )piperidin-1-yl)(3-methylazetidin-3-yl)methanone | [M + H]⁺: 372, 374 (3 : 2); ¹H NMR (400 MHz, CD₃OD) δ 7.23 (s, 1H), 6.89 (s, 1H), 4.52 (dd, *J =* 37.7, 13.1 Hz, 1H), 4.10 (d, *J* = 9.0 Hz, 2H), 3.98-3.87 (m, 1H), 3.44 (d, *J =* 8.5 Hz, 2H), 3.34-3.29 (m, 1H), 3.14-2.95 (m, 1H), 2.70-2.49 (m, 1H), 2.11-1.91 (d, *J =* 11.3 Hz, 2H), 1.60 (d, *J* = 8.4 Hz, 3H), 1.49 (d, *J =* 13.6 Hz, 1H), 1.37-0.95 (m, 2H). |
| **113** | 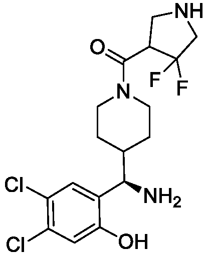 | (4-((R)-amino(4,5-dichloro-2-hydroxyphenyl)methyl )piperidin-1-yl)(4,4-difluoropyrrolidin-3-yl)methanone | [M + H]⁺: 408, 410 (3 : 2); ¹H NMR (400 MHz, CD₃OD) δ 7.22 (d, *J* = 4.1 Hz, 1H), 6.87 (d, *J* = 2.7 Hz, 1H), 4.71-4.47 (m, 1H), 4.22-4.06 (m, 1H), 3.94-3.81 (m, 1H), 3.79-3.63 (m, 1H), 3.58-3.42 (m, 1H), 3.30-3.00 (m, 4H), 2.71-2.42 (m, 1H), 2.13-1.86 (m, 2H), 1.54-1.41 (m, 1H), 1.37-1.08 (m, 2H), ¹⁹F NMR (376 MHz, CD₃OD) δ -95.44 - -97.21, - 104.80- -106.91. |
| **117** | 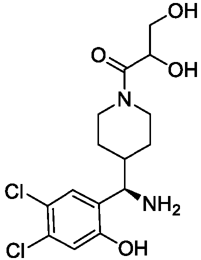 | 1-(4-((*R*)-amino(4,5-dichloro-2-hydroxyphenyl)methyl )piperidin-1-yl)-2,3-dihydroxypropan-1-one | [M + H]⁺: 363, 365 (3 : 2); ¹H NMR (400 MHz, CD₃OD) δ 7.22 (s, 1H), 6.87 (s, 1H), 4.68-4.47 (m, 2H), 4.11 ((dd, *J* = 40.3, 13.8 Hz, 1H), 3.94-3.86 (m, 1H), 3.76-3.53 (m, 2H), 3.20-2.94 (m, 1H), 2.74-2.55 (m, 1H), 2.16-1.92 (m, 2H), 1.57-1.43 (m, 1H), 1.41-1.13 (m, 2H). |

### Example 146. Compound 80 ((4-((R)-amino(4,5-dichloro-2-hydroxyphenyl)methyl)piperidin-1-yl)((R)-pyrrolidin-3-yl)methanone); Compound 84 ((4-((S)-amino(4,5-dichloro-2-hydroxyphenyl)methyl) piperidin-1-yl)((R)-pyrrolidin-3-yl)methanone)

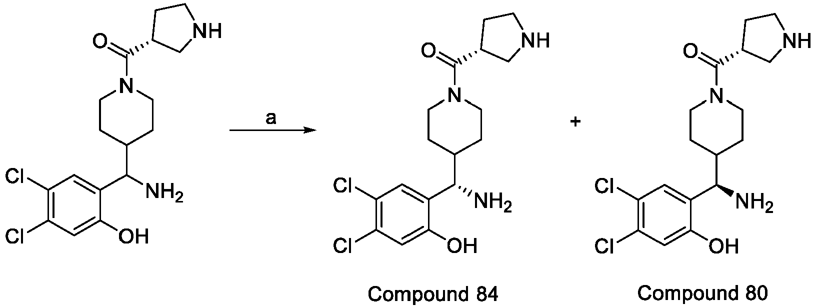

### Step a:

(4-(Amino(4,5-dichloro-2-hydroxyphenyl)methyl)piperidin-1-yl)((*R*)-pyrrolidin-3-yl)methanone (Compound 66's free base, Example 134) (120 mg) was separated by Chiral-Prep-HPLC with the following conditions: Column: CHIRALPAK IG UL001, 20 x 250 mm, 5 µm; Mobile Phase A: Hexane (0.1%IPA), Mobile Phase B: EtOH; Flow rate: 16 mL/min; Gradient: 50% B to 50% B in 20 min; Detector: UV 254/220 nm; Retention time: RT₁: 11.704 min; RT₂: 17.496 min; Injection Volume: 1 mL.

The faster-eluting enantiomer was obtained as Compound 80 ((4-((*R*)-amino(4,5-dichloro-2-hydroxyphenyl)methyl)piperidin-1-yl)((*R*)-pyrrolidin-3-yl)methanone) as an off-white solid (35.8 mg, 30%) at 14.492 min: LCMS (ESI) calc'd for C₁₇H₂₃Cl₂N₃O₂ [M + H]⁺: 372, 374 (3 : 2), found 372, 374 (3 : 2); ¹H NMR (300 MHz, CD₃OD) δ 7.22 (d, *J* = 4.4 Hz, 1H), 6.86 (d, *J =* 2.8 Hz, 1H), 4.56 (dd, *J =* 27.3, 12.2 Hz, 1H), 4.11 (dd, *J =* 13.6, 28.5 Hz, 1H), 3.94-3.86 (m, 1H), 3.30-3.25 (m, 1H), 3.19-2.88 (m, 5H), 2.68-2.48 (m, 1H), 2.23-1.85 (m, 4H), 1.54-1.06 (m, 3H).

The slower-eluting enantiomer was obtained as Compound 84 ((4-((*S*)-amino(4,5-dichloro-2-hydroxyphenyl)methyl)piperidin-1-yl)((R)-pyrrolidin-3-yl)methanone) as an off-white solid (37.4 mg, 31%) at 19.856 min: LCMS (ESI) calc'd for C₁₇H₂₃Cl₂N₃O₂ [M + H]⁺: 372, 374 (3 : 2), found 372, 374 (3 : 2); ¹H NMR (300 MHz, CD₃OD) δ 7.22 (d, *J =* 4.4 Hz, 1H), 6.86 (d, *J =* 2.8 Hz, 1H), 4.56 (dd, *J =* 27.3, 12.2 Hz, 1H), 4.11 (dd, *J =* 13.6, 28.5 Hz, 1H), 3.94-3.86 (m, 1H), 3.30-3.25 (m, 1H), 3.19-2.88 (m, 5H), 2.68-2.48 (m, 1H), 2.23-1.85 (m, 4H), 1.54-1.06 (m, 3H).

### Example 147. (which is a reference example) Compound 86 (2-[amino(piperidin-4-yl)methyl]-3,4-dichlorophenol bis(trifluoroacetic acid)

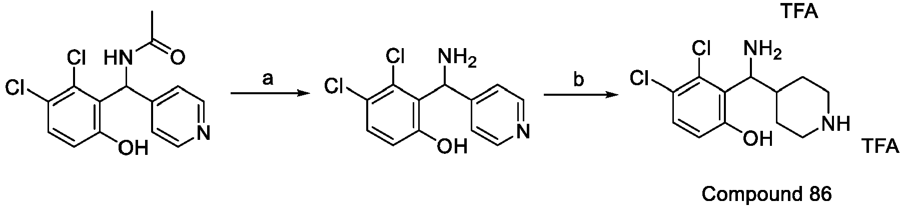

### Step a:

A mixture of*N*-[(2,3-dichloro-6-hydroxyphenyl)(pyridin-4-yl)methyl]acetamide (Intermediate 64, Example 64) (0.20 g, 0.65 mmol) in aq. HCl (6 *N,* 6 mL) was stirred for 30 min at 100 °C. After cooling to room temperature, the resulting mixture was concentrated under reduced pressure to afford 2-[amino(pyridin-4-yl)methyl]-3,4-dichlorophenol as a light yellow solid (0.16 g, crude), which was used in the next step directly without further purification. LCMS (ESI) calc'd for C₁₂H₁₀Cl₂N₂O [M + H]⁺: 269, 271 (3 : 2), found 269, 271 (3 : 2).

### Step b:

To a solution of 2-[amino(pyridin-4-yl)methyl]-3,4-dichlorophenol (0.16 g, 0.60 mmol) in MeOH (7 mL) was added PtO₂ (30 mg, 0.13 mmol) at room temperature. The mixture was stirred at 30 °C under hydrogen atmosphere (50 atm) for 5 h. The reaction was filtered, and the filtrate was concentrated under reduced pressure. The residue was purified by Prep-HPLC with the following conditions: Column: XBridge C18 OBD Prep Column 100 mm, 10 µm, 19 mm x 250 mm; Mobile Phase A: water (plus 0.05% TFA), Mobile Phase B: ACN; Flow rate: 25 mL/min; Gradient: 8% B to 38% B in 6.5 min; Detector: UV 254/210 nm; Retention time: 5.18 min. The fractions containing the desired product were collected and concentrated under reduced pressure to afford Compound 86 (2-[amino(piperidin-4-yl)methyl]-3,4-dichlorophenol bis(trifluoroacetic acid)) as a purple solid (25 mg, 8% overall two steps): LCMS (ESI) calc'd for C₁₂H₁₆Cl₂N₂O [M + H]⁺: 275, 277 (3 : 2), found 275, 277 (3 : 2); ¹H NMR (400 MHz, CD₃OD) δ 7.49 (dd, *J =* 8.9, 1.3 Hz, 1H), 6.95 (d, *J =* 8.9 Hz, 1H), 4.73 (d, *J =* 10.0 Hz, 1H), 3.55 (d, *J =* 13.0 Hz, 1H), 3.39 (d, *J =* 13.0 Hz, 1H), 3.06 (td, *J =* 13.1, 3.1 Hz, 1H), 2.94 (td, *J =* 12.4, 4.5 Hz, 1H), 2.80-2.63 (m, 1H), 2.33-2.22 (m, 1H), 1.76-1.63 (m, 1H), 1.63-1.46 (m, 2H); ¹⁹F NMR (376 MHz, CD₃OD) δ -77.00 (d, *J =* 3.0 Hz).

### Example 148. Compound 92 (2-amino-1-[4-[(R)-amino(4,5-dichloro-2-hydroxyphenyl)methyl]piperidin-1-yl]ethan-1-one trifluoroacetic acid)

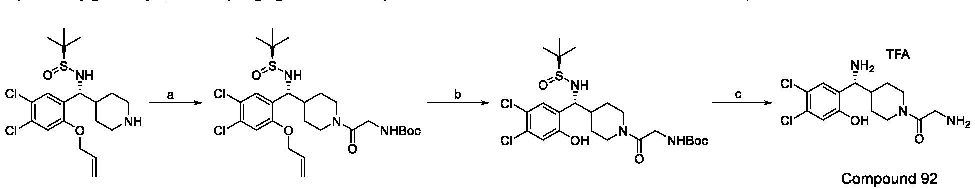

### Step a:

To a stirred solution of 2-((tert-butoxycarbonyl)amino)acetic acid (0.30 g, 1.74 mmol) and HATU (0.66 g, 1.74 mmol) in DMF (5 mL) were added (*S*)-N-((*R*)-(2-(allyloxy)-4,5-dichlorophenyl)(piperidin-4-yl)methyl)-2-methylpropane-2-sulfinamide (0.25 g, 0.58 mmol) and Et₃N (0.18 g, 1.74 mmol) at room temperature under nitrogen atmosphere. The reaction solution was stirred for 3 h at room temperature under nitrogen atmosphere. The resulting solution was quenched with water (40 mL) and extracted with EA (3 x 50 mL). The combined organic layers were washed with water (2 x 20 mL), dried over anhydrous Na₂SO₄. After the filtration, the filtrate was concentrated under reduced pressure. The residue was purified by reverse phase chromatography, eluted with 70% ACN in water with 10 mmol/L NH₄HCO₃ to afford tert-butyl (2-(4-((*R*)-(2-(allyloxy)-4,5-dichlorophenyl)((*S*)-1,1-dimethylethylsulfinamido)methyl)piperidin-1-yl)-2-oxoethyl)carbamate as a yellow oil (0.19 g, 54%): LCMS (ESI) calc'd for C₂₆H₃₉Cl₂N₃O₅S [M + H]⁺: 576, 578 (3 : 2), found 576, 578 (3 : 2); ¹H NMR (300 MHz, CDCl₃) δ 7.22 (d, *J =* 5.1 Hz, 1H), 6.98 (s, 1H), 6.13-6.01 (m, 1H), 5.54-5.29 (m, 4H), 4.65-4.52 (m, 3H), 4.49 (s, 2H), 3.94-3.80 (m, 2H), 3.08-2.82 (m, 1H), 2.64-2.39 (m, 1H), 2.14-1.95 (m, 2H), 1.57-1.19 (m, 3H), 1.47 (s, 9H), 1.16 (s, 9H).

### Step b:

To a stirred solution of tert-butyl (2-(4-((R)-(2-(allyloxy)-4,5-dichlorophenyl)((S)-1,1-dimethylethylsulfinamido)methyl)piperidin-1-yl)-2-oxoethyl)carbamate (0.19 g, 0.33 mmol) and Pd(PPh₃)₄ (33 mg, 0.03 mmol) in THF (5 mL) was added NaBH₄ (19 mg, 0.50 mmol) at room temperature under nitrogen atmosphere. The reaction mixture was stirred for 1 h at room temperature under nitrogen. The resulting mixture was quenched with water (50 mL) at room temperature and extracted with EA (3 x 50 mL). The combined organic layers were washed with brine (3 x 30 mL), dried over anhydrous Na₂SO₄. After filtration, the filtrate was concentrated under reduced pressure to afford crude *tert*-butyl (2-(4-((R)-(4,5-dichloro-2-hydroxyphenyl)((*S*)-1,1-dimethylethylsulfinamido)methyl)piperidin-1-yl)-2-oxoethyl)carbamate. The crude product was used in the next step directly without further purification: LCMS (ESI) calc'd for C₂₃H₃₅Cl₂N₃O₅S [M + H]⁺: 536, 538 (3 : 2), found 536, 538 (3 : 2).

### Step c:

To a stirred solution of tert-butyl (2-(4-((*R*)-(4,5-dichloro-2-hydroxyphenyl)((*S*)-1,1-dimethylethylsulfinamido)methyl)piperidin-1-yl)-2-oxoethyl)carbamate (crude) in 1,4-dioxane (5 mL) was added aq. HCl (6 *N,* 3 mL) at room temperature. The reaction solution was stirred for 1 h at room temperature. The resulting solution was concentrated under reduced pressure. The residue was dissolved in DCM (6 mL), and then TFA (3 mL) was added at room temperature. The reaction solution was stirred for additional 1 h at room temperature. The resulting solution was concentrated under reduced pressure. The residue was purified by Prep-HPLC with the following conditions: Column: SunFire C18 OBD Prep Column, 100 Å, 5 µm, 19 mm x 250 mm; Mobile Phase A: water (plus 0.05% TFA), Mobile Phase B: ACN; Flow rate: 25 mL/min; Gradient: 10% B to 13% B in 6 min; Detector: UV 210 nm; Retention time: 5.57 min. The fractions containing desired product were collected and concentrated under reduce pressure to afford Compound 92 (2-amino-1-[4-[(R)-amino(4,5-dichloro-2-hydroxyphenyl)methyl]piperidin-1-yl]ethan-1-one trifluoroacetic acid) as an off-white solid (20 mg, 14% overall two steps): LCMS (ESI) calc'd for C₁₄H₁₉Cl₂N₃O₂ [M + H]⁺: 332, 334 (3 : 2), found 332, 334 (3 : 2); ¹H NMR (400 MHz, CD₃OD) δ 7.20 (s, 1H), 6.86 (s, 1H), 4.55 (dd, *J =* 35.7, 13.5 Hz, 1H), 3.93-3.74 (m, 2H), 3.45 (dd, *J =* 10.9, 3.7 Hz, 2H), 3.08-2.85 (m, 1H), 2.70-2.56 (m, 1H), 2.10-1.94 (m, 2H), 1.58-1.46 (m, 1H), 1.34-1.10 (m, 2H); ¹⁹F NMR (376 MHz, CD₃OD) δ -77.04.

The compounds in Table 1b below were prepared in an analogous fashion to that described for Compound 92, starting from (S)-N-[(R)-[4,5-dichloro-2-(prop-2-en-1-yloxy)phenyl](piperidin-4-yl)methyl]-2-methylpropane-2-sulfinamide and the corresponding acids, which were prepared as described herein, or which were available from commercial sources.

**Table 1b**

| **Compound Number** | **Structure** | **Chemical Name** | **MS & NMR** |
|---|---|---|---|
| 100 | 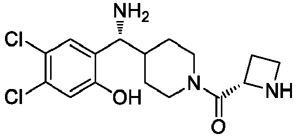 | 2-[(*R*)-amino([1-[(2*S*)-azetidine-2-carbonyl]piperi din-4-yl])methyl]-4,5-dichlorophenol | [M + H]⁺: 358, 360 (3 : 2); ¹H NMR (400 MHz, CD₃OD) δ 7.21 (s, 1H), 6.86 (s, 1H), 4.54 (dd, *J* = 37.5, 13.3 Hz, 2H), 3.89 (t, *J* = 6.7 Hz, 1H), 3.67-3.42 (m, 3H), 3.05-2.85 (m, 1H), 2.85-2.57 (m, 2H), 2.44-2.28 (m, 1H), 1.98 (dd, *J* = 28.7, 12.8 Hz, 2H), 1.56-1.40 (m, 1H), 1.35-1.08 (m, 2H). |
| **131** | 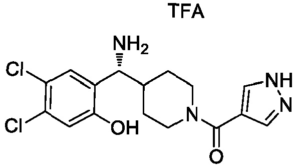 | 2-[(*R*)-amino[1-(1*H-*pyrazole-4-carbonyl)piperi din-4-yl]methyl]-4,5-dichlorophenol trifluoroacetic acid | [M + H]⁺: 369, 371 (3 : 2); ¹H NMR (400 MHz, CD₃OD) δ 7.90 (s, 2H), 7.45 (s, 1H), 7.09 (s, 1H), 4.81-4.25 (m, 2H), 4.18 (d, *J* = 9.9 Hz, 1H), 3.26-2.58 (m, 2H), 2.54-2.33 (m, 1H), 2.16-1.89 (m, 1H), 1.52-1.16 (m, 3H); ¹⁹F NMR (376 MHz, CD₃OD) δ -77.19. |
| **133** | 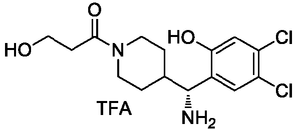 | 1-[4-[(*R*)-amino(4,5-dichloro-2-hydroxyphenyl) methyl]piperidin -1-yl]-3-hydroxypropan -1-one trifluoroacetic acid | [M + H]⁺: 347, 349 (3 : 2); ¹H NMR (400 MHz, CD₃OD) δ 7.43 (d, *J* = 3.0 Hz, 1H), 7.08 (s, 1H), 4.61 (dd, *J* = 54.0, 13.9 Hz, 1H), 4.21-3.96 (m, 2H), 3.82 (t, *J* = 6.3 Hz, 2H), 3.18-2.85 (m, 1H), 2.72-2.50 (m, 3H), 2.45-2.32 (m, 1H), 1.99 (d, *J* = 13.0 Hz, 1H), 1.43-1.11 (m, 3H). |
| **134** | 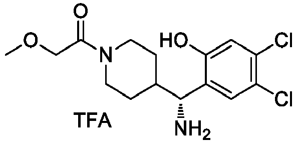 | 1-[4-[(*R*)-amino(4,5-dichloro-2-hydroxyphenyl) methyl]piperidi n-1-yl]-2-methoxyethan-1-one trifluoroacetic acid | [M + H]⁺: 347, 349 (3 : 2); ¹H NMR (400 MHz, CD₃OD) δ 7.47-7.41 (m, 1H), 7.08 (s, 1H), 4.55 (dd, *J* = 55.0, 13.5 Hz, 1H), 4.23-4.08 (m, 3H), 3.92 (dd, *J* = 61.8, 14.0 Hz, 1H), 3.40 (d, *J* = 6.0 Hz, 3H), 3.14-2.94 (m, 1H), 2.64 (dt, *J* = 43.2, 12.9 Hz, 1H), 2.36 (s, 1H), 2.01 (d, *J* = 12.9 Hz, 1H), 1.45-1.10 (m, 3H); ¹⁹F NMR (376 MHz, CD₃OD) δ - 77.10. |
| **135** | 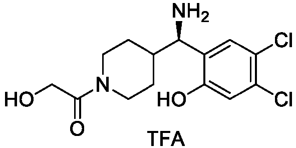 | 1-[4-[(*R*)-amino(4,5-dichloro-2-hydroxyphenyl) methyl]piperidi n-1-yl]-2-hydroxyethan-1-one trifluoroacetic acid | [M + H]⁺: 333, 335 (3 : 2); ¹H NMR (400 MHz, CD₃OD) δ 7.43 (s, 1H), 7.08 (s, 1H), 4.55 (dd, *J* = 54.9, 13.4 Hz, 1H), 4.26-4.11 (m, 3H), 3.80 (dd, *J* = 58.4, 13.8 Hz, 1H), 3.02 (dt, *J* = 46.2, 13.2 Hz, 1H), 2.67 (dt, *J* = 44.0, 12.8 Hz, 1H), 2.36 (s, 1H), 2.00 (d, *J* = 12.9 Hz, 1H), 1.42-1.11 (m, 3H). |
| **136** | 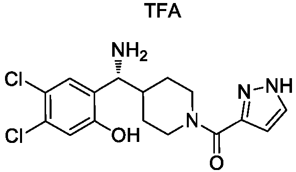 | 2-[(*R*)-amino[1-(1*H-*pyrazole-3-carbonyl)piperi din-4-yl]methyl]-4,5-dichlorophenol trifluoroacetic acid | [M + H]⁺: 369, 371 (3 : 2); ¹H NMR (400 MHz, CD₃OD) δ 7.72 (s, 1H), 7.44 (s, 1H), 7.08 (s, 1H), 6.61 (s, 1H), 4.81-4.45 (m, 2H), 4.18 (d, *J* = 9.9 Hz, 1H), 3.26-2.99 (m, 1H), 2.97-2.71 (m, 1H), 2.49-2.32 (m, 1H), 2.14-1.91 (m, 1H), 1.51-1.19 (m, 3H); ¹⁹F NMR (376 MHz, CD₃OD) δ -77.02. |
| **137** | 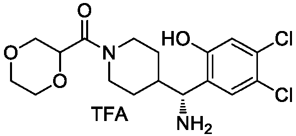 | 2-[(*R*)-amino[1-(1,4-dioxane-2-carbonyl)piperi din-4-yl]methyl]-4,5-dichlorophenol trifluoroacetic acid | [M + H]⁺: 389, 391 (3 : 2); ¹H NMR (400 MHz, CD₃OD) δ 7.43 (s, 1H), 7.08 (s, 1H), 4.64-4.35 (m, 2H), 4.27-4.04 (m, 2H), 3.90-3.65 (m, 6H), 3.07 (dt, *J* = 68.2, 13.3 Hz, 1H), 2.62 (dt, *J* = 29.9, 12.8 Hz, 1H), 2.42-2.31 (m, 1H), 2.01 (t, *J* = 13.9 Hz, 1H), 1.42-1.13 (m, 3H); ¹⁹F NMR (376 MHz, CD₃OD) δ -77.12. |
| **138** | 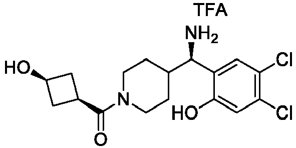 | 2-[(*R*)-amino([1-[(1*S*,3*S*)-3-hydroxycyclob utanecarbonyl] piperidin-4-yl])methyl]-4,5-dichlorophenol trifluoroacetic acid | [M + H]⁺: 373, 375 (3 : 2); ¹H NMR (400 MHz, CD₃OD) δ 7.43 (d, *J* = 3.8 Hz, 1H), 7.08 (s, 1H), 4.55 (dd, *J* = 54.1, 13.4 Hz, 1H), 4.14 (dd, *J* = 15.3, 9.5 Hz, 2H), 3.94 (dd, *J* = 53.6, 14.2 Hz, 1H), 3.01 (dt, *J* = 45.2, 13.3 Hz, 1H), 2.90 -2.75 (m, 1H), 2.66 (t, *J* = 12.9 Hz, 1H), 2.59-2.42 (m, 2H), 2.42-2.28 (m, 1H), 2.18-1.93 (m, 3H), 1.41-1.20 (m, 2H), 1.20-1.02 (m, 1H), ¹⁹F NMR (376 MHz, CD₃OD) δ -77.00. |
| **139** | 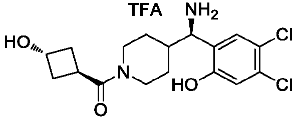 | 2-[(*R*)-amino([1-[(1*R*,3*R*)*-3-*hydroxycyclob utanecarbonyl] piperidin-4-yl])methyl]-4,5-dichlorophenol trifluoroacetic acid | [M + H]⁺: 373, 375 (3 : 2); ¹H NMR (400 MHz, CD₃OD) δ 7.43 (d, *J* = 2.4 Hz, 1H), 7.08 (s, 1H), 4.57 (dd, *J* = 55.5, 13.6 Hz, 1H), 4.37-4.21 (m, 1H), 4.21-4.08 (m, 1H), 3.83 (dd, *J* = 52.6, 13.7 Hz, 1H), 3.29-3.19 (m, 1H), 3.01 (dt, *J* = 46.0, 13.0 Hz, 1H), 2.74-2.43 (m, 3H), 2.35 (s, 1H), 2.19 (s, 2H), 1.99 (d, *J* = 12.9 Hz, 1H), 1.29 (d, *J* = 29.6 Hz, 2H), 1.22-1.06 (m, 1H), ¹⁹F NMR (376 MHz, CD₃OD) δ -77.09. |
| **140** | 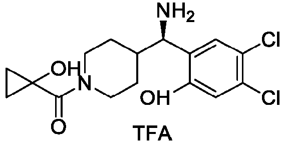 | 2-[(*R*)-amino[1-(1-hydroxycyclopr opanecarbonyl) piperidin-4-yl]methyl]-4,5-dichlorophenol trifluoroacetic acid | [M + H]⁺: 359, 361 (3 : 2); ¹H NMR (400 MHz, CD₃OD) δ 7.44 (s, 1H), 7.09 (s, 1H), 4.75-4.40 (m, 2H), 4.17 (d, *J* = 9.6 Hz, 1H), 3.14-2.67 (m, 2H), 2.38 (d, *J* = 11.4 Hz, 1H), 2.01 *(d, J* = 13.0 Hz, 1H), 1.49-1.31 (m, 2H), 1.31-1.13 (m, 1H), 1.08-0.99 (m, 2H), 0.94-0.84 (m, 2H), ¹⁹F NMR (376 MHz, CD₃OD) δ -77.07. |
| **142** | 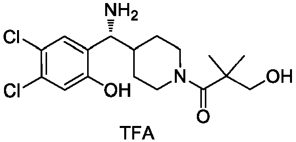 | 1-[4-[(*R*)-amino(4,5-dichloro-2-hydroxyphenyl) methyl]piperidin -1-yl]-3-hydroxy-2,2- dimethylpropan -1-one trifluoroacetic acid | [[M + H]⁺: 375, 377 (3 : 2); ¹H NMR (400 MHz, CD₃OD) δ 7.44 (s, 1H), 7.09 (s, 1H), 4.57 (d, *J* = 13.8 Hz, 1H), 4.39 (d, *J* = 13.7 Hz, 1H), 4.15 (d, *J* = 9.9 Hz, 1H), 3.56 (s, 2H), 2.84 (q, *J* = 13.5 Hz, 2H), 2.45-2.30 (m, 1H), 2.00 (d, *J* = 13.0 Hz, 1H), 1.43-1.30 (m, 2H), 1.30-1.09 (m, 7H); ¹⁹F NMR (376 MHz, CD₃OD) δ -77.08. |
| **143** | 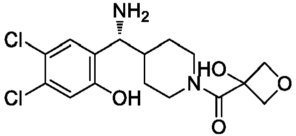 | 3-[4*-*[(*R*)-amino(4,5-dichloro-2-hydroxyphenyl) methyl] piperidine-1-carbonyl] oxetan-3-ol | [M + H]⁺: 375, 377 (3 : 2); ¹H NMR (400 MHz, CD₃OD) δ 7.21 (d, *J* = 3.8 Hz, 1H), 6.86 (d, *J* = 2.4 Hz, 1H), 5.10-4.94 (m, 2H), 4.67-4.36 (m, 3H), 3.89 (t, *J* = 5.8 Hz, 1H), 3.66 (dd, *J =* 40.2, 13.5 Hz, 1H), 2.98 (dt, *J* = 27.4, 12.9 Hz, 1H), 2.65 (dt, *J* = 27.9, 12.6 Hz, 1H), 2.07-1.87 (m, 2H), 1.51-1.40 (m, 1H), 1.40-1.04 (m, 2H). |
| **144** | 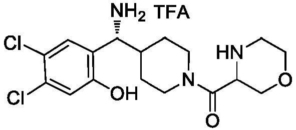 | 2-[(*R*)-amino[1-(morpholine-3-carbonyl) piperidin-4-yl]methyl]-4,5-dichlorophenol trifluoroacetic acid | [M + H]⁺: 388, 390 (3 : 2); ¹H NMR (400 MHz, CD₃OD) δ 7.44 (d, *J* = 9.7 Hz, 1H), 7.09 (d, *J* = 4.0 Hz, 1H), 4.72-4.42 (m, 2H), 4.20 (dd, *J* = 26.6, 10.0 Hz, 2H), 4.05 (d, *J* = 12.9 Hz, 2H), 3.96-3.71 (m, 2H), 3.71-3.49 (m, 1H), 3.40-3.35 (m, 1H), 3.29-3.04 (m, 1H), 2.72 (dt, *J* = 54.2, 12.9 Hz, 1H), 2.43 (d, *J* = 11.4 Hz, 1H), 2.14-2.00 (m, 1H), 1.56-1.06 (m, 3H); ¹⁹F NMR (376 MHz, CD₃OD) δ - 77.02. |
| **152** | 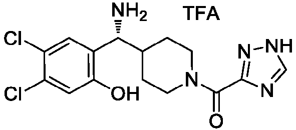 | (*R*)*-*(*4-*(amino(4,5-dichloro-2-hydroxyphenyl) methyl) piperidin-1-yl)(1H-1,2,4-triazol-3-yl)methanone trifluoroacetic acid | [M + H]⁺: 370, 372 (3 : 2); ¹H NMR (400 MHz, CD₃OD) δ 8.50 (d, *J* = 7.4 Hz, 1H), 7.45 (d, *J* = 5.1 Hz, 1H), 7.09 (d, *J* = 6.7 Hz, 1H), 4.87-4.27 (m, 2H), 4.19 (dd, *J* = 9.8, 7.1 Hz, 1H), 3.29-2.96 (m, 1H), 2.96-2.70 (m, 1H), 2.55-2.34 (m, 1H), 2.18-1.92 (m, 1H), 1.58-1.13 (m, 3H); ¹⁹F NMR (376 MHz, CD₃OD) δ -77.34. |
| **153** | 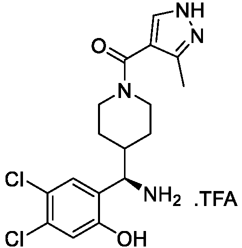 | 2-[(*R*)-amino[1-(3-methyl-1*H-*pyrazole-4-carbonyl)piperi din-4-yl]methyl]-4,5-dichlorophenol trifluoroacetic acid | [M + H]⁺: 383, 385 (3 : 2); ¹H NMR (400 MHz, CD₃OD) δ 7.65 (s, 1H), 7.45 (s, 1H), 7.09 (s, 1H), 4.30-4.15 (m, 3H), 3.01-2.82 (m, 2H), 2.47-2.42 (m, 1H), 2.35 (s, 3H), 2.02 (d, *J* = 13.2 Hz, 1H), 1.50-1.29 (m, 2H), 1.28-1.14 (m, 1H), ¹⁹F NMR (376 MHz, CD₃OD) δ -77.18. |
| **155** | 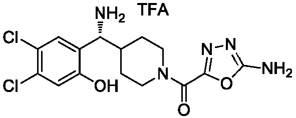 | 2-[(*R*)-amino[1-(5-amino-1,3,4-oxadiazole-2-carbonyl)piperi din-4-yl]methyl]-4,5-dichlorophenol trifluoroacetic acid | [M + H]⁺: 386, 388 (3 : 2); ¹H NMR (400 MHz, CD₃OD) δ 7.45 (s, 1H), 7.09 (s, 1H), 5.16 (dd, *J* = 53.4, 13.8 Hz, 1H), 4.65 (dd, *J* = 53.4, 13.6 Hz, 1H), 4.18 (d, *J* = 9.8 Hz, 1H), 3.31-3.10 (m, 1H), 2.86 (dt, *J* = 42.8, 12.5 Hz, 1H), 2.53-2.39 (m, 1H), 2.14-1.99 (m, 1H), 1.59-1.17 (m, 3H); ¹⁹F NMR (376 MHz, CD₃OD) δ -77.21. |
| **156** | 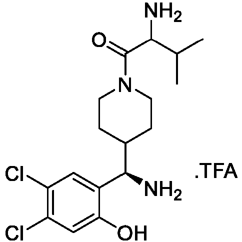 | 2-amino-1-[4-[(*R*)-amino(4,5-dichloro-2-hydroxyphenyl) methyl] piperidin-1-yl]-3-methylbutan-1-one trifluoroacetic acid | [M + H]⁺: 374, 376 (3 : 2); ¹H NMR (400 MHz, CD₃OD) δ 7.48-7.41 (m, 1H), 7.14-7.05 (m, 1H), 4.73-4.48 (m, 1H), 4.39-4.28 (m, 1H), 4.28-4.13 (m, 1H), 4.00 (dd, *J* = 57.0, 14.0 Hz, 1H), 3.27-3.03 (m, 1H), 2.72 (dt, *J* = 45.2, 12.9 Hz, 1H), 2.48-2.34 (m, 1H), 2.23-1.99 (m, 2H), 1.52-1.17 (m, 2H), 1.16-0.91 (m, 7H); ¹⁹F NMR (376 MHz, CD₃OD) δ -76.94. |
| **157** | 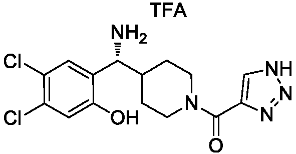 | 2-[(*R*)-amino[1-(1*H-*1,2,3-triazole-4-carbonyl) piperidin-4-yl]methyl]-4,5-dichlorophenol trifluoroacetic acid | [M + H]⁺: 370, 372 (3 : 2); ¹H NMR (400 MHz, CD₃OD) δ 8.17 (s, 1H), 7.45 (s, 1H), 7.09 (d, *J* = 3.9 Hz, 1H), 4.85-4.58 (m, 2H), 4.22-4.16 (m, 1H), 3.29-3.07 (m, 1H), 2.85 (dt, *J* = 43.2, 12.7 Hz, 1H), 2.55-2.35 (m, 1H), 2.14-1.97 (m, 1H), 1.55-1.18 (m, 3H); ¹⁹F NMR (376 MHz, CD₃OD) δ -77.02. |
| **159** | 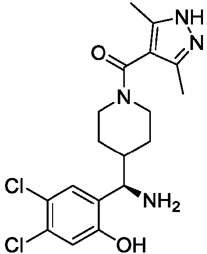 | 2-[(*R*)-amino[1-(3,5-dimethyl-1*H-*pyrazole-4-carbonyl)piperi din-4-yl]methyl]-4,5-dichlorophenol | [M + H]⁺: 397, 399, (3 : 2); ¹H NMR (400 MHz, CD₃OD) δ 7.25 (s, 1H), 6.87 (s, 1H), 4.62 (s, 1H), 4.01-3.58 (m, 2H), 2.91 (s, 2H), 2.22 (s, 6H), 2.15-1.91 (m, 3H), 1.61-1.01 (m, 6H). |
| **160** | 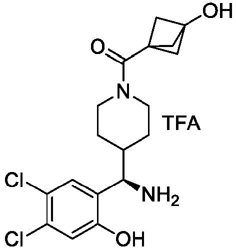 | 3-[4-[(*R*)-amino(4,5-dichloro-2-hydroxyphenyl) methyl]piperidi ne-1-carbonyl]bicyclo [1.1.1]pentan-1-ol trifluoroacetic acid | [M + H]⁺: 385, 387 (3 : 2); ¹H NMR (400 MHz, CD₃OD) δ 7.44 (s, 1H), 7.09 (d, *J* = 1.7 Hz, 1H), 4.56-4.52 (m, 1H), 4.19-4.15 (m, 1H), 3.79-3.74 (m, 1H), 3.61 (d, *J* = 17.8 Hz, 1H), 2.91 (d, *J* = 17.0 Hz, 1H), 2.68-2.63 (m, 1H), 2.44-2.36 (m, 1H), 2.03 (d, *J* = 13.0 Hz, 1H), 1.58-1.54 (m, 3H), 1.43-1.36 (m, 3H), 1.24-1.20 (m, 2H), ¹⁹F NMR (376 MHz, CD₃OD) δ -77.09. |
| **161** | 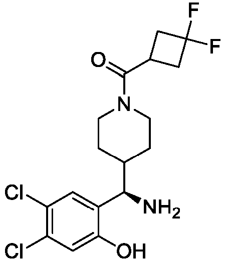 | 2-[(*R*)-amino[1-(3,3-difluorocyclobu tanecarbonyl) piperidin-4-yl]methyl]-4,5-dichlorophenol | [M + H]⁺: 393, 395 (3 : 2); ¹H NMR (400 MHz, CD₃OD) δ 7.23 (dd, *J* = 4.6, 1.4 Hz, 1H), 6.87 (dd, *J =* 3.0, 1.2 Hz, 1H), 4.66-4.42 (m, 1H), 3.98-3.73 (m, 2H), 3.29-3.18 (m, 1H), 3.10-2.91 (m, 1H), 2.90-2.71 (m, 4H), 2.62-2.85 (m, 1H), 2.02 (dd, *J* = 22.2, 11.7 Hz, 2H), 1.47 (d, *J* = 13.5 Hz, 1H), 1.20 (m, *J* = 24.6, 15.9, 8.1 Hz, 2H), ¹⁹F NMR (376 MHz, CD₃OD) δ -84.05, -84.56, -98.03, -98.55. |
| **162** | 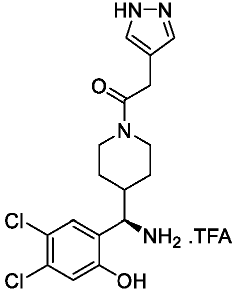 | 1-[4-[(*R*)-amino(4,5-dichloro-2-hydroxyphenyl) methyl]piperidi n-1-yl]-2-(1*H-*pyrazol-4-yl)ethan-1-one trifluoroacetic acid | [M + H]⁺: 383, 385 (3 : 2); ¹H NMR (400 MHz, CD₃OD) δ 7.56 (s, 2H), 7.42 (d, *J* = 2.1 Hz, 1H), 7.08 (s, 1H), 4.60 (dd, *J* = 53.2, 13.5 Hz, 1H), 4.27-3.98 (m, 2H), 3.80-3.53 (m, 2H), 3.08 (dt, *J* = 42.9, 12.3 Hz, 1H), 2.76-2.25 (m, 2H), 2.01-1.97 (m, 1H), 1.48-0.91 (m, 3H); ¹⁹F NMR (376 MHz, CD₃OD) δ-77.30, -77.81. |
| **163** | 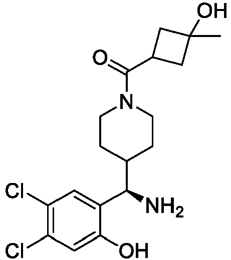 | 2-[(*R*)-amino[1-(3-hydroxy-3-methylcyclobut anecarbonyl) piperidin-4-yl]methyl]-4,5-dichlorophenol | [M + H]⁺: 387, 389 (3 : 2); ¹H NMR (400 MHz, CD₃OD) δ 7.22 (d, *J* = 4.8 Hz, 1H), 6.86 (d, *J* = 2.9 Hz, 1H), 4.54 (dd, *J* = 39.3, 13.2 Hz, 1H), 4.01-3.74 (m, 2H), 3.09-2.82 (m, 2H), 2.58-2.50 (m, 1H), 2.34-2.14 (m, 4H), 2.06-1.99 (m, 2H), 1.46 (t, *J* = 11.3 Hz, 1H), 1.38 (d, *J* = 8.5 Hz, 3H), 1.26-1.04 (m, 2H). |
| **164** | 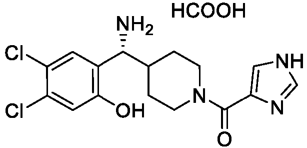 | 2-[(*R*)-amino[1-(1*H-*imidazole-4-carbonyl)piperi din-4-yl]methyl]-4,5-dichlorophenol formic acid | [M + H]⁺: 369, 371 (3 : 2); ¹H NMR (400 MHz, CD₃OD) δ 7.76 (s, 1H), 7.56 (s, 1H), 7.44 (d, *J* = 2.1 Hz, 1H), 7.08 (d, *J* = 1.7 Hz, 1H), 4.78-4.50 (m, 2H), 4.18 (d, *J* = 9.6 Hz, 1H), 3.20-2.68 (m, 2H), 2.51-2.30 (m, 1H), 2.14-1.85 (m, 1H), 1.51-1.07 (m, 3H). |
| **165** | 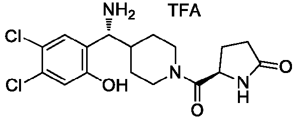 | (5*R*)-5-[4-[(*R*)-amino(4,5-dichloro-2-hydroxyphenyl) methyl]piperidi ne-1-carbonyl]pyrrol idin-2-one trifluoroacetic acid | [M + H]⁺: 386, 388 (3 : 2); ¹H NMR (400 MHz, CD₃OD) δ 7.44 (d, *J* = 1.7 Hz, 1H), 7.09 (s, 1H), 4.75-4.44 (m, 2H), 4.18 (dd, *J* = 14.5, 9.8 Hz, 1H), 4.00 (dd, *J =* 55.7, 14.0 Hz, 1H), 3.24-2.98 (m, 1H), 2.79-2.55 (m, 1H), 2.53-2.42 (m, 1H), 2.42-2.28 (m, 3H), 2.10-1.90 (m, 2H), 1.45-1.01 (m, 3H); ¹⁹F NMR (376 MHz, CD₃OD) δ -77.05. |
| **166** | 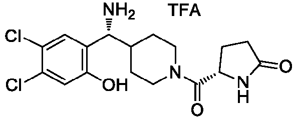 | (5*S*)-5-[4-[(*R*)-amino(4,5-dichloro-2-hydroxyphenyl) methyl]piperidi ne-1-carbonyl]pyrrol idin-2-one trifluoroacetic acid | [M + H]⁺: 386, 388 (3 : 2); ¹H NMR (400 MHz, CD₃OD) δ 7.44 (d, *J* = 6.1 Hz, 1H), 7.09 (d, *J* = 2.3 Hz, 1H), 4.79-4.43 (m, 2H), 4.26-4.07 (m, 1H), 4.07-3.82 (m, 1H), 3.12 (dt, *J* = 36.4, 13.1 Hz, 1H), 2.72 (td, *J* = 13.0, 2.8 Hz, 1H), 2.56-2.44 (m, 1H), 2.44-2.30 (m, 3H), 2.09-1.92 (m, 2H), 1.49-1.03 (m, 3H); ¹⁹F NMR (376 MHz, CD₃OD) δ -77.11. |
| **167** | 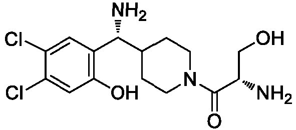 | (2*S*)-2-amino-1*-*[*4-*[(*R*)-amino(4,5-dichloro-2-hydroxyphenyl) methyl]piperidi n-1-yl]-3-hydroxypropan -1-one | [M + H]⁺: 362, 364 (3 : 2); ¹H NMR (400 MHz, CD₃OD) δ 7.21 (d, *J* = 2.7 Hz, 1H), 6.86 (s, 1H), 4.60 (dd, *J* = 45.2, 13.4 Hz, 1H), 4.09 (dd, *J =* 36.5, 13.7 Hz, 1H), 3.98-3.83 (m, 2H), 3.70-3.57 (m, 1H), 3.57-3.47 (m, 1H), 3.16-2.89 (m, 1H), 2.62 (dt, *J =* 24.9, 12.6 Hz, 1H), 2.08-1.89 (m, 2H), 1.50 (d, *J* = 13.5 Hz, 1H), 1.40-1.07 (m, 2H). |
| **168** | 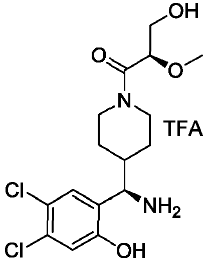 | (2*R*)-1-[4-[(*R*)-amino(4,5-dichloro-2-hydroxyphenyl) methyl]piperidi n-1-yl]-3-hydroxy-2-methoxypropan -1-one trifluoroacetic acid | [M + H]⁺: 377, 379 (3 : 2); ¹H NMR (400 MHz, CD₃OD) δ ppm 7.44 (s, 1H), 7.09 (s, 1H), 4.62-4.45 (m, 1H), 4.43-4.09 (m, 3H), 3.76-3.66 (m, 2H), 3.37 (d, *J* = 13.6 Hz, 3H), 3.20-3.02 (m, 1H), 2.64 (dd, *J* = 44.4, 12.8 Hz, 1H), 2.39 (d, *J* = 10.6 Hz, 1H), 2.03 (s, 1H), 1.45-1.13 (m, 3H). ¹⁹F NMR (376 MHz, CD₃OD) δ -77.07. |
| **169** | 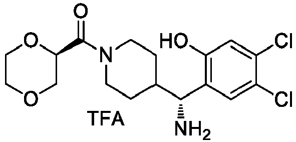 | 2-[(*R*)-amino([1-[(2*R*)-1,4-dioxane-2-carbonyl]piperi din-4-yl])methyl]-4,5-dichlorophenol trifluoroacetic acid | [M + H]⁺: 389, 391 (3 : 2), found 389, 391 (3 : 2). ¹H NMR (400 MHz, CD₃OD) δ7.44 (d, *J* = 3.2 Hz, 1H), 7.09 (s, 1H), 4.59 (d, *J =* 13.5 Hz, 1H), 4.46-4.34 (m, 1H), 4.25-4.00 (m, 2H), 3.91-3.57 (m, 6H), 3.07 (dt, J = 68.1, 12.9 Hz, 1H), 2.73-2.53 (m, 1H), 2.2.45-2.36 (m, 1H), 2.10-1.98 (m, 1H), 1.44-1.06 (m, 3H). ¹⁹F NMR (376 MHz, CD₃OD) δ - 77.12. |
| **170** | 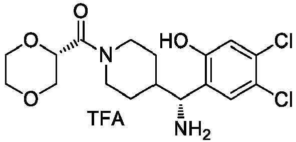 | 2-[(*R*)-amino([1-[(2*S*)-1,4-dioxane-2-carbonyl]piperi din-4-yl])methyl]-4,5-dichlorophenol trifluoroacetic acid | [M + H]⁺: 389, 391 (3 : 2); ¹H NMR (400 MHz, CD₃OD) δ7.44 (d, *J* = 3.2 Hz, 1H), 7.09 (s, 1H), 4.59 (d, *J* = 13.5 Hz, 1H), 4.46-4.34 (m, 1H), 4.25-4.00 (m, 2H), 3.91-3.57 (m, 6H), 3.07 (dt, J = 68.1, 12.9 Hz, 1H), 2.73-2.53 (m, 1H), 2.2.45-2.36 (m, 1H), 2.02 (t, *J* = 13.6 Hz, 1H), 1.44-1.06 (m, 3H); ¹⁹F NMR (376 MHz, CD₃OD) δ -77.10. |
| **171** | 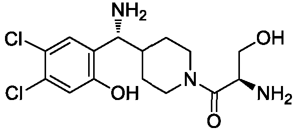 | (2*R*)-2-amino-1-[4-[(*R*)-amino(4,5-dichloro-2-hydroxyphenyl) methyl]piperidi n-1-yl]-3-hydroxypropan -1-one | [M + H]⁺: 362, 364 (3 : 2); ¹H NMR (400 MHz, CD₃OD) δ 7.22 (d, *J* = 6.0 Hz, 1H), 6.86 (d, *J* = 2.9 Hz, 1H), 4.59 (dd, *J* = 30.5, 13.4 Hz, 1H), 4.09 (dd, *J* = 39.2, 13.5 Hz, 1H), 3.91 (t, *J* = 7.4 Hz, 2H), 3.68-3.45 (m, 2H), 3.18-2.94 (m, 1H), 2.62 (dt, *J* = 37.9, 13.0 Hz, 1H), 2.14-1.92 (m, 2H), 1.49 (t, *J =* 16.0 Hz, 1H), 1.42-1.09 (m, 2H). |
| **172** | 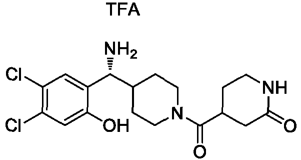 | 4-[4-[(*R*)-amino(4,5-dichloro-2-hydroxyphenyl) methyl]piperidi ne-1-carbonyl]piperi din-2-one trifluoroacetic acid | [M + H]⁺: 400, 402 (3 : 2); ¹H NMR (400 MHz, CD₃OD) δ 7.44 (d, *J* = 4.2 Hz, 1H), 7.09 (s, 1H), 4.59 *(dd, J* = 54.1, 13.5 Hz, 1H), 4.23-3.97 (m, 2H), 3.32-3.23 (m, 3H), 3.12 (dt, *J* = 46.4, 13.2 Hz, 1H), 2.75-2.51 (m, 1H), 2.51-2.31 (m, 3H), 2.09-1.75 (m, 3H), 1.50-1.06 (m, 3H); ¹⁹F NMR (376 MHz, CD₃OD) δ -77.13. |
| **173** | 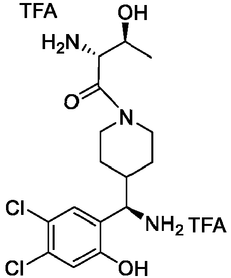 | (2*R*,3*S*)-2-amino-1-(4-((*R*)-amino(4,5-dichloro-2-hydroxyphenyl) methyl)piperidi n-1-yl)-3-hydroxybutan-1-one bis(trifluoroace tic acid) | [M + 1]⁺: 376, 378 (3 : 2); ¹H NMR (400 MHz, CD₃OD) δ 7.44 (s, 1H), 7.09 (s, 1H), 4.59 (dd, *J* = 53.7, 13.5 Hz, 1H), 4.40 *(dd, J* = 47.5, 4.9 Hz, 1H), 4.28-3.84 (m, 3H), 3.23-3.10 (m, 1H), 2.85-2.60 (m, 1H), 2.55-2.32 (m, 1H), 2.07 (d, *J* = 12.9 Hz, 1H), 1.57-1.03 (m, 6H); ¹⁹F NMR (376 MHz, CD₃OD) δ - 76.91. |
| **174** | 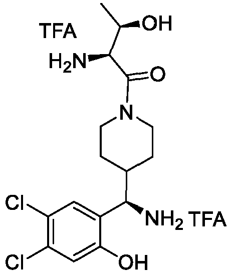 | (*2S,3R*)*-2-*amino-1-[4-[(*R*)-amino(4,5-dichloro-2-hydroxyphenyl) methyl]piperidi n-1-yl]-3-hydroxybutan-1-one bis(trifluoroace tic acid) | [M + H]⁺: 376, 378 (3 : 2); ¹H NMR (400 MHz, CD₃OD) δ 7.45 (d, *J* = 8.9 Hz, 1H), 7.10 *(d, J* = 2.9 Hz, 1H), 4.59 (dd, *J* = 44.5, 13.5 Hz, 1H), 4.32 (dd, *J* = 38.4, 5.2 Hz, 1H), 4.24-3.96 (m, 3H), 3.32-2.99 (m, 1H), 2.83-2.59 (m, 1H), 2.50-2.36 (m, 1H), 2.17-2.01 (m, 1H), 1.52-1.07 (m, 6H); ¹⁹F NMR (376 MHz, CD₃OD) δ -76.90. |
| **175** | 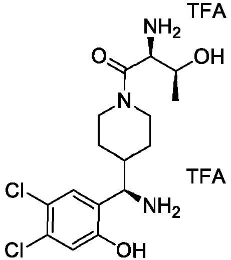 | (2*S*,3*S*)-2-amino-1-[4-[(*R*)-amino(4,5-dichloro-2-hydroxyphenyl) methyl]piperidi n-1-yl]-3-hydroxybutan-1-one bis trifluoroacetic acid | [M + H]⁺: 376, 378 (3 : 2); ¹H NMR (400 MHz, CD₃OD) δ 7.45 (d, *J* = 9.5 Hz, 1H), 7.09 (d, *J* = 3.7 Hz, 1H), 4.59 (dd, *J =* 53.7, 13.5 Hz, 1H), 4.40 *(dd, J* = 47.5, 4.9 Hz, 1H), 4.28-3.84 (m, 3H), 3.23-3.10 (m, 1H), 2.85-2.60 (m, 1H), 2.55-2.32 (m, 1H), 2.07 (d, *J* = 12.9 Hz, 1H), 1.57-1.03 (m, 6H); ¹⁹F NMR (376 MHz, CD₃OD) δ - 77.23. |
| **176** | 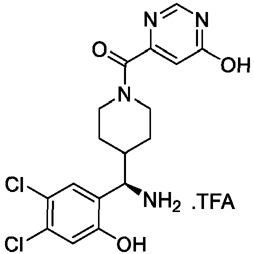 | (*R*)-(4-(amino( 4,5-dichloro-2-hydroxyphenyl) methyl)piperidi n-1-yl)(6-hydro xypyrimidin-4-yl)methanone trifluoroacetic acid | [M + H]⁺: 397, 399 (3 : 2); ¹H NMR (400 MHz, CD₃OD) δ 8.25 (dd, *J* = 11.0, 1.1 Hz, 1H), 7.45 (d, *J* = 4.7 Hz, 1H), 7.0 8 (d, *J* = 8.4 Hz, 1H), 6. 54 (dd, *J* = 11.2, 1.1 Hz , 1H), 4.63 (dd, *J* = 52.9 , 14.0 Hz, 1H), 4.27-4.12 (m, 1H), 3.83 (dd, *J* = 53.3, 14.0 Hz, 1H), 3.2 1-2.96 (m, 1H), 2.95-2.7 0 (m, 1H), 2.52-2.32 (m , 1H), 2.18-1.86 (m, 1H) , 1.54-1.13 (m, 3H); ¹⁹F NMR (376 MHz, CD₃O D) δ -77.10. |
| **177** | 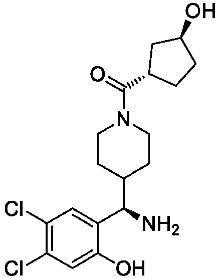 | (4*-*((*R*)-amino(4,5-dichloro-2-hydroxyphenyl) methyl)piperidin -1-yl)(3-hydroxycyclop entyl)methanone isomer 1 | [M + H]⁺: 387, 389 (3 : 2); ¹H NMR (400 MHz, CD₃OD) δ 7.25 (d, *J* = 1.2 Hz, 1H), 6.89 (s, 1H), 4.58 (dd, *J* = 41.3, 13.4 Hz, 1H), 4.39-4.33 (m, 1H), 4.12 (dd, *J* = 40.2, 13.8 Hz, 1H), 3.92 (dd, *J* = 8.1, 5.4 Hz, 1H), 3.20-2.91 (m, 1H), 2.73-2.43 (m, 1H), 2.25-1.87 (m, 5H), 1.85-1.69 (m, 2H), 1.68-1.57 (m, 1H), 1.54-1.36 (m, 1H), 1.37-0.82 (m, 3H). |
| **178** | 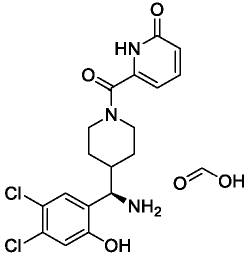 | (*R*)-6-(4-(amino(4,5-dichloro-2-hydroxyphenyl) methyl)piperidi ne-1-carbonyl)pyridin -2(1H)-one formic acid | [M + H]⁺: 396, 398 (3 : 2); ¹H NMR (400 MHz, CD₃OD) δ 8.54 (s, 1H), 7.63 (m, 1H), 7.39 (s, 1 H), 7.04 (s, 1H), 6.62 (d *, J* = 9.1 Hz, 1H), 6.50 ( d, *J* = 6.8 Hz, 1H), 4.61 (d, *J* = 49.2 Hz, 1H), 4. 12 (d, *J* = 9.2 Hz, 1H), 3.79 (d, *J* = 41.1 Hz, ¹H ), 3.16 (d, *J =* 44.1 Hz, 1H), 2.83 (d, *J* = 39.7 H z, 1H), 2.33 (d, *J =* 11.1 Hz, 1H), 2.18-1.91 (m, 1H), 1.56-1.12 (m, 3H). |
| **179** | 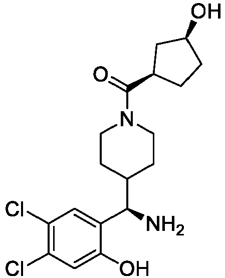 | (4-((*R*)-amino(4,5-dichloro-2-hydroxyphenyl) methyl)piperidi n-1-yl)(3-hydroxycyclop entyl)methanone isomer 2 | [M + H]⁺: 387, 389 (3 : 2); ¹H NMR (400 MHz, CD₃OD) δ 7.28 (d, *J* = 2.2 Hz, 1H), 6.92 (d, *J* = 1.5 Hz, 1H), 4.58 (dd, *J =* 43.9, 13.2 Hz, 1H), 4.36 (m, *J =* 7.9, 5.4, 2.6 Hz, 1H), 4.13 (dd, *J* = 42.6, 13.8 Hz, 1H), 3.96 (dd, *J* = 8.3, 5.3 Hz, 1H), 3.29 (d, *J =* 8.3 Hz, 1H), 3.21-2.93 (m, 1H), 2.65-2.54 (m, 1H), 2.24-1.87 (m, 5H), 1.87-1.57 (m, 3H), 1.54-1.34 (m, 1H), 1.31-1.11 (m, 2H). |
| **185** | 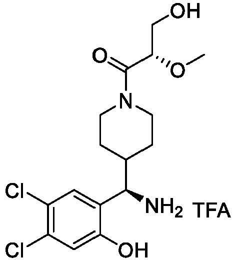 | (2*S*)-1-[4-[(*R*)-amino(4,5-dichloro-2-hydroxyphenyl) methyl]piperidi n-1-yl]-3-hydroxy-2-methoxypropan -1-one trifluoroacetic acid | [M + H]⁺: 377, 379 (3 : 2); ¹H NMR (400 MHz, CD₃OD) δ ppm 7.44 (s, 1H), 7.09 (s, 1H), 4.52 (s, 1H), 4.43-4.09 (m, 3H), 3.76-3.66 (m, 2H), 3.37 (d, *J* = 13.6 Hz, 3H), 3.02 (d, *J =* 13.2 Hz, 1H), 2.64 (dd, *J =* 44.4, 12.8 Hz, 1H), 2.39 (d, *J* = 10.6 Hz, 1H), 2.09-2.02 (m, 1H), 1.45-1.13 (m, 3H). ¹⁹F NMR (376 MHz, CD₃OD) δ - 77.01. |
| **186** | 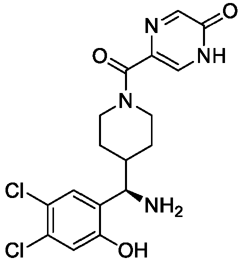 | (*R*)*-5-(4-*(amino(4,5-dichloro-2-hydroxyphenyl) methyl)piperidi ne-1-carbonyl)pyrazin -2(1*H*)-one | [M + H]⁺: 397, 399 (3 : 2); ¹H NMR (400 MHz, CD₃OD) δ 8.02 (s, 1H), 7.78 (d, *J* = 1.3 Hz, 1H), 7.330 (s, 1H), 6.94 (s, 1H), 4.70-4.20 (m, 2H), 4.01 (d, *J* = 8.5 Hz, 1H), 3.10-2.66 (m, 2H), 2.29-2.11 (m, 1H), 2.0-1.92 (m, 1H), 1.50-1.18 (m, 3H). |
| **194** | 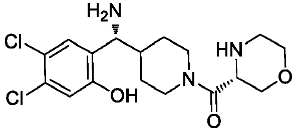 | (4*-*((R)-amino(4,5-dichloro-2-hydroxyphenyl) methyl)piperidin -1-yl)((R)-morpholin-3-yl)methanone | [M + H]⁺: 388, 390 (3 : 2);¹H NMR (400 MHz, CD₃OD) δ 7.23 (d, *J* = 7.9 Hz, 1H), 6.86 (d, *J* = 2.5 Hz, 1H), 4.53 (dd, *J* = 39.4, 13.0 Hz, 1H), 4.14-3.86 (m, 4H), 3.77 (t, *J* = 10.3 Hz, 1H), 3.55-3.45 (m, 1H), 3.44-3.32 (m, 1H), 3.16-2.90 (m, 3H), 2.60 (dt, *J* = 28.6, 12.9 Hz, 1H), 2.14-1.94 (m, 2H), 1.49 (dd, *J* = 26.0, 13.0 Hz, 1H), 1.32-1.09 (m, 2H). |
| **195** | 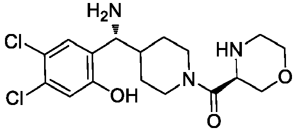 | (4*-*((*R*)-amino(4,5-dichloro-2-hydroxyphenyl) methyl)piperidin -1-yl)((*S*)-morpholin-3-yl)methanone | [M + H]⁺: 388, 390 (3 : 2); ¹H NMR (400 MHz, CD₃OD) δ 7.23 (d, *J* = 7.9 Hz, 1H), 6.86 (d, *J* = 2.5 Hz, 1H), 4.53 (dd, *J* = 39.4, 13.0 Hz, 1H), 4.14-3.86 (m, 4H), 3.77 (t, *J* = 10.3 Hz, 1H), 3.55-3.45 (m, 1H), 3.44-3.32 (m, 1H), 3.16-2.90 (m, 3H), 2.60 (dt, *J* = 28.6, 12.9 Hz, 1H), 2.14-1.94 (m, 2H), 1.49 (dd, *J* = 26.0, 13.0 Hz, 1H), 1.32-1.09 (m, 2H). |
| **199** | 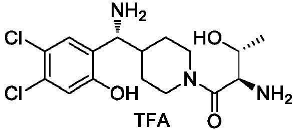 | (2*R*,3*R*)-2-amino-1-[4-[(*R*)-amino(4,5-dichloro-2-hydroxyphenyl) methyl]piperidi n-1-yl]-3-hydroxybutan-1-one trifluoroacetic acid | [M + H]⁺: 376, 378 (3 : 2); ¹H NMR (400 MHz, CD₃OD) δ 7.50-7.42 (m, 1H), 7.09 (d, *J* = 1.1 Hz, 1H), 4.58 (dd, *J* = 53.5, 13.6 Hz, 1H), 4.42 (dd, *J* = 5.0, 2.6 Hz, 1H), 4.30-4.12 (m, 2H), 4.11-3.92 (m, 1H), 3.17 (dt, *J* = 27.0, 12.6 Hz, 1H), 2.82-2.58 (m, 1H), 2.42 (t, *J* = 10.3 Hz, 1H), 2.09 (dd, *J =* 29.6, 13.2 Hz, 1H), 1.47-1.10 (m, 6H); ¹⁹F NMR (376 MHz, CD₃OD) δ -76.85. |
| **200** | 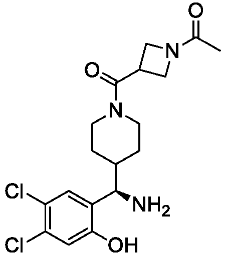 | 1-(3-[4-[(*R*)-amino(4,5-dichloro-2-hydroxyphenyl) methyl]piperidi ne-1-carbonyl]azetid in-1-yl)ethan-1-one | [M + H]⁺: 400, 402 (3 : 2); ¹H NMR (400 MHz, CD₃OD) δ 7.32-7.11 (m, 1H), 6.87 (d, *J* = 3.1 Hz, 1H), 4.56 (dd, *J* = 38.0, 13.5 Hz, 1H), 4.37 (q, *J* = 7.1 Hz, 2H), 4.27-3.96 (m, 2H), 3.96-3.80 (m, 1H), 3.80-3.50 (m, 2H), 3.03 (dt, *J* = 27.5, 12.9 Hz, 1H), 2.64 (dt, *J* = 28.7, 13.0 Hz, 1H), 2.12-1.92 (m, 2H), 1.88 (t, *J* = 2.7 Hz, 3H), 1.58-1.35 (m, 1H), 1.35-0.99 (m, 2H). |
| **203** | 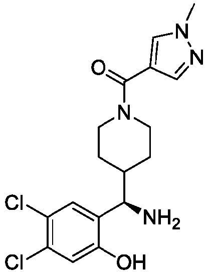 | (*R*)-(4-(amino(4,5-dichloro-2-hydroxyphenyl) methyl)piperidi n-1-yl)(1-methyl-1H-pyrazol-4-yl)methanone | [M + H]⁺: 383, 385 (3 : 2); ¹H NMR (400 MHz, CD₃OD) δ 7.93 (s, 1H), 7.69 (s, 1H), 7.23 (s, ¹H ), 6.86 (s, 1H), 4.74-4.4 7 (m, 1H), 4.37-4.15 (m , 1H), 3.93 (s, 3H), 3.91 (d, *J* = 8.0 Hz, 1H), 3.2 9-2.90 (m, 1H), 2.89-2.6 1 (m, 1H), 2.14-1.95 (m , 2H), 1.57-1.41 (m, 1H) , 1.36-1.16 (m, 2H). |
| **205** | 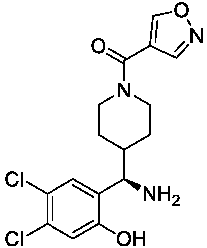 | 2-[(*R*)-amino[1-(1,2-oxazole-4-carbonyl)piperi din-4-yl]methyl]-4,5-dichlorophenol | [M + H]⁺: 370, 372 (3 : 2) ; ¹H NMR (400 MHz, CD₃OD) δ 8.99 (s, 1H), 8.56 (s, 1H), 7.34 (s, 1H), 6.99 (s, 1H), 4.40 (d, 1H), 4.30 (d, *J* = 13.7 Hz, 1H), 4.17-3.90 (m, 1H), 2.83 (dtd, *J =* 44.9, 13.1, 2.7 Hz, 2H), 2.28-2.12 (m, 1H), 2.10-1.93 (m, 1H), 1.50-1.12 (m, 3H). |
| **208** | 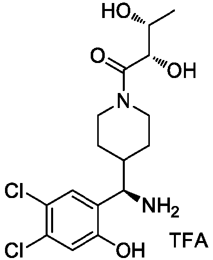 | 1-(4-((*R*)-amino(4,5-dichloro-2-hydroxyphenyl) methyl)piperidi n-1-yl)-2,3-dihydroxybutan -1-one trifluoroacetic acid | [M + H]⁺ 377, 379 (3 : 2); ¹H NMR (400 MHz, CD₃OD) δ 7.43 (s, 1H), 7.09 (s, 1H), 4.72-4.44 (m, 1H), 4.35-4.02 (m, 3H), 3.97-3.83 (m, 1H), 3.21-2.90 (m, 1H), 2.67 (dt, *J* = 44.0, 13.0 Hz, 1H), 2.44-2.30 (m, 1H), 2.05-1.94 (m, 1H), 1.44-1.26 (m, 2H), 1.26-1.10 (m, 4H); ¹⁹F NMR (376 MHz, CD₃OD) δ -77.04. |
| **213** | 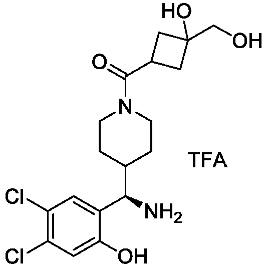 | 2-[(*R*)-amino([1-[3-hydroxy-3-(hydroxymethy l)cyclobutaneca rbonyl]piperidi n-4-yl])methyl]-4,5-dichlorophenol trifluoroacetic acid | [M + H]⁺: 403, 405 (3 : 2); ¹H NMR (400 MHz, CD₃OD) δ 7.43 (d, *J* = 3.4 Hz, 1H), 7.08 (s, 1H), 4.56 (dd, *J* = 54.6, 13.5 Hz, 1H), 4.15 (dd, *J* = 15.3, 9.9 Hz, 1H), 3.97-3.70 (m, 1H), 3.58-3.47 (m, 1H), 3.43 (d, *J* = 2.8 Hz, 2H), 3.14-2.81 (m, 1H), 2.61 (dt, *J* = 43.8, 13.0 Hz, 1H), 2.49-2.28 (m, 3H), 2.28-2.07 (m, 2H), 2.04-1.91 (m, 1H), 1.41-1.03 (m, 3H); ¹⁹F NMR (376 MHz, CD₃OD) δ -76.97. |
| **216** | 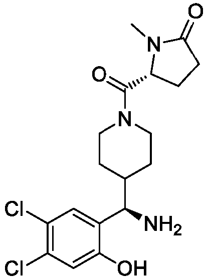 | (5*R*)-5-[4-[(*R*)-amino(4,5-dichloro-2-hydroxyphenyl) methyl]piperidi ne-1-carbonyl]-1-methylpyrrolidi n-2-one | [M + H]⁺: 400, 402 (3 : 2); ¹H NMR (400 MHz, CD₃OD) δ 7.24 (d, *J* = 5.2 Hz, 1H), 6.88 *(d, J* = 2.8 Hz, 1H), 4.77-4.46 (m, 2H), 4.11-3.85 (m, 2H), 3.21 -2.96 (m, 1H), 2.82-2.75 (d, *J =* 8.1 Hz, 3H), 2.73-2.54 (m, 1H), 2.54-2.22 (m, 3H), 2.18-1.97 (m, 2H), 1.97-1.82 (m, 1H), 1.61-1.40 (m, 1H), 1.43-1.08 (m, 2H). |
| **223** | 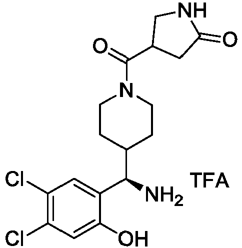 | 4-[4-[(*R*)-amino(4,5-dichloro-2-hydroxyphenyl) methyl]piperidi ne-1-carbonyl]pyrrol idin-2-one trifluoroacetic acid | [M + H]⁺: 386, 388 (3 : 2); ¹H NMR (400 MHz, CD₃OD) δ 7.44 (dd, *J* = 4.7, 2.5 Hz, 1H), 7.09 (s, 1H), 4.59 (dd, *J* = 53.4, 13.5 Hz, 1H), 4.23-3.92 (m, 2H), 3.83-3.64 (m, 1H), 3.63-3.44 (m, 2H), 3.25-3.00 (m, 1H), 2.77-2.49 (m, 3H), 2.47-2.30 (m, 1H), 2.12-1.91 (m, 1H), 1.45-1.04 (m, 3H); ¹⁹F NMR (376 MHz, CD₃OD) δ -77.15. |
| **224** | 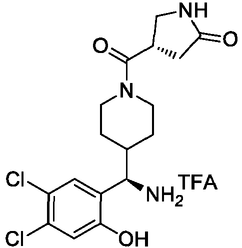 | (4*S*)-4-[4-[(*R*)-amino(4,5-dichloro-2-hydroxyphenyl) methyl]piperidi ne-1-carbonyl]pyrrol idin-2-one trifluoroacetic acid | [M + H]⁺: 386, 388 (3 : 2); ¹H NMR (400 MHz, CD₃OD) δ 7.44 (d, *J* = 2.1 Hz, 1H), 7.09 (d, *J* = 1.3 Hz, 1H), 4.59 (dd, *J* = 51.6, 13.5 Hz, 1H), 4.22-3.93 (m, 2H), 3.84-3.65 (m, 1H), 3.65-3.45 (m, 2H), 3.11 (dt, *J* = 40.1, 13.5 Hz, 1H), 2.80-2.50 (m, 3H), 2.46-2.28 (m, 1H), 2.03 (t, *J* = 12.2 Hz, 1H), 1.48-1.04 (m, 3H); ¹⁹F NMR (376 MHz, CD₃OD) δ - 77.16. |
| **230** | 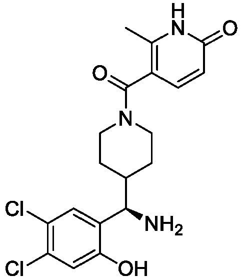 | (*R)-5-(4-*(amino(4,5-dichloro-2-hydroxyphenyl) methyl)piperidi ne-1-carbonyl)-6-methylpyridin-2(1H)-one | [M + H]⁺: 410, 412 (3 : 2); ¹H NMR (400 MHz, DMSO-*d*₆) δ 7.26 (d, *J* = 6.8 Hz, 2H), 6.84 (s, 1H), 6.22 (d, *J* = 9.3 Hz, 1H), 4.33 (d, *J =* 55.9 Hz, 1H), 3.85 *(d, J* = 6.6 Hz, 1H), 3.60-3.35 (m, 1H), 3.01-2.76 (m, 1H), 2.72-2.54 (m, 1H), 2.09 (s, 3H), 1.94-1.63 (m, 2H), 1.47-1.25 (m, 1H), 1.26-0.92 (m, 2H). |
| **268** | 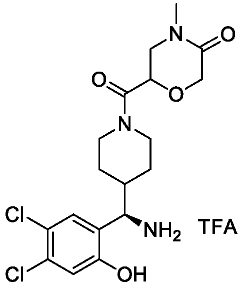 | 6-[4-[(*R*)-amino(4,5-dichloro-2-hydroxyphenyl) methyl]piperidi ne-1-carbonyl]-4-methylmorphol in-3-one trifluoroacetic acid | [M + H]⁺: 416, 418 (3 : 2); ¹H NMR (400 MHz, CD₃OD) δ 7.44 (d, *J* = 3.9 Hz, 1H), 7.09 (d, *J* = 1.1 Hz, 1H), 4.82-4.66 (m, 1H), 4.55 (dd, *J* = 56.0, 13.2 Hz, 1H), 4.32-4.17 (m, 1H), 4.17-4.11 (m, 1H), 4.03 (d, *J* = 13.8 Hz, 1H), 3.80-3.70 (m, 1H), 3.49-3.36 (m, 2H), 3.24-3.06 (m, 1H), 3.02 (d, *J* = 3.9 Hz, 3H), 2.81-2.56 (m, 1H), 2.45-2.31 (m, 1H), 2.04-1.95 (m, 1H), 1.48-1.08 (m, 3H); ¹⁹F NMR (376 MHz, CD₃OD) δ - 77.21. |
| **279** | 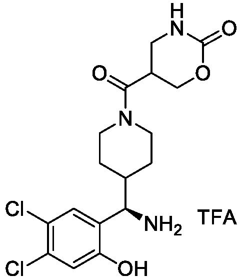 | 5-[4-[(*R*)-amino(4,5-dichloro-2-hydroxyphenyl) methyl]piperidi ne-1-carbonyl]-1,3-oxazinan-2-one trifluoroacetic acid | [M + H]⁺: 402, 404 (3 : 2); ¹H NMR (400 MHz, CD₃OD) δ 7.44 (d, *J* = 6.1 Hz, 1H), 7.09 (s, 1H), 4.57 *(dd, J* = 54.3, 13.7 Hz, 1H), 4.43-4.25 (m, 2H), 4.25-3.97 (m, 2H), 3.55-3.36 (m, 3H), 3.21-2.97 (m, 1H), 2.78-2.51 (m, 1H), 2.51-2.25 (m, 1H), 2.15-1.89 (m, 1H), 1.48-1.01 (m, 3H); ¹⁹F NMR (376 MHz, CD₃OD) δ -77.21 (d, *J* = 18.8 Hz). |
| **301** | 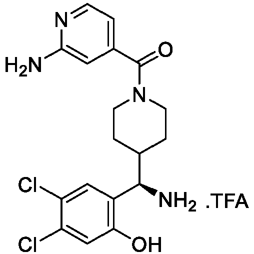 | 2-[(*R*)-amino[1-(2-aminopyridine-4-carbonyl)piperi din-4-yl]methyl]-4,*S-*dichlorophenol trifluoroacetic acid | [M + H]⁺: 395, 397 (3 : 2); ¹H NMR (400 MHz, CD₃OD) δ 7.93 (dd, *J* = 12.6, 6.5 Hz, 1H), 7.44 *(d, J* = 8.8 Hz, 1H), 7.09 *(d, J* = 8.2 Hz, 1H), 6.95 *(d, J* = 9.0 Hz, 1H), 6.91-6.79 (m, 1H), 4.64 *(dd, J* = 55.3, 13.6 Hz, 1H), 4.21 (dd, *J* = 18.2, 9.7 Hz, 1H), 3.68 *(dd, J* = 48.4, 13.8 Hz, 1H), 3.17 *(dt, J* = 46.3, 13.1 Hz, 1H), 2.86 (dt, *J* = 43.3, 12.8 Hz, 1H), 2.55-2.35 (m, 1H), 2.05 *(dd, J* = 53.9, 12.9 Hz, 1H), 1.57-1.16 (m, 3H); ¹⁹F NMR (376 MHz, CD₃OD) δ -76.96. |
| **303** | 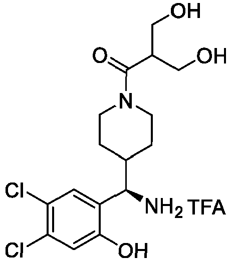 | 1-[4-[(*R*)-amino(4,5-dichloro-2-hydroxyphenyl) methyl]piperidi n-1-yl]-3-hydroxy-2-(hydroxymethy l)propan-1-one trifluoroacetic acid | [M + H]⁺: 377, 379 (3 : 2); ¹H NMR (400 MHz, CD₃OD) δ 7.43 (d, *J* = 1.9 Hz, 1H), 7.08 (s, 1H), 4.66 *(dd, J* = 55.1, 13.5 Hz, 1H), 4.36-4.10 (m, 2H), 3.78-3.59 (m, 4H), 3.28-3.02 (m, 2H), 2.63 (dt, *J* = 44.6, 12.9 Hz, 1H), 2.39 (d, *J =* 11.9 Hz, 1H), 2.01 (t, *J* = 13.5 Hz, 1H), 1.47-1.12 (m, 3H); ¹⁹F NMR (376 MHz, CD₃OD) δ - 76.82. |
| **304** | 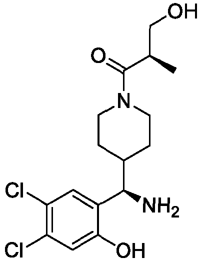 | (2*R*)-1-[4-[(*R*)-amino(4,5-dichloro-2-hydroxyphenyl) methyl]piperidi n-1-yl]-3-hydroxy-2-methylpropan-1-one | [M + H]⁺: 361, 363 (3 : 2); ¹H NMR (400 MHz, CD₃OD) δ 7.22 (d, *J* = 2.4 Hz, 1H), 6.86 (s, 1H), 4.61 (dd, *J* = 35.9, 13.3 Hz, 1H), 4.15 (dd, *J =* 39.2, 13.7 Hz, 1H), 3.89 (t, *J* = 6.5 Hz, 1H), 3.72 (dt, *J* = 10.4, 8.1 Hz, 1H), 3.50 (td, *J =* 10.2, 5.5 Hz, 1H), 3.06 (dt, *J* = 21.4, 13.2 Hz, 2H), 2.71-2.47 (m, 1H), 2.14-1.92 (m, 2H), 1.49 (dd, *J* = 26.4, 13.2 Hz, 1H), 1.39-1.13 (m, 2H), 1.06 (t, *J* = 7.4 Hz, 3H). |
| **305** | 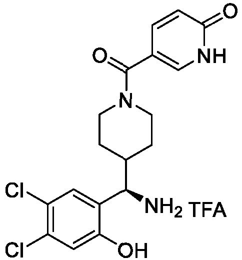 | *S*-[4-[(*R*)-amino(4,5-dichloro-2-hydroxyphenyl) methyl]piperidi ne-1-carbonyl]-1,2-dihydropyridin-2-one trifluoroacetic acid | [M + H]⁺: 396, 398 (3 : 2); ¹H NMR (400 MHz, CD₃OD) δ 7.76-7.59 (m , 2H), 7.44 (s, 1H), 7.09 (s, 1H), 6.56 (dd, *J* = 9. 3, 0.8 Hz, 1H), 4.56-4.0 0 (m, 2H), 4.18 (d, *J =* 9.8 Hz, 1H), 3.16-2.76 ( m, 2H), 2.50-2.30 (m, 1 H), 2.07-1.96 (m, 1H), 1 .54-1.01 (m, 3H); ¹⁹F N MR (376 MHz, CD₃OD ) δ -77.36. |
| **306** | 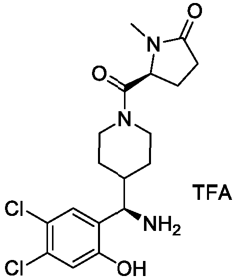 | (*S*)-5-(4-((*R*)-amino(4,5-dichloro-2-hydroxyphenyl) methyl)piperidi ne-1-carbonyl)-1-methylpyrrolidi n-2-one trifluoroacetic acid | [M + H]⁺: 400, 402 (3 : 2); ¹H NMR (400 MHz, CD₃OD) δ 7.44 (d, *J* = 4.0 Hz, 1H), 7.09 (d, *J* = 1.0 Hz, 1H), 4.77-4.37 (m, 2H), 4.18 (dd, *J =* 25.5, 9.8 Hz, 1H), 3.94 *(d, J* = 14.0 Hz, 1H), 3.21-3.03 (m, 1H), 2.85-2.59 (m, 4H), 2.49-2.23 (m, 4H), 2.14-1.96 (m, 1H), 1.96-1.82 (m, 1H), 1.54-1.02 (m, 3H); ¹⁹F NMR (376 MHz, CD₃OD) δ -77.17. |
| **307** | 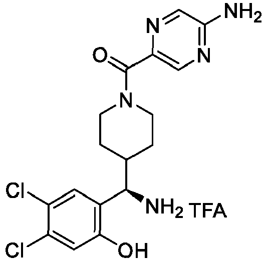 | 2-[(*R*)-amino[1-(5-aminopyrazine-2-carbonyl)piperi din-4-yl]methyl]-4,5-dichlorophenol trifluoroacetic acid | [M + H]⁺: 396, 398 (3 : 2); ¹H NMR (400 MHz, CD₃OD) δ 8.23 (s, 1H), 7.90 (s, 1H), 7.44 (s, 1H), 7.09 (s, 1H), 4.76-4.32 (m, 2H), 4.19 (d, *J* = 9.8 Hz, 1H), 3.21-2.70 (m, 2H), 2.46-2.38 (m, 1H), 2.11-1.93 (s, 1H), 1.49-1.33 (m, 3H); ¹⁹F NMR (376 MHz, CD₃OD) δ -77.36. |
| **320** | 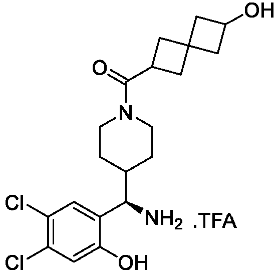 | *S*-[4-[(*R*)-amino(4,5-dichloro-2-hydroxyphenyl) methyl]piperidi ne-1-carbonyl]spiro[ 3.3]heptan-2-ol trifluoroacetic acid | [M +1]+: 413, 415 (3 : 2); 1H NMR (400 MHz, CD₃OD) δ 7.42 (d, J = 3.7 Hz, 1H), 7.08 (s, 1H), 4.53 (dd, J *=* 55.0, 13.4 Hz, 1H), 4.22-4.01 (m, 2H), 3.84 (dd, J = 53.4, 13.9 Hz, 1H), 3.32-3.22 (m, 1H), 3.09-2.84 (m, 1H), 2.72-2.43 (m, 2H), 2.40-2.08 (m, 6H), 2.03-1.82 (m, 3H), 1.38-0.98 (m, 3H); 19F NMR (376 MHz, CD₃OD) δ -77.20. |

### Example 149. Compound 101 (4,5-dichloro-2-[hydroxy([1-[(3R)-pyrrolidine-3-carbonyl]piperidin-4-yl])methyl]phenol);

### Compound 116 (4,5-dichloro-2-[(R)-hydroxy([1-[(3R)-pyrrolidine-3-carbonyl]piperidin-4-yl])methyl]phenol); and

### Compound 115 (4,5-dichloro-2-[(S)-hydroxy([1-[(3R)-pyrrolidine-3-carbonyl]piperidin-4-yl])methyl]phenol)

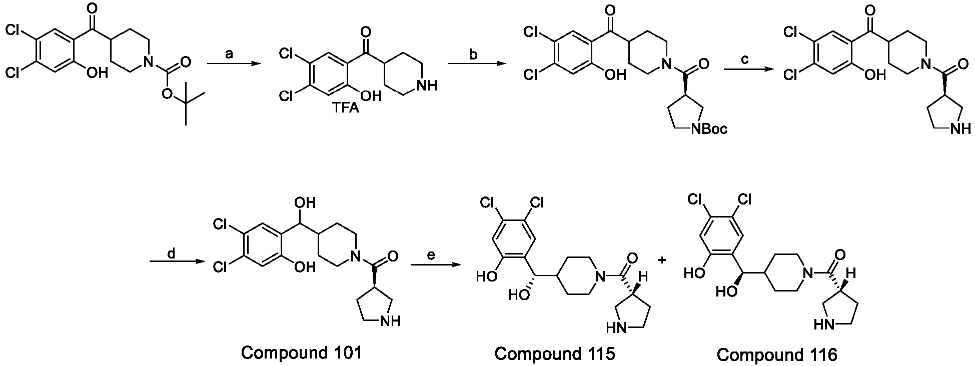

### Step a:

To a stirred solution of *tert-butyl* 4-(4,5-dichloro-2-hydroxybenzoyl)piperidine-1-carboxylate (0.80 g, 2.14 mmol) in DCM (2 mL) was added TFA (2 mL, 26.93 mmol) at room temperature. After stirring for 1 h at room temperature, the resulting mixture was concentrated under reduced pressure. The crude product was used in the next step directly without further purification. LCMS (ESI) calc'd for C₁₂H₁₃Cl₂NO₂ [M + H]⁺: 274, 276 (3 : 2), found 274, 276 (3 : 2).

### Step b:

To the solution of CDI (0.25 g, 1.57 mmol) and *(3R)-1-[(tert-*butoxy)carbonyl]pyrrolidine-3-carboxylic acid (0.39 g, 1.80 mmol) in DMF 2 (mL) were added a solution of 4,5-dichloro-2-(piperidine-4-carbonyl)phenol trifluoroacetic acid (0.10 g, 0.26 mmol) in DMF (2 mL) and Et₃N (1.10 g, 10.87 mmol) at room temperature under nitrogen atmosphere. The reaction solution was stirred for additional 2 h at room temperature. The resulting mixture was diluted with water (50 mL) at room temperature and extracted with EA (3 x 10 mL). The combined organic layers were washed with brine (3 x 10 mL), dried over anhydrous Na₂SO₄. After filtration, the filtrate was concentrated under reduced pressure. The residue was purified by silica gel column chromatography, eluted with PE/EA (1:1) to afford *tert-butyl (3R)-3-[4-(4,5-dichloro-2-hydroxybenzoyl)piperidine-1-carbonyl]pyrrolidine-1-*carboxylate (0.11 g, 88%) as an off-white solid: LCMS (ESI) calc'd for C₂₂H₂₈Cl₂N₂O₅ [M + H]⁺: 471, 473 (3 : 2), found 471, 473 (3 : 2).

### Step c:

To a stirred solution of *tert*-butyl (3*R*)-3-[4-(4,5-dichloro-2-hydroxybenzoyl)piperidine-1-carbonyl]pyrrolidine-1-carboxylate (0.15 g, 0.24 mmol) in DCM (2 mL) was added TFA (2 mL) at room temperature. The resulting mixture was stirred for 1 h at room temperature. The resulting mixture was concentrated under reduced pressure. The crude product was used in the next step directly without further purification: LCMS (ESI) calc'd for C₁₇H₂₀Cl₂N₂O₃ [M + H]⁺: 371, 373 (3 : 2), found 371, 373 (3 : 2).

### Step d:

To a stirred solution of 4,5-dichloro-2-[1-[(3*R*)-pyrrolidine-3-carbonyl]piperidine-4-carbonyl]phenol (80 mg, 0.22 mmol) in MeOH (3 mL) was added NaBH₄ (27.2 mg, 0.72 mmol) at room temperature under air atmosphere. The resulting mixture was stirred for 1.5 h at room temperature under nitrogen atmosphere. The resulting mixture was concentrated under reduced pressure. The residue was purified by Prep-HPLC with the following conditions: Column: XBridge C18 OBD Prep Column, 100 Å, 10 µm, 19 mm x 250 mm; Mobile Phase A: water with 10 mmol/L NH₄HCO₃, Mobile Phase B: ACN; Flow rate: 25 mL/min; Gradient: 5% B to 80% B in 6 min; Detector: UV 254/210 nm; Retention time: 5.43 min. The fractions containing the desired product were collected and concentrated under reduced pressure to afford Compound 101 (4,5-dichloro-2-[hydroxy([1-[(3R)-pyrrolidine-3-carbonyl]piperidin-4-yl])methyl]phenol) as an off-white solid (55 mg, 64%): LCMS (ESI) calc'd for C₁₇H₂₂Cl₂N₂O₃ [M + H]⁺: 373, 375 (3 : 2), found 373, 375 (3 : 2); ¹H NMR (300 MHz, CD₃OD) δ 7.35 (d, *J =* 1.6 Hz, 1H), 6.88 (s, 1H), 4.74 (t, *J =* 5.4 Hz, 1H), 4.54 (s, 1H), 4.09 (t, *J =* 13.1 Hz, 1H), 3.31-3.25 (m, 1H), 3.18-2.83 (m, 5H), 2.58 (t, *J =* 12.8 Hz, 1H), 2.20-1.81 (m, 4H), 1.53 (t, *J =* 14.1 Hz, 1H), 1.44-1.19 (m, 2H).

### Step e:

4,5-Dichloro-2-[hydroxy([1-[(3R)-pyrrolidine-3-carbonyl]piperidin-4-yl])methyl]phenol (55 mg, 0.14 mmol) was separated by Chiral Prep-HPLC with the following conditions: Column: Chiralpak IC, 2 x 25 cm, 5 µm; Mobile Phase A: Hexane (0.2% IPA), Mobile Phase B: EtOH; Flow rate: 20 mL/min; Gradient: 20% B to 20% B in 26 min; Detector: UV 220 nm; Retention time: RT₁: 14.035 min; RT₂: 20.088 min; Temperature: 25 °C.

The faster-eluting enantiomer was obtained as Compound 116 (4,5-dichloro-2-[(R)-hydroxy([1-[(3*R*)-pyrrolidine-3-carbonyl]piperidin-4-yl])methyl]phenol) as an off-white solid (10 mg, 18%) at 14.035 min: LCMS (ESI) calc'd for C₁₇H₂₂Cl₂N₂O₃ [M + H]⁺: 373, 375 (3 : 2), found 373, 375 (3 : 2); ¹H NMR (300 MHz, CD₃OD) δ 7.35 (d, *J* = 1.6 Hz, 1H), 6.88 (s, 1H), 4.74 (t, *J =* 5.4 Hz, 1H), 4.54 (s, 1H), 4.09 (t, *J =* 13.1 Hz, 1H), 3.31-3.25 (m, 1H), 3.18-2.83 (m, 5H), 2.58 (t, *J =* 12.8 Hz, 1H), 2.20-1.81 (m, 4H), 1.53 (t, *J =* 14.1 Hz, 1H), 1.44-1.19 (m, 2H).

The slower-eluting enantiomer was obtained as Compound 115 (4,5-dichloro-2-[(S)-hydroxy([1-[(3R)-pyrrolidine-3-carbonyl]piperidin-4-yl])methyl]phenol) as an off-white solid (21 mg, 21%) at 20.088 min: LCMS (ESI) calc'd for C₁₇H₂₂Cl₂N₂O₃ [M + H]⁺: 373, 375 (3 : 2), found 373, 375 (3 : 2); ¹H NMR (300 MHz, CD₃OD) δ 7.35 (d, *J* = 1.6 Hz, 1H), 6.88 (s, 1H), 4.74 (t, *J =* 5.4 Hz, 1H), 4.54 (s, 1H), 4.09 (t, *J =* 13.1 Hz, 1H), 3.31-3.25 (m, 1H), 3.18-2.83 (m, 5H), 2.58 (t, *J =* 12.8 Hz, 1H), 2.20-1.81 (m, 4H), 1.53 (t, *J =* 14.1 Hz, 1H), 1.44-1.19 (m, 2H).

### Example 150. Compound 102 ((S)-1-(4-((R)-amino(4,5-dichloro-2-hydroxyphenyl)methyl)piperidin-1-yl)-2-hydroxypropan-1-one)

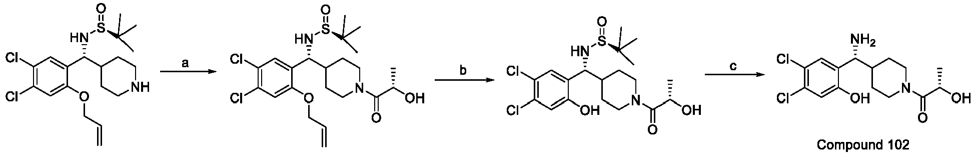

### Step a:

To a stirred solution of (S)-2-hydroxypropanoic acid (0.19 g, 2.16 mmol) and Et₃N (0.22 g, 2.16 mmol) in DMF (3 mL) was added CDI (0.29 g, 2.09 mmol) in portions at room temperature under nitrogen atmosphere. After stirring for 1 h at room temperature under nitrogen atmosphere, a solution of (S)-N-((R)-(2-(allyloxy)-4,5-dichlorophenyl)(piperidin-4-yl)methyl)-2-methylpropane-2-sulfinamide (0.30 g, 0.72 mmol) in DMF (1 mL) was added dropwise to resulting solution. After addition, the reaction solution was stirred for additional 12 h under nitrogen atmosphere at room temperature. The resulting solution was diluted with water (50 mL) at room temperature and extracted with EA (3 x 50 mL). The combined organic layers were washed with brine (2 x 10 mL), dried over anhydrous Na₂SO₄. After filtration, the filtrate was concentrated under reduced pressure. The residue was purified by Prep-TLC, eluted with PE/EA (1/3) to afford (*S*)-*N*-((*R*)-(2-(allyloxy)-4,5-dichlorophenyl)(1-((S)-2-hydroxypropanoyl)piperidin-4-yl)methyl)-2-methylpropane-2-sulfinamide as a yellow solid (0.10 g, 29%): LCMS (ESI) calc'd for C₂₂H₃₂Cl₂N₂O₄S [M + H]⁺: 491, 493 (3 : 2), found 491, 493 (3 : 2).

### Step b:

To a stirred solution of (*S*)-*N*-((*R*)-(2-(allyloxy)-4,5-dichlorophenyl)(1-((S)-2-hydroxypropanoyl)piperidin-4-yl)methyl)-2-methylpropane-2-sulfinamide (0.10 g, 0.20 mmol) and Pd(PPh₃)₄ (22 mg, 0.02 mmol) in THF (4 mL) was added NaBH₄ (11 mg, 0.30 mmol) at room temperature under nitrogen atmosphere. The reaction mixture was stirred for 1 h at room temperature under nitrogen atmosphere. The resulting mixture was quenched with water (40 mL) at room temperature and extracted with EA (3 x 40 mL). The combined organic layers were washed with brine (3 x 20 mL), dried over anhydrous Na₂SO₄. After filtration, the filtrate was concentrated under reduced pressure to afford crude (*S*)-*N*-((*R*)-(4,5-dichloro-2-hydroxyphenyl)(1-((*S*)-2-hydroxypropanoyl)piperidin-4-yl)methyl)-2-methylpropane-2-sulfinamide. The crude product was used in the next step directly without further purification: LCMS (ESI) calc'd for C₁₉H₂₈Cl₂N₂O₄S [M + H]⁺: 451, 453 (3 : 2), found 451, 453 (3 : 2).

### Step c:

To a stirred solution of (*S*)-*N*-((*R*)-(4,5-dichloro-2-hydroxyphenyl)(1-((S)-2-hydroxypropanoyl)piperidin-4-yl)methyl)-2-methylpropane-2-sulfinamide (crude) in 1,4-dioxane (5 mL) was added aq. HCl (6 *N,* 3 mL) at room temperature. The reaction solution was stirred for 1 h at room temperature. The reaction solution was concentrated under reduced pressure. The residue was purified by Prep-HPLC with the following conditions: Column: XBridge C18 OBD Prep Column, 100 Å, 10 µm, 19 mm x 250 mm; Mobile Phase A: water with 10 mmol/L NH₄HCO₃, Mobile Phase B: ACN; Flow rate: 25 mL/min; Gradient:: 20% B to 50% B in 6 min; Detector: UV 254/210 nm; Retention time: 5.58 min. The fractions containing desired product were collected and concentrated under reduce pressure to afford Compound 102 ((S)-1-(4-((R)-amino(4,5-dichloro-2-hydroxyphenyl)methyl)piperidin-1-yl)-2-hydroxypropan-1-one) as an off-white solid (10.0 mg, 14% overall two steps): LCMS (ESI) calc'd for C₁₅H₂₀Cl₂N₂O₃ [M + H]⁺: 347, 349 (3 : 2), found 347, 349 (3 : 2); ¹H NMR (400 MHz, CD₃OD) δ 7.22 (s, 1H), 6.88 (s, 1H), 4.63-4.46 (m, 2H), δ 4.04 (dd, *J =* 13.8, 35.1 Hz, 1H), 3.90 (t, *J =* 6.5 Hz, 1H), 3.12-2.93 (m, 1H), 2.74-2.51 (m, 1H), 2.10-1.90 (m, 2H), 1.50 (d, *J =* 13.4 Hz, 1H), 1.31 (d, *J =* 6.6 Hz, 3H), 1.32-1.08 (m, 2H).

### Example 151. Compound 104 (2-[(R)-amino([1-[(3R)-1-methylpyrrolidine-3-carbonyl]piperidin-4-yl])methyl]-4,5-dichlorophenol)

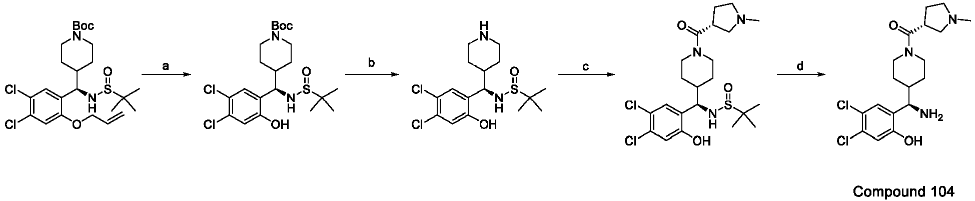

### Step a:

To a stirred solution of *tert*-butyl 4-[(*R*)-[4,5-dichloro-2-(prop-2-en-1-yloxy)phenyl][(2-methylpropane-2-sulfinyl)amino]methyl]piperidine-1-carboxylate (0.25 g, 0.48 mmol) and Pd(PPh₃)₄ (6 mg, 0.005 mmol) in THF (2 mL) was added NaBH₄ (36 mg, 0.96 mmol) at room temperature under nitrogen atmosphere. After stirring for 1 h at room temperature under nitrogen atmosphere, the resulting mixture was quenched with water (2 mL) at room temperature and concentrated under reduced pressure. The residue was purified by reverse phase chromatography, eluted with 70% MeOH in water with 20% NH₄HCO₃ to afford tert-butyl 4-[(R)-(4,5-dichloro-2-hydroxyphenyl)[(2-methylpropane-2-sulfinyl)amino]methyl]piperidine-1-carboxylate as a yellow oil (0.15 g, 65%): LCMS (ESI) calc'd for C₂₁H₃₂Cl₂N₂O₄S [M + H]⁺: 479, 481 (3 : 2), found 479, 481 (3 : 2).

### Step b:

To a stirred solution of *tert*-butyl 4-[(*R*)-(4,5-dichloro-2-hydroxyphenyl)[(2-methylpropane-2-sulfinyl)amino]methyl]piperidine-1-carboxylate (0.15 g, 0.31 mmol) in DCM (2 mL) was added TFA (0.5 mL) at room temperature. The reaction solution was stirred for 30 min at room temperature. The resulting solution was neutralized to pH 8 with saturated aq. NaHCO₃ and concentrated under reduced pressure. The residue was purified by reverse phase chromatography, with 50% ACN in water with 10% NH₄HCO₃ to afford N-[(R)-(4,5-dichloro-2-hydroxyphenyl)(piperidin-4-yl)methyl]-2-methylpropane-2-sulfinamide as a yellow oil (80 mg, 67%): LCMS (ESI) calc'd for C₁₆H₂₄Cl₂N₂O₂S [M + H]⁺: 379, 381 (3 : 2), found 379, 381 (3 : 2); ¹H NMR (300 MHz, CD₃OD) δ 7.18 (s, 1H), 6.83 (s, 1H), 4.26 (d, *J =* 8.8 Hz, 1H), 3.20 (d, *J* = 12.7 Hz, 1H), 3.04 (d, *J =* 12.7 Hz, 1H), 2.76-2.51 (m, 2H), 2.21 (d, *J =* 13.8 Hz, 1H), 2.08-1.90 (m, 1H), 1.48-1.23 (m, 3H), 1.14 (s, 9H).

### Step c:

To a stirred solution of EDCI (61 mg, 0.32 mmol) and (3R)-1-methylpyrrolidine-3-carboxylic acid (25 mg, 0.19 mmol) in DMF (2 mL) were added Et₃N (32 mg, 0.32 mmol) and N-[(R)-(4,5-dichloro-2-hydroxyphenyl)(piperidin-4-yl)methyl]-2-methylpropane-2-sulfinamide (60 mg, 0.16 mmol) at room temperature under nitrogen atmosphere. After stirring for 16 h at room temperature under nitrogen atmosphere, the resulting solution was quenched with water (30 mL) and extracted with EA (3 x 30 mL). The combined organic layers were washed with brine (2 x 20 mL), dried over anhydrous Na₂SO₄. After filtration, the filtrate was concentrated under reduced pressure to afford crude *N*-((*R*)-(4,5-dichloro-2-hydroxyphenyl)(1-((R)-1-methylpyrrolidine-3-carbonyl)piperidin-4-yl)methyl)-2-methylpropane-2-sulfinamide. The crude product was used in the next step directly without further purification: LCMS (ESI) calc'd for C₂₂H₃₃Cl₂N₃O₃S [M + H]⁺: 490, 492 (3 : 2), found 490, 492 (3 : 2).

### Step d:

To a stirred solution of N-((R)-(4,5-dichloro-2-hydroxyphenyl)(1-((R)-1-methylpyrrolidine-3-carbonyl)piperidin-4-yl)methyl)-2-methylpropane-2-sulfinamide (crude) in 1,4-dioxane (2 mL) was added aq. HCl (6 *N,* 1 mL) at room temperature. The reaction solution was stirred for 1 h at room temperature. The resulting solution was concentrated under reduced pressure. The residue was purified by Prep-HPLC with the following conditions: Column: XBridge C18 OBD Prep Column 100 Å, 10 µm, 19 mm x 250 mm; Mobile Phase A: water with 10 mmol/L NH₄HCO₃, Mobile Phase B: ACN; Flow rate: 25 mL/min; Gradient: 30% B to 80% B in 6 min; Detector: UV 254/210 nm; Retention time: 4.42 min. The fractions containing desired product were collected and concentrated under reduced pressure to afford Compound 104 (2-[(R)-amino([1-[(3R)-1-methylpyrrolidine-3-carbonyl]piperidin-4-yl])methyl]-4,5-dichlorophenol) as an off-white solid (13 mg, 33% overall two steps): LCMS (ESI) calc'd for C₁₈H₂₅Cl₂N₃O₂ [M + H]⁺: 386, 388 (3 : 2), found 386, 388 (3 : 2); ¹H NMR (300 MHz, CD₃OD) δ 7.22 (s, 1H), 6.87 (s, 1H), 4.57 (dd, *J =* 29.1, 13.3 Hz, 1H), 4.03 (dd, *J =* 27.8, 13.8 Hz, 1H), 3.90 (t, *J =* 7.2 Hz, 1H), 3.35-3.30 (m, 1H), 3.16-2.86 (m, 2H), 2.84-2.51 (m, 4H), 2.45-2.39 (m, 3H), 2.21-1.90 (m, 4H), 1.57-1.07 (m, 3H).

### Example 152. Compound 105 ((R)-1-(4-((R)-amino(4,5-dichloro-2-hydroxyphenyl)methyl)piperidin-1-yl)-2-hydroxypropan-1-one)

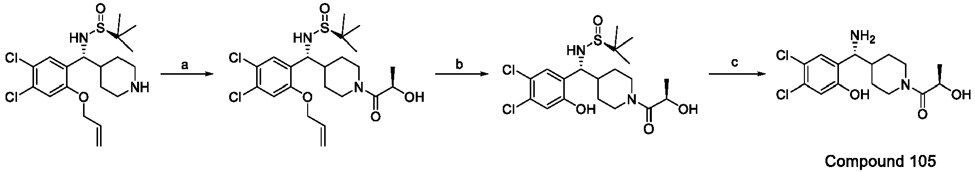

### Step a:

To a stirred solution of (2*R*)-2-hydroxypropanoic acid (86 mg, 0.96 mmol) and Et₃N (97 mg, 0.95 mmol) in DMF (3 mL) was added CDI (0.15 g, 0.95 mmol) in portions at room temperature under nitrogen atmosphere. After stirring for 1 h at room temperature under nitrogen atmosphere, a solution of (*S*)-*N*-((*R*)-(2-(allyloxy)-4,5-dichlorophenyl)(piperidin-4-yl)methyl)-2-methylpropane-2-sulfinamide (0.20 g, 0.48 mmol) in DMF (1 mL) was added dropwise to resulting solution. After addition, the reaction solution was stirred for additional 12 h under nitrogen atmosphere at room temperature. The resulting solution was diluted with water (50 mL) and extracted with EA (3 x 40 mL). The combined organic layers were washed with brine (2 x 20 mL), dried over anhydrous Na₂SO₄ and filtered. The filtrate was concentrated under reduce pressure. The residue was purified by reverse phase chromatography, eluted with 60% ACN in water with 20% NH₄HCO₃ to afford (*S*)-*N*-((*R*)-(2-(allyloxy)-4,5-dichlorophenyl)(1-((*R*)-2-hydroxypropanoyl)piperidin-4-yl)methyl)-2-methylpropane-2-sulfinamide as a yellow solid (0.12 g, 46%): LCMS (ESI) calc'd for C₂₂H₃₂Cl₂N₂O₄S [M + H]⁺: 491, 493 (3 : 2), found 491, 493 (3 : 2); ¹H NMR (400 MHz, CD₃OD): δ 7.45 (s, 1H), 7.18 (s, 1H), 6.18-5.96 (m, 1H), 5.52-5.30 (dd, *J* = 44.4, 14.0 Hz, 2H), 4.70-4.62 (d, *J* = 5.2 Hz, 2H), 4.59-4.51 (m, 3H), 4.10 (dd, *J* = 55.6, 13.7 Hz, 1H), 3.15-2.87 (m, 1H), 2.75-2.57 (m, 1H), 2.30-2.00 (m, 2H), 1.52-1.20 (m, 6H), 1.10 (s, 9H).

### Step b:

To a stirred solution of (*S*)-*N*-((*R*)-(2-(allyloxy)-4,5-dichlorophenyl)(1-((*R*)-2-hydroxypropanoyl)piperidin-4-yl)methyl)-2-methylpropane-2-sulfinamide (0.12 g, 0.19 mmol) and Pd(PPh₃)₄ (22 mg, 0.02 mmol) in THF (4 mL) was added NaBH₄ (11 mg, 0.30 mmol) at room temperature under nitrogen atmosphere. The reaction mixture was stirred for 1 h at room temperature under nitrogen atmosphere. The resulting mixture was quenched with water (40 mL) at room temperature and extracted with EA (3 x 40 mL). The combined organic layers were washed with brine (3 x 20 mL), dried over anhydrous Na₂SO₄. After filtration, the filtrate was concentrated under reduced pressure to afford crude (*S*)-*N*-((*R*)-(4,5-dichloro-2-hydroxyphenyl)(1-((*R*)-2-hydroxypropanoyl) piperidin-4-yl)methyl)-2-methylpropane-2-sulfinamide. The crude product was used in the next step directly without further purification: LCMS (ESI) calc'd for C₁₉H₂₈Cl₂N₂O₄S [M + H]⁺: 451, 453 (3 : 2), found 451, 453 (3 : 2).

### Step c:

To a stirred solution of (*S*)-*N*-((*R*)-(4,5-dichloro-2-hydroxyphenyl)(1-((*R*)-2-hydroxypropanoyl)piperidin-4-yl)methyl)-2-methylpropane-2-sulfinamide (crude) in 1,4-dioxane (5 mL) was added aq. HCl (6 *N*, 3 mL) at room temperature. The reaction solution was stirred for 1 h at room temperature. The reaction solution was concentrated under reduced pressure. The residue was purified by Prep-HPLC with the following conditions: Column: Atlantis HILIC OBD Column, 19 x 150 mm, 5 µm; Mobile Phase A: water with 10 mmol/L NH₄HCO₃+0.1%NH₃·H₂O, Mobile Phase B: ACN; Flow rate: 20 mL/min; Gradient: 30% B to 65% B in 6 min; Detector: UV 210 nm; Retention time: 5.00 min. The fractions containing desired product were collected and concentrated under reduce pressure to afford Compound 105 ((*R*)-1-(4-((*R*)-amino(4,5-dichloro-2-hydroxyphenyl)methyl)piperidin-1-yl)-2-hydroxypropan-1-one) as an off-white solid (3.7 mg, 5% overall two steps): LCMS (ESI) calc'd for C₁₅H₂₀Cl₂N₂O₃ [M + H]⁺: 347, 349 (3 : 2), found 347, 349 (3 : 2); ¹H NMR (400 MHz, CD₃OD) δ 7.22 (s, 1H), 6.88 (s, 1H), 4.63-4.46 (m, 2H), δ 4.04 (dd, *J* = 13.8, 35.1 Hz, 1H), 3.90 (t, *J* = 6.5 Hz, 1H), 3.12-2.93 (m, 1H), 2.74-2.51 (m, 1H), 2.10-1.90 (m, 2H), 1.50 (d, *J* = 13.4 Hz, 1H), 1.35-1.08 (m, 5H).

### Example 153. Compound 106 ((4-((R)-amino(4,5-dichloro-2-hydroxyphenyl)methyl)piperidin-1-yl)((S)-1-methylpyrrolidin-3-yl)methanone)

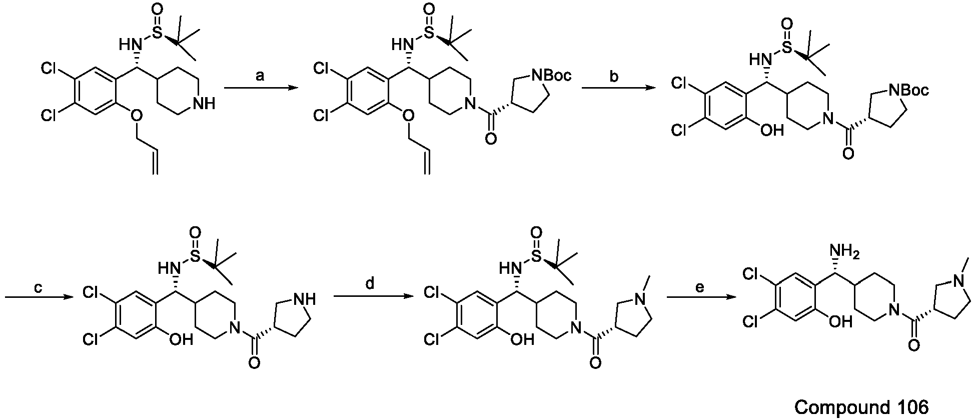

### Step a:

To a stirred solution of (*S*)-*N*-[(*R*)-[4,5-dichloro-2-(prop-2-en-1-yloxy)phenyl](piperidin-4-yl)methyl]-2-methylpropane-2-sulfinamide (0.50 g, 1.19 mmol) and (3*S*)-1-[(*tert*-butoxy)carbonyl]pyrrolidine-3-carboxylic acid (0.51 g, 2.38 mmol) in DMF (5 mL) were added Et₃N (0.24 g, 2.38 mmol) and EDCI (0.46 g, 2.38 mmol) at room temperature under nitrogen atmosphere. The reaction solution was stirred for 2 h at room temperature under nitrogen atmosphere. The resulting solution was quenched with water (50 mL) at room temperature and extracted with EA (3 x 50 mL). The combined organic layers were washed with brine (2 x 10 mL), dried over anhydrous Na₂SO₄. After filtration, the filtrate was concentrated under reduced pressure. The residue was purified by reverse phase chromatography, eluted with 10% to 50% ACN in water with 10 mmol/L NH₄HCO₃ to afford *tert*-butyl (3*S*)-3-[4-[(*R*)-[4,5-dichloro-2-(prop-2-en-1-yloxy)phenyl]([[(*S*)-2-methylpropane-2-sulfinyl]amino])methyl]piperidine-1-carbonyl]pyrrolidine-1-carboxylate as a yellow solid (0.35 g, 43%): LCMS (ESI) calc'd for C₂₉H₄₃Cl₂N₃O₅S [M + H]⁺: 616, 618 (3 : 2), found 616, 618 (3 : 2).

### Step b:

To a stirred solution *tert*-butyl (3*S*)-3-[4-[(*R*)-[4,5-dichloro-2-(prop-2-en-1-yloxy)phenyl]([[(*S*)-2-methylpropane-2-sulfinyl]amino])methyl]piperidine-1-carbonyl]pyrrolidine-1-carboxylate (0.30 g, 0.49 mmol) and Pd(PPh₃)₄ (11 mg, 0.01 mmol) in THF (5 mL) was added NaBH₄ (74 mg, 1.95 mmol) in portions at room temperature under nitrogen atmosphere. The reaction mixture was stirred for 2 h at room temperature under nitrogen atmosphere. The resulting mixture was quenched with water (40 mL) at room temperature and extracted with EA (3 x 40 mL). The combined organic layers were washed with brine (3 x 20 mL), dried over anhydrous Na₂SO₄. After filtration, the filtrate was concentrated under reduced pressure. The residue was purified by reverse phase chromatography, eluted with 50% ACN in water with 10 mmol/L NH₄HCO₃ to afford tert-butyl (3*S*)-3-[4-[(4,5-dichloro-2-hydroxyphenyl)[(2-methylpropane-2-sulfinyl) amino]methyl]piperidine-1-carbonyl]pyrrolidine-1-carboxylate as a yellow oil (0.20 g, 71%): LCMS (ESI) calc'd for C₂₆H₃₉Cl₂N₃O₅S [M + H]⁺: 576, 578 (3 : 2), found 576, 578 (3 : 2).

### Step c:

To a stirred solution of *tert*-butyl (3*S*)-3-[4-[(4,5-dichloro-2-hydroxyphenyl)[(2-methylpropane-2-sulfinyl)amino]methyl]piperidine-1-carbonyl]pyrrolidine-1-carboxylate (0.20 g, 0.35 mmol) in DCM (5 mL) was added TFA (1 mL) dropwise at room temperature. The reaction solution was stirred for 1 h at room temperature. The resulting solution was neutralized with saturated aq. NH₄HCO₃ at 0 °C. The pH value was adjusted to 7. The aqueous layer was extracted with EA (5 x 50 mL). The combined organic layers were dried over anhydrous Na₂SO₄ and filtered. The filtrate was concentrated under reduce pressure. The residue was purified by reverse phase chromatography, eluted with 40% ACN in water with 20 mmol/L NH₄HCO₃ to afford (*S*)-*N*-((*R*)-(4,5-dichloro-2-hydroxyphenyl)(1-((*S*)-pyrrolidine-3-carbonyl)piperidin-4-yl)methyl)-2-methylpropane-2-sulfinamide as a yellow solid (0.10 g, 39%): LCMS (ESI) calc'd for C₂₁H₃₁Cl₂N₃O₃S [M + H]⁺: 476, 478 (3 : 2), found 476, 478 (3 : 2); ¹H NMR (400 MHz, CDCl₃) δ 7.14 (d, *J* = 13.7 Hz, 1H), 7.01 (d, *J* = 13.3 Hz, 1H), 4.62 (dd, *J* = 50.6, 13.2 Hz, 1H), 4.31 (dd, *J* = 39.0, 7.8 Hz, 1H), 4.09-3.87 (m, 1H), 3.30-2.87 (m, 6H), 2.59 - 2.44 (m, 1H), 2.12 - 1.91 (m, 4H), 1.63-1.43 (m, 1H), 1.31-1.13 (m, 11H).

### Step d:

To a stirred solution of (*S*)-*N*-((*R*)-(4,5-dichloro-2-hydroxyphenyl)(1-((*S*)-pyrrolidine-3-carbonyl)piperidin-4-yl)methyl)-2-methylpropane-2-sulfinamide (0.10 g, 0.21 mmol) and formaldehyde (9.5 mg, 0.32 mmol) in MeOH (2 mL) was added HOAc (13 mg, 0.21 mmol). After stirring for 1 h at room temperature, NaBH(AcO)₃ (89 mg, 0.42 mmol) was added in portions at room temperature. The reaction mixture was stirred for additional 1 h at room temperature. The resulting mixture was neutralized with saturated aq. NH₄HCO₃, and extracted with EA (5 x 50 mL). The combined organic layers were concentrated under reduced pressure to afford crude (*S*)-*N*-((*R*)-(4,5-dichloro-2-hydroxyphenyl)(1-((*S*)-1-methylpyrrolidine-3-carbonyl)piperidin-4-yl)methyl)-2-methylpropane-2-sulfinamide. The crude product was used in the next step directly without further purification: LCMS (ESI) calc'd for C₂₂H₃₃Cl₂N₃O₃S [M + H]⁺: 490, 492 (3 : 2), found 490, 492 (3 : 2).

### Step e:

To a stirred solution of (*S*)-*N*-((*R*)-(4,5-dichloro-2-hydroxyphenyl)(1-((*S*)-1-methylpyrrolidine-3-carbonyl)piperidin-4-yl)methyl)-2-methylpropane-2-sulfinamide (crude) in 1,4-dioxane (2 mL) was added aq. HCl (6 *N*, 1 mL) at room temperature. The reaction solution was stirred for 1 h at room temperature. The resulting solution was concentrated under reduce pressure. The residue was purified by Prep-HPLC with the following conditions: Column: XBridge C18 OBD Prep Column, 100 Å, 5 µm, 19 mm x 250 mm; Mobile Phase A: water with 10 mmol/L NH₄HCO₃, Mobile Phase B: ACN; Flow rate: 25 mL/min; Gradient: 20% B to 80% B in 6 min; Detector: UV 254/210 nm; Retention time: 4.72 min. The fractions containing desired product were collected and concentrated under reduced pressure to afford Compound 106 ((4-((*R*)-amino(4,5-dichloro-2-hydroxyphenyl)methyl)piperidin-1-yl)((*S*)-1-methylpyrrolidin-3-yl)methanone) as an off-white solid (21.5 mg, 25% overall two steps): LCMS (ESI) calc'd for C₁₈H₂₅Cl₂N₃O₂ [M + H]⁺: 386, 388 (3 : 2), found 386, 388 (3 : 2); ¹H NMR (400 MHz, CD₃OD) δ 7.22 (d, *J* = 2.0 Hz, 1H), 6.86 (d, *J* = 1.1 Hz, 1H), 4.56 (dd, *J* = 28.9, 13.2 Hz, 1H), 4.03 (dd, *J* = 28.1, 13.7 Hz, 1H), 3.89 (t, *J* = 6.7 Hz, 1H), 3.30-3.23 (m, 1H), 3.14-2.88 (m, 2H), 2.81-2.46 (m, 4H), 2.44-2.32 (s, 3H), 2.20-1.94 (m, 4H), 1.62-1.03 (m, 3H).

### Example 154. Compound 107 (2-[(R)-amino[1-(azetidine-3-sulfonyl)piperidin-4-yl]methyl]-4,5-dichlorophenol)

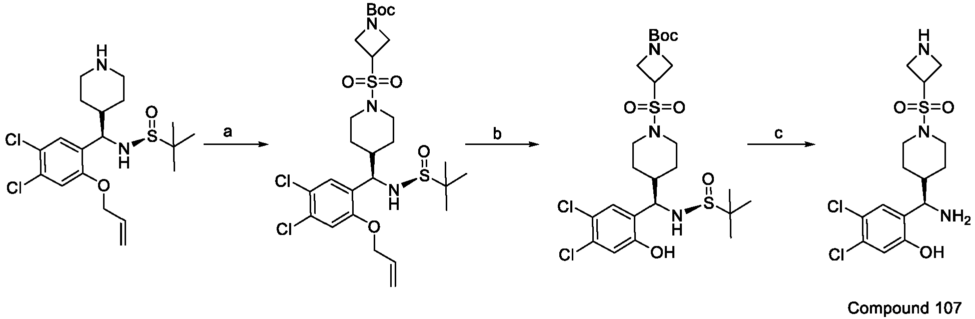

### Step a:

To a stirred solution of *N*-[(*R*)-[4,5-dichloro-2-(prop-2-en-1-yloxy)phenyl](piperidin-4-yl)methyl]-2-methylpropane-2-sulfinamide (0.20 g, 0.48 mmol) and Et₃N (97 mg, 0.96 mmol) in DCM (2 mL) was added *tert*-butyl 3-(chlorosulfonyl)azetidine-1-carboxylate (0.15 g, 0.59 mmol) in portions at room temperature under nitrogen atmosphere. The resulting mixture was stirred for 3 h at room temperature under nitrogen atmosphere. The resulting mixture was diluted with water (50 mL) and extracted with EA (3 x 30 mL). The combined organic layers were washed with water (3 x 10 mL), dried over anhydrous Na₂SO₄. After filtration, the filtrate was concentrated under reduced pressure. The residue was purified by Prep-TLC, eluted with PE/EA (5/1) to afford *tert*-butyl 3-([4-[(*R*)-[4,5-dichloro-2-(prop-2-en-1-yloxy)phenyl][(2-methylpropane-2-sulfinyl)amino]methyl]piperidin-1-yl]sulfonyl)azetidine-1-carboxylate as an off-white solid (0.12 g, 39%): LCMS (ESI) calc'd for C₂₇H₄₁Cl₂N₃O₆S₂ [M + H]⁺: 638, 640 (3:: 2), found 638, 640 (3 : 2); ¹H NMR (400 MHz, CDCl₃) δ 7.21 (s, 1H), 6.99 (s, 1H), 6.09-5.96 (m, 1H), 5.49-5.34 (m, 2H), 4.64-4.52 (m, 2H), 4.43 (s, 1H), 4.28-4.09 (m, 4H), 3.86 (d, *J* = 35.3 Hz, 3H), 2.73 (dd, *J* = 22.9, 11.6 Hz, 2H), 1.89 (s, 1H), 1.47 (s, 9H), 1.38-1.25 (m, 4H), 1.17 (s, 9H).

### Step b:

To a stirred solution of *tert*-butyl 3-([4-[(*R*)-[4,5-dichloro-2-(prop-2-en-1-yloxy)phenyl][(2-methylpropane-2-sulfinyl)amino]methyl]piperidin-1-yl]sulfonyl)azetidine-1-carboxylate (0.12 g, 0.19 mmol) and Pd(PPh₃)₄ (21 mg, 0.02 mmol) in THF (2 mL) was added NaBH₄ (25 mg, 0.66 mmol) in portions at room temperature under nitrogen atmosphere. The resulting mixture was stirred for 6 h at room temperature under nitrogen atmosphere. The resulting mixture was quenched with water (40 mL) at room temperature and extracted with EA (3 x 30 mL). The combined organic layers were washed with water (3 x 10 mL), dried over anhydrous Na₂SO₄. After filtration, the filtrate was concentrated under reduced pressure. The residue was purified by Prep-TLC, eluted with PE/EA (5/1) to afford *tert*-butyl 3-([4-[(*R*)-(4,5-dichloro-2-hydroxyphenyl)[(2-methylpropane-2-sulfinyl) amino]methyl]piperidin-1-yl]sulfonyl)azetidine-1-carboxylate as a light yellow solid (80 mg, 71%): LCMS (ESI) calc'd for C₂₄H₃₇Cl₂N₃O₆S₂ [M + H]⁺: 598, 600 (3 : 2), found 598, 600 (3 : 2).

### Step c:

To a stirred solution of *tert*-butyl 3-([4-[(*R*)-(4,5-dichloro-2-hydroxyphenyl)[(2-methylpropane-2-sulfinyl)amino]methyl]piperidin-1-yl]sulfonyl)azetidine-1-carboxylate (80 mg, 0.15 mmol) in 1,4-dioxane (5 mL) was added aq. HCl (6 *N*, 3 mL) at room temperature. The reaction solution was stirred for 1 h at room temperature. The reaction solution was concentrated under reduced pressure. The residue was dissolved in DCM (6 mL), and then TFA (3 mL) was added at room temperature. The reaction solution was stirred for 1 h at room temperature. The resulting solution was concentrated under reduced pressure. The residue was purified by Prep-HPLC with the following conditions: Column: XBridge C18 OBD Prep Column, 100 Å, 10 µm, 19 mm x 250 mm; Mobile Phase A: water (plus 0.05% TFA), Mobile Phase B:ACN; Flow rate: 25 mL/min; Gradient: 20% B to 35% B in 12 min; Detector: 210 nm; Retention time: 9.5 min. The fractions containing the desired product were collected and concentrated under reduced pressure to afford Compound 107 (2-[(*R*)-amino[1-(azetidine-3-sulfonyl)piperidin-4-yl]methyl]-4,5-dichlorophenol) as an off-white solid (40 mg, 67%): LCMS (ESI) calc'd for C₁₅H₂₁Cl₂N₃O₃S [M + H]⁺: 394, 396 (3 : 2), found 394, 396 (3 : 2); ¹H NMR (300 MHz, CD₃OD) δ 7.22 (s, 1H), 6.86 (s, 1H), 4.37-4.26 (m, 1H), 4.01-3.87 (m, 3H), 3.87-3.65 (m, 4H), 2.86-2.63 (m, 2H), 2.10-1.76 (m, 2H), 1.56-1.15 (m, 3H).

### Example 155. Compound 108 (N-((4,5-dichloro-2-hydroxyphenyl)(1-((R)-pyrrolidine-3-carbonyl)piperidin-4-yl)methyl)acetamide trifluoroacetic acid)

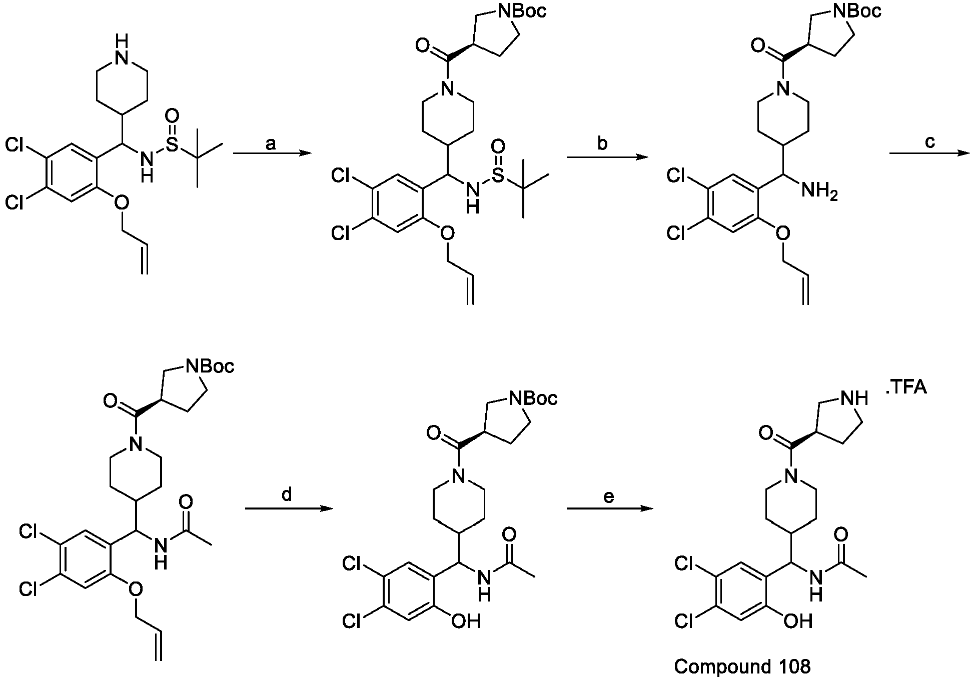

### Step a:

To a stirred mixture of (3*R*)-1-[(*tert*-butoxy)carbonyl]pyrrolidine-3-carboxylic acid (0.46 g, 2.15 mmol) in DMF (2 mL) was added EDCI (0.41 g, 2.15 mmol) at room temperature. After stirring for 10 min, Et₃N (0.43 g, 4.31 mmol) and *N*-[[4,5-dichloro-2-(prop-2-en-1-yloxy)phenyl](piperidin-4-yl)methyl]-2-methylpropane-2-sulfinamide (0.60 g, 1.44 mmol) were added to resulting solution at room temperature. The reaction solution was stirred at room temperature at room temperature for additional 16 h. The resulting solution was diluted with water (40 mL) and extracted with EA (3 x 50 mL). The combined organic layers were washed with brine (2 x 20 mL), dried over anhydrous Na₂SO₄ and filtered. The filtrate was concentrated under reduced pressure. The residue was purified by reverse phase chromatography, eluted with 75% ACN in water (plus 0.05% TFA) to afford *tert*-butyl (3*R*)-3-(4-[[4,5-dichloro-2-(prop-2-en-1-yloxy)phenyl][(2-methylpropane-2-sulfinyl)amino]methyl]piperidine-1-carbonyl)pyrrolidine-1-carboxylate as a light yellow solid (0.40 g, 45%): LCMS (ESI) calc'd for C₂₉H₄₃Cl₂N₃O₅S [M + H]⁺: 616, 618 (3 : 2), found 616, 618 (3 : 2).

### Step b:

To a stirred solution of *tert*-butyl (3*R*)-3-(4-[[4,5-dichloro-2-(prop-2-en-1-yloxy)phenyl][(2-methylpropane-2-sulfinyl)amino]methyl]piperidine-1-carbonyl)pyrrolidine-1-carboxylate (0.40 g, 0.39 mmol) in 1,4-dioxane (2 mL) was added aq. HCl (4 *N*, 0.67 mL) at room temperature. The reaction solution was stirred at room temperature for 30 min. The resulting solution was adjusted pH value to 7 with saturated aq. NaHCO₃. The resulting aqueous solution was extracted with EA (3 x 50 mL). The combined organic layers were washed with brine (2 x 20 mL), dried over anhydrous Na₂SO₄ and filtered. The filtrate was concentrated under reduced pressure to afford *tert-*butyl (3*R*)-3-(4-[amino[4,5-dichloro-2-(prop-2-en-1-yloxy)phenyl]methyl]piperidine-1-carbonyl)pyrrolidine-1-carboxylate as a light yellow solid (0.22 g, crude). LCMS (ESI) calc'd for C₂₅H₃₅Cl₂N₃O₄ [M + H]⁺: 512, 514 (3 : 2), found 512, 514 (3 : 2).

### Step c:

To a stirred solution of *tert*-butyl (3*R*)-3-(4-[amino[4,5-dichloro-2-(prop-2-en-1-yloxy)phenyl]methyl]piperidine-1-carbonyl)pyrrolidine-1-carboxylate (0.20 g, 0.39 mmol) and Et₃N (0.13 g, 1.29 mmol) in DCM (2 mL) was added Ac₂O (88 mg, 0.86 mmol) at room temperature. The reaction solution was stirred at room temperature for 1 h. The resulting solution was quenched with water (50 mL) at room temperature and extracted with EA (3 x 30 mL). The combined organic layers were washed with brine (2 x 20 mL), dried over anhydrous Na₂SO₄ and filtered. The filtrate was concentrated under reduced pressure. The residue was purified by Prep-TLC, eluted with EA to afford *tert*-butyl (3*R*)-3-(4-[[4,5-dichloro-2-(prop-2-en-1-yloxy)phenyl](acetamido)methyl]piperidine-1-carbonyl)pyrrolidine-1-carboxylate as an off-white solid (80 mg, 37% overall 2 steps): LCMS (ESI) calc'd for C₂₇H₃₇Cl₂N₃O₅ [M + H]⁺: 554, 556 (3 : 2), found 554, 556 (3 : 2).

### Step d:

To a solution of *tert*-butyl (3*R*)-3-(4-[[4,5-dichloro-2-(prop-2-en-1-yloxy)phenyl](acetamido)methyl]piperidine-1-carbonyl)pyrrolidine-1-carboxylate (80 mg, 0.14 mmol) and Pd(PPh₃)₄ (3.3 mg, 0.002 mmol) in THF (2 mL) was added NaBH₄ (8.3 mg, 0.22 mmol) at room temperature under nitrogen atmosphere. The reaction mixture was stirred for 1 h at room temperature under nitrogen atmosphere. The resulting mixture was quenched with water (40 mL) at room temperature and extracted with EA (3 x 30 mL). The combined organic layers were washed with brine (2 x 20 mL), dried over anhydrous Na₂SO₄ and filtered. The filtrate was concentrated under reduced pressure to afford *tert*-butyl (3*R*)-3-[4-[(4,5-dichloro-2-hydroxyphenyl)(acetamido)methyl]piperidine -1-carbonyl]pyrrolidine-1-carboxylate as a light yellow solid (74 mg, crude), which was directly used in next step without further purification. LCMS (ESI) calc'd for C₂₄H₃₃Cl₂N₃O₅ [M + H]⁺: 514, 516 (3 : 2), found 514, 516 (3 : 2).

### Step e:

To a stirred solution of *tert*-butyl (3*R*)-3-[4-[(4,5-dichloro-2-hydroxyphenyl)(acetamido)methyl]piperidine- 1-carbonyl]pyrrolidine-1-carboxylate (72 mg, 0.14 mmol) in DCM (1 mL) was added TFA (1 mL) at room temperature. The reaction solution was stirred at room temperature for 0.5 h at room temperature. The resulting solution was concentrated under reduced pressure. The residue was purified by Prep-HPLC with the following conditions: Column: XSelect CSH Prep C18 OBD Column, 5 µm, 19 x 150 mm; Mobile Phase A: water (plus 0.05% TFA), Mobile Phase B: ACN; Flow rate: 25 mL/min; Gradient: 20% B to 30% B in 6 min; Detector: UV 210 nm; Retention time: 4.60 min. The fractions containing product were collected and concentrated under reduced pressure to afford Compound 108 (*N*-((4,5-dichloro-2-hydroxyphenyl)(1-((*R*)-pyrrolidine-3-carbonyl)piperidin-4-yl)methyl)acetamide trifluoroacetic acid) as an off-white solid (66 mg, 85% over 2 steps): LCMS (ESI) calc'd for C₁₉H₂₅Cl₂N₃O₃ [M + H]⁺: 414, 416 (3 : 2), found 414, 416 (3 : 2). ¹H NMR (300 MHz, CD₃OD) δ 7.33 (d, *J* = 1.7 Hz, 1H), 6.95 (s, 1H), 5.07-4.93 (m, 1H), 4.49 (dd, *J* = 28.4, 13.4 Hz, 1H), 4.02 (dd, *J* = 25.5, 13.8 Hz, 1H), 3.73-3.54 (m, 2H), 3.42-3.34 (m, 3H), 3.20-2.98 (m, 1H), 2.75-2.55 (m, 1H), 2.46-2.25 (m, 1H), 2.22-1.84 (m, 6H), 1.55-1.09 (m, 3H); ¹⁹F NMR (282 MHz, CD₃OD) δ -77.05.

### Example 156. (which is a reference example) Compound 111 (4,5-dichloro-2-([1-[(3R)-pyrrolidine-3-carbonyl]piperidin-4-yl]methyl)phenol trifluoroacetic acid)

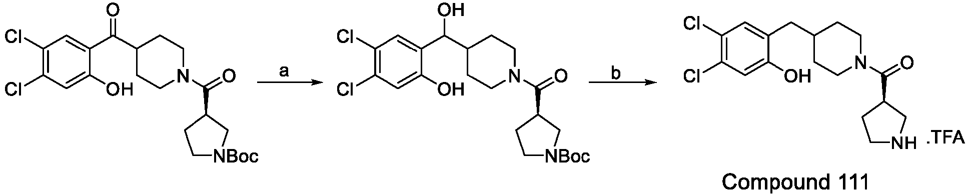

### Step a:

To a stirred solution of *tert*-butyl (3*R*)-3-[4-(4,5-dichloro-2-hydroxybenzoyl)piperidine-1-carbonyl]pyrrolidine-1-carboxylate (0.35 g, 0.74 mmol) in MeOH (3 mL) was added NaBH₄ (56 mg, 1.48 mmol) at room temperature under nitrogen atmosphere. The resulting mixture was stirred for 2 h at room temperature under nitrogen atmosphere. The reaction was quenched with water (50 mL) at room temperature. The resulting mixture was extracted with EA (3 x 30 mL). The combined organic layers were washed with brine (3 x 10 mL), dried over anhydrous Na₂SO₄. After filtration, the filtrate was concentrated under reduced pressure. The residue was purified by silica gel column chromatography, eluted with PE/EA (1/2) to afford *tert*-butyl (3*R*)-3-[4-[(4,5-dichloro-2-hydroxyphenyl)(hydroxy)methyl]piperidine-1-carbonyl]pyrrolidine-1-carboxylate as a light yellow oil (0.20 g, 57%): LCMS (ESI) calc'd for C₂₂H₃₀Cl₂N₂O₅ [M + H]⁺: 473, 475 (3: 2), found 473, 475 (3: 2). ¹H NMR (400 MHz, CDCl₃) δ 7.03 (d, *J* = 11.3 Hz, 2H), 4.73-4.49 (m, 2H), 4.04-3.82 (m, 1H), 3.73-3.61 (m, 1H), 3.59-3.47 (m, 3H), 3.47-3.29 (m, 2H), 3.27-2.94 (m, 2H), 2.68-2.16 (m, 2H), 1.53-1.42 (m, 3H).

### Step b:

To a stirred solution of *tert*-butyl (3*R*)-3-[4-[(4,5-dichloro-2-hydroxyphenyl)(hydroxy)methyl]piperidine-1-carbonyl]pyrrolidine-1-carboxylate (0.17 g, 0.36 mmol) and Et₃SiH (0.25 g, 2.15 mmol) in DCM (1 mL, 15.73 mmol) was added BF₃.Et₂O (0.41 g, 2.86 mmol) at room temperature under nitrogen atmosphere. The resulting mixture was stirred for 8 h at 50 °C under nitrogen atmosphere. After cooling to room temperature, the reaction was quenched with water (10 mL) at room temperature. The resulting mixture was concentrated under reduced pressure. The residue was purified by Prep-HPLC with the following conditions: Column: XBridge C18 OBD Prep Column 100 Å, 10 µm, 19 mm x 250 mm; Mobile Phase A: water (plus 0.05% TFA), Mobile Phase B: ACN; Flow rate: 25 mL/min; Gradient: 20% B to 35% B in 6 min; Detector: UV 210 nm; Retention time: 4.7 min. The fractions containing the desired product were collected and concentrated under reduced pressure to afford Compound 111 (4,5-dichloro-2-([1-[(3*R*)-pyrrolidine-3-carbonyl]piperidin-4-yl]methyl)phenol trifluoroacetic acid) as an off-white solid (10 mg, 8%): LCMS (ESI) calc'd for C₁₇H₂₂Cl₂N₂O₂ [M + H]⁺: 357, 359 (3 : 2), found 357, 359 (3 : 2). ¹H NMR (400 MHz, CD₃OD) δ 7.18 (d, *J* = 2.0 Hz, 1H), 6.91 (s, 1H), 4.61-4.44 (m, 1H), 4.00 (d, *J* = 13.7 Hz, 1H), 3.74-3.57 (m, 2H), 3.43-3.35 (m, 3H), 3.18-3.06 (m, 1H), 2.73-2.58 (m, 1H), 2.59-2.47 (m, 2H), 2.43-2.30 (m, 1H), 2.15-2.00 (m, 1H), 1.99-1.85 (m, 1H), 1.81-1.65 (m, 2H), 1.36-1.07 (m, 2H); ¹⁹F NMR (376 MHz, CD₃OD) δ -76.96.

### Example 157. Compound 114 (2-[(R)-amino[1-(3-fluoroazetidine-3-carbonyl)piperidin-4-yl]methyl]-4,5-dichlorophenol)

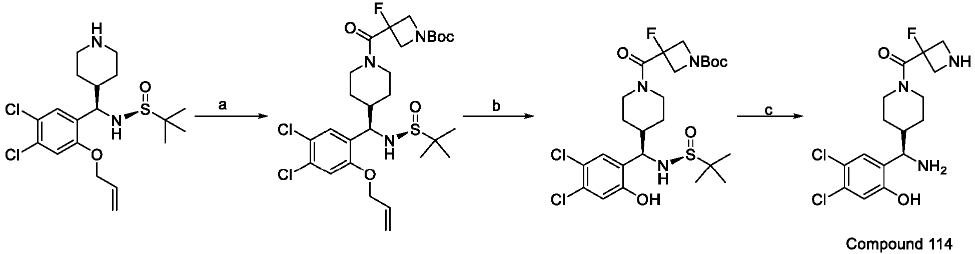

### Step a:

To a stirred mixture of 1-[(*tert*-butoxy)carbonyl]-3-fluoroazetidine-3-carboxylic acid (0.21 g, 0.95 mmol) in DMF (2 mL) was added CDI (0.15 g, 0.95 mmol) at room temperature. After stirred 30 min, to the resulting solution was added *N*-[(*R*)-[4,5-dichloro-2-(prop-2-en-1-yloxy)phenyl](piperidin-4-yl)methyl]-2-methylpropane-2-sulfinamide (0.20 g, 0.48 mmol) at room temperature. Then the reaction was stirred at room temperature for additional 2 h. The resulting solution was diluted with water (30 mL) and extracted with EA (3 x 30 mL). The combined organic layers were washed with brine (2 x 20 mL), dried over anhydrous Na₂SO₄ and filtered. The filtrate was concentrated under reduced pressure. The residue was purified by Prep-TLC, eluted with PE/EA (2/1) to afford *tert*-butyl 3-[4-[(*R*)-[4,5-dichloro-2-(prop-2-en-1-yloxy)phenyl][(2-methylpropane-2-sulfinyl)amino]methyl]piperidine-1-carbonyl]-3-fluoroazetidine-1-carboxylate as a light yellow solid (0.11 g, 25%): LCMS (ESI) calc'd for C₂₈H₄₀Cl₂FN₃O₅S [M + H]⁺: 620, 622 (3 : 2), found 620, 622 (3 : 2).

### Step b:

To a stirred solution of *tert*-butyl 3-[4-[(*R*)-[4,5-dichloro-2-(prop-2-en-1-yloxy)phenyl][(2-methylpropane-2-sulfinyl)amino]methyl]piperidine-1-carbonyl]-3-fluoroazetidine-1-carboxylate (0.11 g, 0.17 mmol) and Pd(PPh₃)₄ (4.0 mg, 0.003 mmol) in THF (2 mL) was added NaBH₄ (13 mg, 0.35 mmol) at room temperature under nitrogen atmosphere. After stirring for 1 h at room temperature under nitrogen atmosphere, the resulting mixture was quenched with water (30 mL) and extracted with EA (3 x 30 mL). The combined organic layers were washed with brine (2 x 20 mL), dried over anhydrous Na₂SO₄ and filtered. The filtrate was concentrated under reduced pressure to afford *tert*-butyl 3-[4-[(*R*)-(4,5-dichloro-2-hydroxyphenyl)[(2-methylpropane-2-sulfinyl)amino]methyl]piperidine-1-carbonyl]-3-fluoroazetidine-1-carboxylate as a light yellow solid (0.12 g, crude), which was directly used in next step without further purification: LCMS (ESI) calc'd for C₂₅H₃₆Cl₂FN₃O₅S [M + H]⁺: 580, 582 (3 : 2), found 580, 582 (3 : 2).

### Step c:

To a stirred solution of *tert*-butyl 3-[4-[(*R*)-(4,5-dichloro-2-hydroxyphenyl)[(2-methylpropane-2-sulfinyl)amino]methyl]piperidine-1-carbonyl]-3-fluoroazetidine-1-carboxylate (98 mg, 0.17 mmol) in 1,4-dioxane (5 mL) was added aq. HCl (6 *N*, 3 mL) at room temperature. The reaction solution was stirred for 1 h at room temperature. The reaction solution was concentrated under reduced pressure. The residue was dissolved in DCM (6 mL), and then TFA (3 mL) was added at room temperature. The reaction solution was stirred for 1 h at room temperature. The resulting solution was concentrated under reduced pressure. The residue was purified by Prep-HPLC with the following conditions: Column: X Bridge C18 OBD Prep Column 100 Å, 10 µm, 19 mm x 250 mm; Mobile Phase A: water with 20 mmol/L NH₄HCO₃, Mobile Phase B: ACN; Flow rate: 20 mL/min; Gradient: 30% B to 80% B in 9 min; Detector: UV 254/220 nm; Retention time: 7.74 min. The fractions containing the desired product were collected and concentrated under reduced pressure to afford Compound 114 (2-[(*R*)-amino[1-(3-fluoroazetidine-3-carbonyl)piperidin-4-yl]methyl]-4,5-dichlorophenol) as an off-white solid (35 mg, 54% overall two steps): LCMS (ESI) calc'd for C₁₆H₂₀Cl₂FN₃O₂ [M + H]⁺: 376, 378 (3 : 2), found 376, 378 (3 : 2); ¹H NMR (400 MHz, CD₃OD) δ 7.22 (s, 1H), 6.80 (s, 1H), 4.51 (d, *J* = 35.4, 9.8 Hz, 1H), 4.30-4.00 (m, 2H), 3.98-3.60 (m, 4H), 3.13-2.90 (m, 1H), 2.80-256 (m, 1H), 2.14-1.95 (m, 2H), 1.55-1.22 (m, 3H); ¹⁹F NMR (376 MHz, CD₃OD) δ -148.94.

### Example 158. Compound 119 ((R)-1-(4-((R)-amino(4,5-dichloro-2-hydroxyphenyl)methyl)piperidin-1-yl)-2,3-dihydroxypropan-1-one); Compound 120 ((S)-1-(4-((R)-amino(4,5-dichloro-2-hydroxyphenyl)methyl)piperidin-1-yl)-2,3-dihydroxypropan-1-one)

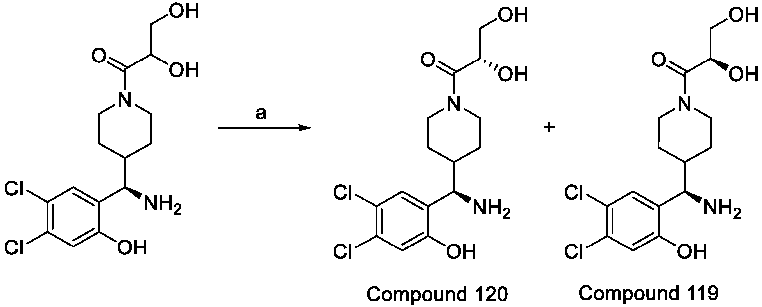

### Step a:

1-(4-((*R*)-amino(4,5-dichloro-2-hydroxyphenyl)methyl)piperidin-1-yl)-2,3-dihydroxypropan-1-one (Compound 117, Example 145) (35 mg) was separated by Chiral-Prep-HPLC with the following conditions: Column: CHIRALPAK IG, 2 x 25 cm, 5 µm; Mobile Phase A: MTBE with 10 mmol/L NH₃/MeOH, Mobile Phase B: MeOH; Flow rate: 20 mL/min; Gradient: 10% B to 10% B in 14 min; Detector: UV 220/254 nm; Retention time: RT₁: 7.47 min; RT₂: 10.05 min.

The faster-eluting enantiomer was obtained as Compound 120 ((*S*)-1-(4-((*R*)-amino(4,5-dichloro-2-hydroxyphenyl)methyl)piperidin-1-yl)-2,3-dihydroxypropan-1-one) as an off-white solid (10.2 mg, 29%) at 7.47 min: LCMS (ESI) calc'd for C₁₅H₂₀Cl₂N₂O₄ [M + H]⁺: 363, 365 (3 : 2), found 363, 365 (3 : 2); ¹H NMR (400 MHz, CD₃OD) δ 7.22 (s, 1H), 6.87 (s, 1H), 4.68-4.47 (m, 2H), 4.11 ((dd, *J* = 40.3, 13.8 Hz, 1H), 3.94-3.86 (m, 1H), 3.76-3.53 (m, 2H), 3.20-2.94 (m, 1H), 2.74-2.55 (m, 1H), 2.16-1.92 (m, 2H), 1.57-1.43 (m, 1H), 1.41-1.13 (m, 2H).

The slower-eluting enantiomer was obtained as compound 119 ((*R*)-1-(4-((*R*)-amino(4,5-dichloro-2-hydroxyphenyl)methyl)piperidin-1-yl)-2,3-dihydroxypropan-1-one) as an off-white solid (10.1 mg, 29%) at 10.05 min: LCMS (ESI) calc'd for C₁₅H₂₀Cl₂N₂O₄ [M + H]⁺: 363, 365 (3 : 2), found 363, 365 (3 : 2); ¹H NMR (400 MHz, CD₃OD) δ 7.22 (s, 1H), 6.87 (s, 1H), 4.68-4.47 (m, 2H), 4.11 ((dd, *J* = 40.3, 13.8 Hz, 1H), 3.94-3.86 (m, 1H), 3.76-3.53 (m, 2H), 3.20-2.94 (m, 1H), 2.74-2.55 (m, 1H), 2.16-1.92 (m, 2H), 1.57-1.43 (m, 1H), 1.41-1.13 (m, 2H).

### Example 159. (which is a reference example) Compound 122 (3,4-dichloro-2-([1-[(3R)-pyrrolidine-3-carbonyl]piperidin-4-yl]methyl)phenol trifluoroacetic acid)

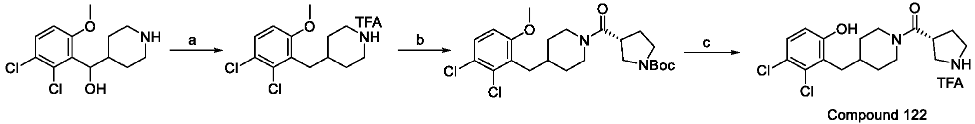

### Step a:

To a solution of (2,3-dichloro-6-methoxyphenyl)(piperidin-4-yl)methanol (Intermediate 44' free base, Example 44) (0.30 g, 1.03 mmol) and Et₃SiH (2.49 g, 21.54 mmol) in DCM (3 mL) was added BF₃.Et₂O (1.47 g, 10.34 mmol) dropwise at room temperature under nitrogen atmosphere. The reaction mixture was stirred at room temperature for 16 h. The resulting mixture was quenched with water (10 mL) and concentrated under reduced pressure. The residue was purified by reverse phase flash chromatography, eluted with 45% ACN in water (plus 0.05% TFA) to afford 4-[(2,3-dichloro-6-methoxyphenyl) methyl]piperidine trifluoroacetic acid as an off-white foam (0.22 g, 57%): LCMS (ESI) calc'd for C₁₃H₁₇Cl₂NO [M + H]⁺: 274, 276 (3 : 2), found 274, 276 (3 : 2); ¹H NMR (400 MHz, CD₃OD) δ 7.40 (d, *J* = 8.9 Hz, 1H), 6.97 (d, *J* = 8.9 Hz, 1H), 3.87 (s, 3H), 3.42-3.35 (m, 2H), 3.01-2.84 (m, 4H), 2.13-1.91 (m, 1H), 1.90-1.77 (m, 2H), 1.65-1.50 (m, 2H).

### Step b:

To a solution of (3*R*)-1-[(*tert*-butoxy)carbonyl]pyrrolidine-3-carboxylic acid (0.35 g, 1.60 mmol) in DMF (5 mL) was added CDI (0.26 g, 1.60 mmol) at room temperature. The mixture was stirred at room temperature for 0.5 h. Then Et₃N (0.73 g, 7.19 mmol) and 4-[(2,3-dichloro-6-methoxyphenyl)methyl]piperidine trifluoroacetic acid (0.220 g, 0.80 mmol) were added at room temperature. The reaction mixture was stirred at room temperature for additional 1 h. The reaction was quenched with water (50 mL) and extracted with EA (3 x 10 mL). The combined organic layers were washed with brine (3 x 10 mL), dried over anhydrous Na₂SO₄. After filtration, the filtrate was concentrated under reduced pressure. The residue was purified by silica gel column chromatography, eluted with PE/EA (3/2) to afford *tert*-butyl (3*R*)-3-[4-[(2,3-dichloro-6-methoxyphenyl)methyl]piperidine-1-carbonyl]pyrrolidine-1-carboxylate as an off-white foam (0.20 g, 72%): LCMS (ESI) calc'd for C₂₃H₃₂Cl₂N₂O₄ [M + H]⁺: 471, 473 (3 : 2), found 471, 473 (3 : 2); ¹H NMR (400 MHz, CDCl₃) δ 7.31 (d, *J* = 8.9 Hz, 1H), 6.75 (d, *J* = 8.9 Hz, 1H), 4.59 (s, 1H), 3.83 (s, 1H), 3.80 (s, 3H), 3.72-3.42 (m, 3H), 3.35 (q, *J* = 8.7 Hz, 1H), 3.26-3.13 (m, 1H), 3.11-2.91 (m, 1H), 2.81 (d, *J* = 7.2 Hz, 2H), 2.56 (s, 1H), 2.22 (s, 1H), 2.10-1.82 (m, 2H), 1.67 (s, 1H), 1.48 (s, 9H), 1.37-1.22 (m, 3H).

### Step c:

To a solution of *tert*-butyl (3*R*)-3-[4-[(2,3-dichloro-6-methoxyphenyl)(hydroxy)methyl]piperidine-1-carbonyl]pyrrolidine-1-carboxylate (0.20 g, 0.41 mmol) in DCM (5 mL) was added BBr₃ (1.57 g, 6.35 mmol) at room temperature. The reaction mixture was stirred at room temperature for 3 h. The reaction was quenched with water (5 mL) and concentrated under reduced pressure. The residue was purified by Prep-HPLC with the following conditions: Column: XBridge C18 OBD Prep Column, 19 mm x 250 mm, 5 µm; Mobile Phase A: water (plus 0.05% TFA), Mobile Phase B: ACN; Flow rate: 25 mL/min; Gradient: 30% B to 40% B in 6 min; Detector: UV 210 nm; Retention time: 5.11 min. The fractions containing the desired product were collected and concentrated under reduced pressure to afford Compound 122 (3,4-dichloro-2-([1-[(3*R*)-pyrrolidine-3-carbonyl]piperidin-4-yl]methyl)phenol trifluoroacetic acid) as an off-white solid (109.8 mg, 57%): LCMS (ESI) calc'd for C₁₇H₂₂Cl₂N₂O₂ [M + H]⁺: 357, 359 (3 : 2), found 357, 359 (3 : 2); ¹H NMR (400 MHz, CD₃OD) δ 7.20 (d, *J* = 8.7 Hz, 1H), 6.74 (d, *J* = 8.7 Hz, 1H), 4.55-4.41 (m, 1H), 4.00 (d, *J* = 13.6 Hz, 1H), 3.71-3.58 (m, 2H), 3.45-3.34 (m, 3H), 3.16-3.04 (m, 1H), 2.82 (d, *J* = 7.2 Hz, 2H), 2.66 (t, *J* = 12.6 Hz, 1H), 2.46-2.27 (m, 1H), 2.16-1.96 (m, 2H), 1.83-1.64 (m, 2H), 1.45-1.21 (m, 2H); ¹⁹F NMR (376 MHz, CD₃OD) δ -77.04.

### Example 160. Compound 123 (3,4-dichloro-2-[(R)-hydroxy([1-[(3R)-pyrrolidine-3-carbonyl]piperidin-4-yl])methyl]phenol trifluoroacetic acid); Compound 321 (3,4-dichloro-2-[(S)-hydroxy([1-[(3R)-pyrrolidine-3-carbonyl]piperidin-4-yl])methyl]phenol trifluoroacetic acid)

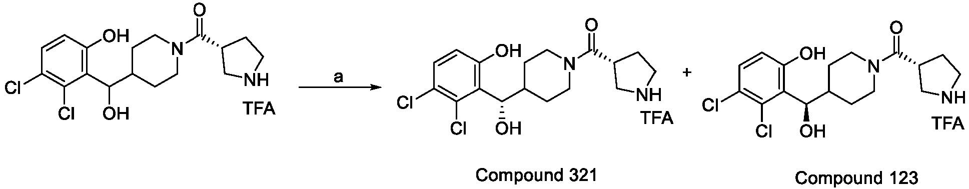

### Step a:

3,4-Dichloro-2-[hydroxy([1-[(3*R*)-pyrrolidine-3-carbonyl]piperidin-4-yl])methyl]phenol trifluoroacetic acid (147.5 mg, 0.40 mmol) was separated by Prep-chiral HPLC with the following conditions: Column: Chiralpak IA, 2 x 25 cm, 5 µm; Mobile Phase A: Hexane (plus 0.1% TFA), Mobile Phase B: EtOH; Flow rate: 20 mL/min; Gradient: 20% B to 20% B in 20 min; Detector: UV 220 nm; Retention time: RT₁: 6.39 min; RT₂: 13.31 min.

The faster-eluting enantiomer was obtained as Compound 123 (3,4-dichloro-2-[(*R*)-hydroxy([1-[(3*R*)-pyrrolidine-3-carbonyl]piperidin-4-yl])methyl]phenol trifluoroacetic acid) as a light pink solid (52 mg, 33%) at 6.39 min: LCMS (ESI) calc'd for C₁₇H₂₂Cl₂N₂O₃ [M + H]⁺: 373, 375 (3 : 2), found 373, 375 (3 : 2); ¹H NMR (400 MHz, CD₃OD) δ 7.31 (d, *J* = 8.8 Hz, 1H), 6.77 (d, *J* = 8.8 Hz, 1H), 5.11 (dd, *J* = 6.8, 3.3 Hz, 1H), 4.58 (t, *J* = 15.6 Hz, 1H), 4.07 (dd, *J* = 24.8, 13.9 Hz, 1H), 3.75-3.58 (m, 2H), 3.46-3.34 (m, 3H), 3.15-3.04 (m, 1H), 2.70-2.54 (m, 1H), 2.44-2.27 (m, 1H), 2.27-2.16 (m, 1H), 2.16-1.93 (m, 2H), 1.59-1.26 (m, 3H); ¹⁹F NMR (376 MHz, CD₃OD) δ -77.35.

The slower-eluting enantiomer was obtained as Compound 321 (3,4-dichloro-2-[(*S*)-hydroxy([1-[(3*R*)-pyrrolidine-3-carbonyl]piperidin-4-yl])methyl]phenol trifluoroacetic acid) as a light pink solid (59 mg, 38%) at 13.31min: LCMS (ESI) calc'd for C₁₇H₂₂Cl₂N₂O₃ [M + H]⁺: 373, 375 (3 : 2), found 373, 375 (3 : 2); ¹H NMR (400 MHz, CD₃OD) δ 7.31 (d, *J* = 8.8 Hz, 1H), 6.77 (dd, *J* = 8.8, 2.9 Hz, 1H), 5.12 (dd, *J* = 6.6, 2.0 Hz, 1H), 4.58 (dd, *J* = 22.9, 13.3 Hz, 1H), 4.14-3.96 (m, 1H), 3.73-3.57 (m, 2H), 3.45-3.34 (m, 3H), 3.20-3.03 (m, 1H), 2.71-2.53 (m, 1H), 2.45-2.27 (m, 1H), 2.27-2.15 (m, 1H), 2.15-1.91 (m, 2H), 1.59-1.24 (m, 3H); ¹⁹F NMR (376 MHz, CD₃OD) δ -77.21.

### Example 161. Compound 124 (4,5-dichloro-2-(hydroxy(1-(pyrrolidin-3-ylsulfonyl)piperidin-4-yl)methyl)phenol isomer 1);

### Compound 125 (4,5-dichloro-2-(hydroxy(1-(pyrrolidin-3-ylsulfonyl)piperidin-4-yl)methyl)phenol isomer 2);

### Compound 121 (4,5-dichloro-2-(hydroxy(1-(pyrrolidin-3-ylsulfonyl)piperidin-4-yl)methyl)phenol isomer 3);

### Compound 127 (4,5-dichloro-2-(hydroxy(1-(pyrrolidin-3-ylsulfonyl)piperidin-4-yl)methyl)phenol isomer 4)

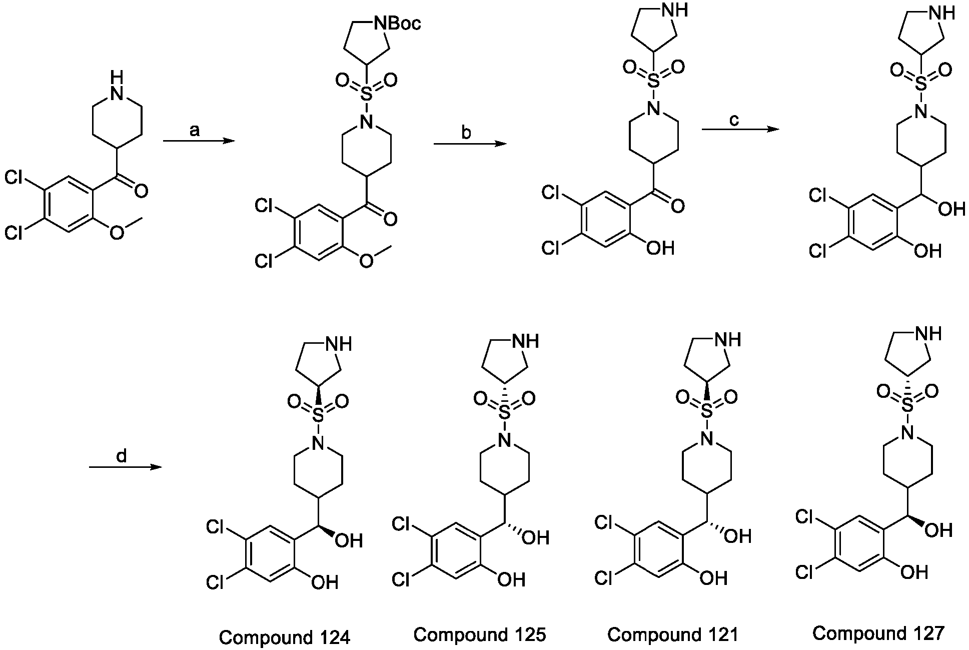

### Step a:

To a stirred solution of Et₃N (0.35 g, 3.47 mmol) and 4-(4,5-dichloro-2-methoxybenzoyl)piperidine (0.50 g, 1.74 mmol) in DCM (5 mL) was added *tert*-butyl 3-(chlorosulfonyl)pyrrolidine-1-carboxylate (0.70 g, 2.60 mmol) at room temperature under nitrogen atmosphere. After stirring for 30 min at room temperature under nitrogen atmosphere, the resulting mixture was quenched with water (50 mL) and extracted with EA (3 x 50 mL). The combined organic layers were washed with brine (2 x 20 mL), dried over anhydrous Na₂SO₄. The filtration, the filtrate was concentrated under reduced pressure. The residue was purified by Prep-TLC, eluted with PE/EA (2/1) to afford *tert*-butyl 3-[[4-(4,5-dichloro-2-methoxybenzoyl)piperidin-1-yl]sulfonyl]pyrrolidine-1-carboxylate as a light yellow oil (0.65 g, 72%): LCMS (ESI) calc'd for C₂₂H₃₀Cl₂N₂O₆S [M + H]⁺: 521, 523 (3 : 2), found 521, 523 (3 : 2); ¹H NMR (400 MHz, CD₃OD) δ 7.68 (s, 1H), 7.36 (s, 1H), 3.97 (s, 3H), 3.97-3.89 (m, 1H), 3.82 (d, *J* = 12.5 Hz, 2H), 3.66 (d, *J* = 6.8 Hz, 2H), 3.60-3.54 (m, 1H), 3.50-3.39 (m, 2H), 3.05 (m, *J* = 12.0 Hz, 2H), 2.37-2.24 (m, 2H), 1.97 (d, *J* = 13.3 Hz, 2H), 1.72-1.60 (m, 2H), 1.484 (s, 9H).

### Step b:

To a stirred solution of *tert-*butyl 3-[[4-(4,5-dichloro-2-methoxybenzoyl)piperidin-1-yl]sulfonyl]pyrrolidine-1-carboxylate (0.65 g, 1.25 mmol) in DCM (4 mL) was added BBr₃ (2.50 g, 9.97 mmol) at room temperature under nitrogen atmosphere. After stirring for 1 h at room temperature under nitrogen atmosphere, the resulting mixture was quenched with water (5 mL) at room temperature. The precipitated solids were formed and collected by filtration. The filter cake was washed with water (3 x 5 mL) and dried in a vacuum oven to afford 4,5-dichloro-2-[1-(pyrrolidine-3-sulfonyl)piperidine-4-carbonyl]phenol as an off-white solid (0.45 g, 88%): LCMS (ESI) calc'd for C₁₆H₂₀Cl₂N₂O₄S [M + H]⁺: 407, 409 (3 : 2), found 407, 409 (3 : 2); ¹H NMR (400 MHz, CD₃OD) δ 8.10 (s, 1H), 7.18 (s, 1H), 4.21-4.10 (m, 1H), 3.90 (d, *J* = 12.5 Hz, 2H), 3.74-3.66 (m, 3H), 3.58-3.40 (m, 2H), 3.19 (t, *J* = 13.0 Hz, 2H), 2.52-2.42 (m, 2H), 2.00 (d, *J* = 13.5 Hz, 2H), 1.86-1.71 (m, 2H).

### Step c:

To a stirred solution of 4,5-dichloro-2-[1-(pyrrolidine-3-sulfonyl)piperidine-4-carbonyl]phenol (0.45 g, 1.10 mmol) in MeOH (10 mL) was added NaBH₄ (63 mg, 1.66 mmol) at room temperature under nitrogen atmosphere. After stirring for additional 1 h at room temperature, the resulting mixture was quenched with water (2 mL) at room temperature and concentrated under reduced pressure. The residue was purified by Prep-HPLC with the following conditions: Column: XBridge Shield RP18 OBD Column 30 x 150 mm, 5 µm; Mobile Phase A: water with 10 mmol/L NH₄HCO₃, Mobile Phase B: ACN; Flow rate: 60 mL/min; Gradient: 27% B to 38% B in 7 min; Detector: UV 254/220 nm; Retention time: 6.92 min. The fractions containing desired product were collected and concentrated under reduced pressure to afford 4,5-dichloro-2-(hydroxy(1-(pyrrolidin-3-ylsulfonyl)piperidin-4-yl)methyl)phenol as an off-white solid (0.40 g, 88%): LCMS (ESI) calc'd for C₁₆H₂₂Cl₂N₂O₄S [M + H]⁺: 409, 411 (3 : 2), found 409, 411 (3 : 2); ¹H NMR (400 MHz, CD₃OD) δ 7.39 (s, 1H), 6.91 (s, 1H), 4.77 (d, *J* = 6.2 Hz, 1H), 3.89-3.68 (m, 3H), 3.21-3.12 (m, 2H), 3.09-3.02 (m, 1H), 2.96-2.77 (m, 3H), 2.20-2.00 (m, 2H), 1.92-1.78 (m, 2H), 1.59-1.39 (m, 3H).

### Step d:

4,5-dichloro-2-(hydroxy(1-(pyrrolidin-3-ylsulfonyl)piperidin-4-yl)methyl)phenol (0.40 g, 0.98 mmol) was separated by Chiral Prep-HPLC with the following conditions: Column: CHIRALPAK AD-H, 2.0 cm x 25 cm; Mobile Phase A: Hexane (0.1%DEA), Mobile Phase B: EtOH; Flow rate: 18 mL/min; Gradient: 50% B to 50% B in 19 min; Detector: UV 220/254 nm; Retention time: RT₁: 7.42 min; RT₂: 10.38 min; RT₃: 13.56 min; RT₄: 15.99 min.

The first enantiomer was obtained at 7.42 min as Compound 124 (4,5-dichloro-2-(hydroxy(1-(pyrrolidin-3-ylsulfonyl)piperidin-4-yl)methyl)phenol isomer 1) as an off-white solid (30.4 mg, 8%): LCMS (ESI) calc'd for C₁₆H₂₂Cl₂N₂O₄S [M + H]⁺: 409, 411 (3 : 2), found 409, 411 (3 : 2); ¹H NMR (400 MHz, CD₃OD) δ 7.39 (s, 1H), 6.91 (s, 1H), 4.77 (d, *J* = 6.2 Hz, 1H), 3.89-3.68 (m, 3H), 3.21-3.12 (m, 2H), 3.09-3.02 (m, 1H), 2.96-2.77 (m, 3H), 2.20-2.00 (m, 2H), 1.92-1.78 (m, 2H), 1.59-1.39 (m, 3H).

The second enantiomer was obtained at 10.38 min as Compound 125 (4,5-dichloro-2-(hydroxy(1-(pyrrolidin-3-ylsulfonyl)piperidin-4-yl)methyl)phenol isomer 2) as an off-white solid (29.8 mg, 7%):LCMS (ESI) calc'd for C₁₆H₂₂Cl₂N₂O₄S [M + H]⁺: 409, 411 (3 : 2), found 409, 411 (3 : 2); ¹H NMR (400 MHz, CD₃OD) δ 7.39 (s, 1H), 6.91 (s, 1H), 4.77 (d, *J* = 6.2 Hz, 1H), 3.89-3.68 (m, 3H), 3.21-3.12 (m, 2H), 3.09-3.02 (m, 1H), 2.96-2.77 (m, 3H), 2.20-2.00 (m, 2H), 1.92-1.78 (m, 2H), 1.59-1.39 (m, 3H).

Th third enantiomer was obtained at 13.56 min as Compound 121 (4,5-dichloro-2-(hydroxy(1-(pyrrolidin-3-ylsulfonyl)piperidin-4-yl)methyl)phenol isomer 3) as an off-white solid (29.3 mg, 7%):LCMS (ESI) calc'd for C₁₆H₂₂Cl₂N₂O₄S [M + H]⁺: 409, 411 (3 : 2), found 409, 411 (3 : 2); ¹H NMR (400 MHz, CD₃OD) δ 7.39 (s, 1H), 6.91 (s, 1H), 4.77 (d, *J* = 6.2 Hz, 1H), 3.89-3.68 (m, 3H), 3.21-3.12 (m, 2H), 3.09-3.02 (m, 1H), 2.96-2.77 (m, 3H), 2.20-2.00 (m, 2H), 1.92-1.78 (m, 2H), 1.59-1.39 (m, 3H).

The fourth enantiomer was obtained at 15.99 min as Compound 127 (4,5-dichloro-2-(hydroxy(1-(pyrrolidin-3-ylsulfonyl)piperidin-4-yl)methyl)phenol isomer 4) as an off-white solid (27.5 mg, 7%):LCMS (ESI) calc'd for C₁₆H₂₂Cl₂N₂O₄S [M + H]⁺: 409, 411 (3 : 2), found 409, 411 (3 : 2); ¹H NMR (400 MHz, CD₃OD) δ 7.39 (s, 1H), 6.91 (s, 1H), 4.77 (d, *J* = 6.2 Hz, 1H), 3.89-3.68 (m, 3H), 3.21-3.12 (m, 2H), 3.09-3.02 (m, 1H), 2.96-2.77 (m, 3H), 2.20-2.00 (m, 2H), 1.92-1.78 (m, 2H), 1.59-1.39 (m, 3H).

### Example 162. Compound 126 (3,4-dichloro-2-[hydroxy([1-[(3R)-pyrrolidine-3-carbonyl]piperidin-4-yl])methyl]phenol trifluoroacetic acid)

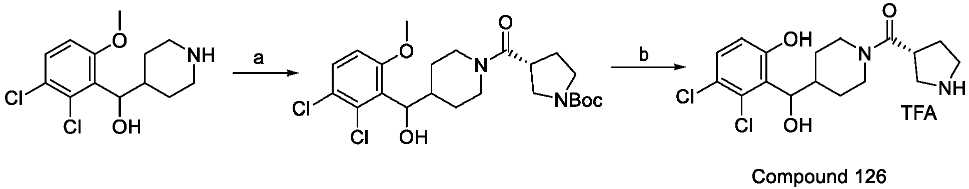

### Step a:

To a solution of (3*R*)-1-[(*tert*-butoxy) carbonyl] pyrrolidine-3-carboxylic acid (0.40 g, 1.86 mmol) in DMF (8 mL) was added CDI (0.34 mg, 2.07 mmol) at room temperature. After stirring at room temperature for 0.5 h, Et₃N (0.11 g, 10.79 mmol) and (2,3-dichloro-6-methoxyphenyl)(piperidin-4-yl)methanol (0.30 g, 1.03 mmol) were added to resulting solution at room temperature. After stirring for additional 1 h at room temperature, the resulting mixture was quenched with water (80 mL) and extracted with EA (3 x 25 mL). The organic phases were combined, washed with brine (4 x 10 mL), dried over anhydrous Na₂SO₄. After filtration, the filtrate was concentrated under reduced pressure. The residue was purified with silica gel column chromatography, eluted with EA to afford *tert*-butyl (3*R*)-3-[4-[(2,3-dichloro-6-methoxyphenyl)(hydroxy)methyl]piperidine-1-carbonyl]pyrrolidine-1-carboxylate as an off-white foam (0.20 g, 50%): LCMS (ESI) calc'd for C₂₃H₃₂Cl₂N₂O₅ [M + H-56]⁺: 431, 433 (3 : 2), found 431, 433 (3 : 2); ¹H NMR (400 MHz, CDCl₃) δ 7.38 (d, *J* = 8.9 Hz, 1H), 6.83 (d, *J* = 9.0 Hz, 1H), 4.99 (d, *J* = 8.5 Hz, 1H), 4.65 (dd, *J* = 57.9, 13.0 Hz, 1H), 3.92 (s, 4H), 3.73-2.87 (m, 7H), 2.52 (dt, *J* = 38.5, 12.3 Hz, 1H), 2.3-1.8 (m, 4H), 1.47 (s, 9H), 1.41-1.29 (m, 3H).

### Step b:

To a solution of *tert*-butyl (3*R*)-3-[4-[(2,3-dichloro-6-methoxyphenyl)(hydroxy)methyl]piperidine-1-carbonyl]pyrrolidine-1-carboxylate (0.20 g, 0.41 mmol) in DCM (5 mL) was added BBr₃ (1.56 g, 6.35 mmol) at room temperature. The reaction mixture was stirred at room temperature for 3 h. Then the reaction was quenched with water (5 mL) at 0 °C. The resulting mixture was concentrated under reduced pressure. The residue was purified by Prep-HPLC with the following conditions: Column: XBridge C18 OBD Prep Column 19 mm x 250 mm, 10 µm; Mobile Phase A: water (plus 0.05% TFA), Mobile Phase B: ACN; Flow rate: 25 mL/min; Gradient: 25% B to 33% B in 6 min; Detector: UV 210 nm; Retentione time: 4.15 min. The fractions containing the desired product were collected and concentrated under reduced pressure to afford Compound 126 (3,4-dichloro-2-[hydroxy([1-[(3*R*)-pyrrolidine-3-carbonyl] piperidin-4-yl])methyl]phenol trifluoroacetic acid) as an off-white solid (147.5 mg, 76%): LCMS (ESI) calc'd for C₁₇H₂₂Cl₂N₂O₃ [M + H]⁺: 373, 375 (3 : 2), found 373, 375 (3 : 2). ¹H NMR (400 MHz, DMSO-*d*₆) δ 10.19 (s, 1H), 8.93 (d, *J* = 29.9 Hz, 2H), 7.37 (d, *J* = 8.8 Hz, 1H), 7.07 (d, *J* = 8.8 Hz, 1H), 6.50 (br, 1H), 4.94 (d, *J* = 7.2 Hz, 1H), 4.42 (dd, *J* = 27.3, 13.0 Hz, 1H), 3.95 (dd, *J* = 27.8, 13.6 Hz, 1H), 3.61-3.45 (m, 1H), 3.33-3.07 (m, 3H), 3.06-2.86 (m, 1H), 2.50-2.40 (m, 2H), 2.29-2.09 (m, 2H), 2.02-1.78 (m, 2H), 1.49-0.97 (m, 3H); ¹⁹F NMR (376 MHz, DMSO-*d*₆) δ -74.32.

### Example 163. Compound 128 (4,5-dichloro-2-[hydroxy([1-[(3S)-pyrrolidine-3-carbonyl]azepan-4-yl])methyl]phenol)

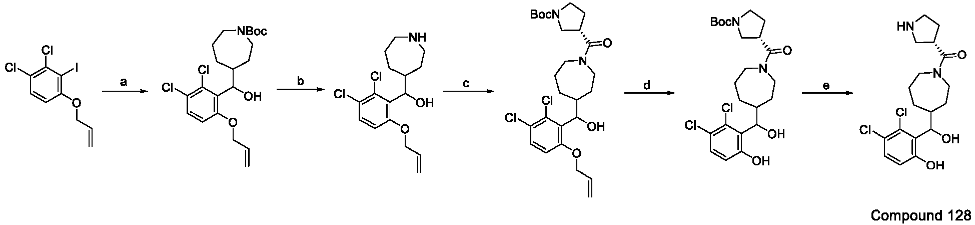

### Step a:

To a stirred solution of 1,2-dichloro-3-iodo-4-(prop-2-en-1-yloxy)benzene (0.60 g, 1.82 mmol) in THF (10 mL) was added *i*-PrMgCl (1.37 mL, 2.74 mmol, 2 M in THF) dropwise at 0 °C under argon atmosphere. The resulting solution was stirred for 0.5 h at 0 °C under argon atmosphere. Then a solution of *tert*-butyl 4-formylazepane-1-carboxylate (0.50 g, 2.19 mmol) in THF (1 mL) was added dropwise to the resulting solution at 0 °C under argon atmosphere. The resulting solution was stirred for additional 1 h at room temperature. The reaction was quenched with saturated aq. NH₄Cl (20 mL) at room temperature. The resulting mixture was extracted with EA (3 x 30 mL). The combined organic layers were washed with brine (2 x 30 mL), dried over anhydrous Na₂SO₄. After filtration, the filtrate was concentrated under reduced pressure. The residue was purified by Prep-TLC, eluted with PE/EA (1/1) to afford *tert*-butyl 4-[[2,3-dichloro-6-(prop-2-en-1-yloxy)phenyl](hydroxy)methyl]azepane-1-carboxylate as a light yellow oil (0.65 g, 75%): LCMS (ESI) calc'd for C₂₁H₂₉Cl₂NO₄ [M + H]⁺: 430, 432 (3 : 2), found 430, 432 (3 : 2); ¹H NMR (400 MHz, CDCl₃) δ 7.34 (d, *J* = 8.9 Hz, 1H), 6.80 (d, *J* = 8.9 Hz, 1H), 6.10-5.92 (m, 1H), 5.47-5.33 (m, 2H), 4.95 (t, *J* = 8.9 Hz, 1H), 4.69-4.57 (m, 2H), 3.69-3.36 (m, 2H), 3.36-3.11 (m, 2H), 2.39 (d, *J* = 14.3 Hz, 1H), 2.07 (s, 1H), 1.85-1.71 (m, 1H), 1.66-1.49 (m, 1H), 1.49-1.42 (m, 10H), 1.43-1.22 (m, 2H).

### Step b:

To a stirred solution of *tert*-butyl 4-[[2,3-dichloro-6-(prop-2-en-1-yloxy)phenyl](hydroxy)methyl]azepane-1-carboxylate (0.65 g, 1.51 mmol) in DCM (8 mL) was added TFA (4 mL) at room temperature. The resulting solution was stirred for 1 h at room temperature. The resulting solution was concentrated under reduced pressure to afford (azepan-4-yl)[2,3-dichloro-6-(prop-2-en-1-yloxy)phenyl]methanol as a yellow oil (0.60 g, crude), which was used in the next step directly without further purification: LCMS (ESI) calc'd for C₁₆H₂₁Cl₂NO₂ [M + H]⁺: 330, 332 (3 : 2), found 330, 332 (3 : 2);

### Step c:

To a stirred solution of (3*S*)-1-[(*tert*-butoxy)carbonyl]pyrrolidine-3-carboxylic acid (0.52 g, 2.42 mmol) and HATU (0.92 g, 2.42 mmol) in DMF (5 mL) was added (azepan-4-yl)[2,3-dichloro-6-(prop-2-en-1-yloxy)phenyl]methanol (0.40 g, 1.21 mmol) and Et₃N (0.24 g, 2.42 mmol) at room temperature. The resulting solution was stirred for 2 h at room temperature. The reaction was diluted with water (20 mL) at room temperature. The resulting mixture was extracted with EA (3 x 50 mL). The combined organic layers were washed with brine (6 x 20 mL), dried over anhydrous Na₂SO₄. After filtration, the filtrate was concentrated under reduced pressure. The residue was purified by silica gel column chromatography, eluted with PE/EA (5/1) to afford *tert-*butyl (3*S*)-3-(4-[[2,3-dichloro-6-(prop-2-en-1-yloxy)phenyl](hydroxy)methyl]azepane-1-carbonyl)pyrrolidine-1-carboxylate as an off-white semi-solid (0.48 g, 60% overall two steps): LCMS (ESI) calc'd for C₂₆H₃₆Cl₂N₂O₅ [M + H]⁺: 527, 529 (3 : 2), found 527, 529 (3 : 2); ¹H NMR (400 MHz, CDCl₃) δ 7.39-7.32 (m, 1H), 6.89-6.71 (m, 1H), 6.09-5.96 (m, 1H), 5.48-5.33 (m, 2H), 4.99-4.90 (m, 1H), 4.68-4.59 (m, 2H), 3.88-3.52 (m, 4H), 3.52-3.27 (m, 4H), 3.27-3.04 (m, 1H), 2.58-2.35 (m, 1H), 2.30-1.96 (m, 3H), 1.88 (dd, *J* = 31.0, 12.6 Hz, 1H), 1.68-1.53 (m, 1H), 1.53-1.43 (m, 10H), 1.43-1.22 (m, 2H).

### Step d:

To a stirred mixture of *tert*-butyl (3*S*)-3-(4-[[2,3-dichloro-6-(prop-2-en-1-yloxy)phenyl](hydroxy)methyl]azepane-1-carbonyl)pyrrolidine-1-carboxylate (0.48 g, 0.91 mmol) and Pd(PPh₃)₄ (21 mg, 0.02 mmol) in THF (5 mL) was added NaBH₄ (69 mg, 1.82 mmol) at room temperature under nitrogen atmosphere. The resulting mixture was stirred for 1 h at room temperature. The reaction was quenched with saturated aq. NH₄Cl (10 mL) at 0 °C. The resulting mixture was extracted with EA (3 x 20 mL). The combined organic layers were washed with brine (2 x 20 mL), dried over anhydrous Na₂SO₄. After filtration, the filtrate was concentrated under reduced pressure. The residue was purified by reverse phase chromatography, eluted with 40% ACN in water (plus 0.05% TFA) to afford *tert*-butyl (3*S*)-3-[4-[(2,3-dichloro-6-hydroxyphenyl)(hydroxy)methyl]azepane-1-carbonyl]pyrrolidine-1-carboxylate as an off-white solid (0.40 g, 90%): LCMS (ESI) calc'd for C₂₃H₃₂Cl₂N₂O₅ [M + H]⁺: 487, 489 (3 : 2), found 487, 489 (3 : 2);

### Step e:

To a stirred solution of *tert*-butyl (3*S*)-3-[4-[(4,5-dichloro-2-hydroxyphenyl)(hydroxy)methyl]azepane-1-carbonyl]pyrrolidine-1-carboxylate (10 mg, 0.06 mmol) in DCM (3 mL) was added TFA (1.5 mL) at room temperature. The resulting solution was stirred for 1 h at room temperature. The resulting solution was concentrated under reduced pressure. The residue was purified by Prep-HPLC with the following conditions: Column: Xselect CSH OBD Column 30 x 150 mm 5 µm n; Mobile Phase A: water with 10 mmoL/L NH₄HCO₃, Mobile Phase B: ACN; Flow rate: 60 mL/min; Gradient: 5% B to 40% B in 7 min; Detector: UV 254/220 nm; Retention time: 6.62 min. The fractions containing the desired product were collected and concentrated under reduced pressure to afford Compound 128 (4,5-dichloro-2-[hydroxy([1-[(3S)-pyrrolidine-3-carbonyl]azepan-4-yl])methyl]phenol) as an off-white solid (2.7 mg, 36% overall two steps): LCMS (ESI) calc'd for C₁₈H₂₄Cl₂N₂O₃ [M + H]⁺: 387, 389 (3 : 2), found 387, 389 (3 : 2); ¹H NMR (400 MHz, CD₃OD) δ 7.31-7.19 (m, 1H), 6.75-6.64 (m, 1H), 5.16-5.01 (m, 1H), 3.86-3.63 (m, 2H), 3.63-3.49 (m, 1H), 3.49-3.34 (m, 2H), 3.29-3.10 (m, 3H), 3.12-2.98 (m, 1H), 2.30-1.81 (m, 5H), 1.81-1.35 (m, 4H).

### Example 164. Compound 322 (3,4-dichloro-2-[(R)-hydroxy[1-[(3S)-pyrrolidine-3-carbonyl]azepan-4-yl]methyl]phenol trifluoroacetic acid isomer 1);

### Compound 129 (3,4-dichloro-2-[(S)-hydroxy[1-[(3S)-pyrrolidine-3-carbonyl]azepan-4-yl]methyl]phenol trifluoroacetic acid isomer 2);

### Compound 323 (3,4-dichloro-2-[(R)-hydroxy[1-[(3S)-pyrrolidine-3-carbonyl]azepan-4-yllmethyl]|phenol trifluoroacetic acid isomer 3); and

### Compound 130 (3,4-dichloro-2-[(S)-hydroxy[1-[(3S)-pyrrolidine-3-carbonyl]azepan-4-yl]methyl]phenol trifluoroacetic acid isomer 4)

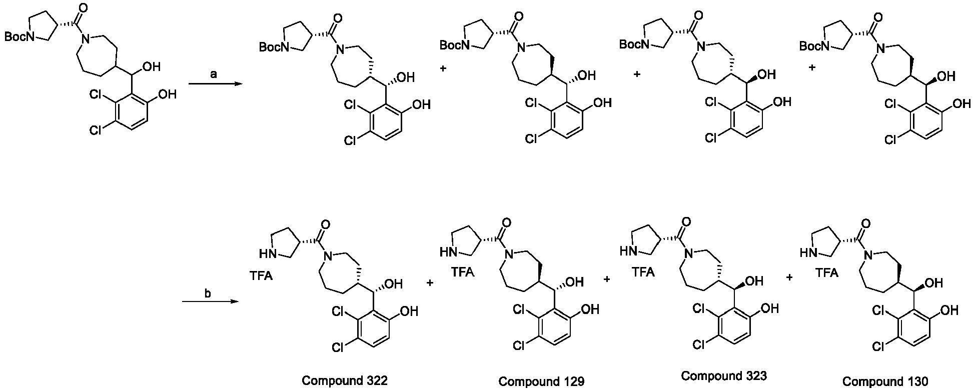

### Step a:

*tert*-butyl (3*S*)-3-[4-[(2,3-dichloro-6-hydroxyphenyl)(hydroxy)methyl]azepane-1-carbonyl]pyrrolidine-1-carboxylate (350 mg, 0.72 mmol) was separated by Prep-SFC with the following conditions: Column: Chiral pak AD-H, 2 x 25 cm (5 µm); Mobile Phase A: CO₂ (70%), Mobile Phase B: IPA (2 mM NH₃-MeOH); Flow rate: 50 mL/min; Gradient: 18% B; Detector: UV 220 nm; Retention time: RT₁: 6.75min, RT₂: 7.90 min, RT₃: 8.67 min, RT₃: 8.67 min, RT₄: 11.57 min. Injection Volume: 0.4 mL.

The first peak at 6.75 min was collected and concentrated under reduced pressure to afford *tert*-butyl (3*S*)-3-[(4*R*)-4-[(*S*)-(2,3-dichloro-6-hydroxyphenyl)(hydroxy)methyl]azepane-1-carbonyl]pyrrolidine-1-carboxylate as an off-white solid (55 mg, 16%).

The second peak at 7.90 min was collected and concentrated under reduced pressure to afford (3*S*)-3-[(4*S*)-4-[(*S*)-(2,3-dichloro-6-hydroxyphenyl)(hydroxy)methyl]azepane-1-carbonyl]pyrrolidine-1-carboxylate as an off-white solid (35 mg, 10%).

The fourth peak at 11.57 min was collected and concentrated under reduced pressure to afford *tert*-butyl (3*S*)-3-[(4*S*)-4-[(*R*)-(2,3-dichloro-6-hydroxyphenyl)(hydroxy)methyl]azepane-1-carbonyl]pyrrolidine-1-carboxylate as an off-white solid (50 mg, 14%).

The third peak at 8.67 min was collected and was concentrated under reduced pressure. The residue was purified by Prep-chiral-HPLC with the following conditions: Column: CHIRAL ART Cellulose-SB, 2 x 25 cm, 5 µm; Mobile Phase A: Hexane (plus 0.1% FA), Mobile Phase B: EtOH; Flow rate: 20 mL/min; Gradient: 10% B to 10% B in 14 min; Detector: UV 254/220 nm; Retention time: 10.26 min. The fractions containing desired product were collected and concentrated under reduced pressure to afford *tert*-butyl (3*S*)-3-[(4*R*)-4-[(*R*)-(2,3-dichloro-6-hydroxyphenyl)(hydroxy)methyl]azepane-1-carbonyl]pyrrolidine-1-carboxylate as an off-white solid (40 mg, 11%).

### Step b:

To a stirred solution of *tert*-butyl (3*S*)-3-[(4*R*)-4-[(*S*)-(2,3-dichloro-6-hydroxyphenyl)(hydroxy)methyl]azepane-1-carbonyl]pyrrolidine-1-carboxylate (55 mg, 0.113 mmol) in DCM (1 mL) was added TFA (0.5 mL) at room temperature. The resulting solution was stirred for 1 h at room temperature. The resulting mixture was concentrated under reduced pressure. The residue was purified by Prep-HPLC with the following conditions: Column: Xselect CSH OBD Column 30 x 150 mm 5 µm, Mobile Phase A: water (plus 0.05% TFA), Mobile Phase B: ACN; Flow rate: 60 mL/min; Gradient: 5% B to 40% B in 7 min; Detector: UV 254/220 nm; Retention time: 6.62 min. The fractions containing the desired product were collected and concentrated under reduced pressure to afford Compound 322 (3,4-dichloro-2-[(R)-hydroxy[-1-[(3S)-pyrrolidine-3-carbonyl]azepan-4-yl]methyl]phenol trifluoroacetic acid isomer 1) as an off-white solid (20 mg, 34%): LCMS (ESI) calc'd for C₁₈H₂₄Cl₂N₂O₃ [M + H]⁺: 387, 389 (3 : 2), found 387, 389 (3 : 2); ¹H NMR (400 MHz, CD₃OD) δ 7.30 (dd, *J* = 8.8, 4.6 Hz, 1H), 6.76 (dd, *J* = 8.8, 6.3 Hz, 1H), 5.09 (dd, *J* = 6.2, 3.2 Hz, 1H), 3.78-3.64 (m, 2H), 3.64-3.47 (m, 3H), 3.47-3.35 (m, 4H), 2.48-2.24 (m, 1H), 2.24-1.90 (m, 4H), 1.78-1.51 (m, 3H), 1.51-1.32 (m, 1H); ¹⁹F NMR (376 MHz, CD₃OD) δ -77.11.

To a stirred solution of *tert*-butyl (3*S*)-3-[(4*S*)-4-[(*S*)-(2,3-dichloro-6-hydroxyphenyl)(hydroxy)methyl]azepane-1-carbonyl]pyrrolidine-1-carboxylate (35 mg, 0.07 mmol) in DCM (1 mL) was added TFA (0.5 mL) at room temperature. The resulting solution was stirred for 1 h at room temperature. The resulting mixture was concentrated under reduced pressure. The residue was purified by Prep-HPLC with the following conditions: Column: XBridge Prep C18 OBD Column 19 x 150 mm 5 µm; Mobile Phase A: water (plus 0.05% TFA), Mobile Phase B: ACN; Flow rate: 20 mL/min; Gradient: 15% B to 42% B in 7 min; Detector: UV 254/220 nm; Retention time: 5.47 min. The fractions containing the desired product were collected and concentrated under reduced pressure to afford Compound 129 (3,4-dichloro-2-[(S)-hydroxy[1-[(3S)-pyrrolidine-3-carbonyl]azepan-4-yl]methyl]phenol trifluoroacetic acid isomer 2) as an off-white solid (19 mg, 50%): LCMS (ESI) calc'd for C₁₈H₂₄Cl₂N₂O₃ [M + H]⁺: 387, 389 (3 : 2), found 387, 389 (3 : 2); ¹H NMR (400 MHz, CD₃OD) δ 7.30 (dd, *J* = 8.8, 4.2 Hz, 1H), 6.75 (dd, *J* = 8.8, 3.1 Hz, 1H), 5.13 (dd, *J* = 11.7, 6.0 Hz, 1H), 3.93-3.72 (m, 1H), 3.72-3.58 (m, 3H), 3.58-3.48 (m, 1H), 3.48-3.34 (m, 4H), 2.44-2.30 (m, 1H), 2.24-1.87 (m, 4H), 1.82-1.43 (m, 4H); ¹⁹F NMR (376 MHz, CD₃OD) δ -77.01.

To a stirred solution of *tert*-butyl (3*S*)-3-[(4*R*)-4-[(*R*)-(2,3-dichloro-6-hydroxyphenyl)(hydroxy)methyl]azepane-1-carbonyl]pyrrolidine-1-carboxylate (40 mg, 0.08 mmol) in DCM (1 mL) was added TFA (0.5 mL) at room temperature. The resulting solution was stirred for 1 h at room temperature. The resulting mixture was concentrated under reduced pressure. The residue was purified by Prep-HPLC with the following conditions: Column: XBridge Shield RP18 OBD Column 19 x 150 mm, 5µm; Mobile Phase A: water (plus 0.05% TFA), Mobile Phase B: ACN; Flow rate: 20 mL/min; Gradient: 2% B to 19% B in 10 min; Detector: UV 254/220 nm; Retention time: 6.63 min. The fractions containing the desired product were collected and concentrated under reduced pressure to afford Compound 323 (3,4-dichloro-2-[(S)-hydroxy[1-[(3S)-pyrrolidine-3-carbonyl]azepan-4-yl]methyl]phenol trifluoroacetic acid isomer 3) as an off-white solid (29 mg, 67%): LCMS (ESI) calc'd for C₁₈H₂₄Cl₂N₂O₃ [M + H]⁺: 387, 389 (3 : 2), found 387, 389 (3 : 2); ¹H NMR (400 MHz, CD₃OD) δ 7.30 (dd, *J* = 8.8, 4.9 Hz, 1H), 6.76 (dd, *J* = 8.8, 7.2 Hz, 1H), 5.09 (dd, *J* = 10.0, 6.3 Hz, 1H), 3.85-3.73 (m, 2H), 3.73-3.49 (m, 3H), 3.47-3.34 (m, 4H), 2.42-2.29 (m, 1H), 2.21-1.99 (m, 3H), 1.99-1.87 (m, 1H), 1.80-1.36 (m, 4H); ¹⁹F NMR (376 MHz, CD₃OD) δ -77.03.

To a stirred solution of *tert*-butyl (3*S*)-3-[(4*S*)-4-[(*R*)-(2,3-dichloro-6-hydroxyphenyl)(hydroxy)methyl]azepane-1-carbonyl]pyrrolidine-1-carboxylate (50 mg, 0.10 mmol) in DCM (1 mL) was added TFA (0.5 mL) at room temperature. The resulting solution was stirred for 1 h at room temperature. The resulting mixture was concentrated under reduced pressure. The residue was purified by Prep-HPLC with the following conditions: Column: Xselect CSH OBD Column 30 x 150 mm 5 µm; Mobile Phase A: water (plus 0.05% TFA), Mobile Phase B: ACN; Flow rate: 60 mL/min; Gradient: 5% B to 40% B in 7 min; Detector: UV 254/220 nm; Retention time: 6.62 min. The fractions containing the desired product were collected and concentrated under reduced pressure to afford Compound 130 (3,4-dichloro-2-[(S)-hydroxy[1-[(3S)-pyrrolidine-3-carbonyl]azepan-4-yl]methyl]phenol trifluoroacetic acid isomer 4) as an off-white solid (43 mg, 79 %): LCMS (ESI) calc'd for C₁₈H₂₄Cl₂N₂O₃ [M + H]⁺: 387, 389 (3 : 2), found 387, 389 (3 : 2); ¹H NMR (400 MHz, CD₃OD) δ 7.30 (dd, *J* = 8.8, 4.3 Hz, 1H), 6.75 (dd, *J* = 8.8, 3.1 Hz, 1H), 5.16-5.06 (m, 1H), 3.83-3.55 (m, 4H), 3.55-3.34 (m, 5H), 2.46-2.23 (m, 1H), 2.23-1.88 (m, 4H), 1.80-1.45 (m, 4H); ¹⁹F NMR (376 MHz, CD₃OD) δ - 77.11.

### Example 165. Compound 132 (2-[(R)-amino[1-(2-methoxyethanesulfonyl)piperidin-4-yl]methyl]-4,5-dichlorophenol trifluoroacetic acid)

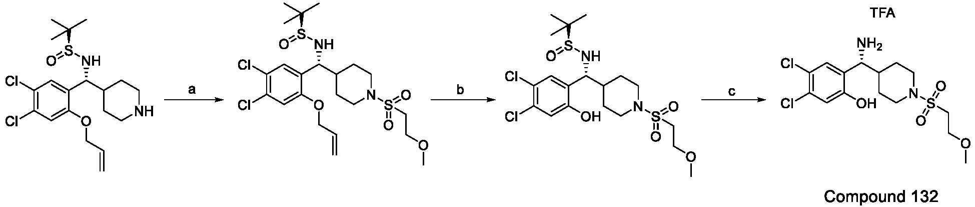

### Step a:

To a stirred mixture of (*S*)-*N*-[(*R*)-[4,5-dichloro-2-(prop-2-en-1-yloxy)phenyl](piperidin-4-yl)methyl]-2-methylpropane-2-sulfinamide (0.10 g, 0.24 mmol) and Et₃N (48 mg, 0.48 mmol) in DCM (1 mL) was added 2-methoxyethane-1-sulfonyl chloride (45 mg, 0.29 mmol) at 0 °C. The resulting mixture was stirred for 2 h at 0 °C. The resulting mixture was diluted with water (20 mL) at 0 °C and extracted with EA (3 x 20 mL). The combined organic layers were washed with brine (3 x 20 mL), dried over anhydrous Na₂SO₄. After filtration, the filtrate was concentrated under reduced pressure. The residue was purified by Prep-TLC, eluted with PE/EA (5/1) to afford (*S*)-*N*-[(*R*)-[4,5-dichloro-2-(prop-2-en-1-yloxy)phenyl][1-(2-methoxyethanesulfonyl)piperidin-4-yl]methyl]-2-methylpropane-2-sulfinamide as a colorless oil (90 mg, 56%): LCMS (ESI) calc'd for C₂₂H₃₄Cl₂N₂O₅S₂ [M + H]⁺: 541, 543 (3 : 2), found 541, 543 (3 : 2); ¹H NMR (400 MHz, CDCl₃) δ 7.23 (s, 1H), 6.98 (s, 1H), 6.10-5.92 (m, 1H), 5.46-5.31 (m, 2H), 4.61-4.41 (m, 3H), 3.86-3.77 (m, 2H), 3.74 (t, *J* = 6.0 Hz, 2H), 3.37 (s, 3H), 3.18 (t, *J* = 6.0 Hz, 2H), 2.78-2.61 (m, 2H), 1.90-1.79 (m, 1H), 1.79-1.59 (m, 1H), 1.54-1.40 (m, 1H), 1.40-1.23 (m, 2H), 1.17 (s, 9H).

### Step b:

To a stirred solution of (*S*)-*N*-[(*R*)-[4,5-dichloro-2-(prop-2-en-1-yloxy)phenyl][1-(2-methoxyethanesulfonyl)piperidin-4-yl]methyl]-2-methylpropane-2-sulfinamide (90 mg, 0.17 mmol) and Pd(PPh₃)₄ (2 mg, 0.002 mmol) in THF (1 mL) was added NaBH₄ (13 mg, 0.34 mmol) at room temperature under nitrogen atmosphere. After stirring for 1 h at room temperature, the resulting mixture was quenched with saturate aq. NH₄Cl (1 mL) and concentrated under reduced pressure to afford (*S*)-*N*-[(*R*)-(4,5-dichloro-2-hydroxyphenyl)[1-(2-methoxyethanesulfonyl)piperidin-4-yl]methyl]-2-methylpropane-2-sulfinamide as a light yellow solid (90 mg, crude), which was used in the next step directly without further purification: LCMS (ESI) calc'd for C₁₉R₃OCl₂N₂O₅S₂ [M + H]⁺: 501, 503 (3 : 2), found 501, 503 (3 : 2).

### Step c:

A solution of (*S*)-*N*-[(*R*)-(4,5-dichloro-2-hydroxyphenyl)[1-(2-methoxyethanesulfonyl) piperidin-4-yl]methyl]-2-methylpropane-2-sulfinamide (90 mg, 0.18 mmol) and HCl (4*N*, 1 mL) in 1,4-dioxane (1 mL) was stirred for 1 h at room temperature. The resulting mixture was concentrated under reduced pressure. The residue was purified by Prep-HPLC with the following conditions: Column: Xselect CSH OBD Column 30 x 150 mm, 5 µm; Mobile Phase A: water (plus 0.05% TFA), Mobile Phase B: ACN; Flow rate: 60 mL/min; Gradient: 10% B to 33% B in 7 min; Detector: UV 254/220 nm; Retention time: 6.63 min. The fractions containing the desired product were collected and concentrated under reduced pressure to afford Compound 132 (2-[(*R*)-amino[1-(2-methoxyethanesulfonyl)piperidin-4-yl]methyl]-4,5-dichlorophenol trifluoroacetic acid) as a purple solid (47.4 mg, 56% overall two steps): LCMS (ESI) calc'd for C₁₅H₂₂Cl₂N₂O₄S [M + H]⁺: 397, 399 (3 : 2), found 397, 399 (3 : 2); ¹H NMR (400 MHz, CD₃OD) δ 7.44 (s, 1H), 7.09 (s, 1H), 4.18 (d, *J* = 9.8 Hz, 1H), 3.84 (d, *J* = 12.5 Hz, 1H), 3.77-3.65 (m, 3H), 3.36 (s, 3H), 3.28 (t, *J* = 5.7 Hz, 2H), 2.87 (td, *J* = 12.3, 2.6 Hz, 1H), 2.76 (td, *J* = 12.2, 2.9 Hz, 1H), 2.28-2.16 (m, 1H), 2.02 (d, *J* = 12.9 Hz, 1H), 1.55-1.22 (m, 3H); ¹⁹F NMR (376 MHz, CD₃OD) δ -76.98.

### Example 166. Compound 110 (2-[(R)-amino[1-(pyrrolidine-3-sulfonyl)piperidin-4-yl]methyl]-4,5-dichlorophenol)

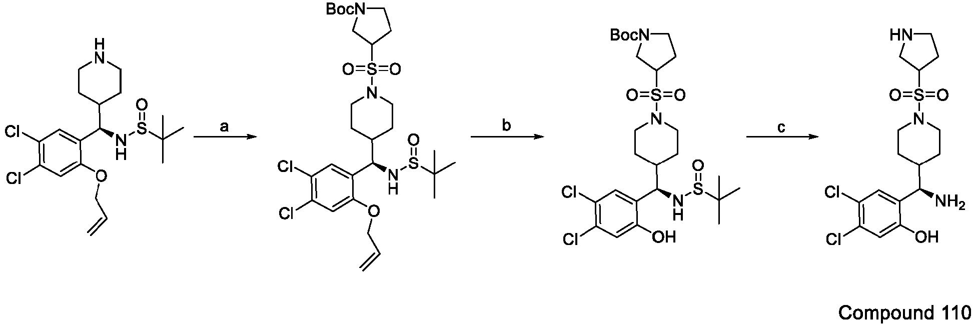

### Step a:

To a stirred solution of *N*-[(*R*)-[4,5-dichloro-2-(prop-2-en-1-yloxy)phenyl](piperidin-4-yl)methyl]-2-methylpropane-2-sulfinamide (50 mg, 0.12 mmol) and Et₃N (25 mg, 0.25 mmol) in DCM (2 mL) was added *tert*-butyl 3-(chlorosulfonyl)pyrrolidine-1-carboxylate (45 mg, 0.17 mmol) at room temperature under argon atmosphere. The resulting mixture was stirred for 4 h at room temperature under argon atmosphere. The resulting mixture was concentrated under reduced pressure. The residue was purified by Prep-TLC, eluted with PE/EA (1/4) to afford *tert*-butyl 3-([4-[(*R*)-[4,5-dichloro-2-(prop-2-en-1-yloxy)phenyl][(2-methylpropane-2-sulfinyl)amino]methyl]piperidin-1-yl]sulfonyl)pyrrolidine-1-carboxylate as a light yellow oil (25 mg, 32%): LCMS (ESI) calc'd for C₂₈H₄₃Cl₂N₃O₆S₂ [M + H]⁺: 652, 654 (3 : 2), found 652, 654 (3 : 2); ¹H NMR (300 MHz, CDCl₃) δ 7.21 (s, 1H), 6.98 (s, 1H), 6.10-5.94 (m, 1H), 5.46-5.29 (m, 2H), 4.59-4.54 (m, 2H), 4.41 (s, 1H), 3.94-3.76 (m, 3H), 3.75-3.53 (m, 4H), 3.37 (dt, *J* = 10.8, 7.7 Hz, 1H), 2.86-2.67 (m, 2H), 2.17-2.02 (m, 1H), 1.96-1.78 (m, 1H), 1.47 (s, 9H), 1.39-1.20 (m, 4H), 1.15 (s, 9H).

### Step b:

To a stirred solution of *tert*-butyl 3-([4-[(*R*)-[4,5-dichloro-2-(prop-2-en-1-yloxy)phenyl][(2-methylpropane-2-sulfinyl)amino]methyl]piperidin-1-yl]sulfonyl)pyrrolidine-1-carboxylate (0.14 g, 0.22 mmol) and Pd(PPh₃)₄ (24 mg, 0.02 mmol) in THF (3 mL) was added NaBH₄ (26 mg, 0.69 mmol) in portions at room temperature under nitrogen atmosphere. The resulting mixture was stirred for 2 h at room temperature under nitrogen atmosphere. The resulting mixture was quenched with water (30 mL) at room temperature and extracted with EA (3 x 20 mL). The combined organic layers were washed with EA (3 x 5 mL), dried over anhydrous Na₂SO₄. After filtration, the filtrate was concentrated under reduced pressure. The residue was purified by Prep-TLC, eluted with PE/EA (1/ 4) to afford *tert*-butyl 3-([4-[(*R*)-(4,5-dichloro-2-hydroxyphenyl)[(2-methylpropane-2-sulfinyl)amino]methyl]piperidin-1-yl]sulfonyl)pyrrolidine-1-carboxylate as a light yellow solid (0.10 g, 73%); LCMS (ESI) calc'd for C₂₅H₃₉Cl₂N₃O₆S₂ [M + H]⁺: 612, 614 (3 : 2), found 612, 614 (3 : 2).

### Step c:

To a stirred solution of *tert*-butyl 3-([4-[(*R*)-(4,5-dichloro-2-hydroxyphenyl)[(2-methylpropane-2-sulfinyl)amino]methyl]piperidin-1-yl]sulfonyl)pyrrolidine-1-carboxylate (0.10 g, 0.16 mmol) in 1,4-dioxane (5 mL) was added aq. HCl (6 *N*, 3 mL) at room temperature. The reaction solution was stirred for 1 h at room temperature. The reaction solution was concentrated under reduced pressure. The residue was dissolved in DCM (6 mL), and then TFA (3 mL) was added at room temperature. The reaction solution was stirred for 1 h at room temperature. The resulting solution was concentrated under reduced pressure. The residue was purified by Prep-HPLC with the following conditions: Column: XBridge C18 OBD Prep Column 100 Å, 5 µm, 19 mm x 250 mm; Mobile Phase A: water with 10 mmol/L NH₄HCO₃, Mobile Phase B: ACN; Flow rate: 25 mL/min; Gradient: 10% B to 80% B in 6 min; Detector: UV 254 nm; Retention time: 5.7 min. The fractions containing the desired product were collected and concentrated under reduced pressure to afford Compound 110 (2-[(*R*)-amino[1-(pyrrolidine-3-sulfonyl)piperidin-4-yl]methyl]-4,5-dichlorophenol) (20 mg, 30%) as an off-white solid. LCMS (ESI) calc'd for C₁₆H₂₃Cl₂N₃O₃S [M + H]⁺: 408, 410 (3 : 2), found 408, 410 (3 : 2); ¹H NMR (300 MHz, CD₃OD) δ 7.23 (s, 1H), 6.87 (s, 1H), 3.98-3.82 (m, 2H), 3.82-3.65 (m, 2H), 3.20-3.11 (m, 2H), 3.02 (dt, *J* = 13.1, 6.7 Hz, 1H), 2.95-2.71 (m, 3H), 2.20-1.81 (m, 4H), 1.55-1.44 (m, 1H), 1.44-1.20 (m, 2H).

### Example 167. Compound 112 (2-[(R)-amino([1-[pyrrolidine-3-sulfonyl]piperidin-4-yl])methyl]-4,5-dichlorophenol isomer 1); Compound 118 (2-[(R)-amino([1-[pyrrolidine-3-sulfonyl]piperidin-4-yl])methyl]-4,5-dichlorophenol isomer 2)

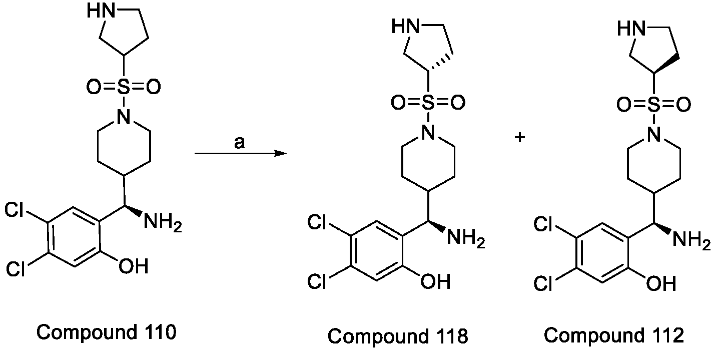

### Step a:

2-[(*R*)-amino[1-(pyrrolidine-3-sulfonyl)piperidin-4-yl]methyl]-4,5-dichlorophenol (20 mg, 0.049 mmol) (Compound 110, Example 166) was separated by Chiral Prep-HPLC with the following conditions: Column: Chiralpak ID-2, 2 x 25 cm, 5 µm; Mobile Phase A: MeOH (0.1% IPA), Mobile Phase B: none; Flow rate: 16 mL/min; Gradient: 100% A to 100% A in 13 min; Detector: UV 220/254 nm; Retention time: RT₁: 6.191 min; RT₂: 10.888 min.

The faster-eluting enantiomer was obtained Compound 118 (2-[(*R*)-amino([1-[pyrrolidine-3-sulfonyl]piperidin-4-yl])methyl]-4,5-dichlorophenol isomer 2) as an off-white solid (8.1 mg, 40%) at 6.191 min: LCMS (ESI) calc'd for C₁₆H₂₃Cl₂N₃O₃S [M + H]⁺: 408, 410 (3 : 2), found 408, 410 (3 : 2); ¹H NMR (300 MHz, CD₃OD) δ 7.23 (s, 1H), 6.87 (s, 1H), 3.91 (d, *J* = 7.7 Hz, 1H), 3.86-3.66 (m, 3H), 3.20-3.11 (m, 2H), 3.09-2.95 (m, 1H), 2.93-2.73 (m, 3H), 2.17-2.02 (m, 2H), 2.05-1.80 (m, 2H), 1.49 (d, *J* = 13.0 Hz, 1H), 1.41-1.24 (m, 2H).

The slower-eluting enantiomer was obtained as Compound 112 (2-[(R)-amino([1-[pyrrolidine-3-sulfonyl]piperidin-4-yl])methyl]-4,5-dichlorophenol isomer 1) as an off-white solid (7.7 mg, 38%) at 10.888 min: LCMS (ESI) calc'd for C₁₆H₂₃Cl₂N₃O₃S [M + H]⁺: 408, 410 (3 : 2), found 408, 410 (3 : 2); ¹H NMR (300 MHz, CD₃OD) δ 7.23 (s, 1H), 6.87 (s, 1H), 3.99-3.85 (m, 2H), 3.81-3.67 (m, 2H), 3.21-3.11 (m, 2H), 3.09-2.95 (m, 1H), 2.94-2.71 (m, 3H), 2.18-2.06 (m, 2H), 2.05-1.79 (m,2H), 1.49 (d, *J* = 13.3 Hz, 1H), 1.44-1.22 (m, 2H).

### Example 168. Compound 141 (2-[(R)-amino[1-(piperazine-1-carbonyl)piperidin-4-yl]methyl]-4,5-dichlorophenol trifluoroacetic acid)

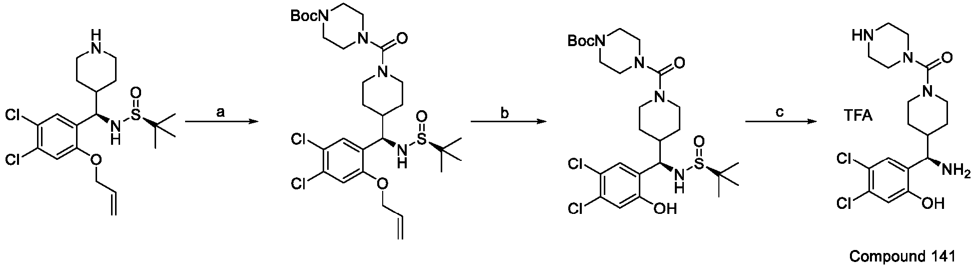

### Step a:

To a stirred solution of (*S*)-*N*-[(*R*)-[4,5-dichloro-2-(prop-2-en-1-yloxy)phenyl](piperidin-4-yl)methyl]-2-methylpropane-2-sulfinamide (0.10 g, 0.238 mmol) and *tert*-butyl piperazine-1-carboxylate (67 mg, 0.358 mmol) in DCM (4 mL) were added ditrichloromethyl carbonate (0.10 g, 0.36 mmol) and Et₃N (48 mg, 0.48 mmol) at 0 °C under nitrogen atmosphere. The resulting mixture was stirred for 2 h at room temperature under nitrogen atmosphere. The resulting mixture was quenched with water (30 mL) and extracted with EA (3 x 15 mL). The combined organic layers were washed with brine (3 x 10 mL), dried over anhydrous Na₂SO₄. After filtration, the filtrate was concentrated under reduced pressure. The residue was purified by reverse phase chromatography, eluted with 75% ACN in water (plus 0.05% TFA) to afford *tert*-butyl 4-[4-[(*R*)-[4,5-dichloro-2-(prop-2-en-1-yloxy)phenyl]([[(*S*)-2-methylpropane-2-sulfinyl]amino])methyl]piperidine-1-carbonyl]piperazine-1-carboxylate as a light yellow solid (0.10 g, 66%): LCMS (ESI) calc'd for C₂₉H₄₄Cl₂N₄O₅S [M + H]⁺: 631, 633 (3 : 2), found 631, 633 (3 : 2); ¹H NMR (400 MHz, CDCl₃) δ 7.22 (s, 1H), 6.97 (s, 1H), 6.06-6.01 (m, 1H), 5.49-5.31 (m, 2H), 4.63-4.54 (m, 2H), 4.45-4.31 (m, 1H), 3.73 (dd, *J* = 25.4, 13.1 Hz, 2H), 3.45-3.40 (m, 4H), 3.22-3.17 (m, 4H), 2.68 (dd, *J* = 29.0, 16.0 Hz, 2H), 2.08-2.04 (m, 1H), 1.92-1.88 (m, 1H), 1.79-1.68 (m, 1H), 1.48 (s, 9H), 1.38-1.34 (m, 1H), 1.30-1.25 (m, 1H), 1.15 (s, 9H).

### Step b:

To a stirred solution of *tert*-butyl 4-[4-[(*R*)-[4,5-dichloro-2-(prop-2-en-1-yloxy)phenyl]([[(*S*)-2-methylpropane-2-sulfinyl]amino])methyl]piperidine-1-carbonyl]piperazine-1-carboxylate (0.15 g, 0.24 mmol) and Pd(PPh₃)₄ (27 mg, 0.02 mmol) in THF (3 mL) was added NaBH₄ (18 mg, 0.47 mmol) in portions at room temperature under nitrogen atmosphere. After stirring for 1 h at room temperature under nitrogen atmosphere, the resulting mixture was quenched with water (2 mL) at room temperature and concentrated under reduced pressure. The crude product was used in the next step directly without further purification: LCMS (ESI) calc'd for C₂₆H₄₀Cl₂N₄O₅S [M + H]⁺: 591, 593 (3 : 2), found 591, 593 (3 : 2).

### Step c:

To a solution of *tert*-butyl 4-[4-[(*R*)-(4,5-dichloro-2-hydroxyphenyl)([[(*S*)-2-methylpropane-2-sulfinyl]amino])methyl]piperidine-1-carbonyl]piperazine-1-carboxylate (90 mg, 0.15 mmol) in 1,4-dioxane (5 mL) was added aq. HCl (6 *N*, 2 mL) at room temperature. The reaction solution was stirred for 1 h at room temperature. The reaction solution was concentrated under reduced pressure. The residue was dissolved in DCM (5 mL), and then TFA (2 mL) was added at room temperature. The reaction solution was stirred for 1 h at room temperature. The resulting solution was concentrated under reduced pressure. The residue was purified by Prep-HPLC with the following conditions: Column: XBridge Shield RP18 OBD Column, 5 µm, 19 x 150 mm; Mobile Phase A: water (plus 0.05% TFA), Mobile Phase B: ACN; Flow rate: 25 mL/min; Gradient: 5% B to 20% B in 7 min; Detector: UV 254/220 nm; Retention time: 5.88 min. The fractions containing the desired product were collected and concentrated under reduced pressure to afford Compound 141 (2-[(*R*)-amino[1-(piperazine-1-carbonyl)piperidin-4-yl]methyl]-4,5-dichlorophenol trifluoroacetic acid) as a purple solid (89 mg, 79% overall two steps): LCMS (ESI) calc'd for C₁₇H₂₄Cl₂N₄O₂ [M + H]⁺: 387, 389 (3 : 2), found 387, 389 (3 : 2); ¹H NMR (400 MHz, CD₃OD) δ 7.43 (s, 1H), 7.08 (s, 1H), 4.16 (d, *J* = 9.7 Hz, 1H), 3.89 (d, *J* = 13.5 Hz, 1H), 3.72 (d, *J* = 13.4 Hz, 1H), 3.46 (t, *J* = 5.1 Hz, 4H), 3.24 (t, *J* = 5.1 Hz, 4H), 2.87 (dt, *J* = 30.4, 12.7 Hz, 2H), 2.30 (d, *J* = 11.1 Hz, 1H), 1.97 (d, *J* = 12.9 Hz, 1H), 1.45-1.19 (m, 3H); ¹⁹F NMR (376 MHz, CD₃OD) δ -77.10.

### Example 169. Compound 145 ((R)-2-(amino(1-((2-hydroxyethyl)sulfonyl)piperidin-4-yl)methyl)-4,5-dichlorophenol trifluoroacetic acid)

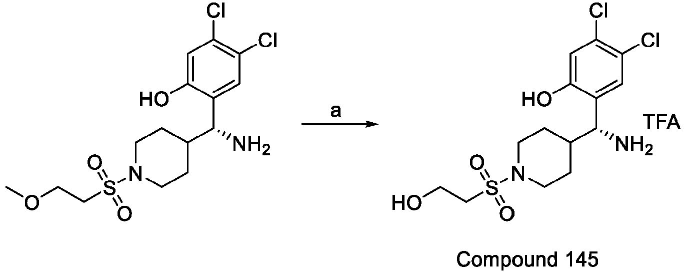

### Step a:

To a stirred solution of 2-[(*R*)-amino[1-(2-methoxyethanesulfonyl)piperidin-4-yl]methyl]-4,5-dichlorophenol (Compound 132's free base, Example 165) (18 mg, 0.05 mmol) in DCM (2 mL) was added BBr₃ (57 mg, 0.23 mmol) at 0 °C under nitrogen atmosphere. The resulting mixture was stirred for 6 h at room temperature under nitrogen atmosphere. The reaction was quenched with water (3 mL) at 0 °C and concentrated under reduced pressure. The residue was purified by Prep-HPLC with the following conditions: Column: X select CSHOBD Column 30 x 150 mm 5 µm; Mobile Phase A: water (plus 0.05% TFA), Mobile Phase B: ACN; Flow rate: 60 mL/min; Gradient: 5% B to 30% B in 7 min; Detector: UV 220 nm; Retention time: 6.65 min. The fractions containing the desired product were collected and concentrated under reduced pressure to afford Compound 145 (2-[(*R*)-amino[1-(2-hydroxyethanesulfonyl)piperidin-4-yl]methyl]-4,5-dichlorophenol trifluoroacetic acid) as a light brown solid (11 mg, 63%): LCMS (ESI) calc'd for C₁₄H₂₀Cl₂N₂O₄S [M + H]⁺: 383, 385 (3 : 2), found 383, 385 (3 : 2); ¹H NMR (400 MHz, DMSO-*d*₆) δ 10.94 (s, 1H), 8.26 (s, 3H), 7.60 (s, 1H), 7.10 (s, 1H), 5.02 (s, 1H), 4.24 (s, 1H), 3.73 (t, *J* = 6.3 Hz, 2H), 3.65 (d, *J* = 12.1 Hz, 1H), 3.55-3.48 (m, 2H), 3.16 (t, *J* = 6.3 Hz, 2H), 2.81-2.66 (m, 2H), 1.96 (t, *J* = 13.7 Hz, 2H), 1.4-1.23 (m, 1H), 1.22-1.12 (m, 1H); ¹⁹F NMR (376 MHz, DMSO-*d*₆) δ -73.61.

### Example 170. Compound 184 (1-[4-[(4,5-dichloro-2-hydroxyphenyl)(methylamino)methyl]piperidin-1-yl]-2,3-dihydroxypropan-1-one trifluoroacetic acid)

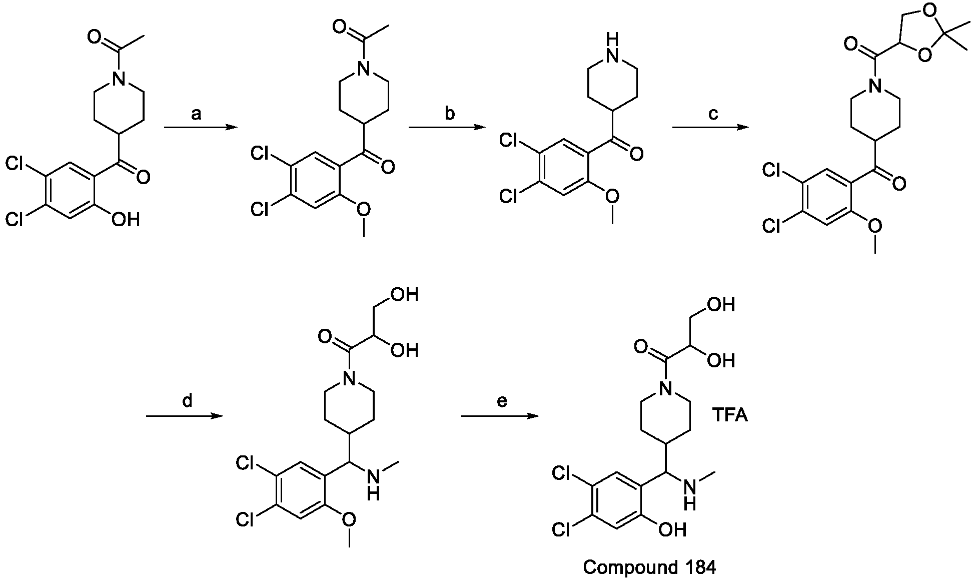

### Step a:

To a stirred mixture of 1-[4-[(4,5-dichloro-2-hydroxyphenyl)carbonyl]piperidin-1-yl]ethan-1-one (Intermediate 1, Example 1) (2.60 g, 8.22 mmol) and K₂CO₃ (2.30 g, 16.45 mmol) in DMF (15 mL) was added iodomethane (2.3 g, 16.45 mmol) dropwise at 0 °C. The resulting mixture was stirred for 16 h at room temperature. The reaction was quenched by the addition of water (30 mL) at 0 °C. The resulting mixture was extracted with EtOAc (8 x 40 mL). The combined organic layers were washed with brine (6 x 10 mL) and dried over anhydrous Na₂SO₄. After filtration, the filtrate was concentrated under reduced pressure to afford 1-[4-[(4,5-dichloro-2-methoxyphenyl)carbonyl]piperidin-1-yl]ethan-1-one. The crude product was used in the next step directly without further purification: LCMS (ESI) calc'd for C₁₅H₁₇F₂NO₃ [M + H]⁺: 330, 332 (3 : 2), found 330, 332 (3 : 2); ¹H NMR (300 MHz, Methanol-*d*₄) δ 7.64 (s, 1H), 7.35 (s, 1H), 4.48-4.34 (m, 1H), 3.95 (s, 3H), 3.90 (q, *J* = 3.4 Hz, 1H), 3.51 (tt, *J* = 10.8, 3.8 Hz, 1H), 3.23 (ddd, *J* = 14.0, 11.8, 2.9 Hz, 1H), 2.90 - 2.75 (m, 1H), 2.10 (s, 3H), 1.91 (dt, *J* = 13.6, 8.3 Hz, 2H), 1.69-1.38 (m, 2H).

### Step b:

A solution of 1-[4-[(4,5-dichloro-2-methoxyphenyl)carbonyl]piperidin-1-yl]ethan-1-one (1.69 g, 1 equiv) in aq. HCl (17 mL, 6 N) was refluxed for 4 h. The resulting mixture was concentrated under vacuum to afford 4-[(4,5-dichloro-2-methoxyphenyl)carbonyl]piperidine as a yellow oil: LCMS (ESI) calc'd for C₁₃H₁₅Cl₂NO₂ [M + H]⁺: 288, 290 (3 : 2), found 288, 290 (3 : 2).

### Step c:

To a stirred solution of 2,2-dimethyl-1,3-dioxolane-4-carboxylic acid (0.48 g, 3.33 mmol) in DMF (4 mL) were added EDCI (0.80 mg, 4.16 mmol) and HOBt (0.56 g, 4.16 mmol) at room temperature. To the above mixture were added 4-(4,5-dichloro-2-methoxybenzoyl) piperidine (0.8 g, 2.77 mmol) and Et₃N (0.84 g, 8.34 mmol) at room temperature. The reaction mixture was stirred for 12 h at room temperature under nitrogen atmosphere. The mixture was diluted with water (15 mL). The resulting mixture was extracted with EA (3 x 25 mL). The combined organic layers were washed with brine (2 x 10 mL), dried over anhydrous Na₂SO₄. After filtration, the filtrate was concentrated under reduced pressure. The residue was purified by reverse phase chromatography, eluted with 50% ACN in water with 10 mmol/L NH₄HCO₃ to afford 4-(4,5-dichloro-2-methoxybenzoyl)-1-(2,2-dimethyl-1,3-dioxolane-4-carbonyl)piperidine as a yellow oil (0.53 g, 39%): LCMS (ESI) calc'd for C₁₉H₂₃Cl₂NO₅ [M + H]⁺: 416, 418 (3 : 2), found 416, 418 (3 : 2).

### Step d:

To a stirred mixture of 4-(4,5-dichloro-2-methoxybenzoyl)-1-(2,2-dimethyl-1,3-dioxolane-4-carbonyl)piperidine (0.10 g, 0.24 mmol) and methanamine hydrochloride (81 mg, 1.20 mmol) in THF (2 mL) was added Ti(OEt)₄ (0.28 g, 1.20 mmol) in one portion at room temperature under nitrogen atmosphere. The reaction mixture was stirred for 12 h at 70 °C under nitrogen atmosphere. After cooling to room temperature, NaBH₄ (18 mg, 0.48 mmol) was added to the above mixture at room temperature. After addition, the resulting mixture was stirred for 1 h at room temperature. The reaction was quenched with H₂O (40 mL) at room temperature and extracted with EA (3 x 30 mL). The combined organic layers were washed with brine (2 x 10 mL), dried over anhydrous Na₂SO₄. After filtration, the filtrate was concentrated under reduced pressure. The residue was purified by reverse phase chromatography, eluted with 50% ACN in water (plus 0.05% TFA) to afford 1-[4-[(4,5-dichloro-2-methoxyphenyl)(methylamino)methyl]piperidin-1-yl]-2,3-dihydroxypropan-1-one as a yellow oil (83 mg, 84%): LCMS (ESI) calc'd for C₁₇H₂₄Cl₂N₂O₄ [M + H]⁺: 391, 393 (3 : 2), found 391, 393 (3 : 2).

### Step e:

To a stirred solution of 1-[4-[(4,5-dichloro-2-methoxyphenyl)(methylamino)methyl]piperidin-1-yl]-2,3-dihydroxypropan-1-one (60 mg, 0.15 mmol) in DCM (1 mL) was added BBr₃ (0.5 mL) dropwise at 0 °C. The reaction mixture was stirred for 1 h at room temperature. The reaction was quenched with MeOH (2 mL). The resulting mixture was concentrated under reduced pressure. The residue was purified with Prep-HPLC with the following conditions: Column: XBridge Shield RP18 OBD Column 19 x 150 mm, 5 µm; Mobile Phase A: water (plus 0.05% TFA), Mobile Phase B: ACN; Flow rate: 25 mL/min; Gradient: 5% B to 30% B in 7 min; Detector: UV 254/220 nm; Retention time: 5.92 min. The fractions containing the desired product were collected and concentrated under reduced pressure to afford Compound 184 (1-[4-[(4,5-dichloro-2-hydroxyphenyl)(methylamino)methyl]piperidin-1-yl]-2,3-dihydroxypropan-1-one trifluoroacetic acid) as an off-white solid (16 mg, 21%): LCMS (ESI) calc'd for C₁₆H₂₂Cl₂N₂O₄ [M + H]⁺: 377, 379 (3 : 2), found 377, 379 (3 : 2); ¹H NMR (400 MHz, CD₃OD) δ 7.43 (s, 1H), 7.11 (s, 1H), 4.72-4.46 (m, 2H), 4.31-4.05 (m, 2H), 3.80-3.55 (m, 2H), 3.18-3.03 (m, 1H), 2.86-2.67 (m, 1H), 2.56 (s, 3H), 2.46 (s, 1H), 2.03 (d, *J* = 12.9 Hz, 1H), 1.45-1.13 (m, 3H); ¹⁹F NMR (376 MHz, CD₃OD) δ -76.95.

### Example 171. Compound 182 (1-[4-[(4,5-dichloro-2-hydroxyphenyl)(ethylamino)methyl]piperidin-1-yl]-2,3-dihydroxypropan-1-one trifluoroacetic acid)

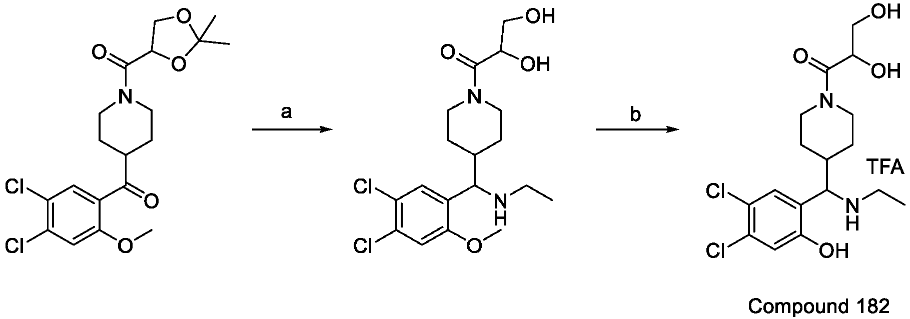

### Step a:

To a stirred mixture of 4-(4,5-dichloro-2-methoxybenzoyl)-1-(2,2-dimethyl-1,3-dioxolane-4-carbonyl)piperidine (Step c, Example 170) (0.10 g, 0.24 mmol) and ethanamine hydrochloride (97 mg, 1.20 mmol) in THF (2 mL) was added Ti(OEt)₄ (0.27 g, 1.20 mmol) at room temperature. The reaction mixture was stirred for 12 h at 70 °C under nitrogen atmosphere. After cooling to room temperature, NaBH₄ (18 mg, 0.48 mmol) was added to the above mixture at room temperature. The resulting mixture was stirred for 1 h at room temperature. The reaction was quenched with H₂O (40 mL) at room temperature. The resulting mixture was extracted with EA (3 x 30 mL). The combined organic layers were washed with brine (2 x 3 mL), dried over anhydrous Na₂SO₄. After filtration, the filtrate was concentrated under reduced pressure. The residue was purified by reverse phase chromatography, eluted with 30% ACN in water (plus 0.5% TFA). The fractions containing the desired product were collected and concentrated under reduced pressure to afford 1-(4-((4,5-dichloro-2-methoxyphenyl)(ethylamino)methyl)piperidin-1-yl)-2,3-dihydroxypropan-1-one as a yellow oil (0.10 g, 74%): LCMS (ESI) calc'd for C₂₁H₂₆Cl₂N₂O₄ [M + H]⁺: 405, 407 (3 : 2), found 405, 407 (3 : 2).

### Step b:

To a stirred solution of 1-(4-((4,5-dichloro-2-methoxyphenyl)(ethylamino)methyl)piperidin-1-yl)-2,3-dihydroxypropan-1-one (60 mg, 0.15 mmol) in DCM (1 mL) was added BBr₃ (0.5 mL) dropwise at 0 °C. The reaction mixture was stirred for 1 h at room temperature. The reaction was quenched with MeOH (2 mL). The mixture was concentrated under reduced pressure. The residue was purified with Prep-HPLC with the following conditions: Column: XBridge Shield RP18 OBD Column 30 x 150 mm, 5 µm; Mobile Phase A: water (plus 0.05% TFA), Mobile Phase B: ACN; Flow rate: 60 mL/min; Gradient: 5 % B to 28% B in 7 min; Detector: UV 254/220 nm; Retention time: 6.42 min. The fractions containing the desired product were collected and concentrated under reduced pressure to afford Compound 182 (1-[4-[(4,5-dichloro-2-hydroxyphenyl)(ethylamino)methyl]piperidin-1-yl]-2,3-dihydroxypropan-1-one trifluoroacetic acid) as an off-white solid (20 mg, 38 %): LCMS (ESI) calc'd for C₁₇H₂₄Cl₂N₂O₄ [M + H]⁺: 391, 393 (3 : 2), found 391, 393 (3 : 2); ¹H NMR (400 MHz, CD₃OD) δ 7.43 (s, 1H), 7.09 (s, 1H), 4.71-4.43 (m, 2H), 4.30-4.05 (m, 2H), 3.74-3.56 (m, 2H), 3.07-2.82 (m, 3H), 2.75-2.53 (m, 1H), 2.50-2.36 (m, 1H), 2.05 (d, *J* = 12.9 Hz, 1H), 1.49-1.34 (m, 2H), 1.30 (t, *J* = 7.3 Hz, 3H), 1.24-1.09 (m, 1H); ¹⁹F NMR (376 MHz, CD₃OD) δ - 77.05.

### Example 172. Compound 187 ((2R)-1-[4-[(R)-amino(2,3-dichloro-6-hydroxyphenyl)methyl]piperidin-1-yl]-2,3-dihydroxypropan-1-one trifluoroacetic acid)

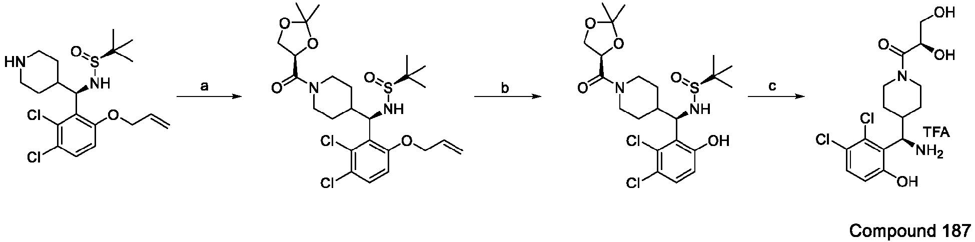

### Step a:

To a stirred solution of HOBt (48 mg, 0.36 mmol), EDCI (68 mg, 0.36 mmol) (5 mL) and (4R)-2,2-dimethyl-1,3-dioxolane-4-carboxylic acid (52 mg, 0.36 mmol) in DMF were added (*S*)-*N*-[(*R*)-[23-dichloro-6-(prop-2-en-1-yloxy)phenyl](piperidin-4-yl)methyl]-2-methylpropane-2-sulfinamide (0.10 g, 0.24 mmol) and Et₃N (48 mg, 0.48 mmol) at room temperature. The reaction solution was stirred for 12 h at room temperature under nitrogen atmosphere. The reaction was quenched with water (30 mL) at room temperature. The resulting mixture was extracted with EA (3 x 20 mL). The combined organic layers were washed with brine (3 x 10 mL), dried over anhydrous Na₂SO₄. After filtration, the filtrate was concentrated under reduced pressure to afford (*S*)-*N*-[(*R*)-[2,3-dichloro-6-(prop-2-en-1-yloxy)phenyl]([1-[(4*R*)-2,2-dimethyl-1,3-dioxolane-4-carbonyl]piperidin-4-yl])methyl]-2-methylpropane-2-sulfinamide as a light yellow oil (0.23 g, 88%): LCMS (ESI) calc'd for C₂₅H₃₆Cl₂N₂O₅S [M + H]⁺: 547, 549 (3 : 2), found 547, 549 (3 : 2).

### Step b:

To a stirred solution of (*S*)-*N*-[(*R*)-[2,3-dichloro-6-(prop-2-en-1-yloxy)phenyl]([1-[(4*R*)-2,2-dimethyl-1,3-dioxolane-4-carbonyl]piperidin-4-yl])methyl]-2-methylpropane-2-sulfinamide (0.13 g, 0.24 mmol) and Pd(PPh₃)₄ (27 mg, 0.02 mmol) in THF (2 mL) was added NaBH₄ (18 mg, 0.48 mmol) at room temperature. The resulting mixture was stirred for 1 h at room temperature under nitrogen atmosphere. The reaction was quenched with aq. NH₄Cl (5 mL). The resulting mixture was concentrated under reduced pressure to afford (*S*)-*N*-[(*R*)-(2, 3-dichloro-6-hydroxyphenyl)([1-[(4*R*)-2,2-dimethyl-1,3- dioxolane-4-carbonyl]piperidin-4-yl])methyl]-2-methylpropane-2-sulfinamide as a light brown solid (0.10 g, 66%): LCMS (ESI) calc'd for C₂₂H₃₂Cl₂N₂O₅S [M + H]⁺: 507, 509 (3 : 2), found 507, 509 (3 : 2).

### Step c:

To a stirred solution of (*S*)-N-[(*R*)-(2,3-dichloro-6-hydroxyphenyl)([1-[(4*R*)-2,2-dimethyl-1,3-dioxolane-4-carbonyl]piperidin-4-yl])methyl]-2-methylpropane-2-sulfinamide (0.10 g, 0.20 mmol) in THF (2 mL) was added aq. HCl (4 *N*, 1.5 mL) at room temperature. The resulting solution was stirred for 30 min at room temperature under nitrogen atmosphere. The resulting solution was concentrated under reduced pressure. The residue was purified by Prep-HPLC with the following conditions: Column: X Bridge Shield RP18 OBD Column, 5 µm, 19 x 150 mm; Mobile Phase A: water (plus 0.05% TFA), Mobile Phase B: ACN; Flow rate: 25 mL/min; Gradient: 5% B to 17% B in 7 min; Detector: UV 220/254 nm; Retention time: 6.5 min. The fractions containing the desired product were collected and concentrated under reduced pressure to afford Compound 187 ((2*R*)-1-[4-[(*R*)-amino(2,3-dichloro-6-hydroxyphenyl)methyl]piperidin-1-yl]-2,3-dihydroxypropan-1-one trifluoroacetic acid) as an off-white solid (37 mg, 38%): LCMS (ESI) calc'd for C₁₅H₂₀Cl₂N₂O₄ [M + H]⁺: 363, 365 (3 : 2), found 363, 365 (3 : 2); ¹H NMR (400 MHz, CD₃OD) δ 7.47 (d, *J* = 8.9 Hz, 1H), 6.92 (d, *J* = 8.9 Hz, 1H), 4.80-4.41 (m, 3H), 4.19 (dd, *J* = 66.7, 13.9 Hz, 1H), 3.79-3.54 (m, 2H), 3.20-2.95 (m, 1H), 2.87-2.44 (m, 2H), 2.07 (s, 1H), 1.55-1.16 (m, 3H).

### Example 173. Compound 146 ((R)-2-(amino(1-(6-aminopyridin-2-yl)piperidin-4-yl)methyl)-4,5-dichlorophenol trifluoroacetic acid)

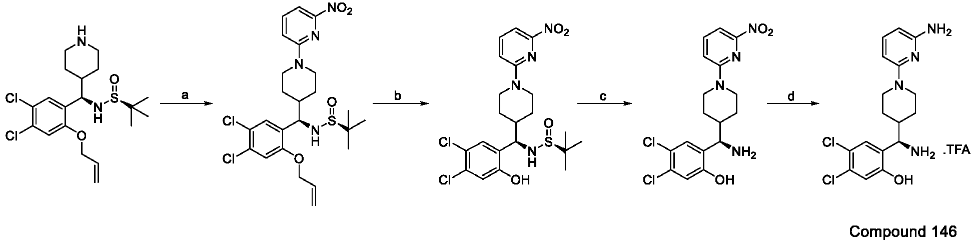

### Step a:

To a stirred solution of (*S*)-*N*-[(*R*)-[4,5-dichloro-2-(prop-2-en-1-yloxy)phenyl](piperidin-4-yl)methyl]-2-methylpropane-2-sulfinamide (0.20 g, 0.48 mmol) and 2-chloro-6-nitropyridine (0.13 g, 0.72 mmol) in DMSO (2 mL) was added K₂CO₃ (0.13 g, 0.96 mmol) at room temperature. The reaction was allowed to warm 80 °C and stirred for 2 h. The resulting mixture was diluted with EA (20 mL) and water (20 mL). Then partitioned aqueous solution was extracted with EA (3 x 30 mL). The combined organic layers were washed with brine (3 x 30 mL), dried over anhydrous Na₂SO₄ and filtered. The filtrate was concentrated under reduced pressure. The residue was purified by Prep-TLC, eluted with PE/EA (1/1) to afford (*S*)-*N*-[(*R*)-[4,5-dichloro-2-(prop-2-en-1-yloxy)phenyl][1-(6-nitropyridin-2-yl)piperidin-4-yl]methyl]-2-methylpropane-2-sulfinamide as a yellow solid (0.12 g, 46%): LCMS (ESI) calc'd for C₂₄H₃₀Cl₂N₄O₄S [M + H]⁺: 541, 543 (3 : 2), found 541, 543 (3 : 2).

### Step b:

To a stirred solution of (*S*)-*N*-[(*R*)-[4,5-dichloro-2-(prop-2-en-1-yloxy)phenyl][1-(6-nitropyridin-2-yl)piperidin-4-yl]methyl]-2-methylpropane-2-sulfinamide (0.12 g, 0.22 mmol) and Pd(PPh₃)₄ (2.56 mg, 0.002 mmol) in THF (1 mL) was added NaBH₄ (16.77 mg, 0.44 mmol) at room temperature under nitrogen atmosphere. After stirring at room temperature for 30 min under nitrogen atmosphere, the resulting mixture was quenched with water (2 mL) and concentrated under reduced pressure to afford *N*-[(*R*)-(4,5-dichloro-2-hydroxyphenyl)[1-(6-nitropyridin-2-yl)piperidin-4-yl]methyl]-2-methylpropane-2-sulfinamide as a light yellow solid (0.11g, crude), which was used in next step directly without further purification: LCMS (ESI) calc'd for C₂₁H₂₆Cl₂N₄O₄S [M + H]⁺: 501, 503 (3 : 2), found 501, 503 (3 : 2).

### Step c:

A solution of *N*-[(*R*)-(4,5-dichloro-2-hydroxyphenyl)[1-(6-nitropyridin-2-yl)piperidin-4-yl]methyl]-2-methylpropane-2-sulfinamide (0.11 g, 0.22 mmol) in HCl (1 mL, 4M) and 1,4-dioxane (1 mL) was stirred at room temperature for 1 h. The resulting solution was purified by reverse phase chromatography, eluted with 42% ACN in water (plus 0.05% TFA) to afford 2-[(*R*)-amino[1-(6-nitropyridin-2-yl)piperidin-4-yl]methyl]-4,5-dichlorophenol as a yellow oil (75 mg, 80%): LCMS (ESI) calc'd for C₁₇H₁₈Cl₂N₄O₃ [M + H]⁺: 397, 399 (3 : 2), found 397, 399 (3 : 2); ¹H NMR (400 MHz, DMSO-*d*₆) δ 10.98 (s, 1H), 7.93-7.81 (m, 1H), 7.68-7.55 (m, 1H), 7.42 (d, *J* = 7.5 Hz, 1H), 7.30 (d, *J* = 8.6 Hz, 1H), 7.11 (d, *J* = 3.9 Hz, 1H), 4.49 (d, *J* = 13.4 Hz, 1H), 4.37-4.17 (m, 2H), 2.97-2.82 (m, 2H), 2.20 (d, *J* = 10.4 Hz, 1H), 2.03-1.88 (m, 1H), 1.38-1.22 (m, 3H).

### Step d:

To a stirred solution of 2-[(*R*)-amino[1-(6-nitropyridin-2-yl)piperidin-4-yl]methyl]-4,5-dichlorophenol (75 mg, 0.19 mmol) and SnCl₂ (0.36 g, 1.89 mmol) in MeOH (3 mL) was added aq. HCl (12 *N*, 3 mL) at room temperature. The resulting mixture was stirred for 1 h at room temperature under nitrogen atmosphere. The residue was purified by reverse phase chromatography with the following conditions: column, C18; mobile phase, ACN in water (plus 0.05% TFA), 5% to 80% gradient in 50 min; Detector: UV 210 nm to afford Compound 146 (2-[(*R*)-amino[1-(6-aminopyridin-2-yl)piperidin-4-yl]methyl]-4,5-dichlorophenol trifluoroacetic acid) as an off-white solid (35 mg, 50%): LCMS (ESI) calc'd for C₁₇H₂₀Cl₂N₄O [M + H]⁺: 367, 369 (3 : 2), found 367, 369 (3 : 2); ¹H NMR (400 MHz, DMSO-*d*₆) δ 11.00 (s, 1H), 8.33 (s, 3H), 7.63 (s, 1H), 7.57 (br, 2H), 7.13 (s, 1H), 6.19-6.14 (m, 1H), 6.03 (s, 1H), 4.24 (s, 1H), 4.13 (d, *J* = 13.2 Hz, 1H), 3.95 (d, *J* = 13.3 Hz, 1H), 2.97-2.92 (m, 2H), 2.18 (d, *J* = 10.5 Hz, 1H), 1.97 (d, *J* = 13.0 Hz, 1H), 1.31-1.21 (m, 3H).

### Example 174. Compound 147 ((R)-2-(amino(1-(5-aminopyridin-2-yl)piperidin-4-yl)methyl)-4,5-dichlorophenol trifluoroacetic acid)

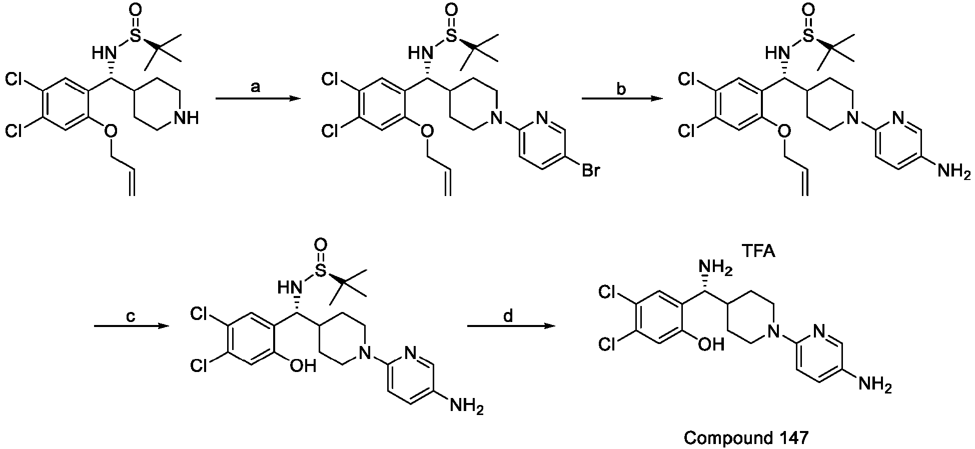

### Step a:

To a solution of (*S*)-*N*-[(*R*)-[4,5-dichloro-2-(prop-2-en-1-yloxy)phenyl](piperidin-4-yl)methyl]-2-methylpropane-2-sulfinamide (0.15 g, 0.36 mmol) and 5-bromo-2-fluoropyridine (94 mg, 0.54 mmol) in ACN (2 mL) was added K₂CO₃ (99 mg, 0.72 mmol) at room temperature. The reaction was allowed to warm to 70 °C and stirred for 3 h at same temperature under nitrogen atmosphere. The resulting mixture was diluted with water (30 mL) and extracted with EA (3 x 20 mL). The combined organic layers were washed with brine (2 x 10 mL), dried over anhydrous Na₂SO₄ and filtered. The filtrate was concentrated under reduced pressure. The residue was purified by Prep-TLC, eluted with EA/PE (1/1) to afford (*S*)-*N*-[(*R*)-[1-(5-bromopyridin-2-yl)piperidin-4-yl][4,5-dichloro-2-(prop-2-en-1-yloxy)phenyl]methyl]-2-methylpropane-2-sulfinamide as a light yellow solid (0.15 g, 68%): LCMS (ESI) calc'd for C₂₄H₃₀BrCl₂N₃O₂S [M + H]⁺: 574, 576, 578, (2 : 3 : 1), found 574, 576, 578, (2 : 3 : 1).

### Step b:

To a stirred solution of (*S*)-*N*-[(*R*)-[1-(5-bromopyridin-2-yl)piperidin-4-yl][4,5-dichloro-2-(prop-2-en-1-yloxy)phenyl]methyl]-2-methylpropane-2-sulfinamide (60 mg, 0.10 mmol) and methyl[2-(methylamino)ethyl]amine (2 mg, 0.02 mmol) and trifluoroacetamide (24 mg, 0.21 mmol) in 1,4-dioxane (3 mL) were added CuI (4 mg, 0.02 mmol) and Cs₂CO₃ (68 mg, 0.21 mmol) at room temperature. The resulting mixture was degassed with nitrogen for three times and stirred for 12 h at 100°C under nitrogen atmosphere. After cooling to room temperature, the resulting mixture was diluted with water (5 mL) and extracted with EA (3 x 25 mL). The combined organic layers were washed with brine (3 x 15 mL), dried over anhydrous Na₂SO₄. After filtration, the filtrate was concentrated under reduced pressure to afford (*S*)-*N-*[(*R*)-[1-(5-aminopyridin-2-yl)piperidin-4-yl][4,5-dichloro-2-(prop-2-en-1-yloxy)phenyl]methyl]-2-methylpropane-2-sulfinamide as an off-white solid (60 mg, 89%): LCMS (ESI) calc'd for C₂₄H₃₂Cl₂N₄O₂S [M + H]⁺: 511, 513 (3 : 2), found 511, 513 (3 : 2).

### Step c:

To a stirred solution of (*S*)-*N*-[(*R*)-[1-(5-aminopyridin-2-yl)piperidin-4-yl][4,5-dichloro-2-(prop-2-en-1-yloxy)phenyl]methyl]-2-methylpropane-2-sulfinamide (60 mg, 0.12 mmol) and Pd(PPh₃)₄ (27 mg, 0.02 mmol) in THF (2 mL) was added NaBH₄ (9 mg, 0.24 mmol) at room temperature under nitrogen atmosphere. The resulting mixture was stirred for 1 h at room temperature under nitrogen atmosphere. The reaction was quenched with saturated aq. NH₄Cl (3 mL) at room temperature and concentrated under reduced pressure to afford (*S*)-*N-*[(*R*)-[1-(5-aminopyridin-2-yl)piperidin-4-yl](4,5-dichloro-2-hydroxyphenyl)methyl]-2-methylpropane-2-sulfinamide as an off-white solid (50 mg, crude), which was used in next step directly without further purification: LCMS (ESI) calc'd for C₂₁H₂₈Cl₂N₄O₂S [M + H]⁺: 471, 473 (3 : 2), found 471, 473 (3 : 2).

### Step d:

To a stirred solution of (*S*)-*N*-[(*R*)-[1-(5-aminopyridin-2-yl)piperidin-4-yl](4,5-dichloro-2-hydroxyphenyl)methyl]-2-methylpropane-2-sulfinamide (50 mg, 0.11 mmol) in 1,4-dioxane (2 mL) was added aq. HCl (4 *N*, 1 mL) at room temperature. The resulting mixture was stirred for 1 h at room temperature. The resulting mixture was concentrated under reduced pressure. The residue was purified by Prep-HPLC with the following conditions: Column: X select CSHOBD Column 30 x 150 mm 5µm; Mobile Phase A: water (plus 0.05% TFA), Mobile Phase B: ACN; Flow rate: 60 mL/min; Gradient: 5% B to 25% B in 7 min; Detector: UV 220 nm; Retention time: 6.25 min. The fractions containing the desired product were collected and concentrated under reduced pressure to afford Compound 147 ((R)-2-(amino(1-(5-aminopyridin-2-yl)piperidin-4-yl)methyl)-4,5-dichlorophenol trifluoroacetic acid) as an off-white solid (15 mg, 38% overall two steps): LCMS (ESI) calc'd for C₁₇H₂₀Cl₂N₄O [M + H]⁺: 367, 369 (3 : 2), found 367, 369 (3 : 2); ¹H NMR (400 MHz, DMSO-*d*₆) δ 11.96 (s, 1H), 11.1-10.9 (m, 1H), 8.30 (s, 3H), 7.68-7.56 (m, 2H), 7.41 (s, 2H), 7.12 (d, *J* = 2.2 Hz, 1H), 6.26 (dd, *J* = 7.0, 1.9 Hz, 1H), 6.00 (s, 1H), 4.24 (s, 1H), 3.92 (d, *J* = 13.2 Hz, 1H), 3.74 (d, *J* = 13.3 Hz, 1H), 3.09-2.89 (m, 2H), 2.25-2.12 (m, 1H), 1.97 (d, *J* = 13.0 Hz, 1H), 1.36-1.28 (m, 2H), 1.28-1.11 (m, 1H); ¹⁹F NMR (376 MHz, DMSO-*d*₆) δ -73.48.

### Example 175. Compound 148 (1-[4-[(R)-amino(4,5-dichloro-2-hydroxyphenyl)methyl]piperidine-1-carbonyl]azetidin-3-ol trifluoroacetic acid)

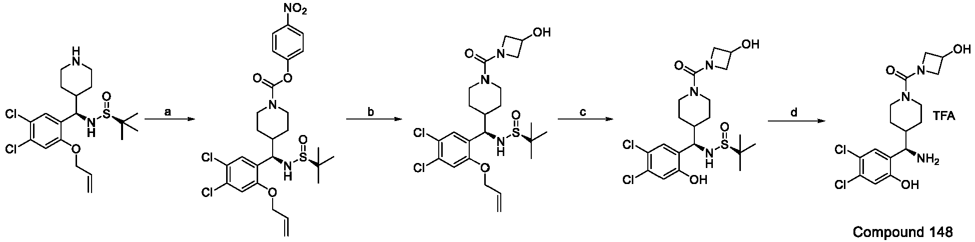

### Step a:

To a stirred solution of (*S*)-*N*-[(*R*)-[4,5-dichloro-2-(prop-2-en-1-yloxy)phenyl](piperidin-4-yl)methyl]-2-methylpropane-2-sulfinamide (0.20 g, 0.48 mmol) and Et₃N (96 mg, 0.95 mmol) in DCM (3 mL) was added 4-nitrophenyl carbonochloridate (0.11 g, 0.57 mmol) at room temperature under nitrogen atmosphere. The resulting mixture was stirred for 16 h at room temperature under nitrogen atmosphere. The resulting mixture was concentrated under reduced pressure. The residue was purified by reverse phase chromatography, eluted with 80% ACN in water (plus 0.05% TFA) to afford 4-nitrophenyl 4-[(*R*)-[4,5-dichloro-2-(prop-2-en-1-yloxy)phenyl]([[(*S*)-2-methylpropane-2-sulfinyl]amino])methyl]piperidine-1-carboxylate as a light yellow solid (0.12 g, 43%): LCMS (ESI) calc'd for C₂₆H₃₁Cl₂N₃O₆S [M + H]⁺: 584, 586 (3 : 2), found 584, 586 (3 : 2).

### Step b:

To a stirred solution of 4-nitrophenyl 4-[(*R*)-[4,5-dichloro-2-(prop-2-en-1-yloxy)phenyl]([[(*S*)-2-methylpropane-2-sulfinyl]amino])methyl]piperidine-1-carboxylate (0.30 g, 0.51 mmol) and azetidin-3-ol (0.11 g, 1.54 mmol) in DMF (4 mL) was added K₂CO₃ (0.14 g, 1.03 mmol) at room temperature. The reaction mixture was irradiated with microwave radiation for 30 min at 150 °C. The resulting mixture was filtered. The filtrate was purified by reverse phase chromatography, eluted with 50% ACN in water (plus 0.05% TFA) to afford (*S*)-*N*-[(*R*)-[4,5-dichloro-2-(prop-2-en-1-yloxy)phenyl][1-(3-hydroxyazetidine-1-carbonyl)piperidin-4-yl]methyl]-2-methylpropane-2-sulfinamide as a light yellow solid (0.19 g, 71%): LCMS (ESI) calc'd for C₂₃H₃₃Cl₂N₃O₄S [M + H]⁺: 518, 520 (3 : 2), found 518, 520 (3 : 2).

### Step c:

To a stirred solution of (*S*)-*N*-[(*R*)-[4,5-dichloro-2-(prop-2-en-1-yloxy)phenyl][1-(3-hydroxyazetidine-1-carbonyl)piperidin-4-yl]methyl]-2-methylpropane-2-sulfinamide (0.10 g, 0.19 mmol) and Pd(PPh₃)₄ (22 mg, 0.02 mmol) in THF (5 mL) was added NaBH₄ (14 mg, 0.38 mmol) at room temperature under nitrogen. The reaction mixture was stirred for 1 h at room temperature under nitrogen. The resulting mixture was quenched with water (50 mL) at room temperature and extracted with EA (3 x 50 mL). The combined organic layers were washed with brine (3 x 30 mL), dried over anhydrous Na₂SO₄. After filtration, the filtrate was concentrated under reduced pressure to afford crude *N*-[(*R*)-(4,5-dichloro-2-hydroxyphenyl)[1-(3-hydroxyazetidine-1-carbonyl)piperidin-4-yl]methyl]-2-methylpropane-2-sulfinamide (50 mg, crude), which was used in next step without further purification: LCMS (ESI) calc'd for C₂₀H₂₉Cl₂N₃O₄S [M + H]⁺: 478, 480 (3 : 2), found 478, 480 (3 : 2).

### Step d:

A solution of *N*-[(*R*)-(4,5-dichloro-2-hydroxyphenyl)[1-(3-hydroxyazetidine-1-carbonyl)piperidin-4-yl]methyl]-2-methylpropane-2-sulfinamide (50 mg, 0.11 mmol) and aq. HCl (6 *N*, 0.5 mL) in 1,4-dioxane (1 mL) was stirred for 30 min at room temperature. The resulting solution was concentrated under reduced pressure. The residue was purified by Prep-HPLC with the following conditions: Column: XBridge Shield RP18 OBD Column, 5 µm, 19 x 150 mm; Mobile Phase A: water (plus 0.05% TFA), Mobile Phase B: ACN; Flow rate: 25 mL/min; Gradient: 5% B to 30% B in 7 min; Detector: UV 220 nm; Retention time: 5.6 min. The fractions containing desired product was collected and concentrated under reduced pressure to afford Compound 148 (1-[4-[(*R*)-amino(4,5-dichloro-2-hydroxyphenyl)methyl]piperidine-1-carbonyl]azetidin-3-ol trifluoroacetic acid) as a purple solid (5 mg, 10% overall two steps): LCMS (ESI) calc'd for C₁₆H₂₁Cl₂N₃O₃ [M + H]⁺: 374, 376 (3 : 2), found 374, 376 (3 : 2); ¹H NMR (400 MHz, CD₃OD) δ 7.45 (s, 1H), 7.10 (s, 1H), 5.27-5.20 (m, 1H), 4.35-4.07 (m, 2H), 4.06-3.55 (m, 5H), 3.32-3.06 (m, 2H), 2.50-2.40 (m, 1H), 2.15-2.03 (m, 1H), 1.53-1.45 (m, 2H), 1.41-1.36 (m, 1H); ¹⁹F NMR (376 MHz, CD₃OD) δ -77.00.

### Example 176. Compound 149 (2-[(R)-amino[1-(1H-pyrazol-4-yl)piperidin-4-yl]methyl]-4,5-dichlorophenol trifluoroacetic acid)

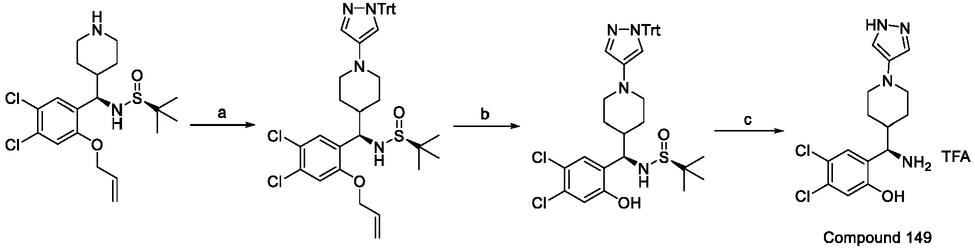

### Step a:

To a stirred solution of (*S*)-*N*-[(*R*)-[4,5-dichloro-2-(prop-2-en-1-yloxy)phenyl](piperidin-4-yl)methyl]-2-methylpropane-2-sulfinamide (0.15 g, 0.36 mmol) and 4-iodo-1-(triphenylmethyl)-1*H*-pyrazole (0.19 g, 0.43 mmol) in DMSO (3 mL) were added Cs₂CO₃ (0.35 g, 1.07 mmol), L-proline (16 mg, 0.14 mmol) and CuI (14 mg, 0.07 mmol) at room temperature under nitrogen atmosphere. The resulting mixture was degassed with nitrogen for three times and stirred for 24 h at 120 °C under nitrogen atmosphere. After cooling to room temperature, the reaction mixture was diluted with water (30 mL) and extracted with EA (3 x 30 mL). The combined organic layers were washed with brine (3 x 20 mL) and dried over anhydrous Na₂SO₄. After filtration, the filtrate was concentrated under reduced pressure. The residue was purified by reverse phase chromatography, eluted with 65% ACN in water (plus 0.05% TFA) to afford (*S*)-*N*-[(*R*)-[4,5-dichloro-2-(prop-2-en-1-yloxy)phenyl]([1-[1-(triphenylmethyl)-1*H*-pyrazol-4-yl]piperidin-4-yl])methyl]-2-methylpropane-2-sulfinamide as a light yellow oil (30 mg, 9%): LCMS (ESI) calc'd for C₄₁H₄₄Cl₂N₄O₂S [M + H]⁺: 727, 729 (3: 2), found 727, 729 (3 : 2).

### Step b:

To a stirred solution of (*S*)-*N*-[(*R*)-[4,5-dichloro-2-(prop-2-en-1-yloxy)phenyl]([1-[1-(triphenylmethyl)-1*H*-pyrazol-4-yl]piperidin-4-yl])methyl]-2-methylpropane-2-sulfinamide (30 mg, 0.04 mmol) and Pd(PPh₃)₄ (5 mg, 0.004 mmol) in THF (1 mL) was added NaBH₄ (3 mg, 0.08 mmol) at room temperature under nitrogen atmosphere. The resulting mixture was stirred for 1 h at room temperature. The reaction was quenched with saturated aq. NH₄Cl (1 mL) at room temperature and concentrated under reduced pressure to afford (*S*)-*N*-[(*R*)-(4,5-dichloro-2-hydroxyphenyl)([1-[1-(triphenylmethyl)-1*H*-pyrazol-4-yl]piperidin-4-yl])methyl]-2-methylpropane-2-sulfinamide as a brown solid (30 mg, crude), which was used in the next step directly without further purification: LCMS (ESI) calc'd for C₃₈H₄₀Cl₂N₄O₂S [M + H]⁺: 687, 689 (3 : 2), found 687, 689 (3 : 2);

### Step c:

To a stirred solution of (*S*)-*N*-[(*R*)-(4,5-dichloro-2-hydroxyphenyl)([1-[1-(triphenylmethyl)-1*H*-pyrazol-4-yl]piperidin-4-yl])methyl]-2-methylpropane-2-sulfinamide (30 mg, 0.04 mmol) in 1,4-dioxane (1 mL) was added aq. HCl (4 *N*, 1 mL) at room temperature. The resulting mixture was stirred for 1 h at room temperature. The resulting mixture was concentrated under reduced pressure. The residue was purified by Prep-HPLC with the following conditions: Column: Xselect CSH OBD Column 30 x 150 mm, 5 µm; Mobile Phase A: water (plus 0.05% TFA), Mobile Phase B: ACN; Flow rate: 60 mL/min; Gradient: 5% B to 20% B in 7 min; Detector: UV 254/220 nm; Retention time: 6.28 min. The fractions containing the desired product were collected and concentrated under reduced pressure to afford Compound 149 (2-[(*R*)-amino[1-(1*H*-pyrazol-4-yl)piperidin-4-yl]methyl]-4,5-dichlorophenol trifluoroacetic acid) as a light yellow solid (4.4 mg, 23% overall two steps): LCMS (ESI) calc'd for C₁₅H₁₈Cl₂N₄O [M + H]⁺: 341, 343 (3 : 2), found 341, 343 (3 : 2); ¹H NMR (400 MHz, CD₃OD) δ 7.74 (s, 2H), 7.49 (s, 1H), 7.11 (s, 1H), 4.27 (d, *J* = 9.7 Hz, 1H), 3.77 (d, *J* = 12.3 Hz, 1H), 3.62-3.52 (m, 1H), 3.20-3.02 (m, 2H), 2.47-2.30 (m, 1H), 2.21 (d, *J* = 13.7 Hz, 1H), 1.83-1.69 (m, 1H), 1.69-1.47 (m, 2H); ¹⁹F NMR (376 MHz, CD₃OD) δ -77.11.

### Example 177. Compound 150 ((R)-2-(amino(1-(4-aminopyridin-2-yl)piperidin-4-yl)methyl)-4,5-dichlorophenol trifluoroacetic acid)

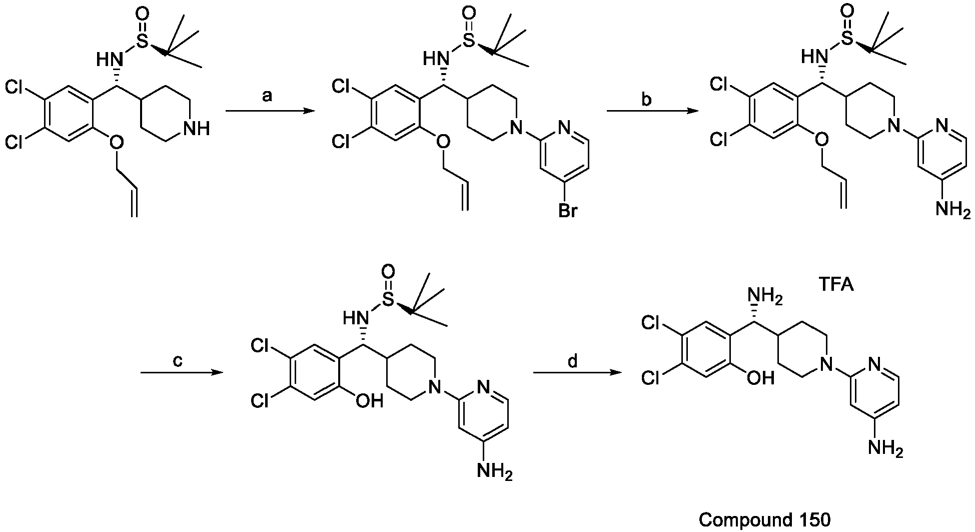

### Step a:

To a stirred solution of (*S*)-*N*-[(*R*)-[4,5-dichloro-2-(prop-2-en-1-yloxy)phenyl](piperidin-4-yl)methyl]-2-methylpropane-2-sulfinamide (80 mg, 0.20 mmol) and 5-bromo-2-fluoropyridine (0.17 g, 0.96 mmol) in ACN (3 mL) was added K₂CO₃ (53 mg, 0.38 mmol) at room temperature. The resulting mixture was stirred for 16 h at 70 °C under nitrogen atmosphere. After cooling to room temperature, the resulting mixture was diluted with water (40 mL) and extracted with EA (3 x 30 mL). The combined organic layers were washed with brine (2 x 20 mL), dried over anhydrous Na₂SO₄. After filtration, the filtrate was concentrated under reduced pressure. The residue was purified by Prep-TLC, eluted with PE/EA (1/2) to afford (*S*)-*N*-[(*R*)-[1-(5-bromopyridin-2-yl)piperidin-4-yl] [4,5-dichloro-2-(prop-2-en-1-yloxy)phenyl]methyl]-2-methylpropane-2-sulfinamide as an off-white solid (60 mg, 49%): LCMS (ESI) calc'd for C₂₄H₃₀BrCl₂N₃O₂S [M + H]⁺: 574, 576, 578 (2 : 3 : 1), found 574, 576, 578 (2 : 3 : 1).

### Step b:

To a stirred solution of (*S*)-*N*-[(*R*)-[1-(4-bromopyridin-2-yl)piperidin-4-yl][4,5-dichloro-2-(prop-2-en-1-yloxy)phenyl]methyl]-2-methylpropane-2-sulfinamide (60 mg, 0.10 mmol) and methyl[2-(methylamino)ethyl]amine (1.9 mg, 0.02 mmol) and trifluoroacetamide (24 mg, 0.21 mmol) in 1,4-dioxane (3 mL) were added CuI (4 mg, 0.02 mmol) and Cs₂CO₃ (68 mg, 0.21 mmol) at room temperature. The resulting mixture was degassed with nitrogen atmosphere for three times and stirred for 12 h at 100 °C under nitrogen atmosphere. The resulting mixture was diluted with water (20 mL) and extracted with EA (3 x 25 mL). The combined organic layers were washed with brine (3 x 5 mL), dried over anhydrous Na₂SO₄. After filtration, the filtrate was concentrated under reduced pressure to afford (*S*)-*N*-[(*R*)-[1-(4-aminopyridin-2-yl)piperidin-4-yl][4,5-dichloro-2-(prop-2-en-1-yloxy)phenyl]methyl]-2-methylpropane-2-sulfinamide as an off-white solid (60 mg, 89%): LCMS (ESI) calc'd for C₂₄H₃₂Cl₂N₄O₂S [M + H]⁺: 511, 513, (3 : 2), found 511, 513, (3 : 2).

### Step c:

To a stirred solution of (*S*)-*N*-[(*R*)-[1-(4-aminopyridin-2-yl)piperidin-4-yl][4,5-dichloro-2-(prop-2-en-1-yloxy)phenyl]methyl]-2-methylpropane-2-sulfinamide (60 mg, 0.12 mmol) and Pd(PPh₃)₄ (27 mg, 0.02 mmol) in THF (3 mL) was added NaBH₄ (8.9 mg, 0.24 mmol) at room temperature. The resulting mixture was stirred for 1 h at room temperature under nitrogen atmosphere. The reaction was quenched with saturated aq. NH₄Cl (3 mL) at room temperature and concentrated under reduced pressure. The residue was purified by reverse phase chromatography with 55% ACN in water (plus 0.05% TFA) to afford (*S*)-*N*-[(*R*)-[1-(4-aminopyridin-2-yl)piperidin-4-yl](4,5-dichloro-2-hydroxyphenyl)methyl]-2-methylpropane-2-sulfinamide as a light yellow solid (50 mg, 54%): LCMS (ESI) calc'd for C₂₁H₂₈Cl₂N₄O₂S [M + H]⁺: 471, 473, (3 : 2), found 471, 473, (3 : 2).

### Step d:

To a stirred solution of (*S*)-*N*-[(*R*)-[1-(4-aminopyridin-2-yl)piperidin-4-yl](4,5-dichloro-2-hydroxyphenyl)methyl]-2-methylpropane-2-sulfinamide (50 mg, 0.11 mmol) in 1,4-dioxane (2 mL) was added aq. HCl (4 *N*, 1 mL) at room temperature. The resulting mixture was stirred for 1 h at room temperature. The reaction mixture was concentrated under reduced pressure. The residue was purified by Prep-HPLC with the following conditions Column: X select CSHOBD Column 30 x 150 mm 5 µm n; Mobile Phase A: water (plus 0.05% TFA), Mobile Phase B: ACN; Flow rate: 60 mL/min; Gradient: 7% B to 23% B in 7 min; Detector: UV 220 nm; Retention time: 6.13 min. The fractions containing desired product was collected and concentrated under reduced pressure to afford Compound 150 ((R)-2-(amino(1-(4-aminopyridin-2-yl)piperidin-4-yl)methyl)-4,5-dichlorophenol trifluoroacetic acid) as an off-white solid (20 mg, 37%): LCMS (ESI) calc'd for C₁₇H₂₀Cl₂N₄O [M + H]⁺: 367, 369 (3 : 2), found 367, 369 (3 : 2); ¹H NMR (400 MHz, CD₃OD) δ 7.53 (d, *J* = 7.2 Hz, 1H), 7.46 (s, 1H), 7.10 (s, 1H), 6.32 (dd, *J* = 7.1, 2.1 Hz, 1H), 6.08 (d, *J* = 2.1 Hz, 1H), 4.20 (d, *J* = 9.9 Hz, 1H), 4.02 (d, *J* = 13.4 Hz, 1H), 3.86 (d, *J* = 13.5 Hz, 1H), 3.11 (d, *J* = 12.6 Hz, 1H), 3.07-2.95 (m, 1H), 2.51-2.41 (m, 1H), 2.12 (d, *J* = 13.3 Hz, 1H), 1.60-1.30 (m, 3H); ¹⁹F NMR (376 MHz, CD₃OD) δ -76.94.

### Example 178. Compound 151 (2-[(R)-amino[1-(5-amino-1,3,4-thiadiazol-2-yl)piperidin-4-yl]methyl]-4,5-dichlorophenol trifluoroacetic acid)

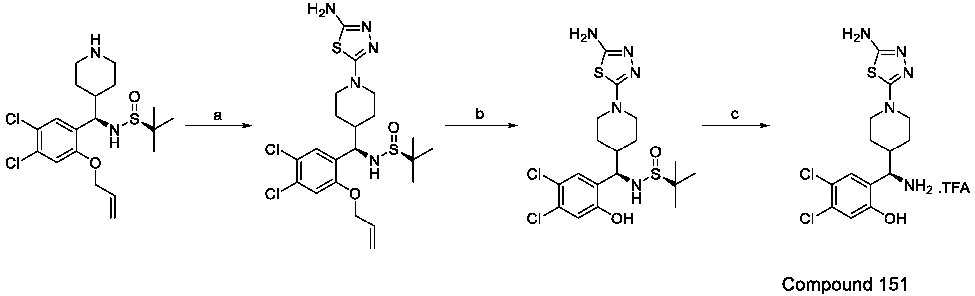

### Step a:

A mixture of (*S*)-*N*-[(*R*)-[4,5-dichloro-2-(prop-2-en-1-yloxy)phenyl](piperidin-4-yl)methyl]-2-methylpropane-2-sulfinamide (0.10 g, 0.24 mmol), 5-bromo-1,3,4-thiadiazol-2-amine (52 mg, 0.29 mmol) and K₂CO₃ (66 mg, 0.48 mmol) in ACN (3 mL) was stirred for 6 h at 80 °C under nitrogen atmosphere. The reaction solution was concentrated under reduced pressure. The residue was purified by Prep-TLC, eluted with PE/EA (1/2) to afford (*S*)-*N*-[(*R*)-[1-(5-amino-1,3,4-thiadiazol-2-yl)piperidin-4-yl][4,5-dichloro-2- (prop-2-en-1-yloxy)phenyl]methyl]-2-methylpropane-2-sulfinamide as a light yellow solid (0.11 g, 71%): LCMS (ESI) calc'd for C₂₁H₂₉Cl₂N₅O₂S₂ [M + H⁺]⁺: 518, 520 (3 : 2), found 518, 520 (3 : 2); ¹H NMR (400 MHz, CDCl₃) δ 7.24 (s, 1H), 6.98 (s, 1H), 6.10-5.96 (m, 1H), 5.40 (dd, *J* = 21.4, 13.8 Hz, 2H), 4.61-4.65 (m, 2H), 4.47-4.39 (m, 1H), 3.98-3.90 (m, 1H), 3.77 (d, *J* = 12.6 Hz, 2H), 3.05-2.84 (m, 2H), 2.12 (d, *J* = 13.4 Hz, 2H), 1.47-1.43 (m, 1H), 1.39-1.20 (m, 2H), 1.16 (s, 9H).

### Step b:

To a stirred solution of (*S*)-*N*-[(*R*)-[1-(5-amino-1,3,4-thiadiazol-2-yl)piperidin-4-yl][4,5-dichloro-2-(prop-2-en-1-yloxy)phenyl]methyl]-2-methylpropane-2-sulfinamide (0.10 g, 0.21 mmol) and Pd(PPh₃)₄ (48 mg, 0.04 mmol) in THF (5 mL) was added NaBH₄ (16 mg, 0.42 mmol) at room temperature under nitrogen. The reaction mixture was stirred for 1 h at room temperature under nitrogen. The resulting mixture was quenched with water (50 mL) at room temperature and extracted with EA (3 x 50 mL). The combined organic layers were washed with brine (3 x 30 mL), dried over anhydrous Na₂SO₄. After filtration, the filtrate was concentrated under reduced pressure to afford crude (*S*)-*N*-[(*R*)-[1-(5-amino-1,3,4-thiadiazol-2-yl)piperidin-4-yl](4,5-dichloro-2-hydroxyphenyl)methyl]-2-methylpropane-2-sulfinamide as a brown solid, which was directly used in next step without further purification: LCMS (ESI) calc'd for C₁₈H₂₅Cl₂N₅O₂S₂ [M + H]⁺: 478, 480 (3 : 2), found 478, 480 (3 : 2).

### Step c:

To a stirred solution of (*S*)-*N*-[(*R*)-[1-(5-amino-1,3,4-thiadiazol-2-yl)piperidin-4-yl](4,5-dichloro-2-hydroxyphenyl)methyl]-2-methylpropane-2-sulfinamide (0.10 g, crude) in 1,4-dioxane (5 mL) was added aq. HCl (6 *N*, 3 mL) at room temperature. The reaction solution was stirred for 1 h at room temperature. The reaction solution was concentrated under reduced pressure. The residue was purified by Prep-HPLC with the following conditions: Column: X select CSH OBD Column 30 x 150 mm 5 µm; Mobile Phase A: water (plus 0.05% TFA), Mobile Phase B: ACN; Flow rate: 60 mL/min; Gradient: 5% B to 23% B in 7 min; Detector: UV 220 nm; Retention time: 6.48 min. The fractions containing desired product were collected and concentrated under reduced pressure to afford Compound 151 (2-[(*R*)-amino[1-(5-amino-1,3,4-thiadiazol-2-yl)piperidin-4-yl]methyl]-4,5-dichlorophenol trifluoroacetic acid) as a purple solid (37 mg, 47% overall 2 steps): LCMS (ESI) calc'd for C₁₄H₁₇Cl₂N₅OS [M + H]⁺: 374, 376 (3 : 2), found 374, 376 (3 : 2); ¹H NMR (400 MHz, CD₃OD) δ 7.45 (s, 1H), 7.09 (s, 1H), 4.20 (d, *J* = 9.9 Hz, 1H), 3.90 (d, *J* = 13.1 Hz, 1H), 3.71 (d, *J* = 13.0 Hz, 1H), 3.22-3.01 (m, 2H), 2.40-2.36 (m, 1H), 2.02-2.10 (m, 1H), 1.51-1.57 (m, 1H), 1.46-1.30 (m, 2H); ¹⁹F NMR (376 MHz, CD₃OD) δ -77.09.

### Example 179. Compound 154 (2-[(R)-amino[1-(1H-pyrazol-3-yl)piperidin-4-yl]methyl]-4,5-dichlorophenol trifluoroacetic acid)

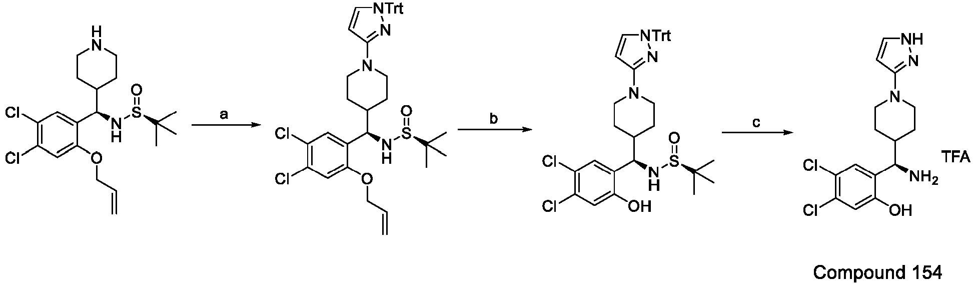

### Step a:

To a stirred solution of (*S*)-*N*-[(*R*)-[4,5-dichloro-2-(prop-2-en-1-yloxy)phenyl](piperidin-4-yl)methyl]-2-methylpropane-2-sulfinamide (0.25 g, 0.60 mmol) and 3-iodo-1-(triphenylmethyl)-1*H*-pyrazole (0.31 g, 0.72 mmol) in DMSO (3 mL) were added Cs₂CO₃ (0.58 g, 1.79 mmol), L-proline (27 mg, 0.24 mmol) and CuI (23 mg, 0.12 mmol) at room temperature under nitrogen atmosphere. The resulting mixture was stirred for 24 h at 120 °C under nitrogen atmosphere. After cooling to room temperature, the reaction solution was diluted with water (30 mL) and extracted with EA (3 x 30 mL). The combined organic layers were washed with brine (3 x 20 mL), dried over anhydrous Na₂SO₄. After filtration, the filtrate was concentrated under reduced pressure. The residue was purified by reverse phase chromatography, eluted with 65% ACN in water (plus 0.05% TFA) to afford (*S*)-*N*-[(*R*)-[4,5-dichloro-2-(prop-2-en-1-yloxy)phenyl]([1-[1-(triphenylmethyl)-1*H*-pyrazol-3-yl]piperidin-4-yl])methyl]-2-methylpropane-2-sulfinamide as a light yellow oil (50 mg, 8%): LCMS (ESI) calc'd for C₄₁H₄₄Cl₂N₄O₂S [M + H]⁺: 727, 729 (3 : 2), found 727, 729 (3 : 2).

### Step b:

To a stirred solution of (*S*)-*N*-[(*R*)-[4,5-dichloro-2-(prop-2-en-1-yloxy)phenyl]([1-[1-(triphenylmethyl)-1H-pyrazol-3-yl]piperidin-4-yl])methyl]-2-methylpropane-2-sulfinamide (50 mg, 0.07 mmol) and Pd(PPh₃)₄ (8 mg, 0.01 mmol) in THF (1 mL) was added NaBH₄ (5 mg, 0.14 mmol) at room temperature under nitrogen. The resulting mixture was stirred for 1 h at room temperature. The reaction was quenched with saturated aq. NH₄Cl (1 mL) at room temperature and concentrated under reduced pressure to afford (*S*)-*N*-[(*R*)-(4,5-dichloro-2-hydroxyphenyl)([1-[1-(triphenylmethyl)-1*H*-pyrazol-3-yl]piperidin-4-yl])methyl]-2-methylpropane-2-sulfinamide as a brown solid (50 mg, crude), which was used in the next step directly without further purification: LCMS (ESI) calc'd for C₃₈H₄₀Cl₂N₄O₂S [M + H]⁺: 687, 689 (3 : 2), found 687, 689 (3 : 2);

### Step c:

To a stirred solution of (*S*)-*N*-[(*R*)-(4,5-dichloro-2-hydroxyphenyl)([1-[1-(triphenylmethyl)-1*H*-pyrazol-3-yl]piperidin-4-yl])methyl]-2-methylpropane-2-sulfinamide (50 mg, 0.07 mmol) in 1,4-dioxane (1 mL) was added aq. HCl (4 *N*, 1 mL) at room temperature. The reaction solution was stirred for 1 h at room temperature. The resulting mixture was concentrated under reduced pressure. The residue was purified by Prep-HPLC with the following conditions: Column: Xselect CSH OBD Column 30 x 150 mm 5 µm; Mobile Phase A: water (plus 0.05% TFA), Mobile Phase B: ACN; Flow rate: 60 mL/min; Gradient: 5% B to 28% B in 7 min; Detector: UV 254/220 nm; Retention time: 6.65 min. The fractions containing the desired product were collected and concentrated under reduced pressure to afford Compound 154 (2-[(*R*)-amino[1-(1*H*-pyrazol-3-yl)piperidin-4-yl]methyl]-4,5-dichlorophenol trifluoroacetic acid) as a purple solid (2.4 mg, 8% overall two steps): LCMS (ESI) calc'd for C₁₅H₁₈Cl₂N₄O [M + H]⁺: 341, 343 (3 : 2), found 341, 343 (3 : 2); ¹H NMR (400 MHz, CD₃OD) δ 7.64 (s, 1H), 7.45 (s, 1H), 7.09 (s, 1H), 5.90 (s, 1H), 4.18 (d, *J* = 9.8 Hz, 1H), 3.84 (d, *J* = 12.5 Hz, 1H), 3.68 (d, *J* = 12.5 Hz, 1H), 2.89 (td, *J* = 12.6, 2.9 Hz, 1H), 2.83-2.70 (m, 1H), 2.33-2.21 (m, 1H), 2.08-1.99 (m, 1H), 1.60-1.48 (m, 1H), 1.44-1.30 (m, 2H); ¹⁹F NMR (376 MHz, CD₃OD) δ -77.09.

### Example 180. Compound 158 (2-[(R)-amino[1-(1H-pyrazole-4-sulfonyl)piperidin-4-yl]methyl]-4,5-dichlorophenol trifluoroacetic acid)

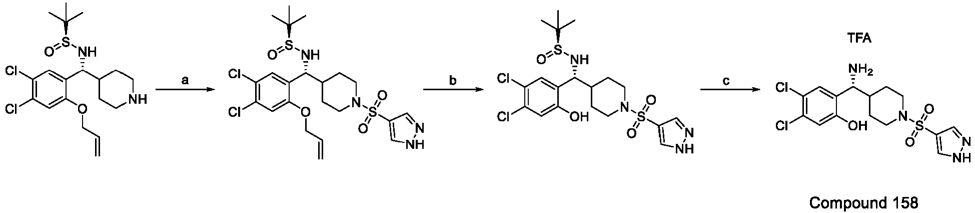

### Step a:

To a stirred solution of (*S*)-*N*-[(*R*)-[4,5-dichloro-2-(prop-2-en-1-yloxy)phenyl](piperidin-4-yl)methyl]-2-methylpropane-2-sulfinamide (50 mg, 0.12 mmol) and Et₃N (24 mg, 0.24 mmol) in DCM (1 mL) was added 1*H*-pyrazole-4-sulfonyl chloride (20 mg, 0.12 mmol) at 0 °C under nitrogen atmosphere. The resulting solution was stirred for 1 h at 0 °C under nitrogen atmosphere. The resulting solution was quenched with water (50 mL) and extracted with EA (3 x 50 mL). The combined organic layers were washed with brine (2 x 20 mL), dried over anhydrous Na₂SO₄. After filtration, the filtrate was concentrated under reduced pressure. The residue was purified by reverse phase chromatography, eluted with 55% ACN in water (plus 0.05 TFA) to afford (*S*)-*N*-[(*R*)-[4,5-dichloro-2-(prop-2-en-1-yloxy)phenyl][1-(1*H-*pyrazole-4-sulfonyl)piperidin-4-yl]methyl]-2-methylpropane-2-sulfinamide as a light yellow oil (70 mg, 85%): LCMS (ESI) calc'd for C₂₂H₃₀Cl₂N₄O₄S₂ [M + H]⁺: 549, 551 (3 : 2), found 549, 551 (3 : 2); ¹H NMR (400 MHz, CDCl₃) δ 7.88 (s, 2H), 7.20 (s, 1H), 6.97 (s, 1H), 6.11-5.86 (m, 1H), 5.46-5.22 (m, 2H), 4.67-4.07 (m, 3H), 3.85-3.71 (m, 2H), 2.18 (t, *J* = 11.5 Hz, 2H), 2.09 (d, *J* = 13.2 Hz, 1H), 1.79-1.68 (m, 1H), 1.61-1.27 (m, 3H), 1.16 (s, 9H).

### Step b:

To a stirred solution of (*S*)-*N*-[(*R*)-[4,5-dichloro-2-(prop-2-en-1-yloxy)phenyl][1-(1*H*-pyrazole-4-sulfonyl)piperidin-4-yl]methyl]-2-methylpropane-2-sulfinamide (70 mg, 0.13 mmol) and Pd(PPh₃)₄ (1.5 mg, 0.001 mmol) in THF (1 mL) was added NaBH₄ (10 mg, 0.26 mmol) at room temperature under nitrogen atmosphere. After stirring for 30 min at room temperature under nitrogen, the resulting mixture was quenched with saturated aq. NH₄Cl (1 mL) and concentrated under reduced pressure to afford (*S*)-*N*-[(*R*)-(4,5-dichloro-2-hydroxyphenyl)[1-(1*H*-pyrazole-4-sulfonyl)piperidin-4-yl]methyl]-2-methylpropane-2-sulfinamide as a brown solid (70 mg, crude), which was used in the next step directly without further purification: LCMS (ESI) calc'd for C₁₉H₂₆Cl₂N₄O₄S₂ [M + H]⁺: 509, 511 (3 : 2), found 509, 511 (3 : 2).

### Step c:

To a stirred mixture of (*S*)-*N*-[(*R*)-(4,5-dichloro-2-hydroxyphenyl)[1-(1*H*-pyrazole-4-sulfonyl)piperidin-4-yl]methyl]-2-methylpropane-2-sulfinamide (70 mg, 0.14 mmol) in 1,4-dioxane (1 mL) was added aq. HCl (4 *N*, 1 mL) at room temperature. The resulting mixture was stirred for 30 min at room temperature. The resulting mixture was concentrated under reduced pressure. The residue was purified by Prep-HPLC with the following conditions: Column: Xselect CSH OBD Column 30 x 150 mm 5 µm; Mobile Phase A: water (plus 0.05% TFA), Mobile Phase B: ACN; Flow rate: 60 mL/min; Gradient: 11% B to 31% B in 7 min; Detector: UV 254/220 nm; Retention time: 6.68 min. The fractions containing the desired product were collected and concentrated under reduced pressure to afford Compound 158 (2-[(*R*)-amino[1-(1*H*-pyrazole-4-sulfonyl)piperidin-4-yl]methyl]-4,5-dichlorophenol trifluoroacetic acid) as a purple solid (49.9 mg, 75% overall two steps): LCMS (ESI) calc'd for C₁₅H₁₈Cl₂N₄O₃S [M + H]⁺: 405, 407 (3 : 2), found 405, 407 (3 : 2); ¹H NMR (400 MHz, CD₃OD) ¹H NMR δ 8.00 (s, 2H), 7.42 (s, 1H), 7.06 (s, 1H), 4.16 (d, *J* = 9.7 Hz, 1H), 3.91-3.78 (m, 1H), 3.75-3.67 (m, 1H), 2.23-2.10 (m, 2H), 2.12-1.97 (m, 2H), 1.57-1.45 (m, 1H), 1.45-1.28 (m, 2H); ¹⁹F NMR (376 MHz, CD₃OD) δ -77.06.

### Example 181. Compound 180 (4-[4-[(R)-amino(4,5-dichloro-2-hydroxyphenyl)methyl]piperidin-1-yl]-1,2-dihydropyridin-2-one trifluoroacetic acid)

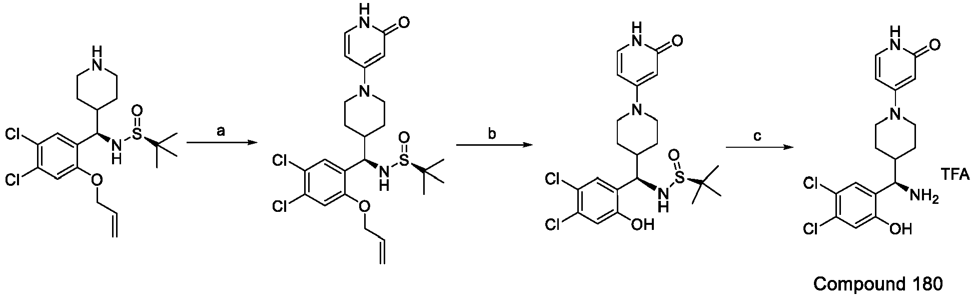

### Step a:

To a stirred solution of (*S*)-*N*-[(*R*)-[4,5-dichloro-2-(prop-2-en-1-yloxy)phenyl](piperidin-4-yl)methyl]-2-methylpropane-2-sulfinamide (80 mg, 0.19 mmol) and 4-fluoro-1,2-dihydropyridin-2-one (32 mg, 0.29 mmol) in ACN (1 mL) was added K₂CO₃ (53 mg, 0.38 mmol) at room temperature. The reaction was stirred at 80 °C for 4 h. After cooling to room temperature, the resulting mixture was filtered. The filtrate was purified by reverse phase chromatography, eluted with 45% ACN in water (plus 0.05% TFA) to afford (*S*)*-N-*[(*R*)-[4,5-dichloro-2-(prop-2-en-1-yloxy)phenyl][1-(2-oxo-1,2-dihydropyridin-4-yl)piperidin-4-yl]methyl]-2-methylpropane-2-sulfinamide as an off-white solid (90 mg, 92%): LCMS (ESI) calc'd for C₂₄H₃₁Cl₂N₃O₃S [M + H]⁺: 512, 514 (3 : 2), found 512, 514 (3 : 2); ¹H NMR (400 MHz, CDCl₃) δ 7.48 (s, 1H), 7.20 (s, 1H), 7.01 (s, 1H), 6.46-6.31 (m, 1H), 6.13-5.93 (m, 2H), 5.48-5.28 (m, 2H), 4.59 (s, 2H), 4.38-4.30 (m, 2H), 4.08-3.88 (m, 2H), 3.08-2.80 (m, 2H), 2.31-2.02 (m, 2H), 1.60-1.42 (m, 1H), 1.42-1.24 (m, 2H), 1.17 (s, 9H).

### Step b:

To a stirred solution of (*S*)-*N*-[(*R*)-[4,5-dichloro-2-(prop-2-en-1-yloxy)phenyl][1-(2-oxo-1,2-dihydropyridin-4-yl)piperidin-4-yl]methyl]-2-methylpropane-2-sulfinamide (90 mg, 0.18 mmol) and Pd(PPh₃)₄ (2 mg, 0.002 mmol) in THF (2 mL) was added NaBH₄ (13 mg, 0.35 mmol) at room temperature. The reaction was stirred at room temperature for 0.5 h. The reaction was quenched with saturated aq. NH₄Cl (1 mL) and concentrated under reduced pressure to afford (*S*)-*N*-[(*R*)-(4,5-dichloro-2-hydroxyphenyl)[1-(2-oxo-1,2-dihydropyridin-4-yl)piperidin-4-yl]methyl]-2-methylpropane-2-sulfinamide as a brown solid (90 mg, crude), which was used in the next step directly without further purification: LCMS (ESI) calc'd for C₂₁H₂₇Cl₂N₃O₃S [M + H]⁺: 472, 474 (3 : 2), found 472, 474 (3 : 2).

### Step c:

To a stirred solution of (*S*)-*N*-[(*R*)-(4,5-dichloro-2-hydroxyphenyl)[1-(2-oxo-1,2-dihydropyridin-4-yl)piperidin-4-yl]methyl]-2-methylpropane-2-sulfinamide (90 mg, 0.19 mmol) in 1,4-dioxane (1 mL) was added aq. HCl (4 *N,* 1 mL) at room temperature. The reaction was stirred at room temperature for 0.5 h. The reaction was concentrated under reduced pressure. The residue was purified by Prep-HPLC with following conditions: Column: X select CSH OBD Column 30 x 150 mm 5 µm; Mobile Phase A: water (plus 0.05% TFA), Mobile Phase B: ACN; Flow rate: 60 mL/min; Gradient: 5% B to 28% B in 7 min; Detector: UV 254/220 nm; Retention time: 6.42 min. The fractions containing the desired product were collected and concentrated under reduced pressure to afford Compound 180 (4-[4-[(*R*)-amino(4,5-dichloro-2-hydroxyphenyl)methyl]piperidin-1-yl]-1,2-dihydropyridin-2-one trifluoroacetic acid) as a light green syrup (46 mg, 55% overall two steps): LCMS (ESI) calc'd for C₁₇H₁₉Cl₂N₃O₂ [M + H]⁺: 368, 370 (3 : 2), found 368, 370 (3 : 2); ¹H NMR (400 MHz, CD₃OD) δ 7.51-7.41 (m, 2H), 7.09 (s, 1H), 6.51 (d, *J* = 7.7 Hz, 1H), 5.94 (d, *J* = 2.6 Hz, 1H), 4.21-4.08 (m, 2H), 4.02 (d, *J* = 13.9 Hz, 1H), 3.17-2.89 (m, 2H), 2.50-2.38 (m, 1H), 2.11-2.01 (m, 1H), 1.53-1.38 (m, 2H), 1.37-1.20 (m, 1H); ¹⁹F NMR (376 MHz, CD₃OD) δ -77.12.

### Example 182. Compound 183 ((2-[(R)-amino[1-(5-amino-1,2,4-oxadiazol-3-yl)piperidin-4-yl]methyl]-4,5-dichlorophenol))

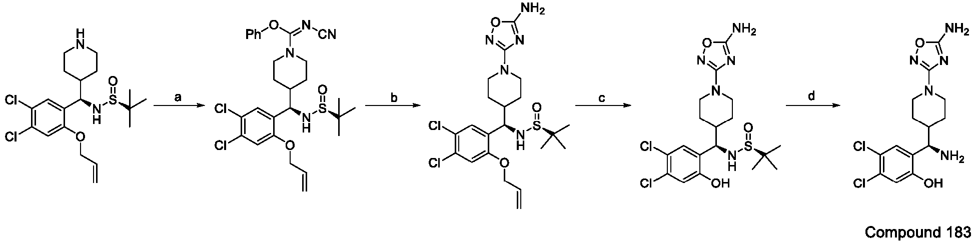

### Step a:

To a solution of (*S*)-*N*-[(*R*)-[4,5-dichloro-2-(prop-2-en-1-yloxy)phenyl](piperidin-4-yl)methyl]-2-methylpropane-2-sulfinamide (0.30 g, 0.72 mmol) in ACN (2 mL) was added cyano(diphenoxymethylidene)amine (0.34 g, 1.43 mmol) at room temperature. The reaction solution was stirred for 3 h at 70 °C. After cooling to room temperature, the resulting solution was concentrated under reduced pressure. The residue was purified by Prep-TLC, eluted with EA/PE (2/1) to afford (phenyl *N*-cyano-4-[(*R*)-[4,5-dichloro-2-(prop-2-en-1-yloxy)phenyl]([[(*S*)-2-methylpropane-2-sulfinyl]amino])methyl]piperidine-1-carboximidate) as an off-white solid (70 mg, 17%): LCMS (ESI) calc'd for C₂₇H₃₂Cl₂N₄O₃S [M + H]⁺: 563, 565 (3 : 2), found 563, 565 (3 : 2).

### Step b:

To a solution of (phenyl *N*-cyano-4-[(*R*)-[4,5-dichloro-2-(prop-2-en-1-yloxy)phenyl]([[(*S*)-2-methylpropane-2-sulfinyl]amino])methyl]piperidine-1-carboximidate) (65 mg, 0.12 mmol) in DMF (1 mL) were added NH₂OH·HCl (48 mg, 0.69 mmol) and DIEA (0.15 g, 1.15 mmol) at room temperature. The reaction mixture was stirred for 16 h at room temperature. The resulting mixture was diluted with water (20 mL) and extracted with EA (3 x 25 mL). The combined organic layers were washed with brine (2 x 15 mL), dried over anhydrous Na₂SO₄ and filtered. The filtrate was concentrated under reduced pressure. The residue was purified by Prep-TLC, eluted with DCM/MeOH (20/1) to afford (*S*)-*N*-[(*R*)-[1-(5-amino-1,2,4-oxadiazol-3-yl)piperidin-4-yl][4,5-dichloro-2-(prop-2-en-1-yloxy)phenyl]methyl]-2-methylpropane-2-sulfinamide as an off-white solid (19 mg, 36%): LCMS (ESI) calc'd for C₂₁H₂₉Cl₂N₅O₃S [M + H]⁺: 502, 504 (3 : 2), found 502, 504 (3 : 2).

### Step c:

To a stirred solution of (*S*)-*N*-[(*R*)-[1-(5-amino-1,2,4-oxadiazol-3-yl)piperidin-4-yl][4,5-dichloro-2-(prop-2-en-1-yloxy)phenyl]methyl]-2-methylpropane-2-sulfinamide (0.10 g, 0.20 mmol) and Pd(PPh₃)₄ (23 mg, 0.02 mmol) in THF (2 mL) was added NaBH₄ (15 mg, 0.40 mmol) at room temperature under nitrogen atmosphere. The reaction mixture was stirred at room temperature for 30 min under nitrogen atmosphere. The resulting mixture was quenched with water (50 mL) at room temperature and extracted with EA (3 x 50 mL). The combined organic layers were washed with brine (3 x 30 mL), dried over anhydrous Na₂SO₄. After filtration, the filtrate was concentrated under reduced pressure to afford crude (*S*)-*N-*[(*R*)-[1-(5-amino-1,2,4-oxadiazol-3-yl)piperidin-4-yl](4,5-dichloro-2-hydroxyphenyl)methyl]-2-methylpropane-2-sulfinamide. The crude product was used in the next step directly without further purification: LCMS (ESI) calc'd for C₂₁H₂₉Cl₂N₅O₃S [M + H]⁺: 462, 464 (3 : 2), found 462, 464 (3 : 2).

### Step d:

To a stirred solution of (*S*)-*N*-[(*R*)-[1-(5-amino-1,2,4-oxadiazol-3-yl)piperidin-4-yl](4,5-dichloro-2-hydroxyphenyl)methyl]-2-methylpropane-2-sulfinamide (80 mg, 0.17 mmol) in 1,4-dioxane (2 mL) was added aq. HCl (4 *N,* 0.8 mL) at 0 °C. The reaction solution was stirred at 0 °C for 30 min. The solution was neutralized to pH 7 with saturated aq. NaHCO₃. The resulting mixture was concentrated under reduced pressure. The residue was purified by Prep-HPLC with the following conditions: Column: XBridge Shield RP18 OBD Column 30 x 150 mm, 5 µm; Mobile Phase A: water with 10 mmol/L NH₄HCO₃, Mobile Phase B: ACN; Flow rate: 60 mL/min; Gradient: 30% B to 50% B in 7 min; Detector: UV 254/220 nm; Retention time: 6.5 min. The fractions containing the desired product were collected and concentrated under reduced pressure to afford Compound 183 (2-[(R)-amino[1-(5-amino-1,2,4-oxadiazol-3-yl)piperidin-4-yl]methyl]-4,5-dichlorophenol) as an off-white solid (9.8 mg, 16% overall two steps): LCMS (ESI) calc'd for C₁₄H₁₇C₁₂N₅O₂ [M + H]⁺: 358, 360 (3 : 2), found 358, 360 (3 : 2); ¹H NMR (400 MHz, CD₃OD) δ 7.22 (s, 1H), 6.87 (s, 1H), 3.90 (d, *J =* 7.7 Hz, 2H), 3.82 (d, *J =* 13.1 Hz, 1H), 2.90-2.78 (m, 1H), 2.75 (td, *J =* 12.6, 3.0 Hz, 1H), 1.94 (d, *J =* 12.5 Hz, 2H), 1.45-1.20 (m, 3H).

### Example 183. Compound 188 ((R)-1-(4-((R)-amino(2,3,4-trichloro-6-hydroxyphenyl)methyl)piperidin-1-yl)-2,3-dihydroxypropan-1-one trifluoroacetic acid)

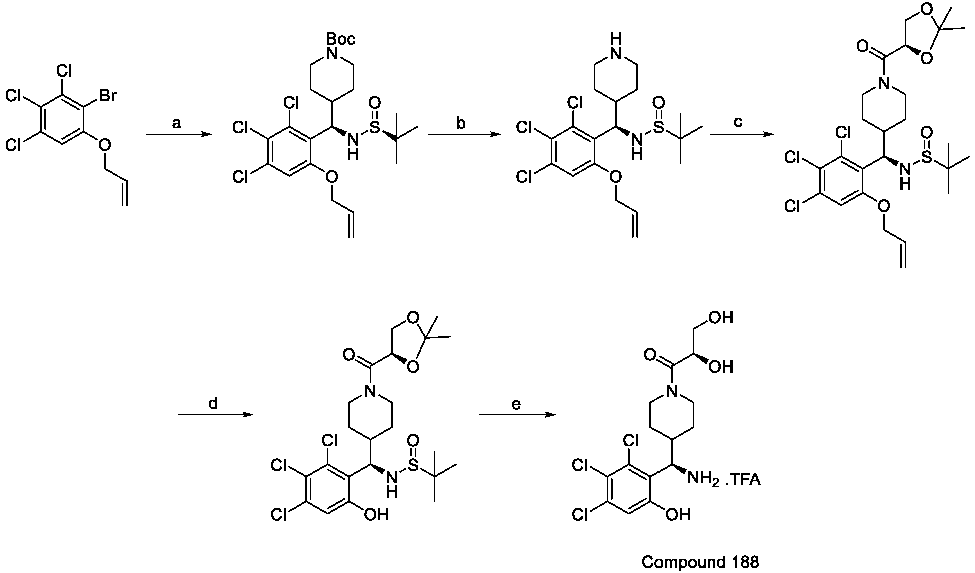

### Step a:

To a stirred mixture of 2-bromo-3,4,5-trichloro-1-(prop-2-en-1-yloxy)benzene (Intermediate 9, Example 9) (0.35 g, 1.11 mmol) in THF (10 mL) were added n-BuLi (0.26 mL 1.65 mmol, 2.50 M in hexane) dropwise at -65 °C under nitrogen atmosphere. After stirring for 0.5 h at -65 °C, a solution of *tert*-butyl 4-([[(*S*)-2-methylpropane-2-sulfinyl]imino]methyl)piperidine-1-carboxylate (0.53 g, 1.66 mmol) in THF (5 mL) was added dropwise over 10 min at -65 °C. The reaction mixture was stirred for additional 0.5 h at -65 °C. The resulting mixture was quenched with water (50 mL) at -65 °C and extracted with EA (2 x 50 mL). The combined organic layers were washed with brine (2 x 20 mL), dried over anhydrous Na₂SO₄. After filtration, the filtrate was concentrated under reduced pressure. The residue was purified by silica gel column chromatography, eluted with PE/EA (5:1) to afford *tert*-butyl 4-[(*R*)-[(2-methylpropane-2-sulfinyl)amino][2,3,4-trichloro-6-(prop-2-en-1-yloxy)phenyl]methyl]piperidine-1-carboxylate as a yellow oil (0.25 g, 37%): LCMS (ESI) calc'd for C₂₄H₃₅Cl₃N₂O₄S [M + H]⁺: 553, 555, 557 (3 : 3 : 1), found 553, 555, 557 (3 : 3 : 1); ¹H NMR (400 MHz, CDCl₃) δ 6.98 (s, 1H), 6.12-5.95 (m, 1H), 5.47-5.40 (m, 2H), 4.76 (t, *J =* 9.5 Hz, 1H), 4.65-4.56 (m, 2H), 4.48-4.41 (d, *J =* 10.1 Hz, 1H), 4.26-4.01 (m, 2H), 2.76-2.46 (m, 3H), 2.35 (d, *J =* 13.7 Hz, 1H), 1.40-1.12 (m, 3H), 1.47 (s, 9H), 1.05 (s, 9H).

### Step b:

To a stirred solution of *tert*-butyl 4-[(*R*)-[(2-methylpropane-2-sulfinyl)amino][2,3,4-trichloro-6-(prop-2-en-1-yloxy)phenyl]methyl]piperidine-1-carboxylate (0.25 g, 0.45 mmol) in DCM (2 mL) was added TFA (1 mL) dropwise at room temperature. The reaction solution was stirred for 1 h at room temperature. The reaction solution was neutralized to pH 7 with saturated aq. NaHCO₃ at 0 °C, and then extracted with EA (5 x 50 mL). The combined organic layers were washed with brine (2 x 10 mL), dried over anhydrous Na₂SO₄. After filtration, the filtrate was concentrated under reduced pressure. The crude product was purified by reverse phase chromatography, eluted with 40% ACN in water with 10 mmol/L NH₄HCO₃ to afford (*S*)*-N-*((*R*)-(6-(allyloxy)-2,3,4-trichlorophenyl)(piperidin-4-yl)methyl)-2-methylpropane-2-sulfinamide as a yellow oil (0.14 g, 62%): LCMS (ESI) calc'd for C₁₉H₂₇Cl₃N₂O₂S [M + H]⁺: 453, 455, 457 (3 : 3 : 1), found 453, 455, 457 (3 : 3 : 1); ¹H NMR (400 MHz, CDCl₃) δ 7.03 (s, 1H), 6.14-6.04 (m, 1H), 5.48-5.39 (m, 2H), 5.18-4.99 (m, 1H), 4.82 (d, *J =* 9.6 Hz, 1H), 4.74-4.55 (m, 2H), 3.59 (d, *J =* 12.8 Hz, 1H), 3.42 (d, *J =* 12.7 Hz, 1H), 3.03-2.79 (m, 2H), 2.55-2.44 (m, 1H), 2.36-2.19 (m, 1H), 1.72-1.41 (m, 3H), 1.09 (s, 9H).

### Step c:

To a stirred solution of HATU (0.20 g, 0.53 mmol) and (4*R*)-2,2-dimethyl-1,3-dioxolane-4-carboxylic acid (77 mg, 0.53 mmol) in DMF (3 mL) were added (*S*)*-N*-((*R*)-(6-(allyloxy)-2,3,4-trichlorophenyl)(piperidin-4-yl)methyl)-2-methylpropane-2-sulfinamide (0.12 g, 0.26 mmol) and Et₃N (54 mg, 0.53 mmol) at room temperature. After stirring for 2 h at room temperature, the resulting solution was quenched with water (30 mL) at room temperature and extracted with EA (3 x 50 mL). The combined organic layers were washed with brine (2 x 20 mL), dried over anhydrous Na₂SO₄. After filtration, the filtrate was concentrated under reduce pressure. The residue was purified by reverse phase chromatography, eluted with 70% ACN in water with 20 mmol/L NH₄HCO₃ to afford (*S*)-*N*-((*R*)-(6-(allyloxy)-2,3,4-trichlorophenyl)(1-((*R*)-2,2-dimethyl-1,3-dioxolane-4-carbonyl)piperidin-4-yl)methyl)-2-methylpropane-2-sulfinamide as a yellow oil (44 mg, 28%): LCMS (ESI) calc'd for C₂₅H₃₅Cl₃N₂O₅S [M + H]⁺: 581, 583, 585 (3 : 3 : 1), found 581, 583, 585 (3 : 3 : 1); ¹H NMR (400 MHz, CDCl₃) δ 7.03 (s, 1H), 6.14-5.93 (m, 1H), 5.54-5.32 (m, 2H), 4.83-4.34 (m, 4H), 3.97-3.48 (m, 4H), 3.18-2.90 (m, 2H), 2.70-2.49 (m, 2H), 2.30-2.16 (m, 1H), 1.50-1.20 (m, 9H), 1.03 (s, 9H).

### Step d:

To a stirred solution of (*S*)-*N*-((*R*)-(6-(allyloxy)-2,3,4-trichlorophenyl)(1-((*R*)-2,2-dimethyl-1,3-dioxolane-4-carbonyl)piperidin-4-yl)methyl)-2-methylpropane-2-sulfinamide (40 mg, 0.07 mmol) and Pd(PPh₃)₄ (9 mg, 0.007 mmol) in THF (3 mL) was added NaBH₄ (6 mg, 0.15 mmol) at room temperature under nitrogen atmosphere. The reaction mixture was stirred for 2 h at room temperature under nitrogen atmosphere. The resulting mixture was quenched with saturated aq. NH₄Cl (0.5 mL) at room temperature and concentrated under reduced pressure to afford crude (*S*)-*N*-((*R*)-(1-((*R*)-2,2-dimethyl-1,3-dioxolane-4-carbonyl)piperidin-4-yl)(2,3,4-trichloro-6-hydroxyphenyl)methyl)-2-methylpropane-2-sulfinamide. The crude product was used in the next step directly without further purification: LCMS (ESI) calc'd for C₂₂H₃₁Cl₃N₂O₅S [M + H]⁺: 541, 543 (1 : 1), found 541, 543 (1 : 1).

### Step e:

A solution of (*S*)-*N*-((*R*)-(1-((*R*)-2,2-dimethyl-1,3-dioxolane-4-carbonyl)piperidin-4-yl)(2,3,4-trichloro-6-hydroxyphenyl)methyl)-2-methylpropane-2-sulfinamide (crude) in 1,4-dioxane (5 mL) was added aq. HCl (6 *N,* 3 mL) at room temperature. The reaction solution was stirred for 1 h at room temperature. The reaction solution was concentrated under reduced pressure. The residue was purified by Prep-HPLC with the following conditions: Column: XBridge Shield RP18 OBD Column, 5 µm, 19 x 150 mm; Mobile Phase A: water (plus 0.05% TFA), Mobile Phase B: ACN; Flow rate: 25 mL/min; Gradient: 15% B to 23% B in 7 min; Detector: UV 220/254 nm; Retention time: 6.5 min. The fractions containing desired product were collected and concentrated under reduced pressure to afford Compound 188 ((*R*)-1-(4-((*R*)-amino(2,3,4-trichloro-6-hydroxyphenyl)methyl)piperidin-1-yl)-2,3-dihydroxypropan-1-one trifluoroacetic acid) as an off-white solid (11.9 mg, 30% overall two steps): LCMS (ESI) calc'd for C₁₅H₁₉Cl₃N₂O₄ [M + H]⁺: 397, 399, 401 (3 : 3 : 1), found 397, 399, 401 (3 : 3 : 1); ¹H NMR (400 MHz, CD₃OD) δ 7.13 (s, 1H), 4.73-4.49 (m, 3H), 4.18 (dd, *J* = 65.2, 13.9 Hz, 1H), 3.79-3.50 (m, 2H), 3.20-2.91 (m, 1H), 2.75-2.48 (m, 2H), 2.17-2.00 (m, 1H), 1.54-1.27 (m, 3H); ¹⁹F NMR (376 MHz, CD₃OD) δ -76.95.

### Example 184. (which is a reference example) Compound 189 ((2R)-1-[4-[(R)-amino(3-hydroxynaphthalen-2-yl)methyl]piperidin-1-yl]-2,3-dihydroxypropan-1-one trifluoroacetic acid)

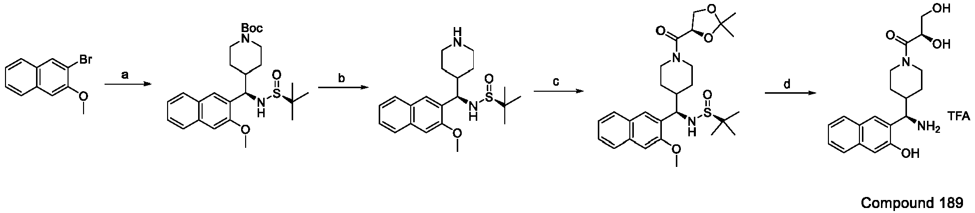

### Step a:

To a stirred solution of 2-bromo-3-methoxynaphthalene (0.30 g, 1.3 mmol) in THF (5 mL) was added *n*-BuLi (0.61 mL, 1.5 mmol, 2.5 M in hexanes) dropwise at -78 °C under nitrogen atmosphere. The reaction mixture was stirred for 30 min at -78 °C under nitrogen atmosphere. To the above mixture was added a solution of *tert*-butyl 4-([[(S)-2-methylpropane-2-sulfinyl]imino]methyl)piperidine-1-carboxylate (0.30 g, 1 mmol) in THF (2 mL) dropwise over 5 min at -78 °C. The resulting mixture was stirred for additional 2 h from -78 °C to room temperature. The resulting solution was quenched with saturated aq. NH₄Cl (30 mL) at room temperature and extracted with EA (3 x 50 mL). The combined organic layers were washed with brine (3 x 20 mL), dried over anhydrous Na₂SO₄. After filtration, the filtrate was concentrated under reduced pressure. The residue was purified by reverse phase chromatography, eluted with 60% ACN in water (plus 0.05% TFA) to afford *tert*-butyl 4-[(*R*)-(3-methoxynaphthalen-2-yl)([[(*S*)-2-methylpropane-2-sulfinyl]amino])methyl]piperidine-1-carboxylate as an off-white solid (0.50 g, 83%): LCMS (ESI) calc'd for C₂₆H₃₈N₂O₄S [M + H]⁺: 475, found 475; ¹H NMR (400 MHz, CDCl₃) δ 7.75 (d, *J =* 8.3 Hz, 2H), 7.55 (s, 1H), 7.49-7.43 (m, 1H), 7.40-7.34 (m, 1H), 7.17 (s, 1H), 4.25-4.12 (m, 2H), 4.08-3.95 (m, 4H), 3.18-3.07 (m, 1H), 2.77-2.49 (m, 2H), 2.20-1.98 (m, 1H), 1.83-1.59 (m, 1H), 1.51-1.28 (m, 11H), 1.26 (s, 9H).

### Step b:

To a stirred solution of *tert*-butyl 4-[(*R*)-(3-methoxynaphthalen-2-yl)([[(*S*)-2-methylpropane-2-sulfinyl]amino])methyl]piperidine-1-carboxylate (0.30 g, 0.60 mmol) in DCM (2 mL) was added TFA (0.5 mL) at room temperature. The reaction solution was stirred for 30 min at room temperature. The mixture was basified to pH 8 with saturated aq. NaHCO₃ at 0 °C and extracted with EA (3 x 50 mL). The combined organic layers were washed with brine (3 x 20 mL), dried over anhydrous Na₂SO₄. After filtration, the filtrate was concentrated under reduced pressure to afford (*S*)-*N*-[(*R*)-(3-methoxynaphthalen-2-yl)(piperidin-4-yl)methyl]-2-methylpropane-2-sulfinamide as a light yellow oil (0.25 g, crude), which used in the next step directly without further purification: LCMS (ESI) calc'd for C₂₁H₃₀N₂O₂S [M + H]⁺: 375, found 375.

### Step c:

To a stirred solution of (4*R*)-2,2-dimethyl-1,3-dioxolane-4-carboxylic acid (0.15 g, 1.00 mmol) and HATU (0.51 g, 1.30 mmol) in DMF (3 mL) were added (*S*)*-N-*[(*R*)-(3-methoxynaphthalen-2-yl)(piperidin-4-yl)methyl]-2-methylpropane-2-sulfinamide (0.25 g, 0.70 mmol) and Et₃N (0.20 g, 2.00 mmol) at room temperature. The reaction solution was stirred at room temperature for 30 min. The resulting solution was quenched with water (30 mL) and extracted with EA (2 x 30 mL). The combined organic layers were washed with brine (2 x 10 mL), dried over anhydrous Na₂SO₄. After filtration, the filtrate was concentrated under reduced pressure. The residue was purified by reverse phase chromatography, eluted with 60% ACN in water (plus 0.05% TFA) to afford (*S*)-*N*-[(*R*)-[1-[(4*R*)-2,2-dimethyl-1,3-dioxolane-4-carbonyl]piperidin-4-yl](3-methoxynaphthalen-2-yl)methyl]-2-methylpropane-2-sulfinamide as a light yellow solid (0.13 g, 38%): LCMS (ESI) calc'd for C₂₇H₃₈N₂O₅S [M + H]⁺: 503, found 503; ¹H NMR (400 MHz,CDCl₃) δ 7.76 (d, *J =* 8.0 Hz, 2H), 7.60-7.44 (m, 2H), 7.40 (t, *J* = 7.5 Hz, 1H), 7.19 (s, 1H), 4.76-4.61 (m, 1H), 4.53-4.28 (m, 2H), 4.26-4.04 (m, 2H), 4.00 (s, 3H), 3.95-3.48 (m, 1H), 3.19-2.82 (m, 1H), 2.69-2.44 (m, 1H), 2.40-2.12 (m, 2H), 1.45-1.23 (m, 9H), 1.12 (d, *J =* 13.4 Hz, 9H).

### Step d:

To a stirred mixture of (*S*)-*N*-[(*R*)-[1-[(4*R*)-2,2-dimethyl-1,3-dioxolane-4-carbonyl]piperidin-4-yl](3-methoxynaphthalen-2-yl)methyl]-2-methylpropane-2-sulfinamide (0.16 g, 0.30 mmol) in DCM (3 mL) was added BBr₃ (0.39 g, 1.6 mmol) at room temperature. The reaction mixture was stirred for 3 h at room temperature. The reaction was quenched with water (3 mL) at 0 °C. The resulting mixture was concentrated under reduced pressure. The residue was purified by Prep-HPLC with the following conditions: Column: XBridge Shield RP18 OBD Column, 5 µm, 19 x 150 mm; Mobile Phase A: water (plus 0.05% TFA), Mobile Phase B: ACN; Flow rate: 25 mL/min; Gradient: 8% B to 25% B in 7 min; Detector: UV 254/220 nm; Retention time: 5.38 min. The fractions containing the desired product were collected and concentrated under reduced pressure to afford Compound 189 ((2*R*)-1-[4-[(*R*)-amino(3-hydroxynaphthalen-2-yl)methyl]piperidin-1-yl]-2,3-dihydroxypropan-1-one trifluoroacetic acid) as a light yellow solid (29 mg, 21%): LCMS (ESI) calc'd for C₁₉H₂₄N₂O₄ [M + H]⁺: 345, found 345; ¹H NMR (400 MHz, CD₃OD) δ 7.82-7.75 (m, 2H), 7.69 (d, *J =* 8.2 Hz, 1H), 7.49-7.42 (m, 1H), 7.35 (t, *J =* 7.5 Hz, 1H), 7.26 (s, 1H), 4.72-4.43 (m, 2H), 4.35-3.96 (m, 2H), 3.77-3.56 (m, 2H), 3.09 (dt, *J =* 60.5, 13.3 Hz, 1H), 2.82-2.49 (m, 2H), 2.10 (d, *J =* 12.6 Hz, 1H), 1.54-1.06 (m, 3H); ¹⁹F NMR (376 MHz, CD₃OD) δ -77.02.

### Example 185. Compound 190 ((2R)-1-[4-[(R)-amino(5-chloro-2-hydroxy-4-methylphenyl)methyl]piperidin-1-yl]-2,3-dihydroxypropan-1-one trifluoroacetic acid)

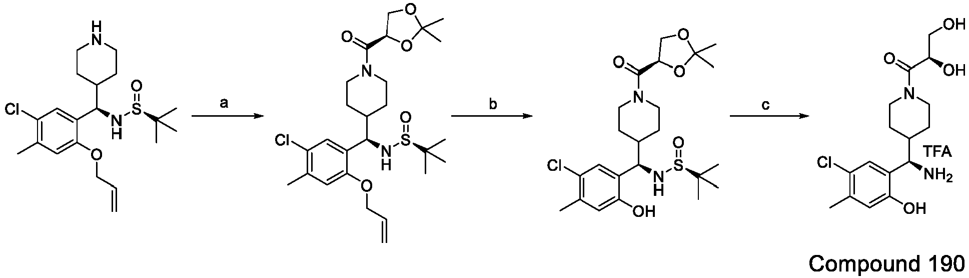

### Step a:

To a stirred mixture of (4*R*)-2,2-dimethyl-1,3-dioxolane-4-carboxylic acid (38 mg, 0.26 mmol) and HATU (0.10 g, 0.26 mmol) in DMF (1 mL) were added a solution of (*S*)*-N-*[(*R*)-[5-chloro-4-methyl-2-(prop-2-en-1-yloxy)phenyl](piperidin-4-yl)methyl]-2-methylpropane-2-sulfinamide (70 mg, 0.18 mmol) in DMF (1 mL) and Et₃N (35 mg, 0.35 mmol) at room temperature. The resulting mixture was stirred for 2 h at room temperature under nitrogen atmosphere. The reaction was quenched with water (40 mL) at room temperature and extracted with EA (3 x 20 mL). The combined organic layers were washed with brine (3 x 10 mL), dried over anhydrous Na₂SO₄. After filtration, the filtrate was concentrated under reduced pressure to afford (*S*)-*N*-((*R*)-(2-(allyloxy)-5-chloro-4-methylphenyl)(1-((*R*)-2,2-dimethyl-1,3-dioxolane-4-carbonyl)piperidin-4-yl)methyl)-2-methylpropane-2-sulfinamide as a light yellow oil (0.12 g, crude): LCMS (ESI) calc'd for C₂₆H₃₉ClN₂O₅S [M + H]⁺: 527, 529 (3 : 1), found 527, 529 (3 : 1).

### Step b:

To a stirred mixture of (*S*)-*N*-((*R*)-(2-(allyloxy)-5-chloro-4-methylphenyl)(1-((*R*)-2,2-dimethyl-1,3-dioxolane-4-carbonyl)piperidin-4-yl)methyl)-2-methylpropane-2-sulfinamide (0.12 g, 0.23 mmol) and Pd(PPh₃)₄ (52 mg, 0.05 mmol) in THF (2 mL) was added NaBH₄ (17 mg, 0.46 mmol) at room temperature under nitrogen atmosphere. The resulting mixture was stirred for 1 h at room temperature under nitrogen atmosphere. The resulting mixture was quenched with water (3 mL) and concentrated under reduced pressure to afford (*S*)-*N*-[(*R*)-(5-chloro-2-hydroxy-4-methylphenyl)([1-[(4*R*)-2,2-dimethyl-1,3-dioxolane-4-carbonyl]piperidin-4-yl])methyl]-2-methylpropane-2-sulfinamide as a brown oil (0.12 g, crude), which was used in next step without further purification: LCMS (ESI) calc'd for C₂₃H₃₅ClN₂O₅S [M + H]⁺: 487, 489 (3 : 1), found 487, 489 (3 : 1).

### Step c:

To a stirred solution of (*S*)-*N*-[(*R*)-(5-chloro-2-hydroxy-4-methylphenyl)([1-[(4*R*)-2,2-dimethyl-1,3-dioxolane-4-carbonyl]piperidin-4-yl])methyl]-2-methylpropane-2-sulfinamide (0.12 g, crude) in THF (2 mL) was added aq. HCl (4 *N,* 1 mL) at room temperature. The resulting mixture was stirred for additional 30 min at room temperature. The resulting mixture was concentrated under reduced pressure. The residue was purified by Prep-HPLC with the following conditions Column: X Bridge Shield RP18 OBD Column, 5 µm, 19 x 150 mm; Mobile Phase A: water (plus 0.05% TFA), Mobile Phase B: ACN; Flow rate: 25 mL/min; Gradient: 10% B to 20% B in 7 min; Detector UV 220 nm; Retention time: 5.62 min. The fractions containing the desired product were collected and concentrated under reduced pressure to afford Compound 190 ((2*R*)-1-[4-[(*R*)-amino(5-chloro-2-hydroxy-4-methylphenyl)methyl]piperidin-1-yl]-2,3-dihydroxypropan-1-one trifluoroacetic acid) as an off-white solid (27 mg, 22% overall three steps): LCMS (ESI) calc'd for C₁₆H₂₃ClN₂O₄ [M + H]⁺: 343, 345 (3 : 1), found 343, 345 (3 : 1); ¹H NMR (400 MHz, CD₃OD) δ 7.24 (s, 1H), 6.85 (s, 1H), 4.73-4.38 (m, 2H), 4.37-3.90 (m, 2H), 3.87-3.47 (m, 2H), 3.22-2.93 (m, 1H), 2.66 (dt, *J =* 45.2, 12.9 Hz, 1H), 2.38 (s, 1H), 2.32 (s, 3H), 2.02 (s, 1H), 1.51-1.08 (m, 3H); ¹⁹F NMR (376 MHz, CD₃OD) δ -77.08.

The compounds in Table 1c below were prepared in an analogous fashion to that described for Compound 190, starting from (*S*)-*N*-[(*R*)-[5-chloro-4-methyl-2-(prop-2-en-1-yloxy)phenyl](piperidin-4-yl)methyl]-2-methylpropane-2-sulfinamide and the corresponding acids, which were prepared as described herein, or which were available from commercial sources. Compounds in Table 1c marked with a * are reference compounds.

**Table 1c**

| **Compound Number** | **Structure** | **Chemical Name** | **MS & NMR** |
|---|---|---|---|
| **233** | 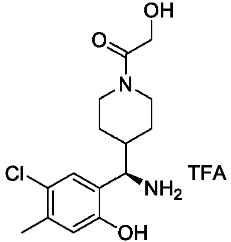 | (*R*)-1-(4-(amino(5-chloro-2-hydroxy-4-methylphenyl)methyl)p iperidin-1-yl)-2-hydroxyethan-1-one trifluoroacetic acid | [M + H]⁺: 313, 315 (3 : 1); ¹H NMR (400 MHz, CD₃OD) δ 7.24 (s, 1H), 6.85 (s, 1H), 4.55 (dd, *J =* 55.8, 13.5 Hz, 1H), 4.26-4.16 (m, 2H), 4.12 (dd, *J* = 14.5, 10.0 Hz, 1H), 3.80 (dd, *J* = 59.0, 13.8 Hz, 1H), 3.01 (dt, *J* = 47.6, 13.0 Hz, 1H), 2.67 (dt, *J* = 46.0, 12.7 Hz, 1H), 2.42-2.28 (m, 4H), 2.00 (d, *J* = 12.9 Hz, 1H), 1.44-1.07 (m, 3H); ¹⁹F NMR (376 MHz, CD₃OD) δ -77.09. |
| **234** | 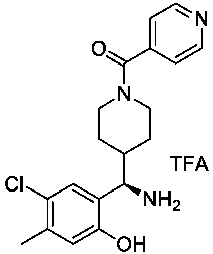 | 2-[(*R*)-amino[1-(pyridine-4-carbonyl)piperidin-4-yl]methyl]-4-chloro-5-methylphenol trifluoroacetic acid | [M + H]⁺: 360, 362 (3 : 1); ¹H NMR (400 MHz, CD₃OD) δ 8.95-8.60 (m, 2H), 7.84-7.51 (m, 2H), 7.25 (d, *J* = 9.2 Hz, 1H), 6.85 (d, *J* = 12.8 Hz, 1H), 4.69 (dd, *J* = 57.3, 13.6 Hz, 1H), 4.16 (t, *J* = 12.0 Hz, 1H), 3.73-3.44 (m, 1H), 3.27-3.01 (m, 1H), 2.88 (dt, *J* = 46.8, 13.0 Hz, 1H), 2.53-2.35 (m, 1H), 2.32 (d, *J* = 13.8 Hz, 3H), 2.18-1.84 (m, 1H), 1.57-1.13 (m, 3H); ¹⁹F NMR (376 MHz, CD₃OD) -77.01. |
| **235** | 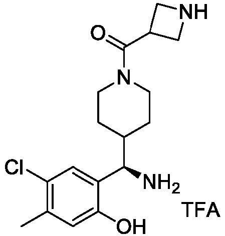 | 2-[(*R*)-amino[1-(azetidine-3-carbonyl)piperidin-4-yl]methyl]-4-chloro-5-methylphenol trifluoroacetic acid | [M + H]⁺: 338, 340 (3 : 1); ¹H NMR (400 MHz, CD₃OD) δ 7.23 (s, 1H), 6.85 (s, 1H), 4.58 (dd, *J =* 53.8, 13.6 Hz, 1H), 4.35-4.18 (m, 4H), 4.18-3.91 (m, 2H), 3.65 (dd, *J* = 58.8, 13.9 Hz, 1H), 3.21-2.92 (m, 1H), 2.69 (dt, *J* = 48.3, 12.8 Hz, 1H), 2.45-2.28 (m, 4H), 2.05-1.95 (m, 1H), 1.51-0.95 (m, 3H); ¹⁹F NMR (376 MHz, CD₃OD) δ -77.32. |
| **237** | 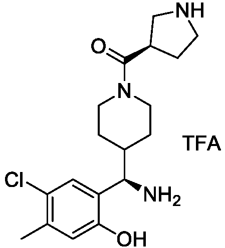 | (4-((*R*)-amino(5-chloro-2-hydroxy-4-methylphenyl)methyl) piperidin-1-yl)((*R*)-pyrrolidin-3-yl)methanone trifluoroacetic acid | [M + H]⁺: 352, 354 (3 : 1); ¹H NMR (400 MHz, CD₃OD) δ 7.24 (d, *J* = 5.3 Hz, 1H), 6.88 (d, *J* = 2.1 Hz, 1H), 4.55 (dd, *J* = 59.2, 13.4 Hz, 1H), 4.24-3.95 (m, 2H), 3.74-3.53 (m, 2H), 3.42-3.35 (m, 3H), 3.26-3.04 (m, 1H), 2.78-2.56 (m, 1H), 2.46-2.33 (m, 2H), 2.31 (s, 3H), 2.11-1.98 (m, 2H), 1.50-1.36 (m, 1H), 1.36-1.04 (m, 2H); ¹⁹F NMR (376 MHz, CD₃OD) δ -77.46. |
| **238** | 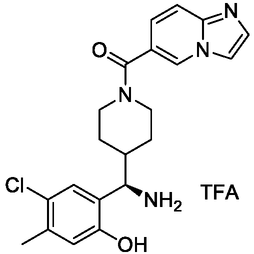 | 2-[(*R*)-amino(1-[imidazo[1,2-*a*]pyridine-6-carbonyl]piperidin-4-yl)methyl]-4-chloro-5-methylphenol trifluoroacetic acid | [M + H]⁺: 399, 401 (3 : 1); ¹H NMR (400 MHz, CD₃OD) δ 8.98 (s, 1H), 8.27 (d, *J* = 2.1 Hz, 1H), 8.13 (d, *J* = 2.1 Hz, 1H), 8.06-7.87 (m, 2H), 7.25 (s, 1H), 6.85 (s, 1H), 4.75-4.46 (m, 1H), 4.25-4.01 (m, 1H), 4.01-3.57 (m, 1H), 3.44-3.35 (m, 1H), 3.16-2.72 (m, 1H), 2.52-2.38 (m, 1H), 2.31 (s, 3H), 2.22-1.73 (m, 1H), 1.64-1.22 (m, 3H); ¹⁹F NMR (376 MHz, CD₃OD) δ -77.25. |
| **242** | 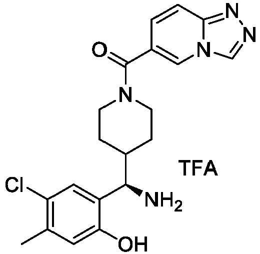 | (*R*)-[1,2,4]triazolo[4,3-a]pyridin-6-yl(4-(amino(5-chloro-2-hydroxy-4-methylphenyl)methyl) piperidin-1-yl)methanone trifluoroacetic acid | [M + H]⁺: 400, 402 (3 : 1); ¹H NMR (400 MHz, CD₃OD) δ 9.28 (s, 1H), 8.74 (s, 1H), 7.88 (d, *J* = 9.3 Hz, 1H), 7.59 (d, *J =* 9.6 Hz, 1H), 7.25 (s, 1H), 6.85 (s, 1H), 4.16 (d*, J* = 9.7 Hz, 1H), 4.01-3.62 (s, 1H), 3.59-3.45 (m, 1H), 3.20-2.75 (m, 2H), 2.62-2.36 (m, 1H), 2.31 (s, 3H), 2.05 (s, 1H), 1.59-1.21 (m, 3H); ¹⁹F NMR (376 MHz, CD₃OD) δ -77.45. |
| **247** | 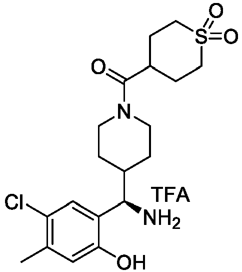 | (*R*)-(4-(amino(5-chloro-2-hydroxy-4-methylphenyl)methyl) piperidin-1-yl)(1,1-dioxidotetrahydro-2*H-*thiopyran-4-yl)methanone trifluoroacetic acid | [M + H]⁺: 415, 417 (3 : 1); ¹H NMR (400 MHz, CD₃OD) δ 7.24 (d, *J* = 4.1 Hz, 1H), 6.85 (s, 1H), 4.58 (dd, *J* = 53.3, 13.5 Hz, 1H), 4.27-3.91 (m, 2H), 3.21-2.99 (m, 6H), 2.79-2.45 (m, 1H), 2.45-2.28 (m, 4H), 2.27-2.10 (m, 5H), 1.48-1.05 (m, 3H); ¹⁹F NMR (376 MHz, CD₃OD) δ - 77.16. |
| **250** | 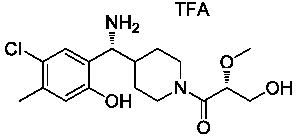 | (*2R*)-1-[4-[(*R*)-amino(5-chloro-2-hydroxy-4-methylphenyl)methyl] piperidin-1-yl]-3-hydroxy-2-methoxypropan-1-one trifluoroacetic acid | [M + H]⁺: 357, 359 (3 : 1); ¹H NMR (400 MHz, CD₃OD) δ 7.25 (s, 1H), 6.85 (s, 1H), 4.72-4.46 (m, 1H), 4.46-4.17 (m, 2H), 4.17-4.01 (m, 1H), 3.80-3.59 (m, 2H), 3.36 (s, 3H), 3.18-2.95 (m, 1H), 2.78-2.54 (m, 1H), 2.48-2.22 (m, 4H), 2.10-1.91 (m, 1H), 1.51-0.97 (m, 3H); ¹⁹F NMR (376 MHz, CD₃OD) δ -77.16. |
| **258** | 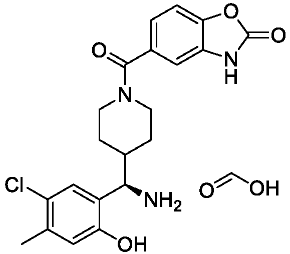 | 5-[4-[(*R*)-amino(5-chloro-2-hydroxy-4-methylphenyl)methyl] piperidine-1-carbonyl]-3*H*-1,3-benzoxazol-2-one formic acid | [M + H]⁺: 416, 418 (3 : 1); ¹H NMR (400 MHz, CD₃OD) δ 8.59 (s, 1H), 7.36-7.22 (m, 2H), 7.21-7.08 (m, 2H), 6.84 (s, 1H), 4.66 (d, *J* = 57.9 Hz, 1H), 4.14 (d, *J* = 9.5 Hz, 1H), 3.82 (d, *J* = 51.6 Hz, 1H), 3.28-2.54 (m, 2H), 2.39-2.31 (m, 1H), 2.30 (s, 3H), 2.17-1.83 (m, 1H), 1.56-1.33 (m, 3H). |
| **265** | 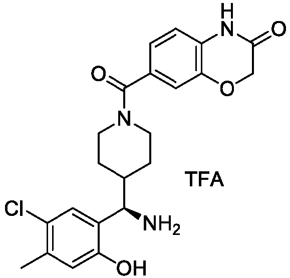 | 7-[4-[(*R*)-amino(5-chloro-2-hydroxy-4-methylphenyl)methyl] piperidine-1-carbonyl]-2,4-dihydro-1,4-benzoxazin-3-one trifluoroacetic acid | [M + H]⁺: 430, 432 (3 : 1); ¹H NMR (400 MHz, CD₃OD) δ 7.25 (s, 1H), 7.08-6.91 (m, 3H), 6.85 (s, 1H), 4.79-4.38 (m, 3H), 4.14 (d, *J* = 9.7 Hz, 1H), 4.07-3.68 (m, 1H), 3.26-2.65 (m, 2H), 2.55-2.32 (m, 1H), 2.32 (s, 3H), 2.09 (s, 1H), 1.63-1.07 (m, 3H); ¹⁹F NMR (376 MHz, CD₃OD) δ -77.05. |
| **271** | 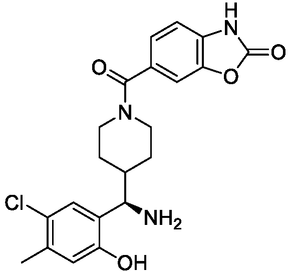 | 6-[4-[(*R*)-amino(5-chloro-2-hydroxy-4-methylphenyl)methyl] piperidine-1-carbonyl]-3*H*-1,3-benzoxazol-2-one | [M + H]⁺: 416, 418 (3 : 1); ¹H NMR (400 MHz, CD₃OD) δ 7.26 (s, 1H), 7.21 (d, *J* = 8.0 Hz, 1H), 7.13 (d, *J* = 7.3 Hz, 2H), 6.71 (s, 1H), 4.71-4.47 (m, 1H), 3.92 (d, *J =* 8.1 Hz, 2H), 3.07-2.78 (m, 2H), 2.27 (s, 3H), 2.14-1.95 (m, 2H), 1.51-1.23 (m, 3H). |
| **324** | 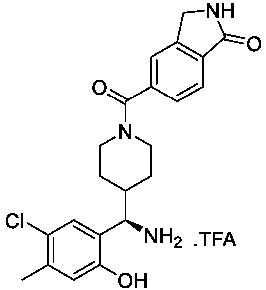 | 5-[4-[(*R*)-amino(5-chloro-2-hydroxy-4-methylphenyl)methyl] piperidine-1-carbonyl]-2,3-dihydroisoindol-1-one trifluoroacetic acid | [M + H]⁺: 414, 416 (3 : 1); ¹H NMR (400 MHz, CD₃O D) δ 7.88 (s, 1H), 7.62 (s, 1 H), 7.54 (s, 1H), 7.25 (s, 1 H), 6.84 (s, 1H), 4.82-4.60 (m, 1H), 4.52 (s, 2H), 4.19-, 4.14 (m, 1H), 3.84 -3.58 (m 1H), 3.24-2.70 (m, 2H), 2. 50-2.22 (m, 4H), 2.19-1.80 (m, 1H), 1.54-1.11 (m, 3H); ¹⁹F NMR (376 MHz, CD₃ OD) δ -77.32. |

### Example 186. (which is a reference example) Compound 191 ((2R)-1-[4-[(R)-amino(2-hydroxynaphthalen-1-yl)methyl]piperidin-1-yl]-2,3-dihydroxypropan-1-one trifluoroacetic acid)

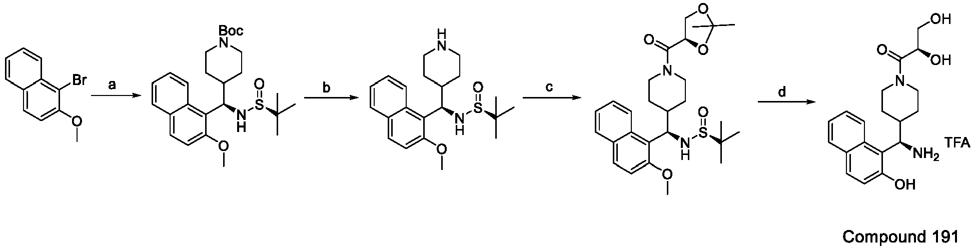

### Step a:

To a stirred mixture of 1-bromo-2-methoxynaphthalene (0.35 g, 1.50 mmol) in THF (5 mL) was added *n*-BuLi (0.71 mL, 1.80 mmol, 2.5 M in hexanes) dropwise at -78 °C under nitrogen atmosphere. The resulting solution was stirred for 30 min at -78 °C under nitrogen atmosphere. To the above solution was added a solution of *tert*-butyl 4-([[(*S*)-2-methylpropane-2-sulfinyl]imino]methyl)piperidine-1-carboxylate (0.35 g, 1.10 mmol) in THF (2 mL) dropwise over 5 min at -78 °C. The resulting solution was allowed to warm to room temperature and stirred for additional 2 h. The reaction was quenched with saturated aq. NH₄Cl (20 mL) at room temperature. The resulting mixture was extracted with EA (3 x 50 mL). The combined organic layers were washed with brine (3 x 20 mL), dried over anhydrous Na₂SO₄. After filtration, the filtrate was concentrated under reduced pressure. The residue was purified by Prep-TLC, eluted with PE/EA (1/1) to afford *tert*-butyl 4-[(*R*)-(2-methoxynaphthalen-1-yl)([[(*S*)-2-methylpropane-2-sulfinyl]amino])methyl]piperidine-1-carboxylate as a light yellow oil (0.30 g, 34%): LCMS (ESI) calc'd for C₂₆H₃₈N₂O₄S [M + H]⁺: 475, found 475; ¹H NMR (400 MHz,CDCl₃) δ 7.97 (d, *J =* 8.7 Hz, 1H), 7.84-7.79 (m, 2H), 7.56-7.48 (m, 1H), 7.42-7.35 (m, 1H), 7.32 (d, *J =* 9.2 Hz, 1H), 5.01 (d, *J =* 10.0 Hz, 1H), 4.30-4.16 (m, 1H), 4.05-3.89 (m, 4H), 2.78-2.63 (m, 1H), 2.52-2.36 (m, 2H), 2.27-2.15 (m, 1H), 1.45 (d, *J =* 3.9 Hz, 12H), 0.97 (s, 9H).

### Step b:

To a stirred solution of *tert*-butyl 4-[(*R*)-(2-methoxynaphthalen-1-yl)([[(*S*)-2-methylpropane-2-sulfinyl]amino])methyl]piperidine-1-carboxylate (0.20 g, 0.42 mmol) in DCM (4 mL) was added TFA (1 mL) at room temperature. The resulting solution was stirred for 30 min at room temperature. The mixture was basified to pH 8 with saturated aq. NaHCO₃ at 0 °C. The resulting mixture was extracted with EA (3 x 30 mL). The combined organic layers were washed with brine (3 x 20 mL), dried over anhydrous Na₂SO₄. After filtration, the filtrate was concentrated under reduced pressure to afford (*S*)-*N*-[(*R*)-(2-methoxynaphthalen-1-yl)(piperidin-4-yl)methyl]-2-methylpropane-2-sulfinamide as a light yellow oil (0.2 g, crude), which was used in the next step directly without further purification: LCMS (ESI) calc'd for C₂₁H₃₀N₂O₂S [M + H]⁺: 375, found 375; ¹H NMR (400 MHz, CD₃OD) δ 8.12-8.04 (m, 1H), 7.93 (d, *J* = 9.0 Hz, 1H), 7.89-7.82 (m, 1H), 7.60-7.53 (m, 1H), 7.53-7.46 (m, 1H), 7.44-7.36 (m, 1H), 5.01 (d, *J =* 9.7 Hz, 1H), 4.07 (s, 3H), 3.55 (d, *J =* 13.3 Hz, 1H), 3.23 (d, *J =* 12.7 Hz, 1H), 3.09-2.98 (m, 1H), 2.88-2.73 (m, 1H), 2.69-2.58 (m, 1H), 2.56-2.29 (m, 1H), 1.70-1.56 (m, 1H), 1.50-1.26 (m, 2H), 0.98 (s, 9H).

### Step c:

To a stirred solution of (4*R*)-2,2-dimethyl-1,3-dioxolane-4-carboxylic acid (78 mg, 0.50 mmol) and HATU (0.30 g, 0.80 mmol) in DMF (2 mL) were added (*S*)-*N-*[(*R*)-(2-methoxynaphthalen-1-yl)(piperidin-4-yl)methyl]-2-methylpropane-2-sulfinamide (0.20 g, 0.50 mmol) and Et₃N (0.16 g, 1.6 mmol) at room temperature. The reaction was stirred at room temperature for 30 min at room temperature. The resulting solution was quenched with water (30 mL) and extracted with EA (2 x 30 mL). The combined organic layers were washed with brine (2 x 10 mL), dried over anhydrous Na₂SO₄. After filtration, the filtrate was concentrated under reduced pressure. The residue was purified by reverse phase chromatography, eluted with 57% ACN in water (plus 0.05% TFA) to afford (*S*)-*N*-[(*R*)-[1-[(4*R*)-2,2-dimethyl-1,3-dioxolane-4-carbonyl]piperidin-4-yl](2-methoxynaphthalen-1-yl)methyl]-2-methylpropane-2-sulfinamide as a light yellow oil (0.16 g, 47 %): LCMS (ESI) calc'd for C₂₇H₃₈N₂O₅S [M + H]⁺: 503, found 503; ¹H NMR (400 MHz, CDCl₃) δ 7.97-7.90 (m, 1H), 7.90-7.80 (m, 3H), 7.59-7.49 (m, 1H), 7.44-7.30 (m, 1H), 4.98-4.83 (m, 1H), 4.82-4.46 (m, 2H), 4.45-4.29 (m, 1H), 4.29-4.19 (m, 1H), 4.10-4.02 (m, 3H), 4.02-3.90 (m, 1H), 3.89-3.49 (m, 1H), 3.20-2.98 (m, 1H), 2.68-2.29 (m, 2H), 1.60-1.22 (m, 9H), 1.01 (d, *J =* 3.6 Hz, 9H).

### Step d:

To a stirred mixture of (*S*)-*N*-[(*R*)-[1-[(4*R*)-2,2-dimethyl-1,3-dioxolane-4-carbonyl]piperidin-4-yl](2-methoxynaphthalen-1-yl)methyl]-2-methylpropane-2-sulfinamide (0.16 g, 0.31 mmol) in DCM (3 mL) was added BBr₃ (0.18 g, 1.90 mmol) at room temperature. The resulting mixture was stirred for 3 h at room temperature. The reaction was quenched with water (5 mL) at 0 °C. The resulting mixture was concentrated under reduced pressure. The residue was purified by Prep-HPLC with the following conditions: Column: XBridge Shield RP18 OBD Column, 5 µm, 19 x 150 mm; Mobile Phase A: Water (plus 0.05% TFA), Mobile Phase B: ACN; Flow rate: 25 mL/min; Gradient: 8% B to 25% B in 7 min; Detector: UV 254/220 nm; Retention time: 4.70 min. The fractions containing the desired product were collected and concentrated under reduced pressure to afford Compound 191 ((2*R*)-1-[4-[(*R*)-amino(2-hydroxynaphthalen-1-yl)methyl]piperidin-1-yl]-2,3-dihydroxypropan-1-one trifluoroacetic acid) as a light yellow solid (38 mg, 25%): LCMS (ESI) calc'd for C₁₉H₂₄N₂O₄ [M + H]⁺: 345, found 345; ¹H NMR (400 MHz, CD₃OD) δ 8.01 (d, *J =* 8.4 Hz, 1H), 7.86 (t, *J =* 7.7 Hz, 2H), 7.56 (t, *J =* 7.7 Hz, 1H), 7.38 (t, *J* = 7.5 Hz, 1H), 7.24 (d, *J =* 8.9 Hz, 1H), 4.89-4.84 (m, 1H), 4.71 (d, *J =* 13.5 Hz, 1H), 4.57-4.39 (m, 1H), 4.13 (dd, *J =* 115.7, 13.8 Hz, 1H), 3.81-3.52 (m, 2H), 3.05 (dt, *J =* 82.6, 13.2 Hz, 1H), 2.86-2.43 (m, 2H), 2.25-2.07 (m, 1H), 1.67-1.40 (m, 1H), 1.40-1.14 (m, 2H); ¹⁹F NMR (376 MHz, CD₃OD) δ -76.97.

### Example 187. Compound 192 (1-[4-[(R)-amino(4,5-dichloro-2-hydroxyphenyl)methyl]piperidin-1-yl]-2-(oxetan-3-yloxy)ethan-1-one)

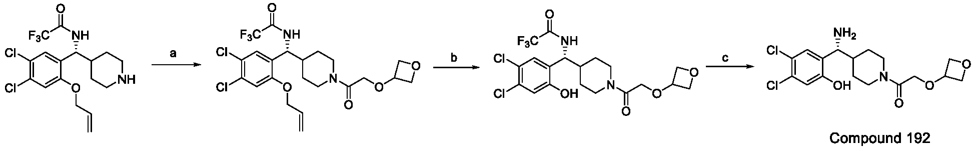

### Step a:

To a stirred solution of lithio 2-(oxetan-3-yloxy)acetate (0.26 g, 1.85 mmol) and HATU (0.70 g, 1.85 mmol) in DMF (4 mL) was added *N*-[(*R*)-[4,5-dichloro-2-(prop-2-en-1-yloxy)phenyl](piperidin-4-yl)methyl]-2,2,2-trifluoroacetamide (Intermediate 38, Example 38) (0.38 g, 0.92 mmol) and Et₃N (0.28 g, 2.77 mmol) at room temperature. The resulting mixture was stirred for 2 h at room temperature. The reaction solution was purified by reverse phase chromatography, eluted with 55% ACN in water (plus 0.05% TFA) to afford *N*-[(*R*)-[4,5-dichloro-2-(prop-2-en-1-yloxy)phenyl]([1-[2-(oxetan-3-yloxy)acetyl]piperidin-4-yl])methyl]-2,2,2-trifluoroacetamide as a light yellow solid (0.20 g, 37% overall two steps): LCMS (ESI) calc'd for C₂₂H₂₅Cl₂F₃N₂O₅ [M + H]⁺: 525, 527 (3 : 2), found 525, 527 (3 : 2).

### Step b:

To a stirred solution of *N*-[(*R*)-[4,5-dichloro-2-(prop-2-en-1-yloxy)phenyl]([1-[2-(oxetan-3-yloxy)acetyl]piperidin-4-yl])methyl]-2,2,2-trifluoroacetamide (0.20 g, 0.38 mmol) and Pd(PPh₃)₄ (4.4 mg, 0.004 mmol) in THF(3 mL) was added NaBH₄ (29 mg, 0.76 mmol) at room temperature. The resulting mixture was stirred for 1 h at room temperature. The reaction was quenched with saturated aq. NH₄Cl (1 mL) at room temperature. The resulting mixture was concentrated under reduced pressure to afford *N*-[(*R*)-(4,5-dichloro-2-hydroxyphenyl)([1-[2-(oxetan-3-yloxy)acetyl]piperidin-4-yl])methyl]-2,2,2-trifluoroacetamide as a brown solid (0.20 g, crude), which was used in the next step directly without further purification: LCMS (ESI) calc'd for C₁₉H₂₁Cl₂F₃N₂O₅ [M + H]⁺: 485, 487 (3 : 2), found 485, 487 (3 : 2);

### Step c:

To a stirred solution of *N*-[(*R*)-(4,5-dichloro-2-hydroxyphenyl)([1-[2-(oxetan-3-yloxy)acetyl]piperidin-4-yl])methyl]-2,2,2-trifluoroacetamide (0.20 g, 0.41 mmol) in MeOH (2 mL) was added KOH (35 mg, 0.62 mmol) at room temperature. The resulting mixture was stirred for 2 h at room temperature. The mixture was acidified to pH 7 with aq. HCl (1 N). The resulting mixture was concentrated under reduced pressure. The residue was purified by Prep-HPLC with the following conditions: Column: XBridge Shield RP18 OBD Column, 5 µm, 19 x 150 mm; Mobile Phase A: water with 10 mmoL/L NH₄HCO₃, Mobile Phase B: ACN; Flow rate: 25 mL/min; Gradient: 24% B to 30% B in 7 min; Detector: UV 254/220 nm; Retention time: 6.35 min. The fractions containing the desired product were collected and concentrated under reduced pressure to afford Compound 192 (1-[4-[(*R*)-amino(4,5-dichloro-2-hydroxyphenyl)methyl]piperidin-1-yl]-2-(oxetan-3-yloxy)ethan-1-one) as an off-white solid (45 mg, 30% overall two steps): LCMS (ESI) calc'd for C₁₇H₂₂Cl₂N₂O₄ [M + H]⁺: 389, 391 (3 : 2), found 389, 391 (3 : 2); ¹H NMR (400 MHz, CD₃OD) δ 7.23 (d, *J* = 2.8 Hz, 1H), 6.87 (d, *J =* 2.0 Hz, 1H), 4.81-4.70 (m, 2H), 4.70-4.41 (m, 4H), 4.30-4.12 (m, 2H), 3.92-3.72 (m, 2H), 3.10-2.91 (m, 1H), 2.71-2.43 (m, 1H), 2.12-1.92 (m, 2H), 1.48 (d, *J =* 13.3 Hz, 1H), 1.42-1.05 (m, 2H).

### Example 188. Compound 196 ((2R)-1-[4-[(4,5-dichloro-2-hydroxyphenyl)(methylamino)methyl]piperidin-1-yl]-2,3-dihydroxypropan-1-one trifluoroacetic acid)

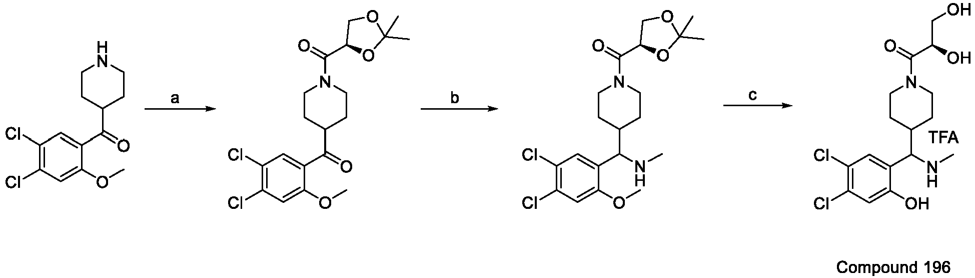

### Step a:

To a stirred solution of (4*R*)-2,2-dimethyl-1,3-dioxolane-4-carboxylic acid (0.61 g, 4.17 mmol) in DMF (10 mL) were added EDCI (1.00 g, 5.22 mmol) and HOBt (0.70 g, 5.18 mmol) at room temperature. To the above mixture were added 4-(4,5-dichloro-2-methoxybenzoyl)piperidine (Example 3, step a) (1.00 g, 3.47 mmol) and Et₃N (1.05 g, 10.37 mmol) at room temperature. The reaction mixture was stirred for 12 h at room temperature. The reaction mixture was diluted with H₂O (80 mL). The resulting mixture was extracted with EA (3 x 40 mL). The combined organic layers were washed with brine (2 x 20 mL), dried over anhydrous Na₂SO₄. After filtration, the filtrate was concentrated under reduced pressure. The residue was purified by reverse phase chromatography, eluted with 30% ACN in water with 10 mmol/L NH₄HCO₃ to afford 4-(4,5-dichloro-2-methoxybenzoyl)-1-[(4*R*)-2,2-dimethyl-1,3-dioxolane-4-carbonyl]piperidine as a yellow oil (0.68 g, 34%): LCMS (ESI) calc'd for C₁₉H₂₃Cl₂NO₅ [M + H]⁺: 416, 418 (3 : 2), found 416, 418 (3 : 2);

### Step b:

To a stirred mixture of 4-(4,5-dichloro-2-methoxybenzoyl)-1-[(4*R*)-2,2-dimethyl-1,3-dioxolane-4-carbonyl]piperidine (0.46 g, 1.10 mmol) and methanamine hydrochloride (0.37 g, 5.52 mmol) in THF (20 mL) was added Ti(OEt)₄ (1.00 g, 5.52 mmol) at room temperature under nitrogen atmosphere. The resulting mixture was stirred for 12 h at 70 °C under nitrogen atmosphere. After cooling to room temperature, NaBH₄ (8 mg, 0.22 mmol) was added to the above mixture at room temperature under nitrogen atmosphere. The resulting mixture was stirred for 1 h at room temperature under nitrogen atmosphere. The reaction was quenched with H₂O (50 mL) at room temperature. The resulting mixture was extracted with EA (3 x 40 mL). The combined organic layers were washed with brine (2 x 10 mL), dried over anhydrous Na₂SO₄. After filtration, the filtrate was concentrated under reduced pressure to afford [(4,5-dichloro-2-methoxyphenyl)([1-[(4*R*)-2,2-dimethyl-1,3-dioxolane-4-carbonyl]piperidin-4-yl])methyl](methyl)amine as a yellow solid (0.39 g, crude): LCMS (ESI) calc'd for C₂₀H₂₈Cl₂N₂O₄ [M + H]⁺: 431, 433 (3 : 2), found 431, 433 (3 : 2).

### Step c:

To a stirred solution of [(4,5-dichloro-2-methoxyphenyl)([1-[(4*R*)-2,2-dimethyl-1,3-dioxolane-4-carbonyl]piperidin-4-yl])methyl](methyl)amine (0.39 g, 0.90 mmol) in DCM (4 mL) was added BBr₃ (1 mL) dropwise at 0 °C. The reaction mixture was stirred for 1 h at room temperature. The reaction was quenched with MeOH (2 mL). The mixture was concentrated under reduced pressure. The residue was purified with Prep-HPLC with the following conditions: Column: XBridge Shield RP18 OBD Column 19 x 150 mm, 5 µm; Mobile Phase A: water (plus 0.05% TFA), Mobile Phase B: ACN; Flow rate: 25 mL/min; Gradient: 5% B to 30% B in 7 min; Detector: UV 254/220 nm; Retention time: 5.92 min. The fractions containing the desired product were collected and concentrated under reduced pressure to afford Compound 196 ((2*R*)-1-[4-[(4,5-dichloro-2-hydroxyphenyl)(methylamino)methyl]piperidin-1-yl]-2,3-dihydroxypropan-1-one trifluoroacetic acid) as an off-white solid (0.14 g, 30%): LCMS (ESI) calc'd for C₁₆H₂₂Cl₂N₂O₄ [M + H]⁺: 377, 379 (3 : 2), found 377, 379 (3 : 2); ¹H NMR (400 MHz, CD₃OD) δ 7.44 (s, 1H), 7.11 (s, 1H), 4.71-4.31 (m, 2H), 4.31-4.03 (m, 2H), 3.85-3.51 (m, 2H), 3.25-2.98 (m, 1H), 2.84-2.36 (m, 5H), 2.05 (s, 1H), 1.54-1.01 (m, 3H); ¹⁹F NMR (376 MHz, CD₃OD) δ -77.19.

### Example 189. Compound 193 ((2R)-1-[4-[(4,5-dichloro-2-hydroxyphenyl)(dimethylamino)methyl]piperidin-1-yl]-2,3-dihydroxypropan-1-one trifluoroacetic acid)

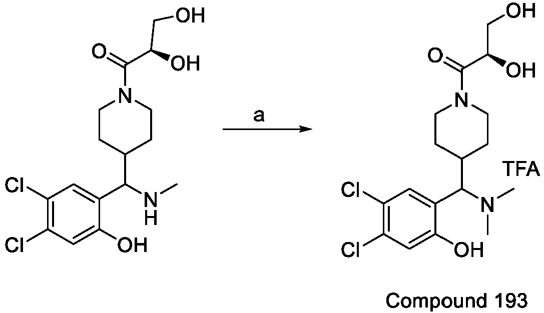

### Step a:

To a stirred mixture of (2*R*)-1-[4-[(4,5-dichloro-2-hydroxyphenyl)(methylamino)methyl]piperidin-1-yl]-2,3-dihydroxypropan-1-one (Compound 196's free base, Example 188) (50 mg, 0.13 mmol), HOAc (35 mg, 0.42 mmol) and HCHO (6 mg, 0.22 mmol) in MeOH (1 mL) was added NaBH₃CN (27 mg, 0.42 mmol) in portions at room temperature. The resulting mixture was stirred for 3 h at room temperature under nitrogen atmosphere. The resulting mixture was quenched with water (5 mL) at room temperature and concentrated under reduced pressure. The residue was purified with Prep-HPLC with the following conditions: Column: XBridge Prep C18 OBD Column 19 x 150 mm, 5 µm; Mobile Phase A: water (plus 0.05% TFA), Mobile Phase B: ACN; Flow rate: 25 mL/min; Gradient: 5 % B to 30% B in 7 min; Detector: UV 254/220 nm; Retention time: 5.85 min. The fractions containing the desired product were collected and concentrated under reduced pressure to afford Compound 193 ((2*R*)-1-[4-[(4,5-dichloro-2-hydroxyphenyl)(dimethylamino)methyl]piperidin-1-yl]-2,3-dihydroxypropan-1-one trifluoroacetic acid) as an off-white solid (29 mg, 43%): LCMS (ESI) calc'd for C₁₇H₂₄Cl₂N₂O₄ [M + H]⁺: 391, 393 (3 : 2), found 391, 393 (3 : 2); ¹H NMR (400 MHz, CD₃OD) δ 7.56 (s, 1H), 7.15 (s, 1H), 4.82-4.36 (m, 3H), 4.33-4.06 (m, 1H), 3.78-3.53 (m, 2H), 3.28-3.03 (m, 1H), 2.98-2.60 (m, 8H), 2.11-1.80 (m, 1H), 1.54 (s, 1H), 1.40-1.01 (m, 2H); ¹⁹F NMR (376 MHz, CD₃OD) δ -76.67.

### Example 190. Compound 198 ((2R)-1-[4-[(R)-amino(4-chloro-2-hydroxy-5-methylphenyl)methyl]piperidin-1-yl]-2,3-dihydroxypropan-1-one)

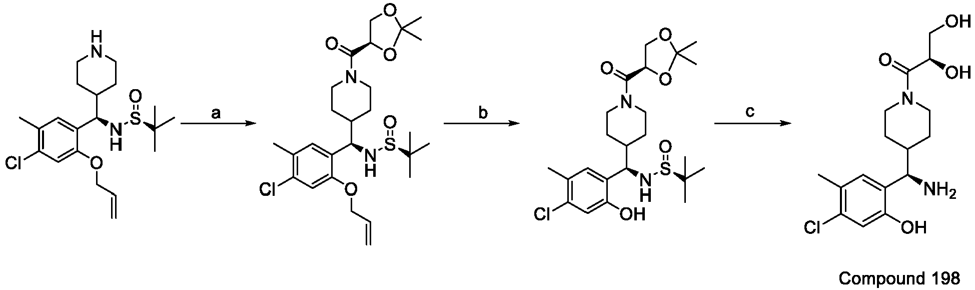

### Step a:

To a stirred solution of (4*R*)-2,2-dimethyl-1,3-dioxolane-4-carboxylic acid (50 mg, 0.34 mmol) and HATU (151 mg, 0.40 mmol) in DMF (2 mL) were added Et₃N (53 mg, 0.53 mmol) and *N*-[(*R*)-[4-chloro-5-methyl-2-(prop-2-en-1-yloxy)phenyl] (piperidin-4-yl)methyl]-2,2-dimethylpropanamide (0.10 g, 0.26 mmol) at room temperature under nitrogen atmosphere. The resulting mixture was stirred for 12 h at room temperature under nitrogen atmosphere. The reaction was quenched with water (20 mL) and extracted with EA (3 x 10 mL). The combined organic layers were washed with brine (3 x 10 mL), dried over anhydrous Na₂SO₄. After filtration, the filtrate was concentrated under reduced pressure to afford *N*-[(*R*)-[4-chloro-5-methyl-2-(prop-2-en-1-yloxy)phenyl]([1-[(4*R*)-2,2-dimethyl-1,3-dioxolane-4-carbonyl]piperidin-4-yl])methyl]-2,2-dimethylpropanamide as a light yellow oil (180 mg, crude): LCMS (ESI) calc'd for C₂₆H₃₉ClN₂O₅S [M + H]⁺: 527, 529 (3 : 1), found 527, 529 (3 : 1).

### Step b:

To a stirred solution of *N*-[(*R*)-[4-chloro-5-methyl-2-(prop-2-en-1-yloxy)phenyl]([1-[(4*R*)-2,2-dimethyl-1,3-dioxolane-4-carbonyl]piperidin-4-yl])methyl]-2,2-dimethylpropanamide (180 mg, crude) and Pd(PPh₃)₄ (15 mg, 0.01 mmol) in THF (5 mL) was added NaBH₄ (27 mg, 0.71 mmol) in three portions under nitrogen atmosphere. The resulting solution was stirred for 1 h at room temperature under nitrogen atmosphere. The resulting mixture was quenched with water (3 mL) and concentrated under reduced pressure to afford (*S*)-*N*-((*R*)-(4-chloro-2-hydroxy-5-methylphenyl)(1-((*R*)-2,2-dimethyl-1,3-dioxolane-4-carbonyl)piperidin-4-yl)methyl)-2-methylpropane-2-sulfinamide as a brown oil (0.12 g, crude), which was used in next step without further purification: LCMS (ESI) calc'd for C₂₆H₃₉ClN₂O₅S [M + H]⁺: 527, 529 (3 : 1), found 527, 529 (3 : 1).

### Step c:

To a stirred solution of (*S*)-*N*-((*R*)-(4-chloro-2-hydroxy-5-methylphenyl)(1-((*R*)-2,2-dimethyl-1,3-dioxolane-4-carbonyl)piperidin-4-yl)methyl)-2-methylpropane-2-sulfinamide (0.12 g, crude) in THF (2 mL) was added aq. HCl (4 *N,* 1 mL) at room temperature. The resulting mixture was stirred for additional 30 min at room temperature. The resulting mixture was concentrated under reduced pressure. The residue was purified by prep-HPLC with the following conditions: Column: XBridge Shield RP18 OBD Column, 5 µm, 19 x 150 mm; Mobile Phase A: Water with 10 mmol/L NH₄HCO₃, Mobile Phase B: ACN; Flow rate: 25 mL/min; Gradient: 11% B to 26% B in 9 min; Detector: UV 220 nm; Retention time: 6.1 min. The fractions containing the desired product were collected and concentrated under reduced pressure to afford Compound 198 ((2*R*)-1-[4-[(*R*)-amino(4-chloro-2-hydroxy-5-methylphenyl)methyl]piperidin-1-yl]-2,3-dihydroxypropan-1-one) as an off-white solid (26.1 mg, 31% overall three steps): LCMS (ESI) calc'd for C₁₆H₂₃ClN₂O₄ [M + H]⁺: 343, 345 (3 : 1), found 343, 345 (3 : 1); ¹H NMR (400 MHz, CD₃OD) δ 6.98 (s, 1H), 6.76 (s, 1H), 4.67-4.40 (m, 2H), 4.10 (dd, *J =* 48.1, 13.7 Hz, 1H), 3.85 (dd, *J =* 7.9, 3.6 Hz, 1H), 3.76-3.53 (m, 2H), 3.10-2.96 (m, 1H), 2.73-2.50 (m, 1H), 2.25 (s, 3H), 2.02 (s, 2H), 1.53-1.40 (m, 1H), 1.33-1.14 (m, 2H).

### Example 191. Compound 201 (1-[4-[(R)-amino(4,5-dichloro-2-hydroxyphenyl)methyl]piperidin-1-yl]-2,3-dihydroxy-2-methylpropan-1-one trifluoroacetic acid)

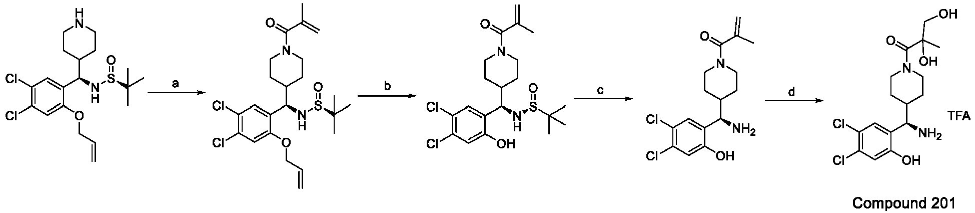

### Step a:

To a stirred solution of 2-methylprop-2-enoic acid (92 mg, 1.07 mmol) and HATU (0.41 g, 1.07 mmol) in DMF (3 mL) were added (*S*)-*N*-[(*R*)-[4,5-dichloro-2-(prop-2-en-1-yloxy)phenyl](piperidin-4-yl)methyl]-2-methylpropane-2-sulfinamide (0.30 g, 0.72 mmol) and Et₃N (0.14 g, 1.43 mmol) at room temperature. The reaction solution was stirred at room temperature for 1 h. The resulting solution was quenched with water (40 mL) and extracted with EA (3 x 30 mL). The combined organic layers were washed with brine (2 x 20 mL), dried over anhydrous Na₂SO₄. After filtration, the filtrate was concentrated under reduced pressure. The residue was purified by reverse phase chromatography, eluted with 55% ACN in water (plus 0.05% TFA) to afford (*S*)-*N*-[(*R*)-[4,5-dichloro-2-(prop-2-en-1-yloxy)phenyl][1-(2-methylprop-2-enoyl)piperidin-4-yl]methyl]-2-methylpropane-2-sulfinamide as an off-white semi-solid (0.30 g, 86%): LCMS (ESI) calc'd for C₂₃H₃₂Cl₂N₂O₃S [M + H]⁺: 487, 489 (3 : 2), found 487, 489 (3 : 2); ¹H NMR (400 MHz, CDCl₃) δ 7.29 (s, 1H), 6.99 (s, 1H), 6.11-5.97 (m, 1H), 5.48-5.32 (m, 2H), 5.21 (s, 1H), 5.05 (s, 1H), 4.72-4.24 (m, 3H), 4.18-3.92 (m, 1H), 3.10-2.87 (m, 1H), 2.72-2.49 (m, 1H), 2.26-2.01 (m, 3H), 1.95 (s, 3H), 1.52-1.39 (m, 3H), 1.25-1.12 (m, 9H).

### Step b:

To a stirred solution of (*S*)-*N*-[(*R*)-[4,5-dichloro-2-(prop-2-en-1-yloxy)phenyl][1-(2-methylprop-2-enoyl)piperidin-4-yl]methyl]-2-methylpropane-2-sulfinamide (0.30 g, 0.62 mmol) and Pd(PPh₃)₄ (7 mg, 0.01 mmol) in THF (2 mL) was added NaBH₄ (47 mg, 1.23 mmol) at room temperature in nitrogen atmosphere. The reaction mixture was stirred at room temperature for 1 h. The resulting mixture was quenched with saturated aq. NH₄Cl (1 mL) at room temperature and concentrated under reduced pressure. The residue was purified by reverse phase chromatography, eluted with 60% ACN in water (plus 0.05% TFA) to afford (*S*)-*N-*[(*R*)-(4,5-dichloro-2-hydroxyphenyl)[1-(2-methylprop-2-enoyl)piperidin-4-yl]methyl]-2-methylpropane-2-sulfinamide as a brown solid (0.24 g, 87%): LCMS (ESI) calc'd for C₂₀H₂₈Cl₂N₂O₃S [M + H]⁺ 447, 449 (3 : 2), found 447, 449 (3 : 2); ¹H NMR (400 MHz, CD₃OD) δ 7.36 (s, 1H), 6.95 (s, 1H), 5.22 (s, 1H), 5.04 (s, 1H), 4.64-4.28 (m, 2H), 4.16-3.88 (m, 2H), 3.18-2.95 (m, 1H), 2.78-2.58 (m, 1H), 2.21 (d, *J =* 13.7 Hz, 1H), 2.15-2.06 (m, 1H), 1.94 (s, 3H), 1.41 (d, *J =* 13.4 Hz, 1H), 1.33-1.19 (m, 1H), 1.14 (s, 9H).

### Step c:

To a stirred solution of (*S*)-*N*-[(*R*)-(4,5-dichloro-2-hydroxyphenyl)[1-(2-methylprop-2-enoyl)piperidin-4-yl]methyl]-2-methylpropane-2-sulfinamide (0.20 g, 0.45 mmol) in 1,4-dioxane (2 mL) was added aq. HCl (4 *N,* 2 mL) at room temperature. The reaction was stirred at room temperature for 1 h. The reaction solution was concentrated under reduced pressure to afford 1-[4-[(*R*)-amino(4,5-dichloro-2-hydroxyphenyl)methyl]piperidin-1-yl] -2-methylprop-2-en-1-one as a light yellow oil (0.15 g, crude), which was used in the next step directly without further purification: LCMS (ESI) calc'd for C₁₆H₂₀Cl₂N₂O₂ [M + H]⁺: 343, 345 (3 : 2), found 343, 345 (3 : 2); ¹H NMR (400 MHz, CD₃OD) δ 7.46 (s, 1H), 7.09 (s, 1H), 5.25 (s, 1H), 5.07 (s, 1H), 4.72-4.40 (m, 1H), 4.23-3.94 (m, 2H), 3.25-2.98 (m, 1H), 2.88-2.52 (m, 1H), 2.47-2.34 (m, 1H), 2.11-1.97 (m, 1H), 1.95 (s, 3H), 1.47-1.28 (m, 3H).

### Step d:

To a stirred mixture of (*S*)-*N*-[(*R*)-(4,5-dichloro-2-hydroxyphenyl)[1-(2-methylprop-2-enoyl)piperidin-4-yl]methyl]-2-methylpropane-2-sulfinamide (0.15 g, 0.34 mmol) and citric acid (48 mg, 0.25 mmol) in *t*-BuOH (1 mL) and water (1 mL) were added K₂OsO₂(OH)₄ (31 mg, 0.08 mmol) and NMO (47 mg, 0.40 mmol) at room temperature. The reaction mixture was stirred at room temperature for 1 h. The resulting solution was purified by reverse phase chromatography, eluted with 20% ACN in water (plus 0.05% TFA). The fractions containing desired product was concentrated under reduced pressure. The residue was purified by Prep-HPLC with following conditions: Column: XBridge Shield RP18 OBD Column, 5 µm, 19 x 150 mm; Mobile Phase A: water (plus 0.05% TFA), Mobile Phase B: ACN; Flow rate: 25 mL/min; Gradient: 12% B to 23% B in 7 min; Detector: UV 254/220 nm; Retention time 6.38 min. The fractions containing desired product were collected and concentrated under reduced pressure to afford Compound 201 (1-[4-[(*R*)-amino(4,5-dichloro-2-hydroxyphenyl)methyl]piperidin-1-yl]-2,3-dihydroxy-2-methylpropan-1-one trifluoroacetic acid) as an off-white solid (55 mg, 25% overall two steps): LCMS (ESI) calc'd for C₁₆H₂₂Cl₂N₂O₄ [M + H]⁺ 377, 379 (3 : 2), found 377, 379 (3 : 2); ¹H NMR (400 MHz, CD₃OD) δ 7.43 (s, 1H), 7.08 (s, 1H), 4.75-4.50 (m, 2H), 4.15 (d, *J* = 9.6 Hz, 1H), 3.72 (d, *J =* 11.2 Hz, 1H), 3.56 (d, *J =* 11.2 Hz, 1H), 3.07-2.54 (m, 2H), 2.49-2.31 (m, 1H), 2.11-1.91 (m, 1H), 1.51-1.09 (m, 6H); ¹⁹F NMR (376 MHz, CD₃OD) δ - 77.02.

### Example 192. Compound 202 ((2R)-1-[4-[(4,5-dichloro-2-hydroxyphenyl)(ethylamino)methyl]piperidin-1-yl]-2,3-dihydroxypropan-1-one trifluoroacetic acid)

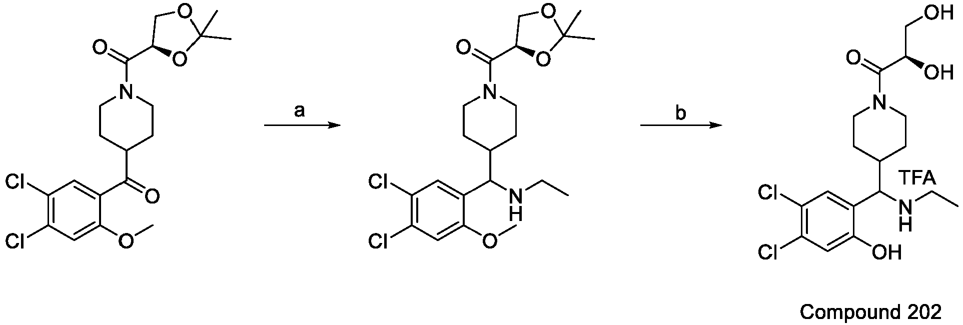

### Step a:

To a stirred mixture of 4-(4,5-dichloro-2-methoxybenzoyl)-1-[(4*R*)-2,2-dimethyl-1,3-dioxolane-4-carbonyl]piperidine (Example 188, step a) (0.22 g, 0.53 mmol) and ethanamine hydrochloride (0.22 g, 2.64 mmol) in THF (10 mL) was added Ti(OEt)₄ (0.60 g, 2.64 mmol) at room temperature. The reaction mixture was stirred for 12 h at 70 °C under nitrogen atmosphere. After cooling to room temperature, NaBH₄ (34 mg, 0.11 mmol) was added to the above mixture at room temperature. The reaction mixture was stirred for 1 h at room temperature. The reaction was quenched with H₂O (40 mL) at room temperature. The resulting mixture was extracted with EA (3 x 30 mL). The combined organic layers were washed with brine (2 x 20 mL), dried over anhydrous Na₂SO₄. After filtration, the filtrate was concentrated under reduced pressure to afford [(4,5-dichloro-2-methoxyphenyl)([1-[(4*R*)-2,2-dimethyl-1,3-dioxolane-4-carbonyl]piperidin-4-yl])methyl](ethyl)amine as a yellow solid (0.23 g, crude): LCMS (ESI) calc'd for C₂₁H₃₀Cl₂N₂O₄ [M + H]⁺: 445, 447 (3 : 2), found 445, 447 (3 : 2).

### Step b:

To a stirred solution of [(4,5-dichloro-2-methoxyphenyl)([1-[(4*R*)-2,2-dimethyl-1,3-dioxolane-4-carbonyl]piperidin-4-yl])methyl](ethyl)amine (0.23 g, 0.52 mmol) in DCM (2 mL) was added BBr₃ (0.5 mL) dropwise at 0 °C. The reaction mixture was stirred for 1 h at room temperature. The reaction was quenched with MeOH (2 mL) at room temperature. The resulting mixture was concentrated under reduced pressure. The residue was purified with Prep-HPLC with the following conditions: Column: XBridge Shield RP18 OBD Column 19 x 150 mm, 5 µm; Mobile Phase A: water (plus 0.05% TFA), Mobile Phase B: ACN; Flow rate: 25 mL/min; Gradient: 10% B to 30% B in 7 min; Detector: UV 254/220 nm; Retention time: 5.57 min. The fractions containing the desired product were collected and concentrated under reduced pressure to afford Compound 202 ((2*R*)-1-[4-[(4,5-dichloro-2-hydroxyphenyl)(ethylamino)methyl]piperidin-1-yl]-2,3-dihydroxypropan-1-one trifluoroacetic acid) as an off-white solid (56 mg, 21%): LCMS (ESI) calc'd for C₁₇H₂₄Cl₂N₂O₄ [M + H]⁺: 391, 393 (3 : 2), found 391, 393 (3 : 2); ¹H NMR (400 MHz, CD₃OD) δ 7.45 (s, 1H), 7.11 (s, 1H), 4.73-4.42 (m, 2H), 4.31-4.02 (m, 2H), 3.66 (d, *J* = 19.9 Hz, 2H), 3.08 (dt, *J* = 45.1, 12.8 Hz, 1H), 2.98-2.80 (m, 2H), 2.66 (dt, *J =* 44.7, 12.8 Hz, 1H), 2.56-2.23 (m, 1H), 2.18-1.95 (m, 1H), 1.54-1.07 (m, 6H); ¹⁹F NMR (376 MHz, CD₃OD) δ -77.24.

### Example 193. Compound 212 ((2R)-1-[(1R,5S,6R)-6-[amino(4,5-dichloro-2-hydroxyphenyl)methyl]-3-azabicyclo[3.1.0]hexan-3-yl]-2,3-dihydroxypropan-1-one)

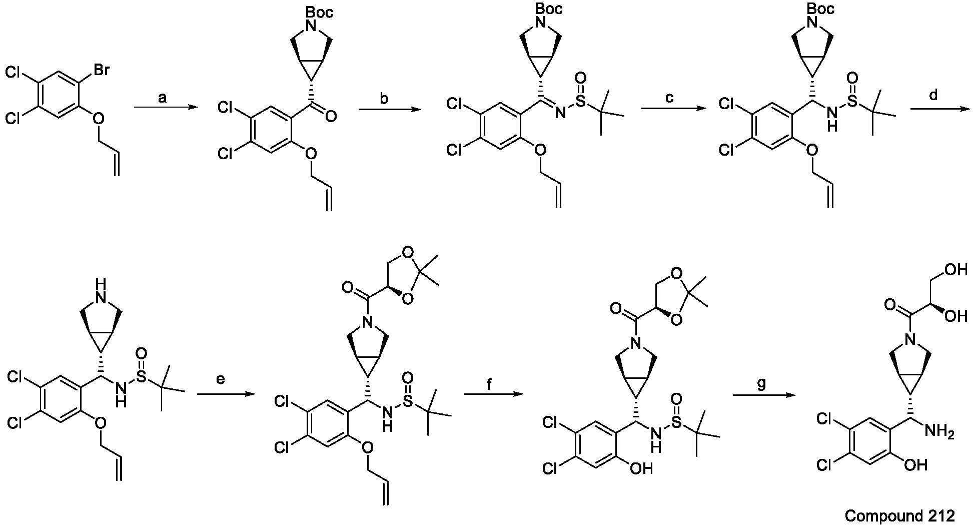

### Step a:

To a stirred solution of 1-bromo-4,5-dichloro-2-(prop-2-en-1-yloxy)benzene (Example 6, step b) (1.12 g, 4.14 mmol) in THF (5 mL) was added *i*-PrMgCl·LiCl (3.98 mL, 5.18 mmol, 1.3 M in THF) dropwise at -10 °C under nitrogen atmosphere. The reaction was stirred at 0 °C for 30 min. Then a solution of *tert-*butyl (1*R*,5*S*,6*R*)-6-[methoxy(methyl)carbamoyl]-3-azabicyclo[*3.1.0*]hexane-3-carboxylate (0.28 g, 1.04 mmol) in THF (2 mL) was added dropwise into over 0.5 h at -10 °C. The reaction was stirred at -10 °C for 2 h. The reaction was quenched with saturated aq. NH₄Cl (30 mL) and extracted with EA (3 x 20 mL). The combined organic layers were washed with brine (2 x 20 mL) and dried over anhydrous Na₂SO₄. After filtration, the filtrate was concentrated under reduced pressure. The residue was purified by Prep-TLC, eluted with PE/EA (5/1) to afford *tert-*butyl (1*R*,5*S*,6*R*)-6-[4,5-dichloro-2-(prop-2-en-1-yloxy)benzoyl]-3-azabicyclo[*3.1.0*]hexane-3-carboxylate as a light yellow oil (0.19 g, 40%): LCMS (ESI) calc'd for C₂₀H₂₃Cl₂NO₄ [M + H]⁺: 412, 414 (3 : 2), found 412, 414 (3 : 2); ¹H NMR (400 MHz, CDCl₃) δ 7.68 (s, 1H), 7.09 (s, 1H), 6.14-5.96 (m, 1H), 5.56-5.35 (m, 2H), 4.66-4.57 (m, 2H), 3.75-3.54 (m, 3H), 3.54-3.39 (m, 2H), 2.32-2.27 (m, 2H), 1.47 (d, *J* = 2.6 Hz, 9H).

### Step b:

To a stirred solution of *tert*-butyl (1*R*,5*S*,6*R*)-6-[4,5-dichloro-2-(prop-2-en-1-yloxy)benzoyl]-3-azabicyclo[*3.1.0*]hexane-3-carboxylate (0.19 g, 0.46 mmol) and *tert-*butanesulfinamide (84 mg, 0.69 mmol) in THF (5 mL) was added Ti(OEt)₄ (0.42 g, 1.84 mmol) at room temperature under nitrogen atmosphere. The reaction was stirred at 70 °C for 16 h. After cooling to room temperature, the reaction was quenched with saturated aq. Na₂CO₃ (30 mL). The resulting mixture was filtered through celite and the filtrate was diluted with EA (30 mL) and water (30 mL). The aqueous layer was extracted with EA (3 x 30 mL). The combined organic layers were washed with brine (3 x 20 mL), dried over anhydrous Na₂SO₄. After filtration, the filtrate was concentrated under reduced pressure to afford *tert-*butyl (1*R*,5*S*,6*R*)-6-[[4,5-dichloro-2-(prop-2-en-1-yloxy)phenyl][(2-methylpropane-2-sulfinyl)imino]methyl]-3-azabicyclo[*3.1.0*]hexane-3-carboxylate as a yellow oil (0.20 g, crude), which was use in the next step directly without further purification: LCMS (ESI) calc'd for C₂₄H₃₂Cl₂N₂O₄S [M + H]⁺: 515, 517 (3 : 2), found 515, 517 (3 : 2).

### Step c:

To a stirred solution of *tert*-butyl (1*R*,5*S*,6*R*)-6-[[4,5-dichloro-2-(prop-2-en-1-yloxy)phenyl][(2-methylpropane-2-sulfinyl)imino]methyl]-3-azabicyclo[*3*.*1*.*0*]hexane-3-carboxylate (0.20 g, 0.39 mmol) in THF (5 mL) was added NaBH₄ (29 mg, 0.78 mmol) at room temperature. The reaction was stirred at room temperature for 1 h. The reaction was quenched with saturated aq. NH₄Cl (2 mL), and diluted with EA (20 mL) and water (30 mL). The aqueous layer was extracted with EA (3 x 20 mL). The combined organic layers were washed with brine (2 x 20 mL) and dried over anhydrous Na₂SO₄. After filtration, the filtrate was concentrated under reduced pressure to afford *tert*-butyl (1*R*,5*S*,6*R*)-6-[[4,5-dichloro-2-(prop-2-en-1-yloxy)phenyl][(2-methylpropane-2-sulfinyl)amino]methyl]-3-azabicyclo[*3.1.0*]hexane-3-carboxylate as a light yellow oil (0.18 g, 90%), which was use in the next step directly without further purification: LCMS (ESI) calc'd for C₂₄H₃₄Cl₂N₂O₄S [M + H]⁺: 517, 519 (3 : 2), found 517, 519 (3 : 2).

### Step d:

To a stirred solution of *tert*-butyl (1*R*,5*S*,6*R*)-6-[[4,5-dichloro-2-(prop-2-en-1-yloxy)phenyl][(2-methylpropane-2-sulfinyl)amino]methyl]-3-azabicyclo[*3.1.0*]hexane-3-carboxylate (0.18 g, 0.35 mmol) in DCM (5 mL) was added TFA (1 mL) at room temperature. The reaction was stirred at room temperature for 0.5 h. The reaction was quenched with saturated aq. Na₂CO₃ (20 mL) and neutralized to pH 7 with saturated aq. Na₂CO₃ (30 mL). The resulting solution was extracted with EA (3 x 30 mL). The combined organic layers were washed with brine (3 x 20 mL), dried over anhydrous Na₂SO₄. After filtration, the filtrate was concentrated under reduced pressure to afford *N*-[[(1*R*,5*S*,6*R*)-3-azabicyclo[*3.1.0*]hexan-6-yl][4,5-dichloro-2-(prop-2-en-1-yloxy)phenyl]methyl]-2-methylpropane-2-sulfinamide as a yellow oil (0.18 g, crude), which was used in the next step directly without further purification: LCMS (ESI) calc'd for C₁₉H₂₆Cl₂N₂O₂S [M + H]⁺: 417, 419, (3 : 2), found 417, 419, (3 : 2).

### Step e:

To a stirred solution of (*4R*)-2,2-dimethyl-1,3-dioxolane-4-carboxylic acid (0.13 g, 0.86 mmol) and HATU (0.33 g, 0.86 mmol) in DMF (2 mL) were added *N*-[[(1*R*,5*S*,6*R*)-3-azabicyclo[*3.1.0*]hexan-6-yl][4,5-dichloro-2-(prop-2-en-1-yloxy)phenyl]methyl]-2-methylpropane-2-sulfinamide (0.18 g, 0.43 mmol) and Et₃N (0.13 g, 1.29 mmol) at room temperature. The reaction was stirred at room temperature for 1 h. The resulting solution was quenched with water (50 mL) and extracted with EA (3 x 40 mL). The combined organic layers were washed with brine (2 x 30 mL), dried over anhydrous Na₂SO₄. After filtration, the filtrate was concentrated under reduced pressure. The residue was purified by reverse phase chromatography, eluted with 56% ACN in water (plus 0.05% TFA) to afford *N*-[[4,5-dichloro-2-(prop-2-en-1-yloxy)phenyl][(1*R*,5*S*,6*R*)-3-[(4*R*)-2,2-dimethyl-1,3-dioxolane-4-carbonyl]-3-azabicyclo[*3.1.0*]hexan-6-yl]methyl]-2-methylpropane-2-sulfinamide as a yellow oil (0.14 g, 56% overall four steps): LCMS (ESI) calc'd for C₂₅H₃₄Cl₂N₂O₅S [M + H]⁺: 545, 547, (3 : 2), found 545, 547 (3 : 2).

### Step f:

To a stirred solution of *N*-[[4,5-dichloro-2-(prop-2-en-1-yloxy)phenyl][(1*R*,5*S*,6*R*)-3-[(4*R*)-2,2-dimethyl-1,3-dioxolane-4-carbonyl]-3-azabicyclo[*3.1.0*]hexan-6-yl]methyl]-2-methylpropane-2-sulfinamide (0.14 g, 0.26 mmol) and Pd(PPh₃)₄ (3 mg, 0.003 mmol) in THF (2 mL) was added NaBH₄ (19 mg, 0.51 mmol) at room temperature under nitrogen atmosphere. The reaction was stirred at room temperature for 30 min under nitrogen atmosphere. The reaction was quenched with saturated aq. NH₄Cl (1 mL) and concentrated under reduced pressure to afford *N*-[(4,5-dichloro-2-hydroxyphenyl)[(1*R*,5*S*,6*R*)-3-[(4*R*)-2,2-dimethyl-1,3-dioxolane-4-carbonyl]-3-azabicyclo[*3.1.0*]hexan-6-yl]methyl]-2-methylpropane-2-sulfinamide as a brown oil (0.20 g, crude), which was used in the next step directly without further purification: LCMS (ESI) calc'd for C₂₂H₃₀Cl₂N₂O₅S [M + H]⁺ 505, 507 (3 : 2), found 505, 507 (3 : 2).

### Step g:

To a stirred solution of *N*-[(4,5-dichloro-2-hydroxyphenyl)[(1*R*,5*S*,6*R*)-3-[(4*R*)-2,2-dimethyl-1,3-dioxolane-4-carbonyl]-3-azabicyclo[*3.1.0*]hexan-6-yl]methyl]-2-methylpropane-2-sulfinamide (30 mg, 0.06 mmol) in 1,4-dioxane (5 mL) was added aq. HCl (4 *N,* 2 mL) at room temperature. The reaction was stirred at room temperature for 1 h. The reaction was diluted with EA (20 mL) and HCl (1 *N,* 20 mL). The organic layers were washed with aq. HCl (1 *N,* 2 x 20 mL). The combined aqueous layers were concentrated under reduced pressure. The residue was purified by Prep-HPLC with following conditions: Column: Xselect CSH OBD Column 30 x 150 mm 5 µm; Mobile Phase A: water with 10 mmoL/L NH₄HCO₃, Mobile Phase B: ACN; Flow rate: 60 mL/min; Gradient: 7% B to 20% B in 8 min; Detector: UV 254/220 nm; Retention time: 6.55 min. The fractions containing the desired product were collected and concentrated under reduced pressure to afford Compound 212 ((2*R*)-1-[(1*R*,5*S*,6*R*)-6-[amino(4,5-dichloro-2-hydroxyphenyl)methyl]-3-azabicyclo[*3.1.0*]hexan-3-yl]-2,3-dihydroxypropan-1-one) as an off-white solid (3 mg, 16%): LCMS (ESI) calc'd for C₁₅H₁₈Cl₂N₂O₄ [M + H - 17]⁺: 344, 346 (3 : 2), found 344, 346 (3 : 2); ¹H NMR (400 MHz, CD₃OD) δ 7.53-7.40 (m, 1H), 7.08 (s, 1H), 4.40-4.12 (m, 1H), 4.04-3.85 (m, 2H), 3.85-3.58 (m, 3H), 3.56-3.38 (m, 2H), 2.01-1.68 (m, 2H), 1.42-1.27 (m, 1H).

### Example 194. (which is a reference example) Compound 214 ((2R)-1-[4-[1-(4,5-dichloro-2-hydroxyphenyl)-2,2-difluoroethyl]piperidin-1-yl]-2,3-dihydroxypropan-1-one)

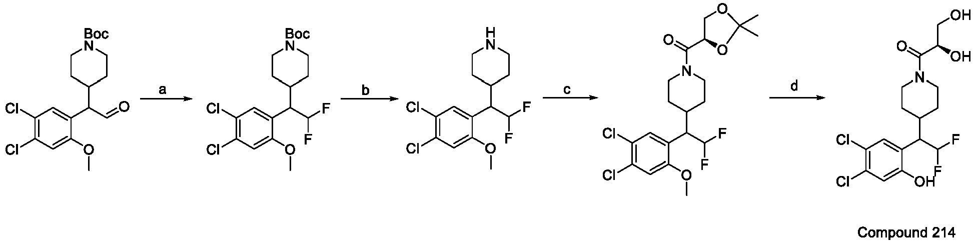

### Step a:

To a stirred solution of *tert*-butyl 4-[1-(4,5-dichloro-2-methoxyphenyl)-2-oxoethyl]piperidine-1-carboxylate (Intermediate 49, Example 49) (0.30 g, 0.75 mmol) in DCM (3 mL) was added DAST (0.72 g, 4.47 mmol) dropwise at -70 °C. The resulting mixture was allowed to warm to room temperature and stirred for 16 h under nitrogen atmosphere. The reaction was quenched with water (30 mL) at 0 °C and extracted with EA (3 x 35 mL). The combined organic layers were washed with brine (2 x 10 mL), dried over anhydrous Na₂SO₄. After filtration, the filtrate was concentrated under reduced pressure. The residue was purified by Prep-TLC, eluted with PE/EA (5/1) to afford *tert*-butyl 4-[1-(4,5-dichloro-2-methoxyphenyl)-2,2-difluoroethyl]piperidine-1-carboxylate as a light yellow oil (0.22 g, 57%): LCMS (ESI) calc'd for C₁₉H₂₅Cl₂F₂NO₃ [M + H]⁺: 424, 426 (3 : 2), found 424, 426 (3 : 2).

### Step b:

To a stirred solution of *tert*-butyl 4-[1-(4,5-dichloro-2-methoxyphenyl)-2,2-difluoroethyl]piperidine-1-carboxylate (0.22 g, 0.52 mmol) in DCM (5 mL) was added TFA (1 mL) at 0 °C. The resulting mixture was stirred for 30 min at room temperature. The resulting mixture was concentrated under reduced pressure. The crude product was used in the next step directly without further purification: LCMS (ESI) calc'd for C₁₄H₁₇Cl₂F₂NO [M + H]⁺: 324, 326 (3 : 2), found 324, 326 (3 : 2).

### Step c:

To a stirred solution of 4-[1-(4,5-dichloro-2-methoxyphenyl)-2,2-difluoroethyl]piperidine (0.17 g, 0.52 mmol) and (4*R*)-2,2-dimethyl-1,3-dioxolane-4-carboxylic acid (0.92 g, 0.63 mmol) and HOBt (0.11 g, 0.78 mmol) and Et₃N (0.16 g, 1.57 mmol) in DMF (4 mL) was added EDCI (0.16 g, 0.79 mmol) in portions at room temperature. The resulting mixture was stirred for 2 h at room temperature. The resulting mixture was diluted with water (40 mL) and extracted with EA (3 x 30 mL). The combined organic layers were washed with brine (3 x 20 mL), dried over anhydrous Na₂SO₄. After filtration, the filtrate was concentrated under reduced pressure. The residue was purified by Prep-TLC, eluted with PE/EA (2/1) to afford 4-[1-(4,5-dichloro-2-methoxyphenyl)-2,2-difluoroethyl]-1-[(4*R*)-2,2-dimethyl-1,3-dioxolane-4-carbonyl]piperidine as a light yellow oil (90 mg, 38%): LCMS (ESI) calc'd for C₂₀H₂₅Cl₂F₂NO₄ [M + H]⁺: 452, 454 (3 : 2), found 452, 454 (3 : 2).

### Step d:

To a stirred solution of 4-[1-(4,5-dichloro-2-methoxyphenyl)-2,2-difluoroethyl]-1-[(4R)-2,2-dimethyl-1,3-dioxolane-4-carbonyl]piperidine (90 mg, 0.20 mmol) in DCM (1 mL) was added BBr₃ (0.15 g, 0.60 mmol) dropwise at 0 °C. The resulting mixture was stirred for 30 min at 0 °C. The reaction was quenched with saturated aq. NH₄Cl at 0 °C. The resulting mixture was extracted with EA (3 x 15 mL). The combined organic layers were washed with brine (2 x 5 mL), dried over anhydrous Na₂SO₄. After filtration, the filtrate was concentrated under reduced pressure. The crude product was purified by Prep-HPLC with the following conditions: Column: XBridge Shield RP18 OBD Column 30 x 150 mm, 5 µm; Mobile Phase A: water with 10 mmol/L NH₄HCO₃, Mobile Phase B: ACN; Flow rate: 60 mL/min; Gradient: 30% B to 55% B in 7 min; Detector: 254/220 nm; Retention time: 6.5 min. The fractions containing the desired product were collected and concentrated under reduced pressure to afford Compound 214 ((2R)-1-[4-[1-(4,5-dichloro-2-hydroxyphenyl)-2,2-difluoroethyl]piperidin-1-yl]-2,3-dihydroxypropan-1-one) as an off-white solid (21.2 mg, 25%): LCMS (ESI) calc'd for C₁₆H₁₉Cl₂F₂NO₄ [M + H]⁺: 398, 400 (3 : 2), found 398, 400 (3 : 2); ¹H NMR (400 MHz, CD₃OD) δ 7.33 (s, 1H), 6.96 (s, 1H), 6.26 (td, *J =* 56.3, 4.3 Hz, 1H), 4.62-4.34 (m, 2H), 4.07 (dd, *J =* 45.8, 13.8 Hz, 1H), 3.78-3.59 (m, 2H), 3.39-3.32 (m, 1H), 3.08 (dt, *J =* 40.5, 12.8 Hz, 1H), 2.68 (dt, *J =* 41.1, 13.0 Hz, 1H), 2.23 (d, *J =* 11.2 Hz, 1H), 2.08 (d, *J =* 17.2 Hz, 1H), 1.67-1.25 (m, 2H), 1.26-0.98 (m, 1H); ¹⁹F NMR (376 MHz, CD₃OD) δ -119.02, -119.20.

### Example 195. (which is a reference example) Compound 209 (4-[(R)-amino(4,5-dichloro-2-hydroxyphenyl)methyl]-N-(2-hydroxyethyl)piperidine-1-carboxamide trifluoroacetic acid)

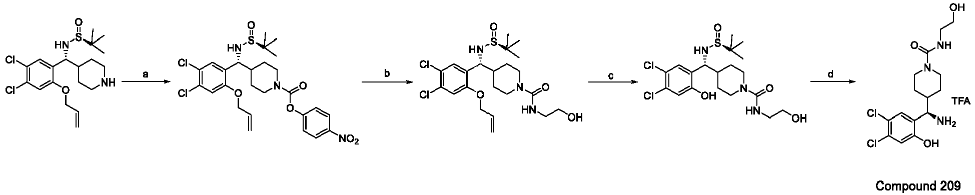

### Step a:

To a stirred mixture of (*S*)-*N*-[(*R*)-[4,5-dichloro-2-(prop-2-en-1-yloxy)phenyl](piperidin-4-yl)methyl]-2-methylpropane-2-sulfinamide (0.20 g, 0.47 mmol) and Et₃N (96 mg, 0.95 mmol) in DCM (2 mL) was added 4-nitrophenyl carbonochloridate (0.10 g, 0.57 mmol) dropwise at room temperature under nitrogen atmosphere. The reaction mixture was stirred for 12 h at room temperature. The resulting mixture was quenched with water (40 mL) and extracted with EA (3 x 30 mL). The combined organic layers were washed with brine (2 x 20 mL), dried over anhydrous Na₂SO₄. After filtration, the filtrate was concentrated under reduced pressure. The residue was purified by silica gel column chromatography, eluted with PE/EA (1/3) to afford 4-(dihydroxyamino)phenyl 4-([[*tert*-butyl(hydroxy)-lambda3-sulfanyl]amino](4,5-dichloro-2-propoxycyclohexyl)methyl)piperidine-1-carboxylate as an off-white solid (0.25 g, 75%): LCMS (ESI) calc'd for C₂₆H₃₁Cl₂N₃O₆S [M + H]⁺: 584, 586 (3 : 2), found 584, 586 (3 : 2); ¹H NMR (400 MHz, CD₃OD) δ 8.30 (d, *J* = 8.7 Hz, 2H), 7.48 (s, 1H), 7.38 (t, *J =* 7.6 Hz, 2H), 7.19 (s, 1H), 6.20-6.05 (m, 1H), 5.47 (dd, *J =* 17.4, 1.7 Hz, 1H), 5.36 (dd, *J =* 10.5, 1.5 Hz, 1H), 4.70-4.53 (m, 3H), 4.45-4.07 (m, 2H), 3.18-2.78 (m, 2H), 2.30-2.20 (m, 1H), 2.13-1.99 (m, 1H), 1.50-1.25 (m, 3H), 1.15 (s, 9H).

### Step b:

To a stirred mixture of 4-nitrophenyl 4-[(*R*)-[4,5-dichloro-2-(prop-2-en-1-yloxy)phenyl]([[(*R*)-2-methylpropane-2-sulfinyl]amino])methyl]piperidine-1-carboxylate (0.20 g, 0.34 mmol) in DMF (2 mL) was added 2-aminoethan-1-ol (0.40 g, 6.84 mmol) at room temperature. The reaction solution was stirred for 12 h at 90 °C. The reaction mixture was diluted with water (30 mL) and extracted with EA (3 x 35 mL). The combined organic layers were washed with brine (3 x 10 mL), dried over anhydrous Na₂SO₄. After filtration, the filtrate was concentrated under reduced pressure. The residue was purified by reverse phase chromatography, eluted with 25% ACN in water (plus 0.5% TFA) to afford 4-[(*R*)-[4,5-dichloro-2-(prop-2-en-1-yloxy)phenyl]([[(*R*)-2-methylpropane-2-sulfinyl]amino])methyl]-*N*-(2-hydroxyethyl)piperidine-1-carboxamide as a yellow solid (0.12 g, 62%): LCMS (ESI) calc'd for C₂₂H₃₃Cl₂N₃O₄S [M + H]⁺: 506, 508 (3 : 2), found 506, 508 (3 : 2); ¹H NMR (400 MHz, CD₃OD) δ 7.45 (s, 1H), 7.17 (s, 1H), 6.16-6.04 (m, 1H), 5.50-5.29 (m, 2H), 4.67-4.48 (m, 3H), 4.14-3.92 (m, 2H), 3.58 (t, *J =* 5.8 Hz, 2H), 3.28 (t, *J =* 5.9 Hz, 2H), 2.86-2.62 (m, 2H), 2.14 (d, *J =* 13.5 Hz, 1H), 2.03-1.88 (m, 1H), 1.36-1.17 (m, 3H), 1.14 (s, 9H).

### Step c:

To a stirred mixture of 4-[(*R*)-[4,5-dichloro-2-(prop-2-en-1-yloxy)phenyl]([[(*S*)-2-methylpropane-2-sulfinyl]amino])methyl-*N*-(2-hydroxyethyl)piperidine-1-carboxamide (0.11 g, 0.21 mmol) and Pd(PPh₃)₄ (13 mg, 0.01 mmol) in THF (2 mL) was added NaBH₄ (16 mg, 0.43 mmol) in portions at room temperature under nitrogen atmosphere. The reaction mixture was stirred for 30 min at room temperature under nitrogen atmosphere. The resulting mixture was quenched with water (30 mL) and extracted with EA (3 x 40 mL). The combined organic layers were washed with brine (2 x 10 mL), dried over anhydrous Na₂SO₄. After filtration, the filtrate was concentrated under reduced pressure to afford 4-[(*R*)-(4,5-dichloro-2-hydroxyphenyl)([[(*S*)-2-methylpropane-2-sulfinyl]amino])methyl]-*N*-(2-hydroxyethyl)piperidine-1-carboxamide as a brown semi-solid (66 mg, crude): LCMS (ESI) calc'd for C₁₉H₂₉Cl₂N₃O₄S [M + H]⁺: 466, 468 (3 : 2), found 466, 468 (3 : 2); ¹H NMR (400 MHz, CD₃OD) δ 7.34 (s, 1H), 6.93 (s, 1H), 4.45-4.31 (m, 1H), 4.12-3.90 (m, 2H), 3.58 (t, *J =* 5.8 Hz, 2H), 3.28 (t, *J =* 5.9 Hz, 2H), 2.85-2.63 (m, 2H), 2.15 (d, *J =* 13.7 Hz, 1H), 2.04-1.90 (m, 1H), 1.36-1.17 (m, 3H), 1.14 (s, 9H).

### Step d:

To a stirred solution of 4-[(*R*)-(4,5-dichloro-2-hydroxyphenyl)([[(*S*)-2-methylpropane-2-sulfinyl]amino])methyl]-*N-*(2-hydroxyethyl)piperidine-1-carboxamide (66 mg, 0.14 mmol) in THF (1 mL) was added aq. HCl (4 *N,* 0.5 mL) at room temperature. The reaction solution was stirred for 1 h at room temperature. The resulting solution was concentrated under reduced pressure. The residue was purified with Prep-HPLC with the following conditions: Column: XBridge Shield RP18 OBD Column 19 x 150 mm, 5 µm; Mobile Phase A: water (plus 0.05% TFA), Mobile Phase B: ACN; Flow rate: 25 mL/min; Gradient: 10% B to 33% B in 7 min; Detector: UV 220 nm; Retention time: 4.7 min. The fractions containing the desired product were collected and concentrated under reduced pressure to afford Compound 209 (4-[(*R*)-amino(4,5-dichloro-2-hydroxyphenyl)methyl]-*N*-(2-hydroxyethyl)piperidine-1-carboxamide trifluoroacetic acid) as an off-white solid (25 mg, 37% overall two steps): LCMS (ESI) calc'd for C₁₅H₂₁Cl₂N₃O₃ [M + H]⁺: 362, 364 (3 : 2), found, 362, 364 (3 : 2); ¹H NMR (400 MHz, CD₃OD) δ 7.43 (s, 1H), 7.08 (s, 1H), 4.30-4.08 (m, 2H), 3.97 (d, *J =* 13.6 Hz, 1H), 3.69-3.42 (m, 2H), 3.28 (t, *J =* 5.8 Hz, 2H), 2.91-2.67 (m, 2H), 2.40-2.15 (m, 1H), 2.02-1.87 (m, 1H), 1.41-1.22 (m, 2H), 1.21-1.06 (m, 1H); ¹⁹F NMR (376 MHz, CD₃OD) δ -77.09.

### Example 196. Compound 215 ((2R)-1-[6-[amino(4,5-dichloro-2-hydroxyphenyl)methyl]-3-azabicyclo[3.1.1]heptan-3-yl]-2,3-dihydroxypropan-1-one trifluoroacetic acid)

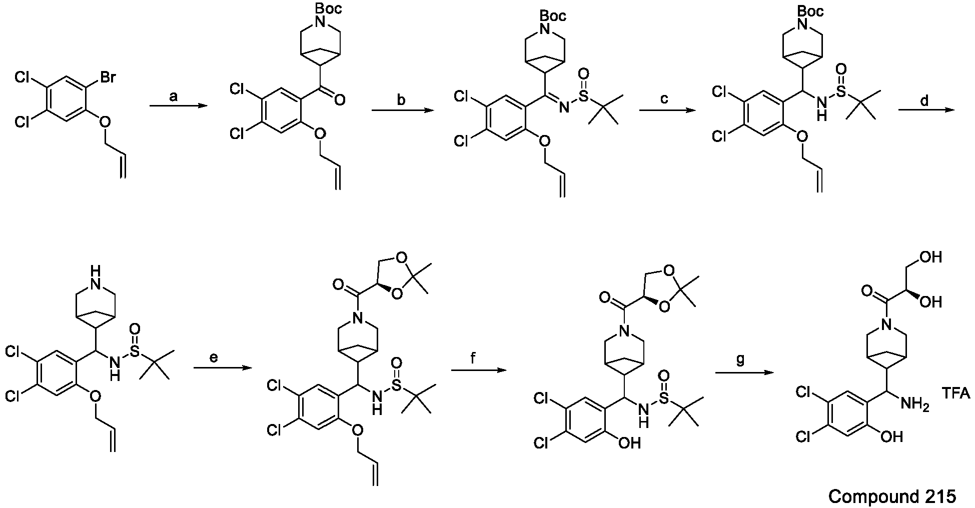

### Step a:

To a stirred mixture of 1-bromo-4,5-dichloro-2-(prop-2-en-1-yloxy)benzene (Example 6, step b) (1.07 g, 3.80 mmol) in THF (15 mL) was added *i*-PrMgCl·LiCl (3.90 mL, 5.07 mmol, 1.3 M solution in THF) dropwise at -10 °C under nitrogen atmosphere. The resulting mixture was stirred for 30 min at -10 °C under nitrogen atmosphere. To the above a solution was added *tert*-butyl 6-[methoxy(methyl)carbamoyl]-3-azabicyclo[*3.1.1*]heptane-3-carboxylate (0.36 g, 1.27 mmol) in THF (2 mL) dropwise over 5 min at -10 °C. The resulting solution was stirred for additional 2 h at -10 °C under nitrogen atmosphere. The reaction was quenched with saturated aq. NH₄Cl (20 mL) at room temperature and extracted with EA (3 x 50 mL). The combined organic layers were washed with brine (3 x 20 mL), dried over anhydrous Na₂SO₄. After filtration, the filtrate was concentrated under reduced pressure. The residue was purified by Prep-TLC, eluted with PE/EA (1/1) to afford *tert-*butyl 6-[4,5-dichloro-2-(prop-2-en-1-yloxy)benzoyl]-3-azabicyclo[*3.1.1*]heptane-3-carboxylate as a light yellow oil (30 mg, 6%). LCMS (ESI) calc'd for C₂₁H₂₅Cl₂NO₄ [M + Na]⁺: 448, 450 (3 : 2), found 448, 450 (3 : 2); ¹H NMR (400 MHz, CDCl₃) δ 7.46 (d, *J =* 27.1 Hz, 1H), 6.99 (d, *J =* 27.9 Hz, 1H), 6.14-5.96 (m, 1H), 5.49-5.28 (m, 2H), 4.64-4.53 (m, 2H), 4.37-4.16 (m, 1H), 3.70-3.39 (m, 2H), 3.22 (dd, *J =* 11.6, 5.7 Hz, 1H), 2.80 (d, *J =* 27.5 Hz, 1H), 2.10 (d, *J =* 9.6 Hz, 1H), 1.63-1.55 (m, 1H), 1.50-1.43 (m, 10H), 1.37-1.19 (m, 1H).

### Step b:

To a stirred solution of *tert*-butyl 6-[4,5-dichloro-2-(prop-2-en-1-yloxy)benzoyl]-3-azabicyclo[*3.1.1*]heptane-3-carboxylate (30 mg, 0.07 mmol) and 2-methylpropane-2-sulfinamide (26 mg, 0.21 mmol) in THF (2 mL) was added Ti(OEt)₄ (96 mg, 0.42 mmol) at room temperature. The resulting solution was stirred for 16 h at 70 °C under nitrogen atmosphere. After cooling to room temperature, the reaction was quenched with saturated aq. NaHCO₃ (30 mL) at room temperature. The resulting mixture was extracted with EA (3 x 30 mL). The combined organic layers were washed with brine (3 x 20 mL), dried over anhydrous Na₂SO₄. After filtration, the filtrate was concentrated under reduced pressure to afford *tert*-butyl 6-[[4,5-dichloro-2-(prop-2-en-1-yloxy)phenyl][(2-methylpropane-2-sulfinyl)imino]methyl]-3-azabicyclo[*3.1.1*]heptane-3-carboxylate as a light yellow oil (30 mg, crude), which was used in the next step directly without further purification: LCMS (ESI) calc'd for C₂₅H₃₄Cl₂N₂O₄S [M + H]⁺: 529, 531 (3 : 2), found 529, 531 (3 : 2).

### Step c:

To a stirred solution of *tert*-butyl 6-[(1*E*)-[4,5-dichloro-2-(prop-2-en-1-yloxy)phenyl][(2-methylpropane-2-sulfinyl)imino]methyl]-3-azabicyclo[*3.1.1*]heptane-3-carboxylate (30 mg, 0.06 mmol) in THF (1 mL) was added NaBH₄ (10 mg, 0.26 mmol) at room temperature. The resulting mixture was stirred for 2 h at room temperature. The reaction was quenched with water (20 mL) at room temperature and extracted with EA (3 x 20 mL). The combined organic layers were washed with brine (3 x 20 mL), dried over anhydrous Na₂SO₄. After filtration, the filtrate was concentrated under reduced pressure to afford *tert*-butyl 6-[[4,5-dichloro-2-(prop-2-en-1-yloxy)phenyl][(2-methylpropane-2-sulfinyl)amino]methyl]-3-azabicyclo[*3.1.1*]heptane-3-carboxylate as a light yellow oil (40 mg, crude), which was used in the next step directly without further purification: LCMS (ESI) calc'd for C₂₅H₃₆Cl₂N₂O₄S [M + H]⁺: 531, 533 (3 : 2), found 531, 533 (3 : 2).

### Step d:

To a stirred solution of *tert*-butyl 6-[[4,5-dichloro-2-(prop-2-en-1-yloxy)phenyl][(2-methylpropane-2-sulfinyl)amino]methyl]-3-azabicyclo[3.1.1]heptane-3-carboxylate (40 mg, 0.08 mmol) in DCM (2 mL) was added TFA (0.5 mL) at room temperature. The resulting solution was stirred for 30 min at room temperature. The reaction solution was basified to pH 8 with saturated aq. NaHCO₃ (30 mL) and extracted with EA (3 x 30 mL). The combined organic layers were washed with brine (3 x 10 mL), dried over anhydrous Na₂SO₄. After filtration, the filtrate was concentrated under reduced pressure to afford *N*-([3-azabicyclo[3.1.1]heptan-6-yl][4,5-dichloro-2-(prop-2-en-1-yloxy)phenyl]methyl)-2-methylpropane-2-sulfinamide as a light yellow oil (40 mg, crude), which was used in the next step directly without further purification. LCMS (ESI) calc'd for C₂₀H₂₈Cl₂N₂O₂S [M + H]⁺: 431, 433 (3 : 2), found 431, 433 (3 : 2).

### Step e:

To a stirred solution of (4*R*)-2,2-dimethyl-1,3-dioxolane-4-carboxylic acid (20 mg, 0.14 mmol) and HATU (71 mg, 0.19 mmol) in DMF (1 mL) were added *N-*([3-azabicyclo[*3.1.1*]heptan-6-yl][4,5-dichloro-2-(prop-2-en-1-yloxy)phenyl]methyl)-2-methylpropane-2-sulfinamide (40 mg, 0.09 mmol) and Et₃N (28 mg, 0.28 mmol) at room temperature. The reaction was stirred at room temperature for 30 min. The reaction was purified by reverse phase chromatography, eluted with 55% ACN in water (plus 0.05% TFA) to afford *N*-[[4,5-dichloro-2-(prop-2-en-1-yloxy)phenyl]([3-[(4*R*)-2,2-dimethyl-1,3-dioxolane-4-carbonyl]-3-azabicyclo[*3.1.1*]heptan-6-yl])methyl]-2-methylpropane-2-sulfinamide as a light yellow oil (10 mg, 26% overall four steps): LCMS (ESI) calc'd for C₂₆H₃₆Cl₂N₂O₅S [M + H]⁺: 559, 561 (3 : 2), found 559, 561 (3 : 2).

### Step f:

To a stirred solution of *N*-[[4,5-dichloro-2-(prop-2-en-1-yloxy)phenyl]([3-[(4*R*)-2,2-dimethyl-1,3-dioxolane-4-carbonyl]-3-azabicyclo[*3.1.1*]heptan-6-yl])methyl]-2-methylpropane-2-sulfinamide (10 mg, 0.02 mmol) and Pd(PPh₃)₄ (1 mg, 0.001 mmol) in THF (1 mL) was added NaBH₄ (1.3 mg, 0.03 mmol) at room temperature. The resulting mixture was stirred for 30 min at room temperature. The resulting mixture was quenched with saturated aq. NH₄Cl (1 mL) and concentrated under reduced pressure to afford *N*-((4,5-dichloro-2-hydroxyphenyl)(3-((4*R*)-2,2-dimethyl-1,3-dioxolane-4-carbonyl)-3-azabicyclo[*3.1.1*]heptan-6-yl)methyl)-2-methylpropane-2-sulfinamide as a brown solid (10 mg, crude), which was used in the next step directly without further purification: LCMS (ESI) calc'd for C₂₃H₃₂Cl₂N₂O₅S [M + H]⁺: 519, 521 (3 : 2), found 519, 521 (3 : 2).

### Step g:

To a stirred mixture of *N*-((4,5-dichloro-2-hydroxyphenyl)(3-((4*R*)-2,2-dimethyl-1,3-dioxolane-4-carbonyl)-3-azabicyclo[*3.1.1*]heptan-6-yl)methyl)-2-methylpropane-2-sulfinamide (10 mg, 0.02 mmol) in 1,4-dioxane (1 mL) was added aq. HCl (4 *N,* 1 mL) at room temperature. The resulting mixture was stirred for 30 min at room temperature. The resulting mixture was concentrated under reduced pressure. The residue was purified by Prep-HPLC with the following conditions: Column: Sunfire Prep C18 OBD Column, 10 µm, 19 x 250 mm; Mobile Phase A: water (plus 0.05% TFA), Mobile Phase B: ACN; Flow rate: 25 mL/min; Gradient: 10% B to 30% B in 7 min; Detector: UV 254/220 nm; Retention time: 6.50 min. The fractions containing the desired product were collected and concentrated under reduced pressure to afford Compound 215 ((2*R*)-1-[6-[amino(4,5-dichloro-2-hydroxyphenyl)methyl]-3-azabicyclo[*3.1.1*]heptan-3-yl]-2,3-dihydroxypropan-1-one trifluoroacetic acid) as an off-white solid (3.3 mg, 38% overall two steps): LCMS (ESI) calc'd for C₁₆H₂₀Cl₂N₂O₄ [M + H]⁺: 375, 377 (3 : 2), found 375, 377 (3 : 2); ¹H NMR (400 MHz, CD₃OD): ¹H NMR δ 7.65-7.46 (m, 1H), 7.09 (dd, *J =* 6.9, 4.3 Hz, 1H), 4.85-4.45 (m, 2H), 4.25-3.87 (m, 1H), 3.87-3.62 (m, 4H), 3.62-3.40 (m, 1H), 3.10-2.97 (m, 1H), 2.78-2.55 (m, 1H), 2.46-2.27 (m, 1H), 2.27-2.06 (m, 1H), 1.59-1.41 (m, 1H); ¹⁹F NMR (376 MHz, CD₃OD) δ -77.23.

### Example 197. Compound 217 (1-(4-((R)-amino(4,5-dichloro-2-hydroxyphenyl)methyl)piperidin-1-yl)-2,3-dihydroxy-2-methylpropan-1-one isomer 1) Compound 218 (1-(4-((R)-amino(4,5-dichloro-2-hydroxyphenyl)methyl)piperidin-1-yl)-2,3-dihydroxy-2-methylpropan-1-one isomer 2)

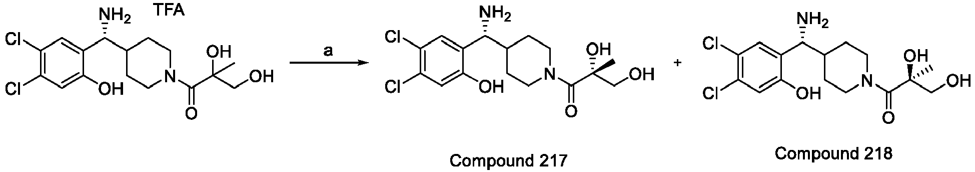

### Step a:

1-[4-[(*R*)-amino(4,5-dichloro-2-hydroxyphenyl)methyl]piperidin-1-yl]-2,3-dihydroxy-2-methylpropan-1-one trifluoroacetic acid (Compound 201, Example 191) (55 mg) was separated by Prep-chiral HPLC with following conditions: Column: CHIRALPAK IG, 2 x 25 cm, 5 µm; Mobile Phase A: Hexane (plus 0.2% IPA), Mobile Phase B: EtOH; Flow rate: 20 mL/min; Gradient: 30% B to 30% B in 20 min; Detector: UV: 220/254 nm; Retention time: RT₁: 12.76 min; RT₂: 17.37 min; Injection Volume: 0.3 mL.

The faster-eluting enantiomer was obtained Compound 217 (1-(4-((R)-amino(4,5-dichloro-2-hydroxyphenyl)methyl)piperidin-1-yl)-2,3-dihydroxy-2-methylpropan-1-one isomer 1) as an off-white solid (10 mg, 24%) at 12.76 min: LCMS (ESI) calc'd for C₁₆H₂₂Cl₂N₂O₄ [M + H]⁺: 377, 379 (3 : 2), found 377, 379 (3 : 2); ¹H NMR (400 MHz, CD₃OD) δ 7.21 (s, 1H), 6.86 (s, 1H), 4.74-4.45 (m, 2H), 3.89 (d, *J =* 7.7 Hz, 1H), 3.71 (d*, J* = 11.2 Hz, 1H), 3.56 (d, *J =* 11.2 Hz, 1H), 3.04-2.37 (m, 2H), 2.15-1.93 (m, 2H), 1.48-1.40 (m, 1H), 1.38 (s, 3H), 1.33-1.09 (m, 2H).

The slower-eluting enantiomer was obtained Compound 218 (1-(4-((R)-amino(4,5-dichloro-2-hydroxyphenyl)methyl)piperidin-1-yl)-2,3-dihydroxy-2-methylpropan-1-one isomer 2) as an off-white solid (15 mg, 36%) at 17.37 min: LCMS (ESI) calc'd for C₁₆H₂₂Cl₂N₂O₄ [M + H]⁺: 377, 379 (3 : 2), found 377, 379 (3 : 2); ¹H NMR (400 MHz, CD₃OD) δ 7.22 (s, 1H), 6.86 (s, 1H), 4.74-4.45 (m, 1H), 3.90 (d, *J =* 7.8 Hz, 1H), 3.71 (d, *J =* 11.2 Hz, 1H), 3.63-3.41 (m, 2H), 3.08-2.44 (m, 2H), 2.01 (dd, *J =* 34.1, 10.7 Hz, 2H), 1.50-1.42 (m, 1H), 1.38 (s, 3H), 1.34-1.06 (m, 2H).

### Example 198. Compound 219 (3-[4-[(R)-amino(4,5-dichloro-2-hydroxyphenyl)methyl]piperidine-1-carbonyl]azetidin-3-ol trifluoroacetic acid)

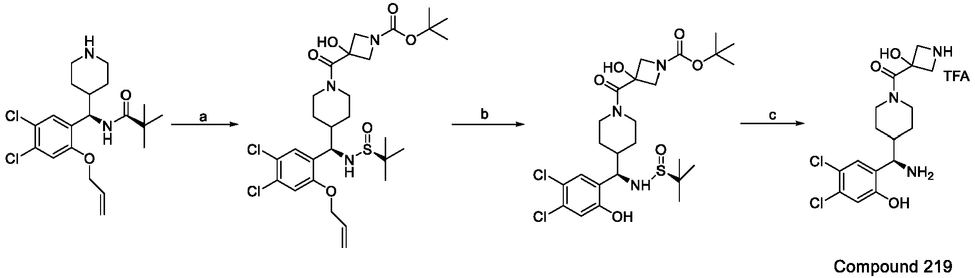

### Step a:

To a stirred solution of 1-[(*tert*-butoxy)carbonyl]-3-hydroxyazetidine-3-carboxylic acid (87 mg, 0.40 mmol), EDCI (96 mg, 0.50 mmol) and HOBt (67 mg, 0.50 mmol) in DMF (2 mL) were added (*S*)-*N*-[(*R*)-[4,5-dichloro-2-(prop-2-en-1-yloxy)phenyl](piperidin-4-yl)methyl]-2-methylpropane-2-sulfinamide (0.14 g, 0.34 mmol) and Et₃N (0.10 g, 1.00 mmol) at room temperature. The reaction mixture was stirred for 12 h at room temperature. The resulting mixture was diluted with water (20 mL) and extracted with EA (3 x 30 mL). The combined organic layers were washed with brine (3 x 10 mL), dried over anhydrous Na₂SO₄. After filtration, the filtrate was concentrated under reduced pressure to afford *tert*-butyl 3-[4-[(*R*)-[4,5-dichloro-2-(prop-2-en-1-yloxy)phenyl]([[(*S*)-2-methylpropane-2-sulfinyl]amino])methyl]piperidine-1-carbonyl]-3-hydroxyazetidine-1-carboxylate as an off-white solid (0.18 g, crude): LCMS (ESI) calc'd for C₂₈H₄₁Cl₂N₃O₆S [M + H]⁺: 618, 620 (3 : 2), found, 618, 620 (3 : 2); ¹H NMR (400 MHz, CD₃OD) δ 7.46 (s, 1H), 7.17 (s, 1H), 6.15-6.04 (m, 1H), 5.45 (d, *J =* 17.3 Hz, 1H), 5.34 (d, *J =* 10.6 Hz, 1H), 4.66-4.60 (m, 2H), 4.58-4.26 (m, 4H), 3.98-3.76 (m, 3H), 3.11-2.90 (m, 1H), 2.75-2.56 (m, 1H), 2.23-2.13 (m, 1H), 2.11-2.00 (m, 1H), 1.46 (s, 9H), 1.39-1.17 (m, 3H), 1.14 (s, 9H).

### Step b:

To a stirred mixture of *tert*-butyl 3-[4-[(*R*)-[4,5-dichloro-2-(prop-2-en-1-yloxy)phenyl]([[(*S*)-2-methylpropane-2-sulfinyl]amino])methyl]piperidine-1-carbonyl]-3-hydroxyazetidine-1-carboxylate (0.18 g, 0.29 mmol) and Pd (PPh₃)₄ (17 mg, 0.02 mmol) in THF (2 mL) was added NaBH₄ (22 mg, 0.58 mmol) in portions at room temperature under nitrogen atmosphere. The reaction mixture was stirred for 1 h at room temperature under nitrogen atmosphere. The resulting mixture was quenched with H₂O (2 mL) at room temperature and extracted with EA (3 x 30 mL). The combined organic layers were washed with brine (2 x 10 mL), dried over anhydrous Na₂SO₄. After filtration, the filtrate was concentrated under reduced pressure to afford *tert-*butyl 3-[4-[(*R*)-(4,5-dichloro-2-hydroxyphenyl)([[(*S*)-2-methylpropane-2-sulfinyl]amino])methyl]piperidine-1-carbonyl]-3-hydroxyazetidine-1-carboxylate as a brown solid (90 mg, crude): LCMS (ESI) calc'd for C₂₅H₃₇Cl₂N₃O₆S [M + H]⁺: 578, 580 (3 : 2), found, 578, 580 (3 : 2).

### Step c:

To a stirred solution of *tert*-butyl 3-[4-[(*R*)-(4,5-dichloro-2-hydroxyphenyl)([[(*S*)-2-methylpropane-2-sulfinyl]amino])methyl]piperidine-1-carbonyl]-3-hydroxyazetidine-1-carboxylate (90 mg, 0.15 mmol) in THF (1 mL) was added aq. HCl (4 *N*, 0.50 mL) dropwise at room temperature. The reaction mixture was stirred for 1 h at room temperature. The resulting solution was concentrated under reduced pressure. The residue was purified with Prep-HPLC with the following conditions: Column: XBridge Shield RP18 OBD Column 30 x 150 mm, 5 µm; Mobile Phase A: water (plus 0.05% TFA), Mobile Phase B: ACN; Flow rate: 60 mL/min; Gradient: 5% B to 15% B in 7 min; Detector: UV 220 nm; Retention time: 6.42 min. The fractions containing the desired product were collected and concentrated under reduced pressure to afford Compound 219 (3-[4-[(*R*)-amino(4,5-dichloro-2-hydroxyphenyl)methyl]piperidine-1-carbonyl]azetidin-3-ol trifluoroacetic acid) as a brown solid (38 mg, 50%): LCMS (ESI) calc'd for C₁₆H₂₁Cl₂N₃O₃ [M + H]⁺: 374, 376 (3 : 2), found, 374, 376 (3 : 2); ¹H NMR (400 MHz, CD₃OD) δ 7.44 (s, 1H), 7.09 (s, 1H), 4.71-4.40 (m, 3H), 4.25-3.88 (m, 3H), 3.18-2.93 (m, 1H), 2.83-2.59 (m, 1H), 2.48-2.30 (m, 1H), 2.03 (t, *J =* 12.5 Hz, 1H), 1.49-1.12 (m, 4H).

The compounds in Table 1d below were prepared in an analogous fashion to that described for Compound 219, starting from (*S*)-*N*-((*R*)-(2-(allyloxy)-4,5-dichlorophenyl)(piperidin-4-yl)methyl)-2-methylpropane-2-sulfinamide and the corresponding acids, which were prepared as described herein, or which were available from commercial sources. Compounds in Table 1d marked with a * are reference compounds.

**Table 1d**

| **Compound Number** | **Structure** | **Chemical Name** | **MS & NMR** |
|---|---|---|---|
| **181** | 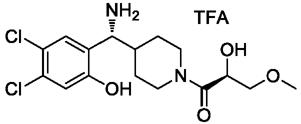 | (2*S*)-1-[4-[(*R*)-amino(4,5-dichloro-2-hydroxyphenyl) methyl]piperidin-1-yl]-2-hydroxy-3-methoxypropan-1-one trifluoroacetic acid | [M + H]⁺: 377, 379 (3 : 2); ¹H NMR (400 MHz, CD₃OD) δ 7.44 (s, 1H), 7.09 (s, 1H), 4.69-4.39 (m, 2H), 4.31-4.06 (m, 2H), 3.63-3.47 (m, 2H), 3.37 (d, *J* = 3.0 Hz, 3H), 3.07 (dt, *J* = 43.4, 12.3 Hz, 1H), 2.66 (dt, *J* = 46.7, 13.1 Hz, 1H), 2.43-2.30 (m, 1H), 2.06-1.95 (m, 1H), 1.48-1.05 (m, 3H); ¹⁹F NMR (376 MHz, CD₃OD) δ -76.99. |
| **197** | 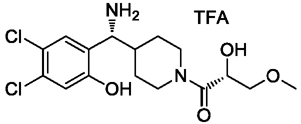 | (2*R*)-1-[4-[(*R*)-amino(4,5-dichloro-2-hydroxyphenyl) methyl]piperidin-1-yl]-2-hydroxy-3-methoxypropan-1-one trifluoroacetic acid | [M + H]⁺: 377, 379 (3 : 2); ¹H NMR (400 MHz, CD₃OD) δ 7.44 (s, 1H), 7.09 (s, 1H), 4.68-4.45 (m, 2H), 4.32-4.04 (m, 2H), 3.58-3.43 (m, 2H), 3.37 (d, *J* = 7.3 Hz, 3H), 3.17-2.94 (m, 1H), 2.66 (dt, *J* = 41.3, 12.9 Hz, 1H), 2.45-2.29 (m, 1H), 2.01 (d, *J* = 12.9 Hz, 1H), 1.47-1.07 (m, 3H); ¹⁹F NMR (376 MHz, CD₃OD) δ -77.15. |
| **204** | 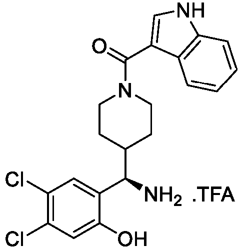 | (*R*)-(4-(amino(4,5-dichloro-2-hydroxyphenyl) methyl)piperidin-1-yl)(1*H*-indol-3-yl)methanone trifluoroacetic acid | [M + H]⁺: 418, 420 (3 : 2); ¹H NMR (400 MHz, CD₃OD) δ 7.73-7.55 (m, 2H), 7.42 (d, *J* = 8.5 Hz, 2H), 7.29-7.14 (m, 2H), 7.10 (s, 1H), 4.65-4.32 (m, 2H), 4.20 (d, *J* = 9.8 Hz, 1H), 3.17-2.90 (m, 2H), 2.50-2.32 (m, 1H), 2.13-1.95 (m, 1H), 1.56-1.26 (m, 3H); ¹⁹F NMR (376 MHz, CD₃OD) δ - 77.07. |
| **206*** | 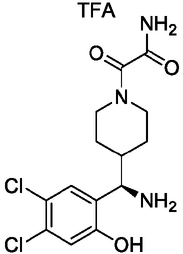 | 2-[4-[(*R*)-amino(4,5-dichloro-2-hydroxyphenyl) methyl]piperidin-1-yl]-2-oxoacetamide trifluoroacetic acid | [M + H]⁺: 346, 348 (3 : 2); ¹H NMR (400 MHz, CD₃OD) δ 7.44 (s, 1H), 7.09 (s, 1H), 4.61-4.37 (m, 1H), 4.24-3.94 (m, 2H), 3.24-3.04 (m, 1H), 2.85-2.62 (m, 1H), 2.49-2.33 (m, 1H), 2.09-1.95 (m, 1H), 1.53-1.13 (m, 3H); ¹⁹F NMR (376 MHz, CD₃OD) δ -77.19. |
| **211** | 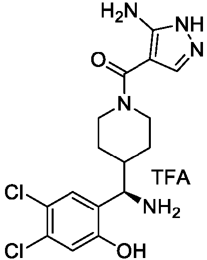 | 2-[(*R*)-amino[1-(5-amino-1*H*-pyrazole-4-carbonyl)piperidin-4-yl]methyl]-4,5-dichlorophenol trifluoroacetic acid | [M + H]⁺: 384, 386 (3 : 2); ¹H NMR (400 MHz, CD₃OD) δ 7.63 (s, 1H), 7.44 (s, 1H), 7.09 (s, 1H), 4.52 (d, *J* = 13.7 Hz, 1H), 4.36 (d, *J* = 13.6 Hz, 1H), 4.17 (d, *J* = 9.8 Hz, 1H), 3.15-2.85 (m, 2H), 2.52-2.35 (m, 1H), 2.08-1.99 (m, 1H), 1.42-1.23 (m, 3H); ¹⁹F NMR (376 MHz, CD₃OD) δ - 77.26. |
| **252** | 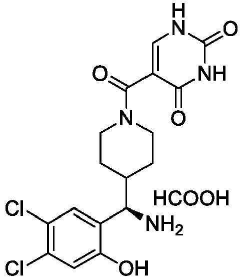 | 5-[4-[(*R*)-amino(4,5-dichloro-2-hydroxyphenyl) methyl]piperidine-1-carbonyl]-1,3-dihydropyrimidine-2,4-dione formic acid | [M + H]⁺: 413, 415 (3 : 2); ¹H NMR (400 MHz, CD₃OD) δ 8.63 (s, 1H), 7.66 (s, 1H), 7.42 (s, 1H), 7.08 (s, 1H), 4.59 (d, *J* = 54.9 Hz, 1H), 4.16 (s, 1H), 3.74 (d, *J* = 49.4 Hz, 1H), 3.09 (d, *J* = 50.6 Hz, 1H), 2.77 (d, *J =* 43.9 Hz, 1H), 2.45-2.27 (m, 1H), 2.13-1.88 (m, 1H), 1.61-1.20 (m, 3H). |
| **254** | 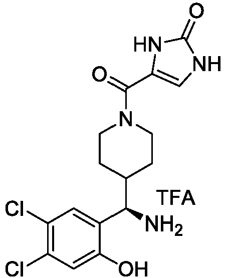 | 4-[4-[(*R*)-amino(4,5-dichloro-2-hydroxyphenyl) methyl]piperidine-1-carbonyl]-1,3-dihydroimidazol-2-one trifluoroacetic acid | [M + H]⁺: 385, 387 (3 : 2); ¹H NMR (400 MHz, CD₃OD) δ 7.44 (s, 1H), 7.09 (s, 1H), 6.91 (s, 1H), 4.52 (d, *J* = 13.6 Hz, 1H), 4.35 (d, *J* = 13.6 Hz, 1H), 4.17 (d, *J* = 9.8 Hz, 1H), 3.14-2.90 (m, 2H), 2.48-2.38 (m, 1H), 2.04 (d, *J* = 13.4 Hz, 1H), 1.50-1.15 (m, 3H); ¹⁹F NMR (376 MHz, CD₃OD) δ - 77.20. |
| **281** | 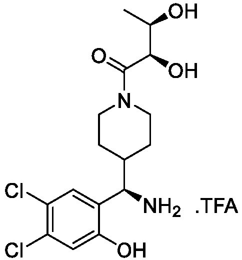 | 1-[4-[(*R*)-amino(4,5-dichloro-2-hydroxyphenyl)met hyl]piperidin-1-yl]-(2R,3R)-rel-dihydroxybutan-1-one trifluoroacetic acid | [M + H]⁺: 377, 379 (3 : 2), found 377, 379 (3 : 2); ¹H NMR (400 MHz, CD₃OD) δ 7.43 (d, *J* = 2.6 Hz, 1H), 7.09 (s, 1H), 4.60 (dd, *J* = 54.4, 13.6 Hz, 1H), 4.31-4.08 (m, 3H), 3.92-3.79 (m, 1H), 3.08 (dt, *J* = 43.4, 12.4 Hz, 1H), 2.77-2.55 (m, 1H), 2.45-2.34 (m, 1H), 2.08-1.96 (m, 1H), 1.49-1.12 (m, 6H); ¹⁹F NMR (376 MHz, CD₃OD) δ - 77.15. |

### Example 199. Compound 220 (1-[4-[(R)-amino(4,5-dichloro-2-hydroxyphenyl)methyl]piperidine-1-carbonyl]-3-(hydroxymethyl)azetidin-3-ol)

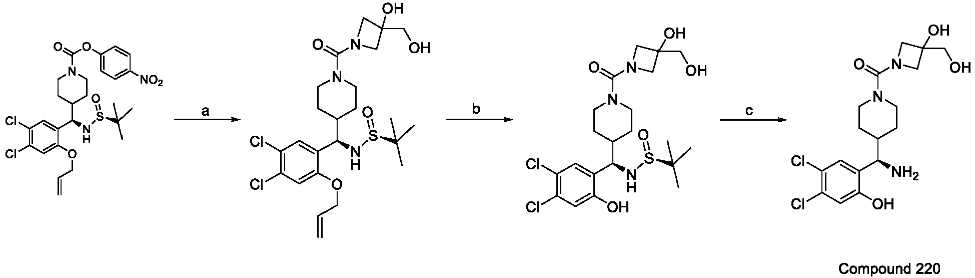

### Step a:

To a stirred solution of 4-nitrophenyl 4-((*R*)-(2-(allyloxy)-4,5-dichlorophenyl)(((*S*)-*tert*-butylsulfinyl)amino)methyl)piperidine-1-carboxylate (0.35 g, crude) and 3-(hydroxymethyl)azetidin-3-ol (0.13 g, 1.24 mmol) in 1,4-dioxane (4 mL) was added DIEA (1.60 g, 12.40 mmol) dropwise at room temperature. The reaction mixture was stirred for 12 h at 90 °C. After cooling to room temperature, the reaction mixture was diluted with water (20 mL) and extracted with EA (3 x 25 mL). The combined organic layers were washed with brine (2 x 10 mL), dried over anhydrous Na₂SO₄. After filtration, the filtrate was concentrated under reduced pressure. The residue was purified by reverse phase chromatography, eluted with 50% ACN in water (plus 0.5% TFA) to afford (*S*)-N-[(*R*)-[4,5-dichloro-2-(prop-2-en-1-yloxy)phenyl]([1-[3-hydroxy-3-(hydroxymethyl)azetidine-1-carbonyl]piperidin-4-yl])methyl]-2-methylpropane-2-sulfinamide (0.14 g, 38%): LCMS (ESI) calc'd for C₂₄H₃₅Cl₂N₃O₅S [M + H]⁺: 548, 550 (3 : 2), found, 548, 550 (3 : 2); ¹H NMR (400 MHz, CDCl₃) δ 7.22 (s, 1H), 6.97 (s, 1H), 6.09-5.96 (m, 1H), 5.48-5.31 (m, 2H), 4.63-4.49 (m, 2H), 4.45-4.32 (m, 1H), 4.08-3.82 (m, 8H), 2.80-2.59 (m, 2H), 2.04-1.84 (m, 2H), 1.32-1.22 (m, 3H), 1.15 (s, 9H).

### Step b:

To a stirred solution of (*S*)-*N*-[(*R*)-[4,5-dichloro-2-(prop-2-en-1-yloxy)phenyl]([1-[3-hydroxy-3-(hydroxymethyl)azetidine-1-carbonyl]piperidin-4-yl])methyl]-2-methylpropane-2-sulfinamide (0.14 g, 0.25 mmol) and Pd (PPh₃)₄ (15 mg, 0.01 mmol) in THF (2 mL) was added NaBH₄ (19 mg, 0.51 mmol) in portions at room temperature under nitrogen atmosphere. The reaction mixture was stirred for 1 h at room temperature. The resulting mixture was quenched with water (3 mL) at room temperature and concentrated under reduced pressure to afford (*S*)*-N-*[(*R*)-(4,5-dichloro-2-hydroxyphenyl)([1-[3-hydroxy-3-(hydroxymethyl)azetidine-1-carbonyl]piperidin-4-yl])methyl]-2-methylpropane-2-sulfinamide as a yellow solid (0.14 g, crude): LCMS (ESI) calc'd for C₂₁H₃₁Cl₂N₃O₅S [M + H]⁺: 508, 510 (3 : 2), found, 508, 50 (3 : 2)

### Step c:

To a stirred solution of (*S*)-*N*-[(*R*)-(4,5-dichloro-2-hydroxyphenyl)([1-[3-hydroxy-3-(hydroxymethyl)azetidine-1-carbonyl]piperidin-4-yl])methyl]-2-methylpropane-2-sulfinamide (0.14 g, crude) in THF (1 mL) was added aq. HCl (4 *N,* 0.5 mL,) dropwise at room temperature. The reaction solution was stirred at room temperature for 1 h. The resulting solution was concentrated under reduced pressure. The residue was purified with Prep-HPLC with the following conditions: Column: XBridge Shield RP18 OBD Column 19 x 150 mm, 5 µm; Mobile Phase A: water with 10 mmol/L NH₄HCO₃, Mobile Phase B: ACN; Flow rate: 20 mL/min; Gradient: 13% B to 35% B in 7 min; Detector: UV 220 nm; Retention time: 7.4 min. The fractions containing the desired product were collected and concentrated under reduced pressure to afford Compound 220 (1-[4-[(*R*)-amino(4,5-dichloro-2-hydroxyphenyl)methyl]piperidine-1-carbonyl]-3-(hydroxymethyl)azetidin-3-ol) as an off-white solid (16 mg, 14% overall two steps): LCMS (ESI) calc'd for C₁₇H₂₃Cl₂N₃O₄ [M + H]⁺: 404, 406 (3 : 2), found, 404, 406 (3 : 2); ¹H NMR (400 MHz, CD₃OD) δ 7.21 (s, 1H), 6.86 (s, 1H), 4.27-3.95 (m, 3H), 3.92-3.70 (m, 4H), 3.58 (s, 2H), 2.75 (dt, *J =* 25.9, 12.7 Hz, 2H), 2.09-1.87 (m, 2H), 1.49-1.09 (m, 3H).

### Example 200. Compound 221 (5-[4-[(R)-amino(4,5-dichloro-2-hydroxyphenyl)methyl]piperidine-1-carbonyl]piperidin-2-one isomer 1); Compound 222 (5-[4-[(R)-amino(4,5-dichloro-2-hydroxyphenyl)methyl]piperidine-1-carbonyl]piperidin-2-one isomer 2)

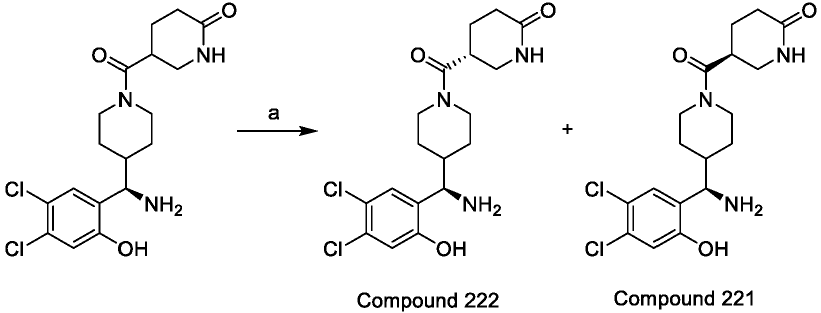

### Step a:

5-[4-[(*R*)-amino(4,5-dichloro-2-hydroxyphenyl)methyl]piperidine-1-carbonyl]piperidin-2-one (35 mg, 0.09 mmol) was separated by Chiral Prep-HPLC with the following conditions: Column: Chiralpak IC, 2 x 25 cm, 5 µm; Mobile Phase A: Hexane (0.2% IPA), Mobile Phase B: EtOH; Flow rate: 20 mL/min; Gradient: 40% B to 40% B in 26 min; Detector: 220/254 nm; Retention time: RT₁:18.17 min; RT₂:22.14 min. The faster-eluting enantiomer was obtained as Compound 222 (5-[4-[(R)-amino(4,5-dichloro-2-hydroxyphenyl)methyl]piperidine-1-carbonyl]piperidin-2-one isomer 2) as an off-white solid (6.7 mg, 19%) at 18.17 min: LCMS (ESI) calc'd for C₁₈H₂₃Cl₂N₂O₃ [M + H]⁺: 400, 402 (3 : 2), found 400, 402 (3 : 2); ¹H NMR (400 MHz, CD₃OD) δ 7.25 (s, 1H), 6.89 (s, 1H), 4.57 (dd, *J =* 39.7, 13.3 Hz, 1H), 4.10 (dd, *J =* 37.1, 13.8 Hz, 1H), 3.92 (t, *J =* 8.5 Hz, 1H), 3.51-3.36 (m, 1H), 3.23-2.97 (m, 2H), 2.68-2.50 (m, 1H), 2.47-2.35 (m, 2H), 2.15-1.84 (m, 4H), 1.50 (t, *J =* 15.3 Hz, 1H), 1.40-1.04 (m, 3H).

The slower-eluting enantiomer was obtained as Compound 221 (5-[4-[(R)-amino(4,5-dichloro-2-hydroxyphenyl)methyl]piperidine-1-carbonyl]piperidin-2-one isomer 1) as an off-white solid (6.4 mg, 18%) at 22.142 min: LCMS (ESI) calc'd for C₁₈H₂₃Cl₂N₂O₃ [M + H]⁺: 400, 402 (3 : 2), found 400, 402 (3 : 2); ¹H NMR (400 MHz, CD₃OD) δ 7.23 *(d, J* = 7.0 Hz, 1H), 6.88 (d, *J =* 2.2 Hz, 1H), 4.57 (dd, *J =* 38.7, 13.3 Hz, 1H), 4.10 (dd, *J =* 38.9, 13.8 Hz, 1H), 3.96-3.84 (m, 1H), 3.53-3.35 (m, 1H), 3.24 -2.99 (m, 2H), 2.71-2.49 (m, 1H), 2.46-2.32 (m, 2H), 2.13-1.83 (m, 4H), 1.60-1.42 (m, 1H), 1.38-1.08 (m, 3H).

### Example 201. Compound 296 (4-[4-[(R)-amino(4,5-dichloro-2-hydroxyphenyl)methyl]piperidine-1-carbonyl]pyrrolidin-2-one isomer 1); Compound 246 (4-[4-[(R)-amino(4,5-dichloro-2-hydroxyphenyl)methyl]piperidine-1-carbonyl]pyrrolidin-2-one isomer 2)

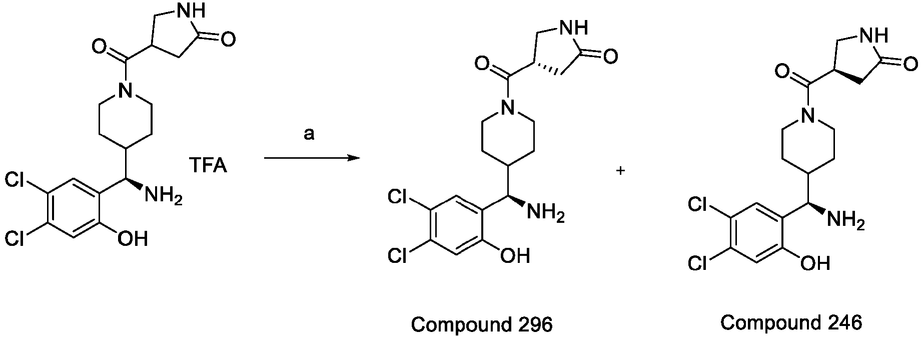

### Step a:

4-[4-[(*R*)-amino(4,5-dichloro-2-hydroxyphenyl)methyl]piperidine-1-carbonyl]pyrrolidin-2-one trifluoroacetic acid (80 mg, 0.21 mmol) was separated by Chiral Prep-HPLC with the following conditions: Column: CHIRALPAK IC, 2 x 25 cm, 5 µm; Mobile Phase A: MTBE (plus 0.2% IPA), Mobile Phase B: EtOH; Flow rate: 16 mL/min; Gradient: 30% B to 30% B in 26 min; Detector: UV 220/254 nm; Retention time: RT₁: 14.14 min; RT₂: 22.44 min.

The faster-eluting enantiomer at 14.14 min was obtained as Compound 296 (4-[4-[(R)-amino(4,5-dichloro-2-hydroxyphenyl)methyl]piperidine-1-carbonyl]pyrrolidin-2-one isomer 1) (free amine) as an off-white solid (20.6 mg, 32%) at 14.14 min: LCMS (ESI) calc'd for C₁₇H₂₁Cl₂N₃O₃ [M + H]⁺: 386, 388 (3 : 2), found 386, 388 (3 : 2); ¹H NMR (400 MHz, CD₃OD) δ 7.23 (d, *J* = 6.0 Hz, 1H), 6.87 (d, *J* = 2.3 Hz, 1H), 4.57 (dd, *J* = 38.6, 13.4 Hz, 1H), 4.13-3.84 (m, 2H), 3.84-3.67 (m, 1H), 3.67-3.57 (m, 1H), 3.57-3.43 (m, 1H), 3.16-2.93 (m, 1H), 2.72-2.45 (m, 3H), 2.13-1.95 (m, 2H), 1.49 (t, *J* = 16.3 Hz, 1H), 1.41-1.06 (m, 2H).

The slower-eluting enantiomer at 22.44 min was obtained as Compound 246 (4-[4-[(R)-amino(4, 5-dichloro-2-hydroxyphenyl)methyl]piperidine-1-carbonyl]pyrrolidin-2-one isomer 2) as an off-white solid (18.9 mg, 30%) at 22.44 min: LCMS (ESI) calc'd for C₁₇H₂₁Cl₂N₃O₃ [M + H]⁺: 386, 388 (3 : 2), found 386, 388 (3 : 2); ¹H NMR (400 MHz, CD₃OD) δ 7.24 (s, 1H), 6.87 (d, *J* = 1.7 Hz, 1H), 4.57 (dd, *J* = 38.4, 13.4 Hz, 1H), 4.13-3.84 (m, 2H), 3.81-3.67 (m, 1H), 3.67-3.43 (m, 2H), 3.18-2.94 (m, 1H), 2.74-2.44 (m, 3H), 2.11-1.91 (m, 2H), 1.49 (d, *J* = 13.4 Hz, 1H), 1.35-1.04 (m, 2H).

### Example 202. Compound 225 ((2R)-1-((3aR,5R,6aS)-5-(amino(4,5-dichloro-2-hydroxyphenyl)methyl)hexahydrocyclopenta[c]pyrrol-2(1H)-yl)-2,3-dihydroxypropan-1-one)

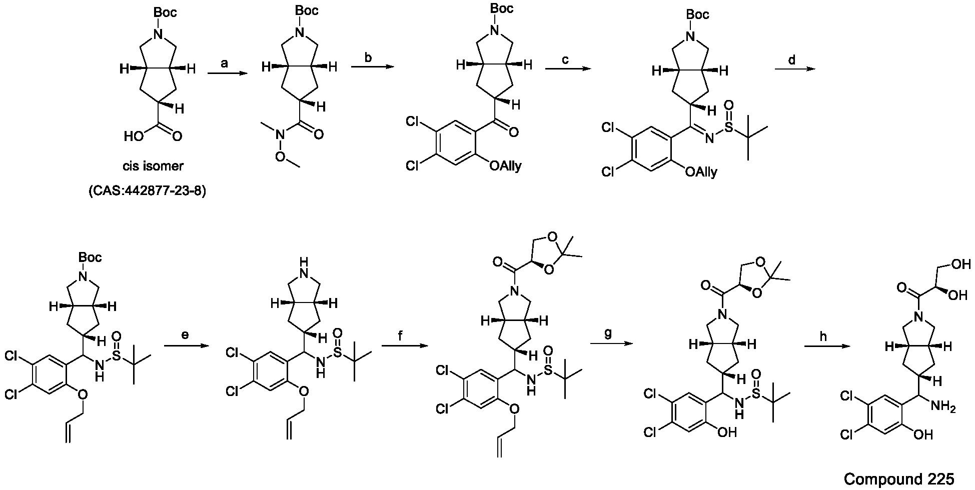

### Step a:

To a stirred mixture of (3a*R*,5*R*,6a*S*)-2-(*tert*-butoxycarbonyl)-hexahydro-1*H-*cyclopenta[*c*]pyrrole-5-carboxylic acid (0.10 g, 0.39 mmol), EDCI (0.11 g, 0.59 mmol) and HOBt (79 mg, 0.59 mmol) in DMF (2 mL) were added Et₃N (59 mg, 0.59 mmol) and *N*,*O-*dimethylhydroxylamine HCl (36 mg, 0.59 mmol) in portions at room temperature under nitrogen atmosphere. The reaction mixture was stirred for 2 h at room temperature under nitrogen atmosphere. The resulting mixture was quenched with water (30 mL) at room temperature and extracted with EA (3 x 50 mL). The combined organic layers were washed with brine (2 x 30 mL), dried over anhydrous Na₂SO₄. After filtration, the filtrate was concentrated under reduce pressure. The residue was purified by reverse phase chromatography, eluted with 60% ACN in water (plus 0.05% TFA) to afford *tert*-butyl (3a*R*,5*R*,6a*S*)-5-[methoxy(methyl)carbamoyl]-hexahydro-1*H*-cyclopenta[*c*]pyrrole-2-carboxylate as a colorless oil (0.10 g, 81%): LCMS (ESI) calc'd for C₁₅H₂₆N₂O₄ [M + H]⁺: 299, found 299; ¹H NMR (400 MHz, CDCl₃) δ 4.84-4.55 (m, 2H), 3.70 (s, 3H), 3.60-3.48 (m, 2H), 3.35-3.25 (m, 1H), 3.22 (s, 3H), 2.76-2.62 (m, 2H), 2.21-2.09 (m, 2H), 1.84-1.68 (m, 2H), 1.45 (s, 9H).

### Step b:

A solution of 1-bromo-4,5-dichloro-2-(prop-2-en-1-yloxy)benzene (0.47 g, 1.68 mmol) in THF (10 mL) was added *i*-PrMgCl·LiCl (1.30 mL, 1.68 mmol, 1.3 M in THF) over 15 min at -5 °C under nitrogen atmosphere. After stirring for additional 30 min, a solution of *tert-*butyl (3a*R*,5*R*,6a*S*)-5-[methoxy(methyl)carbamoyl]-hexahydro-1*H*-cyclopenta[*c*]pyrrole-2-carboxylate (0.10 g, 0.34 mmol) in THF (5 mL) was added dropwise. The reaction mixture was stirred for 0.5 h at -5 °C under nitrogen atmosphere. The resulting mixture was quenched with saturated aq. NH₄Cl (40 mL) at -5 °C and extracted with EA (2 x 100 mL). The combined organic layers were washed with brine (2 x 10 mL), dried over anhydrous Na₂SO₄. After filtration, the filtrate was concentrated under reduced pressure. The crude product was purified by reverse phase chromatography, eluted with 70% ACN in water (0.05% TFA) to afford *tert-*butyl (3a*R*,5*R*,6a*S*)-5-[4,5-dichloro-2-(prop-2-en-1-yloxy)benzoyl]-hexahydro-1*H-*cyclopenta[c]pyrrole-2-carboxylate as a yellow oil (32 mg, 22%): LCMS (ESI) calc'd for C₂₂H₂₇Cl₂NO₄ [M + H]⁺: 440, 442 (3 : 2), found 440, 442 (3 : 2); ¹H NMR (400 MHz, CDCl₃) δ 7.66 (s, 1H), 6.98 (s, 1H), 6.11-6.00 (m, 1H), 5.51-5.37 (m, 2H), 4.70 (d, *J* = 5.6 Hz, 2H), 3.75 (dd, *J* = 20.7, 11.3 Hz, 1H), 3.64-3.39 (m, 2H), 3.31-3.22 (d, *J* = 11.2 Hz, 2H), 2.75-2.56 (m, 2H), 2.23-2.11 (m, 2H), 1.84-1.67 (m, 2H), 1.47 (s, 9H).

### Step c:

To a stirred solution of tert-butyl (3a*R*,5*R*,6a*S*)-5-[4,5-dichloro-2-(prop-2-en-1-yloxy)benzoyl]-hexahydro-1*H*-cyclopenta[c]pyrrole-2-carboxylate (50 mg, 0.11 mmol) and Ti(OEt)₄ (0.10 g, 0.45 mmol) in THF (5 mL) was added *tert*-butanesulfinamide (21 mg, 0.17 mmol) in portions at room temperature under nitrogen atmosphere. The reaction solution was allowed to warm to 70 °C and stirred for 24 h under nitrogen atmosphere. After cooling to room temperature, the resulting solution was quenched with water (60 mL) and extracted with EA (2 x 80 mL). The combined organic layers were washed with brine (3 x 10 mL), dried over anhydrous Na₂SO₄. After filtration, the filtrate was concentrated under reduced pressure to afford crude (3a*R*,5*R*,6a*S*)-*tert*-butyl 5-((2-(allyloxy)-4,5-dichlorophenyl)((*tert-*butylsulfinyl)imino)methyl)hexahydrocyclopenta[*c*]pyrrole-2(1*H*)-carboxylate. The crude product was used in the next step directly without further purification: LCMS (ESI) calc'd for C₂₆H₃₆Cl₂N₂O₄S [M + H]⁺: 543, 545 (3 : 2), found 543, 545 (3 : 2).

### Step d:

To a stirred solution of (3a*R*,5*R*,6a*S*)-*tert*-butyl 5-((2-(allyloxy)-4,5-dichlorophenyl)((*tert*-butylsulfinyl)imino)methyl)hexahydrocyclopenta[*c*]pyrrole-2(1*H*)-carboxylate (crude) in THF (2 mL) was added NaBH₄ (5 mg, 0.13 mmol) in portions at room temperature under nitrogen atmosphere. After stirring for 1 h, the resulting mixture was quenched with water (20 mL) at room temperature and extracted with EA (2 x 30 mL). The combined organic layers were washed with brine (2 x 15 mL), dried over anhydrous Na₂SO₄. After filtration, the filtrate was concentrated under reduced pressure to afford crude (3a*R*,5*R*,6a*S*)-*tert*-butyl 5-((2-(allyloxy)-4,5-dichlorophenyl)(1,1-dimethylethylsulfinamido)methyl)hexahydrocyclopenta[c]pyrrole-2(1*H*)-carboxylate. The crude product was used in the next step directly without further purification: LCMS (ESI) calc'd for C₂₆H₃₈Cl₂N₂O₄S [M + H]⁺: 545, 547 (3 : 2), found 545, 547 (3 : 2).

### Step e:

To a stirred solution of (3a*R*,5*R*,6a*S*)-*tert*-butyl 5-((2-(allyloxy)-4,5-dichlorophenyl)(1,1-dimethylethylsulfinamido)methyl)hexahydrocyclopenta[*c*]pyrrole-2(1*H*)-carboxylate (crude) in DCM (2 mL) was added TFA (0.5 mL) dropwise at room temperature. After stirring for 1 h at room temperature, the reaction solution was basified to pH 8 with saturated aq. NaHCO₃ and extracted with EA (2 x 50 mL). The combined organic layers were washed with brine (2 x 10 mL), dried over anhydrous Na₂SO₄. After filtration, the filtrate was concentrated under reduced pressure. The crude product was purified by reverse phase chromatography, eluted with 70% ACN in water to afford *N*-[(3a*R*,5*R*,6a*S*)-octahydrocyclopenta[*c*]pyrrol-5-yl[4,5-dichloro-2-(prop-2-en-1-yloxy)phenyl]methyl]-2-methylpropane-2-sulfinamide as a yellow oil (18 mg, 61% overall three steps): LCMS (ESI) calc'd for C₂₁H₃₀Cl₂N₂O₂S [M + H]⁺: 445, 447 (3 : 2), found 445, 447 (3 : 2).

### Step f:

To a stirred solution of (4*R*)-2,2-dimethyl-1,3-dioxolane-4-carboxylic acid (9 mg, 0.06 mmol) and HATU (23 mg, 0.06 mmol) in DMF (1 mL) were added *N*-[(3a*R*,5*R*,6a*S*)-octahydrocyclopenta[c]pyrrol-5-yl[4,5-dichloro-2-(prop-2-en-1-yloxy)phenyl]methyl]-2-methylpropane-2-sulfinamide (18 mg, 0.04 mmol) and Et₃N (16 mg, 0.16 mmol) at room temperature. The reaction solution was stirred for 2 h at room temperature. The resulting solution was quenched with water (20 mL) at room temperature and extracted with EA (2 x 20 mL). The combined organic layers were washed with brine (2 x 15 mL), dried over anhydrous Na₂SO₄. After filtration, the filtrate was concentrated under reduced pressure to afford *N*-((4,5-dichloro-2-hydroxyphenyl)((3a*R*,5*R*,6a*S*)-2-((*R*)-2,2-dimethyl-1,3-dioxolane-4-carbonyl)octahydrocyclopenta[*c*]pyrrol-5-yl)methyl)-2-methylpropane-2-sulfinamide. The resulting mixture was used in the next step directly without further purification: LCMS (ESI) calc'd for C₂₇H₃₈Cl₂N₂O₅S [M + H]⁺: 573, 575 (3 : 2), found 573, 575 (3 : 2).

### Step g:

To a stirred solution of *N*-((4,5-dichloro-2-hydroxyphenyl)((3a*R*,5*R*,6a*S*)-2-((*R*)-2,2-dimethyl-1,3-dioxolane-4-carbonyl)octahydrocyclopenta[*c*]pyrrol-5-yl)methyl)-2-methylpropane-2-sulfinamide (crude) and Pd(PPh₃)₄ (6 mg, 0.005 mmol) in THF (2 mL) was added NaBH₄ (4 mg, 0.11 mmol) at room temperature under nitrogen atmosphere. After stirring for 1 h at room temperature, the resulting mixture was quenched with saturated aq. NH₄Cl (1 mL) at room temperature and concentrated under reduced pressure to afford *N*-((4,5-dichloro-2-hydroxyphenyl)((3a*R*,5*R*,6a*S*)-2-((*R*)-2,2-dimethyl-1,3-dioxolane-4-carbonyl)octahydrocyclopenta[*c*]pyrrol-5-yl)methyl)-2-methylpropane-2-sulfinamide. The crude product was used in the next step directly without further purification: LCMS (ESI) calc'd for C₂₄H₃₄Cl₂N₂O₅S [M + H]⁺: 533, 535 (3 : 2), found 533, 535 (3 : 2).

### Step h:

To a stirred mixture of N-((4,5-dichloro-2-hydroxyphenyl)((3aR,5R,6aS)-2-((R)-2,2-dimethyl-1,3-dioxolane-4-carbonyl)octahydrocyclopenta[*c*]pyrrol-5-yl)methyl)-2-methylpropane-2-sulfinamide (crude) in THF (2 mL) was added aq. HCl (6 *N,* 2 mL) dropwise at room temperature. The reaction solution was stirred for 2 h at room temperature. The reaction solution was stirred for 1 h at room temperature. The resulting solution was diluted with EA (10 mL). The organic layer was extracted with aq. HCl (1 *N,* 3 x 10 mL). The combined aqueous layers were washed with EA (3 x 10 mL) and concentrated under reduced pressure. The residue was purified by Prep-HPLC with the following conditions: Column: Sunfire Prep C18 OBD Column, 10 µm, 19 x 250 mm; Mobile Phase A: water (plus 0.05% TFA), Mobile Phase B: ACN; Flow rate: 25 mL/min; Gradient: 5% B to 35% B in 7 min; Detector: UV 254 nm; Retention time: 5.8 min. The fractions containing the desired product were collected and concentrated under reduced pressure to afford Compound 225 ((2R)-1-((3a*R*,5*R*,6a*S*)-5-(amino(4,5-dichloro-2-hydroxyphenyl)methyl)hexahydrocyclopenta[c]pyrrol-2(1*H*)-yl)-2,3-dihydroxypropan-1-one) as an off-white solid (0.6 mg, 4% overall three steps): LCMS (ESI) calc'd for C₁₇H₂₂Cl₂N₂O₄ [M + H]⁺: 389, 391 (3 : 2), found 389, 391 (3 : 2); ¹H NMR (400 MHz, CD₃OD) δ 7.46 (s, 1H), 7.07 (s, 1H), 4.41-4.32 (m, 1H), 4.30-4.15 (m, 1H), 3.8-3.45 (m, 5H), 2.95-2.60 (m, 3H), 2.38-2.25 (m, 1H), 1.83-1.70 (m, 1H), 1.42-1.10 (m, 3H).

### Example 203. (which is a reference example) Compound 226 ((2R)-1-[4-[(R)-amino[5-chloro-4-(fluoromethyl)-2-hydroxyphenyl]methyl]piperidin-1-yl]-2,3-dihydroxypropan-1-one trifluoroacetic acid)

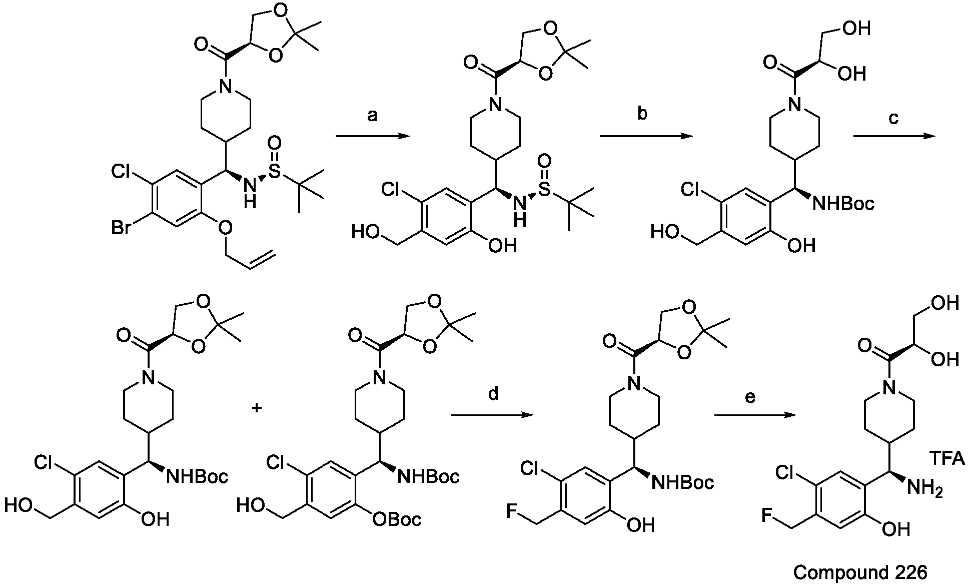

### Step a:

To a stirred solution of (*S*)-*N*-[(*R*)-[4-bromo-5-chloro-2-(prop-2-en-1-yloxy)phenyl]([1-[(4*R*)-2,2-dimethyl-1,3-dioxolane-4-carbonyl]piperidin-4-yl])methyl]-2-methylpropane-2-sulfinamide (Intermediate 24, Example 24) (3.80 g, 6.42 mmol) and (tributylstannyl)methanol (3.09 g, 9.629 mmol) in dioxane (30 mL) was added Pd(PPh₃)₄ (0.74 g, 0.64 mmol) at room temperature under nitrogen atmosphere. The resulting mixture was stirred for 16 h at 100 °C under nitrogen atmosphere. The resulting mixture was extracted with EtOAc (3 x 50 mL). The combined organic layers were washed with brine (2 x 50 mL), dried over anhydrous Na₂SO₄ and filtered. The filtrate was concentrated under reduced pressure. The residue was purified by reverse phase chromatography, eluted with 50% CH₃CN in water with 10 mmol/L NH₄HCO₃ to afford (*S*)-*N*-[(*R*)-[5-chloro-2-hydroxy-4-(hydroxymethyl)phenyl]([1-[(4*R*)-2,2-dimethyl-1,3-dioxolane-4-carbonyl]piperidin-4-yl])methyl]-2-methylpropane-2-sulfinamide as a light yellow semi-solid (1.80 g, 57%): LCMS (ESI) calc'd for C₂₃H₃₅ClN₂O₆S [M + H]⁺ 503, 505 (3 : 1) found 503, 505 (3 : 1); ¹H NMR (400 MHz, CDCl₃) δ 7.11-6.96 (m, 2H), 4.74-4.65 (m, 3H), 4.65-4.54 (m, 1H), 4.51-4.40 (m, 0H), 4.41-4.26 (m, 3H), 4.24-4.16 (m, 1H), 4.11-4.02 (m, 1H), 3.09-2.83 (m, 2H), 2.59-2.35 (m, 2H), 2.14-1.93 (m, 2H), 1.44-1.33 (m, 6H), 1.20 (d, *J* = 7.1 Hz, 9H).

### Step b:

To a stirred solution of *N*-[(*R*)-[5-chloro-2-hydroxy-4-(hydroxymethyl)phenyl]([1-[(4*R*)-2,2-dimethyl-1,3-dioxolane-4-carbonyl]piperidin-4-yl])methyl]-2-methylpropane-2-sulfinamide (120 mg, 0.239 mmol, 1.00 equiv) in dioxane (0.50 mL) was added HCl (4 M) (2.00 mL) at room temperature under air atmosphere. The reaction was stirred at room temperature for 1 h. The pH value was adjusted to 8 with saturated aq. NaHCO₃. Then to this were added Boc₂O (62 mg, 0.286 mmol, 1.20 equiv) and Na₂CO₃ (75.85 mg, 0.716 mmol, 3.00 equiv). The reaction was stirred at room temperature for 16 h. The reaction was diluted with EtOAc (30 mL) and water (30 mL). The aqueous layer was extracted with EtOAc (3 x 30 mL). The combined organic layers were washed with brine (3 x 30 mL), dried over anhydrous Na₂SO₄. After filtration, the filtrate was concentrated under reduced pressure to afford *tert*-butyl N-[(*R*)-[5-chloro-2-hydroxy-4-(hydroxymethyl)phenyl]([1-[(2*R*)-2,3-dihydroxypropanoyl]piperidin-4-yl])methyl]carbamate (100 mg, 91.34%) as a light yellow solid: LCMS (ESI) calc'd for C₂₁H₃₁ClN₂O₇ [M + H]⁺ 459, 461 (3 : 1) found 459, 461 (3 : 1).

### Step c:

To a stirred solution of *tert*-butyl *N*-[(*R*)-[5-chloro-2-hydroxy-4-(hydroxymethyl)phenyl]([1-[(2*R*)-2,3-dihydroxypropanoyl]piperidin-4-yl])methyl]carbamate (0.80 g, 1.74 mmol) and 2,2-dimethoxypropane (0.36 g, 3.49 mmol) in acetone (10 mL) was added TsOH (30 mg, 0.17 mmol) at room temperature. The reaction was stirred at room temperature for 1 h. The reaction was diluted with EtOAc (30 mL) and water (30 mL). The aqueous layer was extracted with EtOAc (3 x 30 mL). The combined organic layers were washed with brine (3 x 30 mL) and dried over anhydrous Na₂SO₄. After filtration, the filtrate was concentrated under reduced pressure. The residue was purified by reverse phase chromatography, eluted with 38% CH₃CN in water with 10 mmol/L NH₄HCO₃ to afford *tert-*butyl *N*-[(*R*)-[5-chloro-2-hydroxy-4-(hydroxymethyl)phenyl]([1-[(4*R*)-2,2-dimethyl-1,3-dioxolane-4-carbonyl]piperidin-4-yl])methyl]carbamate as a light yellow solid (0.50 g, 57%: LCMS (ESI) calc'd for C₂₄H₃₅ClN₂O₇ [M + H]⁺ 499, 501 (3 : 1) found 499, 501 (3 : 1); ¹H NMR (400 MHz, CDCl₃) δ 7.07-6.86 (m, 2H), 5.66-5.32 (m, 1H), 4.74-4.52 (m, 4H), 4.52-4.34 (m, 2H), 4.27-4.03 (m, 2H), 3.96-3.53 (m, 1H), 3.12-2.80 (m, 1H), 2.69-2.42 (m, 1H), 2.13-1.84 (m, 2H), 1.81-1.61 (m, 1H), 1.52-1.36 (m, 15H) and 2-[(*R*)-[(*tert*-butoxycarbonyl)amino]([1-[(4*R*)-2,2-dimethyl-1,3-dioxolane-4-carbonyl]piperidin-4-yl])methyl]-4-chloro-5-(hydroxymethyl)phenyl *tert*-butyl carbonate as a light yellow solid (0.21 g, 20%): LCMS (ESI) calc'd for C₂₉H₄₃ClN₂O₉ [M + H]⁺ 599, 601 (3 : 1) found 599, 601 (3 : 1). ¹H NMR (400 MHz, CDCl₃) δ 7.38 (s, 1H), 7.08-6.92 (m, 1H), 5.11-4.95 (m, 1H), 4.79-4.72 (m, 1H), 4.74-4.56 (m, 3H), 4.56-4.35 (m, 3H), 4.25-4.05 (m, 1H), 3.93-3.56 (m, 2H), 3.14-2.76 (m, 1H), 2.80-2.41 (m, 1H), 2.15-1.69 (m, 2H), 1.59 (s, 6H), 1.49-1.36 (m, 18H).

### Step d:

To a stirred solution of *tert*-butyl *N*-[(*R*)-[5-chloro-2-hydroxy-4-(hydroxymethyl)phenyl]([1-[(*4R*)-2,2-dimethyl-1,3-dioxolane-4-carbonyl]piperidin-4-yl])methyl]carbamate (65 mg, 0.13 mmol) in DCM (2 mL) was added DAST (42 mg, 0.26 mmol) at room temperature under nitrogen atmosphere. The reaction was stirred at room temperature for 1 h under nitrogen atmosphere. The reaction was quenched with water (20 mL). The aqueous layer was extracted with DCM (3 x 20 mL). The combined organic layers were washed with brine (2 x 20 mL), dried over anhydrous Na₂SO₄. After filtration, the filtrate was concentrated under reduced pressure to afford *tert*-butyl *N*-[(*R*)-[5-chloro-4-(fluoromethyl)-2-hydroxyphenyl]([1-[(*4R*)-2,2-dimethyl-1,3-dioxolane-4-carbonyl]piperidin-4-yl])methyl]carbamate as a light yellow oil (65 mg, crude), which was used in the next step directly without further purification: LCMS (ESI) calc'd for C₂₄H₃₄ClFN₂O₆ [M + H]⁺: 501, 503 (3 : 1), found 501, 503 (3 : 1).

### Step e:

To a stirred solution of *tert*-butyl *N*-[(*R*)-[5-chloro-4-(fluoromethyl)-2-hydroxyphenyl]([1-[(4*R*)-2,2-dimethyl-1,3-dioxolane-4-carbonyl]piperidin-4-yl])methyl]carbamate (65 mg, 0.13 mmol) in DCM (2 mL) was added TFA (0.5 mL) at room temperature. The reaction was stirred at room temperature for 1 h. The reaction was concentrated under reduced pressure. The residue was purified by Prep-HPLC with following conditions: Column: Xselect CSH OBD Column 30 x 150 mm 5 µm, n; Mobile Phase A: water (plus 0.05% TFA), Mobile Phase B: ACN; Flow rate: 60 mL/min; Gradient: 5% B to 15% B in 7 min; Detector: UV 254/220 nm; Retention time: 6.32 min. The fractions containing the desired product were collected and concentrated under reduced pressure to afford Compound 226 ((*2R*)-1-[4-[(*R*)-amino[5-chloro-4-(fluoromethyl)-2-hydroxyphenyl]methyl]piperidin-1-yl]-2,3-dihydroxypropan-1-one trifluoroacetic acid) as an off-white solid (12.9 mg, 21% overall two steps): LCMS (ESI) calc'd for C₁₆H₂₂ClFN₂O₄ [M + H]⁺: 361, 363 (3 : 1), found 361, 363 (3 : 1); ¹H NMR (400 MHz, CD₃OD) δ 7.34 (s, 1H), 7.09 (s, 1H), 5.46 (d, *J* = 47.1 Hz, 2H), 4.75-4.44 (m, 2H), 4.33-3.98 (m, 2H), 3.85-3.54 (m, 2H), 3.19-2.92 (m, 1H), 2.77-2.48 (m, 1H), 2.48-2.29 (m, 1H), 2.10-1.81 (m, 1H), 1.52-0.99 (m, 3H); ¹⁹F NMR (376 MHz, CD₃OD) δ -77.10, - 219.80.

### Example 204. Compound 227 ((2R)-1-[4-[(R)-amino[5-chloro-4-(difluoromethyl)-2-hydroxyphenyl]methyl]piperidin-1-yl]-2,3-dihydroxypropan-1-one trifluoroacetic acid)

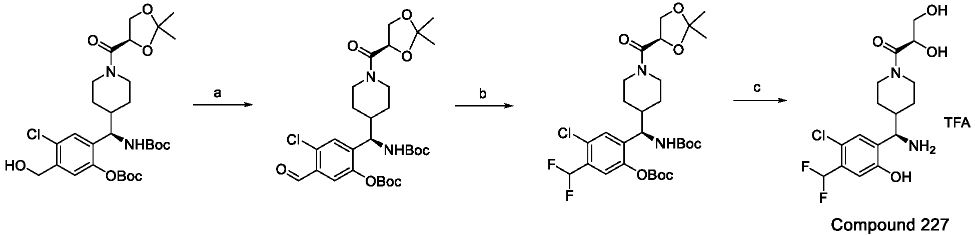

### Step a:

To a stirred solution of 2-[(*R*)-[(*tert*-butoxycarbonyl)amino]([1-[(*4R*)-2,2-dimethyl-1,3-dioxolane-4-carbonyl]piperidin-4-yl])methyl]-4-chloro-5-(hydroxymethyl)phenyl *tert*-butyl carbonate (Example 203, step c) (0.21 g, 0.35 mmol) in DCM (3 mL) was added Dess-Martin reagent (0.16 g, 0.37 mmol) at room temperature. The resulting mixture was stirred for 1 h at room temperature. The reaction was quenched with saturated aq. Na₂SO₃ (1 mL) at room temperature. The resulting mixture was extracted with DCM (3 x 20 mL). The combined organic layers were washed with brine (2 x 10 mL), dried over anhydrous Na₂SO₄. After filtration, the filtrate was concentrated under reduced pressure. The residue was purified by reverse phase chromatography, eluted with 55% ACN in water (plus 0.05% TFA) to afford 2-[(*R*)-[(*tert*-butoxycarbonyl)amino]([1-[(4*R*)-2,2-dimethyl-1,3-dioxolane-4-carbonyl]piperidin-4-yl])methyl]-4-chloro-5-formylphenyl *tert*-butyl carbonate as a light yellow solid (0.12 g, 57%): LCMS (ESI) calc'd for C₂₉H₄₁ClN₂O₉ [M + H]⁺: 597, 599 (3 : 1), found 597, 599 (3 : 1); ¹H NMR (400 MHz, DMSO-*d*₆) δ 10.25 (s, 1H), 7.84-7.65 (m, 2H), 7.62-7.52 (m, 1H), 4.88-4.59 (m, 2H), 4.43-4.17 (m, 2H), 4.17-3.83 (m, 2H), 3.02-2.76 (m, 2H), 2.50-2.38 (m, 2H), 1.87-1.59 (m, 3H), 1.51 (d, *J* = 5.2 Hz, 9H), 1.36 (s, 9H), 1.29 (s, 6H).

### Step b:

To a stirred solution of 2-[(*R*)-[(*tert*-butoxycarbonyl)amino]([1-[(*4R*)-2,2-dimethyl-1,3-dioxolane-4-carbonyl]piperidin-4-yl])methyl]-4-chloro-5-formylphenyl *tert*-butyl carbonate (0.12 g, 0.20 mmol) in DCM (2 mL) was added DAST (65 mg, 0.40 mmol) at room temperature under nitrogen atmosphere. The reaction was stirred at room temperature for 1 h under nitrogen atmosphere. The reaction was quenched with water (20 mL). The aqueous layer was extracted with DCM (3 x 20 mL). The combined organic layers were washed with brine (2 x 20 mL), dried over anhydrous Na₂SO₄. After filtration, the filtrate was concentrated under reduced pressure to afford 2-[(*R*)-[(*tert*-butoxycarbonyl)amino]([1-[(4R)-2,2-dimethyl-1,3-dioxolane-4-carbonyl]piperidin-4-yl])methyl]-4-chloro-5-(difluoromethyl)phenyl *tert*-butyl carbonate as a light yellow oil (0.12 g, crude), which was used in the next step directly without further purification: LCMS (ESI) calc'd for C₂₉H₄₁ClF₂N₂O₈ [M + H]⁺: 619, 621 (3 : 1), found 619, 621 (3 : 1).

### Step c:

To a stirred solution of 2-[(*R*)-[(*tert*-butoxycarbonyl)amino]([1-[(*4R*)-2,2-dimethyl-1,3-dioxolane-4-carbonyl]piperidin-4-yl])methyl]-4-chloro-5-(difluoromethyl)phenyl *tert*-butyl carbonate (0.12 g, 0.19 mmol) in DCM (3 mL) was added TFA (1 mL) at room temperature. The reaction was stirred at room temperature for 1 h. The reaction was concentrated under reduced pressure. The residue was purified by Prep-HPLC with following conditions: Column: Xselect CSH OBD Column 30 x 150 mm 5 µm; Mobile Phase A: water (plus 0.05% TFA), Mobile Phase B: ACN; Flow rate: 60 mL/min; Gradient: 5% B to 20% B in 7 min; Detector: UV 254/220 nm; Retention time: 6.56 min. The fractions containing the desired product were collected and concentrated under reduced pressure to afford Compound 227 ((*2R*)-1-[4-[(*R*)-amino[5-chloro-4-(difluoromethyl)-2-hydroxyphenyl]methyl]piperidin-1-yl]-2,3-dihydroxypropan-1-one trifluoroacetic acid) as an off-white solid (36.5 mg, 37% overall two steps): LCMS (ESI) calc'd for C₁₆H₂₁ClF₂N₂O₄ [M + H]⁺ 379, 381 (3 : 1), found 379, 381 (3 : 1); ¹H NMR (400 MHz, CD₃OD) δ 7.43 (s, 1H), 7.23 (s, 1H), 7.00 (t, *J* = 54.7 Hz, 1H), 4.71-4.39 (m, 2H), 4.31-4.02 (m, 2H), 3.75-3.57 (m, 2H), 3.08 (dt, *J* = 44.4, 12.6 Hz, 1H), 2.67 (dt, *J* = 44.8, 12.9 Hz, 1H), 2.49-2.34 (m, 1H), 2.09-1.95 (m, 1H), 1.49-1.11 (m, 3H); ¹⁹F NMR (376 MHz, CD₃OD) δ -77.27, -117.12.

### Example 205. Compound 228 (5-[4-[(R)-amino(5-chloro-2-hydroxy-4-methylphenyl)methyl]piperidine-1-carbonyl]-1H-pyridin-2-one)

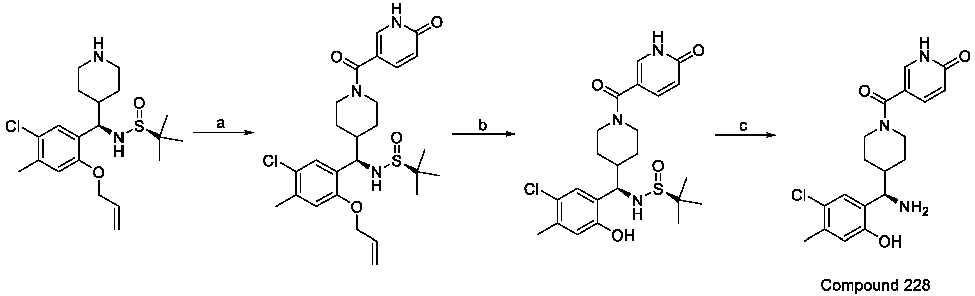

### Step a:

To a stirred mixture of 6-oxo-1*H*-pyridine-3-carboxylic acid (0.10 g, 0.75 mmol), HOBt (0.14 g, 1.00 mmol), EDCI (0.19 g, 1.00 mmol) in DMF (2 mL) were added (*S*)*-N-*[(*R*)-[5-chloro-4-methyl-2-(prop-2-en-1-yloxy)phenyl](piperidin-4-yl)methyl]-2-methylpropane-2-sulfinamide (0.20 g, 0.50 mmol) and Et₃N (0.15 g, 1.50 mmol) at room temperature. The resulting mixture was stirred for 2 h at room temperature. The reaction was quenched with water (40 mL) and extracted with EA (3 x 30 mL). The combined organic layers were washed with brine (2 x 10 mL), dried over anhydrous Na₂SO₄. After filtration, the filtrate was concentrated under reduced pressure. The residue was purified by reverse phase chromatography, eluted with 70% ACN in water (plus 0.05% TFA) to afford *(S)-N-[(R)-[5-*chloro-4-methyl-2-(prop-2-en-1-yloxy)phenyl] [1-(6-oxo-1*H*-pyridine-3-carbonyl)piperidin-4-yl]methyl]-2-methylpropane-2-sulfinamide as a light yellow solid (0.10 g, 31%): LCMS (ESI) calc'd for C₂₆H₃₄ClN₃O₄S [M + H]⁺: 520, 522 (3 : 1), found 520, 522 (3 : 1); ¹H NMR (400 MHz, CD₃Cl) δ 7.59 (s, 1H), 7.52 (d, *J* = 10.0 Hz, 1H), 7.10 (s, 1H), 6.76 (s, 1H), 6.57 (d, *J* = 9.4 Hz, 1H), 6.13-5.99 (m, 1H), 5.38 (dd, *J* = 28.6, 13.9 Hz, 2H), 4.66-4.48 (m, 2H), 4.46-4.09 (m, 3H), 2.95-2.66 (m, 2H), 2.36 (s, 3H), 2.23-1.95 (m, 2H), 1.53-1.19 (m, 3H), 1.15 (s, 9H).

### Step b:

To a stirred solution of (*S*)-*N*-[(*R*)-[5-chloro-4-methyl-2-(prop-2-en-1-yloxy)phenyl][1-(6-oxo-1*H*-pyridine-3-carbonyl)piperidin-4-yl]methyl]-2-methylpropane-2-sulfinamide (0.10 g, 0.19 mmol) and Pd(PPh₃)₄ (2 mg, 0.002 mmol) in THF (2 mL) was added NaBH₄ (15 mg, 0.39 mmol) at room temperature. The resulting mixture was stirred for 1 h at room temperature. The resulting mixture was quenched with saturated aq. NH₄Cl (1 mL) and concentrated under reduced pressure to afford (*S*)-*N*-[(*R*)-(5-chloro-2-hydroxy-4-methylphenyl)[1-(6-oxo-1*H*-pyridine-3-carbonyl)piperidin-4-yl]methyl]-2-methylpropane-2-sulfinamide as a brown solid (90 mg, crude), which was used in the next step directly without further purification: LCMS (ESI) calc'd for C₂₃H₃₀ClN₃O₄S [M + H]⁺: 480, 482 (3 : 1), found 480, 482 (3 : 1).

### Step c:

To a stirred mixture of (*S*)-*N*-[(*R*)-(5-chloro-2-hydroxy-4-methylphenyl)[1-(6-oxo-1*H*-pyridine-3-carbonyl)piperidin-4-yl]methyl]-2-methylpropane-2-sulfinamide (90 mg, 0.18 mmol) in 1,4-dioxane (1 mL) was added aq. HCl (4 *N,* 1 mL) at room temperature. The resulting mixture was stirred for 1 h at room temperature. The resulting mixture was concentrated under reduced pressure. The residue was purified by Prep-HPLC with the following conditions: Column: XBridge Shield RP18 OBD Column, 30 x 150 mm, 5 µm; Mobile Phase A: Water with 10 mmol/L NH₄HCO₃, Mobile Phase B: ACN; Flow rate: 60 mL/min; Gradient: 10% B to 40% B in 7 min; Detector: UV 254/220 nm; Retention time: 6.50 min. The fractions containing the desired product were collected and concentrated under reduced pressure to afford Compound 228 (5-[4-[(*R*)-amino(5-chloro-2-hydroxy-4-methylphenyl)methyl]piperidine-1-carbonyl]-1*H*-pyridin-2-one) as an off-white solid (38 mg, 53% overall two steps): LCMS (ESI) calc'd for C₁₉H₂₂ClN₃O₃ [M + H]⁺: 376, 378 (3 : 1), found 376, 378 (3 : 1); ¹H NMR (400 MHz, CD₃OD) δ 7.68-7.56 (m, 2H), 7.10 (s, 1H), 6.69 (s, 1H), 6.55 (d, *J* = 9.2 Hz, 1H), 4.55-3.97 (m, 2H), 3.88 (d, *J* = 7.7 Hz, 1H), 3.11-2.79 (m, 2H), 2.27 (s, 3H), 2.07-1.93 (m, 2H), 1.52-1.40 (m, 1H), 1.39-1.16 (m, 2H).

### Example 206. Compound 229 ((6S)-6-[4-[(R)-amino(4,5-dichloro-2-hydroxyphenyl)methyl]piperidine-1-carbonyl]morpholin-3-one trifluoroacetic acid)

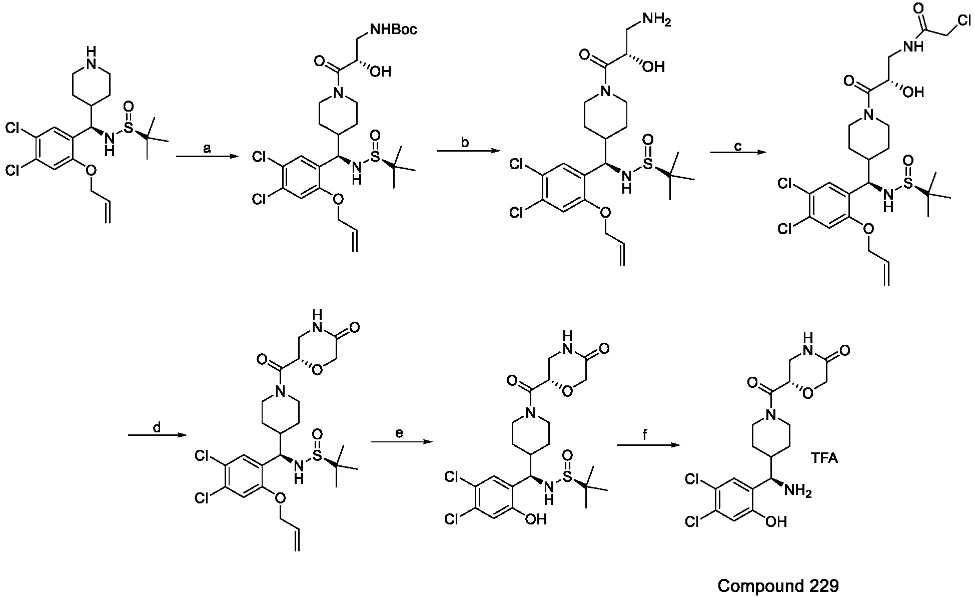

### Step a:

To a stirred solution of (2*S*)-3-[(*tert*-butoxycarbonyl)amino]-2-hydroxypropanoic acid (0.44 g, 2.14 mmol) and HATU (0.81 g, 2.14 mmol) in DMF (5 mL) were added (*S*)*-N-*[(*R*)-[4,5-dichloro-2-(prop-2-en-1-yloxy)phenyl](piperidin-4-yl)methyl]-2-methylpropane-2-sulfinamide (0.60 g, 1.43 mmol) and Et₃N (0.43 g, 4.29 mmol) at room temperature. The resulting solution was stirred for 1 h at room temperature. The reaction was purified by reverse phase chromatography, eluted with 60% ACN in water (plus 0.05% TFA) to afford *tert*-butyl *N-*[(2*S*)-3-[4-[(*R*)-[4,5-dichloro-2-(prop-2-en-1-yloxy)phenyl]([[(*S*)-2-methylpropane-2-sulfinyl]amino])methyl]piperidin-1-yl]-2-hydroxy-3-oxopropyl]carbamate as a yellow oil (0.54 g, 62%): LCMS (ESI) calc'd for C₂₇H₄₁Cl₂N₃O₆S [M + H]⁺: 606, 608 (3 : 2), found 606, 608 (3 : 2); ¹H NMR (400 MHz, CDCl₃) δ 7.22 (d, *J* = 13.9 Hz, 1H), 6.98 (s, 1H), 6.10-5.96 (m, 1H), 5.40 (dd, *J* = 19.7, 14.3 Hz, 2H), 4.67-4.50 (m, 3H), 4.50-4.37 (m, 2H), 4.03-3.88 (m, 1H), 3.48-3.34 (m, 1H), 3.17-2.88 (m, 2H), 2.63-2.50 (m, 1H), 2.20-2.12 (m, 1H), 2.04-1.93 (m, 1H), 1.64-1.54 (m, 1H), 1.48-1.39 (m, 11H), 1.17 (d, *J* = 4.8 Hz, 9H).

### Step b:

To a stirred solution of *tert*-butyl *N*-[(2*S*)-3-[4-[(*R*)-[4,5-dichloro-2-(prop-2-en-1-yloxy)phenyl]([[(*S*)-2-methylpropane-2-sulfinyl]amino])methyl]piperidin-1-yl]-2-hydroxy-3-oxopropyl]carbamate (0.54 g, 0.89 mmol) in DCM (5 mL) was added TFA (1 mL) at room temperature. The resulting solution was stirred for 0.5 h at room temperature. The reaction was neutralized with saturate aq. NaHCO₃ (20 mL) to pH 7. The aqueous layer was extracted with EA (3 x 50 mL). The combined organic layers were washed with brine (3 x 20 mL) and dried over anhydrous Na₂SO₄. After filtration, the filtrate was concentrated under reduced pressure to afford (*S*)-*N*-[(*R*)-[1-[(2*S*)-3-amino-2-hydroxypropanoyl]piperidin-4-yl][4,5-dichloro-2-(prop-2-en-1-yloxy)phenyl]methyl]-2-methylpropane-2-sulfinamide as a light yellow oil (0.43 g, crude), which was used in the next step directly without further purification: LCMS (ESI) calc'd for C₂₂H₃₃Cl₂N₃O₄S [M + H]⁺: 506, 508 (3 : 2), found 506, 508 (3 : 2); ¹H NMR (400 MHz, CDCl₃) δ 7.26 (s, 1H), 7.00-6.90 (m, 1H), 6.10-5.96 (m, 1H), 5.47-5.33 (m, 2H), 4.97-4.72 (m, 1H), 4.65-4.45 (m, 3H), 4.45-4.23 (m, 1H), 4.20-3.91 (m, 2H), 3.20 (d, *J* = 45.5 Hz, 2H), 3.07-2.85 (m, 1H), 2.62-2.44 (m, 1H), 2.03-1.88 (m, 1H), 1.80-1.59 (m, 2H), 1.51-1.33 (m, 1H), 1.11 (d, *J* = 11.1 Hz, 9H).

### Step c:

To a stirred solution of (*S*)-*N*-[(*R*)-[1-[(2*S*)-3-amino-2-hydroxypropanoyl]piperidin-4-yl][4,5-dichloro-2-(prop-2-en-1-yloxy)phenyl]methyl]-2-methylpropane-2-sulfinamide (0.20 g, 0.39 mmol) and Et₃N (0.12 g, 1.18 mmol) in DCM (2 mL) was added chloroacetyl chloride (45 mg, 0.39 mmol) dropwise at 0 °C. The resulting mixture was stirred for 1 h at 0 °C .The resulting mixture was diluted with water (30 mL) and extracted with EA (3 x 30 mL). The combined organic layers were washed with brine (3 x 20 mL) and dried over anhydrous Na₂SO₄. After filtration, the filtrate was concentrated under reduced pressure to afford 2-chloro-*N*-[(2*S*)-3-[4-[(*R*)-[4,5-dichloro-2-(prop-2-en-1-yloxy)phenyl]([[(*S*)-2-methylpropane-2-sulfinyl]amino])methyl]piperidin-1-yl]-2-hydroxy-3-oxopropyl]acetamide as a yellow green oil (0.20 g, crude), which was used in the next step directly without further purification: LCMS (ESI) calc'd C₂₄H₃₄Cl₃N₃O₅S for [M + H]⁺: 582, 584, 586 (3 : 3 : 1), found 582, 584, 586 (3 : 3 : 1).

### Step d:

To a stirred solution of 2-chloro-*N*-[(2*S*)-3-[4-[(*R*)-[4,5-dichloro-2-(prop-2-en-1-yloxy)phenyl]([[(*S*)-2-methylpropane-2-sulfinyl]amino])methyl]piperidin-1-yl]-2-hydroxy-3-oxopropyl]acetamide (0.20 g, 0.34 mmol) and t-BuOK (77 mg, 0.68 mmol) in THF (2 mL) at room temperature. The resulting mixture was stirred for 1 h at room temperature. The reaction mixture was diluted with water (30 mL) and extracted with EA (3 x 20 mL). The combined organic layers were washed with brine (3 x 20 mL) and dried over anhydrous Na₂SO₄. After filtration, the filtrate was concentrated under reduced pressure. The residue was purified by reverse phase chromatography, eluted with 50% ACN in water (plus 0.05% TFA) to afford (S)-*N*-[(*R*)-[4,5-dichloro-2-(prop-2-en-1-yloxy)phenyl]([1-[(2*S*)-5-oxomorpholine-2-carbonyl]piperidin-4-yl])methyl]-2-methylpropane-2-sulfinamide as a light yellow oil (70 mg, 32% overall two steps): LCMS (ESI) calc'd for C₂₄H₃₃Cl₂N₃O₅S for [M + H]⁺: 546, 548 (3 : 2), found 546, 548 (3 : 2); ¹H NMR (400 MHz, CDCl₃) δ 7.22 (d, *J* = 9.2 Hz, 1H), 6.98 (s, 1H), 6.10-5.97 (m, 2H), 5.46-5.34 (m, 2H), 4.68-4.46 (m, 3H), 4.39 (t, *J* = 8.4 Hz, 1H), 4.35-4.28 (m, 1H), 4.27-4.18 (m, 1H), 4.09-3.99 (m, 1H), 3.99-3.83 (m, 1H), 3.82-3.62 (m, 1H), 3.52-3.40 (m, 1H), 3.05 (dt, *J* = 43.8, 12.5 Hz, 1H), 2.55 (dt, *J* = 40.6, 12.6 Hz, 1H), 2.28-2.17 (m, 1H), 2.05-1.96 (m, 1H), 1.74-1.51 (m, 2H), 1.46-1.34 (m, 1H), 1.21-1.10 (m, 9H).

### Step e:

To a stirred solution of (*S*)-*N*-[(*R*)-[4,5-dichloro-2-(prop-2-en-1-yloxy)phenyl]([1-[(2*S*)-5-oxomorpholine-2-carbonyl]piperidin-4-yl])methyl]-2-methylpropane-2-sulfinamide (70 mg, 0.13 mmol) and Pd(PPh₃)₄ (1 mg, 0.001 mmol) in THF (1 mL) was added NaBH₄ (9 mg, 0.25 mmol) at room temperature. The resulting mixture was stirred for 30 min at room temperature. The reaction was quenched with saturate aq. NH₄Cl (1 mL) at room temperature. The resulting mixture was concentrated under reduced pressure to afford (*S*)*-N-*[(*R*)-(4,5-dichloro-2-hydroxyphenyl)([1-[(2*S*)-5-oxomorpholine-2-carbonyl]piperidin-4-yl])methyl]-2-methylpropane-2-sulfinamide (70 mg, crude), which was used in the next step directly without further purification: LCMS (ESI) calc'd for C₂₁H₂₉Cl₂N₃O₅S for [M + H]⁺: 506, 508 (3 : 2), found 506, 508 (3 : 2).

### Step f:

To a stirred mixture of (*S*)-*N*-[(*R*)-(4,5-dichloro-2-hydroxyphenyl)([1-[(2*S*)-5-oxomorpholine-2-carbonyl]piperidin-4-yl])methyl]-2-methylpropane-2-sulfinamide (70 mg, 0.14 mmol) in 1,4-dioxane (1 mL) was added aq. HCl (4 *N,* 1 mL) at room temperature. The resulting mixture was stirred for 30 min at room temperature. The resulting mixture was concentrated under reduced pressure. The residue was purified by Prep-HPLC with the following conditions: Column: Xselect CSH OBD Column 30 x 150 mm 5 µm, n; Mobile Phase A: Water (plus 0.05% TFA), Mobile Phase B: ACN; Flow rate: 60 mL/min; Gradient: 5% B to 25% B in 7 min; Detector: UV 220/254 nm; Retention time: 6.55 min. The fractions containing the desired product were collected and concentrated under reduced pressure to afford Compound 229 ((6*S*)-6-[4-[(*R*)-amino(4,5-dichloro-2-hydroxyphenyl)methyl]piperidine-1-carbonyl]morpholin-3-one trifluoroacetic acid) as an off-white solid (10 mg, 15% overall two steps): LCMS (ESI) calc'd for C₁₇H₂₁Cl₂N₃O₄ for [M + H]⁺: 402, 404 (3 : 2), found 402, 404 (3 : 2); ¹H NMR (400 MHz, CD₃OD) δ 7.43 (s, 1H), 7.09 (s, 1H), 4.72-4.36 (m, 2H), 4.33-4.24 (m, 1H), 4.24-3.99 (m, 3H), 3.76-3.58 (m, 1H), 3.43-3.36 (m, 1H), 3.18-3.04 (m, 1H), 2.68 (dt, *J* = 57.8, 12.8 Hz, 1H), 2.48-2.28 (m, 1H), 2.09-1.97 (m, 1H), 1.52-1.00 (m, 3H); ¹⁹F NMR (376 MHz, CD₃OD) δ -77.08.

### Example 207. Compound 231 (6-[4-[(R)-amino(4,5-dichloro-2-hydroxyphenyl)methyl]piperidine-1-carbonyl]morpholin-3-one)

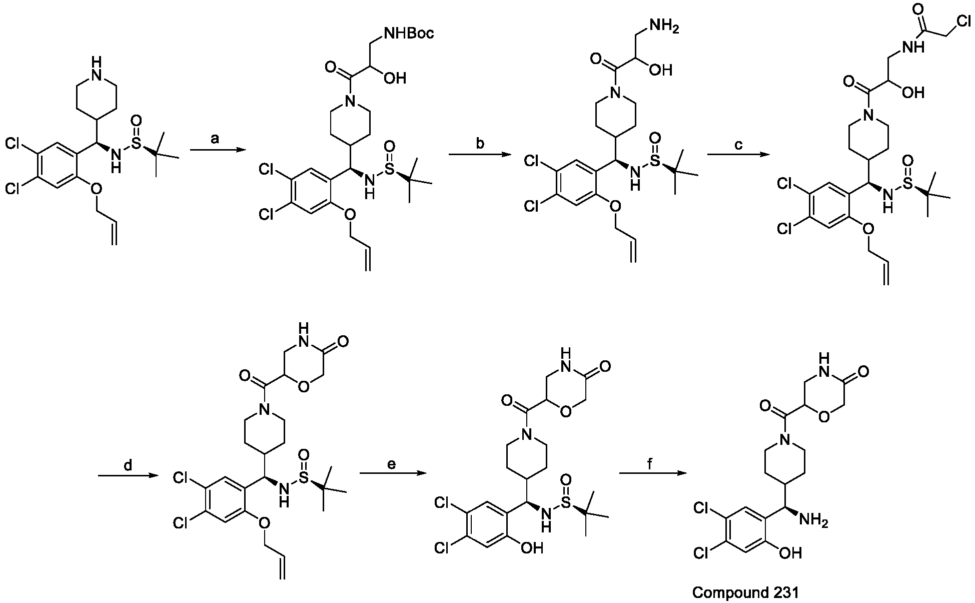

### Step a:

To a stirred solution of 3-[(*tert*-butoxycarbonyl)amino]-2-hydroxypropanoic acid (0.59 g, 2.88 mmol) and HATU (1.09 g, 2.87 mmol) in DMF (10 mL) was added (*S*)*-N-*[(*R*)-[4,5-dichloro-2-(prop-2-en-1-yloxy)phenyl](piperidin-4-yl)methyl]-2-methylpropane-2-sulfinamide (1.00 g, 2.38 mmol) and Et₃N (0.72 g, 7.15 mmol) at room temperature. The resulting mixture was stirred for 1 h at room temperature. The reaction was purified by reverse phase chromatography, eluted with 60% ACN in water (plus 0.05% TFA) to afford *tert*-butyl *N-*(3-[4-[(*R*)-[4,5-dichloro-2-(prop-2-en-1-yloxy)phenyl]([[(*S*)-2-methylpropane-2-sulfinyl]amino])methyl]piperidin-1-yl]-2-hydroxy-3-oxopropyl)carbamate as a light yellow oil (0.80 g, 44%): LCMS (ESI) calc'd for C₂₇H₄₁Cl₂N₃O₆S [M + H]⁺: 606, 608 (3 : 2), found 606, 608 (3 : 2); ¹H NMR (400 MHz, CDCl₃) δ 7.26-7.18 (m, 1H), 6.98 (s, 1H), 6.11-5.96 (m, 1H), 5.47-5.32 (m, 2H), 4.71-4.50 (m, 2H), 4.50-4.37 (m, 2H), 4.06-3.77 (m, 3H), 3.48-3.34 (m, 1H), 3.19-2.90 (m, 2H), 2.66-2.50 (m, 1H), 2.21-1.96 (m, 1H), 1.63-1.48 (m, 2H), 1.48-1.41 (m, 10H), 1.19-1.15 (m, 9H).

### Step b:

To a stirred solution of *tert*-butyl *N*-(3-[4-[(*R*)-[4,5-dichloro-2-(prop-2-en-1-yloxy)phenyl]([[(*S*)-2-methylpropane-2-sulfinyl]amino])methyl]piperidin-1-yl]-2-hydroxy-3-oxopropyl)carbamate (0.80 g, 1.32 mmol) in DCM (8 mL) was added TFA (2 mL) at room temperature. The resulting solution was stirred for 30 min at room temperature. The reaction was neutralized with saturated aq. NaHCO₃ (20 mL) to pH 7 at 0 °C. The resulting mixture was extracted with EA (3 x 30 mL). The combined organic layers were washed with brine (3 x 20 mL) and dried over anhydrous Na₂SO₄. After filtration, the filtrate was concentrated under reduced pressure to afford (*S*)-*N*-[(*R*)-[1-(3-amino-2-hydroxypropanoyl)piperidin-4-yl][4,5-dichloro-2-(prop-2-en-1-yloxy)phenyl]methyl]-2-methylpropane-2-sulfinamide as a light yellow solid (0.7 g, crude), which was used in the next step directly without further purification: LCMS (ESI) calc'd for C₂₃H₃₃Cl₂N₃O₄S [M + H]⁺: 506, 508 (3 : 2), found 506, 508 (3 : 2); ¹H NMR (400 MHz, CDCl₃) δ 7.22 (s, 1H), 6.98 (s, 1H), 6.09-5.96 (m, 1H), 5.45-5.32 (m, 2H), 4.68-4.23 (m, 5H), 4.12-3.84 (m, 1H), 3.38-3.07 (m, 2H), 3.07-2.90 (m, 1H), 2.64-2.41 (m, 1H), 2.09-1.96 (m, 2H), 1.59-1.20 (m, 3H), 1.13 (d, *J* = 8.3 Hz, 9H).

### Step c:

To a stirred solution of (*S*)-*N*-[(*R*)-[1-(3-amino-2-hydroxypropanoyl)piperidin-4-yl][4,5-dichloro-2-(prop-2-en-1-yloxy)phenyl]methyl]-2-methylpropane-2-sulfinamide (0.70 g, 1.38 mmol) and Et₃N (0.42 g, 4.15 mmol) in DCM (10 mL) was added chloroacetyl chloride (0.16 g, 1.38 mmol) dropwise at 0 °C. The resulting mixture was stirred for 1 h at 0 °C. The resulting mixture was diluted with water (20 mL) and extracted with EA (3 x 30 mL). The combined organic layers were washed with brine (3 x 30 mL) and dried over anhydrous Na₂SO₄. After filtration, the filtrate was concentrated under reduced pressure to afford 2-chloro-*N*-(3-[4-[(*R*)-[4,5-dichloro-2-(prop-2-en-1-yloxy)phenyl]([[(S)-2-methylpropane-2-sulfinyl]amino])methyl]piperidin-1-yl]-2-hydroxy-3-oxopropyl)acetamide as a light yellow oil (0.7 g, crude), which was used in the next step directly without further purification: LCMS (ESI) calc'd for C₂₄H₃₄Cl₃N₃O₅S [M + H]⁺: 582, 584, 586 (3 : 3 : 1), found 582, 584, 586 (3 : 3 : 1).

### Step d:

A mixture of 2-chloro-*N*-(3-[4-[(*R*)-[4,5-dichloro-2-(prop-2-en-1-yloxy)phenyl]([[(*S*)-2-methylpropane-2-sulfinyl]amino])methyl]piperidin-1-yl]-2-hydroxy-3-oxopropyl)acetamide (0.70 g, 1.20 mmol) and t-BuOK (0.27 g, 2.40 mmol) in THF (70 mL) was stirred for 1 h at room temperature. The resulting mixture was diluted with water (100 mL) and extracted with EA (3 x 50 mL). The combined organic layers were washed with brine (3 x 30 mL) and dried over anhydrous Na₂SO₄. After filtration, the filtrate was concentrated under reduced pressure. The residue was purified by reverse phase chromatography, eluted with 60% ACN in water (plus 0.05% TFA) to afford (*S*)-*N*-[(*R*)-[4,5-dichloro-2-(prop-2-en-1-yloxy)phenyl][1-(5-oxomorpholine-2-carbonyl)piperidin-4-yl]methyl]-2-methylpropane-2-sulfinamide as a light yellow oil (0.14 g, 19% overall three steps): LCMS (ESI) calc'd for C₂₄H₃₃Cl₂N₃O₅S [M + H]⁺: 546, 548 (3 : 2), found 546, 548 (3 : 2); ¹H NMR (400 MHz, CDCl₃) δ 7.26-7.17 (m, 1H), 6.99 (s, 1H), 6.48-6.33 (m, 1H), 6.10-5.94 (m, 1H), 5.48-5.34 (m, 2H), 4.68-4.49 (m, 3H), 4.47-4.19 (m, 3H), 4.10-3.97 (m, 1H), 3.97-3.82 (m, 2H), 3.52-3.41 (m, 1H), 3.17-2.87 (m, 1H), 2.64-2.44 (m, 1H), 2.28-1.91 (m, 2H), 1.62-1.24 (m, 3H), 1.19-1.14 (m, 9H).

### Step e:

To a stirred solution of (*S*)-*N*-[(*R*)-[4,5-dichloro-2-(prop-2-en-1-yloxy)phenyl][1-(5-oxomorpholine-2-carbonyl)piperidin-4-yl]methyl]-2-methylpropane-2-sulfinamide (0.10 g, 0.18 mmol) and Pd(PPh₃)₄ (4 mg, 0.004 mmol) in THF(4 mL) was added NaBH₄ (28 mg, 0.73 mmol) at room temperature. The resulting mixture was stirred for 30 min at room temperature. The resulting mixture was quenched with saturated aq. NH₄Cl (1 mL) and concentrated under reduced pressure to afford (*S*)-*N*-[(*R*)-(4,5-dichloro-2-hydroxyphenyl)[1-(5-oxomorpholine-2-carbonyl)piperidin-4-yl]methyl]-2-methylpropane-2-sulfinamide (0.20 g, crude), which was used in the next step directly without further purification: LCMS (ESI) calc'd for C₂₁H₂₉Cl₂N₃O₅S [M + H]⁺: 506, 508 (3 : 2), found 506, 508 (3 : 2).

### Step f:

To a stirred mixture of (*S*)-*N*-[(*R*)-(4,5-dichloro-2-hydroxyphenyl)[1-(5-oxomorpholine-2-carbonyl)piperidin-4-yl]methyl]-2-methylpropane-2-sulfinamide (0.10 g, 0.20 mmol) in 1,4-dioxane (1 mL) was added aq. HCl (4 *N,* 1 mL) at room temperature. The resulting mixture was stirred for 1 h at room temperature. The resulting mixture was concentrated under reduced pressure. The residue was purified by Prep-HPLC with the following conditions: Column: XBridge Shield RP18 OBD Column, 30 x 150 mm, 5 µm; Mobile Phase A: Water with 10 mmol/L NH₄HCO₃, Mobile Phase B: ACN; Flow rate: 60 mL/min; Gradient: 15% B to 40% B in 7 min; Detector: UV 254/220 nm; Retention time: 6.50 min. The fractions containing the desired product were collected and concentrated under reduced pressure to afford Compound 231 (6-[4-[(*R*)-amino(4,5-dichloro-2-hydroxyphenyl)methyl]piperidine-1-carbonyl]morpholin-3-one) as an off-white solid (7.5 mg, 10% overall two steps): LCMS (ESI) calc'd for C₁₇H₂₁Cl₂N₃O₄ [M + H]⁺: 402, 404 (3 : 2), found 402, 404 (3 : 2); ¹H NMR (400 MHz, CD₃OD) δ 7.23 (d, *J* = 5.6 Hz, 1H), 6.88 (s, 1H), 4.70-4.43 (m, 2H), 4.34-3.98 (m, 2H), 3.98-3.76 (m, 1H), 3.69-3.57 (m, 1H), 3.45-3.34 (m, 1H), 3.21-2.89 (m, 1H), 2.78-2.50 (m, 1H), 2.28-1.65 (m, 3H), 1.60-1.40 (m, 1H), 1.40-1.02 (m, 2H).

### Example 208. Compound 236 (2-[(R)-amino[1-(pyrrolidine-3-sulfonyl)piperidin-4-yl]methyl]-4-chloro-5-methylphenol trifluoroacetic acid)

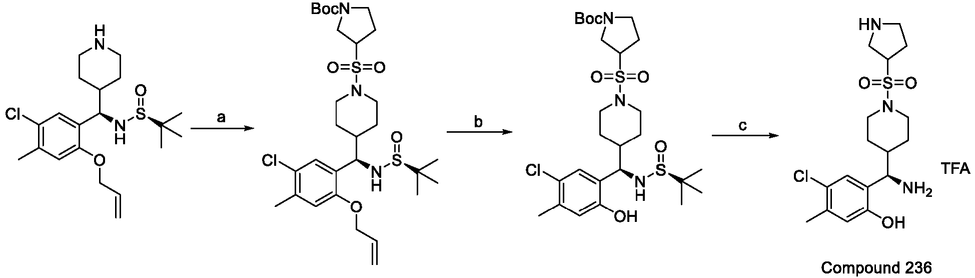

### Step a:

To a stirred solution of (*S*)-*N*-[(*R*)-[5-chloro-4-methyl-2-(prop-2-en-1-yloxy)phenyl](piperidin-4-yl)methyl]-2-methylpropane-2-sulfinamide (0.20 g, 0.50 mmol) and Et₃N (0.15 g, 1.50 mmol) in DCM (3 mL) was added *tert*-butyl 3-(chlorosulfonyl)pyrrolidine-1-carboxylate (0.16 g, 0.60 mmol) at 0 °C. The resulting mixture was stirred for 1 h at 0 °C. The reaction was quenched with water (20 mL) at room temperature and extracted with EA (3 x 20 mL). The combined organic layers were washed with brine (3 x 20 mL), dried over anhydrous Na₂SO₄. After filtration, the filtrate was concentrated under reduced pressure to afford *tert*-butyl 3-[4-[(*R*)-[5-chloro-4-methyl-2-(prop-2-en-1-yloxy)phenyl]([[(*S*)-2-methylpropane-2-sulfinyl]amino])methyl]piperidin-1-ylsulfonyl]pyrrolidine-1-carboxylate as a light yellow oil (0.25 g, crude), which was used in the next step directly without further purification: LCMS (ESI) calc'd for C₂₉H₄₆ClN₃O₆S₂ [M + H]⁺: 632, 634 (3 : 1), found 632, 634 (3 : 1); ¹H NMR (400 MHz, CD₃Cl) δ 7.07 (s, 1H), 6.75 (s, 1H), 6.15-5.89 (m, 1H), 5.46-5.29 (m, 2H), 4.65-4.47 (m, 2H), 4.42-4.23 (m, 1H), 4.04-3.75 (m, 2H), 3.75-3.52 (m, 4H), 3.48-3.32 (m, 1H), 2.90-2.59 (m, 2H), 2.36 (s, 3H), 2.30-2.14 (m, 2H), 2.02-1.67 (m, 2H), 1.53-1.40 (m, 10H), 1.40-1.20 (m, 2H), 1.14 (s, 9H).

### Step b:

To a stirred solution of *tert*-butyl 3-[4-[(*R*)-[5-chloro-4-methyl-2-(prop-2-en-1-yloxy)phenyl]([[(*S*)-2-methylpropane-2-sulfinyl]amino])methyl]piperidin-1-ylsulfonyl]pyrrolidine-1-carboxylate (0.25 g, 0.40 mmol) and Pd(PPh₃)₄ (5 mg, 0.004 mmol) in THF (3 mL) was added NaBH₄ (30 mg, 0.79 mmol) at room temperature. The resulting mixture was stirred for 1 h at room temperature. The resulting mixture was quenched with saturated aq. NH₄Cl (1 mL) and concentrated under reduced pressure to afford *tert*-butyl 3-[4-[(*R*)-(5-chloro-2-hydroxy-4-methylphenyl)([[(*S*)-2-methylpropane-2-sulfinyl]amino])methyl]piperidin-1-ylsulfonyl]pyrrolidine-1-carboxylate as a brown solid (0.25 g, crude), which was used in the next step directly without further purification: LCMS (ESI) calc'd for C₂₆H₄₂ClN₃O₆S₂ [M + H]⁺: 592, 594 (3 : 1), found 592, 594 (3 : 1).

### Step c:

To a stirred mixture of *tert*-butyl 3-[4-[(*R*)-(5-chloro-2-hydroxy-4-methylphenyl)([[(*S*)-2-methylpropane-2-sulfinyl]amino])methyl]piperidin-1-ylsulfonyl]pyrrolidine-1-carboxylate (0.25 g, 0.42 mmol) in 1,4-dioxane (1 mL) was added aq. HCl (4 *N,* 1 mL) at room temperature. The resulting mixture was stirred for 1 h at room temperature. The resulting mixture was concentrated under reduced pressure. The residue was purified by Prep-HPLC with the following conditions: Column: Xselect CSH OBD Column 30 x 150 mm 5 µm; Mobile Phase A: Water (plus 0.05% TFA), Mobile Phase B: ACN; Flow rate: 60 mL/min; Gradient: 7% B to 18% B in 7 min; Detector: UV 220/254 nm; Retention time: 6.92 min. The fractions containing the desired product were collected and concentrated under reduced pressure to afford Compound 236 (2-[(*R*)-amino[1-(pyrrolidine-3-sulfonyl)piperidin-4-yl]methyl]-4-chloro-5-methylphenol trifluoroacetic acid) as an off-white solid (81 mg, 32% overall three steps): LCMS (ESI) calc'd for C₁₇H₂₆ClN₃O₃S [M + H]⁺: 388, 390 (3 : 1), found 388, 390 (3 : 1); ¹H NMR (400 MHz, CD₃OD) δ 7.25 (s, 1H), 6.86 (s, 1H), 4.21-4.02 (m, 2H), 3.94 (d, *J* = 13.1 Hz, 1H), 3.79 (d, *J* = 12.9 Hz, 1H), 3.72-3.58 (m, 2H), 3.55-3.46 (m, 1H), 3.44-3.35 (m, 1H), 3.05-2.94 (m, 1H), 2.94-2.82 (m, 1H), 2.48-2.35 (m, 2H), 2.35-2.21 (m, 4H), 2.08-1.99 (m, 1H), 1.52-1.37 (m, 2H), 1.37-1.23 (m, 1H); ¹⁹F NMR (376 MHz, CD₃OD) δ - 77.17.

### Example 209. Compound 240 (2-[(R)-amino(1-[imidazo[1,2-a]pyrimidine-6-carbonyl]piperidin-4-yl)methyl]-4-chloro-5-methylphenol trifluoroacetic acid)

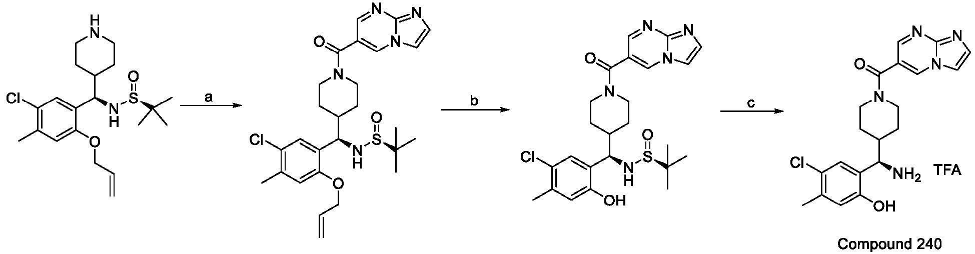

### Step a:

To a stirred solution of imidazo[1,2-*a*]pyrimidine-6-carboxylic acid (0.12 g, 0.75 mmol) and HATU (0.29 g, 0.75 mmol) in DMF (2 mL) were added (*S*)-*N*-[(*R*)-[5-chloro-4-methyl-2-(prop-2-en-1-yloxy)phenyl](piperidin-4-yl)methyl]-2-methylpropane-2-sulfinamide (0.20 g, 0.50 mmol) and Et₃N (0.15 g, 1.50 mmol) at room temperature. The reaction was stirred at room temperature for 0.5 h. The resulting mixture was diluted with EA (20 mL) and water (20 mL). The aqueous solution was extracted with EA (3 x 20 mL). The combined organic layers were washed with brine (5 x 20 mL), dried over anhydrous Na₂SO₄. After filtration, the filtrate was concentrated under reduced pressure to afford (*S*)-*N*-[(*R*)-[5-chloro-4-methyl-2-(prop-2-en-1-yloxy)phenyl](1-[imidazo[*1,2-a*]pyrimidine-6-carbonyl]piperidin-4-yl)methyl]-2-methylpropane-2-sulfinamideas a yellow foam (0.30 g, crude), which was used in the next step directly without further purification: LCMS (ESI) calc'd for C₂₇H₃₄ClN₅O₃S [M + H]⁺ 544, 546 (3 : 1), found 544, 546 (3 : 1); ¹H NMR (400 MHz, CDCl₃) δ 8.72 (d, *J* = 2.4 Hz, 1H), 8.59 (s, 1H), 7.91 (s, 1H), 7.66 (s, 1H), 7.09 (s, 1H), 6.77 (s, 1H), 6.12-5.99 (m, 1H), 5.48-5.28 (m, 2H), 4.68-4.49 (m, 2H), 4.42-4.27 (m, 1H), 4.10-3.97 (m, 2H), 3.14-2.97 (m, 1H), 2.45-2.25 (m, 4H), 2.21 (d, *J* = 13.1 Hz, 1H), 2.14-2.08 (m, 1H), 1.59-1.42 (m, 1H), 1.42-1.31 (m, 2H), 1.14 (s, 9H).

### Step b:

To a stirred solution of (*S*)-*N*-[(*R*)-[5-chloro-4-methyl-2-(prop-2-en-1-yloxy)phenyl](1-[imidazo[1,2-*a*]pyrimidine-6-carbonyl]piperidin-4-yl)methyl]-2-methylpropane-2-sulfinamide (0.15 g, 0.28 mmol) in HOAc (3 mL) was added Pd(PPh₃)₄ (0.16 g, 0.14 mmol) at room temperature. The reaction was stirred at room temperature for 16 h. The reaction was concentrated under reduced pressure to afford (*S*)-*N*-[(*R*)-(5-chloro-2-hydroxy-4-methylphenyl)(1-[imidazo[1,2-*a*]pyrimidine-6-carbonyl]piperidin-4-yl)methyl]-2-methylpropane-2-sulfinamide as a yellow solid (150 mg, crude), which was used in the next step directly without further purification: LCMS (ESI) calc'd for C₂₄H₃₀ClN₅O₃S [M + H]⁺: 504, 506 (3 : 1), found 504, 506 (3 : 1).

### Step c:

To a stirred solution of (*S*)-*N*-[(*R*)-(5-chloro-2-hydroxy-4-methylphenyl)(1-[imidazo[1,2-*a*]pyrimidine-6-carbonyl]piperidin-4-yl)methyl]-2-methylpropane-2-sulfinamide (0.15 g, 0.30 mmol) in 1,4-dioxane(3 mL) was added aq. HCl (4 *N*, 1 mL) at room temperature. The reaction was stirred at room temperature for 1 h. The reaction was diluted with EA (20 mL) and water (10 mL). The organic layers were washed with aq. HCl (1 *N,* 2 x 20 mL).The combined aqueous solution was concentrated under reduced pressure. The residue was purified by Prep-HPLC with following conditions: Column: XBridge Prep C18 OBD Column, 5 µm, 19 x 150 mm; Mobile Phase A: water (plus 0.05% TFA), Mobile Phase B: ACN; Flow rate: 25 mL/min; Gradient: 3% B to 29% B in 7 min; Detector: UV 254/220 nm; Retention time: 5.32 min. The fractions containing the desired product were collected and concentrated under reduced pressure to afford Compound 240 (2-[(*R*)-amino(1-[imidazo[1,2-*a*]pyrimidine-6-carbonyl]piperidin-4-yl)methyl]-4-chloro-5-methylphenol trifluoroacetic acid) as a light yellow solid (75 mg, 58% overall three steps): LCMS (ESI) calc'd for C₂₀H₂₂ClN₅O₂ [M + H]⁺: 400, 402 (3 : 1), found 400, 402; ¹H NMR (400 MHz, DMSO-*d*₆) δ 9.30 (s, 1H), 8.81 (d, *J* = 2.1 Hz, 1H), 8.09 (dd, *J* = 11.9, 2.0 Hz, 2H), 7.36 (s, 1H), 6.87 (s, 1H), 4.62-4.31(m, 1 H), 4.28-4.12 (m, 1H), 4.09-3.55 (m, 1H), 3.27-2.65 (m, 2H), 2.31-2.10 (m, 4H), 2.12-1.76 (m, 1H), 1.48-1.00 (m, 3H); ¹⁹F NMR (376 MHz, CD₃OD) δ -77.22.

The compounds in Table 1e below were prepared in an analogous fashion to that described for Compound 240, starting from (*S*)-*N*-[(*R*)-[5-chloro-4-methyl-2-(prop-2-en-1-yloxy)phenyl](piperidin-4-yl)methyl]-2-methylpropane-2-sulfinamide and the corresponding acids, which were prepared as described herein, or which were available from commercial sources. Compounds in Table 1e marked with a * are reference compounds.

**Table 1e**

| **Compound Number** | **Structure** | **Chemical Name** | **MS & NMR** |
|---|---|---|---|
| **239** | 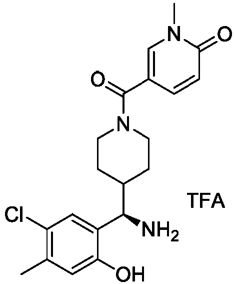 | 5-[4-[(*R*)-amino(5-chloro-2-hydroxy-4-methylphenyl)methyl] piperidine-1-carbonyl]-1-methylpyridin-2(1*H*)-one trifluoroacetic acid | [M + H]⁺: 390, 392 (3 : 1); ¹H NMR (400 MHz, DMSO-*d*₆) δ 7.89 (d, *J* = 2.5 Hz, 1H), 7.46 (dd, *J* = 9.4, 2.5 Hz, 1H), 7.31 (s, 1H), 6.85 (s, 1H), 6.42 (d, *J* = 9.3 Hz, 1H), 4.40-3.82 (m, 3H), 3.44 (s, 3H), 3.02-2.70 (m, 2H), 2.23 (s, 3H), 2.18-2.01 (m, 1H), 1.87 (d, *J* = 12.9 Hz, 1H), 1.31-0.96 (m, 3H); ¹⁹F NMR (376 MHz, CD₃OD) δ -77.02. |
| **241** | 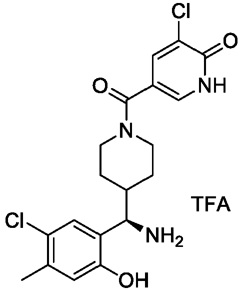 | 5-[4-[(*R*)-amino(5-chloro-2-hydroxy-4-methylphenyl)methyl] piperidine-1-carbonyl]-3-chloro-1*H-*pyridin-2-one trifluoroacetic acid | [M + H]⁺: 410, 412 (3 : 2); ¹H NMR (400 MHz, CD₃OD) δ 7.84 (d, *J* = 2.3 Hz, 1H), 7.63 (d, *J* = 2.3 Hz, 1H), 7.25 (s, 1H), 6.85 (s, 1H), 4.56-4.19 (m, 2H), 4.14 (d, *J* = 9.8 Hz, 1H), 3.15-2.86 (m, 2H), 2.50-2.33 (m, 1H), 2.32 (s, 3H), 2.03 (d, *J* = 13.0 Hz, 1H), 1.48-1.34 (m, 2H), 1.34-1.12 (m, 1H); ¹⁹F NMR (376 MHz, CD₃OD) δ -77.22. |
| **243** | 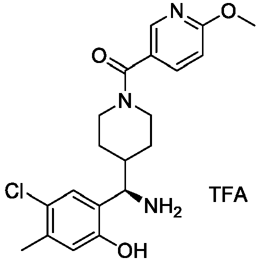 | 2-[(*R*)-amino[1-(6-methoxypyridine-3-carbonyl)piperidin-4-yl]methyl]-4-chloro-5-methylphenol trifluoroacetic acid | [M + H]⁺: 390, 392 (3 : 1); ¹H NMR (400 MHz, CD₃OD) δ 8.25 (d, *J* = 2.4 Hz, 1H), 7.74 (dd, *J* = 8.7, 2.4 Hz, 1H), 7.25 (s, 1H), 6.97-6.75 (m, 2H), 4.80-4.45 (m, 1H), 4.22-4.07 (m, 1H), 3.97 (s, 3H), 3.87-3.69 (m, 1H), 3.10-2.72 (m, 2H), 2.53-2.36 (m, 1H), 2.32 (s, 3H), 2.14-1.92 (m, 1H), 1.53-1.18 (m, 3H); ¹⁹F NMR (376 MHz, CD₃OD) δ -77.34. |
| **245** | 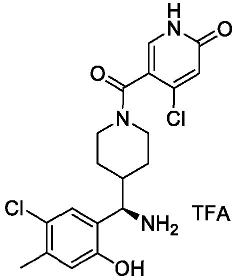 | 5-[4-[(*R*)-amino(5-chloro-2-hydroxy-4-methylphenyl)methyl]piperidi ne-1-carbonyl]-4-chloro-1*H-*pyridin-2-one trifluoroacetic acid | [M + H]⁺: 410, 412 (3 : 2); ¹H NMR (400 MHz, CD₃OD) δ 7.70-7.45 (m, 1H), 7.25 (d, *J* = 6.4 Hz, 1H), 6.85 (d, *J* = 6.7 Hz, 1H), 6.69 (d, *J* = 9.6 Hz, 1H), 4.66 (dd, *J* = 55.4, 13.7 Hz, 1H), 4.24-4.04 (m, 1H), 3.81-3.53 (m, 1H), 3.27-2.99 (m, 1H), 2.82 (dt, *J* = 45.9, 13.0 Hz, 1H), 2.47-2.23 (m, 4H), 2.20-1.87 (m, 1H), 1.58-1.08 (m, 3H); ¹⁹F NMR (376 MHz, CD₃OD) δ-77.32. |
| **248** | 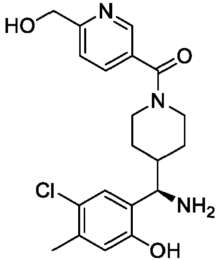 | 2-[(*R*)-amino([1-[6-(hydroxymethyl)pyridine-3-carbonyl]piperidin-4-yl])methyl]-4-chloro-5-methylphenol | [M + H]⁺: 390, 392 (3 : 1); ¹H NMR (400 MHz, CD₃OD) δ 8.53 (s, 1H), 7.89 (d, *J* = 7.9 Hz, 1H), 7.65 (d, *J* = 8.0 Hz, 1H), 7.12 (s, 1H), 6.71 (s, 1H), 4.81-4.54 (m, 3H), 3.93 (d, *J* = 8.0 Hz, 1H), 3.82-3.63 (m, 1H), 3.22-3.01 (m, 1H), 2.96-2.70 (m, 1H), 2.27 (s, 3H), 2.15-1.86 (m, 2H), 1.59-1.15 (m, 3H). |
| **249** | 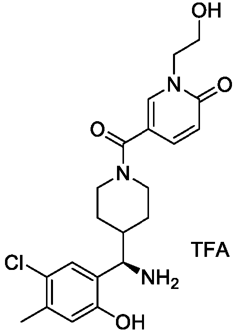 | 5-[4-[(*R*)-amino(5-chloro-2-hydroxy-4-methylphenyl)methyl]piperidi ne-1-carbonyl]-1-(2-hydroxyethyl)pyridin-2-one trifluoroacetic acid | [M + H]⁺: 420, 422 (3 : 1); ¹H NMR (400 MHz, CD₃OD) δ 7.87 (d, *J* = 2.5 Hz, 1H), 7.60 (dd, *J* = 9.3, 2.5 Hz, 1H), 7.25 (s, 1H), 6.85 (s, 1H), 6.58 (d*, J* = 9.4 Hz, 1H), 4.53-3.99 (m, 5H), 3.83 (t, *J* = 5.0 Hz, 2H), 3.12-2.87 (m, 2H), 2.50-2.36 (m, 1H), 2.32 (s, 3H), 2.03 (d, *J* = 13.0 Hz, 1H), 1.47-1.33 (m, 2H), 1.33-1.16 (m, 1H); ¹⁹F NMR (376 MHz, CD₃OD) δ -77.17 |
| **256** | 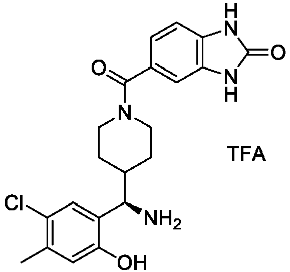 | 5-[4-[(*R*)-amino(5-chloro-2-hydroxy-4-methylphenyl)methyl]piperidi ne-1-carbonyl]-1,3-dihydro-1,3-benzodiazol-2-one trifluoroacetic acid | [M + H]⁺: 415, 417 (3 : 1); ¹H NMR (400 MHz, CD₃OD) δ 7.25 (s, 1H), 7.11 (d, *J* = 2.5 Hz, 3H), 6.85 (s, 1H), 4.75-4.44 (brs, 1H), 4.15 (d, *J* = 9.8 Hz, 1H), 4.02-3.76 (m, 1H), 3.13-2.82 (m, 2H), 2.41 (q, *J* = 11.2 Hz, 1H), 2.31 (s, 3H), 2.14-1.91 (m, 1H), 1.50-1.16 (m, 3H); ¹⁹F NMR (400 MHz, CD₃OD) δ -77.01. |
| **262** | 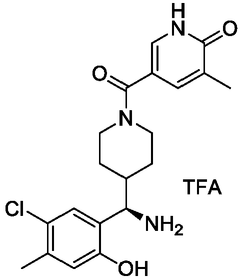 | 5-[4-[(*R*)-amino(5-chloro-2-hydroxy-4-methylphenyl)methyl]piperidi ne-1-carbonyl]-3-methyl-1*H-*pyridin-2-one trifluoroacetic acid | [M + H]⁺: 390, 392 (3 : 1); ¹H NMR (400 MHz, CD₃OD) δ 7.56-7.44 (m, 2H), 7.25 (s, 1H), 6.85 (s, 1H), 4.54-3.99 (m, 3H), 3.15-2.85 (m, 2H), 2.48-2.33 (m, 1H), 2.32 (s, 3H), 2.13 (s, 3H), 2.02 (d, *J* = 13.2 Hz, 1H), 1.49-1.34 (m, 2H), 1.34-1.15 (m, 1H); ¹⁹F NMR (376 MHz, CD₃OD) δ -77.18. |
| **263** | 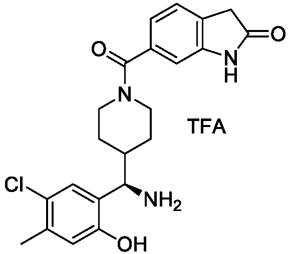 | 6-[4-[(*R*)-amino(5-chloro-2-hydroxy-4-methylphenyl)methyl] piperidine-1-carbonyl]-1,3-dihydroindol-2-one trifluoroacetic acid | [M + H]⁺: 414, 416 (3 : 1); ¹H NMR (400 MHz, CD₃OD) δ 7.33 *(d, J* = 7.5 Hz, 1H), 7.25 (s, 1H), 7.03 (d, *J* = 7.6 Hz, 1H), 6.90 (s, 1H), 6.84 (s, 1H), 4.67 (d, *J* = 55.5 Hz, 1H), 4.15 (d, *J* = 9.7 Hz, 1H), 3.83 (d, *J* = 52.8 Hz, 1H), 3.58 (s, 2H), 3.19-2.79 (m, 2H), 2.41 (d, *J* = 10.3 Hz, 1H), 2.31 (s, 3H), 2.16-1.87 (m, 1H), 1.36 (d, *J* = 57.8 Hz, 3H); ¹⁹F NMR (400 MHz, CD₃OD) δ - 77.19. |
| **267** | 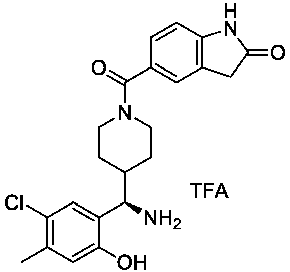 | 5-[4-[(*R*)-amino(5-chloro-2-hydroxy-4-methylphenyl)methyl] piperidine-1-carbonyl]-1,3-dihydroindol-2-one trifluoroacetic acid | [M + H]⁺: 414, 416 (3 : 1); ¹H NMR (400 MHz, CD₃OD) δ 7.34-7.28 (m, 2H), 7.25 (s, 1H), 6.96 (d, *J* = 7.9 Hz, 1H), 6.85 (s, 1H), 4.75-4.41 (m, 1H), 4.14 (d, *J* = 9.8 Hz, 1H), 4.04-3.69 (m, 1H), 3.58 (s, 2H), 3.23-2.71 (m, 2H), 2.48-2.33 (m, 1H), 2.32 (s, 3H), 2.13-1.85 (m, 1H), 1.55-1.08 (m, 3H); ¹⁹F NMR (376 MHz, CD₃OD) δ -77.18. |
| **269** | 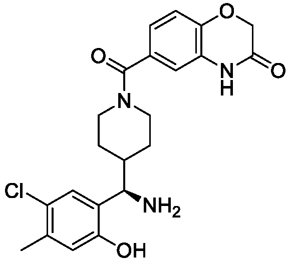 | 6-[4-[(*R*)-amino(5-chloro-2-hydroxy-4-methylphenyl)methyl] piperidine-1-carbonyl]-2,4-dihydro-1,4-benzoxazin-3-one | [M + H]⁺: 430, 432 (3 : 1); ¹H NMR (400 MHz, CD₃OD) δ 7.25 (s, 1H), 7.03 (d*, J* = 1.8 Hz, 2H), 6.96 (s, 1H), 6.85 (s, 1H), 4.71-4.53 (m, 3H), 4.14 (d, *J* = 9.7 Hz, 1H), 4.00-3.73 (m, 1H), 3.11-2.69 (m, 2H), 2.47-2.36 (m, 1H), 2.32 (s, 3H), 2.15-1.81 (m, 1H), 1.53-1.10 (m, 3H); |
| **273** | 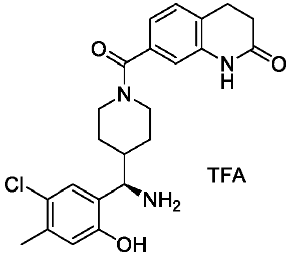 | 7-[4-[(*R*)-amino(5-chloro-2-hydroxy-4-methylphenyl)methyl]piperidi ne-1-carbonyl]-3,4-dihydro-1*H*-quinolin-2-one trifluoroacetic acid | [M + H]⁺: 428, 430 (3 : 1); ¹H NMR (400 MHz, CD₃OD) δ 7.29 (d, *J* = 7.8 Hz, 1H), 7.25 (s, 1H), 7.02 (d, *J* = 7.7 Hz, 1H), 6.89 (s, 1H), 6.85 (s, 1H), 4.67 (d, *J* = 68.8 Hz, 1H), 4.14 (d, *J* = 9.8 Hz, 1H), 3.83 (d, *J* = 48.9 Hz, 1H), 3.13-2.68 (m, 4H), 2.60 (t, *J* = 7.6 Hz, 2H), 2.46-2.36 (m, 1H), 2.32 (s, 3H), 2.00 (d, *J* = 63.2 Hz, 1H), 1.54-1.13 (m, 3H); ¹⁹F NMR (376 MHz, CD₃OD) δ - 76.99. |
| **280** | 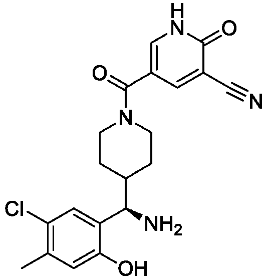 | (R)-5-(4-(amino(5-chloro-2-hydroxy-4-methylphenyl)methyl) piperidine-1-carbonyl)-2-oxo-1,2-dihydropyridine-3-carbonitrile | [M + H]⁺: 401, 403 (3: 1); ¹H NMR (400 MHz, CD₃OD) δ 8.09 (d, *J* = 2.8 Hz, 1H), 7.91 (d, *J* = 2.7 Hz, 1H), 7.19 (s, 1H), 6.92 (s, 1H), 4.33-4.17 (m, 2H), 4.04 (d, *J* = 9.0 Hz, 1H), 3.06-2.84 (m, 2H), 2.32-2.15 (m, 4H), 2.01 (d, *J* = 13.0 Hz, 1H), 1.49-1.16 (m, 3H). |
| **325** | 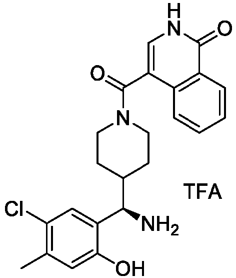 | 4-[4-[(*R*)-amino(5-chloro-2-hydroxy-4-methylphenyl)methyl]piperidi ne-1-carbonyl]-2*H-*isoquinolin-1-one trifluoroacetic acid | [M + H]⁺: 426, 428 (3 : 1); ¹H NMR (400 MHz, CD₃OD) δ 8.39 (d, *J* = 8.1 Hz, 1H), 7.81 (t, *J* = 7.6 Hz, 1H), 7.66-7.58 (m, 1H), 7.27-7.22 (m, 2H), 6.84 (s, 1H), 4.16 ( d, *J* = 9.7 Hz, 1H), 3.93 -3.59 (m, 2H), 3.04-3.00 (m, 2H), 2.40 (d, *J* = 1 1.5 Hz, 1H), 2.31 (s, 3H ), 2.17-1.81 (m, 2H), 1.3 3-1.29 (m, 3H); ¹⁹F NM R (376 MHz, CD₃OD) δ -77.22. |

### Example 210. Compound 244 ((2R)-1-[4-[(R)-amino(3-chloro-2-fluoro-6-hydroxy-4-methylphenyl)methyl]piperidin-1-yl]-2,3-dihydroxypropan-1-one)

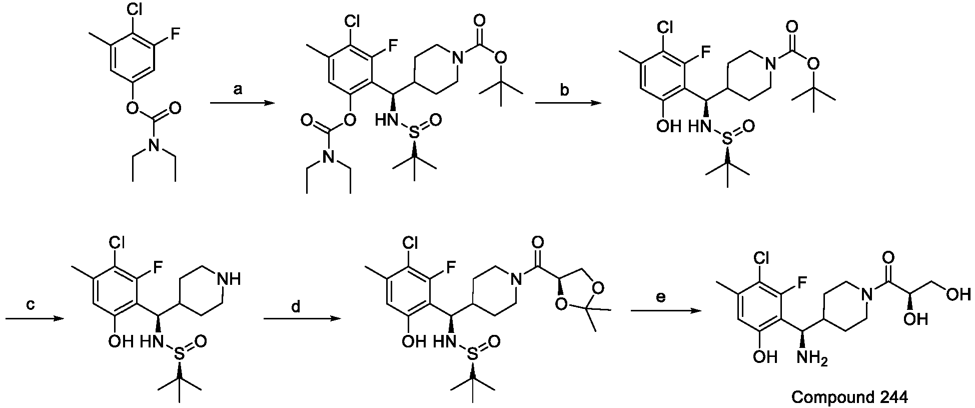

### Step a:

To a solution of *i*-Pr₂NH (8.98 g, 88.73 mmol) in THF (70 mL) was added *n*-BuLi (25.4 mL, 63.53 mmol, 2.5 M in hexane) dropwise at -65 °C over 15 min under nitrogen atmosphere. After stirring for 45 min, the reaction solution was cooled to -85 °C. And then a solution of 4-chloro-3-fluoro-5-methylphenyl *N*,*N*-diethylcarbamate (Intermediate 73, Example 73) (15.00 g, 57.76 mmol) in THF (90 mL) was added dropwise over 0.5 h at -85 °C under nitrogen atmosphere. After stirring at -85 °C for 40 min under nitrogen atmosphere, a solution of *tert*-butyl 4-[[(*S*)-2-methylpropane-2-sulfinyl]imino]methyl]piperidine-1-carboxylate (22.00 g, 69.52 mmol) in THF (90 mL) was added dropwise over 0.5 h at-80 °C. The resulting mixture was stirred under -65 °C for 1 h. The reaction was quenched with saturated aq. NH₄Cl (50 mL) at -65 °C and diluted with brine (100 mL). The resulting mixture was extracted with EA (3 x 100 mL). The combined organic layers were washed with brine (2 x 50 mL), dried over anhydrous Na₂SO₄. After filtration, the filtrate was concentrated under reduced pressure. The residue was purified with silica gel column chromatography, eluted with PE/EA (1/3) to afford *tert*-butyl 4-[(*R*)-[3-chloro-6-[(diethylcarbamoyl)oxy]-2-fluoro-4-methylphenyl]([[(*S*)-2-methylpropane-2-sulfinyl]amino])methyl]piperidine-1-carboxylate as an off-white foam (25 g, 75%): LCMS (ESI) calc'd for C₂₇H₄₃ClFN₃O₅S [M + H]⁺: 576, 578 (3 : 1), found 576, 578 (3 : 1); ¹H NMR (400 MHz, CDCl₃) δ 6.87 (s, 1H), 4.53-3.72 (m, 4H), 3.56-3.25 (m, 4H), 2.75 (s, 3H), 2.63 (d, *J* = 41.6 Hz, 2H), 2.26 (dd, *J* = 27.8, 17.3 Hz, 1H), 1.96 (s, 1H), 1.47 (d, *J* = 2.5 Hz, 9H), 1.34-1.17 (m, 9H), 1.10 (s, 9H); ¹⁹F NMR (376 MHz, CDCl₃) δ -115.04.

### Step b:

To a solution of *tert*-butyl 4-[(*R*)-[3-chloro-6-[(diethylcarbamoyl)oxy]-2-fluoro-4-methylphenyl]([[(*S*)-2-methylpropane-2-sulfinyl]amino])methyl]piperidine-1-carboxylate (25.00 g, 43.39 mmol) in MeOH (350 mL) was added NaOH (8.68 g, 216.95 mmol) at room temperature. The reaction mixture was stirred at 50 °C for 3 h. After cooling to room temperature, the resulting mixture was acidified with aq. HCl (1 *N*) to pH 7 and extracted with DCM (3 x 100 mL). The combined organic layers were washed with brine (2 x 30 mL), dried over anhydrous Na₂SO₄. After filtration, the filtrate was concentrated under reduced pressure to afford *tert*-butyl 4-[(*R*)-(3-chloro-2-fluoro-6-hydroxy-4-methylphenyl)([[(S)-2-methylpropane-2-sulfinyl]amino])methyl]piperidine-1-carboxylate as light yellow foam (20 g, crude): LCMS (ESI) calc'd for C₂₂H₃₄ClFN₂O₄S [M + H]⁺: 477, 479 (3 : 1), found 477, 479 (3 : 1).

### Step c:

To a solution of *tert*-butyl 4-[(*R*)-(3-chloro-2-fluoro-6-hydroxy-4-methylphenyl)([[(*S*)-2-methylpropane-2-sulfinyl]amino])methyl]piperidine-1-carboxylate (20.00 g, 41.93 mmol) in DCM (160 mL) was added TFA (40 mL) at 0 °C. The reaction mixture was allowed to warm to room temperature and stirred for 1.5 h. The resulting solution was neutralized with saturated aq. Na₂CO₃ at 0 °C to pH 8 and extracted with DCM (8 x 60 mL). The combined organic layers were combined, dried over anhydrous Na₂SO₄. After filtration, the filtrate was concentrated under reduced pressure to afford (*S*)-*N*-[(*R*)-(3-chloro-2-fluoro-6-hydroxy-4-methylphenyl)(piperidin-4-yl)methyl]-2-methylpropane-2-sulfinamide as a light yellow solid (20.00 g, 88%): LCMS (ESI) calc'd for C₁₇H₂₆ClFN₂₂S [M + H]⁺: 377, 379 (3 : 1), found 377, 379 (3 : 1).

### Step d:

To a stirred solution of HATU (1.66 g, 4.38 mmol) and (4*R*)-2,2-dimethyl-1,3-dioxolane-4-carboxylic acid (0.51 g, 3.50 mmol) in DMF (10 mL) were added Et₃N (0.89 g, 8.76 mmol) and a solution of (*S*)-*N*-[(*R*)-(3-chloro-2-fluoro-6-hydroxy-4-methylphenyl)(piperidin-4-yl)methyl]-2-methylpropane-2-sulfinamide (1.10 g, 2.92 mmol) in DMF (5 mL) at room temperature. The reaction solution was stirred at room temperature for 1 h. The resulting solution was quenched with water (80 mL) and extracted with EA (3 x 50 mL). The combined organic layers were washed with brine (5 x 50 mL), dried over anhydrous Na₂SO₄. After filtration, the filtrate was concentrated under reduced pressure to afford (*S*)-*N-*[(*R*)-(3-chloro-2-fluoro-6-hydroxy-4-methylphenyl)([1-[(2*R*)-2,3-dihydroxypropanoyl]piperidin-4-yl])methyl]-2-methylpropane-2-sulfinamide as a light yellow oil (1.5 g, crude): C₂₃H₃₄ClFN₂O₅S [M + H]⁺: 505, 507 (3 : 1), found 505, 507 (3 : 1).

### Step e:

To a solution of (*S*)-*N*-[(*R*)-(3-chloro-2-fluoro-6-hydroxy-4-methylphenyl)([1-[(4*R*)-2,2-dimethyl-1,3-dioxolane-4-carbonyl]piperidin-4-yl])methyl]-2-methylpropane-2-sulfinamide (1.50 g, 2.97 mmol) in THF (15 mL) was added aq. HCl (4 *N*, 5 mL) at 0 °C. The reaction solution was stirred at room temperature for 1 h. The resulting solution was concentrated under reduced pressure. The residue was purified with the following conditions: Column: XSelect CSH Prep C18 OBD Column, 5 µm, 19 x 150 mm; Mobile Phase A: water with 10 mmol/L NH₄HCO₃, Mobile Phase B: ACN; Flow rate: 25 mL/min; Gradient: 15% B to 20% B in 6 min; 210 nm; Detector: UV 254 nm; Retention time: 5.02 min. The fractions containing the desired product were collected and concentrated under reduced pressure to afford Compound 244 ((2*R*)-1-[4-[(*R*)-amino(3-chloro-2-fluoro-6-hydroxy-4-methylphenyl)methyl]piperidin-1-yl]-2,3-dihydroxypropan-1-one) as an off-white solid (296 mg, 28% overall two steps): LCMS (ESI) calc'd for C₁₆H₂₂ClFN₂O₄ [M + H]⁺: 361, 363 (3 : 1), found 361, 363 (3 : 1); ¹H NMR (400 MHz, CD₃OD) δ 6.52 (s, 1H), 4.67-4.44 (m, 2H), 4.13 (dd, *J* = 48.6, 10.5 Hz, 2H), 3.73-3.62 (m, 2H), 3.12-3.01 (m, 1H), 2.74-2.54 (m, 1H), 2.29 (s, 3H), 2.13-2.04 (m, 2H), 1.55-1.51 (m, 1H), 1.40-1.15 (m, 2H); ¹⁹F NMR (376 MHz, CDCl₃) δ -119.35.

### Example 211. Compound 251 (5-[4-[(R)-amino(5-chloro-2-hydroxy-4-methylphenyl)methyl]piperidine-1-carbonyl]-3-methoxy-1_{H}-pyridin-2-one trifluoroacetic acid)

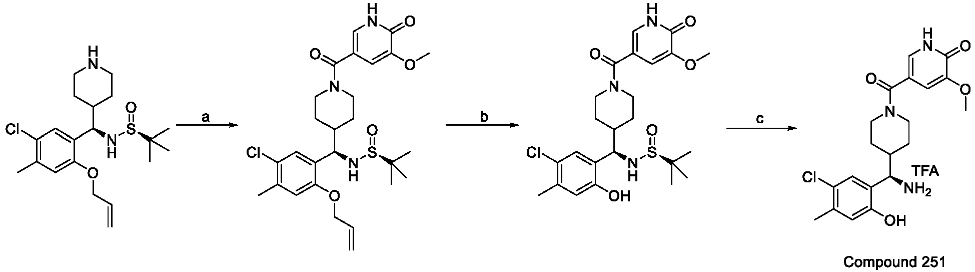

### Step a:

To a stirred solution of 5-methoxy-6-oxo-1*H*-pyridine-3-carboxylic acid (0.19 g, 0.94 mmol), EDCI (0.18 g, 0.94 mmol) and HOBt (0.13 g, 0.94 mmol) in DMF (3 mL) were added (*S*)-*N*-[(*R*)-[5-chloro-4-methyl-2-(prop-2-en-1-yloxy)phenyl] (piperidin-4-yl)methyl]-2-methylpropane-2-sulfinamide (0.25 g, 0.63 mmol) and Et₃N (0.19 g, 1.88 mmol) at room temperature. The reaction mixture was stirred for 12 h at room temperature. The resulting mixture was quenched with water (20 mL) and extracted with EA (3 x 20 mL). The combined organic layers were washed with brine (2 x 10 mL), dried over anhydrous Na₂SO₄. After filtration, the filtrate was concentrated under reduced pressure to afford (*S*)-*N*-[(*R*)-[5-chloro-4-methyl-2-(prop-2-en-1-yloxy)phenyl][1-(5-methoxy-6-oxo-1*H*-pyridine-3-carbonyl)piperidin-4-yl]methyl]-2-methylpropane-2-sulfinamide as a yellow semi solid (0.25 g, crude): LCMS (ESI) calc'd for C₂₇H₃₆ClN₃O₅S [M + H]⁺: 550, 552 (3 : 1), found 550, 552 (3 : 1).

### Step b:

To a stirred mixture of (*S*)-*N*-[(*R*)-[5-chloro-4-methyl-2-(prop-2-en-1-yloxy)phenyl][1-(5-methoxy-6-oxo-1*H*-pyridine-3-carbonyl)piperidin-4-yl]methyl]-2-methylpropane-2-sulfinamide (0.25 g, crude) in HOAc (2 mL) was added Pd(PPh₃)₄ (0.21 g, 0.18 mmol) at room temperature under nitrogen atmosphere. The resulting mixture was stirred for 2 h at room temperature under nitrogen atmosphere. The resulting mixture was concentrated under reduced pressure to afford (*S*)-*N*-[(*R*)-(5-chloro-2-hydroxy-4-methylphenyl)[1-(5-methoxy-6-oxo-1*H*-pyridine-3-carbonyl)piperidin-4-yl]methyl]-2-methylpropane-2-sulfinamide as a yellow oil (0.20 g, crude): LCMS (ESI) calc'd for C₂₄H₃₂ClN₃O₅S [M + H]⁺: 510, 512 (3 : 1), found 510, 512 (3 : 1).

### Step c:

To a stirred mixture of (*S*)-*N*-[(*R*)-(5-chloro-2-hydroxy-4-methylphenyl)[1-(5-methoxy-6-oxo-1H-pyridine-3-carbonyl)piperidin-4-yl]methyl]-2-methylpropane-2-sulfinamide (0.20 g, crude) in THF (2 mL) was added aq. HCl (4 *N,* 0.5 mL) at room temperature under air atmosphere. The resulting mixture was stirred for 1 h at room temperature. Then the reaction mixture was concentrated under reduced pressure. The residue was purified with Prep-HPLC with the following conditions: Column: XBridge Prep C 18 OBD Column 19 x 150 mm, 5 µm; Mobile Phase A: water (plus 0.05% TFA), Mobile Phase B: ACN; Flow rate: 25 mL/min; Gradient: 5% B to 27% B in 7 min; Detector: UV 254/220 nm; Retention time: 7.07 min. The fractions containing the desired product were collected and concentrated under reduced pressure to afford Compound 251 (5-[4-[(*R*)-amino(5-chloro-2-hydroxy-4-methylphenyl)methyl]piperidine-1-carbonyl]-3-methoxy-1_{H}-pyridin-2-one trifluoroacetic acid) as an off-white solid (118 mg, 73% overall three steps): LCMS (ESI) calc'd for C₂₀H₂₄ClN₃O₄ [M + H]⁺: 406, 408 (3 : 1), found 406, 408 (3 : 1); ¹H NMR (400 MHz, CD₃OD) δ 7.28-7.19 (m, 2H), 6.95 (d, *J* = 2.2 Hz, 1H), 6.85 (s, 1H), 4.57-4.00 (m, 3H), 3.86 (s, 3H), 3.21-2.86 (m, 2H), 2.52-2.36 (m, 1H), 2.31 (s, 3H), 2.08-2.00 (m, 1H), 1.51-1.32 (m, 2H), 1.31-1.13 (m, 1H); ¹⁹F NMR (376 MHz, CD₃OD) δ -77.18.

The compounds in Table 1f below were prepared in an analogous fashion to that described for Compound 251, starting from (*S*)-*N*-[(*R*)-[5-chloro-4-methyl-2-(prop-2-en-1-yloxy)phenyl](piperidin-4-yl)methyl]-2-methylpropane-2-sulfinamide and the corresponding acids or acid salts, which were prepared as described herein, or which were available from commercial sources. Compounds marked with a * in Table 1f are reference compounds.

The acid salt used to prepare Compound 207, lithium 5-(hydroxymethyl)-6-oxo-1,6-dihydropyridine-3-carboxylate, was prepared as follows.

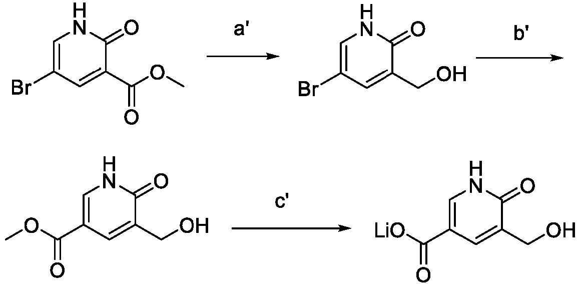

### Step a':

To a stirred solution of methyl 5-bromo-2-hydroxy-1,2-dihydropyridine-3-carboxylate (2.00 g, 8.55 mmol) in THF (20 mL) and MeOH (1.00 mL) was added LiBH₄ (8.55 mL, 17.09 mmol, 2 M in THF) at 0 °C under nitrogen atmosphere and the resulting mixture was stirred for 3 h at room temperature. The reaction was then quenched with water (10mL). The resulting mixture was concentrated under reduced pressure. The residue was purified by silica gel column chromatography, eluted with DCM/MeOH (10/1) to afford 5-bromo-3-(hydroxymethyl)-1*H*-pyridin-2-one as an off-white solid (1.00g, 57%): LCMS (ESI) calc'd for C₆H₆BrNO₂ [M + H]⁺: 204, 206 (1 : 1) found 204, 206 (1 : 1); ¹H NMR (400 MHz, DMSO-*d*₆) δ 6.90-6.82 (m, 1H), 6.72-6.68 (m, 1H), 3.68 (s, 3H).

### Step b':

To a stirred solution of 5-bromo-3-(hydroxymethyl)-1*H*-pyridin-2-one (0.50 g, 2.45 mmol) and Pd(dppf)Cl₂ CH₂Cl₂ adduct (0.20 g, 0.25 mmol) in MeOH (5 mL) was added TEA (0.50 g, 4.90 mmol) at room temperature. The resulting mixture was stirred overnight at 100 °C under CO (5 atm) atmosphere and then concentrated under reduced pressure. The residue was purified by Prep-TLC (DCM/MeOH 20/1) to afford methyl 5-(hydroxymethyl)-6-oxo-1*H-*pyridine-3-carboxylate as a light pink solid (60 mg, 13%): LCMS (ESI) calc'd for C₈H₉NO₄ [M + H]⁺: 184 found 184.

### Step c':

A solution of methyl 5-(hydroxymethyl)-6-oxo-1*H*-pyridine-3-carboxylate (60 mg, 0.33 mmol) and LiOH.H₂O (27 mg, 0.66 mmol) in MeOH (1 mL) and H₂O (0.25 mL) was stirred for 1 h at 40 °C. The resulting mixture was concentrated under reduced pressure to afford lithium 5-(hydroxymethyl)-6-oxo-1,6-dihydropyridine-3-carboxylate as an off-white solid (70 mg, crude), which was used in the next step directly without further purification: LCMS (ESI) calc'd for C₇H₇NO₄ [M + H]⁺: 170, found 170.

**Table 1f**

| **Compound Number** | **Structure** | **Chemical Name** | **MS & NMR** |
|---|---|---|---|
| **259** | 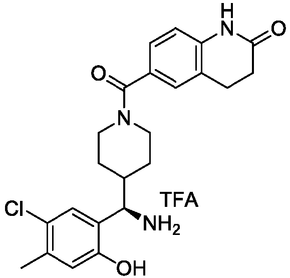 | 6-[4-[(*R*)-amino(5-chloro-2-hydroxy-4-methylphenyl)methyl]pipe ridine-1-carbonyl]-3,4-dihydro-1*H*-quinolin-2-one trifluoroacetic acid | [M + H]⁺: 428, 430 (3 : 1); ¹H NMR (400 MHz, CD₃OD) δ 7.32-7.19 (m, 3H), 6.93 (d, *J* = 8.1 Hz, 1H), 6.85 (s, 1H), 4.83-4.31 (m, 1H), 4.14 (d, *J* = 9.8 Hz, 1H), 4.13-3.61 (m, 1H), 3.25-2.71 (m, 4H), 2.61 (dd, *J* = 8.5, 6.6 Hz, 2H), 2.49-2.35 (m, 1H), 2.31 (s, 3H), 2.20-1.77 (m, 1H), 1.51-1.12 (m, 3H); ¹⁹F NMR (376 MHz, CD₃OD) δ -77.07. |
| **270** | 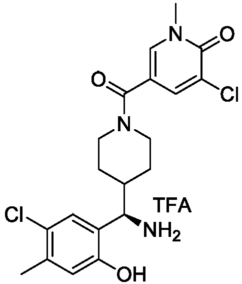 | 5-[4-[(R)-amino(5-chloro-2-hydroxy-4-methylphenyl)methyl]pipe ridine-1-carbonyl]-3-chloro-1-methylpyridin-2-one trifluoroacetic acid | [M + H]+: 424, 426 (3 : 2); 1H NMR (400 MHz, CD3OD) δ 7.90 (d, J = 2.3 Hz, 1H), 7.80 (d, J = 2.3 Hz, 1H), 7.25 (s, 1H), 6.85 (s, 1H), 4.67-3.95 (m, 3H), 3.65 (s, 3H), 3.18-2.84 (m, 2H), 2.48-2.35 (m, 1H), 2.32 (s, 3H), 2.09-1.96 (m, 1H), 1.49-1.34 (m, 2H), 1.33-1.18 (m, 1H); 19F NMR (376 MHz, CD3OD) δ -77.13. |
| **207** | 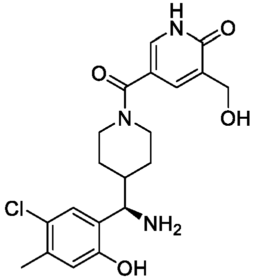 | (*R*)-5-(4-(amino(5-chloro-2-hydroxy-4-methylphenyl)methyl)pipe ridine-1-carbonyl)-3-(hydroxymethyl)pyridin-2(1H)-one | [M + H]⁺: 406, 408 (3 : 1) found406, 408 (3 : 1); ¹H NMR (400 MHz, CD₃OD) δ 7.69-7.63 (m, 1H), 7.61-7.56 (m, 1H), 7.25 (s, 1H), 6.85 (s, 1H), 4.52 (s, 2H), 4.47-4.26 (m, 2H), 4.14 (d, *J* = 9.8 Hz, 1H), 3.12-2.91 (m, 2H), 2.48-2.33 (m, 1H), 2.32 (s, 3H), 2.08-1.99 (m, 1H), 1.49-1.33 (m, 2H), 1.33-1.18 (m, 1H). |

### Example 212. Compound 257 (5-[4-[(R)-amino(3,4-dichloro-2-fluoro-6-hydroxyphenyl)methyl]piperidine-1-carbonyl]-1H-pyridin-2-one trifluoroacetic acid)

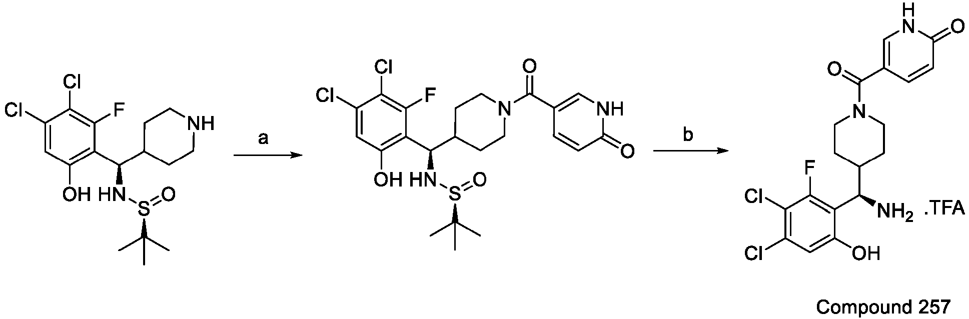

### Step a:

To a stirred solution of 6-oxo-1*H*-pyridine-3-carboxylic acid (84 mg, 0.60 mmol), EDCI (0.12 g, 0.60 mmol) and HOBt (82 mg, 0.60 mmol) in DMF (4 mL) were added (*R*)*-N-*[(*S*)-(3,4-dichloro-2-fluoro-6-hydroxyphenyl)(piperidin-4-yl)methyl]-2-methylpropane-2-sulfinamide (Intermediate 18, Example 18) (0.20 g, 0.50 mmol) and Et₃N (0.15 g, 1.51 mmol) in portions at room temperature. After stirring for 2 h at room temperature, the reaction solution was quenched with water (20 mL) at room temperature and extracted with EA (2 x 50 mL). The combined organic layers were washed with brine (2 x 10 mL), dried over anhydrous Na₂SO₄. After filtration, the filtrate was concentrated under reduced pressure to afford (*S*)*-N-*((*R*)-(3,4-dichloro-2-fluoro-6-hydroxyphenyl)(1-(6-oxo-1,6-dihydropyridine-3-carbonyl)piperidin-4-yl)methyl)-2-methylpropane-2-sulfinamide. The crude product was used in the next step directly without further purification: LCMS (ESI) calc'd for C₂₂H₂₆Cl₂FN₃O₄S [M + H]⁺: 518, 520 (3 : 2), found 518, 520 (3 : 2).

### Step b:

To a stirred mixture of (*S*)-*N*-((*R*)-(3,4-dichloro-2-fluoro-6-hydroxyphenyl)(1-(6-oxo-1,6-dihydropyridine-3-carbonyl)piperidin-4-yl)methyl)-2-methylpropane-2-sulfinamide (crude) in THF (4 mL) was added aq. HCl (6 *N,* 2 mL) dropwise at room temperature. The reaction solution was stirred for 2 h at room temperature. The reaction solution was stirred for 1 h at room temperature. The resulting solution was diluted with EA (10 mL). The organic layer was extracted with aq. HCl (1 *N,* 3 x 10 mL). The combined aqueous layers were washed with EA (3 x 10 mL) and concentrated under reduced pressure. The residue was purified by Prep-HPLC with the following conditions: Column: Xselect CSH OBD Column 30 x 150 mm, 5 µm; Mobile Phase A: water (plus 0.05% TFA), Mobile Phase B: ACN; Flow rate: 60 mL/min; Gradient: 5% B to 28% B in 7 min; Detector: UV 220 nm; Retention time: 6.43 min. The fractions containing the desired product were collected and concentrated under reduced pressure to afford Compound 257 (5-[4-[(*R*)-amino(3,4-dichloro-2-fluoro-6-hydroxyphenyl)methyl]piperidine-1-carbonyl]-1*H*-pyridin-2-one trifluoroacetic acid) as an off-white solid (64 mg, 59% overall two steps): LCMS (ESI) calc'd for C₁₈H₁₈Cl₂FN₃O₃ [M + H]⁺: 414, 416 (3 : 2), found 414, 416 (3 : 2); ¹H NMR (400 MHz, CD₃OD) δ 7.72-7.56 (m, 2H), 6.98 (d, *J* = 1.8 Hz, 1H), 6.56 (d, *J* = 9.4 Hz, 1H), 4.43 (d, *J* = 10.0 Hz, 1H), 4.38-4.06 (m, 2H), 3.15-2.88 (m, 2H), 2.62-2.39 (m, 1H), 2.12-1.93 (m, 1H), 1.55-1.15 (m, 3H); ¹⁹F NMR (376 MHz, CD₃OD) δ -77.29, -113.13.

### Example 213. Compound 260 ((2R,3S)-2-[4-[(R)-amino(4,5-dichloro-2-hydroxyphenyl)methyl]piperidine-1-carbonyl]oxolan-3-ol trifluoroacetic acid)

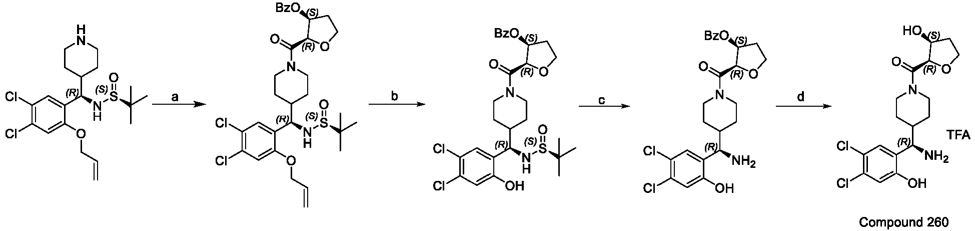

### Step a:

To a stirred solution of (*2R,3S*)-3-(benzoyloxy)oxolane-2-carboxylic acid (0.13 g, 0.54 mmol) and HATU (0.20 g, 0.54 mmol) in DMF (3 mL) were added (*S*)*-N-*[(*R*)-[4,5-dichloro-2-(prop-2-en-1-yloxy)phenyl](piperidin-4-yl)methyl]-2-methylpropane-2-sulfinamide (0.15 g, 0.34 mmol) and Et₃N (0.11 g, 1.07 mmol) at room temperature. The reaction was stirred at room temperature for 0.5 h. The reaction was purified by reverse phase chromatography, eluted with 55% ACN in water with 10 mmoL/L NH₄HCO₃ to afford (*2R,3S*)-2-[4-[(*R*)-[4,5-dichloro-2-(prop-2-en-1-yloxy)phenyl]([[(S)-2-methylpropane-2-sulfinyl]amino])methyl]piperidine-1-carbonyl]oxolan-3-yl benzoate as a light yellow solid (0.15 g, 57%): LCMS (ESI) calc'd for C₃₁H₃₈Cl₂N₂O₆S [M + H]⁺: 637, 639 (3 : 2), found 637, 639 (3 : 2); ¹H NMR (400 MHz, CDCl₃) δ 7.98 (t, *J* = 9.6 Hz, 2H), 7.67-7.56 (m, 1H), 7.51-7.37 (m, 2H), 7.25-7.17 (m, 1H), 6.97-6.86 (m, 1H), 6.07-5.92 (m, 1H), 5.88 (t, *J* = 6.1 Hz, 1H), 5.41-5.28 (m, 2H), 4.92 (t, *J* = 5.6 Hz, 1H), 4.74-4.39 (m, 4H), 4.39-4.25 (m, 2H), 4.07-3.94 (m, 2H), 3.82-3.61 (m, 1H), 2.86 (dt, *J* = 94.8, 12.5 Hz, 1H), 2.47-2.30 (m, 2H), 2.30-2.14 (m, 1H), 1.97-1.80 (m, 1H), 1.67-1.52 (m, 2H), 1.13 (d, *J* = 24.7 Hz, 9H).

### Step b:

To a stirred solution of (*2R,3S*)-2-[4-[(*R*)-[4,5-dichloro-2-(prop-2-en-1-yloxy)phenyl]([[(*S*)-2-methylpropane-2-sulfinyl]amino])methyl]piperidine-1-carbonyl]oxolan-3-yl benzoate (0.15 g, 0.24 mmol) and Pd(PPh₃)₄ (5 mg, 0.01 mmol) in THF (3 mL) was added NaBH₄ (18 mg, 0.47 mmol) at room temperature. The reaction was stirred at room temperature for 1 h. The reaction was quenched with saturate aq. NH₄Cl (0.5 mL) and concentrated under reduced pressure to afford (*2R,3S*)-2-[4-[(*R*)-(4,5-dichloro-2-hydroxyphenyl)([[(*S*)-2-methylpropane-2-sulfinyl]amino])methyl]piperidine-1-carbonyl]oxolan-3-yl benzoate as a brown oil (0.15 g, crude), which was used in the next step directly without further purification: LCMS (ESI) calc'd for C₂₈H₃₄Cl₂N₂O₆S [M + H]⁺: 597, 599 (3 : 2), found 597, 599 (3 : 2).

### Step c:

To a stirred solution of (*2R,3S*)-2-[4-[(*R*)-(4,5-dichloro-2-hydroxyphenyl)([[(*S*)-2-methylpropane-2-sulfinyl]amino])methyl]piperidine-1-carbonyl]oxolan-3-yl benzoate (0.15 g, 0.25 mmol) in THF (3 mL) was added aq. HCl (4 *N,* 2 mL) at room temperature. The reaction was stirred at room temperature for 1 h. The reaction was concentrated under reduced pressure to afford (*2R,3S*)-2-[4-[(*R*)-amino(4,5-dichloro-2-hydroxyphenyl)methyl]piperidine-1-carbonyl]oxolan-3-yl benzoate as a brown solid (0.15 g, crude), which was used in the next step directly without further purification: LCMS (ESI) calc'd for C₂₄H₂₆Cl₂N₂O₅ [M + H]⁺: 493, 495 (3 : 2), found 493, 495 (3 : 2).

### Step d:

A solution of (*2R,3S*)-2-[4-[(*R*)-amino(4,5-dichloro-2-hydroxyphenyl)methyl]piperidine-1-carbonyl]oxolan-3-yl benzoate (0.15 g, 0.30 mmol) in Methylamine in methanol (5 mL) was stirred for 16 h at room temperature. The reaction was concentrated under reduced pressure. The residue was dissolved in aq. HCl (1 *N,* 30 mL) and EA (30 mL). The organic layers were washed with aq. HCl (1 *N,* 2 x 30 mL). The combined aqueous layers were concentrated under reduced pressure. The residue was purified by Prep-HPLC with following conditions: Column: Xselect CSH OBD Column 30 x 150 mm 5 µm; Mobile Phase A: Water (plus 0.05% TFA), Mobile Phase B: ACN; Flow rate: 60 mL/min; Gradient: 5% B to 25% B in 7 min; Detector: UV 254/220 nm; Retention time: 6.65 min. The fractions containing the desired product were collected and concentrated under reduced pressure to afford Compound 260 ((2R,3S)-2-[4-[(*R*)-amino(4,5-dichloro-2-hydroxyphenyl)methyl]piperidine-1-carbonyl]oxolan-3-ol trifluoroacetic acid) as an off-white solid (42 mg, 36% overall three steps): LCMS (ESI) calc'd for C₁₇H₂₂Cl₂N₂O₄ [M + H]⁺: 389, 391 (3 : 2), found 389, 391 (3 : 2); ¹H NMR (400 MHz, CD₃OD) δ 7.43 (d, *J* = 4.0 Hz, 1H), 7.08 (s, 1H), 4.75-4.42 (m, 3H), 4.29-3.95 (m, 3H), 3.95-3.87 (m, 1H), 3.18-2.91 (m, 1H), 2.64 (dt, *J* = 39.0, 12.8 Hz, 1H), 2.50-2.28 (m, 1H), 2.28-2.07 (m, 1H), 2.07-1.87 (m, 2H), 1.57-1.07 (m, 3H); ¹⁹F NMR (376 MHz, CD₃OD) δ -77.02.

### Example 214. Compound 264 (5-[4-[(R)-amino(3-chloro-2-fluoro-6-hydroxy-4-methylphenyl)methyl]piperidine-1-carbonyl]-3-chloro-1H-pyridin-2-one trifluoroacetic acid)

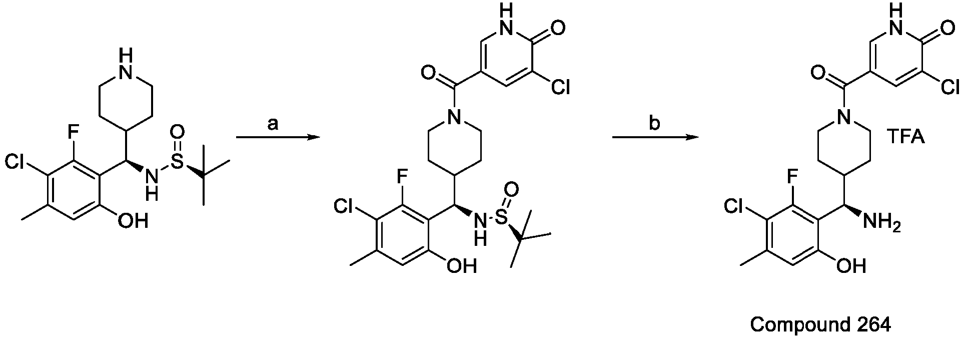

### Step a:

To a stirred solution of *N*-[(*R*)-(3-chloro-2-fluoro-6-hydroxy-4-methylphenyl)(piperidin-4-yl)methyl]-2,2-dimethylpropanamide (0.10 g, 0.28 mmol) and Et₃N (60 mg, 0.56 mmol) in DMF (2 mL) were added EDC·HCl (80 m, 0.42 mmol) and HOBt (60 mg, 0.42 mmol) and 5-chloro-6-oxo-1*H*-pyridine-3-carboxylic acid (60 mg, 0.36 mmol) at room temperature under nitrogen atmosphere. The resulting mixture was stirred for 2 h at room temperature under nitrogen atmosphere. The residue was purified by reverse phase chromatography, eluted with 55% ACN in water (plus 0.05% TFA) to afford *N*-[(*R*)-(3-chloro-2-fluoro-6-hydroxy-4-methylphenyl)[1-(5-chloro-6-oxo-1*H*-pyridine-3-carbonyl)piperidin-4-yl]methyl]-2,2-dimethylpropanamide as a light yellow foam (0.10 g, 73%): LCMS (ESI) calc'd for C₂₃H₂₈Cl₂FN₃O₄S [M + H]⁺: 532, 534 (3 : 2), found 532, 534 (3 : 2): ¹H NMR (400 MHz, CD₃OD) δ 7.85 (t, *J* = 2.7 Hz, 1H), 7.64 (d, *J* = 2.4 Hz, 1H), 6.60 (s, 1H), 4.47-4.17 (m, 2H), 3.11-2.86 (m, 2H), 2.40-2.21 (m, 4H), 1.50-1.24 (m, 5H), 1.14 (d, *J* = 28.3 Hz, 9H).

### Step b:

A solution of (*S*)-*N*-[(*R*)-(3-chloro-2-fluoro-6-hydroxy-4-methylphenyl)[1-(5-chloro-6-oxo-1*H*-pyridine-3-carbonyl)piperidin-4-yl]methyl]-2-methylpropane-2-sulfinamide (0.10 g, 0.19 mmol) in 1,4-dioxane (2 mL) and aq. HCl (6 *N,* 1 mL) was stirred for 1 h at room temperature. The resulting mixture was concentrated under reduced pressure. The crude product was purified by Prep-HPLC with the following conditions: Column: Xselect CSH OBD Column 30 x 150 mm 5 µm; Mobile Phase A: water (plus 0.05% TFA), Mobile Phase B: ACN; Flow rate: 60 mL/min; Gradient: 5% B to 29 B% in 7 min; Detector: UV 220 nm; Retention time: 6.88 min. The fractions containing the desired product were collected and concentrated under reduced pressure to afford Compound 264 (5-[4-[(*R*)-amino(3-chloro-2-fluoro-6-hydroxy-4-methylphenyl)methyl]piperidine-1-carbonyl]-3-chloro-1*H*-pyridin-2-one trifluoroacetic acid) (56 mg, 55%) as an off-white solid: LCMS (ESI) calc'd for C₁₉H₂₀Cl₂FN₃O₃ [M + H]⁺: 428, 430 (3 : 2), found 428, 430 (3 : 2); ¹H NMR (400 MHz, CD₃OD) δ 7.85 (d, *J* = 2.3 Hz, 1H), 7.64 (d, *J* = 2.3 Hz, 1H), 6.73 (d, *J* = 1.6 Hz, 1H), 4.47-4.07 (m, 3H), 3.05-2.97 (m, 2H), 2.49 (q, *J* = 11.1 Hz, 1H), 2.40-2.31 (m, 3H), 2.05 (d, *J* = 13.0 Hz, 1H), 1.52-1.21 (m, 3H). ¹⁹F NMR (400 MHz, CD₃OD) δ -77.19, -117.77.

### Example 215. Compound 266 (5-[4-[(R)-amino(3,4-dichloro-2-fluoro-6-hydroxyphenyl)methyl]piperidine-1-carbonyl]-3-chloro-1H-pyridin-2-one trifluoroacetic acid)

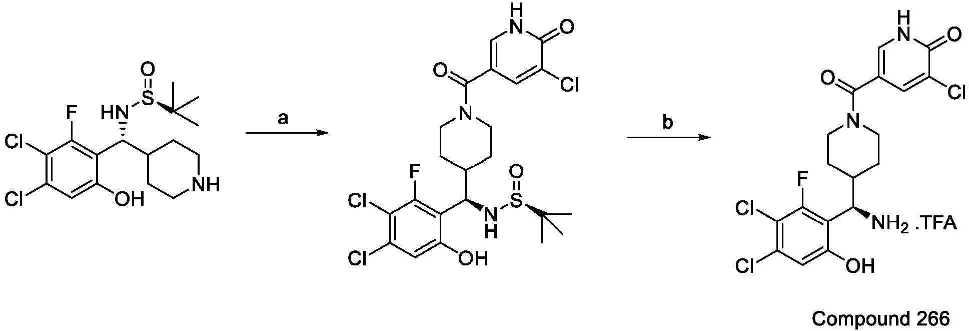

### Step a:

To a stirred solution of 5-chloro-6-oxo-1*H*-pyridine-3-carboxylic acid (61 mg, 0.35 mmol) and EDCI (0.12 g, 0.60 mmol) and HOBt (82 mg, 0.60 mmol) in DMF (4 mL) were added (*R*)-*N*-[(*S*)-(3,4-dichloro-2-fluoro-6-hydroxyphenyl)(piperidin-4-yl)methyl]-2-methylpropane-2-sulfinamide (Intermediate 18, Example 18) (0.14 g, 0.35 mmol) and Et₃N (0.15 g, 1.51 mmol) in portions at room temperature. After stirring for 2 h at room temperature, the reaction solution was quenched with water (20 mL) at room temperature and extracted with EA (2 x 50 mL). The combined organic layers were washed with brine (2 x 10 mL), dried over anhydrous Na₂SO₄. After filtration, the filtrate was concentrated under reduced pressure to afford (*S*)-*N*-((*R*)-(1-(5-chloro-6-oxo-1,6-dihydropyridine-3-carbonyl)piperidin-4-yl)(3,4-dichloro-2-fluoro-6-hydroxyphenyl)methyl)-2-methylpropane-2-sulfinamide. The crude product was used in the next step directly without further purification: LCMS (ESI) calc'd for C₂₂H₂₅Cl₃FN₃O₄S [M + H]⁺: 552, 554, 556 (3 : 3 : 1), found 552, 554, 556 (3 : 3 : 1).

### Step b:

To a stirred mixture of (*S*)-*N*-((*R*)-(1-(5-chloro-6-oxo-1,6-dihydropyridine-3-carbonyl)piperidin-4-yl)(3,4-dichloro-2-fluoro-6-hydroxyphenyl)methyl)-2-methylpropane-2-sulfinamide (crude) in THF (4 mL) was added aq. HCl (6 *N,* 2 mL) dropwise at room temperature. The reaction solution was stirred for 2 h at room temperature. The reaction solution was stirred for 1 h at room temperature. The resulting solution was diluted with EA (10 mL). The organic layer was extracted with aq. HCl (1 *N,* 3 x 10 mL). The combined aqueous layers were washed with EA (3 x 10 mL) and concentrated under reduced pressure. The residue was purified by Prep-HPLC with the following conditions: Column: Xselect CSH OBD Column 30 x 150 mm, 5 µm; Mobile Phase A: water (plus 0.05% TFA), Mobile Phase B: ACN; Flow rate: 60 mL/min; Gradient: 7% B to 30% B in 7 min; Detector: UV 220 nm; Retention time: 7.80 min. The fractions containing the desired product were collected and concentrated under reduced pressure to afford Compound 266 (5-[4-[(*R*)-amino(3,4-dichloro-2-fluoro-6-hydroxyphenyl)methyl]piperidine-1-carbonyl]-3-chloro-1*H*-pyridin-2-one trifluoroacetic acid) as an off-white solid (46 mg, 42% overall two steps): LCMS (ESI) calc'd for C₁₈H₁₇Cl₃FN₃O₃ [M + H]⁺: 448, 450, 452 (3 : 3 : 1), found 448, 450, 452 (3 : 3 : 1); ¹H NMR (400 MHz, CD₃OD) δ 7.84 (d, *J* = 2.3 Hz, 1H), 7.63 (d, *J* = 2.4 Hz, 1H), 6.95 (d, *J* = 1.8 Hz, 1H), 4.43 (d, *J* = 9.9 Hz, 1H), 4.39-4.03 (m, 2H), 3.14-2.81 (m, 2H), 2.54-2.37 (m, 1H), 2.13-1.93 (m, 1H), 1.53-1.17 (m, 3H); ¹⁹F NMR (376 MHz, CD₃OD) δ -76.99, -113.23.

### Example 216. Compound 272 (5-[4-[(R)-amino(3-chloro-2-fluoro-6-hydroxy-4-methylphenyl)methyl]piperidine-1-carbonyl]-1H-pyridin-2-one trifluoroacetic acid)

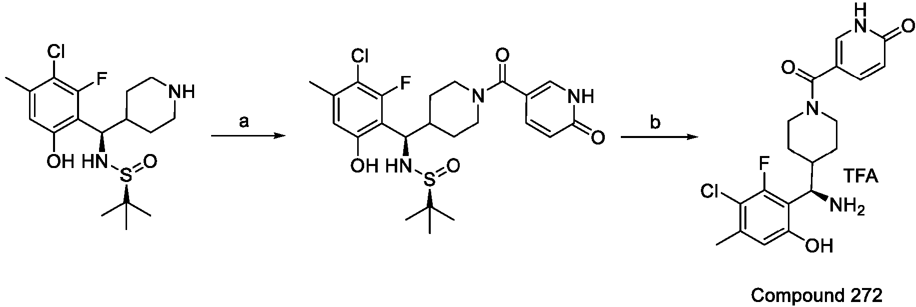

### Step a:

To a stirred solution of 6-oxo-1*H*-pyridine-3-carboxylic acid (44 mg, 0.32 mmol) in DMF (2 mL) were added EDCI (0.10 g, 0.60 mmol) and HOBt (81 mg, 0.60 mmol) at room temperature. To the above mixture were added (*S*)-*N*-[(*R*)-(3-chloro-2-fluoro-6-hydroxy-4-methylphenyl)[1-(6-oxo-1*H*-pyridine-3-carbonyl)piperidin-4-yl]methyl]-2-methylpropane-2-sulfinamide (0.20 g, 0.40 mmol) and Et₃N (0.12 g, 1.20 mmol) at room temperature. The resulting mixture was stirred for 12 h at room temperature. The reaction mixture was purified by reverse phase chromatography, eluted with 45% ACN in water (plus 0.5% TFA) to afford (*S*)-*N*-[(*R*)-(3-chloro-2-fluoro-6-hydroxy-4-methylphenyl)[1-(6-oxo-1*H*-pyridine-3-carbonyl)piperidin-4-yl]methyl]-2-methylpropane-2-sulfinamide as a colorless oil (0.12 g, 48%): LCMS (ESI) calc'd for C₂₃H₂₉ClFN₃O₄S [M + H]⁺: 498, 500 (3 : 1), found 498, 500 (3 : 1).

### Step b:

To a stirred mixture of (*S*)-*N*-[(*R*)-(3-chloro-2-fluoro-6-hydroxy-4-methylphenyl)[1-(6-oxo-1*H*-pyridine-3-carbonyl)piperidin-4-yl]methyl]-2-methylpropane-2-sulfinamide (0.1 g, 0.20 mmol) in THF (1 mL) was added aq. HCl (4 *N,* 0.25 mL) dropwise at room temperature. The resulting mixture was stirred for 1 h at room temperature. The reaction mixture was concentrated under reduced pressure. The residue was purified with Prep-HPLC with the following conditions: Column: XBridge Prep C18 OBD Column 19 x 150 mm, 5 µm; Mobile Phase A: water (with 10 mmol/L NH₄HCO), Mobile Phase B: ACN; Flow rate: 25 mL/min; Gradient: 5% B to 30% B in 7 min; Detector: UV 254/220 nm; Retention time: 5.97 min. The fractions containing the desired product were collected and concentrated under reduced pressure to afford Compound 272 (5-[4-[(*R*)-amino(3-chloro-2-fluoro-6-hydroxy-4-methylphenyl)methyl]piperidine-1-carbonyl]-1H-pyridin-2-one trifluoroacetic acid) as an off-white solid (48 mg, 46%): LCMS (ESI) calc'd for C₁₉H₂₁ClFN₃O₃ [M + H]⁺: 394, 396 (3 : 1), found 394, 396 (3 : 1); ¹H NMR (400 MHz, CD₃OD) δ 7.76-7.56 (m, 2H), 6.73 (s, 1H), 6.56 (d, *J* = 9.4 Hz, 1H), 4.54-3.96 (m, 3H), 3.18-2.82 (m, 2H), 2.56-2.43 (m, 1H), 2.35 (s, 3H), 2.12-2.00 (m, 1H), 1.53-1.35 (m, 2H), 1.34-1.18 (m, 1H); ¹⁹F NMR (376 MHz, CD₃OD) δ -77.19, - 117.75.

### Example 217. Compound 275 (5-[4-[(R)-amino(4,5-dichloro-2-hydroxyphenyl)methyl]piperidine-1-carbonyl]-1,3-diazinan-2-one trifluoroacetic acid)

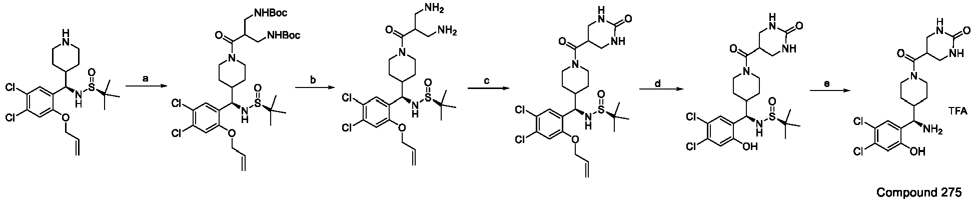

### Step a:

To a stirred solution of lithio 3-[(*tert*-butoxycarbonyl)amino]-2-[[(*tert-*butoxycarbonyl)amino]methyl]propanoate (0.70 g, 2.15 mmol, and HATU (0.81 g, 2.15 mmol) in DMF (6 mL) was added (*S*)-*N*-[(*R*)-[4,5-dichloro-2-(prop-2-en-1-yloxy)phenyl](piperidin-4-yl)methyl]-2-methylpropane-2-sulfinamide (0.60 g, 1.43 mmol) and Et₃N (0.43 g, 4.29 mmol) at room temperature. The reaction was stirred at room temperature for 0.5 h. The residue was purified by reverse phase chromatography, eluted with 60% ACN in water (plus 0.05% TFA) to afford *tert*-butyl *N*-(2-[[(*tert*-butoxycarbonyl)amino]methyl]-3-[4-[(*R*)-[4,5-dichloro-2-(prop-2-en-1-yloxy)phenyl]([[(*S*)-2-methylpropane-2-sulfinyl]amino])methyl]piperidin-1-yl]-3-oxopropyl)carbamate as a light yellow semi-solid (0.74 g, 72% overall three steps): LCMS (ESI) calc'd for C₃₃H₅₂Cl₂N₄O₇S for [M + H]⁺: 719, 721 (3 : 2), found 719, 721 (3 : 2); ¹H NMR (400 MHz, CDCl₃) δ 7.22 (d, *J* = 3.9 Hz, 1H), 6.98 (d, *J* = 1.9 Hz, 1H), 6.11-5.95 (m, 1H), 5.69-5.49 (m, 2H), 5.49-5.30 (m, 2H), 4.71-4.50 (m, 3H), 4.48-4.21 (m, 1H), 4.05-3.79 (m, 1H), 3.55-3.40 (m, 3H), 3.19-2.89 (m, 4H), 2.59-2.37 (m, 1H), 2.22-1.92 (m, 2H), 1.63-1.36 (m, 19H), 1.33-1.20 (m, 2H), 1.17 (s, 9H).

### Step b:

To a stirred solution of *tert*-butyl N-(2-[[(*tert*-butoxycarbonyl)amino]methyl]-3-[4-[(R)-[4,5-dichloro-2-(prop-2-en-1-yloxy)phenyl]([[(S)-2-methylpropane-2-sulfinyl]amino])methyl]piperidin-1-yl]-3-oxopropyl)carbamate (0.74 g, 1.03 mmol) in DCM (8 mL) was added TFA (2 mL) dropwise at room temperature. The resulting mixture was stirred for 1 h at room temperature. The reaction was neutralized with saturate aq. NaHCO₃ (6 mL) to pH 8 at room temperature. The resulting mixture was extracted with DCM/MeOH (10/1) (3 x 100 mL). The combined organic layers were washed with brine (3 x 20 mL), dried over anhydrous Na₂SO₄. After filtration, the filtrate was concentrated under reduced pressure. The residue was purified by reverse phase chromatography, eluted with 47% ACN in water with 10 mmol/L NH₄HCO₃ to afford (*S*)-*N*-[(*R*)-[1-[3-amino-2-(aminomethyl)propanoyl]piperidin-4-yl][4,5-dichloro-2-(prop-2-en-1-yloxy)phenyl]methyl]-2-methylpropane-2-sulfinamide as a light yellow semi-solid (0.20 g, 33%): LCMS (ESI) calc'd for C₂₃H₃₆Cl₂N₄O₃S for [M + H]⁺: 519, 521 (3 : 2), found 519, 521 (3 : 2); ¹H NMR (400 MHz, CDCl₃) δ 7.23 (d, *J* = 11.5 Hz, 1H), 6.98 (s, 1H), 6.10-5.96 (m, 1H), 5.47-5.33 (m, 2H), 4.73 (dd, *J* = 42.4, 13.5 Hz, 1H), 4.62-4.27 (m, 3H), 4.10 (dd, *J* = 35.9, 13.7 Hz, 1H), 3.94-3.70 (m, 1H), 3.11-2.94 (m, 3H), 2.94-2.78 (m, 2H), 2.59-2.41 (m, 1H), 2.25-1.95 (m, 2H), 1.58-1.19 (m, 3H), 1.16 (d, *J* = 9.1 Hz, 9H).

### Step c:

To a stirred solution of (*S*)-*N*-[(*R*)-[1-[3-amino-2-(aminomethyl)propanoyl]piperidin-4-yl][4,5-dichloro-2-(prop-2-en-1-yloxy)phenyl]methyl]-2-methylpropane-2-sulfinamide (0.20 g, 0.39 mmol) in DCM (15 mL) was added CDI (56 mg, 0.35 mmol) in DCM (5 mL) at 0 °C. The resulting mixture was stirred for 16 h at room temperature. The resulting mixture was concentrated under reduced pressure. The residue was purified by reverse phase chromatography, eluted with 50% ACN in water (plus 0.05% TFA) to afford (*S*)-*N*-[(*R*)-[4,5-dichloro-2-(prop-2-en-1-yloxy)phenyl][1-(2-oxo-1,3-diazinane-5-carbonyl)piperidin-4-yl]methyl]-2-methylpropane-2-sulfinamide as a colorless oil (0.10 g, 48%): LCMS (ESI) calc'd for C₂₄H₃₄Cl₂N₄O₄S for [M + H]⁺: 545, 547 (3 : 2), found 545, 547 (3 : 2); ¹H NMR (400 MHz, CDCl₃) δ 7.20 (d, *J* = 10.2 Hz, 1H), 7.00 (s, 1H), 6.12-5.97 (m, 1H), 5.48-5.25 (m, 2H), 4.76-4.22 (m, 3H), 4.22-3.75 (m, 2H), 3.65-3.49 (m, 2H), 3.49-3.31 (m, 2H), 3.18-2.94 (m, 2H), 2.62-2.35 (m, 1H), 2.35-1.95 (m, 2H), 1.68-1.24 (m, 3H), 1.17 (d, *J* = 8.5 Hz, 9H).

### Step d:

To a stirred solution of (*S*)-*N*-[(*R*)-[4,5-dichloro-2-(prop-2-en-1-yloxy)phenyl][1-(2-oxo-1,3-diazinane-5-carbonyl)piperidin-4-yl]methyl]-2-methylpropane-2-sulfinamide (0.10 g, 0.18 mmol) and Pd(PPh₃)₄ (2 mg, 0.002 mmol) in THF (1 mL) was added NaBH₄ (14 mg, 0.37 mmol) at room temperature. The reaction was quenched with saturate aq. NH₄Cl (1 mL) at room temperature. The resulting mixture was concentrated under reduced pressure to afford (S)-*N*-[(*R*)-(4,5-dichloro-2-hydroxyphenyl)[1-(2-oxo-1,3-diazinane-5-carbonyl)piperidin-4-yl]methyl]-2-methylpropane-2-sulfinamide as a yellow oil (0.10 g, crude), which was used in the next step directly without further purification: LCMS (ESI) calc'd for C₂₁H₃₀Cl₂N₄O₄S for [M + H]⁺: 505, 507 (3 : 2), found 505, 507 (3 : 2).

### Step e:

To a stirred mixture of (*S*)-*N*-[(*R*)-(4,5-dichloro-2-hydroxyphenyl)[1-(2-oxo-1,3-diazinane-5-carbonyl)piperidin-4-yl]methyl]-2-methylpropane-2-sulfinamide (0.10 g, 0.20 mmol) in 1,4-dioxane (1 mL) was added aq. HCl (4 *N,* 1 mL) at room temperature. The resulting mixture was stirred for 1 h at room temperature. The resulting mixture was concentrated under reduced pressure. The residue was purified by Prep-HPLC with the following conditions: Column: XBridge Prep C18 OBD Column, 5 µm, 19 x 150 mm; Mobile Phase A: Water (plus 0.05% TFA), Mobile Phase B: ACN; Flow rate: 25 mL/min; Gradient: 5% B to 30% B in 7 min; Detector: UV 254/220 nm; Retention time: 5.68 min. The fractions containing the desired product were collected and concentrated under reduced pressure to afford Compound 275 (5-[4-[(*R*)-amino(4,5-dichloro-2-hydroxyphenyl)methyl]piperidine-1-carbonyl]-1,3-diazinan-2-one trifluoroacetic acid) as an off-white solid (50 mg, 53% over two steps): LCMS (ESI) calc'd for C₁₇H₂₂Cl₂N₄O₃ for [M + H]⁺: 401, 403 (3 : 2), found 401, 403 (3 : 2); ¹H NMR (400 MHz, DMSO-*d*₆) δ 7.56 (s, 1H), 7.10 (s, 1H), 4.36 (dd, *J* = 58.0, 13.1 Hz, 1H), 4.17 (t, *J* = 8.7 Hz, 1H), 3.99 (dd, *J =* 54.6, 13.8 Hz, 1H), 3.13 (d, *J =* 8.5 Hz, 4H), 3.07-2.88 (m, 2H), 2.47-2.37 (m, 1H), 2.22-2.07 (m, 1H), 1.90 (t, *J* = 16.2 Hz, 1H), 1.30-0.85 (m, 3H); ¹⁹F NMR (376 MHz, CD₃OD) δ -77.38.

### Example 218. (which is a reference example) Compound 284 ((2R)-1-[4-[(R)-amino[5-chloro-2-hydroxy-4-(trifluoromethyl)phenyl]methyl]piperidin-1-yl]-2,3-dihydroxypropan-1-one)

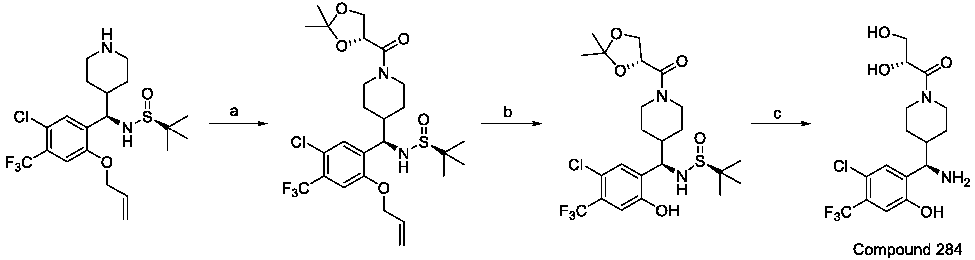

### Step a:

To a stirred mixture of (4*R*)-2,2-dimethyl-1,3-dioxolane-4-carboxylic acid (0.37 g, 2.49 mmol) and HATU (1.18 g, 3.11 mmol) in DMF (10 mL) was added N-[(R)-[5-chloro-2-(prop-2-en-1-yloxy)-4-(trifluoromethyl)phenyl](piperidin-4-yl) methyl]-2-methylpropane-2-sulfinamide (0.94 g, 2.08 mmol) and Et₃N (0.42 g, 4.15 mmol) dropwise at room temperature. The final reaction mixture was stirred for 2 h at room temperature. The resulting mixture was diluted with water (20 mL). The resulting mixture was extracted with EA (3 x 50 mL). The combined organic layers were washed with brine (5 x 50 mL), dried over anhydrous Na₂SO₄. After filtration, the filtrate was concentrated under reduced pressure. The residue was purified by reverse phase chromatography, eluted with 50% ACN in water with 20 mmol/L NH₄HCO₃ to afford *N*-[(*R*)-[5-chloro-2- (prop-2-en-1-yloxy)-4-(trifluoromethyl)phenyl]([1-[(4*R*)-2,2-dimethyl-1,3-dioxolane-4-carbonyl]piperidin-4-yl])methyl]-2-methylpropane-2-sulfinamide as a light yellow oil (0.68 g, 56%): LCMS (ESI) calc'd for C₂₆H₃₆ClF₃N₂O₅S [M + H]⁺: 581, 583 (3 : 1), found 581, 583 (3 : 1).

### Step b:

To a stirred mixture of *N*-[(*R*)-[5-chloro-2-(prop-2-en-1-yloxy)-4-(trifluoromethyl)phenyl](1-[[(4*R*)-2,2-dimethyl-1,3-dioxolan-4-yl](hydroxy)methyl]piperidin-4-yl)methyl]-2-methylpropane-2-sulfinamide (0.68 g, 1.17 mmol) and Pd(PPh₃)₄ (0.13 g, 0.12 mmol) in THF (8 mL) was added NaBH₄ (0.88 g, 2.33 mmol) in portions at room temperature under nitrogen atmosphere. The reaction mixture was stirred for 1 h at room temperature. The reaction was quenched with saturated aq. NH₄Cl (10 mL) at 0 °C. The resulting mixture was concentrated under reduced pressure. The residue was purified by reverse phase chromatography, eluted with 45% ACN in water with 10 mmol/L NH₄HCO₃ to afford *N*-[(*R*)-[5-chloro-2-hydroxy-4-(trifluoromethyl)phenyl]([1-[(4*R*)-2,2-dimethyl-1,3-dioxolane-4-carbonyl]piperidin-4-yl])methyl]-2-methylpropane-2-sulfinamide as a light yellow oil (0.33 g, 47%): LCMS (ESI) calc'd for C₂₃H₃₂ClF₃N₂O₅S [M + H]⁺: 541, 543 (3 : 1), found 541, 543 (3 : 1). ¹H NMR (400 MHz, CD₃OD) δ 7.46 (d, *J* = 2.7 Hz, 1H), 7.17 (s, 1H), 4.94-4.83 (m, 1H), 4.65-4.37 (m, 2H), 4.37-4.17 (m, 3H), 3.17-2.95 (m, 2H), 2.73-2.60 (m, 1H), 2.24-2.10 (m, 2H), 1.44-1.32 (m, 8H), 1.16 (d, *J* = 5.6 Hz, 9H).

### Step c:

To a stirred solution of *N*-[(*R*)-[5-chloro-2-hydroxy-4-(trifluoromethyl)phenyl]([1-[(4*R*)-2,2-dimethyl-1,3-dioxolane-4-carbonyl]piperidin-4-yl])methyl]-2-methylpropane-2-sulfinamide (0.33 g, 0.61 mmol) in THF (4 mL) was added aq. HCl (4 *N*, 2 mL) dropwise at room temperature. The reaction mixture was stirred for 2 h at room temperature. The mixture was basified to pH 8 with saturated aq. NaHCO₃. The resulting mixture was concentrated under reduced pressure. The residue was purified by Prep-HPLC with the following conditions: Column: XBridge Shield RP18 OBD Column, 30 x 150 mm, 5 µm; Mobile Phase A: water with 10 mmol/L NH₄HCO₃, Mobile Phase B: ACN; Flow rate: 25 mL/min; Gradient: 15% B to 30% B in 7 min; Detector: UV 254/220 nm; Retention time: 6.5 min. The fractions containing the desired product were collected and concentrated under reduced pressure to afford Compound 284 ((2*R*)-1-[4-[(*R*)-amino[5-chloro-2-hydroxy-4-(trifluoromethyl)phenyl]methyl]piperidin-1-yl]-2,3-dihydroxypropan-1-one) as an off-white solid (106.2 mg, 43%). LCMS (ESI) calc'd for C₁₆H₂₀ClF₃N₂O₄ [M + H]⁺: 397, 399 (3 : 1), found 397, 399 (3 : 1): ¹H NMR (400 MHz, CD₃OD) δ 7.31 (d, *J* = 5.1 Hz, 1H), 7.15-7.03 (m, 1H), 4.68-4.46 (m, 2H), 4.12 (dd, *J* = 40.8, 14.0 Hz, 1H), 3.98 (t, *J* = 7.8 Hz, 1H), 3.76-3.56 (m, 2H), 3.05 (dt, *J* = 25.9, 12.8 Hz, 1H), 2.64 (dt, *J* = 26.8, 12.7 Hz, 1H), 2.14-1.95 (m, 2H), 1.48 (s, 1H), 1.43-1.16 (m, 2H); ¹⁹F NMR (376 MHz, CD₃OD) δ -63.69.

### Example 219. Compound 274 (6-[4-[(R)-amino(4,5-dichloro-2-hydroxyphenyl)methyl]piperidine-1-carbonyl]-4-methylmorpholin-3-one isomer 1); Compound 278 (6-[4-[(R)-amino(4,5-dichloro-2-hydroxyphenyl)methyl]piperidine-1-carbonyl]-4-methylmorpholin-3-one isomer 2)

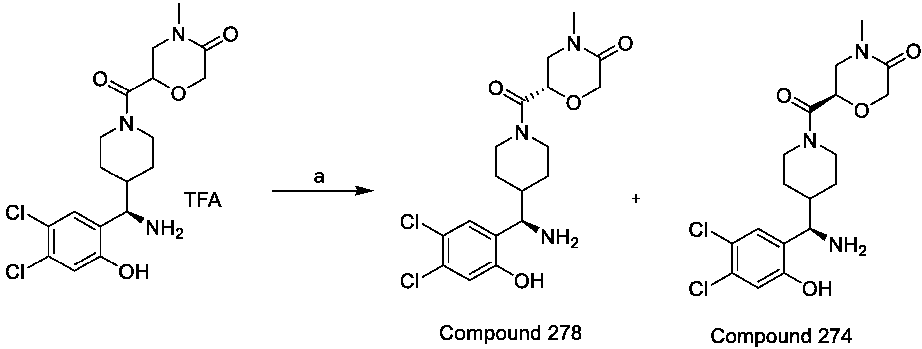

### Step a:

6-[4-[(R)-amino(4,5-dichloro-2-hydroxyphenyl)methyl]piperidine-1-carbonyl]-4-methylmorpholin-3-one trifluoroacetic acid (66.5 mg 0.13 mmol) was separated by Prep-chiral HPLC with following conditions: Column: CHIRALPAK IF, 2 x 25 cm, 5 µm; Mobile Phase A: Hexane (plus 0.2% IPA), Mobile Phase B: EtOH; Flow rate: 19 mL/min; Gradient: 40% B to 40% B in 22 min; Detector: UV 220/254 nm; Retention time: RT₁:12.67 min; RT₂: 17.32 min; Injection Volume: 0.6 mL.

The faster-eluting enantiomer was obtained as Compound 278 (6-[4-[(R)-amino(4,5-dichloro-2-hydroxyphenyl)methyl]piperidine-1-carbonyl]-4-methylmorpholin-3-one isomer 2) as an off-white solid (18.1 mg, 11 %) at 12.67 min: LCMS (ESI) calc'd for C₁₈H₂₃Cl₂N₃O₄ [M + H]⁺: 416, 418 (3 : 2), found 416, 418 (3 : 2); ¹H NMR (400 MHz, CD₃OD) δ 7.23 (s, 1H), 6.88 (s, 1H), 4.81-4.66 (m, 1H), 4.53 (dd, *J* = 41.3, 13.4 Hz, 1H), 4.30-4.16 (m, 2H), 4.14-3.97 (m, 1H), 3.91 (dd, *J* = 18.3, 7.7 Hz, 1H), 3.74 (dd, *J* = 12.1, 8.6 Hz, 1H), 3.45-3.35 (m, 1H), 3.19-2.94 (m, 4H), 2.72-2.53 (m, 1H), 2.16-1.92 (m, 2H), 1.59-1.43 (m, 1H), 1.38-1.04 (m, 2H).

The slower-eluting enantiomer was obtained as Compound 274 (6-[4-[(R)-amino(4,5-dichloro-2-hydroxyphenyl)methyl]piperidine-1-carbonyl]-4-methylmorpholin-3-one isomer 1) as an off-white solid (17.3 mg, 11%) at 17.32 min: LCMS (ESI) calc'd for C₁₈H₂₃Cl₂N₃O₄ [M + H]⁺: 416, 418 (3 : 2), found 416, 418 (3 : 2); ¹H NMR (400 MHz, CD₃OD) δ 7.23 (d, *J =* 7.5 Hz, 1H), 6.88 *(d, J* = 3.0 Hz, 1H), 4.75 (td, *J* = 8.2, 3.8 Hz, 1H), 4.52 (dd, *J =* 35.4, 13.3 Hz, 1H), 4.31-4.06 (m, 2H), 4.12-3.98 (m, 1H), 3.91 (dd, *J* = 7.6, 2.4 Hz, 1H), 3.80-3.68 (m, 1H), 3.46-3.35 (m, 1H), 3.12-2.98 (m, 4H), 2.75-2.51 (m, 1H), 2.16-1.95 (m, 2H), 1.51 (t, *J* = 15.2 Hz, 1H), 1.40-1.04 (m, 2H).

### Example 220. Compound 276 (6-[4-[(R)-amino(4,5-dichloro-2-hydroxyphenyl)methyl]piperidine-1-carbonyl]morpholin-3-one isomer 2)

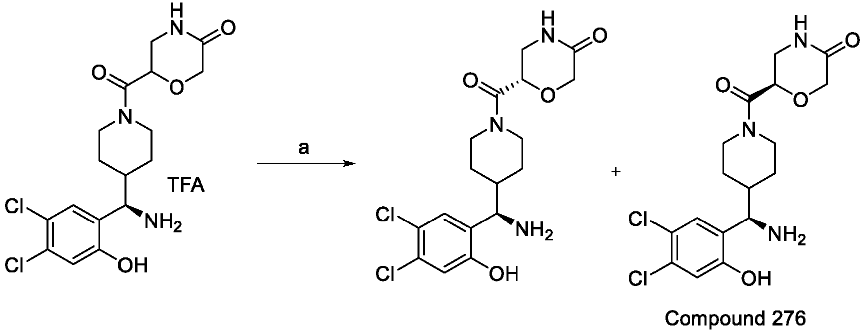

### Step a:

6-[4-[(*R*)-amino(4,5-dichloro-2-hydroxyphenyl)methyl]piperidine-1-carbonyl]morpholin-3-one (0.12 g, 0.23 mmol) was separated by Chiral Prep-HPLC with the following conditions: Column: CHIRALPAK IG, 2 x 25 cm, 5 µm; Mobile Phase A: MTBE (plus 0.2% IPA), Mobile Phase B: EtOH; Flow rate: 20 mL/min; Gradient: 10% B to 10% B in 21 min; Detector: UV 220/254 nm; Retention time: RT₁: 12.96 min; RT₂: 18.67 min; Injection Volumn:0.3 mL; Number Of Runs: 17.

The faster-eluting enantiomer was obtained as (6*S*)-6-[4-[(*R*)-amino(4,5-dichloro-2-hydroxyphenyl)methyl]piperidine-1-carbonyl]morpholin-3-one as an off-white solid (37 mg, 41%) at 12.96 min: LCMS (ESI) calc'd for C₁₇H₂₁Cl₂N₃O₄ [M + H]⁺: 402, 404 (3 : 2), found 402, 404 (3 : 2); ¹H NMR (400 MHz, CD₃OD) δ 7.23 (d, *J* = 5.6 Hz, 1H), 6.88 (s, 1H), 4.70-4.43 (m, 2H), 4.34-3.98 (m, 2H), 3.98-3.76 (m, 1H), 3.69-3.57 (m, 1H), 3.45-3.34 (m, 1H), 3.21-2.89 (m, 1H), 2.78-2.50 (m, 1H), 2.28-1.65 (m, 3H), 1.60-1.40 (m, 1H), 1.40-1.02 (m, 2H).

The slower-eluting enantiomer was obtained as Compound 276 (6-[4-[(R)-amino(4,5-dichloro-2-hydroxyphenyl)methyl]piperidine-1-carbonyl]morpholin-3-one isomer 2) as an off-white solid (41 mg, 44%): LCMS (ESI) calc'd for C₁₇H₂₁Cl₂N₃O₄ [M + H]⁺: 402, 404 (3 : 2), found 402, 404 (3 : 2); ¹H NMR (400 MHz, CD₃OD) δ 7.24 (s, 1H), 6.88 (s, 1H), 4.72-4.43 (m, 2H), 4.24 (qd, *J* = 16.6, 3.7 Hz, 2H), 4.07 (dd, *J* = 40.0, 13.5 Hz, 1H), 3.91 (dd, *J* = 16.3, 7.7 Hz, 1H), 3.65 (dd, *J* = 12.8, 9.0 Hz, 1H), 3.45-3.35 (m, 1H), 3.19-2.92 (m, 1H), 2.72-2.56 (m, 1H), 2.12-1.94 (m, 2H), 1.56-1.40 (m, 1H), 1.40-1.11 (m, 2H).

### Example 221. (which is a reference example) Compound 318 ((2R)-1-[4-[(R)-amino[5-chloro-4-(cyclopent-1-en-1-yl)-2-hydroxyphenyl]methyl]piperidin-1-yl]-2,3-dihydroxypropan-1-one trifluoroacetic acid)

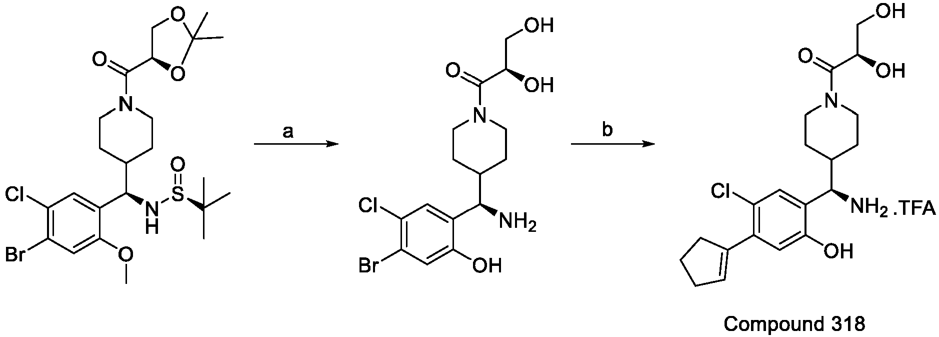

### Step a:

To a stirred mixture of (*S*)-*N*-[(*R*)-(4-bromo-5-chloro-2-methoxyphenyl)([1-[(4*R*)-2,2-dimethyl-1,3-dioxolane-4-carbonyl]piperidin-4-yl])methyl]-2-methylpropane-2-sulfinamide (Intermediate 23, Example 23) (0.10 g, 0.18 mmol) in DCM (2 mL) were added BBr₃ (0.27 g, 1.06 mmol) at room temperature. The resulting mixture was stirred for 2 h at room temperature. The reaction was quenched with water (3 mL) at 0 °C. The resulting mixture was concentrated under reduced pressure. The residue was purified by reverse phase chromatography, eluted with 30% ACN in water (plus 0.05% TFA) to afford (2*R*)-1-[4-[(*R*)-amino(4-bromo-5-chloro-2-hydroxyphenyl)methyl]piperidin-1-yl]-2,3-dihydroxypropan-1-one as a light yellow oil (60 mg, 67%): LCMS (ESI) calc'd for C₁₅H₂₀BrClN₂O₄ [M + H]⁺: 407, 409, 411 (2 : 3 : 1), found 407, 409, 411 (2 : 3 : 1); ¹H NMR (400 MHz, CD₃OD) δ 7.43 (s, 1H), 7.25 (s, 1H), 4.71-4.43 (m, 1H), 4.30-4.05 (m, 2H), 3.76-3.58 (m, 2H), 3.19-2.95 (m, 1H), 2.67 (dt, *J =* 42.6, 12.9 Hz, 1H), 2.48-2.31 (m, 1H), 2.08-1.97 (m, 2H), 1.48-1.07 (m, 3H).

### Step b:

To a solution of (2*R*)-1-[4-[(*R*)-amino(4-bromo-5-chloro-2-hydroxyphenyl)methyl]piperidin-1-yl]-2,3-dihydroxypropan-1-one (30 mg, 0.07 mmol) and 2-(cyclopent-1-en-1-yl)-4,4,5,5-tetramethyl-1,3,2-dioxaborolane (29 mg, 0.15 mmol) in 1,4-dioxane (2 mL) and water (0.5 mL) were added Na₂CO₃ (23 mg, 0.22 mmol) and Pd(dppf)Cl₂·CH₂Cl₂ (12 mg, 0.02 mmol). The reaction mixture was degassed with nitrogen for three times. After stirring for 2 h at 80 °C under nitrogen atmosphere, the resulting mixture was cooled to room temperature and diluted with EA (15 mL). The resulting solution was extracted with aq. HCl (1 *N,* 3 x 10 mL). The combined aqueous layers were concentrated under reduced pressure. The residue was purified by Prep-HPLC with the following conditions: Column: Xselect CSH OBD Column 30 x 150 mm 5µm; Mobile Phase A: water (plus 0.05% TFA), Mobile Phase B: ACN; Flow rate: 60 mL/min; Gradient: 8% B to 35% B in 7 min; Detector: UV 220/254 nm; Retention time: 6.50 min. The fractions containing the desired product were collected and concentrated under reduced pressure to afford Compound 318 ((2*R*)-1-[4-[(*R*)-amino[5-chloro-4-(cyclopent-1-en-1-yl)-2-hydroxyphenyl]methyl]piperidin-1-yl]-2,3-dihydroxypropan-1-one trifluoroacetic acid) as an off-white solid (12 mg, 33%): LCMS (ESI) calc'd for C₂₀H₂₇ClN₂O₄ [M + H]⁺: 395, 397 (3 : 1), found 395, 397 (3 : 1); ¹H NMR (400 MHz, CD₃OD) δ 7.28 (s, 1H), 6.88 (s, 1H), 6.14 (q, *J =* 2.3 Hz, 1H), 4.71-4.46 (m, 2H), 4.28-4.03 (m, 2H), 3.83-3.56 (m, 2H), 3.17-2.95 (m, 1H), 2.78-2.61 (m, 3H), 2.59-2.51 (m, 2H), 2.48-2.30 (m, 1H), 2.08-1.88 (m, 3H), 1.50-1.11 (m, 3H).

### Example 222. (which is a reference example) Compound 282 ((2R)-1-(4-((R)-amino(5-chloro-2-hydroxy-4-isopropylphenyl)methyl)piperidin-1-yl)-2,3-dihydroxypropan-1-one)

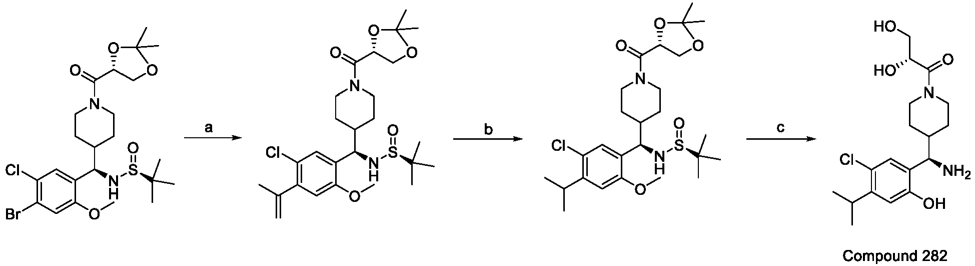

### Step a:

To a stirred solution of (*S*)-*N*-[(*R*)-(4-bromo-5-chloro-2-methoxyphenyl)([1-[(4*R*)-2,2-dimethyl-1,3-dioxolane-4-carbonyl]piperidin-4-yl])methyl]-2-methylpropane-2-sulfinamide (Intermediate 23, Example 23) (0.50 g, 0.88 mmol) and 4,4,5,5-tetramethyl-2-(prop-1-en-2-yl)-1,3,2-dioxaborolane (0.22 g, 1.32 mmol) in 1,4-dioxane (5 mL) and H₂O (1.25 mL) were added Na₂CO₃ (0.28 g, 2.65 mmol) and Pd(dppf)Cl₂·CH₂Cl₂ (72 mg, 0.08 mmol) at room temperature under nitrogen atmosphere. The resulting mixture was degassed with nitrogen for three times and stirred for 2 h at 80 °C under nitrogen atmosphere. The resulting mixture was diluted with EA (20 mL) and water (30 mL). The aqueous layer was extracted with EA (3 x 40 mL). The combined organic layers were washed with brine (3 x 20 mL) and dried over anhydrous Na₂SO₄. After filtration, the filtrate was concentrated under reduced pressure. The residue was purified by reverse phase chromatography, eluted with 50% ACN in water (plus 0.05% TFA) to afford (*S*)-*N*-[(*R*)-[5-chloro-2-methoxy-4-(prop-1-en-2-yl)phenyl]([1-[(4*R*)-2,2-dimethyl-1,3-dioxolane-4-carbonyl]piperidin-4-yl])methyl]-2-methylpropane-2-sulfinamide as a yellow solid (0.37 g, 79%): LCMS (ESI) calc'd for C₂₆H₃₉ClN₂O₅S [M + H]⁺: 527, 529 (3 : 1), found 527, 529 (3 : 1); ¹H NMR (400 MHz, CDCl₃) δ 7.11 (d, *J* = 20.5 Hz, 1H), 6.73 (s, 1H), 5.30-5.22 (m, 1H), 5.02 (d, *J* = 5.1 Hz, 1H), 4.74-4.61 (m, 2H), 4.61-4.43 (m, 1H), 4.43-4.28 (m, 1H), 4.28-4.05 (m, 2H), 3.84 (s, 3H), 3.12-2.85 (m, 1H), 2.55 (t, *J =* 12.7 Hz, 1H), 2.13 (s, 3H), 2.05-1.91 (m, 2H), 1.68-1.48 (m, 3H), 1.45-1.38 (m, 6H), 1.18 (d, *J* = 12.9 Hz, 9H).

### Step b:

To a stirred solution of (S)-N-[(R)-[5-chloro-2-methoxy-4-(prop-1-en-2-yl)phenyl]([1-[(4R)-2,2-dimethyl-1,3-dioxolane-4-carbonyl]piperidin-4-yl])methyl]-2-methylpropane-2-sulfinamide (0.10 g, 0.19 mmol) in MeOH (2 mL) was added PtO₂ (30 mg, 0.13 mmol) at room temperature under air atmosphere. The resulting mixture was degassed with hydrogen for three times and stirred for 1 h at room temperature under hydrogen atmosphere (1.5 atm). The resulting mixture was filtered. The filter cake was washed with MeOH (3 x 30 mL). The filtrate was concentrated under reduced pressure to afford (*S*)-*N*-[(*R*)-(5-chloro-4-isopropyl-2-methoxyphenyl)([1-[(4*R*)-2,2-dimethyl-1,3-dioxolane-4-carbonyl]piperidin-4-yl])methyl]-2-methylpropane-2-sulfinamide as a brown oil (0.10 g, 99%): LCMS (ESI) calc'd for C₂₆H₄₁ClN₂O₅S [M + H]⁺: 529, 531 (3 : 1), found 529, 531 (3 : 1); ¹H NMR (400 MHz, CD₃OD) δ 7.26 (d, *J =* 2.0 Hz, 1H), 6.93 (s, 1H), 4.58-4.35 (m, 2H), 4.30-3.98 (m, 3H), 3.87 (s, 3H), 3.44-3.37 (m, 1H), 3.18-2.88 (m, 1H), 2.63 (dt, *J* = 23.0, 12.6 Hz, 1H), 2.26-1.97 (m, 3H), 1.48-1.30 (m, 7H), 1.30-1.21 (m, 8H), 1.14 (d, *J =* 7.7 Hz, 9H).

### Step c:

To a stirred solution of (*S*)-*N*-[(*R*)-(5-chloro-4-isopropyl-2-methoxyphenyl)[1-(2,2-dimethyl-1,3-dioxolane-4-carbonyl)piperidin-4-yl]methyl]-2-methylpropane-2-sulfinamide (0.10 g, 0.19 mmol) in DCM (2 mL) was added BBr₃ (0.47 g, 1.89 mmol) at room temperature. The resulting mixture was stirred for 1 h at room temperature. The reaction was quenched with MeOH (2 mL) at 0°C. The mixture was neutralized to pH 7 with saturated aq. NaHCO₃ (5 mL). The resulting mixture was concentrated under reduced pressure. The residue was purified by Prep-HPLC with the following conditions: Column: XBridge Shield RP18 OBD Column 30 x 150 mm, 5 µm; Mobile Phase A: water with 10 mmol/L NH₄HCO₃, Mobile Phase B: ACN; Flow rate: 60 mL/min; Gradient: 25% B to 45% B in 7 min; Detector: UV 254/220 nm; Retention time: 6.50 min. The fractions containing the desired product were collected and concentrated under reduced pressure to afford Compound 282 ((2*R*)-1-[4-[(*R*)-amino(5-chloro-2-hydroxy-4-isopropylphenyl)methyl]piperidin-1-yl]-2,3-dihydroxypropan-1-one) as an off-white solid (16 mg, 22% over two steps): LCMS (ESI) calc'd for C₁₈H₂₇ClN₂O₄ [M + H]⁺: 371, 373 (3 : 1), found 371, 373 (3 : 1); ¹H NMR (400 MHz, CD₃OD) δ 7.10 (s, 1H), 6.76 (s, 1H), 4.65-4.44 (m, 2H), 4.11 (dd, *J =* 46.7, 13.7 Hz, 1H), 3.85 (d, *J =* 7.2 Hz, 1H), 3.78-3.55 (m, 2H), 3.30-3.26 (m, 1H), 3.11-2.93 (m, 1H), 2.75-2.49 (m, 1H), 2.07-1.93 (m, 2H), 1.58-1.38 (m, 1H), 1.38-1.07 (m, 8H).

### Example 223. Compound 283 ((2R)-1-[4-[(R)-amino(5-fluoro-2-hydroxy-4-methylphenyl)methyl]piperidin-1-yl]-2,3-dihydroxypropan-1-one)

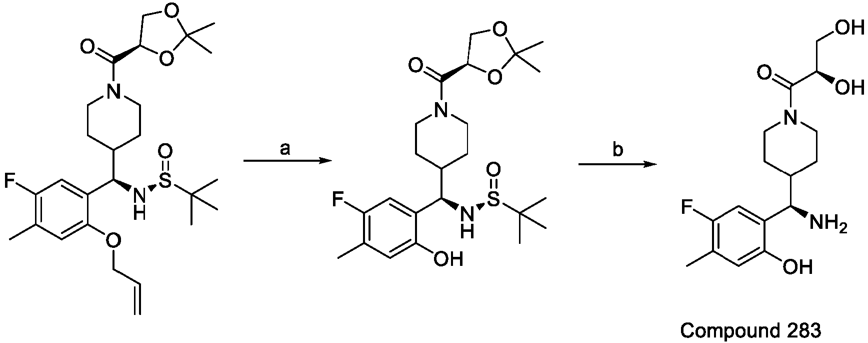

### Step a:

To a stirred solution of (*S*)-*N*-[(*R*)-[1-[(4*R*)-2,2-dimethyl-1,3-dioxolane-4-carbonyl]piperidin-4-yl][5-fluoro-4-methyl-2-(prop-2-en-1-yloxy)phenyl]methyl]-2-methylpropane-2-sulfinamide (30 mg, 0.06 mmol) and Pd(PPh₃)₄ (7 mg, 0.006 mmol) in THF (2 mL) was added NaBH₄ (5 mg, 0.12 mmol) at room temperature under nitrogen atmosphere. The resulting mixture was stirred for 30 min at room temperature under nitrogen atmosphere. The reaction was quenched with saturated aq. NH₄Cl (2 mL) at room temperature. And concentrated under reduced pressure to afford (*S*)-*N*-[(*R*)-[1-[(4*R*)-2,2-dimethyl-1,3-dioxolane-4-carbonyl]piperidin-4-yl](5-fluoro-2-hydroxy-4-methylphenyl)methyl]-2-methylpropane-2-sulfinamide as a light yellow solid (20 mg, crude): LCMS (ESI) calc'd for C₂₃H₃₅FN₂O₅S [M + H]⁺: 471, found 471.

### Step b:

A solution of (*S*)-*N*-[(*R*)-[1-[(4*R*)-2,2-dimethyl-1,3-dioxolane-4-carbonyl]piperidin-4-yl](5-fluoro-2-hydroxy-4-methylphenyl)methyl]-2-methylpropane-2-sulfinamide (20 mg, 0.042 mmol) and aq. HCl (6 *N,* 0.50 mL) in 1,4-dioxane (1 mL) was stirred for 1 h at room temperature under air atmosphere. The resulting mixture was concentrated under reduced pressure. The residue was purified by Prep-HPLC with the following conditions: Column: XBridge Prep C18 OBD Column, 5 µm, 19 x 150 mm; Mobile Phase A: water with 10 mmol/L NH₄HCO₃, Mobile Phase B: ACN; Flow rate: 25 mL/min; Gradient: 5% B to 32% B in 7 min; Detector: UV 220 nm; Retention time: 7.38 min. The fractions containing desired product was collected and concentrated under reduced pressure to afford Compound 283 ((2*R*)-1-[4-[(*R*)-amino(5-fluoro-2-hydroxy-4-methylphenyl)methyl]piperidin-1-yl]-2,3-dihydroxypropan-1-one) as an off-white solid (4 mg, 29%): LCMS (ESI) calc'd for C₁₆H₂₃FN₂O₄ [M + H]⁺: 327, found 327; ¹H NMR (400 MHz, CD₃OD) δ 6.82 (d, *J =* 10.2 Hz, 1H), 6.63 (d, *J =* 6.7 Hz, 1H), 4.61 (d, *J* = 13.6 Hz, 1H), 4.53-4.46 (m, 1H), 4.11 (dd, *J =* 48.1, 13.8 Hz, 1H), 3.88 (d, *J =* 8.3 Hz, 1H), 3.70-3.57 (m, 2H), 3.09-2.99 (m, 1H), 2.70-2.60 (m, 1H), 2.19 (s, 3H), 2.07-1.94 (m, 2H), 1.51-1.42 (m, 1H), 1.40-1.15 (m, 2H); ¹⁹F NMR (376 MHz, CD₃OD) δ -131.97.

### Example 224. (which is a reference example) Compound 285 (2R)-1-[4-[(R)-amino(5-chloro-4-fluoro-2-hydroxyphenyl)methyl]piperidin-1-yl]-2,3-dihydroxypropan-1-one trifluoroacetic acid

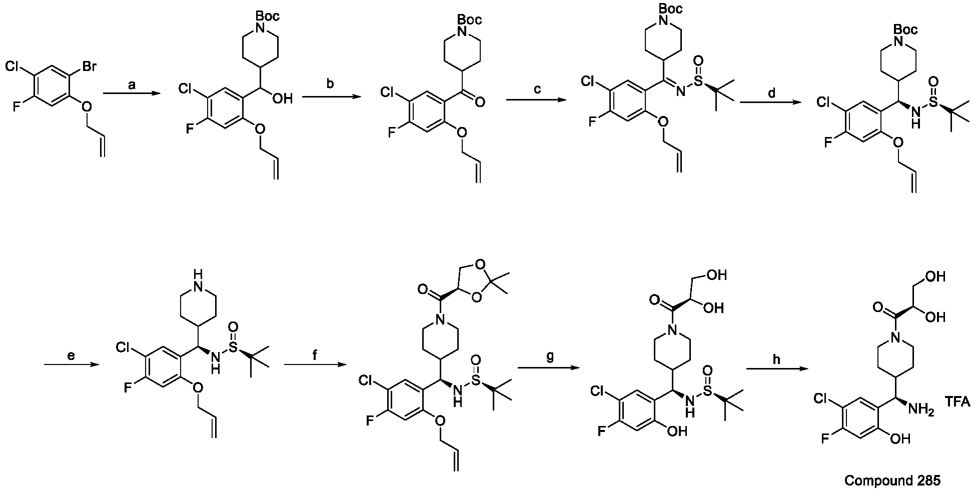

### Step a:

To a stirred solution of 1-bromo-5-chloro-4-fluoro-2-(prop-2-en-1-yloxy)benzene (Intermediate 21, Example 21) (0.60 g, 2.25 mmol) in THF (3 mL) was added i-PrMgCl (2.25 mL, 2.25 mmol, 2 M in THF) dropwise at 0 °C under nitrogen atmosphere. The reaction mixture was stirred at 0 °C for 30 min. Then a solution of *tert*-butyl 4-formylpiperidine-1-carboxylate (0.40 g, 1.88 mmol) in THF (4 mL) was added dropwise into above solution at 0 °C. The resulting solution was stirred at 0 °C for additional 1 h. The reaction was quenched with saturated aq. NH₄Cl (1 mL). The resulting mixture was diluted with water (30 mL), and then the aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (2/1) to afford *tert*-butyl 4-[[5-chloro-4-fluoro-2-(prop-2-en-1-yloxy)phenyl](hydroxy)methyl]piperidine-1-carboxylate as a light yellow oil (0.60 g, 80%): LCMS (ESI) calc'd for C₂₀H₂₇ClFNO₄ [M + H]⁺: 400, 402 (3 :1), found 400, 402 (3 :1); ¹H NMR (400 MHz, CDCl₃) δ 7.36 (d, *J =* 8.4 Hz, 1H), 6.70 (d, *J =* 10.8 Hz, 1H), 6.10-5.96 (m, 1H), 5.39 (dd, *J =* 24.6, 13.8 Hz, 2H), 4.71 (d, *J =* 7.1 Hz, 1H), 4.55 (d, *J =* 4.6 Hz, 2H), 4.24-4.06 (m, 3H), 2.77-2.50 (m, 2H), 1.95-1.68 (m, 2H), 1.47 (d, *J =* 2.6 Hz, 9H), 1.45-1.20 (m, 3H).

### Step b:

To a stirred solution of *tert*-butyl 4-[[5-chloro-4-fluoro-2-(prop-2-en-1-yloxy)phenyl](hydroxy)methyl]piperidine-1-carboxylate (0.60 g, 1.50 mmol) in DCM (4 mL) was added Dess-Martin reagent (0.95 g, 2.25 mmol) at room temperature. The reaction mixture was stirred for 1 h at room temperature. The reaction was quenched with saturated aq. Na₂SO₃ (5 mL). The resulting mixture was diluted with EA (30 mL) and water (30 mL), and then the aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (8/1) to afford *tert*-butyl 4-[5-chloro-4-fluoro-2-(prop-2-en-1-yloxy)benzoyl]piperidine-1-carboxylate as a light yellow oil (0.60 g, 98%): LCMS (ESI) calc'd for C₂₀H₂₅ClFNO₄ [M + Na]⁺: 420, 422 (3 : 1), found 420, 422 (3 : 1); ¹H NMR (400 MHz, CDCl₃) δ 7.72 (d, *J =* 8.6 Hz, 1H), 6.79 (d, *J =* 10.6 Hz, 1H), 6.15-5.98 (m, 1H), 5.58-5.33 (m, 2H), 4.62 (d, *J* = 5.7 Hz, 2H), 4.29-4.06 (m, 3H), 3.47-3.33 (m, 1H), 2.93-2.62 (m, 3H), 1.96-1.68 (m, 2H), 1.48 (s, 9H).

### Step c:

To a stirred solution of *tert*-butyl 4-[5-chloro-4-fluoro-2-(prop-2-en-1-yloxy)benzoyl]piperidine-1-carboxylate (0.60 g, 1.51 mmol) and (*S*)-2-methylpropane-2-sulfinamide (0.55 g, 4.52 mmol) in THF (4 mL) was added Ti(OEt)₄ (3.44 g, 15.08 mmol) at room temperature. The reaction was allowed to warm to 70 °C and stirred for 16 h under nitrogen atmosphere. The reaction solution was quenched with saturated aq. Na₂CO₃ (30 mL). The resulting mixture was filtered through celite. The filtrate was diluted with water (30 mL) and extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (2/1) to afford *tert*-butyl 4-[[5-chloro-4-fluoro-2-(prop-2-en-1-yloxy)phenyl]([[(*S*)-2-methylpropane-2-sulfinyl]imino])methyl]piperidine-1-carboxylate as a light yellow oil (0.53 g, 70%): LCMS (ESI) calc'd for C₂₄H₃₄ClFN₂O₄S [M + H]⁺: 501, 503 (3 : 1), found 501, 503 (3 : 1); ¹H NMR (400 MHz, CDCl₃) δ 7.12 (s, 1H), 6.72 (d, 2.9 Hz, 1H), 6.09-5.91 (m, 1H), 5.37 (dd, *J =* 33.6, 14.0 Hz, 2H), 4.58 (s, 2H), 4.23-4.07 (m, 1H), 2.90-2.55 (m, 4H), 1.95-1.83 (m, 1H), 1.83-1.64 (m, 1H), 1.65-1.54 (m, 2H), 1.48 (s, 9H), 1.23 (s, 9H)

### Step d:

To a stirred solution of tert-butyl 4-[(*1E*)-[5-chloro-4-fluoro-2-(prop-2-en-1-yloxy)phenyl]([[(*S*)-2-methylpropane-2-sulfinyl]imino])methyl]piperidine-1-carboxylate (0.53 g, 1.06 mmol) in THF (5 mL) was added DIBAL-H (2.08 mL, 2.08 mmol, 1M in toluene) dropwise at -70 °C under nitrogen atmosphere. The resulting solution was stirred for 1 h at -70 °C under nitrogen atmosphere. The reaction was quenched with saturated aq. NH₄Cl (1 mL). The resulting mixture was diluted with EA (30 mL) and water (30 mL), and then the aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (2/1) to afford *tert*-butyl 4-[(*R*)-[5-chloro-4-fluoro-2-(prop-2-en-1-yloxy)phenyl]([[(*S*)-2-methylpropane-2-sulfinyl]amino])methyl]piperidine-1-carboxylate as an off-white solid (0.43 g, 81%): LCMS (ESI) calc'd for C₂₄H₃₆ClFN₂O₄S [M + H]⁺: 503, 505 (3 : 1), found 503, 505 (3 : 1); ¹H NMR (400 MHz, CDCl₃) δ 7.18 (d, *J =* 8.3 Hz, 1H), 6.71 (d, *J =* 10.7 Hz, 1H), 6.11-5.93 (m, 1H), 5.48-5.28 (m, 2H), 4.64-4.39 (m, 2H), 4.31-4.04 (m, 1H), 4.23-4.04 (m, 1H), 3.83-3.61 (m, 1H), 2.74-2.52 (m, 2H), 2.03-1.78 (m, 2H), 1.62-1.60 (m, 3H), 1.46 (s, 9H), 1.17 (s, 9H).

### Step e:

To a stirred solution of *tert*-butyl 4-[(*R*)-[5-chloro-4-fluoro-2-(prop-2-en-1-yloxy)phenyl]([[(*S*)-2-methylpropane-2-sulfinyl]amino])methyl]piperidine-1-carboxylate (0.20 g, 0.40 mmol) in DCM (5 mL) was added TFA (1 mL) at room temperature. The reaction was stirred at room temperature for 0.5 h. The reaction was neutralized with saturated aq. Na₂CO₃ to pH 8. The resulting mixture was diluted with water (20 mL) and extracted with EA (3 x 50 mL). The combined organic layers were washed with brine (3 x 40 mL), dried over anhydrous Na₂SO₄. After filtration, the filtrate was concentrated under reduced pressure to afford (*S*)-*N-*[(*R*)-[5-chloro-4-fluoro-2-(prop-2-en-1-yloxy)phenyl](piperidin-4-yl)methyl]-2-methylpropane-2-sulfinamide as a light yellow oil (0.16 g, crude), which was used in the next step directly without further purification: LCMS (ESI) calc'd for C₁₉H₂₈ClFN₂O₂S [M + H]⁺: 403, 405 (3 : 1), found 403, 405 (3 : 1); ¹H NMR (400 MHz, CDCl₃) δ 7.20 (d, *J =* 8.3 Hz, 1H), 6.73 (d, *J =* 10.6 Hz, 1H), 6.09-5.89 (m, 1H), 5.48-5.31 (m, 2H), 4.68-4.49 (m, 2H), 4.49-4.32 (m, 1H), 4.32-4.23 (m, 1H), 3.42 (dd, *J =* 33.0, 12.6 Hz, 1H), 2.91-2.71 (m, 2H), 2.38-2.12 (m, 1H), 2.12-1.94 (m, 1H), 1.78-1.51 (m, 3H), 1.14 (s, 9H).

### Step f:

To a stirred solution of (*4R*)-2,2-dimethyl-1,3-dioxolane-4-carboxylic acid (87 mg, 0.60 mmol) and HATU (0.23 g, 0.60 mmol) in DMF (2 mL) were added (*S*)-*N*-[(*R*)-[5-chloro-4-fluoro-2-(prop-2-en-1-yloxy)phenyl](piperidin-4-yl)methyl]-2-methylpropane-2-sulfinamide (0.16 g, 0.40 mmol) and Et₃N (80 mg, 0.79 mmol) at room temperature. The reaction was stirred at room temperature for 0.5 h. The resulting solution was quenched with water (50 mL) and extracted with EA (3 x 40 mL). The combined organic layers were washed with brine (2 x 30 mL), dried over anhydrous Na₂SO₄. After filtration, the filtrate was concentrated under reduced pressure. The residue was purified by reverse phase chromatography, eluted with 58% ACN in water (plus 0.05% TFA) to afford (*S*)-*N*-[(*R*)-[5-chloro-4-fluoro-2-(prop-2-en-1-yloxy)phenyl]([1-[(*4R*)-2,2-dimethyl-1,3-dioxolane-4-carbonyl]piperidin-4-yl])methyl]-2-methylpropane-2-sulfinamide as a colorless oil (0.18 g, 85%): LCMS (ESI) calc'd for C₂₅H₃₆ClFN₂O₅S [M + H]⁺: 531, 533 (3 : 1), found 531, 533 (3 : 1).

### Step g:

To a stirred solution of (*S*)-*N*-[(*R*)-[5-chloro-4-fluoro-2-(prop-2-en-1-yloxy)phenyl]([1-[(*4R*)-2,2-dimethyl-1,3-dioxolane-4-carbonyl]piperidin-4-yl])methyl]-2-methylpropane-2-sulfinamide (0.18 g, 0.34 mmol) and Pd(PPh₃)₄ (4 mg, 0.003 mmol) in THF (3 mL) was added NaBH₄ (26 mg, 0.69 mmol) at room temperature. The reaction was stirred at room temperature for 1 h. The reaction solution was quenched with water (0.5 mL) and concentrated under reduced pressure to afford (*S*)-*N*-[(*R*)-(5-chloro-4-fluoro-2-hydroxyphenyl)([1-[(2*R*)-2,3-dihydroxypropanoyl]piperidin-4-yl])methyl]-2-methylpropane-2-sulfinamide as an off-white solid (0.15 g, crude), which was used in next step directly without further purification: LCMS (ESI) calc'd for C₁₉H₂₈ClFN₂O₅S [M + H]⁺: 451, 453 (3 : 1), found 451, 453 (3 : 1);

### Step h:

To a stirred solution of (*S*)-*N*-[(*R*)-(5-chloro-4-fluoro-2-hydroxyphenyl)([1-[(2*R*)-2,3-dihydroxypropanoyl]piperidin-4-yl])methyl]-2-methylpropane-2-sulfinamide (0.13 g, 0.29 mmol) in 1,4-dioxane (2 mL) was added aq. HCl (4 *N,* 2 mL) at room temperature. The resulting solution was stirred for 1 h at room temperature. The reaction was concentrated under reduced pressure. The residue was purified by Prep-HPLC with following conditions: Column: Xselect CSH OBD Column 30 x 150 mm 5 µm; Mobile Phase A: water (plus 0.05% TFA), Mobile Phase B: ACN; Flow rate: 60 mL/min; Gradient: 5% B to 18% B in 7 min; Detector: UV 254/220 nm; Retention time: 5.95 min. The fractions containing the desired product were collected and concentrated under reduced pressure to afford Compound 285 ((2*R*)-1-[4-[(*R*)-amino(5-chloro-4-fluoro-2-hydroxyphenyl)methyl]piperidin-1-yl]-2,3-dihydroxypropan-1-one trifluoroacetic acid) as an off-white solid (30 mg, 25% overall two steps): LCMS (ESI) calc'd for C₁₅H₂₀ClFN₂O₄ [M + H]⁺: 347, 349 (3 : 1), found 347, 349 (3 : 1); ¹H NMR (400 MHz, CD₃OD) δ 7.39 (d, *J =* 8.1 Hz, 1H), 6.81 (d, *J =* 10.5 Hz, 1H), 4.72-4.45 (m, 2H), 4.36-3.97 (m, 2H), 3.77-3.57 (m, 2H), 3.20-2.95 (m, 1H), 2.81-2.56 (m, 1H), 2.46-2.29 (m, 1H), 2.14-1.94 (m, 1H), 1.51-1.09 (m, 3H); ¹⁹F NMR (376 MHz, CD₃OD) δ -77.20, -114.76.

### Example 225. (which is a reference example) Compound 286 (4-[(R)-amino([1-[(2R)-2,3-dihydroxypropanoyl]piperidin-4-yl])methyl]-2-chloro-5-hydroxybenzonitrile trifluoroacetic acid)

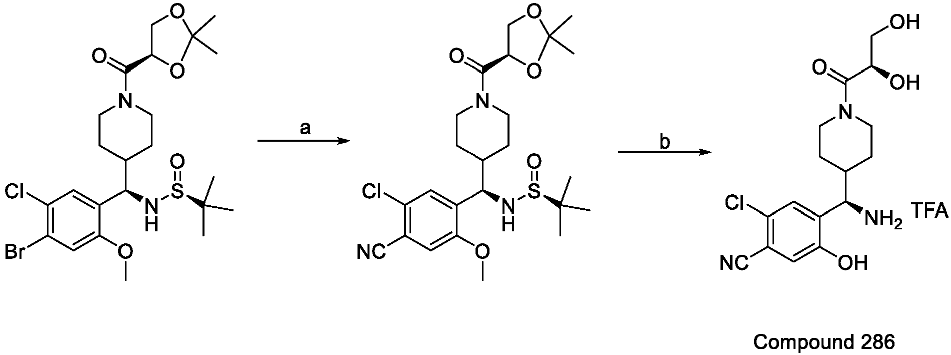

### Step a:

To a stirred mixture of (*S*)-*N*-[(*R*)-(4-bromo-5-chloro-2-methoxyphenyl)([1-[(4*R*)-2,2-dimethyl-1,3-dioxolane-4-carbonyl]piperidin-4-yl])methyl]-2-methylpropane-2-sulfinamide (Intermediate 23, Example 23) (90 mg, 0.16 mmol) and Pd(PPh₃)₄ (18 mg, 0.02 mmol) in NMP (2 mL) was added Zn(CN)₂ (37 mg, 0.32 mmol) at room temperature under nitrogen atmosphere. The reaction mixture was irradiated with microwave radiation for 2 h at 150 °C. The reaction was quenched with saturated aq. NaHCO₃ at room temperature. The resulting mixture was diluted with water (20 mL) and extracted with EA (3 x 20 mL). The combined organic layers were washed with brine (3 x 20 mL), dried over anhydrous Na₂SO₄. After filtration, the filtrate was concentrated under reduced pressure. The residue was purified by reverse phase chromatography, eluted with 50% ACN in water (plus 0.05% TFA) to afford (*S*)-*N*-[(*R*)-(5-chloro-4-cyano-2-methoxyphenyl)([1-[(4*R*)-2,2-dimethyl-1,3-dioxolane-4-carbonyl]piperidin-4-yl])methyl]-2-methylpropane-2-sulfinamide as an off-white solid (20 mg, 17%): LCMS (ESI) calc'd for C₂₄H₃₄ClN₃O₅S [M + H]⁺: 512, 514 (3 : 1), found 512, 514 (3 : 1); ¹H NMR (400 MHz, CDCl₃) δ 7.27-7.20 (m, 1H), 7.13 (s, 1H), 4.71-4.60 (m, 2H), 4.59-4.36 (m, 1H), 4.29-4.07 (m, 2H), 3.92-3.77 (m, 4H), 2.97 (dt, *J =* 63.9, 13.0 Hz, 1H), 2.53 (t, *J =* 12.8 Hz, 1H), 2.20-2.09 (m, 1H), 2.03-1.91 (m, 1H), 1.53-1.24 (m, 9H), 1.18 (d, *J =* 10.6 Hz, 9H).

### Step b:

To a stirred solution of (*S*)-*N*-[(*R*)-(5-chloro-4-cyano-2-methoxyphenyl)([1-[(4*R*)-2,2-dimethyl-1,3-dioxolane-4-carbonyl]piperidin-4-yl])methyl]-2-methylpropane-2-sulfinamide (20 mg, 0.04 mmol) in DCM (2 mL) was added BBr₃ (59 mg, 0.23 mmol) at room temperature. The resulting mixture was stirred for 2 h at room temperature. The reaction was quenched with water (1 mL) at 0 °C. The mixture was concentrated under reduced pressure. The residue was purified by Prep-HPLC with the following conditions: Column: Xselect CSH OBD Column 30 x 150 mm 5 µm, n; Mobile Phase A: Water (plus 0.05% TFA), Mobile Phase B: ACN; Flow rate: 60 mL/min; Gradient: 5% B to 23% B in 7 min; Detector: UV 220/254 nm; Retention time: 6.03 min. The fractions containing the desired product were collected and concentrated under reduced pressure to afford Compound 286 (4-[(*R*)-amino([1-[(2*R*)-2,3-dihydroxypropanoyl]piperidin-4-yl])methyl]-2-chloro-5-hydroxybenzonitrile trifluoroacetic acid) as an orange solid (4 mg, 22%): LCMS (ESI) calc'd for C₁₆H₂₀ClN₃O₄ [M + H]⁺: 354, 356 (3 : 1), found 354, 356 (3 : 1); ¹H NMR (400 MHz, CD₃OD) δ 7.55 (s, 1H), 7.27 (s, 1H), 4.71-4.44 (m, 2H), 4.30-4.05 (m, 2H), 3.76-3.56 (m, 2H), 3.20-2.95 (m, 1H), 2.66 (dt, *J =* 44.9, 12.9 Hz, 1H), 2.48-2.31 (m, 1H), 2.09-1.95 (m, 1H), 1.50-1.13 (m, 3H); ¹⁹F NMR (376 MHz, CD₃OD) δ -76.93.

### Example 226. Compound 287 ((R)-1-(4-((S)-amino(3,4-dichloro-2-fluoro-6-hydroxyphenyl)methyl)piperidin-1-yl)-2,3-dihydroxypropan-1-one); Compound 291 ((R)-1-(4-((R)-amino(3,4-dichloro-2-fluoro-6-hydroxyphenyl)methyl)piperidin-1-yl)-2,3-dihydroxypropan-1-one)

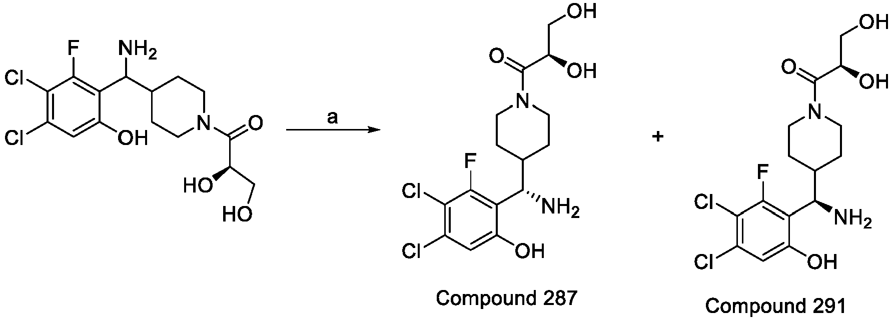

### Step a:

(2*R*)-1-[4-[amino(3,4-dichloro-2-fluoro-6-hydroxyphenyl)methyl]piperidin-1-yl]-2,3-dihydroxypropan-1-one (106.00 mg) was separated by Chiral-Prep-HPLC with the following conditions: Column: CHIRALPAK IG, 20 x 250 mm, 5 µm; Mobile Phase A: Hexane (plus 0.2% IPA), Mobile Phase B: EtOH; Flow rate: 20 mL/min; Gradient: 30% B to 30% B in 23 min; Detector: UV 220/254 nm; Retention time: RT₁: 13.338 min; RT₂: 18.463 min; Injection Volume: 0.4 ml.

The faster-eluting enantiomer was obtained as Compound 291 ((*R*)-1-(4-((*R*)-amino(3,4-dichloro-2-fluoro-6-hydroxyphenyl)methyl)piperidin-1-yl)-2,3-dihydroxypropan-1-one) as an off-white solid (31 mg, 29%) at 7.47 min: LCMS (ESI) calc'd for C₁₅H₁₉Cl₂FN₂O₄ [M + H]⁺: 381, 383 (3 : 2), found 381, 383 (3 : 2); ¹H NMR (400 MHz, CD₃OD) δ 6.66 (s, 1H), 4.68-4.44 (m, 2H), 4.27-4.04 (m, 2H), 3.81-3.50 (m, 2H), 3.21-2.96 (m, 1H), 2.77-2.59 (m, 1H), 2.31-2.16 (m, 1H), 2.05 (d, *J =* 9.6 Hz, 1H), 1.56 (d, *J =* 13.3 Hz, 1H), 1.46-1.15 (m, 2H); ¹⁹F NMR (376 MHz, CD₃OD) δ -116.15.

The slower-eluting enantiomer was obtained as Compound 287 ((R)-1-(4-((S)-amino(3,4-dichloro-2-fluoro-6-hydroxyphenyl)methyl)piperidin-1-yl)-2,3-dihydroxypropan-1-one) as an off-white solid (28 mg, 26%) at 10.05 min: LCMS (ESI) calc'd for C₁₅H₁₉Cl₂FN₂O₄ [M + H]⁺: 381, 383 (3 : 2), found 381, 383 (3 : 2); ¹H NMR (400 MHz, CD₃OD) δ 6.66 (s, 1H), 4.68-4.44 (m, 2H), 4.27-4.04 (m, 2H), 3.81-3.50 (m, 2H), 3.21-2.96 (m, 1H), 2.77-2.59 (m, 1H), 2.31-2.16 (m, 1H), 2.05 (d, *J =* 9.6 Hz, 1H), 1.56 (d, *J =* 13.3 Hz, 1H), 1.46-1.15 (m, 2H); ¹⁹F NMR (376 MHz, CD₃OD) δ -116.15.

### Example 227. (which is a reference example) Compound 288 ((2R)-1-[4-[(R)-amino(5-chloro-4-cyclobutyl-2-hydroxyphenyl)methyl]piperidin-1-yl]-2,3-dihydroxypropan-1-one trifluoroacetic acid)

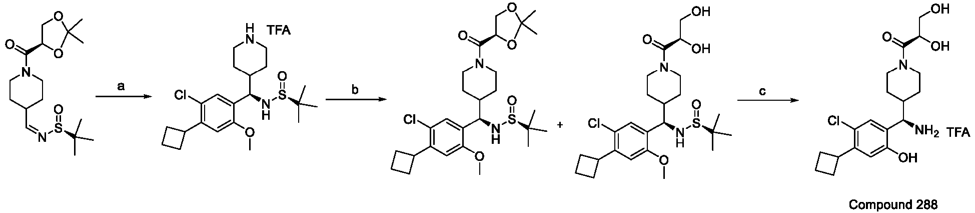

### Step a:

To a stirred solution of 1-bromo-5-chloro-4-cyclobutyl-2-methoxybenzene (0.24 g, 0.87 mmol) in THF (4 mL) was added *n*-BuLi (0.35 mL, 0.88 mmol, 2.5 M in hexane) dropwise at -78 °C under nitrogen atmosphere. The reaction solution was stirred at -78 °C for 30 min. Then a solution of (*S*)-*N*-([1-[(4*R*)-2,2-dimethyl-1,3-dioxolane-4-carbonyl]piperidin-4-yl]methylidene)-2-methylpropane-2-sulfinamide (0.15 g, 0.44 mmol) in THF (3 mL) was added into the solution. The resulting solution was stirred at -78 °C for additional 2 h. The reaction was quenched with saturated aq. NH₄Cl (1 mL) and diluted with water (30 mL). The aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by reverse phase chromatography, eluted with 43% ACN in water (plus 0.05% TFA) to afford (*S*)-*N*-[(*R*)-(5-chloro-4-cyclobutyl-2-methoxyphenyl)(piperidin-4-yl)methyl]-2-methylpropane-2-sulfinamide trifluoroacetic acid as a colorless oil (0.12 g, 64%): LCMS (ESI) calc'd for C₂₁H₃₃ClN₂O₂S [M + H]⁺: 413, 415 (3 :1), found 413, 415 (3 : 1); ¹H NMR (400 MHz, CD₃OD) δ 7.25 (s, 1H), 6.98 (s, 1H), 4.48 (d, *J* = 9.0 Hz, 1H), 3.90 (s, 3H), 3.85-3.72 (m, 1H), 3.47 (d, *J =* 13.3 Hz, 1H), 3.04-2.81 (m, 3H), 2.47-2.31 (m, 3H), 2.23-1.98 (m, 4H), 1.92-1.83 (m, 1H), 1.67-1.34 (m, 2H), 1.34-1.21 (m, 1H), 1.13 (s, 9H).

### Step b:

To a stirred solution of (*4R*)-2,2-dimethyl-1,3-dioxolane-4-carboxylic acid (64 mg, 0.44 mmol) and HATU (0.17 g, 0.45 mmol) in DMF (2 mL) were added (*S*)-*N*-[(*R*)-(5-chloro-4-cyclobutyl-2-methoxyphenyl)(piperidin-4-yl)methyl]-2-methylpropane-2-sulfinamide (0.12 g, 0.29 mmol) and Et₃N (88 mg, 0.87 mmol) at room temperature. The resulting solution was stirred for 30 min at room temperature. The resulting solution was quenched with water (50 mL) and extracted with EA (3 x 40 mL). The combined organic layers were washed with brine (2 x 30 mL), dried over anhydrous Na₂SO₄. After filtration, the filtrate was concentrated under reduced pressure. The residue was purified by reverse phase chromatography, eluted with 65% ACN in water (plus 0.05% TFA) to afford (*S*)-*N*-[(*R*)-(5-chloro-4-cyclobutyl-2-methoxyphenyl)([1-[(4*R*)-2,2-dimethyl-1,3-dioxolane-4-carbonyl]piperidin-4-yl])methyl]-2-methylpropane-2-sulfinamide as a light yellow oil (50 mg, 38%); LCMS (ESI) calc'd for C₂₇H₄₁ClN₂O₅S [M + H]⁺: 541, 543 (3 : 1), found 541, 543 (3 : 1) and (*S*)-*N*-[(*R*)-(5-chloro-4-cyclobutyl-2-methoxyphenyl)([1-[(2*R*)-2,3-dihydroxypropanoyl]piperidin-4-yl])methyl]-2-methylpropane-2-sulfinamide as a light yellow oil (30 mg, 25%): LCMS (ESI) calc'd for C₂₄H₃₇ClN₂O₅S [M + H]⁺: 501, 503 (3 : 1), found 501, 503 (3 : 1).

### Step c:

To a stirred solution of (*S*)-*N*-[(*R*)-(5-chloro-4-cyclobutyl-2-methoxyphenyl)([1-[(*4R*)-2,2-dimethyl-1,3-dioxolane-4-carbonyl]piperidin-4-yl])methyl]-2-methylpropane-2-sulfinamide (50 mg, 0.09 mmol) and (*S*)-*N*-[(*R*)-(5-chloro-4-cyclobutyl-2-methoxyphenyl)([1-[(*2R*)-2,3-dihydroxypropanoyl]piperidin-4-yl])methyl]-2-methylpropane-2-sulfinamide (30 mg, 0.06 mmol) in DCM (2 mL) was added BBr₃ (0.14 g, 0.55 mmol) at room temperature. The reaction was stirred at room temperature for 2 h. The reaction was quenched with MeOH (1 mL) at 0 °C and concentrated under reduced pressure. The residue was purified by Prep-HPLC with following conditions: Column: XBridge Prep C18 OBD Column, 5 µm, 19 x 150 mm; Mobile Phase A: water (plus 0.05% TFA), Mobile Phase B: ACN; Flow rate: 25 mL/min; Gradient: 5% B to 45% B in 7 min; Detector: UV 254/220 nm; Retention time: 6.62 min. The fractions containing the desired product were collected and concentrated under reduced pressure to afford Compound 288 ((2*R*)-1-[4-[(*R*)-amino(5-chloro-4-cyclobutyl-2-hydroxyphenyl)methyl]piperidin-1-yl]-2,3-dihydroxypropan-1-one trifluoroacetic acid) as an off-white solid (28.3 mg, 25% overall two steps): LCMS (ESI) calc'd for C₁₉H₂₇ClN₂O₄ [M + H]⁺: 383, 385 (3 : 1), found 383, 385 (3 : 1); ¹H NMR (400 MHz, CD₃OD) δ 7.22 (s, 1H), 6.94 (s, 1H), 4.70-4.38 (m, 2H), 4.32-4.03 (m, 2H), 3.87-3.56 (m, 3H), 3.19-2.92 (m, 1H), 2.67 (dt, *J* = 45.2, 12.8 Hz, 1H), 2.48-2.29 (m, 3H), 2.19-1.93 (m, 4H), 1.93-1.80 (m, 1H), 1.48-1.06 (m, 3H); ¹⁹F NMR (376 MHz, CD₃OD) δ -76.99.

### Example 228. (which is a reference example) Compound 289 ((2R)-1-[4-[(R)-amino(5-chloro-4-cyclopropyl-2-hydroxyphenyl)methyl]piperidin-1-yl]-2,3-dihydroxypropan-1-one)

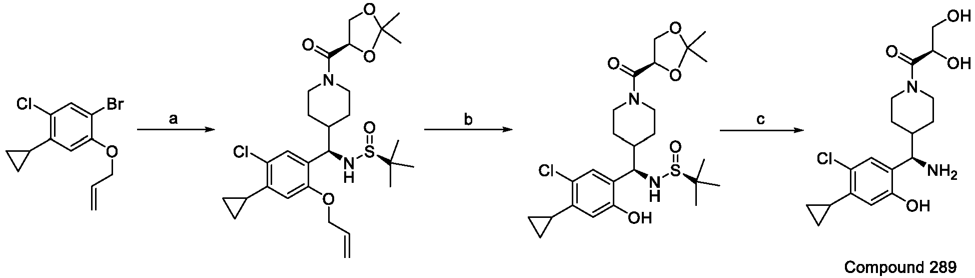

### Step a:

To a stirred solution of 1-bromo-5-chloro-4-cyclopropyl-2-(prop-2-en-1-yloxy)benzene (Intermediate 72, Example 72) (0.1 g, 0.43 mmol) in THF (3 ml) was added *n-*BuLi (0.17 mL, 0.43 mmol, 2.5 M in hexanes) dropwise at -78 °C under nitrogen atmosphere. The reaction was stirred at -78 °C for 30 min. Then (*S*)-*N*-[[1-[(4*R*)-2,2-dimethyl-1,3-dioxolane-4-carbonyl]piperidin-4-yl]methylidene]-2-methylpropane-2-sulfinamide (0.10 g, 0.29 mmol) was added in 10 min. The resulting solution was stirred at -78 °C for 2 h. The reaction was quenched with saturated aq. NH₄Cl (1 mL). The mixture was diluted with water (30 mL). The resulting mixture was extracted with EA (3 x 30 mL). The combined organic layers were washed with brine (3 x 20 mL), dried over anhydrous Na₂SO₄. After filtration, the filtrate was concentrated under reduced pressure. The residue was purified by reverse phase chromatography, eluted with 62% ACN in water (plus 0.01% TFA) to afford (*S*)-*N*-[(*S*)-[5-chloro-4-cyclopropyl-2-(prop-2-en-1-yloxy)phenyl]([1-[(4*R*)-2,2-dimethyl-1,3-dioxolane-4-carbonyl]piperidin-4-yl])methyl]-2-methylpropane-2-sulfinamide as a colorless oil (30 mg, 19%): LCMS (ESI) calc'd for C₂₈H₄₁ClN₂O₅S [M + H]⁺: 553, 555 (3 : 1), found, 553, 555 (3 : 1); ¹H NMR (400 MHz, CD₃OD) δ 7.28 (d, *J =* 3.2 Hz, 1H), 6.55 (s, 1H), 6.16-6.00 (m, 1H), 5.49-5.23 (m, 2H), 4.83-4.77 (m, 1H), 4.62-4.35 (m, 4H), 4.32-4.01 (m, 3H), 3.16-2.87 (m, 1H), 2.69-2.53 (m, 2H), 2.27-1.99 (m, 3H), 1.45-1.22 (m, 8H), 1.14 (d, *J =* 6.7 Hz, 9H), 1.05-0.98 (m, 2H), 0.76-0.66 (m, 2H).

### Step b:

To a stirred mixture of (*S*)-*N*-[(*R*)-[5-chloro-4-cyclopropyl-2-(prop-2-en-1-yloxy)phenyl]([1-[(4*R*)-2,2-dimethyl-1,3-dioxolane-4-carbonyl]piperidin-4-yl])methyl]-2-methylpropane-2-sulfinamide (35 mg, 0.06 mmol) and Pd(PPh₃)₄ (4 mg) in THF (1 mL) was added NaBH₄ (5 mg, 0.13 mmol) at room temperature under nitrogen atmosphere. The resulting mixture was stirred for 1 h at room temperature under nitrogen atmosphere. The reaction was quenched with H₂O (2 mL). The resulting mixture was concentrated under reduced pressure to afford (*S*)-*N*-[(*R*)-(5-chloro-4-cyclopropyl-2-hydroxyphenyl)([1-[(4R)-2,2-dimethyl-1,3-dioxolane-4-carbonyl]piperidin-4-yl])methyl]-2-methylpropane-2-sulfinamide as a brown solid (35 mg, crude): LCMS (ESI) calc'd for C₂₅H₃₇ClN₂O₅S [M + H]⁺: 513, 515 (3 : 1), found, 513, 515 (3 : 1);

### Step c:

To a stirred solution of (*S*)-*N*-[(*R*)-(5-chloro-4-cyclopropyl-2-hydroxyphenyl)([1-[(4*R*)-2,2-dimethyl-1,3-dioxolane-4-carbonyl]piperidin-4-yl])methyl]-2-methylpropane-2-sulfinamide (35 mg, 0.07 mmol) in THF (1 mL) was added aq. HCl (4 *N,* 0.5 mL) at room temperature. The reaction mixture was stirred for 1 h at room temperature. The mixture was concentrated under reduced pressure. The residue was purified with Prep-HPLC with the following conditions: Column: XBridge Shield RP18 OBD Column 19 x 15 mm, 5 µm; Mobile Phase A: water with 10 mmol/L NH₄HCO₃, Mobile Phase B: ACN; Flow rate: 25 mL/min; Gradient: 5% B to 30% B in 7 min; Detector: UV 254/220 nm; Retention time: 5.03 min. The fractions containing the desired product were collected and concentrated under reduced pressure to afford Compound 289 ((2*R*)-1-[4-[(*R*)-amino(5-chloro-4-cyclopropyl-2-hydroxyphenyl)methyl]piperidin-1-yl]-2,3-dihydroxypropan-1-one) as an off-white solid (11 mg, 44%): LCMS (ESI) calc'd for C₁₈H₂₅ClN₂O₄ [M + H]⁺: 369, 371 (3 : 1), found 369, 371 (3 : 1); ¹H NMR (400 MHz, CD₃OD) 7.10 (s, 1H), 6.38 (s, 1H), 4.67-4.47 (m, 2H), 4.11 (dd, *J =* 47.7, 13.8 Hz, 1H), 3.89-3.81 (m, 1H), 3.74-3.54 (m, 2H), 3.13-2.93 (m, 1H), 2.69-2.56 (m, 1H), 2.20-1.95 (m, 3H), 1.53-1.39 (m, 1H), 1.40-1.11 (m, 2H), 1.06-0.84 (m, 2H), 0.70-0.53 (m, 2H).

### Example 229. (which is a reference example) Compound 290 (2R)-1-[4-[amino(4,5-dichloro-3-fluoro-2-hydroxyphenyl)methyl]piperidin-1-yl]-2,3-dihydroxypropan-1-one trifluoroacetic acid

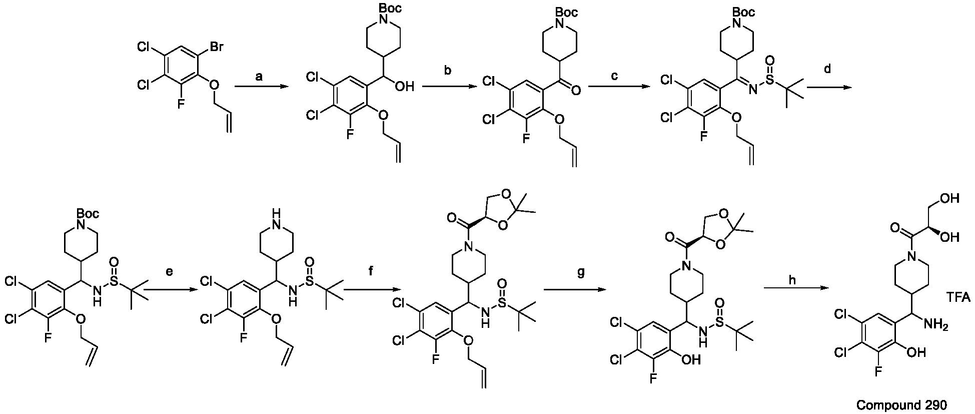

### Step a:

To a stirred solution of 1-bromo-4,5-dichloro-3-fluoro-2-(prop-2-en-1-yloxy)benzene (Intermediate 46, Example 46) (0.80 g, 2.67 mmol) in THF (6 mL) was added i-PrMgCl (1.60 mL, 3.20 mmol, 2 M in THF) dropwise at 0 °C under nitrogen atmosphere. After stirring for 30 min at 0 °C under nitrogen atmosphere, a solution of *tert*-butyl 4-formylpiperidine-1-carboxylate (0.62 g, 2.93 mmol) in THF (4 mL) was added dropwise at 0 °C under nitrogen atmosphere. The resulting mixture was allowed to warm to room temperature and stirred at room temperature for 2 h under nitrogen atmosphere. The reaction was quenched with saturated aq. NH₄Cl (20 mL) and extracted with EA (2 x 10 mL). The organic phase was dried over anhydrous Na₂SO₄. After filtration, the filtrate was concentrated under reduced pressure. The residue was purified by silica gel column chromatography, eluted with PE/EA (1/1) to afford *tert*-butyl 4-[[4,5-dichloro-3-fluoro-2-(prop-2-en-1-yloxy)phenyl](hydroxy)methyl]piperidine-1-carboxylate as a colorless oil (0.55 g, 48%): LCMS (ESI) calc'd for C₂₀H₂₆Cl₂FNO₄ [M + H - 100]⁺: 334, 336 (3 : 2), found 334, 336 (3 : 2).

### Step b:

To a solution of *tert*-butyl 4-[[4,5-dichloro-3-fluoro-2-(prop-2-en-1-yloxy)phenyl](hydroxy)methyl]piperidine-1-carboxylate (0.55 g, 1.27 mmol) in DCM (10 mL) was added Dess-Martin reagent (0.81 g, 1.90 mmol) at 0 °C. Then the reaction was stirred at room temperature for 2 h. The resulting mixture were washed with co-solvent aq. NaHCO₃/aq. Na₂SO₃ (20 mL, v/v = 1/1). The organic phase was washed with brine, dried over anhydrous Na₂SO₄. After filtration, the filtrate was concentrated under reduced pressure. The residue was purified with silica gel column chromatography, eluted with PE/EA (1/1) to afford *tert*-butyl 4-[4,5-dichloro-3-fluoro-2-(prop-2-en-1-yloxy)benzoyl]piperidine-1-carboxylate as a light yellow oil (0.50 g, 91%): LCMS (ESI) calc'd for C₂₀H₂₄Cl₂FNO₄ [M + H - 56]⁺: 376, 378 (3 : 2), found 376, 378 (3 : 2); ¹H NMR (400 MHz, CDCl₃) δ 7.40 (d, *J =* 2.1 Hz, 1H), 6.08-5.95 (m, 1H), 5.46-5.30 (m, 2H), 4.75-4.67 (m, 2H), 4.11 (s, 2H), 3.28-3.20 (m, 1H), 2.84 (t, *J =* 12.4 Hz, 2H), 1.85 (d, *J =* 12.9 Hz, 2H), 1.67-1.53 (m, 2H), 1.48 (d, *J =* 1.8 Hz, 9 H).

### Step c:

To a stirred solution of *tert*-butyl 4-[4,5-dichloro-3-fluoro-2-(prop-2-en-1-yloxy)benzoyl]piperidine-1-carboxylate (0.50g, 1.16 mmol) and 2-methylpropane-2-sulfinamide (0.28 g, 2.31 mmol) in THF (20 mL) was added Ti(OEt)₄ (2.64 g, 11.57 mmol) in one portion under nitrogen atmosphere. The reaction solution was stirred for 30 h at 80 °C under nitrogen atmosphere. After cooling to room temperature, the resulting solution was quenched with saturated aq. NaHCO₃ (60 mL). The resulting mixture was filtered. The filtrate was extracted with EA (2 x 20 mL). The combined organic layers were washed with brine (2 x 10 mL), dried over anhydrous Na₂SO₄. After filtration, the filtrate was concentrated under reduced pressure to afford *tert*-butyl 4-[[4,5-dichloro-3-fluoro-2-(prop-2-en-1-yloxy)phenyl][(2-methylpropane-2-sulfinyl)imino]methyl]piperidine-1-carboxylate as a light yellow oil (0.80 g, crude): LCMS (ESI) calc'd for C₂₄H₃₃Cl₂FN₂O₄S [M + H - 100]⁺: 435, 437 (3 : 2), found 435, 437 (3 : 2).

### Step d:

To a stirred solution of *tert*-butyl 4-[[4,5-dichloro-3-fluoro-2-(prop-2-en-1-yloxy)phenyl][(2-methylpropane-2-sulfinyl)imino]methyl]piperidine-1-carboxylate (0.80 g, 2.24 mmol) in MeOH (10 mL) was added NaBH₄ (0.17 g, 4.48 mmol) in portions at 0 °C under nitrogen atmosphere. The reaction mixture was stirred for 2 h at room temperature under nitrogen atmosphere. The resulting mixture was quenched with aq. NH₄Cl (40 mL) and concentrated to remove MeOH. Then aqueous layer was extracted with EA (2 x 30 mL). The combined organic layers were washed with brine (2 x 10 mL), dried over anhydrous Na₂SO₄. After filtration, the filtrate was concentrated under reduced pressure to afford *tert*-butyl 4-[[4,5-dichloro-3-fluoro-2-(prop-2-en-1-yloxy)phenyl][(2-methylpropane-2-sulfinyl)amino]methyl]piperidine-1-carboxylate as a light yellow oil (0.80 g, crude): LCMS (ESI) calc'd for C₂₄H₃₅Cl₂FN₂O₄S [M + H - 100]⁺: 437, 439 (3 : 2), found 437, 439 (3 : 2).

### Step e:

To a stirred solution of *tert*-butyl 4-[[4,5-dichloro-3-fluoro-2-(prop-2-en-1-yloxy)phenyl][(2-methylpropane-2-sulfinyl)amino]methyl]piperidine-1-carboxylate (0.80 g, crude) in DCM (8 mL) was added TFA (2 mL) at room temperature. The reaction solution was stirred for 1 h at room temperature under nitrogen atmosphere. The reaction mixture was neutralized with saturated aq. NaHCO₃ to pH 8. The resulting mixture was extracted with DCM (3 x 25 mL). The combined organic layers were washed with brine (10 mL), dried over Na₂SO₄. After filtration, the filtrate was concentrated under reduced pressure to afford *N*-[[4,5-dichloro-3-fluoro-2-(prop-2-en-1-yloxy)phenyl](piperidin-4-yl)methyl]-2-methylpropane-2-sulfinamide as a light yellow oil (0.60 g, crude): LCMS (ESI) calc'd for C₁₉H₂₇Cl₂FN₂O₂S [M + H]⁺: 437, 439 (3 : 2), found 437, 439 (3 : 2).

### Step f:

To a stirred solution of (4R)-2,2-dimethyl-1,3-dioxolane-4-carboxylic acid (0.13 g, 0.89 mmol) and HATU (0.39 g, 1.03 mmol) in DMF (8 mL) were added *N*-[[4,5-dichloro-3-fluoro-2-(prop-2-en-1-yloxy)phenyl](piperidin-4-yl)methyl]-2-methylpropane-2-sulfinamide (0.60 g, crude) and Et₃N (0.48 mL, 4.71 mmol) under nitrogen atmosphere. The reaction solution was stirred for 2 h at room temperature under nitrogen atmosphere. The resulting solution was poured into water (50 mL). The resulting mixture was extracted with EA (2 x 20 mL). The organic layers were washed with brine (5 x 10 mL), dried over anhydrous Na₂SO₄. After filtration, the filtrate was concentrated under reduced pressure to afford *N*-[[4,5-dichloro-3-fluoro-2-(prop-2-en-1-yloxy)phenyl]([1-[(4*R*)-2,2-dimethyl-1,3-dioxolane-4-carbonyl]piperidin-4-yl])methyl]-2-methylpropane-2-sulfinamide as a light yellow oil (0.50 g, crude): LCMS (ESI) calc'd for C₂₅H₃₅Cl₂FN₂O₅S [M + H]⁺: 565, 567 (3 : 2), found 565, 567 (3 : 2).

### Step g:

To a stirred mixture of *N*-[[4,5-dichloro-3-fluoro-2-(prop-2-en-1-yloxy)phenyl]([1-[(4R)-2,2-dimethyl-1,3-dioxolane-4-carbonyl]piperidin-4-yl])methyl]-2-methylpropane-2-sulfinamide (0.50 g, crude) and Pd(PPh₃)₄ (20 mg, 0.02 mmol) in THF (6 mL) was added NaBH₄ (0.13 g, 3.54 mmol) in portions at room temperature under nitrogen atmosphere. The reaction mixture was stirred for 1 h at room temperature. The reaction mixture was quenched with saturated aq. NH₄Cl (3 mL) at 0 °C and concentrated under reduced pressure to afford *N-*[(4,5-dichloro-3-fluoro-2-hydroxyphenyl)([1-[(4*R*)-2,2-dimethyl-1,3-dioxolane-4-carbonyl]piperidin-4-yl])methyl]-2-methylpropane-2-sulfinamide as a light yellow oil (0.50 g, crude): LCMS (ESI) calc'd for C₂₂H₃₁Cl₂FN₂O₅S [M + H]⁺: 525, 527 (3 : 2), found 525, 527 (3 : 2).

### Step h:

To a stirred mixture of *N*-[(4,5-dichloro-3-fluoro-2-hydroxyphenyl)([1-[(4*R*)-2,2-dimethyl-1,3-dioxolane-4-carbonyl]piperidin-4-yl])methyl]-2-methylpropane-2-sulfinamide (0.50 g, crude) in THF (3 mL) was added aq. HCl (6 *N,* 3 mL)) dropwise at room temperature. The reaction mixture was stirred for 1 h at room temperature. The resulting mixture was concentrated under reduced pressure. The residue was purified with Prep-HPLC with the following conditions: Column: Xselect CSH OBD Column, 30 x 150 mm, 5 µm; Mobile Phase A: water (plus 0.05% TFA), Mobile Phase B: ACN; Flow rate: 60 mL/min; Gradient: 5% B to 22% B in 7 min; Detector: 220 nm; Retention time: 6.48 min. The fractions containing the desired product were collected and concentrated under reduced pressure to afford Compound 290 ((2*R*)-1-[4-[amino(4,5-dichloro-3-fluoro-2-hydroxyphenyl)methyl]piperidin-1-yl]-2,3-dihydroxypropan-1-one trifluoroacetic acid) as an off-white solid (55.9 mg, 10% overall 6 steps): LCMS (ESI) calc'd for C₁₅H₁₉Cl₂FN₂O₄ [M + H]⁺: 381, 383 (3 : 2), found 381, 383 (3 : 2); ¹H NMR (400 MHz, CD₃OD) δ 7.34 (s, 1H), 4.72-4.41 (m, 2H), 4.32-4.03 (m, 2H), 3.77-3.56 (m, 2H), 3.08 (dt, *J =* 44.1, 12.7 Hz, 1H), 2.67 (dt, *J =* 44.2, 12.9 Hz, 1H), 2.38-2.35 (m, 1H), 2.02 (d, *J =* 12.8 Hz, 1H), 1.50-1.16 (m, 3H); ¹⁹F NMR (376 MHz, CDCl₃) δ -77.014, - 132.61.

### Example 230. (which is a reference example) Compound 292 ((2R)-1-[4-[(R)-amino(5-chloro-4-ethyl-2-hydroxyphenyl)methyl]piperidin-1-yl]-2,3-dihydroxypropan-1-one trifluoroacetic acid)

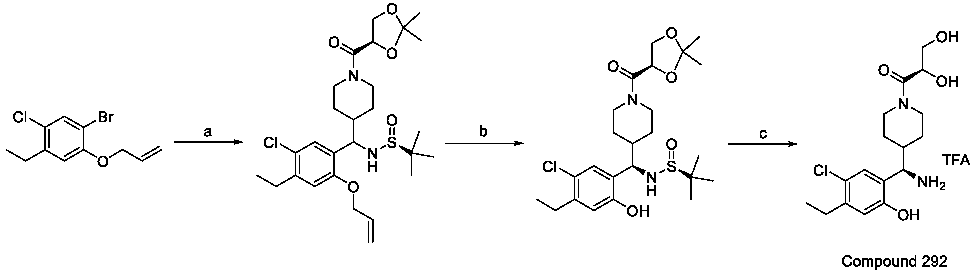

### Step a:

To a stirred solution of 1-bromo-5-chloro-4-ethyl-2-(prop-2-en-1-yloxy)benzene (Intermediate 57, Example 57) (0.24 g, 0.87 mmol) in THF (4 mL) was added *n*-BuLi (0.34 mL, 0.85 mmol, 2.5 M in hexane) at -78 °C under nitrogen atmosphere. The solution was stirred at - 78 °C for 30 min. Then a solution of (*S*)-*N*-([1-[(4*R*)-2,2-dimethyl-1,3-dioxolane-4-carbonyl]piperidin-4-yl]methylidene)-2-methylpropane-2-sulfinamide (0.20 g, 0.58 mmol) in THF (2 mL) was added into the solution. The reaction was stirred at -78 °C for 2 h. The reaction was quenched with saturated aq. NH₄Cl (1 mL) and diluted with water (30 mL). The aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by reverse phase chromatography, eluted with 65% ACN in water (plus 0.05% TFA) to afford (*S*)-*N*-[(*R*)-[5-chloro-4-ethyl-2-(prop-2-en-1-yloxy)phenyl]([1-[(4*R*)-2,2-dimethyl-1,3-dioxolane-4-carbonyl]piperidin-4-yl])methyl]-2-methylpropane-2-sulfinamide as a colorless oil (0.15 g, 48%): LCMS (ESI) calc'd for C₂₇H₄₁ClN₂O₅S [M + H]⁺: 541, 543 (3 : 1), found 541, 543 (3 : 1); ¹H NMR (400 MHz, CD₃OD) δ 7.31-7.20 (m, 1H), 6.92 (s, 1H), 6.19-5.97 (m, 1H), 5.37-5.23 (m, 2H), 4.76-4.47 (m, 4H), 4.36-3.97 (m, 2H), 3.79-3.57 (m, 2H), 3.20-2.83 (m, 1H), 2.82-2.39 (m, 3H), 2.30-2.03 (m, 2H), 1.44-1.35 (m, 6H), 1.35-1.29 (m, 2H), 1.29-1.19 (m, 3H), 1.16-1.11 (m, 10H).

### Step b:

To a stirred solution of (*S*)-*N*-[(*R*)-[5-chloro-4-ethyl-2-(prop-2-en-1-yloxy)phenyl]([1-[(4*R*)-2,2-dimethyl-1,3-dioxolane-4-carbonyl]piperidin-4-yl])methyl]-2-methylpropane-2-sulfinamide (0.15 g, 0.28 mmol) and Pd(PPh₃)₄ (3 mg, 0.003 mmol) in THF (5 mL) was added NaBH₄ (21 mg, 0.55 mmol) at room temperature. The resulting mixture was stirred for 30 min at room temperature. The reaction was quenched with saturated aq. NH₄Cl (0.5 mL) at room temperature. The resulting mixture was concentrated under reduced pressure to afford (*S*)-*N*-[(*R*)-(5-chloro-4-ethyl-2-hydroxyphenyl)([1-[(4*R*)-2,2-dimethyl-1,3-dioxolane-4-carbonyl]piperidin-4-yl])methyl]-2-methylpropane-2-sulfinamide as a yellow oil (0.15 g, crude), which was used in the next step directly without further purification: LCMS (ESI) calc'd for C₂₄H₃₇ClN₂O₅S [M + H]⁺: 501, 503 (3 : 1), found 501, 503 (3 : 1).

### Step c:

To a stirred solution of (*S*)-*N*-[(*R*)-(5-chloro-4-ethyl-2-hydroxyphenyl)([1-[(4*R*)-2,2-dimethyl-1,3-dioxolane-4-carbonyl]piperidin-4-yl])methyl]-2-methylpropane-2-sulfinamide (0.15 g, 0.30 mmol) in 1,4-dioxane (1 mL) was added aq. HCl (4 *N,* 1 mL) at room temperature. The resulting solution was stirred for 30 min at room temperature. The resulting mixture was concentrated under reduced pressure. The residue was purified by Prep-HPLC with the following conditions: Column: Xselect CSH OBD Column 30 x 150 mm 5µm n; Mobile Phase A: water (plus 0.05% TFA), Mobile Phase B: ACN; Flow rate: 60 mL/min; Gradient: 7% B to 25% B in 7 min; Detector: UV 254/220 nm; Retention time: 6.34 min. The fractions containing the desired product were collected and concentrated under reduced pressure to afford Compound 292 ((2*R*)-1-[4-[(*R*)-amino(5-chloro-4-ethyl-2-hydroxyphenyl)methyl]piperidin-1-yl]-2,3-dihydroxypropan-1-one) trifluoroacetic acid as an off-white solid (23 mg, 18% overall two steps): LCMS (ESI) calc'd for C₁₇H₂₅ClN₂O₄ [M + H]⁺: 357, 359 (3 : 1), found 357, 359 (3 : 1); ¹H NMR (400 MHz, CD₃OD) δ 7.25 (s, 1H), 6.86 (s, 1H), 4.66-4.47 (m, 2H), 4.29-4.04 (m, 2H), 3.69-3.55 (m, 2H), 3.16-3.01 (m, 1H), 2.79-2.54 (m, 3H), 2.48-2.26 (m, 1H), 2.08-1.91 (m, 1H), 1.41-1.18 (m, 6H); ¹⁹F NMR (376 MHz, CD₃OD) δ -76.96.

### Example 231. (which is a reference example) Compound 293 ((2R)-1-[4-[(R)-amino(2-hydroxy-4,5-dimethylphenyl)methyl]piperidin-1-yl]-2,3-dihydroxypropan-1-one trifluoroacetic acid)

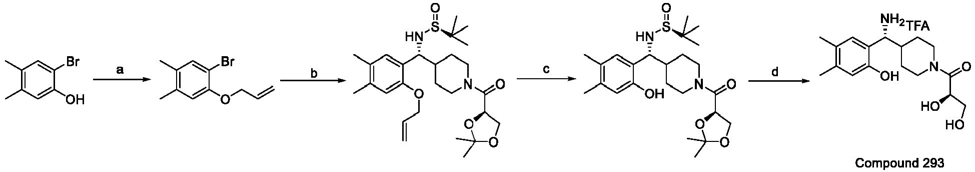

### Step a:

To a stirred solution of 2-bromo-4,5-dimethylphenol (2.00 g, 9.94 mmol) in DMF (10 mL) were added 3-bromoprop-1-ene (1.81 g, 14.92 mmol) and K₂CO₃ (2.75 g, 19.89 mmol) at room temperature. The reaction was stirred at 40 °C for 16 h. The reaction was diluted with water (50 mL). The resulting mixture was extracted with EA (3 x 20 mL). The combined organic layers were washed with brine (2 x 10 mL), dried over anhydrous Na₂SO₄. After filtration, the filtrate was concentrated under reduced pressure. The residue was purified by silica gel column chromatography, eluted with PE to afford 1-bromo-4,5-dimethyl-2-(prop-2-en-1-yloxy)benzene as off-white oil (1.29 g, 54 %): ¹H NMR (400 MHz, CD₃OD) δ 7.28 (s, 1H), 6.82 (s, 1H), 6.15-6.00 (m, 1H), 5.53-5.42 (m, 1H), 5.31-5.20 (m, 1H), 4.70-4.45 (m, 2H), 2.21 (d, *J =* 16.0 Hz, 6H).

### Step b:

To a stirred solution of 1-bromo-4,5-dimethyl-2-(prop-2-en-1-yloxy)benzene(0.10 g, 0.43 mmol) in THF (3 ml) was added *n*-BuLi (0.18 mL, 0.450 mmol, 2.5 M in hexanes) dropwise at -78 °C under nitrogen atmosphere. The reaction was stirred at -78 °C for 30 min. Then (*S*)-*N*-[[1-[(4*R*)-2,2-dimethyl-1,3-dioxolane-4-carbonyl]piperidin-4-yl]methylidene]-2-methylpropane-2-sulfinamide (0.10 g, 0.29 mmol) was added. The resulted solution was stirred at -78 °C for 1 h. The reaction was quenched with saturated aq. NH₄Cl (1 mL). The resulting mixture was diluted with EA (20 mL) and water (20 mL), the aqueous layer was extracted with EA (3 x 20 mL). The combined organic layers were washed with brine (3 x 10 mL), dried over anhydrous Na₂SO₄. After filtration, the filtrate was concentrated under reduced pressure. The residue was purified by Prep-TLC, eluted with PE/EA (1/4) to afford (*S*)-*N*-[(*S*)-[1-[(4*R*)-2,2-dimethyl-1,3-dioxolane-4-carbonyl]piperidin-4-yl][4,5-dimethyl-2-(prop-2-en-1-yloxy)phenyl]methyl]-2-methylpropane-2-sulfinamide as a colorless oil (42 mg, 29 %): LCMS (ESI) calc'd for C₂₇H₄₂N₂O₅S [M + H]⁺: 507, found 507; ¹H NMR (400 MHz, CD₃OD) δ 7.00 *(d, J* = 5.2 Hz, 1H), 6.78 (s, 1H), 6.21-6.06 (m, 1H), 5.44 (d, *J =* 16.8 Hz, 1H), 5.29 (d, *J =* 10.5 Hz, 1H), 4.85-4.78 (m, 1H), 4.56 (d, *J =* 5.0 Hz, 2H), 4.47-4.33 (m, 1H), 4.29-4.00 (m, 4H), 3.17-2.87 (m, 1H), 2.73-2.53 (m, 1H), 2.25 (s, 3H), 2.21 (s, 3H), 2.15-2.01 (m, 2H), 1.42-1.33 (m, 7H), 1.30-1.24 (m, 2H), 1.15-1.05 (m, 9H).

### Step c:

To a stirred mixture of (*S*)-*N*-[(*R*)-[1-[(4R)-2,2-dimethyl-1,3-dioxolane-4-carbonyl]piperidin-4-yl][4,5-dimethyl-2-(prop-2-en-1-yloxy)phenyl]methyl]-2-methylpropane-2-sulfinamide (43 mg, 0.08 mmol) and Pd(PPh₃)₄ (5 mg, 0.01 mmol) in THF (1 mL) was added NaBH₄ (6 mg, 0.16 mmol) at room temperature under nitrogen atmosphere. The reaction mixture was stirred for 1 h at room temperature under nitrogen atmosphere. The reaction was quenched with H₂O (30 mL) at room temperature. The resulting mixture was extracted with EA (3 x 20 mL). The combined organic layers were washed with brine (2 x 10 mL), dried over anhydrous Na₂SO₄. After filtration, the filtrate was concentrated under reduced pressure to afford (*S*)-N*-*[(*R*)-[1-[(4*R*)-2,2-dimethyl-1,3-dioxolane-4-carbonyl]piperidin-4-yl](2-hydroxy-4,5-dimethylphenyl)methyl]-2-methylpropane-2-sulfinamide as a brown solid (43 mg, crude): LCMS (ESI) calc'd for C₂₄H₃₈N₂O₅S [M + H]⁺: 467, found 467.

### Step d:

To a stirred solution of *N*-[(*R*)-[1-[(4*R*)-2,2-dimethyl-1,3-dioxolane-4-carbonyl]piperidin-4-yl](2-hydroxy-4,5-dimethylphenyl)methyl]-2,2-dimethylpropanamide (43 mg, 0.09 mmol) in THF (1 mL) was added aq. HCl (4 *N,* 0.50 mL) dropwise at room temperature. The reaction mixture was stirred for 1h at room temperature. The reaction mixture was concentrated under reduced pressure. The residue was purified with Prep-HPLC with the following conditions: Column: XBridge Shield RP18 OBD Column 19 x 150 mm, 5 µm; Mobile Phase A: water (plus 0.05% TFA), Mobile Phase B: ACN; Flow rate: 25 mL/min; Gradient: 5% B to 30% B in 7 min; Detector: UV 254/220 nm; Retention time: 5.03 min. The fractions containing the desired product were collected and concentrated under reduced pressure to afford Compound 293 ((2R)-1-[4-[(*R*)-amino(2-hydroxy-4,5-dimethylphenyl)methyl]piperidin-1-yl]-2,3-dihydroxypropan-1-one trifluoroacetic acid) as an off-white solid (11 mg, 25% overall two steps): LCMS (ESI) calc'd for C₁₇H₂₆N₂O₄ [M + H]⁺: 323, found 323; ¹H NMR (400 MHz, CD₃OD) δ 6.94 (s, 1H), 6.71 (s, 1H), 4.72-4.41 (m, 2H), 4.31-3.98 (m, 2H), 3.78-3.57 (m, 2H), 3.15-2.97 (m, 1H), 2.66 (dt, *J =* 50.5, 13.3 Hz, 1H), 2.48-2.30 (m, 1H), 2.21 (d, *J =* 7.7 Hz, 6H), 2.10-1.88 (m, 1H), 1.45-1.09 (m, 3H); ¹⁹F NMR (376 MHz, CD₃OD) δ -77.02.

### Example 232. (which is a reference example) Compound 294 ((2R)-1-[4-[(R)-amino(2-hydroxy-4-methylphenyl)methyl]piperidin-1-yl]-2,3-dihydroxypropan-1-one trifluoroacetic acid)

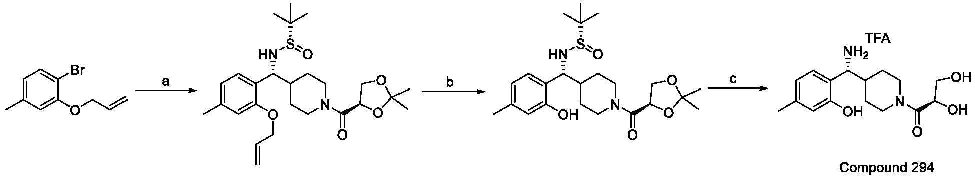

### Step a:

To a stirred solution of 1-bromo-4-methyl-2-(prop-2-en-1-yloxy)benzene (Intermediate 53, Example 53) (99 mg, 0.44 mmol) in THF (3 mL) was added *n*-BuLi (0.17 mL, 0.425 mmol, 2.5 M in n-hexanes) dropwise at -78 °C under nitrogen atmosphere. The reaction was stirred at -78 °C for 30 min. Then (*S*)-*N*-[[1-[(4*R*)-2,2-dimethyl-1,3-dioxolane-4-carbonyl]piperidin-4-yl]methylidene]-2-methylpropane-2-sulfinamide (0.10 g, 0.29 mmol) was added into the reaction. The resulting solution was stirred at -78 °C for 1 h. The reaction was quenched with saturated aq. NH₄Cl (1 mL) at -78 °C. The resulting mixture was diluted with EA (30 mL) and water (30 mL), and then the aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by Prep-TLC, eluted with PE/EA (1/4) to afford (*S*)-*N*-[(*R*)-[1-[(4*R*)-2,2-dimethyl-1,3-dioxolane-4-carbonyl]piperidin-4-yl][4-methyl-2-(prop-2-en-1-yloxy)phenyl]methyl]-2-methylpropane-2-sulfinamide as a colorless oil (35 mg, 24%): LCMS (ESI) calc'd for C₂₆H₄₀N₂O₅S [M + H]⁺: 493, found 493.

### Step b:

To a stirred solution of (*S*)-*N*-[(*R*)-[1-[(4*R*)-2,2-dimethyl-1,3-dioxolane-4-carbonyl]piperidin-4-yl][4-methyl-2-(prop-2-en-1-yloxy)phenyl]methyl]-2-methylpropane-2-sulfinamide (30 mg, 0.06 mmol) and Pd(PPh₃)₄(0.70 mg, 0.001 mmol) in THF (0.5 mL) were added NaBH₄ (5 mg, 0.12 mmol) at room temperature. The resulting mixture was stirred for 1 h at room temperature. The reaction was quenched with saturate aq NH₄Cl (0.5 mL) at room temperature. The resulting mixture was concentrated under reduced pressure to afford (*S*)-*N-*[(*R*)-[1-[(4*R*)-2,2-dimethyl-1,3-dioxolane-4-carbonyl]piperidin-4-yl](2-hydroxy-4-methylphenyl)methyl]-2-methylpropane-2-sulfinamide as a yellow oil (20 mg, crude), which was used in the next step directly without further purification. LCMS (ESI) calc'd for C₂₃H₃₆N₂O₅S [M + H]⁺: 453, found 453.

### Step c:

To a stirred solution of (*S*)-*N*-[(*R*)-[1-[(4R)-2,2-dimethyl-1,3-dioxolane-4-carbonyl]piperidin-4-yl](2-hydroxy-4-methylphenyl)methyl]-2-methylpropane-2-sulfinamide (30 mg, 0.07 mmol) in 1,4-dioxane (1 mL) was added aq. HCl (4 *N,* 0.5 mL) at room temperature. The resulting solution was stirred for 30 min at room temperature. The resulting mixture was concentrated under reduced pressure. The residue was purified by Prep-HPLC with the following conditions: Column: XBridge Prep C18 OBD Column, 5 µm, 19 x 150 mm; Mobile Phase A: water (plus 0.05% TFA), Mobile Phase B: ACN; Flow rate: 25 mL/min; Gradient: 2% B to 18% B in 7 min; Detector: UV 254/220 nm; Retention time: 6.00 min. The fractions containing the desired product were collected and concentrated under reduced pressure to afford Compound 294 ((2*R*)-1-[4-[(*R*)-amino(2-hydroxy-4-methylphenyl)methyl]piperidin-1-yl]-2,3-dihydroxypropan-1-one trifluoroacetic acid) as a brown oil (4 mg, 15% overall two steps): LCMS (ESI) calc'd for C₁₆H₂₄N₂O₄ for [M + H]⁺: 309, found 309; ¹H NMR (400 MHz, CD₃OD) δ 7.07 (d, *J* = 7.6 Hz, 1H), 6.74 (d, *J* = 7.1 Hz, 2H), 4.68-4.40 (m, 2H), 4.30-4.01 (m, 2H), 3.78-3.52 (m, 2H), 3.07 (dt, *J =* 50.3, 10.9 Hz, 1H), 2.66 (dt, *J =* 50.7, 12.8 Hz, 1H), 2.49-2.33 (m, 1H), 2.30 (s, 3H), 2.03 (t, *J =* 10.4 Hz, 1H), 1.53-0.99 (m, 3H); ¹⁹F NMR (376 MHz, CD₃OD) δ -76.96.

### Example 233. (which is a reference example) Compound 295 ((2R)-1-[4-[(R)-amino(5-chloro-2-hydroxyphenyl)methyl]piperidin-1-yl]-2,3-dihydroxypropan-1-one)

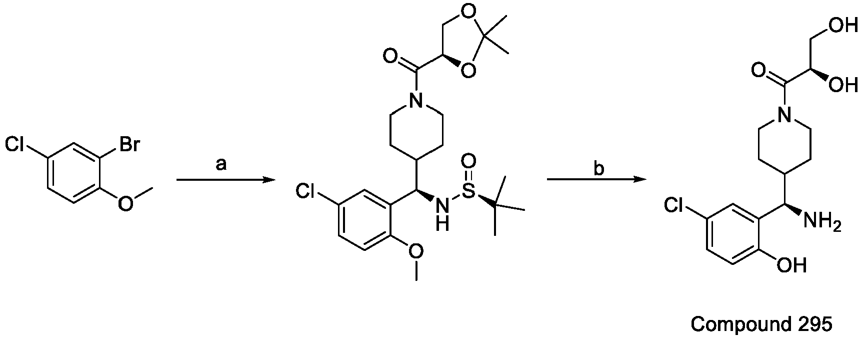

### Step a:

To a stirred solution of 2-bromo-4-chloro-1-methoxybenzene (96 mg, 0.44 mmol) in THF (3 ml) was added *n*-BuLi (0.17 mL, 0.43 mmol, 2.5 M in hexanes) at -78 °C under nitrogen atmosphere. The reaction was stirred at -78 °C for 30 min. Then (*S*)-*N*-[[1-[(4*R*)-2,2-dimethyl-1,3-dioxolane-4-carbonyl]piperidin-4-yl]methylidene]-2-methylpropane-2-sulfinamide (0.10 g, 0.29 mmol) was added into the reaction. The resulting solution was stirred at -78 °C for 1 h. The reaction was quenched with saturated aq. NH₄Cl (2 mL). The resulting mixture was diluted with EA (30 mL) and water (30 mL), and then the aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by Prep-TLC, eluted with PE/EA (1/4) to afford (S)-N-[(R)-(5-chloro-2-methoxyphenyl)([1-[(4*R*)-2,2-dimethyl-1,3-dioxolane-4-carbonyl]piperidin-4-yl])methyl]-2-methylpropane-2-sulfinamide as a light yellow oil (55 mg, 52%): LCMS (ESI) calc'd for C₂₃H₃₅ClN₂O₅S [M + H]⁺: 487, 489 (3 : 1), found 487, 489 (3 : 1); ¹H NMR (400 MHz, CDCl₃) δ 7.27-7.20 (m, 1H), 7.10 (s, 1H), 6.89-6.80 (m, 1H), 4.74-4.59 (m, 1H), 4.60-4.40 (m, 2H), 4.38-4.05 (m, 1H), 3.97-3.69 (m, 4H), 3.68-3.50 (m, 1H), 3.15-2.46 (m, 2H), 2.31-2.21 (m, 1H), 2.04-1.94 (m, 1H), 1.55-1.21 (m, 9H), 1.21-1.11 (m, 9H).

### Step b:

(*S*)-*N*-[(*R*)-(5-chloro-2-methoxyphenyl)([1-[(4*R*)-2,2-dimethyl-1,3-dioxolane-4-carbonyl]piperidin-4-yl])methyl]-2-methylpropane-2-sulfinamide (60 mg, 0.12 mmol) in DCM (2 mL) was added BBr₃ (93 mg, 0.37 mmol) at 0 °C. The resulting mixture was stirred for 1 h at room temperature under nitrogen atmosphere. The reaction was quenched with water (2 mL) at 0 °C. The resulting mixture was concentrated under reduced pressure. The residue was purified by Prep-HPLC with the following conditions: Column: XBridge Shield RP18 OBD Column 30 x 150 mm, 5 µm; Mobile Phase A: Water with 10 mmol/L NH₄HCO₃, Mobile Phase B: ACN; Flow rate: 60 mL/min; Gradient: 20% B to 40% B in 7 min; Detector: UV 254/220 nm; Retention time: 6.43 min. The fractions containing the desired product were collected and concentrated under reduced pressure to afford Compound 295 (2*R*)-1-[4-[(*R*)-amino(5-chloro-2-hydroxyphenyl)methyl]piperidin-1-yl]-2,3-dihydroxypropan-1-one as an off-white solid (11 mg, 28%): LCMS (ESI) calc'd for C₁₅H₂₁ClN₂O₄ [M + H]⁺: 329, 331 (3 : 1), found 329, 331 (3 : 1); ¹H NMR (400 MHz, CD₃OD) δ 7.33-7.18 (m, 2H), 6.91 (d, *J =* 9.1 Hz, 1H), 4.69-4.45 (m, 2H), 4.29-4.05 (m, 2H), 3.77-3.56 (m, 2H), 3.08 (dt, *J =* 45.2, 12.6 Hz, 1H), 2.67 (dt, *J =* 45.8, 13.0 Hz, 1H), 2.46-2.31 (m, 1H), 2.09-1.96 (m, 1H), 1.50-1.08 (m, 3H).

### Example 234. Compound 297 (4-[4-[(R)-amino(4,5-dichloro-2-hydroxyphenyl)methyl]piperidin-1-yl]-1-methylpyridin-2-one)

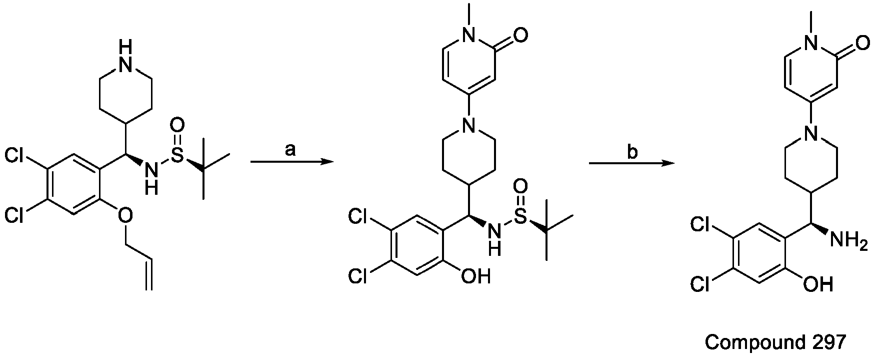

### Step a:

To a stirred solution of (*S*)-*N*-[(*R*)-[4,5-dichloro-2-(prop-2-en-1-yloxy)phenyl](piperidin-4-yl)methyl]-2-methylpropane-2-sulfinamide (0.20 g, 0.48 mmol) and 4-bromo-1-methylpyridin-2-one (0.27 g, 1.43 mmol) in toluene (2 mL) were added Pd(OAc)₂ (11 mg, 0.05 mmol), BINAP (30 mg, 0.05 mmol) and t-BuONa (0.14 g, 1.43 mmol) at room temperature under nitrogen atmosphere. The reaction was degassed with nitrogen for three times and stirred at 100 °C for 24 h under nitrogen atmosphere. After cooling to room temperature, the reaction was diluted with EA (30 mL) and water (30 mL). The aqueous solution was extracted with EA (3 x 30 mL). The combined organic layers were washed with brine (3 x 10 mL) and dried over anhydrous Na₂SO₄. After filtration, the filtrate was concentrated under reduced pressure. The residue was purified by reverse phase chromatography, eluted with 40% ACN in water (plus 0.05% TFA) to afford (*S*)-*N*-[(*R*)-(4,5-dichloro-2-hydroxyphenyl)[1-(1-methyl-2-oxopyridin-4-yl) piperidin-4-yl]methyl]-2-methylpropane-2-sulfinamide as a yellow oil (70 mg, 50%): LCMS (ESI) calc'd for C₂₂H₂₉Cl₂N₃O₃S [M + H]⁺: 486, 488 (3 : 2), found 486, 488 (3 : 2); ¹H NMR (400 MHz, CD₃OD) δ 7.39-7.30 (m, 2H), 6.94 (s, 1H), 6.24 (dd, *J =* 7.8, 2.8 Hz, 1H), 5.72 (d, *J =* 2.8 Hz, 1H), 4.40 (d, *J* = 9.0 Hz, 1H), 3.99 (d, *J* = 13.4 Hz, 1H), 3.87 (d, *J* = 13.3 Hz, 1H), 3.44 (s, 3H), 2.97-2.76 (m, 2H), 2.22 (d, *J* = 13.5 Hz, 1H), 2.15-2.05 (m, 1H), 1.50-1.31 (m, 3H), 1.14 (s, 9H).

### Step b:

To a stirred solution of (*S*)-*N*-[(*R*)-(4,5-dichloro-2-hydroxyphenyl)[1-(1-methyl-2-oxopyridin-4-yl)piperidin-4-yl]methyl]-2-methylpropane-2-sulfinamide (70 mg, 0.14 mmol) in 1,4-dioxane (2 mL) was added aq. HCl (4 *N,* 1 mL) at room temperature. The reaction solution was stirred at room temperature for 1 h. The resulting solution was concentrated under reduced pressure. The residue was purified by Prep-HPLC with following conditions: Column: XBridge Prep C18 OBD Column, 5 µm, 19 x 150 mm; Mobile Phase A: water with 10 mmoL/L NH₄HCO₃, Mobile Phase B: ACN; Flow rate: 25 mL/min; Gradient: 15% B to 50% B in 7 min; Detector: UV 254/220 nm; Retention time: 6.17 min. The fractions containing the desired product were collected and concentrated under reduced pressure to afford Compound 297 (4-[4-[(R)-amino(4,5-dichloro-2-hydroxyphenyl)methyl]piperidin-1-yl]-1-methylpyridin-2-one) as an off-white solid (30 mg, 55%): LCMS (ESI) calc'd for C₁₈H₂₁Cl₂N₃O₂ [M + H]⁺: 382, 384 (3 : 2), found 382, 384 (3 : 2); ¹H NMR (400 MHz, CD₃OD) δ 7.37 (d, *J* = 7.7 Hz, 1H), 7.22 (s, 1H), 6.87 (s, 1H), 6.23 (dd, *J =* 7.8, 2.8 Hz, 1H), 5.72 (d, *J =* 2.8 Hz, 1H), 4.03-3.95 (m, 1H), 3.95-3.83 (m, 2H), 3.44 (s, 3H), 2.97-2.76 (m, 2H), 2.11-1.96 (m, 2H), 1.53-1.42 (m, 1H), 1.42-1.23 (m, 2H).

### Example 235. Compound 298 (5-[4-[(R)-amino(4,5-dichloro-2-hydroxyphenyl)methyl]piperidine-1-carbonyl]-1-methylpyrazin-2-one trifluoroacetic acid)

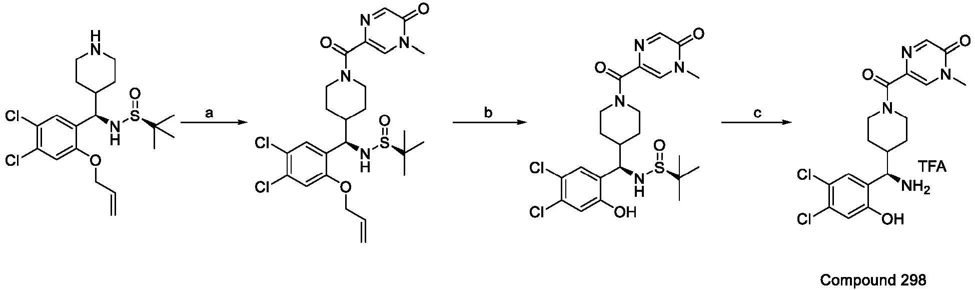

### Step a:

To a stirred mixture of 4-methyl-5-oxopyrazine-2-carboxylic acid (44 mg, 0.28 mmol) and HATU (0.14 g, 0.36 mmol) in DMF (2 mL) were added (*S*)-*N*-[(*R*)-[4,5-dichloro-2-(prop-2-en-1-yloxy)phenyl](piperidin-4-yl)methyl]-2-methylpropane-2-sulfinamide (0.10 g, 0.24 mmol) and Et₃N (85 mg, 0.84 mmol) at room temperature. The resulting mixture was stirred for 2 h at room temperature under nitrogen atmosphere. The resulting mixture was diluted with water (20 mL). The resulting mixture was extracted with EA (3 x 20 mL). The combined organic layers were washed with brine (2 x 15 mL), dried over anhydrous Na₂SO₄. After filtration, the filtrate was concentrated under reduced pressure to afford (*S*)-*N*-[(*R*)-[4,5-dichloro-2-(prop-2-en-1-yloxy)phenyl][1-(4-methyl-5-oxopyrazine-2-carbonyl)piperidin-4-yl]methyl]-2-methylpropane-2-sulfinamide as a light grey solid (0.11 g, 80%): LCMS (ESI) calc'd for C₂₅H₃₂Cl₂N₄O₄S for [M + H]⁺: 555, 557 (3 : 2), found 555, 557 (3 : 2).

### Step b:

To a solution of (S)-N-[(R)-[4,5-dichloro-2-(prop-2-en-1-yloxy)phenyl][1-(4-methyl-5-oxopyrazine-2-carbonyl)piperidin-4-yl]methyl]-2-methylpropane-2-sulfinamide (80 mg, 0.07 mmol) in HOAc (2 mL) was added Pd(PPh₃)₄ (66 mg, 0.03 mmol) at room temperature under nitrogen atmosphere. The resulting mixture was stirred for 1 h at room temperature under nitrogen atmosphere. The resulting mixture was concentrated under reduced pressure. The residue was purified by reverse phase chromatography, eluted with 50% ACN in water (plus 0.1% TFA) to afford (*S*)-*N*-[(*R*)-(4,5-dichloro-2-hydroxyphenyl)[1-(4-methyl-5-oxopyrazine-2-carbonyl)piperidin-4-yl]methyl]-2-methylpropane-2-sulfinamide as a light yellow oil (80 mg, 80%): LCMS (ESI) calc'd for C₂₂H₂₈Cl₂N₄O₄S for [M + H]⁺: 515, 517, (3 : 2), found 515, 517, (3 : 2).

### Step c:

To a stirred solution of (S)-N-[(R)-(4,5-dichloro-2-hydroxyphenyl)[1-(4-methyl-5-oxopyrazine-2-carbonyl)piperidin-4-yl]methyl]-2-methylpropane-2-sulfinamide (80 mg, 0.16 mmol) in 1,4-dioxane (2 mL) was added aq. HCl (4 *N,* 1 mL) at room temperature. The resulting mixture was stirred for 1 h at room temperature under nitrogen atmosphere. The reaction mixture was concentrated under reduced pressure. The residue was purified by Prep-HPLC with the following conditions Column: X Bridge Prep C18 OBD Column, 5 µm, 19 x 150 mm; Mobile Phase A: water (plus 0.05% TFA), Mobile Phase B: ACN; Flow rate: 25 mL/min; Gradient: 10% B to 30% B in 7 min; Detector: UV 220/254 nm; Retention time: 5.83 min. The fractions containing the desired product were collected and concentrated under reduced pressure to afford Compound 298 (5-[4-[(*R*)-amino(4,5-dichloro-2-hydroxyphenyl)methyl]piperidine-1-carbonyl]-1-methylpyrazin-2-one trifluoroacetic acid) as a pink solid (22 mg): LCMS (ESI) calc'd for C₁₈H₂₀Cl₂N₄O₃ for [M + H]⁺: 411, 413, (3 : 2), found 411, 413, (3 : 2); ¹H NMR (400 MHz, CD₃OD) δ 8.06 (s, 1H), 7.98 (s, 1H), 7.44 (s, 1H), 7.08 (s, 1H), 4.68-4.25 (m, 2H), 4.19 (d, *J* = 9.7 Hz, 1H), 3.59 (s, 3H), 3.19-2.65 (m, 2H), 2.48-2.39 (m, 1H), 2.12-2.04 (m, 1H), 1.59-1.21 (m, 3H); ¹⁹F NMR (376 MHz, CD₃OD) δ -77.03.

The compounds in Table 1g below were prepared in an analogous fashion to that described for Compound 298, described below, starting from (*S*)-*N*-[(*R*)-[4,5-dichloro-2-(prop-2-en-1-yloxy)phenyl](piperidin-4-yl)methyl]-2-methylpropane-2-sulfinamide and the corresponding acids, which were prepared as described herein, or which were available from commercial sources.

**Table 1g**

| **Compound Number** | **Structure** | **Chemical Name** | **MS & NMR** |
|---|---|---|---|
| **232** | 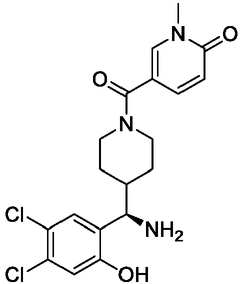 | *5-[4-[(R)-*amino(4,5-dichloro-2-hydroxyphenyl) methyl]piperidin e-1-carbonyl]-1-methylpyridin-2-one | [M + H]⁺: 410, 412 (3 : 2);¹H NMR (400 MHz, CD₃OD) δ 7.91 (d, *J* = 2.5 Hz, 1H), 7.58 (dd, *J* = 9.3, 2.5 Hz, 1H), 7.25 (s, 1H), 6.88 (s, 1H), 6.56 (d, *J =* 9.3 Hz, 1H), 4.47-4.06 (m, 2H), 3.94 (d, *J* = 7.7 Hz, 1H), 3.60 (s, 3H), 3.08-2.85 (m, 2H), 2.12-1.95 (m, 2H), 1.54-1.43 (m, 1H), 1.40-1.20 (m, 2H). |
| **253** | 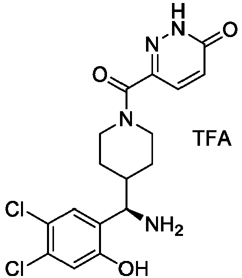 | 6-[4-[(*R*)-amino(4,5-dichloro-2-hydroxyphenyl) methyl]piperidin e-1-carbonyl]-2*H*-pyridazin-3-one trifluoroacetic acid | [M + H]⁺: 397, 399 (3 : 2); ¹H NMR (400 MHz, CD₃OD) δ 7.65 (dd, *J* = 9.8, 5.1 Hz, 1H), 7.44 (d, *J =* 4.8 Hz, 1H), 7.12-7.00 (m, 2H), 4.66 (dd, *J* = 54.0, 13.7 Hz, 1H), 4.32-4.12 (m, 2H), 3.15-3.06 (m, 1H), 2.95-2.75 (m, 1H), 2.44 (s, 1H), 2.13-1.93 (m, 1H), 1.49-1.31 (m, 3H). ¹⁹F NMR (400 MHz, CD₃OD) δ -77.06. |
| **255** | 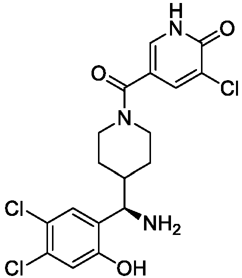 | 5-[4-[(*R*)-amino(4,5-dichloro-2-hydroxyphenyl) methyl]piperidin e-1-carbonyl]-3-chloro-1*H-*pyridin-2-one | [M + H]⁺: 430, 432 (3 : 2); ¹H NMR (400 MHz, CD₃OD) δ 8.05 (s, 1H), 7.87 (s, 1H), 7.48 (s, 1H), 7.11 (s, 1H), 4.53-4.05 (m, 2H), 3.36-3.09 (m, 2H), 2.40-2.17 (m, 2H), 1.85-1.47 (m, 3H). |
| **261** | 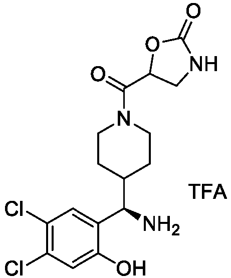 | 5-[4-[(*R*)-amino(4,5-dichloro-2-hydroxyphenyl) methyl]piperidin e-1-carbonyl]-1,3-oxazolidin-2-one trifluoroacetic acid | [M + H]⁺: 388, 390 (3 : 2);¹H NMR (400 MHz, CD₃OD) δ 7.44 (d, *J* = 2.6 Hz, 1H), 7.09 (s, 1H), 4.84-4.78 (m, 1H), 4.71-4.43 (m, 2H), 4.43-4.26 (m, 1H), 4.21-4.07 (m, 1H), 3.98-3.72 (m, 1H), 3.09 (dt, *J =* 40.9, 13.1 Hz, 1H), 2.69 (dt, *J* = 45.0, 13.0 Hz, 1H), 2.51-2.29 (m, 1H), 2.03 (d, *J* = 12.9 Hz, 1H), 1.54-1.02 (m, 3H); ¹⁹F NMR (376 MHz, CD₃OD) δ - 77.16. |
| **277** | 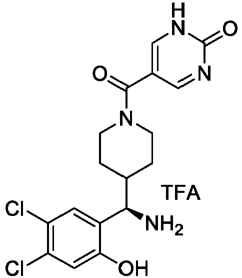 | 5-[4-[(*R*)-amino(4,5-dichloro-2-hydroxyphenyl) methyl]piperidine -1-carbonyl]-1*H*-pyrimidin-2-one trifluoroacetic acid | [M + H]⁺: 397, 399 (3 : 2); ¹H NMR (400 MHz, CD₃OD) δ 8.34 (s, 2H), 7.58 (s, 1H), 7.11 (s, 1H), 4.31-3.81 (m, 3H), 2.88 (s, 2H), 2.51-2.29 (m, 1H), 2.11-1.95 (m, 1H), 1.52-1.13 (m, 3H); ¹⁹F NMR (376 MHz, CD₃OD) δ - 74.02. |

### Example 236. Compound 299 (5-[4-[(R)-amino(4,5-dichloro-2-hydroxyphenyl)methyl]piperidine-1-carbonyl]piperidin-2-one trifluoroacetic acid)

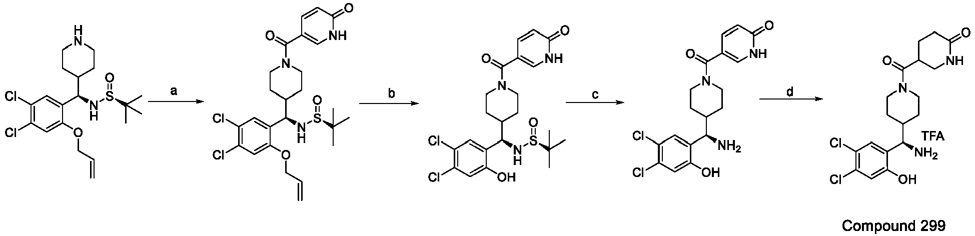

### Step a:

To a stirred solution of 6-oxo-1,6-dihydropyridine-3-carboxylic acid (109 mg, 0.79 mmol) in DMF (3 mL) were added EDCI (0.20 g, 1.07 mmol) and HOBt (0.15 g 1.07 mmol) at room temperature. To the above mixture were added (*S*)-*N*-[(*R*)-[4,5-dichloro-2-(prop-2-en-1-yloxy)phenyl](piperidin-4-yl)methyl]-2-methylpropane-2-sulfinamide (0.30 g, 0.76 mmol) and Et₃N (0.14 g, 1.43 mmol) at room temperature. The resulting mixture was stirred for 12 h at room temperature. The resulting mixture was diluted with water (12 mL). The mixture was extracted with EA (3 x 10 mL). The combined organic layers were washed with brine (2 x 20 mL), dried over anhydrous Na₂SO₄. After filtration, the filtrate was concentrated under reduced pressure to afford (*S*)-*N-*[(*R*)-[4,5-dichloro-2-(prop-2-en-1-yloxy)phenyl][1-(6-oxo-1,6-dihydropyridine-3-carbonyl)piperidin-4-yl]methyl]-2-methylpropane-2-sulfinamide as a yellow solid (0.25 g, crude): LCMS (ESI) calc'd for C₂₅H₃₁Cl₂N₃O₄S [M + H]⁺: 540, 542 (3 : 2), found 540, 542 (3 : 2); ¹H NMR (400 MHz, CDCl₃) δ 7.61 (s, 1H), 7.57-7.48 (m, 1H), 7.24 (s, 1H), 6.99 (s, 1H), 6.59 (d, *J =* 9.1 Hz, 1H), 6.11-5.96 (m, 1H), 5.48-5.34 (m, 2H), 4.58 (s, 2H), 4.48-4.07 (m, 2H), 2.98-2.68 (m, 2H), 2.21-1.97 (m, 2H), 1.53-1.39 (m, 2H), 1.37-1.25 (m, 2H), 1.16 (s, 9H).

### Step b:

To a stirred mixture of (*S*)-*N*-[(*R*)-[4,5-dichloro-2-(prop-2-en-1-yloxy)phenyl][1-(6-oxo-1, 6-dihydropyridine-3-carbonyl)piperidin-4-yl]methyl]-2-methylpropane-2-sulfinamide (0.25 g, crude) and Pd(PPh₃)₄ (27 mg, 0.02 mmol) in THF (3 mL) was added NaBH₄ (35 mg, 0.93 mmol) at room temperature under nitrogen atmosphere. The reaction mixture was stirred for 1 h at room temperature under nitrogen atmosphere. The resulting mixture was quenched with H₂O (3 mL) at room temperature and concentrated under reduced pressure to afford (*S*)-*N-*[(*R*)-(4,5-dichloro-2-hydroxyphenyl)[1-(6-oxo-1,6-dihydropyridine-3-carbonyl)piperidin-4-yl]methyl]-2-methylpropane-2-sulfinamide as a brown yellow solid (0.25 g, crude): LCMS (ESI) calc'd for C₂₂H₂₇Cl₂N₃O₄S [M + H]⁺: 500, 502 (3 : 2), found 500, 502 (3 : 2).

### Step c:

To a stirred solution of (*S*)-*N*-[(*R*)-(4,5-dichloro-2-hydroxyphenyl)[1-(6-oxo-1*H-*pyridine-3-carbonyl)piperidin-4-yl]methyl]-2-methylpropane-2-sulfinamide (0.25 g, 0.50 mmol) in THF (3 mL) was added aq. HCl (4 *N,* 1.5 mL) dropwise at room temperature. The reaction mixture was stirred for 1 h at room temperature. The reaction mixture was concentrated under reduced pressure. The residue was purified by reverse phase chromatography, eluted with 45% ACN in water (plus 0.5% TFA). The fractions containing the desired product were collected and concentrated under reduced pressure to afford 5-[4-[(*R*)-amino(4,5-dichloro-2-hydroxyphenyl)methyl]piperidine-1-carbonyl]-1*H*-pyridin-2-one as a yellow solid (0.18 g, 90%): LCMS (ESI) calc'd for C₁₈H₁₉Cl₂N₃O₃ [M + H]⁺: 396, 398 (3 : 2), found 396, 398 (3 : 2); ¹H NMR (400 MHz, CD₃OD) δ 8.08 (d, *J =* 14.0 Hz, 2H), 7.49 (s, 1H), 7.10 (s, 1H), 7.01 (d, *J* = 8.6 Hz, 1H), 4.69-3.72 (m, 3H), 3.05 (s, 2H), 2.55-2.36 (m, 1H), 2.14-1.95 (m, 1H), 1.64-1.21 (m, 3H).

### Step d:

To a stirred mixture of 5-[4-[(*R*)-amino(4,5-dichloro-2-hydroxyphenyl)methyl]piperidine-1-carbonyl]-1*H*-pyridin-2-one (0.18 g, 0.45 mmol) in MeOH (20 mL) and HCl (12 *N,* 2 mL) was added PtO₂ (90 mg, 0.40 mmol) in portions at room temperature under nitrogen atmosphere. The resulting mixture was stirred for 2 h at room temperature under hydrogen atmosphere (1.5 atm). The resulting mixture was filtered and filter cake were washed with MeOH (2 x 5 mL). The filtrate was concentrated under reduced pressure. The residue was purified by Prep-HPLC with the following conditions: Column: XBridge Shield RP18 OBD Column 19 x 150 mm, 5 µm; Mobile Phase A: water (plus 0.05% TFA), Mobile Phase B: ACN; Flow rate: 25 mL/min; Gradient: 8% B to 27% B in 7 min; Detector: UV 220 nm; Retention time: 6.02 min. The fractions containing the desired product were collected and concentrated under reduced pressure to afford Compound 299 (5-[4-[(*R*)-amino(4,5-dichloro-2-hydroxyphenyl)methyl]piperidine-1-carbonyl]piperidin-2-one trifluoroacetic acid) as an off-white solid (40 mg, 17%): LCMS (ESI) calc'd for C₁₈H₂₃Cl₂N₃O₃ [M + H]⁺: 400, 402 (3 : 2), found 400, 402 (3 : 2); ¹H NMR (400 MHz, CD₃OD) δ 7.44 (d, *J =* 3.9 Hz, 1H), 7.09 (s, 1H), 4.59 (dd, *J =* 54.2, 13.5 Hz, 1H), 4.24-4.01 (m, 2H), 3.72-3.37 (m, 2H), 3.26-3.00 (m, 2H), 2.63 (dt, *J =* 44.4, 13.2 Hz, 1H), 2.50-2.29 (m, 3H), 2.14-1.82 (m, 3H), 1.51-1.04 (m, 3H); ¹⁹F NMR (376 MHz, CD₃OD) δ -77.12.

### Example 237. Compound 300 (5-[4-[(R)-amino(4,5-dichloro-2-hydroxyphenyl)methyl]piperidine-1-carbonyl]-1-methylpiperazin-2-one trifluoroacetic acid)

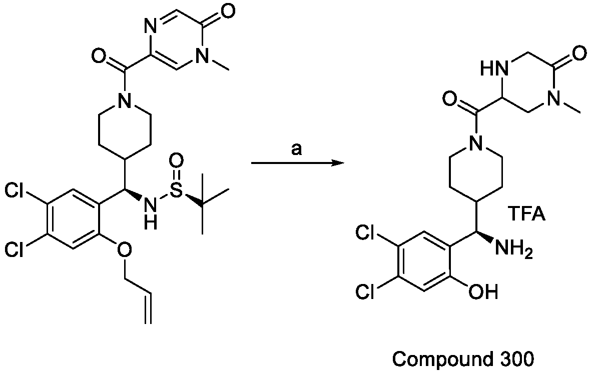

### Step a:

To a stirred mixture of (*S*)-*N*-[(*R*)-[4,5-dichloro-2-(prop-2-en-1-yloxy)phenyl][1-(4-methyl-5-oxopyrazine-2-carbonyl)piperidin-4-yl]methyl]-2-methylpropane-2-sulfinamide (Example 235, step a) (0.11 g, 0.20 mmol) and Pd(PPh₃)₄ (23 mg, 0.02 mmol) in THF (2 mL) was added NaBH₄ (15 mg, 0.40 mmol) at room temperature under nitrogen atmosphere. The resulting mixture was stirred for 1 h at room temperature under nitrogen atmosphere. The reaction was quenched with saturated aq. NH₄Cl at room temperature. After 5 mins, the mixture was added aq. HCl (6 *N,* 1.5 mL) at room temperature. After stirring for additional 30 min at room temperature, the resulting mixture was concentrated under reduced pressure. The residue was purified by Prep-HPLC with the following conditions Column: X Bridge Prep C18 OBD Column, 5 µm, 19 x 150 mm; Mobile Phase A: water (plus 0.05% TFA), Mobile Phase B: ACN; Flow rate: 25 mL/min; Gradient: 15% B to 35% B in 7 min; Detector: UV 220/254 nm; Retention time: 5.89 min. The fractions containing desired product was collected and concentrated under reduced pressure to afford Compound 300 (5-[4-[(R)-amino(4,5-dichloro-2-hydroxyphenyl)methyl]piperidine-1-carbonyl]-1-methylpiperazin-2-one trifluoroacetic acid) as an off-white solid (21 mg, 21%): LCMS (ESI) calc'd for C₁₈H₂₄Cl₂N₄O₃ for [M + H]⁺: 415, 417 (3 : 2), found 415, 417 (3 : 2); ¹H NMR (400 MHz, CD₃OD) δ 7.45 (d, *J =* 8.5 Hz, 1H), 7.10 (d, *J =* 3.5 Hz, 1H), 4.73-4.37 (m, 1H), 4.35-4.00 (m, 2H), 3.96-3.81 (d, *J =* 18.8 Hz, 2H), 3.81-3.46 (m, 2H), 3.16 (s, 2H), 3.06-2.99 (m, 3H), 2.93-2.60 (m, 1H), 2.52-2.35 (m, 1H), 2.18-2.02 (m, 1H), 1.54-1.41 (m, 1H), 1.40-1.07 (m, 2H); ¹⁹F NMR (376 MHz, CD₃OD) δ -77.08.

### Example 238. Compound 302 ((R)-5-(4-(amino(4,5-dichloro-2-hydroxyphenyl)methyl)piperidin-1-yl)pyridin-2-ol)

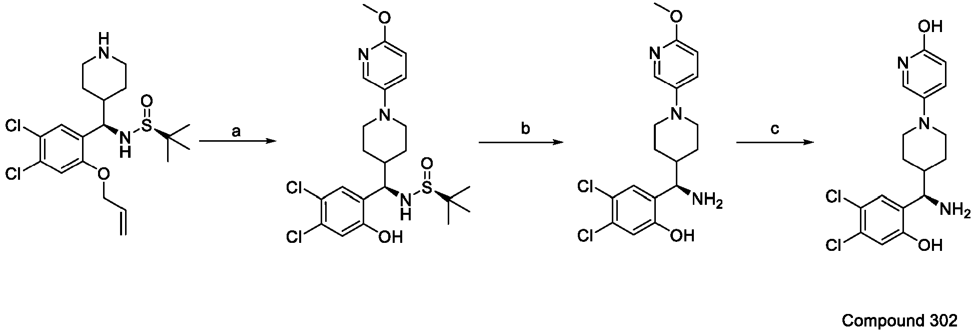

### Step a:

To a stirred solution of (*S*)-*N*-[(*R*)-[4,5-dichloro-2-(prop-2-en-1-yloxy)phenyl](piperidin-4-yl)methyl]-2-methylpropane-2-sulfinamide (0.30 g, 0.72 mmol), BINAP (45 mg, 0.072 mmol) and *t*-BuONa (0.14 g, 1.43 mmol) in toluene (5 mL) were added Pd₂(dba)₃ adduct CHCl₃ (74 mg, 0.072 mmol) and 5-bromo-2-methoxypyridine (0.20 g, 1.5 equiv) at room temperature. The reaction mixture was degassed with nitrogen atmosphere for three times and stirred for 12 h at 120 °C under nitrogen atmosphere. After cooling to room temperature, the resulting mixture was diluted with water (30 mL) and extracted with EA (2 x 60 mL). The combined organic layers were washed with brine (2 x 10 mL), dried over anhydrous Na₂SO₄. After filtration, the filtrate was concentrated under reduced pressure. The residue was purified by reverse phase chromatography, eluted with 70% ACN in water (plus 0.05% TFA) to afford (*S*)-*N*-[(*R*)-(4,5-dichloro-2-hydroxyphenyl)[1-(6-methoxypyridin-3-yl)piperidin-4-yl]methyl]-2-methylpropane-2-sulfinamide as a yellow oil (80 mg, 32%): LCMS (ESI) calc'd for C₂₂H₂₉Cl₂N₃O₃S [M + H]⁺: 486, 488 (3 : 2), found 486, 488 (3 : 2).

### Step b:

To a solution of (*S*)-*N*-[(*R*)-(4,5-dichloro-2-hydroxyphenyl)[1-(6-methoxypyridin-3-yl)piperidin-4-yl]methyl]-2-methylpropane-2-sulfinamide (20 mg, 0.04 mmol) in THF (2 mL) was added aq. HCl (4 *N,* 2 mL) dropwise at room temperature. The reaction solution was stirred for 1 h at room temperature. The resulting solution was concentrated under reduced pressure to afford crude (*R*)-2-(amino(1-(6-methoxypyridin-3-yl)piperidin-4-yl)methyl)-4,5-dichlorophenol. The crude product was used in the next step directly without further purification: LCMS (ESI) calc'd for C₁₈H₂₁Cl₂N₃O₂ [M + H]⁺: 382, 384 (3 : 2), found 382, 384 (3 : 2).

### Step c:

A solution of 2-[(*R*)-amino[1-(6-methoxypyridin-3-yl)piperidin-4-yl]methyl]-4,5-dichlorophenol (crude) in HBr (60% in HOAc, 1 mL) was stirred for 2 h at 120 °C. After cooling to room temperature, the reaction solution was adjusted to pH 9 with saturated aq. NaHCO₃. The resulting mixture was concentrated under reduced pressure. The residue was purified by Prep-HPLC with the following conditions: Column: XBridge Prep C18 OBD Column, 5 µm, 19 x 150 mm; Mobile Phase A: water with 20 mmol/L NH₄HCO₃, Mobile Phase B: ACN; Flow rate: 25 mL/min; Gradient: 7% B to 25% B in 7 min; Detector: UV 220 nm; Retention time: 7.85 min. The fractions containing the desired product were collected and concentrated under reduced pressure to afford Compound 302 ((*R*)-5-(4-(amino(4,5-dichloro-2-hydroxyphenyl)methyl)piperidin-1-yl)pyridin-2-ol) as a yellow oil (1.3 mg, 25% overall two steps): LCMS (ESI) calc'd for C₁₇H₁₉Cl₂N₃O₂ [M + H]⁺: 368, 370 (3 : 2), found 368, 370 (3 : 2); ¹H NMR (400 MHz, CD₃OD) δ 7.68 (dd, *J* = 9.7, 3.1 Hz, 1H), 7.48 (s, 1H), 7.10 (s, 1H), 7.00 *(d, J* = 3.0 Hz, 1H), 6.62 (d, *J* = 9.8 Hz, 1H), 4.24 (d, *J* = 9.8 Hz, 1H), 3.52 (d, *J* = 12.0 Hz, 1H), 3.31 (d, *J =* 12.0 Hz, 1H), 2.70-2.60 (m, 1H), 2.58-2.46 (m, 1H), 2.28-2.17 (m, 1H), 2.13-1.99 (m, 1H), 1.69-1.54 (m, 1H), 1.49-1.38 (m, 2H).

### Example 239. Compound 308 ((2R)-1-[(3S)-3-[amino(4,5-dichloro-2-hydroxyphenyl)methyl]pyrrolidin-1-yl]-2,3-dihydroxypropan-1-one isomer 1); Compound 312 ((2R)-1-[(3S)-3-[amino(4,5-dichloro-2-hydroxyphenyl)methyl]pyrrolidin-1-yl]-2,3-dihydroxypropan-1-one isomer 2)

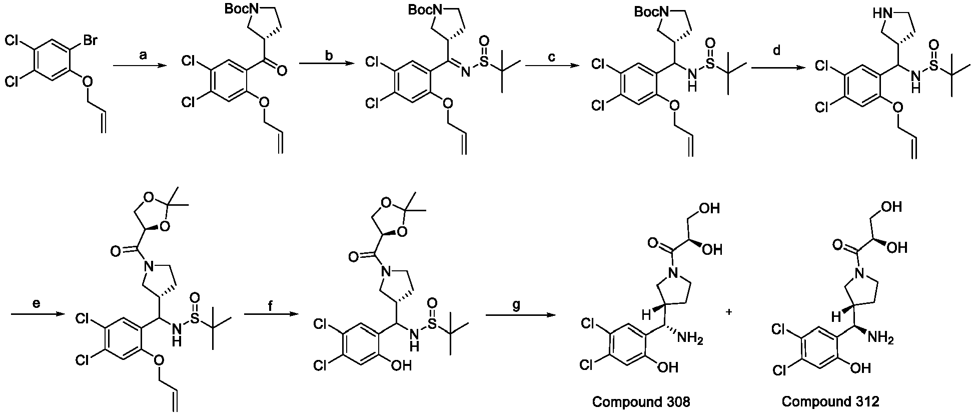

### Step a:

To a stirred solution of 1-bromo-4,5-dichloro-2-(prop-2-en-1-yloxy)benzene (Example 6, step b) (2.73 g, 9.68 mmol) in THF (20 mL) was added *i*-PrMgCl·LiCl (9 mL, 11.61 mmol, 1.3 M in THF) dropwise at -10 °C under nitrogen atmosphere. The resulting mixture was stirred for 30 min at -10 °C under nitrogen atmosphere. To the above mixture was added *tert*-butyl (3*S*)-3-[methoxy(methyl)carbamoyl]pyrrolidine-1-carboxylate (0.50 g, 1.94 mmol) in THF (1 mL) dropwise over 5 min at -10 °C. The resulting mixture was stirred for additional 2 h at -10 °C. The reaction was quenched with saturated aq. NH₄Cl (20 mL) at room temperature. The resulting mixture was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (4/1) to afford *tert*-butyl (3*S*)-3-[4,5-dichloro-2-(prop-2-en-1-yloxy)benzoyl]pyrrolidine-1-carboxylate as a light yellow oil (0.25 g, 32%): LCMS (ESI) calc'd for C₁₉H₂₃Cl₂NO₄ [M + Na]⁺: 422, 424 (3 : 2), found 422, 424 (3 : 2); ¹H NMR (400 MHz, CDCl₃) δ 7.79 (s, 1H), 7.08 (s, 1H), 6.13-6.00 (m, 1H), 5.55-5.24 (m, 2H), 4.69-4.56 (m, 2H), 4.03-3.90 (m, 1H), 3.71-3.54 (m, 2H), 3.54-3.32 (m, 2H), 2.26-2.01 (m, 2H), 1.48 (s, 9H).

### Step b:

To a stirred solution of *tert*-butyl (3*S*)-3-[4,5-dichloro-2-(prop-2-en-1-yloxy)benzoyl]pyrrolidine-1-carboxylate (0.25 g, 0.63 mmol) and *tert*-butanesulfinamide (0.23 g, 1.87 mmol) in THF (5 mL) was added Ti(OEt)₄ (0.85 g, 3.75 mmol) at room temperature. The resulting solution was stirred for 16 h at 70 °C under nitrogen atmosphere. The reaction was quenched with saturated aq. NaHCO₃ (20 mL) at room temperature. The resulting mixture was filtered and the filter cake were washed with EA (3 x 10 mL). The resulting mixture was extracted with EA (3 x 20 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (1/1) to afford *tert*-butyl (3*S*)-3-[[4,5-dichloro-2-(prop-2-en-1-yloxy)phenyl][(2-methylpropane-2-sulfinyl)imino]methyl]pyrrolidine-1-carboxylate as a light yellow oil (0.30 g, 76 %): LCMS (ESI) calc'd for C₂₃H₃₂Cl₂N₂O₄S [M + Na]⁺: 525, 527 (3 : 2), found 525, 527 (3 : 2); ¹H NMR (400 MHz, CDCl₃) δ 7.18 (s, 1H), 6.98 (s, 1H), 6.06-5.91 (m, 1H), 5.45-5.28 (m, 2H), 4.63-4.52 (m, 2H), 3.77-3.64 (m, 3H), 3.59-3.46 (m, 1H), 3.40-3.29 (m, 1H), 2.29-1.92 (m, 2H), 1.50-1.45 (m, 9H), 1.24 (s, 9H).

### Step c:

To a stirred solution of *tert*-butyl (3*S*)-3-[[4,5-dichloro-2-(prop-2-en-1-yloxy)phenyl][(2-methylpropane-2-sulfinyl)imino]methyl]pyrrolidine-1-carboxylate (0.30 g, 0.60 mmol) in THF (5 mL) was added NaBH₄ (68 mg, 1.79 mmol) at room temperature. The resulting mixture was stirred for 1 h at room temperature. The reaction was quenched with water (20 mL) and extracted with EA (3 x 20 mL). The combined organic layers were washed with brine (3 x 20 mL), dried over anhydrous Na₂SO₄. After filtration, the filtrate was concentrated under reduced pressure to afford *tert*-butyl (3*S*)-3-[[4,5-dichloro-2-(prop-2-en-1-yloxy)phenyl][(2-methylpropane-2-sulfinyl)amino]methyl]pyrrolidine-1-carboxylate as a light yellow oil (0.30 g, crude), which was used in the next step directly without further purification: LCMS (ESI) calc'd for C₂₃H₃₄Cl₂N₂O₄S [M + H]⁺: 505, 507 (3 : 2), found 505, 507 (3 : 2); ¹H NMR (400 MHz, CDCl₃) δ 7.31 (s, 1H), 6.97 (s, 1H), 6.10-5.96 (m, 1H), 5.40 (dd, *J =* 26.3, 13.9 Hz, 2H), 4.65-4.51 (m, 2H), 3.77-3.65 (m, 3H), 3.55-3.42 (m, 1H), 3.37-3.15 (m, 2H), 3.04-2.58 (m, 1H), 1.88-1.71 (m, 1H), 1.51-1.44 (m, 9H), 1.17 (d, *J =* 8.2 Hz, 9H).

### Step d:

To a stirred solution of *tert*-butyl (3*S*)-3-[[4,5-dichloro-2-(prop-2-en-1-yloxy)phenyl][(2-methylpropane-2-sulfinyl)amino]methyl]pyrrolidine-1-carboxylate (0.30 g, 0.60 mmol) in DCM (2 mL) were added TFA (0.4 mL) at room temperature. The resulting mixture was stirred for 30 min at room temperature. The reaction was neutralized to pH 7 with saturated aq. NaHCO₃ (20 mL) at room temperature and extracted with EA (3 x 20 mL). The combined organic layers were washed with brine (3 x 20 mL), dried over anhydrous Na₂SO₄. After filtration, the filtrate was concentrated under reduced pressure to afford *N*-[[4,5-dichloro-2-(prop-2-en-1-yloxy)phenyl]((3*S*)-pyrrolidin-3-yl)methyl]-2-methylpropane-2-sulfinamide as a light yellow solid (0.30 g, crude), which was used in the next step directly without further purification: LCMS (ESI) calc'd for C₁₈H₂₆Cl₂N₂O₂S [M + H]⁺: 405, 407 (3 : 2), found 405, 407 (3 : 2).

### Step e:

To a stirred mixture of (4*R*)-2,2-dimethyl-1,3-dioxolane-4-carboxylic acid (0.32 g, 2.22 mmol) and HATU (1.13 g, 2.96 mmol) in DMF (5 mL) were added *N*-[[4,5-dichloro-2-(prop-2-en-1-yloxy)phenyl]((3*S*)-pyrrolidin-3-yl)methyl]-2-methylpropane-2-sulfinamide (0.30 g, 0.74 mmol) and Et₃N (0.22 g, 2.22 mmol) at room temperature. The resulting mixture was stirred for 1 h at room temperature. The residue was purified by reverse phase chromatography, eluted with 57% ACN in water (plus 0.05% TFA) to afford *N*-[[4,5-dichloro-2-(prop-2-en-1-yloxy)phenyl][(3*S*)-1-[(4*R*)-2,2-dimethyl-1,3-dioxolane-4-carbonyl]pyrrolidin-3-yl]methyl]-2-methylpropane-2-sulfinamide as an off-white solid (0.20 g, 60% overall three steps): LCMS (ESI) calc'd for C₂₄H₃₄Cl₂N₂O₅S [M + H]⁺: 533, 535 (3 : 2), found 533, 535 (3 : 2); ¹H NMR (400 MHz, CDCl₃) δ 7.28-7.23 (m, 1H), 7.02-6.96 (m, 1H), 6.13-5.98 (m, 1H), 5.51-5.33 (m, 2H), 4.83-4.43 (m, 4H), 4.40-4.26 (m, 1H), 4.26-4.09 (m, 1H), 3.92-3.57 (m, 2H), 3.48-3.24 (m, 1H), 3.10-2.56 (m, 2H), 2.48-2.13 (m, 1H), 1.95-1.67 (m, 1H), 1.47-1.39 (m, 6H), 1.17-1.14 (m, 9H).

### Step f:

To a stirred solution of *N*-[[4,5-dichloro-2-(prop-2-en-1-yloxy)phenyl][(3S)-1-[(4R)-2,2-dimethyl-1,3-dioxolane-4-carbonyl]pyrrolidin-3-yl]methyl]-2-methylpropane-2-sulfinamide (0.20 g, 0.38 mmol) and Pd(PPh₃)₄ (4 mg, 0.004 mmol) in THF (3 mL) was added NaBH₄ (28 mg, 0.75 mmol) at room temperature. The resulting mixture was stirred for 30 min at room temperature. The resulting mixture was quenched with saturated aq. NH₄Cl (1 mL) and concentrated under reduced pressure to afford *N*-[(4,5-dichloro-2-hydroxyphenyl)[(3*S*)-1-[(4*R*)-2,2-dimethyl-1,3-dioxolane-4-carbonyl]pyrrolidin-3-yl]methyl]-2-methylpropane-2-sulfinamide as a brown solid (0.20 g, crude), which was used in the next step directly without further purification: LCMS (ESI) calc'd for C₂₁H₃₀Cl₂N₂O₅S [M + H]⁺: 493, 495 (3 : 2), found 493, 495 (3 : 2).

### Step g:

To a stirred mixture of *N*-[(4,5-dichloro-2-hydroxyphenyl)[(3*S*)-1-[(4*R*)-2,2-dimethyl-1,3-dioxolane-4-carbonyl]pyrrolidin-3-yl]methyl]-2-methylpropane-2-sulfinamide (0.20 g, 0.41 mmol) in 1,4-dioxane (1 mL) was added aq. HCl (4 *N,* 1 mL) at room temperature. The resulting mixture was stirred for 30 min at room temperature. The resulting mixture was concentrated under reduced pressure. The residue was purified by Prep-HPLC with the following conditions: Column: Xselect CSH OBD Column 30 x 150 mm 5 µm, n; Mobile Phase A: water (plus 0.05% TFA), Mobile Phase B: ACN; Flow rate: 60 mL/min; Gradient: 5% B to 20% B in 9 min; Detector: UV 254/220 nm; Retention time: RT₁:6.05 min, RT₂: 8.92 min.

The fractions containing the desired product at 6.05 min were collected and concentrated under reduced pressure to afford Compound 308 ((2*R*)-1-[(3*S*)-3-[amino(4,5-dichloro-2-hydroxyphenyl)methyl]pyrrolidin-1-yl]-2,3-dihydroxypropan-1-one isomer 1) as an off-white solid (17.5 mg, 13% overall two steps): LCMS (ESI) calc'd for C₁₄H₁₈Cl₂N₂O₄ [M + H]⁺: 349, 351 (3 : 2), found 349, 351 (3 : 2); ¹H NMR (400 MHz, CD₃OD) δ 7.32 (d, *J =* 5.5 Hz, 1H), 6.90 (s, 1H), 4.28 (dt, *J =* 48.6, 5.8 Hz, 1H), 4.08-3.78 (m, 2H), 3.78-3.53 (m, 3H), 3.50-3.35 (m, 1H), 3.28-3.00 (m, 1H), 2.82-2.58 (m, 1H), 2.38-2.17 (m, 1H), 1.97-1.67 (m, 1H).

The fractions containing the desired product at 8.92 min were collected and concentrated under reduced pressure to Compound 312 ((2*R*)-1-[(3*S*)-3-[amino(4,5-dichloro-2-hydroxyphenyl)methyl]pyrrolidin-1-yl]-2,3-dihydroxypropan-1-one isomer 2) as an off-white solid (34.1 mg, 26% overall two steps): LCMS (ESI) calc'd for C₁₄H₁₈Cl₂N₂O₄ [M + H]⁺: 349, 351 (3 : 2), found 349, 351 (3 : 2); ¹H NMR (400 MHz, CD₃OD) δ 7.32 (d, *J =* 11.6 Hz, 1H), 6.91 (d, *J =* 7.4 Hz, 1H), 4.39 (dt, *J =* 29.3, 5.8 Hz, 1H), 4.06-3.90 (m, 2H), 3.91-3.60 (m, 3H), 3.61-3.41 (m, 1H), 3.30-3.23 (m, 1H), 2.81-2.58 (m, 1H), 1.81-1.50 (m, 2H).

### Example 240. Compound 309 ((2R)-1-[4-[amino(4,5-dichloro-2-hydroxyphenyl)methyl]-4-methylpiperidin-1-yl]-2,3-dihydroxypropan-1-one trifluoroacetic acid)

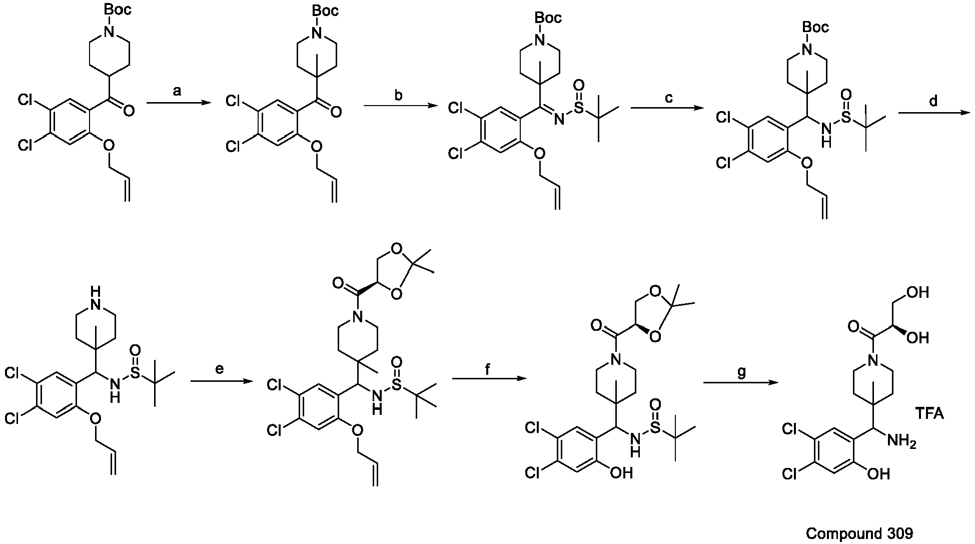

### Step a:

To a stirred solution of tert-butyl 4-[4,5-dichloro-2-(prop-2-en-1-yloxy)benzoyl]piperidine-1-carboxylate (0.50 g, 1.21 mmol) in THF (5 mL) was added LiHMDS (2.41 mL, 2.41 mmol, 1 M in THF) at -60 °C under nitrogen atmosphere. The reaction was stirred at -60°C for 0.5 h. Then MeI (0.51 g, 3.62 mmol) was added into the reaction. The reaction was stirred at -60°C to room temperature for 2 h. The reaction was quenched with saturated aq. NH₄Cl (2 mL) and diluted with EA (20 mL) and water (30 mL). The aqueous layer was extracted with EA (3 x 20 mL). The combined organic layers were washed with brine (2 x 20 mL) and dried over anhydrous Na₂SO₄. After filtration, the filtrate was concentrated under reduced pressure. The residue was purified by silica gel column chromatography, eluted with PE/EA (10/1) to afford *tert*-butyl 4-[4,5-dichloro-2-(prop-2-en-1-yloxy)benzoyl]-4-methylpiperidine-1-carboxylate as a light yellow oil (0.28 g, 54%): LCMS (ESI) calc'd for C₂₁H₂₇Cl₂NO₄ [M + Na]⁺: 450, 452 (3 : 2), found 428, 430 (3 : 2); ¹H NMR (400 MHz, CDCl₃) δ 7.14 (s, 1H), 7.02 (s, 1H), 6.07-5.91 (m, 1H), 5.43-5.30 (m, 2H), 4.54 (d, *J =* 5.7 Hz, 2H), 3.62-3.42 (m, 2H), 3.42-3.31 (m, 2H), 2.05-1.95 (m, 2H), 1.62-1.54 (m, 2H), 1.54 (s, 9H), 1.30 (s, 3H).

### Step b:

To a stirred solution of *tert*-butyl 4-[4,5-dichloro-2-(prop-2-en-1-yloxy)benzoyl]-4-methylpiperidine-1-carboxylate (0.28 g, 0.65 mmol) and *tert*-butanesulfinamide (0.24 g, 1.96 mmol) in THF (5 mL) was added Ti(OEt)₄ (1.49 g, 6.54 mmol) at room temperature under nitrogen atmosphere. The reaction was stirred at 70 °C for 16 h. The reaction was quenched with saturated aq. Na₂CO₃ (30 mL). The resulting mixture was filtered through celite and the filtrate was diluted with water (30 mL). The aqueous layer was extracted with EA (3 x 30 mL). The combined organic layers were washed with brine (3 x 20 mL), dried over anhydrous Na₂SO₄. After filtration, the filtrate was concentrated under reduced pressure to afford *tert*-butyl 4-[[4,5-dichloro-2-(prop-2-en-1-yloxy)phenyl][(2-methylpropane-2-sulfinyl)imino]methyl]-4-methylpiperidine-1-carboxylate as a light yellow oil (0.44 g, crude), which was used in the next step directly without further purification: LCMS (ESI) calc'd for C₂₅H₃₆Cl₂N₂O₄S [M + H]⁺: 531, 533 (3 : 2), found 531, 533 (3 : 2); ¹H NMR (400 MHz, CD₃OD) δ 7.27-7.20 (m, 2H), 6.11-5.97 (m, 1H), 5.49-5.39 (m, 1H), 5.35-5.28 (m, 1H), 4.65-4.57 (m, 2H), 3.88-3.56 (m, 2H), 3.38-3.33 (m, 1H), 3.31-3.21 (m, 1H), 2.02-1.84 (m, 2H), 1.78-1.58 (m, 2H), 1.46 (d, *J =* 1.8 Hz, 9H), 1.29 (s, 3H), 1.23 (s, 9H).

### Step c:

To a stirred solution of *tert*-butyl 4-[[4,5-dichloro-2-(prop-2-en-1-yloxy)phenyl][(2-methylpropane-2-sulfinyl)imino]methyl]-4-methylpiperidine-1-carboxylate (0.44 g, 0.83 mmol) in THF (5 mL) and MeOH (2 mL) was added NaBH₄ (0.13 g, 3.31 mmol) in portions at room temperature. The reaction was stirred at room temperature for 8 h. The reaction was quenched with saturated aq. NH₄Cl (2 mL) and diluted with water (30 mL). The aqueous layer was extracted with EA (3 x 20 mL). The combined organic layers were washed with brine (2 x 20 mL) and dried over anhydrous Na₂SO₄. After filtration, the filtrate was concentrated under reduced pressure to afford *tert*-butyl 4-[[4,5-dichloro-2-(prop-2-en-1-yloxy)phenyl][(2-methylpropane-2-sulfinyl)amino]methyl]-4-methylpiperidine-1-carboxylate as a light yellow solid (0.33 g, 75%): LCMS (ESI) calc'd for C₂₅H₃₈Cl₂N₂O₄S [M + H]⁺: 533, 535 (3 : 2), found 533, 535 (3 : 2); ¹H NMR (400 MHz, CD₃OD) δ 7.52 (s, 1H), 7.18 (s, 1H), 6.16-6.05 (m, 1H), 5.46 (d, *J =* 17.1 Hz, 1H), 5.34 (d, *J =* 10.7 Hz, 1H), 4.70-4.56 (m, 2H), 3.92-3.75 (m, 3H), 3.13-2.93 (m, 4H), 1.66-1.60 (m, 2H), 1.46 (s, 9H), 1.23 (s, 3H), 1.14 (s, 9H).

### Step d:

To a stirred solution of *tert*-butyl 4-[[4,5-dichloro-2-(prop-2-en-1-yloxy)phenyl][(2-methylpropane-2-sulfinyl)amino]methyl]-4-methylpiperidine-1-carboxylate (0.33 g, 0.62 mmol) in DCM (5 mL) was added TFA (1 mL) at room temperature. The reaction was stirred at room temperature for 0.5 h. The reaction was neutralized to pH 7 with saturated aq. Na₂CO₃ (20 mL) at 0 °C. The resulting solution was diluted with water (30 mL). The aqueous layer was extracted with EA (3 x 30 mL). The combined organic layers were washed with brine (3 x 20 mL), dried over anhydrous Na₂SO₄. After filtration, the filtrate was concentrated under reduced pressure to afford *N*-[[4,5-dichloro-2-(prop-2-en-1-yloxy)phenyl](4-methylpiperidin-4-yl)methyl]-2-methylpropane-2-sulfinamide as a yellow oil (400 mg, crude), which was used in the next step directly without further purification: LCMS (ESI) calc'd for C₂₀H₃₀Cl₂N₂O₂S [M + H]⁺: 433, 435 (3 : 2), found 433, 435 (3 : 2); ¹H NMR (400 MHz, CD₃OD) δ 7.56 (s, 1H), 7.22 (s, 1H), 6.18-6.04 (m, 1H), 5.51-5.30 (m, 2H), 4.68-4.57 (m, 3H), 3.31-3.23 (m, 2H), 3.22-3.02 (m, 2H), 1.94-1.69 (m, 4H), 1.23 (s, 3H), 1.14 (s, 9H).

### Step e:

To a stirred solution of (*4R*)-2,2-dimethyl-1,3-dioxolane-4-carboxylic acid (0.20 g, 1.38 mmol) and HATU (0.53 g, 1.38 mmol) in DMF (3 mL) were added *N*-[[4,5-dichloro-2-(prop-2-en-1-yloxy)phenyl](4-methylpiperidin-4-yl)methyl]-2-methylpropane-2-sulfinamide (0.40 g, 0.92 mmol) and Et₃N (0.28g, 2.77 mmol) at room temperature. The reaction was stirred at room temperature for 1 h. The reaction was purified by reverse phase chromatography, eluted with 50% ACN in water (plus 0.05% TFA) to afford *N*-[[4,5-dichloro-2-(prop-2-en-1-yloxy)phenyl]([1-[(*4R*)-2,2-dimethyl-1,3-dioxolane-4-carbonyl]-4-methylpiperidin-4-yl])methyl]-2-methylpropane-2-sulfinamide as a colorless oil (0.13 g, 37% overall two steps): LCMS (ESI) calc'd for C₂₆H₃₈Cl₂N₂O₅S [M + H]⁺: 561, 563 (3 : 2), found 561, 563 (3 : 2); ¹H NMR (400 MHz, CD₃OD) δ 7.52 (s, 1H), 7.18 (s, 1H), 6.20-6.02 (m, 1H), 5.52-5.38 (m, 1H), 5.38-5.28 (m, 1H), 4.71-4.58 (m, 2H), 4.35-4.16 (m, 4H), 4.04-3.82 (m, 1H), 3.77-3.54 (m, 1H), 3.24-2.85 (m, 2H), 1.88-1.51 (m, 4H), 1.42-1.34 (m, 6H), 1.27 (d, *J =* 7.1 Hz, 3H), 1.16 (d, *J =* 14.9 Hz, 9H).

### Step f:

To a stirred solution of *N*-[[4,5-dichloro-2-(prop-2-en-1-yloxy)phenyl]([1-[(*4R*)-2,2-dimethyl-1,3-dioxolane-4-carbonyl]-4-methylpiperidin-4-yl])methyl]-2-methylpropane-2-sulfinamide (0.13 g, 0.23 mmol) and Pd(PPh₃)₄ (5 mg, 0.01 mmol) in THF (2 mL) was added NaBH₄ (18 mg, 0.46 mmol) at room temperature. The reaction was stirred at room temperature for 0.5 h. The reaction was concentrated under reduced pressure to afford *N*-[(4,5-dichloro-2-hydroxyphenyl)([1-[(*4R*)-2,2-dimethyl-1,3-dioxolane-4-carbonyl]-4-methylpiperidin-4-yl])methyl]-2-methylpropane-2-sulfinamide as a brown solid (0.12 g, crude), which was used in the next step directly without further purification: LCMS (ESI) calc'd for C₂₃H₃₄Cl₂N₂O₅S [M + H]⁺: 521, 523 (3 : 2), found 521, 523 (3 : 2).

### Step g:

To a stirred solution of *N*-[(4,5-dichloro-2-hydroxyphenyl)([1-[(*4R*)-2,2-dimethyl-1,3-dioxolane-4-carbonyl]-4-methylpiperidin-4-yl])methyl]-2-methylpropane-2-sulfinamide (0.12 g, 0.23 mmol) in THF (2 mL) was added aq. HCl (4 *N,* 1 mL) at room temperature. The reaction was stirred at room temperature for 1 h. The reaction was diluted with EA (20 mL) and HCl (1 *N,* 20 mL). The organic layer was washed with aq. HCl (1 *N,* 2 x 20 mL). The combined aqueous layers were concentrated under reduced pressure. The residue was purified by Prep-HPLC with following conditions: Column: Xselect CSH OBD Column 30 x 150 mm 5µm n; Mobile Phase A: water (plus 0.05% TFA), Mobile Phase B: ACN; Flow rate: 60 mL/min; Gradient: 10% B to 25% B in 7 min; Detector: UV 254/220 nm; Retention time: 5.58 min. The fractions containing the desired product were collected and concentrated under reduced pressure to afford Compound 309 ((*2R*)-1-[4-[amino(4,5-dichloro-2-hydroxyphenyl)methyl]-4-methylpiperidin-1-yl]-2,3-dihydroxypropan-1-one trifluoroacetic acid) as an off-white solid (63 mg, 56% overall two steps): LCMS (ESI) calc'd for C₁₆H₂₂Cl₂N₂O₄ [M + H]⁺: 377, 379 (3 : 2), found 377, 379 (3 : 2); ¹H NMR (400 MHz, CD₃OD) δ 7.42 (s, 1H), 7.09 (s, 1H), 4.63-4.26 (m, 3H), 4.09-3.93 (m, 1H), 3.78-3.60 (m, 2H), 3.30-3.20 (m, 1H), 3.12-2.85 (m, 1H), 1.78-1.54 (m, 3H), 1.41-1.28 (m, 1H), 1.21 (s, 3H); ¹⁹F NMR (376 MHz, CD₃OD) δ -77.15.

### Example 241. Compound 310 ((2R)-1-[(3R)-3-[(S)-amino(4,5-dichloro-2-hydroxyphenyl)methyl]pyrrolidin-1-yl]-2,3-dihydroxypropan-1-one); Compound 311 ((2R)-1-[(3R)-3-[(R)-amino(4,5-dichloro-2-hydroxyphenyl)methyl]pyrrolidin-1-yl]-2,3-dihydroxypropan-1-one)

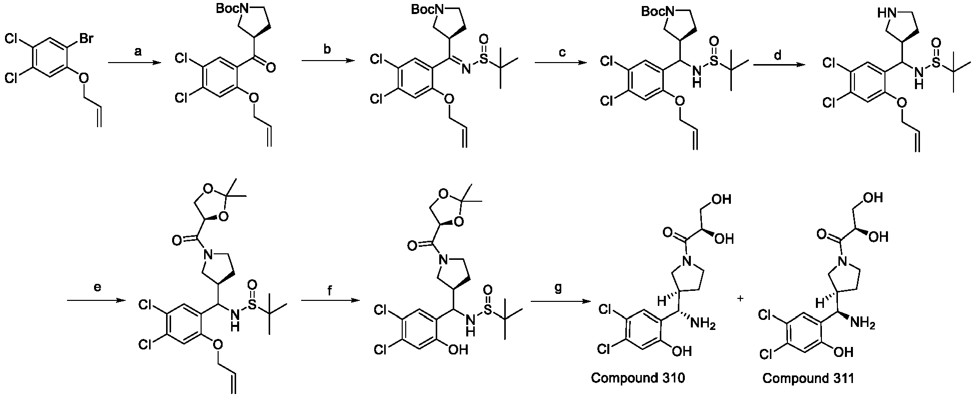

### Step a:

To a stirred mixture of 1-bromo-4,5-dichloro-2-(prop-2-en-1-yloxy)benzene (Example 6, step b) (5.46 g, 19.36 mmol) in THF (30 mL) was added *i*-PrMgCl·LiCl (18 mL, 23.23 mmol, 1.3 M in THF) dropwise at -10 °C under nitrogen atmosphere. The resulting mixture was stirred for 30 min at -10 °C under nitrogen atmosphere. To the above mixture was added a solution of *tert*-butyl (3*R*)-3-[methoxy(methyl)carbamoyl]pyrrolidine-1-carboxylate (1.00 g, 3.87 mmol) in THF (1 mL) dropwise over 5 min at -10 °C. The resulting mixture was stirred for additional 2 h at -10 °C. The reaction was quenched with saturated aq. NH₄Cl (20 mL) at room temperature. The resulting mixture was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (4/1) to afford *tert*-butyl (3*R*)-3-[4,5-dichloro-2-(prop-2-en-1-yloxy)benzoyl]pyrrolidine-1-carboxylate as a light yellow oil (0.45 g, 23%): LCMS (ESI) calc'd for C₁₉H₂₃Cl₂NO₄ [M + Na]⁺: 422, 424 (3 : 2), found 422, 424 (3 : 2); ¹H NMR (400 MHz, CDCl₃) δ 7.79 (s, 1H), 7.08 (s, 1H), 6.14-5.95 (m, 1H), 5.53-5.33 (m, 2H), 4.64 (d, *J =* 5.7 Hz, 2H), 4.03-3.91 (m, 1H), 3.68-3.54 (m, 2H), 3.51-3.35 (m, 2H), 2.21-2.04 (m, 2H), 1.48 (s, 9H).

### Step b:

To a stirred solution of tert-butyl (3*R*)-3-[4,5-dichloro-2-(prop-2-en-1-yloxy)benzoyl]pyrrolidine-1-carboxylate (0.25 g, 0.63 mmol) and *tert*-butanesulfinamide (0.23 g, 1.87 mmol) in THF (5 mL) was added Ti(OEt)₄ (0.85 g, 3.75 mmol) at room temperature. The resulting solution was stirred for 16 h at 70 °C under nitrogen atmosphere. The reaction was quenched with saturated aq. NaHCO₃ (20 mL) at room temperature. The resulting mixture was filtered and the filter cake were washed with EA (3 x 10 mL). The resulting mixture was extracted with EA (3 x 20 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (1/1) to afford *tert*-butyl (3*R*)-3-[[4,5-dichloro-2-(prop-2-en-1-yloxy)phenyl][(2-methylpropane-2-sulfinyl)imino]methyl]pyrrolidine-1-carboxylate as a light yellow oil (0.30 g, 67%): LCMS (ESI) calc'd for C₂₃H₃₂Cl₂N₂O₄S [M + Na]⁺: 525, 527 (3 : 2), found 525, 527 (3 : 2); ¹H NMR (400 MHz, CDCl₃) δ 7.18 (s, 1H), 6.99 (s, 1H), 6.08-5.94 (m, 1H), 5.48-5.28 (m, 2H), 4.62-4.52 (m, 2H), 3.76-3.61 (m, 3H), 3.60-3.46 (m, 1H), 3.41-3.29 (m, 1H), 2.34-1.94 (m, 2H), 1.47 (s, 9H), 1.24 (s, 9H).

### Step c:

To a stirred solution of *tert*-butyl (3*R*)-3-[[4,5-dichloro-2-(prop-2-en-1-yloxy)phenyl][(2-methylpropane-2-sulfinyl)imino]methyl]pyrrolidine-1-carboxylate (0.30 g, 0.60 mmol) in THF (5 mL) was added NaBH₄ (68 mg, 1.79 mmol) at room temperature. The resulting mixture was stirred for 1 h at room temperature. The reaction was quenched with water (20 mL) and extracted with EA (3 x 20 mL). The combined organic layers were washed with brine (3 x 20 mL), dried over anhydrous Na₂SO₄. After filtration, the filtrate was concentrated under reduced pressure to afford *tert*-butyl (3*R*)-3-[[4,5-dichloro-2-(prop-2-en-1-yloxy)phenyl][(2-methylpropane-2-sulfinyl)amino]methyl]pyrrolidine-1-carboxylate as a light yellow oil (0.30 g, crude), which was used in the next step directly without further purification: LCMS (ESI) calc'd for C₂₃H₃₄Cl₂N₂O₄S [M + H]⁺: 505, 507 (3 : 2), found 505, 507 (3 : 2); ¹H NMR (400 MHz, CDCl₃) δ 7.33 (s, 1H), 6.97 (s, 1H), 6.10-5.95 (m, 1H), 5.48-5.30 (m, 2H), 4.66-4.48 (m, 2H), 3.77-3.41 (m, 4H), 3.38-3.13 (m, 2H), 2.73-2.58 (m, 1H), 1.90-1.65 (m, 1H), 1.50-1.43 (m, 9H), 1.25 (s, 9H).

### Step d:

To a stirred solution of *tert*-butyl (3*R*)-3-[[4,5-dichloro-2-(prop-2-en-1-yloxy)phenyl][(2-methylpropane-2-sulfinyl)amino]methyl]pyrrolidine-1-carboxylate (0.30 g, 0.60 mmol) in DCM (2 mL) were added TFA (0.4 mL) at room temperature. The resulting mixture was stirred for 30 min at room temperature. The reaction was neutralized to pH 7 with saturated aq. NaHCO₃ (20 mL) at room temperature and extracted with EA (3 x 20 mL). The combined organic layers were washed with brine (3 x 20 mL), dried over anhydrous Na₂SO₄. After filtration, the filtrate was concentrated under reduced pressure to afford *N*-[[4,5-dichloro-2-(prop-2-en-1-yloxy)phenyl]((3*R*)-pyrrolidin-3-yl)methyl]-2-methylpropane-2-sulfinamide as a light yellow solid (0.30 g, crude), which was used in the next step directly without further purification: LCMS (ESI) calc'd for C₁₈H₂₆Cl₂N₂O₂S [M + H]⁺: 405, 407 (3 : 2), found 405, 407 (3 : 2).

### Step e:

To a stirred mixture of (4R)-2,2-dimethyl-1,3-dioxolane-4-carboxylic acid (0.32 g, 2.22 mmol) and HATU (1.13 g, 2.96 mmol) in DMF (5 mL) were added *N*-[[4,5-dichloro-2-(prop-2-en-1-yloxy)phenyl]((3*R*)-pyrrolidin-3-yl)methyl]-2-methylpropane-2-sulfinamide (0.30 g, 0.74 mmol) and Et₃N (0.22 g, 2.22 mmol) at room temperature. The resulting mixture was stirred for 1 h at room temperature. The residue was purified by reverse phase chromatography, eluted with 57% ACN in water (plus 0.05% TFA) to afford *N*-[[4,5-dichloro-2-(prop-2-en-1-yloxy)phenyl][(3*R*)-1-[(4*R*)-2,2-dimethyl-1,3-dioxolane-4-carbonyl]pyrrolidin-3-yl]methyl]-2-methylpropane-2-sulfinamide as an off-white solid (0.20 g, 60% overall four steps): LCMS (ESI) calc'd for C₂₄H₃₄Cl₂N₂O₅S [M + H]⁺: 533, 535 (3 : 2), found 533, 535 (3 : 2); ¹H NMR (400 MHz, CDCl₃) δ 7.27-7.21 (m, 1H), 7.03-6.95 (m, 1H), 6.14-5.97 (m, 1H), 5.50-5.31 (m, 2H), 4.81-4.67 (m, 1H), 4.67-4.50 (m, 3H), 4.45-4.20 (m, 1H), 4.20-4.09 (m, 1H), 4.05-3.74 (m, 1H), 3.71-3.55 (m, 1H), 3.52-3.28 (m, 1H), 2.86-2.58 (m, 1H), 2.27-1.96 (m, 2H), 1.92-1.59 (m, 1H), 1.53-1.28 (m, 6H), 1.20-1.12 (m, 9H).

### Step f:

To a stirred solution of *N*-[[4,5-dichloro-2-(prop-2-en-1-yloxy)phenyl][(3*R*)-1-[(4*R*)-2,2-dimethyl-1,3-dioxolane-4-carbonyl]pyrrolidin-3-yl]methyl]-2-methylpropane-2-sulfinamide (0.20 g, 0.38 mmol) and Pd(PPh₃)₄ (4 mg, 0.004 mmol) in THF (3 mL) was added NaBH₄ (43 mg, 1.13 mmol) at room temperature. The resulting mixture was stirred for 30 min at room temperature. The resulting mixture was quenched with saturated aq. NH₄Cl (1 mL) and concentrated under reduced pressure to afford *N*-[(4,5-dichloro-2-hydroxyphenyl)[(3R)-1-[(4R)-2,2-dimethyl-1,3-dioxolane-4-carbonyl]pyrrolidin-3-yl]methyl]-2-methylpropane-2-sulfinamide as a brown solid (0.20 g, crude), which was used in the next step directly without further purification: LCMS (ESI) calc'd for C₂₁H₃₀Cl₂N₂O₅S [M + H]⁺: 493, 495 (3 : 2), found 493, 495 (3 : 2).

### Step g:

To a stirred mixture of *N*-[(4,5-dichloro-2-hydroxyphenyl)[(3*R*)-1-[(4*R*)-2,2-dimethyl-1,3-dioxolane-4-carbonyl]pyrrolidin-3-yl]methyl]-2-methylpropane-2-sulfinamide (0.20 g, 0.41 mmol) in 1,4-dioxane (1 mL) was added aq. HCl (4 *N,* 1 mL) at room temperature. The resulting mixture was stirred for 30 min at room temperature. The resulting mixture was concentrated under reduced pressure. The residue was purified by Prep-HPLC with the following conditions: Column: XBridge Shield RP18 OBD Column, 30 x 150 mm, 5 µm; Mobile Phase A: water with 10 mmol/L NH₄HCO₃, Mobile Phase B: ACN; Flow rate: 60 mL/min; Gradient: 15% B to 40% B in 7 min; Detector: UV 254/220 nm; Retention time: RT₁: 6.50 min; RT₂: 6.70 min.

The fractions containing the desired product at 6.50 min were collected and concentrated under reduced pressure to afford Compound 310 ((2R)-1-[(3R)-3-[(S)-amino(4,5-dichloro-2-hydroxyphenyl)methyl]pyrrolidin-1-yl]-2,3-dihydroxypropan-1-one) as an off-white solid (15.6 mg, 11% overall two steps): LCMS (ESI) calc'd for C₁₄H₁₈Cl₂N₂O₄ [M + H]⁺: 349, 351 (3 : 2), found 349, 351 (3 : 2); ¹H NMR (400 MHz, CD₃OD) δ 7.32 (d, *J* = 4.9 Hz, 1H), 6.90 (s, 1H), 4.30 (dt, *J =* 51.2, 5.4 Hz, 1H), 4.07-3.84 (m, 2H), 3.78-3.41 (m, 4H), 3.31-3.01 (m, 1H), 2.82-2.63 (m, 1H), 2.38-2.15 (m, 1H), 1.97-1.66 (m, 1H).

The fractions containing the desired product at 6.70 min were collected and concentrated under reduced pressure to afford Compound 311 ((2*R*)-1-[(3*R*)-3-[(*R*)-amino(4,5-dichloro-2-hydroxyphenyl)methyl]pyrrolidin-1-yl]-2,3-dihydroxypropan-1-one) as an off-white solid (31.3 mg, 22% overall two steps): LCMS (ESI) calc'd for C₁₄H₁₈Cl₂N₂O₄ [M + H]⁺: 349, 351 (3 : 2), found 349, 351 (3 : 2); ¹H NMR (400 MHz, CD₃OD) δ 7.32 (d, *J =* 9.7 Hz, 1H), 6.90 (d, *J* = 7.4 Hz, 1H), 4.40 (dt, *J =* 24.9, 5.5 Hz, 1H), 4.08-3.89 (m, 2H), 3.85-3.36 (m, 4H), 3.30-3.22 (m, 1H), 2.81-2.58 (m, 1H), 1.80-1.52 (m, 2H).

### Example 242. Compound 313 ((2R)-1-[8-[(R)-amino(4,5-dichloro-2-hydroxyphenyl)methyl]-3-azabicyclo[3.2.1]octan-3-yl]-2,3-dihydroxypropan-1-one trifluoroacetic acid);

### Compound 314 ((2R)-1-[8-[(S)-amino(4,5-dichloro-2-hydroxyphenyl)methyl]-3-azabicyclo[3.2.1]octan-3-yl]-2,3-dihydroxypropan-1-one trifluoroacetic acid)

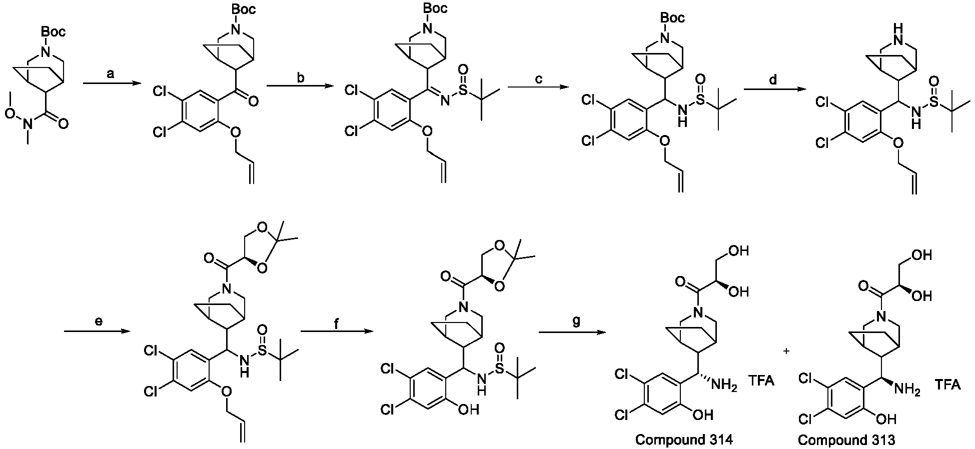

### Step a:

To a stirred solution of 1-bromo-4,5-dichloro-2-(prop-2-en-1-yloxy)benzene (0.61 g, 2.16 mmol) in THF (5 mL) was added *i*-PrMgCl (1.44 mL, 2.88 mmol, 2 M in THF) dropwise at 0 °C under nitrogen atmosphere. After stirring at 0 °C for 30 min under nitrogen atmosphere, a solution of *tert*-butyl 8-[methoxy(methyl)carbamoyl]-3-azabicyclo[*3*.*2*.*1*]octane-3-carboxylate (Intermediate 22, Example 22) (0.43 g, 1.44 mmol) in THF (2 mL) was added dropwise into resulting solution. The reaction was stirred at 0 °C for additional 1 h. The reaction was quenched with saturated aq. NH₄Cl (2 mL) at 0 °C and diluted with EA (20 mL) and water (30 mL). The aqueous layer was extracted with EA (3 x 20 mL). The combined organic layers were washed with brine (2 x 20 mL) and dried over anhydrous Na₂SO₄. After filtration, the filtrate was concentrated under reduced pressure. The residue was purified by silica gel column chromatography, eluted with PE/EA (6/1) to afford *tert*-butyl 8-[4,5-dichloro-2-(prop-2-en-1-yloxy)benzoyl]-3-azabicyclo[*3.2.1*]octane-3-carboxylate as a colorless oil (0.30 g, 45%): LCMS (ESI) calc'd for C₂₂H₂₇Cl₂NO₄ [M + Na]⁺: 462, 464 (3 : 2), found 462, 464 (3 : 2); ¹H NMR (400 MHz, CDCl₃) δ 7.68 (d, *J =* 9.9 Hz, 1H), 7.07 (d, *J =* 6.3 Hz, 1H), 6.14-5.96 (m, 1H), 5.44 (dt, *J =* 25.1, 8.5 Hz, 2H), 4.63 (dd, *J* = 18.1, 5.5 Hz, 2H), 4.07-3.79 (m, 1H), 3.79-3.48 (m, 1H), 3.27-2.84 (m, 2H), 2.58-2.37 (m, 2H), 1.86-1.72 (m, 1H), 1.72-1.50 (m, 4H), 1.47 (d, *J* = 9.5 Hz, 9H).

### Step b:

To a stirred solution of *tert*-butyl 8-[4,5-dichloro-2-(prop-2-en-1-yloxy)benzoyl]-3-azabicyclo[*3.2.1*]octane-3-carboxylate (0.30 g, 0.65 mmol) and *tert*-butanesulfinamide (0.24 g, 1.95 mmol) in THF (5 mL) was added Ti(OEt)₄ (1.48 g, 6.50 mmol) at room temperature under nitrogen atmosphere. The reaction was allowed to warm to 70 °C and stirred for 16 h under nitrogen atmosphere. The reaction was quenched with saturated aq. Na₂CO₃ (30 mL). The resulting mixture was filtered through celite and the filtrate was diluted with EA (30 mL) and water (30 mL). The aqueous layer was extracted with EA (3 x 30 mL). The combined organic layers were washed with brine (3 x 20 mL), dried over anhydrous Na₂SO₄. After filtration, the filtrate was concentrated under reduced pressure to afford *tert*-butyl 8-[[4,5-dichloro-2-(prop-2-en-1-yloxy)phenyl][(2-methylpropane-2-sulfinyl)imino]methyl]-3-azabicyclo[*3.2.1*]octane-3-carboxylate as a yellow oil (0.43 g, crude), which was used in the next step directly without further purification: LCMS (ESI) calc'd for C₂₆H₃₆Cl₂N₂O₄S [M + H]⁺: 543, 545 (3 : 2), found 543, 545 (3 : 2); ¹H NMR (400 MHz, CDCl₃) δ 7.29 (s, 1H), 6.98 (s, 1H), 6.09-5.90 (m, 1H), 5.36 (dd, *J =* 35.9, 13.9 Hz, 2H), 4.73-4.49 (m, 2H), 4.04-3.54 (m, 5H), 2.99-2.65 (m, 2H), 2.60-2.13 (m, 1H), 2.00-1.57 (m, 3H), 1.47 (d, *J =* 3.8 Hz, 9H), 1.24 (s, 9H).

### Step c:

To a stirred solution of *tert*-butyl 8-[[4,5-dichloro-2-(prop-2-en-1-yloxy)phenyl][(2-methylpropane-2-sulfinyl)imino]methyl]-3-azabicyclo[*3.2.1*]octane-3-carboxylate (0.43 g, 0.79 mmol) in THF (5 mL) and MeOH (3 mL) was added NaBH₄ (0.24 g, 6.33 mmol) at room temperature. The reaction was stirred at room temperature for 8 h. The reaction was quenched with saturated aq. NH₄Cl (2 mL), and then diluted water (30 mL). The aqueous layer was extracted with EA (3 x 20 mL). The combined organic layers were washed with brine (2 x 20 mL) and dried over anhydrous Na₂SO₄. After filtration, the filtrate was concentrated under reduced pressure to afford *tert*-butyl 8-[[4,5-dichloro-2-(prop-2-en-1-yloxy)phenyl][(2-methylpropane-2-sulfinyl)amino]methyl]-3-azabicyclo[*3.2.1*]octane-3-carboxylate as a light yellow solid (0.34 g, 96% overall two steps): LCMS (ESI) calc'd for C₂₆H₃₈Cl₂N₂O₄S [M + H]⁺: 545, 547 (3 : 2), found 545, 547 (3 : 2); ¹H NMR (400 MHz, CD₃OD) δ 7.58-7.44 (m, 1H), 7.17 (s, 1H), 6.15-5.98 (m, 1H), 5.57-5.26 (m, 2H), 4.70-4.49 (m, 2H), 3.99-3.88 (m, 1H), 3.80-3.66 (m, 1H), 3.54-3.35 (m, 1H), 3.03-2.82 (m, 1H), 2.82-2.42 (m, 2H), 2.02-1.68 (m, 3H), 1.66-1.51 (m, 3H), 1.46 (s, 9H), 1.23 (s, 9H).

### Step d:

To a stirred solution of *tert*-butyl 8-[[4,5-dichloro-2-(prop-2-en-1-yloxy)phenyl][(2-methylpropane-2-sulfinyl)amino]methyl]-3-azabicyclo[*3.2.1*]octane-3-carboxylate (0.34 g, 0.62 mmol) in DCM (5 mL) was added TFA (1 mL) at room temperature. The reaction was stirred at room temperature for 0.5 h. The reaction was quenched with saturated aq. Na₂CO₃ (20 mL) and neutralized to pH 7 with saturated aq. Na₂CO₃ (30 mL). The resulting solution was diluted with EA (30 mL) and water (30 mL) and the aqueous layer was extracted with EA (3 x 30 mL). The combined organic layers were washed with brine (3 x 20 mL), dried over anhydrous Na₂SO₄. After filtration, the filtrate was concentrated under reduced pressure to afford *N*-[3-azabicyclo[*3*.*2.1*]octan-8-yl[4,5-dichloro-2-(prop-2-en-1-yloxy)phenyl]methyl]-2-methylpropane-2-sulfinamide as an off-white solid (0.30 g, crude), which was used in the next step directly without further purification: LCMS (ESI) calc'd for C₂₁H₃₀Cl₂N₂O₂S [M + H]⁺: 445, 447, (3 : 2), found 445, 447, (3 : 2).

### Step e:

To a stirred solution of (*4R*)-2,2-dimethyl-1,3-dioxolane-4-carboxylic acid (0.15 g, 1.01 mmol) and HATU (0.38 g, 1.01 mmol) in DMF (5 mL) were added *N*-[3-azabicyclo[*3*.*2.1*]octan-8-yl[4,5-dichloro-2-(prop-2-en-1-yloxy)phenyl]methyl]-2-methylpropane-2-sulfinamide (0.30 g, 0.67 mmol) and Et₃N (0.20 g, 2.02 mmol) at room temperature. The reaction was stirred at room temperature for 1 h. The reaction was purified by reverse phase chromatography, eluted with 56% ACN in water (plus 0.05% TFA) to afford *N-*[[4,5-dichloro-2-(prop-2-en-1-yloxy)phenyl]([3-[(*4R*)-2,2-dimethyl-1,3-dioxolane-4-carbonyl]-3-azabicyclo[*3.2.1*]octan-8-yl])methyl]-2-methylpropane-2-sulfinamide as a colorless oil (0.20 g, 56% overall two steps): LCMS (ESI) calc'd for C₂₇H₃₈Cl₂N₂O₅S [M + H]⁺: 573, 575 (3 : 2), found 573, 575 (3 : 2). ¹H NMR (400 MHz, CD₃OD) δ 7.65-7.47 (m, 1H), 7.22-7.16 (m, 1H), 6.25-6.00 (m, 1H), 5.40 (dd, *J* = 43.9, 14.0 Hz, 2H), 4.76-4.41 (m, 2H), 4.44-4.17 (m, 2H), 4.17-4.07 (m, 1H), 4.02-3.88 (m, 1H), 3.86-3.50 (m, 2H), 3.21-2.77 (m, 1H), 2.75-2.64 (m, 1H), 2.64-2.53 (m, 1H), 2.32-2.09 (m, 1H), 2.01-1.82 (m, 2H), 1.73-1.59 (m, 3H) 1.45-1.30 (m, 6H), 1.12 (s, 9H).

### Step f:

To a stirred solution of *N*-[[4,5-dichloro-2-(prop-2-en-1-yloxy)phenyl]([3-[(*4R*)-2,2-dimethyl-1,3-dioxolane-4-carbonyl]-3-azabicyclo[*3.2.1*]octan-8-yl])methyl]-2-methylpropane-2-sulfinamide (0.20 g, 0.35 mmol) and Pd(PPh₃)₄ (40 mg, 0.04 mmol) in THF (5 mL) was added NaBH₄ (26 mg, 0.70 mmol) at room temperature. The reaction was stirred at room temperature for 30 min. The reaction was quenched with saturated aq. NH₄Cl (1 mL) and concentrated under reduced pressure to afford *N*-[(4,5-dichloro-2-hydroxyphenyl)([3-[(*4R*)-2,2-dimethyl-1,3-dioxolane-4-carbonyl]-3-azabicyclo[*3.2.1*]octan-8-yl])methyl]-2-methylpropane-2-sulfinamide as a yellow oil (0.20 g, crude), which was used in the next step directly without further purification: LCMS (ESI) calc'd for C₂₄H₃₄Cl₂N₂O₅S [M + H]⁺ 533, 535 (3 : 2), found 533, 535 (3 : 2).

### Step g:

To a stirred solution of *N*-[(4,5-dichloro-2-hydroxyphenyl)([3-[(*4R*)-2,2-dimethyl-1,3-dioxolane-4-carbonyl]-3-azabicyclo[*3.2.1*]octan-8-yl])methyl]-2-methylpropane-2-sulfinamide (0.20 g, 0.38 mmol) in 1,4-dioxane (5 mL) was added aq. HCl (4 *N,* 2 mL) at room temperature. The reaction was stirred at room temperature for 1 h. The reaction was diluted with EA (20 mL) and HCl (1 *N,* 20 mL). The organic layers were washed with aq. HCl (1 *N,* 2 x 20 mL). The combined aqueous layers were concentrated under reduced pressure. The residue was purified by Prep-HPLC with following conditions: Column: Xselect CSH OBD Column 30 x 150 mm 5 µm; Mobile Phase A: water (plus 0.05% TFA), Mobile Phase B: ACN; Flow rate: 60 mL/min; Gradient: 5% B to 25% B in 8 min; Detector: UV 254/220 nm; Retention time: RT₁: 8.65 min, RT₂: 9.32 min.

The fractions containing the desired product at 8.65 min were collected and concentrated under reduced pressure to afford Compound 313 ((*2R*)-1-[8-[(*R*)-amino(4,5-dichloro-2-hydroxyphenyl)methyl]-3-azabicyclo[*3.2.1*]octan-3-yl]-2,3-dihydroxypropan-1-one trifluoroacetic acid) as an off-white solid (57.7 mg, 33% overall two steps): LCMS (ESI) calc'd for C₁₇H₂₂Cl₂N₂O₄ [M + H]⁺: 389, 391 (3 :2), found 389, 391 (3 :2); ¹H NMR (400 MHz, CD₃OD) δ 7.48 (d, *J =* 2.8 Hz, 1H), 7.11 (s, 1H), 4.58-4.24 (m, 1H), 4.24-4.01 (m, 2H), 3.93-3.55 (m, 3H), 3.30-3.05 (m, 1H), 2.78-2.72 (m, 1H), 2.63-2.39 (m, 2H), 2.03-1.93 (m, 1H), 1.93-1.82 (m, 1H), 1.80-1.43 (m, 3H); ¹⁹F NMR (376 MHz, CD₃OD) δ -77.19.

The fractions containing the desired product at 9.32 min were collected and concentrated under reduced pressure to afford Compound 314 ((*2R*)-1-[8-[(*S*)-amino(4,5-dichloro-2-hydroxyphenyl)methyl]-3-azabicyclo[*3.2.1*]octan-3-yl]-2,3-dihydroxypropan-1-one trifluoroacetic acid) as an off-white solid (13.3 mg, 10% overall two steps): LCMS (ESI) calc'd for C₁₇H₂₂Cl₂N₂O₄ [M + H]⁺ 389, 391 (3 :2), found 389, 391 (3 :2); ¹H NMR (400 MHz, CD₃OD) δ 7.59 (d, *J* = 6.3 Hz, 1H), 7.12 (s, 1H), 5.02 (s, 1H), 4.64-4.44 (m, 1H), 4.27-3.95 (m, 1H), 3.95-3.59 (m, 3H), 3.54-3.48 (m, 1H), 3.26-3.01 (m, 1H), 2.62-2.49 (m, 1H), 2.49-2.36 (m, 1H), 1.99-1.83 (m, 1H), 1.83-1.75 (m, 2H), 1.75-1.47 (m, 2H); ¹⁹F NMR (376 MHz, CD₃OD) δ - 76.98.

### Example 243. Compound 315 ((2R)-1-(5-((R)-amino(4,5-dichloro-2-hydroxyphenyl)methyl)-2-azabicyclo[2.2.1]heptan-2-yl)-2,3-dihydroxypropan-1-one trifluoroacetic acid);

### Compound 316 ((2R)-1-(5-((S)-amino(4,5-dichloro-2-hydroxyphenyl)methyl)-2-azabicyclo[2.2.1]heptan-2-yl)-2,3-dihydroxypropan-1-one trifluoroacetic acid)

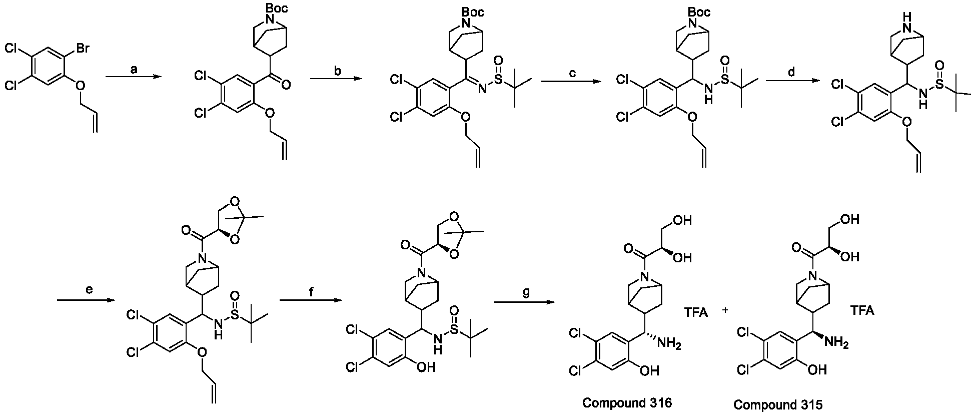

### Step a:

To a stirred solution of 1-bromo-4,5-dichloro-2-(prop-2-en-1-yloxy)benzene (Example 6, step b) (0.97 g, 3.43 mmol) in THF (5 mL) was added *i*-PrMgCl (1.71 mL, 3.42 mmol, 2 M in THF) dropwise at 0 °C under nitrogen atmosphere. The resulting solution was stirred for 30 min at 0 °C under nitrogen atmosphere. To the above mixture was added a solution of *tert*-butyl 5-[methoxy(methyl)carbamoyl]-2-azabicyclo[*2.2.1*]heptane-2-carboxylate (0.65 g, 2.29 mmol) in THF (2 mL) at 0 °C under nitrogen atmosphere. The resulting solution was stirred for 1 h at room temperature under nitrogen atmosphere. The reaction was quenched with saturated aq. NH₄Cl (20 mL) at 0 °C. The reaction mixture was extracted with EA (3 x 30 mL). The combined organic layers were washed with brine (2 x 30 mL) and dried over anhydrous Na₂SO₄. After filtration, the filtrate was concentrated under reduced pressure. The residue was purified by silica gel column chromatography, eluted with PE/EA (4/1) to afford *tert*-butyl 5-[4,5-dichloro-2-(prop-2-en-1-yloxy)benzoyl]-2-azabicyclo[*2.2.1*]heptane-2-carboxylate as a yellow oil (0.15 g, 20%): LCMS (ESI) calc'd for C₂₁H₂₅Cl₂NO₄ [M + H - 15]⁺: 411, 413 (3 : 2), found 411, 413 (3 : 2); ¹H NMR (400 MHz, CDCl₃) δ 7.77 (s, 1H), 7.08 (s, 1H), 6.12-6.00 (m, 1H), 5.48-5.39 (m, 2H), 4.65 (d, *J =* 5.4 Hz, 2H), 4.28-4.23 (m, 1H), 3.51 (dd, *J =* 8.8, 5.5 Hz, 1H), 3.30 (dd, *J =* 9.8, 3.5 Hz, 1H), 3.12 (d, *J =* 9.8 Hz, 1H), 2.80-2.75 (m, 1H), 2.09-2.02 (m, 1H), 1.95-1.87 (m, 1H), 1.66-1.55 (m, 2H), 1.49 (s, 9H).

### Step b:

To a stirred solution of *tert*-butyl 5-[4,5-dichloro-2-(prop-2-en-1-yloxy)benzoyl]-2-azabicyclo[*2.2.1*]heptane-2-carboxylate (0.15 g, 0.35 mmol) and 2-methylpropane-2-sulfinamide (85 mg, 0.70 mmol) in THF (3 mL) were added Ti(OEt)₄ (0.80 g, 3.52 mmol) at room temperature. The resulting mixture was stirred for 16 h at 70 °C under nitrogen atmosphere. The reaction was quenched with saturated aq. NaHCO₃ (10 mL) at room temperature. The resulting mixture was filtered. The filter cake were washed with EA (5 x 20 mL). The filtrate was concentrated under reduced pressure to afford *tert*-butyl 5-[[4,5-dichloro-2-(prop-2-en-1-yloxy)phenyl][(2-methylpropane-2-sulfinyl)imino]methyl]-2-azabicyclo[*2.2.1*]heptane-2-carboxylate as a yellow oil (0.22 g, crude), which was used in the next step directly without further purification: LCMS (ESI) calc'd for C₂₅H₃₄Cl₂N₂O₄S for [M + H]⁺: 529, 531 (3 : 2), found 529, 531 (3 : 2);

### Step c:

To a stirred solution of tert-butyl 5-[[4,5-dichloro-2-(prop-2-en-1-yloxy)phenyl][(2-methylpropane-2-sulfinyl)imino]methyl]-2-azabicyclo[*2.2.1*]heptane-2-carboxylate (0.22 g, 0.42 mmol) in THF (3 mL) was added NaBH₄ (31 mg, 0.83 mmol) at room temperature. The resulting mixture was stirred for 1 h at room temperature. The reaction was quenched with saturated aq. NH₄Cl (20 mL) at room temperature. The reaction mixture was extracted with EA (3 x 20 mL). The combined organic layers were washed with brine (3 x 20 mL) and dried over anhydrous Na₂SO₄. After filtration, the filtrate was concentrated under reduced pressure to afford *tert*-butyl 5-[[4,5-dichloro-2-(prop-2-en-1-yloxy)phenyl][(2-methylpropane-2-sulfinyl)amino]methyl]-2-azabicyclo[*2.2.1*]heptane-2-carboxylate as a yellow oil (0.18 g, 90% overall two steps): LCMS (ESI) calc'd for C₂₅H₃₆Cl₂N₂O₄S for [M + H]⁺: 531, 533 (3 : 2), found 531, 533 (3 : 2); ¹H NMR (400 MHz, CD₃OD) δ 7.50 (d, *J =* 18.3 Hz, 1H), 7.17 (dd, *J =* 12.9, 2.8 Hz, 1H), 6.22-6.01 (m, 1H), 5.55-5.39 (m, 1H), 5.39-5.26 (m, 1H), 4.71-4.56 (m, 3H), 4.56-4.44 (m, 1H), 4.38-4.18 (m, 1H), 4.17-4.04 (m, 1H), 3.18-2.98 (m, 1H), 2.92-2.74 (m, 1H), 2.36-2.05 (m, 2H), 1.81-1.55 (m, 2H), 1.52-1.41 (m, 9H), 1.25-1.18 (m, 9H).

### Step d:

A solution of *tert*-butyl 5-[[4,5-dichloro-2-(prop-2-en-1-yloxy)phenyl][(2-methylpropane-2-sulfinyl)amino]methyl]-2-azabicyclo[*2.2.1*]heptane-2-carboxylate (0.18 g, 0.34 mmol) in TFA (1 mL) and DCM (5 mL) was stirred for 30 min at room temperature. The reaction was quenched with saturated aq. NaHCO₃ (10 mL) and neutralized to pH 7 with saturated aq. NaHCO₃ at 0 °C. The resulting mixture was extracted with EA (3 x 30 mL). The combined organic layers were washed with brine (3 x 20 mL) and dried over anhydrous Na₂SO₄. After filtration, the filtrate was concentrated under reduced pressure to *N*-[2-azabicyclo[*2.2.1*]heptan-5-yl[4,5-dichloro-2-(prop-2-en-1-yloxy)phenyl]methyl]-2-methylpropane-2-sulfinamide as a yellow oil (0.28 g, crude), which was used in the next step directly without further purification: LCMS (ESI) calc'd for C₂₀H₂₈Cl₂N₂O₂S for [M + H]⁺: 431, 433 (3 : 2), found 431, 433 (3 : 2).

### Step e:

To a stirred solution of (4*R*)-2,2-dimethyl-1,3-dioxolane-4-carboxylic acid (0.14 g, 0.97 mmol) and HATU (0.37 g, 0.97 mmol) in DMF (5 mL) were added *N*-[2-azabicyclo[*2.2.1*]heptan-5-yl[4,5-dichloro-2-(prop-2-en-1-yloxy)phenyl]methyl]-2-methylpropane-2-sulfinamide (0.28 g, 0.65 mmol) and Et₃N (0.13 g, 1.30 mmol) at room temperature. The resulting mixture was stirred for 1 h at room temperature. The reaction was purified by reverse phase chromatography, eluted with 55% ACN in water (plus 0.05% TFA) to afford *N*-[[4,5-dichloro-2-(prop-2-en-1-yloxy)phenyl]([2-[(4R)-2,2-dimethyl-1,3-dioxolane-4-carbonyl]-2-azabicyclo[*2.2.1*]heptan-5-yl])methyl]-2-methylpropane-2-sulfinamide as a yellow oil (0.12 g, 63% overall two steps): LCMS (ESI) calc'd for C₂₆H₃₆Cl₂N₂O₅S for [M + H]⁺: 559, 561(3 : 2), found 559, 561 (3 : 2).

### Step f:

To a stirred solution of *N*-[[4,5-dichloro-2-(prop-2-en-1-yloxy)phenyl]([2-[(4*R*)-2,2-dimethyl-1,3-dioxolane-4-carbonyl]-2-azabicyclo[*2.2.1*]heptan-5-yl])methyl]-2-methylpropane-2-sulfinamide (0.12 g, 0.21 mmol) and Pd(PPh₃)₄ (3 mg, 0.002 mmol) in THF (5 mL) was added NaBH₄ (16 mg, 0.43 mmol) at room temperature. The resulting mixture was stirred for 0.5 h at room temperature. The reaction was quenched with saturated aq. NH₄Cl (0.5 mL) at room temperature. The resulting mixture was concentrated under reduced pressure to afford *N*-[(4,5-dichloro-2-hydroxyphenyl)([2-[(4*R*)-2,2-dimethyl-1,3-dioxolane-4-carbonyl]-2-azabicyclo[*2.2.1*]heptan-5-yl])methyl]-2-methylpropane-2-sulfinamide as a brown solid (0.10 g, crude), which was used in the next step directly without further purification: LCMS (ESI) calc'd for C₂₃H₃₂Cl₂N₂O₅S for [M + H]⁺: 519, 521 (3 : 2), found 519, 521 (3 : 2).

### Step g:

A solution of *N*-[(4,5-dichloro-2-hydroxyphenyl)([2-[(4*R*)-2,2-dimethyl-1,3-dioxolane-4-carbonyl]-2-azabicyclo[*2.2.1*]heptan-5-yl])methyl]-2-methylpropane-2-sulfinamide (0.10 g) in aq. HCl (4 *N,* 1 mL) and dioxane (1 mL) was stirred for 30 min at room temperature. The resulting solution was concentrated under reduced pressure. The residue was purified by Prep-HPLC with the following conditions: Column: Xselect CSH OBD Column 30 x 150 mm 5µm n; Mobile Phase A: Water (plus 0.05% TFA), Mobile Phase B: ACN; Flow rate: 60 mL/min; Gradient: 20% B to 40% B in 7 min; Detector: UV 254/220 nm; Retention time: RT₁: 7.03 min; RT₂: 10.12 min.

The fractions containing the desired product at 7.03 min were collected and concentrated under reduced pressure to afford Compound 315 ((2*R*)-1-[5-[(*R*)-amino(4,5-dichloro-2-hydroxyphenyl)methyl]-2-azabicyclo[*2.2.1*]heptan-2-yl]-2,3-dihydroxypropan-1-one trifluoroacetic acid) as a light pink solid (7.2 mg, 7% over two steps): LCMS (ESI) calc'd for C₁₆H₂₀Cl₂N₂O₄ for [M + H]⁺: 375, 377 (3 : 2), found 375, 377 (3 : 2); ¹H NMR (400 MHz, CD₃OD) δ 7.49 (s, 1H), 7.07 (d, *J =* 1.8 Hz, 1H), 4.65-4.47 (m, 1H), 4.36-4.11 (m, 2H), 3.75-3.57 (m, 2H), 3.55-3.45 (m, 1H), 3.27-3.11 (m, 1H), 2.85-2.72 (m, 1H), 2.66-2.51 (m, 1H), 1.86-1.56 (m, 3H), 1.40-1.16 (m, 1H); ¹⁹F NMR (376 MHz, CD₃OD) δ -76.96.

The fractions containing the desired product at 10.12 min were collected and concentrated under reduced pressure to afford Compound 316 ((2*R*)-1-[5-[(*S*)-amino(4,5-dichloro-2-hydroxyphenyl)methyl]-2-azabicyclo[*2.2.1*]heptan-2-yl]-2,3-dihydroxypropan-1-one trifluoroacetic acid) as a purple solid (8.3 mg, 7% over two steps): LCMS (ESI) calc'd for C₁₆H₂₀Cl₂N₂O₄ for [M + H]⁺: 375, 377 (3 : 2), found 375, 377 (3 : 2); ¹H NMR (400 MHz, CD₃OD) δ 7.51 (d, *J* = 2.5 Hz, 1H), 7.11 (d, *J* = 1.5 Hz, 1H), 4.79-4.60 (m, 1H), 4.40-4.13 (m, 2H), 3.81-3.59 (m, 2H), 3.56-3.45 (m, 1H), 3.04 (dd, *J =* 27.2, 11.2 Hz, 1H), 2.52-2.39 (m, 1H), 2.24-2.05 (m, 2H), 1.85-1.55 (m, 3H); ¹⁹F NMR (376 MHz, CD₃OD) δ -77.11.

### Example 244. (which is a reference example) Compound 317 ((2R)-1-[4-[(R)-amino(4-bromo-5-chloro-2-hydroxyphenyl)methyl]piperidin-1-yl]-2,3-dihydroxypropan-1-one trifluoroacetic acid)

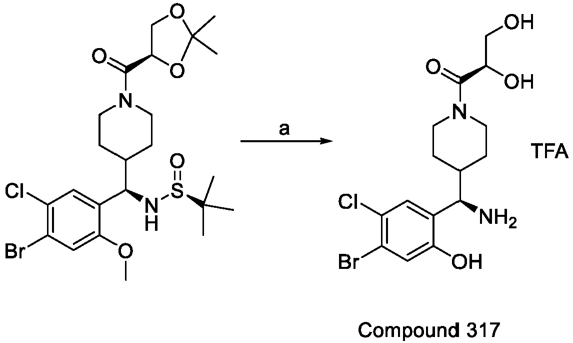

### Step a:

To a stirred mixture of *N*-[(*R*)-(4-bromo-5-chloro-2-methoxyphenyl)([1-[(4*R*)-2,2-dimethyl-1,3-dioxolane-4-carbonyl]piperidin-4-yl])methyl]-2-methylpropane-2-sulfinamide (Intermediate 23, Example 23) (40 mg, 0.07 mmol) in DCM (0.6 mL) was added BBr₃ (0.3 mL) dropwise at 0 °C under nitrogen atmosphere. The reaction mixture was stirred for 30 min at 0 °C. The resulting mixture was quenched with water (5 mL) and concentrated under reduced pressure. The residue was purified by Prep-HPLC with the following conditions: Column: XBridge Prep C18 OBD Column, 5 µm, 19 x 150 mm; Mobile Phase A: water (plus 0.05% TFA), Mobile Phase B: ACN; Flow rate: 25 mL/min; Gradient: 10% B to 35% B in 7 min; Detector: 220 nm; Retention time: 6.10 min. The fractions containing the desired product were collected and concentrated under reduced pressure to afford Compound 317 ((2*R*)-1-[4-[(*R*)-amino(4-bromo-5-chloro-2-hydroxyphenyl)methyl]piperidin-1-yl]-2,3-dihydroxypropan-1-one trifluoroacetic acid as a light yellow solid) (3.9 mg, 10%): LCMS (ESI) calc'd for C₁₅H₂₀BrClN₂O₄ [M + H]⁺: 407, 409 (2 : 3), found 407, 409 (2 : 3); ¹H NMR (400 MHz, CD₃OD) δ 7.43 (s, 1H), 7.25 (s, 1H), 4.58 (dd, *J =* 53.9, 13.6 Hz, 2H), 4.33-4.02 (m, 2H), 3.67 (d, *J =* 22.6 Hz, 2H), 3.09 (dt, *J* = 42.2, 12.2 Hz, 1H), 2.67 (dt, *J* = 42.3, 12.9 Hz, 1H), 2.38 (s, 1H), 2.01 (d, *J =* 12.1 Hz, 1H), 1.46-1.12 (m, 3H); ¹⁹F NMR (376 MHz, CD₃OD) δ -76.87.

### Example 245. Compound 326 ((2R)-1-[(2S,4R)-4-[(R)-amino(4,5-dichloro-2-hydroxyphenyl)methyl]-2-methylpiperidin-1-yl]-2,3-dihydroxypropan-1-one trifluoroacetic acid);

### Compound 327 ((2R)-1-[(2R,4S)-4-[(R)-amino(4,5-dichloro-2-hydroxyphenyl)methyl]-2-methylpiperidin-1-yl]-2,3-dihydroxypropan-1-one trifluoroacetic acid)

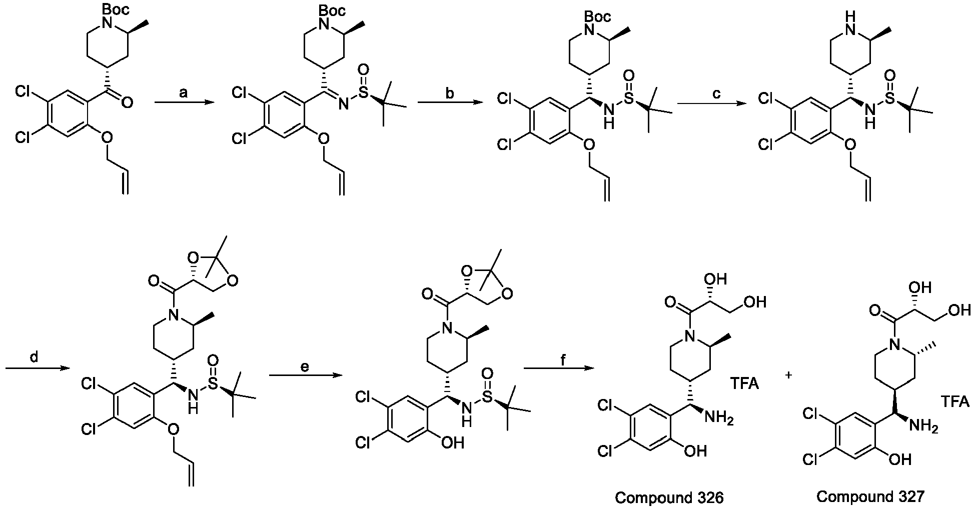

### Step a:

To a stirred solution of *tert*-butyl 4-[4,5-dichloro-2-(prop-2-en-1-yloxy)benzoyl]-2-methylpiperidine-1-carboxylate (Intermediate 43b, Example 43) containing two trans isomers (0.30 g, 0.70 mmol) and tert-butanesulfinamide (0.13 g, 1.05 mmol) in THF (5 mL) was added Ti(OEt)₄ (1.60 g, 7.00 mmol) at room temperature under nitrogen atmosphere. The reaction was stirred at 70 °C for 16 h. After cooling to room temperature, the reaction was quenched with saturated aq. Na₂CO₃ (30 mL). The resulting mixture was filtered through celite, and then the filtrate was diluted with EA (30 mL) and water (30 mL), the aqueous layer was extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (5/1) to afford *tert*-butyl 4-[[4,5-dichloro-2-(prop-2-en-1-yloxy)phenyl]([[(*S*)-2-methylpropane-2-sulfinyl]imino])methyl]-2-methylpiperidine-1-carboxylate containing two trans isomers as a yellow solid (0.30 g, 73%): LCMS (ESI) calc'd for C₂₅H₃₆Cl₂N₂O₄S [M + Na]⁺: 551, 552 (3 : 2), found 551, 553 (3 : 2); ¹H NMR (400 MHz, CDCl₃) δ 7.18 (s, 1H), 6.98 (s, 1H), 6.10-5.89 (m, 1H), 5.45-5.36 (m, 1H), 5.32 (d, *J =* 10.6 Hz, 1H), 4.63-4.40 (m, 3H), 4.16-3.94 (m, 1H), 2.93-2.76 (m, 2H), 1.93-1.80 (m, 1H), 1.79-1.65 (m, 1H), 1.65-1.48 (m, 2H), 1.47 (s, 9H), 1.23 (s, 9H), 1.13 (dd, *J =* 15.2, 6.9 Hz, 3H).

### Step b:

To a stirred solution of *tert*-butyl 4-[[4,5-dichloro-2-(prop-2-en-1-yloxy)phenyl]([[(*S*)-2-methylpropane-2-sulfinyl]imino])methyl]-2-methylpiperidine-1-carboxylate containing two trans isomers (0.20 g, 0.38 mmol) in THF (5 mL) was added DIBAL-H (0.56 mL, 0.564 mmol, 1 M in toluene) at -70 °C under nitrogen atmosphere. The reaction was stirred at -70 °C for 2 h. The reaction was quenched with saturated aq. NH₄Cl (2 mL), and then diluted with EA (30 mL) and water (30 mL). The aqueous layer was extracted with EA (3 x 30 mL). The combined organic layers were washed with brine (2 x 30 mL) and dried over anhydrous Na₂SO₄. After filtration, the filtrate was concentrated under reduced pressure to afford *tert-*butyl 4-[(*R*)-[4,5-dichloro-2-(prop-2-en-1-yloxy)phenyl]([[(*S*)-2-methylpropane-2-sulfinyl]amino])methyl]-2-methylpiperidine-1-carboxylate containing two trans isomers as a yellow oil (0.30 g, crude), which was used in the next step directly without further purification: LCMS (ESI) calc'd for C₂₅H₃₈Cl₂N₂O₄S [M + H]⁺: 533, 535 (3 : 2), found 533, 535 (3 : 2).

### Step c:

To a stirred solution of *tert*-butyl 4-[(*R*)-[4,5-dichloro-2-(prop-2-en-1-yloxy)phenyl]([[(*S*)-2-methylpropane-2-sulfinyl]amino])methyl]-2-methylpiperidine-1-carboxylate containing two trans isomers (0.30 g, 0.56 mmol) in DCM (5 mL) was added TFA (1 mL) at room temperature. The reaction was stirred at room temperature for 0.5 h. The reaction was quenched with saturated aq. Na₂CO₃ (20 mL) and neutralized to pH 7 with saturated aq. Na₂CO₃ (30 mL). The resulting solution was diluted with EA (30 mL) and water (30 mL), and then the aqueous layer was extracted with EA (3 x 30 mL). The combined organic layers were washed with brine (3 x 20 mL), dried over anhydrous Na₂SO₄. After filtration, the filtrate was concentrated under reduced pressure to afford (*S*)-*N*-[(*R*)-[4,5-dichloro-2-(prop-2-en-1-yloxy)phenyl][2-methylpiperidin-4-yl]methyl]-2-methylpropane-2-sulfinamide containing two trans isomers as a yellow oil (0.25 g, crude), which was used in the next step directly without further purification: LCMS (ESI) calc'd for C₂₀H₃₀Cl₂N₂O₂S [M + H]⁺: 433, 435, (3 : 2), found 433, 435 (3 : 2).

### Step d:

To a stirred solution of (4R)-2,2-dimethyl-1,3-dioxolane-4-carboxylic acid (0.13 g, 0.87 mmol) and HATU (0.33 g, 0.87 mmol) in DMF (3 mL) were added (*S*)-*N*-[(*R*)-[4,5-dichloro-2-(prop-2-en-1-yloxy)phenyl][2-methylpiperidin-4-yl]methyl]-2-methylpropane-2-sulfinamide containing two trans isomers (0.25 g, 0.58 mmol) and Et₃N (0.12 g, 1.15 mmol) at room temperature. The reaction was stirred at room temperature for 1 h. The reaction was purified by reverse phase chromatography, eluted with 52% ACN in water (plus 0.05% TFA) to afford (*S*)-*N*-[(*R*)-[4,5-dichloro-2-(prop-2-en-1-yloxy)phenyl][1-[(4*R*)-2,2-dimethyl-1,3-dioxolane-4-carbonyl]-2-methylpiperidin-4-yl]methyl]-2-methylpropane-2-sulfinamide containing two trans isomers as a light yellow oil (0.15 g, 71% overall three steps): LCMS (ESI) calc'd for C₂₆H₃₈Cl₂N₂O₅S [M + H]⁺: 561, 563 (3 : 2), found 561, 563 (3 : 2).

### Step e:

To a stirred solution of (*S*)-*N*-[(*R*)-[4,5-dichloro-2-(prop-2-en-1-yloxy)phenyl][1-[(4*R*)-2,2-dimethyl-1,3-dioxolane-4-carbonyl]-2-methylpiperidin-4-yl]methyl]-2-methylpropane-2-sulfinamide containing two trans isomers (0.15 g, 0.27 mmol) and Pd(PPh₃)₄ (31 mg, 0.03 mmol) in THF (5 mL) was added NaBH₄ (20 mg, 0.53 mmol) at room temperature. The reaction was stirred at room temperature for 30 min. The reaction was quenched with saturated aq. NH₄Cl (1 mL) and concentrated under reduced pressure to afford (*S*)-*N*-[(*R*)-(4,5-dichloro-2-hydroxyphenyl)[1-[(4*R*)-2,2-dimethyl-1,3-dioxolane-4-carbonyl]-2-methylpiperidin-4-yl]methyl]-2-methylpropane-2-sulfinamide containing two trans isomers as a light brown oil (0.10 g, crude), which was used in the next step directly without further purification: LCMS (ESI) calc'd for C₂₃H₃₄Cl₂N₂O₅S [M + H]⁺ 521, 523 (3 : 2), found 521, 523 (3 : 2).

### Step f:

To a stirred solution of (*S*)-*N*-[(*R*)-(4,5-dichloro-2-hydroxyphenyl)[1-[(4*R*)-2,2-dimethyl-1,3-dioxolane-4-carbonyl]-2-methylpiperidin-4-yl]methyl]-2-methylpropane-2-sulfinamide containing two trans isomers (0.10 g, 0.19 mmol) in 1,4-dioxane (2 mL) was added aq. HCl (4 *N,* 1 mL) at room temperature. The reaction was stirred at room temperature for 1 h. The reaction was diluted with EA (20 mL) and HCl (1 *N,* 20 mL). The organic layers were washed with aq. HCl (1 *N,* 2 x 20 mL). The combined aqueous layers were concentrated under reduced pressure. The residue was purified by Prep-HPLC with following conditions: Column: Xselect CSH OBD Column 30 x 150 mm 5 µm; Mobile Phase A: Water (plus 0.05% TFA), Mobile Phase B: ACN; Flow rate: 60 mL/min; Gradient: 8% B to 17% B in 9 min; Detector: UV 254/220 nm; Retention time: RT₁: 8.47 min, RT₂: 9.32 min.

The fractions containing the desired product at 8.47 min were collected and concentrated under reduced pressure to afford Compound 326 ((2*R*)-1-[(2*S*,4*R*)-4-[(*R*)-amino(4,5-dichloro-2-hydroxyphenyl)methyl]-2-methylpiperidin-1-yl]-2,3-dihydroxypropan-1-one trifluoroacetic acid) as an off-white solid (24 mg, 18% overall two steps): LCMS (ESI) calc'd for C₁₆H₂₂Cl₂N₂O₄ [M + H]⁺: 377, 379 (3 :2), found 377, 379 (3 :2); ¹H NMR (400 MHz, CD₃OD) δ 7.43 (d, *J =* 4.0 Hz, 1H), 7.09 (s, 1H), 5.09-4.96 (m, 1H), 4.67-4.26 (m, 2H), 4.16-4.02 (m, 1H), 3.92 (d, *J =* 14.1 Hz, 1H), 3.73-3.52 (m, 2H), 3.25-2.74 (m, 1H), 2.64-2.49 (m, 1H), 1.93 (t, *J =* 12.0 Hz, 1H), 1.64-1.40 (m, 1H), 1.31 (dd, *J =* 46.9, 7.0 Hz, 3H), 1.23-1.03 (m, 1H); ¹⁹F NMR (376 MHz, CD₃OD) δ -77.02.

The fractions containing the desired product at 9.32 min were collected and concentrated under reduced pressure to afford Compound 327 ((2*R*)-1-[(2*R*,4*S*)-4-[(*R*)-amino(4,5-dichloro-2-hydroxyphenyl)methyl]-2-methylpiperidin-1-yl]-2,3-dihydroxypropan-1-one trifluoroacetic acid) as an off-white solid (32 mg, 24% overall two steps): LCMS (ESI) calc'd for C₁₆H₂₂Cl₂N₂O₄ [M + H]⁺ 377, 379 (3 :2), found 377, 379 (3 :2); ¹H NMR (400 MHz, CD₃OD) δ 7.41 (d, *J* = 2.1 Hz, 1H), 7.09 (s, 1H), 4.86-4.77 (m, 1H), 4.59-4.42 (m, 2H), 4.09 (dd, *J =* 14.9, 9.8 Hz, 2H), 3.78-3.55 (m, 2H), 3.30-2.83 (m, 1H), 2.78-2.53 (m, 1H), 2.07-1.85 (m, 1H), 1.50-1.29 (m, 1H), 1.29-1.04 (m, 4H), ¹⁹F NMR (376 MHz, CD₃OD) δ -76.96.

### Example 246. Compound 328 ((2R)-1-[4-[amino(3,4-dichloro-2-fluoro-6-hydroxyphenyl)methyl]piperidin-1-yl]-2,3-dihydroxypropan-1-one trifluoroacetic acid)

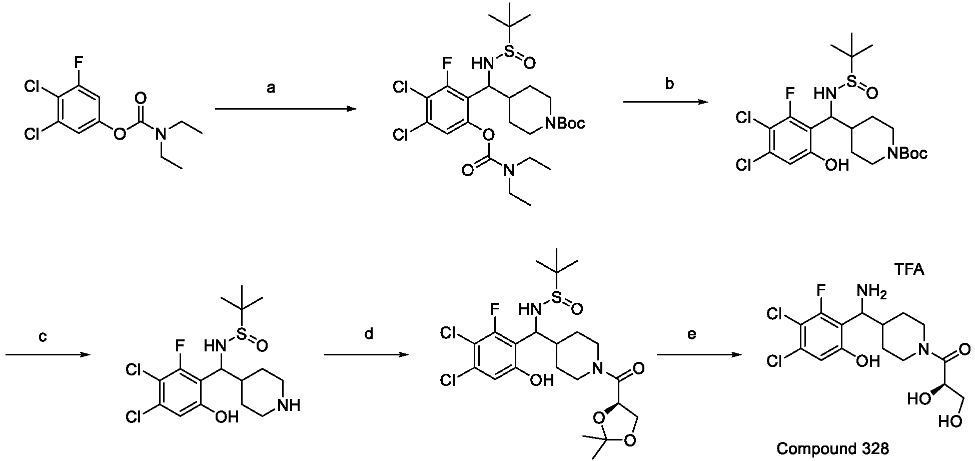

### Step a:

To a solution of diisopropylamine (2.71 g, 2.67 mmol) in THF (10 mL) was added *n-*BuLi (1.28 mL, 3.213 mmol, 2.5 mol/L in hexane) over 10 min at -65 °C under nitrogen atmosphere. After addition, the reaction solution was stirred for 30 min, and then a solution of 3,4-dichloro-5-fluorophenyl *N,N*-diethylcarbamate (Example 18, step c) (0.50 g, 1.78 mmol) in THF (3) was added dropwise at -65 °C under nitrogen atmosphere. After stirring for additional 35 min, to the resulting mixture was added a solution of *tert*-butyl 4-[[(2-methylpropane-2-sulfinyl)imino]methyl]piperidine-1-carboxylate (Intermediate 11, Example 11) (0.85 g, 2.67 mmol) in THF (3 mL) dropwise over 10 min at -65 °C. The reaction mixture was stirred for additional 1 h at -65 °C under nitrogen atmosphere. The resulting mixture was quenched with water (30 mL) at -65 °C and extracted with EA (2 x 70 mL). The combined organic layers were washed with brine (2 x 10 mL), dried over anhydrous Na₂SO₄. After filtration, the filtrate was concentrated under reduced pressure. The residue was purified by silica gel column chromatography, eluted with PE/EA (5/1) to afford *tert*-butyl 4-([3,4-dichloro-6-[(diethylcarbamoyl)oxy]-2-fluorophenyl][(2-methylpropane-2-sulfinyl)amino]methyl)piperidine-1-carboxylate as a yellow oil (0.57 g, 48%): LCMS (ESI) calc'd for C₂₆H₄₀Cl₂FN₃O₅S [M + H]⁺: 596, 598 (3 : 2), found 596, 598 (3 : 2).

### Step b:

To a solution of *tert*-butyl 4-([3,4-dichloro-6-[(diethylcarbamoyl)oxy]-2-fluorophenyl][(2-methylpropane-2-sulfinyl)amino]methyl)piperidine-1-carboxylate (0.57 g, 0.96 mmol) in MeOH (5 mL) was added NaOH (0.38 g, 9.58 mmol) in portions at room temperature. The resulting mixture was stirred for 1 h at room temperature. The reaction mixture was diluted with water (20 mL) at room temperature and acidified to pH 4 with citric acid. The resulting solution was extracted with EA (2 x 100 mL). The combined organic layers were washed with brine (2 x 10 mL), dried over anhydrous Na₂SO₄. After filtration, the filtrate was concentrated under reduced pressure to afford *tert*-butyl 4-[(3,4-dichloro-2-fluoro-6-hydroxyphenyl)[(2-methylpropane-2-sulfinyl)amino]methyl]piperidine-1-carboxylate (0.56 g, 1.13 mmol). The crude product was used in the next step directly without further purification: LCMS (ESI) calc'd for C₂ₗH₃ₗCl₂FN₂O₄S [M + H]⁺: 497, 499 (3 : 2), found 497, 499 (3 : 2).

### Step c:

To a solution of *tert*-butyl 4-[(3,4-dichloro-2-fluoro-6-hydroxyphenyl)[(2-methylpropane-2-sulfinyl)amino]methyl]piperidine-1-carboxylate (crude) in DCM (10 mL) was added TFA (2 mL) dropwise at room temperature. The reaction solution was stirred for 1 h at room temperature. The resulting solution was adjusted to pH 9 with saturated aq. NaHCO₃ and extracted with EA (2 x 200 mL). The combined organic layers were washed with brine (2 x 10 mL), dried over anhydrous Na₂SO₄. After filtration, the filtrate was concentrated under reduced pressure to afford *N*-[(3,4-dichloro-2-fluoro-6-hydroxyphenyl)(piperidin-4-yl)methyl]-2-methylpropane-2-sulfinamide (0.56 g, 1.41 mmol). The crude product was used in the next step directly without further purification: LCMS (ESI) calc'd for C₁₆H₂₃Cl₂FN₂O₂S [M + H]⁺: 397, 399 (3 : 2), found 397, 399 (3 : 2).

### Step d:

To a stirred solution of (*R*)-2,2-dimethyl-1,3-dioxolane-4-carboxylic acid (0.30, 2.11 mmol) and HATU (1.07 g, 2.82 mmol) in DMF (5 mL) were added Et₃N (0.43 g, 4.23 mmol) and *N*-[(3,4-dichloro-2-fluoro-6-hydroxyphenyl)(piperidin-4-yl)methyl]-2-methylpropane-2-sulfinamide (0.56 g, 1.41 mmol) at room temperature. After stirring for 2 h at room temperature, the reaction solution was quenched with water (30 mL) at room temperature and extracted with EA (3 x 100 mL). The combined organic layers were washed with brine (2 x 20 mL), dried over anhydrous Na₂SO₄. After filtration, the filtrate was concentrated under reduced pressure. The residue was purified by reverse phase chromatography, eluted with 70% ACN in water to afford *N*-[(3,4-dichloro-2-fluoro-6-hydroxyphenyl)[1-(2,2-dimethyl-1,3-dioxolane-4-carbonyl)piperidin-4-yl]methyl]-2-methylpropane-2-sulfinamide as a yellow oil (0.28 g, 42% overall three steps): LCMS (ESI) calc'd for C₂₂H₃₁Cl₂FN₂O₅S [M + H]⁺: 525, 527 (3 : 2), found 525, 527 (3 : 2); ¹H NMR (400 MHz, CD₃OD) δ 6.85 (s, 1H), 4.63-4.41 (m, 2H), 4.20-4.00 (m,2H), 3.75-3.55 (m, 2H), 3.20-2.94 (m, 1H), 2.79-2.50 (m, 1H), 2.43-2.25 (m, 1H), 2.08-1.96 (m, 1H), 1.64-1.28 (m, 3H), 1.31-1.19 (m, 6H), 1.12 (s, 9H).

### Step e:

To a stirred mixture of *N*-[(3,4-dichloro-2-fluoro-6-hydroxyphenyl)[1-(2,2-dimethyl-1,3-dioxolane-4-carbonyl)piperidin-4-yl]methyl]-2-methylpropane-2-sulfinamide (0.28 g, 0.53 mmol) in THF (4 mL) was added aq. HCl (6 *N,* 2 mL) at room temperature. The reaction solution was stirred for 1 h at room temperature. The resulting solution was concentrated under reduced pressure. The residue was purified by Prep-HPLC with the following conditions: Column: Xselect CSH OBD Column 30 x 150 mm, 5 µm; Mobile Phase A: water (plus 0.05% TFA), Mobile Phase B: ACN; Flow rate: 60 mL/min; Gradient: 7% B to 22% B in 7 min; Detector: UV 220 nm; Retention time: 6.25 min. The fractions containing desired product were collected and concentrated under reduced pressure to afford Compound 328 ((2*R*)-1-[4-[amino(3,4-dichloro-2-fluoro-6-hydroxyphenyl)methyl]piperidin-1-yl]-2,3-dihydroxypropan-1-one trifluoroacetic acid) as an off-white solid (106 mg, 52%): LCMS (ESI) calc'd for C₁₅H₁₉Cl₂FN₂O₄ [M + H]⁺: 381, 383 (3 : 2), found 381, 383 (3 : 2); ¹H NMR (400 MHz, CD₃OD) δ 6.98 (d, *J =* 1.8 Hz, 1H), 4.72-4.34 (m, 3H), 4.21 (dd, *J =* 58.5, 13.9 Hz, 1H), 3.80-3.56 (m, 2H), 3.23-2.95 (m, 1H), 2.79-2.58 (m, 1H), 2.54-2.36 (m, 1H), 2.04-1.95 (m, 1H) 1.57-1.06 (m, 3H); ¹⁹F NMR (376 MHz, CD₃OD) δ -76.85, 113.17.

### Example 247. Compound 329 ((2R)-1-[(3R)-3-[(R)-amino(4,5-dichloro-2-hydroxyphenyl)methyl]piperidin-1-yl]-2,3-dihydroxypropan-1-one trifluoroacetic acid);

### Compound 330 ((2R)-1-[(3R)-3-[(S)-amino(4,5-dichloro-2-hydroxyphenyl)methyl]piperidin-1-yl]-2,3-dihydroxypropan-1-one trifluoroacetic acid)

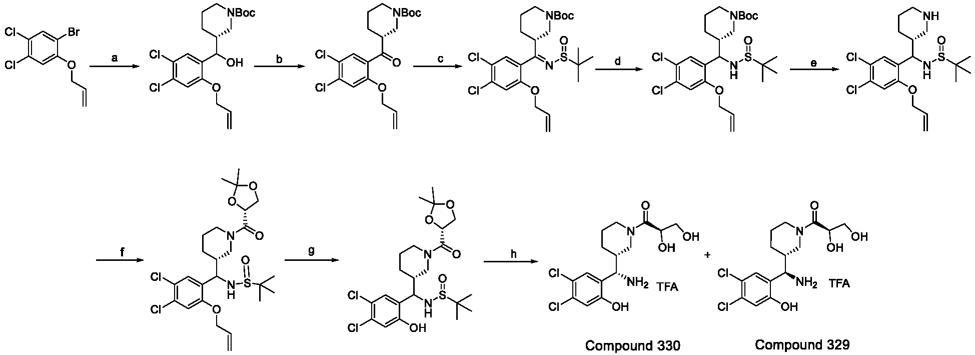

### Step a:

To a stirred solution of 1-bromo-4,5-dichloro-2-(prop-2-en-1-yloxy)benzene (Example 6, step b) (0.52 g, 1.83 mmol) in THF (5 mL) was added i-PrMgCl (1 mL, 2.00 mmol, 2 M in THF) dropwise at 0 °C under nitrogen atmosphere. After stirring for 30 min at 0 °C, a solution *tert*-butyl (3*R*)-3-formylpiperidine-1-carboxylate (0.30 g, 1.41 mmol) in THF (3 mL) was added dropwise at 0 °C to resulting mixture under nitrogen atmosphere. The reaction mixture was allowed to warm to room temperature and stirred for 2 h. The reaction was quenched with saturated aq. NH₄Cl (40 mL). The resulting mixture was extracted with EA (2 x 10 mL). The combined organic layers were washed with brine (2 x 20 mL), dried over anhydrous Na₂SO₄, filtered and concentrated under reduced pressure. The residue was purified by silica gel column chromatography, eluted with EA to afford *tert*-butyl (3R)-3-[[4,5-dichloro-2-(prop-2-en-1-yloxy)phenyl](hydroxy)methyl]piperidine-1-carboxylate as a colorless oil (0.40 g, 68%): LCMS (ESI) calc'd for C₂₀H₂₇Cl₂NO₄ [M + H]⁺: 416, 418 (3 : 2), found 416, 418 (3 : 2).

### Step b:

To a solution of tert-butyl (3R)-3-[[4,5-dichloro-2-(prop-2-en-1-yloxy)phenyl](hydroxy)methyl]piperidine-1-carboxylate (0.40 g, 0.96 mmol) in DCM (10 mL) was added Dess-Martin reagent (0.49 g, 1.15 mmol) at 0 °C. The reaction mixture was stirred at room temperature for 2 h. The resulting mixture were washed with co-solvent aq. NaHCO₃/aq. Na₂SO₃ (20 mL, v/v = 1/1). The combined organic layers were washed with brine (20 mL), dried over anhydrous Na₂SO₄. After filtration, the filtrate was concentrated under reduced pressure. The residue was purified with silica gel column chromatography, eluted with EA/PE (1/1) to afford *tert*-butyl (3*R*)-3-[4,5-dichloro-2-(prop-2-en-1-yloxy)benzoyl]piperidine-1-carboxylate as a light yellow oil (0.36 g, 90%): LCMS (ESI) calc'd for C₂₀H₂₅Cl₂NO₄ [M + H]⁺: 414, 416 (3 : 2), found 414, 416 (3 : 2); ¹H NMR (400 MHz, CDCl₃) δ 7.69 (s, 1H), 7.07 (s, 1H), 6.11-6.01 (m, 1H), 5.51-5.33 (m, 2H), 4.73-4.57 (m, 2H), 4.34-3.85 (m, 2H), 3.47-3.32 (m, 1H), 2.88-2.71 (m, 2H), 2.04-1.92 (m, 2H), 1.76-1.71 (m, 2H), 1.48 (s, 9H).

### Step c:

To a stirred solution of *tert*-butyl (3*R*)-3-[4,5-dichloro-2-(prop-2-en-1-yloxy)benzoyl]piperidine-1-carboxylate (0.36 g, 0.87 mmol) and 2-methylpropane-2-sulfinamide (0.16 g, 1.30 mmol) in THF (20 mL) was added Ti(OEt)₄ (1.98 g, 8.69 mmol) in one portion under nitrogen atmosphere. The reaction solution was stirred for 48 h at 80 °C under nitrogen atmosphere. After cooling to room temperature, the reaction mixture was poured into saturated aq. NaHCO₃ (100 mL), solid was separated out, then the mixture was filtered, the filtrate was extracted with EA (2 x 50 mL). The combined organic layers were washed with brine (20 mL), dried over anhydrous Na₂SO₄. After filtration, the filtrate was concentrated under reduced pressure to afford *tert*-butyl (3*R*)-3-[4,5-dichloro-2-(prop-2-en-1-yloxy)phenyl][(2-methylpropane-2-sulfinyl)imino]methyl]piperidine-1-carboxylate as a light yellow oil (0.40 g, crude): LCMS (ESI) calc'd for C₂₄H₃₄Cl₂N₂O₄S [M + H]⁺: 517, 519 (3 : 2), found 517, 519 (3 : 2).

### Step d:

To a stirred solution of *tert*-butyl (3*R*)-3-[4,5-dichloro-2-(prop-2-en-1-yloxy)phenyl][(2-methylpropane-2-sulfinyl)imino]methyl]piperidine-1-carboxylate (0.40 g, 0.77 mmol) in MeOH (10 mL) was added NaBH₄ (59 mg, 1.55 mmol) in portions at 0 °C. The resulting mixture was stirred for 2 h at room temperature. The reaction was quenched with saturated aq. NH₄Cl (50 mL) and extracted with EA (3 x 30 mL). The combined organic layers were combined, dried over anhydrous Na₂SO₄. After filtration, the filtrate was concentrated under reduced pressure to afford *tert*-butyl (3*R*)-3-[[4,5-dichloro-2-(prop-2-en-1-yloxy)phenyl][(2-methylpropane-2-sulfinyl)amino]methyl]piperidine-1-carboxylate as a light yellow oil (0.40 g, crude): LCMS (ESI) calc'd for C₂₄H₃₆Cl₂N₂O₄S [M + H]⁺: 519, 521 (3 : 2), found 519, 521 (3 : 2).

### Step e:

To a stirred solution of *tert*-butyl (3*R*)-3-[[4,5-dichloro-2-(prop-2-en-1-yloxy)phenyl][(2-methylpropane-2-sulfinyl)amino]methyl]piperidine-1-carboxylate (0.40 g, 0.77 mmol) in DCM (4 mL) was added TFA (1 mL) at room temperature. The reaction solution was stirred for 1 h at room temperature. The resulting solution was basified with saturated aq. NaHCO₃ to pH 8. The resulting mixture was extracted with DCM (3 x 20 mL). The combined organic layers were washed with brine (2 x 10 mL), dried over anhydrous Na₂SO₄. After filtration, the filtrate was concentrated under reduced pressure to afford *N*-[[4,5-dichloro-2-(prop-2-en-1-yloxy)phenyl][(3*R*)-piperidin-3-yl]methyl]-2-methylpropane-2-sulfinamide as a light yellow oil (260 mg, crude): LCMS (ESI) calc'd for C₁₉H₂₈Cl₂N₂O₂S [M + H]⁺: 419, 421 (3 : 2), found 419, 421 (3 : 2).

### Step f:

To a stirred solution of HATU (0.35 g, 0.93 mmol) and (4*R*)-2,2-dimethyl-1,3-dioxolane-4-carboxylic acid (0.14 g, 0.93 mmol) in DMF (4 mL) were added Et₃N (0.13 g, 1.24 mmol) and *N*-[[4,5-dichloro-2-(prop-2-en-1-yloxy)phenyl][(3*R*)-piperidin-3-yl]methyl]-2-methylpropane-2-sulfinamide (0.26 g, 0.62 mmol) at room temperature. After stirring for 2 h at room temperature, the resulting solution was quenched with water (30 mL) and extracted with EA (3 x 20 mL). The combined organic layers were washed with brine (3 x 15 mL), dried over anhydrous Na₂SO₄. After filtration, the filtrate was concentrated under reduced pressure to afford *N*-[[4,5-dichloro-2-(prop-2-en-1-yloxy)phenyl][(3*R*)-1-[(4*R*)-2,2-dimethyl-1,3-dioxolane-4-carbonyl]piperidin-3-yl]methyl]-2-methylpropane-2-sulfinamide as a light yellow oil (0.35 g, crude): LCMS (ESI) calc'd for C₂₅H₃₆Cl₂N₂O₅ [M + H]⁺: 547, 549 (3 : 2), found 547, 549 (3 : 2).

### Step g:

To a stirred solution of *N*-[[4,5-dichloro-2-(prop-2-en-1-yloxy)phenyl][(3*R*)-1-[(4*R*)-2,2-dimethyl-1,3-dioxolane-4-carbonyl]piperidin-3-yl]methyl]-2-methylpropane-2-sulfinamide (0.35 g, 0.64 mmol) and Pd(PPh₃)₄ (15 mg, 0.01 mmol) in THF (5 mL) was added NaBH₄ (48 mg, 1.28 mmol) in portions at room temperature under nitrogen atmosphere. The reaction mixture was stirred for 2 h at room temperature. The resulting mixture was quenched with saturated aq. NH₄Cl (5 mL) at 0 °C and extracted with EA (3 x 20 mL). The combined organic layers were washed with brine (2 x 10 mL), dried over anhydrous Na₂SO₄. After filtration, the filtrate was concentrated under reduced pressure to afford *N*-[(4,5-dichloro-2-hydroxyphenyl)[(3*R*)-1-[(4*R*)-2,2-dimethyl-1,3-dioxolane-4-carbonyl]piperidin-3-yl]methyl]-2-methylpropane-2-sulfinamide as a light yellow oil (0.40 g, crude): LCMS (ESI) calc'd for C₂₂H₃₂Cl₂N₂O₅ [M + H]⁺: 507, 509 (3 : 2), found 507, 509 (3 : 2).

### Step h:

To a stirred solution of *N*-[(4,5-dichloro-2-hydroxyphenyl)[(3*R*)-1-[(4*R*)-2,2-dimethyl-1,3-dioxolane-4-carbonyl]piperidin-3-yl]methyl]-2-methylpropane-2-sulfinamide (0.40 g, crude) in THF (10 mL) was added aq. HCl (4 *N,* 5 mL) dropwise at room temperature. The resulting mixture was stirred for 2 h at room temperature. The reaction mixture was concentrated under reduced pressure. The residue was purified with Prep-HPLC with the following conditions: Column: Xselect CSH OBD Column 30 x 150 mm, 5 µm; Mobile Phase A: water (plus 0.05% TFA), Mobile Phase B: ACN; Flow rate: 60 mL/min; Gradient: 5% B to 20% B in 7 min; Detector: UV 220 nm; Retention time: RT₁: 6.10 min; RT₂: 6.72 min.

The faster-eluting enantiomer was obtained as compound 330 ((2*R*)-1-[(3*R*)-3-[(*S*)-amino(4,5-dichloro-2-hydroxyphenyl)methyl]piperidin-1-yl]-2,3-dihydroxypropan-1-one trifluoroacetic acid) as an off-white solid (23 mg, 7% overall 6 steps): LCMS (ESI) calc'd for C₁₅H₂₀Cl₂N₂O₄ [M + H]⁺: 363, 365 (3 : 2), found 363, 365 (3 : 2); ¹H NMR (400 MHz, CD₃OD) δ 7.46 (d, *J* = 14.1 Hz, 1H), 7.11 (d, *J =* 12.6 Hz, 1H), 4.57-4.41 (m, 1H), 4.23 (d, *J =* 9.9 Hz, 1H), 4.17-3.73 (m, 2H), 3.64 (qt, *J =* 18.4, 8.7 Hz, 2H), 2.96 (dt, *J =* 117.9, 12.3 Hz, 1H), 2.57 (dt, *J =* 56.1, 12.1 Hz, 1H), 2.42-2.07 (m, 2H), 1.99-1.83 (m, 1H), 1.70-140 (m, 2H); ¹⁹F NMR (376 MHz, CD₃OD) δ -76.99.

The slower-eluting enantiomer was obtained Compound 329 ((2*R*)-1-[(3*R*)-3-[(*R*)-amino(4,5-dichloro-2-hydroxyphenyl)methyl]piperidin-1-yl]-2,3-dihydroxypropan-1-one trifluoroacetic acid) as an off-white solid (48.9 mg, 16% overall 6 steps): LCMS (ESI) calc'd for C₁₅H₂₀Cl₂N₂O₄ [M + H]⁺: 363, 365 (3 : 2), found 363, 365 (3 : 2); ¹H NMR (400 MHz, CD₃OD) δ 7.44 (d, *J* = 8.7 Hz, 1H), 7.09 (s, 1H), 4.62-4.58 (m, 1H), 4.43-4.19 (m, 1H), 4.11 (d, *J =* 13.7 Hz, 1H), 3.83-3.65 (m, 3H), 3.63-3.44 (m, 1H), 3.43-3.35 (m, 1H), 2.48-2.30 (m, 1H), 1.88-1.69 (m, 1H), 1.67-1.45 (m, 2H), 1.39-1.19 (m, 1H); ¹⁹F NMR (376 MHz, CD₃OD) δ -77.03.

### Example 248. Compound 331 ((2R)-1-((3R,4R)-rel-4-((R)-amino(4,5-dichloro-2-hydroxyphenyl)methyl)-3-methylpiperidin-1-yl)-2,3-dihydroxypropan-1-one trifluoroacetic acid isomer 1);

### Compound 332 ((2R)-1-((3R,4R)-rel-4-((R)-amino(4,5-dichloro-2-hydroxyphenyl)methyl)-3-methylpiperidin-1-yl)-2,3-dihydroxypropan-1-one trifluoroacetic acid isomer 2)

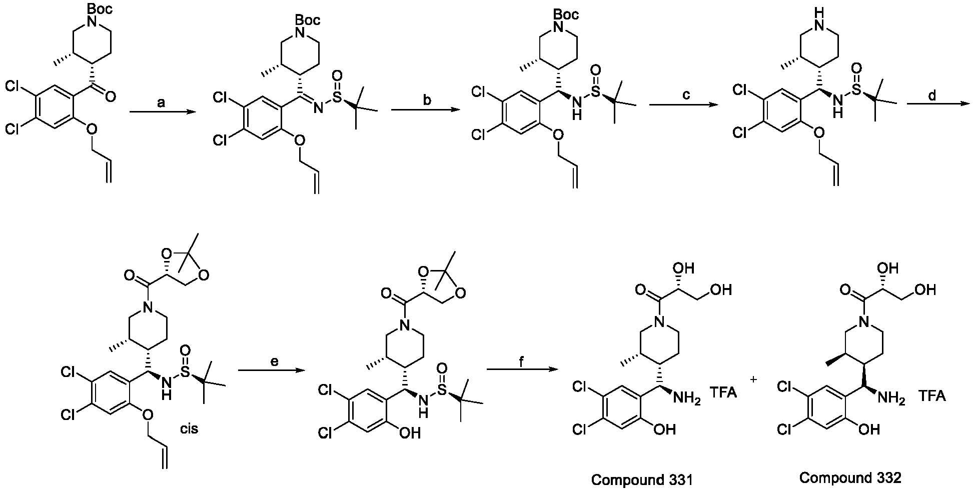

### Step a:

To a stirred solution of *tert*-butyl 4-[4,5-dichloro-2-(prop-2-en-1-yloxy)benzoyl]-3-methylpiperidine-1-carboxylate (*cis* isomer) (0.38 g, 0.88 mmol) and (*S*)-2-methylpropane-2-sulfinamide (0.21 g, 1.77 mmol) in THF (5 mL) were added Ti(OEt)₄ (2.00 g, 8.87 mmol) at room temperature under nitrogen atmosphere. The resulting mixture was stirred for 16 h at 70 °C under nitrogen atmosphere. After cooling to room temperature, the resulting mixture was quenched with saturated aq. Na₂CO₃ (30 mL). The solid was formed and filtered, the filter cake were washed with EA (3 x 100 mL). The filtrate was concentrated under reduced pressure. The residue was purified by silica gel column chromatography, eluted with PE/EA (3/1) to afford *tert*-butyl 4-[[4,5-dichloro-2-(prop-2-en-1-yloxy)phenyl]([[(*S*)-2-methylpropane-2-sulfinyl]imino])methyl]-3-methylpiperidine-1-carboxylate as a yellow oil (0.24 g, 50%): LCMS (ESI) calc'd for C₂₅H₃₆Cl₂N₂O₄S [M + Na]⁺: 553, 555 (3 : 2), found 553, 555 (3 : 2); ¹H NMR (400 MHz, CDCl₃) δ 7.03-6.91 (m, 2H), 6.11-5.88 (m, 1H), 5.35-5.26 (m, 2H), 4.67-4.53 (m, 2H), 4.40-4.18 (m, 1H), 4.07-3.88 (m, 1H), 3.01-2.64 (m, 2H), 2.54-2.25 (m, 1H), 2.03-1.85 (m, 1H), 1.68-1.54 (m, 2H), 1.51-1.45 (m, 9H), 1.26-1.18 (m, 9H), 1.02 (d, *J =* 7.0 Hz, 3H).

### Step b:

To a stirred solution of *tert*-butyl 4-[[4,5-dichloro-2-(prop-2-en-1-yloxy)phenyl]([[(*S*)-2-methylpropane-2-sulfinyl]imino])methyl]-3-methylpiperidine-1-carboxylate (0.24 g, 0.45 mmol) in THF (3 mL) was added DIBAL-H (0.11 mL, 0.80 mmol, 1 M solution in toluene) dropwise at -65 °C under nitrogen atmosphere. The resulting mixture was stirred for 3 h at -65 °C under nitrogen atmosphere. The reaction was quenched with saturated aq. NH₄Cl (0.5 mL) at room temperature and diluted with water (50 mL). The aqueous layer was extracted with EA (3 x 30 mL). The combined organic layers were washed with brine (3 x 20 mL) and dried over anhydrous Na₂SO₄. After filtration, the filtrate was concentrated under reduced pressure to afford *tert*-butyl 4-[(*R*)-[4,5-dichloro-2-(prop-2-en-1-yloxy)phenyl]([[(*S*)-2-methylpropane-2-sulfinyl]amino])methyl]-3-methylpiperidine-1-carboxylate as a yellow oil (0.25 g, crude), which was used in the next step directly without further purification: LCMS (ESI) calc'd for C₂₅H₃₈Cl₂N₂O₄S [M + H]⁺: 533, 535 (3 : 2), found 533, 535 (3 : 2); ¹H NMR (400 MHz, CDCl₃) δ 7.01-6.81 (m, 2H), 6.19-5.90 (m, 1H), 5.40-5.31 (m, 2H), 4.82-4.48 (m, 3H), 4.39-4.21 (m, 1H), 4.06-3.77 (m, 1H), 2.90-2.56 (m, 2H), 2.56-2.40 (m, 1H), 1.83-1.64 (m, 1H), 1.63-1.53 (m, 2H), 1.51-1.43 (m, 9H), 1.09 (s, 9H), 0.83 (d, *J =* 6.9 Hz, 3H).

### Step c:

To a stirred mixture of *tert*-butyl 4-[(*R*)-[4,5-dichloro-2-(prop-2-en-1-yloxy)phenyl]([[(*S*)-2-methylpropane-2-sulfinyl]amino])methyl]-3-methylpiperidine-1-carboxylate (0.25 g, 0.47 mmol) in DCM (5 mL) was added TFA (1 mL) at room temperature. The resulting mixture was stirred for 30 min at room temperature. The reaction solution was neutralized to pH 8 with saturate aq. NaHCO₃ at 0 °C and diluted water (50 mL). The aqueous layer was extracted with EA (3 x 30 mL). The combined organic layers were washed with brine (3 x 20 mL) and dried over anhydrous Na₂SO₄. After filtration, the filtrate was concentrated under reduced pressure to afford (*S*)-*N*-[(*R*)-[4,5-dichloro-2-(prop-2-en-1-yloxy)phenyl](3-methylpiperidin-4-yl)methyl]-2-methylpropane-2-sulfinamide as a yellow oil (0.2 g, crude), which was used in the next step directly without further purification: LCMS (ESI) calc'd for C₂₀H₃₀Cl₂N₂O₂S [M + H]⁺: 433, 435 (3 : 2), found 433, 435 (3 : 2); ¹H NMR (400 MHz, CDCl₃) δ 7.09-6.90 (m, 2H), 6.08-5.95 (m, 1H), 5.52-5.28 (m, 2H), 4.65-4.49 (m, 3H), 3.99-3.81 (m, 1H), 3.43-3.21 (m, 2H), 3.21-2.95 (m, 1H), 2.95-2.61 (m, 1H), 2.55-2.15 (m, 1H), 2.00-1.71 (m, 2H), 1.14-1.01 (m, 12H).

### Step d:

To a stirred solution of (*4R*)-2,2-dimethyl-1,3-dioxolane-4-carboxylic acid (0.10 g, 0.69 mmol) and HATU (0.26 g, 0.69 mmol) in DMF (3 mL) were added (*S*)-*N-*[(*R*)-[4,5-dichloro-2-(prop-2-en-1-yloxy)phenyl](3-methylpiperidin-4-yl)methyl]-2-methylpropane-2-sulfinamide (0.20 g, 0.46 mmol) and Et₃N (93 mg, 0.92 mmol) at room temperature. The resulting mixture was stirred for 0.5 h at room temperature. The reaction was purified by reverse phase chromatography, eluted with 40% ACN in water (plus 0.05% TFA) to afford (*S*)-*N*-[(*R*)-[4,5-dichloro-2-(prop-2-en-1-yloxy)phenyl]([1-[(*4R*)-2,2-dimethyl-1,3-dioxolane-4-carbonyl]-3-methylpiperidin-4-yl])methyl]-2-methylpropane-2-sulfinamide as a yellow oil (0.17 g, 67% overall three steps): LCMS (ESI) calc'd for C₂₆H₃₈Cl₂N₂O₅S [M + H]⁺: 561, 563(3 : 2), found 561, 563(3 : 2); ¹H NMR (400 MHz, CDCl₃) δ 7.28-7.14 (m, 1H), 7.06-6.91 (m, 1H), 6.15-5.92 (m, 1H), 5.52-5.30 (m, 2H), 4.81-4.48 (m, 3H), 4.45-4.25 (m, 2H), 4.22-4.02 (m, 2H), 4.02-3.65 (m, 1H), 3.31-2.88 (m, 2H), 2.85-2.60 (m, 2H), 1.69-1.48 (m, 2H), 1.47-1.34 (m, 6H), 1.10 (s, 9H), 0.93-0.75 (m, 3H).

### Step e:

To a stirred solution of (*S*)-*N*-[(*R*)-[4,5-dichloro-2-(prop-2-en-1-yloxy)phenyl]([1-[(*4R*)-2,2-dimethyl-1,3-dioxolane-4-carbonyl]-3-methylpiperidin-4-yl])methyl]-2-methylpropane-2-sulfinamide (0.17 g, 0.30 mmol) and Pd(PPh₃)₄ (4 mg, 0.003 mmol) in THF (2 mL) was added NaBH₄ (22 mg, 0.60 mmol) at room temperature. The resulting mixture was stirred for 30 min at room temperature. The reaction was quenched with saturated aq. NH₄Cl (1 mL) at room temperature. The resulting mixture was concentrated under reduced pressure to afford (*S*)-*N*-[(*R*)-(4,5-dichloro-2-hydroxyphenyl)([1-[(4*R*)-2,2-dimethyl-1,3-dioxolane-4-carbonyl]-3-methylpiperidin-4-yl])methyl]-2-methylpropane-2-sulfinamide (0.10 g, 63%), which was used in the next step directly without further purification: LCMS (ESI) calc'd for C₂₃H₃₄Cl₂N₂O₅S [M + H]⁺: 521, 523 (3 : 2), found 521, 523 (3 : 2).

### Step f:

To a stirred mixture of (*S*)-*N*-[(*R*)-(4,5-dichloro-2-hydroxyphenyl)([1-[(4*R*)-2,2-dimethyl-1,3-dioxolane-4-carbonyl]-3-methylpiperidin-4-yl])methyl]-2-methylpropane-2-sulfinamide (0.10 g, 0.19 mmol) in 1,4-dioxane (1 mL) was added aq. HCl (4 *N,* 1 mL) at room temperature. The resulting solution was stirred for 30 min at room temperature. The resulting mixture was concentrated under reduced pressure. The residue was purified by Prep-HPLC with the following conditions: Column: Xselect CSH OBD Column 30 x 150 mm 5 µm n; Mobile Phase A: water (plus 0.05% TFA), Mobile Phase B: ACN; Flow rate: 60 mL/min; Gradient: 5% B to 20% B in 8.5 min; 220 nm; Detector: UV 254/220 nm; Retention time: RT₁: 7.90 min, RT₂: 8.60 min.

The fractions containing the desired product at 7.90 min were collected and concentrated under reduced pressure to afford Compound 331 ((2*R*)-1-((3*S*,4*S*)-4-((*R*)-amino(4,5-dichloro-2-hydroxyphenyl)methyl)-3-methylpiperidin-1-yl)-2,3-dihydroxypropan-1-one trifluoroacetic acid trifluoroacetic acid) as an off-white solid (37 mg, 25% overall two steps): LCMS (ESI) calc'd for C₁₆H₂₂Cl₂N₂O₄ [M + H]⁺ 377, 379 (3 : 2), found 377, 379 (3 : 2); ¹H NMR (400 MHz, CD₃OD) δ 7.46 (s, 1H), 7.08 (s, 1H), 4.65-4.38 (m, 2H), 4.38-3.98 (m, 2H), 3.80-3.51 (m, 2H), 2.96 (t, *J =* 13.0 Hz, 1H), 2.83 (d, *J =* 13.0 Hz, 1H), 2.61-2.39 (m, 1H), 2.29-2.08 (m, 1H), 1.62-1.20 (m, 1H), 1.20-0.85 (m, 4H); ¹⁹F NMR (376 MHz, CD₃OD) δ -77.09.

The fractions containing the desired product at 8.60 min were collected and concentrated under reduced pressure to afford Compound 332 ((2*R*)-1-((3*R*,4*R*)-4-((*R*)-amino(4,5-dichloro-2-hydroxyphenyl)methyl)-3-methylpiperidin-1-yl)-2,3-dihydroxypropan-1-one trifluoroacetic acid) as an off-white solid (31 mg, 21% overall two steps): LCMS (ESI) calc'd for C₁₆H₂₂Cl₂N₂O₄ [[M + H]⁺: 377, 379 (3 : 2), found 377, 379 (3 : 2); ¹H NMR (400 MHz, CD₃OD) δ 7.54 (d, *J =* 5.6 Hz, 1H), 7.11 (s, 1H), 4.78-4.51 (m, 2H), 4.48-4.14 (m, 2H), 3.92-3.50 (m, 3H), 3.25-3.05 (m, 1H), 2.81-2.51 (m, 2H), 1.87-1.68 (m, 1H), 1.68-1.46 (m, 1H), 0.99-0.76 (m, 3H); ¹⁹F NMR (376 MHz, CD₃OD) δ -77.04.

### Example 249. Compound 333 ((2R)-1-[4-[(R)-amino(4,5-dichloro-2-hydroxyphenyl)methyl]azepan-1-yl]-2,3-dihydroxypropan-1-one trifluoroacetic acid isomer 1);

### Compound 334 ((2R)-1-[4-[(R)-amino(4,5-dichloro-2-hydroxyphenyl)methyl]azepan-1-yl]-2,3-dihydroxypropan-1-one trifluoroacetic acid isomer 2

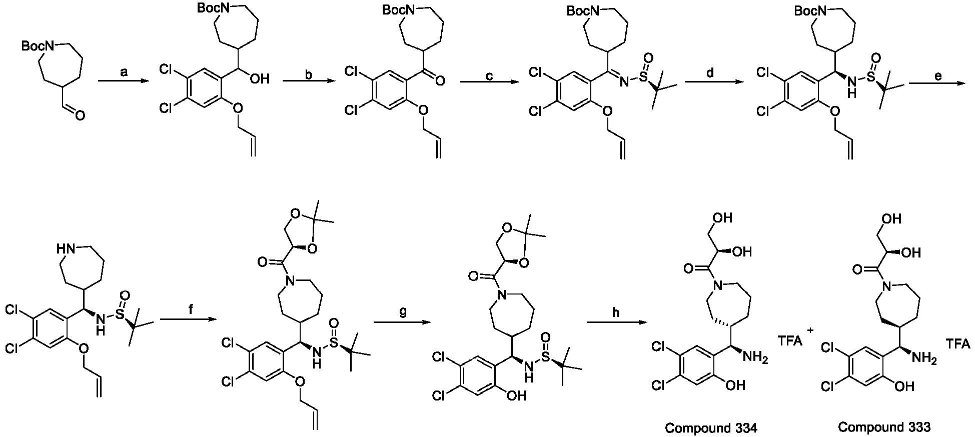

### Step a:

To a stirred solution of 4-bromo-1,2-dichloro-5-(prop-2-en-1-yloxy)cyclohexa-1,3-diene (2.50 g, 8.80 mmol) in THF (25 mL) was added i-PrMgCl (5.5 mL, 11.00 mmol, 2 M solution in THF) dropwise at 0 °C under nitrogen atmosphere. The resulting solution was stirred for 30 min at 0 °C under nitrogen atmosphere. To the above mixture was added *tert*-butyl 4-formylazepane-1-carboxylate (1.00 g, 4.40 mmol) in THF (2 mL) dropwise over 5 min at 0 °C. The resulting solution was stirred for additional 1 h at 0 °C. The reaction was quenched with saturated aq. NH₄Cl (50 mL) at 0 °C and extracted with EA (3 x 50 mL). The combined organic layers were washed with brine (3 x 20 mL), dried over anhydrous Na₂SO₄. After filtration, the filtrate was concentrated under reduced pressure. The residue was purified by Prep-TLC, eluted with PE/EA (1/1) to afford *tert*-butyl 4-[[4,5-dichloro-2-(prop-2-en-1-yloxy)phenyl](hydroxy)methyl]azepane-1-carboxylate as a light yellow oil (0.60 g, 27%): LCMS (ESI) calc'd for C₂₁H₂₉Cl₂NO₄ [M + Na]⁺: 452, 454 (3 : 2), found 452, 454 (3 : 2); ¹H NMR (400 MHz, CDCl₃) δ 7.43 (d, *J =* 2.1 Hz, 1H), 6.94 (s, 1H), 6.08-5.93 (m, 1H), 5.47-5.30 (m, 2H), 4.77 (dd, *J =* 24.0, 6.2 Hz, 1H), 4.65-4.49 (m, 2H), 3.62-3.44 (m, 2H), 3.33-3.12 (m, 2H), 2.11-1.53 (m, 3H), 1.54-1.40 (m, 12H), 1.37-1.17 (m, 1H).

### Step b:

To a stirred solution of *tert*-butyl 4-[[4,5-dichloro-2-(prop-2-en-1-yloxy)phenyl](hydroxy)methyl]azepane-1-carboxylate (0.60 g, 1.40 mmol) in DCM (5 mL) was added Dess-Martin (0.12 g, 2.80 mmol) in portions at room temperature. The resulting solution was stirred for 2 h at room temperature. The reaction was quenched with saturated aq. Na₂SO₃ (10 mL) at room temperature. The resulting mixture was diluted with water (20 mL) and extracted with EA (3 x 50 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (5/1) to afford *tert-butyl* 4-[4,5-dichloro-2-(prop-2-en-1-yloxy)benzoyl]azepane-1-carboxylate as an off-white solid (0.60 g, 80%): LCMS (ESI) calc'd for C₂₁H₂₇Cl₂NO₄ [M + Na]⁺: 450, 452 (3 : 2), found 450, 452 (3 : 2); ¹H NMR (400 MHz, CDCl₃) δ 7.67 (s, 1H), 7.06 (s, 1H), 6.13-5.97 (m, 1H), 5.51-5.33 (m, 2H), 4.64 (dt, *J =* 5.5, 1.5 Hz, 2H), 3.70-3.48 (m, 2H), 3.45-3.34 (m, 2H), 3.29-3.20 (m, 1H), 2.16-1.95 (m, 2H), 1.95-1.83 (m, 1H), 1.83-1.69 (m, 1H), 1.65-1.53 (m, 2H), 1.49 (s, 9H).

### Step c:

To a stirred solution of *tert*-butyl 4-[4,5-dichloro-2-(prop-2-en-1-yloxy)benzoyl]azepane-1-carboxylate (0.60 g, 1.40 mmol) and (*S*)-2-methylpropane-2-sulfinamide (0.34 g, 2.80 mmol) in THF (3 mL) was added Ti(OEt)₄ (1.60 g, 7.00 mmol) dropwise at room temperature. The resulting solution was stirred for 16 h at 70 °C under nitrogen atmosphere. The reaction was quenched with saturated aq. NaHCO₃ (50 mL) at room temperature. The resulting mixture was extracted with EA (3 x 50 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (1/1) to afford *tert*-butyl 4-[[4,5-dichloro-2-(prop-2-en-1-yloxy)phenyl]([[(*S*)-2-methylpropane-2-sulfinyl]imino])methyl]azepane-1-carboxylate as a light yellow oil (0.30 g, 32%): LCMS (ESI) calc'd for C₂₅H₃₆Cl₂N₂O₄S [M + H]⁺: 531, 533 (3 : 2), found 531, 533 (3 : 2); ¹H NMR (400 MHz, CDCl₃) δ 7.18 (s, 1H), 6.98 (s, 1H), 6.14-5.89 (m, 1H), 5.47-5.26 (m, 2H), 4.62-4.51 (m, 2H), 3.69-3.42 (m, 2H), 3.42-3.15 (m, 2H), 2.73-2.31 (m, 2H), 2.23-2.08 (m, 1H), 2.05-1.68 (m, 2H), 1.67-1.51 (m, 2H), 1.50-1.46 (m, 9H), 1.23 (s, 9H).

### Step d:

To a stirred solution of *tert*-butyl 4-[[4,5-dichloro-2-(prop-2-en-1-yloxy)phenyl]([[(*S*)-2-methylpropane-2-sulfinyl]imino])methyl]azepane-1-carboxylate (0.30 g, 0.56 mmol) in THF (5 mL) were added DIBALH (1.13 mL, 1.13 mmol, 1 M in toluene) dropwise at -78 °C under nitrogen atmosphere. The resulting mixture was stirred for 2 h at -78 °C under nitrogen atmosphere. The reaction was quenched with saturated aq. NH₄Cl (2 mL) at - 78 °C. The resulting mixture was diluted with water (30 mL) and extracted with EA (3 x 30 mL). The combined organic layers were washed with brine (3 x 20 mL), dried over anhydrous Na₂SO₄. After filtration, the filtrate was concentrated under reduced pressure. The residue was purified by silica gel column chromatography, eluted with PE/EA (3/1) to afford *tert*-butyl 4-[(*R*)-[4,5-dichloro-2-(prop-2-en-1-yloxy)phenyl]([[(*S*)-2-methylpropane-2-sulfinyl]amino])methyl]azepane-1-carboxylate as an off-white solid (0.20 g, 53%): LCMS (ESI) calc'd for C₂₅H₃₈Cl₂N₂O₄S [M + H]⁺: 533, 535 (3 : 2), found 533, 535 (3 : 2); ¹H NMR (400 MHz, CDCl₃) δ 7.26 (s, 1H), 6.95 (s, 1H), 6.09-5.95 (m, 1H), 5.46-5.32 (m, 2H), 4.67 (d, *J =* 31.6 Hz, 1H), 4.59-4.44 (m, 2H), 3.76-3.37 (m, 2H), 3.34-3.08 (m, 2H), 2.03-1.80 (m, 3H), 1.80-1.59 (m, 1H), 1.57-1.30 (m, 11H), 1.24-1.15 (m, 10H).

### Step e:

To a stirred solution of *tert*-butyl 4-[(*R*)-[4,5-dichloro-2-(prop-2-en-1-yloxy)phenyl]([[(*S*)-2-methylpropane-2-sulfinyl]amino])methyl]azepane-1-carboxylate (0.12 g, 0.23 mmol) in DCM (2 mL) was added TFA (0.5 mL) at room temperature. The resulting solution was stirred for 30 min at room temperature. The residue was basified to pH 8 with saturated aq. NaHCO₃. The resulting mixture was diluted with water (20 mL) and extracted with EA (3 x 30 mL). The combined organic layers were washed with brine (3 x 20 mL), dried over anhydrous Na₂SO₄. After filtration, the filtrate was concentrated under reduced pressure to afford (*S*)-*N*-[(*R*)-azepan-4-yl[4,5-dichloro-2-(prop-2-en-1-yloxy)phenyl]methyl]-2-methylpropane-2-sulfinamide as a light yellow solid (0.12 g, crude), which was used in the next step directly without further purification: LCMS (ESI) calc'd for C₂₀H₃₀Cl₂N₂O₂S [M + H]⁺ : 433, 435 (3 : 2), found 433, 435 (3 : 2).

### Step f:

To a stirred solution of (4*R*)-2,2-dimethyl-1,3-dioxolane-4-carboxylic acid (81 mg, 0.55 mmol) and HATU (0.26 g, 0.69 mmol) in DMF (3 mL) were added (*S*)-*N*-[(*R*)-azepan-4-yl[4,5-dichloro-2-(prop-2-en-1-yloxy)phenyl]methyl]-2-methylpropane-2-sulfinamide (0.12 g, 0.28 mmol) and Et₃N (83 mg, 0.82 mmol) at room temperature. The reaction was stirred for 30 min at room temperature. The resulting solution was quenched with water (50 mL) and extracted with EA (3 x 40 mL). The combined organic layers were washed with brine (2 x 30 mL), dried over anhydrous Na₂SO₄. After filtration, the filtrate was concentrated under reduced pressure. The residue was purified by reverse phase chromatography, eluted with 63% ACN in water (plus 0.05% TFA) to afford (*S*)-*N*-[(*R*)-[4,5-dichloro-2-(prop-2-en-1-yloxy)phenyl]([1-[(4*R*)-2,2-dimethyl-1,3-dioxolane-4-carbonyl]azepan-4-yl])methyl]-2-methylpropane-2-sulfinamide as a light yellow oil (90 mg, 46%): LCMS (ESI) calc'd for C₂₆H₃₈Cl₂N₂O₅S [M + H]⁺: 561, 563 (3 : 2), found 561, 563 (3 : 2).

### Step g:

To a stirred solution of (*S*)-*N*-[(*R*)-[4,5-dichloro-2-(prop-2-en-1-yloxy)phenyl]([1-[(4*R*)-2,2-dimethyl-1,3-dioxolane-4-carbonyl]azepan-4-yl])methyl]-2-methylpropane-2-sulfinamide (90 mg, 0.16 mmol) and Pd(PPh₃)₄ (2 mg, 0.002 mmol) in THF (2 mL) was added NaBH₄ (18 mg, 0.48 mmol) at room temperature. The resulting mixture was stirred for 1 h at room temperature. The resulting mixture was quenched with saturated aq. NH₄Cl (1 mL) and concentrated under reduced pressure to afford (*S*)-*N*-[(*R*)-(4,5-dichloro-2-hydroxyphenyl)([1-[(4*R*)-2,2-dimethyl-1,3-dioxolane-4-carbonyl]azepan-4-yl])methyl]-2-methylpropane-2-sulfinamide as a brown solid (90 mg, crude), which was used in the next step directly without further purification: LCMS (ESI) calc'd for C₂₃H₃₄Cl₂N₂O₅S [M + H]⁺: 521, 523 (3 : 2), found 521, 523 (3 : 2).

### Step h:

To a stirred mixture of (*S*)-*N*-[(*R*)-(4,5-dichloro-2-hydroxyphenyl)([1-[(4*R*)-2,2-dimethyl-1,3-dioxolane-4-carbonyl]azepan-4-yl])methyl]-2-methylpropane-2-sulfinamide (90 mg, 0.17 mmol) in 1,4-dioxane (1 mL) was added aq. HCl (4 *N,* 1.00 mL) at room temperature. The resulting mixture was stirred for 1 h at room temperature. The resulting mixture was concentrated under reduced pressure. The residue was purified by Prep-HPLC with the following conditions: Column: XBridge Prep C18 OBD Column, 5 µm, 19 x 150 mm; Mobile Phase A: water (plus 0.05% TFA), Mobile Phase B: ACN; Flow rate: 25 mL/min; Gradient: 10% B to 29% B in 7 min; Detector: UV 220/254 nm; Retention Time: RT₁: 6.30 min; RT₂: 6.60 min.

The fractions containing the desired product at 6.30 min were collected and concentrated under reduced pressure to afford Compound 333 ((2*R*)-1-[4-[(*R*)-amino(4,5-dichloro-2-hydroxyphenyl)methyl]azepan-1-yl]-2,3-dihydroxypropan-1-one trifluoroacetic acid isomer 1) as an off-white solid (10.3 mg, 12% overall two steps): LCMS (ESI) calc'd for C₁₆H₂₂Cl₂N₂O₄ [M + H]⁺: 377, 379 (3 : 2), found 377, 379 (3 : 2). ¹H NMR (400 MHz, CD₃OD) δ 7.43 (d, *J* = 11.8 Hz, 1H), 7.08 (d, *J =* 6.5 Hz, 1H), 4.47 (dt, *J =* 26.7, 5.6 Hz, 1H), 4.22 (t, *J =* 8.9 Hz, 1H), 3.84-3.46 (m, 5H), 3.46-3.33 (m, 1H), 2.36-2.18 (m, 1H), 2.18-1.94 (m, 2H), 1.88-1.58 (m, 2H), 1.51-1.27 (m, 2H); ¹⁹F NMR (376 MHz, CD₃OD) δ -76.95.

The fractions containing the desired product at 6.60 min were collected and concentrated under reduced pressure to afford Compound 334 ((2*R*)-1-[4-[(*R*)-amino(4,5-dichloro-2-hydroxyphenyl)methyl]azepan-1-yl]-2,3-dihydroxypropan-1-one trifluoroacetic acid isomer 2) as an off-white solid (10.6 mg 12% overall two steps): LCMS (ESI) calc'd for C₁₆H₂₂Cl₂N₂O₄ [M + H]⁺: 377, 379 (3 : 2), found 377, 379 (3 : 2). ¹H NMR (400 MHz, CD₃OD) δ 7.43 (s, 1H), 7.08 (d, *J =* 2.2 Hz, 1H), 4.50 (t, *J =* 5.4 Hz, 1H), 4.34-4.19 (m, 1H), 3.98-3.52 (m, 6H), 2.31-2.02 (m, 2H), 2.02-1.83 (m, 1H), 1.72-1.44 (m, 3H), 1.41-1.21 (m, 1H); ¹⁹F NMR (376 MHz, CD₃OD) δ -76.94.

### Example 250. Compound 335 ((2R,3S)-2-[4-[(R)-amino(5-chloro-2-hydroxy-4-methylphenyl)methyl]piperidine-1-carbonyl]oxolan-3-ol trifluoroacetic acid)

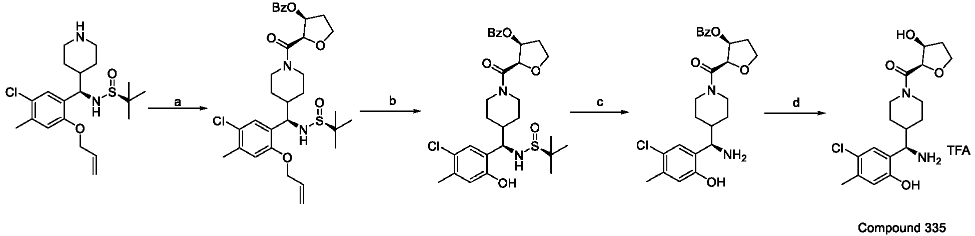

### Step a:

To a stirred solution of (2*R*,3*S*)-3-(benzoyloxy)oxolane-2-carboxylic acid (0.12 g, 0.49 mmol) and HATU (0.19 g, 0.49 mmol) in DMF (3 mL) were added (*S*)-*N*-[(*R*)-[5-chloro-4-methyl-2-(prop-2-en-1-yloxy)phenyl](piperidin-4-yl)methyl]-2-methylpropane-2-sulfinamide (Intermediate 25, Example 25) (0.13 g, 0.33 mmol) and Et₃N (99 mg, 0.98 mmol) at room temperature. The reaction solution was stirred at room temperature for 0.5 h. The resulting solution was quenched with water (50 mL) and extracted with EA (3 x 40 mL). The combined organic layers were washed with brine (2 x 30 mL), dried over anhydrous Na₂SO₄. After filtration, the filtrate was concentrated under reduced pressure. The residue was purified by reverse phase chromatography, eluted with 55% ACN in water with 10 mmoL/L NH₄HCO₃ to afford (2*R*,3*S*)-2-[4-[(*R*)-[5-chloro-4-methyl-2-(prop-2-en-1-yloxy)phenyl]([[(S)-2-methylpropane-2-sulfinyl]amino])methyl]piperidine-1-carbonyl]oxolan-3-yl benzoate as a light yellow foam (0.13 g, 65%): LCMS (ESI) calc'd for C₃₂H₄₁ClN₂O₆S [M + H]⁺: 617, 619 (3 : 1), found 617, 619 (3 : 1); ¹H NMR (400 MHz, CDCl₃) δ 8.04 (d, *J* = 7.6 Hz, 1H), 7.97 (d, *J =* 7.7 Hz, 1H), 7.70-7.53 (m, 1H), 7.53-7.37 (m, 2H), 7.11-7.01 (m, 1H), 6.70 (d, *J =* 22.3 Hz, 1H), 6.10 -5.92 (m, 1H), 5.88 (td, *J =* 6.1, 2.5 Hz, 1H), 5.45-5.23 (m, 2H), 4.92 (d, *J* = 5.6 Hz, 1H), 4.72-4.37 (m, 4H), 4.37-4.28 (m, 1H), 4.10-3.96 (m, 1H), 3.81-3.71 (m, 1H), 3.05-2.67 (m, 1H), 2.46-2.29 (m, 4H), 2.29-2.12 (m, 1H), 1.99-1.81 (m, 1H), 1.76-1.54 (m, 1H), 1.52-1.38 (m, 1H), 1.25-1.17 (m, 3H), 1.11 (d, *J =* 29.8 Hz, 9H)

### Step b:

To a stirred solution of (2*R*,3*S*)-2-[4-[(*R*)-[5-chloro-4-methyl-2-(prop-2-en-1-yloxy)phenyl]([[(*S*)-2-methylpropane-2-sulfinyl]amino])methyl]piperidine-1-carbonyl]oxolan-3-yl benzoate (0.13 g, 0.21 mmol) and Pd(PPh₃)₄ (5 mg, 0.004 mmol) in THF (3 mL) was added NaBH₄ (16 mg, 0.42 mmol) at room temperature. The reaction mixture was stirred at room temperature for 1 h. The resulting mixture was quenched with saturate aq. NH₄Cl (0.5 mL) and concentrated under reduced pressure to afford (*2R*,*3S*)-2-[4-[(*R*)-(5-chloro-2-hydroxy-4-methylphenyl)([[(*S*)-2-methylpropane-2-sulfinyl]amino])methyl]piperidine-1-carbonyl]oxolan-3-yl benzoate as a brown oil (0.12 g, crude), which was used in the next step directly without further purification: LCMS (ESI) calc'd for C₂₉H₃₇ClN₂O₆S [M + H]⁺: 577, 579 (3 : 1), found 577, 579 (3 : 1).

### Step c:

To a stirred solution of (*2R*, *3S*)-2-[4-[(*R*)-(5-chloro-2-hydroxy-4-methylphenyl)([[(*S*)-2-methylpropane-2-sulfinyl]amino])methyl]piperidine-1-carbonyl]oxolan-3-yl benzoate (0.12 g, 0.21 mmol) in THF (2 mL) was added aq. HCl (4 *N,* 1 mL) at room temperature. The reaction solution was stirred at room temperature for 1 h, and concentrated under reduced pressure to afford (*2R,3S*)-2-[4-[(*R*)-amino(5-chloro-2-hydroxy-4-methylphenyl)methyl]piperidine-1-carbonyl]oxolan-3-yl benzoate as a brown solid (95 mg, crude), which was used in the next step directly without further purification: LCMS (ESI) calc'd for C₂₅H₂₉ClN₂O₅ [M + H]⁺: 473, 475 (3 : 1), found 473, 475 (3 : 1).

### Step d:

A solution of (*2R*,*3S*)-2-[4-[(*R*)-amino(5-chloro-2-hydroxy-4-methylphenyl)methyl]piperidine-1-carbonyl]oxolan-3-yl benzoate (95 mg, 0.20 mmol) in methylamine in methanol (4 mL) was stirred for 16 h at room temperature. The reaction was concentrated under reduced pressure. The residue was dissolved in HCl (1 *N,* 30 mL). The aqueous layer was extracted with EA (2 x 30 mL). The aqueous layer was concentrated under reduced pressure. The residue was purified by Prep-HPLC with following conditions: Column: Xselect CSH OBD Column 30 x 150 mm 5 µm; Mobile Phase A: water (plus 0.05% TFA), Mobile Phase B: ACN; Flow rate: 60 mL/min; Gradient: 7% B to 20% B in 7 min; Detector: UV 254/220 nm; Retention time: 6.93 min. The fractions containing the desired product were collected and concentrated under reduced pressure to afford Compound 335 ((2*R*,3*S*)-2-[4-[(*R*)-amino(5-chloro-2-hydroxy-4-methylphenyl)methyl]piperidine-1-carbonyl]oxolan-3-ol trifluoroacetic acid) as an off-white solid (52.1 mg, 51% overall three steps): LCMS (ESI) calc'd for C₁₈H₂₅ClN₂O₄ [M + H]⁺: 369, 371 (3 : 1), found 369, 371 (3 : 1); ¹H NMR (400 MHz, CD₃OD) δ 7.24 (s, 1H), 6.85 (s, 1H), 4.73-4.46 (m, 3H), 4.29-4.00 (m, 3H), 3.95-3.82 (m, 1H), 3.17-2.90 (m, 1H), 2.78-2.48 (m, 1H), 2.44-2.27 (m, 4H), 2.27-2.10 (m, 1H), 2.08-1.90 (m, 2H), 1.55-1.07 (m, 3H); ¹⁹F NMR (376 MHz, CD₃OD) δ -77.07.

### Example 251. Compound 336 ((2R)-1-[4-[(S)-(4,5-dichloro-2-hydroxyphenyl)(methylamino)methyl]piperidin-1-yl]-2,3-dihydroxypropan-1-one);

### Compound 337 ((2R)-1-[4-[(R)-(4,5-dichloro-2-hydroxyphenyl)(methylamino)methyl]piperidin-1-yl]-2,3-dihydroxypropan-1-one)

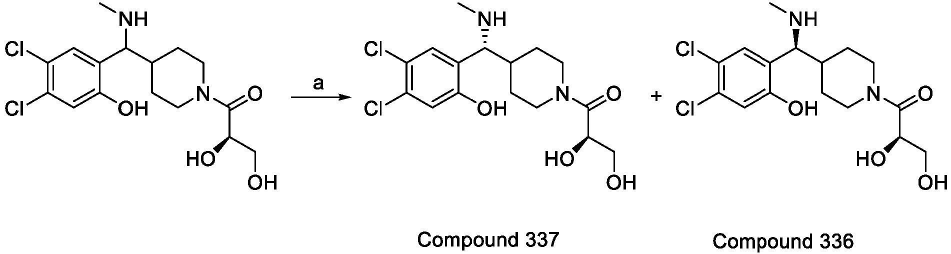

### Step a:

(2*R*)-1-[4-[(4,5-dichloro-2-hydroxyphenyl)(methylamino)methyl]piperidin-1-yl]-2,3-dihydroxypropan-1-one (Compound 196's free base, Example 188) (40 mg) was separated by Chiral Prep-HPLC with the following conditions: Column: Chiralpak IF, 2 x 25 cm, 5 µm; Mobile Phase A: Hexane (0.1% IPA), Mobile Phase B: EtOH; Flow rate: 20 mL/min; Gradient: 30% B to 30% B in 18 min; Detector: UV 254/220 nm; Retention time: RT₁: 10.21 min; RT₂: 14.65 min.

The faster-eluting enantiomer was obtained as Compound 337 ((2*R*)-1-[4-[(*R*)-(4,5-dichloro-2-hydroxyphenyl)(methylamino)methyl]piperidin-1-yl]-2,3-dihydroxypropan-1-one) as an off-white solid (4 mg, 10%) at 10.21 min: LCMS (ESI) calc'd for C₁₆H₂₂Cl₂N₂O₄ [M + H]⁺: 377, 379 (3 : 2), found 377, 379 (3 : 2); ¹H NMR (400 MHz, CD₃OD) δ 7.44 (s, 1H), 7.11 (s, 1H), 4.71-4.31 (m, 2H), 4.31-4.03 (m, 2H), 3.85-3.51 (m, 2H), 3.25-2.98 (m, 1H), 2.84-2.36 (m, 2H), 2.58 (s, 3H), 2.15-1.9 (m, 1H), 1.54-1.01 (m, 3H).

The slower-eluting enantiomer was obtained as Compound 336 ((2*R*)-1-[4-[(*S*)-(4,5-dichloro-2-hydroxyphenyl)(methylamino)methyl]piperidin-1-yl]-2,3-dihydroxypropan-1-one) as an off-white solid (11 mg, 28%) at 14.65 min: LCMS (ESI) calc'd for C₁₆H₂₂ Cl₂N₂O₄ [M + H]⁺: 377, 379 (3 : 2), found 377, 379 (3 : 2); ¹H NMR (400 MHz, CD₃OD) δ 7.17 (s, 1H), 6.87 (s, 1H), 4.65-4.44 (m, 2H), 4.13 (dd, *J =* 31.3, 13.6 Hz, 1H), 3.73-3.57 (m, 3H), 3.21-2.89 (m, 1H), 2.80-2.51 (m, 1H), 2.39 (s, 3H), 2.21-2.02 (m, 1H), 1.97 (d, *J =* 12.9 Hz, 1H), 1.55 (d, *J =* 13.0 Hz, 1H), 1.37-1.16 (m, 2H).

### Example 252. Compound 338 (5-[4-[(R)-amino(4,5-dichloro-2-hydroxyphenyl)methyl]piperidine-1-carbonyl]-1,3-oxazinan-2-one isomer 1);

### Compound 210 (5-[4-[(R)-amino(4,5-dichloro-2-hydroxyphenyl)methyl]piperidine-1-carbonyl]-1,3-oxazinan-2-one isomer 2)

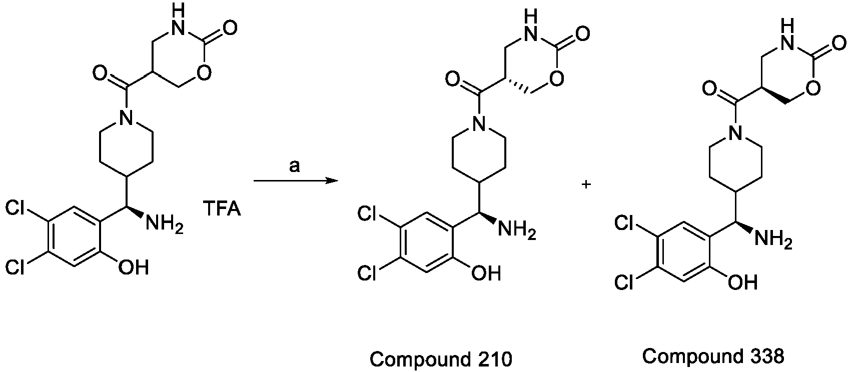

### Step a:

The 5-[4-[(*R*)-amino(4,5-dichloro-2-hydroxyphenyl)methyl]piperidine-1-carbonyl]-1,3-oxazinan-2-one trifluoroacetic acid (Compound 279, Table 1b) (0.14 g, 0.27 mmol) was purified by Prep-CHIRAL-HPLC with the following conditions: Column: CHIRALPAK IE, 2 x 25 cm, 5 µm; Mobile Phase A: Hexane (plus 0.2% IPA), Mobile Phase B: EtOH; Flow rate: 15 mL/min; Gradient: 50% B to 505 B in 23 min; Detector: UV 220/254 nm; Retention time: RT₁: 15.37 min; RT₂: 19.03 min; Injection Volumn: 0.3 mL; Number Of Runs: 8.

The faster-eluting enantiomer at 15.37 min was obtained as Compound 210 (5-[4-[(*R*)-amino(4,5-dichloro-2-hydroxyphenyl)methyl]piperidine-1-carbonyl]-1,3-oxazinan-2-one isomer 2) as an off-white solid (32.6 mg, 30%): LCMS (ESI) calc'd for C₁₇H₂₁Cl₂N₃O₄ [M + H]⁺: 402, 404 (3 : 2), found 402, 404 (3 : 2); ¹H NMR (400 MHz,CD₃OD) δ 7.23 (d, *J =* 7.2 Hz, 1H), 6.87 (s, 1H), 4.55 (dd, *J =* 41.0, 13.3 Hz, 1H), 4.42-4.24 (m, 2H), 4.10 (dd, *J =* 40.0, 13.8 Hz, 1H), 3.91 (dd, *J =* 13.5, 7.6 Hz, 1H), 3.54-3.36 (m, 3H), 3.18-2.97 (m, 1H), 2.60 (dt, *J =* 33.9, 12.8 Hz, 1H), 2.16-1.93 (m, 2H), 1.51 (dd, *J* = 28.7, 13.3 Hz, 1H), 1.42-1.06 (m, 2H).

The slower-eluting enantiomer was obtained as Compound 338 (5-[4-[(*R*)-amino(4,5-dichloro-2-hydroxyphenyl)methyl]piperidine-1-carbonyl]-1,3-oxazinan-2-one isomer 1) as an off-white solid (28.5 mg, 26%) at 19.03 min: LCMS (ESI) calc'd for C₁₇H₂₁Cl₂N₃O₄ [M + H]⁺: 402, 404 (3 : 2), found 402, 404 (3 : 2); ¹H NMR (400 MHz, CD₃OD) δ 7.24 (s, 1H), 6.87 (s, 1H), 4.55 (dd, *J =* 36.5, 13.4 Hz, 1H), 4.44-4.26 (m, 2H), 4.10 (dd, *J =* 36.6, 13.7 Hz, 1H), 3.91 (t, *J =* 7.3 Hz, 1H), 3.52-3.38 (m, 3H), 3.11 (dt, *J =* 41.2, 13.2 Hz, 1H), 2.70-2.49 (m, 1H), 2.11-1.94 (m, 2H), 1.51 (t, *J =* 16.2 Hz, 1H), 1.35-1.09 (m, 2H).

### Example 253. Compound 78 (2-[amino[1-(2-carbamoylethyl)piperidin-4-yl]methyl]-4,5-dichlorophenyl formate);

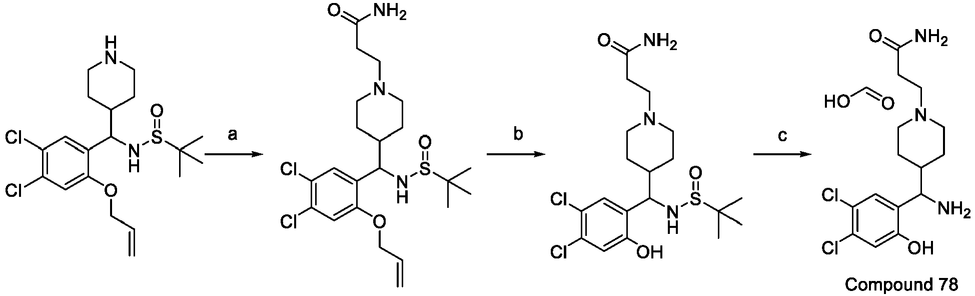

### Step a:

A mixture of *N*-[[4,5-dichloro-2-(prop-2-en-1-yloxy)phenyl](piperidin-4-yl)methyl]-2-methylpropane-2-sulfinamide (0.20 g, 0.48 mmol) in prop-2-enamide (3 mL) was stirred for 12 h at 120 °C under nitrogen atmosphere. The resulting mixture was diluted with EA (20 mL) and water (20 mL). The aqueous solution was extracted with EA (2 x 20 mL). The combined organic layer was washed with brine (2 x 20 mL) and dried over anhydrous Na₂SO₄. After filtration, the filtrate was concentrated under reduced pressure to afford 3-(4-[[4,5-dichloro-2-(prop-2-en-1-yloxy)phenyl][(2-methylpropane-2-sulfinyl)amino]methyl]piperidin-1-yl)propanamide as a yellow oil (0.13 g, 56%); LCMS (ESI) calc'd for C₂₂H₃₃Cl₂N₃O₃S [M + H]⁺: 490, 492 (3 : 2) found 490, 492 (3 : 2); ¹H NMR (300 MHz, DMSO-d₆) δ 7.54 (s, 1H), 7.23 (s, 1H), 6.11-5.91 (m, 1H), 5.47-5.13 (m, 2H), 4.74-4.47 (m, 2H), 4.47-4.31 (m, 1H), 3.54-3.43 (m, 1H), 3.40-3.29 (m, 1H), 3.29-3.10 (m, 2H), 2.90-2.61 (m, 2H), 2.23-1.79 (m, 3H), 1.54-1.21 (m, 3H), 1.02-0.93 (m, 10H).

### Step b:

To a stirred solution of 3-(4-[[4,5-dichloro-2-(prop-2-en-1-yloxy)phenyl][(2-methylpropane-2-sulfinyl)amino]methyl]piperidin-1-yl)propanamide (0.10 g, 0.20 mmol) in THF (4 mL) was added L-proline (47 mg, 0.41 mmol) and Pd(PPh₃)₄ (47 mg, 0.04 mmol) at room temperature. The resulting mixture was stirred for 16 h at room temperature under nitrogen atmosphere. The reaction was quenched with water (1 mL) at room temperature. The resulting mixture was concentrated under reduced pressure to afford 3-[4-[(4,5-dichloro-2-hydroxyphenyl)[(2-methylpropane-2-sulfinyl)amino]methyl]piperidin-1-yl]propanamide (0.10 g, crude), which was used directly in the next step without further purification: LCMS (ESI) calc'd for C₁₉H₂₉Cl₂N₃O₃S [M + H]⁺ 450, 452 (3 : 2) found 450, 452 (3 : 2);

### Step c:

To a stirred solution of 3-[4-[(4,5-dichloro-2-hydroxyphenyl)[(2-methylpropane-2-sulfinyl)amino]methyl]piperidin-1-yl]propanamide (0.10 g, 0.22 mmol) in dioxane (2 mL) was added HCl (4 *N,* 1 mL) at room temperature. The reaction was stirred at room temperature for 5 h. The mixture was neutralized to pH=7 with saturated aq. NaHCO₃. The mixture was concentrated under reduced pressure.

The resulting mixture was concentrated under vacuum. The crude product was purified by Prep-HPLC with the following conditions (Column: XBridge C18 OBD Prep Column, 19 mm x 250 mm; Mobile Phase A:Water (0.05%TFA ), Mobile Phase B: ACN; Flow rate: 25 mL/min; Gradient: 4% B to 24% B in 8 min; 254/210 nm; Rt: 6.67 min. The fractions containing desired product were collected and concentrated under reduced pressure to afford Compound 78 (2-[amino[1-(2-carbamoylethyl)piperidin-4-yl]methyl]-4,5-dichlorophenyl formate) as an off-white solid (10 mg, 14% overall two steps): LCMS (ESI) calc'd for C₁₅H₂₁Cl₂N₃O₂ [M + H]⁺: 346, 348 (3 : 2) found 346, 348 (3 : 2); ¹H NMR (300 MHz, CD₃OD) δ 7.19 (s, 1H), 6.85 (s, 1H), 3.89 (d, *J =* 7.8 Hz, 1H), 3.10-3.00 (m, 1H), 3.00-2.91 (m, 1H), 2.72-2.60 (m, 2H), 2.41 (t, *J =* 7.3 Hz, 2H), 2.14-1.86 (m, 3H), 1.86-1.73 (m, 1H), 1.51-1.18 (m, 3H).

### Example 254. (which is a reference example) Compound 81 (4-[(S)-amino(4,5-dichloro-2-hydroxyphenyl)methyl]piperidine-1-carboxamide);

### Compound 82 (4-[(R)-amino(4,5-dichloro-2-hydroxyphenyl)methyl]piperidine-1-carboxamide)

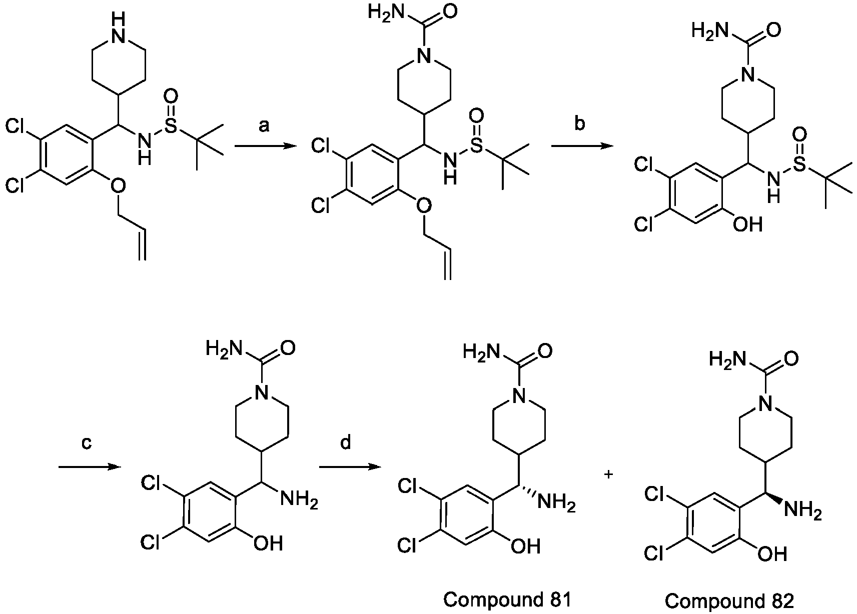

### Step a:

To a stirred mixture of *N*-[[4,5-dichloro-2-(prop-2-en-1-yloxy)phenyl](piperidin-4-yl)methyl]-2-methylpropane-2-sulfinamide (0.20 g, 0.48 mmol) and Et₃N (73 mg, 0.72 mmol) in DCM (2 mL) was added isocyanatotrimethylsilane (66 mg, 0.57 mmol) at room temperature. The resulting mixture was stirred for 1 h at room temperature. The resulting mixture was concentrated under reduced pressure. The residue was purified by reverse phase chromatography, eluted with 70% ACN in water (0.05% TFA) to afford 4-[[4,5-dichloro-2-(prop-2-en-1-yloxy)phenyl][(2-methylpropane-2-sulfinyl)amino]methyl]piperidine-1-carboxamide as a yellow oil (0.15 g, 68%); LCMS (ESI) calc'd for C₂₀H₂₉Cl₂N₃O₃S [M + H]⁺: 462, 464 (3 : 2) found 462, 464 (3 : 2).

### Step b:

To a stirred solution of 4-[[4,5-dichloro-2-(prop-2-en-1-yloxy)phenyl][(2-methylpropane-2-sulfinyl)amino]methyl]piperidine-1-carboxamide (0.15 g, 0.33 mmol) and Pd(PPh₃)₄ (7 mg, 0.01 mmol) in THF (2 mL) was added NaBH₄ (25 mg, 0.65 mmol) at room temperature. The reaction was stirred at room temperature for 1 h. The reaction solution was quenched with saturated aq. NH₄Cl (1 mL) and concentrated under reduced pressure to afford 4-[(4,5-dichloro-2-hydroxyphenyl)[(2-methylpropane-2-sulfinyl)amino]methyl]piperidine-1-carboxamide as a brown solid (0.13 g, crude), which was used to next step directly without further purification: LCMS (ESI) calc'd for C₁₇H₂₅Cl₂N₃O₃S [M + H]⁺ 422, 424 (3 : 2) found 422, 424 (3 : 2).

### Step c:

To a stirred solution of 4-[(4,5-dichloro-2-hydroxyphenyl)[(2-methylpropane-2-sulfinyl)amino]methyl]piperidine-1-carboxamide (0.13 g, 0.31 mmol) in dioxane (2 mL) was added HCl (4 *N,* 1 mL) at room temperature. The reaction was stirred at room temperature for 5 h. The reaction solution was concentrated under reduced pressure. The residue was purified by Prep-HPLC with the following conditions: Column: XBridge C18 OBD Prep Column 100 Å, 10 µm, 19 mm x 250 mm; Mobile Phase A: Water with 20 mmol/L NH₄HCO₃, Mobile Phase B: ACN; Flow rate: 25 mL/min; Gradient: 20% B to 80% B in 6.5 min; Detector: UV 254/210 nm; Retention time 5.95 min. The fractions containing the desired product were collected and concentrated under reduced pressure to afford 4-[amino(4,5-dichloro-2-hydroxyphenyl)methyl]piperidine-1-carboxamide as an off-white solid (69.6 mg, 67% overall two steps): LCMS (ESI) calc'd for C₁₃H₁₇Cl₂N₃O₂ [M + H]⁺: 318, 320 (3 : 2) found 318, 320 (3 : 2); ¹H NMR (300 MHz, CD₃OD) δ 7.21 (s, 1H), 6.86 (s, 1H), 4.18-4.03 (m, 1H), 4.03-3.93 (m, 1H), 3.89 (d, *J =* 7.6 Hz, 1H), 2.86-2.62 (m, 2H), 2.10-1.85 (m, 2H), 1.50-1.34 (m, 1H), 1.34-1.12 (m, 2H).

### Step d:

4-(amino(4,5-dichloro-2-hydroxyphenyl)methyl)piperidine-1-carboxamide (65mg, 0.20 mmol) was separated by Prep Chiral HPLC with the following conditions: Column: CHIRALPAK IG, 20 x 250 mm, 5 µm; Mobile Phase A: Hexane (plus 0.2% IPA)-HPLC, Mobile Phase B: EtOH-HPLC; Flow rate: 20 mL/min; Gradient: 30% B to 30% B in 15 min; Detector: UV 220/254 nm; Retention time: RT₁: 9.17 min; RT₂: 12.55 min. The faster-eluting enantiomer at 9.17 min was obtained as Compound 81 (4-[(*S*)-amino(4,5-dichloro-2-hydroxyphenyl)methyl]piperidine-1-carboxamide) as an off-white solid (10 mg, 15%): LCMS (ESI) calc'd for C₁₃H₁₇Cl₂N₃O₂ [M + H]⁺: 318, 320 (3 : 2) found 318, 320 (3 : 2); ¹H NMR (300 MHz, CD₃OD) δ 7.19 (s, 1H), 6.85 (s, 1H), 4.15-4.03 (m, 1H), 4.02-3.91 (m, 1H), 3.87 (d, *J =* 7.6 Hz, 1H), 2.85-2.66 (m, 2H), 2.04-1.84 (m, 2H), 1.50-1.32 (m, 1H), 1.32-1.07 (m, 2H). The slower-eluting enantiomer at 12.55 min was obtained as Compound 82 (4-[(*R*)-amino(4,5-dichloro-2-hydroxyphenyl)methyl]piperidine-1-carboxamide) as an off-white solid (8 mg, 12%): LCMS (ESI) calc'd for C₁₃H₁₇CL₂N₃O₂ [M + H]⁺: 318, 320 (3 : 2) found 318, 320 (3 : 2); ¹H NMR (300 MHz, CD₃OD) δ 7.21 (s, 1H), 6.86 (s, 1H), 4.16-4.06 (m, 1H), 4.02-3.93 (m, 1H), 3.89 (d, *J =* 7.6 Hz, 1H), 2.86-2.62 (m, 2H), 2.06-1.85 (m, 2H), 1.48-1.37 (m, 1H), 1.37-1.09 (m, 2H).

### Example 255. Evaluation of Kv1.3 potassium channel blocker activities

The assay described below is used to evaluate the disclosed 'compound's activities as Kv1.3 potassium channel blockers.

### Cell culture

CHO-K1 cells stably expressing Kv1.3 were grown in DMEM containing 10% heat-inactivated FBS, 1 mM Sodium Pyruvate, 2 mM L-Glutamine and G418 (500 µg/ml). Cells were grown in culture flasks at 37 °C in a 5% CO₂-humidified incubator.

### Solutions

The cells were bathed in an extracellular solution containing 140 mM NaCl, 4 mM KCl, 2 mM CaCl₂, 1 mM MgCl₂, 5 mM Glucose, 10 mM HEPES; pH adjusted to 7.4 with NaOH; 295-305 mOsm. The internal solution contained 50 mM KCl, 10 mM NaCl, 60 mM KF, 20 mM EGTA, 10 mM HEPES; pH adjusted to 7.2 with KOH; 285 mOsm. All compounds were dissolved in DMSO at 30 mM. Compound stock solutions were freshly diluted with external solution to concentrations of 30 nM, 100 nM, 300 nM, 1 µM, 3 µM, 10 µM, 30 µM and 100 µM. The highest content of DMSO (0.3%) was present in 100 µM.

### Voltage protocol

The currents were evoked by applying 100 ms depolarizing pulses from -90 mV (holding potential) to +40 mV were applied with 0.1 Hz frequency. Control (compound-free) and Compound pulse trains for each Compound concentration applied contained 20 pulses. 10 second breaks were used between pulse trains (*see* Table A below).

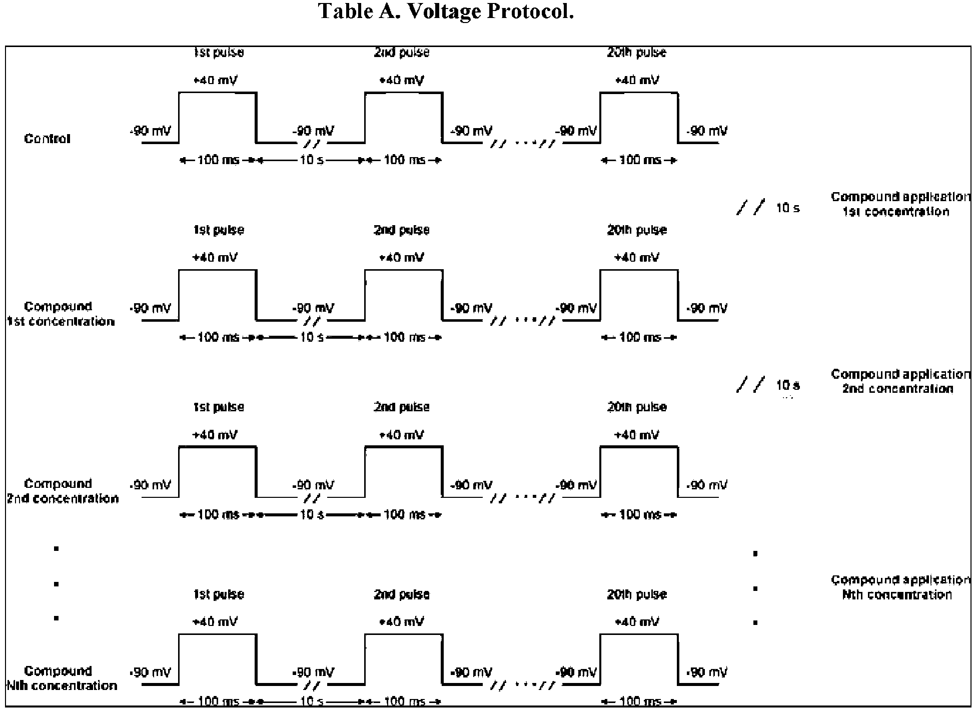

### Patch clamp recordings and compound application

Whole cell current recordings and compound application were enabled by means of an automated patch clamp platform Patchliner (Nanion Technologies GmbH). EPC 10 patch clamp amplifier (HEKA Elektronik Dr. Schulze GmbH) along with Patchmaster software (HEKA Elektronik Dr. Schulze GmbH) was used for data acquisition. Data were sampled at 10kHz without filtering. Passive leak currents were subtracted online using a P/4 procedure (HEKA Elektronik Dr. Schulze GmbH). Increasing compound concentrations were applied consecutively to the same cell without washouts in between. Total compound incubation time before the next pulse train was not longer than 10 seconds. Peak current inhibition was observed during compound equilibration.

### Data analysis

AUC and peak values were obtained with Patchmaster (HEKA Elektronik Dr. Schulze GmbH). To determine IC₅₀, the last single pulse in the pulse train corresponding to a given compound concentration was used. Obtained AUC and peak values in the presence of compound were normalized to control values in the absence of compound. Using Origin (OridinLab), IC₅₀ was derived from data fit to Hill equation: I_{compound}/I_{control}=(100-A)/(1 + ([compound]/IC₅₀)nH)+A, where IC₅₀ value is the concentration at which current inhibition is half-maximal, [compound] is the applied compound concentration, A is the fraction of current that is not blocked and nH is the Hill coefficient.

### Example 256. Evaluation of hERG activities

### hERG electrophysiology

This assay is used to evaluate the disclosed compounds' inhibition activities against the hERG channel.

### Cell culture

CHO-K1 cells stably expressing hERG were grown in Ham's F-12 Medium with Glutamine containing 10% heat-inactivated FBS, 1% Penicillin/Streptomycin, Hygromycin (100 µg/ml) and G418 (100 µg/ml). Cells were grown in culture flasks at 37°C in a 5% CO₂-humidified incubator.

### Solutions

The cells were bathed in an extracellular solution containing 140 mM NaCl, 4 mM KCl, 2 mM CaCl₂, 1 mM MgCl₂, 5 mM Glucose, 10 mM HEPES; pH adjusted to 7.4 with NaOH; 295-305 mOsm. The internal solution contained 50 mM KCl, 10 mM NaCl, 60 mM KF, 20 mM EGTA, 10 mM HEPES; pH adjusted to 7.2 with KOH; 285 mOsm. All compounds were dissolved in DMSO at 30 mM. Compound stock solutions were freshly diluted with external solution to concentrations of 30 nM, 100 nM, 300 nM, 1 µM, 3 µM, 10 µM, 30 µM and 100 µM. The highest content of DMSO (0.3%) was present in 100 µM.

### Voltage protocol

The voltage protocol (*see* Table B) was designed to simulate voltage changes during a cardiac action potential with a 300 ms depolarization to +20 mV (analogous to the plateau phase of the cardiac action potential), a repolarization for 300 ms to -50 mV (inducing a tail current) and a final step to the holding potential of -80 mV. The pulse frequency was 0.3 Hz. Control (compound-free) and compound pulse trains for each compound concentration applied contained 70 pulses.

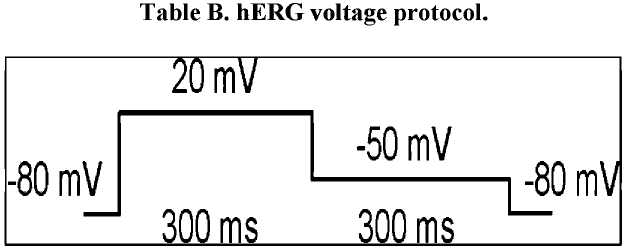

### Patch clamp recordings and compound application

Whole cell current recordings and compound application were enabled by means of an automated patch clamp platform Patchliner (Nanion). EPC 10 patch clamp amplifier (HEKA) along with Patchmaster software (HEKA Elektronik Dr. Schulze GmbH) was used for data acquisition. Data were sampled at 10 kHz without filtering. Increasing compound concentrations were applied consecutively to the same cell without washouts in between.

### Data analysis

AUC and PEAK values were obtained with Patchmaster (HEKA Elektronik Dr. Schulze GmbH). To determine IC₅₀ the last single pulse in the pulse train corresponding to a given compound concentration was used. Obtained AUC and PEAK values in the presence of compound were normalized to control values in the absence of compound. Using Origin (OridinLab), IC₅₀ was derived from data fit to Hill equation: I_{compound}/I_{control}=(100-A)/(1 + ([compound]/IC₅₀)nH)+A, where IC₅₀ is the concentration at which current inhibition is half-maximal, [compound] is the applied compound concentration, A is the fraction of current that is not blocked and nH is the Hill coefficient.

Table 1 provides a summary of the inhibition activities of certain selected compounds against Kv1.3 potassium channel and hERG channel. Compound numbers marked with a ** are reference compounds.

**Table 1. IC₅₀ (µM) values of certain exemplified compounds against Kv1.3 potassium channel and hERG channel.**

| Compound Number | Structure | Kv1.3_IC₅₀ (µM) | hERG_IC₅₀(µM) |
|---|---|---|---|
| 1** | 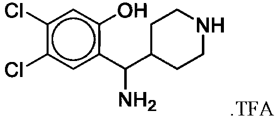 | <10 | >30 |
| 2** | 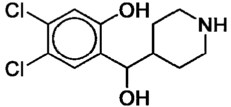 | <10 | >30 |
| 3** | 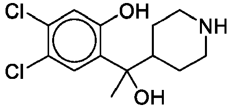 | <10 | * |
| 4 | 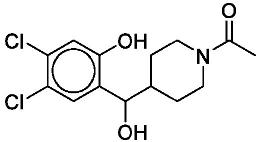 | <10 | >30 |
| 5** | 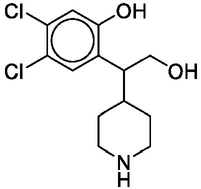 | <10 | * |
| 6** | 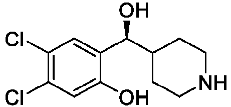 | <10 | >30 |
| 7** | 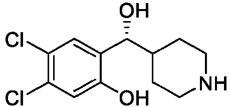 | <10 | >30 |
| 8** | 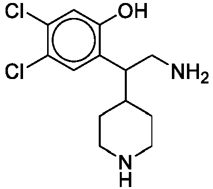 | <10 | * |
| 9 | 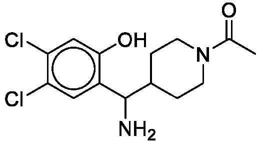 | <10 | >30 |
| 10** | 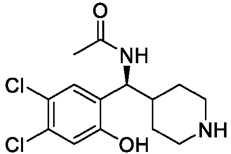 | <10 | >30 |
| 11** | 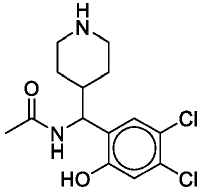 | <10 | >30 |
| 12** | 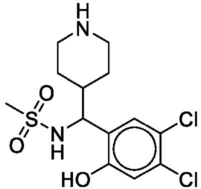 | <10 | * |
| 13** | 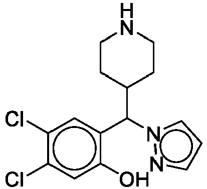 | <10 | * |
| 14** | 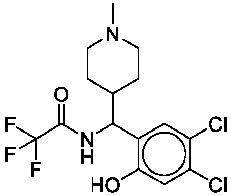 | <10 | * |
| 15** | 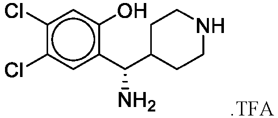 | <10 | * |
| 16** | 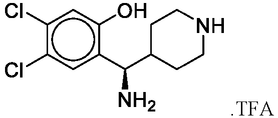 | <0.5 | >30 |
| 17** | 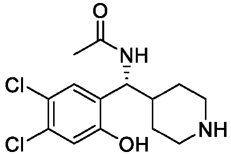 | <30 | * |
| 18** | 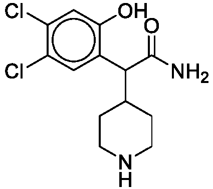 | <10 | * |
| 19 | 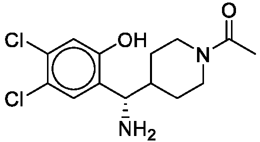 | <10 | <30 |
| 20 | 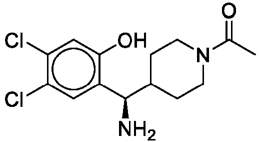 | <0.5 | <30 |
| 21** | 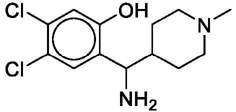 | <10 | <30 |
| 22** | 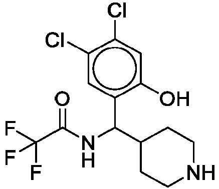 | <10 | * |
| 23 | 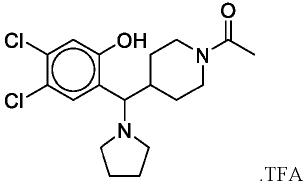 | <10 | * |
| 24** | 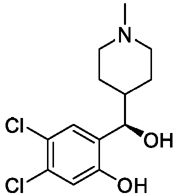 | <10 | * |
| 25** | 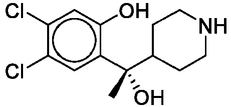 | <10 | * |
| 26** | 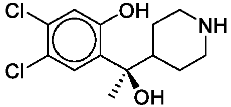 | <10 | * |
| 27 | 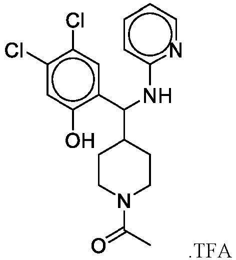 | <30 | * |
| 28 | 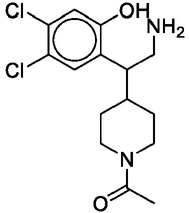 | <30 | * |
| 29** | 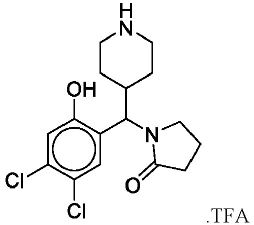 | <10 | * |
| 30** | 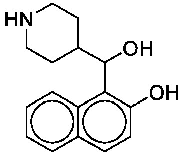 | <10 | >30 |
| 31** | 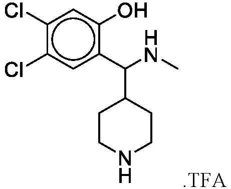 | <10 | >30 |
| 32** | 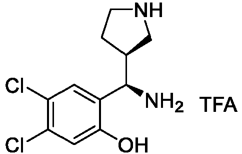 | <10 | >30 |
| 33** | 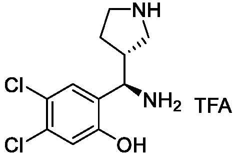 | <10 | >30 |
| 34** | 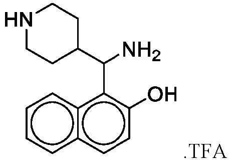 | <30 | >30 |
| 35 | 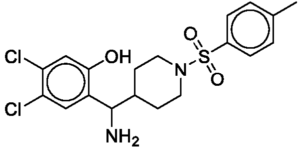 | <10 | * |
| 36** | 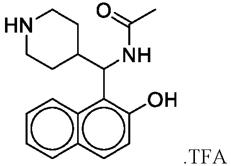 | <10 | >30 |
| 37** | 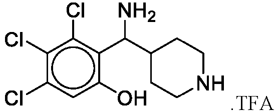 | <10 | >30 |
| 38** | 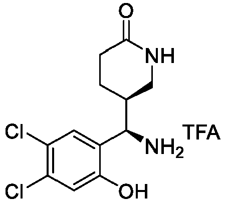 | <10 | * |
| 39** | 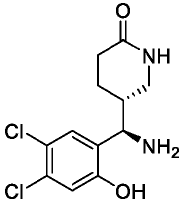 | <10 | >30 |
| 40** | 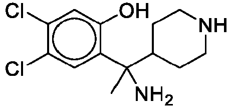 | <30 | * |
| 41** | 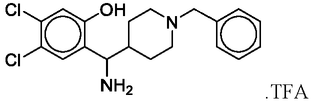 | <10 | * |
| 42** | 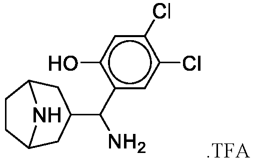 | <10 | >30 |
| 43 | 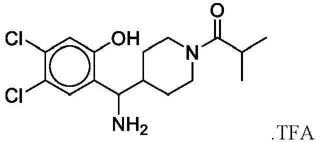 | <10 | <30 |
| 44 | 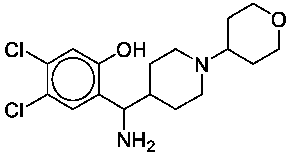 | <10 | <30 |
| 45 | 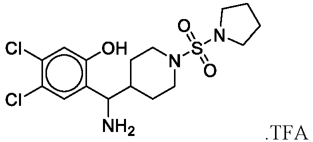 | <10 | <30 |
| 46** | 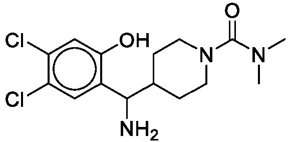 | <10 | <30 |
| 47 | 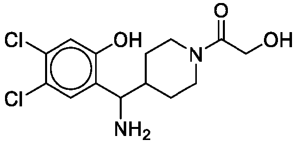 | <0.5 | >30 |
| 48 | 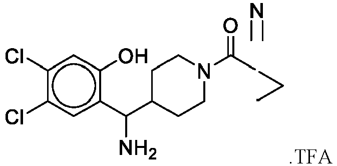 | <0.5 | <30 |
| 49 | 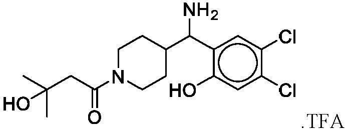 | <10 | <30 |
| 50 | 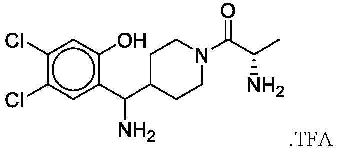 | <0.5 | >30 |
| 51** | 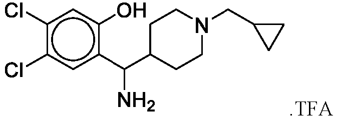 | <10 | >30 |
| 52** | 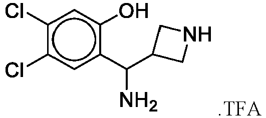 | <10 | >30 |
| 53** | 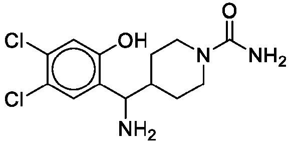 | <0.5 | >30 |
| 54 | 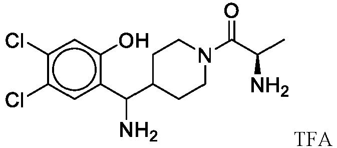 | <0.5 | >30 |
| 55** | 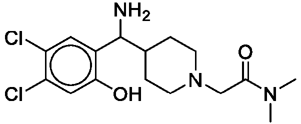 | <10 | * |
| 56 | 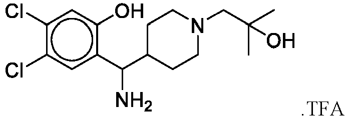 | <10 | <30 |
| 57* | 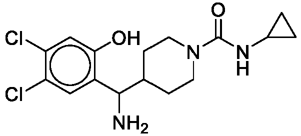 | <10 | * |
| 58** | 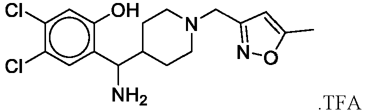 | <10 | * |
| 59** | 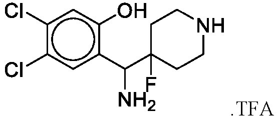 | <10 | <30 |
| 60** | 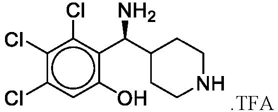 | <30 | >30 |
| 61** | 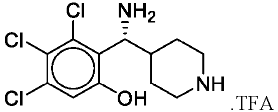 | <10 | >30 |
| 62** | 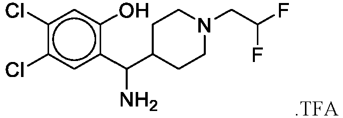 | <10 | * |
| 63 | 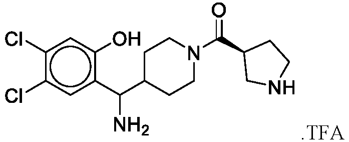 | <0.5 | >30 |
| 64 | 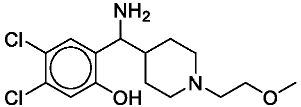 | <10 | * |
| 65 | 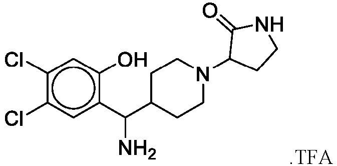 | <10 | * |
| 66 | 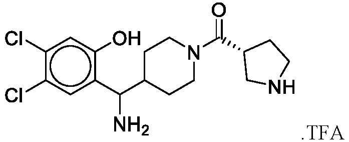 | <0.5 | >30 |
| 67 | 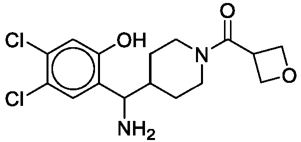 | <0.5 | <30 |
| 68 | 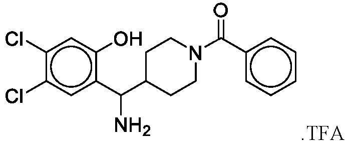 | <10 | <30 |
| 69 | 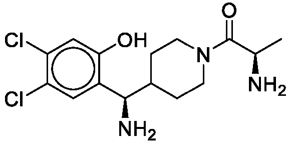 | <0.5 | >30 |
| 70 | 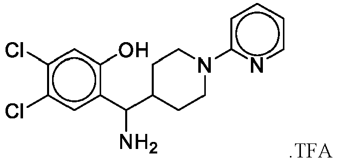 | <0.5 | <30 |
| 71 | 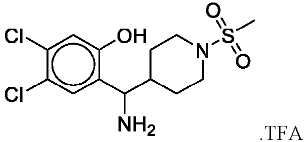 | <10 | * |
| 72** | 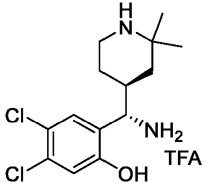 | <30 | * |
| 73** | 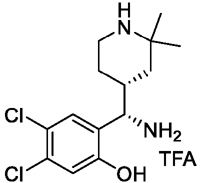 | <10 | * |
| 74 | 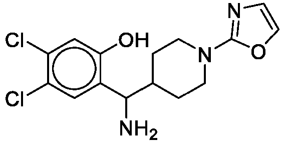 | <10 | <30 |
| 75 | 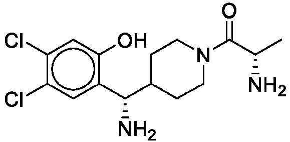 | <10 | >30 |
| 76 | 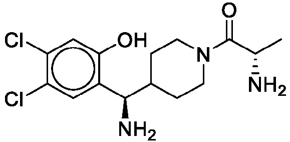 | <0.5 | >30 |
| 77 | 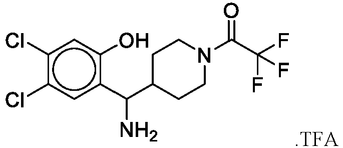 | <10 | * |
| 78 | 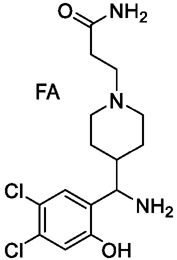 | <10 | * |
| 79 | 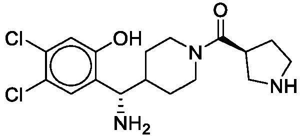 | <10 | * |
| 80 | 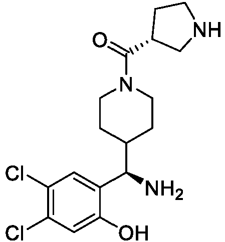 | <0.5 | >30 |
| 81** | 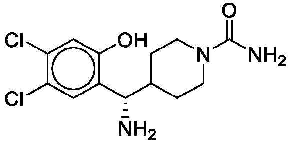 | <0.5 | >30 |
| 82** | 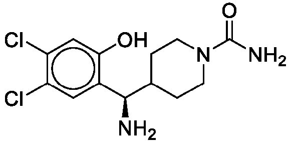 | <10 | >30 |
| 83 | 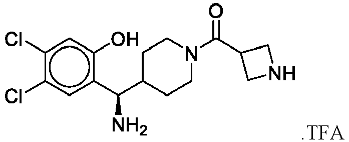 | <0.5 | >30 |
| 84 | 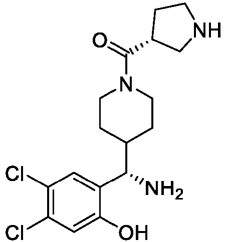 | <10 | * |
| 85 | 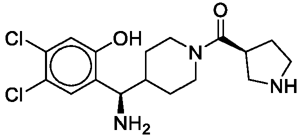 | <0.5 | >30 |
| 86** | 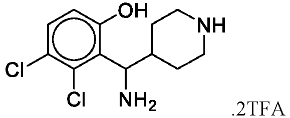 | <30 | * |
| 87 | 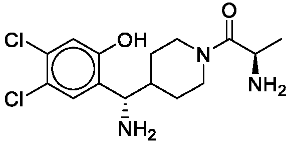 | <10 | * |
| 88 | 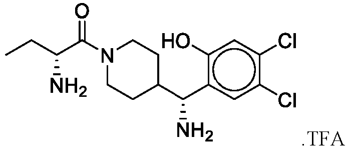 | <0.5 | * |
| 89 | 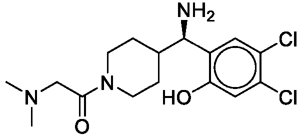 | <0.5 | >30 |
| 90 | 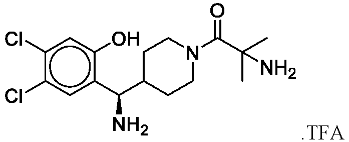 | <0.5 | * |
| 91 | 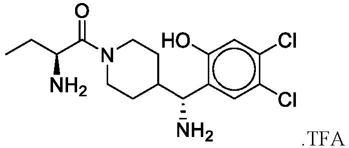 | <0.5 | * |
| 92 | 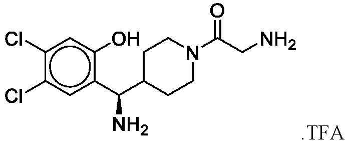 | <0.5 | >30 |
| 93 | 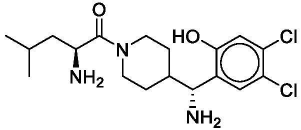 | <10 | * |
| 94 | 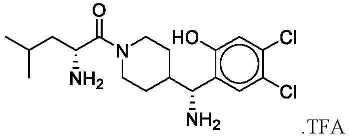 | <0.5 | * |
| 95 | 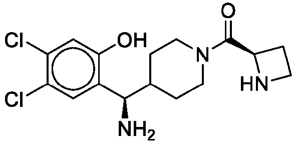 | <0.5 | >30 |
| 96 | 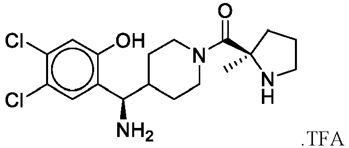 | <0.5 | >30 |
| 97 | 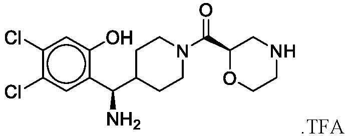 | <0.5 | >30 |
| 98 | 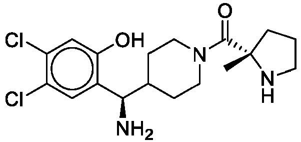 | <0.5 | >30 |
| 99 | 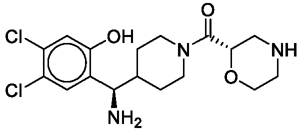 | <0.5 | >30 |
| 100 | 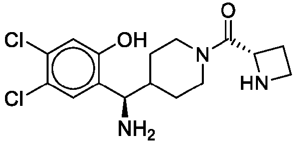 | <0.5 | >30 |
| 101 | 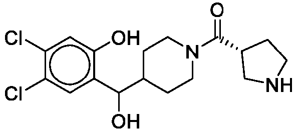 | <0.5 | >30 |
| 102 | 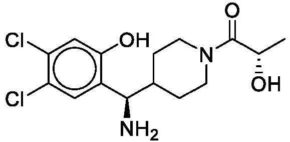 | <0.5 | >30 |
| 103 | 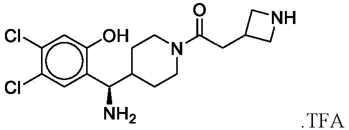 | <0.5 | >30 |
| 104 | 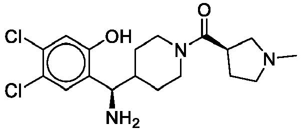 | <0.5 | >30 |
| 105 | 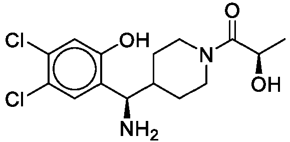 | <10 | >30 |
| 106 | 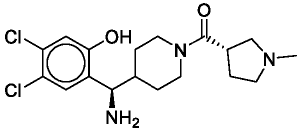 | <0.5 | >30 |
| 107 | 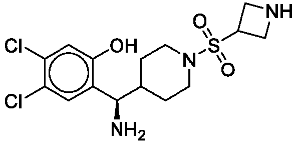 | <0.5 | >30 |
| 108 | 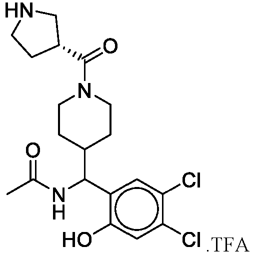 | <10 | * |
| 109 | 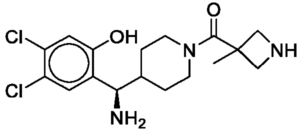 | <0.5 | >30 |
| 110 | 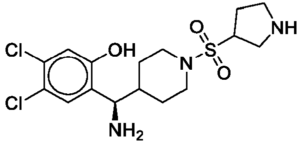 | <0.5 | >30 |
| 111** | 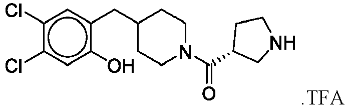 | <0.5 | <30 |
| 112 | 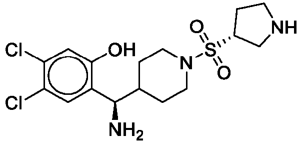 | <0.5 | >30 |
| 113 | 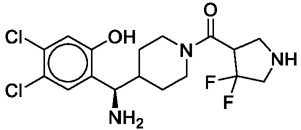 | <10 | * |
| 114 | 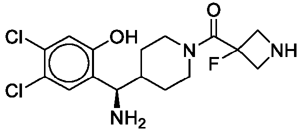 | <0.5 | >30 |
| 115 | 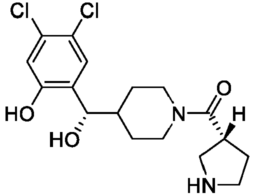 | <0.5 | >30 |
| 116 | 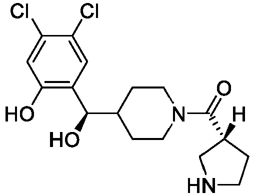 | <0.5 | >30 |
| 117 | 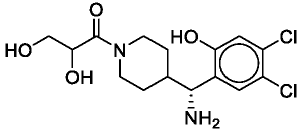 | <0.5 | >30 |
| 118 | 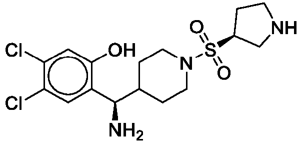 | <0.5 | >30 |
| 119 | 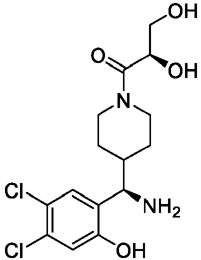 | <0.5 | >30 |
| 120 | 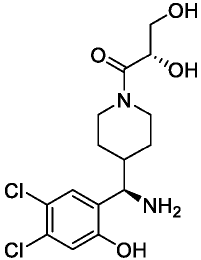 | <0.5 | >30 |
| 121 | 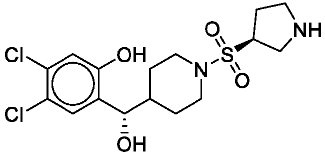 | <0.5 | >30 |
| 122** | 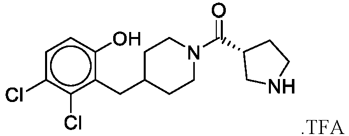 | <0.5 | <30 |
| 123 | 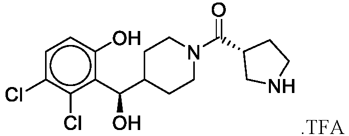 | <0.5 | >30 |
| 124 | 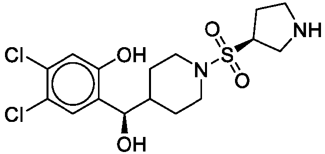 | <0.5 | * |
| 125 | 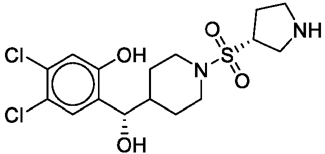 | <10 | * |
| 126 | 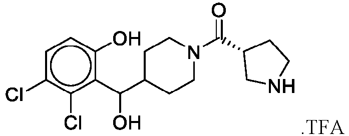 | <10 | >30 |
| 127 | 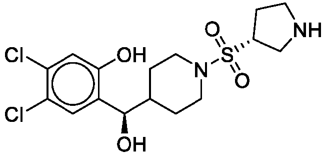 | <0.5 | * |
| 128 | 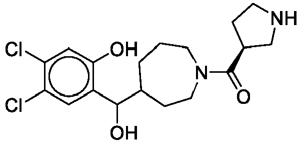 | <10 | * |
| 129 | 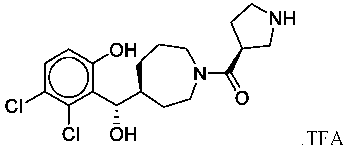 | <10 | * |
| 130 | 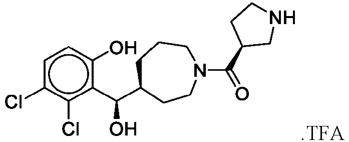 | <10 | * |
| 131 | 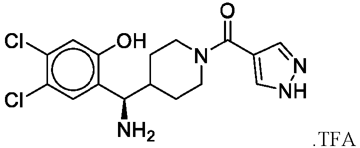 | <0.5 | >30 |
| 132 | 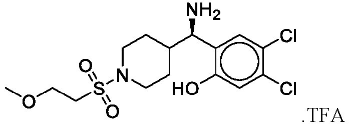 | <10 | <30 |
| 133 | 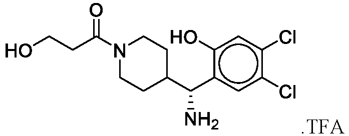 | <0.5 | >30 |
| 134 | 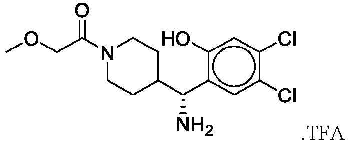 | <0.5 | <30 |
| 135 | 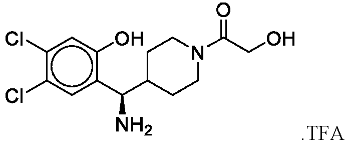 | <0.5 | >30 |
| 136 | 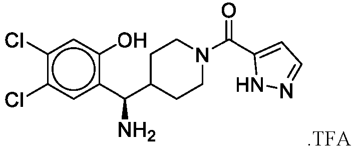 | <10 | >30 |
| 137 | 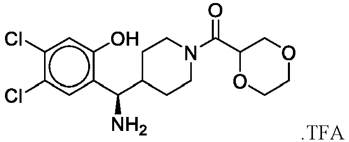 | <0.5 | <30 |
| 138 | 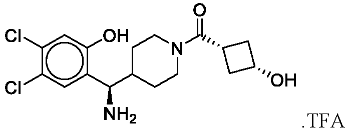 | <10 | >30 |
| 139 | 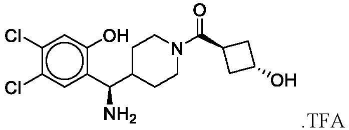 | <0.5 | >30 |
| 140 | 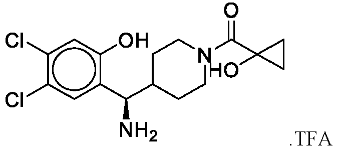 | <10 | <30 |
| 141 | 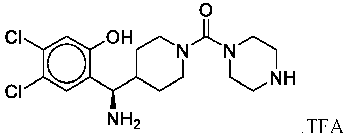 | <0.5 | >30 |
| 142 | 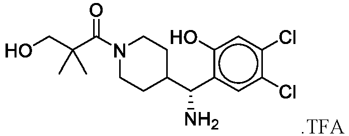 | <10 | <30 |
| 143 | 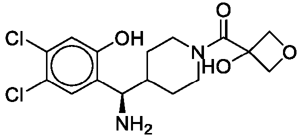 | <10 | >30 |
| 144 | 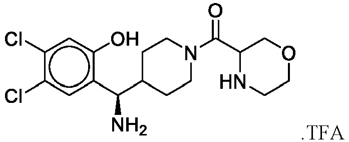 | <10 | >30 |
| 145 | 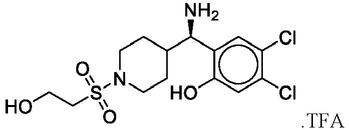 | <0.5 | <30 |
| 146 | 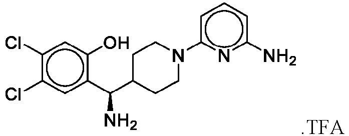 | <10 | <30 |
| 147 | 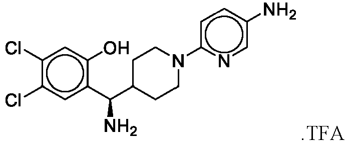 | <0.5 | <30 |
| 148 | 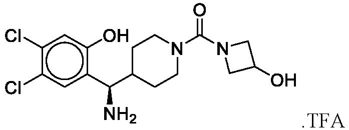 | <0.5 | >30 |
| 149 | 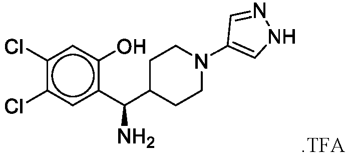 | <10 | <30 |
| 150 | 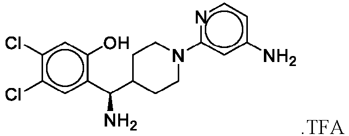 | <0.5 | <30 |
| 151 | 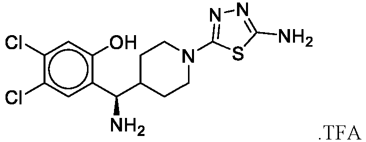 | <10 | <30 |
| 152 | 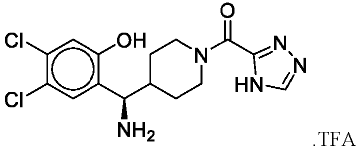 | <10 | >30 |
| 153 | 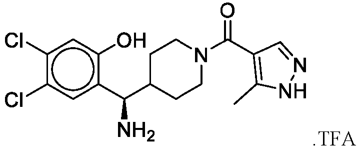 | <0.5 | >30 |
| 154 | 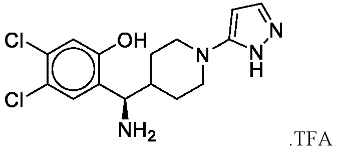 | <10 | >30 |
| 155 | 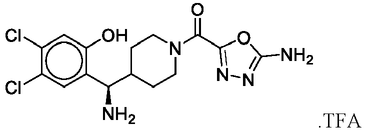 | <10 | >30 |
| 156 | 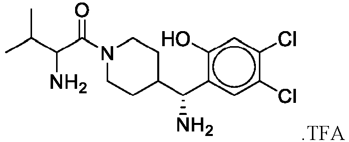 | <10 | >30 |
| 157 | 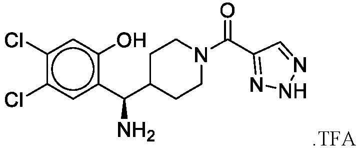 | <0.5 | >30 |
| 158 | 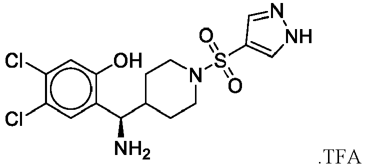 | <10 | <30 |
| 159 | 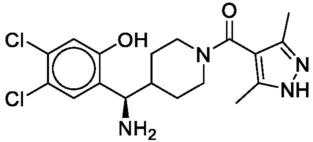 | <10 | >30 |
| 160 | 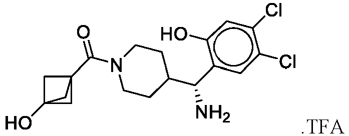 | <10 | <30 |
| 161 | 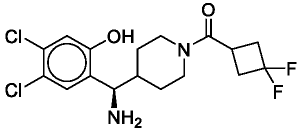 | <10 | <30 |
| 162 | 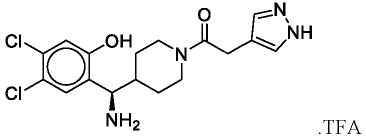 | <0.5 | >30 |
| 163 | 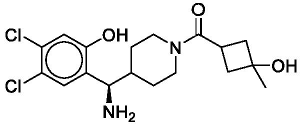 | <10 | >30 |
| 164 | 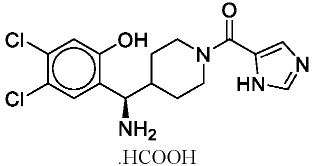 | <10 | >30 |
| 165 | 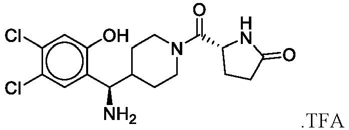 | <0.5 | >30 |
| 166 | 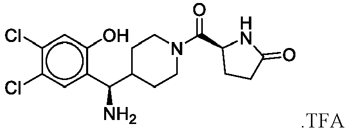 | <10 | >30 |
| 167 | 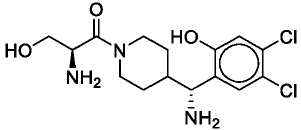 | <0.5 | >30 |
| 168 | 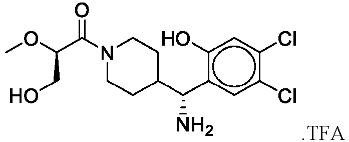 | <0.5 | >30 |
| 169 | 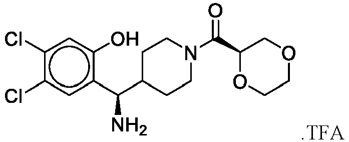 | <10 | <30 |
| 170 | 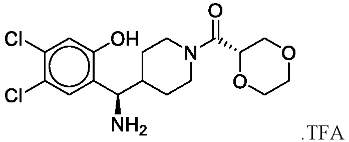 | <10 | <30 |
| 171 | 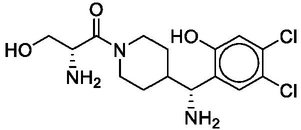 | <0.5 | >30 |
| 172 | 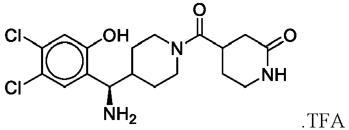 | <10 | >30 |
| 173 | 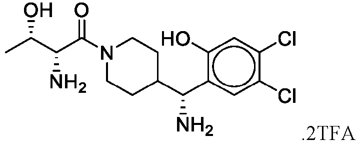 | <0.5 | >30 |
| 174 | 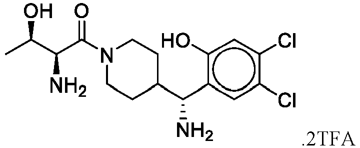 | <10 | >30 |
| 175 | 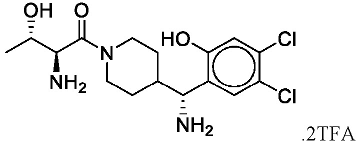 | <0.5 | >30 |
| 176 | 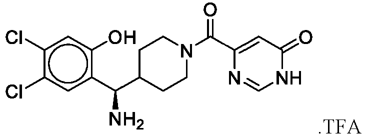 | <10 | >30 |
| 177 | 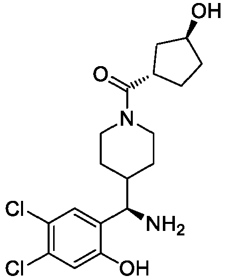 | <10 | <30 |
| 178 | 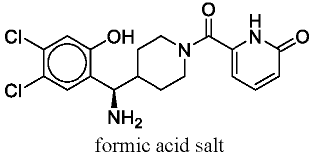 | <0.5 | >30 |
| 179 | 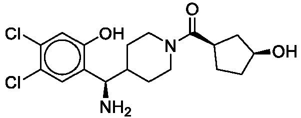 | <0.5 | <30 |
| 180 | 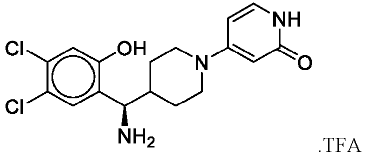 | <0.5 | >30 |
| 181 | 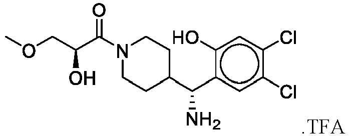 | <0.5 | >30 |
| 182 | 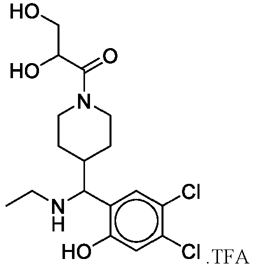 | <10 | * |
| 183 | 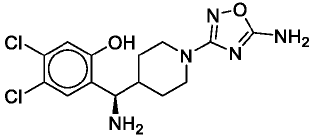 | <0.5 | <30 |
| 184 | 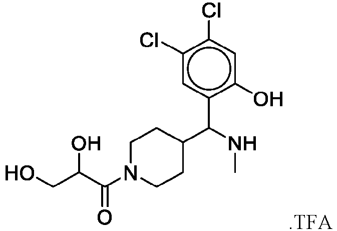 | <10 | * |
| 185 | 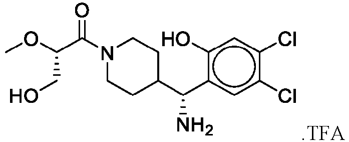 | <10 | >30 |
| 186 | 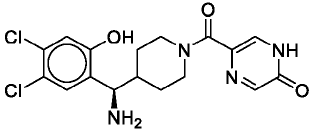 | <0.5 | >30 |
| 187 | 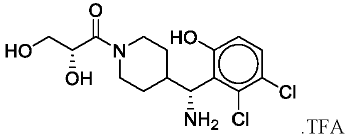 | <10 | * |
| 188 | 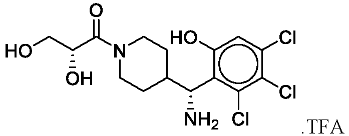 | <10 | >30 |
| 189* | 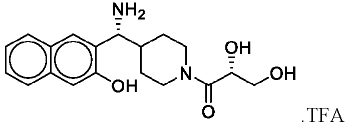 | <0.5 | >30 |
| 190 | 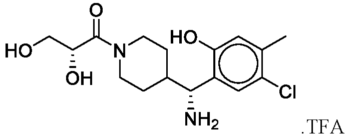 | <0.5 | >30 |
| 191** | 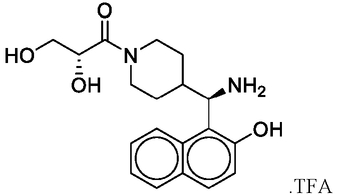 | <10 | >30 |
| 192 | 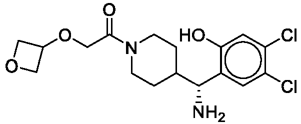 | <0.5 | >30 |
| 193 | 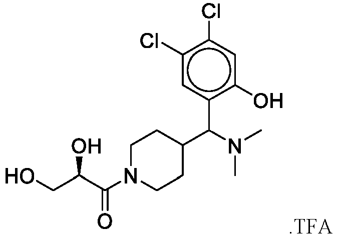 | <30 | * |
| 194 | 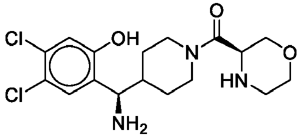 | <0.5 | >30 |
| 195 | 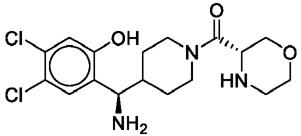 | <0.5 | >30 |
| 196 | 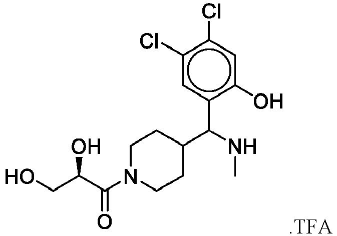 | <10 | >30 |
| 197 | 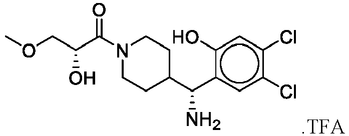 | <10 | >30 |
| 198 | 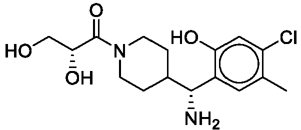 | <10 | >30 |
| 199 | 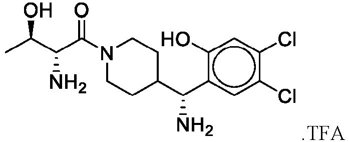 | <0.5 | >30 |
| 200 | 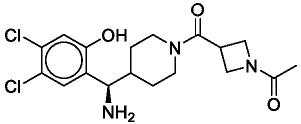 | <10 | >30 |
| 201 | 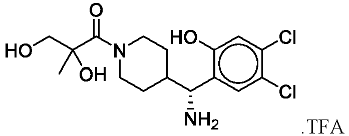 | <10 | >30 |
| 202 | 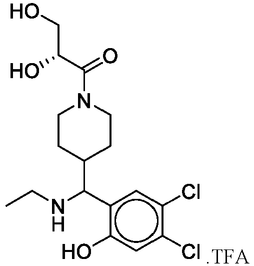 | <30 | * |
| 203 | 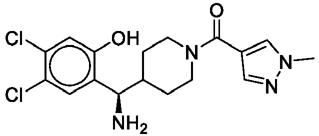 | <0.5 | <30 |
| 204 | 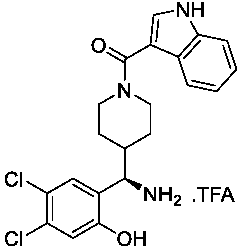 | <10 | <30 |
| 205 | 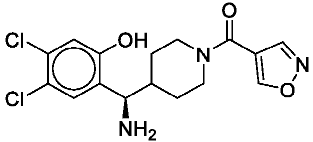 | <0.5 | >30 |
| 206** | 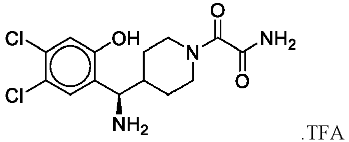 | <0.5 | >30 |
| 207 | 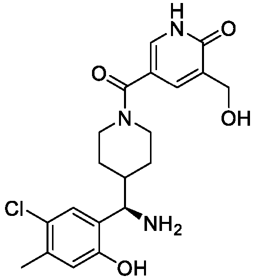 | <0.5 | >30 |
| 208 | 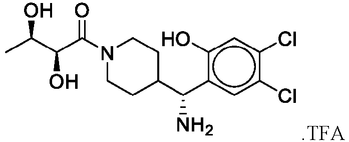 | <0.5 | >30 |
| 209** | 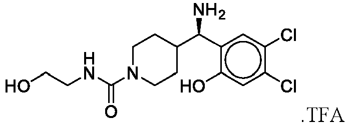 | <0.5 | >30 |
| 210 | 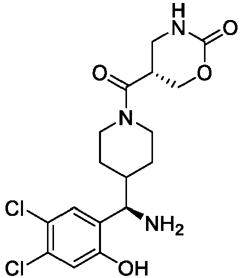 | <0.5 | >30 |
| 211 | 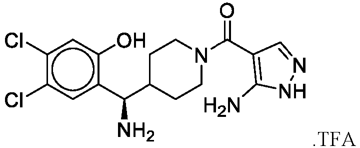 | <0.5 | >30 |
| 212 | 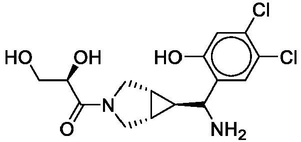 | * | * |
| 213 | 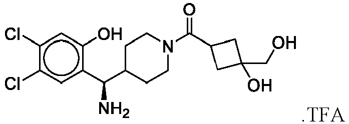 | <0.5 | >30 |
| 214* | 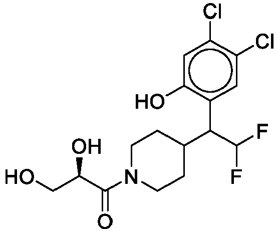 | * | * |
| 215 | 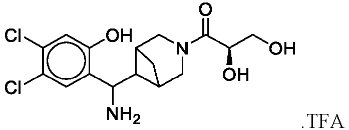 | * | * |
| 216 | 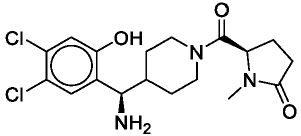 | <10 | >30 |
| 217 | 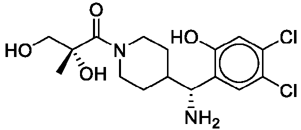 | <10 | >30 |
| 218 | 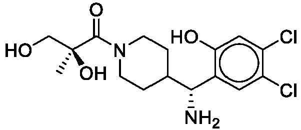 | <10 | >30 |
| 219 | 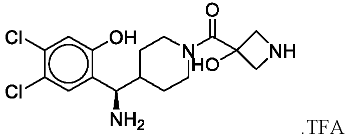 | <0.5 | >30 |
| 220 | 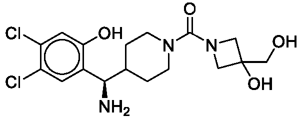 | <0.5 | >30 |
| 221 | 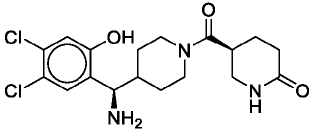 | <0.5 | >30 |
| 222 | 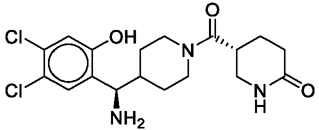 | <0.5 | >30 |
| 223 | 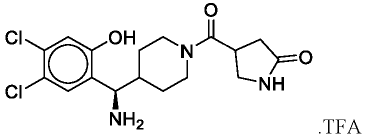 | <0.5 | >30 |
| 224 | 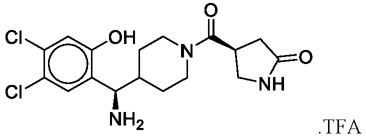 | <0.5 | >30 |
| 225 | 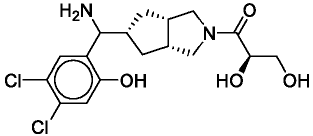 | <10 | * |
| 226** | 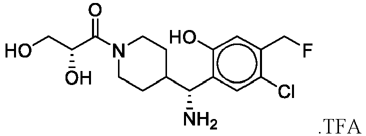 | <0.5 | >30 |
| 227 | 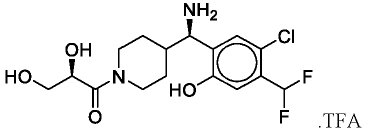 | <0.5 | * |
| 228 | 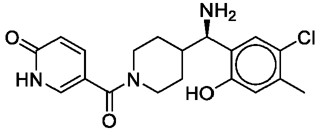 | <0.5 | >30 |
| 229 | 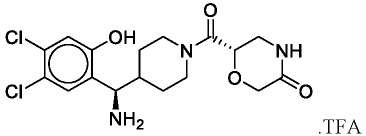 | <10 | * |
| 230 | 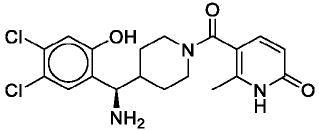 | <10 | * |
| 231 | 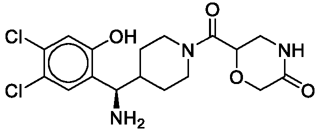 | <0.5 | >30 |
| 232 | 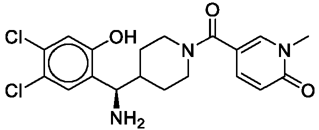 | <0.5 | >30 |
| 233 | 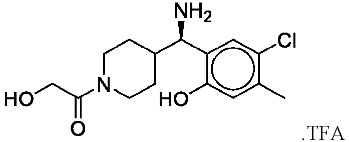 | <0.5 | >30 |
| 234 | 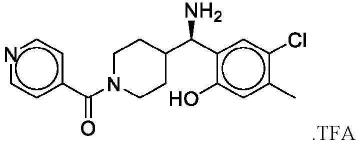 | <0.5 | * |
| 235 | 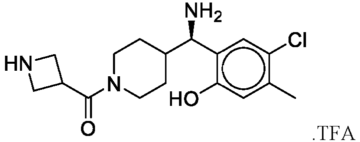 | <0.5 | >30 |
| 236 | 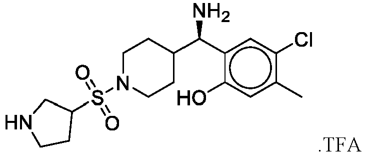 | <0.5 | >30 |
| 237 | 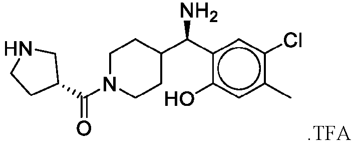 | <0.5 | >30 |
| 238 | 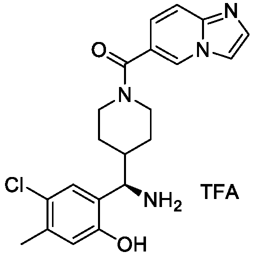 | <0.5 | <30 |
| 239 | 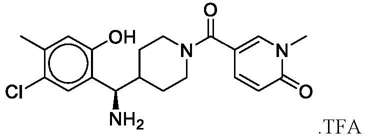 | <0.5 | >30 |
| 240 | 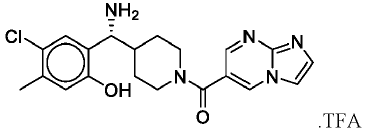 | <0.5 | >30 |
| 241 | 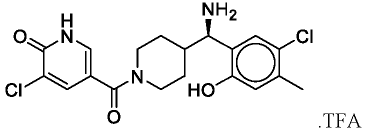 | <0.5 | >30 |
| 242 | 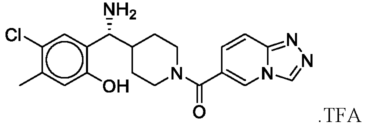 | <0.5 | >30 |
| 243 | 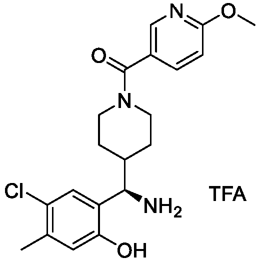 | <0.5 | <30 |
| 244 | 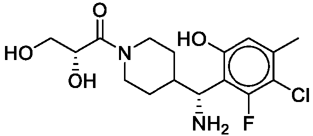 | <0.5 | >30 |
| 245 | 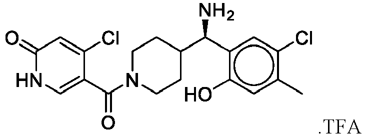 | <0.5 | >30 |
| 246 | 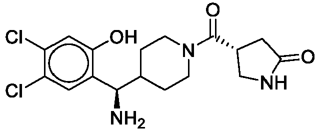 | <0.5 | >30 |
| 247 | 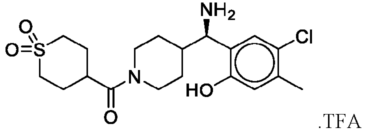 | <0.5 | >30 |
| 248 | 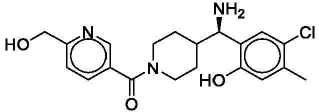 | <0.5 | >30 |
| 249 | 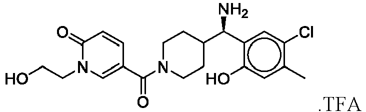 | <0.5 | >30 |
| 250 | 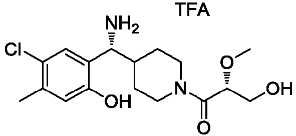 | <0.5 | * |
| 251 | 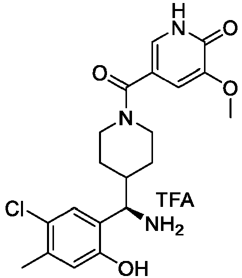 | <0.5 | * |
| 252 | 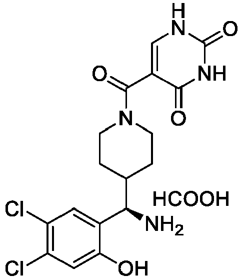 | <0.5 | * |
| 253 | 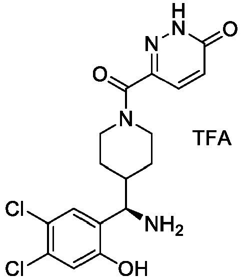 | <0.5 | * |
| 254 | 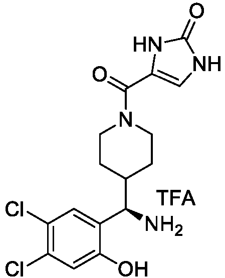 | <0.5 | * |
| 255 | 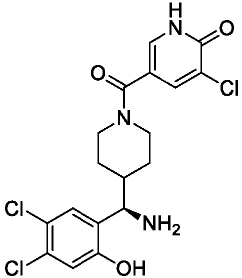 | <0.5 | * |
| 256 | 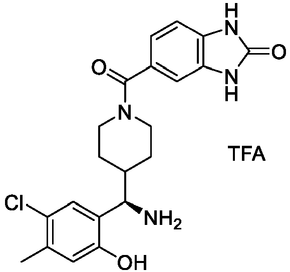 | <0.5 | * |
| 257 | 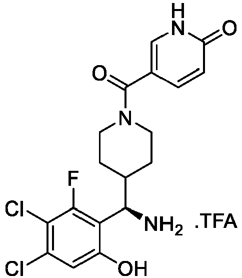 | <0.5 | >30 |
| 258 | 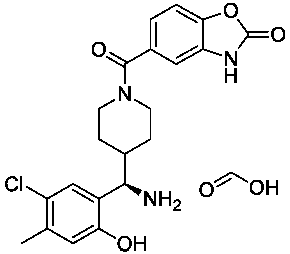 | <0.5 | * |
| 259 | 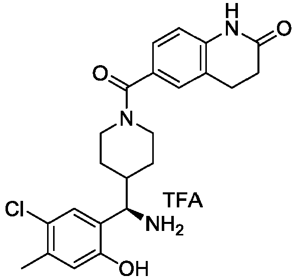 | <0.5 | * |
| 260 | 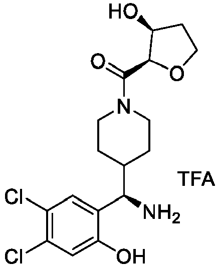 | <0.5 | >30 |
| 261 | 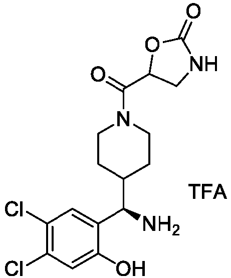 | <0.5 | * |
| 262 | 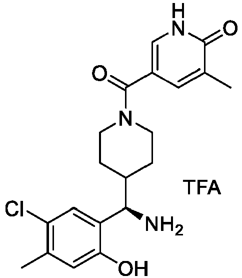 | <0.5 | >30 |
| 263 | 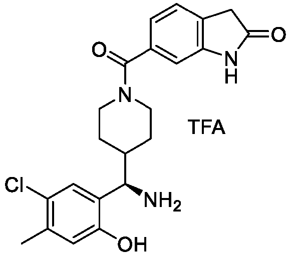 | <0.5 | * |
| 264 | 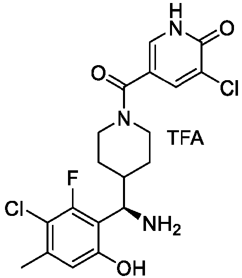 | <10 | >30 |
| 265 | 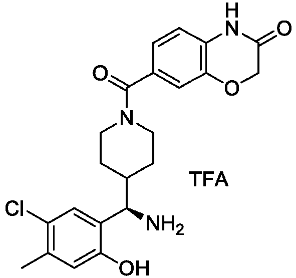 | <0.5 | * |
| 266 | 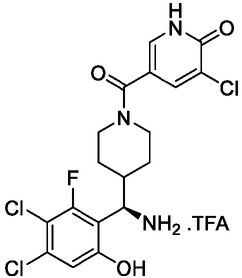 | <0.5 | * |
| 267 | 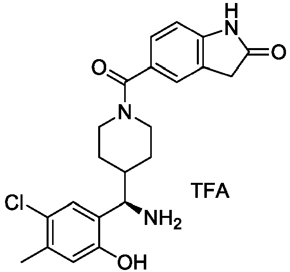 | <0.5 | * |
| 268 | 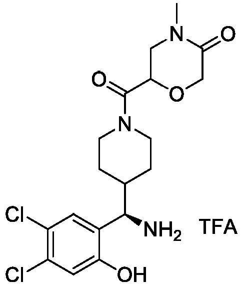 | <0.5 | * |
| 269 | 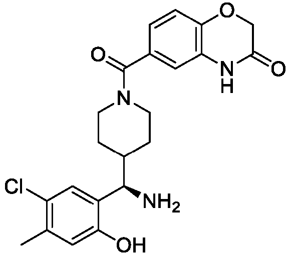 | <10 | * |
| 270 | 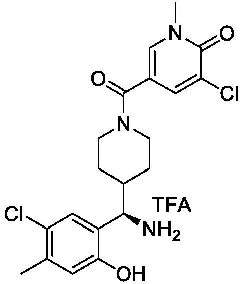 | <0.5 | * |
| 271 | 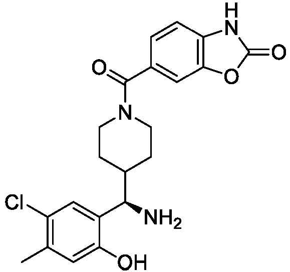 | <10 | * |
| 272 | 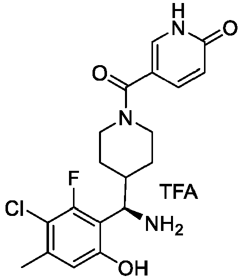 | <0.5 | >30 |
| 273 | 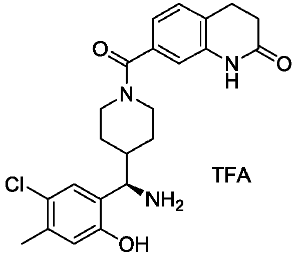 | <10 | * |
| 274 | 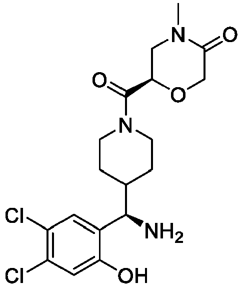 | <0.5 | * |
| 275 | 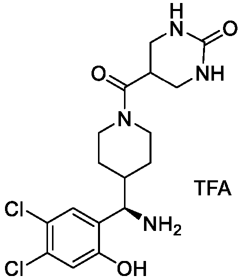 | <0.5 | >30 |
| 276 | 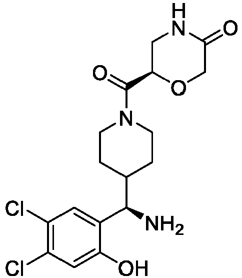 | <0.5 | >30 |
| 277 | 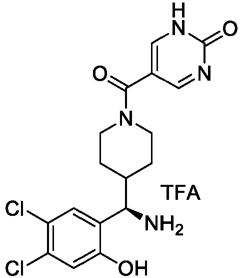 | <0.5 | >30 |
| 278 | 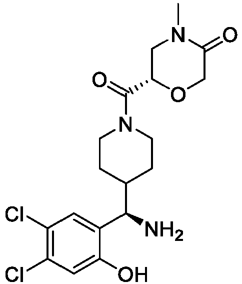 | <10 | * |
| 279 | 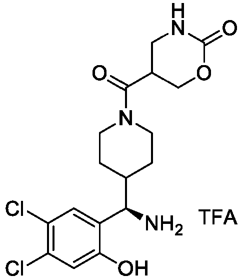 | <0.5 | >30 |
| 280 | 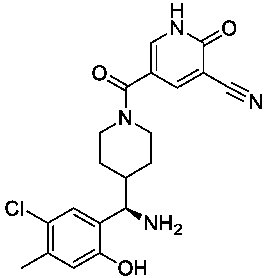 | <0.5 | >30 |
| 281 | 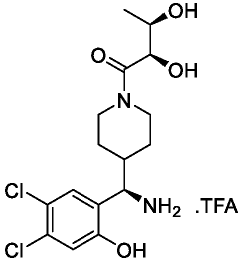 | <0.5 | >30 |
| 282** | 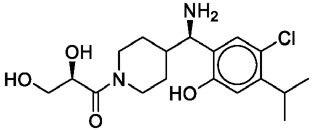 | <30 | * |
| 283 | 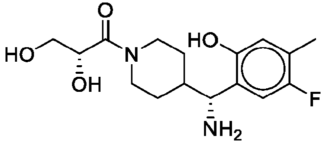 | <10 | >30 |
| 284** | 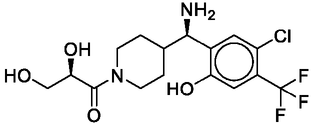 | <10 | >30 |
| 285** | 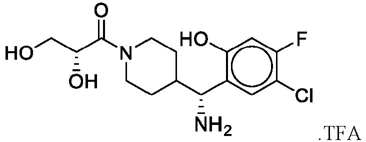 | <10 | * |
| 286** | 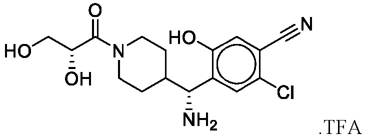 | <10 | * |
| 287 | 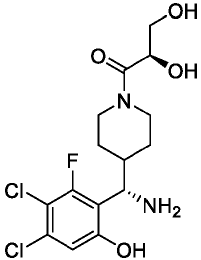 | <10 | * |
| 288** | 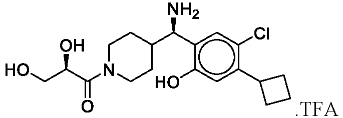 | <30 | * |
| 289** | 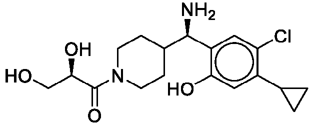 | <0.5 | >30 |
| 290** | 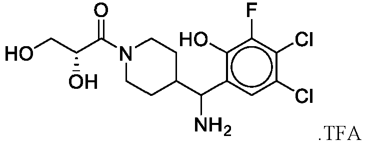 | <10 | * |
| 291 | 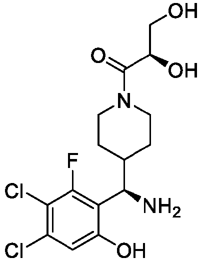 | <0.5 | >30 |
| 292** | 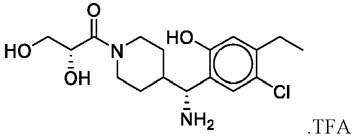 | <0.5 | >30 |
| 293** | 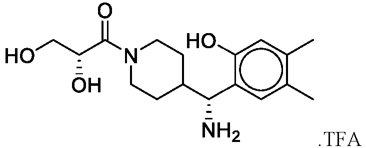 | <0.5 | >30 |
| 294** | 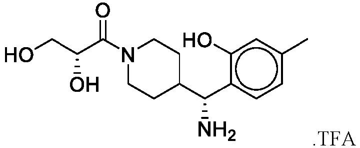 | <10 | * |
| 295** | 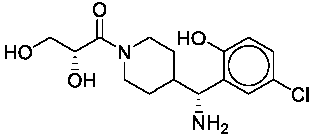 | <10 | * |
| 296 | 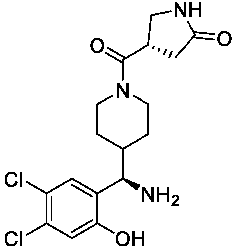 | <0.5 | >30 |
| 297 | 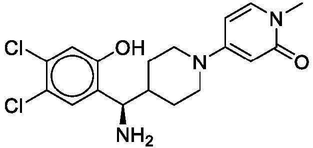 | <0.5 | <30 |
| 298 | 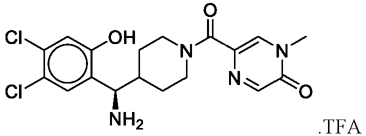 | <0.5 | <30 |
| 299 | 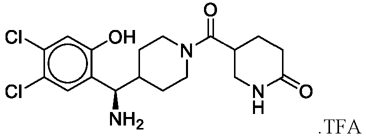 | <0.5 | >30 |
| 300 | 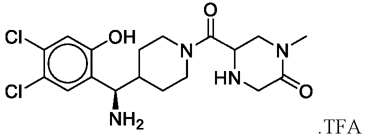 | <0.5 | >30 |
| 301 | 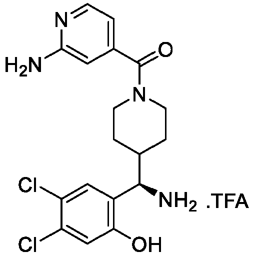 | <0.5 | >30 |
| 302 | 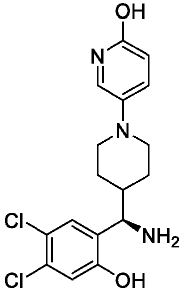 | <10 | >30 |
| 303 | 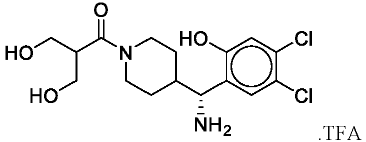 | <0.5 | >30 |
| 304 | 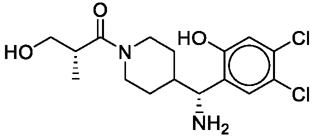 | <0.5 | >30 |
| 305 | 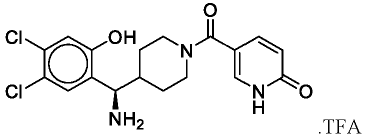 | <0.5 | >30 |
| 306 | 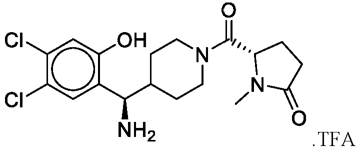 | <0.5 | >30 |
| 307 | 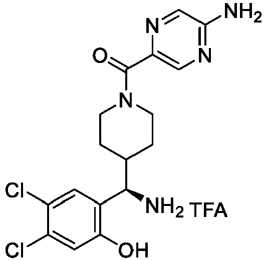 | <10 | * |
| 308 | 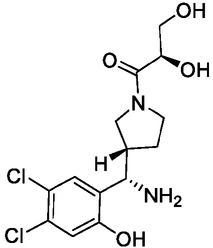 | <10 | * |
| 309 | 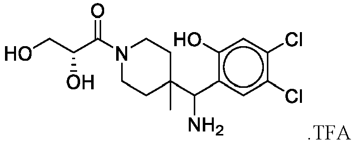 | <10 | * |
| 310 | 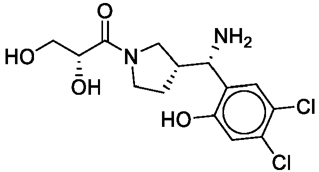 | <10 | * |
| 311 | 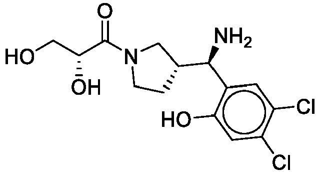 | <10 | * |
| 312 | 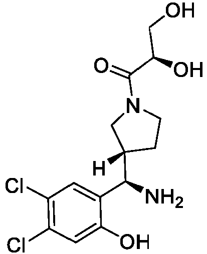 | <10 | * |
| 313 | 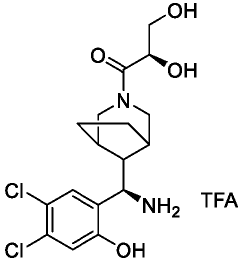 | <10 | * |
| 314 | 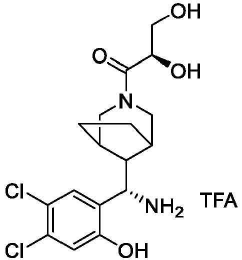 | <30 | * |
| 315 | 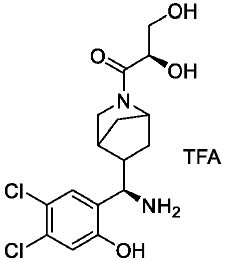 | <10 | * |
| 316 | 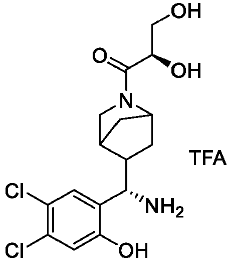 | <10 | * |
| 317** | 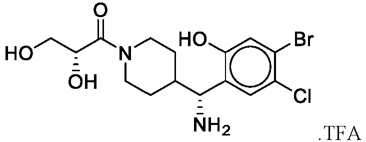 | <0.5 | >30 |
| 318** | 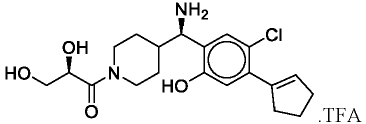 | <30 | * |
| 319** | 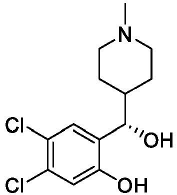 | <10 | * |
| 320 | 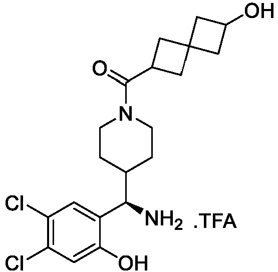 | <10 | <30 |
| 321 | 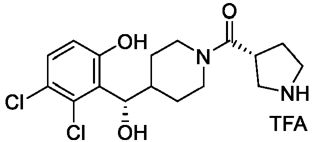 | <10 | * |
| 322 | 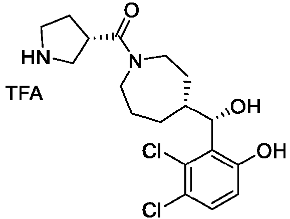 | <10 | * |
| 323 | 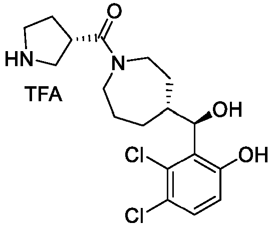 | <10 | * |
| 324 | 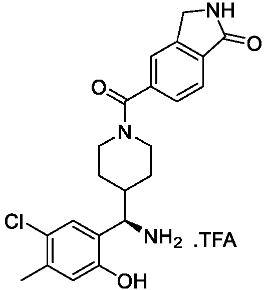 | <0.5 | >30 |
| 325 | 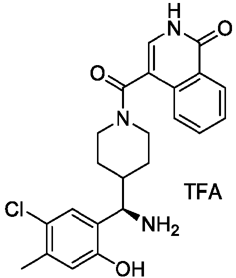 | <0.5 | * |
| 326 | 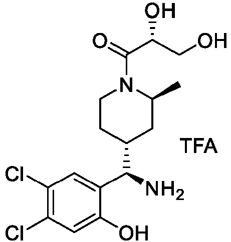 | <0.5 | >30 |
| 327 | 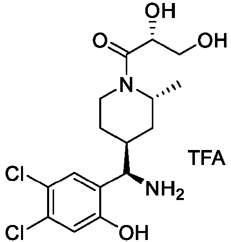 | <10 | >30 |
| 328 | 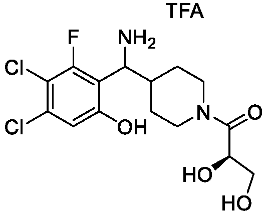 | <0.5 | >30 |
| 329 | 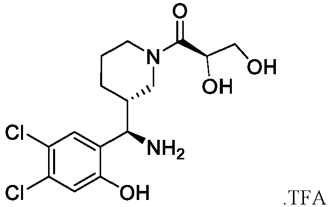 | <10 | * |
| 330 | 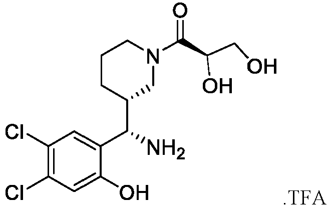 | >10 | * |
| 331 | 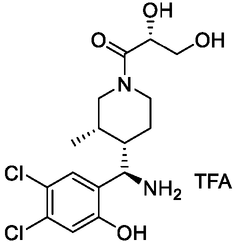 | <0.5 | >30 |
| 332 | 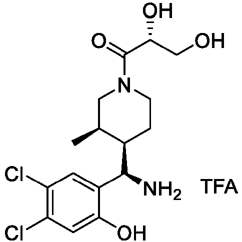 | <0.5 | >30 |
| 333 | 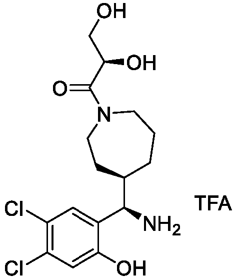 | <10 | * |
| 334 | 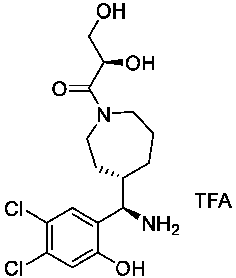 | <10 | * |
| 335 | 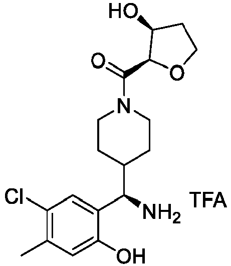 | <0.5 | >30 |
| 336 | 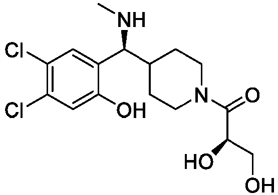 | <10 | * |
| 337 | 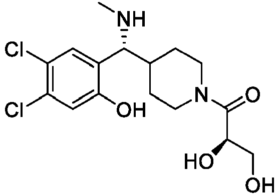 | <10 | * |
| 338 | 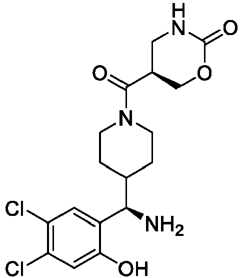 | <0.5 | >30 |

| | | | |
|---|---|---|---|
| *Not Tested. | | | |

## Claims

1. A compound of Formula I or a pharmaceutically acceptable salt thereof, 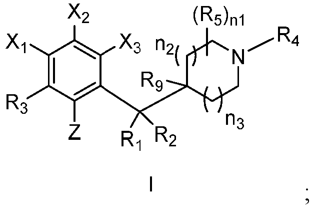 wherein
the structural moiety 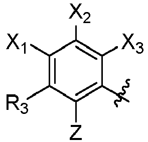 has the structure of 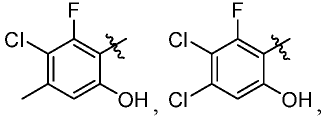 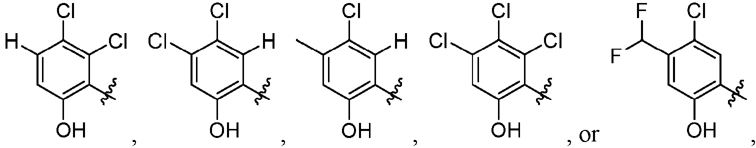 each of which is substituted by R₃;
R₃ is H, halogen, or alkyl;
R₁ and R₂ are each independently H, alkyl, (CR₆R₇)ₙ₄ORₐ, (CR₆R₇)ₙ₄NRₐR_{b}, (CR₆R₇)ₙ₄NRₐ(C=O)R_{b}, (CR₆R₇)ₙ₄NRₐSO₂R_{b}, or (CR₆R₇)ₙ₄CONRₐR_{b}, wherein at least one occurrence of R₁ and R₂ is (CR₆R₇)ₙ₄NRₐR_{b}, (CR₆R₇)ₙ₄ORₐ, (CR₆R₇)ₙ₄NRₐ(C=O)R_{b}, (CR₆R₇)ₙ₄NRₐSO₂R_{b}, or (CR₆R₇)ₙ₄CONRₐR_{b}; or alternatively R₁, R₂ and the carbon atom they are connected to taken together form a 3-5 membered carbocycle;
R₄ is (CR₆R₇)ₙ₄(C=O)R_{c}, (C=O)(CR₆R₇)ₙ₄R_{c}, (CR₆R₇)ₙ₄OR_{c}, (CR₆R₇)ₙ₄COOR_{c}, (CR₆R₇)ₙ₄NR_{c}(C=O)R_{d}, (C=O)(CR₆R₇)ₙ₄OR_{c}, (CR₆R₇)ₙ₄SO₂R_{c}, optionally substituted saturated heterocycle, optionally substituted aryl, optionally substituted heteroaryl, optionally substituted cycloalkyl, or optionally substituted -cycloalkyl-alkyl;
each occurrence of R₅ is independently H, alkyl, cycloalkyl, or oxo;
or two R₅ groups taken together with the carbon atom(s) that they are connected to form a 3-7 membered optionally substituted saturated carbocycle;
or two R₅ groups are connected to different carbon atoms on the ring and taken together form a bond or an alkyl chain containing 1-3 carbons;
each occurrence of R₆ and R₇ are independently H, alkyl, or cycloalkyl;
each occurrence of Rₐ and R_{b} are independently H, alkyl, cycycloalkyl, saturated heterocycle, aryl, or heteroaryl; or alternatively Rₐ and R_{b} together with the nitrogen atom that they are connected to form an optionally substituted heterocycle comprising the nitrogen atom and 0-3 additional heteroatoms each selected from the group consisting of N, O, and S;
each occurrence of R_{c} and R_{d} are independently H, alkyl, alkyl substituted by 1-4 substituents each of which is independently halogen, OR₈ or N(R₈)₂, alkenyl, optionally substituted cycloalkyl, optionally substituted bicycloalkyl, optionally substituted spiroalkyl, optionally substituted saturated heterocycle, optionally substituted aryl, optionally substituted heteroaryl, optionally substituted -alkyl-aryl, optionally substituted -alkyl-heteroaryl, optionally substituted -alkyl-heterocycle, optionally substituted -alkyl-cycloalkyl, or optionally substituted -cycloalkyl-alkyl; or alternatively R_{c} and R_{d} together with the nitrogen atom that they are connected to form an optionally substituted heterocycle comprising the nitrogen atom and 0-3 additional heteroatoms each selected from the group consisting of N, O, and S;
each occurrence of R₈ is independently H, alkyl, or an optionally substituted heterocycle; or alternatively the two R₈ groups together with the nitrogen atom that they are connected to form an optionally substituted heterocycle comprising the nitrogen atom and 0-3 additional heteroatoms each selected from the group consisting of N, O, and S;
R₉ is H, alkyl, halogen, or (CR₆R₇)ₙ₄OR_{b};
the alkyl, cycloalkyl, spiroalkyl, bicycloalkyl, heterocycle, aryl, and heteroaryl in R₁, R₂, R₃, R₄, R₅, R₆, R₇, R₉, Rₐ, R_{b}, R_{c}, and R_{d}, where applicable, are optionally substituted by 1-4 substituents each independently selected from the group consisting of alkyl, cycloalkyl, halogenated cycloalkyl, halogenated alkyl, halogen, CN, -(CH₂)₀₋₂OR₈, N(R₈)₂, (C=O)C₁₋₄alkyl, (C=O)N(R₈)₂, and oxo where valence permits;
each occurrence of n₁ is independently an integer from 0-3 where valence permits;
each occurrence of n₂ and n₃ is independently an integer that is 1, 2 or 0; and
each occurrence of n₄ is independently an integer from 0-3.

2. The compound or pharmaceutically acceptable salt thereof of claim 1, wherein:
(a) each occurrence of n₂ and n₃ is independently an integer from 0-1; or
(b) the structural motif 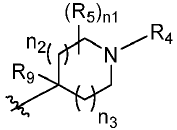 has the structure of 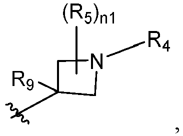 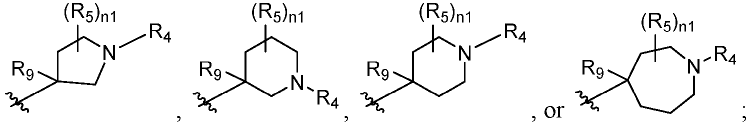 or
(c) the structural motif 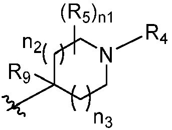 has the structure of

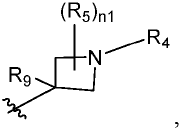

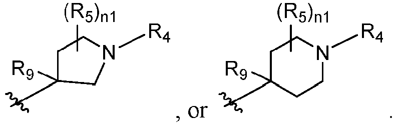

3. The compound or pharmaceutically acceptable salt thereof of claim 1 or 2, wherein the structural motif 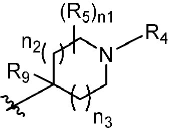 has the structure of

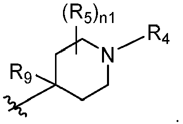

4. The compound or pharmaceutically acceptable salt thereof of any one of claims 1-3, wherein at least one occurrence of R₁ and R₂ is (CR₆R₇)ₙ₄ORₐ, (CR₆R₇)ₙ₄NRₐR_{b}, (CR₆R₇)ₙ₄NRₐ(C=O)R_{b}, (CR₆R₇)ₙ₄NRₐSO₂R_{b}, or (CR₆R₇)ₙ₄CONRₐR_{b}.

5. The compound or pharmaceutically acceptable salt thereof of claim 4, wherein at least one occurrence of R₁ and R₂ is ORₐ or NRₐR_{b}.

6. The compound or pharmaceutically acceptable salt thereof of any one of claims 1-3, wherein R₁, R₂, and the carbon atom they are connected to taken together form a 3-5 membered carbocycle.

7. The compound or pharmaceutically acceptable salt thereof of any one of claims 1-6, wherein R₄ is (CR₆R₇)ₙ₄OR_{c}, (CR₆R₇)ₙ₄COR_{c}, (C=O)(CR₆R₇)ₙ₄R_{c}, (CR₆R₇)ₙ₄COOR_{c}, (CR₆R₇)ₙ₄NR_{c}(C=O)R_{d}, (C=O)(CR₆R₇)ₙ₄OR_{c}, or (CR₆R₇)ₙ₄SO₂R_{c}.

8. The compound or pharmaceutically acceptable salt thereof of claim 7, wherein R₄ is (CR₆R₇)₂OR_{c}, (C=O)R_{c}, (C=O)(CR₆R₇)₁₋₂R_{c}, COOR_{c}, (CR₆R₇)₁₋₂NR_{c}(C=O)R_{d}, (C=O)(CR₆R₇)₁₋₂OR_{c}, or SO₂R_{c}.

9. The compound or pharmaceutically acceptable salt thereof of claim 8, wherein R₄ is (CH₂)₂OH, (CH₂)₂OMe, (C=O)H, (C=O)Me, (C=O)CH₂OH, (C=O)CH₂OMe, (C=O)Et, (C=O)Ph, (C=O)isopropyl, (C=O)CH₂NH₂, (C=O)CH₂NHMe, (C=O)CH(OH)CH₂OH, (C=O)CH(OMe)CH₂OH, (C=O)CH(OH)CH₂OMe, (C=O)OMe, SO₂Me, SO₂Et, SO₂CH₂OH, or SO₂CH₂OMe.

10. The compound or pharmaceutically acceptable salt thereof of claim 8, wherein R₄ is (C=O)R_{c}, (C=O)(CR₆R₇)₁₋₂R_{c}, (C=O)(CR₆R₇)₁₋₂OR_{c}, or SO₂R_{c}; and wherein R_{c} is selected from the group consisting of H, alkyl, alkyl substituted by 1-4 substituents each independently selected from the group consisting of halogen, OR₈ and N(R₈)₂, alkenyl, optionally substituted cycloalkyl, optionally substituted bicycloalkyl, optionally substituted spiroalkyl, optionally substituted saturated heterocycle, optionally substituted aryl, optionally substituted heteroaryl, optionally substituted -alkyl-aryl, optionally substituted -alkyl-heteroaryl, optionally substituted -alkyl-heterocycle, optionally substituted -alkyl-cycloalkyl, and optionally substituted - cycloalkyl-alkyl.

11. The compound or pharmaceutically acceptable salt thereof of any one of claims 1-6, wherein R₄ is (CR₆R₇)₂OR_{c}, (C=O)R_{c}, (C=O)(CR₆R₇)₁₋₂R_{c}, COOR_{c}, (CR₆R₇)₁₋₂NR_{c}(C=O)R_{d}, (C=O)(CR₆R₇)₁₋₂OR_{c}, or SO₂R_{c}, and:
(a) R_{c} or R_{d} is H, Me, Et, 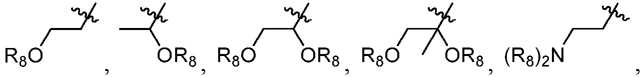 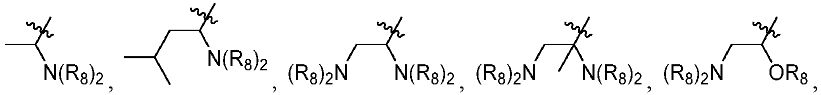 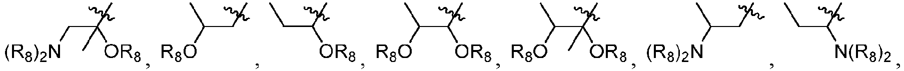 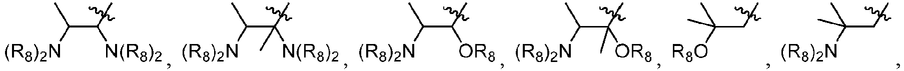 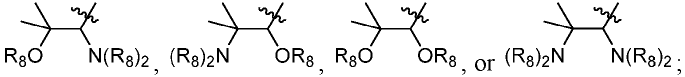 or
(b) R_{c} or R_{d} is a heterocycle selected from the group consisting of 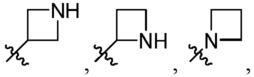 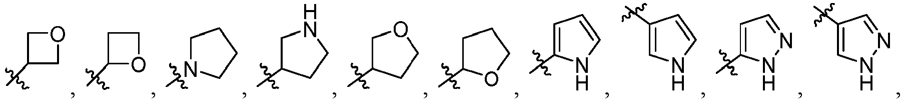 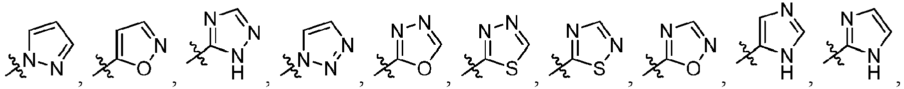 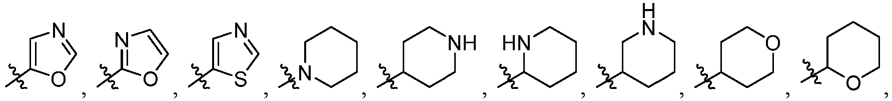 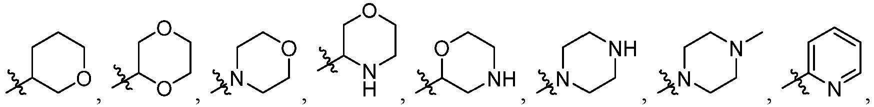 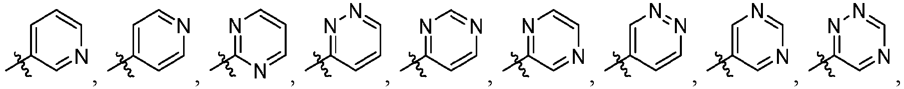 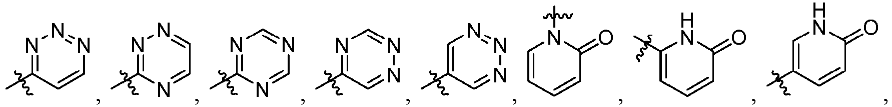 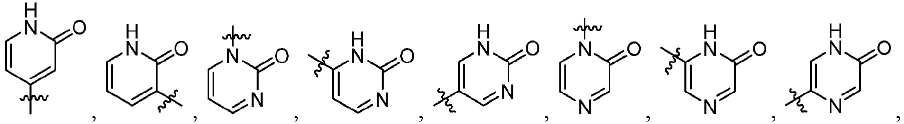 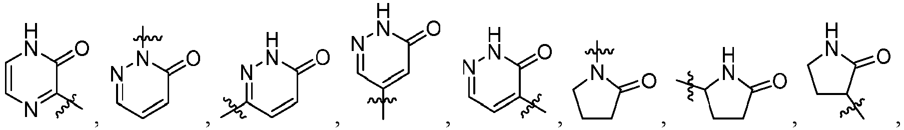 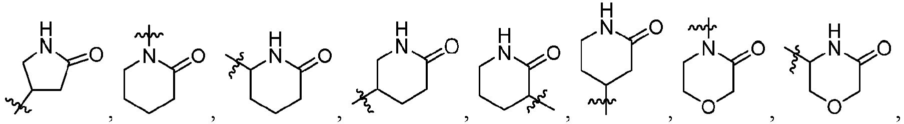 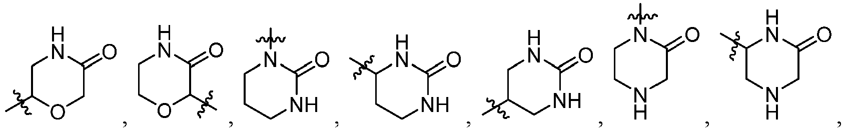 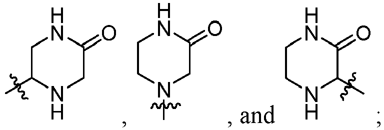 wherein the heterocycle is optionally substituted by one or more cyano, cycloalkyl, fluorinated alkyl, fluorinated cycloalkyl, halogen, OH, NH₂, oxo, or (C=O)C₁₋₄alkyl where valence permits; or
(c) R_{c} is cycloalkyl, spiroalkyl, or bicycloalkyl each optionally substituted by 1-4 substituents each independently selected from the group consisting of alkyl, halogen, CN, - (CH₂)₀₋₂OR₈, N(R₈)₂, (C=O)N(R₈)₂, and oxo where valence permits; or
(d) R_{c} is 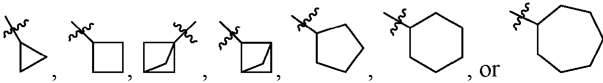 each optionally substituted by 1-4 substituents each independently selected from the group consisting of alkyl, halogen, CN, -(CH₂)₀₋₂OR₈, N(R₈)₂, (C=O)N(R₈)₂, and oxo where valence permits.

12. The compound or pharmaceutically acceptable salt thereof of any one of claims 1-6, wherein:
(a) R₄ is optionally substituted saturated heterocycle, optionally substituted aryl, optionally substituted heteroaryl, optionally substituted cycloalkyl, or optionally substituted -cycloalkyl-alkyl; or
(b) R₄ is a heterocycle selected from the group consisting of 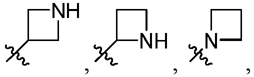 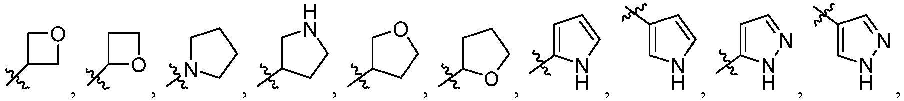 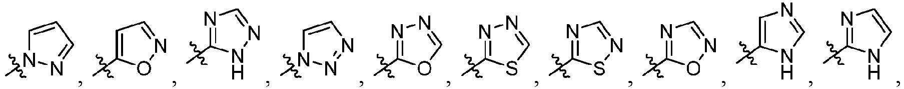 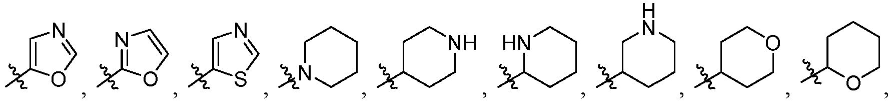 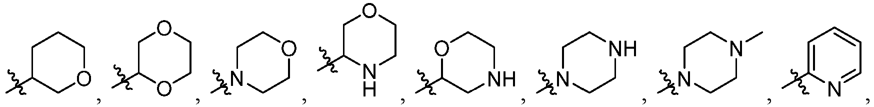 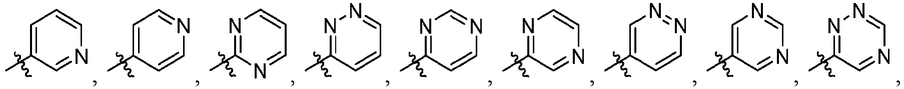 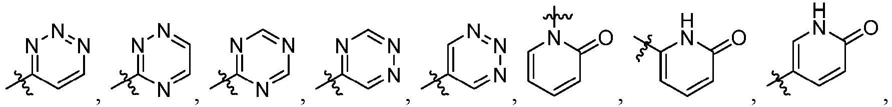 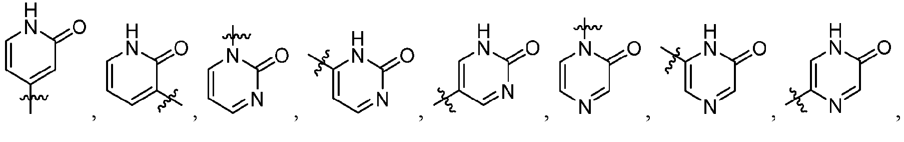 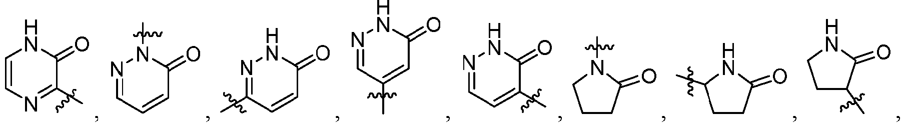 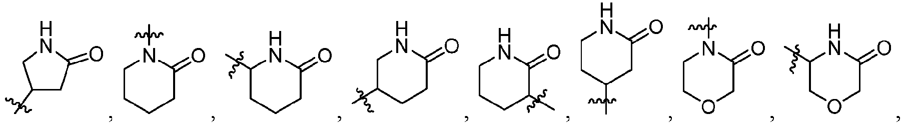 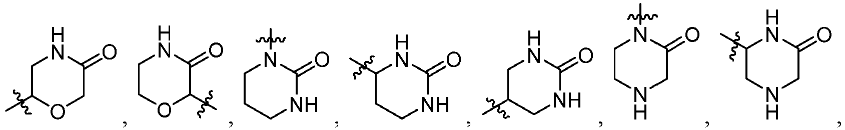 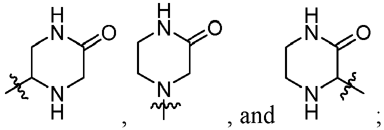 wherein the heterocycle is optionally substituted by one or more cyano, cycloalkyl, fluorinated alkyl, fluorinated cycloalkyl, halogen, OH, NH₂, oxo, or (C=O)C₁₋₄alkyl where valence permits; or
(c) R₄ is cycloalkyl optionally substituted by 1-4 substituents each independently selected from the group consisting of alkyl, halogen, CN, -(CH₂)₀₋₂OR₈, N(R₈)₂, (C=O)N(R₈)₂, and oxo where valence permits; or
(d) R₄ is 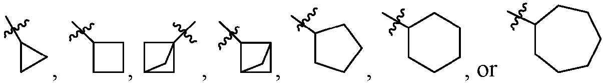 each optionally substituted by 1-4 substituents each independently selected from the group consisting of alkyl, halogen, CN, -(CH₂)₀₋₂OR₈, N(R₈)₂, (C=O)N(R₈)₂, and oxo where valence permits.

13. The compound or pharmaceutically acceptable salt thereof of any one of claims 1-6, wherein R₄ is 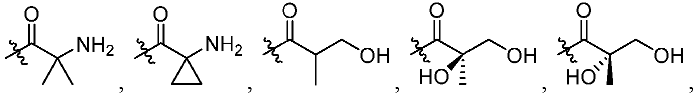 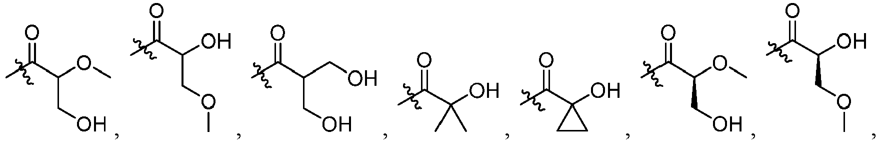 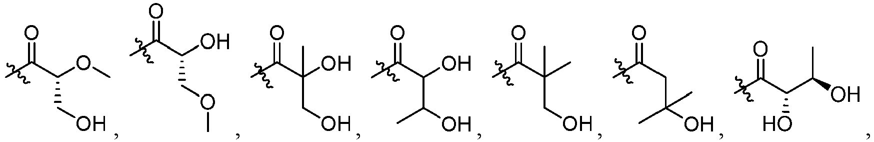 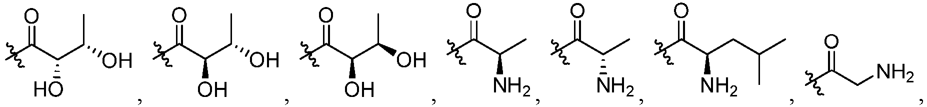 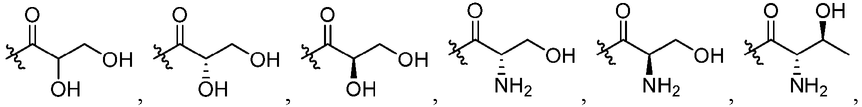 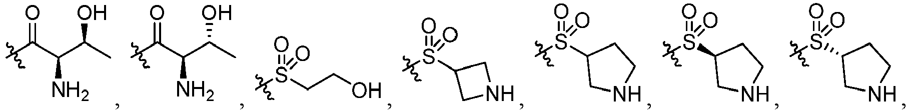 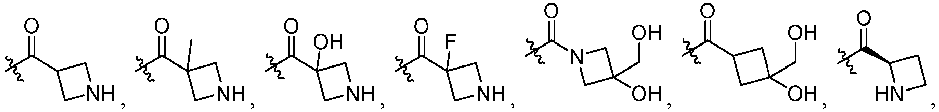 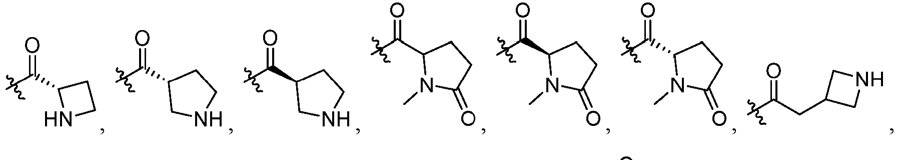 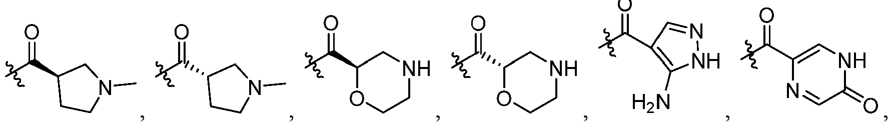 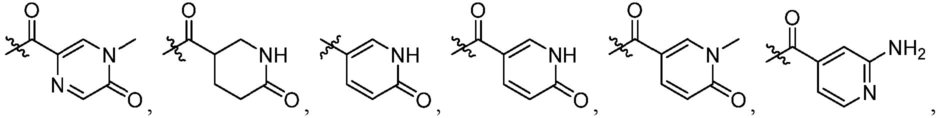 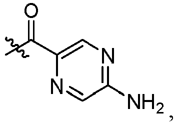 or a tautomer thereof.

14. The compound or pharmaceutically acceptable salt thereof of any one of claims 1-13, wherein:
(a) at least one occurrence of R₅ is H, alkyl or cycloalkyl; or
(b) at least one occurrence of R₅ is oxo; or
(c) two R₅ groups are connected to different carbon atoms on the ring and taken together form a bond or an alkyl chain containing 1-3 carbons; or
(d) two R₅ groups taken together with the carbon atom(s) that they are connected to form a 3-7 membered optionally substituted saturated carbocycle.

15. The compound or pharmaceutically acceptable salt thereof of any one of the preceding claims, wherein:
(a) at least one of Rₐ and R_{b} is independently H, alkyl, cycloalkyl, saturated heterocycle, aryl, or heteroaryl; or
(b) Rₐ and R_{b} together with the nitrogen atom that they are connected to form an optionally substituted heterocycle comprising the nitrogen atom and 0-3 additional heteroatoms each selected from the group consisting of N, O, and S.

16. The compound or pharmaceutically acceptable salt thereof of any one of the preceding claims, wherein:
(a) each occurrence of R₆ and R₇ are independently H or alkyl; and/or
(b) R₉ is (i) H, alkyl, or halogen, or (ii) (CR₆R₇)ₙ₄OR_{b}; or (iii) H, F, or OH.

17. The compound or pharmaceutically acceptable salt thereof of any one of claims 1-16, wherein R₃ is H or alkyl.

18. The compound or pharmaceutically acceptable salt thereof of any one of claims 1-16, wherein R₃ is halogen.

19. The compound or pharmaceutically acceptable salt thereof of any one of the preceding claims, wherein:
(a) at least one occurrence of R₈ is H, alkyl, or optionally substituted heterocycle, optionally wherein R₈ is H, Me, Et, Pr, Bu, or a heterocycle selected from the group consisting 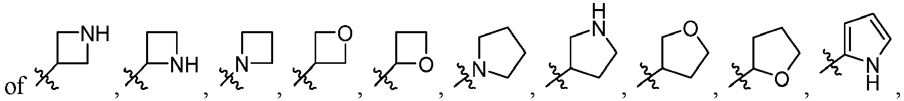 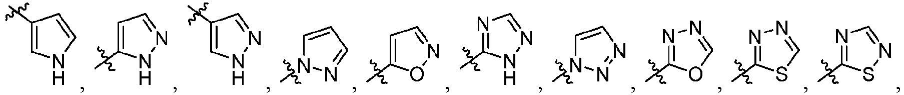 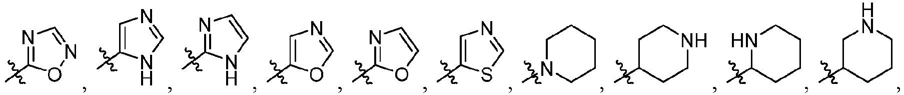 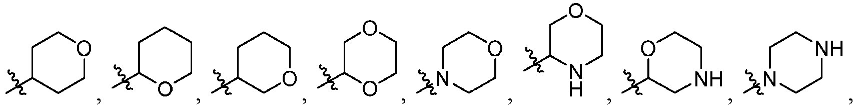 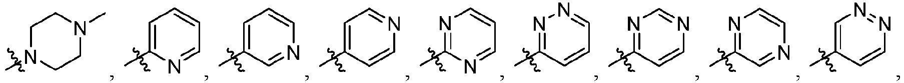 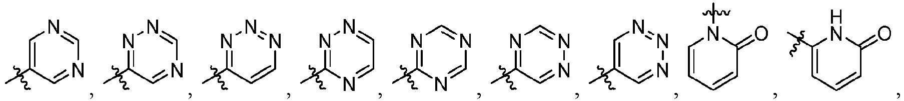 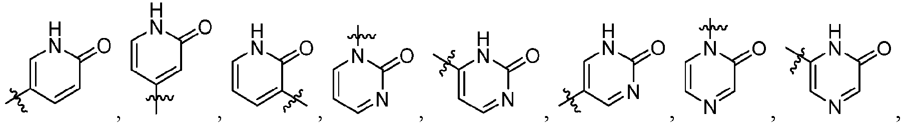 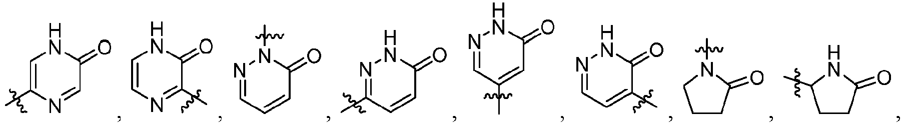 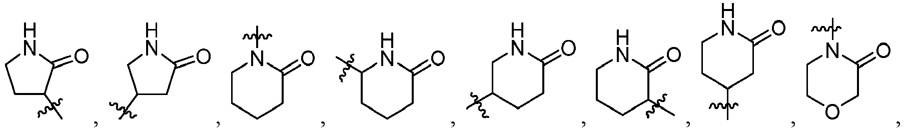 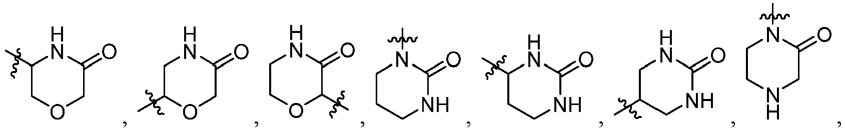 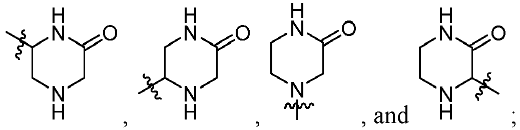 wherein the heterocycle is optionally substituted by one or more cyano, cycloalkyl, fluorinated alkyl, fluorinated cycloalkyl, halogen, OH, NH₂, oxo, or (C=O)C₁₋₄alkyl where valence permits; or
(b) the two R₈ groups together with the nitrogen atom that they are connected to form an optionally substituted heterocycle comprising the nitrogen atom and 0-3 additional heteroatoms each selected from the group consisting of N, O, and S.

20. The compound or pharmaceutically acceptable salt thereof of any one of claim 1-6, wherein:
(a) at least one occurrence of R_{c} or R_{d} is independently H, alkyl, alkyl substituted by 1-4 substituents each independently selected from the group consisting of halogen, OR₈ and N(R₈)₂, alkenyl, optionally substituted cycloalkyl, optionally substituted bicycloalkyl, optionally substituted spiroalkyl, optionally substituted saturated heterocycle, optionally substituted aryl, optionally substituted heteroaryl, optionally substituted -alkyl-aryl, optionally substituted -alkyl-heteroaryl, optionally substituted -alkyl-heterocycle, optionally substituted -alkyl-cycloalkyl, or optionally substituted -cycloalkyl-alkyl; or
(b) at least one occurrence of R_{c} or R_{d} is independently H, Me, Et, 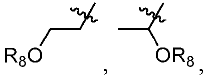 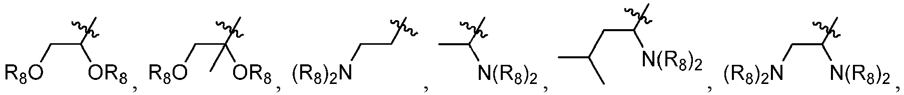 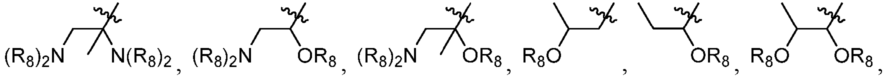 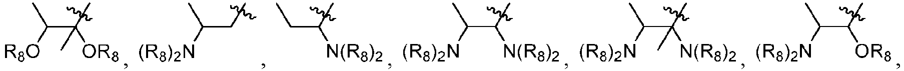 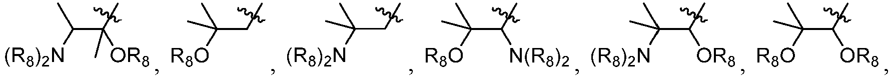 or

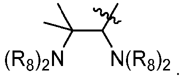

21. The compound or pharmaceutically acceptable salt thereof of claim 1, wherein the compound is selected from the group consisting of the following compounds and pharmaceutically acceptable salts thereof

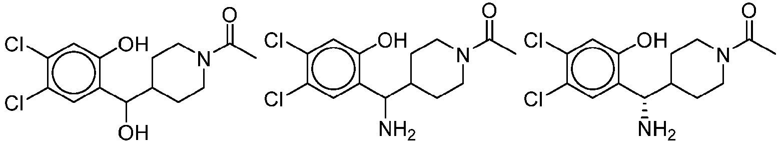

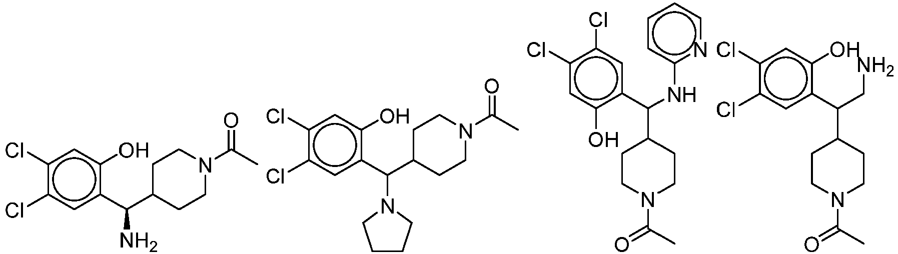

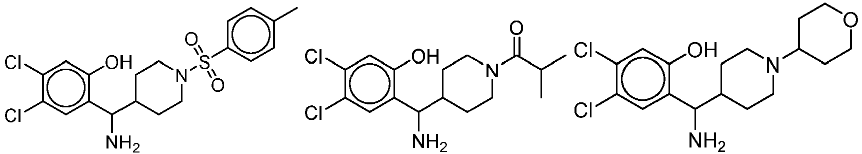

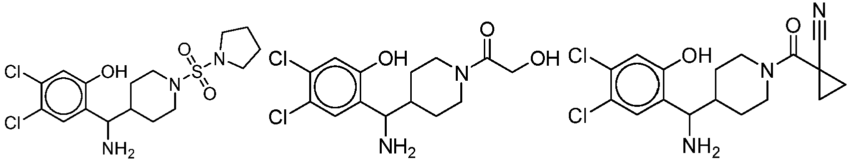

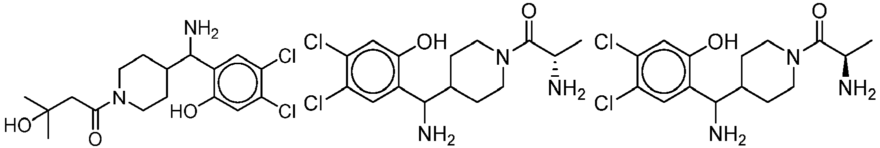

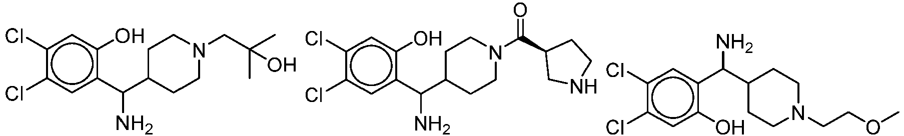

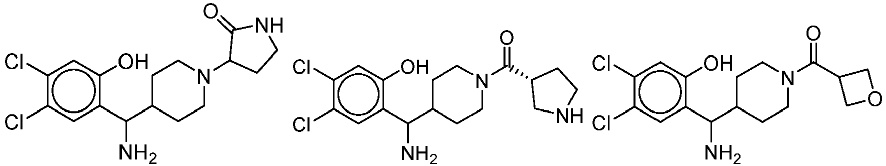

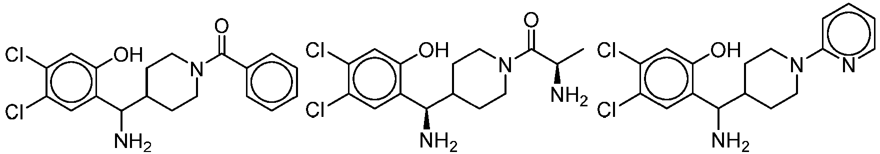

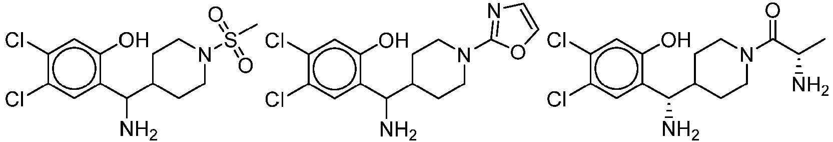

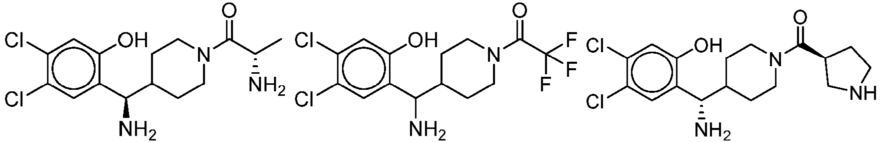

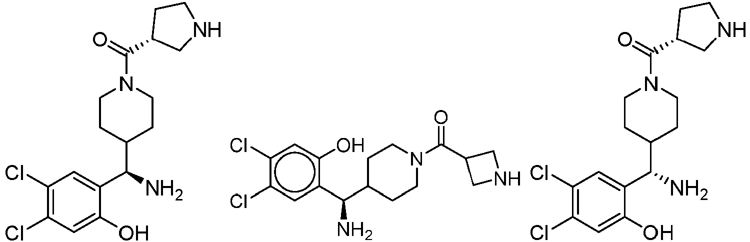

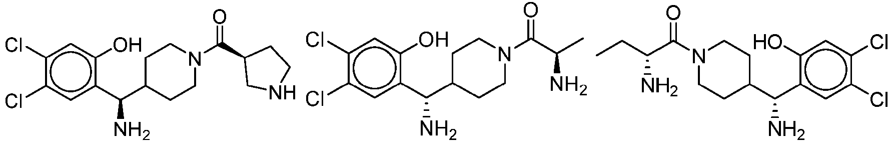

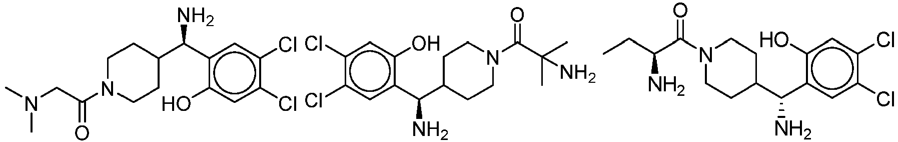

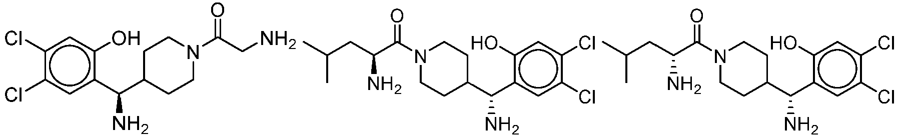

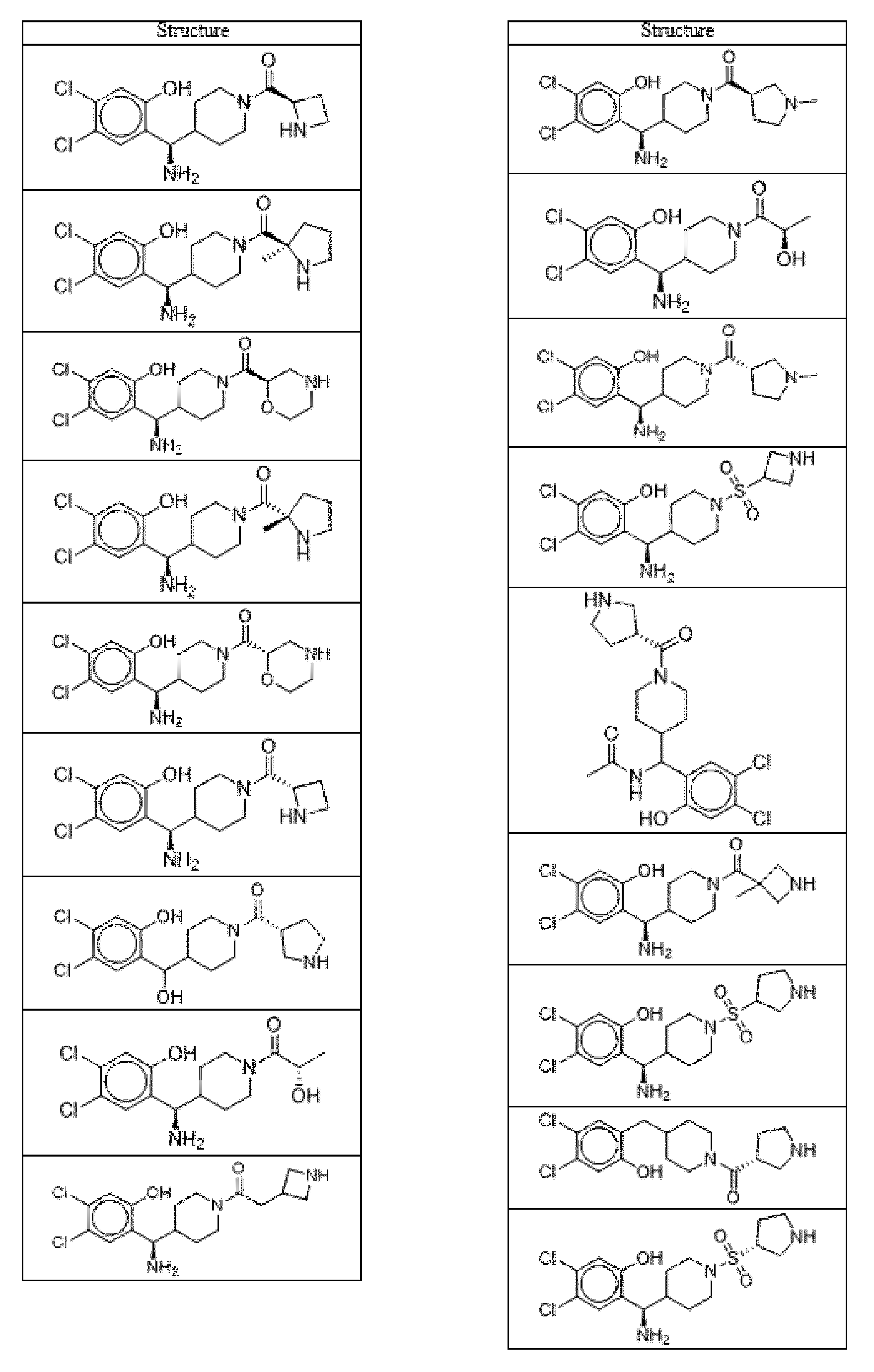

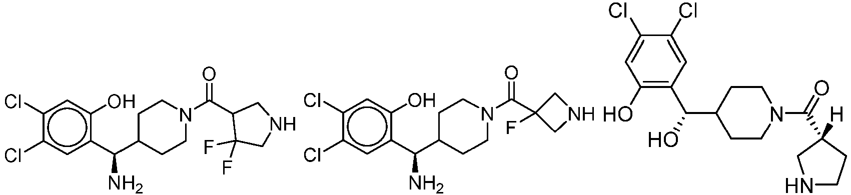

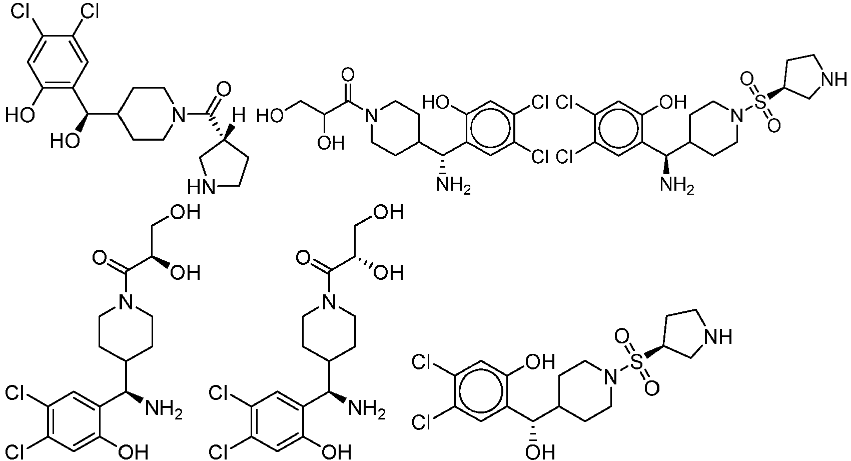

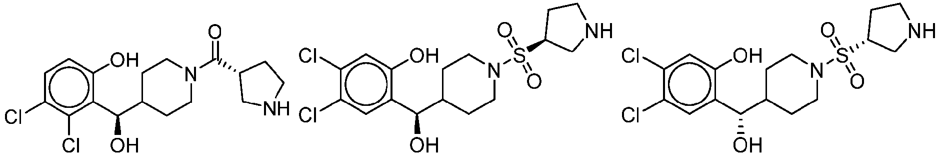

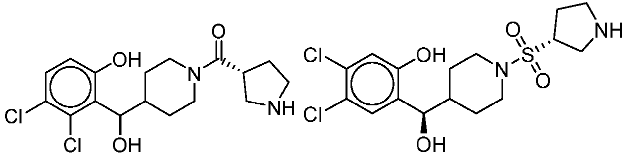

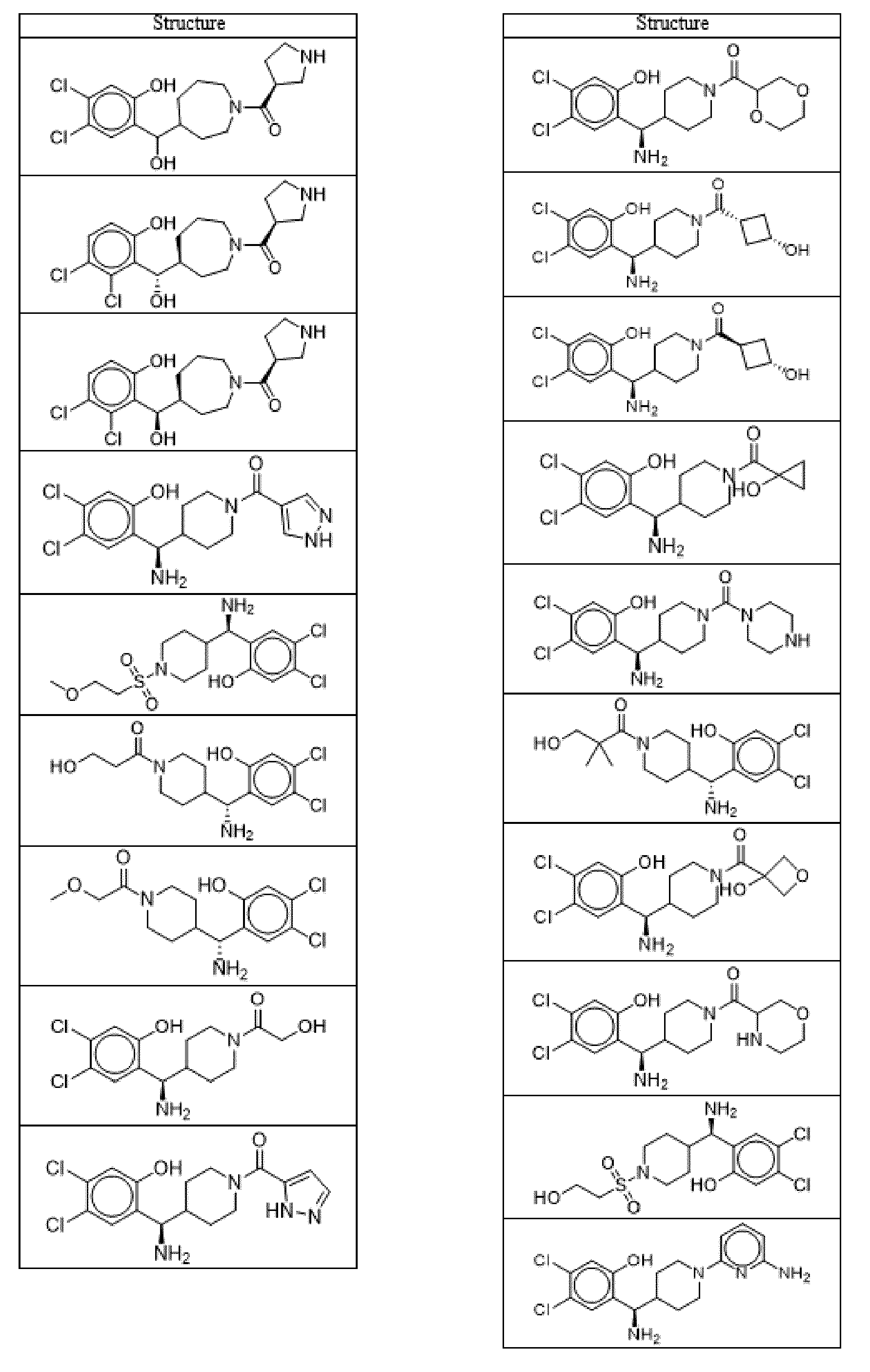

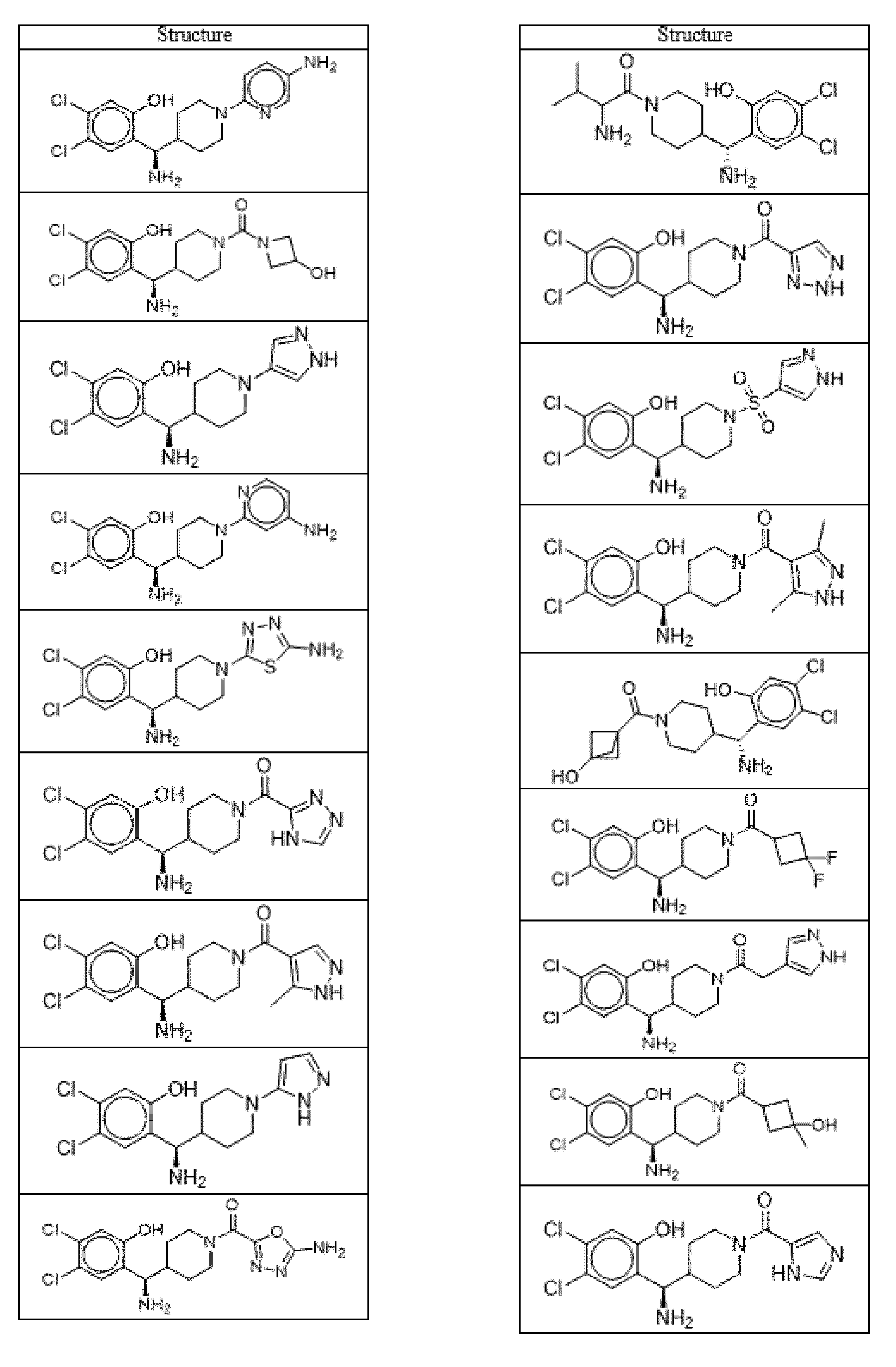

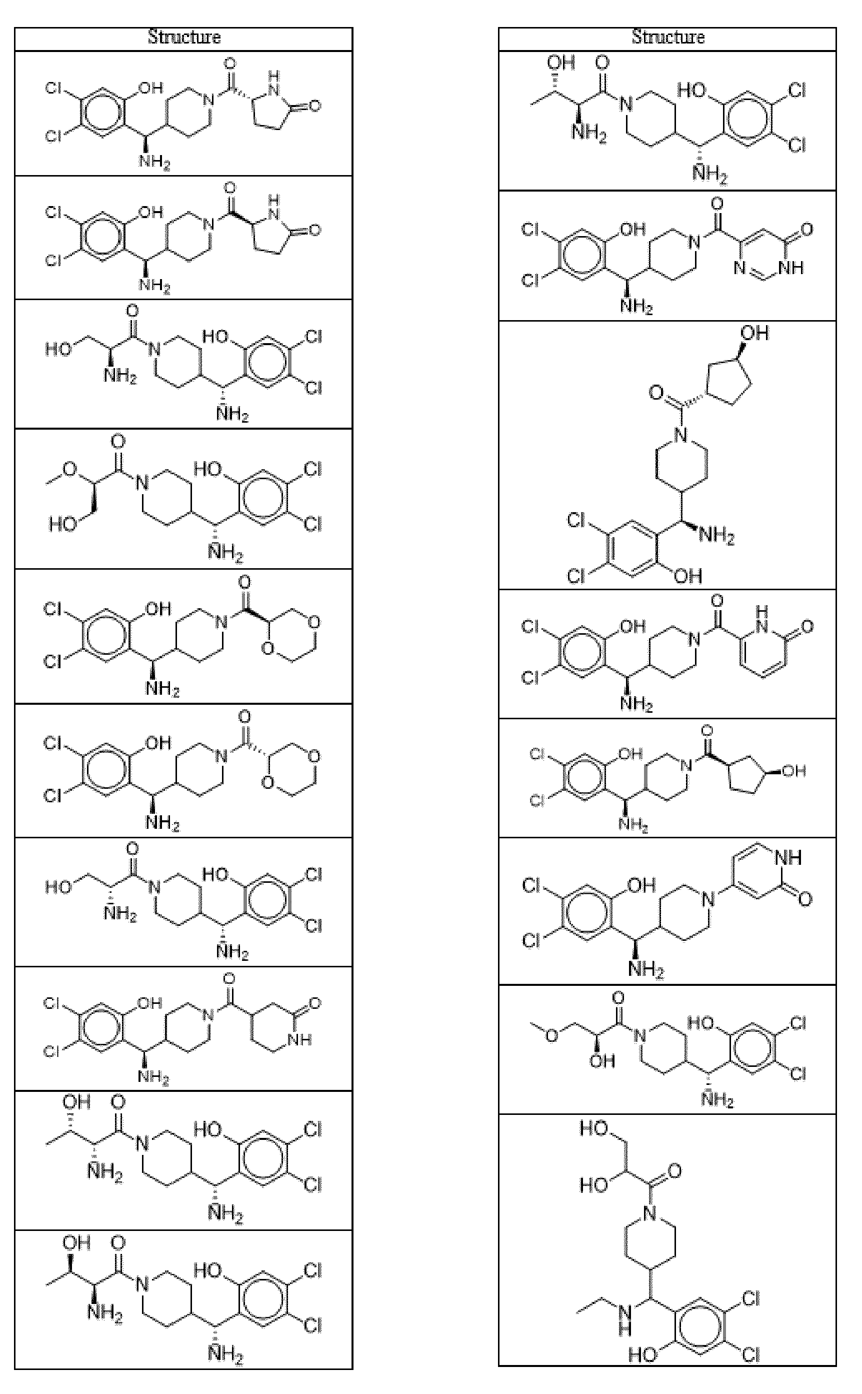

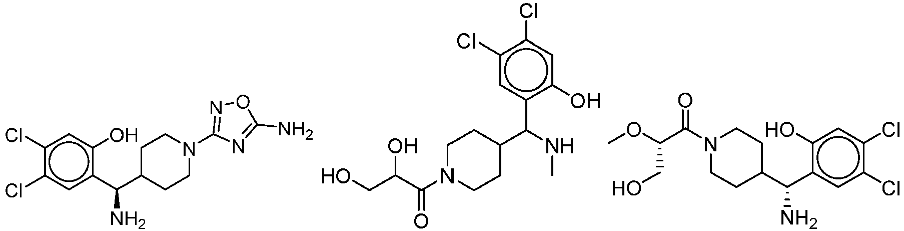

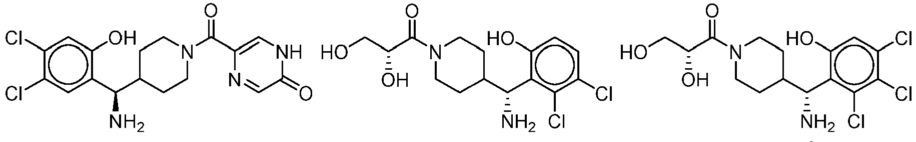

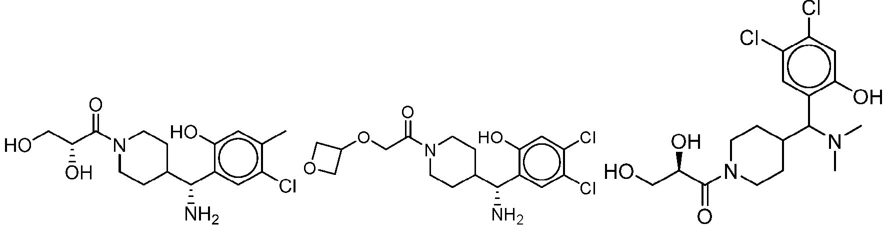

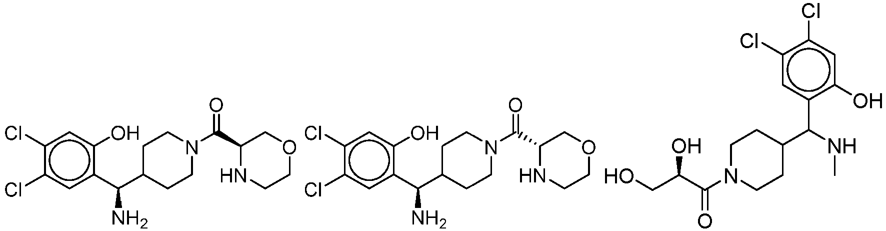

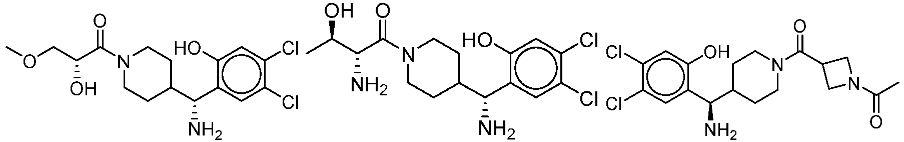

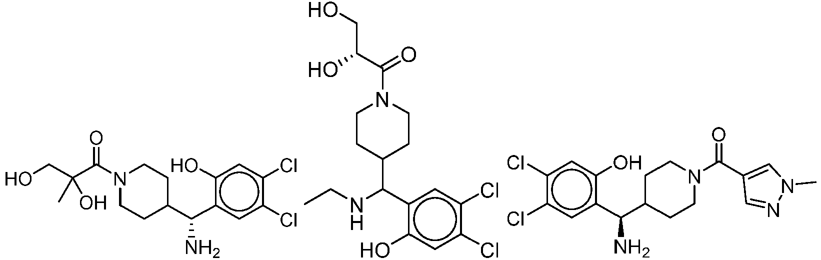

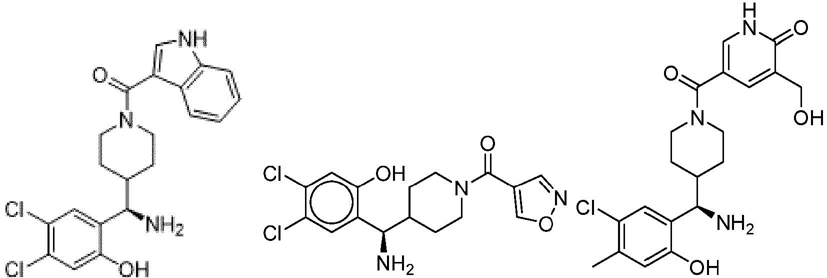

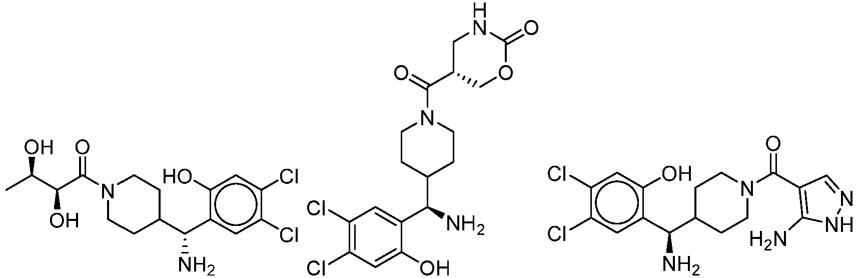

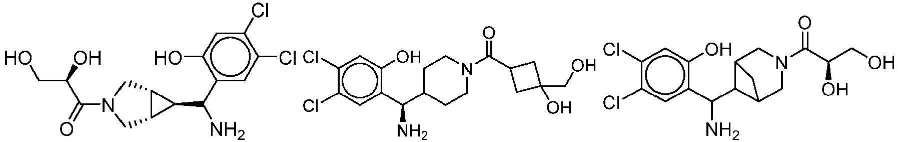

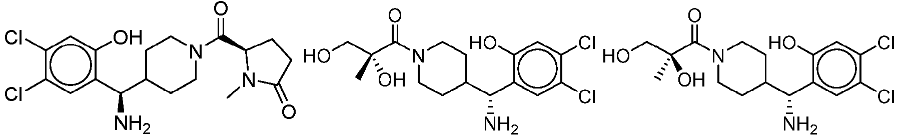

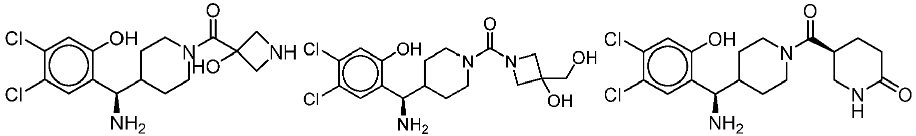

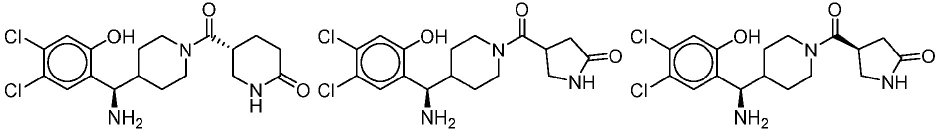

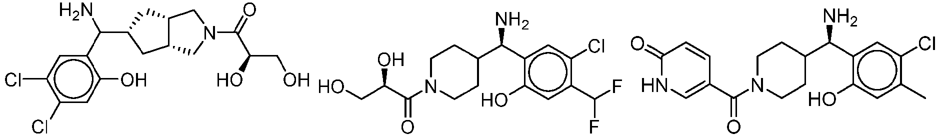

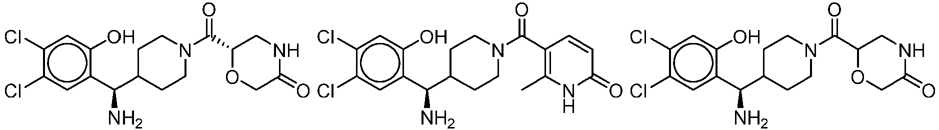

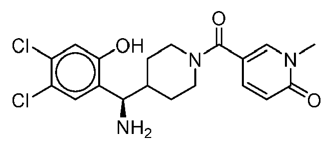

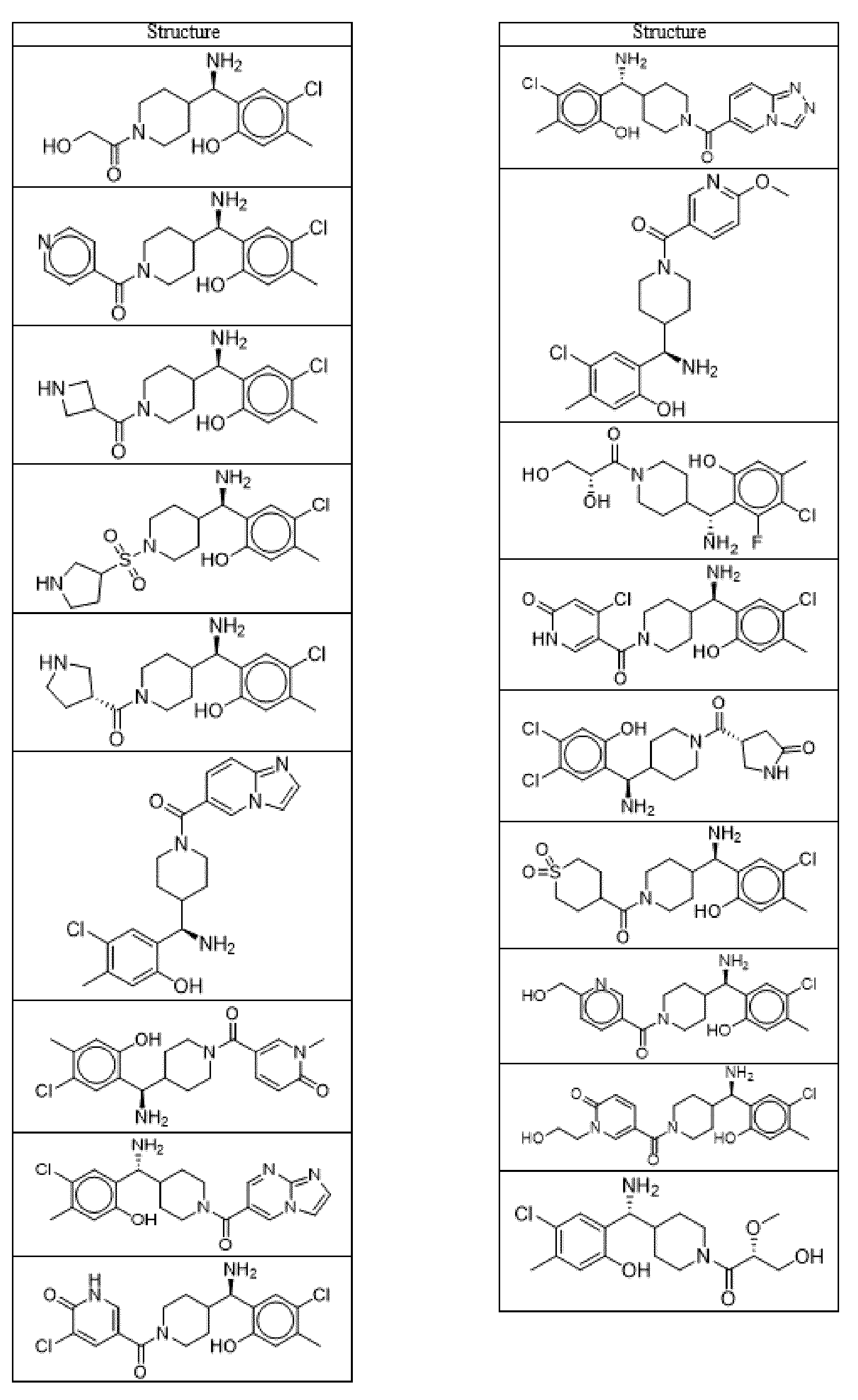

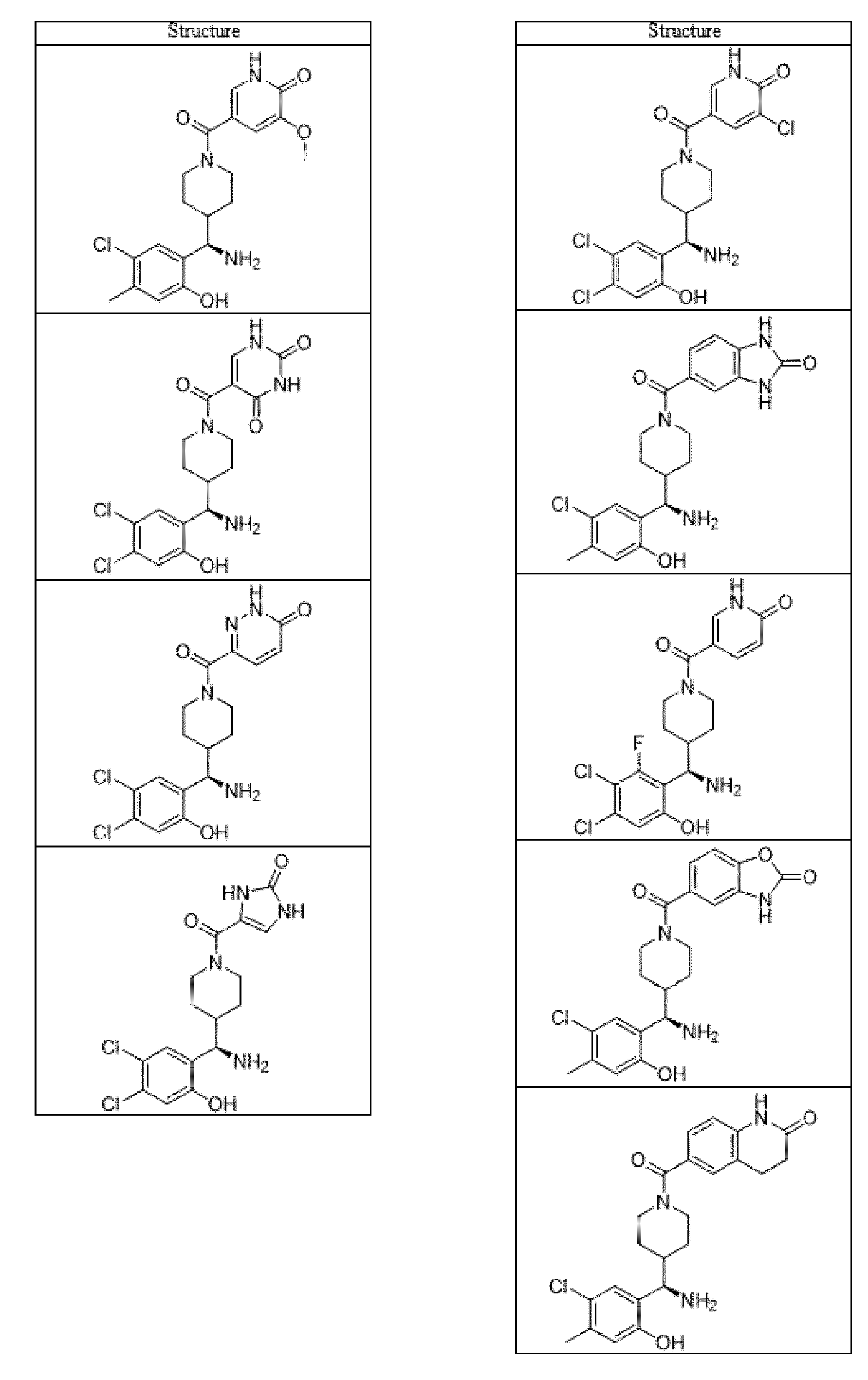

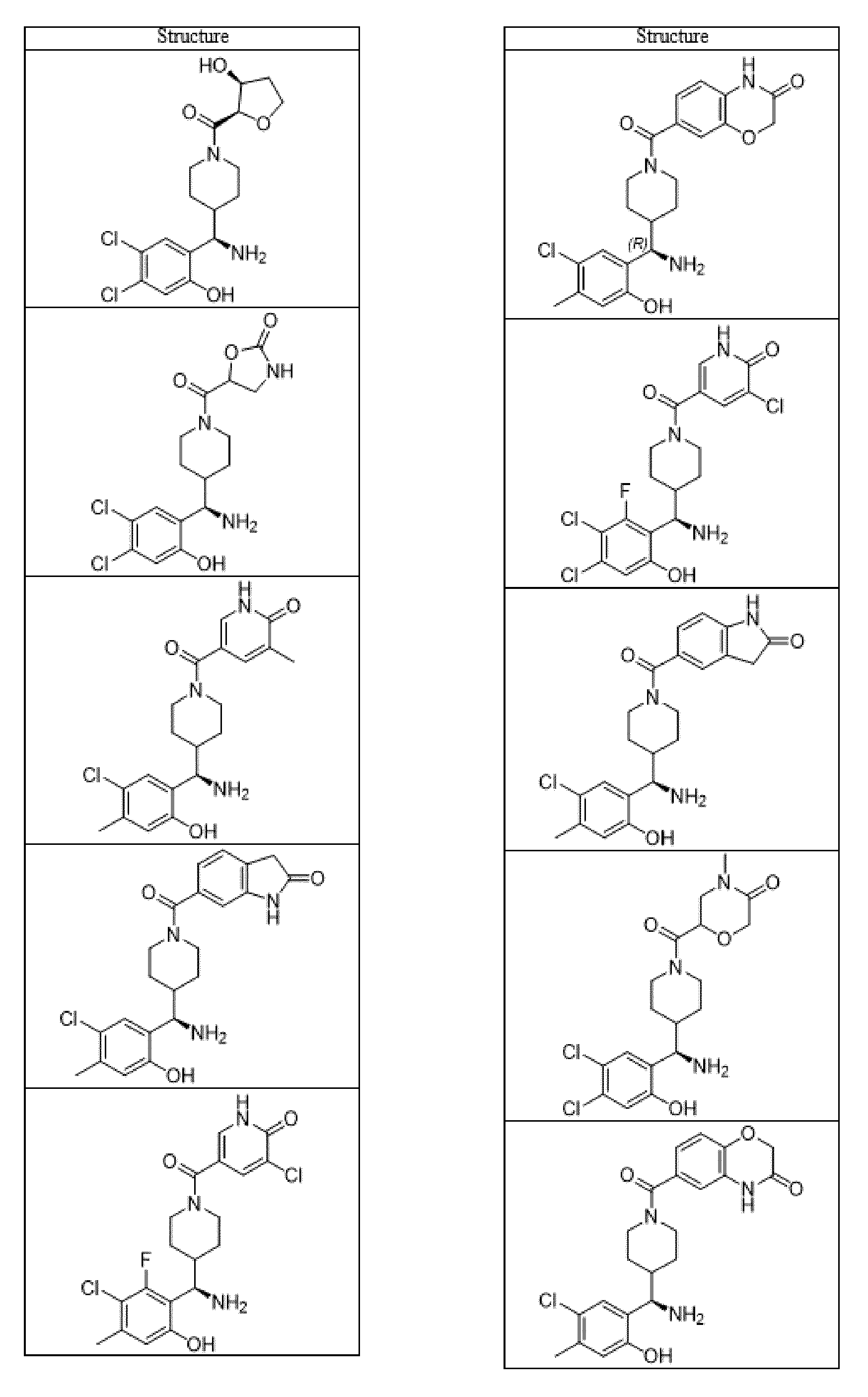

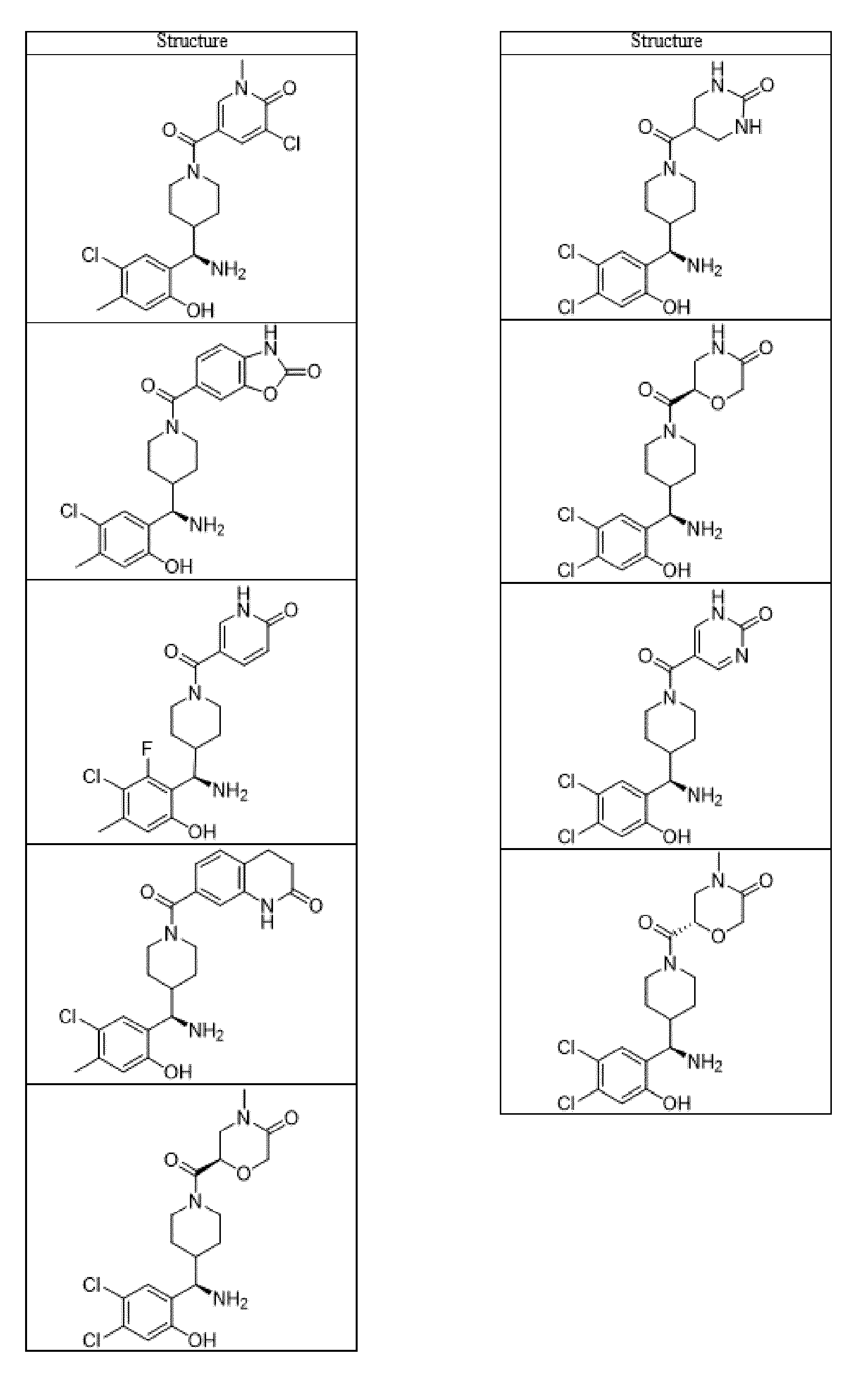

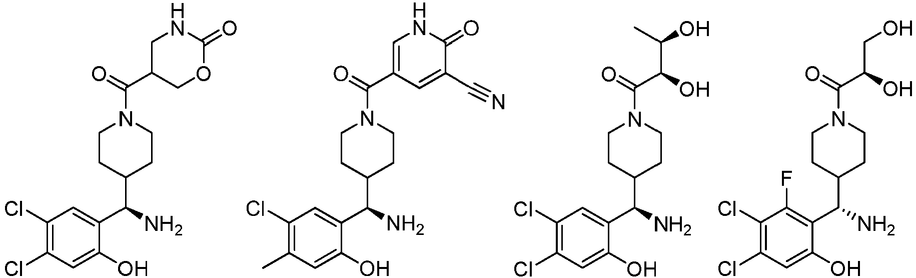

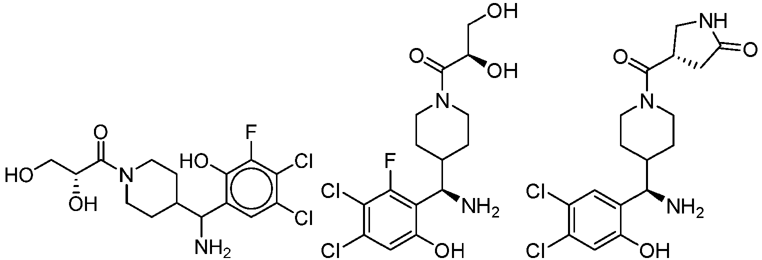

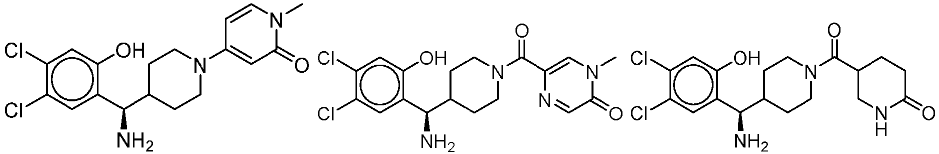

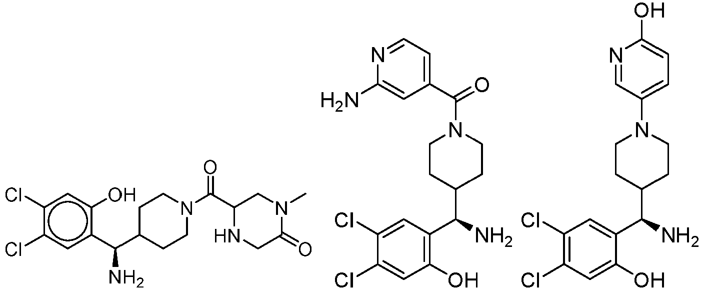

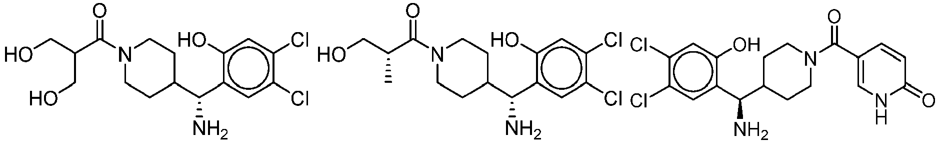

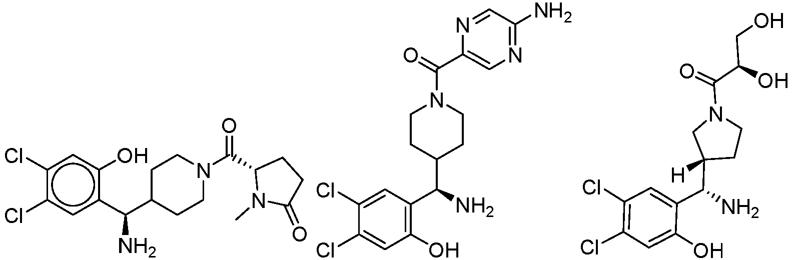

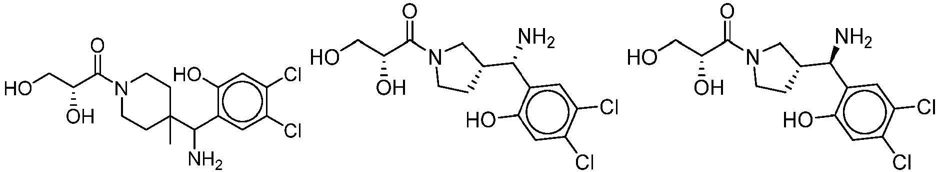

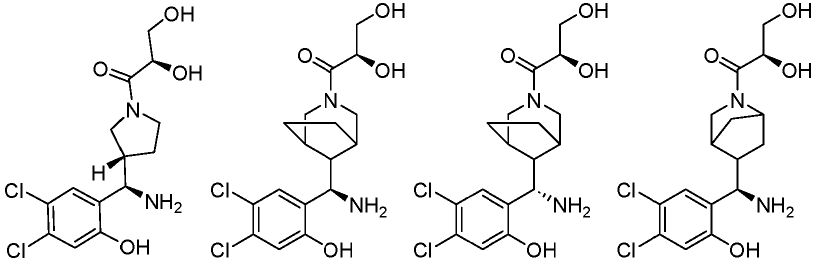

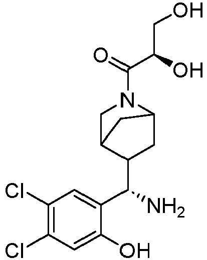

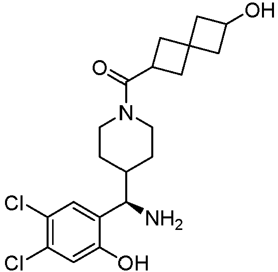

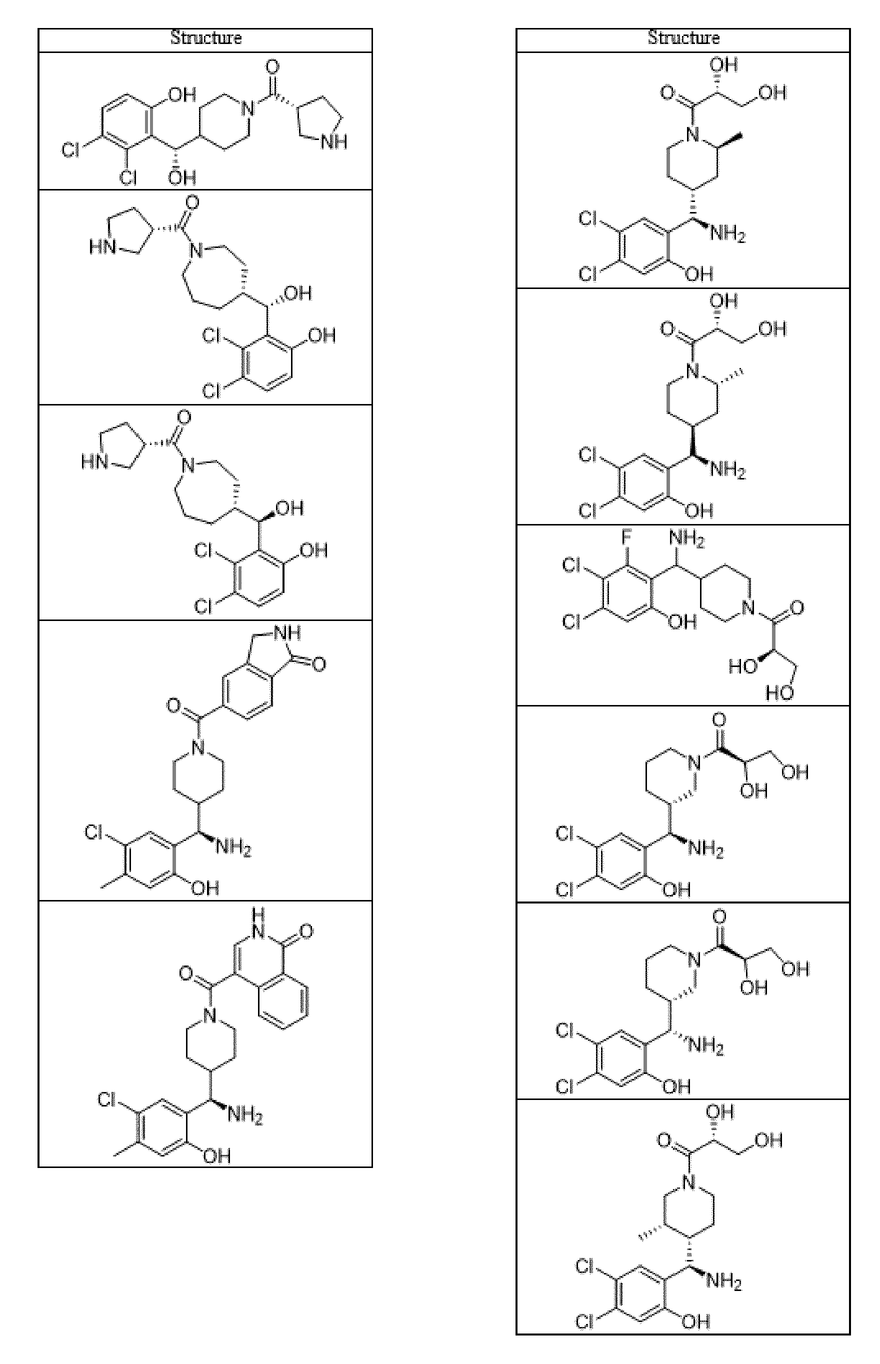

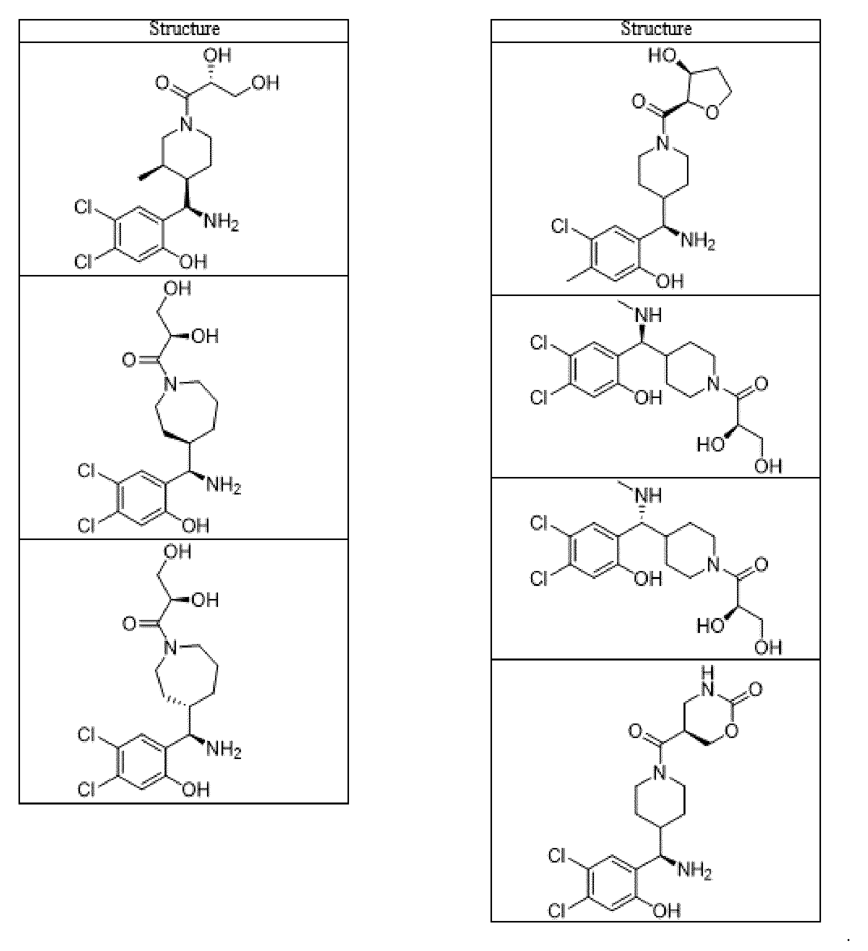

22. A pharmaceutical composition comprising at least one compound according to any one of claims 1-21 or a pharmaceutically acceptable salt thereof and a pharmaceutically acceptable carrier or diluent.

23. A compound or pharmaceutically acceptable salt thereof according to any one of claims 1-21 for use as a medicament.

24. A compound or pharmaceutically acceptable salt thereof according to any one of claims 1-21 for use in a method of treating a condition in a mammalian species in need thereof, the method comprising administering to the mammalian species a therapeutically effective amount of the compound or a pharmaceutically acceptable salt thereof, wherein the condition is selected from the group consisting of cancer, an immunological disorder, a Central Nerve System (CNS) disorder, an inflammatory disorder, a gastroenterological disorder, a metabolic disorder, a cardiovascular disorder, and a kidney disease;
optionally wherein the condition is:
(a) an immunological disorder that is transplant rejection or an autoimmune disease, wherein the autoimmune disease is optionally rheumatoid arthritis, multiple sclerosis, systemic lupus erythematosus, or Type I diabetes mellitus; or
(b) a Central Nerve System (CNS) disorder that is Alzheimer's disease;
(c) an inflammatory disorder that is an inflammatory skin condition, arthritis, psoriasis, spondylitis, parodontitits, or an inflammatory neuropathy;
(d) an gastroenterological disorder that is an inflammatory bowel disease; or
(e) a metabolic disorder that is obesity or Type II diabetes mellitus; or
(f) a cardiovascular disorder that is an ischemic stroke; or
(g) a kidney disease that is chronic kidney disease, nephritis, or chronic renal failure; or
(h) selected from the group consisting of cancer, transplant rejection, rheumatoid arthritis, multiple sclerosis, systemic lupus erythematosus, Type I diabetes mellitus, Alzheimer's disease, inflammatory skin condition, inflammatory neuropathy, psoriasis, spondylitis, parodontitis, Crohn's disease, ulcerative colitis, obesity, Type II diabetes mellitus, ischemic stroke, chronic kidney disease, nephritis, chronic renal failure, and a combination thereof;
and optionally wherein the mammalian species is human.

## Patentansprüche

1. Verbindung von Formel I oder ein pharmazeutisch zulässiges Salz davon, 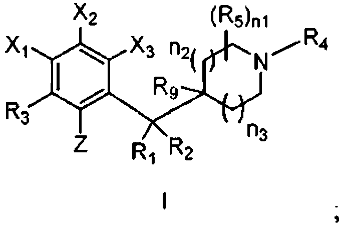 wobei
der strukturelle Rest 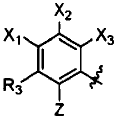 die Struktur 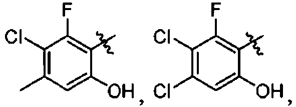 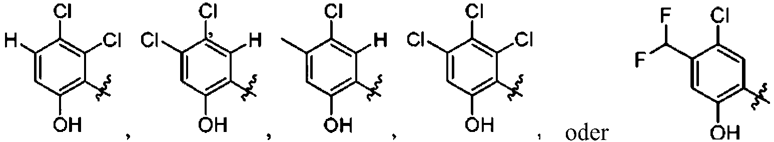 hat, die jeweils
substituiert ist durch R₃;
R₃ ist H, Halogen oder Alkyl;
R₁ und R₂ sind jeweils unabhängig H, Alkyl, (CR₆R₇)ₙ₄OR₈, (CR₆R₇)ₙ₄NRₐR_{b}, (CR₆R₇)ₙ₄NRₐ(C=O)R_{b}, (CR₆R₇)ₙ₄NRₐSO₂R_{b} oder (CR₆R₇)ₙ₄CONRₐR_{b}, wobei mindestens ein Auftreten von R₁ und R₂ (CR₆R₇)ₙ₄NRₐR_{b}, (CR₆R₇)ₙ₄ORₐ, (CR₆R₇)ₙ₄NRₐ(C=O)R_{b}, (CR₆R₇)ₙ₄NRₐSO₂R_{b} oder (CR₆R₇)ₙ₄CONRₐR_{b} ist; oder alternativ R₁, R₂ und das Kohlenstoffatom, an das sie gebunden sind, zusammengenommen einen 3-5-gliedrigen Carbocyclus bilden;
R₄ ist (CR₆R₇)ₙ₄(C=O)R_{c}, (C=O)(CR₆R₇)ₙ₄R_{c}, (CR₆R₇)ₙ₄OR_{c}, (CR₆R₇)ₙ₄COOR_{c}, (CR₆R₇)ₙ₄NR_{c}(C=O)R_{d}, (C=O(CR₆R₇)ₙ₄OR_{c}, (CR₆R₇)ₙ₄SO₂R_{c}, optional substituierter gesättigter Heterocyclus, optional substituiertes Aryl, optional substituiertes Heteroaryl, optional substituiertes Cycloalkyl oder optional substituiertes -Cycloalkyl-Alkyl;
jedes Auftreten von R₅ ist unabhängig H, Alkyl, Cycloalkyl oder Oxo;
oder zwei R₅-Gruppen, zusammengenommen mit dem/den Kohlenstoffatom(en), an die sie gebunden sind, bilden einen 3-7-gliedrigen optional substituierten gesättigten Carbocyclus; oder zwei R₅-Gruppen sind an verschiedene Kohlenstoffatome an dem Ring gebunden und bilden zusammengenommen eine Bindung oder eine Alkylkette, die 1-3 Kohlenstoffe enthält;
jedes Auftreten von R₆ und R₇ ist unabhängig H, Alkyl oder Cycloalkyl;
jedes Auftreten von Rₐ und R_{b} ist unabhängig H, Alkyl, Cycycloalkyl, gesättigter Heterocyclus, Aryl oder Heteroaryl; oder alternativ bilden Rₐ und R_{b} zusammen mit dem Stickstoffatom, an das sie gebunden sind, einen optional substituierten Heterocyclus, der das Stickstoffatom und 0-3 zusätzliche Heteroatome umfasst, die jeweils ausgewählt sind aus der Gruppe bestehend aus N, O und S;
jedes Auftreten von R_{c} und R_{d} ist unabhängig H, Alkyl, Alkyl substituiert durch 1-4 Substituenten, die jeweils unabhängig Halogen, OR₈ oder N(R₈)₂, Alkenyl, optional substituiertes Cycloalkyl, optional substituiertes Bicycloalkyl, optional substituiertes Spiroalkyl, optional substituierter gesättigter Heterocyclus, optional substituiertes Aryl, optional substituiertes Heteroaryl, optional substituiertes -Alkyl-Aryl, optional substituiertes -Alkyl-Heteroaryl, optional substituierter -Alkyl-Heterocyclus, optional substituiertes -Alkyl-Cycloalkyl oder optional substituiertes -Cycloalkyl-Alkyl sind; oder alternativ bilden R_{c} und R_{d} zusammen mit dem Stickstoffatom, an das sie gebunden sind, einen optional substituierten Heterocyclus, der das Stickstoffatom und 0-3 zusätzliche Heteroatome umfasst, jeweils ausgewählt aus der Gruppe bestehend aus N, O und S;
jedes Auftreten von R₈ ist unabhängig H, Alkyl oder ein optional substituierter Heterocyclus oder alternativ bilden die beiden R₈-Gruppen, zusammen mit dem Stickstoffatom, an das sie gebunden sind, einen optional substituierten Heterocyclus, der das Stickstoffatom und 0-3 zusätzliche Heteroatome umfasst, jeweils ausgewählt aus der Gruppe bestehend aus N, O und S;
R₉ ist H, Alkyl, Halogen oder (CR₆R₇)ₙ₄OR_{b};
das Alkyl, Cycloalkyl, Spiroalkyl, Bicycloalkyl, der Heterocyclus, das Aryl und Heteroaryl in R₁, R₂, R₃, R₄, R₅, R₆, R₇, R₉, Rₐ, R_{b}, R_{c} und R_{d} sind, wo zutreffend, optional substituiert durch 1-4 Substituenten, jeweils unabhängig ausgewählt aus der Gruppe bestehend aus Alkyl, Cycloalkyl, halogeniertem Cycloalkyl, halogeniertem Alkyl, Halogen, CN, -(CH₂)₀-₂OR₈, N(R₈)₂, (C=O)C₁₋₄Alkyl, (C=O)N(R₈)₂ und Oxo, wo es die Valenz erlaubt,
jedes Auftreten von n₁ ist unabhängig eine ganze Zahl von 0-3, wo es die Valenz erlaubt, jedes Auftreten von n₂ und n₃ ist unabhängig eine ganze Zahl, die 1, 2 oder 0 ist, und
jedes Auftreten von n₄ ist unabhängig eine ganze Zahl von 0-3.

2. Verbindung oder ein pharmazeutisch zulässiges Salz davon nach Anspruch 1, wobei
(a) jedes Auftreten von n₂ und n₃ unabhängig eine ganze Zahl von 0-1 ist; oder
(b) das strukturelle Motiv 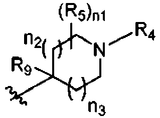 die Struktur 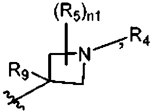 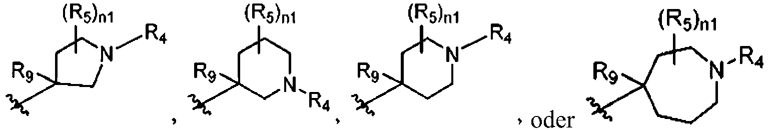 hat, oder
(c) das strukturelle Motiv 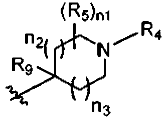 die Struktur 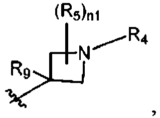 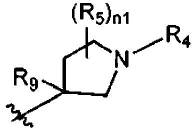 oder 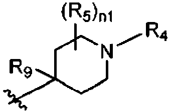 hat.

3. Verbindung oder ein pharmazeutisch zulässiges Salz davon nach Anspruch 1 oder 2, wobei das strukturelle Motiv 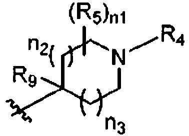 die Struktur 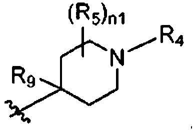 hat.

4. Verbindung oder ein pharmazeutisch zulässiges Salz davon nach einem der Ansprüche 1 bis 3, wobei mindestens ein Auftreten von R₁ und R₂ (CR₆R₇)ₙ₄ORₐ, CR₆R₇)ₙ₄NRₐR_{b}, (CR₆R₇)ₙ₄NRₐ(C=O)R_{b}, (CR₆R₇)ₙ₄NRₐSO₂R_{b} oder (CR₆R₇)ₙ₄CONRₐR_{b} ist.

5. Verbindung oder ein pharmazeutisch zulässiges Salz davon nach Anspruch 4, wobei mindestens ein Auftreten von R₁ und R₂ ORₐ oder NRₐR_{b} ist.

6. Verbindung oder ein pharmazeutisch zulässiges Salz davon nach einem der Ansprüche 1 bis 3, wobei R₁, R₂ und das Kohlenstoffatom, an das sie gebunden sind, zusammen einen 3-5-gliedrigen Carbocyclus bilden.

7. Verbindung oder ein pharmazeutisch zulässiges Salz davon nach einem der Ansprüche 1 bis 6, wobei R₄ (CR₆R₇)ₙ₄OR_{c}, (CR₆R₇)ₙ₄COR_{c}, (C=O)(CR₆R₇)ₙ₄R_{c}, (CR₆R₇)ₙ₄COOR_{c}, (CR₆R₇)ₙ₄NR_{c}(C=O)R_{d}, (C=O)(CR₆R₇)ₙ₄OR_{c} oder (CR₆R₇)ₙ₄SO₂R_{c} ist.

8. Verbindung oder ein pharmazeutisch zulässiges Salz davon nach Anspruch 7,
wobei R₄ (CR₆R₇)₂OR_{c}, (C=O)R_{c}, (C=O)(CR₆R₇)₁₋₂R_{c}, COOR_{c}, (CR₆R₇)₁₋₂NR_{c}(C=O)R_{d}, (C=O)(CR₆R₇)₁₋₂OR_{c} oder SO₂R_{c} ist.

9. Verbindung oder ein pharmazeutisch zulässiges Salz davon nach Anspruch 8, wobei R₄ (CH₂)₂OH, (CH₂)₂OMe, (C=O)H, (C=O)Me, (C=O)CH₂OH, (C=O)CH₂OMe, (C=O)Et, (C=O)Ph, (C=O)isopropyl, (C=O)CH₂NH₂, (C=O)CH₂NHMe, (C=O)CH(OH)CH₂OH, (C=O)CH(OMe)CH₂OH, (C=O)CH(OH)CH₂OMe, (C=O)OMe, SO₂Me, SO₂Et, SO₂CH₂OH oder SO₂CH₂OMe ist.

10. Verbindung oder ein pharmazeutisch zulässiges Salz davon nach Anspruch 8, wobei R₄ (C=O)R_{c}, (C=O)(CR₆R₇)₁₋₂R_{c}, (C=O)(CR₆R₇)₁₋₂OR_{c} oder SO₂R_{c} ist und wobei R_{c} ausgewählt ist aus der Gruppe bestehend aus H, Alkyl, Alkyl substituiert durch 1-4 Substituenten, jeweils unabhängig ausgewählt aus der Gruppe bestehend aus Halogen, OR₈ und N(R₈)₂, Alkenyl, optional substituiertem Cycloalkyl, optional substituiertem Bicycloalkyl, optional substituiertem Spiroalkyl, optional substituiertem gesättigtem Heterocyclus, optional substituiertem Aryl, optional substituiertem Heteroaryl, optional substituiertem -Alkyl-Aryl, optional substituiertem -Alkyl-Heteroaryl, optional substituiertem -Alkyl-Heterocyclus, optional substituiertem -Alkyl-Cycloalkyl und optional substituiertem -Cycloalkyl-Alkyl.

11. Verbindung oder ein pharmazeutisch zulässiges Salz davon nach einem der Ansprüche 1 bis 6, wobei R₄ (CR₆R₇)₂OR_{c}, (C=O)R_{c}, (C=O)(CR₆R₇)₁₋₂R_{c}, COOR_{c}, (CR₆R₇)₁₋₂NR_{c}(C=O)R_{d}, (C=O)(CR₆R₇)₁₋₂OR_{c} oder SO₂R_{c} ist, und:
(a) R_{c} oder R₄ H, Me, Et, 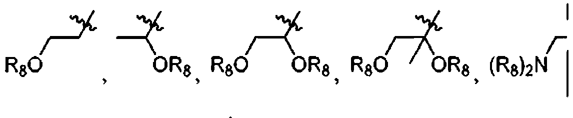 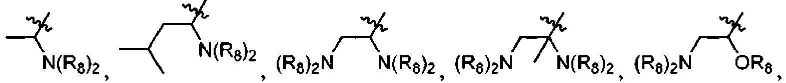 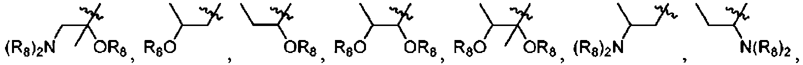 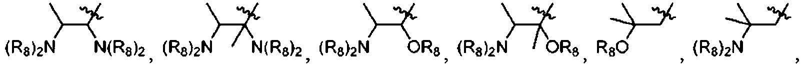 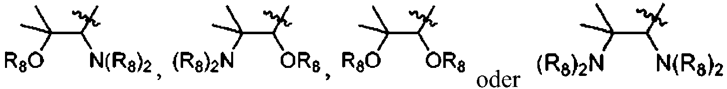 ist; oder
(b) R_{c} oder R_{d} ein Heterocyclus ist, ausgewählt aus der Gruppe bestehend aus 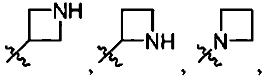 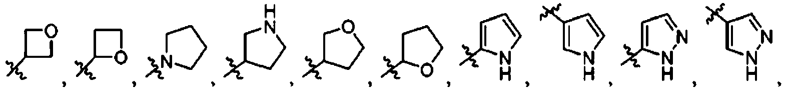 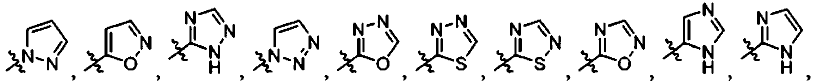 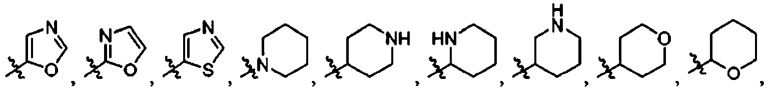 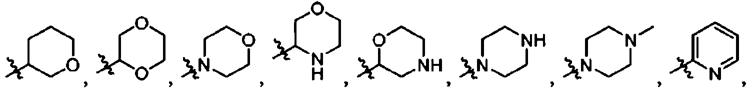 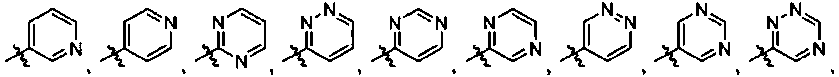 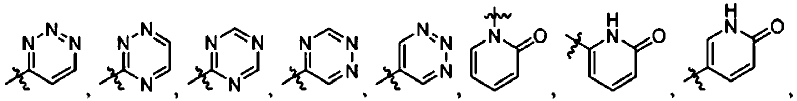 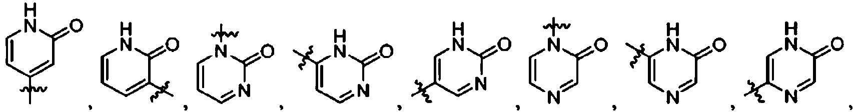 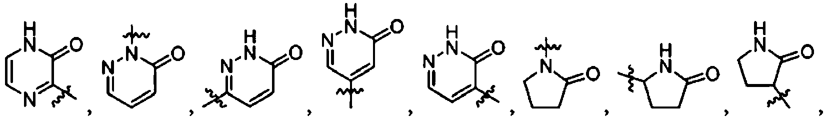 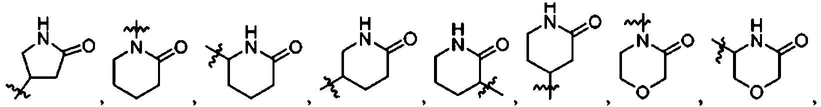 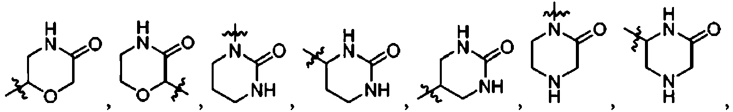 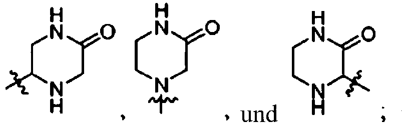 wobei der Heterocyclus optional substituiert ist durch eines oder mehrere aus Cyano, Cycloalkyl, fluoriertes Alkyl, fluoriertes Cycloalkyl, Halogen, OH, NH₂, Oxo oder (C=O)C₁₋₄Alkyl, wo es die Valenz erlaubt; oder
(c) R_{c} Cycloalkyl, Spiroalkyl oder Bicycloalkyl ist, jeweils optional substituiert durch 1-4 Substituenten, jeweils unabhängig ausgewählt aus der Gruppe bestehend aus Alkyl, Halogen, CN, - (CH₂)₀₋₂OR₈, N(R₈)₂, (C=O)N(R₈)₂ und Oxo, wo es die Valenz erlaubt, oder
(d) R_{c} 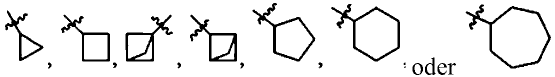 ist, jeweils optional substituiert durch 1-4 Substituenten, jeweils unabhängig ausgewählt aus der Gruppe bestehend aus Alkyl, Halogen, CN, -(CH₂)₀₋₂OR₈, N(R₈)₂, (C=O)N(R₈)₂ und Oxo, wo es die Valenz erlaubt.

12. Verbindung oder ein pharmazeutisch zulässiges Salz davon nach einem der Ansprüche 1 bis 6, wobei:
(a) R₄ ein optional substituierter gesättigter Heterocyclus, optional substituiertes Aryl, optional substituiertes Heteroaryl, optional substituiertes Cycloalkyl oder optional substituiertes -Cycloalkyl-Alkyl ist; oder
(b) R₄ ein Heterocyclus ist, ausgewählt aus der Gruppe bestehend aus 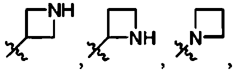 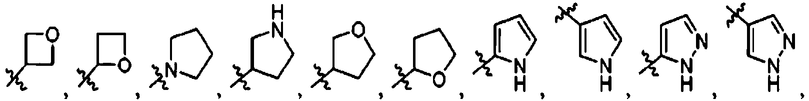 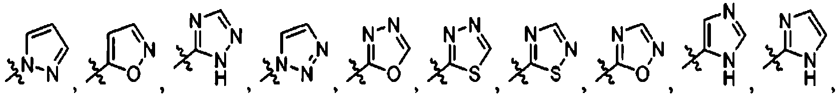 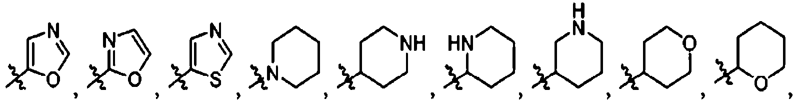 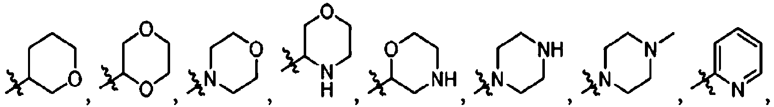 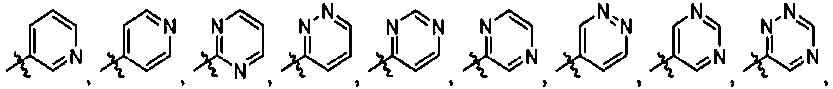 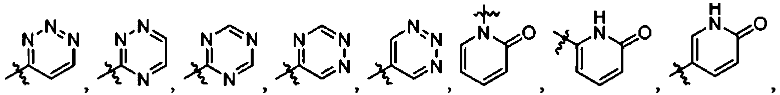 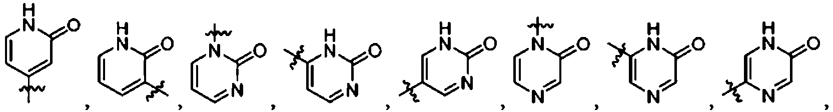 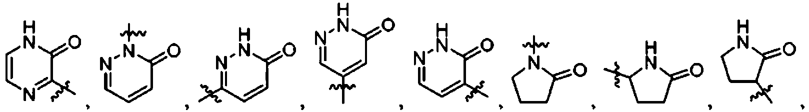 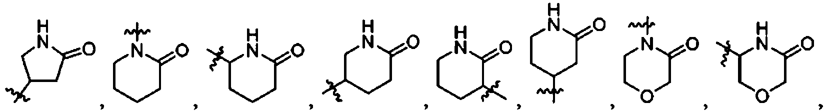 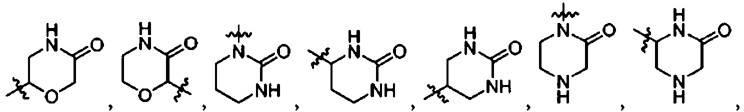 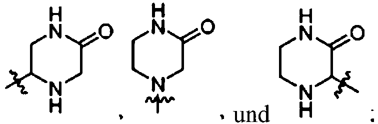 wobei der Heterocyclus optional substituiert ist durch eines oder mehrere ausgewählt aus Cyano, Cycloalkyl, fluoriertem Alkyl, fluoriertem Cycloalkyl, Halogen, OH, NH₂, Oxo oder (C=O)C₁₋₄Alkyl, wo es die Valenz erlaubt, oder
(c) R₄ Cycloalkyl ist, optional substituiert durch 1-4 Substituenten, jeweils unabhängig ausgewählt aus der Gruppe bestehend aus Alkyl, Halogen, CN, -(CH₂)₀₋₂OR₈, N(R₈)₂,(C=O)N(R₈)₂
und Oxo, wo es die Valenz erlaubt, oder
(d) R4 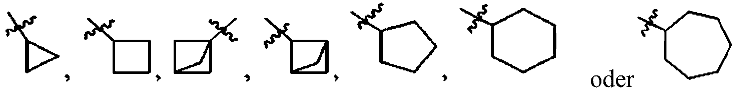 ist, jeweils
optional substituiert durch 1-4 Substituenten, jeweils unabhängig ausgewählt aus der Gruppe bestehend
aus Alkyl, Halogen, CN, -(CH₂)₀₋₂OR₈, N(R₈)₂, (C=O)N(R₈)₂ und Oxo, wo es die Valenz erlaubt.

13. Verbindung oder ein zulässiges Salz davon nach einem der Ansprüche 1 bis 6, wobei R₄ 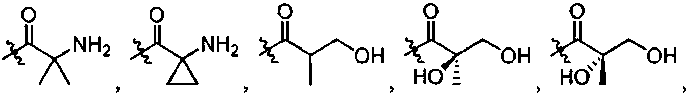 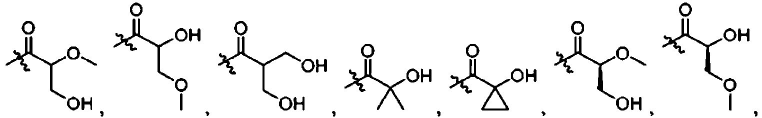 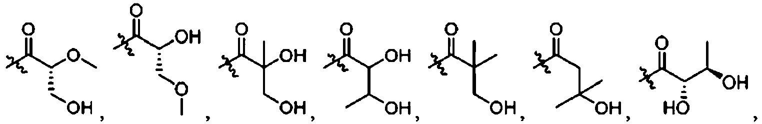 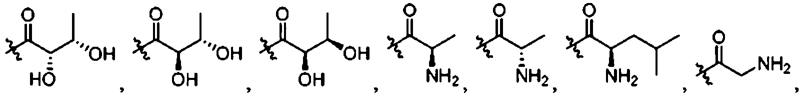 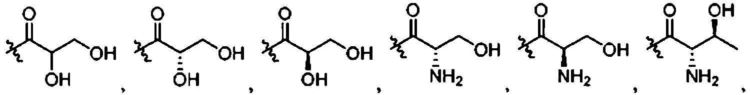 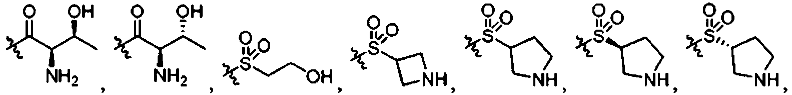 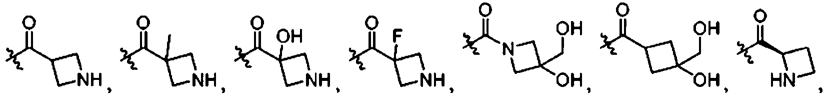 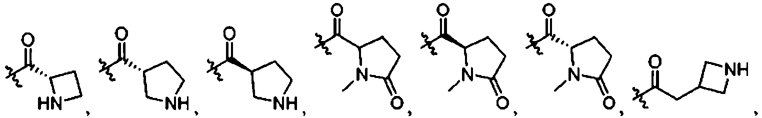 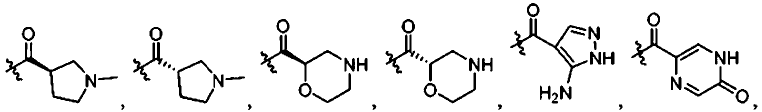 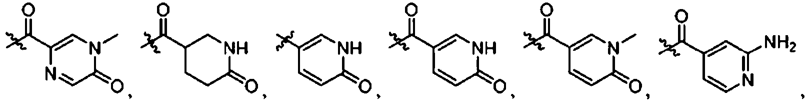 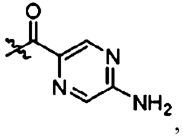 oder ein Tautomer davon ist.

14. Verbindung oder ein pharmazeutisch zulässiges Salz davon nach einem der Ansprüche 1 bis 13, wobei:
(a) mindestens ein Auftreten von R₅ H, Alkyl oder Cycloalkyl ist; oder
(b) mindestens ein Auftreten von R₅ Oxo ist; oder
(c) zwei R₅-Gruppen an verschiedene Kohlenstoffatome an dem Ring gebunden sind und zusammengenommen eine Bindung oder eine Alkylkette bilden, die 1-3 Kohlenstoffe enthält; oder
(d) zwei R₅-Gruppen, zusammengenommen mit dem/den Kohlenstoffatom(en), an die sie gebunden sind, einen 3-7-gliedrigen optional substituierten gesättigten Carbocyclus bilden;

15. Verbindung oder ein pharmazeutisch zulässiges Salz davon nach einem der vorstehenden Ansprüche, wobei:
(a) mindestens eines aus Rₐ und R_{b} unabhängig H, Alkyl, Cycloalkyl, gesättigter Heterocyclus, Aryl oder Heteroaryl ist; oder
(b) Rₐ und R_{b} zusammen mit dem Stickstoffatom, an das sie gebunden sind, einen optional substituierten Heterocyclus bilden, der das Stickstoffatom und 0-3 zusätzliche Heteroatome umfasst, jeweils ausgewählt aus der Gruppe bestehend aus N, O und S.

16. Verbindung oder ein pharmazeutisch zulässiges Salz davon nach einem der vorstehenden Ansprüche, wobei:
(a) jedes Auftreten von R₆ und R₇ unabhängig H oder Alkyl ist, und/oder
(b) R₉ (i) H, Alkyl oder Halogen ist oder (ii) (CR₆R₇)ₙ₄OR_{b}; oder (iii) H, F oder OH ist.

17. Verbindung oder ein pharmazeutisch zulässiges Salz davon nach einem der Ansprüche 1 bis 16, wobei R₃ H oder Alkyl ist.

18. Verbindung oder ein pharmazeutisch zulässiges Salz davon nach einem der Ansprüche 1 bis 16, wobei R₃ Halogen ist.

19. Verbindung oder ein pharmazeutisch zulässiges Salz davon nach einem der vorstehenden Ansprüche, wobei:
(a) mindestens ein Auftreten von R₈ H, Alkyl oder optional ein substituierter Heterocyclus ist, optional, wobei R₈ H, Me, Et, Pr, Bu oder ein Heterocyclus ist, ausgewählt aus der Gruppe bestehend aus 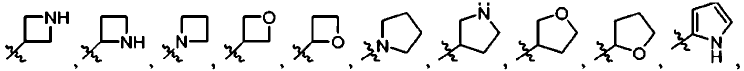 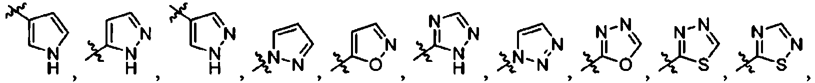 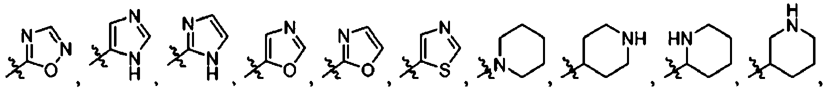 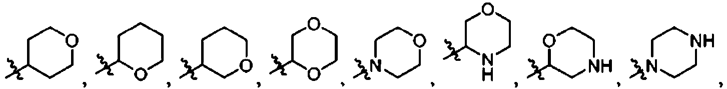 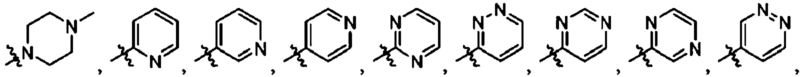 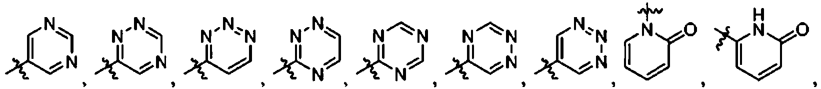 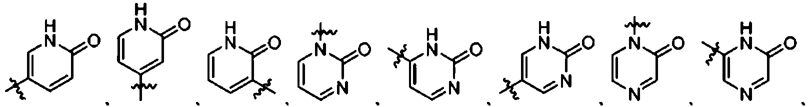 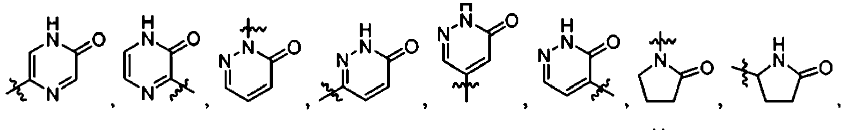 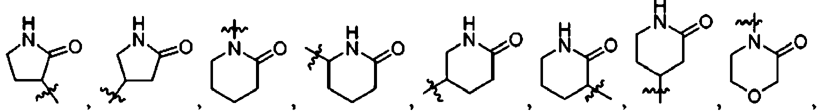 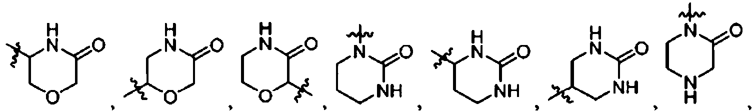 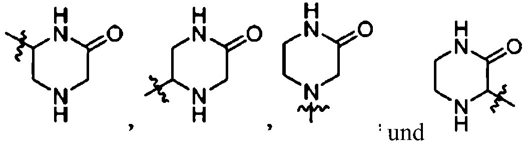 wobei der Heterocyclus optional substituiert ist durch eines oder mehrere aus Cyano, Cycloalkyl, fluoriertem Alkyl, fluoriertem Cycloalkyl, Halogen, OH, NH₂, Oxo oder (C=O)C₁₋₄Alkyl, wo es die Valenz erlaubt; oder
(b) die beiden R₈-Gruppen zusammen mit dem Stickstoffatom, an das sie gebunden sind, einen optional substituierten Heterocyclus bilden, der das Stickstoffatom und 0-3 zusätzliche Heteroatome umfasst, jeweils ausgewählt aus der Gruppe bestehend aus N, O und S.

20. Verbindung oder ein pharmazeutisch zulässiges Salz davon nach einem der Ansprüche 1 bis 6, wobei:
(a) mindestens ein Auftreten von R_{c} oder R_{d} unabhängig H, Alkyl, Alkyl substituiert durch 1-4 Substituenten ist, jeweils unabhängig ausgewählt aus der Gruppe bestehend aus Halogen, OR₈ und N(R₈)₂, Alkenyl, optional substituiertem Cycloalkyl, optional substituiertem Bicycloalkyl, optional substituiertem Spiroalkyl, optional substituiertem gesättigtem Heterocyclus, optional substituiertem Aryl, optional substituiertem Heteroaryl, optional substituiertem -Alkyl-Aryl, optional substituiertem -Alkyl-Heteroaryl, optional substituiertem -Alkyl-Heterocyclus, optional substituiertem -Alkyl-Cycloalkyl oder optional substituiertem -Cycloalkyl-Alkyl; oder
(b) mindestens ein Auftreten von R_{c} oder R_{d} ist unabhängig H, Me, Et 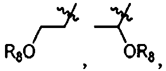 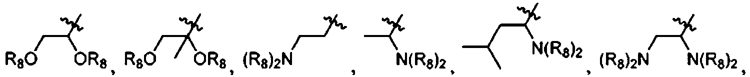 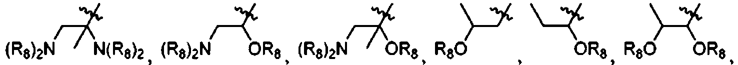 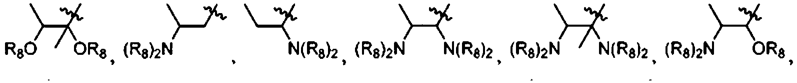 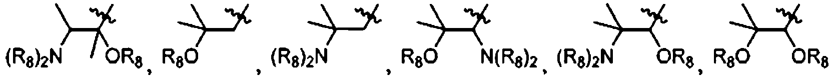 oder

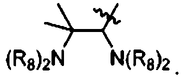

21. Verbindung oder ein pharmazeutisch zulässiges Salz davon nach Anspruch 1, wobei die Verbindung ausgewählt ist aus der Gruppe bestehend aus den folgenden Verbindungen und pharmazeutisch zulässigen Salzen davon

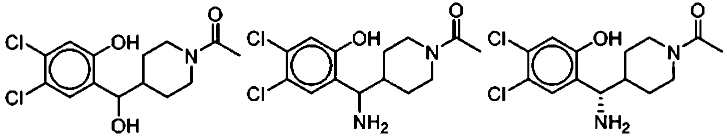

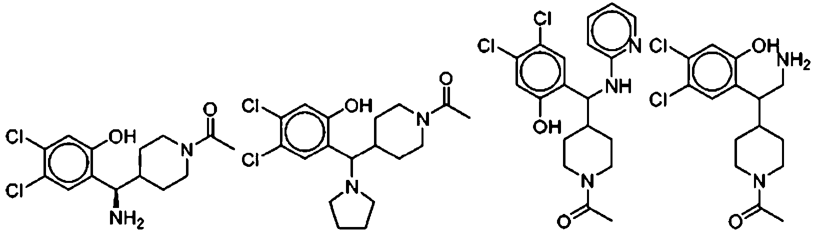

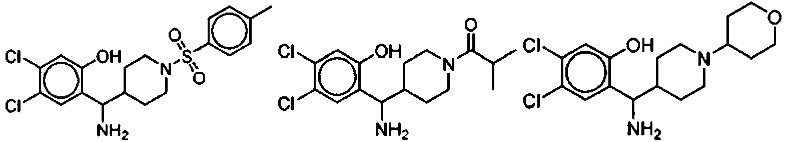

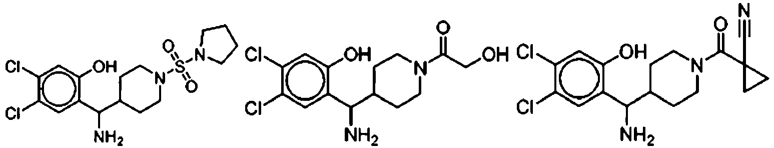

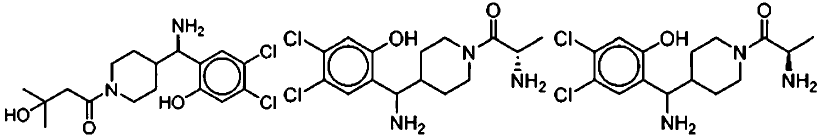

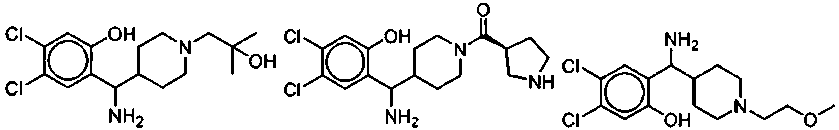

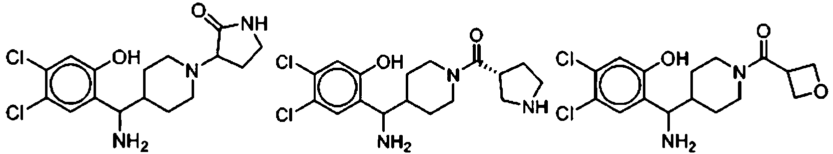

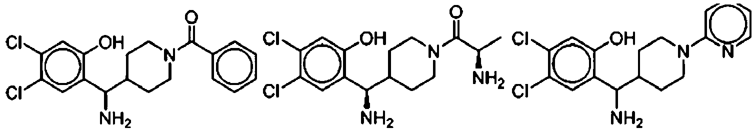

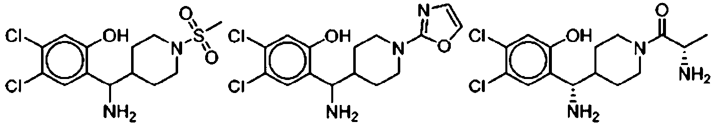

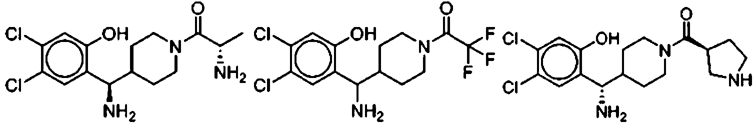

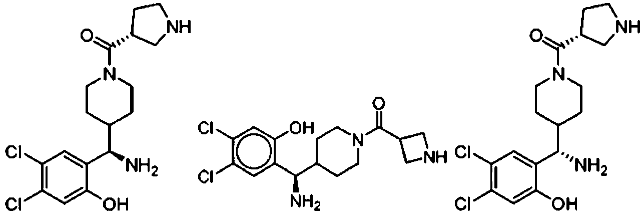

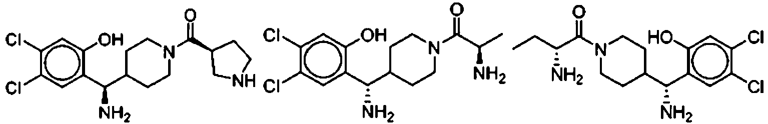

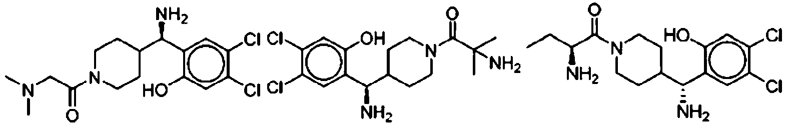

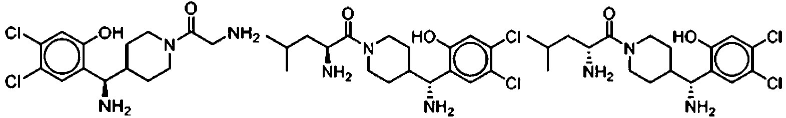

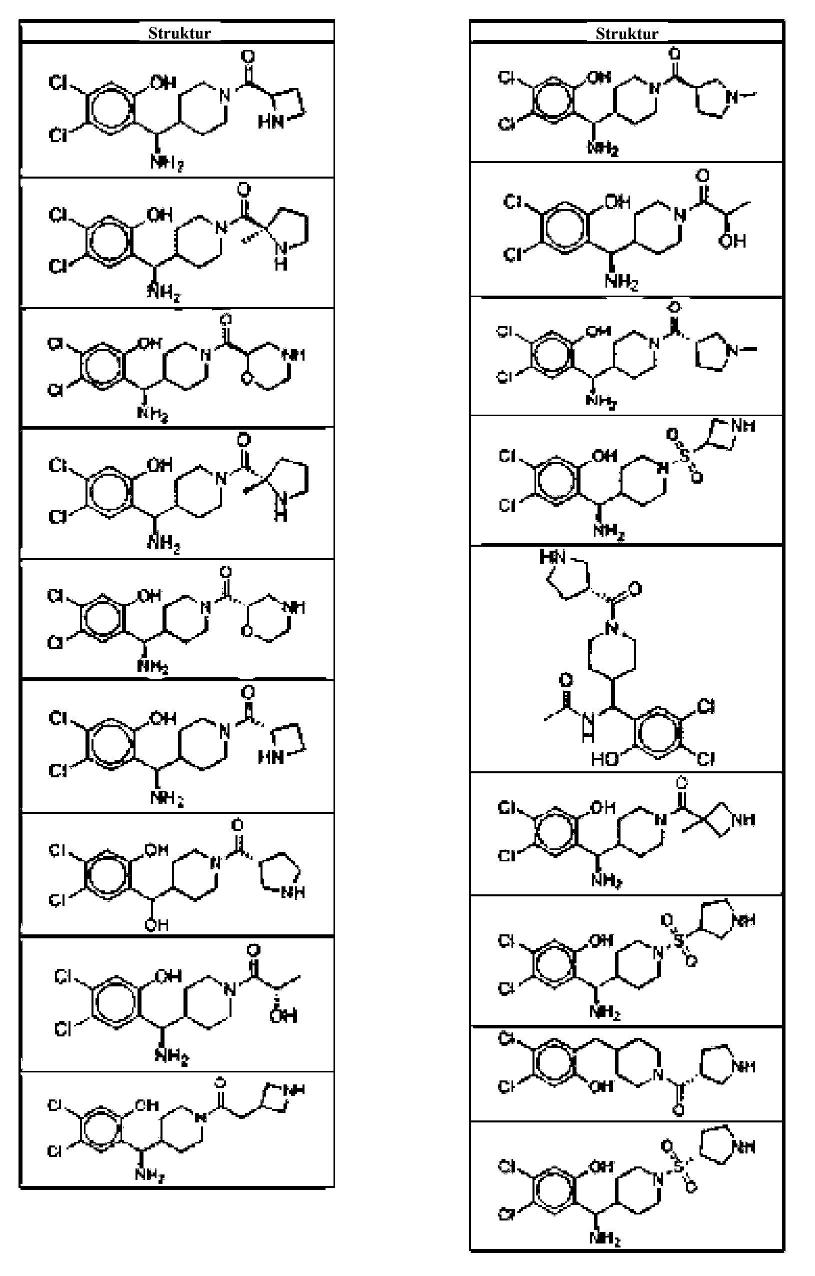

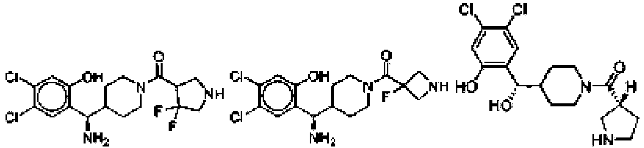

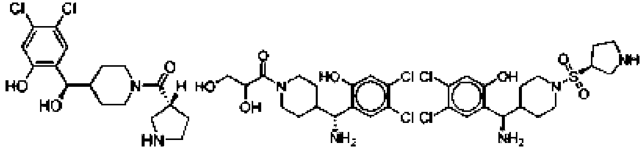

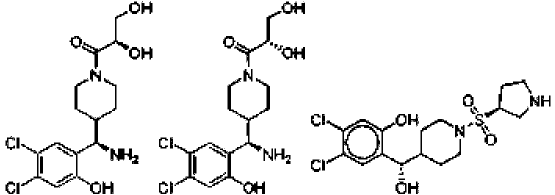

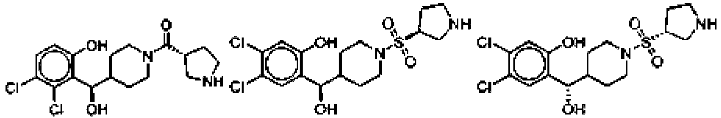

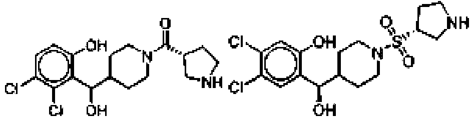

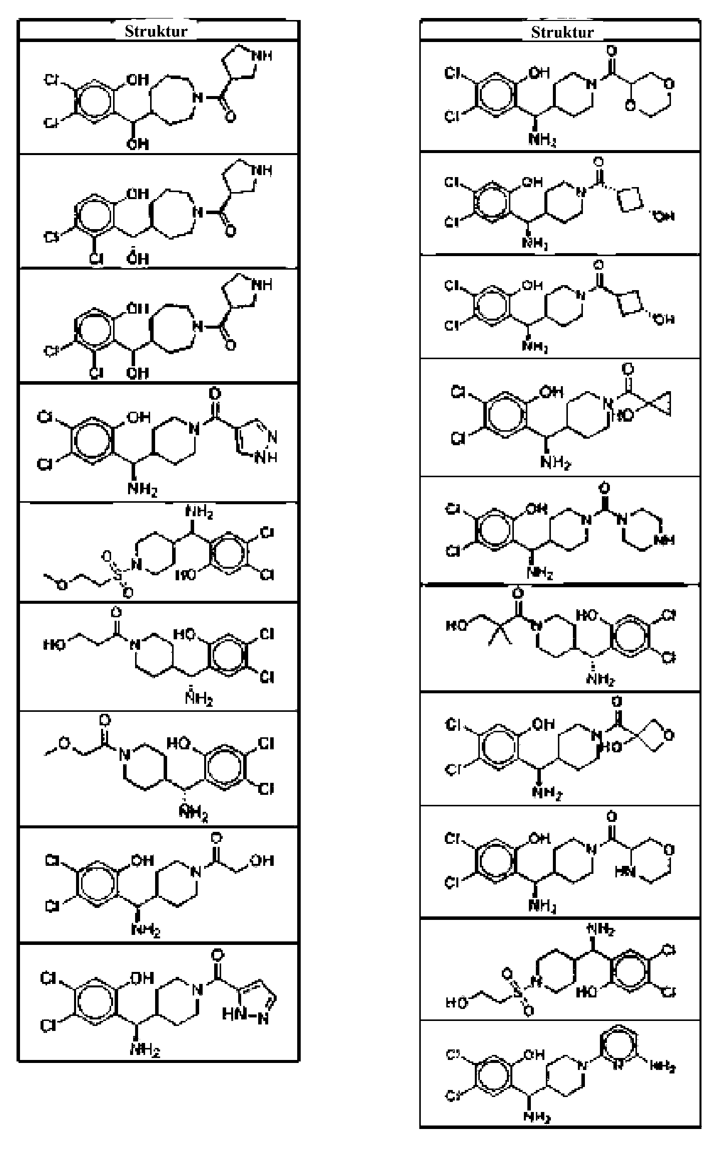

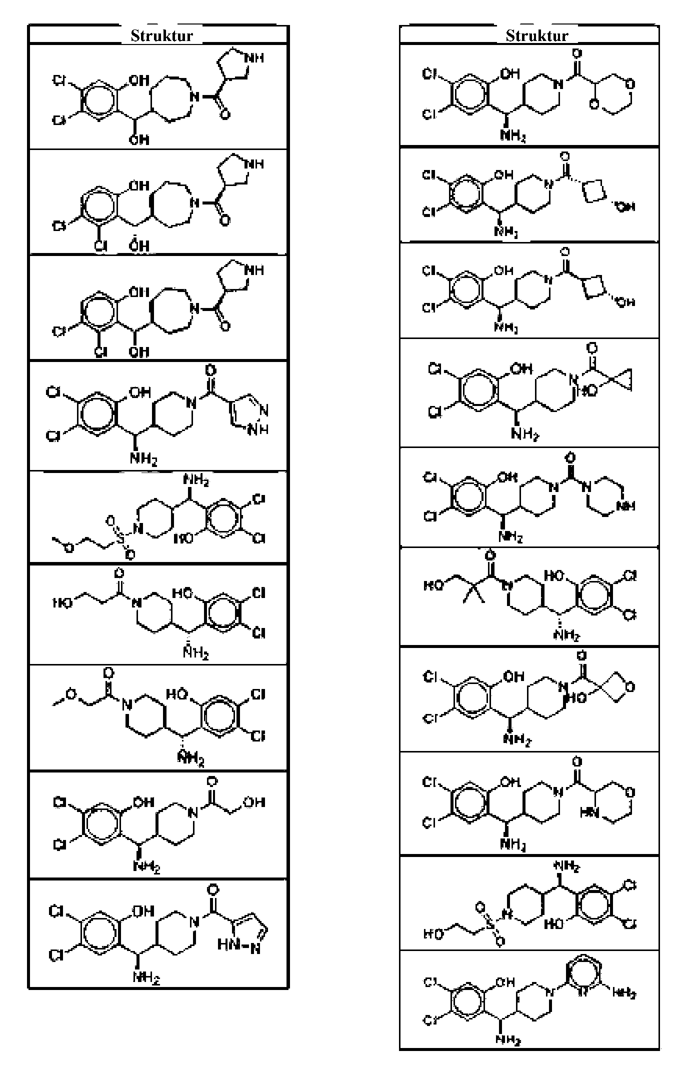

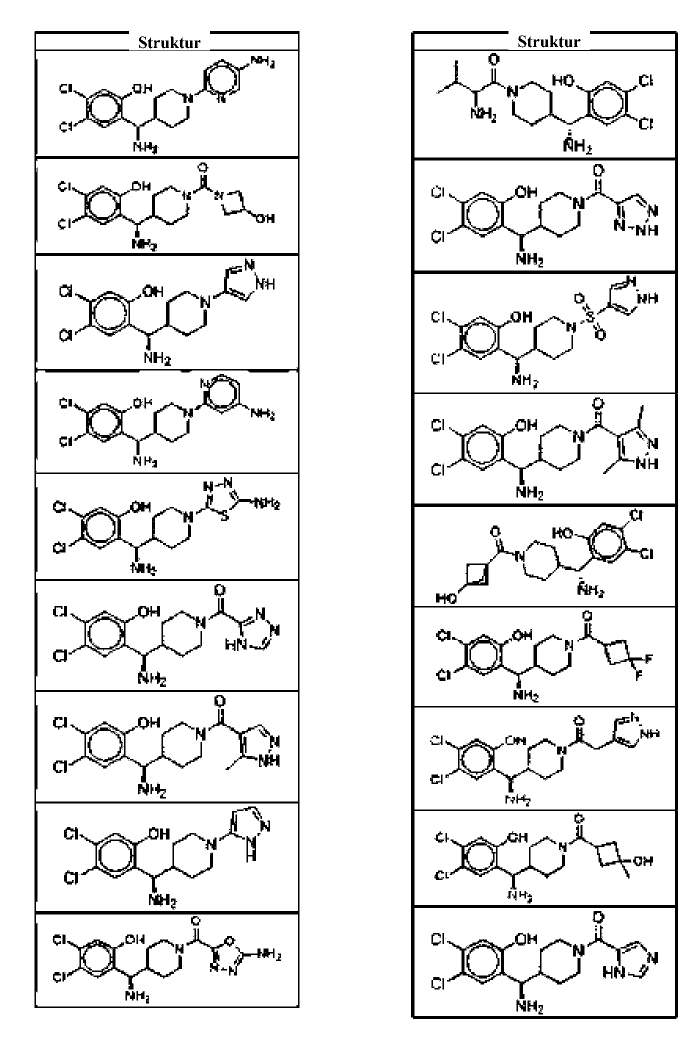

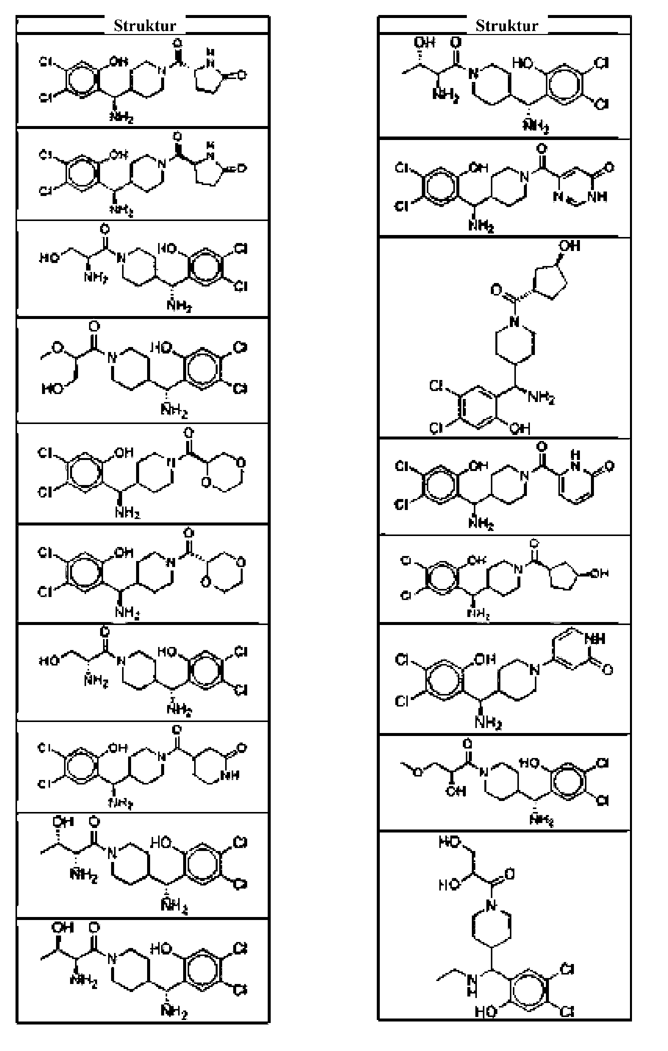

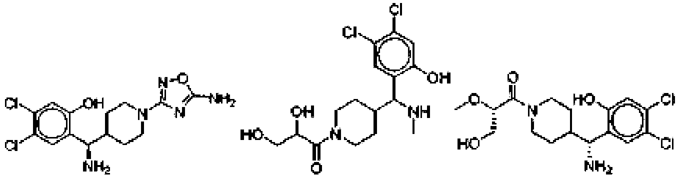

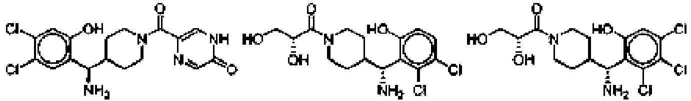

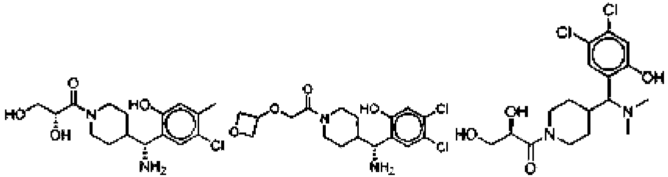

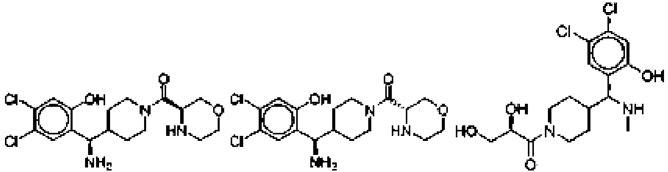

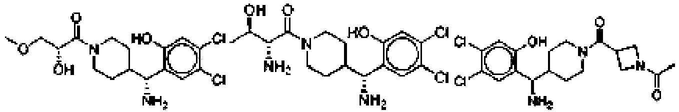

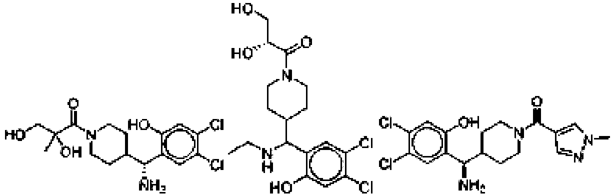

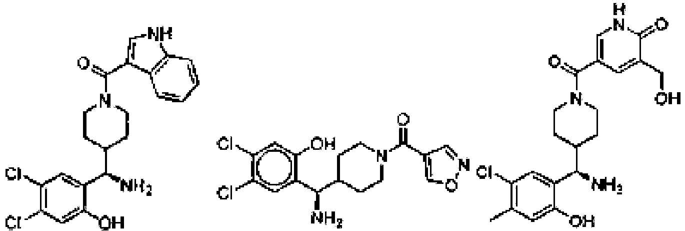

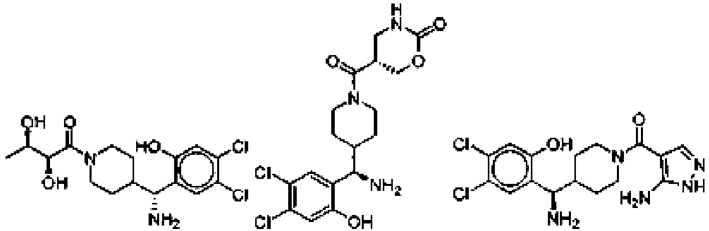

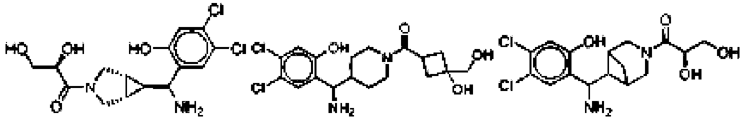

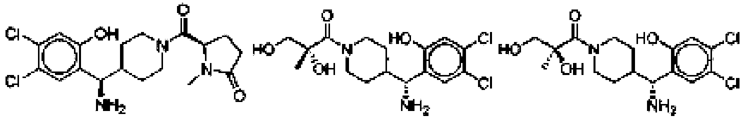

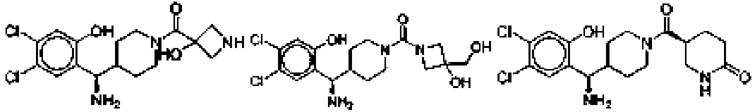

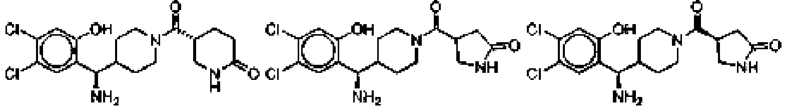

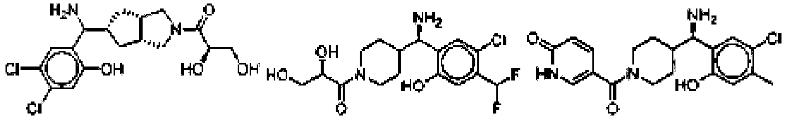

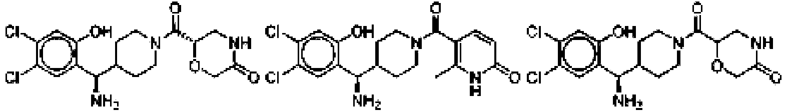

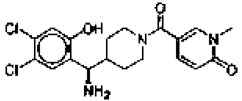

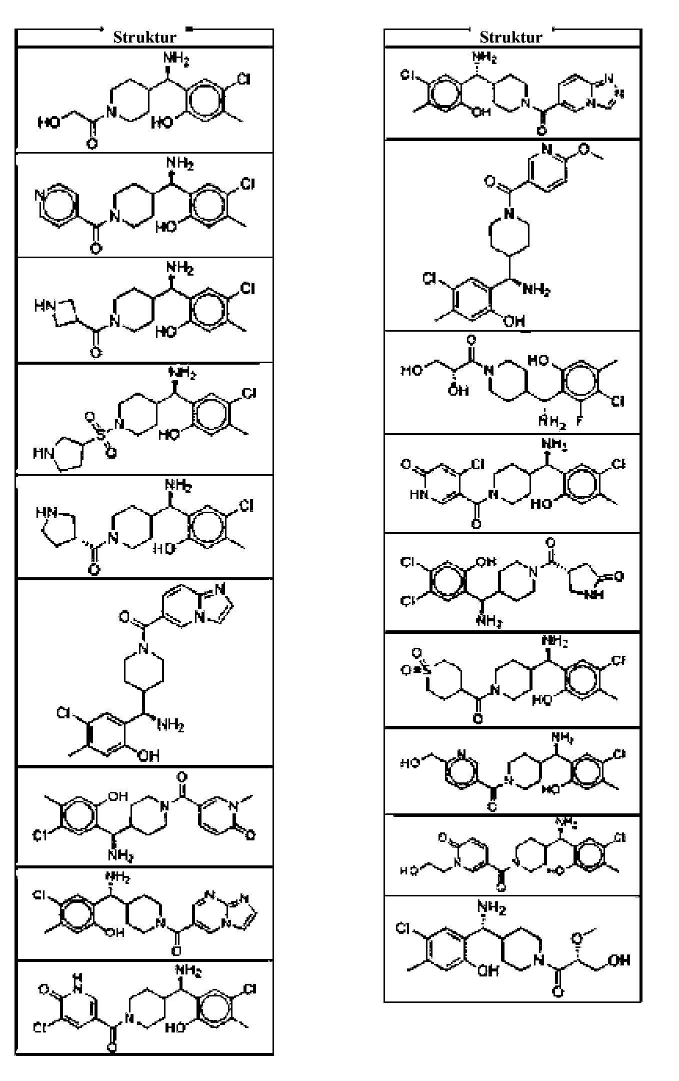

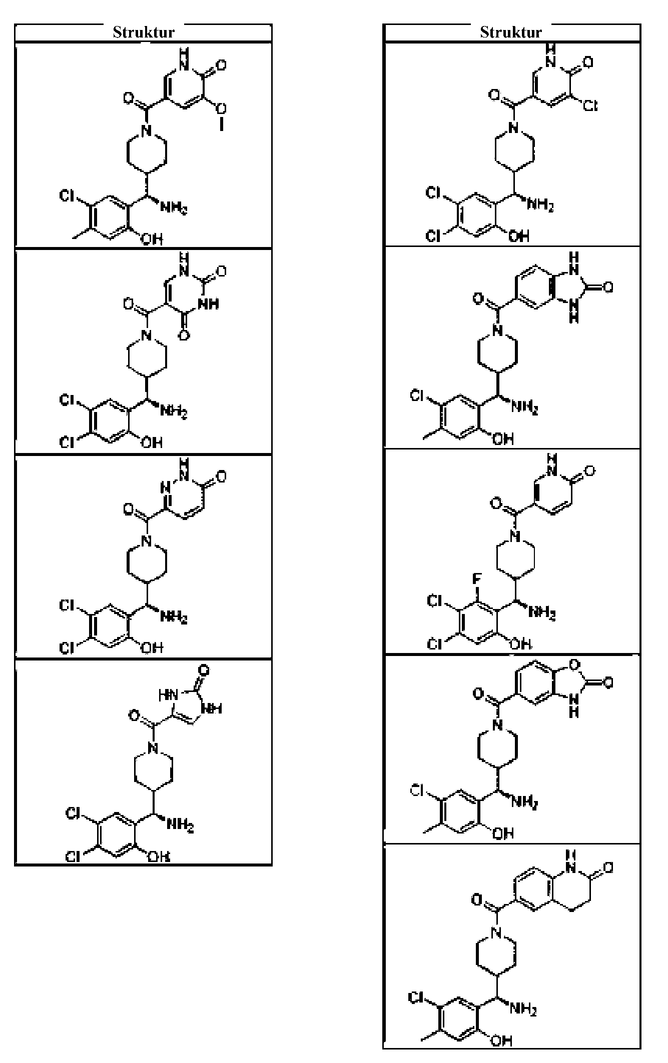

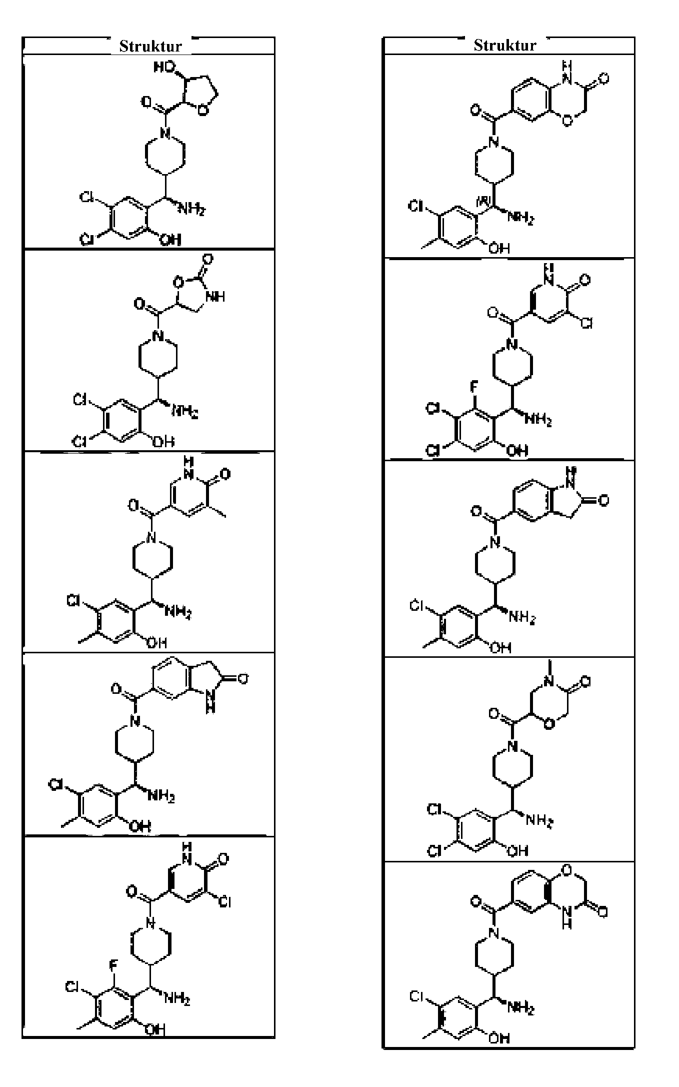

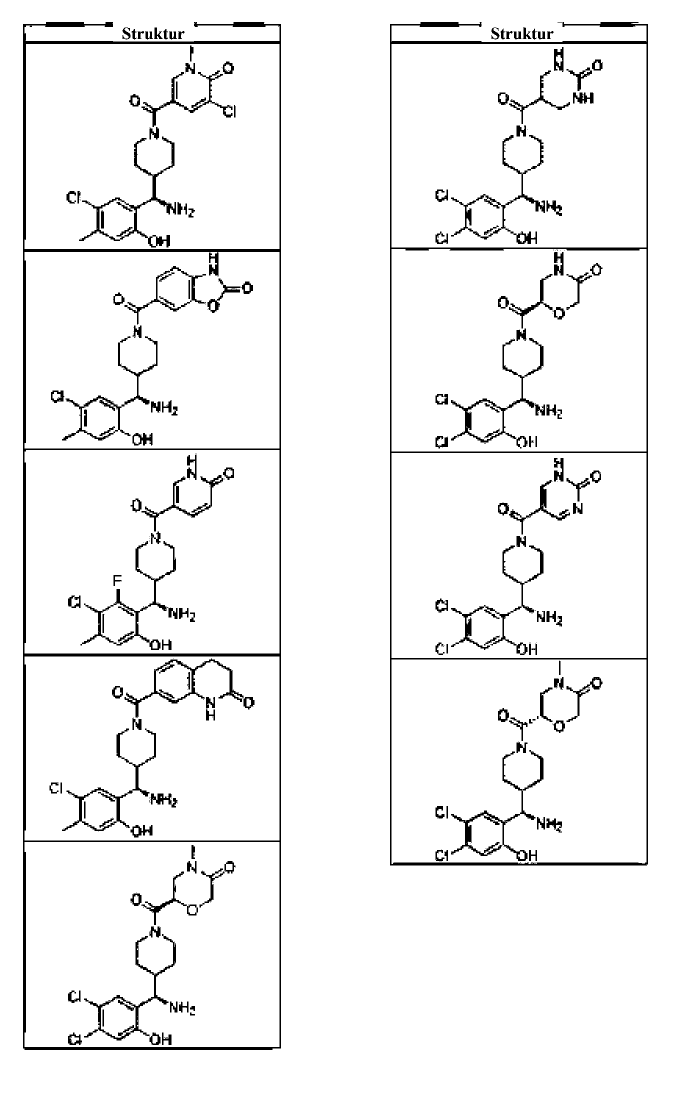

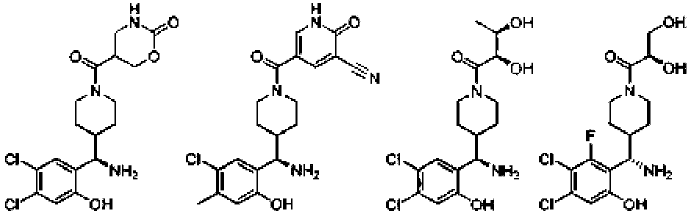

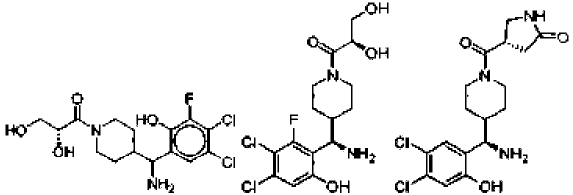

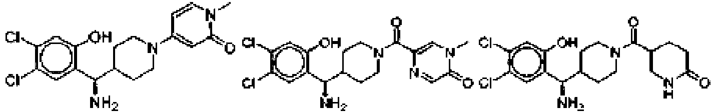

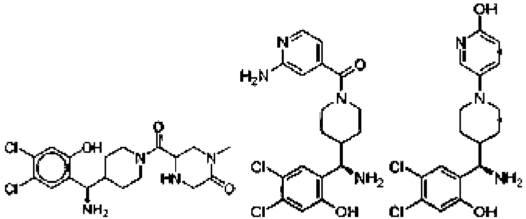

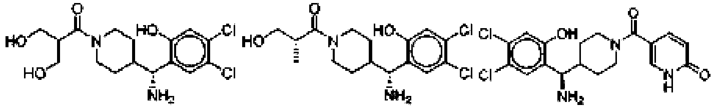

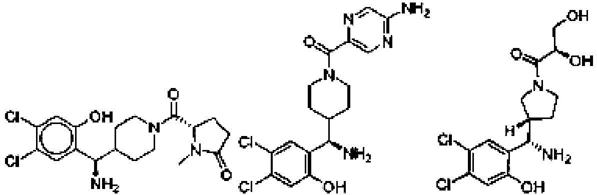

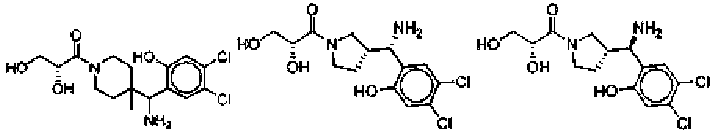

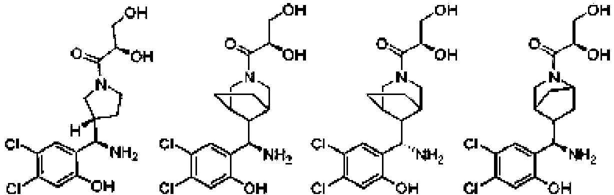

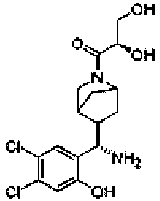

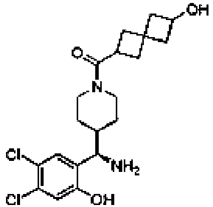

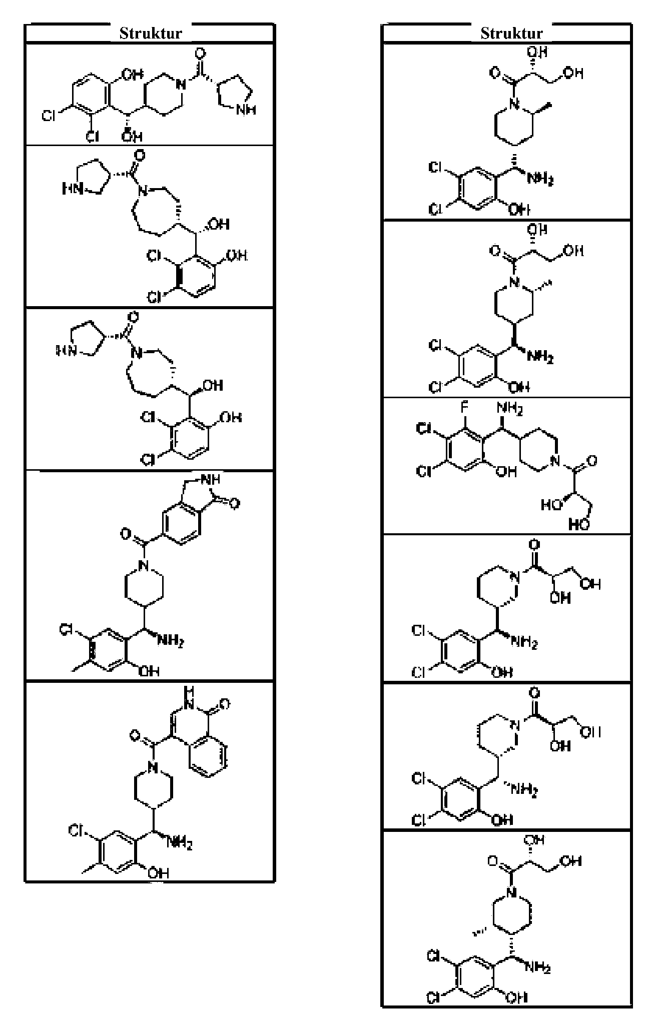

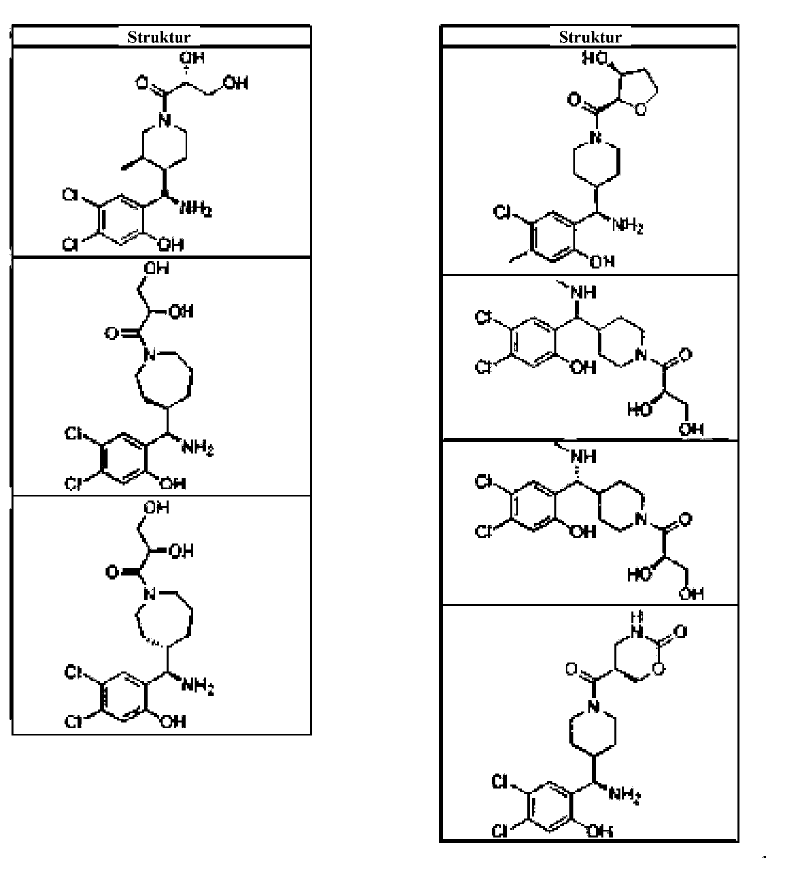

22. Pharmazeutische Zusammensetzung, umfassend mindestens eine Verbindung nach einem der Ansprüche 1 bis 21 oder ein pharmazeutisch zulässiges Salz davon und einen pharmazeutisch zulässigen Träger oder ein pharmazeutisch zulässiges Verdünnungsmittel.

23. Verbindung oder ein pharmazeutisch zulässiges Salz davon nach einem der Ansprüche 1 bis 21 zur Verwendung als Medikament.

24. Verbindung oder ein pharmazeutisch zulässiges Salz davon nach einem der Ansprüche 1 bis 21 zur Verwendung in einem Verfahren zum Behandeln eines Leidens in einer Säugetierart, das diese Behandlung erfordert, das Verfahren umfassend das Verabreichen an die Säugetierart einer therapeutisch wirksamen Menge der Verbindung oder eines pharmazeutisch zulässigen Salzes davon, wobei das Leiden ausgewählt ist aus der Gruppe bestehend aus Krebs, einer immunologischen Störung, einer Erkrankung des Zentralnervensystems (ZNS), einer entzündlichen Erkrankung, einer gastroenterologischen Erkrankung, einer Stoffwechselerkrankung, einer kardiovaskulären Erkrankung und einer Nierenerkrankung; optional wobei das Leiden folgendes ist:
(a) eine immunologische Störung, bei der es sich um eine Transplantatabstoßung oder eine Autoimmunerkrankung handelt, wobei die Autoimmunerkrankung optional rheumatoide Arthritis, Multiple Sklerose, systemischer Lupus erythematodes oder Typ-1-Diabetes mellitus ist, oder
(b) eine Erkrankung des Zentralnervensystems (ZNS), nämlich die Alzheimer-Krankheit;
(c) eine entzündliche Erkrankung, die sich in Form von Hautentzündungen, Arthritis, Psoriasis, Spondylitis, Parodontitis oder einer entzündlichen Neuropathie zeigt;
(d) einer gastroenterologischen Erkrankung, bei der es sich um eine entzündliche Darmerkrankung handelt; oder
(e) eine Stoffwechselstörung, wie Adipositas oder Typ-2-Diabetes mellitus; oder
(f) eine Herz-Kreislauf-Erkrankung, bei der es sich um einen ischämischen Schlaganfall handelt, oder
(g) eine Nierenerkrankung, wie chronische Nierenerkrankung, Nephritis oder chronisches Nierenversagen; oder
(h) die ausgewählt ist aus der Gruppe bestehend aus Krebs, Transplantatabstoßung, rheumatoide Arthritis, Multiple Sklerose, systemischer Lupus erythematodes, Typ-1-Diabetes mellitus, Alzheimer-Krankheit, entzündliche Hauterkrankung, entzündliche Neuropathie, Psoriasis, Spondylitis, Parodontitis, Morbus Crohn, Colitis ulcerosa, Adipositas, Typ-2-Diabetes mellitus, ischämischer Schlaganfall, chronische Nierenerkrankung, Nephritis, chronisches Nierenversagen sowie Kombinationen davon; und optional wobei die Säugetierart ein Mensch ist.

## Revendications

1. Composé de formule (I) ou sel pharmaceutiquement acceptable de celui-ci, 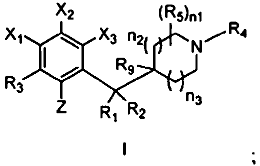 où
le fragment structurel 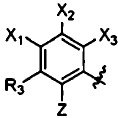 présente la structure de 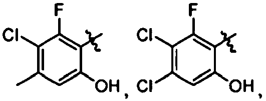 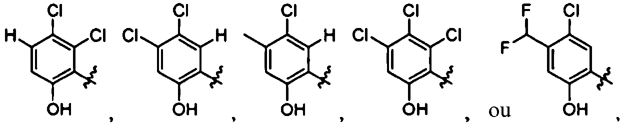 chacun étant substitué par R₃,
R₃ représente H, halogéno ou alkyle,
R₁ et R₂ représentent chacun indépendamment H, alkyle, (CR₆R₇)ₙ₄ORₐ, (CR₆R₇)ₙ₄NRₐR_{b}, (CR₆R₇)ₙ₄NRₐ(C=O)R_{b}, (CR₆R₇)ₙ₄NRₐSO₂R_{b} ou (CR₆R₇)ₙ₄CONRₐR_{b} ; au moins une occurrence de R₁ et R₂ représentant (CR₆R₇)ₙ₄NRₐR_{b}, (CR₆R₇)ₙ₄ORₐ, (CR₆R₇)ₙ₄NRₐ(C=)R_{b}, (CR₆R₇)ₙ₄NRₐSO₂R_{b} ou (CR₆R₇)ₙ₄CONRₐR_{b}, ou bien R₁, R₂ et l'atome de carbone auquel ils sont liés forment conjointement un carbocycle de 3 à 5 chaînons,
R₄ représente (CR₆R₇)ₙ₄(C=O)R_{c}, (C=O)(CR₆R₇)ₙ₄R_{c}, (CR₆R₇)ₙ₄OR_{c}, (CR₆R₇)ₙ₄COOR_{c}, (CR₆R₇)ₙ₄NR_{c}(C=O)R_{d}, (C=O)(CR₆R₇)ₙ₄OR_{c} ou (CR₆R₇)ₙ₄SO₂R_{c}, un hétérocycle saturé éventuellement substitué, un aryle éventuellement substitué, un hétéroaryle éventuellement substitué, un cycloalkyle éventuellement substitué, ou un -cycloalkyl-alkyle éventuellement substitué,
chaque occurrence de R₅ représente indépendamment H, alkyle, cycloalkyle ou oxo,
ou deux groupes R₅, pris conjointement avec le ou les atomes de carbone auxquels ils sont liés, forment un carbocycle saturé de 3 à 7 chaînons éventuellement substitué,
ou deux groupes R₅ sont liés à des atomes de carbone différents sur le cycle et, pris conjointement, forment une liaison ou une chaîne alkyle contenant 1 à 3 carbones,
chaque occurrence de R₆ et R₇ représente indépendamment H, alkyle ou cycloalkyle,
chaque occurrence de Rₐ et R_{b} représente indépendamment H, alkyle, cycloalkyle, hétérocycle saturé, aryle ou hétéroaryle, ou bien Rₐ et R_{b}, conjointement avec l'atome d'azote auquel ils sont liés, forment un hétérocycle éventuellement substitué comprenant l'atome d'azote et 0 à 3 hétéroatomes supplémentaires chacun choisis dans le groupe constitué par N, O et S,
chaque occurrence de R_{c} et R_{d} est indépendamment H, alkyle, ou alkyle substitué par 1 à 4 substituants, chacun étant indépendamment halogéno, OR₈ ou N(R₈)₂, alcényle, cycloalkyle éventuellement substitué, bicycloalkyle éventuellement substitué, spiroalkyle éventuellement substitué, hétérocycle saturé éventuellement substitué, aryle éventuellement substitué, hétéroaryle éventuellement substitué, -alkyl-aryle éventuellement substitué, -alkyl-hétéroaryle éventuellement substitué, -alkyl-hétérocycle éventuellement substitué, -alkyl-cycloalkyle éventuellement substitué, ou -cycloalkyl-alkyle éventuellement substitué ; ou bien R_{c} et R_{d}, conjointement avec l'atome d'azote auquel ils sont liés, forment un hétérocycle éventuellement substitué comprenant l'atome d'azote et 0 à 3 hétéroatomes supplémentaires chacun choisis dans le groupe constitué par N, O et S,
chaque occurrence de R₈ est indépendamment H, alkyle ou un hétérocycle éventuellement substitué, ou bien les deux groupes R₈, conjointement avec l'atome d'azote auquel ils sont liés, forment un hétérocycle éventuellement substitué comprenant l'atome d'azote et 0 à 3 hétéroatomes supplémentaires chacun choisis dans le groupe constitué par N, O et S,
R₉ represente H, alkyle, halogéno ou (CR₆R₇)ₙ₄OR_{b},
les alkyle, cycloalkyle, spiroalkyle, bicycloalkyle, hétérocycle, aryle et hétéroaryle de R₁, R₂, R₃, R₄, R₅, R₆, R₇, R₉, Rₐ, R_{b}, R_{c} et R_{d}, le cas échéant, sont éventuellement substitués par 1 à 4 substituants chacun choisis indépendamment dans le groupe constitué par alkyle, cycloalkyle, cycloalkyle halogéné, alkyle halogéné, halogéno, CN, -(CH₂)₀₋₂OR₈, N(R₈)₂, (C=O)(alkyle en C₁₋₄), (C=O)N(R₈)₂ et oxo lorsque la valence le permet,
chaque occurrence de n₁ représente indépendamment un nombre entier de 0 à 3 lorsque la valence le permet,
chaque occurrence de n₂ et n₃ représente indépendamment un nombre entier égal à 1, 2 ou 0, et
chaque occurrence de n₄ représente indépendamment un nombre entier de 0 à 3.

2. Composé ou sel pharmaceutiquement acceptable de celui-ci selon la revendication 1, dans lequel :
(a) chaque occurrence de n₂ et n₃ représente indépendamment un nombre entier égal à 0 ou 1, et
(b) le motif structurel 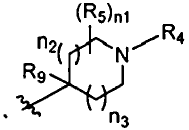 présente la structure de 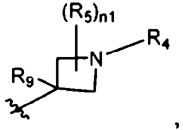 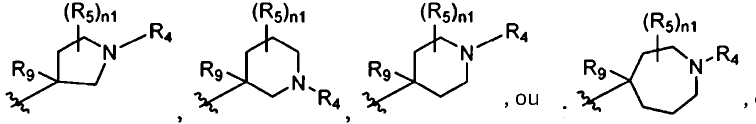 ou
(c) le motif structurel 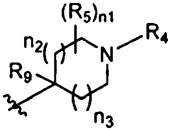 présente la structure de

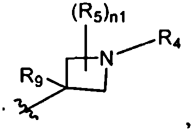

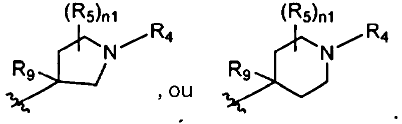

3. Composé ou sel pharmaceutiquement acceptable de celui-ci selon la revendication 1 ou 2, dans lequel le motif structurel 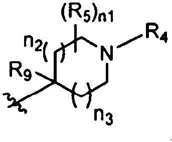 présente la structure de

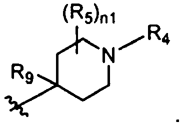

4. Composé ou sel pharmaceutiquement acceptable de celui-ci selon l'une quelconque des revendications 1 à 3, dans lequel au moins une occurrence de R₁ et R₂ représente (CR₆R₇)ₙ₄ORₐ, (CR₆R₇)ₙ₄NRₐR_{b}, (CR₆R₇)ₙ₄NRₐ(C=O)R_{b}, (CR₆R₇)ₙ₄NRₐSO₂R_{b} ou (CR₆R₇)ₙ₄CONRₐR_{b}.

5. Composé ou sel pharmaceutiquement acceptable de celui-ci selon la revendication 4, dans lequel au moins une occurrence de R₁ et R₂ représente ORₐ ou NRₐR_{b}.

6. Composé ou sel pharmaceutiquement acceptable de celui-ci selon l'une quelconque des revendications 1 à 3, dans lequel R₁, R₂ et l'atome de carbone auquel ils sont liés forment conjointement un carbocycle de 3 à 5 chaînons.

7. Composé ou sel pharmaceutiquement acceptable de celui-ci selon l'une quelconque des revendications 1 à 6, dans lequel R₄ représente (CR₆R₇)ₙ₄OR_{c}, (CR₆R₇)ₙ₄COR_{c}, (C=O)(CR₆R₇)ₙ₄R_{c}, (CR₆R₇)ₙ₄COOR_{c}, (CR₆R₇)ₙ₄NR_{c}(C=O)R_{d}, (C=O)(CR₆R₇)ₙ₄OR_{c} ou (CR₆R₇)ₙ₄SO₂R_{c}.

8. Composé ou sel pharmaceutiquement acceptable de celui-ci selon la revendication 7, dans lequel R₄ représente (CR₆R₇)₂OR_{c}, (C=O)R_{c}, (C=O)(CR₆R₇)₁₋₂R_{c}, COOR_{c}, (CR₆R₇)₁₋₂NR_{c}(C=O)R_{d}, (C=O)(CR₆R₇)₁₋₂OR_{c} ou SO₂R_{c}.

9. Composé ou sel pharmaceutiquement acceptable de celui-ci selon la revendication 8, dans lequel R₄ représente (CH₂)₂OH, (CH₂)₂OMe, (C=O)H, (C=O)Me, (C=O)CH₂OH, (C=O)CH₂OMe, (C=O)Et, (C=O)Ph, (C=O)isopropyle, (C=O)CH₂NH₂, (C=O)CH₂NHMe, (C=O)CH(OH)CH₂OH, (C=O)CH(OMe)CH₂OH, (C=O)CH(OH)CH₂OMe, (C=O)OMe, SO₂Me, SO₂Et, SO₂CH₂OH ou SO₂CH₂OMe.

10. Composé ou sel pharmaceutiquement acceptable de celui-ci selon la revendication 8, dans lequel R₄ représente (C=O)R_{c}, (C=O)(CR₆R₇)₁₋₂R_{c}, (C=O)(CR₆R₇)₁₋₂OR_{c} ou SO₂R_{c} ; R_{c} étant choisi dans le groupe constitué par H, alkyle, alkyle substitué par 1 à 4 substituants chacun choisis indépendamment dans le groupe constitué par halogéno, OR₈ et N(R₈)₂, alcényle, cycloalkyle éventuellement substitué, bicycloalkyle éventuellement substitué, spiroalkyle éventuellement substitué, hétérocycle saturé éventuellement substitué, aryle éventuellement substitué, hétéroaryle éventuellement substitué, -alkyl-aryle éventuellement substitué, -alkyl-hétéroaryle éventuellement substitué, -alkyl-hétérocycle éventuellement substitué, -alkyl-cycloalkyle éventuellement substitué et -cycloalkyl-alkyle éventuellement substitué.

11. Composé ou sel pharmaceutiquement acceptable de celui-ci selon l'une quelconque des revendications 1 à 6, dans lequel R₄ représente (CR₆R₇)₂OR_{c}, (C=O)R_{c}, (C=O)(CR₆R₇)₁₋₂R_{c}, COOR_{c}, (CR₆R₇)₁₋₂NR_{c}(C=O)R_{d}, (C=O)(CR₆R₇)₁₋₂OR_{c} ou SO₂R_{c}, et
(a) R_{c} ou R_{d} représente H, Me, Et, 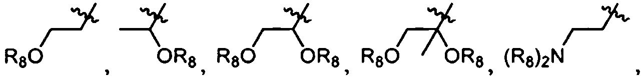 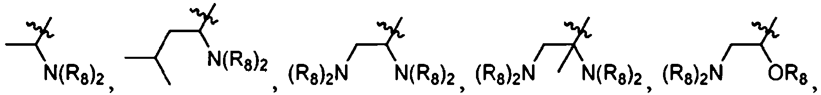 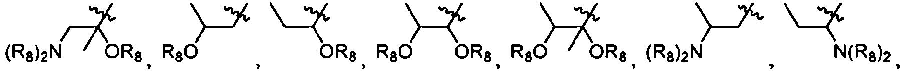 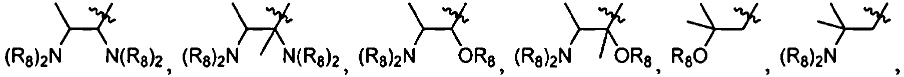 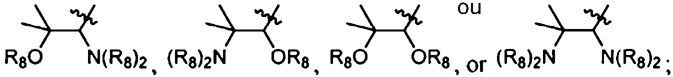 ou or
(b) R_{c} ou R_{d} représente un hétérocycle choisi dans le groupe constitué par 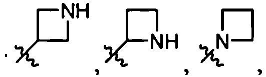 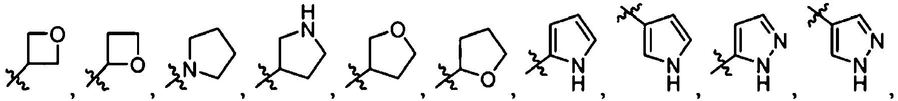 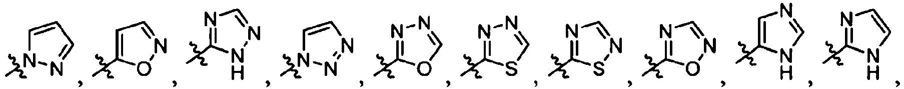 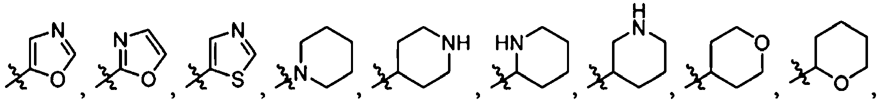 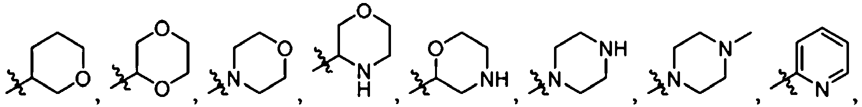 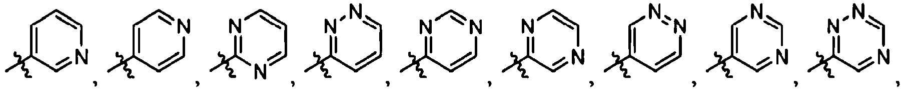 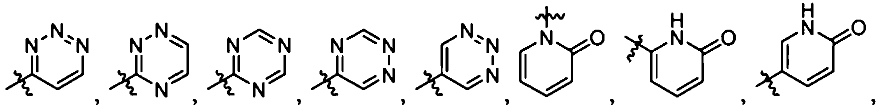 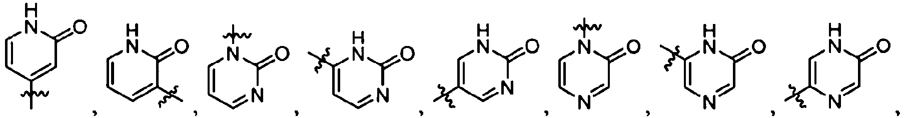 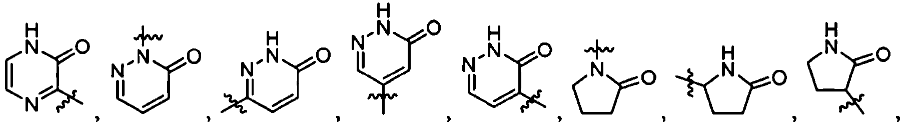 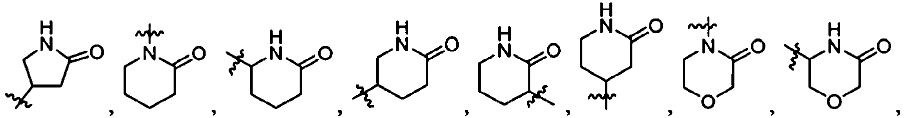 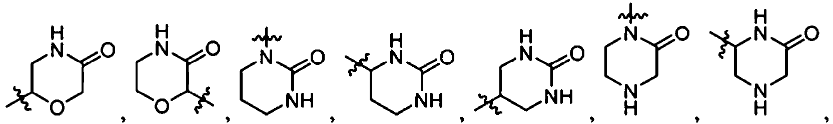 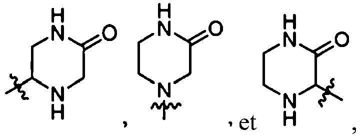 l'hétérocycle étant éventuellement substitué par un ou plusieurs cyano, cycloalkyle, alkyle fluoré, cycloalkyle fluoré, halogéno, OH, NH₂, oxo ou (C=O)(alkyle en C₁₋₄) lorsque la valence le permet, ou
(c) R_{c} représente cycloalkyle, spiroalkyle ou bicycloalkyle, chacun étant éventuellement substitué par 1 à 4 substituants chacun choisis indépendamment dans le groupe constitué par alkyle, halogéno, CN, -(CH₂)₀₋₂OR₈, N(R₈)₂, (C=O)N(R₈)₂ et oxo lorsque la valence le permet, ou
(d) R_{c} représente 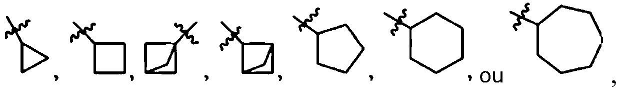 chacun étant éventuellement substitué par 1 à 4 substituants chacun choisis indépendamment dans le groupe constitué par alkyle, halogéno, CN, -(CH₂)₀₋₂OR₈, N(R₈)₂, (C=O)N(R₈)₂ et oxo lorsque la valence le permet.

12. Composé ou sel pharmaceutiquement acceptable de celui-ci selon l'une quelconque des revendications 1 à 6, dans lequel :
(a) R₄ représente un hétérocycle saturé éventuellement substitué, aryle éventuellement substitué, hétéroaryle éventuellement substitué, cycloalkyle éventuellement substitué ou - cycloalkyl-alkyle éventuellement substitué, ou
(b) R₄ est un hétérocycle sélectionné dans le groupe constitué par 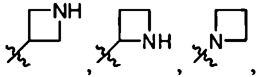 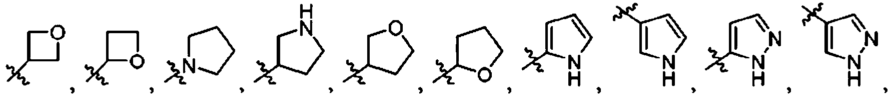 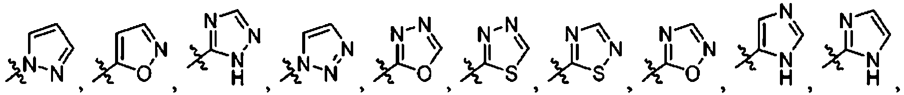 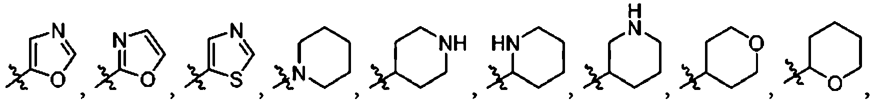 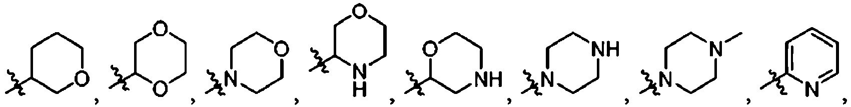 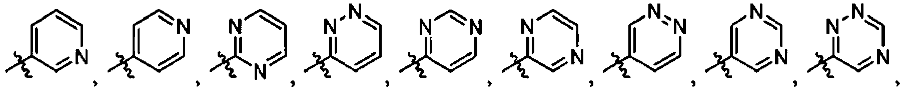 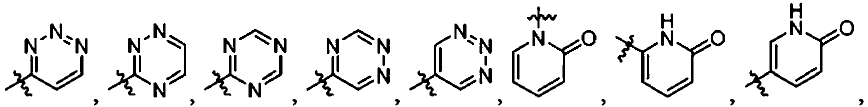 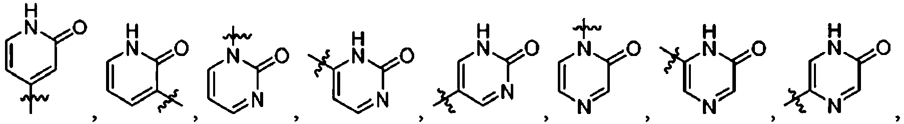 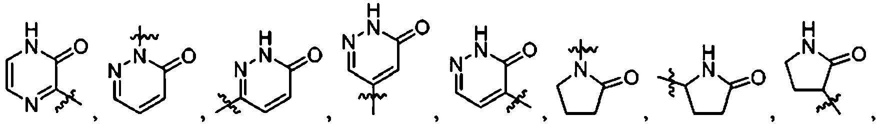 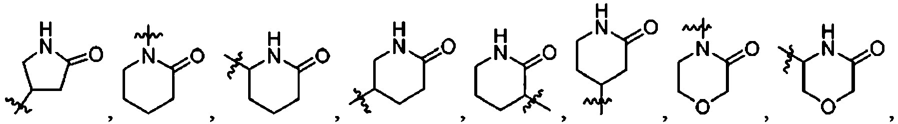 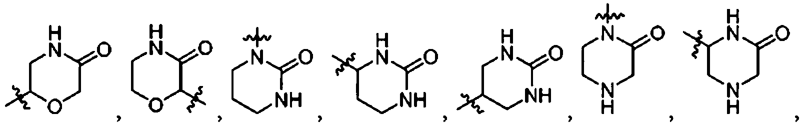 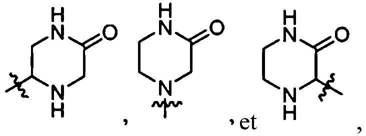 l'hétérocycle étant éventuellement substitué par un ou plusieurs cyano, cycloalkyle, alkyle fluoré, cycloalkyle fluoré, halogéno, OH, NH₂, oxo ou (C=O)(alkyle en C₁₋₄) lorsque la valence le permet, ou
(c) R₄ représente cycloalkyle éventuellement substitué par 1 à 4 substituants chacun choisis indépendamment dans le groupe constitué par alkyle, halogéno, CN, -(CH₂)₀₋₂OR₈, N(R₈)₂, (C=O)N(R₈)₂ et oxo lorsque la valence le permet, ou
(d) R₄ représente 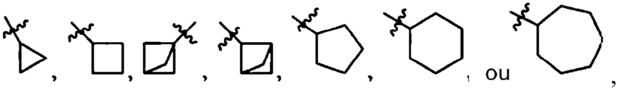 chacun étant éventuellement substitué par 1 à 4 substituants chacun choisis indépendamment dans le groupe constitué par alkyle, halogéno, CN, -(CH₂)₀₋₂OR₈, N(R₈)₂, (C=O)N(R₈)₂ et oxo lorsque la valence le permet.

13. Composé ou sel pharmaceutiquement acceptable de celui-ci selon l'une quelconque des revendications 1 à 6, dans lequel R₄ représente 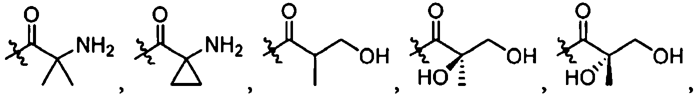 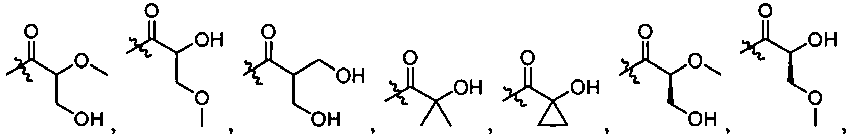 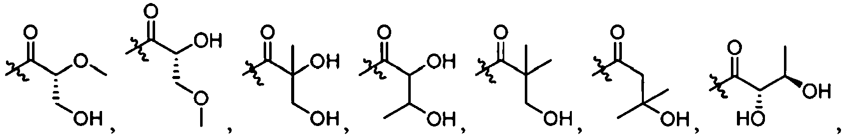 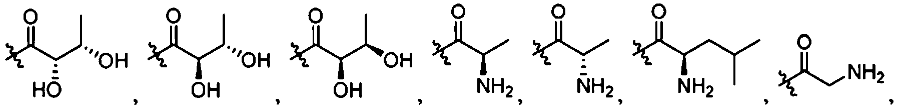 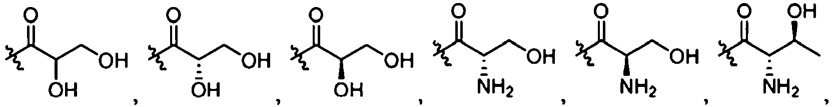 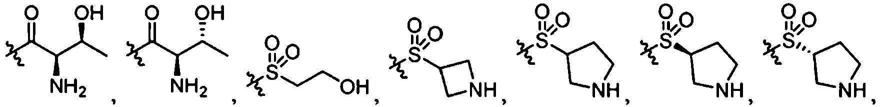 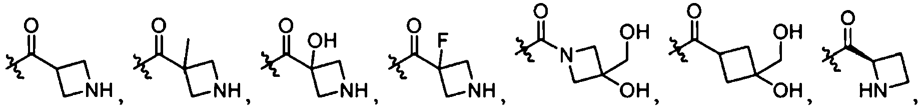 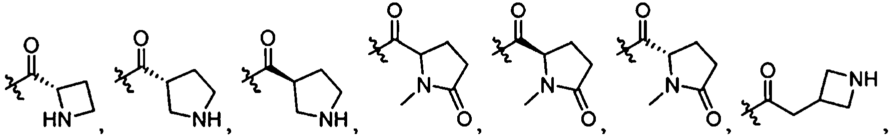 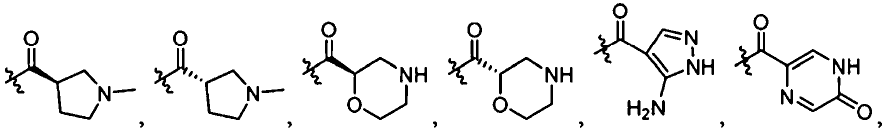 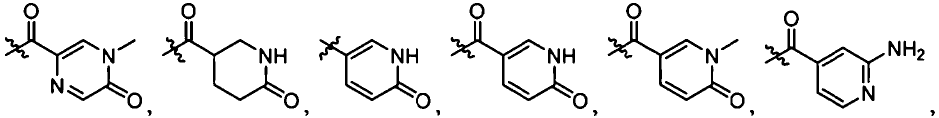 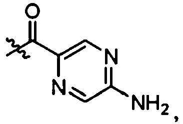 ou un de ses tautomères.

14. Composé ou sel pharmaceutiquement acceptable de celui-ci selon l'une quelconque des revendications 1 à 13, dans lequel :
(a) au moins une occurrence de R₅ représente H, alkyle ou cycloalkyle, ou
(b) au moins une occurrence de R₅ représente oxo, ou
(c) deux groupes R₅ sont liés à des atomes de carbone différents sur le cycle et, pris conjointement, forment une liaison ou une chaîne alkyle contenant 1 à 3 carbones, ou
(d) deux groupes R₅, pris conjointement avec le ou les atomes de carbone auxquels ils sont liés, forment un carbocycle saturé de 3 à 7 chaînons éventuellement substitué.

15. Composé ou sel pharmaceutiquement acceptable de celui-ci selon l'une quelconque des revendications précédentes, dans lequel :
(a) Rₐ et/ou R_{b} représentent indépendamment H, alkyle, cycloalkyle, hétérocycle saturé, aryle ou hétéroaryle, ou
(b) Rₐ et R_{b}, conjointement avec l'atome d'azote auquel ils sont liés, forment un hétérocycle éventuellement substitué comprenant l'atome d'azote et 0 à 3 hétéroatomes supplémentaires chacun choisis dans le groupe constitué par N, O et S.

16. Composé ou sel pharmaceutiquement acceptable de celui-ci selon l'une quelconque des revendications précédentes, dans lequel :
(a) chaque occurrence de R₆ et R₇ représente indépendamment H ou alkyle, et/ou
(b) R₉ représente (i) H, alkyle ou halogéno, ou (ii) (CR₆R₇)ₙ₄OR_{b}, ou (iii) H, F ou OH.

17. Composé ou sel pharmaceutiquement acceptable de celui-ci selon l'une quelconque des revendications 1 à 16, dans lequel R₃ représente H ou alkyle.

18. Composé ou sel pharmaceutiquement acceptable de celui-ci selon l'une quelconque des revendications 1 à 16, dans lequel R₃ représente halogéno.

19. Composé ou sel pharmaceutiquement acceptable de celui-ci selon l'une quelconque des revendications précédentes, dans lequel :
(a) au moins une occurrence de R₈ représente H, alkyle ou un hétérocycle éventuellement substitué, R₈ représentant éventuellement H, Me, Et, Pr, Bu ou un hétérocycle choisi dans le groupe constitué par 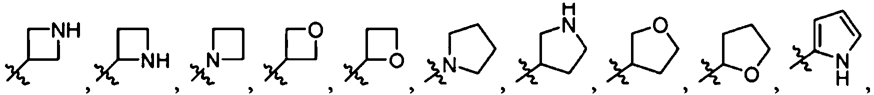 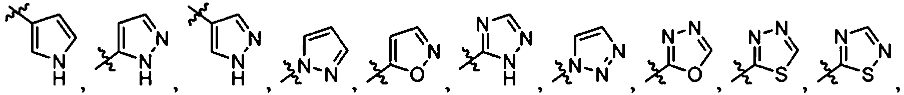 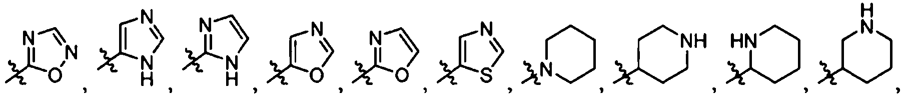 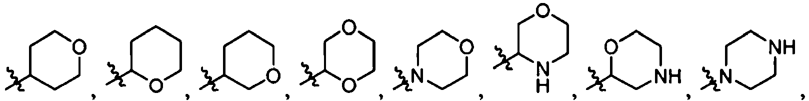 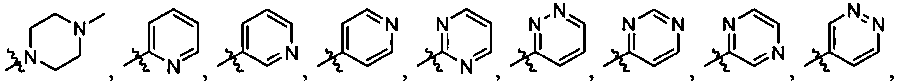 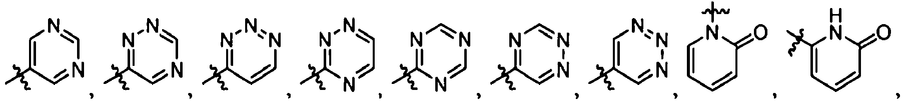 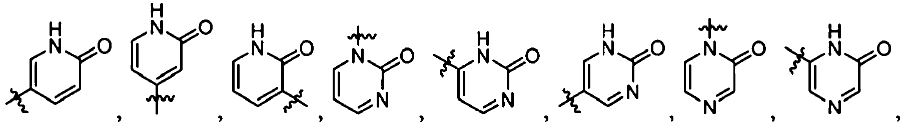 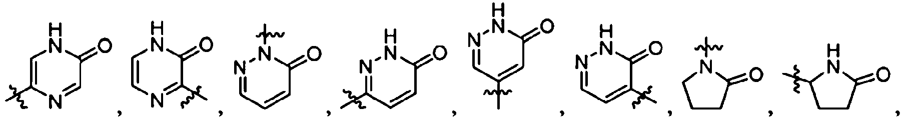 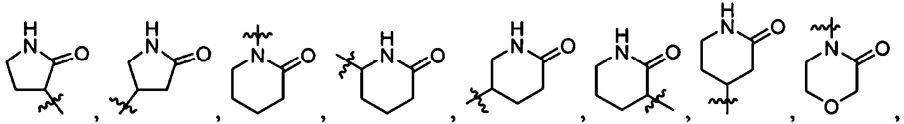 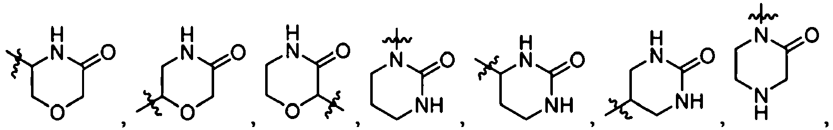 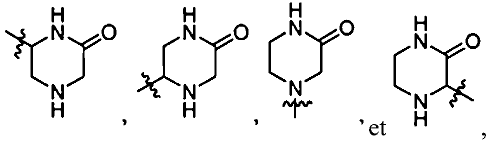 l'hétérocycle étant éventuellement substitué par un ou plusieurs cyano, cycloalkyle, alkyle fluoré, cycloalkyle fluoré, halogéno, OH, NH₂, oxo ou (C=O)(alkyle en C₁₋₄) lorsque la valence le permet, ou
(b) les deux groupes R₈, conjointement avec l'atome d'azote auquel ils sont liés, forment un hétérocycle éventuellement substitué comprenant l'atome d'azote et 0 à 3 hétéroatomes supplémentaires chacun choisis dans le groupe constitué par N, O et S.

20. Composé ou sel pharmaceutiquement acceptable de celui-ci selon l'une quelconque des revendications 1 à 6, dans lequel :
(a) au moins une occurrence de R_{c} ou R_{d} représente indépendamment H, alkyle, alkyle substitué par 1 à 4 substituants, chacun indépendamment choisi dans le groupe constitué par halogéno, OR₈ et N(R₈)₂, alcényle, cycloalkyle éventuellement substitué, bicycloalkyle éventuellement substitué, spiroalkyle éventuellement substitué, hétérocycle saturé éventuellement substitué, aryle éventuellement substitué, hétéroaryle éventuellement substitué, -alkyl-aryle éventuellement substitué, -alkyl-hétéroaryle éventuellement substitué, -alkyl-hétérocycle éventuellement substitué, -alkyl-cycloalkyle éventuellement substitué, ou - cycloalkyl-alkyle éventuellement substitué, ou
(b) au moins une occurrence de R_{c} ou R_{d} est indépendamment H, Me, Et, 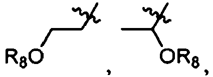 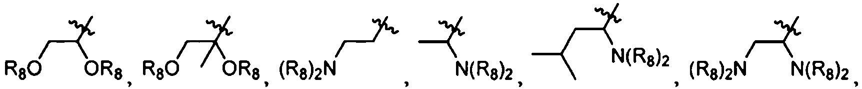 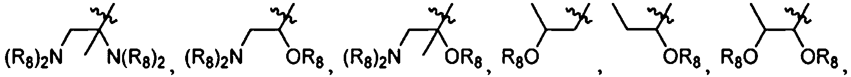 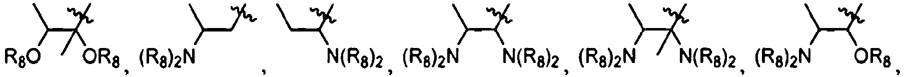 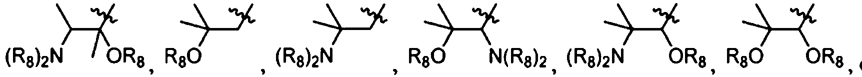 , ou or

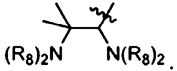

21. Composé ou sel pharmaceutiquement acceptable de celui-ci selon la revendication 1, ledit composé étant choisi dans le groupe constitué par les composés suivants et sels pharmaceutiquement acceptables de ceux-ci

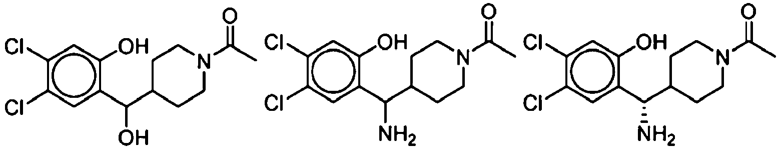

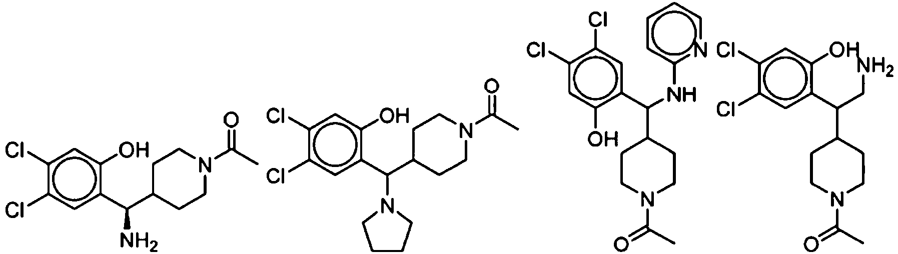

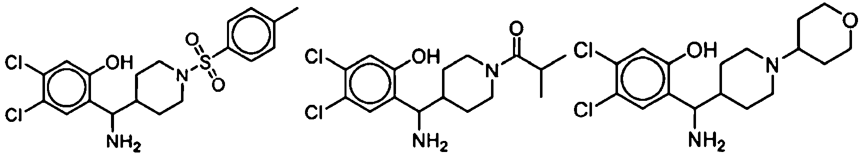

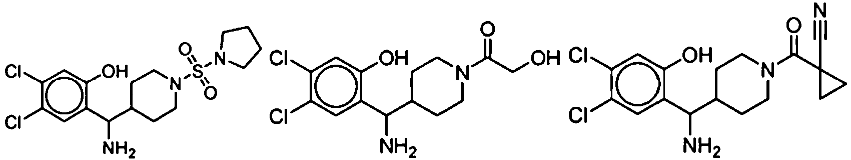

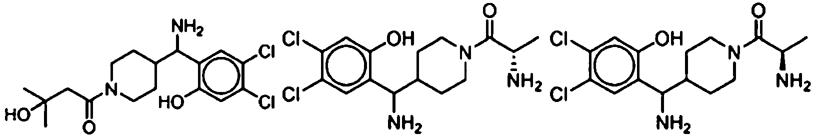

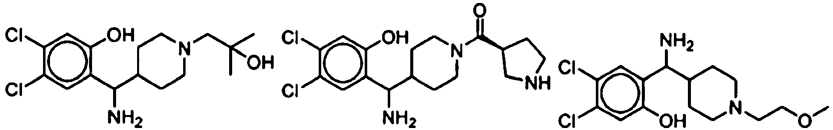

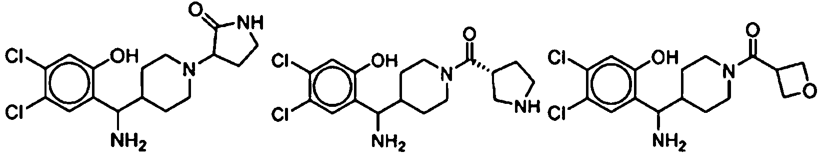

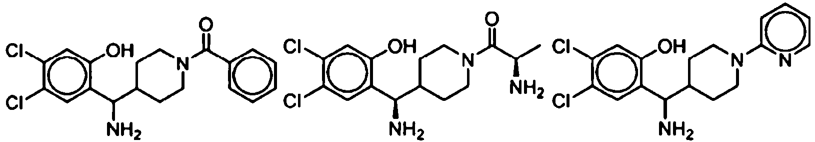

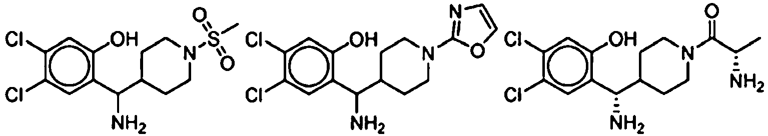

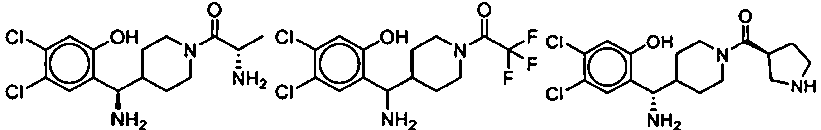

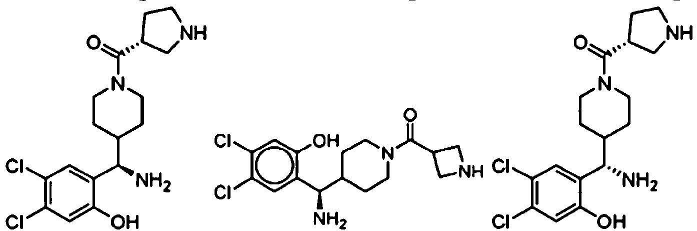

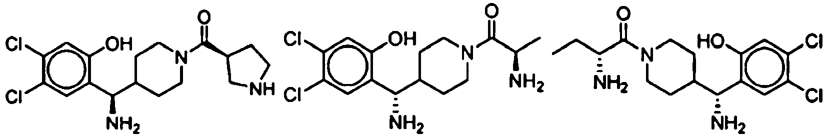

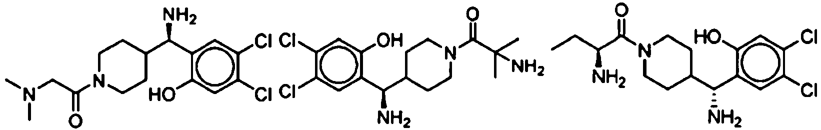

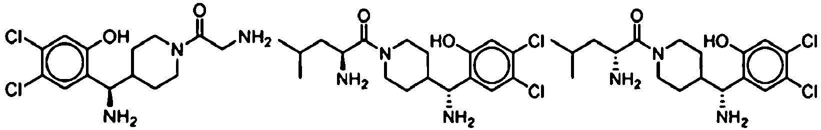

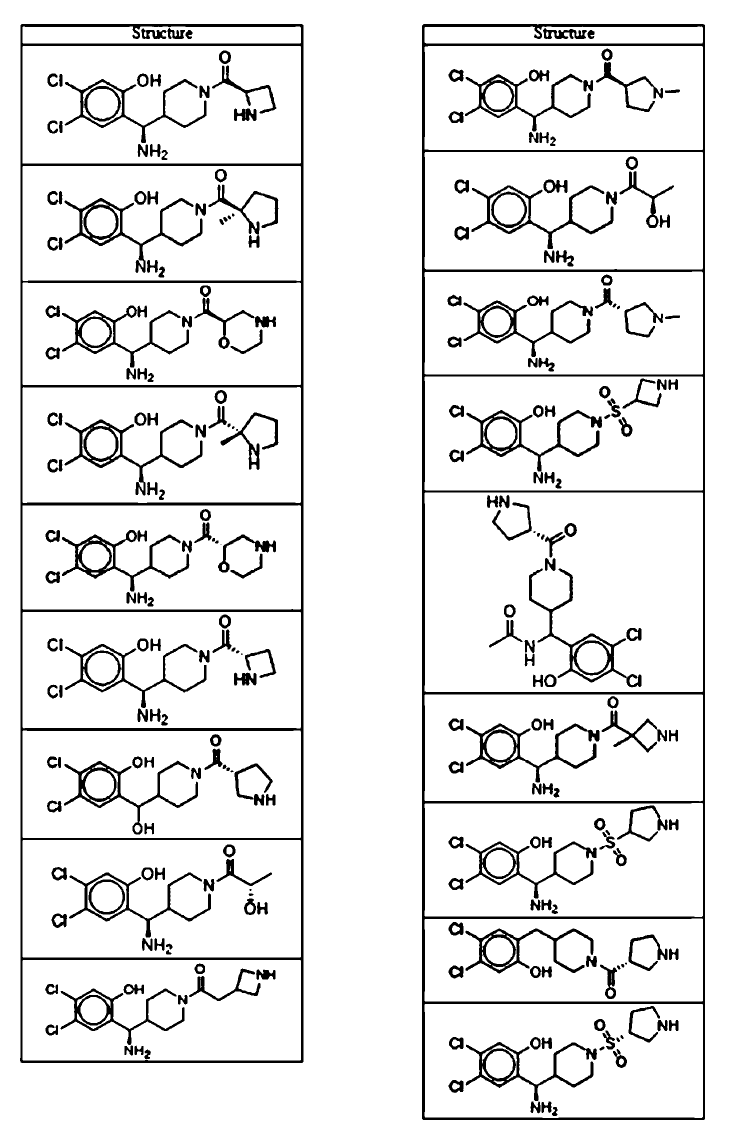

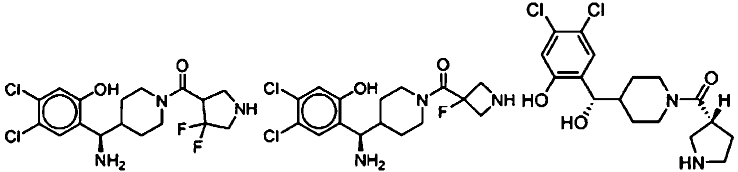

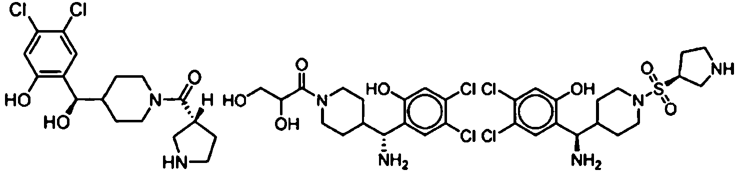

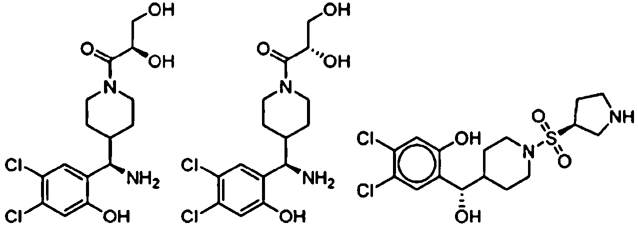

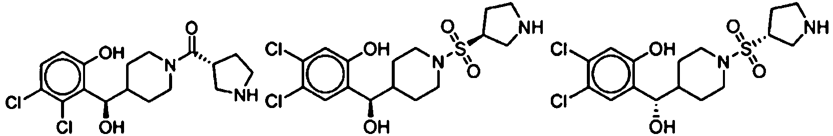

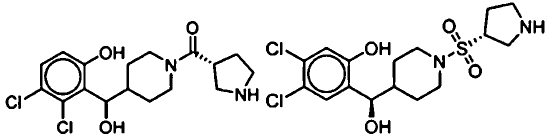

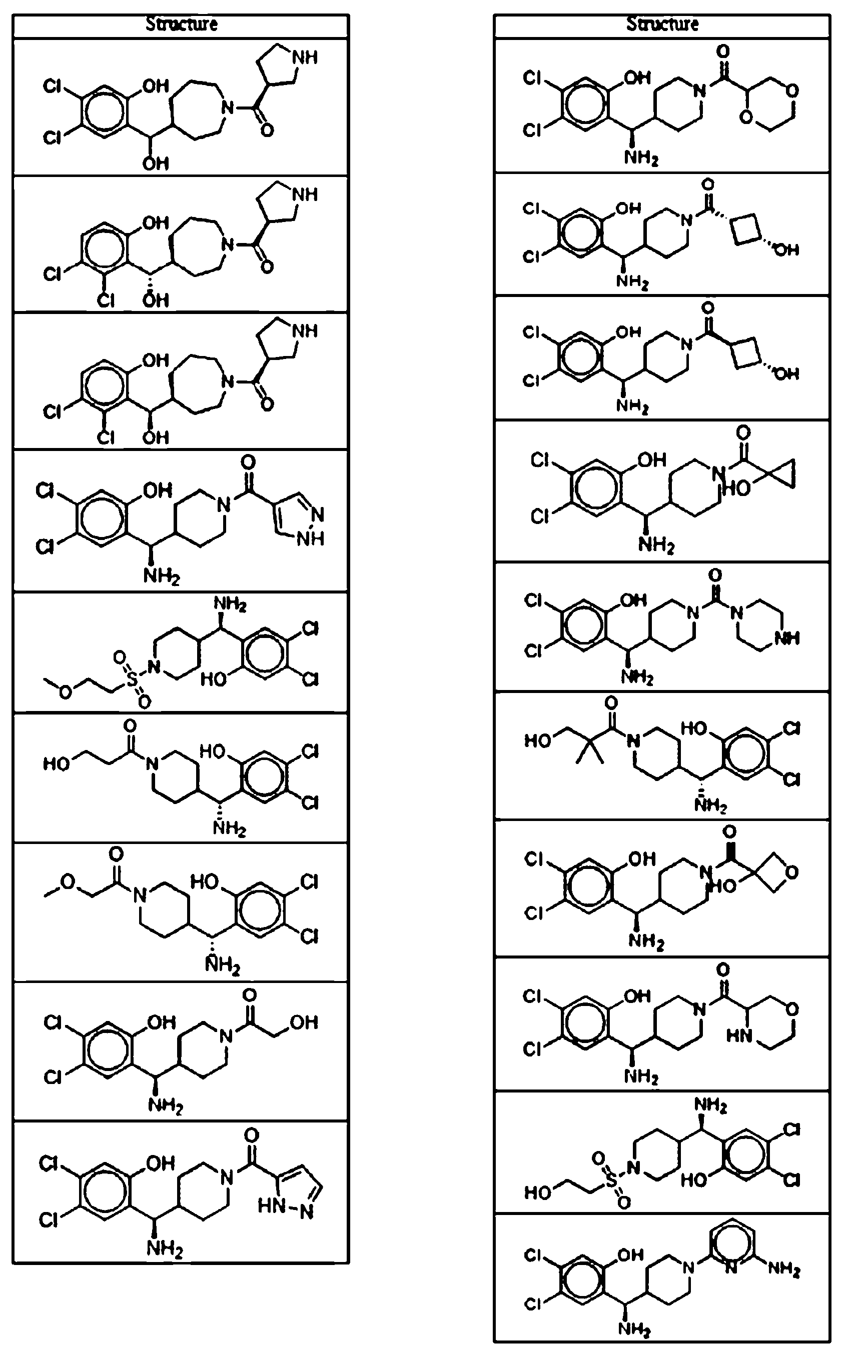

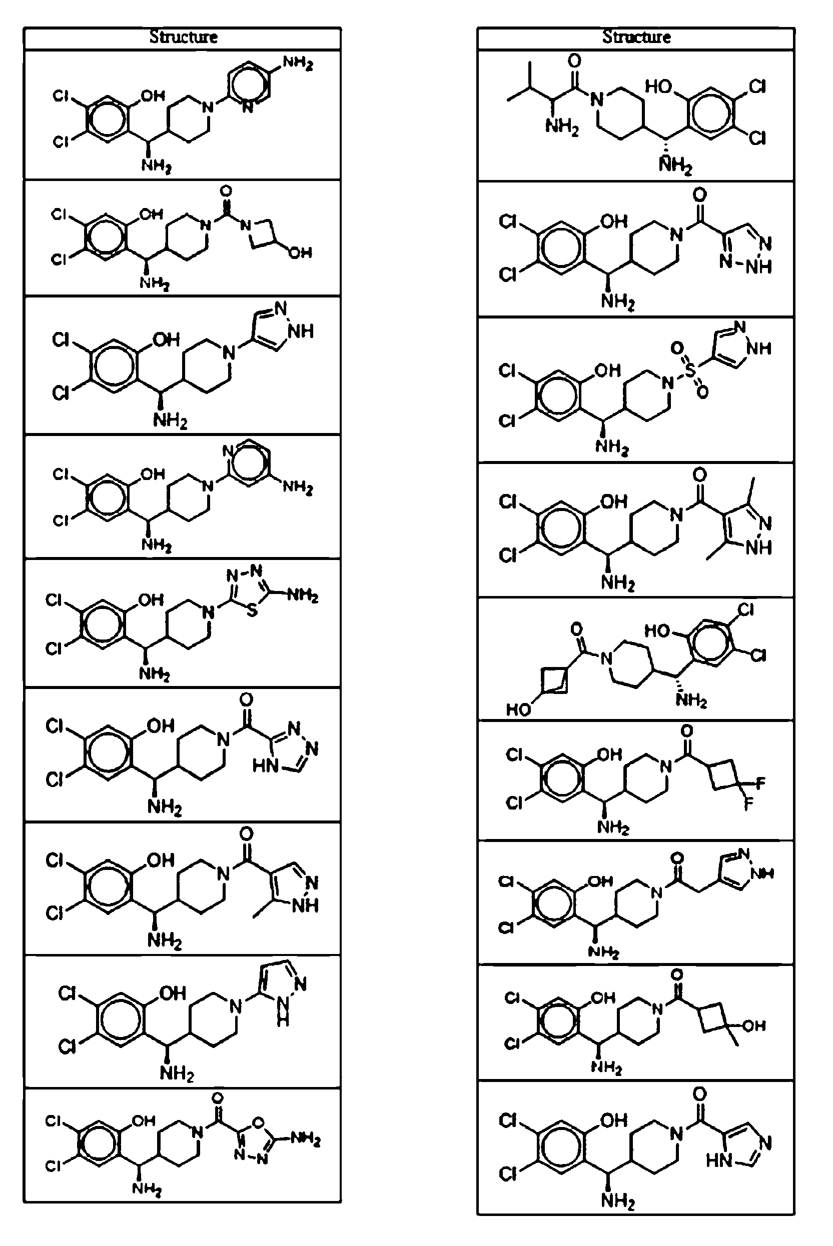

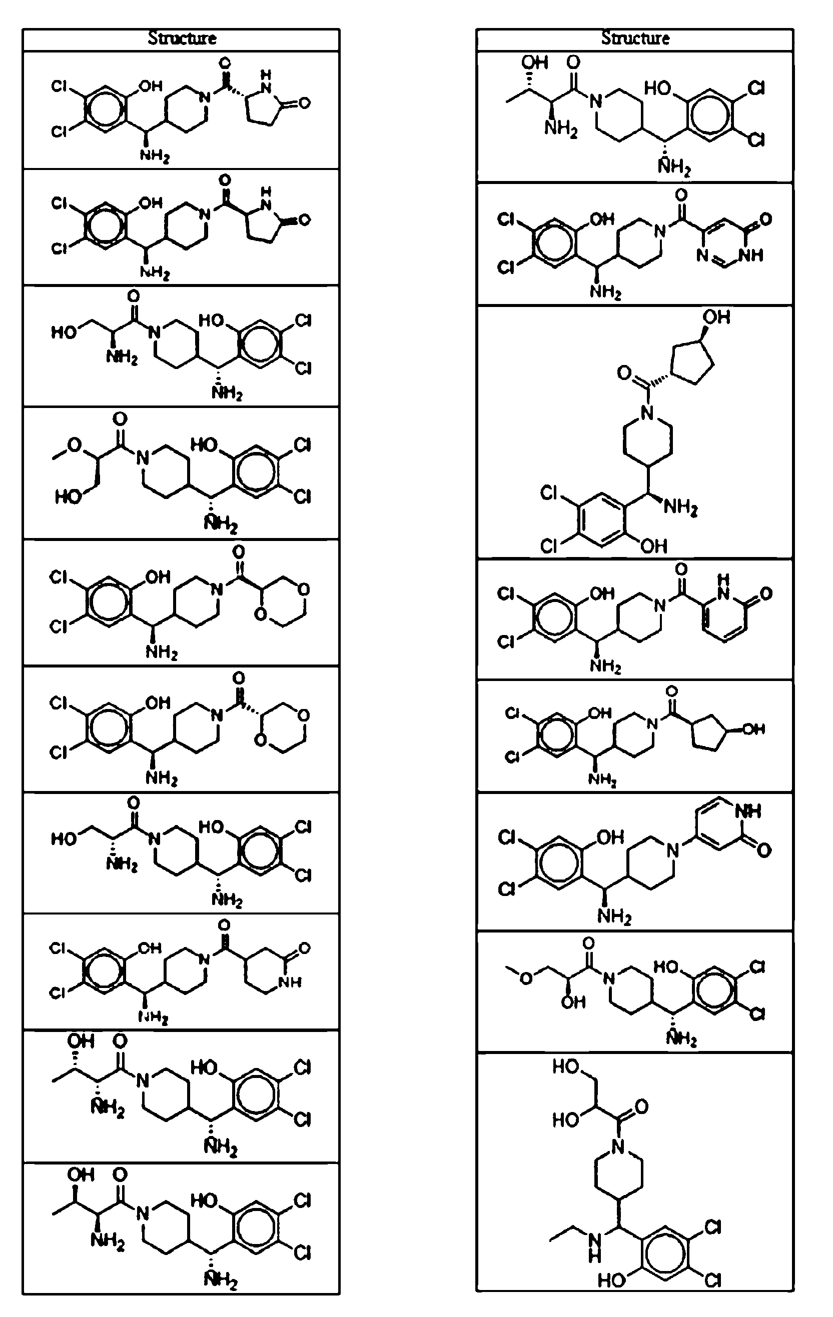

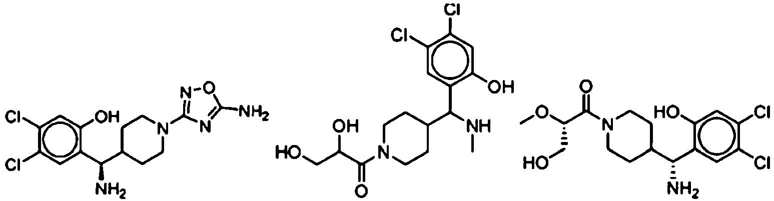

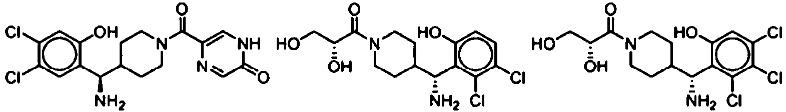

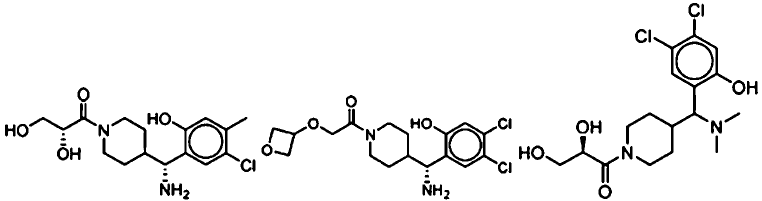

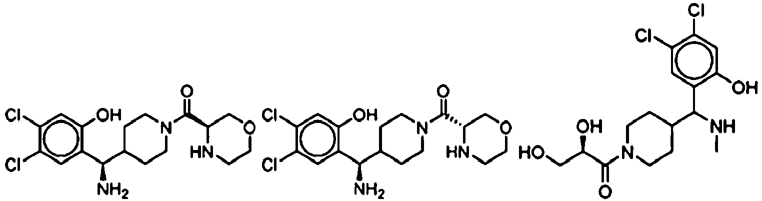

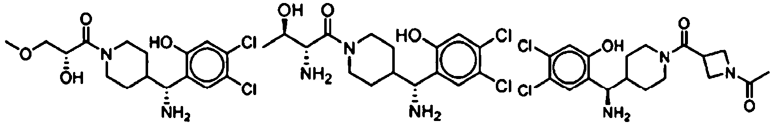

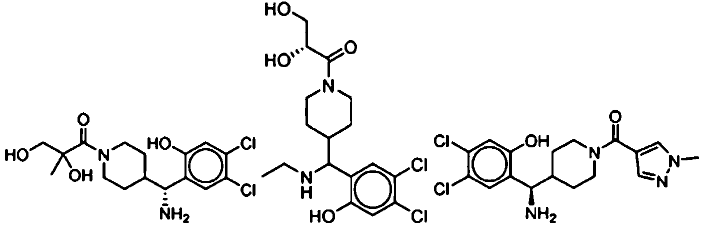

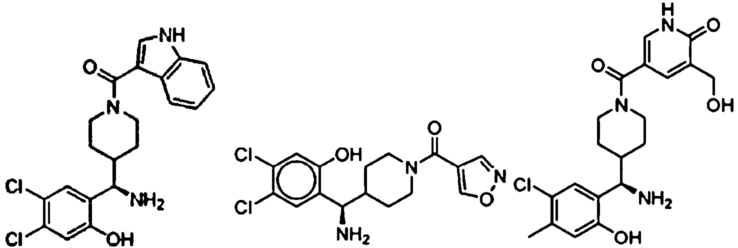

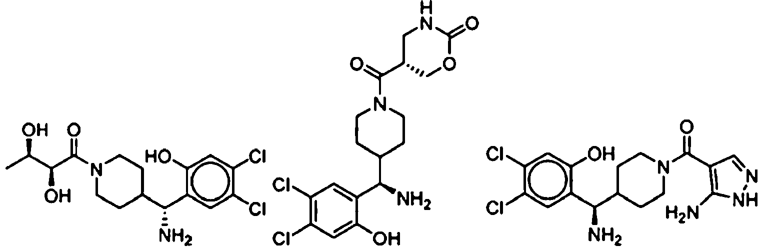

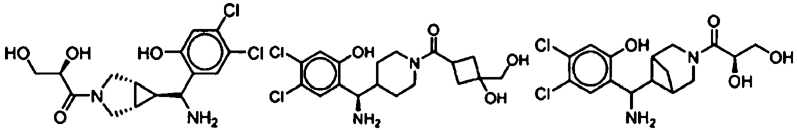

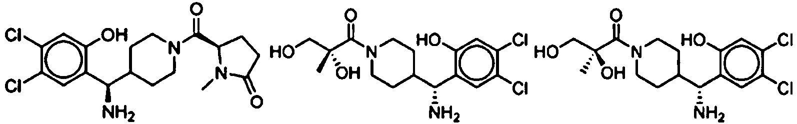

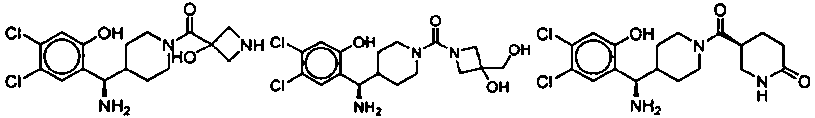

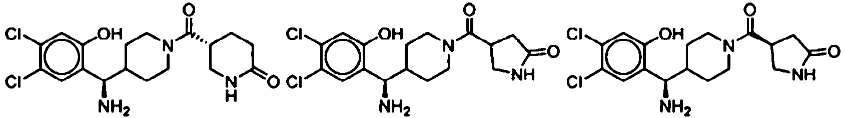

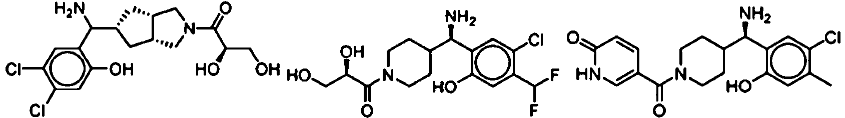

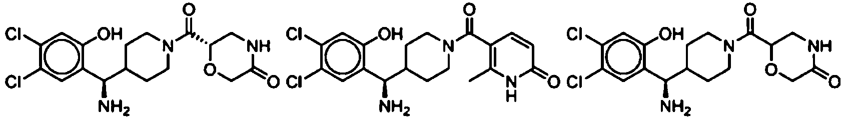

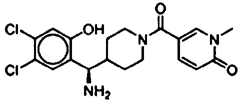

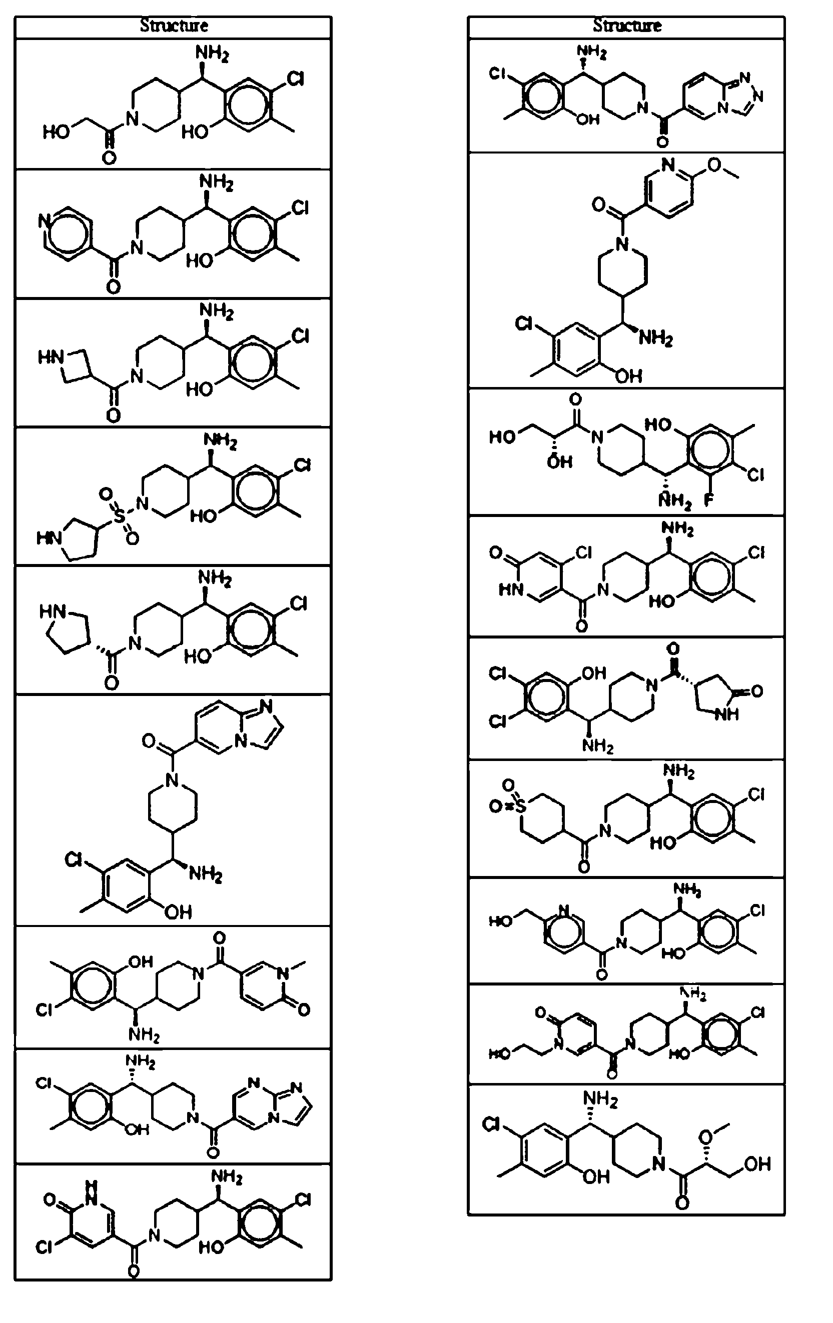

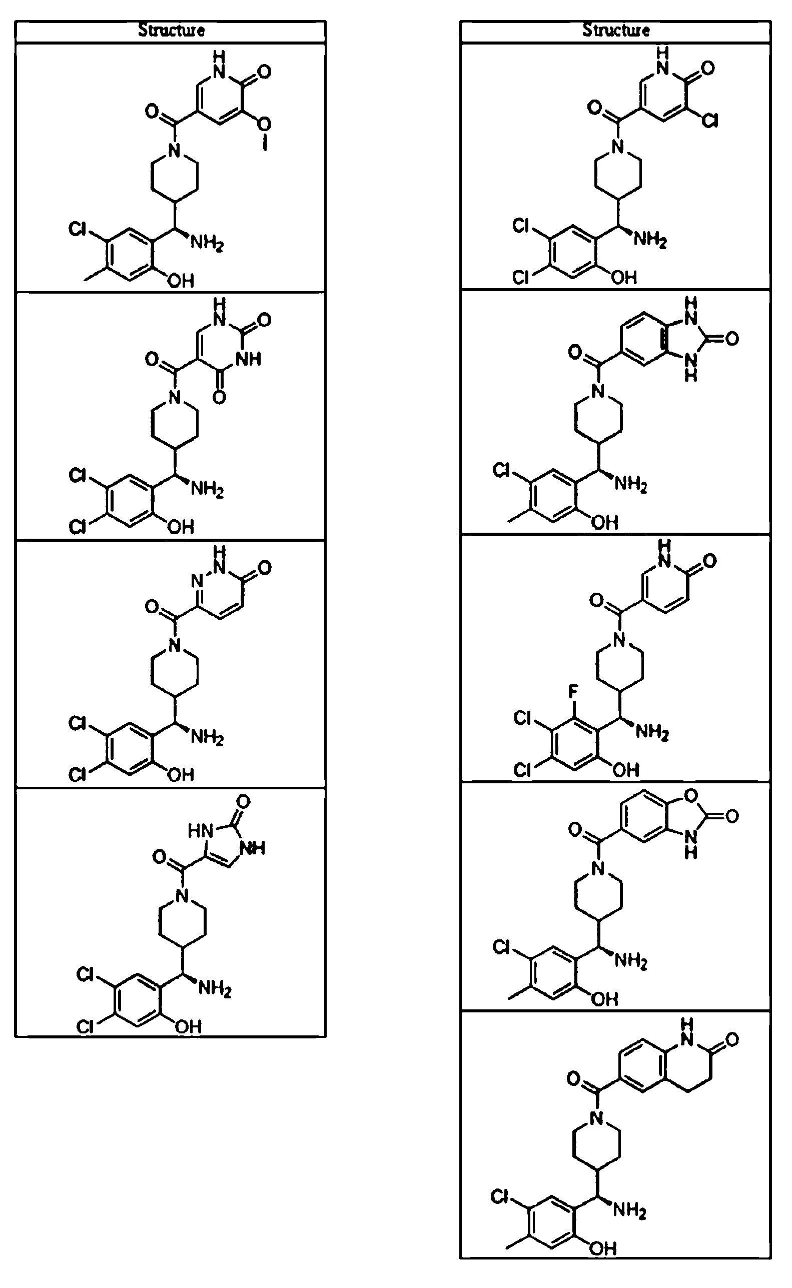

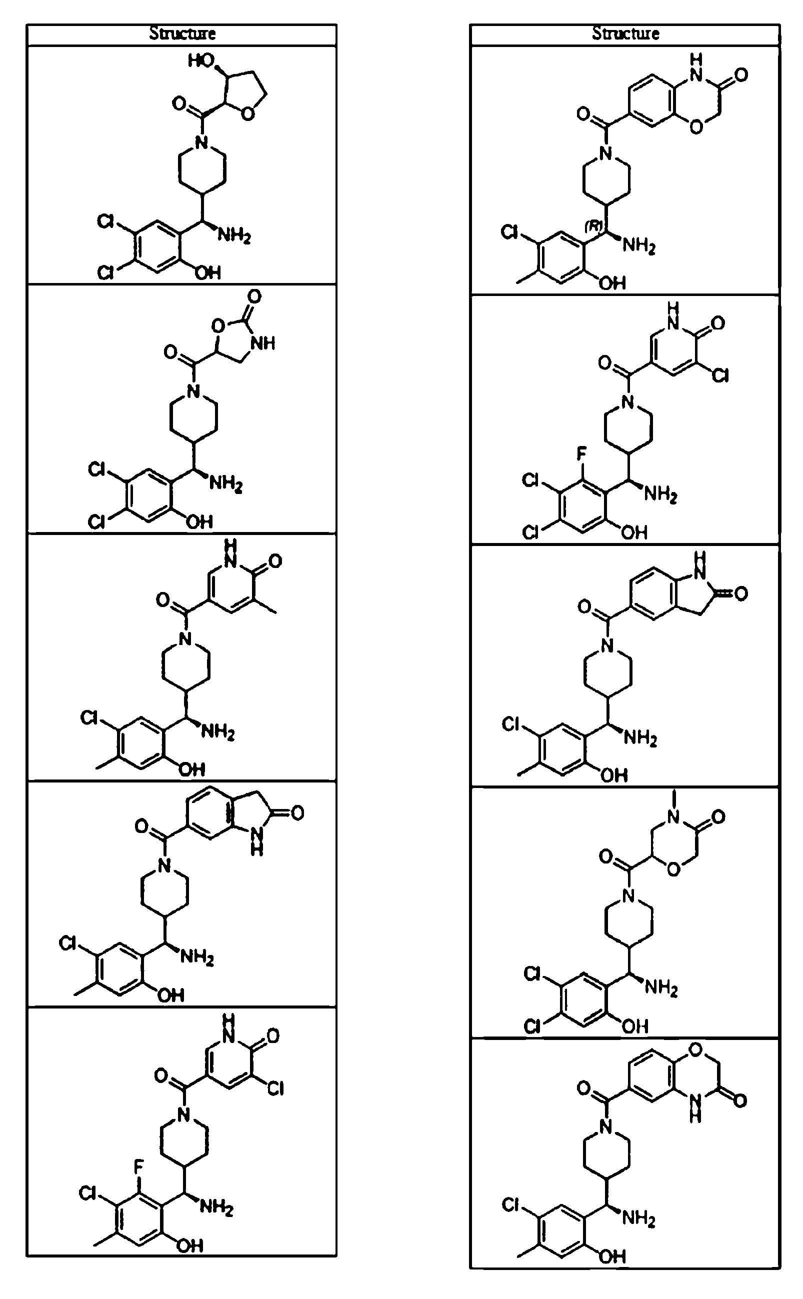

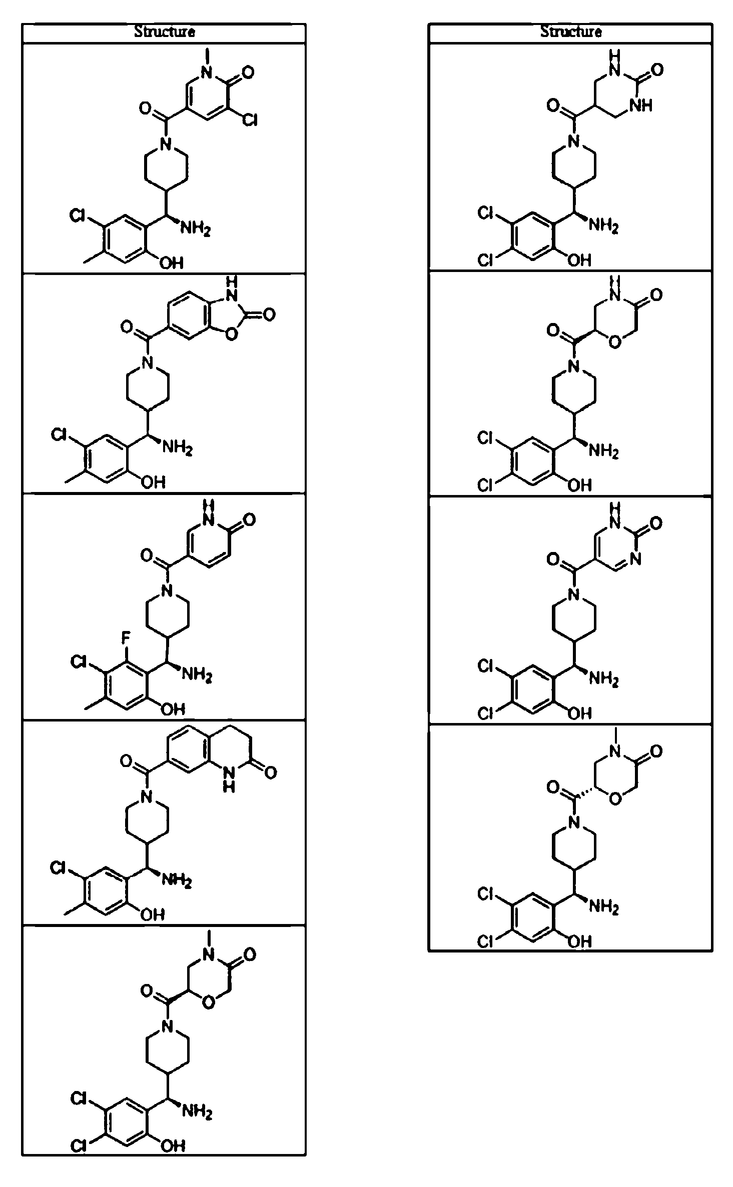

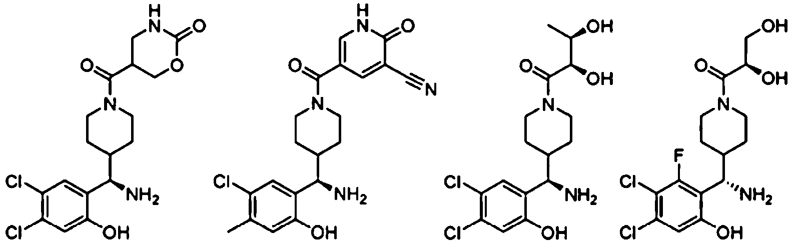

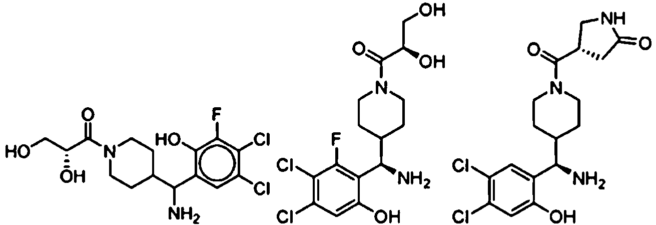

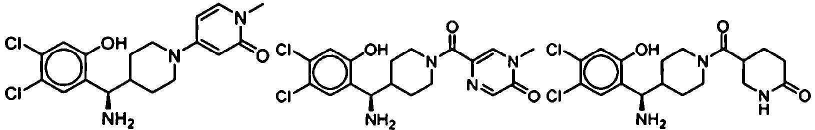

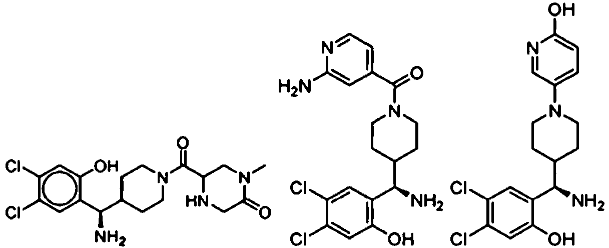

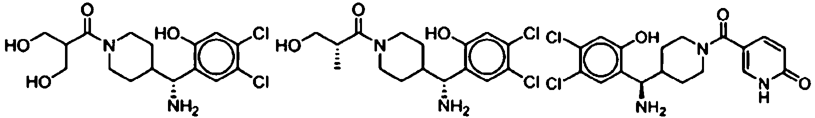

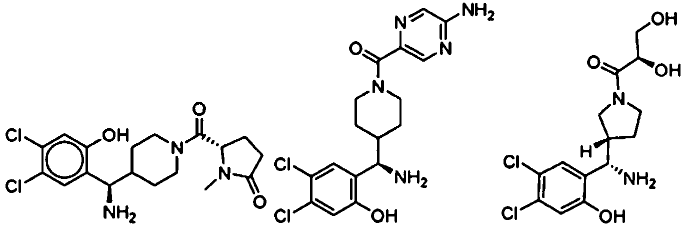

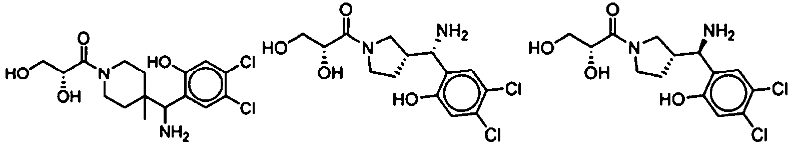

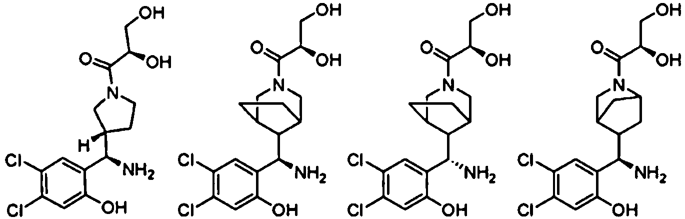

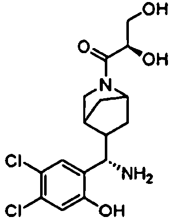

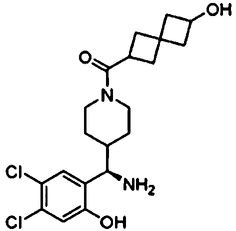

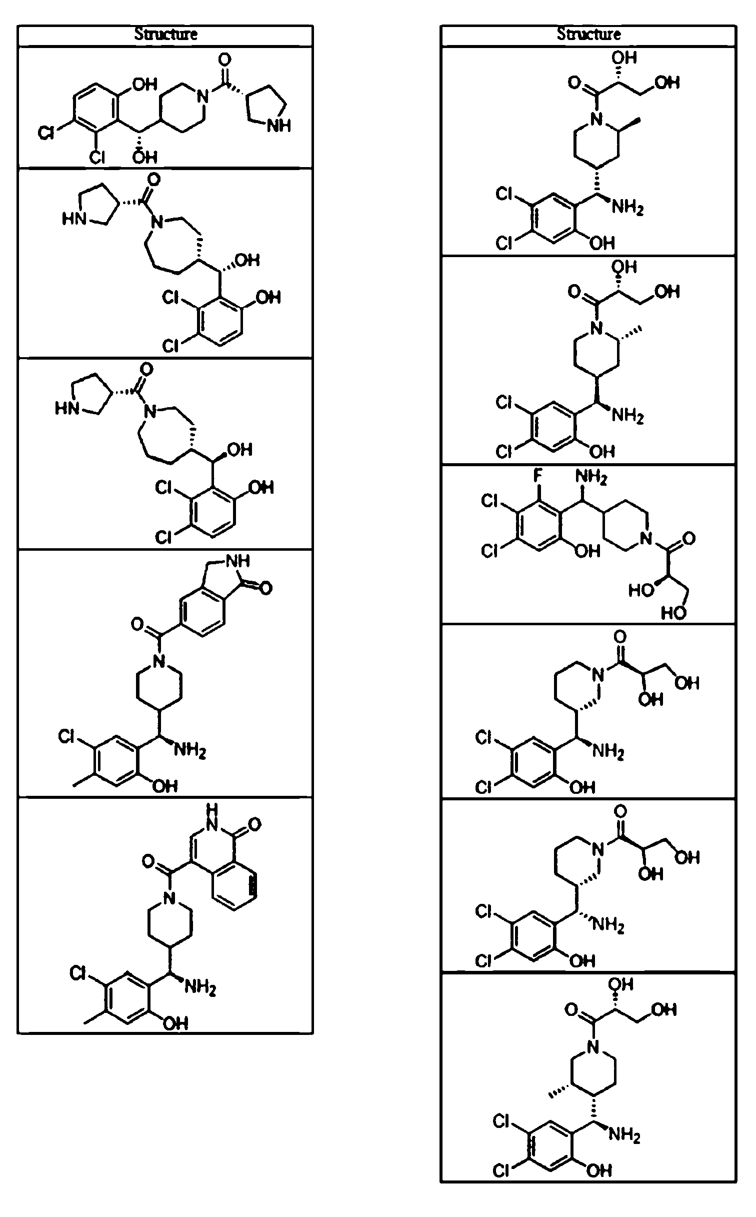

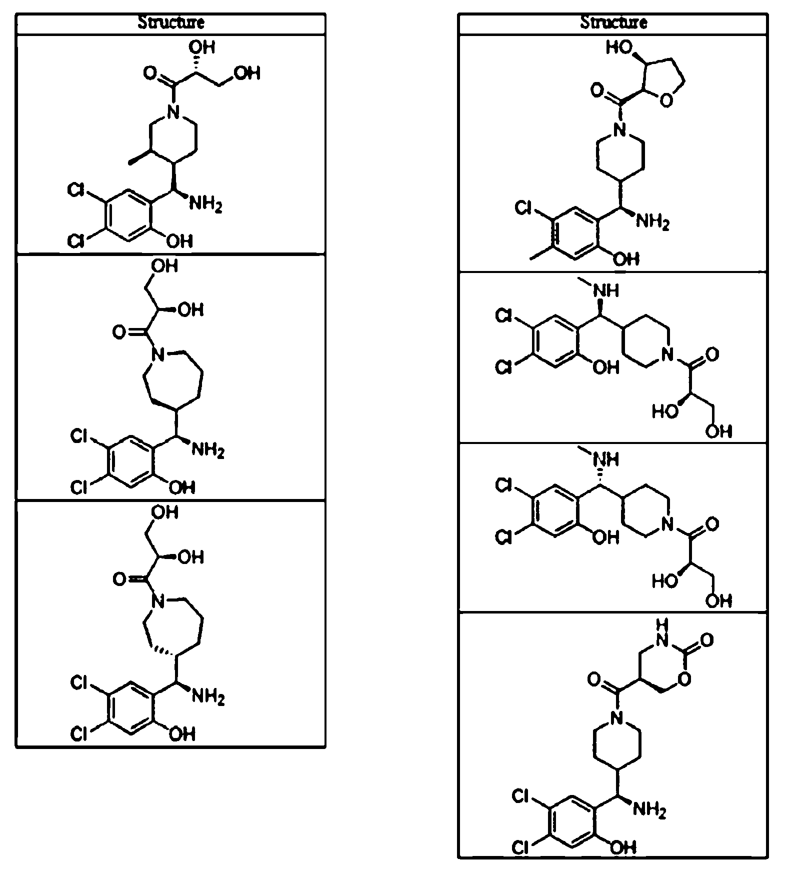

22. Composition pharmaceutique comprenant au moins un composé selon l'une quelconque des revendications 1 à 21 ou un sel pharmaceutiquement acceptable de celui-ci, et un vecteur ou diluant pharmaceutiquement acceptable.

23. Composé ou sel pharmaceutiquement acceptable de celui-ci selon l'une quelconque des revendications 1 à 21, destiné à être utilisé comme médicament.

24. Composé ou sel pharmaceutiquement acceptable de celui-ci selon l'une quelconque des revendications 1 à 21, destiné à être utilisé dans une méthode de traitement d'une affection chez une espèce mammifère qui en a besoin, la méthode comprenant l'administration à l'espèce mammifère d'une quantité thérapeutiquement efficace du composé ou d'un sel pharmaceutiquement acceptable de celui-ci, l'affection étant choisie dans le groupe constitué par le cancer, un trouble immunologique, un trouble du système nerveux central (SNC), un trouble inflammatoire, un trouble gastro-entérologique, un trouble métabolique, un trouble cardiovasculaire et une maladie rénale,
l'affection étant éventuellement :
(a) un trouble immunologique consistant en un rejet de greffe ou une maladie auto-immune, la maladie auto-immune étant éventuellement la polyarthrite rhumatoïde, la sclérose en plaques, le lupus érythémateux systémique ou le diabète de type 1, ou
(b) un trouble du système nerveux central (SNC) consistant en la maladie d'Alzheimer,
(c) un trouble inflammatoire consistant en une affection cutanée inflammatoire, l'arthrite, le psoriasis, la spondylarthrite, la parodontite ou une neuropathie inflammatoire,
(d) un trouble gastro-entérologique consistant en une maladie inflammatoire de l'intestin, ou
(e) un trouble métabolique consistant en l'obésité ou le diabète de type 2, ou
(f) un trouble cardiovasculaire consistant en un accident vasculaire cérébral ischémique, ou
(g) une maladie rénale consistant en une insuffisance rénale chronique, une néphrite ou une insuffisance rénale chronique, ou
(h) une affection choisie dans le groupe constitué par le cancer, les rejets de greffe, la polyarthrite rhumatoïde, la sclérose en plaques, le lupus érythémateux systémique, le diabète de type 1, la maladie d'Alzheimer, une affection cutanée inflammatoire, une neuropathie inflammatoire, le psoriasis, la spondylarthrite, la parodontite, la maladie de Crohn, la colite ulcéreuse, l'obésité, le diabète de type 2, un accident vasculaire cérébral ischémique, la maladie rénale chronique, une néphrite, l'insuffisance rénale chronique, et une combinaison de celles-ci, et
l'espèce mammifère étant éventuellement l'être humain.
